# EUROPEAN PATENT APPLICATION

(11) **EP 4 755 888 A1**
(43) Date of publication of application: **10.06.2026**
(21) Application number: 24849642.4
(22) Date of filing: 01.08.2024
(51) Int. Cl.: C07D 471/04, A61K 31/437, A61K 31/4375, A61P 35/00, C07D 513/04, C07D 487/10

(54) **NOVEL BIPHENYL DERIVATIVE AS PD-L1 INHIBITOR**

(30) Priority: 01.08.2023 KR 20230100507
(71) Applicant: ILAb Co., Ltd., Seongnam-si, Gyeonggi-do 13486 (KR)
(72) Inventor: KIM, Myung Jin, Seoul 05240 (KR); KANG, Yeo Wool, Incheon 21401 (KR); PARK, Cheon Hyeok, Seoul 06558 (KR); PARK, Young Gyun, Anyang-si Gyeonggi-do 14033 (KR); KIM, Ye Eun, Bucheon-si Gyeonggi-do 14583 (KR); SUNG, Jong Min, Seoul 05230 (KR); CHOI, Ga Ram, Incheon 22227 (KR); LEE, Sol, Seoul 02533 (KR); YANG, Deok Mo, Hwaseong-si Gyeonggi-do 18456 (KR); OH, Tae Gwon, Seoul 06346 (KR); KIM, Kwon Woo, Suwon-si Gyeonggi-do 16616 (KR)
(74) Representative: Klöckner, Christoph
(86) International application number: PCT/KR2024/011356
(87) International publication number: WO 2025/029075

(57) **Abstract**

The present invention relates to a novel compound exhibiting an interaction inhibitory effect between PD-1 and PD-L1. The novel biphenyl derivative of the present invention exhibits excellent cancer prevention and treatment effects through the
inhibitory effect.

## Description

### [Technical Field]

The present invention relates to a novel compound exhibiting an inhibitory effect on the interaction between PD-1 and PD-L1, and the novel biphenyl derivative of the present invention is excellent for the prevention and treatment of cancer through inhibition of PD-L1.

### [Background of the Invention]

Cancer, which is one of the leading causes of death worldwide, resulted in nearly ten million deaths in the year 2020 alone. Cancer generally causes from the transformation of normal cells into tumor cells, and treatment of cancer typically includes surgical intervention, radiation therapy, or systemic therapies such as chemotherapy, hormone therapy, and targeted biological therapy.

Cancer cells possess the ability to evade the immune system, and through this characteristic, cancer cells can avoid attacks by immune cells. Cancer cells express immunosuppressive signaling proteins, which cause dysfunction and apoptosis of immune cells, and one of the most well-known immunosuppressive signaling proteins is PD-L1 (programmed death-ligand-1).

PD-L1, also known as CD274 or B7-H1, is a trans-membrane protein that is regarded as a co-inhibitory factor in immune responses. PD-L1 binds to PD-1 (programmed cell death protein-1), thereby reducing the proliferation of PD-1 positive immune cells, and suppressing the secretion of cytokines required for immune responses or inducing apoptosis.

PD-1 regulates T-cell activity, activates apoptosis of antigen-specific T cells, and inhibits apoptosis of regulatory T cells, thereby playing an important role in suppressing immune responses and promoting immune tolerance (self-tolerance). PD-L1 is known to suppress anti-cancer immunity by binding to PD-1 expressed on T cells, B cells, dendritic cells, and NK T cells.

Meanwhile, immune checkpoint inhibitors, as a form of cancer immunotherapy, are a type of drug that blocks proteins known as immune checkpoints, which are expressed on certain immune cells such as T cells, and on cancer cells. These immune checkpoints function to prevent immune responses from becoming excessively strong; however, they can also inhibit T cells from killing cancer cells. Accordingly, blocking such immune checkpoints can restore immune system function and has therefore been useful in the treatment of cancer.

In this regard, PD-1 and PD-L1 inhibitors, representative immune checkpoint inhibitors, inhibit the binding between PD-1 and PD-L1 and block immune tolerance, thereby stimulating the activity of immune cells. Since many tumor cells highly express PD-L1, it has been demonstrated through animal studies and clinical results that inhibition of the interaction between PD-1 and PD-L1 enhances T-cell responses and activates antitumor activity in the body.

Accordingly, the present inventors have completed the present invention by developing novel biphenyl derivatives that are useful for the prevention and treatment of cancer through inhibitor of PD-1 or PD-L1, or through inhibition of the interaction between PD-1 and PD-L1.

### [Detailed Description of the Invention]

### [Technical Problem]

The present inventors have studied compounds having an inhibitory effect on the interaction between PD-1 and PD-L1, synthesized novel biphenyl derivatives, confirmed the structures of the novel compounds, and verified their inhibitory effects on the interaction between PD-1 and PD-L1, thereby completing the present invention.

Accordingly, an object of the present invention is to provide a novel compound that exhibits an inhibitory effect on the interaction between PD-1 and PD-L1 and is useful as an agent for the prevention or treatment of cancer.

### [Solution to the Problem]

The present invention provides a compound of the following Formula 1, or a pharmaceutically acceptable salt thereof: 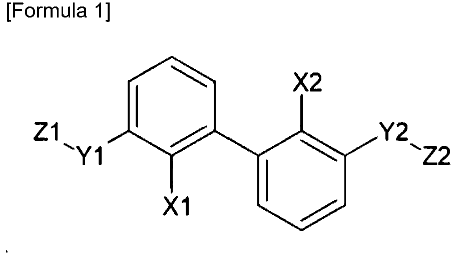 wherein,
X1 and X2 are each independently hydrogen, halo, haloalkyl, cyano, alkyl, alkenyl, alkynyl, - N(Ra)₂, -ORa, -SRa, cycloalkyl, heterocycloalkyl, aryl, or heteroaryl, wherein each Ra is independently hydrogen, halo, alkyl, alkenyl, alkynyl, cycloalkyl, heterocycloalkyl, aryl, or heteroaryl,
Y1 and Y2 are each independently a single bond, or alkyl, alkenyl, alkynyl, -ORb-, -N(Rb)-, - C(=O)Rb-, -C(=O)N(Rb)-, -C(=S)N(Rb)-, -N(Rb)C(=O)-, -N(Rb)S(=O)-, -N(Rb)S(=O)(=NH)-, - CH=C(Rb)-, -N(Rb)CH(OH)-, or -N(Rb)S(=O)₂-, wherein each Rb is independently hydrogen, halo, alkyl, alkenyl, alkynyl, cycloalkyl, heterocycloalkyl, aryl, or heteroaryl, and
Z1 and Z2 are each independently hydrogen, halo, cyano, alkyl, alkenyl, alkynyl, -C(=O)Rc, - C(=O)N(Rc)(Rd), -C(=O)ORc, -N(Rc)(Rd), -N(Rc)C(=O)Rd, -NH(Rc)C(=O)Rd, -NH(Rc)NHC(=O)Rd, - N(Rc)S(=O)Rd, -N(Rc)S(=O)₂Rd, -NO₂, -ORc, -OC(=O)Rc, -SRc, -S(=O)Rc, -S(=O)N(Rc)(Rd), - S(=O)₂Rc, -S(=O)₂N(Rc)(Rd), cycloalkyl, heterocycloalkyl, aryl, or heteroaryl, wherein Rc and Rd are each independently hydrogen, hydroxy, -COOH, alkyl, alkenyl, alkynyl, cycloalkyl, heterocycloalkyl, aryl, or heteroaryl,
wherein the alkyl, alkenyl, alkynyl, cycloalkyl, heterocycloalkyl, aryl, or heteroaryl may be unsubstituted or substituted.

In one embodiment, X1 and X2 are each independently hydrogen, halo, C₁₋₆ alkyl, C C₂₋₆ alkenyl, or C₂₋₆ alkynyl,
Y1 and Y2 are each independently a single bond, or C₁₋₆ alkyl, C₂₋₆ alkenyl, C₂₋₆ alkynyl, -ORb-, -N(Rb)-, -C(=O)Rb-, -C(=O)N(Rb)-, -N(Rb)C(=O)-, -N(Rb)S(=O)-, -N(Rb)S(=O)(=NH)-, -CH=C(Rb)-, - N(Rb)CH(OH)-, or -N(Rb)S(=O)₂-, wherein each Rb is independently hydrogen, halo, C₁₋₆ alkyl, C₂₋₆ alkenyl, or C₂₋₆ alkynyl, and
Z1 and Z2 are each independently hydrogen, C₁₋₆ alkyl, C₂₋₆ alkenyl, or C₂₋₆ alkynyl, - C(=O)N(Rc)(Rd), -C(=O)ORc, -N(Rc)(Rd), -N(Rc)C(=O)Rd, -N(Rc)S(=O)Rd, -N(Rc)S(=O)₂Rd, -NO₂, - ORc, -OC(=O)Rc, cycloalkyl, heterocycloalkyl, aryl, or heteroaryl, wherein Rc and Rd are each independently hydrogen, C₁₋₆ alkyl, C₂₋₆ alkenyl, or C₂₋₆ alkynyl, cycloalkyl, heterocycloalkyl, aryl, or heteroaryl,
wherein the alkyl, alkenyl, alkynyl, cycloalkyl, heterocycloalkyl, aryl, or heteroaryl may be unsubstituted or substituted.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, C₁₋₆ alkyl, C₂₋₆ alkenyl, or C₂₋₆ alkynyl, and
Z1 and Z2 are each independently hydrogen; aryl; heterocycloalkyl substituted with one to three heteroatoms selected from the group consisting of N, O, and S; or heteroaryl substituted with one to three heteroatoms selected from the group consisting of N, O, and S,
wherein the aryl, heterocycloalkyl, or heteroaryl may be unsubstituted or substituted with one or more substituents selected from the group consisting of halo, -OH, -CN, -C₁₋₆ alkyl, -OC₁₋₆ alkyl, haloalkyl, -C₁₋₆ alkyl -OH, -C₁₋₆ alkyl-COOH, -C₁₋₆ alkyl-NH-C₁₋₆ alkyl-COOH, -C₁₋₆ alkyl -NH-C₁₋₆ alkyl - NH-COOH, -C₁₋₆ alkyl-NH-C₁₋₆ alkyl-NH-C(=O)-C₁₋₆ alkyl, -C₁₋₆ alkyl -NH-C₁₋₆ alkyl-OH, -NH-C₁₋₆ alkyl, - N(C₁₋₆ alkyl)(C₁₋₆ alkyl), -NH-C₁₋₆ alkyl-OH, -N(C₁₋₆ alkyl-OH)(C₁₋₆ alkyl-OH), -N(C₁₋₆ alkyl-COOH)(C₁₋₆ alkyl-COOH), -NH-C₁₋₆ alkyl-(C₁₋₆ alkyl-OH)₂, -NH-C₁₋₆ alkyl-COOH, -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, -NH-C₁₋₆ alkyl-NH-SO₂-C₁₋₆ alkyl, heterocycloalkyl, -C₁₋₆ alkyl- heterocycloalkyl, -C₁₋₆ alkyl-NH-C₁₋₆ alkyl- heterocycloalkyl, -C₁₋₆ alkyl - bicycloalkyl -COOH, -NH- heterocycloalkyl, -NH-C₁₋₆ alkyl- heterocycloalkyl and -NH- cycloalkyl-OC₁₋₆ alkyl,
wherein the heterocycloalkyl and cycloalkyl may be unsubstituted or substituted with one or more substituents selected from the group consisting of halo, =O, -OH, -COOH, -C₁₋₆ alkyl-OH, and - C(=O) C₁₋₆ alkyl.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 and Z2 are each independently hydrogen, 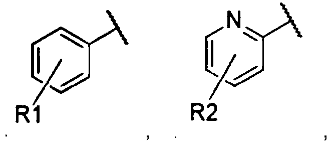 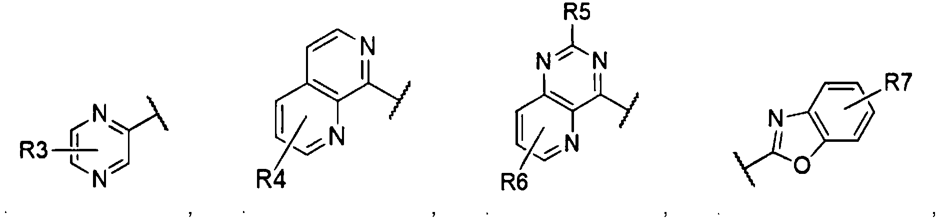 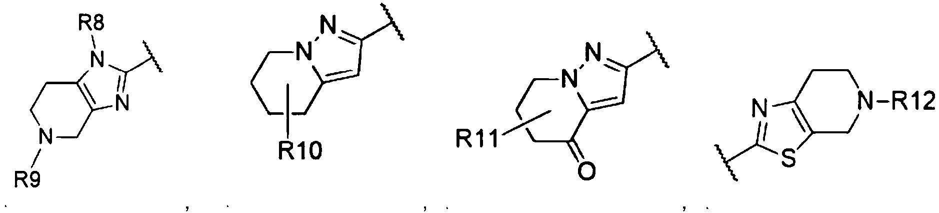 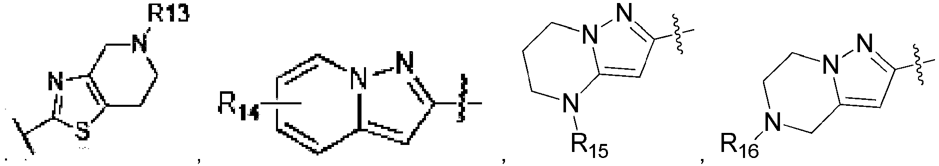 or 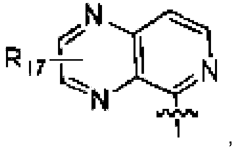
wherein R1 to R17 are each independently hydrogen, halo, -OH, -CN, -C₁₋₆ alkyl, -OC₁₋₆ alkyl, haloalkyl, -C₁₋₆ alkyl-OH, -C₁₋₆ alkyl-COOH, -C₁₋₆ alkyl-NH-C₁₋₆ alkyl-COOH, -C₁₋₆ alkyl-NH-C₁₋₆ alkyl-NH-COOH, -C₁₋₆ alkyl-NH-C₁₋₆ alkyl-NH-C(=O)-C₁₋₆ alkyl, -C₁₋₆ alkyl-NH-C₁₋₆ alkyl-OH, -NH-C₁₋₆ alkyl, - N(C₁₋₆ alkyl)(C₁₋₆ alkyl), -NH-C₁₋₆ alkyl-OH, -N(C₁₋₆ alkyl-OH)(C₁₋₆ alkyl-OH), -N(C₁₋₆ alkyl-COOH)(C₁₋₆ alkyl-COOH), -NH-C₁₋₆ alkyl-(C₁₋₆ alkyl-OH)₂, -NH-C₁₋₆ alkyl-COOH, -NH-C₁₋₆ alkyl-NH-C(=O)-C₁₋₆ alkyl, -NH-C₁₋₆ alkyl-C(=O)-OC₁₋₆ alkyl, -NH-C₁₋₆ alkyl-OC₁₋₆ alkyl, -NH-C₁₋₆ alkyl-NH-SO₂-C₁₋₆ alkyl, heterocycloalkyl, -C₁₋₆ alkyl-heterocycloalkyl, -C₁₋₆ alkyl-NH-C₁₋₆ alkyl-heterocycloalkyl, -C₁₋₆ alkyl-bicycloalkyl-COOH, -NH-heterocycloalkyl, -NH-C₁₋₆ alkyl-heterocycloalkyl, or -NH-cycloalkyl-OC₁₋₆ alkyl,
wherein thhe heterocycloalkyl and cycloalkyl may be unsubstituted or substituted with one or more substituents selected from the group consisting of halo, =O, -OH, -COOH, -C₁₋₆ alkyl-OH, and - C(=O)C₁₋₆ alkyl.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 and Z2 are each independently hydrogen, 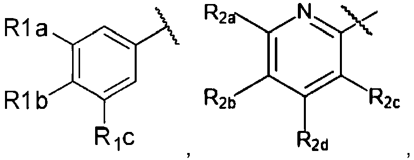 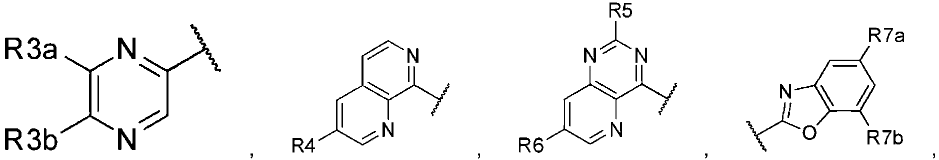 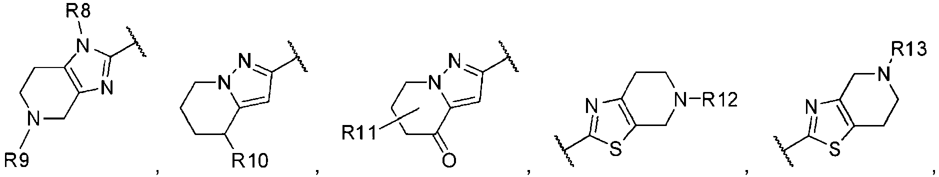 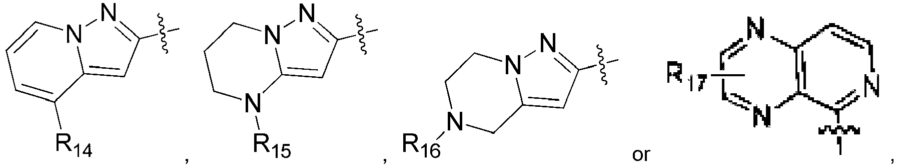
wherein R1a to R1c, R2a to R2d, R3a to R3b, R4 to R6, R7a to R7b, and R8 to R17 are each independently hydrogen, halo, -OH, -CN, -C₁₋₆ alkyl, haloalkyl, -C₁₋₆ alkyl-OH, -C₁₋₆ alkyl-COOH, -C₁₋₆ alkyl-NH-C₁₋₆ alkyl-COOH, -C₁₋₆ alkyl-NH-C₁₋₆ alkyl-NH-C(=O)-C₁₋₆ alkyl, -C₁₋₆ alkyl-NH-C₁₋₆ alkyl-OH, - NH-C₁₋₆ alkyl, -N(C₁₋₆ alkyl)(C₁₋₆ alkyl), -NH-C₁₋₆ alkyl-OH, -N(C₁₋₆ alkyl-OH)(C₁₋₆ alkyl-OH), -NH-C₁₋₆ alkyl-(C₁₋₆ alkyl-OH)₂, -NH-C₁₋₆ alkyl-COOH, -NH-C₁₋₆ alkyl-NH-C(=O)-C₁₋₆ alkyl, -NH-C₁₋₆ alkyl-C(=O)-OC₁₋₆ alkyl, -NH-C₁₋₆ alkyl-OC₁₋₆ alkyl, -NH-C₁₋₆ alkyl-NH-SO₂-C₁₋₆ alkyl, heterocycloalkyl, -C₁₋₆ alkyl-heterocycloalkyl, -C₁₋₆ alkyl-NH-C₁₋₆ alkyl-heterocycloalkyl, -C₁₋₆ alkyl-bicycloalkyl-COOH, -NH-heterocycloalkyl, -NH-C₁₋₆ alkyl-heterocycloalkyl, or -NH-cycloalkyl-OC₁₋₆ alkyl,
wherein the heterocycloalkyl is pyrrolidinyl or azetidinyl, which may be unsubstituted or substituted with one or more substituents selected from the group consisting of halo, =O, -OH, and - COOH, and
wherein the cycloalkyl may be unsubstituted or substituted with one or more substituents selected from the group consisting of =O, -OH, -COOH, -C₁₋₆ alkyl-OH, and -C(=O)C₁₋₆ alkyl.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 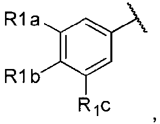
wherein R1a, R1b, and R1c are each independently hydrogen, halo, -C₁₋₆ alkyl, -OC₁₋₆ alkyl, -C₁₋₆ alkyl-NH-C₁₋₆ alkyl-heterocycloalkyl, -C₁₋₆ alkyl-heterocycloalkyl, -C₁₋₆ alkyl-NH-C₁₋₆ alkyl-COOH, -C₁₋₆ alkyl-NH-C₁₋₆ alkyl-NH-C(=O)-C₁₋₆ alkyl, or -C₁₋₆ alkyl-NH-C₁₋₆ alkyl-OH,
wherein the heterocycloalkyl may be unsubstituted or substituted with one or more substituents selected from the group consisting of =O, -OH, and -COOH.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 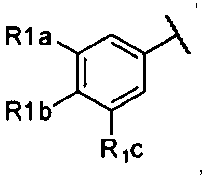
wherein R1a and R1c are each independently hydrogen, halo, -C₁₋₆ alkyl, or -OC₁₋₆ alkyl; and R1b is hydrogen, halo, -C₁₋₆ alkyl, -OC₁₋₆ alkyl, -C₁₋₆ alkyl-NH-C₁₋₆ alkyl-heterocycloalkyl, -C₁₋₆ alkyl-heterocycloalkyl, -C₁₋₆ alkyl-NH-C₁₋₆ alkyl-COOH, -C₁₋₆ alkyl-NH-C₁₋₆ alkyl-NH-C(=O)-C₁₋₆ alkyl, or -C₁₋₆ alkyl-NH-C₁₋₆ alkyl-OH, and
wherein the heterocycloalkyl may be

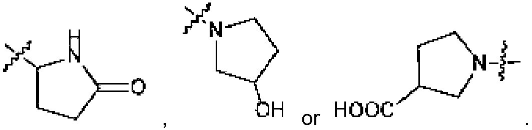

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be 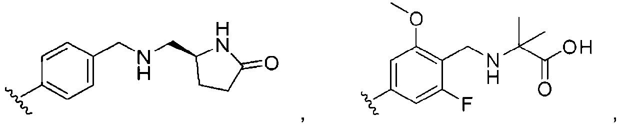 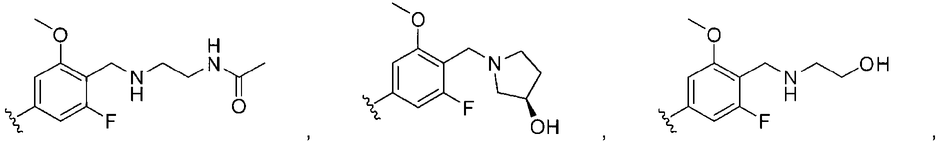 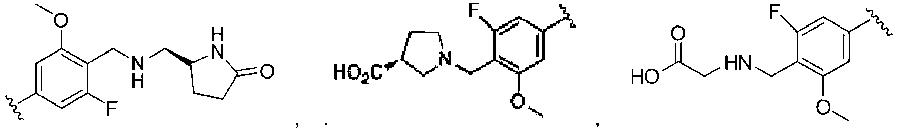 or

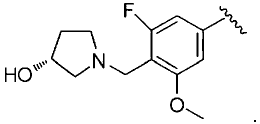

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 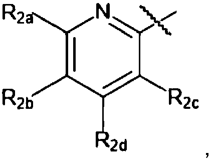
wherein R2a, R2b, R2c, and R2d are each independently hydrogen, halo, -C₁₋₆ alkyl, -OC₁₋₆ alkyl, -C₁₋₆ alkyl-heterocycloalkyl, or -C₁₋₆ alkyl-NH-C₁₋₆ alkyl-OH, and
wherein the heterocycloalkyl may be pyrrolidinyl, which may be unsubstituted or substituted with -OH.

In one embodiment, X1 and X2 are each independently hydrogen, halo, or ₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 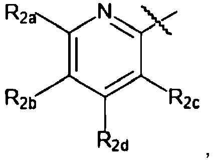
wherein R2a, R2b, R2c, and R2d are each independently hydrogen, halo, -C₁₋₆ alkyl, -OC₁₋₆ alkyl, -C₁₋₆ alkyl-heterocycloalkyl, or -C1-6 alkyl-NH-C1-6 alkyl-OH, and
wherein the heterocycloalkyl may be

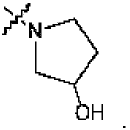

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

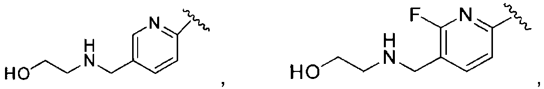

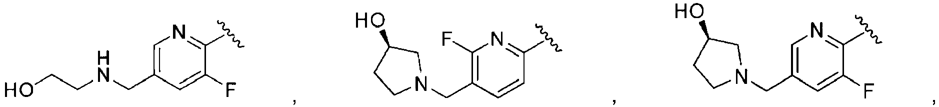

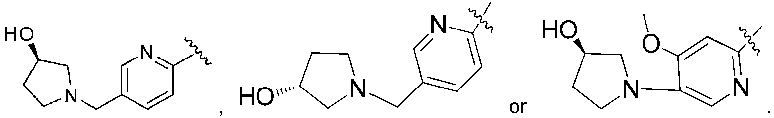

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 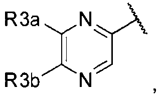
wherein R3a and R3b are each independently hydrogen, -OC₁₋₆ alkyl, or -C₁₋₆ alkyl-NH-C₁₋₆ alkyl-heterocycloalkyl, and
wherein the heterocycloalkyl may be pyrrolidinyl, which may be unsubstituted or substituted with =O.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 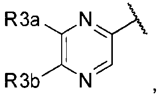
wherein R3a and R3b are each independently hydrogen, -OC₁₋₆ alkyl, or -C₁₋₆ alkyl-NH-C₁₋₆ alkyl-heterocycloalkyl, and
wherein the heterocycloalkyl may be

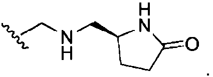

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

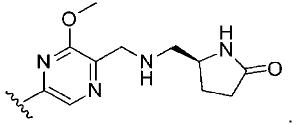

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 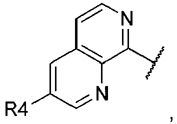
wherein R4 is hydrogen or -C₁₋₆ alkyl-heterocycloalkyl, and
wherein the heterocycloalkyl may be pyrrolidinyl or azetidinyl, which may be unsubstituted or substituted with -OH or halo.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 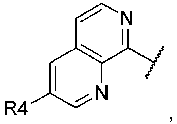
wherein R4 may be one or more selected from the group consisting of hydrogen and -C₁₋₆ alkyl-heterocycloalkyl, and
wherein the heterocycloalkyl may be

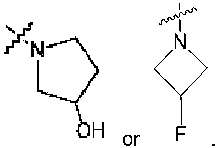

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be 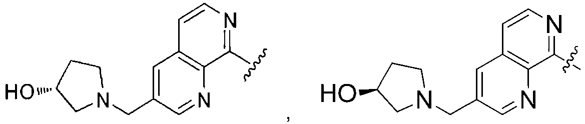 or

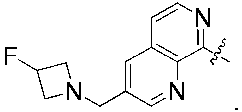

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 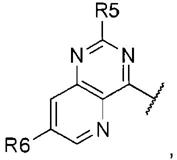
wherein R5 is hydrogen, -C₁₋₆alkyl, or haloalkyl, and
R6 is hydrogen or -C₁₋₆ alkyl-heterocycloalkyl,
wherein the heterocycloalkyl may be pyrrolidinyl or azetidinyl, which may be unsubstituted or substituted with -OH or halo.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 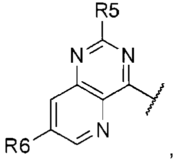
wherein R5 is hydrogen, -C₁₋₆alkyl, or haloalkyl, and
R6 is hydrogen or -C₁₋₆ alkyl-heterocycloalkyl,
wherein the heterocycloalkyl may be

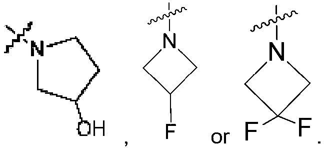

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be 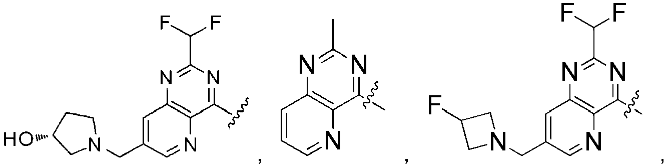 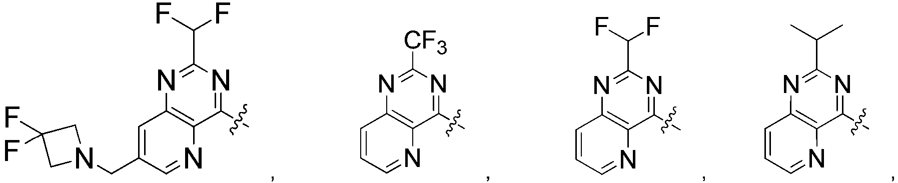 or

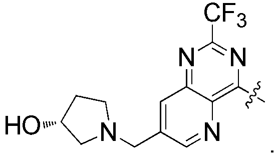

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 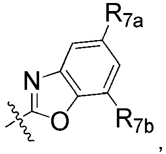
wherein R7a and R7b are each independently hydrogen, -CN, or -C₁₋₆ alkyl-heterocycloalkyl,
wherein the heterocycloalkyl may be pyrrolidinyl, which may be unsubstituted or substituted with -COOH.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

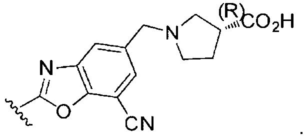

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 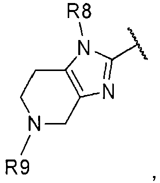
wherein R8 and R9 may each independently be hydrogen, -C₁₋₆ alkyl, or -C₁₋₆alkyl-bicycloalkyl-COOH.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

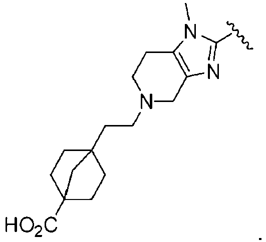

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 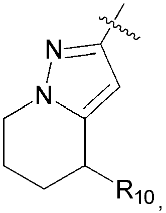
wherein R10 is hydrogen, halo, -OH, -C₁₋₆ alkyl, -NH-C₁₋₆ alkyl, -N(C₁₋₆ alkyl)(C₁₋₆ alkyl), -NH-C₁₋₆ alkyl-NH-C(=O)-C₁₋₆ alkyl, -NH-C₁₋₆ alkyl-C(=O)-OC₁₋₆ alkyl, -NH-C₁₋₆ alkyl-O C₁₋₆ alkyl, -OC₁₋₆ alkyl, heterocycloalkyl, -NH-heterocycloalkyl, -NH-C₁₋₆ alkyl-heterocycloalkyl, -NH-cycloalkyl, -NH-C1-6 alkyl-COOH, -NH-C1-6 alkyl-OH, -N(C₁₋₆ alkyl-OH)(C₁₋₆ alkyl-OH), -NH-C₁₋₆ alkyl-(C₁₋₆ alkyl-OH)₂, or -NH-C₁₋₆ alkyl-NH-SO₂-C₁₋₆ alkyl,
wherein the heterocycloalkyl and cycloalkyl may be unsubstituted or substituted with one or more substituents selected from the group consisting of =O, -OH, -COOH, -C₁₋₆ alkyl-OH, and -C(=O)C₁₋₆ alkyl.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 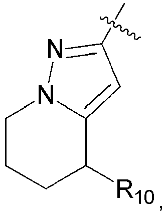
wherein R10 is hydrogen, -OH, -C₁₋₆ alkyl, -NH-C₁₋₆ alkyl, -N(C₁₋₆ alkyl)(C₁₋₆ alkyl), -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, heterocycloalkyl, -NH-heterocycloalkyl, -NH-C₁₋₆ alkyl-heterocycloalkyl, -NH-cycloalkyl, -NH-C₁₋₆ alkyl-COOH, -NH-C₁₋₆ alkyl-OH, -N(C₁₋₆ alkyl-OH)(C₁₋₆ alkyl-OH), -NH-C₁₋₆ alkyl-(C₁₋₆ alkyl-OH)₂, or - NH-C₁₋₆ alkyl-NH-SO₂-C₁₋₆ alkyl,
wherein the heterocycloalkyl and cycloalkyl may be

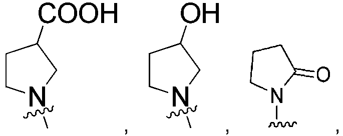

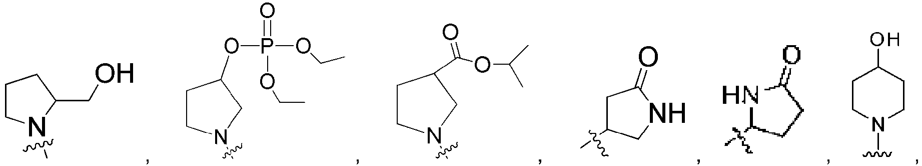

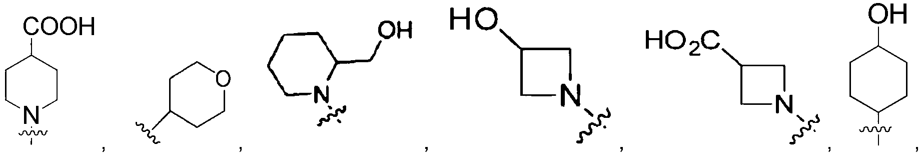

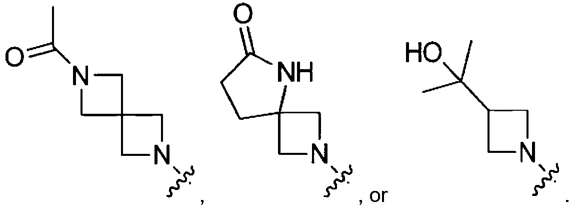

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 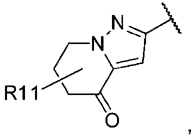
wherein R11 may be hydrogen, halo, or -C₁₋₆ alkyl.

In one embodiment, X1 and X2 are each independently hydrogen, halo, or C1₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

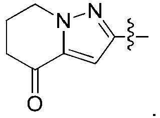

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 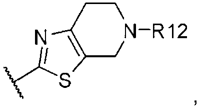
wherein R12 may be hydrogen, or -C₁₋₆ alkyl which may be unsubstituted or substituted with halo.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

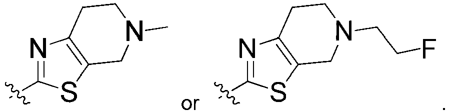

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 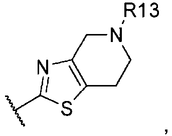
wherein R13 may be hydrogen or C₁₋₆ alkyl.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

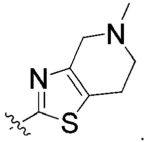

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 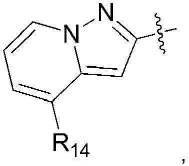
wherein R14 may be hydrogen, -C₁₋₆ alkyl, or -NH(C₁₋₆ alkyl)COOH.

In one embodiment, X1 and X2 are each independently hydrogen, halo, or C1-6 alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

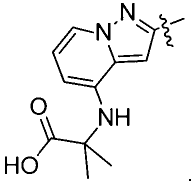

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 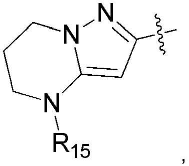
wherein R15 may be hydrogen, -C₁₋₆ alkyl, or -C₁₋₆ alkyl-OH.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

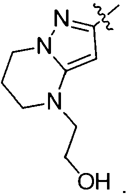

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 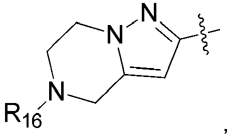
wherein R16 may be hydrogen, -C₁₋₆ alkyl, -C₁₋₆ alkyl-OH, or -C₁₋₆ alkyl-COOH.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

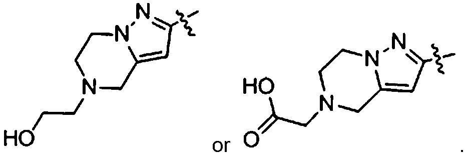

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 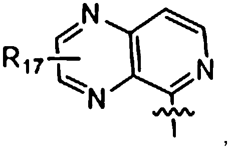
wherein R17 may be hydrogen or C₁₋₆ alkyl.

In one embodiment, X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 may be

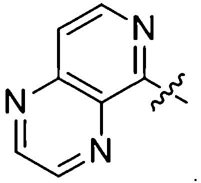

The present invention provides a composition for treating or preventing cancer, comprising the compound of the present invention.

The present invention provides a use of the compound or the composition of the present invention for treating or preventing cancer.

The present invention provides a method for treating cancer, comprising administering the compound of the present invention to a cancer patient.

The present invention provides a compound selected from the group consisting of the following compounds, or a pharmaceutically acceptable salt thereof.

**[Table 1]**

| No. | Chemical Structure | Chemical Name |
|---|---|---|
| 1 | 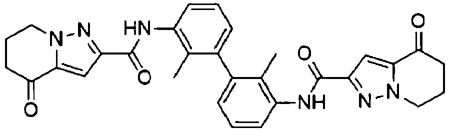 | N,N'-(2,2'-dimethyl-[1,1'-biphenyl]-3,3'-diyl)bis(4-oxo-4,5,6,7-tetrahydropyrazolo[1,5-a]pyridine-2-carboxamide |
| 2 | 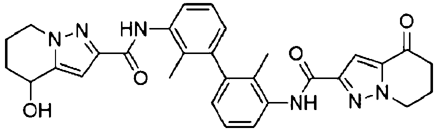 | N-(2,2'-dimethyl-3'-(4-oxo-4,5,6,7-tetrahydropyrazolo[1,5-a]pyridine-2-carboxamido)-[1,1'-biphenyl]-3-yl)-4-hydroxy-4,5,6,7-tetrahydropyrazolo[1,5-a]pyridine-2-carboxamide |
| 2-1 | 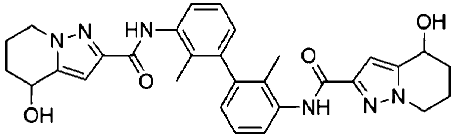 | N,N'-(2,2'-dimethyl-[1,1'-biphenyl]-3,3'-diyl)bis(4-hydroxy-4,5,6,7-tetrahydropyrazolo[1,5-a]pyridine-2-carboxamide |
| 3 | 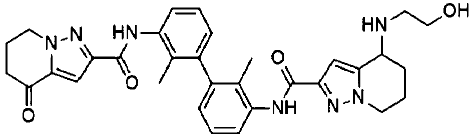 | N-(2,2'-dimethyl-3'-(4-oxo-4,5,6,7-tetrahydropyrazolo[1,5-a]pyridine-2-carboxamido)-[1,1'-biphenyl]-3-yl)-4-((2-hydroxyethyl)amino)-4,5,6,7-tetrahydropyrazolo[1,5-a]pyridine-2-carboxamide |
| 3-1 | 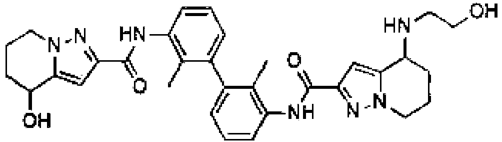 | 4-hydroxy-N-(3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)-4,5,6,7-tetrahydropyrazolo[1,5-a]pyridine-2-carboxamide |
| 4 | 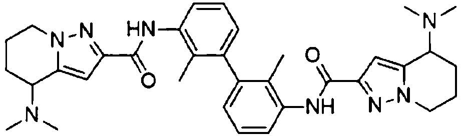 | N,N'-(2,2'-dimethyl-[1,1'-biphenyl]-3,3'-diyl)bis(4-(methylamino)-4,5,6,7-tetrahydropyrazolo[1,5-a]pyridine-2-carboxamide |
| 5 | 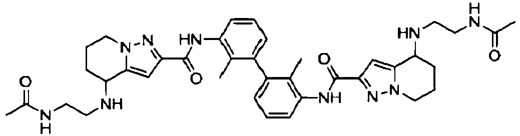 | N,N'-(2,2'-dimethyl-[1,1'-biphenyl]-3,3'-diyl)bis(4-((2-acetamidoethyl)amino)-4,5,6,7-tetrahydropyrazolo[1,5-a]pyridine-2-carboxamide |
| 6 | 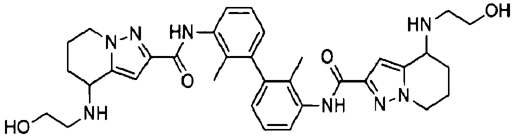 | N,N'-(2,2'-dimethyl-[1,1'-biphenyl]-3,3'-diyl)bis(4-((2-hydroxyethyl)amino)-4,5,6,7-tetrahydropyrazolo[1,5-a]pyridine-2-carboxamide |
| 8 | 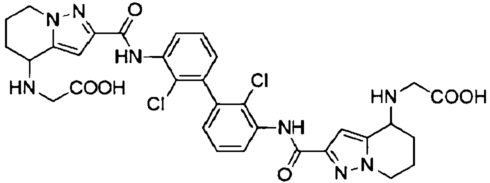 | 2-[[2-[[3-[3-[[4-(carboxymethylamino)-4,5,6,7-tetrahydropyrazolo[1,5-a]pyridine-2-carbonyl]amino]-2-chloro-phenyl]-2-chlorophenyl]carbamoyl]-4,5,6,7-tetrahydropyrazolo[1,5-a]pyridin-4-yl]amino]acetic acid |
| 11 | 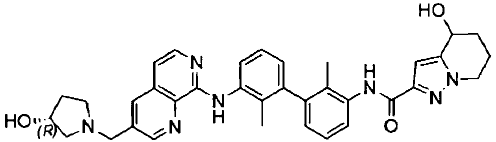 | 4-hydroxy-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide |
| 12 | 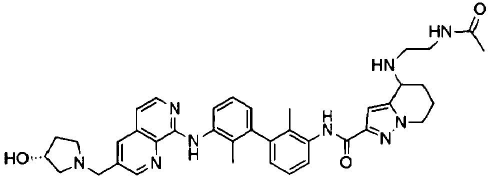 | 4-((2-acetamidoethyl)amino)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide |
| 13 | 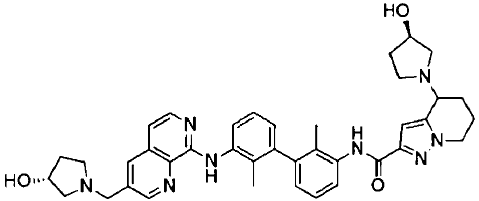 | 4-((R)-3-hydroxypyrrolidin-1-yl)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide |
| 14 | 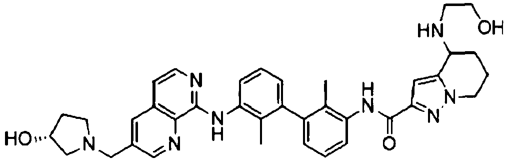 | 4-((2-hydroxyethyl)amino)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide |
| 15 | 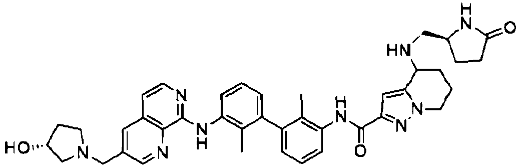 | N-(3'-((3-(((R)-3-hydroxypyrrolidin-1-yl)methyl)-1,7-naphthyridin-8-yl)amino)-2,2'-dimethyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 16 | 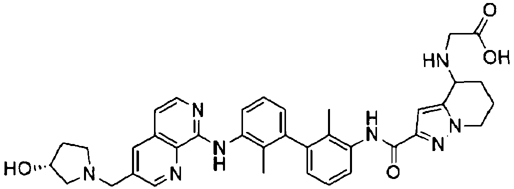 | 2-((2-((3'-((3-(((R)-3-hydroxypyrrolidin-1-yl)methyl)-1,7-naphthyridin-8-yl)amino)-2,2'-dimethyl-[1,1'-biphenyl]-3-yl)carbamoyl)-4,5,6,7-tetrahydropyrazolo[1,5-a]pyridin-4-yl)amino)acetic acid |
| 17 | 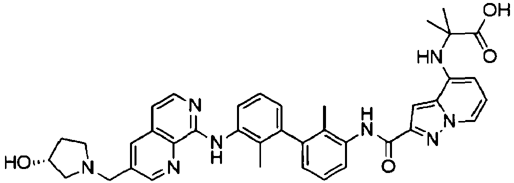 | (R)-2-((2-((3'-((3-((3-hydroxypyrrolidin-1-yl)methyl)-1,7-naphthyridin-8-yl)amino)-2,2'-dimethyl-[1,1'-biphenyl]-3-yl)carbamoyl)pyrazolo[1,5-a]pyridin-4-yl)amino)-2-methylpropanoic acid |
| 18 | 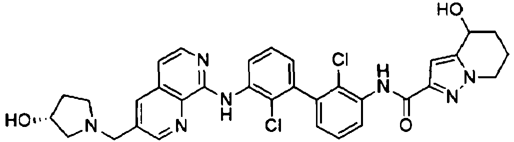 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-hydroxy-4,5,6,7-tetrahydropyrazolo[1,5-a]pyridine-2-carboxamide |
| 19 | 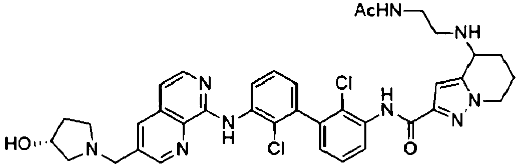 | 4-((2-acetamidoethyl)amino)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridine-2-carboxamide |
| 20 | 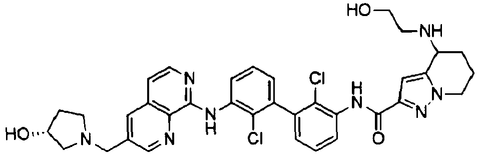 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((2-hydroxyethyl)amino)-4,5,6,7-tetrahydropyrazolo[1,5-a]pyridine-2-carboxamide |
| 21 | 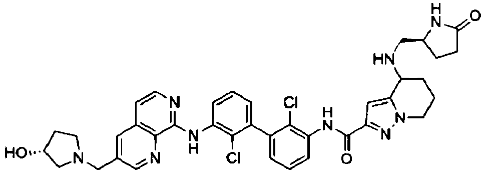 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 22 | 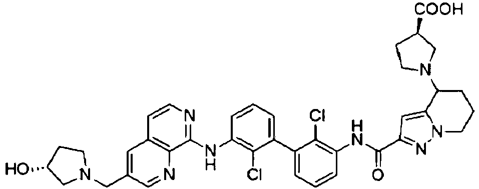 | (3R)-1-(2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)pyrrolidine-3-carboxylic acid |
| 23 | 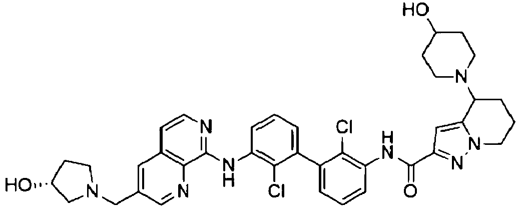 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 24 | 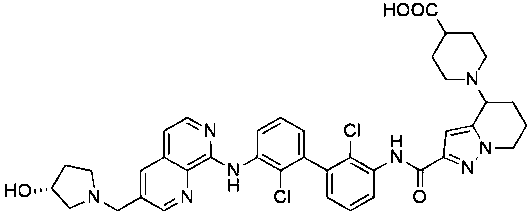 | 1-(2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid |
| 25 | 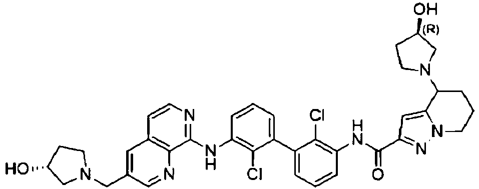 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 26 | 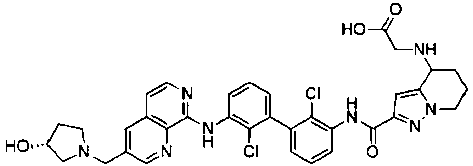 | 2-((2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetic acid |
| 27 | 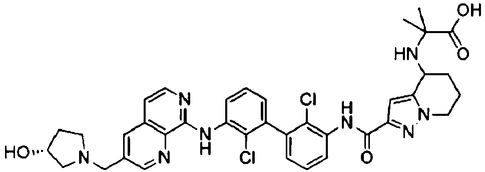 | 2-((2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)-2-methylpropanoic acid |
| 28 | 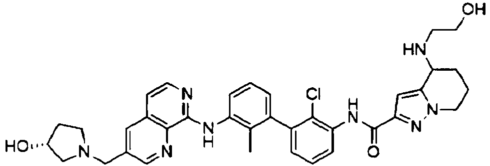 | N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-((2-hydroxyethyl)amino)-4,5,6,7-tetrahydropyrazolo[1,5-a]pyridine-2-carboxamide |
| 29 | 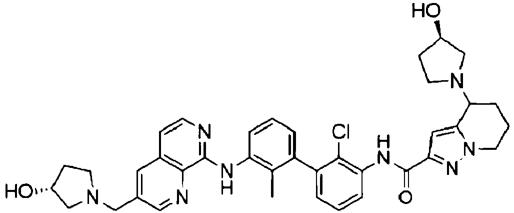 | N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-((R)-3-hydroxypyrrolidin-1-yl)-4,5,6,7-tetrahydropyrazolo[1,5-a]pyridine-2-carboxamide |
| 30 | 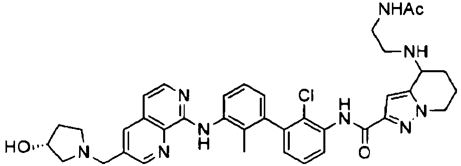 | 4-((2-acetamidoethyl)amino)-N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4,5,6,7-tetrahydropyrazolo[1,5-a]pyridine-2-carboxamide |
| 31 | 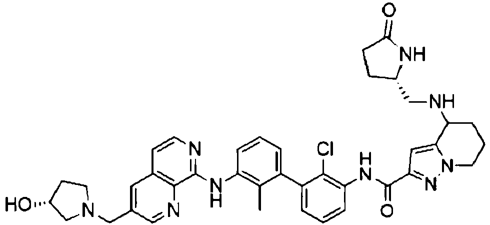 | N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 32 | 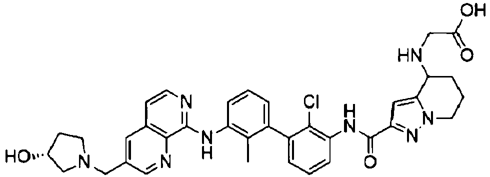 | 2-((2-((2-chloro-3'-((3-(((R)-3-hydroxypyrrolidin-1-yl)methyl)-1,7-naphthyridin-8-yl)amino)-2'-methyl-[1,1'-biphenyl]-3-yl)carbamoyl)-4,5,6,7-tetrahydropyrazolo[1,5-a]pyridin-4-yl)amino)acetic acid |
| 33 | 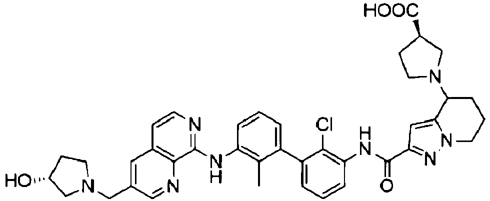 | (3R)-1-(2-((2-chloro-3'-((3-(((R)-3-hydroxypyrrolidin-1-yl)methyl)-1,7-naphthyridin-8-yl)amino)-2'-methyl-[1,1'-biphenyl]-3-yl)carbamoyl)-4,5,6,7-tetrahydropyrazolo[1,5-a]pyridin-4-yl)pyrrolidine-3-carboxylic acid |
| 34 | 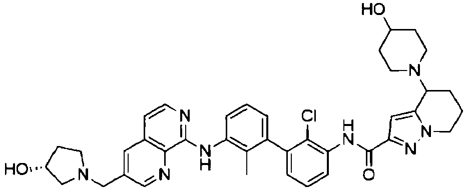 | N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-(4-hydroxypiperidin-1-yl)-4,5,6,7-tetrahydropyrazolo[1,5-a]pyridine-2-carboxamide |
| 35 | 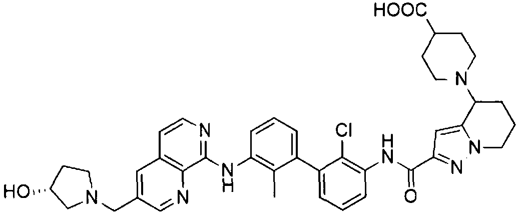 | 1-(2-((2-chloro-3'-((3-(((R)-3-hydroxypyrrolidin-1-yl)methyl)-1,7-naphthyridin-8-yl)amino)-2'-methyl-[1,1'-biphenyl]-3-yl)carbamoyl)-4,5,6,7-tetrahydropyrazolo[1,5-a]pyridin-4-yl)piperidine-4-carboxylic acid |
| 36 | 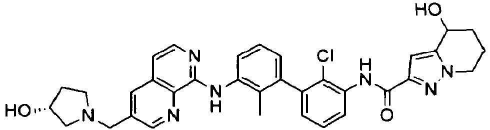 | N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-hydroxy-4,5,6,7-tetrahydropyrazolo[1,5-a]pyridine-2-carboxamide |
| 37 | 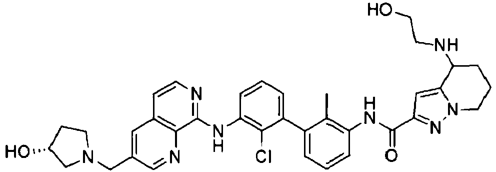 | N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4-((2-hydroxyethyl)amino)-4,5,6,7-tetrahydropyrazolo[1,5-a]pyridine-2-carboxamide |
| 38 | 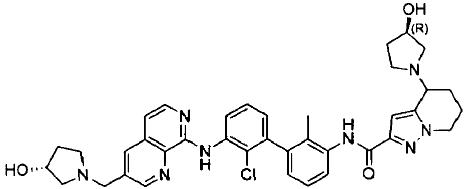 | N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4-((R)-3-hydroxypyrrolidin-1-yl)-4,5,6,7-tetrahydropyrazolo[1,5-a]pyridine-2-carboxamide |
| 39 | 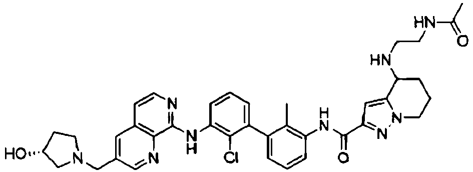 | 4-((2-acetamidoethyl)amino)-N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4,5,6,7-tetrahydropyrazolo[1,5-a]pyridine-2-carboxamide |
| 40 | 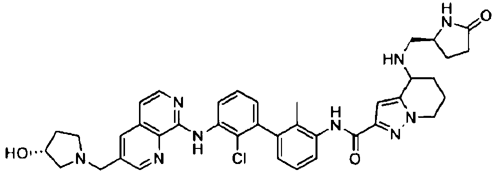 | N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 41 | 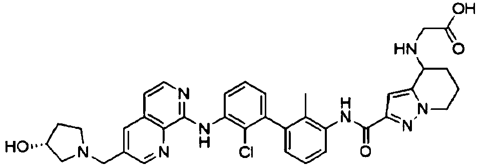 | 2-((2-((2'-chloro-3'-((3-(((R)-3-hydroxypyrrolidin-1-yl)methyl)-1,7-naphthyridin-8-yl)amino)-2-methyl-[1,1'-biphenyl]-3-yl)carbamoyl)-4,5,6,7-tetrahydropyrazolo[1,5-a]pyridin-4-yl)amino)acetic acid |
| 42 | 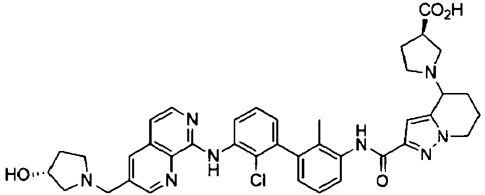 | (3R)-1-(2-((2'-chloro-3'-((3-(((R)-3-hydroxypyrrolidin-1-yl)methyl)-1,7-naphthyridin-8-yl)amino)-2-methyl-[1,1'-biphenyl]-3-yl)carbamoyl)-4,5,6,7-tetrahydropyrazolo[1,5-a]pyridin-4-yl)pyrrolidine-3-carboxylic acid |
| 43 | 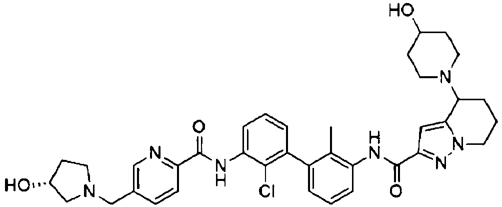 | N-(2'-chloro-3'-(5-(((R)-3-hydroxypyrrolidin-1-yl)methyl)picolinamido)-2-methyl-[1,1'-biphenyl]-3-yl)-4-(4-hydroxypiperidin-1-yl)-4,5,6,7-tetrahydropyrazolo[1,5-a]pyridine-2-carboxamide |
| 44 | 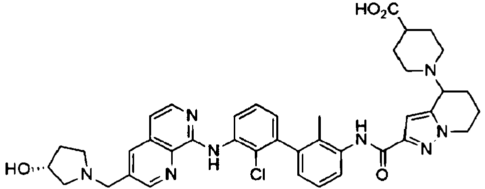 | 1-(2-((2'-chloro-3'-((3-(((R)-3-hydroxypyrrolidin-1-yl)methyl)-1,7-naphthyridin-8-yl)amino)-2-methyl-[1,1'-biphenyl]-3-yl)carbamoyl)-4,5,6,7-tetrahydropyrazolo[1,5-a]pyridin-4-yl)piperidine-4-carboxylic acid |
| 45 | 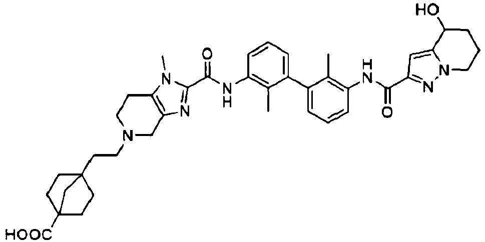 | 4-(2-(2-((3'-(4-hydroxy-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 46 | 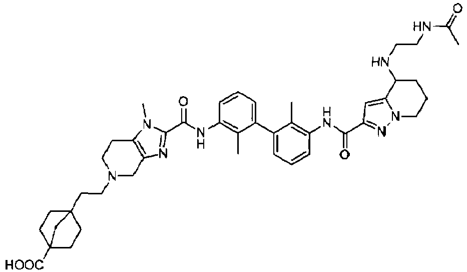 | 4-(2-(2-((3'-(4-((2-acetamidoethyl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 47 | 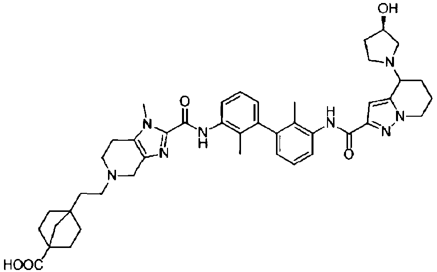 | 4-(2-(2-((3'-(4-((R)-3-hydroxypyrrolidin-1-yl)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-ylethyl)bicyclo[2.2.1]heptane-1-carboxylic acid |
| 48 | 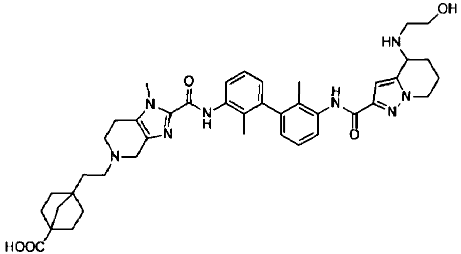 | 4-(2-(2-((3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 49 | 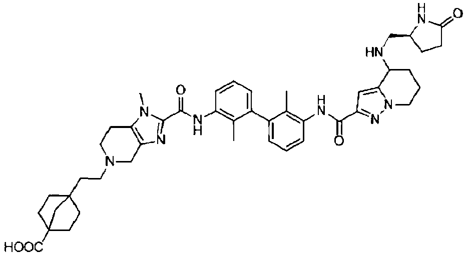 | 4-(2-(2-((2,2'-dimethyl-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 50 | 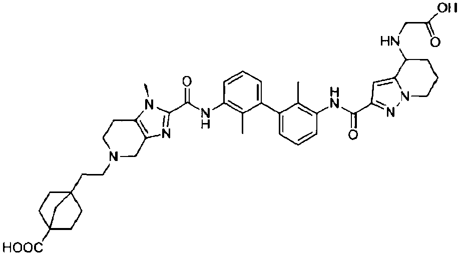 | 4-(2-(2-((3'-(4-((carboxymethyl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 51 | 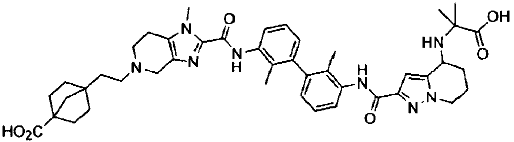 | 4-(2-(2-((3'-(4-((2-carboxypropan-2-yl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 52 | 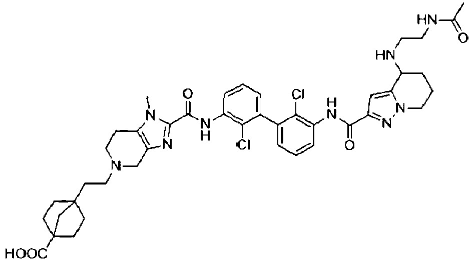 | 4-(2-(2-((3'-(4-((2-acetamidoethyl)amino)-4,5,6,7-tetrahydropyrazolo[1,5-a]pyridine-2-carboxamido)-2,2'-dichloro-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 53 | 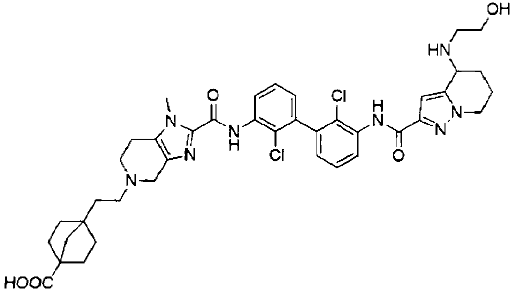 | 4-(2-(2-((2,2'-dichloro-3'-(4-((2-hydroxyethyl)amino)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 54 | 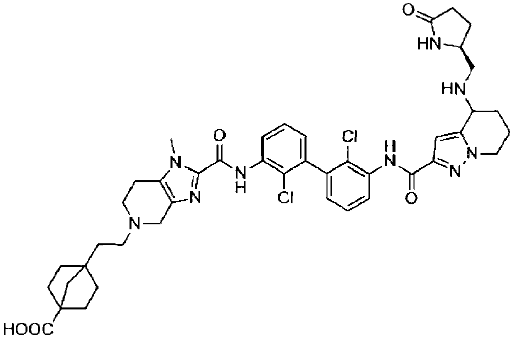 | 4-(2-(2-((2,2'-dichloro-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 56 | 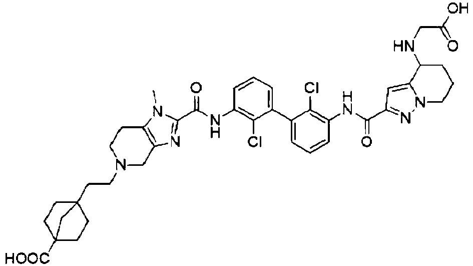 | 4-(2-(2-((3'-(4-((carboxymethyl)amino)-4,5,6,7-tetrahydropyrazolo[1,5-a]pyridine-2-carboxamido)-2,2'-dichloro-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 57 | 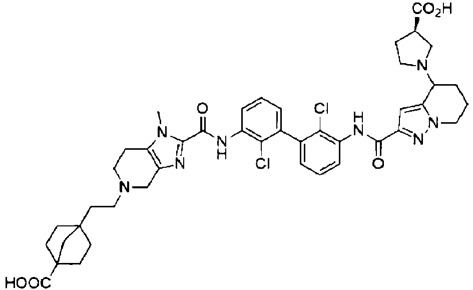 | (3R)-1-(2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid |
| 58 | 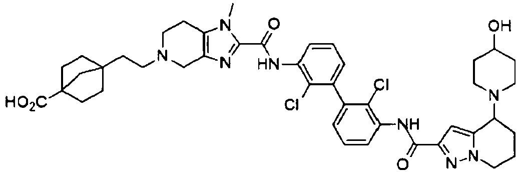 | 4-(2-(2-((2,2'-dichloro-3'-(4-(4-hydroxypiperidin-1-yl)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 59 | 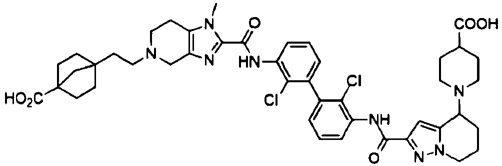 | 1-(2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)piperidine-4-carboxylic acid |
| 60 | 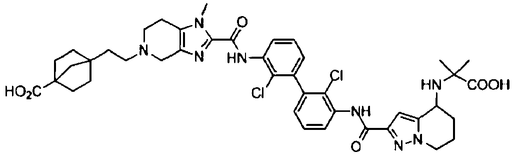 | 4-(2-(2-((3'-(4-((2-carboxypropan-2-yl)amino)-4,5,6,7-tetrahydropyrazolo[1,5-a]pyridine-2-carboxamido)-2,2'-dichloro-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid |
| 61 | 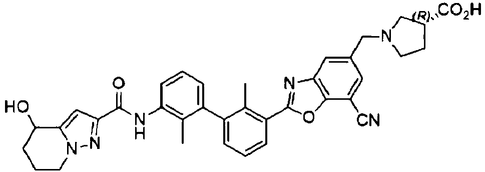 | (3R)-1-((7-cyano-2-(3'-(4-hydroxy-4,5,6,7-tetrahydropyrazolo[1,5-a]pyridine-2-carboxamido)-2,2'-dimethyl-[1,1'-biphenyl]-3-yl)benzo[d]oxazol-5-yl)methyl)pyrrolidine-3-carboxylic acid |
| 62 | 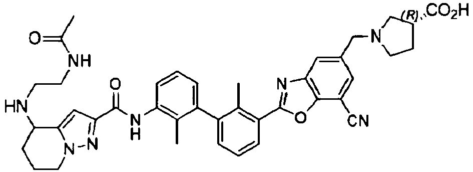 | (3R)-1-((2-(3'-(4-((2-acetamidoethyl)amino)-4,5,6,7-tetrahydropyrazolo[1,5-a]pyridine-2-carboxamido)-2,2'-dimethyl-[1,1'-biphenyl]-3-yl)-7-cyanobenzo[d]oxazol-5-yl)methyl)pyrrolidine-3-carboxylic acid |
| 63 | 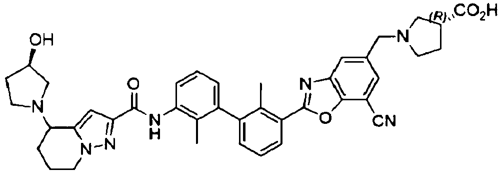 | (3R)-1-((7-cyano-2-(3'-(4-((R)-3-hydroxypyrrolidin-1-yl)-4,5,6,7-tetrahydropyrazolo[1,5-a]pyridine-2-carboxamido)-2,2'-dimethyl-[1,1'-biphenyl]-3-yl)benzo[d]oxazol-5-yl)methyl)pyrrolidine-3-carboxylic acid |
| 64 | 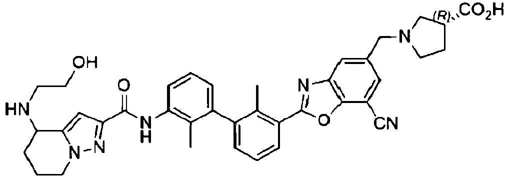 | (3R)-1-((7-cyano-2-(3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)benzo[d]oxazol-5-yl)methyl)pyrrolidine-3-carboxylic acid |
| 65 | 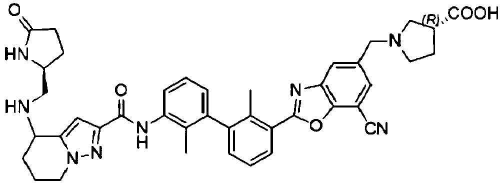 | (3R)-1-((7-cyano-2-(2,2'-dimethyl-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid |
| 66 | 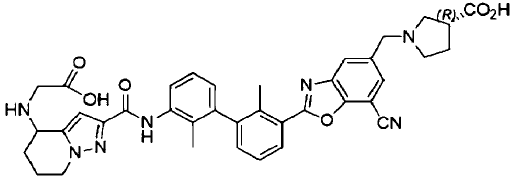 | (3R)-1-((2-(3'-(4-((carboxymethyl)amino)-4,5,6,7-tetrahydropyrazolo[1,5-a]pyridine-2-carboxamido)-2,2'-dimethyl-[1,1'-biphenyl]-3-yl)-7-cyanobenzo[d]oxazol-5-yl)methyl)pyrrolidine-3-carboxylic acid |
| 67 | 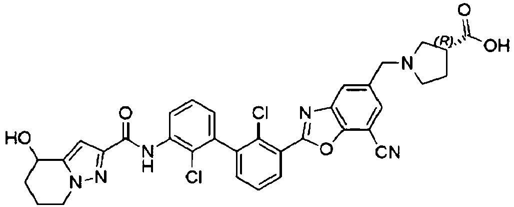 | (3R)-1-((7-cyano-2-(2,2'-dichloro-3'-(4-hydroxy-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid |
| 68 | 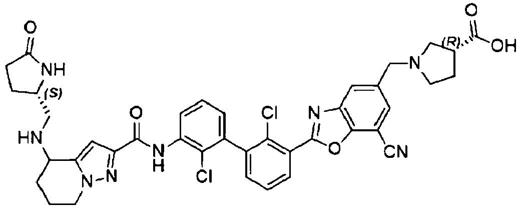 | (3R)-1-((7-cyano-2-(2,2'-dichloro-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid |
| 69 | 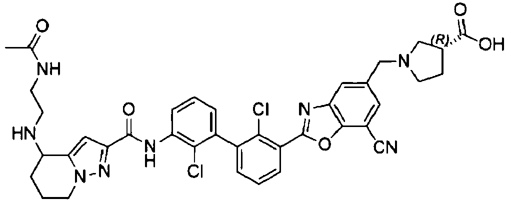 | (3R)-1-((2-(3'-(4-((2-acetamidoethyl)amino)-4,5,6,7-tetrahydropyrazolo[1,5-a]pyridine-2-carboxamido)-2,2'-dichloro-[1,1'-biphenyl]-3-yl)-7-cyanobenzo[d]oxazol-5-yl)methyl)pyrrolidine-3-carboxylic acid |
| 70 | 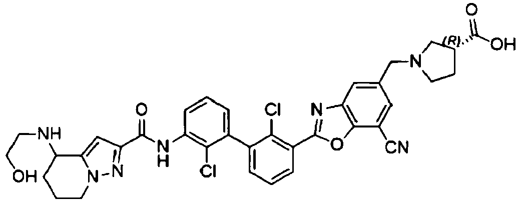 | (3R)-1-((7-cyano-2-(2,2'-dichloro-3'-(4-((2-hydroxyethyl)amino)-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid |
| 71 | 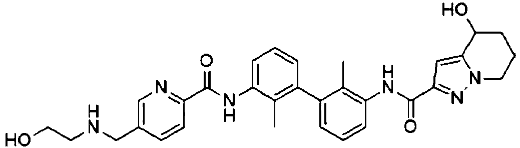 | 4-hydroxy-N-(3'-(5-(((2-hydroxyethyl)amino)methyl)pi colinamido)-2,2'-dimethyl-[1,1'-biphenyl]-3-yl)-4,5,6,7-tetrahydropyrazolo[1,5-a]pyridine-2-carboxamide |
| 72 | 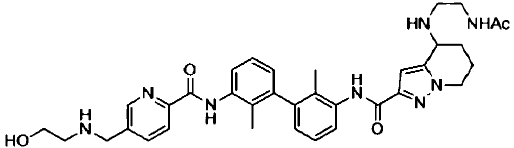 | 4-((2-acetamidoethyl)amino)-N-(3'-(5-(((2-hydroxyethyl)amino)methyl)pi colinamido)-2,2'-dimethyl-[1,1'-biphenyl]-3-yl)-4,5,6,7-tetrahydropyrazolo[1,5-a]pyridine-2-carboxamide |
| 73 | 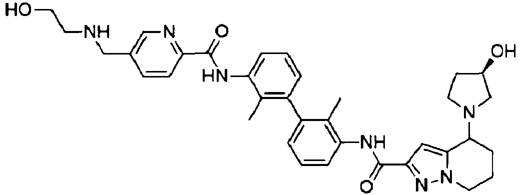 | N-(3'-(5-(((2-hydroxyethyl)amino)methyl)pi colinamido)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide |
| 74 | 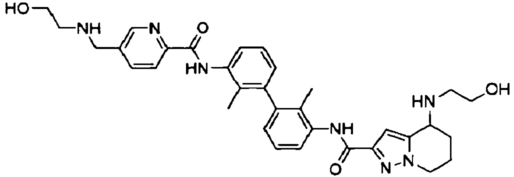 | N-(3'-(5-(((2-hydroxyethyl)amino)methyl)pi colinamido)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide |
| 75 | 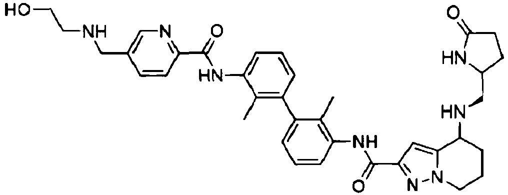 | N-(3'-(5-(((2-hydroxyethyl)amino)methyl)pi colinamido)-2,2'-dimethyl-[1,1'-biphenyl]-3-yl)-4-(((5-oxopyrrolidin-2-yl)methyl)amino)-4,5,6,7-tetrahydropyrazolo[1,5-a]pyridine-2-carboxamide |
| 76 | 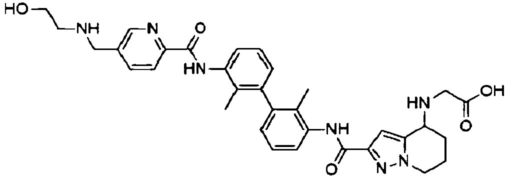 | 2-((2-((3'-(5-(((2-hydroxyethyl)amino)methyl)pi colinamido)-2,2'-dimethyl-[1,1'-biphenyl]-3-yl)carbamoyl)-4,5,6,7-tetrahydropyrazolo[1,5-a]pyridin-4-yl)amino)acetic acid |
| 77 | 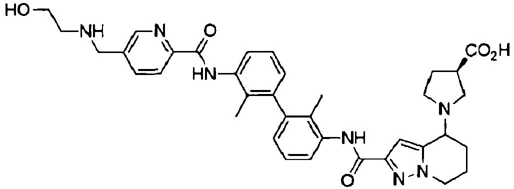 | (3R)-1-(2-((3'-(5-(((2-hydroxyethyl)amino)methyl)pi colinamido)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid |
| 78 | 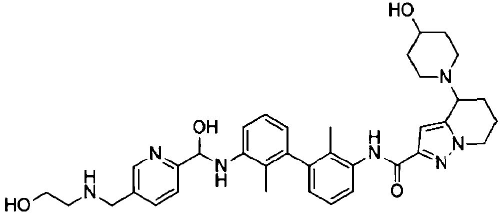 | N-(3'-(5-(((2-hydroxyethyl)amino)methyl)pi colinamido)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide |
| 79 | 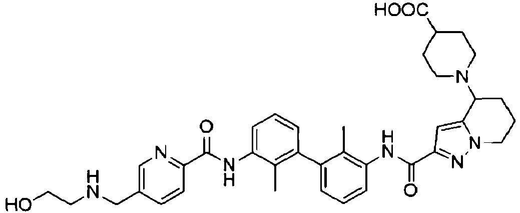 | 1-(2-((3'-(5-(((2-hydroxyethyl)amino)methyl)pi colinamido)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 80 | 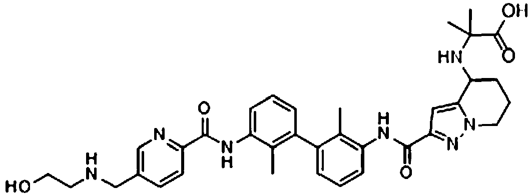 | 2-((2-((3'-(5-(((2-hydroxyethyl)amino)methyl)pi colinamido)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid |
| 81 | 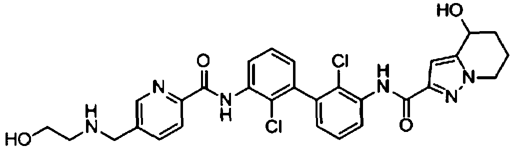 | N-(2,2'-dichloro-3'-(5-(((2-hydroxyethyl)amino)methyl)pi colinamido)-[1,1'-biphenyl]-3-yl)-4-hydroxy-4,5,6,7-tetrahydropyrazolo[1,5-a]pyridine-2-carboxamide |
| 82 | 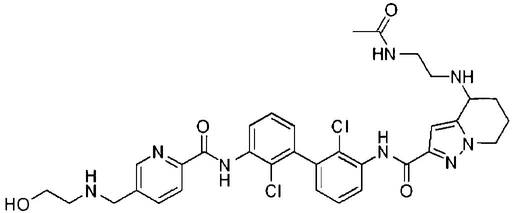 | 4-((2-acetamidoethyl)amino)-N-(2,2'-dichloro-3'-(5-(((2-hydroxyethyl)amino)methyl)pi colinamido)-[1,1'-biphenyl]-3-yl)-4,5,6,7-tetrahydropyrazolo[1,5-a]pyridine-2-carboxamide |
| 83 | 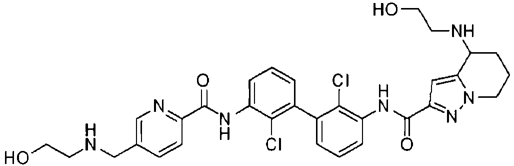 | N-(2,2'-dichloro-3'-(5-(((2-hydroxyethyl)amino)methyl)pi colinamido)-[1,1'-biphenyl]-3-yl)-4-((2-hydroxyethyl)amino)-4,5,6,7-tetrahydropyrazolo[1,5-a]pyridine-2-carboxamide |
| 84 | 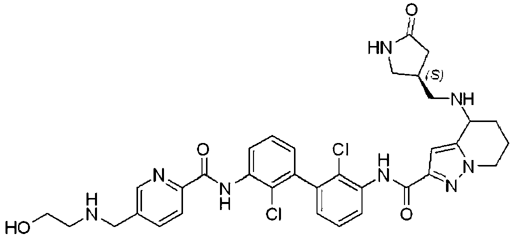 | N-(2,2'-dichloro-3'-(5-(((2-hydroxyethyl)amino)methyl)pi colinamido)-[1,1'-biphenyl]-3-yl)-4-((((S)-5-oxopyrrolidin-3-yl)methyl)amino)-4,5,6,7-tetrahydropyrazolo[1,5-a]pyridine-2-carboxamide |
| 85 | 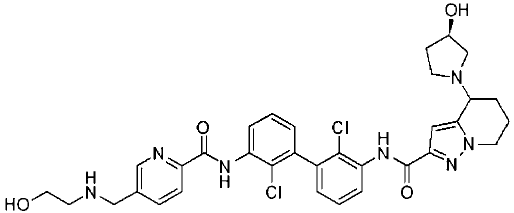 | N-(2,2'-dichloro-3'-(5-(((2-hydroxyethyl)amino)methyl)pi colinamido)-[1,1'-biphenyl]-3-yl)-4-((R)-3-hydroxypyrrolidin-1-yl)-4,5,6,7-tetrahydropyrazolo[1,5-a]pyridine-2-carboxamide |
| 86 | 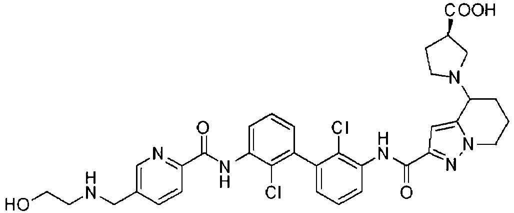 | (3R)-1-(2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)pyrrolidine-3-carboxylic acid |
| 87 | 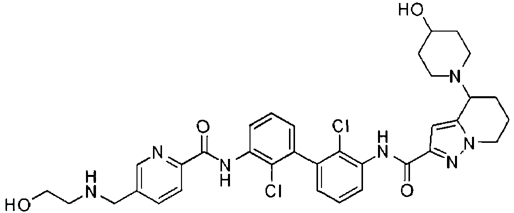 | N-(2,2'-dichloro-3'-(5-(((2-hydroxyethyl)amino)methyl)pi colinamido)-[1,1'-biphenyl]-3-yl)-4-(4-hydroxypiperidin-1-yl)-4,5,6,7-tetrahydropyrazolo[1,5-a]pyridine-2-carboxamide |
| 88 | 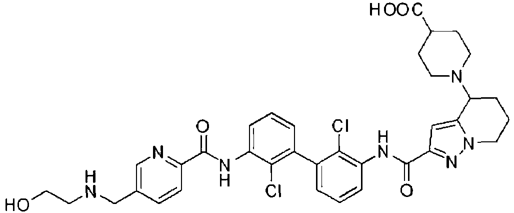 | 1-(2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)piperidine-4-carboxylic acid |
| 89 | 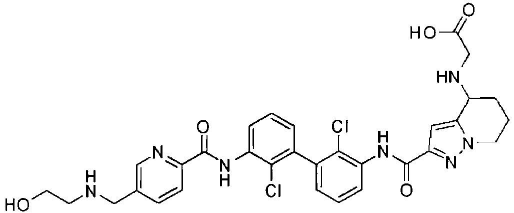 | 2-((2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)amino)acetic acid |
| 90 | 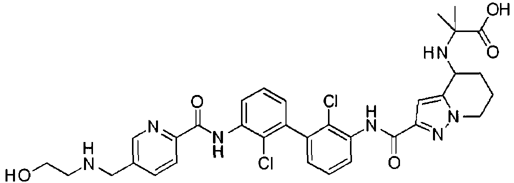 | 2-((2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)amino)-2-methylpropanoic acid |
| 91 | 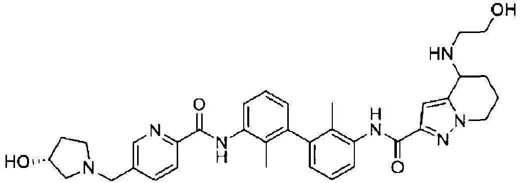 | 4-((2-hydroxyethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 92 | 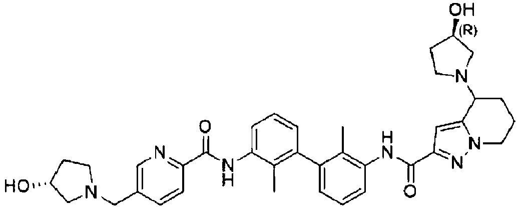 | 4-((R)-3-hydroxypyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 93 | 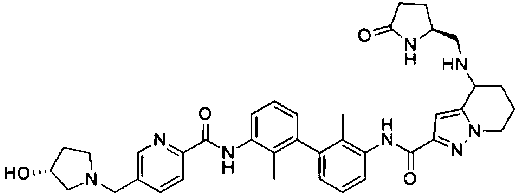 | N-(3'-(5-(((R)-3-hydroxypyrrolidin-1-yl)methyl)picolinamido)-2,2'-dimethyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 94 | 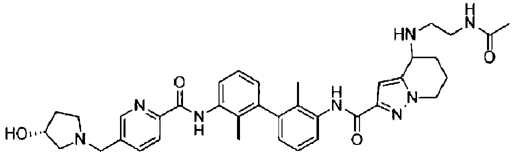 | 4-((2-acetamidoethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 95 | 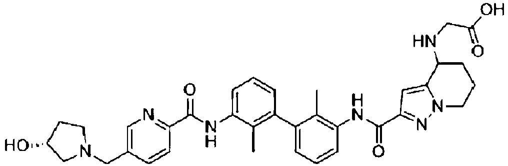 | 2-((2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)amino)acetic acid |
| 96 | 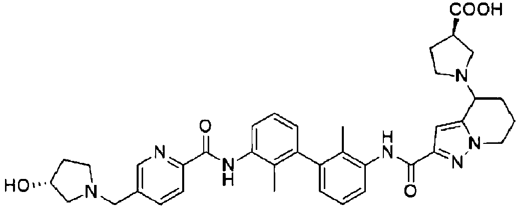 | (3R)-1-(2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid |
| 97 | 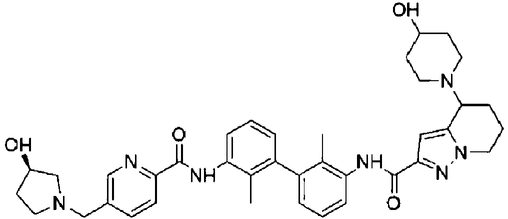 | N-(2,2'-dichloro-3'-((3-(((S)-3-hydroxypyrrolidin-1-yl)methyl)-1,7-naphthyridin-8-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 98 | 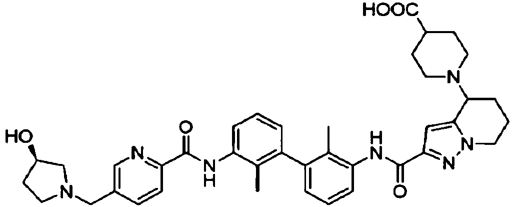 | 1-(2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 99 | 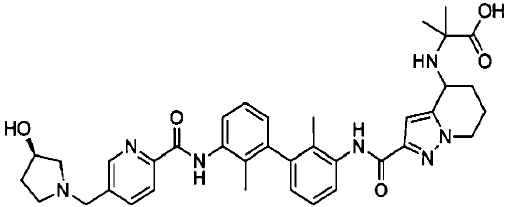 | 2-((2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid |
| 100 | 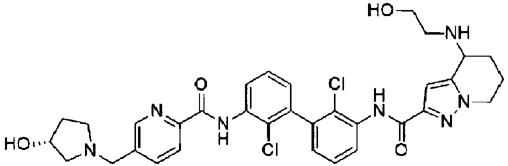 | N-(2,2'-dichloro-3'-(5-(((R)-3-hydroxypyrrolidin-1-yl)methyl)picolinamido)-[1,1'-biphenyl]-3-yl)-4-((2-hydroxyethyl)amino)-4,5,6,7-tetrahydropyrazolo[1,5-a]pyridine-2-carboxamide |
| 101 | 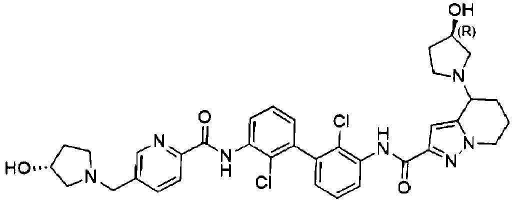 | N-(2,2'-dichloro-3'-(5-(((R)-3-hydroxypyrrolidin-1-yl)methyl)picolinamido)-[1,1'-biphenyl]-3-yl)-4-((R)-3-hydroxypyrrolidin-1-yl)-4,5,6,7-tetrahydropyrazolo[1,5-a]pyridine-2-carboxamide |
| 102 | 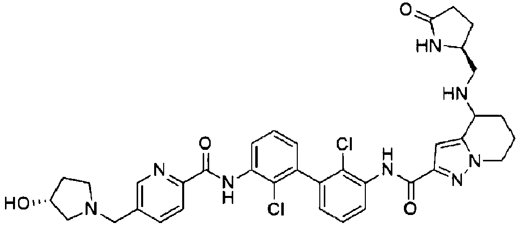 | N-(2,2'-dichloro-3'-(5-(((R)-3-hydroxypyrrolidin-1-yl)methyl)picolinamido)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 103 | 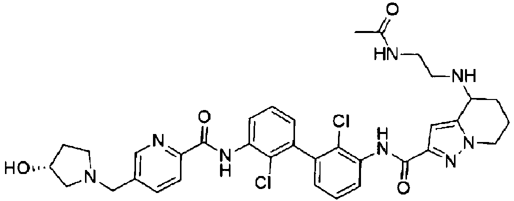 | 4-((2-acetamidoethyl)amino)-N-(2,2'-dichloro-3'-(5-(((R)-3-hydroxypyrrolidin-1-yl)methyl)picolinamido)-[1,1'-biphenyl]-3-yl)-4,5,6,7-tetrahydropyrazolo[1,5-a]pyridine-2-carboxamide |
| 104 | 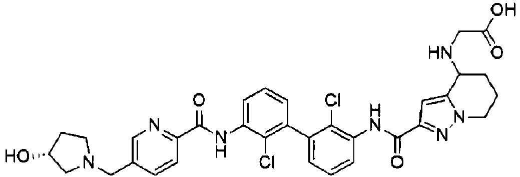 | 2-((2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid |
| 105 | 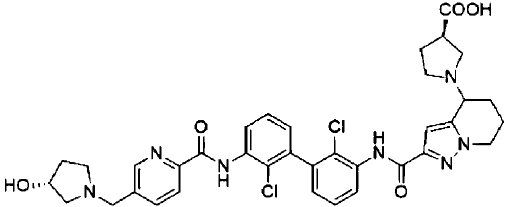 | (3R)-1-(2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid |
| 106 | 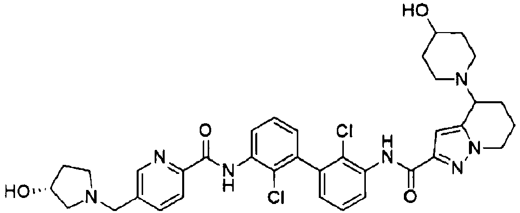 | N-(2,2'-dichloro-3'-(5-(((R)-3-hydroxypyrrolidin-1-yl)methyl)picolinamido)-[1,1'-biphenyl]-3-yl)-4-(4-hydroxypiperidin-1-yl)-4,5,6,7-tetrahydropyrazolo[1,5-a]pyridine-2-carboxamide |
| 107 | 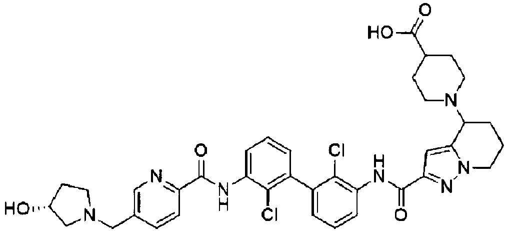 | 1-(2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)piperidine-4-carboxylic acid |
| 108 | 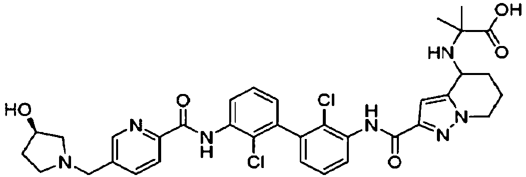 | 2-((2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)-2-methylpropanoic acid |
| 109 | 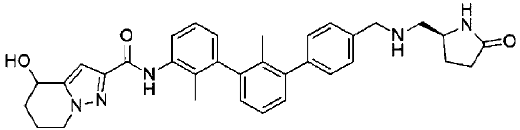 | N-(2,2'-dimethyl-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide |
| 110 | 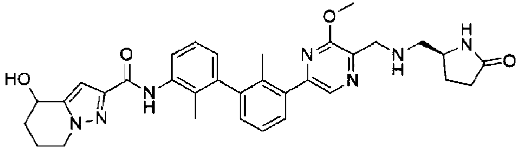 | 4-hydroxy-N-(3'-(6-methoxy-5-(((((S)-5-oxopyrrolidin-2-yl)methyl)amino)methyl)pyrazi n-2-yl)-2,2'-dimethyl-[1,1'-biphenyl]-3-yl)-4,5,6,7-tetrahydropyrazolo[1,5-a]pyridine-2-carboxamide |
| 111 | 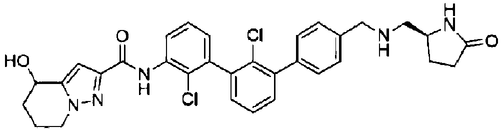 | N-(2,2'-dichloro-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide |
| 112 | 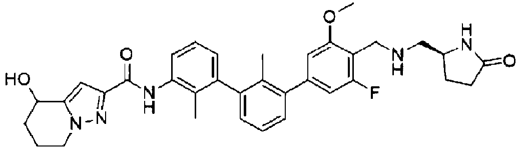 | N-(3"-fluoro-5"-methoxy-2,2'-dimethyl-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide |
| 112 -1 | 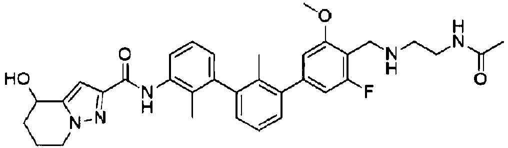 | N-(4"-(((2-acetamidoethyl)amino)methyl) -3"-fluoro-5"-methoxy-2,2'-dimethyl-[1,1':3',1"-terphenyl]-3-yl)-4-hydroxy-4,5,6,7-tetrahydropyrazolo[1,5-a]pyridine-2-carboxamide |
| 113 | 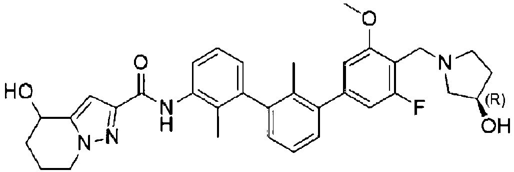 | N-(3"-fluoro-4"-(((R)-3-hydroxypyrrolidin-1-yl)methyl)-5"-methoxy-2,2'-dimethyl-[1,1':3',1"-terphenyl]-3-yl)-4-hydroxy-4,5,6,7-tetrahydropyrazolo[1,5-a]pyridine-2-carboxamide |
| 114 | 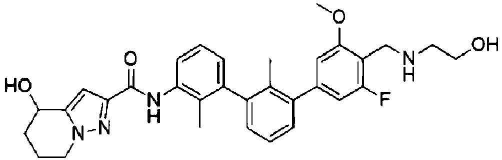 | N-(3"-fluoro-4"-(((2-hydroxyethyl)amino)methyl)-5"-methoxy-2,2'-dimethyl-[1,1':3',1"-terphenyl]-3-yl)-4-hydroxy-4,5,6,7-tetrahydropyrazolo[1,5-a]pyridine-2-carboxamide |
| 115 | 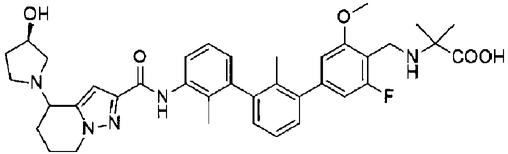 | 2-(((3-fluoro-3"-(4-((R)-3-hydroxypyrrolidin-1-yl)-4,5,6,7-tetrahydropyrazolo[1,5-a]pyridine-2-carboxamido)-5-methoxy-2',2"-dimethyl-[1,1':3',1"-terphenyl]-4-yl)methyl)amino)-2-methylpropanoic acid |
| 116 | 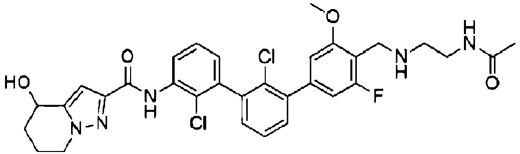 | N-(4"-(((2-acetamidoethyl)amino)methyl) -2,2'-dichloro-3"-fluoro-5"-methoxy-[1,1':3',1"-terphenyl]-3-yl)-4-hydroxy-4,5,6,7-tetrahydropyrazolo[1,5-a]pyridine-2-carboxamide |
| 117 | 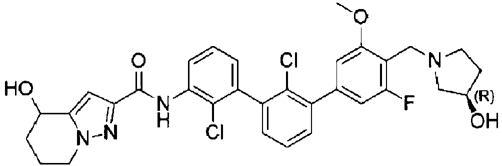 | N-(2,2'-dichloro-3"-fluoro-4"-(((R)-3-hydroxypyrrolidin-1-yl)methyl)-5"-methoxy-[1,1':3',1"-terphenyl]-3-yl)-4-hydroxy-4,5,6,7-tetrahydropyrazolo[1,5-a]pyridine-2-carboxamide |
| 118 | 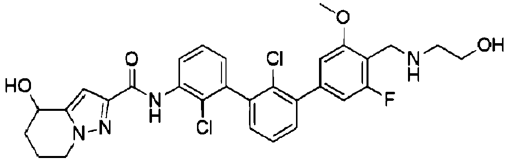 | N-(2,2'-dichloro-3"-fluoro-4"-(((2-hydroxyethyl)amino)methyl)-5"-methoxy-[1,1':3',1"-terphenyl]-3-yl)-4-hydroxy-4,5,6,7-tetrahydropyrazolo[1,5-a]pyridine-2-carboxamide |
| 119 | 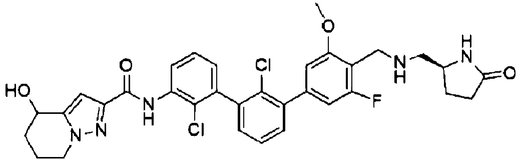 | N-(2,2'-dichloro-3"-fluoro-5"-methoxy-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide |
| 120 | 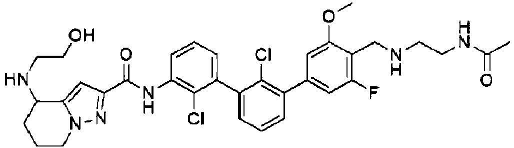 | N-(4"-(((2-acetamidoethyl)amino)methyl) -2,2'-dichloro-3"-fluoro-5"-methoxy-[1,1':3',1"-terphenyl]-3-yl)-4-((2-hydroxyethyl)amino)-4,5,6,7-tetrahydropyrazolo[1,5-a]pyridine-2-carboxamide |
| 121 | 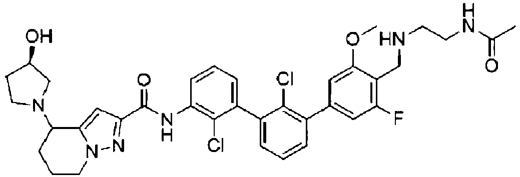 | N-(4"-(((2-acetamidoethyl)amino)methyl) -2,2'-dichloro-3"-fluoro-5"-methoxy-[1,1':3',1"-terphenyl]-3-yl)-4-((R)-3-hydroxypyrrolidin-1-yl)-4,5,6,7-tetrahydropyrazolo[1,5-a]pyridine-2-carboxamide |
| 122 | 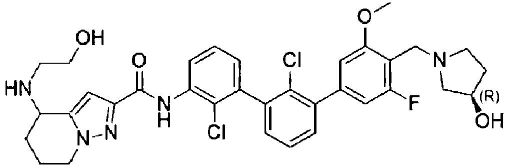 | N-(2,2'-dichloro-3"-fluoro-4"-(((R)-3-hydroxypyrrolidin-1-yl)methyl)-5"-methoxy-[1,1':3',1"-terphenyl]-3-yl)-4-((2-hydroxyethyl)amino)-4,5,6,7-tetrahydropyrazolo[1,5-a]pyridine-2-carboxamide |
| 123 | 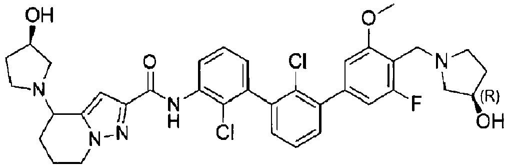 | N-(2,2'-dichloro-3"-fluoro-4"-(((R)-3-hydroxypyrrolidin-1-yl)methyl)-5"-methoxy-[1,1':3',1"-terphenyl]-3-yl)-4-((R)-3-hydroxypyrrolidin-1-yl)-4,5,6,7-tetrahydropyrazolo[1,5-a]pyridine-2-carboxamide |
| 124 | 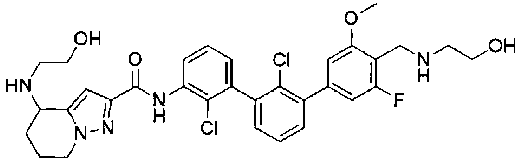 | N-(2,2'-dichloro-3"-fluoro-4"-(((2-hydroxyethyl)amino)methyl)-5"-methoxy-[1,1':3',1"-terphenyl]-3-yl)-4-((2-hydroxyethyl)amino)-4,5,6,7-tetrahydropyrazolo[1,5-a]pyridine-2-carboxamide |
| 125 | 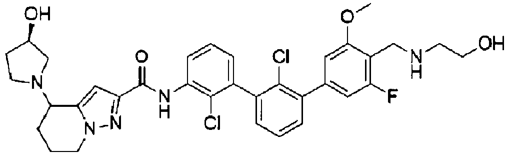 | N-(2,2'-dichloro-3"-fluoro-4"-(((2-hydroxyethyl)amino)methyl)-5"-methoxy-[1,1':3',1"-terphenyl]-3-yl)-4-((R)-3-hydroxypyrrolidin-1-yl)-4,5,6,7-tetrahydropyrazolo[1,5-a]pyridine-2-carboxamide |
| 126 | 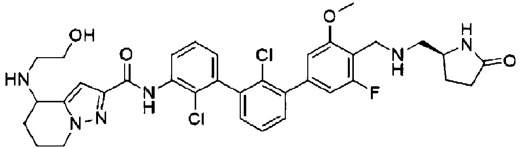 | N-(2,2'-dichloro-3"-fluoro-5"-methoxy-4"-(((((S)-5-oxopyrrolidin-2-yl)methyl)amino)methyl)-[1,1':3',1"-terphenyl]-3-yl)-4-((2-hydroxyethyl)amino)-4,5,6,7-tetrahydropyrazolo[1,5-a]pyridine-2-carboxamide |
| 127 | 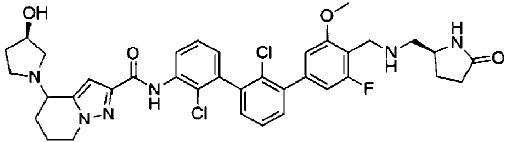 | N-(2,2'-dichloro-3"-fluoro-5"-methoxy-4"-(((((S)-5-oxopyrrolidin-2-yl)methyl)amino)methyl)-[1,1':3',1"-terphenyl]-3-yl)-4-((R)-3-hydroxypyrrolidin-1-yl)-4,5,6,7-tetrahydropyrazolo[1,5-a]pyridine-2-carboxamide |
| 128 | 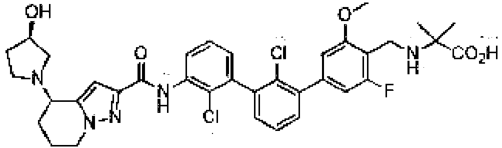 | 2-(((2',2"-dichloro-3-fluoro-3"-(4-((R)-3-hydroxypyrrolidin-1-yl)-4,5,6,7-tetrahydropyrazolo[1,5-a]pyridine-2-carboxamido)-5-methoxy-[1,1':3',1"-terphenyl]-4-yl)methyl)amino)-2-methylpropanoic acid |
| 129 | 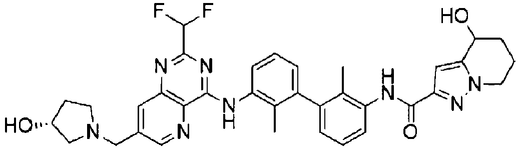 | N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-hydroxy-4,5,6,7-tetrahydropyrazolo[1,5-a]pyridine-2-carboxamide |
| 130 | 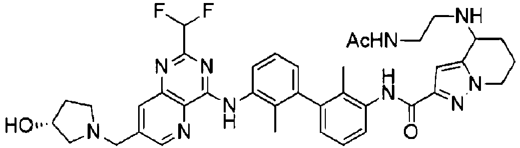 | 4-((2-acetamidoethyl)amino)-N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4,5,6,7-tetrahydropyrazolo[1,5-a]pyridine-2-carboxamide |
| 131 | 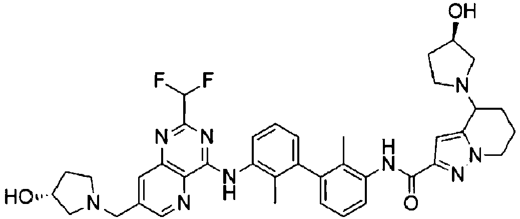 | N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide |
| 132 | 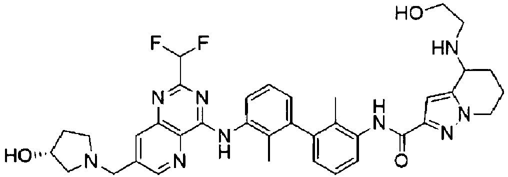 | N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-((2-hydroxyethyl)amino)-4,5,6,7-tetrahydropyrazolo[1,5-a]pyridine-2-carboxamide |
| 133 | 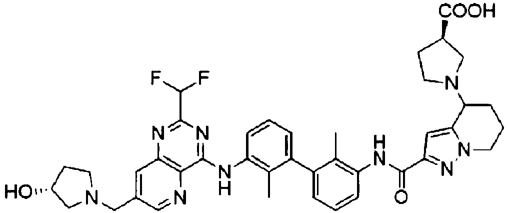 | (3R)-1-(2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid |
| 134 | 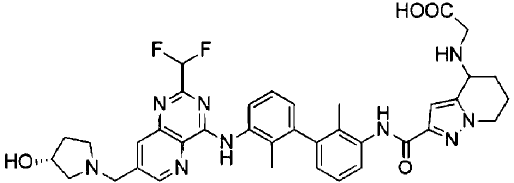 | 2-((2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetic acid |
| 135 | 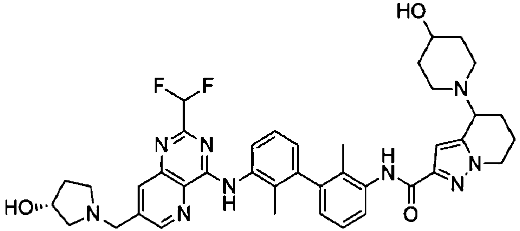 | N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide |
| 136 | 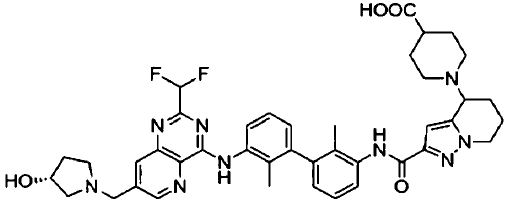 | 1-(2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 137 | 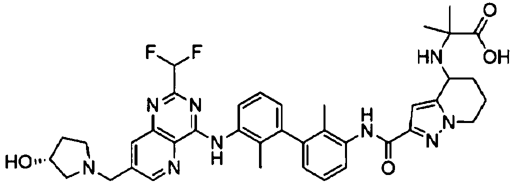 | 2-((2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid |
| 138 | 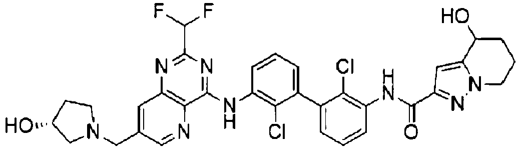 | N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-hydroxy-4,5,6,7-tetrahydropyrazolo[1,5-a]pyridine-2-carboxamide |
| 139 | 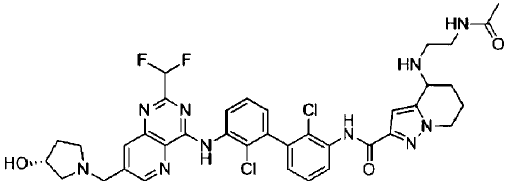 | 4-((2-acetamidoethyl)amino)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4,5,6,7-tetrahydropyrazolo[1,5-a]pyridine-2-carboxamide |
| 140 | 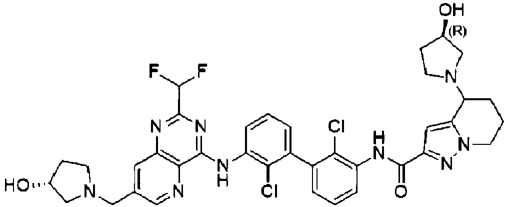 | N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 141 | 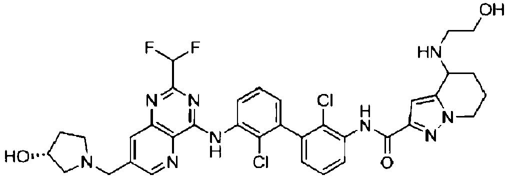 | N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-((2-hydroxyethyl)amino)-4,5,6,7-tetrahydropyrazolo[1,5-a]pyridine-2-carboxamide |
| 142 | 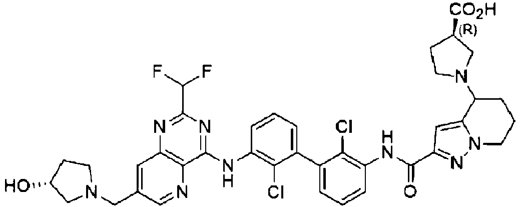 | (3R)-1-(2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)pyrrolidine-3-carboxylic acid |
| 143 | 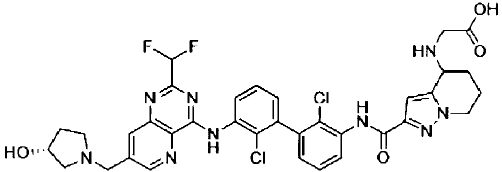 | 2-((2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetic acid |
| 144 | 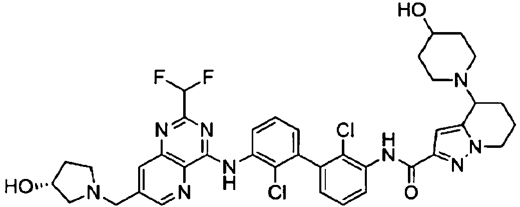 | N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 145 | 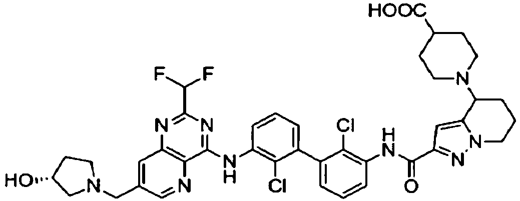 | 1-(2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 146 | 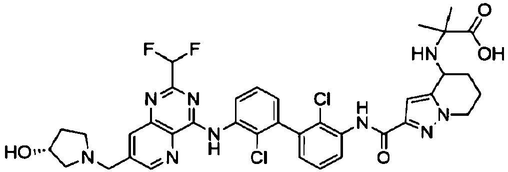 | 2-((2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 147 | 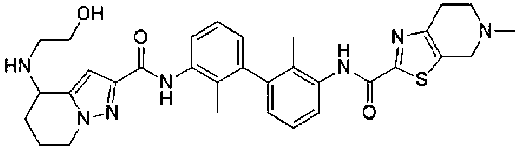 | N-(3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)-5-methyl-4,5,6,7-tetrahydrothiazolo[5,4-c]pyridine-2-carboxamide |
| 148 | 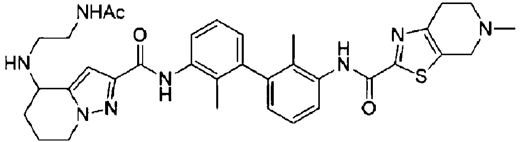 | N-(3'-(4-((2-acetamidoethyl)amino)-4,5,6,7-tetrahydropyrazolo[1,5-a]pyridine-2-carboxamido)-2,2'-dimethyl-[1,1'-biphenyl]-3-yl)-5-methyl-4,5,6,7-tetrahydrothiazolo[5,4-c]pyridine-2-carboxamide |
| 149 | 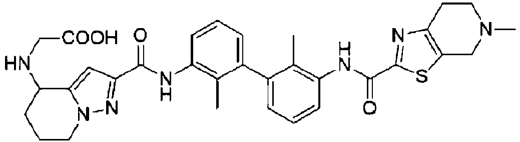 | 2-((2-((2,2'-dimethyl-3'-(5-methyl-4,5,6,7-tetrahydrothiazolo[5,4-c]pyridine-2-carboxamido)-[1,1'-biphenyl]-3-yl)carbamoyl)-4,5,6,7-tetrahydropyrazolo[1,5-a]pyridin-4-yl)amino)acetic acid |
| 150 | 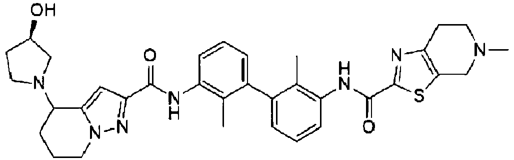 | N-(3'-(4-((R)-3-hydroxypyrrolidin-1-yl)-4,5,6,7-tetrahydropyrazolo[1,5-a]pyridine-2-carboxamido)-2,2'-dimethyl-[1,1'-biphenyl]-3-yl)-5-methyl-4,5,6,7-tetrahydrothiazolo[5,4-c]pyridine-2-carboxamide |
| 150 -1 | 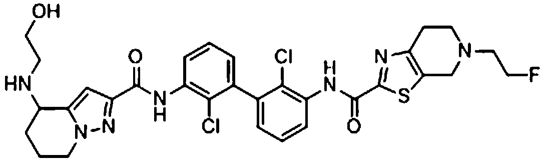 | N-(2,2'-dichloro-3'-(4-((2-hydroxyethyl)amino)-4,5,6,7-tetrahydropyrazolo[1,5-a]pyridine-2-carboxamido)-[1,1'-biphenyl]-3-yl)-5-(2-fluoroethyl)-4,5,6,7-tetrahydrothiazolo[5,4-c]pyridine-2-carboxamide |
| 150 -2 | 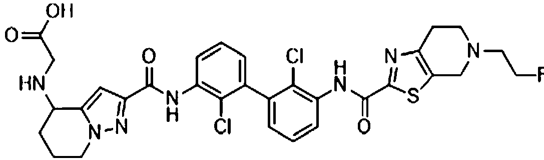 | 2-((2-((2,2'-dichloro-3'-(5-(2-fluoroethyl)-4,5,6,7-tetrahydrothiazolo[5,4-c]pyridine-2-carboxamido)-[1,1'-biphenyl]-3-yl)carbamoyl)-4,5,6,7-tetrahydropyrazolo[1,5-a]pyridin-4-yl)amino)acetic acid |
| 151 | 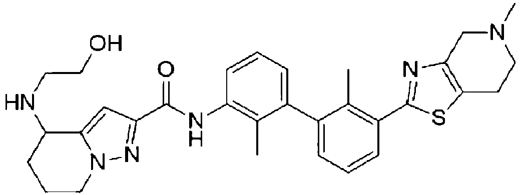 | N-(2,2'-dimethyl-3'-(5-methyl-4,5,6,7-tetrahydrothiazolo[4,5-c]pyridin-2-yl)-[1,1'-biphenyl]-3-yl)-4-((2-hydroxyethyl)amino)-4,5,6,7-tetrahydropyrazolo[1,5-a]pyridine-2-carboxamide |
| 152 | 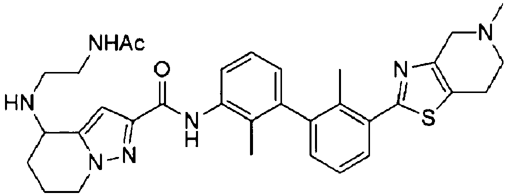 | 4-((2-acetamidoethyl)amino)-N-(2,2'-dimethyl-3'-(5-methyl-4,5,6,7-tetrahydrothiazolo[4,5-c]pyridin-2-yl)-[1,1'-biphenyl]-3-yl)-4,5,6,7-tetrahydropyrazolo[1,5-a]pyridine-2-carboxamide |
| 153 | 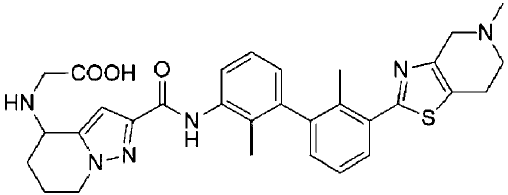 | 2-((2-((2,2'-dimethyl-3'-(5-methyl-4,5,6,7-tetrahydrothiazolo[4,5-c]pyridin-2-yl)-[1,1'-biphenyl]-3-yl)carbamoyl)-4,5,6,7-tetrahydropyrazolo[1,5-a]pyridin-4-yl)amino)acetic acid |
| 154 | 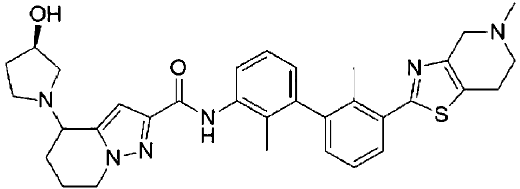 | N-(2,2'-dimethyl-3'-(5-methyl-4,5,6,7-tetrahydrothiazolo[4,5-c]pyridin-2-yl)-[1,1'-biphenyl]-3-yl)-4-((R)-3-hydroxypyrrolidin-1-yl)-4,5,6,7-tetrahydropyrazolo[1,5-a]pyridine-2-carboxamide |
| 155 | 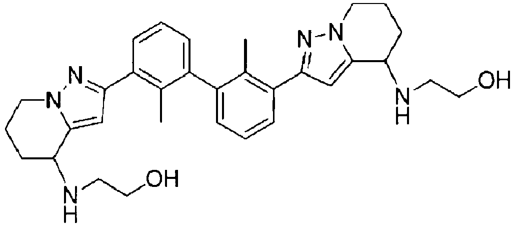 | 2,2'-((2,2'-(2,2'-dimethyl-[1,1'-biphenyl]-3,3'-diyl)bis(4,5,6,7-tetrahydropyrazolo[1,5-a]pyridine-4,2-diyl))bis(azanediyl))diethanol |
| 156 | 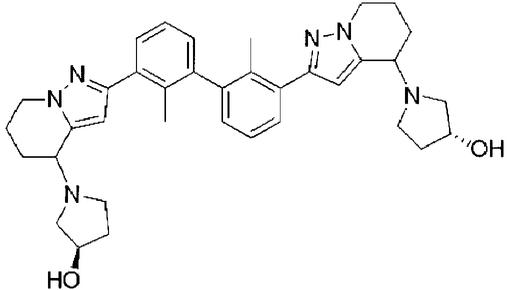 | (3R,3'R)-1,1'-(2,2'-(2,2'-dimethyl-[1,1'-biphenyl]-3,3'-diyl)bis(4,5,6,7-tetrahydropyrazolo[1,5-a]pyridine-4,2-diyl))bis(pyrrolidin-3-ol) |
| 157 | 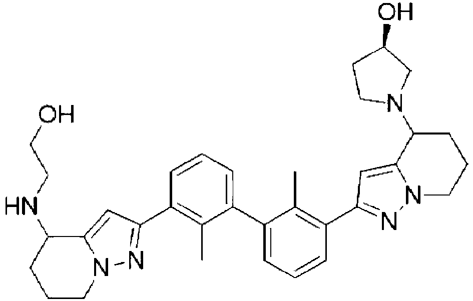 | (3R)-1-(2-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a)pyridin-4-yl)pyrrolidin-3-ol |
| 158 | 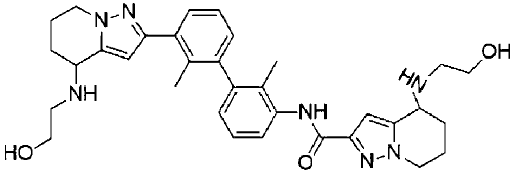 | 4-((2-hydroxyethyl)amino)-N-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridine-2-carboxamide |
| 159 | 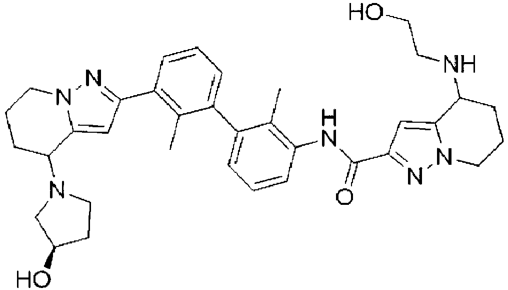 | 4-((2-hydroxyethyl)amino)-N-(3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridine-2-carboxamide |
| 160 | 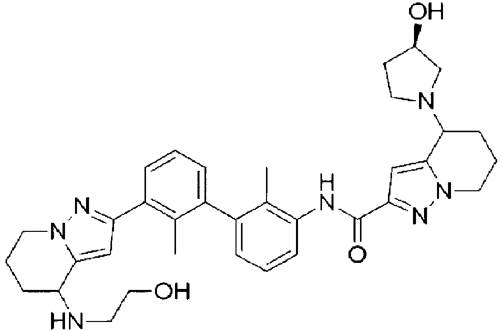 | N-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide |
| 161 | 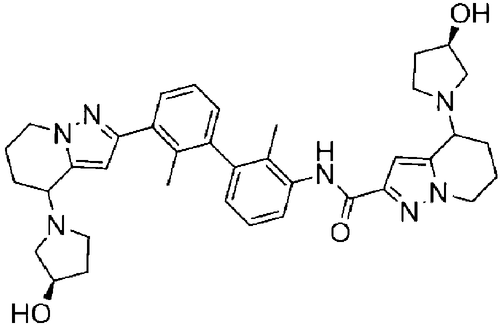 | 4-((R)-3-hydroxypyrrolidin-1-yl)-N-(3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridine-2-carboxamide |
| 162 | 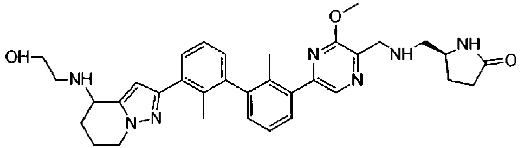 | (5S)-5-((((5-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-3-methoxypyrazin-2-yl)methyl)amino)methyl)pyrroli din-2-one |
| 163 | 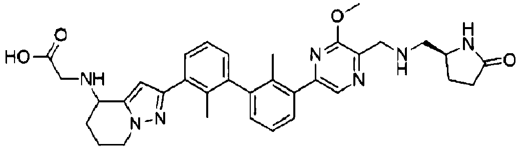 | 2-((2-(3'-(6-methoxy-5-(((((S)-5-oxopyrrolidin-2-yl)methyl)amino)methyl)pyrazi n-2-yl)-2,2'-dimethyl-[1,1'-biphenyl]-3-yl)-4,5,6,7-tetrahydropyrazolo[1,5-a]pyridin-4-yl)amino)acetic acid |
| 164 | 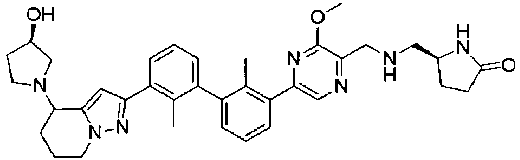 | (5S)-5-((((5-(3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)-3-methoxypyrazin-2-yl)methyl)amino)methyl)pyrroli din-2-one |
| 165 | 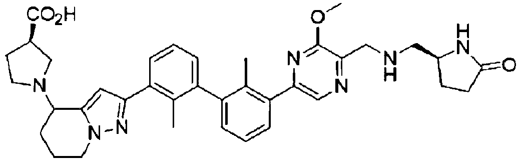 | (3R)-1-(2-(3'-(6-methoxy-5-(((((S)-5-oxopyrrolidin-2-yl)methyl)amino)methyl)pyrazi n-2-yl)-2,2'-dimethyl-[1,1'-biphenyl]-3-yl)-4,5,6,7-tetrahydropyrazolo[1,5-a]pyridin-4-yl)pyrrolidine-3-carboxylic acid |
| 166 | 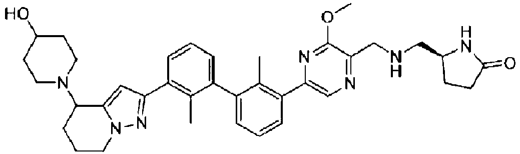 | (5S)-5-((((5-(3'-(4-(4-hydroxypiperidin-1-yl)-4,5,6,7-tetrahydropyrazolo[1,5-a]pyridin-2-yl)-2,2'-dimethyl-[1,1-biphenyl]-3-yl)-3-methoxypyrazin-2-yl)methyl)amino)methyl)pyrroli din-2-one |
| 167 | 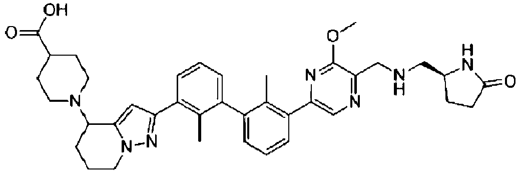 | 1-(2-(3'-(6-methoxy-5-(((((S)-5-oxopyrrolidin-2-yl)methyl)amino)methyl)pyrazi n-2-yl)-2,2'-dimethyl-[1,1'-biphenyl]-3-yl)-4,5,6,7-tetrahydropyrazolo[1,5-a]pyridin-4-yl)piperidine-4-carboxylic acid |
| 168 | 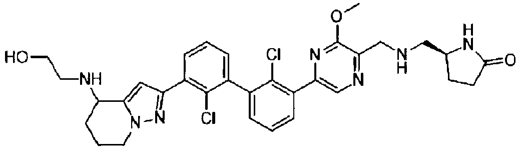 | (5S)-5-((((5-(2,2'-dichloro-3'-(4-((2-hydroxyethyl)amino)-4,5,6,7-tetrahydropyrazolo[1,5-a]pyridin-2-yl)-[1,1'-biphenyl]-3-yl)-3-methoxypyrazin-2-yl)methyl)amino)methyl)pyrroli din-2-one |
| 169 | 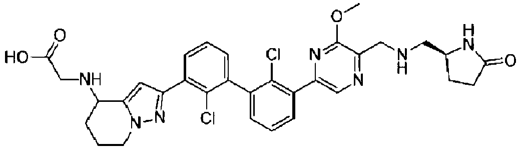 | 2-((2-(2,2'-dichloro-3'-(6-methoxy-5-(((((S)-5-oxopyrrolidin-2-yl)methyl)amino)methyl)pyrazi n-2-yl)-[1,1'-biphenyl]-3-yl)-4,5,6,7-tetrahydropyrazolo[1,5-a]pyridin-4-yl)amino)acetic acid |
| 170 | 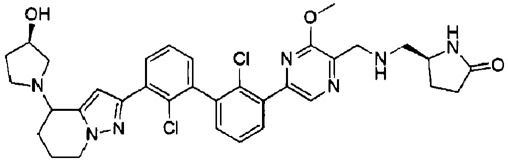 | (5S)-5-((((5-(2,2'-dichloro-3'-(4-((R)-3-hydroxypyrrolidin-1-yl)-4,5,6,7-tetrahydropyrazolo[1,5-a]pyridin-2-yl)-[1,1'-biphenyl]-3-yl)-3-methoxypyrazin-2-yl)methyl)amino)methyl)pyrroli din-2-one |
| 171 | 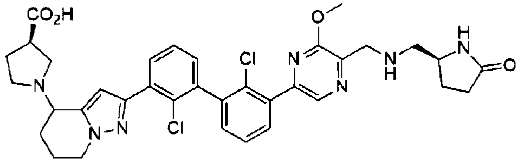 | (3R)-1-(2-(2,2'-dichloro-3'-(6-methoxy-5-(((((S)-5-oxopyrrolidin-2-yl)methyl)amino)methyl)pyrazi n-2-yl)-[1,1'-biphenyl]-3-yl)-4,5,6,7-tetrahydropyrazolo[1,5-a]pyridin-4-yl)pyrrolidine-3-carboxylic acid |
| 174 | 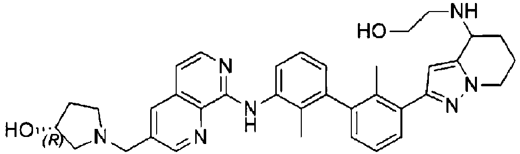 | (3R)-1-((8-((3'-(4-((2-hydroxyethyl)amino)-4,5,6,7-tetrahydropyrazolo[1,5-a]pyridin-2-yl)-2,2'-dimethyl-[1,1'-biphenyl]-3-yl)amino)-1,7-naphthyridin-3-yl)methyl)pyrrolidin-3-ol |
| 175 | 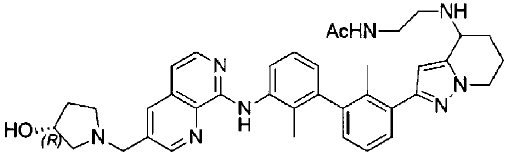 | N-(2-((2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)amino)ethyl)acetamide |
| 176 | 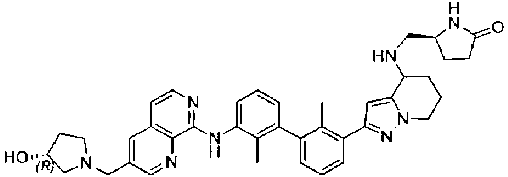 | (5S)-5-(((2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)amino)methyl)pyrrolidin-2-one |
| 177 | 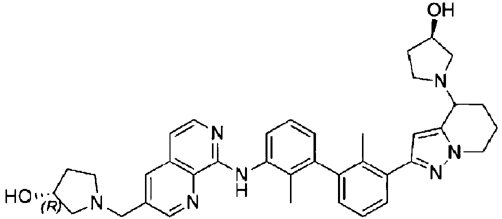 | (3R)-1-((8-((3'-(4-((R)-3-hydroxypyrrolidin-1-yl)-4,5,6,7-tetrahydropyrazolo[1,5-a]pyridin-2-yl)-2,2'-dimethyl-[1,1-biphenyl]-3-yl)amino)-1,7-naphthyridin-3-yl)methyl)pyrrolidin-3-ol |
| 178 | 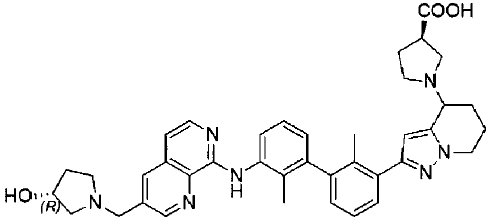 | (3R)-1-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)pyrrolidine-3-carboxylic acid |
| 179 | 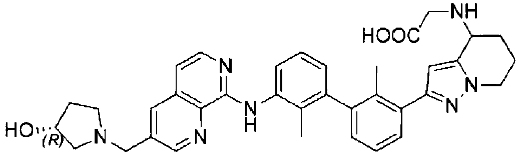 | 2-((2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)amino)acetic acid |
| 180 | 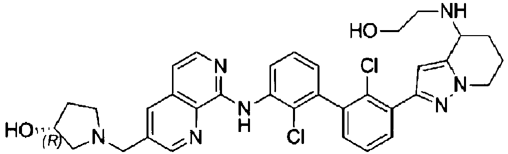 | (3R)-1-((8-((2,2'-dichloro-3'-(4-((2-hydroxyethyl)amino)-4,5,6,7-tetrahydropyrazolo[1,5-a]pyridin-2-yl)-[1,1'-biphenyl]-3-yl)amino)-1,7-naphthyridin-3-yl)methyl)pyrrolidin-3-ol |
| 181 | 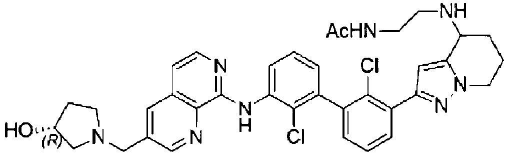 | N-(2-((2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)amino)ethyl)acetamide |
| 182 | 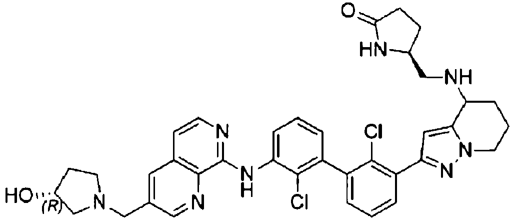 | (5S)-5-(((2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)amino)methyl)pyrrolidin-2-one |
| 183 | 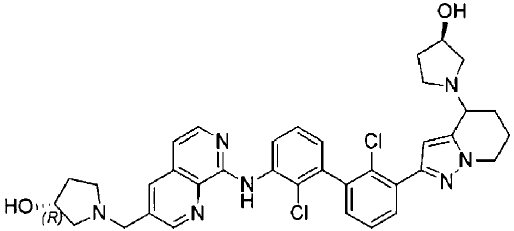 | (3R)-1-(2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)pyrrolidin-3-ol |
| 184 | 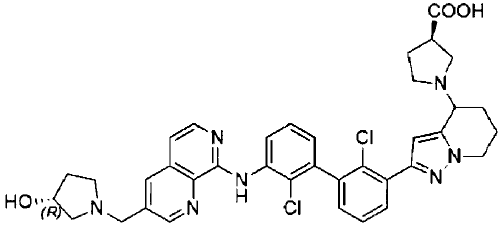 | (3R)-1-(2-(2,2'-dichloro-3'-((3-(((R)-3-hydroxypyrrolidin-1 - yl)methyl)-1,7-naphthyridin-8-yl)amino)-[1,1'-biphenyl]-3-yl)-4,5,6,7-tetrahydropyrazolo[1,5-a]pyridin-4-yl)pyrrolidine-3-carboxylic acid |
| 185 | 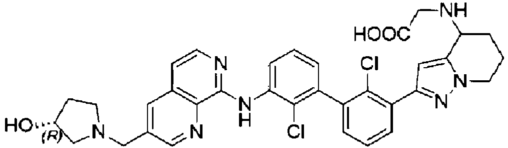 | 2-((2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)amino)acetic acid |
| 186 | 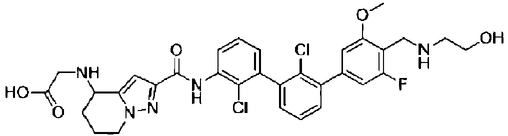 | 2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetic acid |
| 187 | 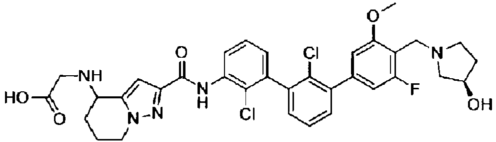 | 2-((2-((2,2'-dichloro-3"-fluoro-4"-(((R)-3-hydroxypyrrolidin-1-yl)methyl)-5"-methoxy-[1,1':3',1"-terphenyl]-3-yl)carbamoyl)-4,5,6,7-tetrahydropyrazolo[1,5-a]pyridin-4-yl)amino)acetic acid |
| 188 | 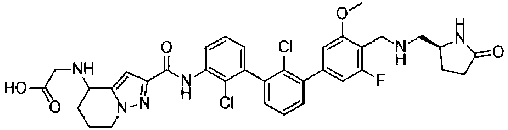 | 2-((2-((2,2'-dichloro-3"-fluoro-5"-methoxy-4"-(((((S)-5-oxopyrrolidin-2-yl)methyl)amino)methyl)-[1,1':3',1"-terphenyl]-3-yl)carbamoyl)-4,5,6,7-tetrahydropyrazolo[1,5-a]pyridin-4-yl)amino)acetic acid |
| 189 | 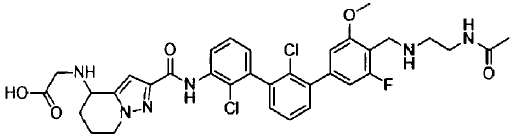 | 2-((2-((4"-(((2-acetamidoethyl)amino)methyl) -2,2'-dichloro-3"-fluoro-5"-methoxy-[1,1':3',1"-terphenyl]-3-yl)carbamoyl)-4,5,6,7-tetrahydropyrazolo[1,5-a]pyridin-4-yl)amino)acetic acid |
| 190 | 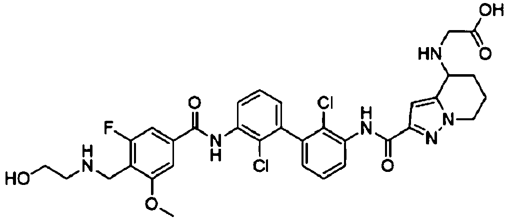 | 2-((2-((2,2'-dichloro-3'-(3-fluoro-4-(((2-hydroxyethyl)amino)methyl)-5-methoxybenzamido)-[1,1'-biphenyl]-3-yl)carbamoyl)-4,5,6,7-tetrahydropyrazolo[1,5-a]pyridin-4-yl)amino)acetic acid |
| 191 | 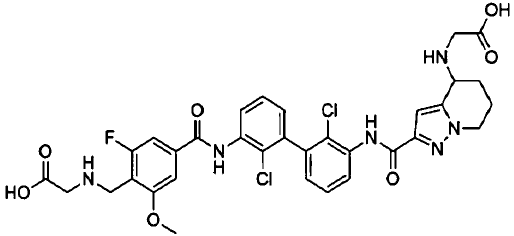 | 2-((4-((3'-(4-((carboxymethyl)amino)-4,5,6,7-tetrahydropyrazolo[1,5-a]pyridine-2-carboxamido)-2,2'-dichloro-[1,1'-biphenyl]-3-yl)carbamoyl)-2-fluoro-6-methoxybenzyl)amino)acetic acid |
| 192 | 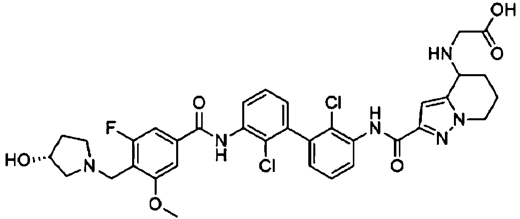 | 2-((2-((2,2'-dichloro-3'-(3-fluoro-4-(((R)-3-hydroxypyrrolidin-1-yl)methyl)-5-methoxybenzamido)-[1,1'-biphenyl]-3-yl)carbamoyl)-4,5,6,7-tetrahydropyrazolo[1,5-a]pyridin-4-yl)amino)acetic acid |
| 193 | 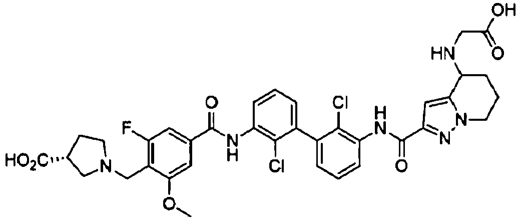 | (3R)-1-(4-((3'-(4-((carboxymethyl)amino)-4,5,6,7-tetrahydropyrazolo[1,5-a]pyridine-2-carboxamido)-2,2'-dichloro-[1,1'-biphenyl]-3-yl)carbamoyl)-2-fluoro-6-methoxybenzyl)pyrrolidine-3-carboxylic acid |
| 194 | 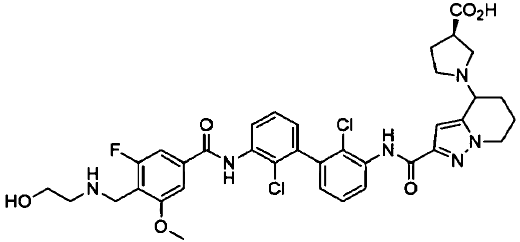 | (3R)-1-(2-((2,2'-dichloro-3'-(3-fluoro-4-(((2-hydroxyethyl)amino)methyl)-5-methoxybenzamido)-[1,1'-biphenyl]-3-yl)carbamoyl)-4,5,6,7-tetrahydropyrazolo[1,5-a]pyridin-4-yl)pyrrolidine-3-carboxylic acid |
| 195 | 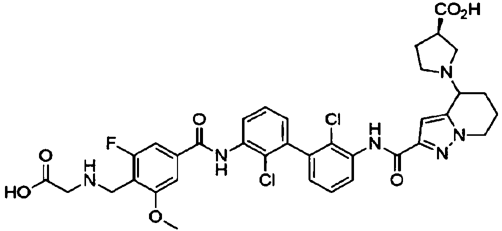 | (3R)-1-(2-((3'-(4-(((carboxymethyl)amino)meth yl)-3-fluoro-5-methoxybenzamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid |
| 196 | 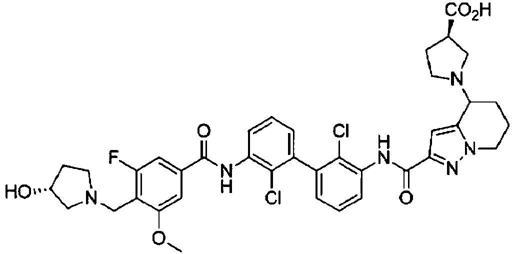 | (3R)-1-(2-((2,2'-dichloro-3'-(3-fluoro-4-(((R)-3-hydroxypyrrolidin-1-yl)methyl)-5-methoxybenzamido)-[1,1'-biphenyl]-3-yl)carbamoyl)-4,5,6,7-tetrahydropyrazolo[1,5-a]pyridin-4-yl)pyrrolidine-3-carboxylic acid |
| 197 | 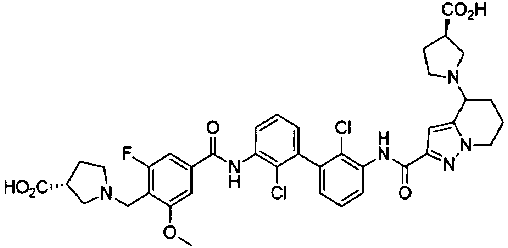 | (3R)-1-(4-((3'-(4-((R)-3-carboxypyrrolidin-1-yl)-4,5,6,7-tetrahydropyrazolo[1,5-a]pyridine-2-carboxamido)-2,2'-dichloro-[1,1'-biphenyl]-3-yl)carbamoyl)-2-fluoro-6-methoxybenzyl)pyrrolidine-3-carboxylic acid |
| 198 | 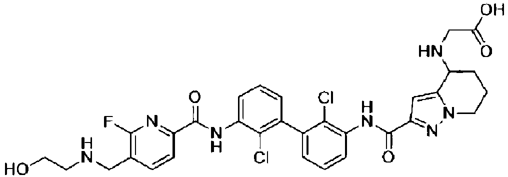 | 2-((2-((2,2'-dichloro-3'-(6-fluoro-5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)amino)acetic acid |
| 199 | 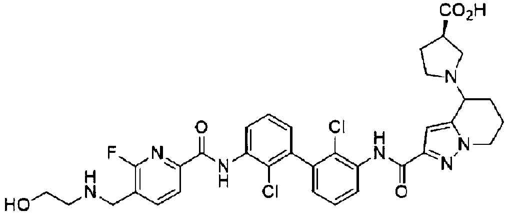 | (3R)-1-(2-((2,2'-dichloro-3'-(6-fluoro-5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)pyrrolidine-3-carboxylic acid |
| 200 | 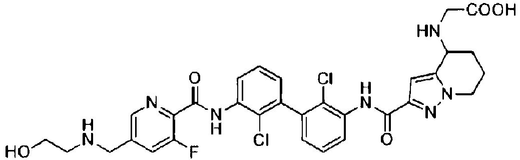 | 2-((2-((2,2'-dichloro-3'-(3-fluoro-5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)amino)acetic acid |
| 201 | 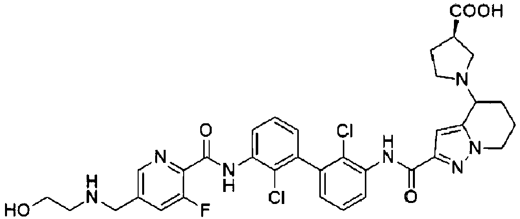 | (3R)-1-(2-((2,2'-dichloro-3'-(3-fluoro-5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)pyrrolidine-3-carboxylic acid |
| 202 | 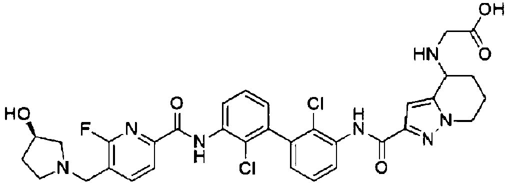 | 2-((2-((2,2'-dichloro-3'-(6-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid |
| 203 | 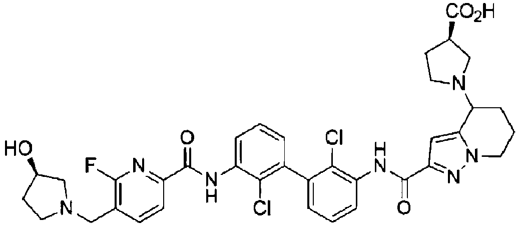 | (3R)-1-(2-((2,2'-dichloro-3'-(6-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid |
| 204 | 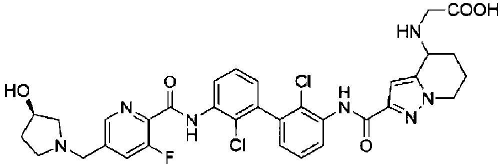 | 2-((2-((2,2'-dichloro-3'-(3-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid |
| 205 | 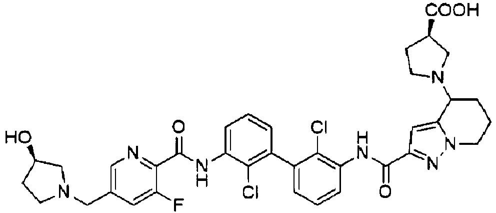 | (3R)-1-(2-((2,2'-dichloro-3'-(3-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid |
| 206 | 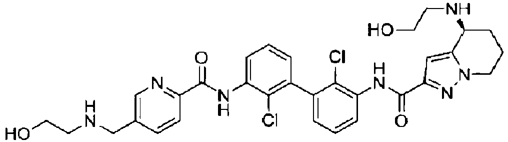 | (S)-N-(2,2'-dichloro-3'-(5-(((2-hydroxyethyl)amino)methyl)pi colinamido)-[1,1'-biphenyl]-3-yl)-4-((2-hydroxyethyl)amino)-4,5,6,7-tetrahydropyrazolo[1,5-a]pyridine-2-carboxamide |
| 207 | 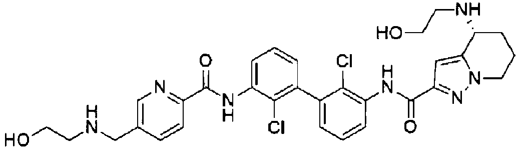 | (R)-N-(2,2'-dichloro-3'-(5-(((2-hydroxyethyl)amino)methyl)pi colinamido)-[1,1'-biphenyl]-3-yl)-4-((2-hydroxyethyl)amino)-4,5,6,7-tetrahydropyrazolo[1,5-a]pyridine-2-carboxamide |
| 208 | 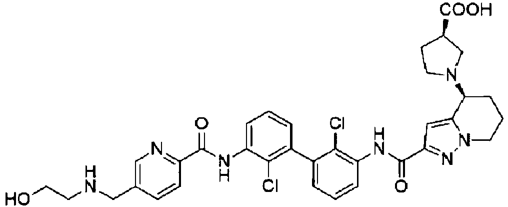 | (R)-1-((S)-2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)pyrrolidine-3-carboxylic acid |
| 209 | 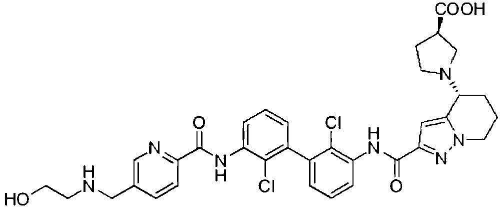 | (R)-1-((R)-2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a)pyridin-4-yl)pyrrolidine-3-carboxylic acid |
| 210 | 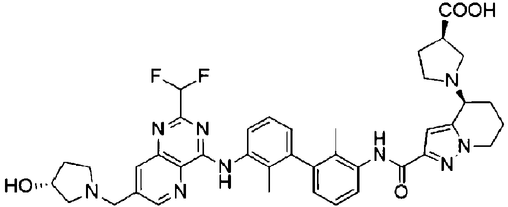 | (R)-1 -((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid |
| 211 | 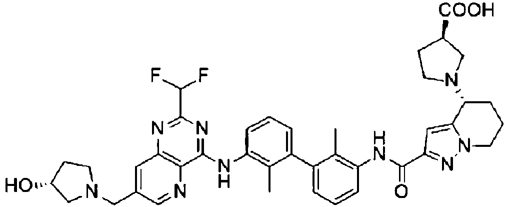 | (R)-1-((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid |
| 212 | 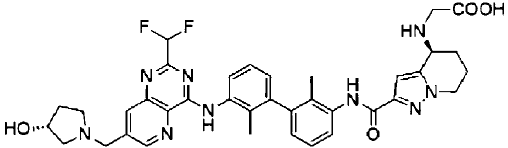 | 2-(((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetic acid |
| 213 | 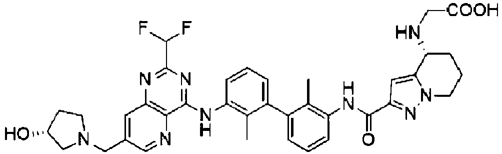 | 2-(((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetic acid |
| 214 | 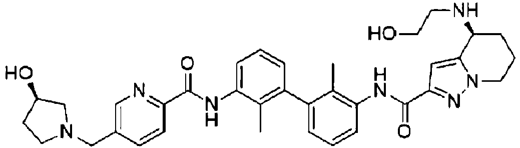 | (S)-4-((2-hydroxyethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 215 | 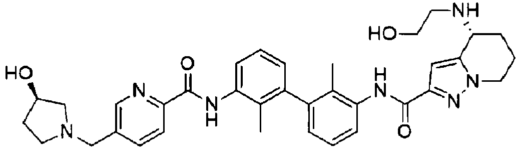 | (R)-4-((2-hydroxyethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 216 | 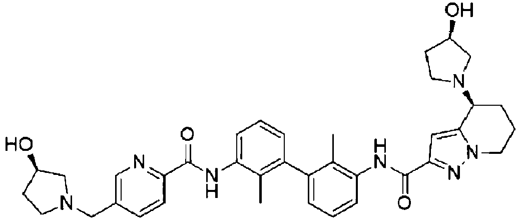 | (S)-4-((R)-3-hydroxypyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 217 | 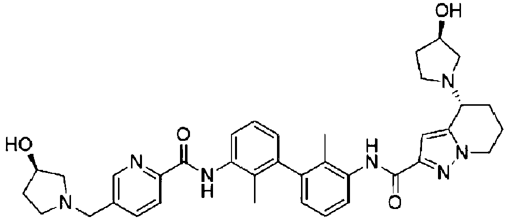 | ((R)-4-((R)-3-hydroxypyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 218 | 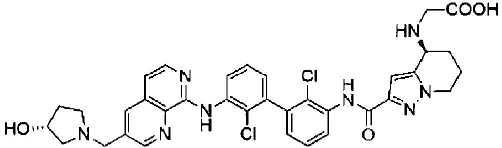 | 2-(((S)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetic acid |
| 219 | 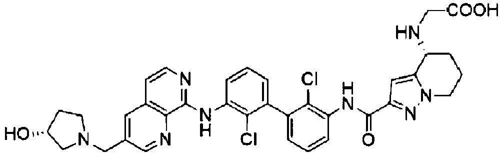 | 2-(((R)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetic acid |
| 220 | 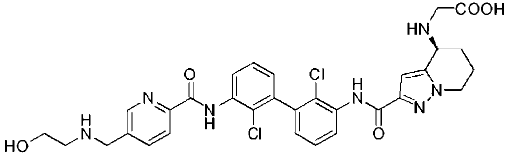 | (S)-2-((2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)amino)acetic acid |
| 221 | 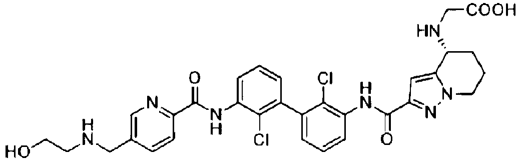 | (R)-2-((2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)amino)acetic acid |
| 222 | 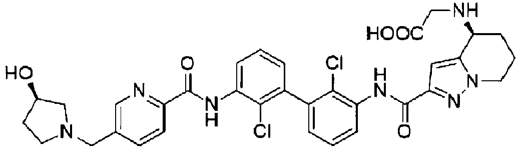 | 2-(((S)-2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid |
| 223 | 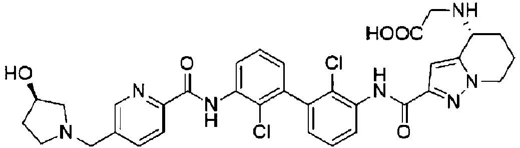 | 2-(((R)-2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid |
| 224 | 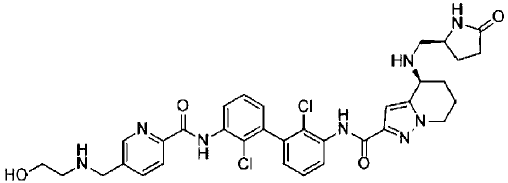 | (S)-N-(2,2'-dichloro-3'-(5-(((2-hydroxyethyl)amino)methyl)pi colinamido)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 225 | 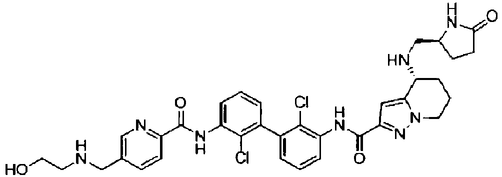 | (R)-N-(2,2'-dichloro-3'-(5-(((2-hydroxyethyl)amino)methyl)pi colinamido)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide |
| 226 | 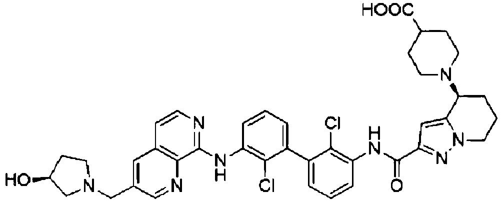 | 1-((S)-2-((2,2'-dichloro-3'-((3-(((S)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid |
| 227 | 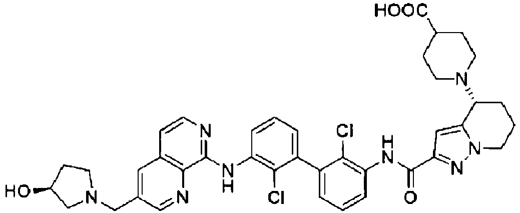 | 1-((R)-2-((2,2'-dichloro-3'-((3-(((S)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid |
| 228 | 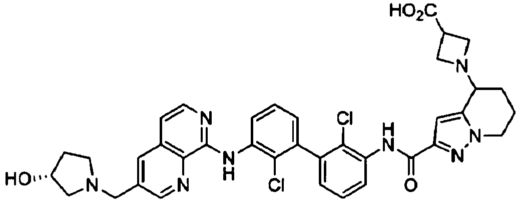 | 1-(2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)azetidine-3-carboxylic acid |
| 229 | 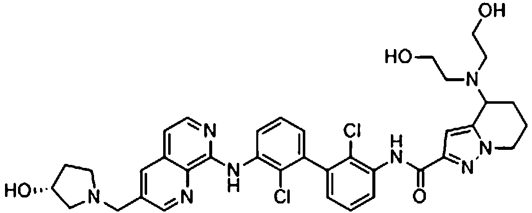 | 4-(bis(2-hydroxyethyl)amino)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridine-2-carboxamide |
| 230 | 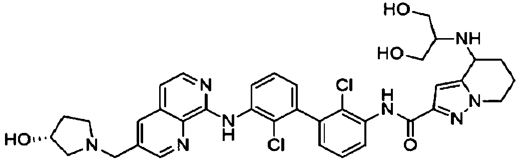 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((1,3-dihydroxypropan-2-yl)amino)-4,5,6,7-tetrahydropyrazolo[1,5-a]pyridine-2-carboxamide |
| 231 | 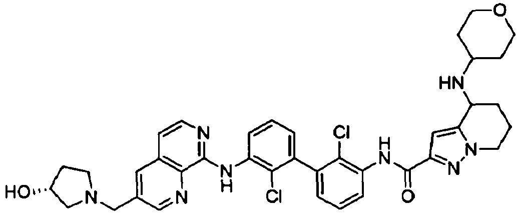 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((tetrahydro-2H-pyran-4-yl)amino)-4,5,6,7-tetrahydropyrazolo[1,5-a]pyridine-2-carboxamide |
| 232 | 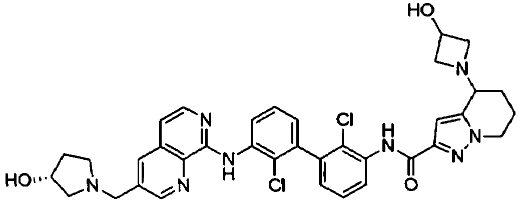 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(3-hydroxyazetidin-1-yl)-4,5,6,7-tetrahydropyrazolo[1,5-a]pyridine-2-carboxamide |
| 233 | 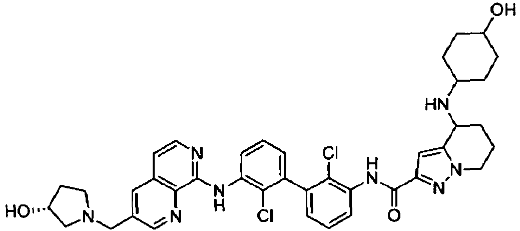 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((4-hydroxycyclohexyl)amino)-4,5,6,7-tetrahydropyrazolo[1,5-a]pyridine-2-carboxamide |
| 234 | 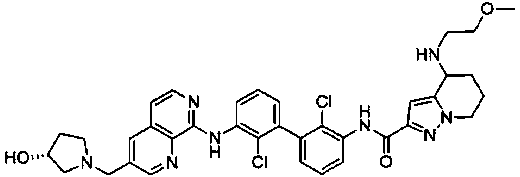 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((2-methoxyethyl)amino)-4,5,6,7-tetrahydropyrazolo[1,5-a]pyridine-2-carboxamide |
| 235 | 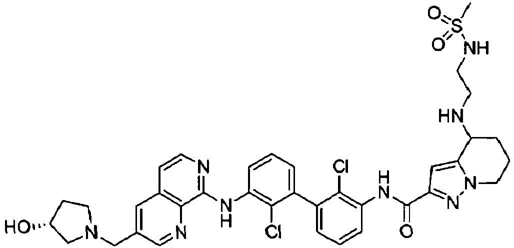 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((2-(methylsulfonamido)ethyl)ami no)-4,5,6,7-tetrahydropyrazolo[1,5-a]pyridine-2-carboxamide |
| 236 | 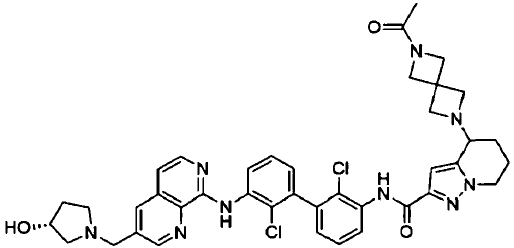 | 4-(6-acetyl-2,6-diazaspiro[3.3]heptan-2-yl)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridine-2-carboxamide |
| 237 | 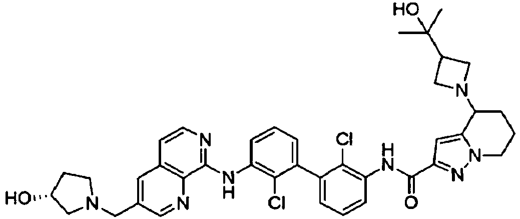 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(3-(2-hydroxypropan-2-yl)azetidin-1-yl)-4,5,6,7-tetrahydropyrazolo[1,5-a]pyridine-2-carboxamide |
| 238 | 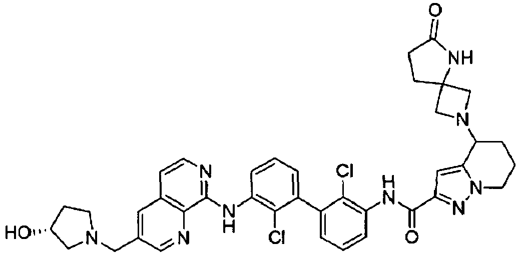 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(6-oxo-2,5-diazaspiro[3.4]octan-2-yl)-4,5,6,7-tetrahydropyrazolo[1,5-a]pyridine-2-carboxamide |
| 239 | 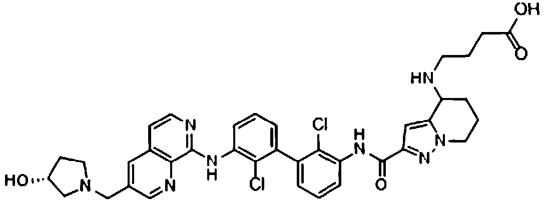 | 4-((2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)butanoic acid |
| 240 | 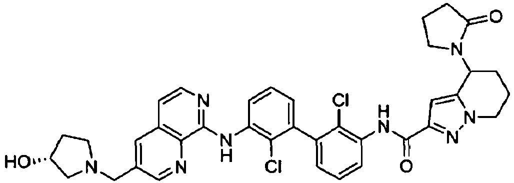 | N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(2-oxopyrrolidin-1-yl)-4,5,6,7-tetrahydropyrazolo[1,5-a]pyridine-2-carboxamide |
| 241 | 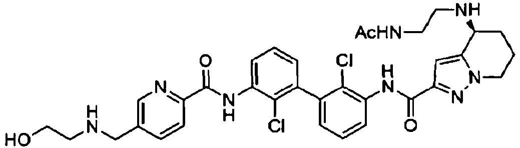 | (S)-4-((2-acetamidoethyl)amino)-N-(2,2'-dichloro-3'-(5-(((2-hydroxyethyl)amino)methyl)pi colinamido)-[1,1'-biphenyl]-3-yl)-4,5,6,7-tetrahydropyrazolo[1,5-a]pyridine-2-carboxamide |
| 242 | 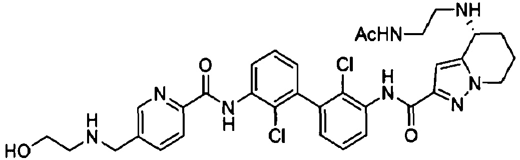 | (R)-4-((2-acetamidoethyl)amino)-N-(2,2'-dichloro-3'-(5-(((2-hydroxyethyl)amino)methyl)pi colinamido)-[1,1'-biphenyl]-3-yl)-4,5,6,7-tetrahydropyrazolo[1,5-a]pyridine-2-carboxamide |
| 243 | 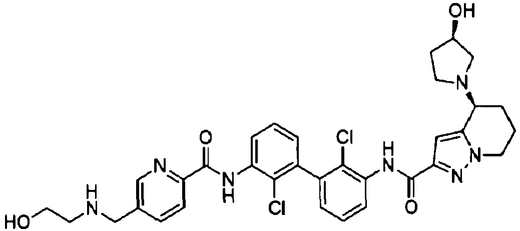 | (S)-N-(2,2'-dichloro-3'-(5-(((2-hydroxyethyl)amino)methyl)pi colinamido)-[1,1'-biphenyl]-3-yl)-4-((R)-3-hydroxypyrrolidin-1-yl)-4,5,6,7-tetrahydropyrazolo[1,5-a]pyridine-2-carboxamide |
| 244 | 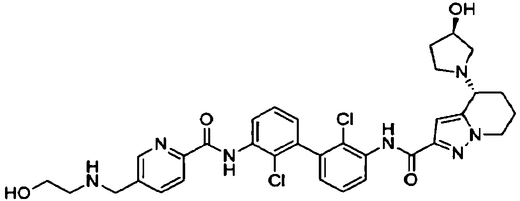 | (R)-N-(2,2'-dichloro-3'-(5-(((2-hydroxyethyl)amino)methyl)pi colinamido)-[1,1'-biphenyl]-3-yl)-4-((R)-3-hydroxypyrrolidin-1-yl)-4,5,6,7-tetrahydropyrazolo[1,5-a]pyridine-2-carboxamide |
| 245 | 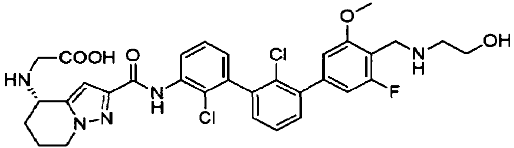 | (S)-2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetic acid |
| 246 | 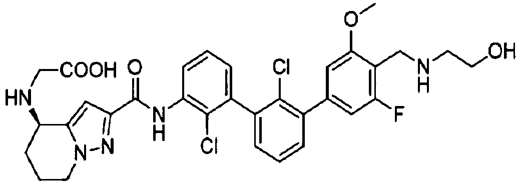 | (R)-2-((2-((2,2'-dichloro-3"-fluoro-4"-(((2-hydroxyethyl)amino)methyl)-5"-methoxy-[1,1":3',1"-terphenyl]-3-yl)carbamoyl)-4,5,6,7-tetrahydropyrazolo[1,5-a]pyridin-4-yl)amino)acetic acid |
| 247 | 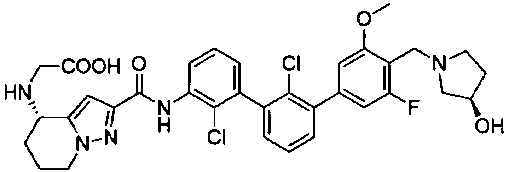 | 2-(((S)-2-((2,2'-dichloro-3"-fluoro-4"-(((R)-3-hydroxypyrrolidin-1-yl)methyl)-5"-methoxy-[1,1':3',1"-terphenyl]-3-yl)carbamoyl)-4,5,6,7-tetrahydropyrazolo[1,5-a]pyridin-4-yl)amino)acetic acid |
| 248 | 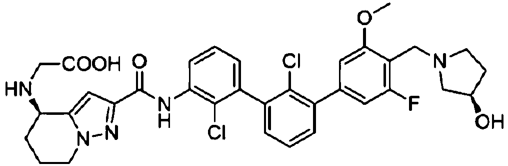 | 2-(((R)-2-((2,2'-dichloro-3"-fluoro-4"-(((R)-3-hydroxypyrrolidin-1-yl)methyl)-5"-methoxy-[1,1':3',1"-terphenyl]-3-yl)carbamoyl)-4,5,6,7-tetrahydropyrazolo[1,5-a]pyridin-4-yl)amino)acetic acid |
| 249 | 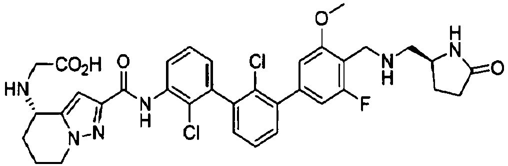 | 2-(((S)-2-((2,2'-dichloro-3"-fluoro-5"-methoxy-4"-(((((S)-5-oxopyrrolidin-2-yl)methyl)amino)methyl)-[1,1':3',1"-terphenyl]-3-yl)carbamoyl)-4,5,6,7-tetrahydropyrazolo[1,5-a]pyridin-4-yl)amino)acetic acid |
| 250 | 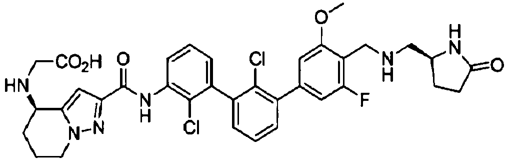 | 2-(((R)-2-((2,2'-dichloro-3"-fluoro-5"-methoxy-4"-(((((S)-5-oxopyrrolidin-2-yl)methyl)amino)methyl)-[1,1':3',1"-terphenyl]-3-yl)carbamoyl)-4,5,6,7-tetrahydropyrazolo[1,5-a]pyridin-4-yl)amino)acetic acid |
| 251 | 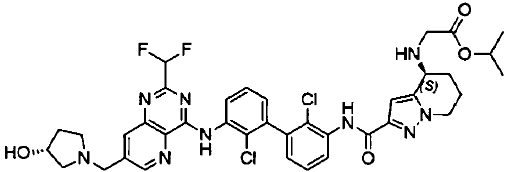 | isopropyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate |
| 252 | 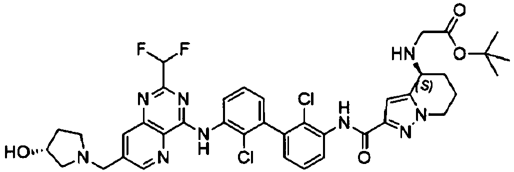 | tert-butyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate |
| 253 | 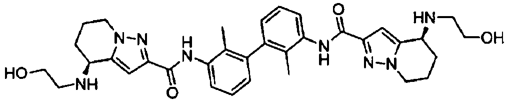 | (4S,4'S)-N,N'-(2,2'-dimethyl-[1,1'-biphenyl]-3,3'-diyl)bis(4-((2-hydroxyethyl)amino)-4,5,6,7-tetrahydropyrazolo[1,5-a]pyridine-2-carboxamide) |
| 254 | 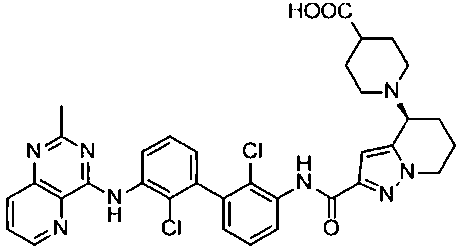 | (S)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 255 | 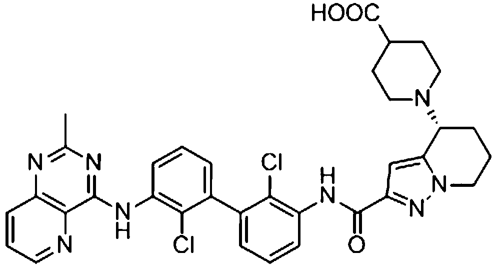 | (R)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 256 | 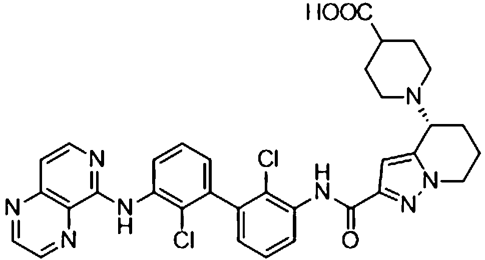 | (R)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)piperidine-4-carboxylic acid |
| 257 | 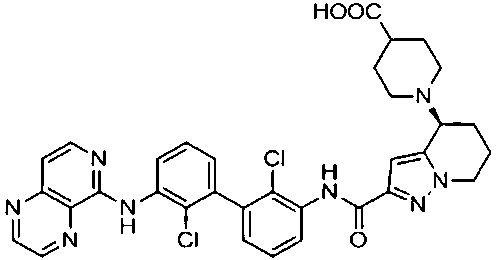 | (S)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)piperidine-4-carboxylic acid |
| 258 | 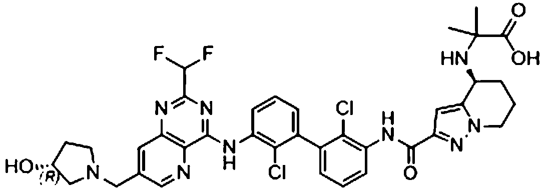 | 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 259 | 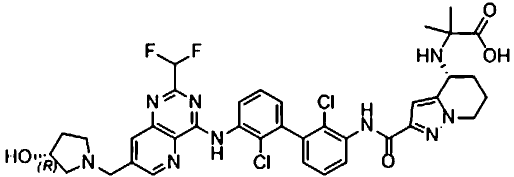 | 2-(((R)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 259 -1 | 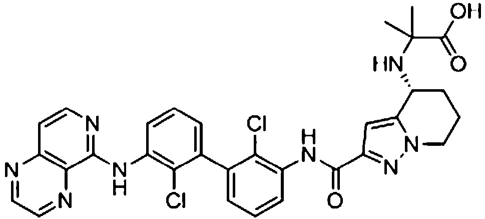 | (R)-2-((2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)amino)-2-methylpropanoic acid |
| 260 | 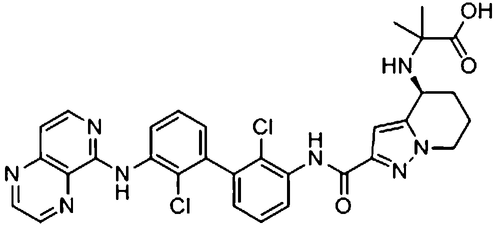 | (S)-2-((2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)amino)-2-methylpropanoic acid |
| 261 | 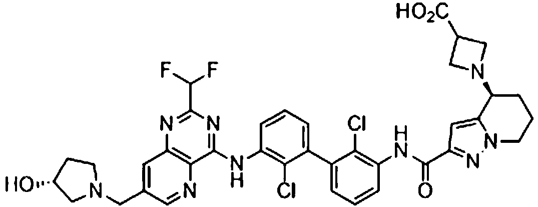 | 1-((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 262 | 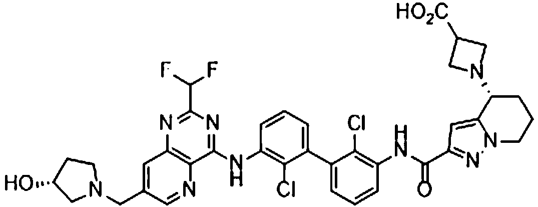 | 1-((R)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 263 | 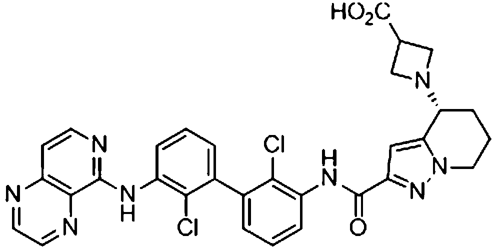 | (R)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)azetidine-3-carboxylic acid |
| 264 | 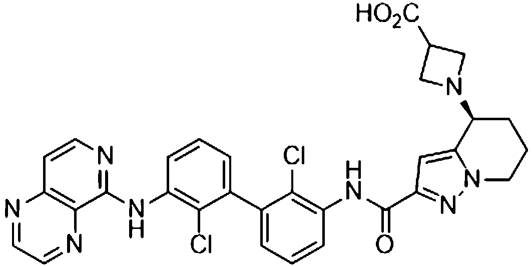 | (S)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)azetidine-3-carboxylic acid |
| 265 | 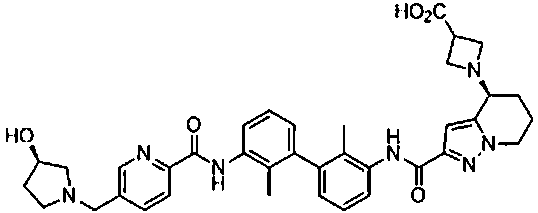 | 1-((S)-2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)azetidine-3-carboxylic acid |
| 266 | 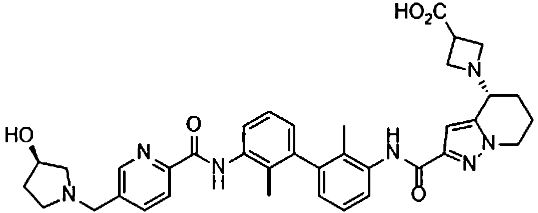 | 1-((R)-2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)azetidine-3-carboxylic acid |
| 267 | 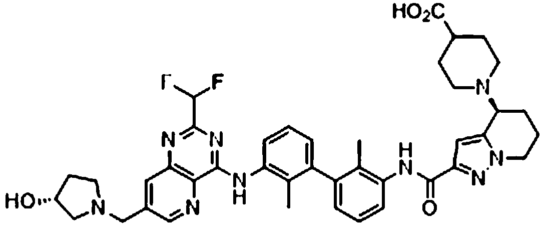 | 1-((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 268 | 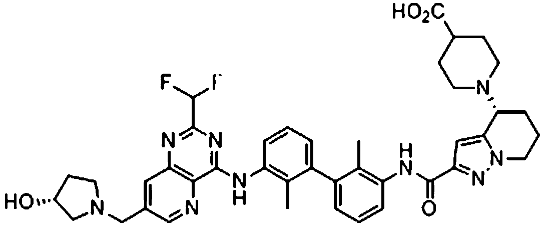 | 1-((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 269 | 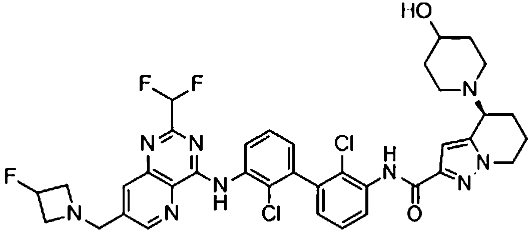 | (S)-N-(2,2'-dichloro-3'-((2-(difluoromethyl)-7-((3-fluoroazetidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 270 | 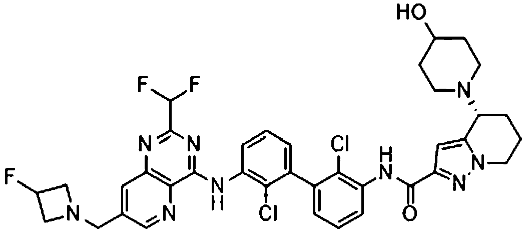 | (R)-N-(2,2'-dichloro-3'-((2-(difluoromethyl)-7-((3-fluoroazetidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 271 | 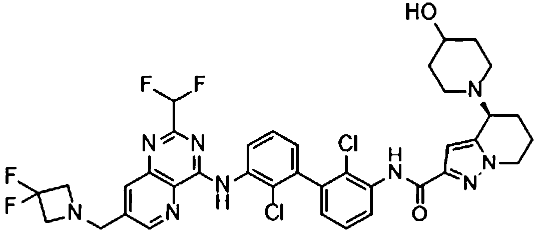 | (S)-N-(2,2'-dichloro-3'-((7-((3,3-difluoroazetidin-1-yl)methyl)-2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 272 | 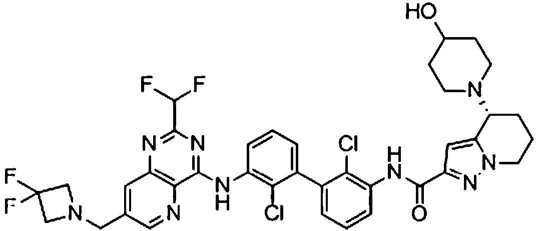 | (R)-N-(2,2'-dichloro-3'-((7-((3,3-difluoroazetidin-1-yl)methyl)-2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 273 | 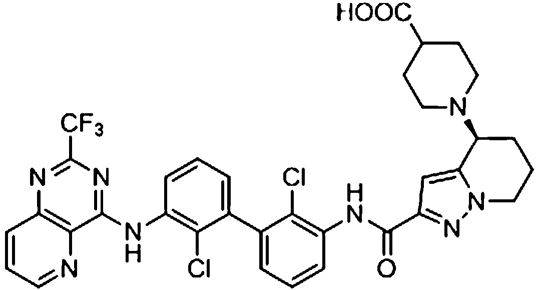 | (S)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 274 | 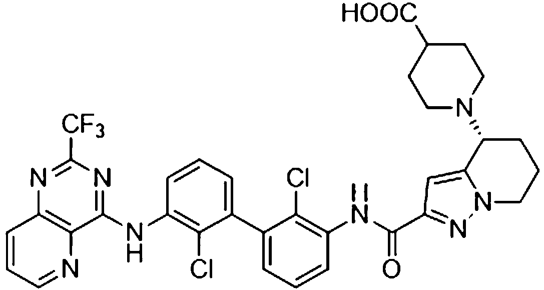 | (R)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 275 | 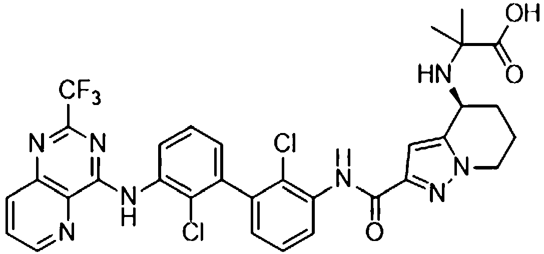 | (S)-2-((2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 276 | 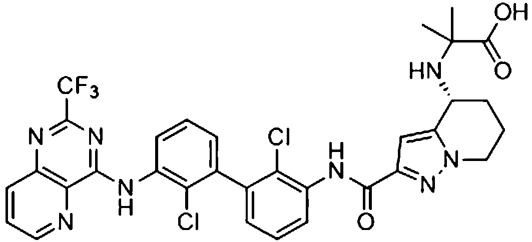 | (R)-2-((2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 277 | 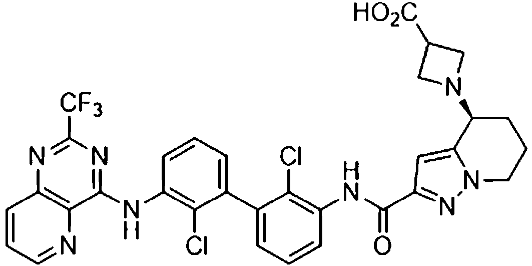 | (S)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 278 | 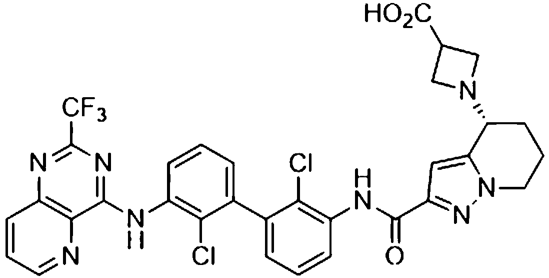 | (R)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 279 | 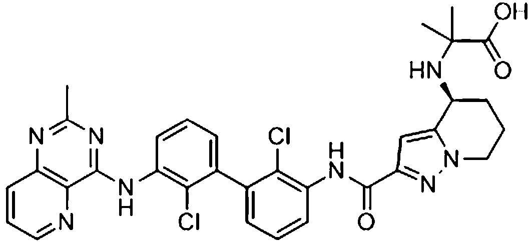 | (S)-2-((2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 280 | 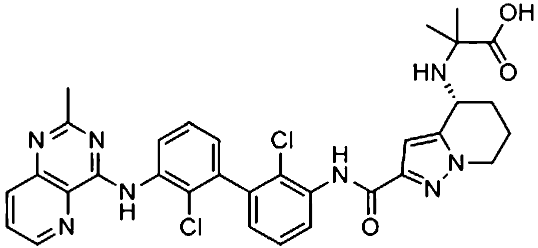 | (R)-2-((2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 281 | 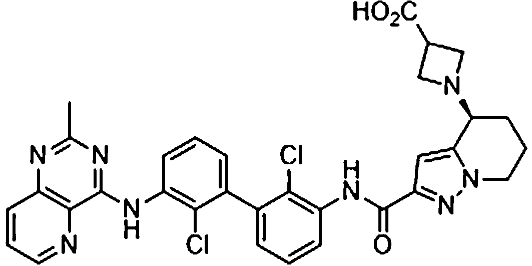 | (S)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 282 | 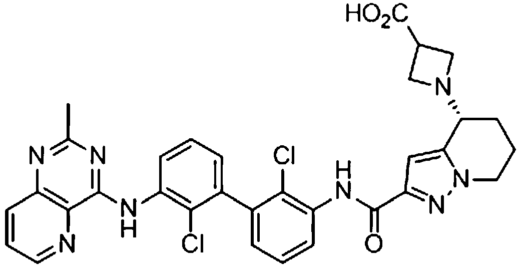 | (R)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 283 | 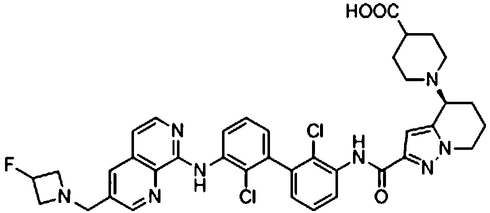 | (S)-1-(2-((2,2'-dichloro-3'-((3-((3-fluoroazetidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid |
| 284 | 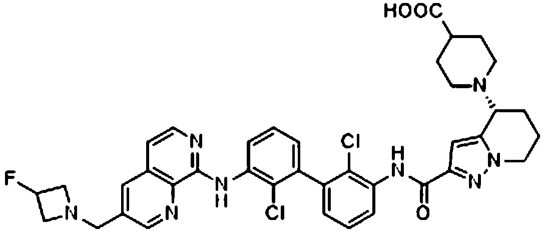 | (R)-1-(2-((2,2'-dichloro-3'-((3-((3-fluoroazetidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid |
| 285 | 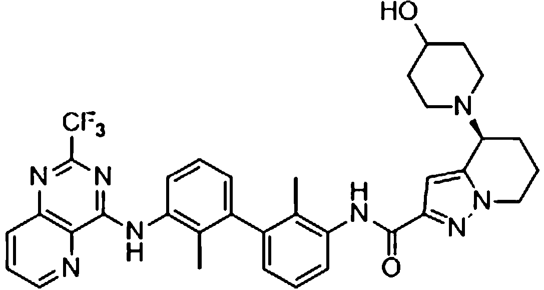 | (S)-N-(2,2'-dimethyl-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 286 | 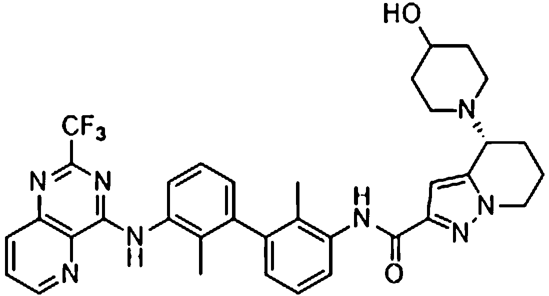 | (R)-N-(2,2'-dimethyl-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 287 | 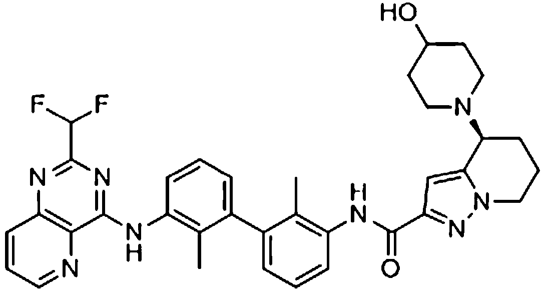 | (S)-N-(3'-((2-(difluoromethyl)pyrido[3,2-d]pyrimidin-4-yl)amino)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide |
| 288 | 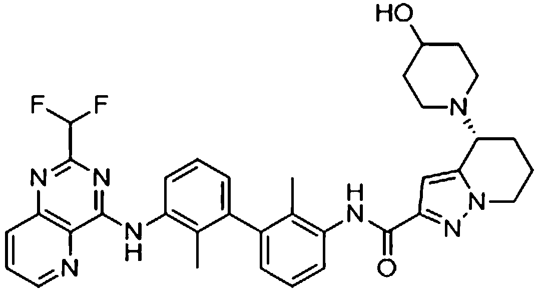 | (R)-N-(3'-((2-(difluoromethyl)pyrido[3,2-d]pyrimidin-4-yl)amino)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide |
| 289 | 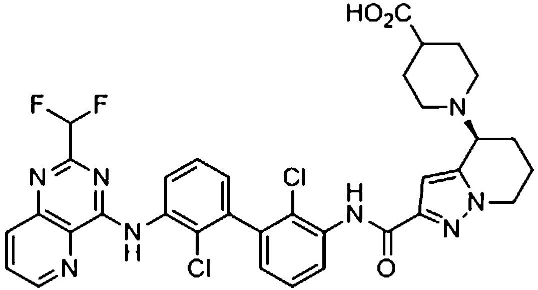 | (S)-1-(2-((2,2'-dichloro-3'-((2-(difluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 290 | 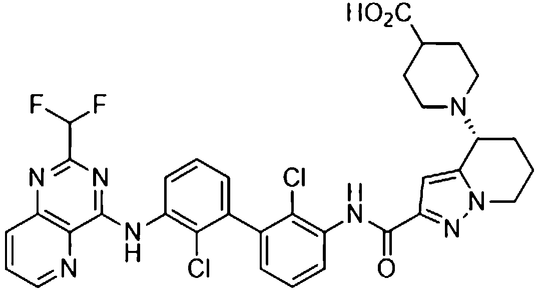 | (R)-1-(2-((2,2'-dichloro-3'-((2-(difluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 291 | 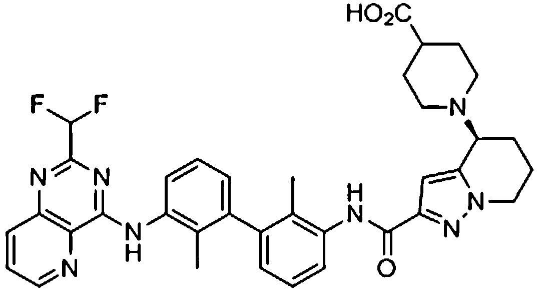 | (S)-1-(2-((3'-((2-(difluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 292 | 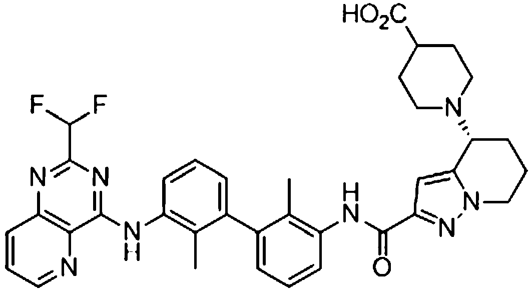 | (R)-1-(2-((3'-((2-(difluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 293 | 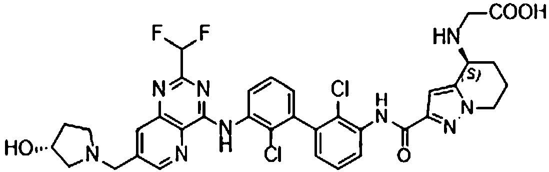 | 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetic acid |
| 294 | 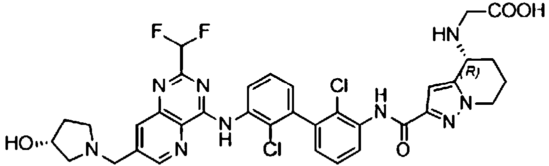 | 2-(((R)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetic acid |
| 295 | 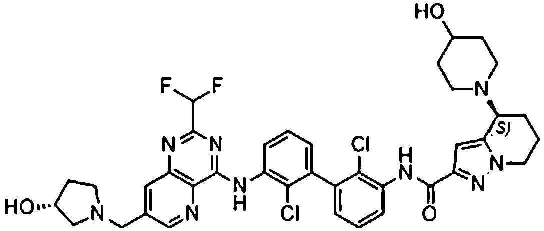 | (S)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 296 | 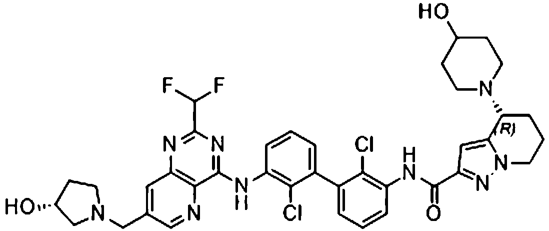 | (R)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 297 | 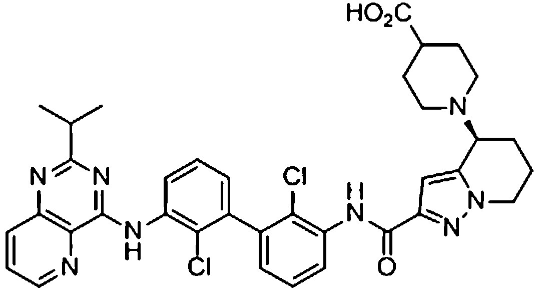 | (S)-1-(2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 298 | 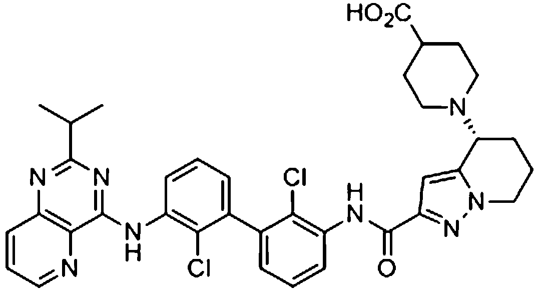 | (R)-1-(2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 299 | 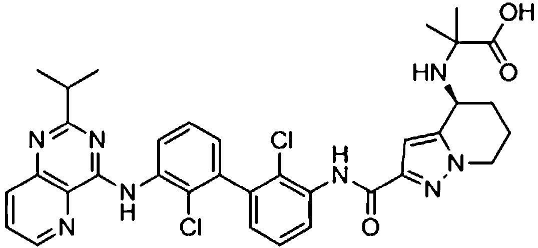 | (S)-2-((2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 300 | 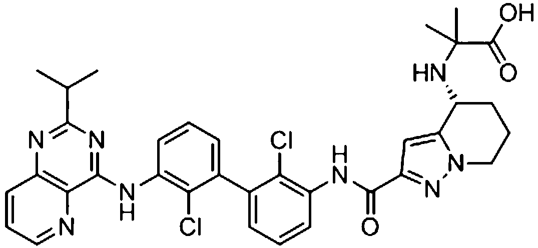 | (R)-2-((2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 301 | 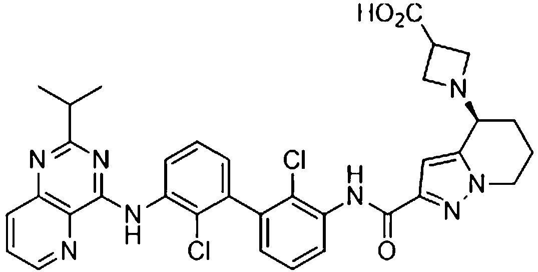 | (S)-1-(2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 302 | 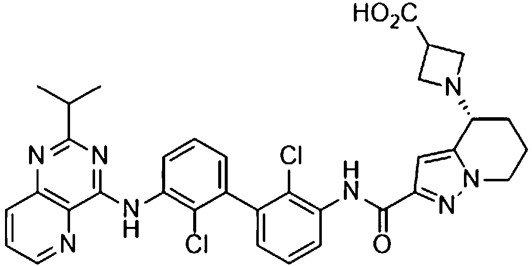 | (R)-1-(2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid |
| 303 | 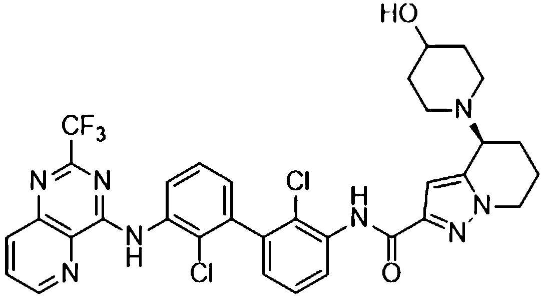 | (S)-N-(2,2'-dichloro-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 304 | 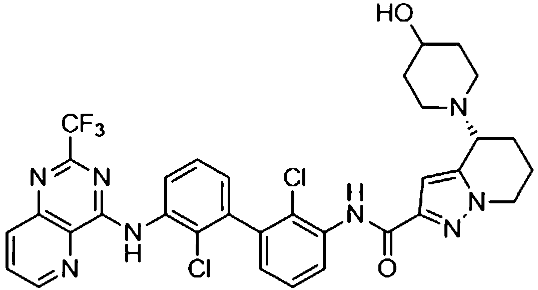 | (R)-N-(2,2'-dichloro-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 305 | 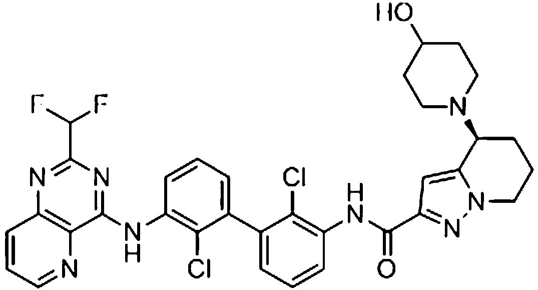 | (S)-N-(2,2'-dichloro-3'-((2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 306 | 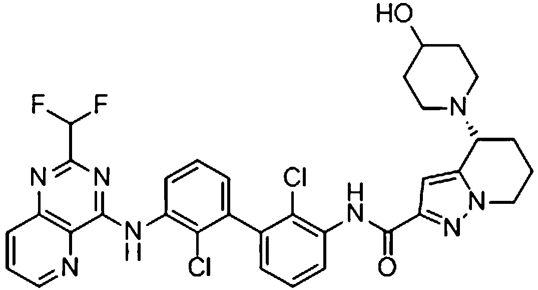 | (R)-N-(2,2'-dichloro-3'-((2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide |
| 307 | 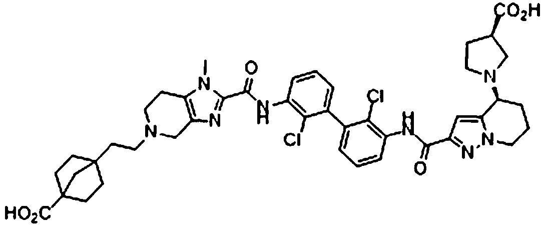 | (R)-1-((S)-2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid |
| 308 | 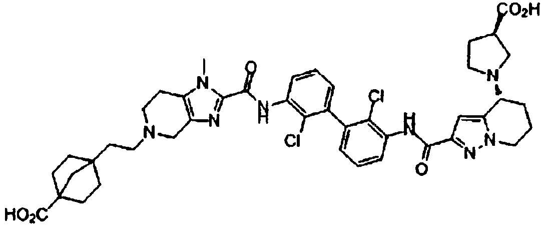 | (R)-1-((R)-2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid |
| 309 | 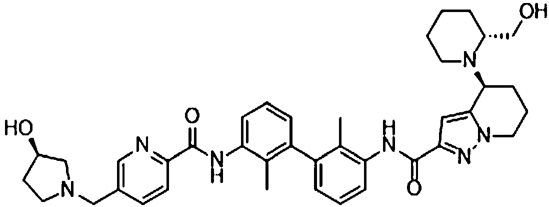 | (S)-4-((R)-2-(hydroxymethyl)piperidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 309 -1 | 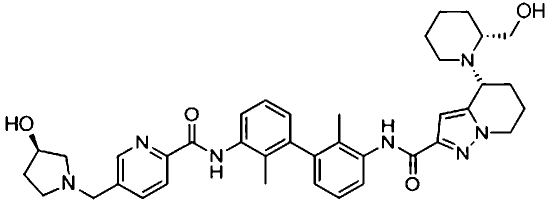 | (R)-4-((R)-2-(hydroxymethyl)piperidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 310 | 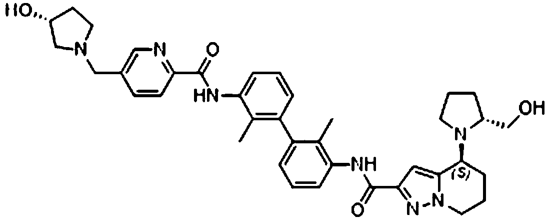 | (S)-4-((R)-2-(hydroxymethyl)pyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 310 -1 | 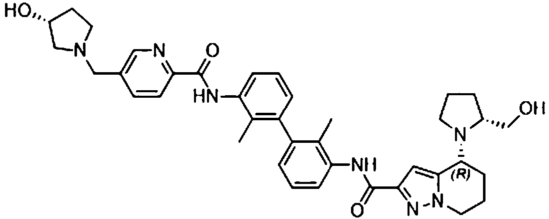 | (R)-4-((R)-2-(hydroxymethyl)pyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide |
| 311 | 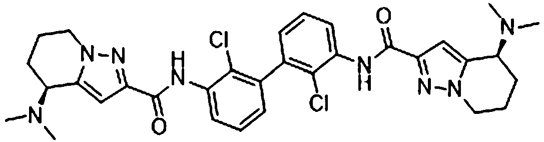 | (4S,4'S)-N,N'-(2,2'-dichloro-[1,1'-biphenyl]-3,3'-diyl)bis(4-(dimethylamino)-4,5,6,7-tetrahydropyrazolo[1,5-a]pyridine-2-carboxamide) |
| 312 | 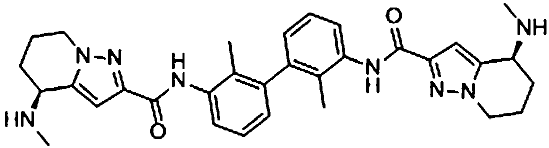 | (4S,4'S)-N,N'-(2,2'-dimethyl-[1,1'-biphenyl]-3,3'-diyl)bis(4-(methylamino)-4,5,6,7-tetrahydropyrazolo[1,5-a]pyridine-2-carboxamide) |
| 313 | 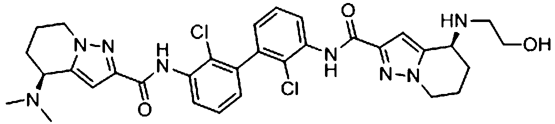 | (S)-N-(2,2'-dichloro-3'-((S)-4-((2-hydroxyethyl)amino)-4,5,6,7-tetrahydropyrazolo[1,5-a]pyridine-2-carboxamido)-[1,1'-biphenyl]-3-yl)-4-(dimethylamino)-4,5,6,7-tetrahydropyrazolo[1,5-a]pyridine-2-carboxamide |
| 314 | 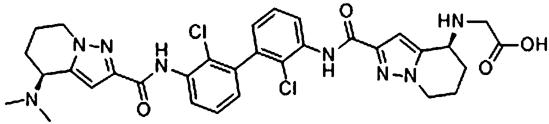 | 2-(((S)-2-((2,2'-dichloro-3'-((S)-4-(dimethylamino)-4,5,6,7-tetrahydropyrazolo[1,5-a]pyridine-2-carboxamido)-[1,1'-biphenyl]-3-yl)carbamoyl)-4,5,6,7-tetrahydropyrazolo[1,5-a]pyridin-4-yl)amino)acetic acid |
| 315 | 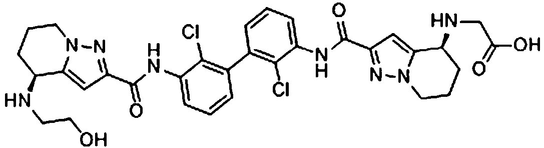 | 2-(((S)-2-((2,2'-dichloro-3'-((S)-4-((2-hydroxyethyl)amino)-4,5,6,7-tetrahydropyrazolo[1,5-a]pyridine-2-carboxamido)-[1,1'-biphenyl]-3-yl)carbamoyl)-4,5,6,7-tetrahydropyrazolo[1,5-a]pyridin-4-yl)amino)acetic acid |
| 316 | 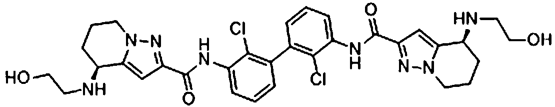 | (4S,4'S)-N,N'-(2,2'-dichloro-[1,1'-biphenyl]-3,3'-diyl)bis(4-((2-hydroxyethyl)amino)-4,5,6,7-tetrahydropyrazolo[1,5-a]pyridine-2-carboxamide) |
| 317 | 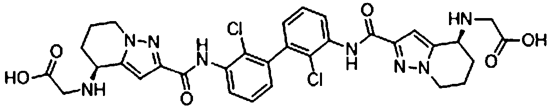 | 2,2'-(((4S,4'S)-2,2'-(((2,2'-dichloro-[1,1'-biphenyl]-3,3'-diyl)bis(azanediyl))bis(carbony l))bis(4,5,6,7-tetrahydropyrazolo[1,5-a]pyridine-4,2-diyl))bis(azanediyl))diacetic acid |
| 318 | 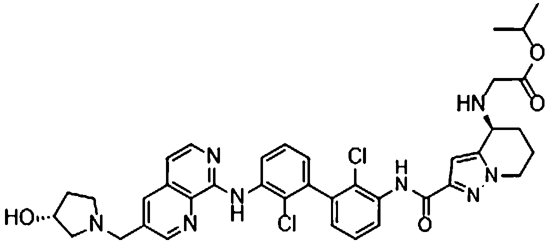 | isopropyl 2-(((S)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetate |
| 319 | 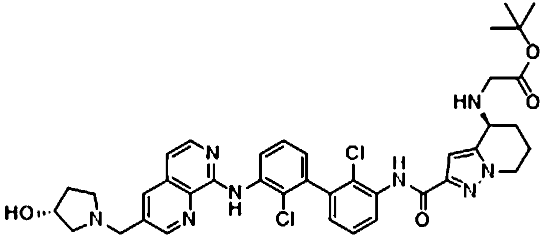 | tert-butyl 2-(((S)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetate |
| 320 | 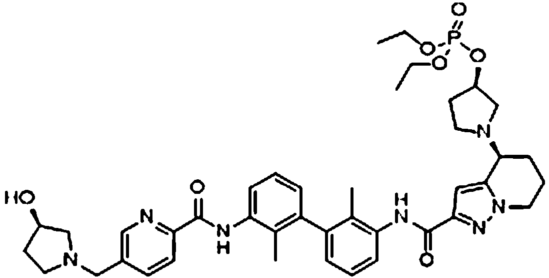 | diethyl ((R)-1-((S)-2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)pyrrolidin-3-yl) phosphate |
| 321 | 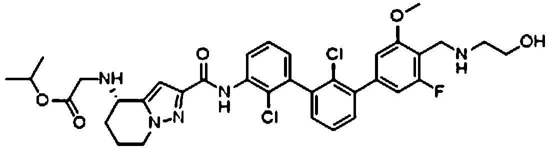 | (S)-isopropyl 2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetate |
| 322 | 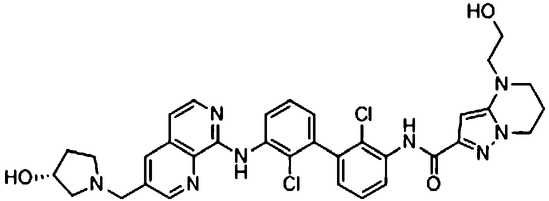 | (R)-N-(2,2'-dichloro-3'-((3-((3-hydroxypyrrolidin-1-yl)methyl)-1,7-naphthyridin-8-yl)amino)-[1,1'-biphenyl]-3-yl)-4-(2-hydroxyethyl)-4,5,6,7-tetrahydropyrazolo[1,5-a]pyrimidine-2-carboxamide |
| 323 | 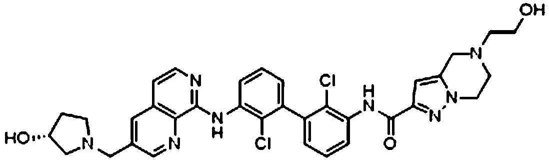 | (R)-N-(2,2'-dichloro-3'-((3-((3-hydroxypyrrolidin-1-yl)methyl)-1,7-naphthyridin-8-yl)amino)-[1,1'-biphenyl]-3-yl)-5-(2-hydroxyethyl)-4,5,6,7-tetrahydropyrazolo[1,5-a]pyrazine-2-carboxamide |
| 324 | 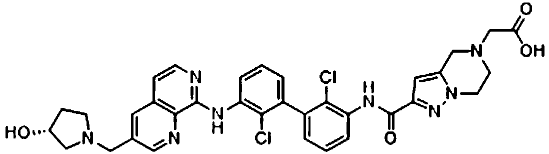 | (R)-2-(2-((2,2'-dichloro-3'-((3-((3-hydroxypyrrolidin-1-yl)methyl)-1,7-naphthyridin-8-yl)amino)-[1,1'-biphenyl]-3-yl)carbamoyl)-6,7-dihydropyrazolo[1,5-a]pyrazin-5(4H)-yl)acetic acid |
| 325 | 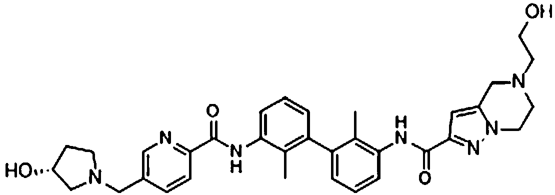 | (R)-5-(2-hydroxyethyl)-N-(3'-(5-((3-hydroxypyrrolidin-1-yl)methyl)picolinamido)-2,2'-dimethyl-[1,1'-biphenyl]-3-yl)-4,5,6,7-tetrahydropyrazolo[1,5-a]pyrazine-2-carboxamide |
| 326 | 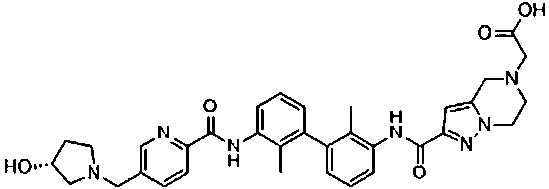 | (R)-2-(2-((3'-(5-((3-hydroxypyrrolidin-1-yl)methyl)picolinamido)-2,2'-dimethyl-[1,1'-biphenyl]-3-yl)carbamoyl)-6,7-dihydropyrazolo[1,5-a]pyrazin-5(4H)-yl)acetic acid |
| 327 | 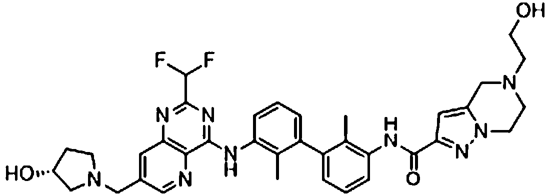 | (R)-N-(3'-((2-(difluoromethyl)-7-((3-hydroxypyrrolidin-1-yl)methyl)pyrido[3,2-d]pyrimidin-4-yl)amino)-2,2'-dimethyl-[1,1'-biphenyl]-3-yl)-5-(2-hydroxyethyl)-4,5,6,7-tetrahydropyrazolo[1,5-a]pyrazine-2-carboxamide |
| 328 | 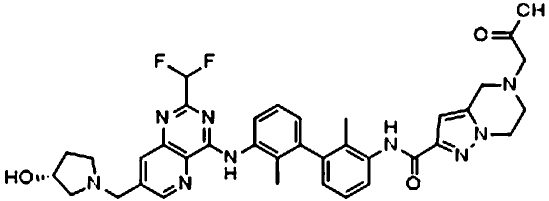 | (R)-2-(2-((3'-((2-(difluoromethyl)-7-((3-hydroxypyrrolidin-1-yl)methyl)pyrido[3,2-d]pyrimidin-4-yl)amino)-2,2'-dimethyl-[1,1'-biphenyl]-3-yl)carbamoyl)-6,7-dihydropyrazolo[1,5-a]pyrazin-5(4H)-yl)acetic acid |
| 329 | 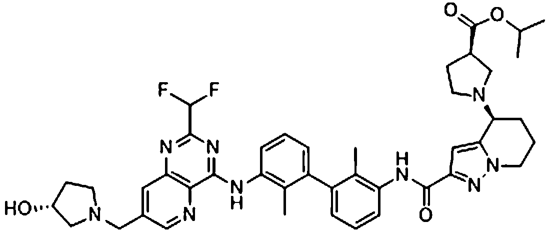 | (R)-isopropyl 1-((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylate |
| 329 -1 | 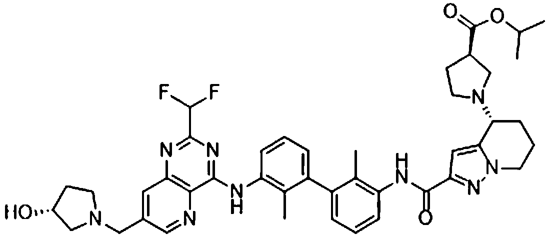 | (R)-isopropyl 1-((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylate |
| 330 | 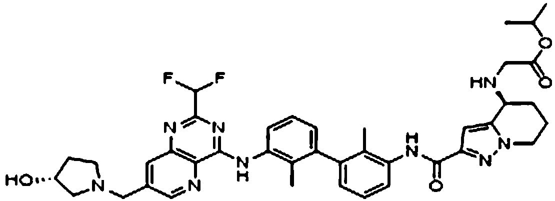 | isopropyl 2-(((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetate |
| 331 | 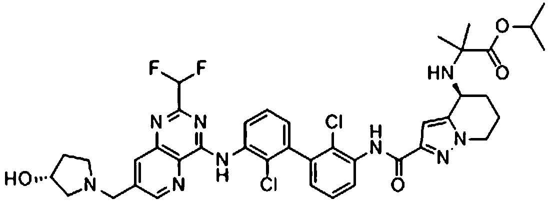 | isopropyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoate |
| 331 -1 | 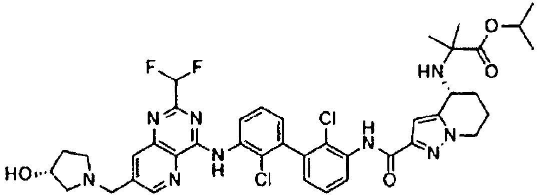 | isopropyl 2-(((R)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoate |
| 332 | 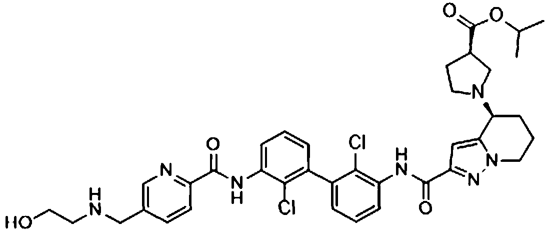 | (R)-isopropyl 1-((S)-2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)pyrrolidine-3-carboxylate |
| 332 -1 | 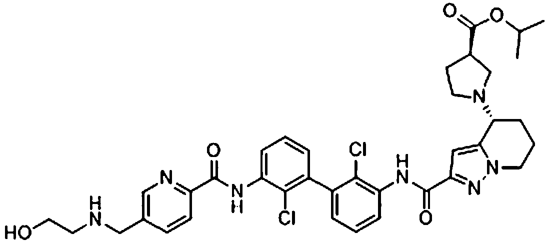 | (R)-isopropyl 1-((R)-2-((2,2'-dichloro-3'-(5-(((2-hydroxyethyl)amino)methyl)pi colinamido)-[1,1'-biphenyl]-3-yl)carbamoyl)-4,5,6,7-tetrahydropyrazolo[1,5-a]pyridin-4-yl)pyrrolidine-3-carboxylate |
| 333 | 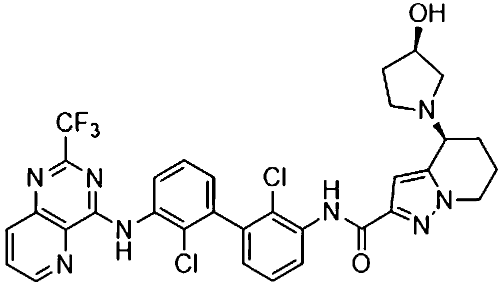 | (S)-N-(2,2'-dichloro-3'-((2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 334 | 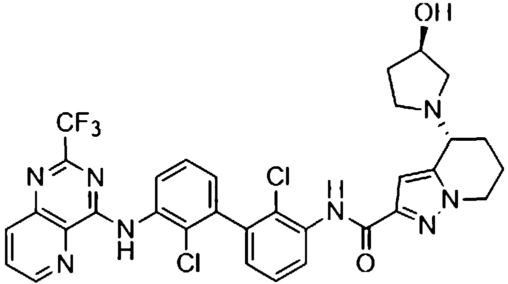 | (R)-N-(2,2'-dichloro-3'-((2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 335 | 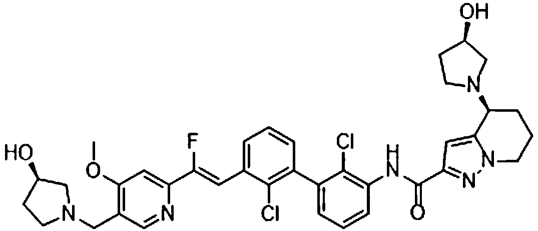 | (S)-N-(2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)-4-((R)-3-hydroxypyrrolidin-1-yl)-4,5,6,7-tetrahydropyrazolo[1,5-a]pyridine-2-carboxamide |
| 336 | 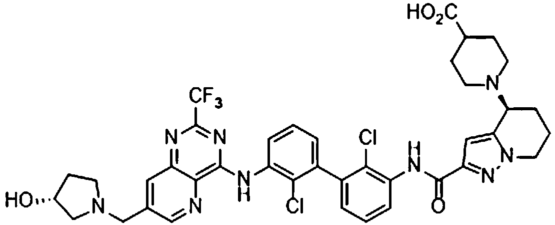 | 1-((S)-2-((2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 337 | 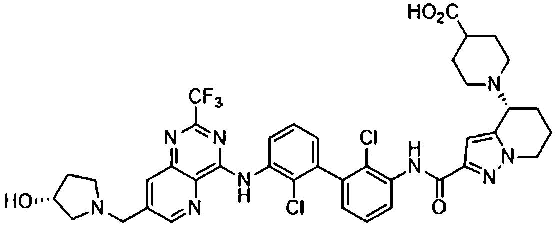 | 1-((R)-2-((2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid |
| 338 | 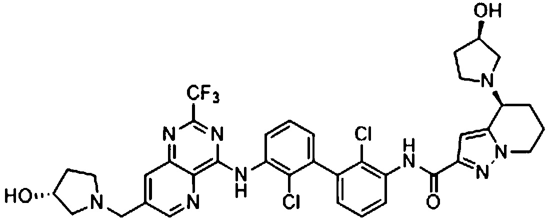 | (S)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 339 | 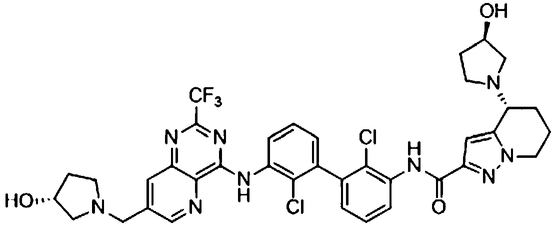 | (R)-N-(2,2'-dichloro-3'-((7-(((R)-3-hydroxypyrrolidin-1 - yl)methyl)-2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 340 | 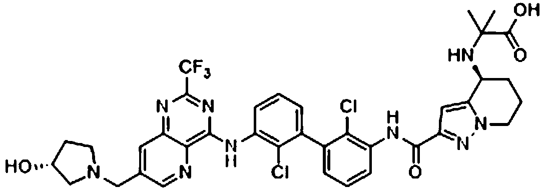 | 2-(((S)-2-((2,2'-dichloro-3'-((7-(((R)-3-hydroxypyrrolidin-1 - yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 341 | 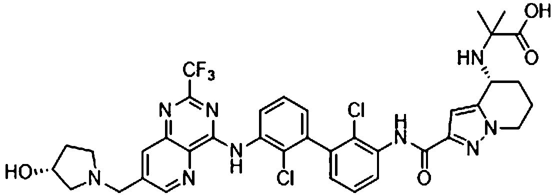 | 2-(((R)-2-((2,2'-dichloro-3'-((7-(((R)-3-hydroxypyrrolidin-1 - yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid |
| 342 | 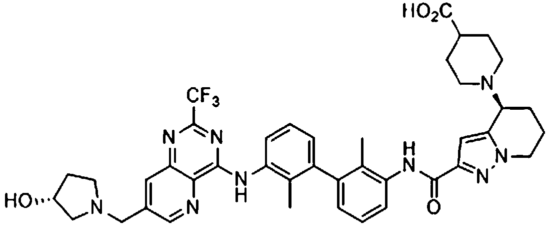 | 1-((S)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 343 | 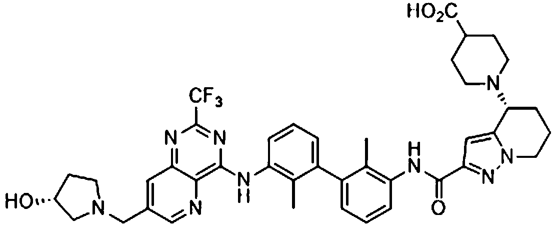 | 1-((R)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid |
| 344 | 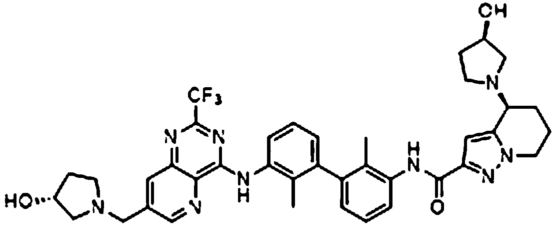 | (S)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide |
| 345 | 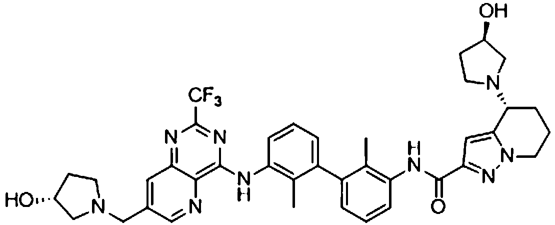 | (R)-4-((R)-3-hydroxypyrrolidin-1-yl)-N-(3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)-4,5,6,7-tetrahydropyrazolo[1,5-a]pyridine-2-carboxamide |
| 346 | 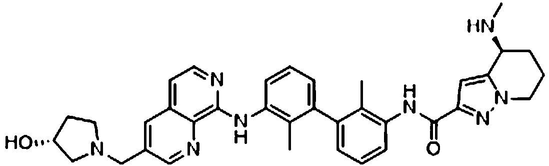 | (S)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4-(methylamino)-4,5,6,7-tetrahydropyrazolo[1,5-a]pyridine-2-carboxamide |
| 347 | 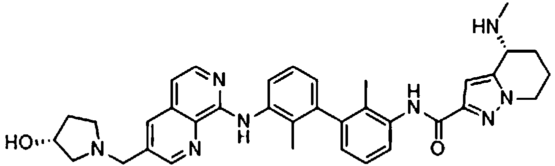 | (R)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4-(methylamino)-4,5,6,7-tetrahydropyrazolo[1,5-a]pyridine-2-carboxamide |
| 348 | 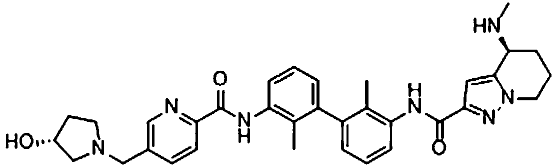 | (S)-N-(3'-(5-(((R)-3-hydroxypyrrolidin-1-yl)methyl)picolinamido)-2,2'-dimethyl-[1,1'-biphenyl]-3-yl)-4-(methylamino)-4,5,6,7-tetrahydropyrazolo[1,5-a]pyridine-2-carboxamide |
| 349 | 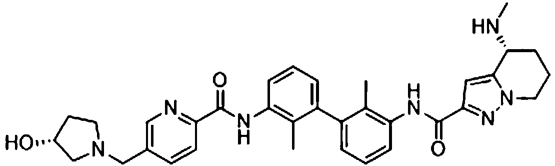 | (R)-N-(3'-(5-(((R)-3-hydroxypyrrolidin-1-yl)methyl)picolinamido)-2,2'-dimethyl-[1,1'-biphenyl]-3-yl)-4-(methylamino)-4,5,6,7-tetrahydropyrazolo[1,5-a]pyridine-2-carboxamide |
| 350 | 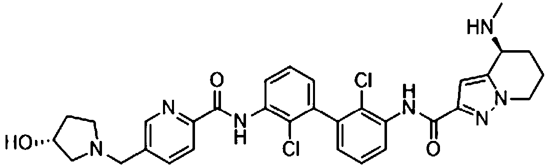 | (S)-N-(2,2'-dichloro-3'-(5-(((R)-3-hydroxypyrrolidin-1-yl)methyl)picolinamido)-[1,1'-biphenyl]-3-yl)-4-(methylamino)-4,5,6,7-tetrahydropyrazolo[1,5-a]pyridine-2-carboxamide |
| 351 | 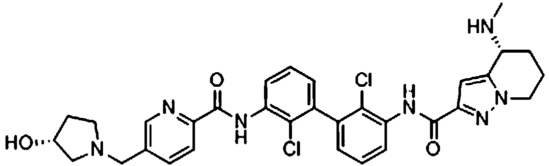 | (R)-N-(2,2'-dichloro-3'-(5-(((R)-3-hydroxypyrrolidin-1-yl)methyl)picolinamido)-[1,1'-biphenyl]-3-yl)-4-(methylamino)-4,5,6,7-tetrahydropyrazolo[1,5-a]pyridine-2-carboxamide |
| 352 | 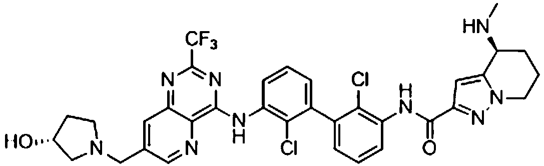 | (S)-N-(2,2'-dichloro-3'-((7-(((R)-3-hydroxypyrrolidin-1 - yl)methyl)-2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide |
| 353 | 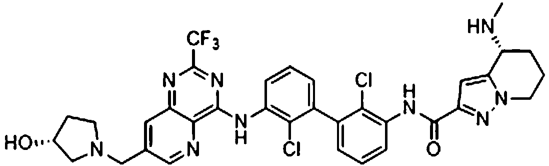 | (R)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide |
| 354 | 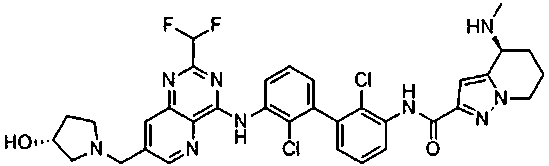 | (S)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide |
| 355 | 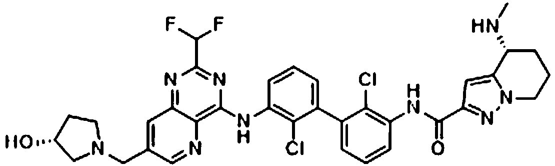 | (R)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide |
| 356 | 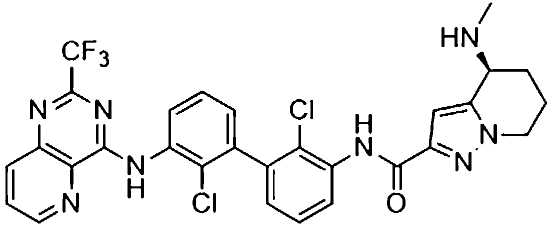 | (S)-N-(2,2'-dichloro-3'-((2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide |
| 357 | 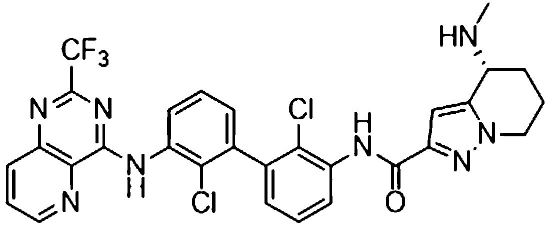 | (R)-N-(2,2'-dichloro-3'-((2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide |
| 358 | 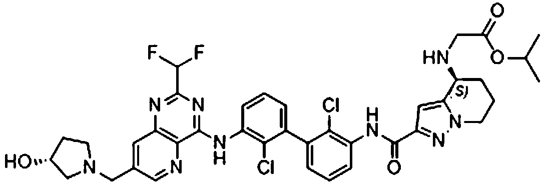 | isopropyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate |
| 359 | 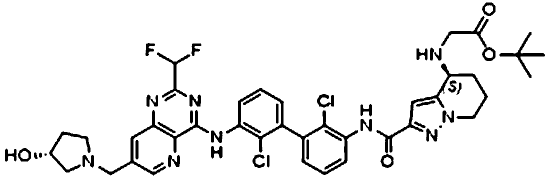 | tert-butyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate |
| 360 | 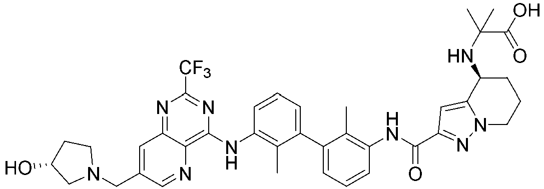 | 2-(((S)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid |
| 361 | 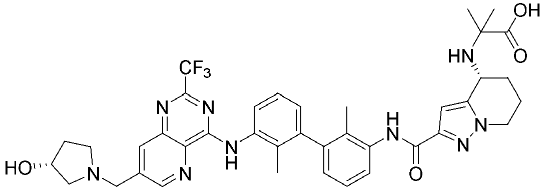 | 2-(((R)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid |
| 362 | 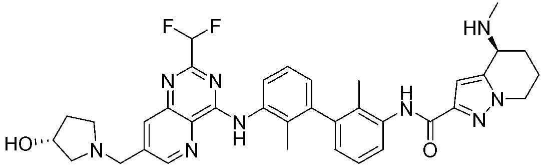 | (S)-N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide |
| 363 | 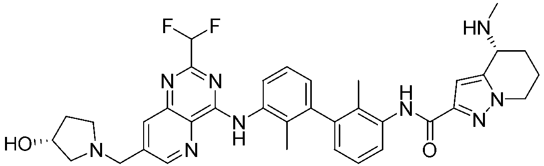 | (R)-N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide |
| 364 | 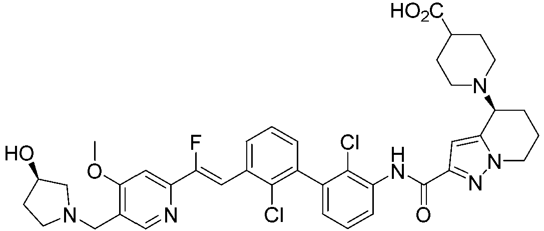 | 1-((S)-2-((2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)carbamoyl)-4,5,6,7-tetrahydropyrazolo[1,5-a]pyridin-4-yl)piperidine-4-carboxylic acid |
| 365 | 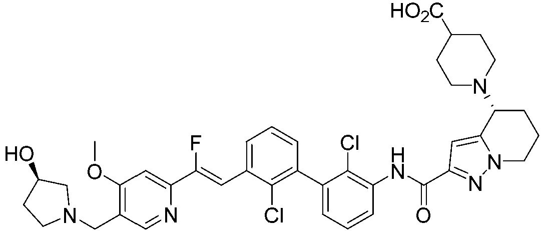 | 1-((R)-2-((2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)carbamoyl)-4,5,6,7-tetrahydropyrazolo[1,5-a]pyridin-4-yl)piperidine-4-carboxylic acid |
| 366 | 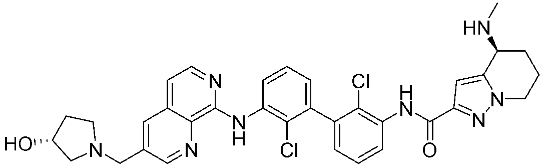 | (S)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(methylamino)-4,5,6,7-tetrahydropyrazolo[1,5-a]pyridine-2-carboxamide |
| 367 | 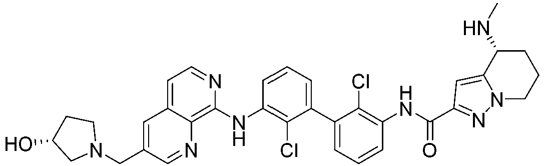 | (R)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(methylamino)-4,5,6,7-tetrahydropyrazolo[1,5-a]pyridine-2-carboxamide |
| 368 | 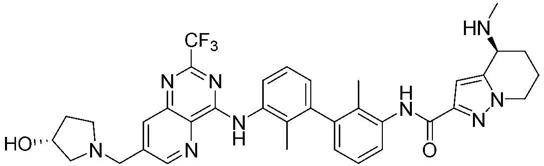 | (S)-N-(3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide |
| 369 | 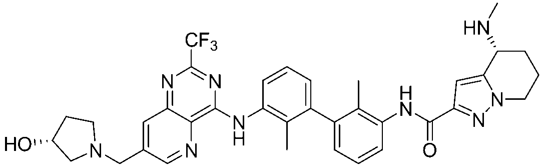 | (R)-N-(3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide |
| 370 | 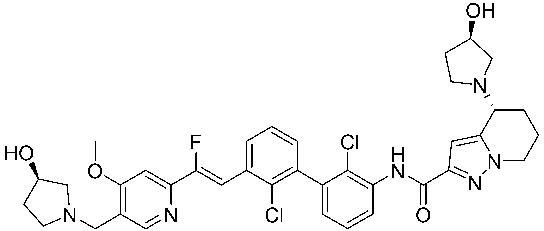 | (R)-N-(2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)-4-((R)-3-hydroxypyrrolidin-1-yl)-4,5,6,7-tetrahydropyrazolo[1,5-a]pyridine-2-carboxamide |

### [Effects of the Invention]

Unlike conventional anticancer agents that directly attack cancer itself, immuno- anticancer agents stimulate the immune system to induce immune cells to selectively attact only cancer cells.

The novel compounds provided according to the present invention exhibit an inhibitory effect on the interaction between PD-1 and PD-L1, enhance T-cell responses, and consequently possess antitumor activity. Accordingly, the compounds of the present invention may be used as novel immuno-anticancer agents capable of minimizing the side effects observed with conventional chemo-anticancer agents.

### [Brief Description of the Drawings]

FIG. 1 is a graph showing the tumor growth inhibitory effect in a group administered with Compound 218.
FIG. 2 is a graph showing changes in body weight in a group administered with Compound 218.

### [Best Mode for Carrying Out the Invention]

Hereinafter, embodiments and examples of the present invention will be described in detail with reference to the accompanying drawings so that a person having ordinary skill in the art to which the present invention pertains can readily carry out the invention. However, the present invention may be embodied in various forms and is not limited to the embodiments and examples described herein.

Throughout the present specification, when any part is described as "comprising" a constituent, it does not exclude other constituents unless otherwise stated, but rather means that it may further include other constituents.

As used herein, the term "alkyl" refers to a hydrocarbon having unsubstituted or substituted primary, secondary, tertiary, and/or quaternary carbon atoms, and includes saturated aliphatic groups that may be straight-chain, branched, cyclic, or combinations thereof. For example, an alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Unless otherwise defined, in preferred embodiments, the term "alkyl" refers to C₁-C₆ alkyl. Examples of suitable alkyl groups include, but are not limited to, methyl (Me, -CH₃), ethyl (Et, -CH₂CH₃), 1-propyl (n-Pr, n-propyl, -CH₂CH₂CH₃), 2-propyl (i-Pr, isopropyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, - CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃).

As used herein, the term "Cx-y" or "Cx-Cy," when used in connection with a chemical moiety such as acyl, acyloxy, alkyl, haloalkyl, cycloalkyl, alkenyl, alkynyl, or alkoxy, refers to a group containing from x to y carbon atoms in the chain. A C₀ alkyl denotes hydrogen when the substituent is at a terminal position, and referes to a bond when the substituent is at an internal position. For example, a C₁-C₆ alkyl group contains from 1 to 6 carbon atoms in the chain.

As used herein, the term "alkenyl" refers to a hydrocarbon having primary, secondary, tertiary, and/or quaternary carbon atoms, comprising straight-chain, branched, and cyclic structures, or combinations thereof, and having one or more unsaturated moieties, that is, carbon-carbon sp2 double bonds. For example, an alkenyl group may have 2 to 20 carbon atoms (i.e., C₂-C₂₀ alkenyl), 2 to 12 carbon atoms (i.e., C₂-C₁₂ alkenyl), 2 to 10 carbon atoms (i.e., C₂-C₁₀ alkenyl), or 2 to 6 carbon atoms (i.e., C₂-C₆ alkenyl). Examples of suitable alkenyl groups include, but are not limited to, vinyl (-CH=CH₂), allyl (-CH₂CH=CH₂), cyclopentenyl (-C₅H₇), and 5-hexenyl (-CH₂CH₂CH₂CH₂CH=CH₂).

As used herein, the term "alkynyl" refers to a hydrocarbon having primary, secondary, tertiary, and/or quaternary carbon atoms, comprising straight-chain, branched, and cyclic groups, or combinations thereof, and having one or more carbon-carbon sp triple bonds. For example, an alkynyl group may have 2 to 20 carbon atoms (i.e., C₂-C₂₀ alkynyl), 2 to 12 carbon atoms (i.e., C₂-C₁₂ alkynyl), 2 to 10 carbon atoms (i.e., C₂-C₁₀ alkynyl), or 2 to 6 carbon atoms (i.e., C₂-C₆ alkynyl). Examples of suitable alkynyl groups include, but are not limited to, acetylenic (-C≡CH) and propargyl (-CH₂C≡CH).

As used herein, the term "alkoxy" refers to an alkyl group attached to a parent compound through an oxygen atom and may be represented as -O-alkyl, wherein the alkyl group is as defined herein and may be unsubstituted or substituted. The alkyl group of the alkoxy group may have, for example, 1 to 20 carbon atoms (i.e., C₁-C₂₀ alkoxy), 1 to 12 carbon atoms (i.e., C₁-C₁₂ alkoxy), 1 to 10 carbon atoms (i.e., C₁-C₁₀ alkoxy), or 1 to 6 carbon atoms (i.e., C₁-C₆ alkoxy). Examples of suitable alkoxy groups include, but are not limited to, methoxy (-O-CH₃ or -OMe), ethoxy (-OCH₂CH₃ or -OEt), and t-butoxy (-OC(CH₃)₃ or -O-tBu).

As used herein, the term "alkoxyalkyl" refers to an alkyl group substituted with an alkoxy group as defined herein, and may be represented as -alkyl-O-alkyl.

As used herein, the symbol " 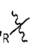 ", in accordance with conventions commonly used in the art, indicates that a residue or substituent "R" is attached to a scaffold structure.

As used herein, the term "cycloalkyl" refers to non-aromatic saturated or unsaturated ring that is a unsubstituted or substituted monocyclic, bicyclic, or polycyclic, in which each atom of the ring is carbon. A cycloalkyl may be a polycyclic cycloalkyl consisted of two or more rings in which one or more carbon atoms are common to adjacent rings. The polycyclic cycloalkyl may be a fused ring system, a spirocyclic ring system, or a bridged ring system, and one or more of the rings may be cycloalkyl, while the other ring(s) may be, for example, cycloalkyl, aryl, heteroaryl, and/or heterocycloalkyl as defined herein. Examples of suitable cycloalkyl groups include, but are not limited to, cyclopropyl, cyclobutyl, cyclopentyl, cyclohexyl, cycloheptyl, and cyclooctyl.

As used herein, the term "heterocycloalkyl" refers to a unsubstituted or substituted monovalent or divalent, saturated or partially saturated non-aromatic ring that is monocyclic, bicyclic, or polycyclic and contains one or more heteroatoms in the ring, preferably 1 to 4 heteroatoms, more preferably 1 to 2 heteroatoms. Further, when the "heterocycloalkyl" is a bicyclic or polycyclic ring system having two or more cyclic rings in which two or more carbon atoms are common to two adjacent rings, one or more of the rings may be heterocyclic, and the other cyclic ring(s) may be, for example, cycloalkyl, cycloalkenyl, cycloalkynyl, aryl, heteroaryl, and/or heterocycloalkyl. The bicyclic or polycyclic ring system may be a fused, bridged, or spiro ring system. Examples of "heterocycloalkyl" include, for example, piperidine, piperazine, pyrrolidine, morpholine, lactone, lactam, and the like (each of which may be unsubstituted or substituted).

As used herein, the term "aryl" refers to a unsubstituted or substituted monovalent or divalent aromatic hydrocarbon group that is monocyclic, bicyclic, or polycyclic, in which each atom of the ring is carbon. Preferably, the aryl ring is a 6- to 20-membered ring, a 6- to 14-membered ring, a 6- to 10-membered ring, or more preferably a 6-membered ring. An aryl group may be a polycyclic ring system having two or more cyclic rings in which two or more carbon atoms are common to two adjacent rings, wherein one or more of the rings are aromatic, and for example, the other cyclic ring(s) may be cycloalkyl, cycloalkenyl, cycloalkynyl, aryl, heteroaryl, and/or heterocycloalkyl. Examples of aryl groups include benzene, naphthalene, phenanthrene, anthracene, indene, indane, phenol, and aniline.

As used herein, the term "heteroaryl" refers to a unsubstituted or substituted monovalent or divalent aromatic group that is monocyclic, bicyclic, or polycyclic and contains one or more heteroatoms in the ring. Non-limiting examples of suitable heteroatoms that may be contained in the aromatic ring include oxygen, sulfur, and nitrogen. When the "heteroaryl" is a bicyclic or polycyclic ring system having two or more cyclic rings in which two or more carbon atoms are common to two adjacent rings, one or more of the rings may be heteroaromatic, and the other cyclic ring(s) may be, for example, cycloalkyl, cycloalkenyl, cycloalkynyl, aryl, heteroaryl, and/or heterocycloalkyl. Examples of "heteroaryl" include, for example, benzofuran, benzothiophene, pyrrole, furan, thiophene, imidazole, indole, isoindole, isoxazole, isothiazole, oxazole, thiazole, quinoline, isoquinoline, pyrazole, pyridine, pyrazine, pyridazine, and pyrimidine (each of which may be unsubstituted or substituted).

As used herein, the term "amino" refers to -NH₂ in which one or more hydrogen atoms may be unsubstituted or substituted with substituents such as alkyl, aryl, and the like, wherein the substituents substituting the hydrogen atoms are as defined herein and may be unsubstituted or substituted. Examples of suitable amino groups include, but are not limited to, -NH₂, -N(CH₃)₂, and -N(CH₃)-CH₂CH₂-N(CH₃)₂.

As used herein, the terms "halo" and "halogen" both refer to halogens and include chloro, fluoro, bromo, and iodo.

As used herein, the term "cyano" refers to a -CN group.

As used herein, the term "carboxyl" refers to a -C(O)OH group.

As used herein, the term "haloalkyl" refers to an alkyl group as defined above in which one or more hydrogen atoms of the alkyl group are substituted with halogen atoms. The alkyl residue of the haloalkyl group may have 1 to 20 carbon atoms (i.e., C₁-C₂₀ haloalkyl), 1 to 12 carbon atoms (i.e., C₁-C₁₂ haloalkyl), 1 to 10 carbon atoms (i.e., C₁-C₁₀ haloalkyl), or 1 to 6 carbon atoms (i.e., C₁-C₆ haloalkyl). Examples of suitable haloalkyl groups include, but are not limited to, -CF₃, -CHF₂, -CFH₂, and -CH₂CF₃, and the like.

As used herein, the term "cycloalkenyl" refers to a non-aromatic monocyclic or polycyclic hydrocarbon containing at least one carbon-carbon double bond. In some embodiments, the cycloalkenyl contains 3 to 20 carbon atoms, 3 to 10 carbon atoms, or 3 to 7 carbon atoms. Cycloalkenyl includes monocyclic and polycyclic groups (including fused, bridged, and spirocyclic rings). Examples of suitable cycloalkenyl groups include, but are not limited to, cyclopentenyl (-C₅H₇).

As used herein, the term "substituted," for example "substituted alkyl," means that one or more hydrogen atoms of the alkyl are each independently replaced with a non-hydrogen substituent. The substituent may include, but is not limited to, any substituent described herein, for example halogen, hydroxyl, alkyl, hydroxyalkyl, haloalkyl, cyanoalkyl, alkoxyalkyl, carbonyl (e.g., carboxyl, alkoxycarbonyl, formyl, or acyl), thiocarbonyl (e.g., thioester, thioacetate, or thioformate), alkoxyl, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, aryl, or heteroaryl. A substituted residue on a hydrocarbon chain may itself be further substituted in some cases.

As used herein, the term "pharmaceutically acceptable salt" means any acid addition salt or base addition salt that is non-toxic and harmless to a patient and for which side effects attributable to the salt do not diminish the beneficial efficacy of the compound of the present invention.

Hereinafter, the present invention will be described in more detail through examples; however, the following examples are provided only for illustrative purposes and are not intended to limit the scope of the present invention.

### [Preparation Example 1]

### Preparation of Compound 1, N,N'-(2,2'-dimethyl-[1,1'-biphenyl]-3,3'-diyl)bis(4-oxo-4,5,6,7-tetrahydropyrazolo[1,5-a]pyridine-2-carboxamide)

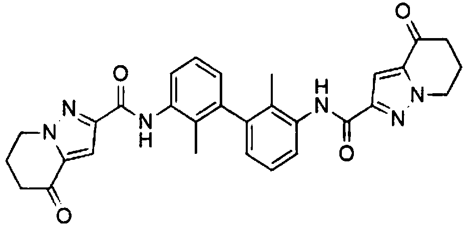

Compound 1 of the present invention was prepared according to the following method.

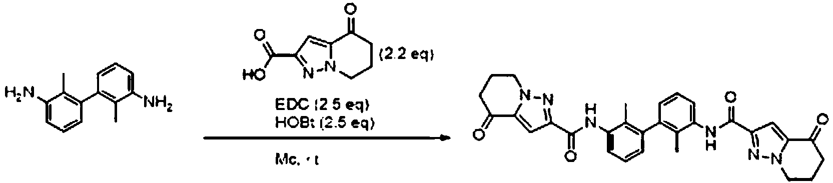

2,2'-Dimethyl-[1,1'-biphenyl]-3,3'-diamine (5.0 g, 4.71 mmol) was dissolved in DCM (50 mL), and 4-oxo-4,5,6,7-tetrahydropyrazolo[1,5-a]pyridine-2-carboxylic acid (1.7 g, 9.44 mmol), EDC (2.3 g, 11.78 mmol), and HOBt (1.6 g, 11.78 mmol) were added thereto, followed by stirring at room temperature for 12 hours. After completion of the reaction, the solvent was removed by evaporation under reduced pressure, and H₂O and ether were added to the resulting solid, followed by stirring for 30 minutes. The resulting solid was collected by filtration and dried in a vacuum oven to afford Compound 1 (2.1 g, 82%).

¹H NMR (500 MHz, DMSO) d 9.90 (s, 1H), 7.46 (d, J = 7.8 Hz, 1H), 7.29 (t, J = 7.8 Hz, 1H), 7.26 (s, 1H), 7.01 (d, J = 7.5 Hz, 1H), 4.50 (t, J = 12.0, 2H), 2.82 (t, 6.1 Hz, 2H), 2.34 (dt, J = 12.0, 6.1 Hz, 2H), 1.93 (s, 3H).

### [Preparation Example 2]

### Preparation of Compound 2, N-(2,2'-dimethyl-3'-(4-oxo-4,5,6,7-tetrahydropyrazolo[1,5-a]pyridine-2-carboxamido)-[1,1'-biphenyl]-3-yl)-4-hydroxy-4,5,6,7-tetrahydropyrazolo[1,5-a]pyridine-2-carboxamide

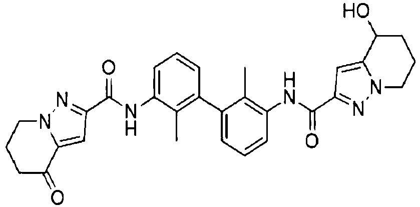

Compound 2 of the present invention was prepared according to the following method.

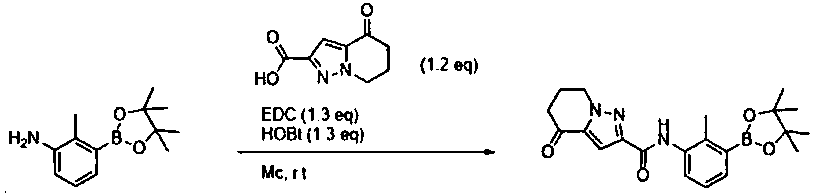

2-Methyl-3-(4,4,5,5-tetramethyl-1,3,2-dioxaborolan-2-yl)aniline (3.6 g, 15.44 mmol) was dissolved in DCM (60 mL), and 4-oxo-4,5,6,7-tetrahydropyrazolo[1,5-a]pyridine-2-carboxylic acid (3.34 g, 18.53 mmol), EDC (3.85 g, 20.07 mmol), and HOBt (2.7 g, 20.07 mmol) were added thereto, followed by stirring at room temperature for 12 hours. After completion of the reaction, the reaction mixture was cooled to room temperature, and evaporated under reduced pressure to remove the solvent. The resulting christalline was extracted with H₂O and ethyl acetate (EA). The organic layer was dried over anhydrous MgSO₄, and the solvent was removed by evaporation under reduced pressure. The residue was purified by column chromatography (n-hexane:EtOAc = 2:1) to obtain 4.76 g (78%) of an intermediate (N-(2-methyl-3-(4,4,5,5-tetramethyl-1,3,2-dioxaborolan-2-yl)phenyl)-4-oxo-4,5,6,7-tetrahydropyrazolo[1,5-a]pyridine-2-carboxamide).

¹H NMR (500 MHz, CDCl3) d 9.75 (s, 1H), 7.40 (d, J = 7.8 Hz, 1H), 7.35 (t, J = 7.8 Hz, 1H), 7.23 (s, 1H), 6.89 (d, J = 7.5 Hz, 1H), 4.44 (t, J = 12.0, 2H), 2.81 (t, 6.1 Hz, 2H), 2.36-2.33 (m, 2H), 1.95 (s, 3H), 1.25 (s, 12H)

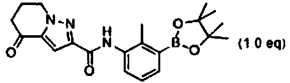

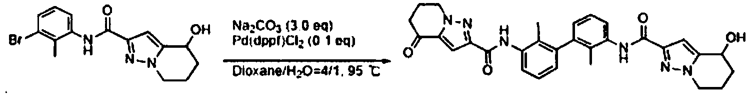

N-(3-bromo-2-methylphenyl)-4-hydroxy-4,5,6,7-tetrahydropyrazolo[1,5-a]pyridine-2-carboxamide (500 mg, 0.29 mmol) was dissolved in dioxane (8 mL) and H₂O (2 mL), and N-(3-(1,5-dimethyl-2,4-dioxa-3-borabicyclo[3.1.0]hexan-3-yl)-2-methylphenyl)-4-oxo-4,5,6,7-tetrahydropyrazolo[1,5-a]pyridine-2-carboxamide (390 mg, 0.29 mmol), Na₂CO₃ (460 mg, 106 mmol), and Pd(PPh₃)₄ (168 mg, 0.029 mmol) were added thereto, followed by heating under reflux for 6 hours. After completion of the reaction, the reaction mixture was cooled to room temperature and filtered under reduced pressure with EtOAc. The filtrate was concentrated by evaporation under reduced pressure to remove the solvent. The resulting christalline was extracted with H₂O and EA, and the organic layer was dried over anhydrous MgSO₄ and concentrated under reduced pressure. The residue was purified by column chromatography (n-hexane:EtOAc = 1:1) to afford Compound 2 (128 mg, 82%).

¹H NMR (500 MHz, CDCl3) d 8.70 (s, 2H), 8.12-8.08 (m, 2H), 7.46 (s, 1H), 7.29-7.25 (m, 2H), 7.04-6.92 (m, 2H), 6.89 (s, 1H), 4.98 (d, J = 3.2 Hz, 1H), 4.47 (t, J = 3.2 Hz, 2H), 4.26-4.04 (m, 2H), 2.77-2.72 (m, 2H), 2.47-2.41 (m, 2H), 2.40-2.30 (m, 1H), 2.26-2.20 (m, 1H), 2.15-2.05 (m, 1H), 2.08-2.05 (m, 6H), 205-1.91 (m, 2H).

### [Preparation Example 3]

### Preparation of Compound 2-1, N,N'-(2,2'-dimethyl-[1,1'-biphenyl]-3,3'-diyl)bis(4-hydroxy-4,5,6,7-tetrahydropyrazolo[1,5-a]pyridine-2-carboxamide)

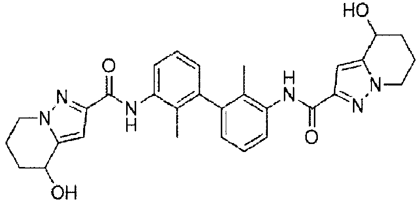

Compound 2-1 of the present invention was prepared according to the following method.

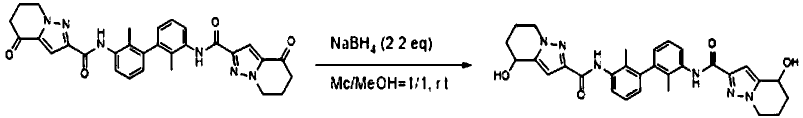

N,N'-(2,2'-dimethyl-[1,1'-biphenyl]-3,3'-diyl)bis(4-oxo-4,5,6,7-tetrahydropyrazolo[1,5-a]pyridine-2-carboxamide) (600 mg, 1.12 mmol) was dissolved in a mixed solvent (DCM 8 mL, MeOH 8 mL), and NaBH₄ (93 mg, 2.46 mmol) was added thereto, followed by stirring at room temperature for 4 hours. After completion of the reaction, the solvent was removed by evaporation under reduced pressure, and H₂O was added to the resulting christalline, followed by stirring for 30 minutes. The resulting christalline was collected by filtration and dried in a vacuum oven to afford Compound 2-1 (538.8 mg, 89%).

¹H NMR (500 MHz, CDCl3) d 8.70 (d, J = 3.7 Hz, 2H), 8.13 (dd, J = 13.9, 6.9 Hz, 2H), 7.29 (t, J = 7.5 Hz, 2H), 6.96 (d, J = 7.5 Hz, 2H), 6.88 (d, J = 6.9 Hz, 2H), 4.98 (s, 2H), 4.23-4.02 (m, 4H), 2.48 (s, 2H), 2.33-2.35 (m, 2H), 2.15-2.07 (m, 2H), 2.05 (d, J = 2.1 Hz, 6H), 1.99 (d, J = 4.3 Hz, 4H)

### [Preparation Example 4]

### Preparation of Compound 3, N-(2,2'-dimethyl-3'-(4-oxo-4,5,6,7-tetrahydropyrazolo[1,5-a]pyridine-2-carboxamido)-[1,1'-biphenyl]-3-yl)-4-((2-hydroxyethyl)amino)-4,5,6,7-tetrahydropyrazolo[1,5-a]pyridine-2-carboxamide

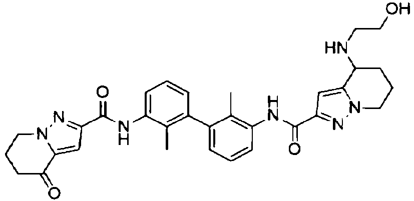

Compound 3 of the present invention was prepared according to the following method.

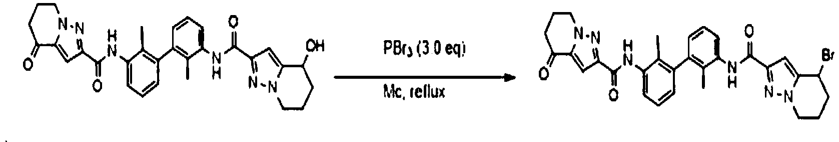

N,N'-(2,2'-dimethyl-[1,1'-biphenyl]-3,3'-diyl)bis(4-hydroxy-4,5,6,7-tetrahydropyrazolo[1,5-a]pyridine-2-carboxamide) (300 mg, 0.56 mmol) was dissolved in DCM (25 mL), and PBr₃ (0.16 mL, 1.67 mmol) was added thereto, followed by heating under reflux for 3 hours. After completion of the reaction, the reaction mixture was cooled to room temperature, and the solvent was removed by evaporation under reduced pressure. H₂O was added to the resulting christalline, followed by stirring for 30 minutes. The resulting christalline was collected by filtration and dried in a vacuum oven to obtain an intermediate, N,N'-(2,2'-dimethyl-[1,1'-biphenyl]-3,3'-diyl)bis(4-bromo-4,5,6,7-tetrahydropyrazolo[1,5-a]pyridine-2-carboxamide) (303 mg, 90%).

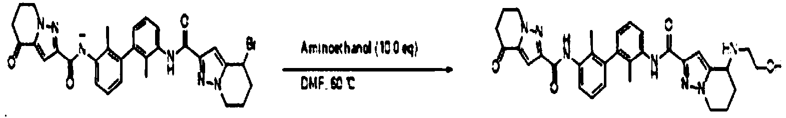

4-Bromo-N-(2,2'-dimethyl-3'-(4-oxo-4,5,6,7-tetrahydropyrazolo[1,5-a]pyridine-2-carboxamido)-[1,1'-biphenyl]-3-yl)-4,5,6,7-tetrahydropyrazolo[1,5-a]pyridine-2-carboxamide (150 mg, 0.25 mmol) was dissolved in DMF (10 mL), and aminoethanol (0.15 mL, 2.5 mmol) was added thereto, followed by stirring at 60 °C for 12 hours. After completion of the reaction, the reaction mixture was cooled to room temperature, and the solvent was removed by evaporation under reduced pressure. The residue was purified by column chromatography (EtOAc:MeOH = 5:1) to afford Compound 3 (80 mg, 32%)

¹H NMR (500 MHz, CDCl3) d 8.69 (s, 2H), 8.23-8.05 (m, 2H), 7.47 (s, 1H), 7.29-7.25 (m, 2H), 6.99-6.92 (m, 2H), 6.86 (s, 1H), 4.48-4.41 (m, 1H), 4.47 (t, J = 6.3 Hz, 2H), 4.17 (t, J = 6.3 Hz, 2H), 4.02-3.95 (m, 1H), 3.69-3.65 (m, 2H), 2.99-2.87 (m, 2H), 2.75 (t, J = 6.3 Hz, 2H), 2.35 (p, J = 5.4 Hz, 2H), 2.28-2.4 (m, 2H) 2.20-2.16 (m, 1H), 2.15-2.05 (m, 6H), 2.04-2.00 (m, 1H), 1.83-1.74 (m, 1H)

### [Preparation Example 5]

### Preparation of Compound 3-1, 4-hydroxy-N-(3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)-4,5,6,7-tetrahydropyrazolo[1,5-a]pyridine-2-carboxamide

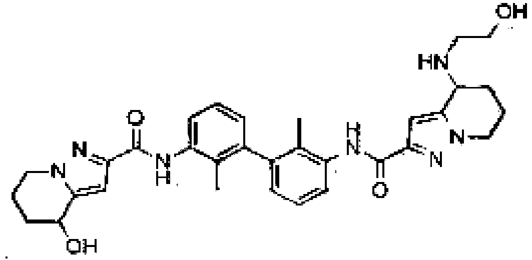

N-(2,2'-dimethyl-3'-(4-oxo-4,5,6,7-tetrahydropyrazolo[1,5-a]pyridine-2-carboxamido)-[1,1'-biphenyl]-3-yl)-4-((2-hydroxyethyl)amino)-4,5,6,7-tetrahydropyrazolo[1,5-a]pyridine-2-carboxamide (11 mg, 0.019 mmol) was dissolved in DCM (1 mL) and MeOH (1 mL), and NaBH₄ (1.1 mg, 0.029 mmol) was added thereto, followed by stirring at room temperature for 3 hours. After completion of the reaction, the solvent was removed by evaporation under reduced pressure, and H₂O was added to the resulting christalline, followed by stirring for 30 minutes. The resulting christalline was collected by filtration and dried in a vacuum oven to afford Compound 3-1 (3.44 mg, 31%).

¹H NMR (500 MHz, CDCl3) d 8.70 (s, 2H), 8.24-8.04 (m, 2H), 7.45 (s, 1H), 7.29-7.23 (m, 2H), 6.99-6.91 (m, 2H), 6.85 (s, 1H), 5.31 (s, 1H), 4.49-4.35 (m, 3H), 4.46 (t, J = 6.3 Hz, 2H), 4.17 (t, J = 6.3 Hz, 2H), 4.02-3.95 (m, 1H), 3.69-3.65 (m, 2H), 2.99-2.87 (m, 2H), 2.75 (t, J = 6.3 Hz, 2H), 2.35 (p, J = 5.4 Hz, 2H), 2.28-2.4 (m, 2H) 2.20-2.16 (m, 1H), 2.15-2.05 (m, 6H), 2.04-2.00 (m, 1H), 1.83-1.74 (m, 1H)

### [Preparation Example 6]

### Preparation of Compound 4, N,N'-(2,2'-dimethyl-[1,1'-biphenyl]-3,3'-diyl)bis(4-(methylamino)-4,5,6,7-tetrahydropyrazolo[1,5-a]pyridine-2-carboxamide)

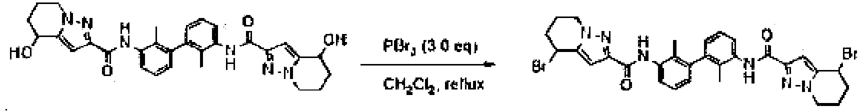

N,N'-(2,2'-dimethyl-[1,1'-biphenyl]-3,3'-diyl)bis(4-hydroxy-4,5,6,7-tetrahydropyrazolo[1,5-a]pyridine-2-carboxamide) (1.5 g, 2.78 mmol) was dissolved in DCM (25 mL), and PBr₃ (0.8 mL, 8.32 mmol) was added thereto, followed by heating under reflux for 3 hours. After completion of the reaction, the reaction mixture was cooled to room temperature, and the solvent was removed by evaporation under reduced pressure. H₂O was added to the resulting solid, followed by stirring for 30 minutes. The resulting solid was collected by filtration and dried in a vacuum oven to obtain the product (1.57 mg, 85%).

¹H NMR (500 MHz, CDCl3) d 8.70 (d, J = 3.7 Hz, 2H), 8.13 (dd, J = 13.9, 6.9 Hz, 2H), 7.29 (t, J = 7.5 Hz, 2H), 6.96 (d, J = 7.5 Hz, 2H), 6.88 (d, J = 6.9 Hz, 2H), 4.98 (s, 2H), 4.23-4.02 (m, 4H), 2.48 (s, 2H), 2.33-2.35 (m, 2H), 2.15-2.07 (m, 2H), 2.05 (d, J = 2.1 Hz, 6H), 1.99 (d, J = 4.3 Hz, 4H)

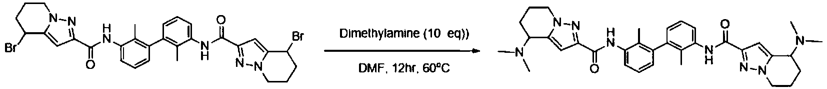

N,N'-(2,2'-dimethyl-[1,1'-biphenyl]-3,3'-diyl)bis(4-bromo-4,5,6,7-tetrahydropyrazolo[1,5-a]pyridine-2-carboxamide) (1.0 eq) was dissolved in DMF, and dimethylamine (10 eq) was added thereto, followed by stirring at 60 °C for 12 hours. After completion of the reaction, the reaction mixture was cooled to room temperature, and the solvent was removed by evaporation under reduced pressure. The residue was purified by column chromatography (EtOAc:MeOH = 5:1) to afford Compound 4.

¹H NMR (500 MHz, DMSO) d 9.76 (s, 2H), 9.05 (s, 2H), 7.49 (d, J = 6.9 Hz, 2H), 7.29 (t, J = 7.5 Hz, 2H), 7.05 (s, 2H), 6.99 (d, J = 7.3 Hz, 2H), 4.60-4.58 (m, 2H), 4.22-4.20 (m, 4H), 3.38-3.36 (m, 4H) 2.66 (s, 6H), 2.37 (s, 2H), 2.25-2.23 (s, 4H), 2.02-2.00 (s, 4H)

### [Preparation Example 7]

### Preparation of Compound 5, N,N'-(2,2'-dimethyl-[1,1'-biphenyl]-3,3'-diyl)bis(4-((2-acetamidoethyl)amino)-4,5,6,7-tetrahydropyrazolo[1,5-a]pyridine-2-carboxamide)

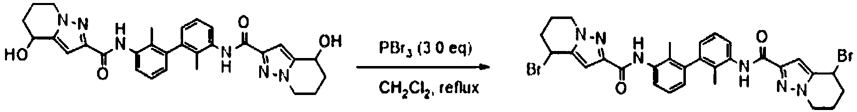

N,N'-(2,2'-dimethyl-[1,1'-biphenyl]-3,3'-diyl)bis(4-hydroxy-4,5,6,7-tetrahydropyrazolo[1,5-a]pyridine-2-carboxamide) (1.5 g, 2.78 mmol) was dissolved in DCM (25 mL), and PBr₃ (0.8 mL, 8.32 mmol) was added thereto, followed by heating under reflux for 3 hours. After completion of the reaction, the reaction mixture was cooled to room temperature, and the solvent was removed by evaporation under reduced pressure. H₂O was added to the resulting christalline, followed by stirring for 30 minutes. The resulting christalline was collected by filtration and dried in a vacuum oven to obtain the product (1.57 mg, 85%).

¹H NMR (500 MHz, CDCl3) d 8.70 (d, J = 3.7 Hz, 2H), 8.13 (dd, J = 13.9, 6.9 Hz, 2H), 7.29 (t, J = 7.5 Hz, 2H), 6.96 (d, J = 7.5 Hz, 2H), 6.88 (d, J = 6.9 Hz, 2H), 4.98 (s, 2H), 4.23-4.02 (m, 4H), 2.48 (s, 2H), 2.33-2.35 (m, 2H), 2.15-2.07 (m, 2H), 2.05 (d, J = 2.1 Hz, 6H), 1.99 (d, J = 4.3 Hz, 4H)

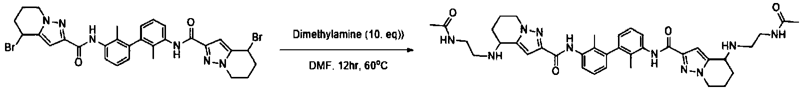

N,N'-(2,2'-dimethyl-[1,1'-biphenyl]-3,3'-diyl)bis(4-bromo-4,5,6,7-tetrahydropyrazolo[1,5-a]pyridine-2-carboxamide) (1.0 eq) was dissolved in DMF, and N-(2-aminoethyl)acetamide (10 eq) was added thereto, followed by stirring at 60 °C for 12 hours. After completion of the reaction, the reaction mixture was cooled to room temperature, and the solvent was removed by evaporation under reduced pressure. The residue was purified by column chromatography (EtOAc:MeOH = 5:1) to afford Compound 5.

¹H NMR (500 MHz, CDCl3) δ 8.70 (s, 2H), 8.12 (d, J = 7.9 Hz, 2H), 7.28 (t, J = 7.5 Hz, 2H), 6.96 (d, J = 7.5 Hz, 2H), 6.83 (s, 2H), 5.97 (s, 2H), 4.25-4.10 (m, 2H), 3.97-3.95 (m, 2H), 3.37 (q, J = 6.8 Hz, 2H), 2.89-2.87 (m, 2H), 2.28-2.26 (m, 2H), 2.18-2.16 (m, 2H), 2.06 (s, 3H), 2.02 (s, 3H), 1.99 (d, J = 4.6 Hz, 2H), 1.74-1.72 (m, 2H), 1.64-1.62 (m, 2H).

### [Preparation Example 8]

### Preparation of Compound 6, N,N'-(2,2'-dimethyl-[1,1'-biphenyl]-3,3'-diyl)bis(4-((2-hydroxyethyl)amino)-4,5,6,7-tetrahydropyrazolo[1,5-a]pyridine-2-carboxamide)

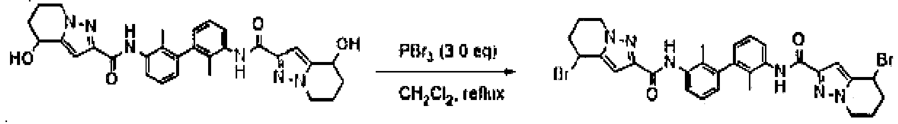

N,N'-(2,2'-dimethyl-[1,1'-biphenyl]-3,3'-diyl)bis(4-hydroxy-4,5,6,7-tetrahydropyrazolo[1,5-a]pyridine-2-carboxamide) (1.5 g, 2.78 mmol) was dissolved in DCM (25 mL), and PBr₃ (0.8 mL, 8.32 mmol) was added thereto, followed by heating under reflux for 3 hours. After completion of the reaction, the reaction mixture was cooled to room temperature, and the solvent was removed by evaporation under reduced pressure. H₂O was added to the resulting christalline, followed by stirring for 30 minutes. The resulting christalline was collected by filtration and dried in a vacuum oven to obtain the product (1.57 mg, 85%).

¹H NMR (500 MHz, CDCl3) d 8.70 (d, J = 3.7 Hz, 2H), 8.13 (dd, J = 13.9, 6.9 Hz, 2H), 7.29 (t, J = 7.5 Hz, 2H), 6.96 (d, J = 7.5 Hz, 2H), 6.88 (d, J = 6.9 Hz, 2H), 4.98 (s, 2H), 4.23-4.02 (m, 4H), 2.48 (s, 2H), 2.33-2.35 (m, 2H), 2.15-2.07 (m, 2H), 2.05 (d, J = 2.1 Hz, 6H), 1.99 (d, J = 4.3 Hz, 4H)

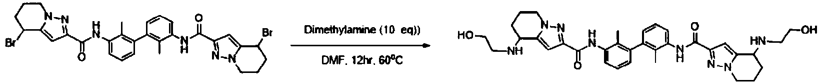

N,N'-(2,2'-dimethyl-[1,1'-biphenyl]-3,3'-diyl)bis(4-bromo-4,5,6,7-tetrahydropyrazolo[1,5-a]pyridine-2-carboxamide) (1.0 eq) was dissolved in DMF, and 2-aminoethanol (10 eq) was added thereto, followed by stirring at 60 °C for 12 hours. After completion of the reaction, the reaction mixture was cooled to room temperature, and the solvent was removed by evaporation under reduced pressure. The residue was purified by column chromatography (EtOAc:MeOH = 5:1) to afford Compound 6.

¹H NMR (500 MHz, CDCl3) δ 8.71 (d, J = 8.1 Hz, 2H), 7.28-7.26 (m, 2H), 8.11 (d, J = 8.1 Hz, 2H), 6.96 (d, J = 7.5 Hz, 2H), 6.85 (s, 2H), 4.16-4.14 (m, 4H), 3.97 (t, J = 6.0 Hz, 2H), 3.69 (t, J = 5.0 Hz, 4H), 2.92-2.90 (m, 4H), 2.42-2.20 (m, 6H), 2.20-2.08 (m, 2H), 2.02 (s, 6H), 2.08-1.92 (m, 2H), 1.88-1.70 (m, 2H)

### [Preparation Example 9]

### Preparation of Compound 8, 2-[[2-[[3-[3-[[4-(carboxymethylamino)-4,5,6,7-tetrahydropyrazolo[1,5-a]pyridine-2-carbonyl]amino]-2-chlorophenyl]-2-chlorophenyl]carbamoyl]-4,5,6,7-tetrahydropyrazolo[1,5-a]pyridin-4-yl]amino]acetic acid

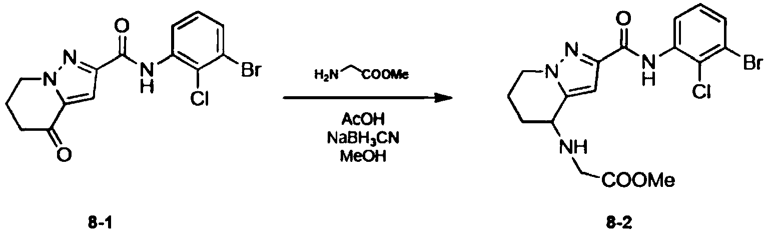

N-(3-bromo-2-chloro-phenyl)-4-oxo-6,7-dihydro-5H-pyrazolo[1,5-a]pyridine-2-carboxamide (1.0 g, 2.71 mmol, 1 eq) and methyl 2-aminoacetate (1.21 g, 9.63 mmol, 3.55 eq) were dissolved in MeOH (15 mL), and NaBH₃CN (1.70 g, 27.13 mmol, 10 eq) and AcOH (1.63 g, 27.13 mmol, 1.55 mL, 10 eq) were added thereto. The reaction mixture was stirred at 60 °C for 12 hours. After concentration, the residue was purified by column chromatography (SiO₂, Petroleum ether/Ethyl acetate = 1:1 to 0:1) to afford Compound 8-2 (800 mg, 50.1% yield) as a white solid.

LCMS m/z 443.0 [M+3]⁺.

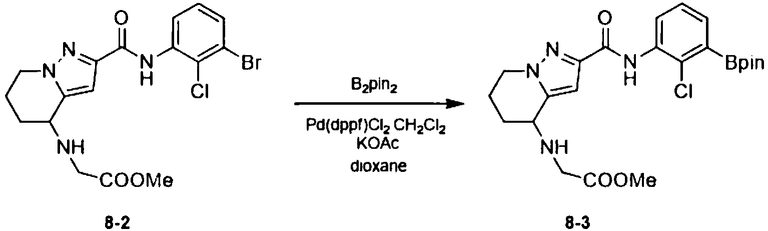

Methyl 2-[[2-[(3-bromo-2-chlorophenyl)carbamoyl]-4,5,6,7-tetrahydropyrazolo[1,5-a]pyridin-4-yl]amino]acetate (200 mg, 452.79 µmol, 1 eq), 4,4,5,5-tetramethyl-2-(4,4,5,5-tetramethyl-1,3,2-dioxaborolan-2-yl)-1,3,2-dioxaborolane (344.94 mg, 1.36 mmol, 3 eq), KOAc (133.31 mg, 1.36 mmol, 3 eq), and Pd(dppf)Cl₂·CH₂Cl₂ (36.98 mg, 45.28 µmol, 0.1 eq) were mixed in dioxane (5 mL) and stirred at 90 °C for 5 hours under a nitrogen atmosphere. After concentration under reduced pressure, the residue was purified by prep-TLC (SiO₂, petroleum ether:ethyl acetate = 0:1) to afford Compound 8-3 (257 mg, 81.3% yield) as a white solid.

LCMS m/z 489.3 [M+1]⁺.

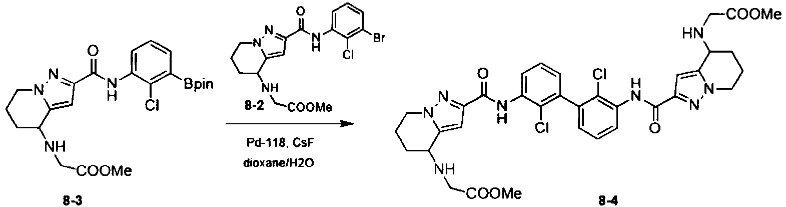

Methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (199.18 mg, 407.51 µmol, 1.2 eq), methyl 2-[[2-[(3-bromo-2-chlorophenyl)carbamoyl]-4,5,6,7-tetrahydropyrazolo[1,5-a]pyridin-4-yl]amino]acetate (150 mg, 339.59 µmol, 1 eq), di-tert-butyl(cyclopentyl)phosphane-dichloropalladium-iron (22.13 mg, 33.96 µmol, 0.1 eq), and CsF (154.76 mg, 1.02 mmol, 37.61 µL, 3 eq) were mixed in dioxane (5 mL) and H₂O (1 mL), and the reaction mixture was stirred at 90 °C for 5 hours under a nitrogen atmosphere. After concentration under reduced pressure, the residue was purified by prep-TLC (SiO₂, DCM:MeOH = 11:1) to afford Compound 8-4 (110 mg, 41.6% yield) as a white solid.

LCMS m/z 723.1 [M+1]⁺.

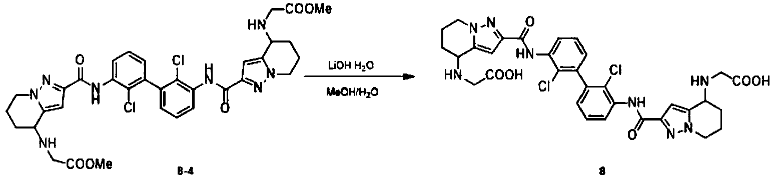

Methyl 2-[[2-[[2-chloro-3-[2-chloro-3-[[4-[(2-methoxy-2-oxoethyl)amino]-4,5,6,7-tetrahydropyrazolo[1,5-a]pyridine-2-carbonyl]amino]phenyl]phenyl]carbamoyl]-4,5,6,7-tetrahydropyrazolo[1,5-a]pyridin-4-yl]amino]acetate (110 mg, 152.02 µmol, 1 eq) was dissolved in H₂O (3 mL) and MeOH (3 mL), and LiOH·H₂O (31.90 mg, 760.08 µmol, 5 eq) was added thereto. The reaction mixture was stirred at room temperature for 12 hours. After concentration under reduced pressure, the residue was purified by reversed-phase HPLC (column: Waters XBridge Prep OBD C18, 150*40 mm*10 µm; mobile phase: [water (NH₄HCO₃)-ACN]; gradient: 8%-38% B over 15 min) to afford Compound 8 (57 mg, 53.37% yield) as a white solid.

LCMS m/z 695.4 [M+1]⁺.

¹H NMR (400 MHz, DMSO-d6) δ ppm 1.70 - 1.79 (m, 2 H) 1.91 (br d, J = 7.50 Hz, 2 H) 2.01 (br s, 2 H) 2.19 (br s, 2 H) 3.31 (br s, 4 H) 4.04 (br t, J = 5.88 Hz, 2 H) 4.16 (br t, J=6.00 Hz, 4 H) 6.82 (s, 2 H) 7.17 (d, J = 7.63 Hz, 2 H) 7.49 (t, J=7.94 Hz, 2 H) 8.27 (d, J = 8.26 Hz, 2 H) 9.56 (s, 2 H).

### [Preparation Example 10]

### Preparation of Compounds 11 to 17

### 10-1. Preparation of Compound 11, 4-hydroxy-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide

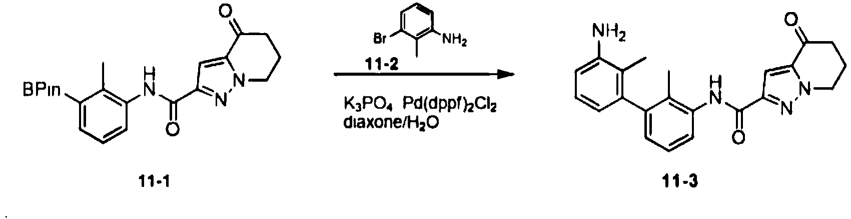

A mixture of Starting material 1 (500 mg, 1.26 mmol, 1 eq), 3-bromo-2-methylaniline (258.89 mg, 1.39 mmol, 171.45 µL, 1.1 eq), K₃PO₄ (805.55 mg, 3.79 mmol, 3 eq), and Pd(dppf)Cl₂ (92.56 mg, 126.50 µmol, 0.1 eq) in dioxane (5 mL)/H₂O (1 mL) was stirred at 80 °C for 16 hours under a nitrogen atmosphere. After concentration under reduced pressure, the residue was purified by column chromatography (SiO₂, Petroleum ether/Ethyl acetate = 100/1 to 1/1) to afford Compound 11-3 (420 mg, 88.8% yield) as a white solid, which was used in the next step without further purification.

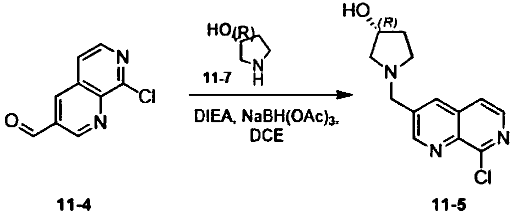

To a solution of Starting material 4 (10 g, 51.92 mmol, 1 eq) and (3R)-pyrrolidin-3-ol (9.05 g, 103.84 mmol, 8.62 mL, 2 eq) in DCE (500 mL) was added DIPEA (6.71 g, 51.92 mmol, 9.04 mL, 1 eq), and the mixture was stirred at room temperature for 1 hour. NaBH(OAc)₃ (33.01 g, 155.76 mmol, 3 eq) was added in three portions, followed by stirring at room temperature for 16 hours. After concentration under reduced pressure, the residue was purified by column chromatography (SiO₂, DCM:MeOH = 100:1 to 50:1) to afford Compound 11-5 (20 g, 87.64% yield) as a yellow oil.

¹H NMR (400 MHz, chloroform-d) δ = 9.12 (d, J = 2.1 Hz, 1H), 8.40 (d, J = 5.6 Hz, 1H), 8.31 (d, J = 2.0 Hz, 1H), 7.65 (d, J = 5.6 Hz, 1H), 5.31 (s, 1H), 4.22 - 4.03 (m, 2H), 3.48 (s, 1H), 3.25 - 3.08 (m, 2H), 2.95 (s, 2H), 1.43 (d, J = 6.7 Hz, 2H)

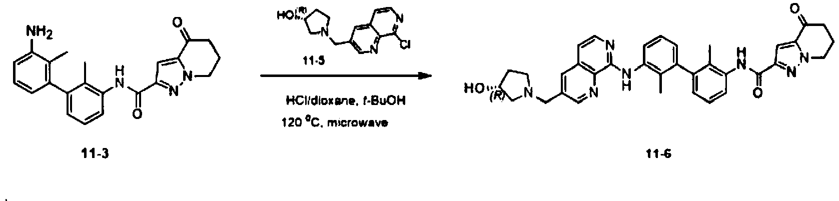

A mixture of Starting material 3 (420 mg, 1.59 mmol, 1 eq) and HCl/dioxane (4 M, 796.28 µL, 2 eq) in t-BuOH (10 mL) was stirred at 120 °C for 4 hours in a microwave reactor. After concentration under reduced pressure, the residue was purified by reversed-phase HPLC (conditions: water (NH₄HCO₃)-ACN; column: Waters XBridge C18, 150*50 mm*10 µm) to afford Compound 11-6 as a yellow solid.

LCMS m/z 602.2 [M+1]⁺.

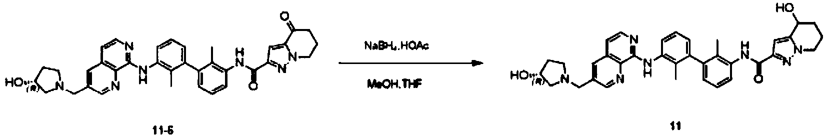

To a solution of Compound 11-6 (35 mg, 58.17 µmol, 1 eq) in MeOH (1 mL)/THF (1 mL) was added NaBH₄ (11.01 mg, 290.84 µmol, 5 eq), and the mixture was stirred at room temperature for 2 hours. After concentration under reduced pressure, the residue was purified by reversed-phase HPLC (column: Waters XBridge C18, 150*50 mm*10 µm; mobile phase: water (NH₄HCO₃)-ACN; B%: 32%-62% over 10 min) to afford Compound 11 (15 mg, 42.7% yield) as a white solid.

### LCMS m/z 604.3 [M+1]⁺.

¹H NMR (400 MHz, DMSO-d6) δ = 9.52 (s, 1H), 9.30 (s, 1H), 8.86 (s, 1H), 8.48 - 8.40 (m, 1H), 8.18 (s, 1H), 8.09 - 8.03 (m, 1H), 7.66 - 7.55 (m, 1H), 7.35 - 7.25 (m, 2H), 7.20 - 7.16 (m, 1H), 7.00 (s, 1H), 6.86 (d, J = 8.0 Hz, 1H), 6.69 (s, 1H), 5.55 (d, J = 5.3 Hz, 1H), 4.79 - 4.70 (m, 1H), 4.22 (dd, J = 3.3, 5.8 Hz, 1H), 4.17 - 4.10 (m, 1H), 3.81 (d, J = 10.9 Hz, 1H), 2.67 (d, J = 1.6 Hz, 3H), 2.39 (d, J = 3.3 Hz, 4H), 2.33 - 2.32 (m, 2H), 2.08 (s, 4H), 1.97 (s, 4H).

### 10-2. Preparation of Compound 12, 4-((2-acetamidoethyl)amino)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide

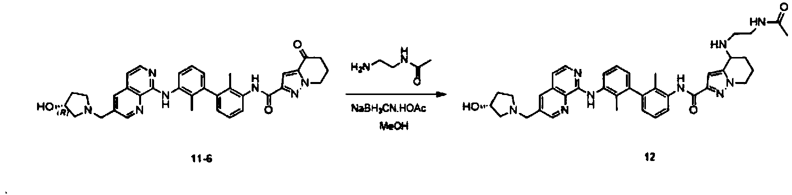

To a solution of starting material 11-6 (35 mg, 58.17 µmol, 1 eq) and N-(2-aminoethyl)acetamide (59.41 mg, 581.69 µmol, 55.52 µL, 10 eq) in MeOH (2 mL), HOAc (104.79 mg, 1.75 mmol, 99.80 µL, 30 eq) was added, followed by stirring at room temperature for 30 minutes. NaBH₃CN (18.28 mg, 290.84 µmol, 5 eq) was then added, and the reaction mixture was stirred at 40 °C for 1 hour. After concentration under reduced pressure, the residue was purified by reversed-phase HPLC (column: Waters XBridge C18, 150*50 mm*10 µm; mobile phase: [water (NH₄HCO₃)-ACN]; B%: 28%-58% over 10 min) to afford Compound 12 (18 mg, 45.0% yield) as a yellow solid.

LCMS 688.4 m/z [M+1]⁺.

¹H NMR (400 MHz, DMSO-d6) δ = 9.50 (s, 1H), 9.30 (s, 1H), 8.86 (s, 1H), 8.42 (d, J = 8.3 Hz, 1H), 8.18 (s, 1H), 8.05 (d, J = 5.6 Hz, 1H), 7.82 (s, 1H), 7.59 (d, J = 8.1 Hz, 1H), 7.34 - 7.25 (m, 2H), 7.18 (d, J = 5.9 Hz, 1H), 7.00 (d, J = 7.3 Hz, 1H), 6.86 (d, J = 7.1 Hz, 1H), 6.73 (s, 1H), 4.72 (d, J = 4.6 Hz, 1H), 4.22 (s, 1H), 4.13 (s, 2H), 3.89 - 3.76 (m, 3H), 3.13 (q, J = 5.9 Hz, 3H), 2.74 (dd, J = 6.3, 9.6 Hz, 1H), 2.68 - 2.63 (m, 4H), 2.41 - 2.30 (m, 3H), 2.20 (s, 2H), 2.10 - 2.06 (m, 3H), 1.97 (s, 4H), 1.80 (s, 3H)

### 10-3. Preparation of Compound 13, 4-((R)-3-hydroxypyrrolidin-1-yl)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide

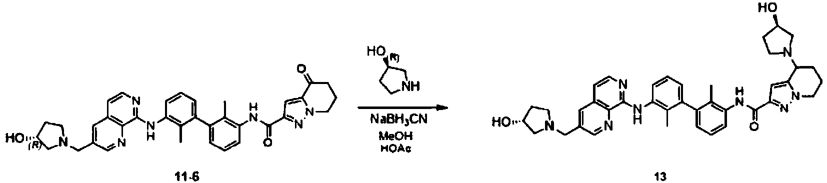

To a solution of starting material 11-6 (100 mg, 166.20 µmol, 1 eq) and (3R)-pyrrolidin-3-ol (144.79 mg, 1.66 mmol, 137.90 µL, 10 eq) in MeOH (2 mL), HOAc (299.41 mg, 4.99 mmol, 285.16 µL, 30 eq) was added, followed by stirring at room temperature for 30 minutes. NaBH₃CN (52.22 mg, 830.98 µmol, 5 eq) was then added, and the reaction mixture was stirred at 40 °C for 16 hours. After concentration under reduced pressure, the residue was purified by reversed-phase HPLC (column: YMC Triart C18, 150*25 mm*5 µm; mobile phase: [water (HCl)-ACN]; B%: 2%-32% over 10 min) to afford Compound 13 (62 mg, 55.5% yield) as a yellow solid.

LCMS 673.4 m/z [M+1]⁺.

¹H NMR (400 MHz, DMSO-d6) δ = 11.98 (s, 1H), 11.59 (s, 1H), 11.35 (s, 1H), 9.73 (d, J = 3.4 Hz, 1H), 9.40 (s, 1H), 8.76 (s, 1H), 7.75 (s, 1H), 7.57 - 7.44 (m, 2H), 7.30 (q, J = 7.1 Hz, 3H), 7.22 (s, 1H), 7.06 (d, J = 7.4 Hz, 1H), 4.93 - 4.79 (m, 2H), 4.72 (d, J = 18.8 Hz, 3H), 4.52 - 4.34 (m, 3H), 4.28 - 4.17 (m, 3H), 3.48 (s, 5H), 3.16 (s, 1H), 3.13 - 3.02 (m, 3H), 2.29 - 2.16 (m, 3H), 2.00 (d, J = 8.6 Hz, 8H)

### 10-4. Preparation of Compound 14, 4-((2-hydroxyethyl)amino)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridine-2-carboxamide

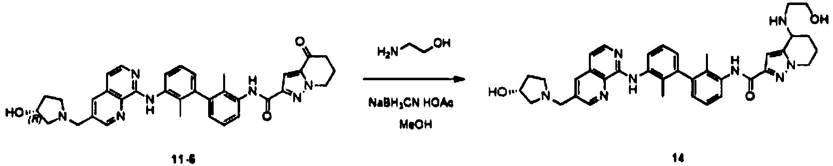

To a solution of starting material 11-6 (35 mg, 58.17 µmol, 1 eq) and 2-aminoethanol (35.53 mg, 581.69 µmol, 35.18 µL, 10 eq) in MeOH (2 mL), HOAc (104.79 mg, 1.75 mmol, 99.80 µL, 30 eq) was added, followed by stirring at room temperature for 30 minutes. NaBH₃CN (18.28 mg, 290.84 µmol, 5 eq) was then added, and the mixture was stirred at 40 °C for 2 hours. After completion of the reaction, the mixture was concentrated under reduced pressure, and the residue was purified by reversed-phase HPLC (column: Waters Xbridge C18 150*50 mm*10 µm; mobile phase: [water (NH₄HCO₃)-ACN]; B%: 28%-58% over 10 min) to afford Compound 14 (18 mg, 47.8% yield) as a yellow solid.

LCMS 647.4 m/z [M+1]⁺.

¹H NMR (400 MHz, DMSO-d6) δ = 9.54 - 9.47 (m, 1H), 9.30 (s, 1H), 8.87 (s, 1H), 8.42 (d, J = 8.5 Hz, 1H), 8.18 (s, 1H), 8.06 (s, 1H), 7.58 (d, J = 7.8 Hz, 1H), 7.36 - 7.24 (m, 2H), 7.18 (s, 1H), 7.01 (d, J = 7.6 Hz, 1H), 6.90 - 6.83 (m, 1H), 6.72 (s, 1H), 4.72 (d, J = 4.5 Hz, 1H), 4.55 - 4.47 (m, 1H), 4.26 - 4.19 (m, 1H), 4.17 - 4.10 (m, 2H), 3.88 (ddt, J = 1.4, 3.3, 6.3 Hz, 1H), 3.81 (d, J = 11.1 Hz, 1H), 3.48 (d, J = 5.9 Hz, 3H), 2.69 - 2.65 (m, 7H), 2.35 - 2.31 (m, 4H), 2.08 (s, 4H), 1.97 (s, 3H).

### 10-5. Preparation of Compound 15, N-(3'-((3-(((R)-3-hydroxypyrrolidin-1-yl)methyl)-1,7-naphthyridin-8-yl)amino)-2,2'-dimethyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

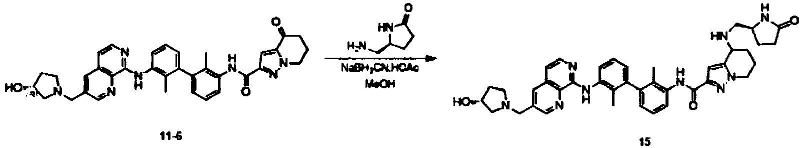

To a solution of starting material 11-6 (35 mg, 58.17 µmol, 1 eq) and (5S)-5-(aminomethyl)pyrrolidin-2-one (66.40 mg, 581.69 µmol, 10 eq) in MeOH (2 mL), HOAc (104.79 mg, 1.75 mmol, 99.80 µL, 30 eq) was added, followed by stirring at room temperature for 30 minutes. NaBH₃CN (18.28 mg, 290.84 µmol, 5 eq) was then added to the reaction mixture, and the mixture was stirred at 40 °C for 2 hours. After completion of the reaction, the reaction mixture was concentrated under reduced pressure, and the residue was purified by reversed-phase HPLC (column: Waters Xbridge C18 150*50mm* 10um; mobile phase: [water (NH₄HCO₃)-ACN]; B%: 28%-58%, 10 min) to afford Compound 15 (20 mg, 49.13% yield) as a yellow solid.

LCMS 700.4 m/z [M+1]⁺

¹H NMR (400 MHz, DMSO-d6) δ = 9.50 (d, J = 2.9 Hz, 1H), 9.30 (s, 1H), 8.86 (d, J = 1.9 Hz, 1H), 8.42 (d, J = 8.1 Hz, 1H), 8.18 (d, J = 1.4 Hz, 1H), 8.05 (d, J = 5.8 Hz, 1H), 7.68 (d, J = 19.6 Hz, 1H), 7.59 (br d, J = 7.4 Hz, 1H), 7.35 - 7.25 (m, 2H), 7.18 (d, J = 5.8 Hz, 1H), 7.01 (d, J = 7.4 Hz, 1H), 6.86 (d, J = 7.5 Hz, 1H), 6.76 (d, J = 9.8 Hz, 1H), 4.72 (d, J = 4.5 Hz, 1H), 4.29 - 4.06 (m, 3H), 3.94 - 3.74 (m, 3H), 3.58 (dd, J = 2.1, 3.7 Hz, 1H), 2.74 (dd, J = 6.0, 9.4 Hz, 1H), 2.70 - 2.65 (m, 3H), 2.64 - 2.57 (m, 3H), 2.38 (dd, J = 3.4, 9.6 Hz, 2H), 2.35 - 2.30 (m, 2H), 2.13 - 2.05 (m, 6H), 1.97 (s, 3H), 1.76 - 1.64 (m, 2H), 1.57 (dd, J = 5.1, 8.4 Hz, 1H).

### 10-6. Preparation of Compound 16, 2-((2-((3'-((3-(((R)-3-hydroxypyrrolidin-1-yl)methyl)-1,7-naphthyridin-8-yl)amino)-2,2'-dimethyl-[1,1'-biphenyl]-3-yl)carbamoyl)-4,5,6,7-tetrahydropyrazolo[1,5-a]pyridin-4-yl)amino)acetic acid

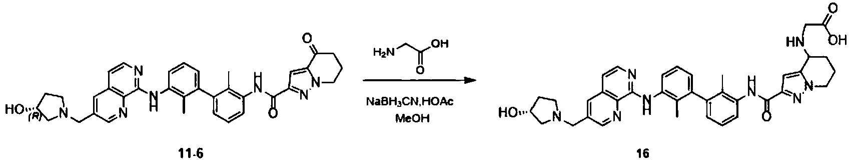

To a solution of starting material 11-6 (100 mg, 166.20 µmol, 1 eq) and glycine (124.76 mg, 1.66 mmol, 10 eq) in MeOH (2 mL), HOAc (299.41 mg, 4.99 mmol, 285.16 µL, 30 eq) was added, followed by stirring at room temperature for 30 minutes. NaBH₃CN (52.22 mg, 830.98 µmol, 5 eq) was then added to the reaction mixture, and the mixture was stirred at 40 °C for 16 hours. After completion of the reaction, the reaction mixture was concentrated under reduced pressure, and the residue was purified by reversed-phase HPLC (column: Waters Xbridge 150*25mm* 5um; mobile phase: [water (NH₄HCO₃)-ACN]; B%: 20%-50%, 8 min) to afford Compound 16 (102 mg, 92.88% yield) as a yellow solid.

LCMS 661.3 m/z [M+1]⁺.

¹H NMR (400 MHz, DMSO-d6) δ = 9.56 (s, 1H), 9.30 (s, 1H), 8.87 (s, 1H), 8.41 (d, J = 7.8 Hz, 1H), 8.19 (s, 1H), 8.06 (d, J = 5.8 Hz, 1H), 7.57 (d, J = 7.5 Hz, 1H), 7.36 - 7.24 (m, 2H), 7.18 (d, J = 5.8 Hz, 1H), 7.01 (d, J = 7.9 Hz, 1H), 6.87 (d, J = 7.8 Hz, 1H), 6.77 (s, 1H), 4.87 - 4.66 (m, 1H), 4.29 - 4.02 (m, 5H), 3.93 - 3.74 (m, 2H), 2.86 - 2.62 (m, 5H), 2.28 - 2.13 (m, 2H), 2.08 (s, 4H), 1.97 (s, 4H), 1.82 - 1.68 (m, 2H), 1.64 - 1.51 (m, 1H), 1.23 (s, 1H).

### 10-7. Preparation of Compound 17, (R)-2-((2-((3'-((3-((3-hydroxypyrrolidin-1-yl)methyl)-1,7-naphthyridin-8-yl)amino)-2,2'-dimethyl-[1,1'-biphenyl]-3-yl)carbamoyl)pyrazolo[1,5-a]pyridin-4-yl)amino)-2-methylpropanoic acid

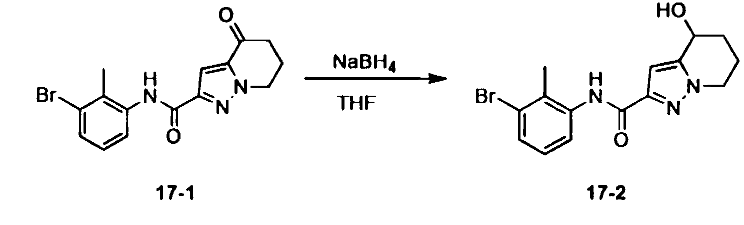

To a solution of N-(3-bromo-2-methyl-phenyl)-4-oxo-6,7-dihydro-5H-pyrazolo[1,5-a]pyridine-2-carboxamide (5 g, 14.36 mmol, 1 eq) in THF (80 mL), NaBH₄ (1.09 g, 28.72 mmol, 2 eq) was slowly added, and the mixture was stirred at room temperature for 2 hours. After completion of the reaction, aq. saturated NH₄Cl (200 mL) was added, and the mixture was extracted with EtOAc (200 mL*2). The organic layer was washed with brine (200 mL), dried over Na₂SO₄, and concentrated under reduced pressure to afford Compound 17-2 (4.46 g, 70.9% yield) as a yellow solid, which was used in the next step without further purification.

LCMS m/z 352.0 [M+3]⁺.

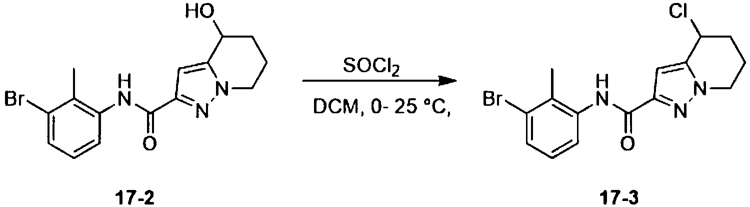

To a solution of N-(3-bromo-2-methyl-phenyl)-4-hydroxy-4,5,6,7-tetrahydropyrazolo[1,5-a]pyridine-2-carboxamide (4.46 g, 12.74 mmol, 1 eq) in DCM (80 mL), SOCl₂ (7.58 g, 63.68 mmol, 4.62 mL, 5 eq) was slowly added, and the mixture was stirred at room temperature for 12 hours. After completion of the reaction, aq. saturated NH₄Cl (200 mL) was added, and the mixture was extracted with EtOAc (200 mL*2). The organic layer was washed with brine (200 mL), dried over Na₂SO₄, and concentrated under reduced pressure to afford Compound 17-3 (3.78 g, 72.4% yield) as a red solid, which was used in the next step without further purification.

LCMS m/z 369.9 [M+3]⁺.

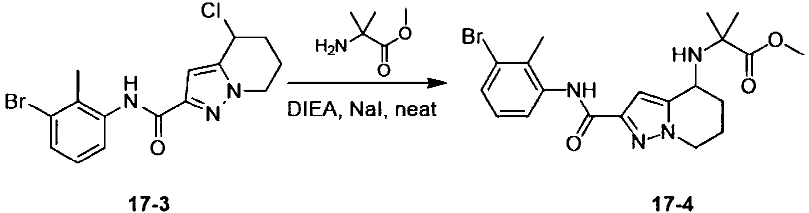

To a mixture of N-(3-bromo-2-methylphenyl)-4-chloro-4,5,6,7-tetrahydropyrazolo[1,5-a]pyridine-2-carboxamide (3.7 g, 10.04 mmol, 1 eq) and methyl 2-amino-2-methylpropanoate (11.76 g, 100.36 mmol, 10 eq), DIPEA (1.95 g, 15.05 mmol, 2.62 mL, 1.5 eq) and NaI (150.44 mg, 1.00 mmol, 0.1 eq) were added, and the reaction mixture was stirred at 90 °C for 12 hours. After completion, the mixture was cooled to room temperature and filtered, and the filtrate was concentrated under reduced pressure. The resulting residue was purified by column chromatography (SiO₂, Petroleum ether/Ethyl acetate = 3/1 to 1/1) to afford Compound 17-4 (3.65 g, 72.8% yield) as a white solid.

LCMS m/z 449.1 [M+1]⁺.

¹H NMR (400 MHz, DMSO-d6) δ ppm 1.32 (d, J=4.63 Hz, 6 H) 1.52 - 1.64 (m, 1 H) 1.87 - 1.99 (m, 3 H) 2.12 - 2.18 (m, 1 H) 2.27 (s, 3 H) 3.65 (s, 3 H) 3.88 (br d, J=2.63 Hz, 1 H) 4.06 - 4.19 (m, 2 H) 6.67 (s, 1 H) 7.15 (t, J=8.00 Hz, 1 H) 7.42 (d, J=7.75 Hz, 1 H) 7.48 (d, J=8.00 Hz, 1 H) 9.80 (s, 1 H).

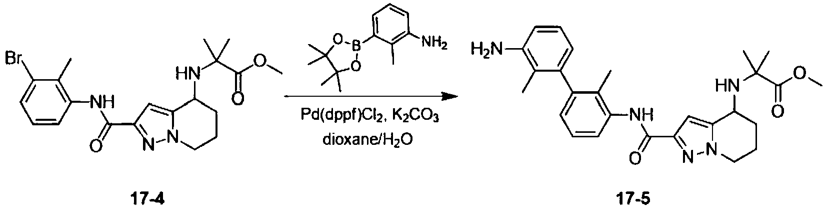

A mixture of methyl 2-[[2-[(3-bromo-2-methylphenyl)carbamoyl]-4,5,6,7-tetrahydropyrazolo[1,5-a]pyridin-4-yl]amino]-2-methylpropanoate (300 mg, 667.65 µmol, 1 eq), 2-methyl-3-(4,4,5,5-tetramethyl-1,3,2-dioxaborolan-2-yl)aniline (186.76 mg, 801.17 µmol, 1.2 eq), K₂CO₃ (276.83 mg, 2.00 mmol, 3 eq), and Pd(dppf)Cl₂ (48.85 mg, 66.76 µmol, 0.1 eq) in a mixture of dioxane (10 mL) and H₂O (2 mL) was stirred under a nitrogen atmosphere at 90 °C for 12 hours. After completion, the reaction mixture was cooled to room temperature and filtered, and the filtrate was concentrated under reduced pressure. The resulting residue was purified by column chromatography (SiO₂, Petroleum ether/Ethyl acetate = 0/1 to 1/1) to afford Compound 17-5 (314 mg, 86.0% yield) as a white solid.

LCMS m/z 476.2 [M+1]⁺.

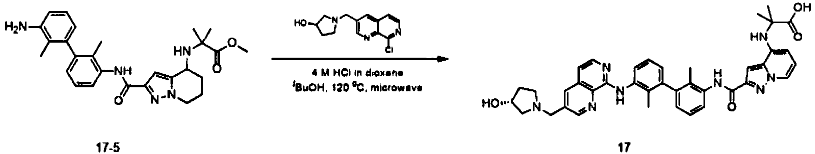

To a solution of methyl 2-[[2-[[3-(3-amino-2-methylphenyl)-2-methylphenyl]carbamoyl]-4,5,6,7-tetrahydropyrazolo[1,5-a]pyridin-4-yl]amino]-2-methylpropanoate (300 mg, 630.81 µmol, 1 eq) and (3R)-1-[(8-chloro-1,7-naphthyridin-3-yl)methyl]pyrrolidin-3-ol (415.89 mg, 1.58 mmol, 2.5 eq) in t-BuOH (10 mL), HCl/dioxane (4 M, 968.21 µL, 6.14 eq) was added, and the reaction mixture was stirred at 120 °C for 12 hours in a microwave reactor. After completion, the mixture was cooled to room temperature and filtered, and the filtrate was concentrated under reduced pressure. The residue was purified by reversed-phase HPLC (column: Waters XBridge Prep OBD C18, 150 * 40 mm * 10 µm; mobile phase: water (NH₄HCO₃)/ACN; gradient: 15%-45% B over 20 min) to afford Compound 17 (23 mg, 5.19% yield) as a yellow solid.

LCMS m/z 689.3 [M+1]⁺.

¹H NMR (400 MHz, DMSO-d6) δ ppm 1.30 (d, J=3.25 Hz, 6 H) 1.54 - 1.75 (m, 3 H) 1.87 - 1.95 (m, 1 H) 1.97 (s, 3 H) 2.03 (br dd, J=13.07, 6.82 Hz, 2 H) 2.08 (s, 3 H) 2.11 - 2.18 (m, 1 H) 2.39 (dd, J=9.57, 3.56 Hz, 1 H) 2.44 - 2.48 (m, 1 H) 2.66 (q, J=7.75 Hz, 1 H) 2.75 (dd, J=9.63, 6.13 Hz, 1 H) 3.75 - 3.87 (m, 3 H) 3.94 (br dd, J=7.32, 5.19 Hz, 1 H) 4.08 - 4.14 (m, 2 H) 4.23 (tt, J=6.71, 3.42 Hz, 1 H) 4.65 - 4.86 (m, 1 H) 6.75 (s, 1 H) 6.87 (d, J=7.00 Hz, 1 H) 7.01 (d, J=6.88 Hz, 1 H) 7.18 (d, J=5.75 Hz, 1 H) 7.30 (dt, J=13.79, 7.74 Hz, 2 H) 7.58 (d, J=7.75 Hz, 1 H) 8.06 (d, J=5.75 Hz, 1 H) 8.18 (d, J=1.75 Hz, 1 H) 8.43 (d, J=7.75 Hz, 1 H) 8.87 (d, J=2.00 Hz, 1 H) 9.30 (s, 1 H) 9.52 (s, 1 H).

### [Preparation Example 11]

### Preparation of Compounds 18 to 27

### 11-1. Preparation of Compound 22, (3R)-1-(2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)pyrrolidine-3-carboxylic acid

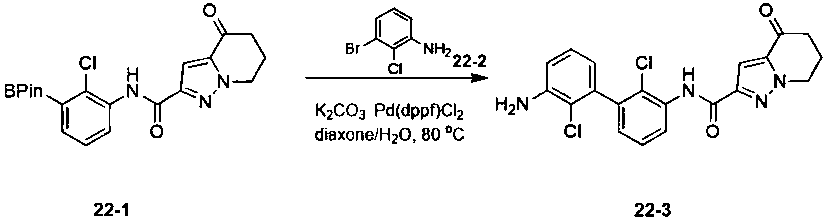

A mixture of N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (900 mg, 2.17 mmol, 1.1 eq), 3-bromo-2-chloroaniline (406.39 mg, 1.97 mmol, 1 eq), K₂CO₃ (816.10 mg, 5.90 mmol, 3 eq), and Pd(dppf)Cl₂ (144.02 mg, 196.83 µmol, 0.1 eq) in a mixture of dioxane (10 mL) and H₂O (2.5 mL) was stirred under a nitrogen atmosphere at 80 °C for 16 hours. After completion, the mixture was concentrated under reduced pressure, and the residue was purified by column chromatography (SiO₂, Petroleum ether/Ethyl acetate = 100/1 to 1/1) to afford Compound 22-3 (400 mg, 38.7% yield) as a yellow solid.

LCMS m/z 415.0 [M+1]⁺.

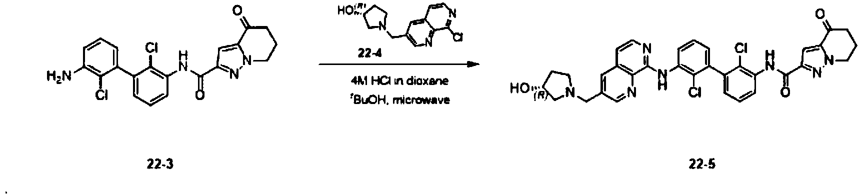

To a solution of (3R)-1-[(8-chloro-1,7-naphthyridin-3-yl)methyl]pyrrolidin-3-ol (317.53 mg, 1.20 mmol, 2.5 eq) and N-[3-(3-amino-2-chloro-phenyl)-2-chlorophenyl]-4-oxo-6,7-dihydro-5H-pyrazolo[1,5-a]pyridine-2-carboxamide (200 mg, 481.61 µmol, 1 eq) in t-BuOH (10 mL) HCl/dioxane (4 M, 240.81 µL, 2 eq) was added, and the reaction mixture was stirred at 120 °C for 12 hours. After completion, the mixture was concentrated under reduced pressure to afford Compound 22-5 (380 mg, 87.1% yield) as a brown solid, which was used in the next step without further purification.

LCMS m/z 642.3 [M+1]⁺.

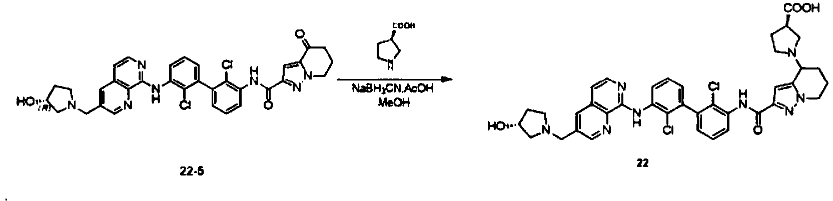

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (200 mg, 311.27 µmol, 1 eq) and pyrrolidine-3-carboxylic acid (179.18 mg, 1.56 mmol, 5 eq) in MeOH (15 mL), AcOH (37.38 mg, 622.54 µmol, 35.64 µL, 2 eq) and NaBH₃CN (97.80 mg, 1.56 mmol, 5 eq) were added, and the reaction mixture was stirred at 60 °C for 12 hours. After completion, the mixture was concentrated under reduced pressure, and the residue was purified by reversed-phase HPLC (0.1% NH₃·H₂O condition; column: Waters XBridge 150*25 mm* 5µm; mobile phase: water (NH₄HCO₃)/ACN; B%: 22%-52%, 9 min) to afford Compound 22 (33 mg, 14.2% yield) as a white solid.

LCMS m/z 741.3 [M+1]⁺.

¹H NMR (400 MHz, DMSO-d6) δ ppm 1.54 - 1.64 (m, 1 H) 1.88 - 1.98 (m, 4 H) 1.99 - 2.04 (m, 1 H) 2.15 - 2.30 (m, 1 H) 2.57 - 2.64 (m, 1 H) 2.65 - 2.75 (m, 3 H) 2.77 - 2.80 (m, 1 H) 2.81 - 2.87 (m, 1 H) 2.88 - 2.96 (m, 2 H) 3.79 - 3.94 (m, 4 H) 4.12 - 4.19 (m, 3 H) 4.20 - 4.25 (m, 2 H) 4.69 - 4.83 (m, 1 H) 6.70 - 6.75 (m, 1 H) 7.00 - 7.06 (m, 1 H) 7.17 - 7.23 (m, 1 H) 7.32 - 7.37 (m, 1 H) 7.46 - 7.55 (m, 2 H) 8.16 - 8.22 (m, 1 H) 8.24 - 8.31 (m, 2 H) 8.90 - 8.95 (m, 1 H) 9.09 - 9.17 (m, 1 H) 9.52 - 9.58 (m, 1 H) 9.85 - 9.92 (m, 1 H).

### 11-2. Preparation of Compound 25, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide

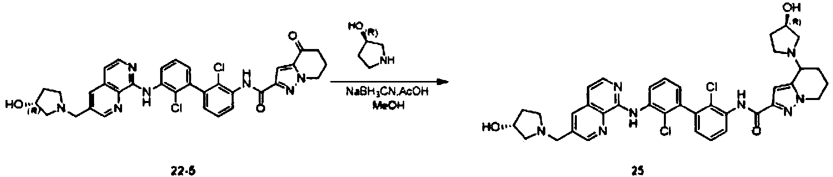

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (200 mg, 311.27 µmol, 1 eq) and (3R)-pyrrolidin-3-ol (135.59 mg, 1.56 mmol, 129.38 µL, 5 eq) in MeOH (15 mL), AcOH (37.38 mg, 622.54 µmol, 35.64 µL, 2 eq) and NaBH₃CN (97.80 mg, 1.56 mmol, 5 eq) were added, and the reaction mixture was stirred at 60 °C for 12 hours. After completion, the mixture was concentrated under reduced pressure, and the residue was purified by reversed-phase HPLC (0.1% HCl condition; column: Welch Xtimate C18 150*25 mm*5 µm; mobile phase: [water (HCl)-ACN]; gradient: 10%-40% B over 8 min) to afford Compound 25 (17 mg, 7.6% yield) as a brown solid.

LCMS m/z 713.3 [M+1]⁺.

¹H NMR (400 MHz, DMSO-d6) δ ppm 1.94 - 2.04 (m, 2 H) 2.05 - 2.11 (m, 1 H) 2.12 - 2.21 (m, 1 H) 2.23 - 2.36 (m, 2 H) 2.97 - 3.13 (m, 2 H) 3.15 - 3.18 (m, 2 H) 3.27 - 3.37 (m, 3 H) 3.44 - 3.52 (m, 2 H) 3.55 - 3.63 (m, 3 H) 4.22 - 4.27 (m, 2 H) 4.40 - 4.48 (m, 2 H) 4.64 - 4.74 (m, 2 H) 4.82 - 4.93 (m, 1 H) 7.24 (br s, 2 H) 7.33 - 7.44 (m, 2 H) 7.48 - 7.63 (m, 2 H) 8.03 - 8.23 (m, 2 H) 8.64 - 8.74 (m, 1 H) 9.20 - 9.34 (m, 1 H) 9.61 - 9.73 (m, 1 H) 11.18 - 11.38 (m, 1 H) 11.79 - 11.98 (m, 1 H).

### 11-3. Preparation of Compound 26, 2-((2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetic acid

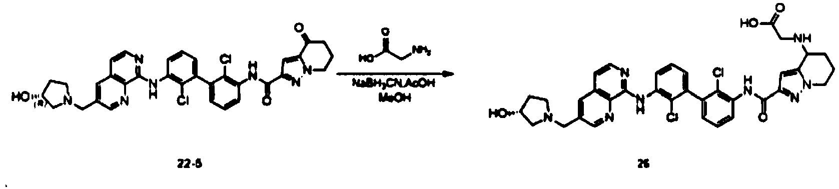

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (180 mg, 280.14 µmol, 1 eq) and 2-aminoacetic acid (105.15 mg, 1.40 mmol, 5 eq) in MeOH (15 mL), AcOH (33.64 mg, 560.28 µmol, 32.07 µL, 2 eq) and NaBH₃CN (88.02 mg, 1.40 mmol, 5 eq) were added, and the reaction mixture was stirred at 60 °C for 12 hours. After completion, the mixture was concentrated under reduced pressure, and the residue was purified by reversed-phase HPLC (0.1% NH₃·H₂O condition; column: Waters Xbridge 150*25 mm*5 µm; mobile phase: water (NH₄HCO₃)/ACN; B%: 20%-50% over 9 min) to afford Compound 26 (30 mg, 15.7% yield) as a white solid.

LCMS m/z 701.3 [M+1]⁺.

¹H NMR (400 MHz, DMSO-d6) δ ppm 1.60 (br dd, J=8.00, 4.88 Hz, 1 H) 1.68 - 1.81 (m, 1 H) 1.91 (br dd, J=13.57, 6.57 Hz, 1 H) 1.98 - 2.06 (m, 2 H) 2.20 (br dd, J=13.63, 6.00 Hz, 1 H) 2.63 - 2.83 (m, 3 H) 3.17 (s, 4 H) 3.81 - 3.94 (m, 3 H) 4.07 (br t, J=5.88 Hz, 1 H) 4.15 (br t, J=6.00 Hz, 2 H) 4.24 (br d, J=6.63 Hz, 1 H) 6.83 (s, 1 H) 7.03 (dd, J=7.50, 1.00 Hz, 1 H) 7.20 (dd, J=7.57, 1.44 Hz, 1 H) 7.35 (d, J=5.88 Hz, 1 H) 7.51 (q, J=8.05 Hz, 2 H) 8.20 (d, J=5.88 Hz, 1 H) 8.25 - 8.30 (m, 2 H) 8.93 (d, J=1.88 Hz, 1 H) 9.13 (dd, J=8.38, 1.38 Hz, 1 H) 9.57 (s, 1 H) 9.89 (s, 1 H)

### 11-4. Preparation of Compound 18, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-hydroxy-4,5,6,7-tetrahydropyrazolo[1,5-a]pyridine-2-carboxamide

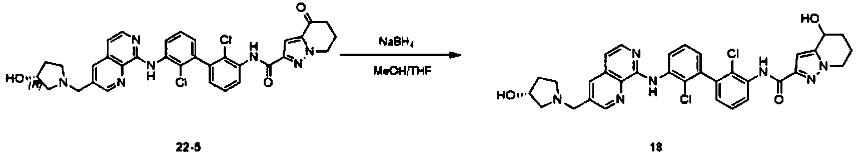

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (100 mg, 155.63 µmol, 1 eq) in MeOH (1 mL)/THF (1 mL), NaBH₄ (778.17 µmol, 5 eq) was added, and the reaction mixture was stirred at 20 °C for 2 hours. The mixture was concentrated under reduced pressure and purified by reversed-phase HPLC (column: Waters Xbridge C18, 150*50 mm*10 µm; mobile phase: water (NH₄HCO₃)/ACN; B%: 44%-74% over 10 min) to afford Compound 18 (20 mg, 19.1% yield) as a white solid.

LCMS m/z 644.3 [M+1]⁺.

¹H NMR (400 MHz, DMSO-d6) δ ppm 2.00 - 2.03 (m, 3 H) 2.33 - 2.34 (m, 4 H) 2.40 - 2.76 (m, 3 H) 3.81 - 3.87 (m, 2 H) 4.13 - 4.22 (m, 3 H) 4.72 - 4.77 (m, 2 H) 5.60 - 5.61 (m, 1 H) 6.75 (s, 1 H) 7.02 - 7.04 (m, 1 H) 7.20 - 7.21 (m, 1 H) 7.34 - 7.36 (m, 2 H) 8.19 - 8.20 (m, 1 H) 8.26 - 8.31 (m, 2 H) 8.92 (s, 1 H) 9.13 - 9.15 (s, 1 H) 9.57 (s, 1 H) 9.89 (s, 1 H)

### 11-5. Preparation of Compound 19, 4-((2-acetamidoethyl)amino)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridine-2-carboxamide

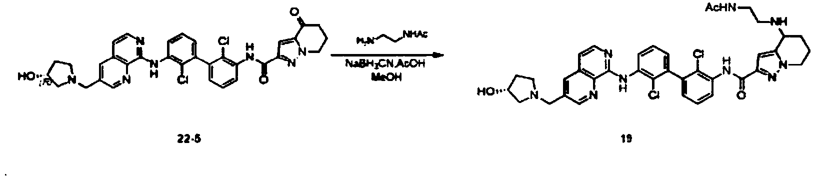

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (25 mg, 38.91 µmol, 1 eq) and N-(2-aminoethyl)acetamide (39.74 mg, 389.08 µmol, 37.14 µL, 10 eq) in MeOH (5 mL), NaBH₃CN (12.23 mg, 194.54 µmol, 5 eq) and HOAc (70.09 mg, 1.17 mmol, 66.76 µL, 30 eq) were added and the reaction mixture was stirred at 60 °C for 2 hours. After completion, the mixture was concentrated under reduced pressure and the residue wase purified by reversed-phase HPLC (column: Waters Xbridge, 150*25 mm*5µm; mobile phase: water (NH₄HCO₃)/ACN; B%: 45%-75% over 8 min) to afford Compound 19 (15 mg, 51.5% yield) as a yellow solid.

LCMS m/z 728.2 [M+1]⁺.

¹H NMR (400 MHz, DMSO-d6) δ ppm 1.80 (s, 3 H) 2.33 - 2.65 (m, 3 H) 2.66 - 2.76 (m, 7 H) 3.12 - 3.14 (m, 2 H) 3.37 - 3.39 (m, 2 H) 3.80 - 4.14 (m, 3 H) 4.14 - 4.23 (m, 3 H) 4.72 - 4.73 (m, 1 H) 6.79 (s, 1 H) 7.02 - 7.04 (m, 1 H) 7.20 - 7.21 (m, 1 H) 7.34 - 7.36 (m, 2 H) 8.19 - 8.20 (m, 1 H) 8.26 - 8.31 (m, 2 H) 8.92 (s, 1 H) 9.13 - 9.15 (s, 1 H) 9.57 (s, 1 H) 9.89 (s, 1 H).

### 11-6. Preparation of Compound 20, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((2-hydroxyethyl)amino)-4,5,6,7-tetrahydropyrazolo[1,5-a]pyridine-2-carboxamide

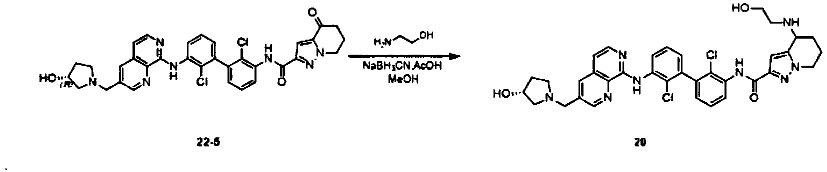

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (30 mg, 46.69 µmol, 1 eq) and 2-aminoethanol (28.52 mg, 466.90 µmol, 28.24 µL, 10 eq) in MeOH (3 mL), NaBH₃CN (14.67 mg, 233.45 µmol, 5 eq) and HOAc (84.12 mg, 1.40 mmol, 80.11 µL, 30 eq) were added, and the reation mixture was stirred at 60 °C for 2 hours. After completion, the mixture was concentrated under reduced pressure, and the residue was purified by reversed-phase HPLC (column: Waters Xbridge, 150*25 mm*5 µm; mobile phase: water (NH₄HCO₃)/ACN; B%: 45%-75% over 8 min) to afford Compound 20 (21 mg, 62.1% yield) as a yellow solid.

LCMS m/z 687.2 [M+1]⁺.

¹H NMR (400 MHz, DMSO-d6) δ ppm 1.59 - 1.98 (s, 2 H) 2.01 - 2.18 (m, 4 H) 2.20 - 2.22 (m, 4 H) 2.32 - 2.351 (m, 5 H) 2.36 - 2.50 (m, 3 H) 2.67 - 3.01 (m, 3 H) 3.60 - 3.68 (m, 3 H) 7.02 - 7.04 (m, 1 H) 7.20 - 7.21 (m, 1 H) 7.34 - 7.36 (m, 2 H) 8.19 - 8.20 (m, 1 H) 8.26 - 8.31 (m, 2 H) 8.92 (s, 1 H) 9.13 - 9.15 (s, 1 H) 9.57 (s, 1 H) 9.89 (s, 1 H).

### 11-7. Preparation of Compound 21, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

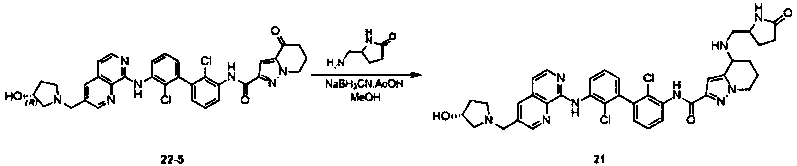

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (35 mg, 54.47 µmol, 1 eq) and (5S)-5-(aminomethyl)pyrrolidin-2-one (62.18 mg, 544.72 µmol, 10 eq) in MeOH (3 mL), HOAc (98.13 mg, 1.63 mmol, 93.46 µL, 30 eq) was added, and the reaction mixture was at 60 °C for 10 minutes. After completion of the reaction, NaBH₃CN (17.12 mg, 272.36 µmol, 5 eq) was added, and the mixture was further stirred at 60 °C for 4 hours. After completion, the mixture was concentrated under reduced pressure, and the residue was purified by reversed-phase HPLC (column: Waters Xbridge, 150*25 mm*5 µm; mobile phase: water (NH₄HCO₃)/ACN; B%: 45%-75% over 8 min) to afford Compound 21 (25 mg, 61.9% yield) as a yellow solid.

LCMS m/z 740.2 [M+1]⁺.

¹H NMR (400 MHz, DMSO-d6) δ ppm 1.68 - 1.70 (m, 2 H) 2.11 - 2.18 (m, 1 H) 2.32 - 2.40 (m, 7 H) 2.67 - 2.75 (m, 5 H) 3.05 - 3.1 (m, 1 H) 3.78 - 3.88 (m, 3 H) 4.12 - 4.21 (m, 3 H) 4.72 - 4.73 (m, 3 H ) 6.82 - 6.85 (m, 2 H) 7.02 - 7.04 (m, 1 H) 7.20 - 7.21 (m, 1 H) 7.34 - 7.36 (m, 2 H) 8.19 - 8.20 (m, 1 H) 8.26 - 8.31 (m, 2 H) 8.92 (s, 1 H) 9.13 - 9.15 (s, 1 H) 9.57 (s, 1 H) 9.89 (s, 1 H).

### 11-8. Preparation of Compound 24, 1-(2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid

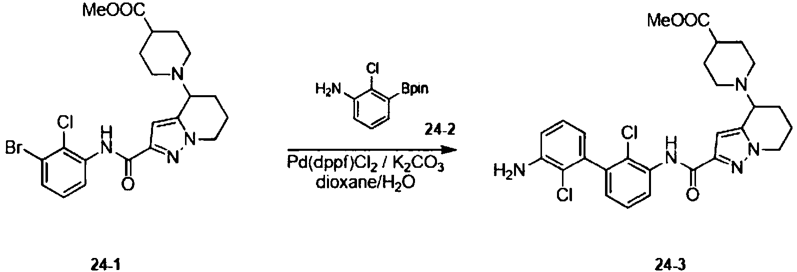

methyl 1-[2-[(3-bromo-2-chlorophenyl)carbamoyl]-4,5,6,7-tetrahydropyrazolo[1,5-a]pyridin-4-yl]piperidine-4-carboxylate (100 mg, 201.70 µmol, 1 eq), 2-chloro-3-(4,4,5,5-tetramethyl-1,3,2-dioxaborolan-2-yl)aniline (51.14 mg, 201.70 µmol, 1 eq), Pd(dppf)Cl₂ (147.58 mg, 201.70 µmol, 1 eq), and K₂CO₃ (27.88 mg, 201.70 µmol, 1 eq) were mixed in dioxane (1.5 mL)/H₂O (0.4 mL) and stirred under a nitrogen atmosphere at 90 °C for 12 hours. The mixture was concentrated under reduced pressure, and the residue was purified by prep-TLC (SiO₂, DCM:MeOH = 10:1) to afford Compound 24-3 (80 mg, 73.1% yield) as a yellow solid.

LCMS m/z 542.2 [M+1]⁺.

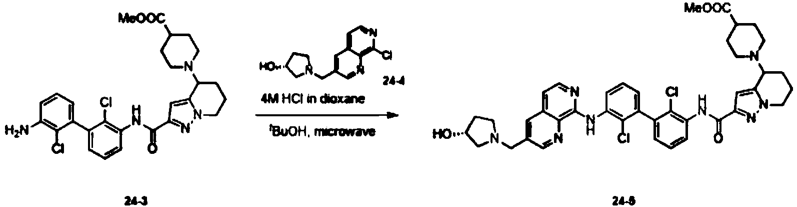

(3R)-1-[(8-chloro-1,7-naphthyridin-3-yl)methyl]pyrrolidin-3-ol (109.39 mg, 414.78 µmol, 3 eq), methyl 1-[2-[[3-(3-amino-2-chloro-phenyl)-2-chlorophenyl]carbamoyl]-4,5,6,7-tetrahydropyrazolo[1,5-a]pyridin-4-yl]piperidine-4-carboxylate (75 mg, 138.26 µmol, 1 eq), and HCl/dioxane (4 M, 34.57 µL, 1 eq) were mixed in t-BuOH (10 mL) and stirred at 120 °C for 3 hours in a microwave reactor. After completion of the reaction, the mixture was filtered and the filtrate was concentrated under reduced pressure to afford Compound 24-5 (80 mg, 75.1% yield) as a black solid, which was used in the next step without further purification.

LCMS m/z 729.4 [M+1]⁺.

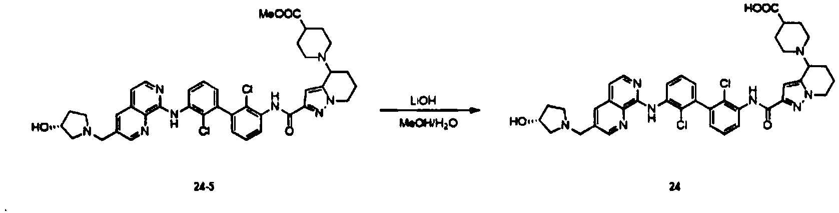

A solution of methyl 1-[2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]piperidine-4-carboxylate (100 mg, 129.92 µmol, 1 eq) and LiOH (9.33 mg, 389.75 µmol, 3 eq) in MeOH (6 m)/H₂O (2 mL) was stirred at room temperature for 12 hours. After completion of the reaction, the mixture was adjusted to pH 6-7 with aqueous 1 N HCl and concentrated under reduced pressure. The resulting residue was purified by prep-HPLC (column: Waters Xbridge Prep OBD C18, 150*40 mm*10 µm; mobile phase: water (NH₄HCO₃)/ACN; gradient: 22-52% B over 15 min) to afford Compound 24 (35 mg, 33.8% yield) as a white solid.

LCMS m/z 755.4 [M+1]⁺.

¹H NMR (400 MHz, DMSO-d6) δ = 9.90 (s, 1H), 9.56 (s, 1H), 9.18 - 9.11 (m, 1H), 8.92 (d, J = 1.9 Hz, 1H), 8.31 - 8.23 (m, 2H), 8.20 (d, J = 5.9 Hz, 1H), 7.58 - 7.45 (m, 2H), 7.35 (d, J = 5.9 Hz, 1H), 7.20 (d, J = 6.9 Hz, 1H), 7.03 (d, J = 7.5 Hz, 1H), 6.67 (s, 1H), 4.21 (br d, J = 7.0 Hz, 2H), 4.10 - 3.94 (m, 2H), 3.88 - 3.77 (m, 2H), 2.81 - 2.72 (m, 2H), 2.70 - 2.63 (m, 2H), 2.48 - 2.36 (m, 3H), 2.30 - 2.12 (m, 3H), 2.06 - 1.94 (m, 2H), 1.91 - 1.76 (m, 3H), 1.73 - 1.49 (m, 4H), 1.39 (br d, J = 11.4 Hz, 1H).

### 11-9. Preparation of Compound 23, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(4-hydroxypiperidin-1-yl)-4,5,6,7-tetrahydropyrazolo[1,5-a]pyridine-2-carboxamide

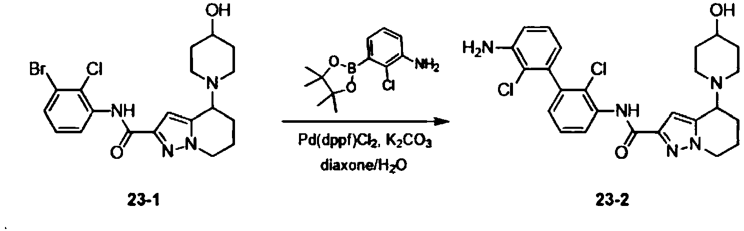

A mixture of N-(3-bromo-2-chloro-phenyl)-4-(4-hydroxy-1-piperidyl)-4,5,6,7-tetrahydropyrazolo[1,5-a]pyridine-2-carboxamide (150 mg, 330.57 µmol, 1 eq), 2-chloro-3-(4,4,5,5-tetramethyl-1,3,2-dioxaborolan-2-yl)aniline (125.72 mg, 495.86 µmol, 1.5 eq), K₂CO₃ (137.06 mg, 991.71 µmol, 3 eq), and Pd(dppf)Cl₂ (24.19 mg, 33.06 µmol, 0.1 eq) in a dioxane (20 mL)/H₂O (5 mL) was stirred under a nitrogen atmosphere at 90 °C for 8 hours. After completion of the reaction, the mixture was concentrated under reduced pressure and purified by column chromatography (SiO₂, DCM:MeOH = 10:1) to afford Compound 23-2 (160 mg, 94.7% yield) as a black solid.

LCMS m/z 500.2 [M+1]⁺.

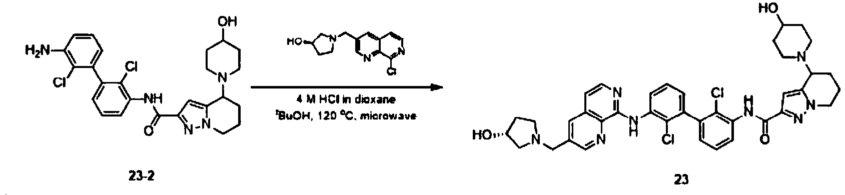

To a solution of N-[3-(3-amino-2-chloro-phenyl)-2-chlorophenyl]-4-(4-hydroxy-1-piperidyl)-4,5,6,7-tetrahydropyrazolo[1,5-a]pyridine-2-carboxamide 23-2 (160 mg, 319.73 µmol, 1 eq) in t-BuOH (10 mL), (3R)-1-[(8-chloro-1,7-naphthyridin-3-yl)methyl]pyrrolidin-3-ol (252.96 mg, 959.20 µmol, 3 eq) and 4 M HCl (in dioxane (0.1 mL)) were added, and the mixture was stirred in a microwave reactor at 120 °C for 12 hours. After completion of the reaction, the mixture was concentrated under reduced pressure, and the residue was purified by reversed-phase HPLC (column: Waters Xbridge 150 * 25 mm * 5 µm; mobile phase: [water (NH₄HCO₃)-ACN]; gradient: 48%-78% B over 9 min) to afford Compound 23 (71 mg, 30.5% yield) as a yellow solid.

LCMS m/z 727.3 [M+1]⁺.

¹H NMR (400 MHz, DMSO-d6) δ ppm 1.32 - 1.47 (m, 2 H) 1.53 - 1.61 (m, 1 H) 1.73 (br s, 2 H) 1.89 (br d, J=7.38 Hz, 1 H) 1.95 - 2.06 (m, 2 H) 2.12 (br s, 1 H) 2.27 (br t, J=9.07 Hz, 1 H) 2.38 (br dd, J=9.76, 3.75 Hz, 2 H) 2.61 - 2.69 (m, 2 H) 2.74 (br dd, J=9.69, 6.19 Hz, 2 H) 3.46 (br d, J=3.88 Hz, 1 H) 3.76 - 3.89 (m, 2 H) 3.92 - 4.10 (m, 2 H) 4.20 (br d, J=7.25 Hz, 2 H) 4.56 (d, J=4.13 Hz, 1 H) 4.73 (d, J=4.50 Hz, 1 H) 6.65 (s, 1 H) 7.03 (d, J=7.50 Hz, 1 H) 7.20 (d, J=7.38 Hz, 1 H) 7.35 (d, J=5.88 Hz, 1 H) 7.51 (q, J=8.05 Hz, 2 H) 8.19 (d, J=5.75 Hz, 1 H) 8.24 - 8.31 (m, 2 H) 8.92 (d, J=1.75 Hz, 1 H) 9.13 (d, J=8.50 Hz, 1 H) 9.55 (s, 1 H) 9.89 (s, 1 H).

### 11-10. Preparation of Compound 27, 2-((2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)-2-methylpropanoic acid,

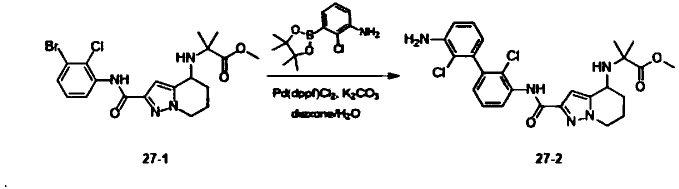

A mixture of methyl 2-[[2-[(3-bromo-2-chlorophenyl)carbamoyl]-4,5,6,7-tetrahydropyrazolo[1,5-a]pyridin-4-yl]amino]-2-methylpropanoate (200 mg, 425.75 µmol, 1 eq), 2-chloro-3-(4,4,5,5-tetramethyl-1,3,2-dioxaborolan-2-yl)aniline (161.91 mg, 638.63 µmol, 1.5 eq), K₂CO₃ (176.53 mg, 1.28 mmol, 3 eq), and Pd(dppf)Cl₂ (31.15 mg, 42.58 µmol, 0.1 eq) in dioxane (10 mL)/H₂O (2 mL) was stirred under a nitrogen atmosphere at 90 °C for 12 hours. After completion of the reaction, the mixture was concentrated under reduced pressure, and the residue was purified by column chromatography (SiO₂, Petroleum ether/Ethyl acetate = 0:1 to 1:1) to afford Compound 27-2 (216 mg, 78.5% yield) as a white solid.

LCMS m/z 516.2 [M+1]⁺.

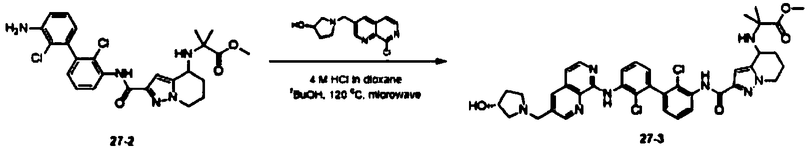

To a mixture of methyl 2-[[2-[[3-(3-amino-2-chloro-phenyl)-2-chlorophenyl]carbamoyl]-4,5,6,7-tetrahydropyrazolo[1,5-a]pyridin-4-yl]amino]-2-methylpropanoate (200 mg, 387.28 µmol, 1 eq) and (3R)-1-[(8-chloro-1,7-naphthyridin-3-yl)methyl]pyrrolidin-3-ol (306.41 mg, 1.16 mmol, 3 eq) in t-BuOH (10 mL), HCl/dioxane (4 M, 968.21 µL, 10 eq) was added, and the resulting mixture was stirred in a microwave reactor at 120 °C for 12 hours. After completion of the reaction, the mixture was concentrated under reduced pressure to afford Compound 27-3 (200 mg, 69.4% yield) as a black solid, which was used in the next step without further purification.

LCMS m/z 743.3 [M+1]⁺.

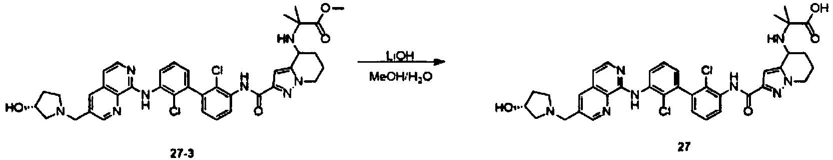

To a solution of methyl 2-[[2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]amino]-2-methylpropanoate 6 (200 mg, 268.93 µmol, 1 eq) in MeOH (5 mL)/H₂O (5 mL), LiOH·H₂O (33.86 mg, 806.80 µmol, 3 eq) was added, andstirred at room temperature for 12 hours. The reaction mixture was concentrated under reduced pressure, and the residue was purified by reversed-phase HPLC (column: Waters Xbridge 150 * 25 mm * 5 µm; mobile phase: [water (NH₄HCO₃)-ACN]; gradient: 24%-54% B over 9 min; column: Phenomenex Luna C18 150 * 25 mm * 10 µm; mobile phase: [water (HCl)-ACN]; gradient: 5%-35% B over 10 min) to afford Compound 27 (44 mg, 21.3% yield) as a yellow solid.

LCMS m/z 729.2 [M+1]⁺.

¹H NMR (400 MHz, DMSO-d6) δ ppm 1.30 (d, J=3.25 Hz, 6 H) 1.54 - 1.69 (m, 2 H) 1.90 - 2.06 (m, 3 H) 2.09 - 2.18 (m, 1 H) 2.31 - 2.42 (m, 3 H) 2.62 - 2.78 (m, 3 H) 3.78 - 3.89 (m, 2 H) 3.94 (br dd, J=6.88, 5.38 Hz, 1 H) 4.06 - 4.18 (m, 2 H) 4.19 - 4.25 (m, 1 H) 4.73 (br d, J=1.63 Hz, 1 H) 6.80 (s, 1 H) 7.04 (dd, J=7.57, 1.31 Hz, 1 H) 7.20 (dd, J=7.63, 1.50 Hz, 1 H) 7.36 (d, J=5.88 Hz, 1 H) 7.52 (q, J=8.13 Hz, 2 H) 8.20 (d, J=5.75 Hz, 1 H) 8.27 (d, J=1.63 Hz, 1 H) 8.31 (d, J=7.75 Hz, 1 H) 8.93 (d, J=1.88 Hz, 1 H) 9.14 (dd, J=8.38, 1.38 Hz, 1 H) 9.55 (s, 1 H) 9.90 (s, 1 H).

### [Preparation Example 12]

### Preparation of Compounds 28 to 36

### 12-1. Preparation of Compound 28, N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-((2-hydroxyethyl)amino)-4,5,6,7-tetrahydropyrazolo[1,5-a]pyridine-2-carboxamide

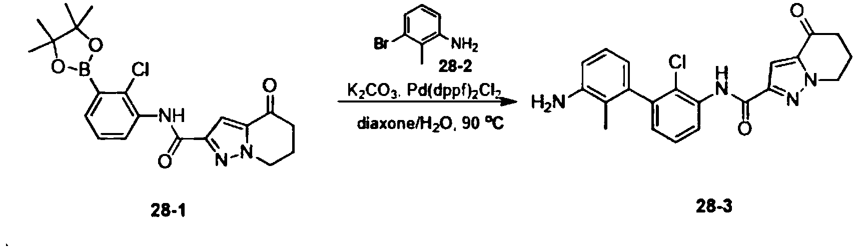

N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (3 g, 7.22 mmol, 1 eq), 3-bromo-2-methylaniline (2.01 g, 10.83 mmol, 1.33 mL, 1.5 eq), K₂CO₃ (2.79 g, 20.21 mmol, 2.8 eq), and Pd(dppf)Cl₂ (528.09 mg, 721.71 µmol, 0.1 eq) were mixed in dioxane (30 mL)/H₂O (3 mL), and the reaction mixture was stirred at 90 °C for 2 hours under a nitrogen atmosphere. Water (40 mL) was added to the mixture, followed by extraction with ethyl acetate (30 mL x 3). The combined organic layers were washed with brine (60 mL), dried over Na₂SO₄, and concentrated under reduced pressure. The residue was purified by flash silica gel chromatography (Petroleum ether/Ethyl acetate = 100/1 to 1/1) to afford Compound 28-3 (2.5 g, 78.9% yield) as a white solid.

LCMS m/z 395.3 [M+1]⁺.

¹H NMR (400 MHz, DMSO-d6) δ = 9.74 (s, 1H), 8.04 (dd, J = 1.4, 8.1 Hz, 1H), 7.41 (t, J = 7.9 Hz, 1H), 7.28 (s, 1H), 7.07 (dd, J = 1.5, 7.6 Hz, 1H), 7.00 - 6.86 (m, 1H), 6.75 - 6.60 (m, 1H), 6.43 - 6.24 (m, 1H), 4.95 (s, 2H), 4.55 - 4.38 (m, 2H), 2.80 - 2.63 (m, 2H), 2.41 - 2.24 (m, 2H), 1.77 (s, 3H).

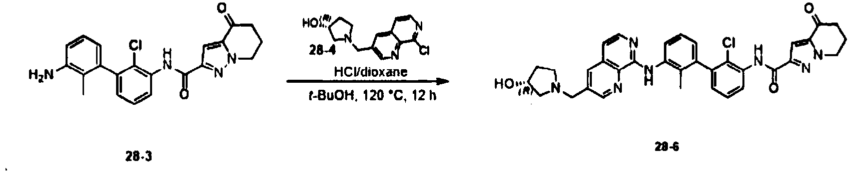

(3R)-1-[(8-chloro-1,7-naphthyridin-3-yl)methyl]pyrrolidin-3-ol (133.58 mg, 506.52 µmol, 2 eq) was added to a solution of N-[3-(3-amino-2-methylphenyl)-2-chlorophenyl]-4-oxo-6,7-dihydro-5H-pyrazolo[1,5-a]pyridine-2-carboxamide (100 mg, 253.26 µmol, 1 eq) in t-BuOH (10 mL), followed by addition of HCl/dioxane (4 M, 253.26 µL, 4 eq), and the mixture was stirred at 120 °C for 12 hours. The pH of the mixture was adjusted to 8 using aq. 1 N NaOH solution, and the precipitated solid was filtered to afford Compound 28-6 (126 mg, 63.9% yield) as a white solid, which was used in the next reaction without further purification.

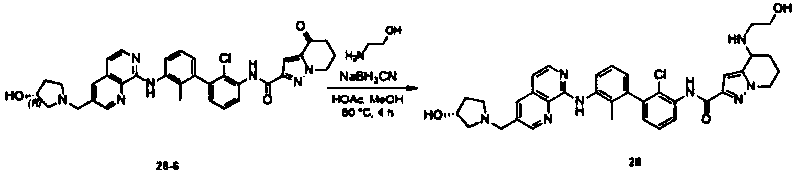

N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (100 mg, 160.74 µmol, 1 eq), 2-aminoethanol (98.19 mg, 1.61 mmol, 97.21 µL, 10 eq), and HOAc (96.53 mg, 1.61 mmol, 92.02 µL, 10 eq) were mixed in MeOH (10 mL) and stirred at 60 °C for 2 hours. NaBH₃CN (30.30 mg, 482.23 µmol, 3 eq) was then added, and the mixture was further stirred at 60 °C for 2 hours. The mixture was concentrated under reduced pressure, and the residue was purified by prep-HPLC (column: Waters Xbridge 150 * 25 mm * 5 µm; mobile phase: [water (NH₄HCO₃)-ACN]; gradient: 35%-65% B over 9 min) to afford Compound 28 (23 mg, 20.3% yield) as a white solid.

LCMS m/z 677.4 [M+1]⁺.

¹H NMR (400 MHz, METHANOL-d4) δ = 8.88 (d, J = 2.0 Hz, 1H), 8.39 (dd, J = 1.5, 8.3 Hz, 1H), 8.23 - 8.09 (m, 2H), 7.94 (d, J = 5.9 Hz, 1H), 7.43 (t, J = 7.9 Hz, 1H), 7.34 (t, J = 7.8 Hz, 1H), 7.14 (dd, J = 1.5, 7.6 Hz, 1H), 7.08 (d, J = 6.0 Hz, 1H), 7.00 (d, J = 6.8 Hz, 1H), 6.89 (s, 1H), 4.44 - 4.32 (m, 1H), 4.25 - 4.16 (m, 2H), 4.04 (dd, J = 5.4, 7.4 Hz, 1H), 3.96 - 3.81 (m, 2H), 3.70 (t, J = 5.7 Hz, 2H), 2.91 - 2.79 (m, 4H), 2.64 - 2.53 (m, 2H), 2.41 - 2.25 (m, 1H), 2.23 - 2.16 (m, 2H), 2.14 (s, 3H), 2.10 - 1.97 (m, 1H), 1.92 - 1.68 (m, 2H).

### 12-2. Preparation of Compound 29, N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-((R)-3-hydroxypyrrolidin-1-yl)-4,5,6,7-tetrahydropyrazolo[1,5-a]pyridine-2-carboxamide

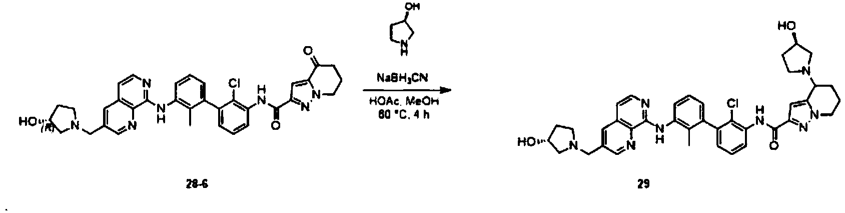

A mixture of N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (100 mg, 160.74 µmol, 1 eq), 2-aminoethanol (98.19 mg, 1.61 mmol, 97.21 µL, 10 eq), and HOAc (96.53 mg, 1.61 mmol, 92.02 µL, 10 eq) in MeOH (10 mL) was stirred at 60 °C for 2 hours. NaBH₃CN (30.30 mg, 482.23 µmol, 3 eq) was added to the mixture, followed by stirring at 60 °C for an additional 2 hours. The mixture was concentrated under reduced pressure, and the residue was purified by prep-HPLC (column: Waters Xbridge 150 * 25 mm * 5 µm; mobile phase: [water (NH₄HCO₃)-ACN]; gradient: 35%-65% B over 9 min) to afford Compound 28 (23 mg, 20.3% yield) as a white solid.

LCMS m/z 693.4 [M+1]⁺.
**LCMS** m/z 677.4 [M+1]⁺.
**¹H NMR** (400 MHz, METHANOL-d4) δ = 8.88 (d, J = 2.0 Hz, 1H), 8.39 (dd, J = 1.5, 8.3 Hz, 1H), 8.23 - 8.09 (m, 2H), 7.94 (d, J = 5.9 Hz, 1H), 7.43 (t, J = 7.9 Hz, 1H), 7.34 (t, J = 7.8 Hz, 1H), 7.14 (dd, J = 1.5, 7.6 Hz, 1H), 7.08 (d, J = 6.0 Hz, 1H), 7.00 (d, J = 6.8 Hz, 1H), 6.89 (s, 1H), 4.44 - 4.32 (m, 1H), 4.25 - 4.16 (m, 2H), 4.04 (dd, J = 5.4, 7.4 Hz, 1H), 3.96 - 3.81 (m, 2H), 3.70 (t, J = 5.7 Hz, 2H), 2.91 - 2.79 (m, 4H), 2.64 - 2.53 (m, 2H), 2.41 - 2.25 (m, 1H), 2.23 - 2.16 (m, 2H), 2.14 (s, 3H), 2.10 - 1.97 (m, 1H), 1.92 - 1.68 (m, 2H).

### 12-3. Preparation of Compound 30, 4-((2-acetamidoethyl)amino)-N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4,5,6,7-tetrahydropyrazolo[1,5-a]pyridine-2-carboxamide

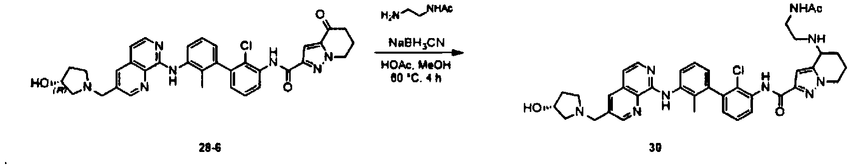

A solution of N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (80 mg, 128.59 µmol, 1 eq), N-(2-aminoethyl)acetamide (131.34 mg, 1.29 mmol, 123.21 µL, 10 eq), and HOAc (77.22 mg, 1.29 mmol, 73.61 µL, 10 eq) in MeOH (15 mL) was stirred at 60 °C for 2 hours. NaBH₃CN (24.24 mg, 385.78 µmol, 3 eq) was added to the mixture, followed by stirring at 60 °C for an additional 2 hours. The mixture was concentrated under reduced pressure and the residue was purified by prep-HPLC (column: Waters Xbridge Prep OBD C18 150 * 40 mm * 10 µm; mobile phase: [water (NH₄HCO₃)-ACN]; gradient: 30%-60% B over 15 min) to afford Compound 30 (26 mg, 27.12% yield) as a white solid.

LCMS m/z 708.4 [M+1]⁺.

¹H NMR (400 MHz, METHANOL-d4) δ = 8.88 (d, J = 1.9 Hz, 1H), 8.39 (dd, J = 1.5, 8.3 Hz, 1H), 8.22 - 8.10 (m, 2H), 7.94 (d, J = 5.9 Hz, 1H), 7.43 (t, J = 7.9 Hz, 1H), 7.34 (t, J = 7.9 Hz, 1H), 7.14 (dd, J = 1.5, 7.6 Hz, 1H), 7.08 (d, J = 5.9 Hz, 1H), 7.00 (d, J = 7.4 Hz, 1H), 6.87 (s, 1H), 4.45 - 4.33 (m, 1H), 4.27 - 4.15 (m, 2H), 4.02 (dd, J = 5.3, 7.4 Hz, 1H), 3.96 - 3.81 (m, 2H), 2.89 - 2.78 (m, 4H), 2.63 - 2.52 (m, 2H), 2.37 - 2.16 (m, 3H), 2.14 (s, 3H), 2.10 - 2.01 (m, 1H), 1.96 (s, 3H), 1.87 - 1.70 (m, 2H).

### 12-4. Preparation of compound 31, N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

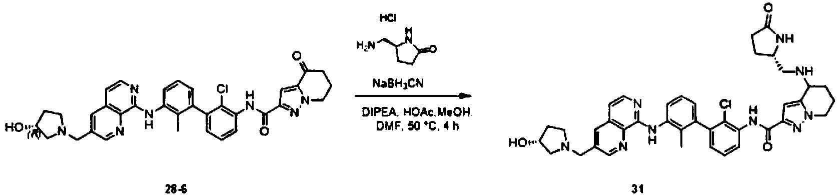

To asolution of N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (100 mg, 160.74 µmol, 1 eq), (5S)-5-(aminomethyl)pyrrolidin-2-one (183.48 mg, 1.61 mmol, 297.04 µL, 10 eq, HCl), and DIPEA (207.74 mg, 1.61 mmol, 279.98 µL, 10 eq) in MeOH (15 mL)/DMF (3 mL), HOAc (193.05 mg, 3.21 mmol, 184.03 µL, 20 eq) was added, and stirred at 50 °C for 2 hours. NaBH₃CN (50.51 mg, 803.71 µmol, 5 eq) was then added, and the reaction mixture was stirred at 50 °C for an additional 2 hours. The mixture was concentrated under reduced pressure, and the residue was purified by prep-HPLC (column: Waters Xbridge 150 * 25 mm * 5 µm; mobile phase: [water (NH₄HCO₃)-ACN]; gradient: 35%-65% B over 9 min) to afford Compound 31 (32 mg, 26.2% yield) as a white solid.

LCMS m/z 720.3 [M+1]⁺.

¹H NMR (400 MHz, DMSO-d6) δ = 9.54 (br s, 1H), 9.30 (br s, 1H), 8.85 (br s, 1H), 8.49 (br d, J = 7.4 Hz, 1H), 8.27 (br d, J = 7.1 Hz, 1H), 8.17 (br s, 1H), 8.05 (br d, J = 4.6 Hz, 1H), 7.71 (br d, J = 19.3 Hz, 1H), 7.54 - 7.45 (m, 1H), 7.40 - 7.27 (m, 1H), 7.24 - 7.07 (m, 2H), 6.97 - 6.78 (m, 2H), 4.74 (br s, 1H), 4.28 - 4.07 (m, 3H), 3.96 - 3.73 (m, 3H), 3.58 (br s, 1H), 2.72 (br d, J = 6.1 Hz, 1H), 2.63 (br dd, J = 7.4, 14.1 Hz, 3H), 2.50 (br s, 3H), 2.37 (br d, J = 7.4 Hz, 1H), 2.27 (br s, 2H), 2.11 (br s, 4H), 2.05 - 1.86 (m, 3H), 1.79 - 1.48 (m, 3H).

### 12-5. Preparation of compound 32, 2-((2-((2-chloro-3'-((3-(((R)-3-hydroxypyrrolidin-1-yl)methyl)-1,7-naphthyridin-8-yl)amino)-2'-methyl-[1,1'-biphenyl]-3-yl)carbamoyl)-4,5,6,7-tetrahydropyrazolo[1,5-a]pyridin-4-yl)amino)acetic acid

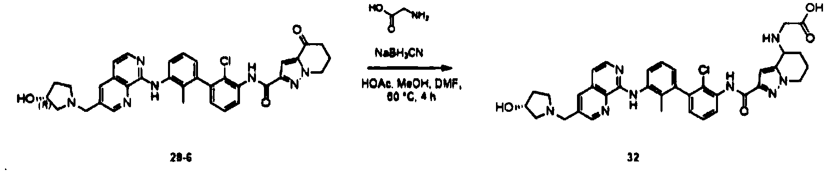

To a solution of N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (100 mg, 160.74 µmol, 1 eq) and 2-aminoacetic acid (120.66 mg, 1.61 mmol, 297.04 µL, 10 eq) in MeOH (5 mL)/DMF (1 mL), HOAc (193.05 mg, 3.21 mmol, 184.03 µL, 20 eq) was added, and stirred at 60 °C for 2 hours. NaBH₃CN (50.51 mg, 803.71 µmol, 5 eq) was then added, and the reaction mixture was stirred at 60 °C for an additional 2 hours. The mixture was concentrated under reduced pressure, and the residue was purified by prep-HPLC (column: Waters Xbridge Prep OBD C18, 150 * 40 mm * 10 µm; mobile phase: [water (NH₄HCO₃)-ACN]; gradient: 12%-42% B over 15 min) to afford Compound 32 (33 mg, 28.6% yield) as a white solid.

LCMS m/z 681.3 [M+1]⁺.

¹H NMR (400 MHz, DMSO-d6) δ = 9.57 (s, 1H), 9.30 (s, 1H), 8.87 (d, J = 1.9 Hz, 1H), 8.48 (d, J = 8.0 Hz, 1H), 8.30 - 8.22 (m, 1H), 8.18 (d, J = 1.8 Hz, 1H), 8.06 (d, J = 5.8 Hz, 1H), 7.47 (t, J = 7.9 Hz, 1H), 7.34 (t, J = 7.9 Hz, 1H), 7.23 - 7.10 (m, 2H), 6.91 (d, J = 7.5 Hz, 1H), 6.82 (s, 1H), 4.26 - 4.19 (m, 1H), 4.15 (br t, J = 6.1 Hz, 2H), 4.04 (br t, J = 6.1 Hz, 1H), 3.81 (br d, J = 10.6 Hz, 2H), 3.31 (s, 2H), 3.17 (s, 3H), 2.76 - 2.63 (m, 2H), 2.38 (dd, J = 3.6, 9.6 Hz, 1H), 2.27 - 2.16 (m, 1H), 2.14 - 2.07 (m, 3H), 2.05 - 1.86 (m, 3H), 1.82 - 1.49 (m, 2H).

### 12-6. Preparation of compound 33, (3R)-1-(2-((2-chloro-3'-((3-(((R)-3-hydroxypyrrolidin-1-yl)methyl)-1,7-naphthyridin-8-yl)amino)-2'-methyl-[1,1'-biphenyl]-3-yl)carbamoyl)-4,5,6,7-tetrahydropyrazolo[1,5-a]pyridin-4-yl)pyrrolidine-3-carboxylic acid

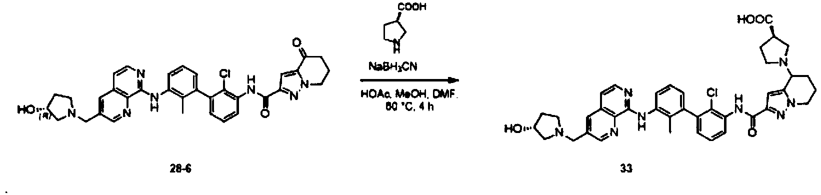

To a solution of N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (100 mg, 160.74 µmol, 1 eq) and (3R)-pyrrolidine-3-carboxylic acid (185.06 mg, 1.61 mmol, 10 eq) in MeOH (15 mL) and DMF (1 mL), HOAc (193.05 mg, 3.21 mmol, 184.03 µL, 20 eq) was added, and stirred at 60 °C for 2 hours. NaBH₃CN (50.51 mg, 803.71 µmol, 5 eq) was then added, and the reaction mixture was stirred at 60 °C for an additional 2 hours. The mixture was concentrated under reduced pressure, and the residue was purified by prep-HPLC (column: Waters Xbridge Prep OBD C18, 150 * 40 mm * 10 µm; mobile phase: [water (NH₄HCO₃)-ACN]; gradient: 12%-42% B over 15 min) to afford Compound 33 (28 mg, 22.9% yield) as a white solid.

LCMS m/z 721.2 [M+1]⁺.

¹H NMR (400 MHz, DMSO-d6) δ = 9.56 (s, 1H), 9.30 (s, 1H), 8.86 (s, 1H), 8.48 (br d, J = 8.0 Hz, 1H), 8.32 - 8.23 (m, 1H), 8.17 (s, 1H), 8.06 (d, J = 5.8 Hz, 1H), 7.46 (t, J = 7.9 Hz, 1H), 7.34 (br t, J = 7.8 Hz, 1H), 7.21 - 7.10 (m, 2H), 6.97 - 6.88 (m, 1H), 6.73 (d, J = 8.8 Hz, 1H), 4.81 - 4.67 (m, 2H), 4.26 - 4.15 (m, 3H), 3.80 (br d, J = 10.4 Hz, 3H), 3.17 (d, J = 4.8 Hz, 4H), 2.90 - 2.72 (m, 2H), 2.70 - 2.58 (m, 2H), 2.46 - 2.34 (m, 2H), 2.23 (br s, 1H), 2.11 (s, 3H), 1.88 (br s, 2H), 1.57 (br d, J = 3.4 Hz, 2H).

### 12-6. Preparation of compound 34, N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-(4-hydroxypiperidin-1-yl)-4,5,6,7-tetrahydropyrazolo[1,5-a]pyridine-2-carboxamide

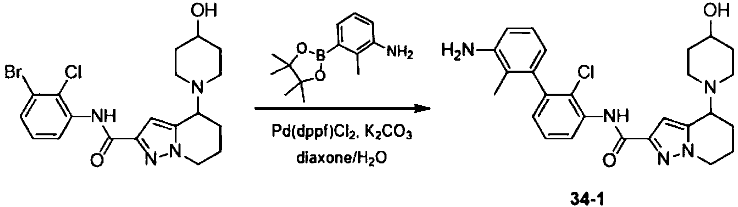

A mixture of N-(3-bromo-2-chloro-phenyl)-4-(4-hydroxy-1-piperidyl)-4,5,6,7-tetrahydropyrazolo[1,5-a]pyridine-2-carboxamide (150 mg, 330.57 µmol, 1 eq), 2-methyl-3-(4,4,5,5-tetramethyl-1,3,2-dioxaborolan-2-yl)aniline (115.59 mg, 495.86 µmol, 1.5 eq), K₂CO₃ (137.06 mg, 991.71 µmol, 3 eq), and Pd(dppf)Cl₂ (24.19 mg, 33.06 µmol, 0.1 eq) in dioxane (10 mL)/H₂O (4 mL) was stirred under a nitrogen atmosphere at 90 °C for 12 hours. After completion, the reaction mixture was concentrated under reduced pressure, and the residue was purified by column chromatography (SiO₂, DCM/MeOH = 10/1) to afford Compound 34-1 (156 mg, 97.3% yield) as a black solid.

LCMS m/z 480.3 [M+1]⁺.

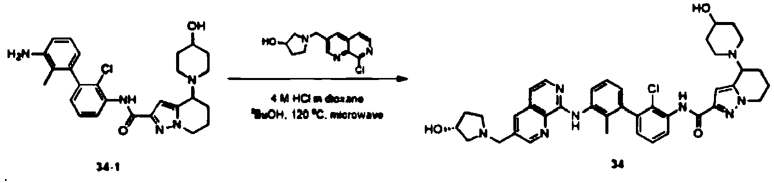

To a solution of N-[3-(3-amino-2-methylphenyl)-2-chlorophenyl]-4-(4-hydroxy-1-piperidyl)-4,5,6,7-tetrahydropyrazolo[1,5-a]pyridine-2-carboxamide (156 mg, 325.00 µmol, 1 eq) in t-BuOH (10 mL), (3R)-1-[(8-chloro-1,7-naphthyridin-3-yl)methyl]pyrrolidin-3-ol (257.13 mg, 975.00 µmol, 3 eq) and 4 M HCl (in dioxane (0.1 mL)) was added. The resulting mixture was heated at 120 °C for 8 hours in a microwave reactor. After cooling, the mixture was concentrated under reduced pressure, and the residue was purified by reversed-phase HPLC (column: Waters Xbridge, 150 * 25 mm * 5 µm; mobile phase: [water (NH₄HCO₃)-ACN]; gradient: 40%-70% B over 9 min) to give compound 34 (85 mg, 36.6% yield) as a yellow solid.

LCMS m/z 707.4 [M+1]⁺.

¹H NMR (400 MHz, DMSO-d6) δ ppm 1.34 - 1.46 (m, 2 H) 1.54 - 1.61 (m, 1 H) 1.72 (br s, 2 H) 2.02 (br dd, J=12.76, 6.88 Hz, 2 H) 2.11 (s, 3 H) 2.28 (br d, J=9.01 Hz, 1 H) 2.37 (br dd, J=9.63, 3.75 Hz, 2 H) 2.62 - 2.67 (m, 2 H) 2.74 (br dd, J=9.51, 6.38 Hz, 2 H) 3.46 (br d, J=4.88 Hz, 1 H) 3.75 - 3.87 (m, 2 H) 3.96 (br dd, J=10.44, 5.32 Hz, 1 H) 4.05 (br s, 1 H) 4.22 (br s, 2 H) 4.57 (d, J=4.13 Hz, 1 H) 4.74 (d, J=4.63 Hz, 1 H) 6.65 (s, 1 H) 6.92 (d, J=7.38 Hz, 1 H) 7.13 (d, J=7.63 Hz, 1 H) 7.19 (d, J=5.88 Hz, 1 H) 7.34 (t, J=7.88 Hz, 1 H) 7.47 (t, J=7.88 Hz, 1 H) 8.06 (d, J=5.75 Hz, 1 H) 8.19 (s, 1 H) 8.23 (t, J=7.86 Hz, 1 H) 8.48 (d, J=8.13 Hz, 1 H) 8.87 (d, J=2.00 Hz, 1 H) 9.31 (s, 1 H) 9.56 (d, J=3.88 Hz, 1 H).

### 12-7. Preparation of compound 35, 1-(2-((2-chloro-3'-((3-(((R)-3-hydroxypyrrolidin-1-yl)methyl)-1,7-naphthyridin-8-yl)amino)-2'-methyl-[1,1'-biphenyl]-3-yl)carbamoyl)-4,5,6,7-tetrahydropyrazolo[1,5-a]pyridin-4-yl)piperidine-4-carboxylic acid

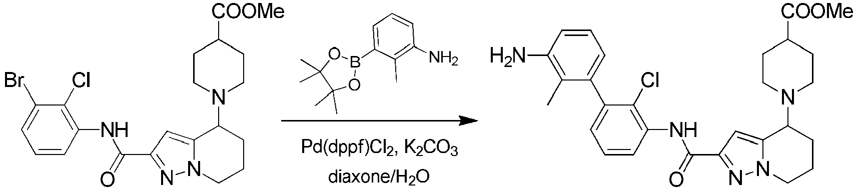

A mixture of methyl 1-[2-[(3-bromo-2-chlorophenyl)carbamoyl]-4,5,6,7-tetrahydropyrazolo[1,5-a]pyridin-4-yl]piperidine-4-carboxylate (200 mg, 403.39 µmol, 1 eq), 2-methyl-3-(4,4,5,5-tetramethyl-1,3,2-dioxaborolan-2-yl)aniline (141.05 mg, 605.09 µmol, 1.5 eq), Pd(dppf)Cl₂ (29.52 mg, 40.34 µmol, 0.1 eq), and K₂CO₃ (167.25 mg, 1.21 mmol, 3 eq) in dioxane (4 mL)/H₂O (1 mL) was stirred under a nitrogen atmosphere at 90 °C for 12 hours. After completion, the reaction mixture was cooled to room temperature and filtered. The filtrate was concentrated under reduced pressure, and the residue was purified by prep-TLC (SiO₂, DCM/MeOH = 20:1) to afford methyl 1-[2-[[3-(3-amino-2-methylphenyl)-2-chlorophenyl]carbamoyl]-4,5,6,7-tetrahydropyrazolo[1,5-a]pyridin-4-yl]piperidine-4-carboxylate (150 mg, 71.2% yield) as a white solid.

LCMS m/z 522.3 [M+1]⁺.

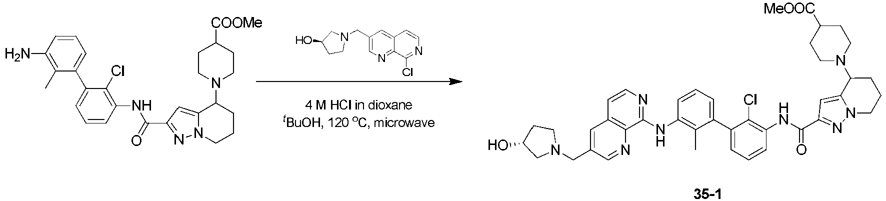

A mixture of (3R)-1-[(8-chloro-1 ,7-naphthyridin-3-yl)methyl]pyrrolidin-3-ol (197.02 mg, 747.07 µmol, 3 eq), methyl 1-[2-[[3-(3-amino-2-methylphenyl)-2-chlorophenyl]carbamoyl]-4,5,6,7-tetrahydropyrazolo[1,5-a]pyridin-4-yl]piperidine-4-carboxylate (130 mg, 249.02 µmol, 1 eq), and HCl in dioxane (4 M, 0.5 mL, 8.03 eq) in t-BuOH (5 mL) was stirred in a microwave reactor at 120 °C for 12 hours. After completion, the reaction mixture was cooled to room temperature, and the precipitated solid was collected by filtration and washed with ethyl acetate (30 mL) to afford Compound 35-1, which was used in the next step without further purification.

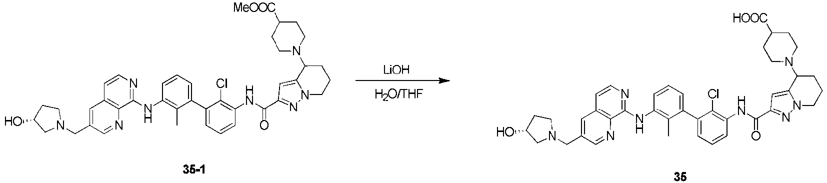

A mixture of (3R)-1-[(8-chloro-1,7-naphthyridin-3-yl)methyl]pyrrolidin-3-ol (197.02 mg, 747.07 µmol, 3 eq), methyl 1-[2-[[3-(3-amino-2-methylphenyl)-2-chlorophenyl]carbamoyl]-4,5,6,7-tetrahydropyrazolo[1,5-a]pyridin-4-yl]piperidine-4-carboxylate (130 mg, 249.02 µmol, 1 eq), and HCl in dioxane (4 M, 0.5 mL, 8.03 eq) in t-BuOH (5 mL) was stirred in a microwave reactor at 120 °C for 12 hours. After cooling the mixture to room temperature, the precipitated solid was collected by filtration and washed with ethyl acetate (30 mL) to afford Compound 35-1, which was used in the next reaction without further purification.

### 12-8. Preparation of compound 36, N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-4-hydroxy-4,5,6,7-tetrahydropyrazolo[1,5-a]pyridine-2-carboxamide

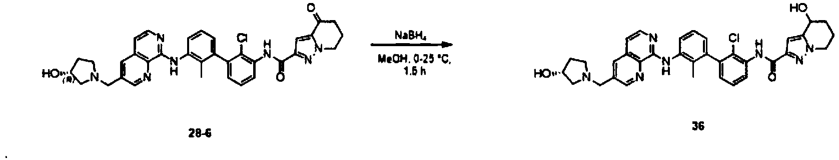

A solution of N-[2-chloro-3-[3-[[3-[[(3R)-3-hydroxypyrrolidin-1-yl]methyl]-1,7-naphthyridin-8-yl]amino]-2-methylphenyl]phenyl]-4-oxo-6,7-dihydro-5H-pyrazolo[1,5-a]pyridine-2-carboxamide (100 mg, 160.74 µmol, 1 eq) in MeOH (6 mL) was treated with NaBH₄ (20.20 mg, 321.48 µmol, 2 eq) at 0 °C, and the mixture was stirred at room temperature for 2 hours. After completion of the reaction, the mixture was concentrated under reduced pressure, and the residue was purified by prep-HPLC (column: Waters Xbridge 150 * 25 mm * 5 µm; mobile phase: [water (NH₄HCO₃)-ACN]; gradient: 38%-68% B over 9 min) to afford Compound 36 (42 mg, 39.7% yield) as a white solid.

LCMS m/z 624.3 [M+1]⁺.

¹H NMR (400 MHz, DMSO-d6) δ = 9.56 (s, 1H), 9.30 (s, 1H), 8.86 (d, J = 1.9 Hz, 1H), 8.48 (d, J = 8.0 Hz, 1H), 8.26 (d, J = 8.3 Hz, 1H), 8.18 (d, J = 1.8 Hz, 1H), 8.06 (d, J = 5.8 Hz, 1H), 7.47 (t, J = 7.9 Hz, 1H), 7.34 (t, J = 7.9 Hz, 1H), 7.24 - 7.11 (m, 2H), 6.91 (d, J = 7.4 Hz, 1H), 6.74 (s, 1H), 5.61 (dd, J = 1.9, 5.5 Hz, 1H), 4.82 - 4.66 (m, 2H), 4.31 - 4.07 (m, 3H), 3.91 - 3.71 (m, 2H), 2.77 - 2.61 (m, 2H), 2.38 (dd, J = 3.6, 9.6 Hz, 1H), 2.26 - 2.15 (m, 1H), 2.11 (s, 3H), 2.05 - 1.89 (m, 3H), 1.79 - 1.51 (m, 2H).

### [Preparation Example 13]

### Preparation of Compounds 37 to 44

### 13-1. Preparation of Compound 37, N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4-((2-hydroxyethyl)amino)-4,5,6,7-tetrahydropyrazolo[1,5-a]pyridine-2-carboxamide

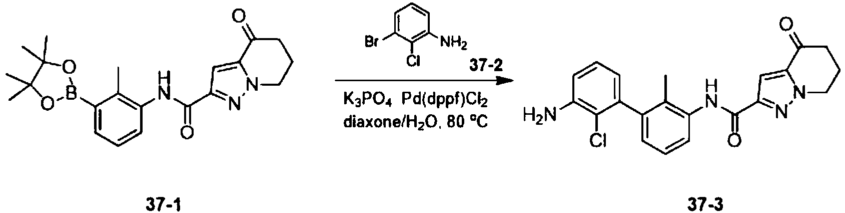

A mixture of N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (2.82 g, 7.14 mmol, 1 eq), 3-bromo-2-chloroaniline (2.95 g, 14.27 mmol, 2 eq), K₃PO₄ (4.54 g, 21.41 mmol, 3 eq), and Pd(dppf)Cl₂ (261.04 mg, 356.76 µmol, 0.05 eq) in dioxane (20 mL)/ H₂O (2 mL) was stirred under a nitrogen atmosphere at 80 °C for 8 hours. After completion of the reaction, the mixture was concentrated under reduced pressure, and the residue was purified by column chromatography (SiO₂, Petroleum ether/Ethyl acetate = 1/1) to afford Compound 37-3 (2.2 g, 69.0% yield) as a brown solid.

LCMS m/z 395.2 [M+1]⁺.

¹H NMR (400 MHz, DMSO-d6) δ ppm 1.94 - 1.98 (m, 3 H) 2.31 - 2.36 (m, 2 H) 2.69 - 2.73 (m, 2 H) 4.42 - 4.48 (m, 2 H) 5.40 - 5.45 (m, 2 H) 6.38 - 6.43 (m, 1 H) 6.79 - 6.85 (m, 1 H) 6.96 - 7.01 (m, 1 H) 7.04 - 7.10 (m, 1 H) 7.21 - 7.29 (m, 2 H) 7.42 - 7.49 (m, 1 H) 9.79 - 9.85 (m, 1 H).

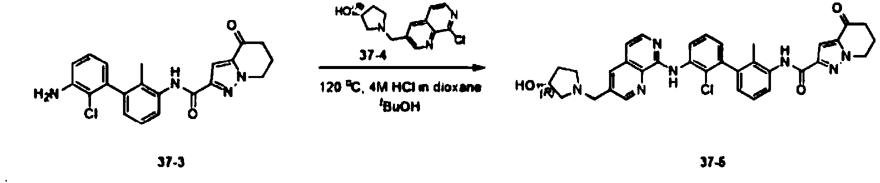

To a solution of (3R)-1-[(8-chloro-1,7-naphthyridin-3-yl)methyl]pyrrolidin-3-ol (500.92 mg, 1.90 mmol, 2.5 eq) and N-[3-(3-amino-2-chloro-phenyl)-2-methyl-phenyl]-4-oxo-6,7-dihydro-5H-pyrazolo[1,5-a]pyridine-2-carboxamide (300 mg, 759.78 µmol, 1 eq) in t-BuOH (10 mL), HCl/dioxane (4 M, 379.89 µL, 2 eq) was added, and the mixture was stirred at 120 °C for 12 hours. After completion of the reaction, the mixture was concentrated under reduced pressure to afford Compound 37-5 (648 mg, 86.1% yield) as a brown solid, which was used in the next step without further purification.

LCMS m/z 622.3 [M+1]⁺.

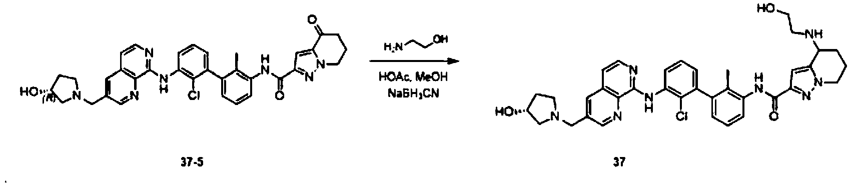

To a solution of N-[3-[2-chloro-3-[[3-[[(3R)-3-hydroxypyrrolidin-1-yl]methyl]-1,7-naphthyridin-8-yl]amino]phenyl]-2-methyl-phenyl]-4-oxo-6,7-dihydro-5H-pyrazolo[1,5-a]pyridine-2-carboxamide (100 mg, 160.74 µmol, 1 eq) and 2-aminoethanol (98.19 mg, 1.61 mmol, 97.21 µL, 10 eq) in MeOH (4 mL), HOAc (96.53 mg, 1.61 mmol, 91.93 µL, 10 eq) and NaBH₃CN (50.51 mg, 803.71 µmol, 5 eq) were added, and the mixture was stirred at 60 °C for 4 hours. After completion of the reaction, the mixture was concentrated under reduced pressure, and the residue was purified by reversed-phase HPLC (column: Waters Xbridge BEH C18 150 * 25 mm * 5 µm; mobile phase: [water (NH₄HCO₃)-ACN]; B%: 42%-72%, 9 min) to afford Compound 37 (23 mg, 20.7% yield) as a white solid.

LCMS m/z 667.4 [M+1]⁺.

¹H NMR (400 MHz, DMSO-d6) δ ppm 1.56 - 1.58 (m, 1 H) 1.70 - 2.01 (m, 1 H) 2.20 - 2.32 (M, 3 H) 2.32 - 2.33 (m, 2 H) 2.33 - 2.39 (m, 3 H) 2.66 - 2.69 (m, 5 H) 3.47 - 3.48 (m, 2 H) 3.81 - 3.87 (m, 2 H) 3.89 - 4.00 (m, 2 H) 4.15 - 4.50 (m, 1 H) 4.71 - 4.73 (m, 1 H) 6.73 (d, J=7.38 Hz, 1 H) 6.94 - 6.96 (m, 1 H) 7.05 - 7.07 (d, J=7.75 Hz, 1 H) 7.30 - 7.34 (m, 2 H) 7.46 - 7.49 (m, 1 H) 7.61 - 7.63 (m, 1 H) 8.18 - 8.20 (br s, 1 H) 8.25 (s, 1 H) 8.92 (m, 1 H) 9.05 - 9.11 (m, 1 H) 9.54 (br s, 1 H) 9.91 (s, 1 H).

### 13-2. Preparation of Compound 38, N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4-((R)-3-hydroxypyrrolidin-1-yl)-4,5,6,7-tetrahydropyrazolo[1,5-a]pyridine-2-carboxamide

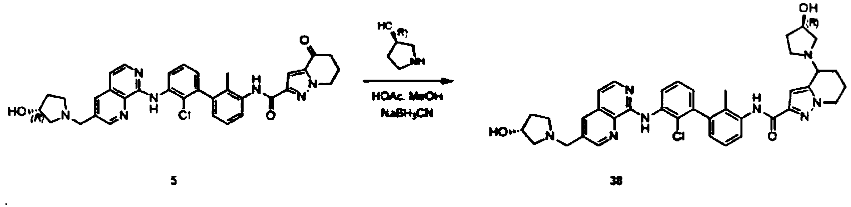

To a solution of N-[3-[2-chloro-3-[[3-[[(3R)-3-hydroxypyrrolidin-1-yl]methyl]-1,7-naphthyridin-8-yl]amino]phenyl]-2-methylphenyl]-4-oxo-6,7-dihydro-5H-pyrazolo[1,5-a]pyridine-2-carboxamide (400 mg, 642.97 µmol, 1 eq) and (3R)-pyrrolidin-3-ol (560.15 mg, 6.43 mmol, 534.50 µL, 10 eq) in MeOH (10 mL), AcOH (77.22 mg, 1.29 mmol, 73.62 µL, 2 eq), and NaBH₃CN (202.03 mg, 3.21 mmol, 5 eq) were added. The reaction mixture was stirred at 60 °C for 12 hours. After completion of the reaction, the mixture was concentrated under reduced pressure, and the residue was purified by reversed-phase HPLC ((0.1% HCl condition); column: Welch Xtimate C18, 150 * 25 mm * 5 µm; mobile phase: [water (HCl)-ACN]; gradient: 5%-35% B over 8 min) to afford Compound 38 (34 mg, 7.42% yield) as a brown solid.

LCMS m/z 693.5 [M+1]⁺.

¹H NMR (400 MHz, DMSO-d6) δ ppm 1.88 - 1.94 (m, 1 H) 2.04 (s, 4 H) 2.18 - 2.30 (m, 2 H) 3.00 - 3.13 (m, 1 H) 3.27 - 3.37 (m, 2 H) 3.48 (br s, 1 H) 3.56 - 3.61 (m, 1 H) 4.19 - 4.30 (m, 3 H) 4.43 (s, 3 H) 4.47 (s, 3 H) 4.67 - 4.78 (m, 5 H) 4.87 (br dd, J=12.63, 6.25 Hz, 2 H) 7.12 (br d, J=7.13 Hz, 1 H) 7.19 - 7.28 (m, 1 H) 7.30 - 7.39 (m, 2 H) 7.53 - 7.62 (m, 2 H) 8.00 (br s, 1 H) 8.36 (br s, 1 H) 8.73 (br d, J=19.51 Hz, 1 H) 9.27 - 9.38 (m, 1 H) 9.70 - 9.77 (m, 1 H) 11.23 - 11.59 (m, 1 H) 11.88 - 12.18 (m, 1 H)

### 13-3. Preparation of Compound 39, 4-((2-acetamidoethyl)amino)-N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4,5,6,7-tetrahydropyrazolo[1,5-a]pyridine-2-carboxamide

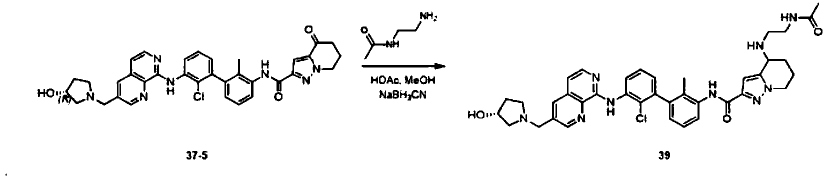

To a solution of N-[3-[2-chloro-3-[[3-[[(3R)-3-hydroxypyrrolidin-1-yl]methyl]-1,7-naphthyridin-8-yl]amino]phenyl]-2-methylphenyl]-4-oxo-6,7-dihydro-5H-pyrazolo[1,5-a]pyridine-2-carboxamide (200 mg, 321.48 µmol, 1 eq) and N-(2-aminoethyl)acetamide (328.35 mg, 3.21 mmol, 308.02 µL, 10 eq) in MeOH (10 mL), AcOH (38.61 mg, 642.97 µmol, 36.81 µL, 2 eq) and NaBH₃CN (101.01 mg, 1.61 mmol, 5 eq) were added. The reaction mixture was stirred at 60 °C for 12 hours. After completion, the mixture was concentrated under reduced pressure, and the residue was purified by reversed-phase HPLC ((0.1% HCl condition); column: Welch Xtimate C18, 150 * 25 mm * 5 µm; mobile phase: [water (HCl)-ACN]; gradient: 6%-36% B over 8 min) to afford Compound 39 (47 mg, 20.5% yield) as a brown solid.

LCMS m/z 708.4 [M+1]⁺.

¹H NMR (400 MHz, DMSO-d6) δ ppm 1.84 (s, 3 H) 2.03 (s, 3 H) 2.20 - 2.27 (m, 1 H) 2.30 - 2.38 (m, 1 H) 3.08 (br d, J=5.88 Hz, 3 H) 3.16 (s, 2 H) 3.40 - 3.49 (m, 1 H) 3.40 - 3.50 (m, 3 H) 3.55 - 3.63 (m, 1 H) 4.17 - 4.23 (m, 5 H) 4.40 - 4.49 (m, 3 H) 4.69 (br dd, J=13.38, 5.38 Hz, 3 H) 7.11 (d, J=7.25 Hz, 1 H) 7.15 (s, 1 H) 7.16 - 7.24 (m, 1 H) 7.32 (t, J=7.82 Hz, 1 H) 7.34 - 7.39 (m, 1 H) 7.54 - 7.60 (m, 2 H) 7.97 - 8.07 (m, 1 H) 8.33 (br t, J=5.13 Hz, 1 H) 8.65 - 8.75 (m, 1 H) 9.25 - 9.36 (m, 1 H) 9.72 (s, 2 H)

### 13-4. Preparation of Compound 40, N-(2'-chloro-3'-((3-(((R)-3-hydroxypyrrolidin-1-yl)methyl)-1,7-naphthyridin-8-yl)amino)-2-methyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

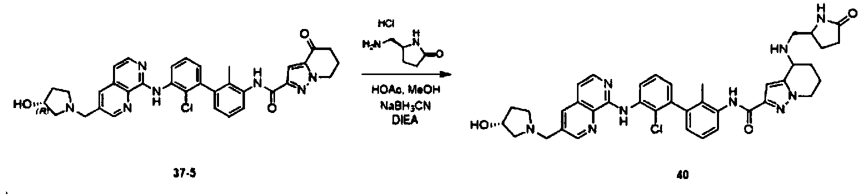

To a solution of N-[3-[2-chloro-3-[[3-[[(3R)-3-hydroxypyrrolidin-1-yl]methyl]-1,7-naphthyridin-8-yl]amino]phenyl]-2-methylphenyl]-4-oxo-6,7-dihydro-5H-pyrazolo[1,5-a]pyridine-2-carboxamide (100 mg, 160.74 µmol, 1 eq) and (5S)-5-(aminomethyl)pyrrolidin-2-one (183.48 mg, 1.61 mmol, 10 eq, HCl) in MeOH (10 mL), AcOH (19.31 mg, 321.48 µmol, 18.40 µL, 2 eq), DIPEA (207.75 mg, 1.61 mmol, 279.98 µL, 10 eq), and NaBH₃CN (50.51 mg, 803.71 µmol, 5 eq) were added. The reaction mixture was stirred at 60 °C for 12 hours. After completion, the mixture was concentrated under reduced pressure, and the residue was purified by reversed-phase HPLC ((0.1% NH₃·H₂O condition); column: Waters Xbridge Prep OBD C18, 150 * 40 mm, 10 µm; mobile phase: [water (NH₄HCO₃)-ACN]; gradient: 30%-60% B over 15 min) to afford Compound 40 (18 mg, 15.27% yield) as a white solid.

LCMS m/z 720.4 [M+1]⁺.

¹H NMR (400 MHz, DMSO-d6) δ ppm 1.52 - 1.62 (m, 1 H) 1.63 - 1.77 (m, 2 H) 1.87 - 1.96 (m, 1 H) 1.98 - 2.06 (m, 5 H) 2.06 - 2.11 (m, 2 H) 2.35 - 2.41 (m, 1 H) 2.58 - 2.70 (m, 3 H) 2.71 - 2.77 (m, 1 H) 3.53 - 3.63 (m, 1 H) 3.80 - 3.88 (m, 2 H) 4.07 - 4.12 (m, 7 H) 4.18 - 4.26 (m, 1 H) 4.70 - 4.75 (m, 1 H) 6.74 - 6.80 (m, 1 H) 6.93 - 6.98 (m, 1 H) 7.03 - 7.08 (m, 1 H) 7.27 - 7.35 (m, 2 H) 7.45 - 7.52 (m, 1 H) 7.60 - 7.73 (m, 2 H) 8.16 - 8.21 (m, 1 H) 8.23 - 8.27 (m, 1 H) 8.90 - 8.94 (m, 1 H) 9.07 - 9.13 (m, 1 H) 9.50 - 9.56 (m, 1 H) 9.88 - 9.95 (m, 1 H)

### 13-5. Preparation of Compound 41, 2-((2-((2'-chloro-3'-((3-(((R)-3-hydroxypyrrolidin-1-yl)methyl)-1,7-naphthyridin-8-yl)amino)-2-methyl-[1,1'-biphenyl]-3-yl)carbamoyl)-4,5,6,7-tetrahydropyrazolo[1,5-a]pyridin-4-yl)amino)acetic acid

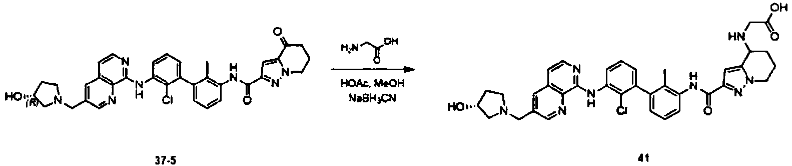

To a solution of N-[3-[2-chloro-3-[[3-[[(3R)-3-hydroxypyrrolidin-1-yl]methyl]-1,7-naphthyridin-8-yl]amino]phenyl]-2-methylphenyl]-4-oxo-6,7-dihydro-5H-pyrazolo[1,5-a]pyridine-2-carboxamide (150 mg, 241.11 µmol, 1 eq) and 2-aminoacetic acid (180.99 mg, 2.41 mmol, 10 eq) in MeOH (10 mL), AcOH (28.96 mg, 482.23 µmol, 27.61 µL, 2 eq), and NaBH₃CN (75.76 mg, 1.21 mmol, 5 eq) were added. The reaction mixture was stirred at 60 °C for 12 hours. After completion, the mixture was concentrated under reduced pressure, and the residue was purified by reversed-phase HPLC ((0.1% NH₃·H₂O condition); column: Waters Xbridge Prep OBD C18, 150 * 40 mm, 10 µm; mobile phase: [water (NH₄HCO₃)-ACN]; gradient: 12%-42% B over 15 min) to afford Compound 41 (14 mg, 8.21% yield) as a white solid.

LCMS m/z 681.3 [M+1]⁺.

¹H NMR (400 MHz, DMSO-d6) δ ppm 1.54 - 1.62 (m, 1 H) 1.71 - 1.80 (m, 1 H) 1.83 - 1.97 (m, 2 H) 1.99 - 2.02 (m, 4 H) 2.04 (br d, J=6.97 Hz, 2 H) 2.11 - 2.27 (m, 2 H) 2.30 - 2.36 (m, 1 H) 2.38 - 2.44 (m, 1 H) 2.65 - 2.72 (m, 1 H) 2.73 - 2.80 (m, 1 H) 3.79 - 3.91 (m, 3 H) 4.03 - 4.10 (m, 2 H) 4.12 - 4.17 (m, 2 H) 4.19 - 4.26 (m, 1 H) 6.76 - 6.79 (m, 1 H) 6.94 - 6.98 (m, 1 H) 7.04 - 7.08 (m, 1 H) 7.27 - 7.35 (m, 2 H) 7.46 - 7.52 (m, 1 H) 7.58 - 7.64 (m, 1 H) 8.16 - 8.21 (m, 1 H) 8.24 - 8.28 (m, 1 H) 8.90 - 8.94 (m, 1 H) 9.07 - 9.12 (m, 1 H) 9.56 (br d, J=2.45 Hz, 1 H) 9.89 - 9.92 (m, 1 H).

### 13-6. Preparation of Compound 42, (3R)-1-(2-((2'-chloro-3'-((3-(((R)-3-hydroxypyrrolidin-1-yl)methyl)-1,7-naphthyridin-8-yl)amino)-2-methyl-[1,1'-biphenyl]-3-yl)carbamoyl)-4,5,6,7-tetrahydropyrazolo[1,5-a]pyridin-4-yl)pyrrolidine-3-carboxylic acid

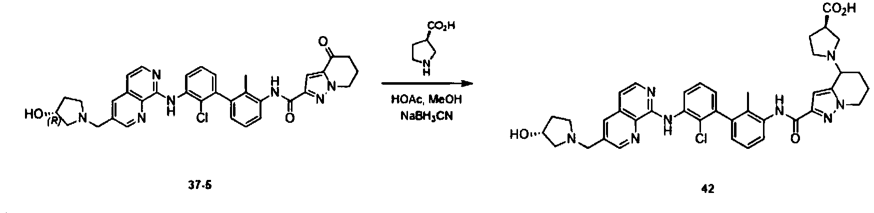

To a solution of N-[3-[2-chloro-3-[[3-[[(3R)-3-hydroxypyrrolidin-1-yl]methyl]-1,7-naphthyridin-8-yl]amino]phenyl]-2-methylphenyl]-4-oxo-6,7-dihydro-5H-pyrazolo[1,5-a]pyridine-2-carboxamide (400 mg, 642.97 µmol, 1 eq) and (3R)-pyrrolidine-3-carboxylic acid (740.25 mg, 6.43 mmol, 10 eq) in MeOH (10 mL), AcOH (77.22 mg, 1.29 mmol, 73.62 µL, 2 eq), and NaBH₃CN (202.03 mg, 3.21 mmol, 5 eq) were added. The reaction mixture was stirred at 60 °C for 12 hours. After completion, The mixture was concentrated under reduced pressure, and the residue was purified by reversed-phase HPLC ((0.1% HCl condition); column: Welch Xtimate C18 150 * 25 mm * 5 µm; mobile phase: [water (HCl)-ACN]; gradient: 5%-35% B over 8 min) to afford Compound 42 (14 mg, 2.88% yield) as a brown solid.

LCMS m/z 721.5 [M+1]⁺.

¹H NMR (400 MHz, DMSO-d6) δ ppm 1.95 - 2.12 (m, 7 H) 2.17 - 2.37 (m, 4 H) 3.08 (br d, J=11.76 Hz, 1 H) 3.16 (s, 1 H) 3.26 - 3.37 (m, 2 H) 3.50 (br d, J=12.76 Hz, 1 H) 3.54 - 3.62 (m, 3 H) 4.19 (dt, J=12.94, 6.28 Hz, 3 H) 4.27 (br s, 1 H) 4.42 (br s, 1 H) 4.48 (br s, 1 H) 4.65 - 4.74 (m, 2 H) 4.86 (br s, 1 H) 7.11 (d, J=7.38 Hz, 1 H) 7.18 - 7.24 (m, 2 H) 7.32 (t, J=7.75 Hz, 1 H) 7.37 (t, J=6.72 Hz, 1 H) 7.53 - 7.60 (m, 2 H) 8.03 (br d, J=6.75 Hz, 1 H) 8.47 (br s, 1 H) 8.64 - 8.76 (m, 1 H) 9.23 - 9.37 (m, 1 H) 9.75 (br s, 1 H) 11.97 (br s, 1 H).

### 13-7. Preparation of Compound 43, N-(2'-chloro-3'-(5-(((R)-3-hydroxypyrrolidin-1-yl)methyl)picolinamido)-2-methyl-[1,1'-biphenyl]-3-yl)-4-(4-hydroxypiperidin-1-yl)-4,5,6,7-tetrahydropyrazolo[1,5-a]pyridine-2-carboxamide,

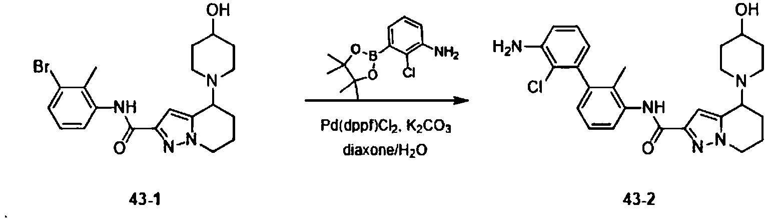

A mixture of N-(3-bromo-2-methylphenyl)-4-(4-hydroxy-1-piperidyl)-4,5,6,7-tetrahydropyrazolo[1,5-a]pyridine-2-carboxamide (150 mg, 346.15 µmol, 1 eq), 2-chloro-3-(4,4,5,5-tetramethyl-1,3,2-dioxaborolan-2-yl)aniline (131.64 mg, 519.22 µmol, 1.5 eq), Pd(dppf)Cl₂ (25.33 mg, 34.61 µmol, 0.1 eq), and K₂CO₃ (143.52 mg, 1.04 mmol, 3 eq) in dioxane (5 mL)/H₂O (0.5 mL) was stirred at 90 °C for 12 hours under a nitrogen atmosphere. After concentration under reduced pressure, the residue was purified by column chromatography (SiO₂, DCM/MeOH = 10:1) to afford Compound 43-2 (160 mg, 94.3% yield) as a black solid.

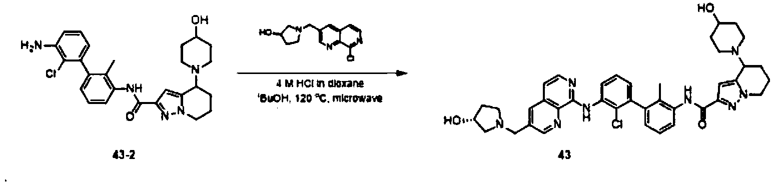

To a solution of N-[3-(3-amino-2-chloro-phenyl)-2-methyl-phenyl]-4-(4-hydroxy-1-piperidyl)-4,5,6,7-tetrahydropyrazolo[1,5-a]pyridine-2-carboxamide (160 mg, 333.33 µmol, 1 eq) in t-BuOH (10 mL), (3R)-1-[(8-chloro-1,7-naphthyridin-3-yl)methyl]pyrrolidin-3-ol (263.72 mg, 1.00 mmol, 3 eq) and 4 M HCl (in dioxane (0.1 mL)) were added, and the reaction mixture was stirred at 120 °C for 8 h in a microwave reactor. After concentration under reduced pressure, the residue was purified by reversed-phase HPLC (column: Waters XBridge, 150 * 25 mm * 5 µm; mobile phase: water (NH₄HCO₃)/ACN; gradient: 44-74% B over 9 min) to afford Compound 43 (56 mg, 23.7% yield) as a yellow solid.

LCMS m/z 707.4 [M+1]⁺.

¹H NMR (400 MHz, METHANOL-d4) δ ppm 1.55 - 1.66 (m, 2 H) 1.75 - 1.85 (m, 2 H) 1.86 - 1.93 (m, 2 H) 2.13 (s, 4 H) 2.17 - 2.22 (m, 1 H) 2.39 - 2.47 (m, 1 H) 2.56 - 2.64 (m, 3 H) 2.85 (br dd, J=9.69, 6.07 Hz, 4 H) 3.60 - 3.67 (m, 1 H) 3.86 - 3.98 (m, 2 H) 4.05 (dd, J=10.57, 5.44 Hz, 1 H) 4.09 - 4.15 (m, 1 H) 4.24 - 4.32 (m, 1 H) 4.36 - 4.42 (m, 1 H) 4.54 - 4.62 (m, 1 H) 4.83 (s, 1 H) 4.88 - 4.91 (m, 3 H) 6.85 (s, 1 H) 6.99 (d, J=7.41 Hz, 1 H) 7.12 (d, J=8.63 Hz, 1 H) 7.22 (d, J=5.88 Hz, 1 H) 7.32 - 7.37 (m, 1 H) 7.43 - 7.48 (m, 1 H) 7.73 (d, J=8.13 Hz, 1 H) 8.16 (d, J=5.88 Hz, 1 H) 8.19 (s, 1 H) 8.92 (d, J=1.88 Hz, 1 H) 9.05 (dd, J=8.25, 1.50 Hz, 1 H).

### 13-8. Preparation of Compound 44, 1-(2-((2'-chloro-3'-((3-(((R)-3-hydroxypyrrolidin-1-yl)methyl)-1,7-naphthyridin-8-yl)amino)-2-methyl-[1,1'-biphenyl]-3-yl)carbamoyl)-4,5,6,7-tetrahydropyrazolo[1,5-a]pyridin-4-yl)piperidine-4-carboxylic acid

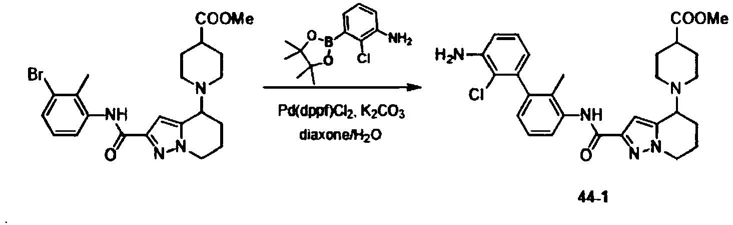

A mixture of methyl 1-[2-[(3-bromo-2-methylphenyl)carbamoyl]-4,5,6,7-tetrahydropyrazolo[1,5-a]pyridin-4-yl]piperidine-4-carboxylate (150 mg, 315.54 µmol, 1 eq), 2-chloro-3-(4,4,5,5-tetramethyl-1,3,2-dioxaborolan-2-yl)aniline (120.00 mg, 473.31 µmol, 1.5 eq), Pd(dppf)Cl₂ (23.09 mg, 31.55 µmol, 0.1 eq), and K₂CO₃ (130.83 mg, 946.62 µmol, 3 eq) in a dioxane (5 mL)/H₂O (0.5 mL) was stirred at 90 °C for 12 h under a nitrogen atmosphere. After concentration under reduced pressure, the residue was purified by column chromatography (SiO₂, DCM/MeOH = 10:1) to afford Compound 44-1 (160 mg, 95.1% yield) as a black solid.

LCMS m/z 522.2 [M+1]⁺.

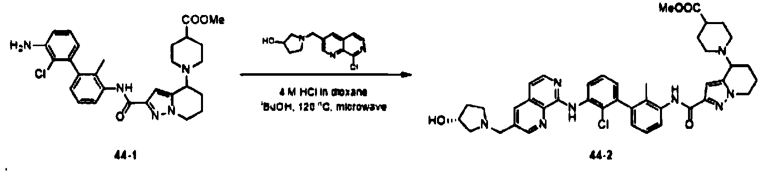

To a solution of methyl 1-[2-[[3-(3-amino-2-chloro-phenyl)-2-methylphenyl]carbamoyl]-4,5,6,7-tetrahydropyrazolo[1,5-a]pyridin-4-yl]piperidine-4-carboxylate (160 mg, 306.49 µmol, 1 eq) in t-BuOH (10 mL), (3R)-1-[(8-chloro-1,7-naphthyridin-3-yl)methyl]pyrrolidin-3-ol (242.49 mg, 919.47 µmol, 3 eq) and 4 M HCl (in dioxane (0.1 mL)) was added. The reaction mixture was stirred at 120 °C for 8 h in a microwave reactor. After concentration under reduced pressure, the residue was purified by reversed-phase HPLC (column: Phenomenex Luna C18, 150 * 25 mm * 10 µm; mobile phase: [water (TFA)-ACN]; gradient: 12-42% B over 9 min) to afford Compound 44-2 (220 mg, 95.8% yield) as a light brown solid.

LCMS m/z 749.3 [M+1]⁺.

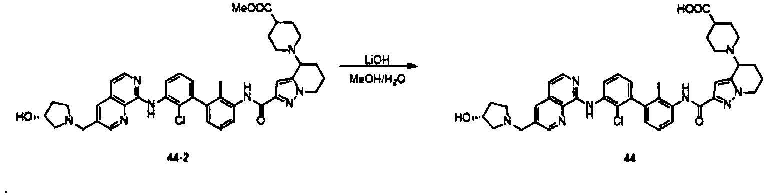

A solution of methyl 1-[2-[[3-[2-chloro-3-[[3-[[(3S)-3-hydroxypyrrolidin-1-yl]methyl]-1,7-naphthyridin-8-yl]amino]phenyl]-2-methylphenyl]carbamoyl]-4,5,6,7-tetrahydropyrazolo[1,5-a]pyridin-4-yl]piperidine-4-carboxylate (220 mg, 293.61 µmol, 1 eq) and LiOH (61.60 mg, 1.47 mmol, 5 eq) in H₂O (4 mL)/MeOH (20 mL) was stirred at room temperature for 12 h. After concentration under reduced pressure, the residue was purified by reversed-phase HPLC (column: Waters XBridge, 150 * 25 mm * 5 µm; mobile phase: water (NH₄HCO₃)/ACN; gradient: 22-52% B over 9 min) to afford Compound 44 (45 mg, 20.8% yield) as a yellow solid.

LCMS m/z 735.4 [M+1]⁺.

¹H NMR (400 MHz, DMSO-d6) δ ppm 1.67 (br d, J=12.23 Hz, 5 H) 1.81 (br t, J=11.92 Hz, 2 H) 1.86 - 1.92 (m, 1 H) 1.93 - 2.04 (m, 5 H) 2.12 - 2.20 (m, 2 H) 2.26 (br s, 1 H) 2.38 (dd, J=9.66, 3.55 Hz, 1 H) 2.42 - 2.48 (m, 1 H) 2.62 - 2.70 (m, 2 H) 2.71 - 2.79 (m, 2 H) 3.82 (br d, J=9.78 Hz, 2 H) 3.95 (br dd, J=9.72, 5.20 Hz, 1 H) 3.99 - 4.08 (m, 1 H) 4.15 - 4.26 (m, 2 H) 6.64 (s, 1 H) 6.95 (d, J=7.19 Hz, 1 H) 7.06 (d, J=7.46 Hz, 1 H) 7.27 - 7.35 (m, 2 H) 7.49 (t, J=7.89 Hz, 1 H) 7.62 (t, J=6.27 Hz, 1 H) 8.18 (d, J=5.75 Hz, 1 H) 8.24 (s, 1 H) 8.91 (d, J=1.59 Hz, 1 H) 9.09 (d, J=7.91 Hz, 1 H) 9.53 (d, J=3.30 Hz, 1 H) 9.90 (s, 1 H).

### [Preparation Example 14]

### Preparation of Compounds 45 to 51

### 14-1. Preparation of Compound 45, 4-(2-(2-((3'-(4-hydroxy-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

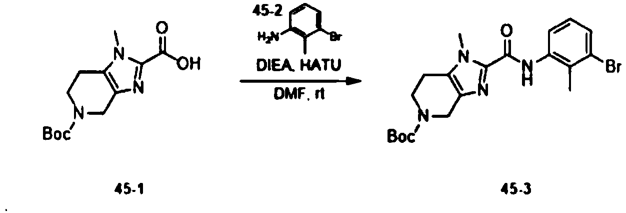

To a solution of 5-tert-Butoxycarbonyl-1-methyl-6,7-dihydro-4H-imidazo[4,5-c]pyridine-2-carboxylic acid (5.08 g, 18.06 mmol, 1 eq), and 3-bromo-2-methyl-aniline (3.36 g, 18.06 mmol, 2.23 mL, 1 eq) in DMF (50 mL), HATU (10.30 g, 27.09 mmol, 1.5 eq), and DIPEA (4.67 g, 36.12 mmol, 6.29 mL, 2 eq) were added. The reaction mixture was stirred at room temperature for 12 hours. Water (200 mL) was added to the reaction mixture, and the precipitated solid was filtered to afford Compound 45-3 (3 g, 5.88 mmol, 32.5% yield) as a pale yellow solid.

LCMS m/z 449.0 [M+1]⁺.

¹H NMR (400 MHz, chloroform-d) δ = 9.30 - 9.03 (m, 1H), 7.86 - 7.78 (m, 1H), 7.37 - 7.28 (m, 1H), 7.07 - 6.98 (m, 1H), 4.53 - 4.31 (m, 2H), 3.96 - 3.80 (m, 3H), 3.78 - 3.61 (m, 2H), 2.66 - 2.56 (m, 2H), 2.42 - 2.35 (m, 3H), 1.60 - 1.48 (m, 4H), 1.58 - 1.45 (m, 4H), 1.46 - 1.37 (m, 9H).

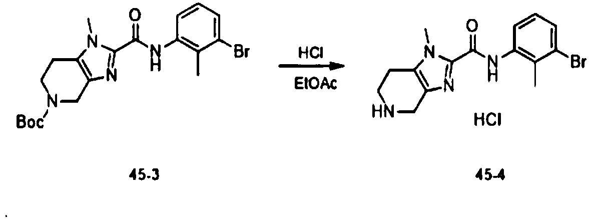

A solution of tert-butyl 2-[(3-bromo-2-methylphenyl)carbamoyl]-1-methyl-6,7-dihydro-4H-imidazo[4,5-c]pyridine-5-carboxylate (3 g, 6.68 mmol, 1 eq) in 4 M HCl/EtOAc (15 mL) was stirred at room temperature for 3 hours. The reaction mixture was concentrated under reduced pressure to afford Compound 45-4 (2.5 g) as a white solid, which was used in the next step without further purification.

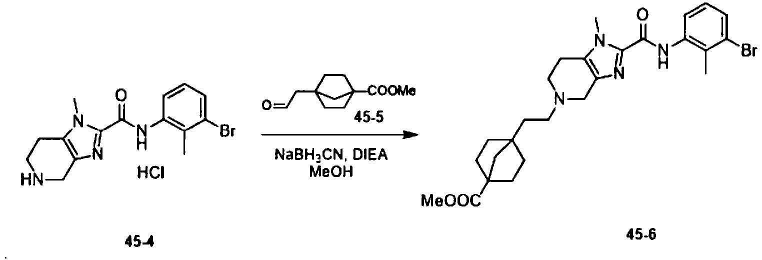

To a solution of N-(3-bromo-2-methylphenyl)-1-methyl-4,5,6,7-tetrahydroimidazo[4,5-c]pyridine-2-carboxamide (2.5 g, 6.48 mmol, 1 eq, HCl salt) in MeOH (100 mL), DIPEA (1.68 g, 12.96 mmol, 2.26 mL, 2 eq) was slowly added dropwise at room temperature until the pH of the reaction mixture reached 8-9. Methyl 4-(2-oxoethyl)norbornane-1-carboxylate (1.40 g, 7.13 mmol, 1.10 eq) was then added dropwise at room temperature, and the reaction mixture was stirred at room temperature for 2 hours. NaBH₃CN (2.04 g, 32.41 mmol, 5 eq) was subsequently added, and the reaction mixture was stirred at room temperature for an additional 18 hours. After completion of the reaction, 1 M aqueous HCl (300 mL) was added, and the mixture was extracted with ethyl acetate (50 mL * 3). The combined organic layers were dried over Na₂SO₄, and concentrated under reduced pressure. The residue was purified by flash silica gel chromatography (ISCO^{®}; 40 g SepaFlash^{®} Silica Flash Column; eluent: ethyl acetate/petroleum ether/TEA = 1:1:0.01, flow rate 80 mL/min) to afford Compound 45-6 (2.2 g, 4.07 mmol, 62.8% yield) as a white solid.

LCMS m/z 529.2 [M+1]⁺.

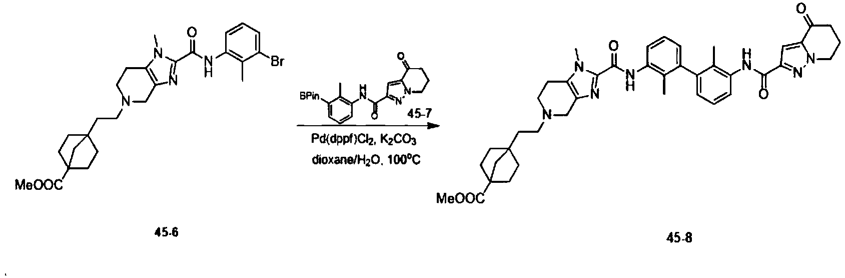

A mixture of N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (492.70 mg, 1.25 mmol, 1.1 eq), methyl 4-[2-[2-[(3-bromo-2-methylphenyl)carbamoyl]-1-methyl-6,7-dihydro-4H-imidazo[4,5-c]pyridin-5-yl]ethyl]norbornane-1-carboxylate (600 mg, 1.13 mmol, 1 eq), Pd(dppf)Cl₂ (82.92 mg, 113.32 µmol, 0.1 eq), and K₂CO₃ (469.85 mg, 3.40 mmol, 3 eq) in 1,4-dioxane (20 mL)/H₂O (5 mL) was stirred at 100 °C for 24 hours under a nitrogen atmosphere. After completion of the reaction, the mixture was filtered and concentrated under reduced pressure, and the residue was purified by column chromatography (SiO₂, Petroleum ether/Ethyl acetate = 1/1 to 0/1) to afford Compound 45-8 (560 mg, 429.06 µmol, 37.9% yield) as a yellow solid.

LCMS m/z 718.3 [M+1]⁺.

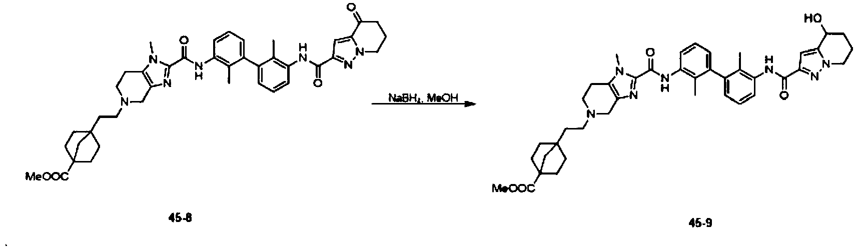

A mixture of methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (98.35 mg, 137.01 µmol, 1 eq) and NaBH₄ (25.9 mg, 685.05 µumol, 5 eq) in MeOH (6 mL) was stirred at room temperature for 6 hours. After completion of the reaction, the mixture was concentrated under reduced pressure, and the residue was purified by prep-HPLC (column: Waters Xbridge 150 * 25 mm * 5 µm; mobile phase: [water (NH₄HCO₃)-ACN]; B%: 20%-50%, 8 min) to afford Compound 45-9 (76 mg, 77.1% yield) as a white solid.

LCMS m/z 720.4 [M+1]⁺.

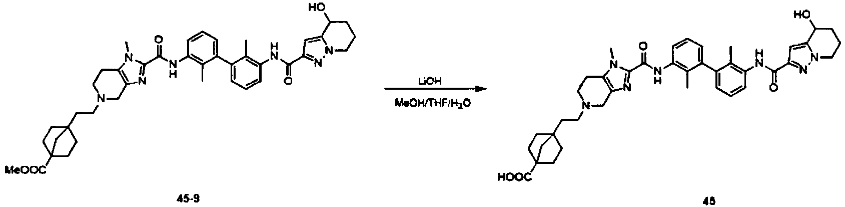

A solution of methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (98.35 mg, 137.01 µmol, 1 eq) and LiOH·H₂O (17.25 mg, 411.03 µmol, 3 eq) in MeOH (6 mL)/H₂O (2 mL)/THF (2 mL) was stirred at room temperature for 6 hours. After completion of the reaction, the mixture was concentrated under reduced pressure, and the residue was purified by prep-HPLC (column: Waters Xbridge 150 * 25 mm * 5 µm; mobile phase: [water (NH₄HCO₃)-ACN]; B%: 20%-50%, 8 min) to afford Compound 45 (48 mg, 48.6% yield) as a white solid.

LCMS m/z 706.4 [M+1]⁺.

¹H NMR (400 MHz, DMSO-d6) δ = 9.72 (s, 1H), 9.52 (s, 1H), 7.72 - 7.65 (m, 1H), 7.61 - 7.56 (m, 1H), 7.28 (t, J = 7.7 Hz, 2H), 6.96 (t, J = 6.6 Hz, 2H), 6.69 (s, 1H), 5.56 (s, 1H), 4.76 (s, 1H), 4.18 - 4.10 (m, 2H), 3.87 (s, 3H), 3.52 - 3.38 (m, 3H), 2.75 (d, J = 5.0 Hz, 2H), 2.70 - 2.63 (m, 3H), 2.40 - 2.27 (m, 1H), 2.18 (d, J = 7.8 Hz, 1H), 2.04 - 1.99 (m, 1H), 1.97 - 1.95 (m, 3H), 1.94 - 1.92 (m, 3H), 1.90 - 1.83 (m, 2H), 1.80 - 1.67 (m, 3H), 1.57 - 1.47 (m, 4H), 1.45 (s, 2H), 1.39 (d, J = 11.1 Hz, 2H).

### 14-2. Preparation of Compound 46, 4-(2-(2-((3'-(4-((2-acetamidoethyl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

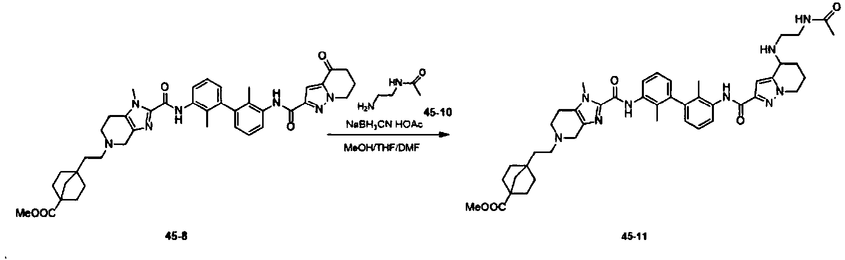

A mixture of N-(2-aminoethyl)acetamide (142.28 mg, 1.39 mmol, 132.97 µL, 10 eq), methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (100 mg, 139.30 µmol, 1 eq), HOAc (83.65 mg, 1.39 mmol, 80 µL, 10 eq), and NaBH₃CN (43.77 mg, 696.52 µmol, 5 eq) in MeOH (6 mL)/DMF (2 mL)/THF (2 mL) was stirred at 60 °C for 6 h. After completion, the reaction mixture was concentrated under reduced pressure, and the residue was purified by prep-HPLC (column: Phenomenex Luna C18, 150 * 25 mm * 10 µm; mobile phase: [water (FA)-ACN]; B %: 11%-41%, 10 min) to afford Compound 45-11 (90 mg, 80.4% yield) as a white solid.

LCMS m/z 804.5 [M+1]⁺.

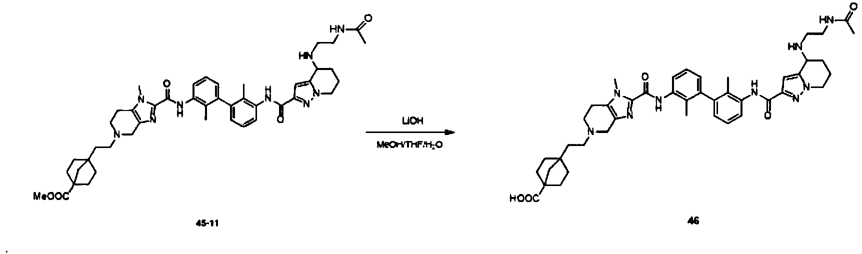

A mixture of methyl 4-[2-[2-[[3-[3-[[4-(2-acetamidoethylamino)-4,5,6,7-tetrahydropyrazolo[1,5-a]pyridine-2-carbonyl]amino]-2-methylphenyl]-2-methylphenyl]carbamoyl]-1-methyl-6,7-dihydro-4H-imidazo[4,5-c]pyridin-5-yl]ethyl]norbornane-1-carboxylate (90 mg, 111.94 µmol, 1 eq) and LiOH·H₂O (14 mg, 335.82 µmol, 3 eq) in a mixture of MeOH (6 mL)/THF (2 mL)/H₂O (2 mL) was stirred at 60 °C for 6 h. After completion, the reaction mixture was concentrated under reduced pressure, and the residue was purified by prep-HPLC (column: Waters Xbridge C18, 150 * 50 mm, 10 µm; mobile phase: [water (NH₄HCO₃)-ACN]; B%: 18%-48%, 10 min) to afford Compound 46 (42 mg, 47.1% yield) as a white solid.

LCMS m/z 790.5 [M+1]⁺.

¹H NMR (400 MHz, DMSO-d6) δ = 9.72 (s, 1H), 9.50 (s, 1H), 7.84 (br s, 1H), 7.68 (d, J = 8.1 Hz, 1H), 7.58 (d, J = 8.1 Hz, 1H), 7.27 (t, J = 7.8 Hz, 2H), 6.96 (dd, J = 4.3, 6.8 Hz, 2H), 6.73 (s, 1H), 4.14 (s, 2H), 3.87 (s, 4H), 3.44 - 3.40 (m, 3H), 3.15 - 3.10 (m, 2H), 2.75 (d, J = 5.0 Hz, 2H), 2.67 - 2.60 (m, 3H), 2.57 - 2.55 (m, 1H), 2.33 (s, 2H), 2.18 (d, J = 6.7 Hz, 1H), 2.01 (s, 1H), 1.95 (s, 3H), 1.93 (s, 3H), 1.89 - 1.82 (m, 2H), 1.81 (s, 3H), 1.76 - 1.70 (m, 2H), 1.69 - 1.63 (m, 1H), 1.53 (s, 4H), 1.44 (s, 2H), 1.39 (d, J = 11.4 Hz, 2H).

### 14-3. Preparation of Compound 47, 4-(2-(2-((3'-(4-((R)-3-hydroxypyrrolidin-1-yl)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

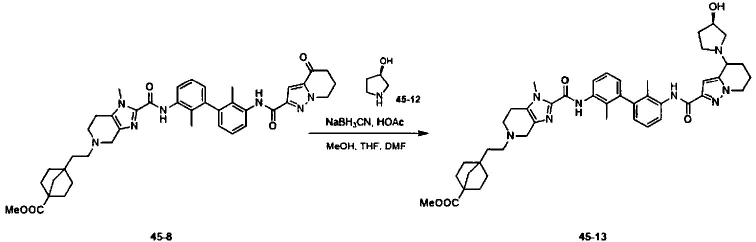

A mixture of (3R)-pyrrolidin-3-ol (119.36 mg, 1.37 mmol, 113.68 µL, 10 eq), methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (98.35 mg, 137.01 µmol, 1 eq), NaBH₃CN (43.05 mg, 685.06 µmol, 5 eq), and HOAc (82.3 mg, 1.37 mmol, 78.35 µL, 10 eq) were added to MeOH (6 mL)/DMF (2 mL)/THF (2 mL), and stirred at 60 °C for 6 h. After completion, the reaction mixture was concentrated under reduced pressure, and the residue was purified by reversed-phase HPLC (column: Phenomenex Luna C18, 150 * 25 mm * 10 µm; mobile phase: [water (FA)-ACN]; gradient: 10%-40% B over 10 min) to afford Compound 45-13 as a white solid (85 mg, 78.6% yield).

LCMS m/z 789.5 [M+1]⁺.

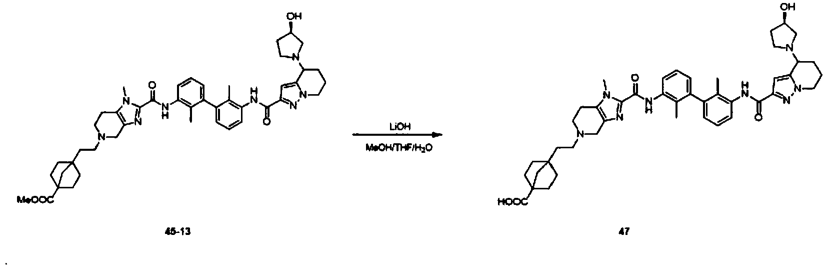

4-[2-[2-[[3-[3-[[4-[(3R)-3-hydroxypyrrolidin-1-yl]-4,5,6,7-tetrahydropyrazolo[1,5-a]pyridine-2-carbonyl]amino]-2-methylphenyl]-2-methylphenyl]carbamoyl]-1-methyl-6,7-dihydro-4H-imidazo[4,5-c]pyridin-5-yl]ethyl]norbornane-1-carboxylate (85 mg, 107.73 µmol, 1 eq) and LiOH·H₂O (13.56 mg, 323.19 µmol, 3 eq) were added to MeOH (6 mL)/THF (2 mL)/H₂O (2 mL), and stirred at 60 °C for 6 h. After completion, the reaction mixture was concentrated under reduced pressure, and the residue was purified by reversed-phase HPLC (column: Waters Xbridge C18, 150 * 50 mm, 10 µm; mobile phase: [water (NH₄HCO₃)-ACN]; B%: 18%-48% B, 10 min) to afford Compound 47 (45 mg, 53.9% yield) as a white solid.

LCMS m/z 775.8 [M+1]⁺.

¹H NMR (400 MHz, DMSO-d6) δ = 9.72 (s, 1H), 9.52 (d, J = 3.5 Hz, 1H), 7.68 (d, J = 8.7 Hz, 1H), 7.58 (s, 1H), 7.28 (t, J = 7.9 Hz, 2H), 6.98 - 6.94 (m, 2H), 6.68 (d, J = 7.6 Hz, 1H), 4.78 - 4.62 (m, 1H), 4.17 (m, 3H), 3.87 (s, 3H), 3.80 (m, J = 5.1 Hz, 1H), 3.44 - 3.40 (m, 2H), 2.81 - 2.71 (m, 3H), 2.71 - 2.62 (m, 4H), 2.60 - 2.53 (m, 4H), 2.33 (s, 1H), 2.28 - 2.18 (m, 1H), 1.95 (s, 3H), 1.94 (s, 3H), 1.91 - 1.83 (m, 5H), 1.78 - 1.68 (m, 2H), 1.52 (m, 5H), 1.44 (s, 2H), 1.41 - 1.34 (m, 2H).

### 14-4. Preparation of Compound 48, 4-(2-(2-((3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

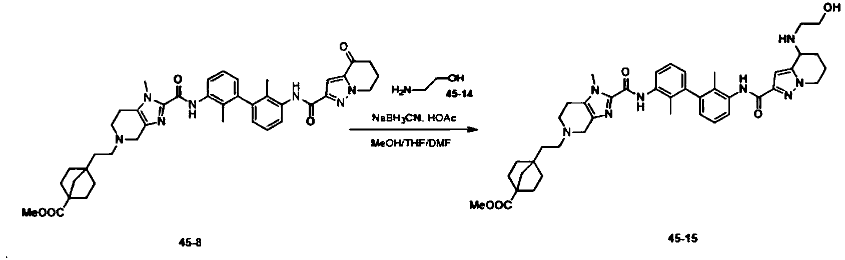

2-aminoethanol (83.69 mg, 1.37 mmol, 82.86 µL, 10 eq), methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (98.35 mg, 137.01 µmol, 1 eq), NaBH₃CN (43.05 mg, 685.06 µmol, 5 eq), and HOAc (16.46 mg, 274.02 µmol, 15.67 µL, 2 eq) were added to a solution of MeOH (6 mL)/DMF (2 mL)/THF (2 mL), and stirred at 60 °C for 6 h. After completion, the reaction mixture was concentrated under reduced pressure, and the residue was purified by prep-HPLC (column: Phenomenex Luna C18, 150 * 25 mm * 10 µm; mobile phase: [water (FA)-ACN]; B%: 10%-40%, 10 min) to afford Compound 45-15 (85 mg, 81.3% yield) as a white solid.

LCMS m/z763.5 [M+1]⁺.

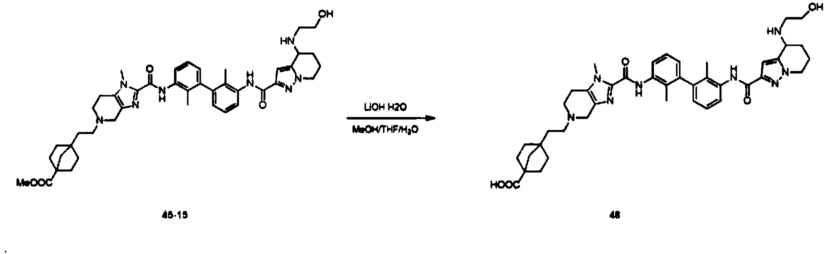

Methyl 4-[2-[2-[[3-[3-[[4-(2-hydroxyethylamino)-4,5,6,7-tetrahydropyrazolo[1,5-a]pyridine-2-carbonyl]amino]-2-methylphenyl]-2-methylphenyl]carbamoyl]-1-methyl-6,7-dihydro-4H-imidazo[4,5-c]pyridin-5-yl]ethyl]norbornane-1-carboxylate (70 mg, 91.75 µmol, 1 eq) and LiOH·H₂O (11.55 mg, 275.25 µmol, 3 eq) were added to a solution of MeOH (15 mL)/THF (3 mL)/H₂O (3 mL), and stirred at 60 °C for 6 h. After completion, the reaction mixture was concentrated under reduced pressure, and the residue was purified by reversed-phase HPLC (column: Waters Xbridge C18, 150 * 50 mm, 10 µm; mobile phase: [water (NH₄HCO₃)-ACN]; B%: 18%-48%, 10 min) to afford Compound 48 (40 mg, 57.6% yield) as a white solid.

LCMS m/z 749.6 [M+1]⁺.

¹H NMR (400 MHz, DMSO-d6) δ = 9.72 (s, 1H), 9.52 (s, 1H), 7.68 (d, J = 7.9 Hz, 1H), 7.58 (d, J = 8.2 Hz, 1H), 7.28 (t, J = 7.8 Hz, 2H), 6.99 - 6.93 (m, 2H), 6.74 (s, 1H), 4.70 - 4.41 (m, 1H), 4.14 (m, 2H), 3.94 (m, 1H), 3.87 (s, 3H), 3.50 (m, 2H), 3.45 - 3.40 (m, 3H), 2.78 - 2.63 (m, 6H), 2.58 - 2.54 (m, 1H), 2.33 (m, 2H), 2.20 (m, 1H), 2.03 (m, 1H), 1.95 (s, 3H), 1.93 (s, 3H), 1.91 - 1.81 (m, 3H), 1.80 - 1.67 (m, 3H), 1.57 - 1.48 (m, 4H), 1.45 (s, 2H), 1.39 (m, 2H)

### 14-5. Preparation of Compound 49, 4-(2-(2-((2,2'-dimethyl-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

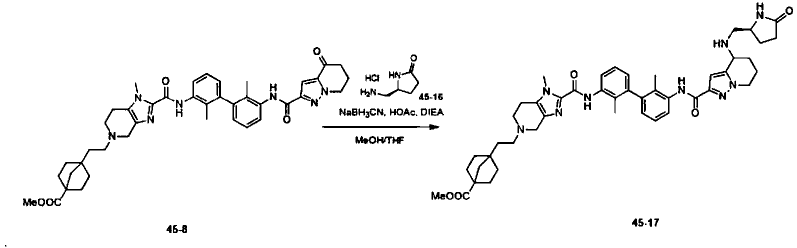

A mixture of methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (70 mg, 236.82 µmol, 1 eq) and 5-(aminomethyl)pyrrolidin-2-one hydrochloride (135.16 mg, 897.42 µmol, 3.79 eq) were added to a solution of MeOH (10 mL)/THF (2 mL), DIPEA (153.03 mg, 1.18 mmol, 206.24 µL, 5 eq), and stirred at room temperature for 15 min. NaBH₃CN (74.41 mg, 1.18 mmol, 5 eq) and HOAc (142.2 mg, 2.37 mmol, 135.4 µL, 10 eq) were then added, and the reaction mixture was stirred at 60 °C for 12 h. After completion, the reaction mixture was concentrated under reduced pressure, and the residue was purified by prep-HPLC (column: Phenomenex Luna C18, 150 * 25 mm, 10 µm; mobile phase: [water (FA)-ACN]; B%: 8%-38%, 10 min) to afford Compound 45-17 (100 mg, 51.8% yield) as a white solid.

LCMS m/z 816.9 [M+1]⁺.

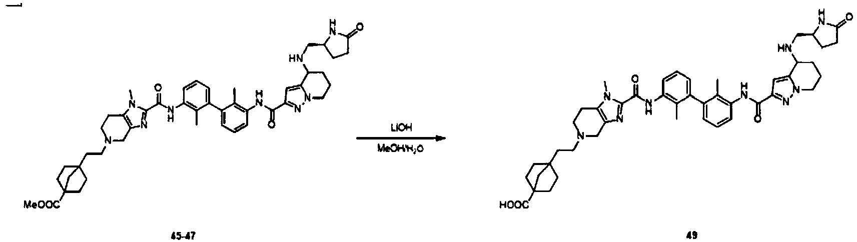

A mixture of methyl 4-[2-[1-methyl-2-[[2-methyl-3-[2-methyl-3-[[4-[(5-oxopyrrolidin-2-yl)methylamino]-4,5,6,7-tetrahydropyrazolo[1,5-a]pyridine-2-carbonyl]amino]phenyl]phenyl]carbamoyl]-6,7-dihydro-4H-imidazo[4,5-c]pyridin-5-yl]ethyl]norbornane-1-carboxylate (100 mg, 122.55 µmol, 1 eq) and LiOH (8.79 mg, 367.65 µmol, 3 eq) in MeOH (10 mL)/H₂O (5 mL) was stirred at room temperature for 12 h. After completion, the reaction mixture was concentrated under reduced pressure, and the residue was purified by prep-HPLC (column: Waters XBridge C18, 150 * 50 mm, 10 µm; mobile phase: [water (NH₄HCO₃)-ACN]; B%: 15%-45% over 10 min) to afford Compound 49 as a white solid (60 mg, 60.4% yield).

LCMS m/z 802.6 [M+1]⁺.

¹H NMR (400 MHz, DMSO-d6) δ = 9.72 (s, 1H), 9.51 (s, 1H), 7.68 (d, J = 7.1 Hz, 1H), 7.58 (d, J = 8.1 Hz, 1H), 7.28 (t, J = 7.8 Hz, 2H), 6.96 (dd, J = 5.0, 6.6 Hz, 2H), 6.77 (d, J = 9.5 Hz, 1H), 4.26 - 4.01 (m, 3H), 3.90 (d, J = 7.6 Hz, 1H), 3.87 (s, 4H), 3.63 - 3.55 (m, 3H), 2.75 (d, J = 5.1 Hz, 2H), 2.68 - 2.58 (m, 4H), 2.22 - 1.98 (m, 5H), 1.95 (s, 3H), 1.93 (s, 3H), 1.91 - 1.80 (m, 3H), 1.79 - 1.64 (m, 4H), 1.58 - 1.48 (m, 4H), 1.47 - 1.32 (m, 4H).

### 14-6. Preparation of Compound 50, 4-(2-(2-((3'-(4-((carboxymethyl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

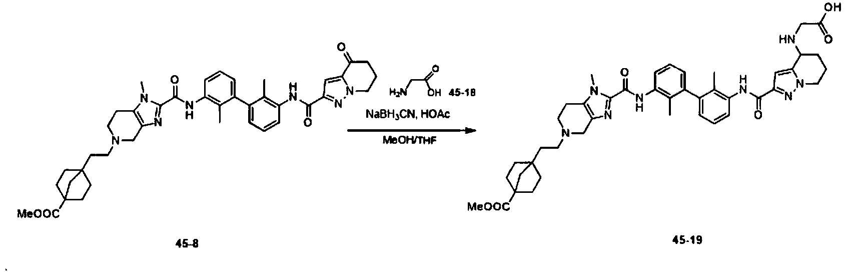

2-aminoacetic acid (177.77 mg, 2.37 mmol, 10 eq), methyl 4-[2-[1-methyl-2-[[2-methyl-3-[2-methyl-3-[(4-oxo-6,7-dihydro-5H-pyrazolo[1,5-a]pyridine-2-carbonyl)amino]phenyl]phenyl]carbamoyl]-6,7-dihydro-4H-imidazo[4,5-c]pyridin-5-yl]ethyl]norbornane-1-carboxylate (170 mg, 236.82 µmol, 1 eq), NaBH₃CN (74.41 mg, 1.18 mmol, 5 eq), and HOAc (142 mg, 2.37 mmol, 135 µL, 10 eq) were added to a solution of MeOH (10 mL)/THF (2 mL), and stirred at 60 °C for 12 h. After completion, the reaction mixture was concentrated under reduced pressure, and the residue was purified by prep-HPLC (column: Phenomenex Luna C18, 150 * 25 mm * 10 µm; mobile phase: [water (FA)-ACN]; B%: 10%-40%, 10 min) to afford Compound 45-19 (100 mg, 54.4% yield) as a white solid.

LCMS m/z 777.6 [M+1]⁺.

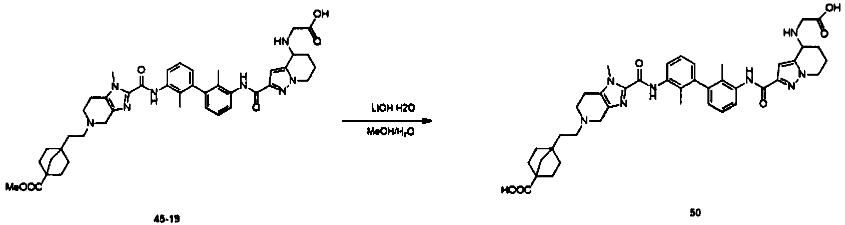

2-[[2-[[3-[3-[[5-[2-(4-methoxycarbonylnorbornan-1-yl)ethyl]-1-methyl-6,7-dihydro-4H-imidazo[4,5-c]pyridine-2-carbonyl]amino]-2-methylphenyl]-2-methylphenyl]carbamoyl]-4,5,6,7-tetrahydropyrazolo[1,5-a]pyridin-4-yl]amino]acetic acid (100 mg, 128.71 µmol, 1 eq) and LiOH·H₂O (16.20 mg, 386.14 µmol, 3 eq) were added to a solution of MeOH (20 mL)/H₂O (5 mL), and stirred at 60 °C for 12 h. After completion, the reaction mixture was concentrated under reduced pressure, and the residue was purified by prep-HPLC (column: Phenomenex Luna C18, 150 * 25 mm * 10 µm; mobile phase: [water (FA)-ACN]; B%: 10%-40% over 10 min) to afford Compound 50 (45 mg, 44.5% yield) as a yellow solid.

LCMS m/z 763.5 [M+1]⁺.

¹H NMR (400 MHz, DMSO-d6) δ = 9.72 (s, 1H), 9.54 (s, 1H), 7.68 (d, J = 8.0 Hz, 1H), 7.64 - 7.51 (m, 1H), 7.27 (t, J = 7.8 Hz, 2H), 6.96 (dd, J = 4.0, 6.9 Hz, 2H), 6.73 (s, 1H), 4.14 (m, 4H), 4.06 - 3.92 (m, 3H), 3.91 - 3.82 (m, 4H), 3.25 - 3.10 (m, 4H), 2.75 (m, 2H), 2.69 - 2.62 (m, 2H), 2.62 - 2.55 (m, 1H), 2.21 (m, 1H), 2.03 - 1.83 (m, 10H), 1.79 - 1.66 (m, 3H), 1.56 - 1.47 (m, 4H), 1.44 (s, 2H), 1.38 (m, 2H)

### 14-7. Preparation of Compound 51, 4-(2-(2-((3'-(4-((2-carboxypropan-2-yl)amino)-4,5,6,7-tetrahydropyrazolo[1,5-a]pyridine-2-carboxamido)-2,2'-dimethyl-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

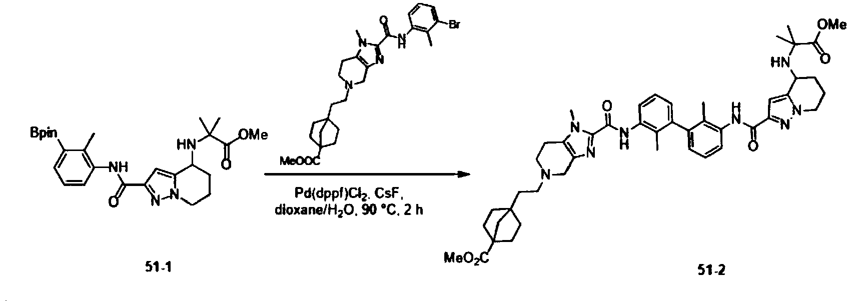

A mixture of methyl 4-[2-[2-[(3-bromo-2-methylphenyl)carbamoyl]-1-methyl-6,7-dihydro-4H-imidazo[4,5-c]pyridin-5-yl]ethyl]norbornane-1-carboxylate (200 mg, 377.74 µmol, 1 eq), methyl 2-methyl-2-[[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]propanoate (206.26 mg, 415.51 µmol, 1.1 eq), CsF (172.14 mg, 1.13 mmol, 3 eq), and di-tert-butyl(cyclopentyl)phosphine palladium dichloride (24.62 mg, 37.77 µmol, 0.1 eq) in a mixture of dioxane (8 mL)/H₂O (2 mL) was stirred at 90 °C for 2 h under a nitrogen atmosphere. After completion, the mixture was concentrated under reduced pressure, and the residue was purified by prep-HPLC (column: Phenomenex Luna C18, 150 * 40 mm, 15 µm; mobile phase: [water (TFA)-ACN]; gradient: 20%-50% B over 15 min) to afford Compound 51-2 (172 mg, 52.8% yield) as a white solid.

LCMS m/z 819.6 [M+1]⁺.

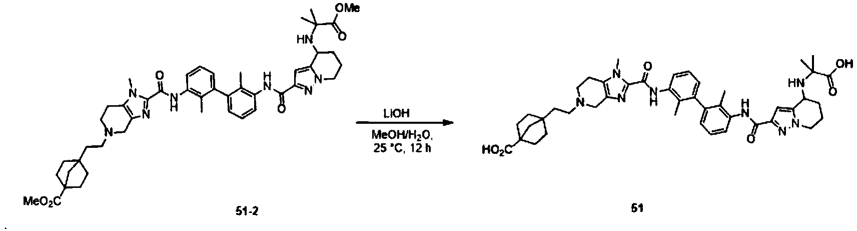

To a solution of methyl 4-[2-[2-[[3-[3-[[4-[(2-methoxy-1,1-dimethyl-2-oxoethyl)amino]-4,5,6,7-tetrahydropyrazolo[1,5-a]pyridine-2-carbonyl]amino]-2-methylphenyl]-2-methylphenyl]carbamoyl]-1-methyl-6,7-dihydro-4H-imidazo[4,5-c]pyridin-5-yl]ethyl]norbornane-1-carboxylate (120 mg, 146.52 µmol, 1 eq) in H₂O (3 mL)/MeOH (10 mL), LiOH (10.54 mg, 439.56 µmol, 3 eq) was added, and stirred at room temperature for 12 h. The reaction mixture was concentrated under reduced pressure, and the residue was purified by prep-HPLC (column: Waters XBridge, 150 * 25 mm * 5 µm; mobile phase: [water (NH₄HCO₃)-ACN]; gradient: 12%-42% B over 9 min) to afford Compound 51 (82 mg, 67.2% yield) as a white solid.

LCMS m/z 791.7 [M+1]⁺.

¹H NMR (400 MHz, DMSO-d6 ) δ = 9.70 (s, 1H), 9.51 (s, 1H), 7.72 (br d, J = 2.8 Hz, 1H), 7.56 (d, J = 7.5 Hz, 1H), 7.27 (t, J = 7.7 Hz, 2H), 6.95 (dd, J = 4.6, 6.8 Hz, 2H), 6.73 (s, 1H), 4.44 - 4.32 (m, 1H), 4.16 - 4.04 (m, 3H), 3.98 - 3.92 (m, 1H), 3.86 (s, 3H), 3.17 (s, 3H), 2.74 (br d, J = 5.5 Hz, 2H), 2.69 - 2.62 (m, 3H), 2.21 - 2.10 (m, 1H), 2.07 - 1.97 (m, 2H), 1.94 (s, 3H), 1.92 (s, 3H), 1.88 - 1.80 (m, 2H), 1.77 - 1.69 (m, 2H), 1.67 - 1.59 (m, 1H), 1.58 - 1.48 (m, 4H), 1.44 (s, 2H), 1.41 - 1.34 (m, 2H), 1.29 (d, J = 3.4 Hz, 5H).

### [Preparation Example 15]

### Preparation of Compounds 52 to 60

### 15-1. Preparation of Compound 52, 4-(2-(2-((3'-(4-((2-acetamidoethyl)amino)-4,5,6,7-tetrahydropyrazolo[1,5-a]pyridine-2-carboxamido)-2,2'-dichloro-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

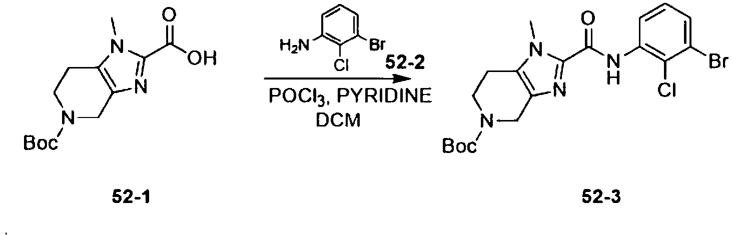

To a solution of 5-tert-Butoxycarbonyl-1-methyl-6,7-dihydro-4H-imidazo[4,5-c]pyridine-2-carboxylic acid (3.79 g, 13.47 mmol, 1 eq) and 3-bromo-2-chloroaniline (5.56 g, 26.93 mmol, 2 eq) were dissolved in DCM (100 mL), POCl₃ (4.81 g, 40.40 mmol, 2.93 mL, 3 eq) and pyridine (5.33 g, 67.33 mmol, 5.43 mL, 5 eq) were added, and the resulting mixture was stirred at room temperature for 36 hours. The mixture was concentrated under reduced pressure, and the residue was purified by column chromatography (SiO₂, Petroleum ether/Ethyl acetate = 100/1 to 2/1) to afford Compound 52-3 (2.28 g, 31.5% yield) as a red solid, which was used in the next step without further purification.

LCMS m/z 471.1 [M+3]⁺.

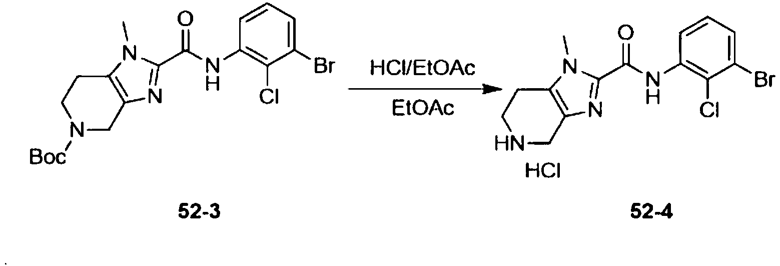

To a mixture of N-(3-Bromo-2-chloro-phenyl)-1-methyl-4,5,6,7-tetrahydroimidazo[4,5-c]pyridine-2-carboxamide (1.03 g, 2.54 mmol, 1 eq, HCl salt) and methyl 4-(2-oxoethyl)norbornane-1-carboxylate (597.27 mg, 3.04 mmol, 1.2 eq) in MeOH (80 mL), NaBH₃CN (796.90 mg, 12.68 mmol, 5 eq) and DIPEA (393.35 mg, 3.04 mmol, 530.12 µL, 1.2 eq) were added, and the reaction mixture was stirred at 60 °C for 12 hours. The mixture was filtered, and the filtrate was concentrated under reduced pressure. The residue was purified by prep-HPLC (column: Waters XBridge Prep OBD C18, 150 * 40 mm, 10 µm; mobile phase: water (NH₄HCO₃)/ACN; gradient: 65-95% B over 15 min) to afford Compound 52-6 (831 mg, 57.8% yield) as a white solid.

LCMS m/z 369.1 [M+1]⁺.

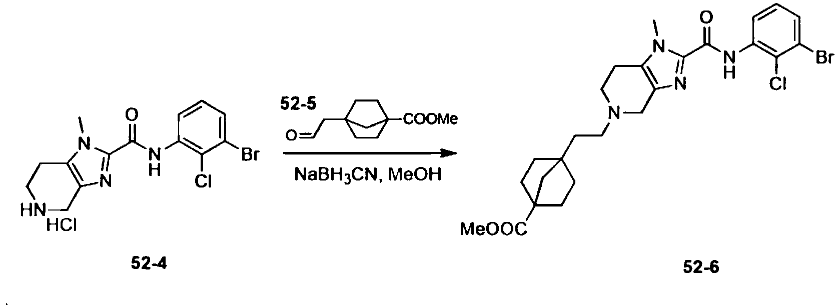

To a mixture of N-(3-bromo-2-chloro-phenyl)-1-methyl-4,5,6,7-tetrahydroimidazo[4,5-c]pyridine-2-carboxamide (1.03 g, 2.54 mmol, 1.0 eq, HCl salt) and methyl 4-(2-oxoethyl)norbornane-1-carboxylate (597.27 mg, 3.04 mmol, 1.2 eq) in MeOH (80 mL), NaBH₃CN (796.90 mg, 12.68 mmol, 5.0 eq) and DIPEA (393.35 mg, 3.04 mmol, 1.2 eq, 530.12 µL) were added, and the reaction mixture was stirred at 60 °C for 12 hours. After completion of the reaction, the mixture was filtered, and the filtrate was concentrated under reduced pressure. The resulting residue was purified by prep-HPLC (column: Waters Xbridge Prep OBD C18, 150 * 40 mm, 10 µm; mobile phase: water (NH₄HCO₃)/ACN; gradient: 65-95% B over 15 min) to afford Compound 52-6 (831 mg, 57.8% yield) as a white solid.

LCMS m/z 551.4 [M+3]⁺.

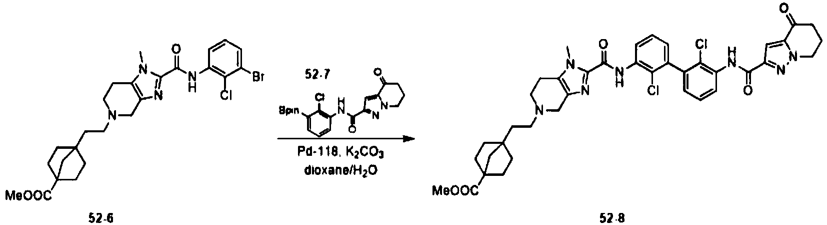

A mixture of methyl 4-[2-[2-[(3-bromo-2-chlorophenyl)carbamoyl]-1-methyl-6,7-dihydro-4H-imidazo[4,5-c]pyridin-5-yl]ethyl]norbornane-1-carboxylate (300 mg, 545.57 µmol, 1.0 eq), N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (340.17 mg, 818.35 µmol, 1.5 eq), K₂CO₃ (226.21 mg, 1.64 mmol, 3.0 eq), and di-tert-butyl(cyclopentyl)phosphane;dichloropalladium;iron (53.34 mg, 81.84 µmol, 0.15 eq) in dioxane (80 mL)/H₂O (16 mL) was stirred at 90 °C for 12 hours under a nitrogen atmosphere. After completion, the mixture was concentrated under reduced pressure, and the residue was purified by flash silica gel chromatography (ISCO^{®}; 12 g SepaFlash^{®} silica column; eluent: 0-20% ethyl acetate/petroleum ether gradient, flow rate 50 mL/min) to afford Compound 52-8 (218 mg, 45.6% yield) as a red solid.

LCMS m/z 760.4 [M+3]⁺.

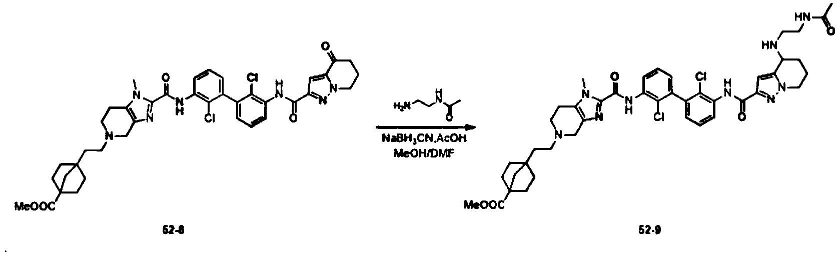

To a mixture of methyl 4-[2-[2-[[2-chloro-3-[2-chloro-3-[(4-oxo-6,7-dihydro-5H-pyrazolo[1,5-a]pyridine-2-carbonyl)amino]phenyl]phenyl]carbamoyl]-1-methyl-6,7-dihydro-4H-imidazo[4,5-c]pyridin-5-yl]ethyl]norbornane-1-carboxylate (60 mg, 79.08 µmol, 1.0 eq) and N-(2-aminoethyl)acetamide (80.77 mg, 790.84 µmol, 75.77 µL, 10 eq) in MeOH (10 mL)/DMF (2 mL), NaBH₃CN (24.85 mg, 395.42 µmol, 5 eq) and AcOH (47.49 mg, 790.84 µmol, 45.27 µL, 10 eq) were added, and the reaction mixture was stirred at 60 °C for 12 hours. After completion, the reaction mixture was concentrated under reduced pressure, and the residue was purified by prep-HPLC (column: Waters XBridge C18, 150 * 25 mm * 5 µm; mobile phase: water (NH₄HCO₃)/ACN; gradient: 50-80% B over 9 min) to afford Compound 52-9 (50 mg, 74.8% yield) as a white solid.

LCMS m/z 844.5 [M+1]⁺.

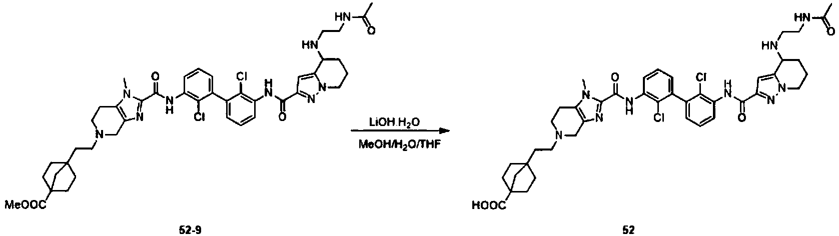

To a solution of methyl 4-[2-[2-[[3-[3-[[4-(2-acetamidoethylamino)-4,5,6,7-tetrahydropyrazolo[1,5-a]pyridine-2-carbonyl]amino]-2-chloro-phenyl]-2-chloro-phenyl]carbamoyl]-1-methyl-6,7-dihydro-4H-imidazo[4,5-c]pyridin-5-yl]ethyl]norbornane-1-carboxylate (50 mg, 59.18 µmol, 1.0 eq) in H₂O (3 mL)/MeOH (2 mL)/THF (1 mL), LiOH·H₂O (7.45 mg, 177.55 µmol, 3.0 eq) was added, and the reaction mixture was stirred at room temperature for 12 hours. After completion, the mixture was concentrated under reduced pressure, and the residue was purified by prep-HPLC (column: Waters XBridge C18, 150 * 25 mm * 5 µm; mobile phase: water (NH₄HCO₃)/ACN; gradient: 20-50% B over 9 min) to afford Compound 52 (37 mg, 74.5% yield) as a white solid.

LCMS m/z 830.5 [M+1]⁺.

¹H NMR (400 MHz, DMSO-d6) δ 9.89 (s, 1H), 9.53 (s, 1H), 8.37 (dd, J = 1.4, 8.2 Hz, 1H), 8.29 (d, J = 7.8 Hz, 1H), 7.82 (t, J = 5.9 Hz, 1H), 7.48 (t, J = 7.8 Hz, 2H), 7.15 (t, J = 8.1 Hz, 2H), 6.79 (s, 1H), 4.19 - 4.09 (m, 2H), 3.90 (s, 3H), 3.88 - 3.86 (m, 1H), 3.42 - 3.39 (m, 2H), 3.16 - 3.10 (m, 2H), 2.74 (br d, J = 5.4 Hz, 2H), 2.69 - 2.60 (m, 4H), 2.55 - 2.52 (m, 2H), 2.17 (br s, 1H), 2.01 (br d, J = 8.6 Hz, 1H), 1.94 - 1.82 (m, 3H), 1.80 (s, 3H), 1.76 - 1.65 (m, 3H), 1.51 (br s, 4H), 1.42 (s, 2H), 1.37 (br d, J = 11.4 Hz, 2H).

### 15-2. Preparation of Compound 53, 4-(2-(2-((2,2'-dichloro-3'-(4-((2-hydroxyethyl)amino)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

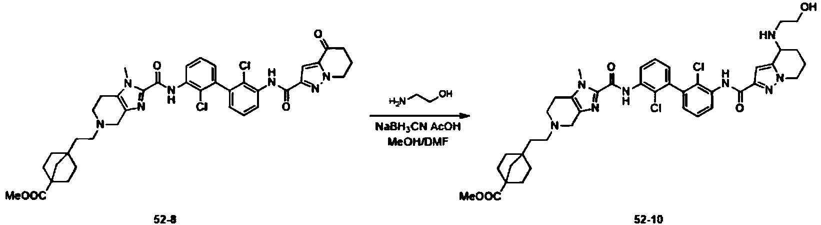

To a solution of methyl 4-[2-[2-[[2-chloro-3-[2-chloro-3-[(4-oxo-6,7-dihydro-5H-pyrazolo[1,5-a]pyridine-2-carbonyl)amino]phenyl]phenyl]carbamoyl]-1-methyl-6,7-dihydro-4H-imidazo[4,5-c]pyridin-5-yl]ethyl]norbornane-1-carboxylate (60 mg, 79.08 µmol, 1.0 eq) and 2-aminoethanol (48.31 mg, 790.84 µmol, 47.73 µL, 10 eq) in MeOH (10 mL)/DMF (2 mL), NaBH₃CN (24.85 mg, 395.42 µmol, 5.0 eq) and AcOH (47.49 mg, 790.84 µmol, 45.27 µL, 10 eq) were added, and the reaction mixture was stirred at 60 °C for 12 hours. After completion, the mixture was concentrated under reduced pressure, and the residue was purified by prep-HPLC (column: Waters XBridge, 150 * 25 mm * 5 µm; mobile phase: water (NH₄HCO₃)/ACN; gradient: 52-82% B over 9 min) to afford Compound 52-10 (50 mg, 78.6% yield) as a white solid.

LCMS m/z 804.6 [M+1]⁺.

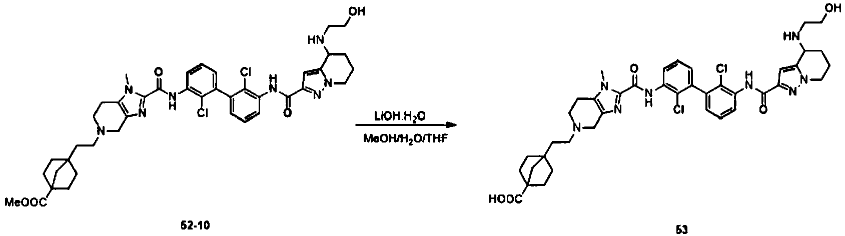

To a solution of methyl 4-[2-[2-[[2-chloro-3-[2-chloro-3-[[4-(2-hydroxyethylamino)-4,5,6,7-tetrahydropyrazolo[1,5-a]pyridine-2-carbonyl]amino]phenyl]phenyl]carbamoyl]-1-methyl-6,7-dihydro-4H-imidazo[4,5-c]pyridin-5-yl]ethyl]norbornane-1-carboxylate (50 mg, 62.21 µmol, 1.0 eq) in H₂O (3 mL)/THF (1 mL)/MeOH (1 mL), LiOH·H₂O (7.83 mg, 186.62 µmol, 3.0 eq) was added, and the reaction mixture was stirred at room temperature for 3 hours. After completion, the mixture was concentrated under reduced pressure, and the residue was purified by prep-HPLC (column: Waters XBridge, 150 * 25 mm * 5 µm; mobile phase: water (NH₄HCO₃)/ACN; gradient: 20-50% B over 50 min) to afford Compound 53 (38 mg, 47.15 µmol, 75.8% yield) as a white solid.

LCMS m/z 789.4 [M+1]⁺.

¹H NMR (400 MHz, DMSO-d6) δ 9.89 (s, 1H), 9.53 (s, 1H), 8.37 (d, J = 8.3 Hz, 1H), 8.29 (d, J = 7.9 Hz, 1H), 7.48 (t, J = 7.9 Hz, 2H), 7.15 (t, J = 8.1 Hz, 2H), 6.78 (s, 1H), 4.63 - 4.41 (m, 1H), 4.19 - 4.10 (m, 2H), 3.90 (s, 4H), 3.47 (br t, J = 5.8 Hz, 2H), 3.42 - 3.39 (m, 2H), 2.74 (br d, J = 5.3 Hz, 2H), 2.67 (br d, J = 3.8 Hz, 4H), 2.57 - 2.52 (m, 2H), 2.33 (br s, 1H), 2.18 (br d, J = 8.0 Hz, 1H), 2.06 - 1.97 (m, 1H), 1.96 - 1.80 (m, 4H), 1.76 - 1.66 (m, 3H), 1.51 (br s, 4H), 1.43 (s, 2H), 1.37 (br d, J = 11.1 Hz, 2H).

### 15-3. Preparation of Compound 54, 4-(2-(2-((2,2'-dichloro-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

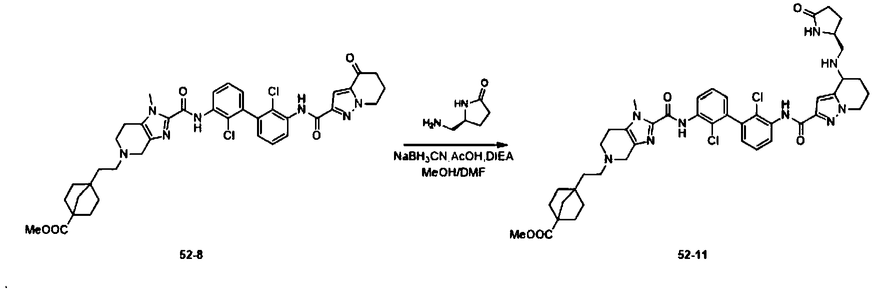

To a solution of methyl 4-[2-[2-[[2-chloro-3-[2-chloro-3-[(4-oxo-6,7-dihydro-5H-pyrazolo[1,5-a]pyridine-2-carbonyl)amino]phenyl]phenyl]carbamoyl]-1-methyl-6,7-dihydro-4H-imidazo[4,5-c]pyridin-5-yl]ethyl]norbornane-1-carboxylate (60 mg, 79.08 µmol, 1.0 eq) and (5S)-5-(aminomethyl)pyrrolidin-2-one hydrochloride (119.10 mg, 790.84 µmol, 10 eq) in MeOH (10 mL)/DMF (2 mL), DIPEA (112.43 mg, 869.92 µmol, 151.52 µL, 11 eq), NaBH₃CN (24.85 mg, 395.42 µmol, 5 eq), and AcOH (47.49 mg, 790.84 µmol, 45.27 µL, 10 eq) were added, and the reaction mixture was stirred at 60 °C for 12 hours. After completion, the mixture was concentrated under reduced pressure, and the residue was purified by prep-HPLC (column: Waters XBridge, 150 * 25 mm * 5 µm; mobile phase: water (NH₄HCO₃)/ACN; gradient: 52-82% B over 9 min) to afford Compound 52-11 (50 mg, 73.7% yield) as a white solid.

LCMS m/z 856.5 [M+1]⁺.

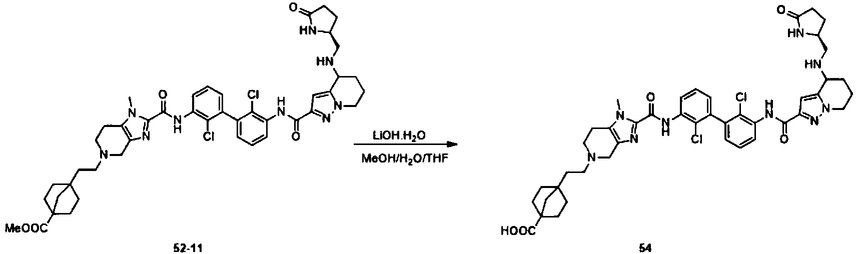

To a solution of methyl 4-[2-[2-[[2-chloro-3-[2-chloro-3-[[4-[[(2S)-5-oxopyrrolidin-2-yl]methylamino]-4,5,6,7-tetrahydropyrazolo[1,5-a]pyridine-2-carbonyl]amino]phenyl]phenyl]carbamoyl]-1-methyl-6,7-dihydro-4H-imidazo[4,5-c]pyridin-5-yl]ethyl]norbornane-1-carboxylate (50 mg, 58.35 µmol, 1.0 eq) in H₂O (3 mL)/THF (1 mL)/MeOH (2 mL), LiOH·H₂O (7.35 mg, 175.06 µmol, 3.0 eq) was added, and the reaction mixture was stirred at room temperature for 3 hours. After completion, the mixture was concentrated under reduced pressure, and the residue was purified by prep-HPLC (column: Waters XBridge, 150 * 25 mm * 5 µm; mobile phase: water (NH₄HCO₃)/ACN; gradient: 22-52% B over 9 min) to afford Compound 54 (30 mg, 60.3% yield) as a white solid.

LCMS m/z 844.4 [M+3]⁺.

¹H NMR (400 MHz, DMSO-d6) δ 9.89 (s, 1H), 9.53 (s, 1H), 8.37 (d, J = 8.4 Hz, 1H), 8.29 (d, J = 8.3 Hz, 1H), 7.69 (d, J = 19.0 Hz, 1H), 7.48 (t, J = 7.7 Hz, 2H), 7.15 (t, J = 8.4 Hz, 2H), 6.83 (d, J = 11.9 Hz, 1H), 4.15 (br d, J = 5.4 Hz, 2H), 3.90 (s, 4H), 3.58 (br s, 1H), 3.42 - 3.40 (m, 2H), 2.74 (br d, J = 4.8 Hz, 2H), 2.68 - 2.57 (m, 5H), 2.24 - 1.96 (m, 6H), 1.94 - 1.81 (m, 3H), 1.77 - 1.61 (m, 5H), 1.51 (br s, 4H), 1.44 - 1.34 (m, 4H).

### 15-4. Preparation of Compound 55

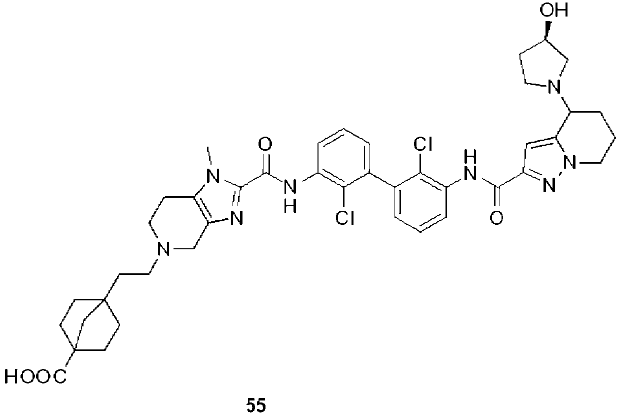

### 15-5. Preparation of Compound 56, 4-(2-(2-((3'-(4-((carboxymethyl)amino)-4,5,6,7-tetrahydropyrazolo[1,5-a]pyridine-2-carboxamido)-2,2'-dichloro-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

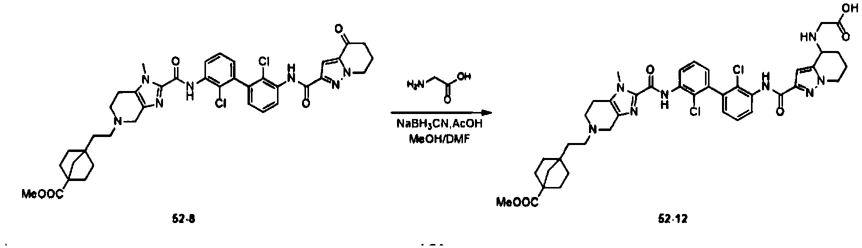

To a solution of methyl 4-[2-[2-[[2-chloro-3-[2-chloro-3-[(4-oxo-6,7-dihydro-5H-pyrazolo[1,5-a]pyridine-2-carbonyl)amino]phenyl]phenyl]carbamoyl]-1-methyl-6,7-dihydro-4H-imidazo[4,5-c]pyridin-5-yl]ethyl]norbornane-1-carboxylate (98 mg, 129.17 µmol, 1.0 eq) in MeOH (10 mL)/DMF (2 mL), 2-aminoacetic acid (96.96 mg, 1.29 mmol, 10 eq), NaBH₃CN (40.59 mg, 645.85 µmol, 5 eq), and AcOH (77.57 mg, 1.29 mmol, 73.94 µL, 10 eq) were added. The reaction mixture was stirred at 60 °C for 12 hours. After completion, the reaction mixture was concentrated under reduced pressure, and the residue was purified by prep-HPLC (column: Waters XBridge, 150 * 25 mm * 5 µm; mobile phase: water (NH₄HCO₃)/ACN; gradient: 30-60% B over 9 min) to afford Compound 52-12 (70 mg, 66.2% yield) as a white solid.

LCMS m/z 817.2 [M+1]⁺.

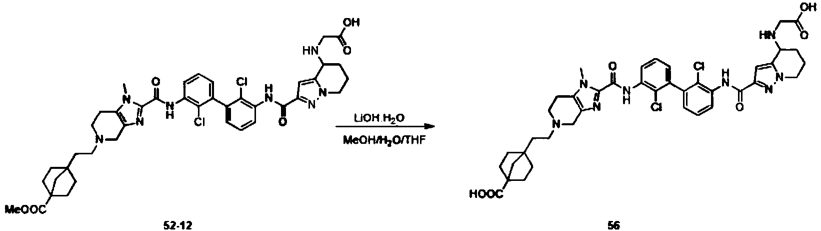

To a solution of 2-[[2-[[2-chloro-3-[2-chloro-3-[[5-[2-(4-methoxycarbonylnorbornan-1-yl)ethyl]-1-methyl-6,7-dihydro-4H-imidazo[4,5-c]pyridine-2-carbonyl]amino]phenyl]phenyl]carbamoyl]-4,5,6,7-tetrahydropyrazolo[1,5-a]pyridin-4-yl]amino]acetic acid (70 mg, 85.60 µmol, 1.0 eq) in H₂O (3 mL)/THF (2 mL)/MeOH (1 mL), LiOH·H₂O (10.78 mg, 256.80 µmol, 3.0 eq) were added, and the reaction mixture was stirred at room temperature for 3 hours. After completion, the reaction mixture was concentrated under reduced pressure, and the residue was purified by prep-HPLC (column: Waters XBridge, 150 * 25 mm * 5 µm; mobile phase: water (NH₄HCO₃)/ACN; gradient: 12-42% B over 9 min) to afford Compound 56 (61 mg, 86.9% yield) as a white solid.

LCMS m/z 803.5 [M+1]⁺.

¹H NMR (400 MHz, DMSO-d6) δ 9.89 (s, 1H), 9.56 (s, 1H), 8.39 - 8.34 (m, 1H), 8.26 (d, J = 8.3 Hz, 1H), 7.48 (t, J = 7.9 Hz, 2H), 7.15 (t, J = 9.2 Hz, 2H), 6.82 (s, 1H), 4.15 (br t, J = 6.1 Hz, 2H), 4.05 (br t, J = 5.9 Hz, 1H), 3.89 (s, 3H), 2.74 (br d, J = 4.8 Hz, 3H), 2.70 - 2.64 (m, 4H), 2.35 - 2.30 (m, 1H), 2.19 (br s, 1H), 2.08 - 2.06 (m, 1H), 2.04 - 1.97 (m, 1H), 1.88 - 1.81 (m, 2H), 1.79 - 1.67 (m, 4H), 1.59 - 1.47 (m, 5H), 1.43 (s, 2H), 1.37 (br d, J = 12.3 Hz, 2H), 1.23 (br s, 2H).

### 15-6. Preparation of Compound 57, (3R)-1-(2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid

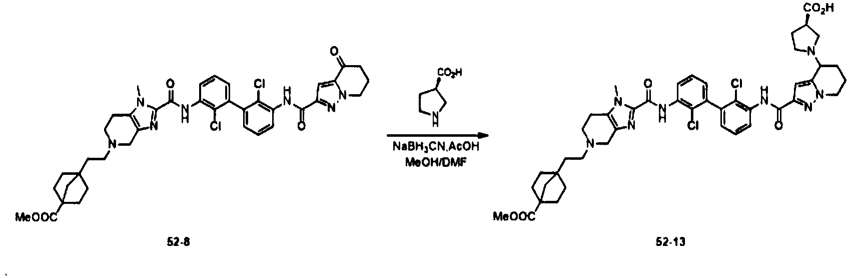

To a solution of methyl 4-[2-[2-[[2-chloro-3-[2-chloro-3-[(4-oxo-6,7-dihydro-5H-pyrazolo[1,5-a]pyridine-2-carbonyl)amino]phenyl]phenyl]carbamoyl]-1-methyl-6,7-dihydro-4H-imidazo[4,5-c]pyridin-5-yl]ethyl]norbornane-1-carboxylate (70 mg, 92.26 µmol, 1 eq) and (3R)-pyrrolidine-3-carboxylic acid (106.22 mg, 922.64 µmol, 10 eq) in MeOH (10 mL)/DMF (2 mL), NaBH₃CN (28.99 mg, 461.32 µmol, 5 eq) and AcOH (55.40 mg, 922.64 µmol, 52.82 µL, 10 eq) were added, and stirred at 60 °C for 12 hours. After completion of the reaction, the mixture was concentrated under reduced pressure, and the resulting residue was purified by prep-HPLC (column: Waters XBridge, 150 * 25 mm * 5um; mobile phase: [water (NH₄HCO₃)-ACN]; gradient: 32-62% B over 9 min) to afford Compound 52-13 (45 mg, 48.9% yield) as a white solid.

LCMS m/z 857.2 [M+1]⁺.

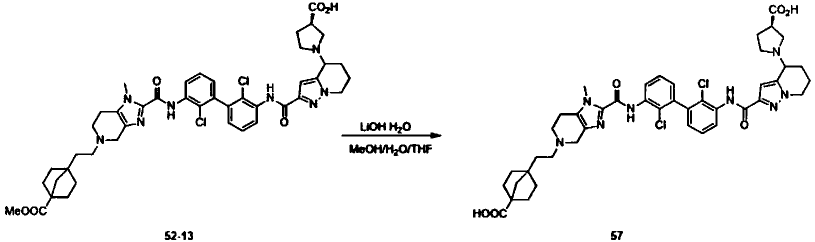

To a solution of (3R)-1-[2-[[2-chloro-3-[2-chloro-3-[[5-[2-(4-methoxycarbonylnorbornan-1-yl)ethyl]-1-methyl-6,7-dihydro-4H-imidazo[4,5-c]pyridine-2-carbonyl]amino]phenyl]phenyl]carbamoyl]-4,5,6,7-tetrahydropyrazolo[1,5-a]pyridin-4-yl]pyrrolidine-3-carboxylic acid (45 mg, 52.46 µmol, 1 eq) in H₂O (3 mL)/THF (2 mL)/MeOH (1 mL), LiOH·H₂O (6.60 mg, 157.38 µmol, 3 eq) was added, and stirred at room temperature for 3 hours. After completion of the reaction, the mixture was concentrated under reduced pressure, and the resulting residue was purified by prep-HPLC (column: Waters Xbridge 150*25mm*5um; mobile phase: [water (NH₄HCO₃)-ACN]; gradient: 12%-42% B over 9 min) to afford Compound 57 (28 mg, 62.6% yield) as a white solid.

LCMS m/z 844.8 [M+3]⁺.

¹H NMR (400 MHz, DMSO-d6) δ 9.89 (s, 1H), 9.55 (s, 1H), 8.37 (dd, J = 1.5, 8.3 Hz, 1H), 8.26 (dd, J = 4.5, 8.1 Hz, 1H), 7.48 (t, J = 7.9 Hz, 2H), 7.15 (dd, J = 7.9, 9.3 Hz, 2H), 6.71 (d, J = 4.9 Hz, 1H), 4.25 - 4.04 (m, 3H), 3.89 (s, 3H), 3.81 (br s, 1H), 2.93 - 2.86 (m, 2H), 2.78 - 2.71 (m, 4H), 2.70 - 2.64 (m, 4H), 2.63 - 2.58 (m, 2H), 2.37 - 2.29 (m, 1H), 2.24 (br s, 1H), 1.98 - 1.81 (m, 8H), 1.75 - 1.67 (m, 2H), 1.55 - 1.48 (m, 4H), 1.43 (s, 2H), 1.37 (br d, J = 10.8 Hz, 2H).

### 15-7. Preparation of Compound 58, 4-(2-(2-((2,2'-dichloro-3'-(4-(4-hydroxypiperidin-1-yl)-4,5,6,7-tetrahydropyrazolo[1,5-a]pyridine-2-carboxamido)-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

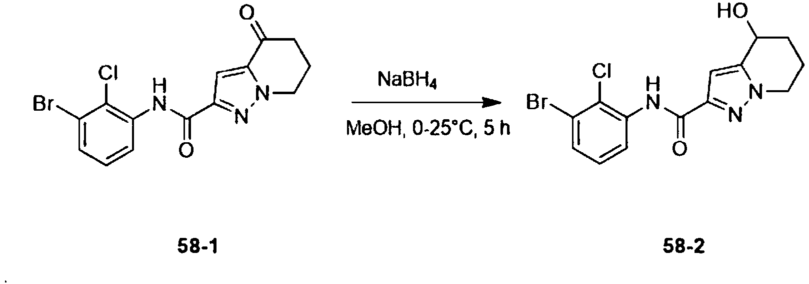

To a solution of N-(3-bromo-2-chloro-phenyl)-4-oxo-6,7-dihydro-5H-pyrazolo[1,5-a]pyridine-2-carboxamide (2.5 g, 6.78 mmol, 1 eq) in MeOH (25 mL), NaBH₄ (852.41 mg, 13.56 mmol, 2 eq) was added at 0 °C. The resulting mixture was stirred at room temperature for 5 hours. After completion of the reaction, aq. sat'd NH₄Cl (120 mL) was added to the mixture, followed by extraction with EtOAc (120 mL*2). The organic layer was washed with brine (200 mL), dried over Na₂SO₄, and concentrated under reduced pressure to afford Compound 58-2 (2.62 g, 93.8% yield) as a white solid, which was used in the next reaction without further purification.

LCMS m/z 372.0 [M+3]⁺.

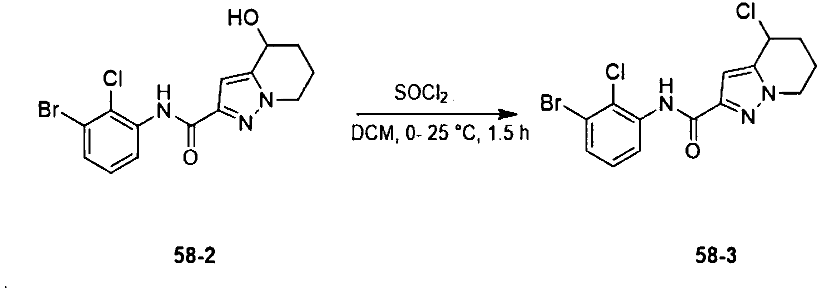

To a solution of N-(3-bromo-2-chloro-phenyl)-4-hydroxy-4,5,6,7-tetrahydropyrazolo[1,5-a]pyridine-2-carboxamide (2.2 g, 5.94 mmol, 1 eq) in DCM (60 mL), SOCl₂ (10.59 g, 89.04 mmol, 6.47 mL, 15 eq) was added at 0 °C. The resulting mixture was stirred at room temperature for 2 hours. After completion of the reaction, H₂O (300 mL) was added to the mixture, followed by extraction with EtOAc (150 mL*2). The organic layer was washed with brine (300 mL), dried over Na₂SO₄, and concentrated under reduced pressure. The residue was purified by flash silica gel chromatography (Petroleum ether/Ethyl acetate=100/1 to 3/1) to afford Compound 58-3 (1.42 g, 56.5% yield) as a white solid.

LCMS m/z 389.8 [M+1]⁺.

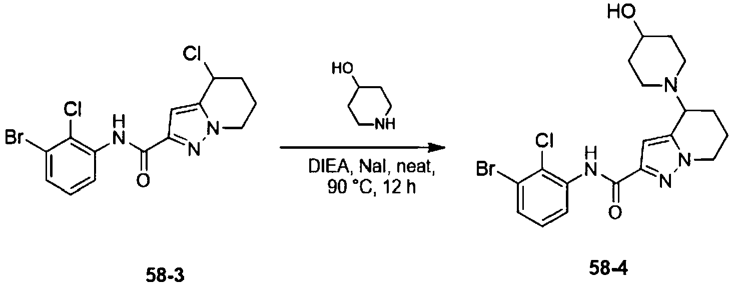

N-(3-bromo-2-chloro-phenyl)-4-chloro-4,5,6,7-tetrahydropyrazolo[1,5-a]pyridine-2-carboxamide (1.5 g, 3.86 mmol, 1 eq) was added to a mixture of piperidin-4-ol (5 mL), followed by addition of DIPEA (1.49 g, 11.57 mmol, 2.01 mL, 3 eq) and Nal (173.36 mg, 1.16 mmol, 0.3 eq). The resulting mixture was stirred at 90 °C for 12 hours. After cooling the mixture to room temperature, H₂O (100 mL) was added, and the mixture was extracted with ethyl acetate (50 mL * 3). The organic layer was washed with brine (200 mL), dried over Na₂SO₄, and concentrated under reduced pressure. The residue was purified by prep-HPLC (column: Kromasil Eternity XT 250*80mm*10µm; mobile phase: [water (NH₄HCO₃)-ACN]; gradient: 35%-65% B over 50 min) to afford Compound 58-4 (1.32 g, 71.6% yield) as a white solid.

LCMS m/z 455.0 [M+3]⁺.

¹H NMR (400 MHz, DMSO-d6) δ = 9.64 (br s, 1H), 8.09 (dd, J = 1.4, 8.1 Hz, 1H), 7.58 (dd, J = 1.4, 8.1 Hz, 1H), 7.33 (t, J = 8.1 Hz, 1H), 6.63 (s, 1H), 4.55 (br s, 1H), 4.25 - 4.16 (m, 1H), 4.06 (dt, J = 4.5, 11.9 Hz, 1H), 3.96 (dd, J = 5.3, 10.0 Hz, 1H), 3.52 - 3.39 (m, 1H), 2.77 - 2.69 (m, 1H), 2.68 - 2.61 (m, 1H), 2.45 - 2.34 (m, 1H), 2.26 (br t, J = 9.3 Hz, 1H), 2.20 - 2.10 (m, 1H), 2.01 - 1.84 (m, 2H), 1.76 - 1.62 (m, 3H), 1.49 - 1.31 (m, 2H).

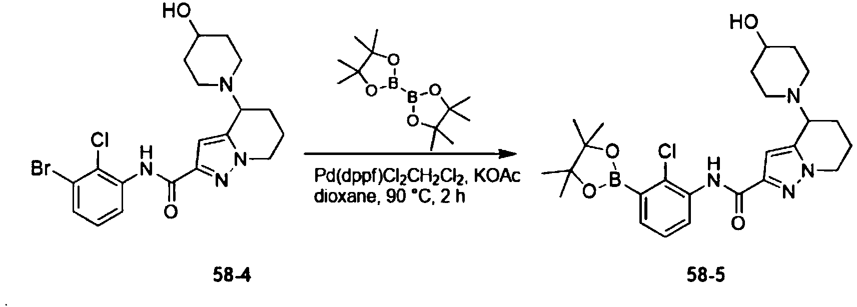

N-(3-bromo-2-chloro-phenyl)-4-(4-hydroxy-1-piperidyl)-4,5,6,7-tetrahydropyrazolo[1,5-a]pyridine-2-carboxamide (250 mg, 550.95 µmol, 1 eq), 4,4,5,5-tetramethyl-2-(4,4,5,5-tetramethyl-1,3,2-dioxaborolan-2-yl)-1,3,2-dioxaborolane (419.72 mg, 1.65 mmol, 3 eq), KOAc (162.21 mg, 1.65 mmol, 3 eq), and Pd(dppf)Cl₂·CH₂Cl₂ (44.99 mg, 55.10 µmol, 0.1 eq) were combined in dioxane (10 mL), and the mixture was stirred under a nitrogen atmosphere at 90 °C for 2 hours. After completion of the reaction, the mixture was filtered, and the filtrate was concentrated under reduced pressure. The resulting residue was purified by prep-TLC (Dichloromethane: Methanol=10/1) to afford Compound 58-5 (236 mg, 59.8% yield) as a white solid.

LCMS m/z 501.2 [M+1]⁺.

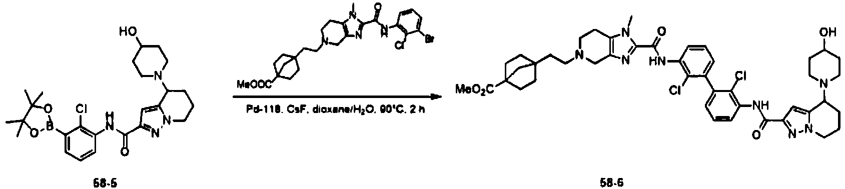

Methyl 4-[2-[2-[(3-bromo-2-chloro-phenyl)carbamoyl]-1-methyl-6,7-dihydro-4H-imidazo[4,5-c]pyridin-5-yl]ethyl]norbornane-1-carboxylate (130 mg, 236.41 µmol, 1 eq), N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (142.08 mg, 283.69 µmol, 1.2 eq), CsF (107.73 mg, 709.24 µmol, 26.18 µL, 3 eq), and ditert-butyl(cyclopentyl)phosphane;dichloropalladium iron (15.41 mg, 23.64 µmol, 0.1 eq) were combined in a mixture of dioxane (8 mL) and H₂O (2 mL), and the resulting mixture was stirred under a nitrogen atmosphere at 90 °C for 2 hours. After completion of the reaction, the mixture was filtered, and the filtrate was concentrated under reduced pressure. The resulting residue was purified by prep-HPLC (column: Phenomenex luna C18 15040mm 15µm; mobile phase: [water (FA)-ACN]; gradient: 12%-42% B over 15 min) to afford Compound 58-6 (82 mg, 39.0% yield) as a white solid.

LCMS m/z 843.2 [M+1]⁺.

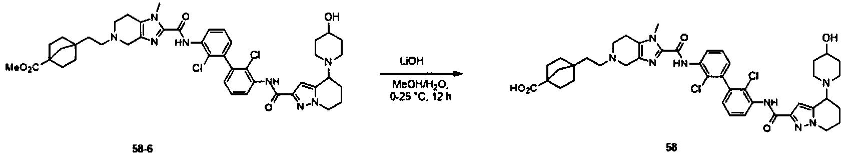

Methyl 4-[2-[2-[[2-chloro-3-[2-chloro-3-[[4-(4-hydroxy-1-piperidyl)-4,5,6,7-tetrahydropyrazolo[1,5-a]pyridine-2-carbonyl]amino]phenyl]phenyl]carbamoyl]-1-methyl-6,7-dihydro-4H-imidazo[4,5-c]pyridin-5-yl]ethyl]norbornane-1-carboxylate (80 mg, 94.80 µmol, 1 eq) was dissolved in a mixed solvent of H₂O (3 mL) and MeOH (10 mL). LiOH (6.80 mg, 284.41 µmol, 3 eq) was added at 0 °C, and the resulting mixture was stirred at room temperature for 12 hours. After completion of the reaction, the mixture was concentrated under reduced pressure, and the resulting residue was purified by prep-HPLC (column: Waters Xbridge Prep OBD C18 150*40mm*10µm; mobile phase: [water (NH₄HCO₃)-ACN]; gradient: 18%-48% B over 15 min) to afford Compound 58 (42 mg, 50.7% yield) as a white solid.

LCMS m/z 829.5 [M+1]⁺.

¹H NMR (400 MHz, DMSO-d6 ) δ = 9.88 (s, 1H), 9.53 (s, 1H), 8.46 - 8.32 (m, 1H), 8.26 (dd, J = 3.9, 6.8 Hz, 1H), 7.48 (t, J = 7.9 Hz, 2H), 7.14 (t, J = 8.4 Hz, 2H), 6.65 (s, 1H), 4.56 (d, J = 4.0 Hz, 2H), 4.19 (br s, 2H), 4.15 - 4.06 (m, 3H), 3.94 (br d, J = 4.5 Hz, 2H), 3.89 (s, 3H), 3.16 (d, J = 5.3 Hz, 4H), 2.74 (br d, J = 4.4 Hz, 3H), 2.65 (br s, 3H), 2.43 - 2.36 (m, 2H), 2.12 (br s, 2H), 1.96 (br d, J = 14.8 Hz, 2H), 1.85 (br s, 2H), 1.73 - 1.69 (m, 3H), 1.51 (s, 3H), 1.43 (s, 3H), 1.37 (br d, J = 10.8 Hz, 2H).

### 15-8. Preparation of Compound 59, 1-(2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)piperidine-4-carboxylic acid

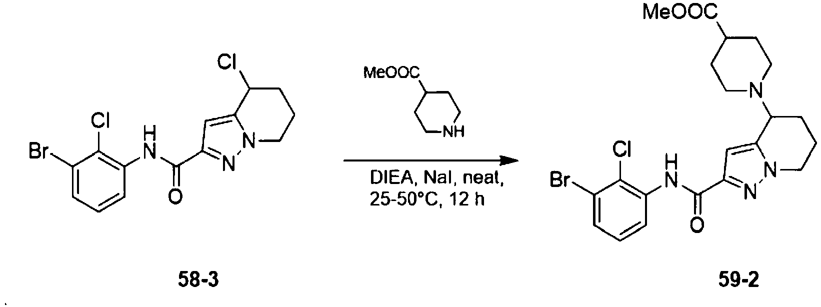

N-(3-bromo-2-chloro-phenyl)-4-chloro-4,5,6,7-tetrahydropyrazolo[1,5-a]pyridine-2-carboxamide (600 mg, 1.54 mmol, 1 eq) was added to a mixture of methyl piperidine-4-carboxylate (662.42 mg, 4.63 mmol, 3 eq), followed by addition of DIPEA (597.91 mg, 4.63 mmol, 805.81 µL, 3 eq) and Nal (69.34 mg, 462.64 µmol, 0.3 eq) at room temperature. The resulting mixture was stirred at 50 °C for 12 hours. After cooling the mixture to room temperature, H₂O (40 mL) was added, and the mixture was extracted with ethyl acetate (40 mL * 3). The organic layer was washed with brine (60 mL), dried over Na₂SO₄, and concentrated under reduced pressure. The residue was purified by prep-HPLC (column: Waters Xbridge Prep OBD C18 150*40mm*10µm; mobile phase: [water (NH₄HCO₃)-ACN]; gradient: 52%-82% B over 15 min) to afford Compound 59-2 (560 mg, 69.5% yield) as a white solid.

LCMS m/z 497.1 [M+3]⁺.

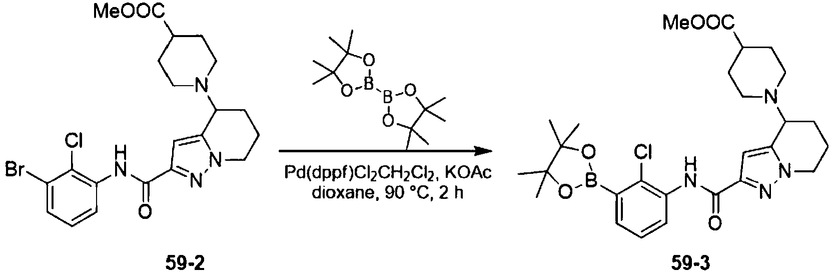

Methyl 4-[2-[(3-bromo-2-chloro-phenyl)carbamoyl]-4,5,6,7-tetrahydropyrazolo[1,5-a]pyridin-4-yl]cyclohexanecarboxylate (70 mg, 141.47 µmol, 1 eq), 4,4,5,5-tetramethyl-2-(4,4,5,5-tetramethyl-1,3,2-dioxaborolan-2-yl)-1,3,2-dioxaborolane (53.89 mg, 212.20 µmol, 1.5 eq), KOAc (41.65 mg, 424.41 µmol, 3 eq), and cyclopentyl(diphenyl)phosphane;dichloromethane;dichloropalladium iron (11.55 mg, 14.15 µmol, 0.1 eq) were combined in dioxane (5 mL), and the resulting mixture was stirred under a nitrogen atmosphere at 90 °C for 2 hours. After completion of the reaction, the mixture was filtered, and the filtrate was concentrated under reduced pressure. The resulting residue was purified by prep-TLC (Dichloromethane: Methanol=10/1) to afford Compound 59-3 (86 mg, 89.7% yield) as a white solid.

LCMS m/z 543.2 [M+1]⁺.

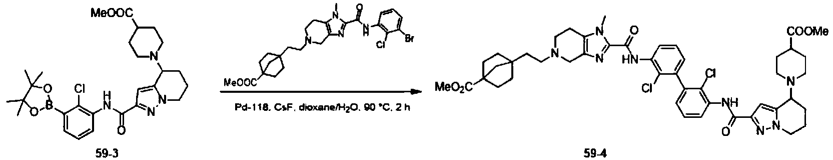

Methyl 4-[2-[2-[(3-bromo-2-chloro-phenyl)carbamoyl]-1-methyl-6,7-dihydro-4H-imidazo[4,5-c]pyridin-5-yl]ethyl]norbornane-1-carboxylate (100 mg, 181.86 µmol, 1 eq), methyl 1-[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (118.47 mg, 218.23 µmol, 1.2 eq), CsF (82.87 mg, 545.57 µmol, 20.14 µL, 3 eq), and ditert-butyl(cyclopentyl)phosphane;dichloropalladium iron (11.85 mg, 18.19 µmol, 0.1 eq) were combined in a mixture of dioxane (8 mL) and H₂O (2 mL), and the resulting mixture was stirred under a nitrogen atmosphere at 90 °C for 2 hours. After completion of the reaction, the mixture was filtered, and the filtrate was concentrated under reduced pressure. The resulting residue was purified by prep-HPLC (column: C18 150Y30mm; mobile phase: [water (FA)-ACN]; gradient: 20%-50% B over 7 min) to afford Compound 59-4 (82 mg, 48.3% yield) as a white solid.

LCMS m/z 887.4 [M+1]⁺.

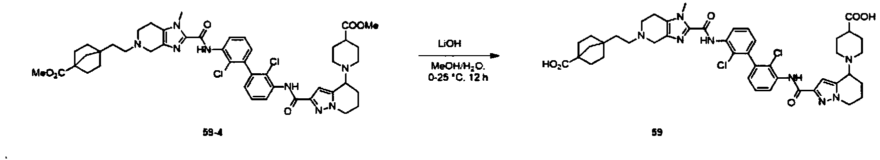

Methyl 1-[2-[[2-chloro-3-[2-chloro-3-[[5-[2-(4-methoxycarbonylnorbornan-1-yl)ethyl]-1-methyl-6,7-dihydro-4H-imidazo[4,5-c]pyridine-2-carbonyl]amino]phenyl]phenyl]carbamoyl]-4,5,6,7-tetrahydropyrazolo[1,5-a]pyridin-4-yl]piperidine-4-carboxylate (60 mg, 67.73 µmol, 1 eq) was dissolved in a mixed solvent of H₂O (3 mL) and MeOH (10 mL). LiOH (4.45 mg, 203.19 µmol, 3 eq) was added at 0 °C, and the resulting mixture was stirred at room temperature for 12 hours. After completion of the reaction, the mixture was concentrated under reduced pressure, and the resulting residue was purified by prep-HPLC (column: Waters Xbridge 15025mm 5µm; mobile phase: [water (NH₄HCO₃)-ACN]; gradient: 15%-45% B over 9 min) to afford Compound 59 (32 mg, 52.3% yield) as a white solid.

LCMS m/z 857.4 [M+1]⁺.

¹H NMR (400 MHz, DMSO-d6 ) δ = 9.90 (s, 1H), 9.54 (s, 1H), 8.37 (dd, J = 1.2, 8.3 Hz, 1H), 8.30 - 8.24 (m, 1H), 7.49 (t, J = 7.3 Hz, 2H), 7.15 (t, J = 8.4 Hz, 2H), 6.67 (s, 1H), 4.20 (br d, J = 13.0 Hz, 1H), 4.11 - 3.93 (m, 2H), 3.90 (s, 3H), 3.42 - 3.39 (m, 3H), 2.81 - 2.71 (m, 3H), 2.67 (br d, J = 3.9 Hz, 3H), 2.58 - 2.52 (m, 3H), 2.45 - 2.38 (m, 1H), 2.36 - 2.10 (m, 4H), 2.00 - 1.93 (m, 1H), 1.84 (br d, J = 13.1 Hz, 4H), 1.75 - 1.59 (m, 4H), 1.55 - 1.48 (m, 4H), 1.43 (s, 2H), 1.38 (br d, J = 10.4 Hz, 2H).

### 15-9. Preparation of Compound 60, 4-(2-(2-((3'-(4-((2-carboxypropan-2-yl)amino)-4,5,6,7-tetrahydropyrazolo[1,5-a]pyridine-2-carboxamido)-2,2'-dichloro-[1,1'-biphenyl]-3-yl)carbamoyl)-1-methyl-6,7-dihydro-1H-imidazo[4,5-c]pyridin-5(4H)-yl)ethyl)bicyclo[2.2.1]heptane-1-carboxylic acid

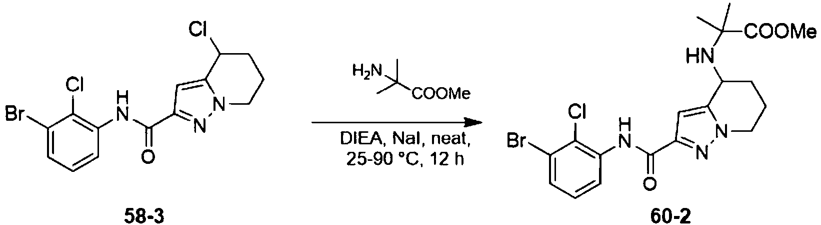

N-(3-bromo-2-chloro-phenyl)-4-chloro-4,5,6,7-tetrahydropyrazolo[1,5-a]pyridine-2-carboxamide (100 mg, 257.02 µmol, 1 eq) was added to a mixture of methyl 2-amino-2-methyl-propanoate (90.33 mg, 771.06 µmol, 3 eq), followed by addition of DIPEA (99.65 mg, 771.06 µmol, 134.30 µL, 3 eq) and Nal (11.56 mg, 77.11 µmol, 0.3 eq) at room temperature. The resulting mixture was stirred at 90 °C for 12 hours. After cooling the mixture to room temperature, H₂O (20 mL) was added, and the mixture was extracted with ethyl acetate (20 mL * 3). The organic layer was washed with brine (40 mL), dried over Na₂SO₄, and concentrated under reduced pressure. The residue was purified by prep-HPLC (column: Waters Xbridge Prep OBD C18 150*40mm*10µm; mobile phase: [water (NH₄HCO₃)-ACN]; gradient: 52%-82% B over 15 min) to afford Compound 60-2 (83 mg, 61.8% yield) as a white solid.

¹H NMR (400 MHz, DMSO-d6) δ = 9.64 (s, 1H), 8.09 (d, J = 8.4 Hz, 1H), 7.62 - 7.57 (m, 1H), 7.34 (t, J = 8.0 Hz, 1H), 6.72 - 6.69 (m, 1H), 4.18 - 4.06 (m, 2H), 3.93 - 3.86 (m, 1H), 3.65 (s, 2H), 3.69 - 3.61 (m, 1H), 2.19 - 2.07 (m, 1H), 1.95 (br d, J = 5.4 Hz, 1H), 1.63 - 1.54 (m, 1H), 1.32 (d, J = 4.3 Hz, 6H).

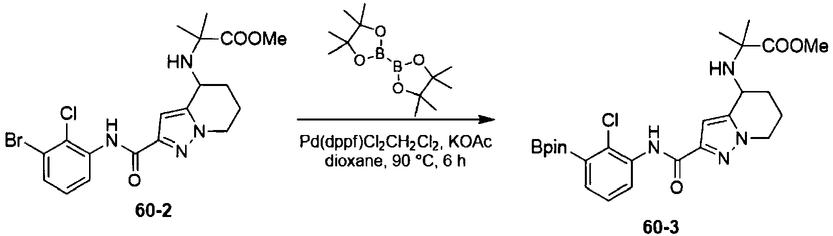

Methyl 2-[[2-[(3-bromo-2-chloro-phenyl)carbamoyl]-4,5,6,7-tetrahydropyrazolo[1,5-a]pyridin-4-yl]amino]-2-methyl-propanoate (80 mg, 170.30 µmol, 1 eq), 4,4,5,5-tetramethyl-2-(4,4,5,5-tetramethyl-1,3,2-dioxaborolan-2-yl)-1,3,2-dioxaborolane (129.74 mg, 510.90 µmol, 3 eq), KOAc (50.14 mg, 510.90 µmol, 3 eq), and cyclopentyl(diphenyl)phosphane;dichloromethane;dichloropalladium;iron (13.91 mg, 17.03 µmol, 0.1 eq) were combined in dioxane (8 mL), and the resulting mixture was stirred under a nitrogen atmosphere at 90 °C for 2 hours. After completion of the reaction, the mixture was filtered, and the filtrate was concentrated under reduced pressure. The resulting residue was purified by prep-TLC (Dichloromethane: Methanol=10/1) to afford Compound 60-3 (112 mg, 99.2% yield) as a white solid.

LCMS m/z 517.2 [M+1]⁺.

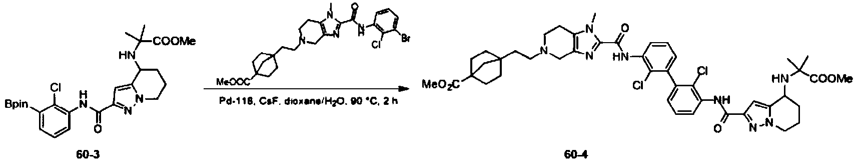

Methyl 4-[2-[2-[(3-bromo-2-chloro-phenyl)carbamoyl]-1-methyl-6,7-dihydro-4H-imidazo[4,5-c]pyridin-5-yl]ethyl]norbornane-1-carboxylate (80 mg, 145.48 µmol, 1 eq), methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (90.23 mg, 174.58 µmol, 1.2 eq), CsF (66.30 mg, 436.45 µmol, 16.11 µL, 3 eq), and ditert-butyl(cyclopentyl)phosphane;dichloropalladium;iron (9.48 mg, 14.55 µmol, 0.1 eq) were combined in a mixture of dioxane (8 mL) and H₂O (2 mL), and the resulting mixture was stirred under a nitrogen atmosphere at 90 °C for 2 hours. After completion of the reaction, the mixture was filtered, and the filtrate was concentrated under reduced pressure. The resulting residue was purified by prep-HPLC (column: C18 150Y30mm; mobile phase: [water (FA)-ACN]; gradient: 25%-55% B over 7 min) to afford Compound 60-4 (32 mg, 24.3% yield) as a white solid.

LCMS m/z 861.2 [M+1]⁺.

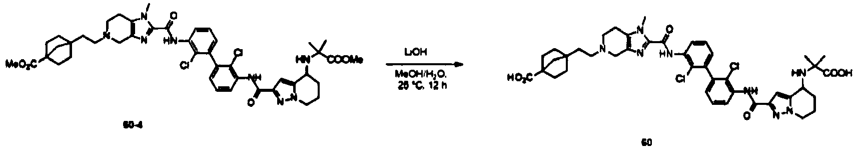

Methyl 4-[2-[2-[[2-chloro-3-[2-chloro-3-[[4-[(2-methoxy-1,1-dimethyl-2-oxo-ethyl)amino]-4,5,6,7-tetrahydropyrazolo[1,5-a]pyridine-2-carbonyl]amino]phenyl]phenyl]carbamoyl]-1-methyl-6,7-dihydro-4H-imidazo[4,5-c]pyridin-5-yl]ethyl]norbornane-1-carboxylate (60 mg, 69.78 µmol, 1 eq) was dissolved in a mixed solvent of H₂O (3 mL) and MeOH (10 mL). LiOH (5.01 mg, 209.34 µmol, 3 eq) was added at 0 °C, and the resulting mixture was stirred at room temperature for 12 hours. After completion of the reaction, the mixture was concentrated under reduced pressure, and the resulting residue was purified by prep-TLC (Dichloromethane: Methanol=10/1) to afford Compound 60 (12 mg, 19.6% yield) as a white solid.

LCMS m/z 831.3 [M+1]⁺.

¹H NMR (400 MHz, DMSO-d6 ) δ = 9.88 (s, 1H), 9.53 (s, 1H), 8.36 (d, J = 7.6 Hz, 1H), 8.27 (d, J = 8.3 Hz, 1H), 7.48 (t, J = 8.3 Hz, 2H), 7.14 (t, J = 8.2 Hz, 2H), 6.79 (s, 1H), 4.14 - 4.07 (m, 2H), 3.92 (br s, 1H), 3.89 (s, 3H), 2.74 (br d, J = 5.1 Hz, 3H), 2.68 - 2.64 (m, 3H), 2.35 - 2.30 (m, 1H), 2.12 (br s, 2H), 2.01 (br s, 2H), 1.88 - 1.77 (m, 3H), 1.76 - 1.66 (m, 3H), 1.65 - 1.58 (m, 1H), 1.56 - 1.45 (m, 5H), 1.42 (s, 2H), 1.39 - 1.34 (m, 2H), 1.28 (d, J = 4.4 Hz, 6H).

### [Preparation Example 16]

### Preparation of Compounds 61 to 66

### 16-1. Preparation of Compound 61, (3R)-1-((7-cyano-2-(3'-(4-hydroxy-4,5,6,7-tetrahydropyrazolo[1,5-a]pyridine-2-carboxamido)-2,2'-dimethyl-[1,1'-biphenyl]-3-yl)benzo[d]oxazol-5-yl)methyl)pyrrolidine-3-carboxylic acid

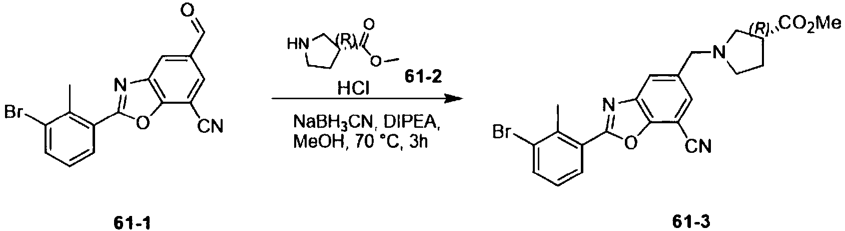

2-(3-bromo-2-methyl-phenyl)-5-formyl-1,3-benzoxazole-7-carbonitrile (8 g, 23.45 mmol, 1 eq) was added to a mixture of MeOH (100 mL), followed by addition of NaBH₃CN (7.37 g, 117.25 mmol, 5 eq), methyl (3R)-pyrrolidine-3-carboxylate (4.54 g, 35.17 mmol, 1.5 eq), and DIPEA (6.06 g, 46.90 mmol, 8.17 mL, 2 eq). The resulting mixture was stirred at 70 °C for 3 hours. After completion of the reaction, the reaction mixture was concentrated under reduced pressure to remove MeOH and then diluted with H₂O (400 mL). The aqueous phase was extracted with EA (150 mL X 3). The combined organic layers were dried over Na₂SO₄ and concentrated under reduced pressure to afford Compound 61-3 (8 g, 67.5% yield), which was used in the next reaction without further purification.

LCMS m/z 456.2 [M+2]⁺.

¹H NMR (400 MHz, DMSO-d6) δ = 8.12 - 8.00 (m, 2H), 7.89 - 7.78 (m, 2H), 7.37 (t, J = 7.9 Hz, 1H), 3.80 - 3.64 (m, 3H), 3.04 (br d, J = 9.4 Hz, 1H), 2.75 (s, 3H), 2.73 - 2.54 (m, 2H), 2.31 - 1.67 (m, 2H).

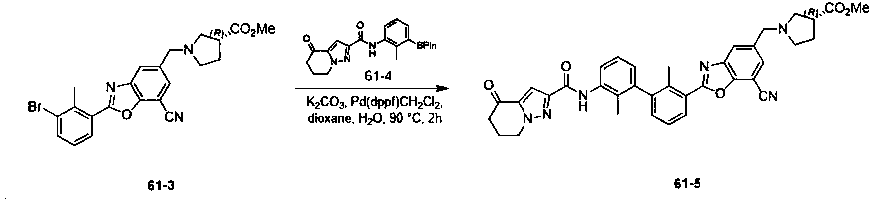

Methyl (3R)-1-[[2-(3-bromo-2-methyl-phenyl)-7-cyano-1,3-benzoxazol-5-yl]methyl]pyrrolidine-3-carboxylate (520 mg, 1.14 mmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (450.60 mg, 1.14 mmol, 1 eq), K₂CO₃ (441.17 mg, 3.19 mmol, 2.8 eq), and Pd(dppf)Cl₂·CH₂Cl₂ (46.55 mg, 57.00 µmol, 0.05 eq) were combined in a mixture of dioxane (5 mL) and H₂O (0.6 mL), and the resulting mixture was stirred under a nitrogen atmosphere at 90 °C for 2 hours. After completion of the reaction, the reaction mixture was diluted with H₂O (40 mL) and extracted with ethyl acetate (30 mL x 3). The organic layer was washed with brine (60 mL), dried over Na₂SO₄, and concentrated under reduced pressure. The residue was purified by flash silica gel chromatography (Petroleum ether/Ethyl acetate=100/1 to 3/1) to afford Compound 61-5 (502 mg, 61.6% yield) as a white solid.

¹H NMR (400 MHz, CHLOROFORM-d) δ = 8.73 (s, 1H), 8.30 - 8.13 (m, 2H), 8.00 (d, J = 0.9 Hz, 1H), 7.69 (d, J = 1.3 Hz,1H), 7.49 (s, 1H), 7.48 - 7.42 (m, 1H), 7.39 - 7.32 (m, 2H), 7.02 (d, J = 6.6 Hz, 1H), 4.52 - 4.43 (m, 2H), 3.78 (s, 2H), 3.71 (s,3H), 3.13 - 3.03 (m, 1H), 2.91 (t, J = 8.8 Hz, 1H), 2.81 - 2.67 (m, 4H), 2.65 - 2.57 (m, 1H), 2.51 (s, 3H), 2.45 (q, J = 6.2 Hz,2H), 2.20 - 2.12 (m, 2H), 2.10 (s, 3H).

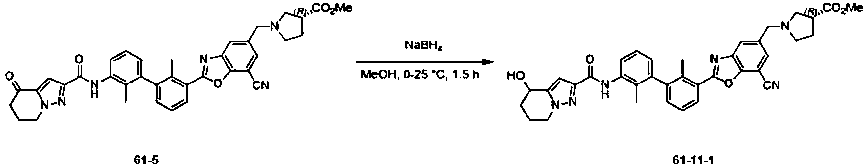

Methyl (3R)-1-[[7-cyano-2-[2-methyl-3-[2-methyl-3-[(4-oxo-6,7-dihydro-5H-pyrazolo[1,5-a]pyridine-2-carbonyl)amino]phenyl]phenyl]-1,3-benzoxazol-5-yl]methyl]pyrrolidine-3-carboxylate (450 mg, 700.17 µmol, 1 eq) was dissolved in MeOH (6 mL), and NaBH₄ (88.00 mg, 1.40 mmol, 2 eq) was added at 0 °C. The reaction mixture was stirred at room temperature for 1.5 hours. After completion of the reaction, the reaction mixture was diluted with H₂O (40 mL) and extracted with ethyl acetate (30 mL x 3). The organic layer was washed with brine (60 mL), dried over Na₂SO₄, and concentrated under reduced pressure. The residue was purified by flash silica gel chromatography (Petroleum ether/Ethyl acetate=100/1 to 1/1) to afford Compound 61-11-1 (432 mg, 86.13% yield) as a white solid.

LCMS m/z 645.3 [M+1]⁺.

¹H NMR (400 MHz, CHLOROFORM-d) δ = 8.72 (s, 1H), 8.30 - 8.12 (m, 2H), 8.00 (s, 1H), 7.69 (s, 1H), 7.52 - 7.40 (m, 1H), 7.38 - 7.29 (m, 2H), 6.99 (d, J = 7.5 Hz, 1H), 6.91 (s, 1H), 5.07 - 4.89 (m, 1H), 4.30 - 4.16 (m, 1H), 3.78 (s, 2H), 3.70 (s, 3H), 3.16 - 3.01 (m, 1H), 2.91 (br t, J = 8.5 Hz, 1H), 2.81 - 2.66 (m, 2H), 2.65 - 2.56 (m, 1H), 2.50 (s, 3H), 2.42 - 2.19 (m, 2H), 2.14(br d, J = 7.4 Hz, 2H), 2.08 (s, 3H), 2.03 - 1.91 (m, 2H), 0.00 (s, 2H).

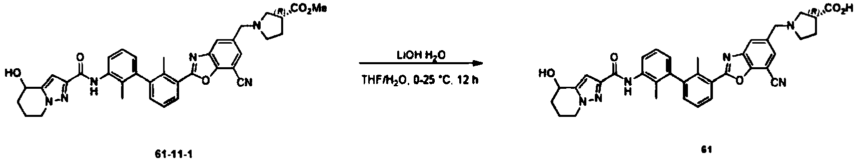

Methyl (3R)-1-[[7-cyano-2-[3-[3-[(4-hydroxy-4,5,6,7-tetrahydropyrazolo[1,5-a]pyridine-2-carbonyl)amino]-2-methyl-phenyl]-2-methyl-phenyl]-1,3-benzoxazol-5-yl]methyl]pyrrolidine-3-carboxylate (380 mg, 589.41 µmol, 13.81 µL, 1 eq) was added to a mixture of THF (8 mL) and H₂O (8 mL), and LiOH·H₂O (74.19 mg, 1.77 mmol, 3 eq) was added at 0 °C. The resulting mixture was stirred at room temperature for 12 hours. After completion of the reaction, the pH of the reaction mixture was adjusted to 7 with aq. 1N HCl, and the precipitated solid was collected by filtration. The filtered solid was purified by prep-HPLC (column: Waters Xbridge C18 150*50mm* 10um; mobile phase: [water (NH₄HCO₃)-ACN]; B%: 18%) to afford Compound 61 (128 mg, yield 95%) as a white solid.

LCMS m/z 631.3 [M+1]⁺.

¹H NMR (400 MHz, DMSO-d6) δ = 9.58 (s, 1H), 8.21 - 8.06 (m, 2H), 7.89 (d, J = 1.1 Hz, 1H), 7.62 (d, J = 8.0 Hz, 1H), 7.56 (t, J = 7.7 Hz, 1H), 7.41 (d, J = 6.6 Hz, 1H), 7.32 (t, J = 7.8 Hz, 1H), 7.04 (d, J = 7.0 Hz, 1H), 6.70 (s, 1H), 5.68 - 5.45 (m, 1H), 4.77 (br t, J = 5.7 Hz, 1H), 4.18 - 4.10 (m, 2H), 3.76 (br d, J = 7.8 Hz, 2H), 3.04 - 2.89 (m, 2H), 2.81 - 2.63 (m, 3H), 2.61 - 2.56 (m, 2H), 2.43 (s, 3H), 2.25 - 2.12 (m, 1H), 2.10 - 1.97 (m, 3H), 1.95 (s, 3H), 1.80 - 1.66 (m, 1H).

### 16-2. Preparation of Compound 62, (3R)-1-((2-(3'-(4-((2-acetamidoethyl)amino)-4,5,6,7-tetrahydropyrazolo[1,5-a]pyridine-2-carboxamido)-2,2'-dimethyl-[1,1'-biphenyl]-3-yl)-7-cyanobenzo[d]oxazol-5-yl)methyl)pyrrolidine-3-carboxylic acid

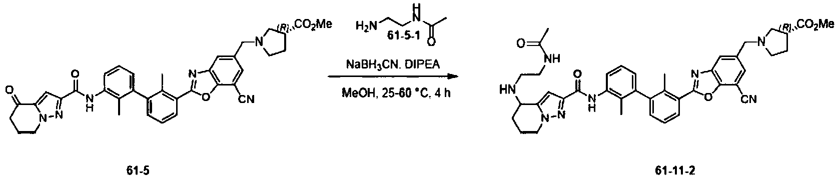

Methyl (3R)-1-[[7-cyano-2-[2-methyl-3-[2-methyl-3-[(4-oxo-6,7-dihydro-5H-pyrazolo[1,5-a]pyridine-2-carbonyl)amino]phenyl]phenyl]-1,3-benzoxazol-5-yl]methyl]pyrrolidine-3-carboxylate (200 mg, 311.19 µmol, 1 eq) was added to a mixture of MeOH (3 mL) and N-(2-aminoethyl)acetamide (317.83 mg, 3.11 mmol, 297.04 µL, 10 eq), followed by addition of DIPEA (40.22 mg, 311.19 µmol, 54.20 µL, 1 eq) at room temperature. The reaction mixture was stirred at 60 °C for 2 hours. NaBH₃CN (39.11 mg, 622.37 µmol, 2 eq) was then added, and the reaction mixture was stirred at 60 °C for an additional 2 hours. After completion of the reaction, the mixture was cooled to room temperature and concentrated under reduced pressure. The resulting residue was purified by prep-HPLC (column: Phenomenex luna C18 15025mm 10um; mobile phase: [water (FA)-ACN]; B%: 10%-40%, 10 min) to afford Compound 61-11-2 (126 mg, 52.7% yield) as a white solid.

¹H NMR (400 MHz, DMSO-d6) δ = 9.56 (d, J = 2.4 Hz, 1H), 8.31 - 8.05 (m, 3H), 7.95 - 7.75 (m, 2H), 7.65 - 7.49 (m, 2H), 7.41(d, J = 7.3 Hz, 1H), 7.32 (t, J = 7.8 Hz, 1H), 7.03 (d, J = 7.4 Hz, 1H), 6.74 (s, 1H), 4.26 - 4.06 (m, 2H), 3.95 - 3.82 (m, 2H),3.76 (s, 2H), 3.61 (s, 5H), 3.20 - 3.11 (m, 3H), 3.06 (br d, J = 1.0 Hz, 2H), 2.82 - 2.72 (m, 1H), 2.70 - 2.61 (m, 3H), 2.59 - 2.53(m, 3H), 2.43 (s, 3H), 2.19 (td, J = 5.8, 7.7 Hz, 1H), 2.09 - 1.96 (m, 3H), 1.95 (s, 3H), 1.81 (s, 3H), 1.74 - 1.62 (m, 1H).

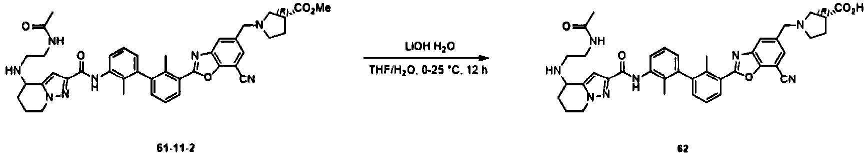

To a solution of methyl (3R)-1-[[2-[3-[3-[[4-(2-acetamidoethylamino)-4,5,6,7-tetrahydropyrazolo[1,5-a]pyridine-2-carbonyl]amino]-2-methyl-phenyl]-2-methyl-phenyl]-7-cyano-1,3-benzoxazol-5-yl]methyl]pyrrolidine-3-carboxylate (100 mg, 137.20 µmol, 1 eq) in THF (8 mL)/H₂O (4 mL), LiOH·H₂O (17.27 mg, 411.61 µmol, 3 eq) was added at 0 °C. The resulting mixture was stirred at room temperature for 12 hours. After completion of the reaction, the pH of the reaction mixture was adjusted to 7 with aq. 1 N HCl, and the precipitated solid was collected by filtration. The filtered solid was purified by prep-HPLC (column: Waters Xbridge C18 150*50mm* 10 µm; mobile phase: [water (NH₄HCO₃)-ACN]; B%: 16%-46%, 10 min) to afford Compound 62 (48 mg, 46.4% yield) as a white solid.

LCMS m/z 715.4 [M+1]⁺.

¹H NMR (400 MHz, DMSO-d6) δ = 9.55 (d, J = 2.6 Hz, 1H), 8.21 - 8.08 (m, 2H), 7.88 (s, 1H), 7.83 (t, J = 5.3 Hz, 1H), 7.61 (d, J = 8.1 Hz, 1H), 7.55 (t, J = 7.7 Hz, 1H), 7.40 (d, J = 6.8 Hz, 1H), 7.34 - 7.26 (m, 1H), 7.03 (d, J = 7.1 Hz, 1H), 6.73 (s,1H), 4.20 - 4.06 (m, 2H), 3.90 - 3.84 (m, 1H), 3.81 - 3.62 (m, 3H), 3.13 (q, J = 6.3 Hz, 2H), 3.00 - 2.90 (m, 1H), 2.78 - 2.70 (m,1H), 2.69 - 2.60 (m, 3H), 2.56 - 2.53 (m, 2H), 2.42 (s, 3H), 2.18 (s, 1H), 2.04 - 1.96 (m, 3H), 1.95 (s, 3H), 1.80 (s, 3H), 1.67 (dd, J = 2.4, 10.3 Hz, 1H).

### 16-3. Preparation of Compound 63, (3R)-1-((7-cyano-2-(3'-(4-((R)-3-hydroxypyrrolidin-1-yl)-4,5,6,7-tetrahydropyrazolo[1,5-a]pyridine-2-carboxamido)-2,2'-dimethyl-[1,1'-biphenyl]-3-yl)benzo[d]oxazol-5-yl)methyl)pyrrolidine-3-carboxylic acid

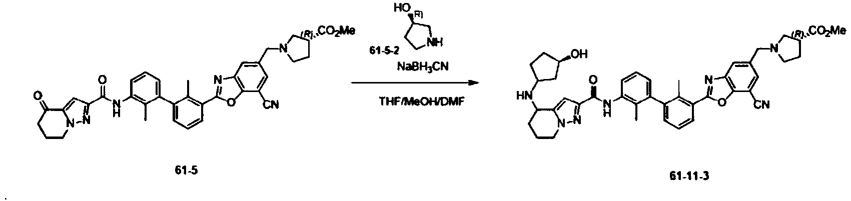

Methyl (3R)-1-[[7-cyano-2-[2-methyl-3-[2-methyl-3-[(4-oxo-6,7-dihydro-5H-pyrazolo[1,5-a]pyridine-2-carbonyl)amino]phenyl]phenyl]-1,3-benzoxazol-5-yl]methyl]pyrrolidine-3-carboxylate (200 mg, 311.19 µmol, 1 eq) and (3R)-pyrrolidin-3-ol (271.11 mg, 3.11 mmol, 258.20 µL, 10 eq) were dissolved in a mixed solvent of MeOH (30 mL), THF (6 mL), and DMF (2 mL). HOAc (1.05 g, 17.48 mmol, 1000 µL, 56.19 eq) was added at room temperature, and the reaction mixture was stirred at 60 °C for 2 hours. NaBH₃CN (97.78 mg, 1.56 mmol, 5 eq) was then added, and the reaction mixture was stirred at 60 °C for an additional 2 hours. After completion of the reaction, the mixture was cooled to room temperature and concentrated under reduced pressure. The resulting residue was purified by prep-HPLC (column: Phenomenex luna C18 150*25mm* 10 µm; mobile phase: [water (FA)-ACN]; B%: 8%-50%, 14 min) to afford Compound 61-11-3 (120 mg, 51.3% yield) as a white solid.

¹H NMR (400 MHz, DMSO-d6) δ = 9.67 (s, 1H), 8.20 - 8.11 (m, 3H), 7.98 - 7.87 (m, 1H), 7.59 - 7.53 (m, 2H), 7.41 (d, J =7.5 Hz, 1H), 7.32 (t, J = 7.8 Hz, 1H), 7.05 (d, J = 7.0 Hz, 1H), 6.52 (s, 1H), 4.44 - 4.13 (m, 4H), 4.00 - 3.73 (m, 2H), 3.62 (s, 4H), 3.20 - 3.04 (m, 3H), 2.67 (d, J = 2.0 Hz, 2H), 2.43 (s, 4H), 2.33 (s, 1H), 2.27 - 2.22 (m, 1H), 2.13 - 1.98 (m, 5H), 1.95 (s, 5H), 1.84 - 1.62 (m, 1H).

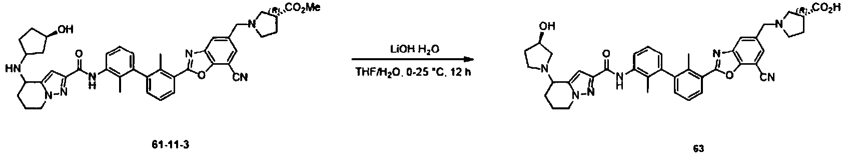

Methyl (3R)-1-[[7-cyano-2-[3-[3-[[4-[(3R)-3-hydroxypyrrolidin-1-yl]-4,5,6,7-tetrahydropyrazolo[1,5-a]pyridine-2-carbonyl]amino]-2-methyl-phenyl]-2-methyl-phenyl]-1,3-benzoxazol-5-yl]methyl]pyrrolidine-3-carboxylate (120 mg, 168.11 µmol, 1 eq) was dissolved in a mixed solvent of THF (3 mL) and H₂O (3 mL), and LiOH·H₂O (21.16 mg, 504.33 µmol, 3 eq) was added at 0 °C. The reaction mixture was allowed to react at room temperature for 12 hours. After completion of the reaction, the pH of the reaction mixture was adjusted to 7 with aq. 1N HCl, and the precipitated solid was collected by filtration. The filtered solid was purified by prep-HPLC (column: Waters Xbridge C18 150*50mm* 10µm; mobile phase: [water (NH₄HCO₃)-ACN]; B%: 20%-50%, 10min) to afford Compound 63 (80 mg, 64.6 yield) as a white solid.

LCMS m/z 730.3 [M+1]⁺.

¹H NMR (400 MHz, DMSO-d6) δ = 9.57 (d, J = 3.8 Hz, 1H), 8.19 - 8.08 (m, 2H), 7.88 (d, J = 1.0 Hz, 1H), 7.55 (s, 2H), 7.40 (d, J = 6.9 Hz, 1H), 7.31 (t, J = 7.8 Hz, 1H), 7.03 (d, J = 7.4 Hz, 1H), 6.68 (d, J = 7.3 Hz, 1H), 4.88 - 4.54 (m, 1H), 4.21 - 4.12 (m, 3H), 3.83 - 3.73 (m, 4H), 2.97 - 2.87 (m, 2H), 2.81 - 2.71 (m, 2H), 2.71 - 2.59 (m, 3H), 2.57 - 2.54 (m, 2H), 2.42 (s, 3H), 2.28 - 2.17 (m, 1H), 2.02 - 1.96 (m, 2H), 1.95 (s, 3H), 1.89 (s, 2H), 1.56 (td, J = 4.1, 8.0 Hz, 1H).

### 16-4. Preparation of Compound 64, (3R)-1-((7-cyano-2-(3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)benzo[d]oxazol-5-yl)methyl)pyrrolidine-3-carboxylic acid

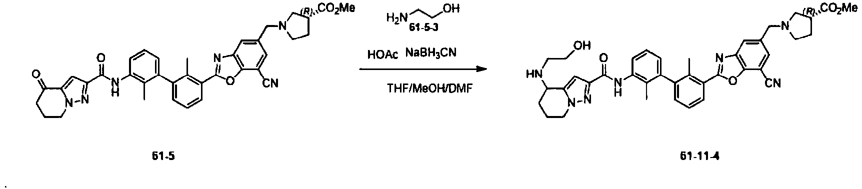

A mixture of methyl (3R)-1-[[7-cyano-2-[2-methyl-3-[2-methyl-3-[(4-oxo-6,7-dihydro-5H-pyrazolo[1,5-a]pyridine-2-carbonyl)amino]phenyl]phenyl]-1,3-benzoxazol-5-yl]methyl]pyrrolidine-3-carboxylate (200 mg, 311.19 µmol, 1 eq) and 2-aminoethanol (190.08 mg, 3.11 mmol, 188.20 µL, 10 eq) was dissolved in a mixed solvent of MeOH (30 mL), THF (6 mL), and DMF (2 mL). HOAc (1.05 g, 17.49 mmol, 1000 µL, 56.19 eq) was added at room temperature, and the reaction mixture was stirred at 60 °C for 2 hours. NaBH₃CN (39.11 mg, 622.38 µmol, 2 eq) was then added, and the reaction mixture was stirred at 60 °C for an additional 2 hours. After completion of the reaction, the reaction mixture was concentrated under reduced pressure, and the resulting residue was purified by prep-HPLC (column: Phenomenex luna C18 150*25mm*10um; mobile phase: [water (FA)-ACN]; B%: 8%-50%, 14min) to afford Compound 61-11-4 (132 mg, 55.5% yield) as a white solid.

¹H NMR (400 MHz, DMSO-d6) δ = 9.60 (d, J = 2.5 Hz, 1H), 8.18 - 8.05 (m, 3H), 7.87 (d, J = 1.0 Hz, 1H), 7.63 - 7.51 (m, 2H), 7.44 - 7.38 (m, 1H), 7.31 (t, J = 7.8 Hz, 1H), 7.03 (d, J = 7.0 Hz, 1H), 6.81 (s, 1H), 4.20 - 4.12 (m, 3H), 4.12 - 4.04 (m, 3H), 4.01 - 3.87 (m, 5H), 3.76 (d, J = 2.0 Hz, 5H), 3.60 (s, 3H), 3.53 (t, J = 5.8 Hz, 2H), 3.14 - 2.99 (m, 1H), 2.83 - 2.72 (m, 3H), 2.72 - 2.64 (m, 1H), 2.56 (t, J = 7.3 Hz, 3H), 2.42 (s, 3H), 2.27 - 2.15 (m, 1H), 2.13 - 1.97 (m, 3H), 1.94 (s, 3H), 1.85 -1.72 (m, 1H).

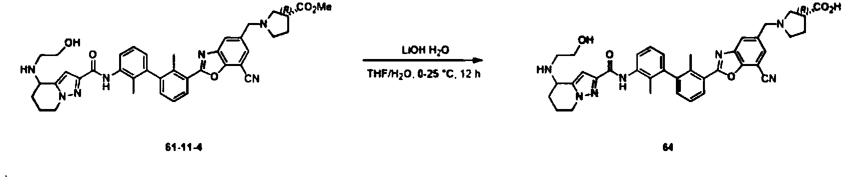

Methyl (3R)-1-[[7-cyano-2-[3-[3-[[4-(2-hydroxyethylamino)-4,5,6,7-tetrahydropyrazolo[1,5-a]pyridine-2-carbonyl]amino]-2-methyl-phenyl]-2-methyl-phenyl]-1,3-benzoxazol-5-yl]methyl]pyrrolidine-3-carboxylate (120 mg, 174.47 µmol, 1 eq) was dissolved in a mixed solvent of THF (3 mL) and H₂O (3 mL), and LiOH·H₂O (21.96 mg, 523.42 µmol, 3 eq) was added at 0 °C. The reaction mixture was stirred at room temperature for 12 hours. After completion of the reaction, the pH of the reaction mixture was adjusted to 7 with aq. 1N HCl, and the precipitated solid was collected by filtration. The filtered solid was purified by prep-HPLC (column: Waters Xbridge C18 150*50mm*10um; mobile phase: [water (NH₄HCO₃)-ACN]; B%: 18%-48%, 10min) to afford Compound 64 (76 mg, 61.4% yield) as a white solid.

LCMS m/z 674.6 [M+1]⁺.

¹H NMR (400 MHz, DMSO-d6) δ = 9.55 (d, J = 2.4 Hz, 1H), 8.21 - 8.08 (m, 2H), 7.87 (d, J = 1.1 Hz, 1H), 7.64 - 7.50 (m, 2H), 7.48 - 7.36 (m, 1H), 7.31 (t, J = 7.8 Hz, 1H), 7.03 (d, J = 6.8 Hz, 1H), 6.72 (s, 1H), 4.18 - 4.10 (m, 2H), 3.90 - 3.86 (m, 1H), 3.75 (d, J = 7.6 Hz, 3H), 3.48 (t, J = 5.8 Hz, 4H), 2.98 - 2.89 (m, 1H), 2.76 - 2.65 (m, 4H), 2.58 - 2.53 (m, 2H), 2.42 (s, 3H), 2.24 - 2.13 (m, 1H), 2.04 - 1.96 (m, 3H), 1.95 (s, 3H), 1.76 - 1.62 (m, 1H).

### 16-5. Preparation of Compound 65, (3R)-1-((7-cyano-2-(2,2'-dimethyl-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid

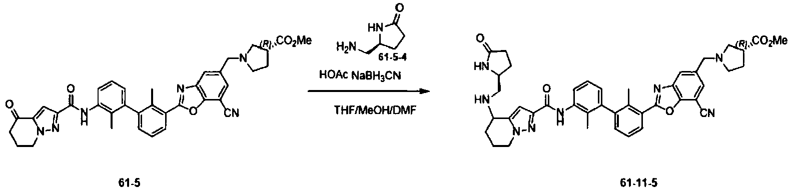

Methyl (3R)-1-[[7-cyano-2-[2-methyl-3-[2-methyl-3-[(4-oxo-6,7-dihydro-5H-pyrazolo[1,5-a]pyridine-2-carbonyl)amino]phenyl]phenyl]-1,3-benzoxazol-5-yl]methyl]pyrrolidine-3-carboxylate (200 mg, 311.19 µmol, 1 eq) and (5S)-5-(aminomethyl)pyrrolidin-2-one (355.21 mg, 3.11 mmol, 297.04 µL, 10 eq) were dissolved in a mixed solvent of MeOH (30 mL), THF (6 mL), and DMF (1 mL). DIPEA (402.18 mg, 3.11 mmol, 542.02 µL, 10 eq) was added at room temperature, and the reaction mixture was stirred at 60 °C for 2 hours. NaBH₃CN (97.78 mg, 1.56 mmol, 5 eq) was then added, and the reaction mixture was stirred at 60 °C for an additional 2 hours. After completion of the reaction, the reaction mixture was concentrated under reduced pressure, and the resulting residue was purified by prep-HPLC (column: Phenomenex luna C18 150*25mm*10um; mobile phase: [water (FA)-ACN]; B%: 8%-38%, 10min) to afford Compound 61-11-5 (140 mg, 57.6% yield) as a white solid.

¹H NMR (400 MHz, DMSO-d6) δ = 9.55 (s, 1H), 8.17 - 8.14 (m, 3H), 8.12 (d, J = 1.1 Hz, 1H), 7.88 (d, J = 1.1 Hz, 1H), 7.69 (d, J = 19.6 Hz, 1H), 7.63 - 7.59 (m, 1H), 7.55 (t, J = 7.8 Hz, 1H), 7.44 - 7.38 (m, 1H), 7.31 (t, J = 7.8 Hz, 1H), 7.03 (d, J = 7.4 Hz, 1H), 6.77 (d, J = 9.6 Hz, 1H), 4.24 - 4.06 (m, 3H), 3.95 - 3.85 (m, 2H), 3.81 - 3.74 (m, 3H), 3.60 (s, 3H), 3.58 (s, 2H), 3.06 (dd, J = 8.3, 9.8 Hz, 1H), 2.79 - 2.65 (m, 3H), 2.62 - 2.53 (m, 4H), 2.42 (s, 3H), 2.20 - 2.08 (m, 3H), 2.07 - 1.97 (m, 3H), 1.95 (s, 4H), 1.77 - 1.62 (m, 2H).

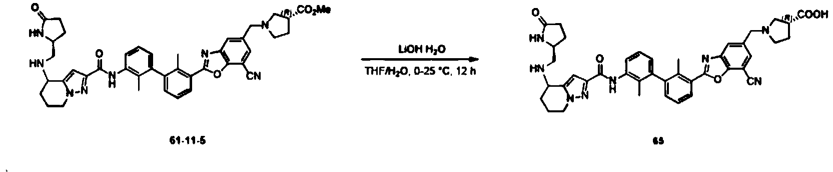

A solution of methyl (3R)-1-[[7-cyano-2-[2-methyl-3-[2-methyl-3-[[4-[[(2S)-5-oxopyrrolidin-2-yl]methylamino]-4,5,6,7-tetrahydropyrazolo[1,5-a]pyridine-2-carbonyl]amino]phenyl]phenyl]-1,3-benzoxazol-5-yl]methyl]pyrrolidine-3-carboxylate (130 mg, 175.47 µmol, 1 eq) in THF (3 mL) and H₂O (1 mL) was treated with LiOH·H₂O (22.09 mg, 526.42 µmol, 3 eq) at 0 °C. The reaction mixture was then stirred at room temperature for 12 hours. After completion of the reaction, the pH of the reaction mixture was adjusted to 7 using an aqueous 1 N HCl solution, and the precipitated solid was collected by filtration. The obtained solid was purified by prep-HPLC (column: Waters XBridge C18, 150 * 50 mm * 10 µm; mobile phase: water (NH₄HCO₃)-ACN; B%: 18-48% over 10 min) to afford Compound 65 (72 mg, 53.6% yield) as a white solid.

LCMS m/z 727.2 [M+1]⁺.

¹H NMR (400 MHz, DMSO-d6) δ = 9.55 (s, 1H), 8.21 - 8.08 (m, 2H), 7.88 (d, J = 1.0 Hz, 1H), 7.69 (d, J = 19.8 Hz, 1H), 7.61 (d, J = 8.0 Hz, 1H), 7.55 (t, J = 7.7 Hz, 1H), 7.40 (d, J = 6.9 Hz, 1H), 7.31 (t, J = 7.8 Hz, 1H), 7.03 (d, J = 7.4 Hz, 1H), 6.77 (d, J = 9.8 Hz, 1H), 4.18 - 4.09 (m, 2H), 3.91 - 3.85 (m, 1H), 3.75 (d, J = 7.5 Hz, 3H), 3.58 (s, 3H), 2.94 (d, J = 7.4 Hz, 1H), 2.77 - 2.70 (m, 1H), 2.68 - 2.58 (m, 3H), 2.58 - 2.53 (m, 2H), 2.42 (s, 3H), 2.11 (s, 4H), 2.01 - 1.96 (m, 2H), 1.95 (s,3H), 1.76 - 1.62 (m, 2H).

### 16-6. Preparation of Compound 66, (3R)-1-((2-(3'-(4-((carboxymethyl)amino)-4,5,6,7-tetrahydropyrazolo[1,5-a]pyridine-2-carboxamido)-2,2'-dimethyl-[1,1'-biphenyl]-3-yl)-7-cyanobenzo[d]oxazol-5-yl)methyl)pyrrolidine-3-carboxylic acid

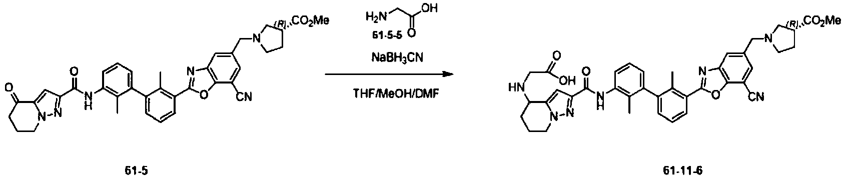

A mixture of methyl (3R)-1-[[7-cyano-2-[2-methyl-3-[2-methyl-3-[(4-oxo-6,7-dihydro-5H-pyrazolo[1,5-a]pyridine-2-carbonyl)amino]phenyl]phenyl]-1,3-benzoxazol-5-yl]methyl]pyrrolidine-3-carboxylate (200 mg, 311.19 µmol, 1 eq) and 2-aminoacetic acid (233.60 mg, 3.11 mmol, 297.04 µL, 10 eq) in MeOH (30 mL)/DMF (1 mL)/THF (6 mL) was stirred at 60 °C for 2 hours. NaBH₃CN (97.78 mg, 1.56 mmol, 5 eq) was added, and the mixture was stirred at 60 °C for 2 hours. After completion of the reaction, the reaction mixture was concentrated under reduced pressure, and the residue was purified by prep-HPLC (column: Phenomenex Luna C18 150*40 mm*15 µm; mobile phase: [water (FA)-ACN]; B%: 10%-40%, 9 min) to afford Compound 61-11-6 (150 mg, 65.2% yield) as a white solid.

¹H NMR (400 MHz, DMSO-d6) δ = 9.60 (d, J = 1.4 Hz, 1H), 8.17 - 8.09 (m, 2H), 7.88 (d, J = 1.1 Hz, 1H), 7.64 - 7.50 (m, 2H), 7.31 (t, J = 7.8 Hz, 1H), 7.03 (d, J = 7.0 Hz, 1H), 6.77 (s, 1H), 4.22 - 4.12 (m, 2H), 4.06 (s, 1H), 3.76 (d, J = 1.3 Hz, 2H), 3.60 (s, 3H), 3.33 (s, 5H), 3.13 - 2.97 (m, 2H), 2.80 - 2.64 (m, 4H), 2.61 - 2.54 (m, 4H), 2.43 (s, 3H), 2.25 - 2.15 (m, 1H), 2.08 - 1.97 (m, 3H), 1.95 (s, 3H), 1.85 - 1.69 (m, 1H).

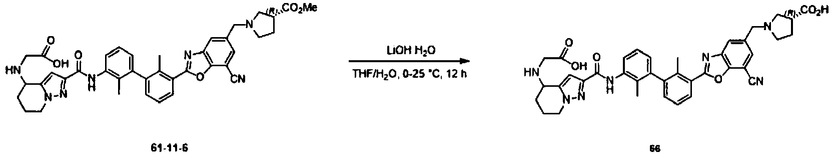

A solution of 2-[[2-[[3-[3-[7-cyano-5-[[(3R)-3-methoxycarbonylpyrrolidin-1-yl]methyl]-1,3-benzoxazol-2-yl]-2-methyl-phenyl]-2-methyl-phenyl]carbamoyl]-4,5,6,7-tetrahydropyrazolo[1,5-a]pyridin-4-yl]amino]acetic acid (120 mg, 171.00 µmol, 1 eq) in THF (3 mL)/H₂O (1 mL) was treated with LiOH·H₂O (21.53 mg, 513.00 µmol, 3 eq) at 0 °C, and the reaction mixture was then stirred at room temperature for 12 hours. After completion of the reaction, the pH of the reaction mixture was adjusted to 7 using an Aq. 1N HCl solution, and the precipitated solid was collected by filtration. The collected solid was purified by prep-HPLC (column: Waters Xbridge C18 150*50 mm*10 um; mobile phase: [water (NH₄HCO₃)-ACN]; B%: 18%-48%, 10 min) to afford Compound 66 (82 mg, 66.24% yield) as a white solid.

LCMS m/z 688.2 [M+1]⁺.

¹H NMR (400 MHz, DMSO-d6) δ = 9.59 (s, 1H), 8.19 - 8.08 (m, 2H), 7.86 (s, 1H), 7.60 (d, J = 8.0 Hz, 1H), 7.54 (s, 1H), 7.39 (d, J = 7.4 Hz, 1H), 7.30 (t, J = 7.8 Hz, 1H), 7.02 (d, J = 7.5 Hz, 1H), 6.77 (s, 1H), 4.14 (d, J = 6.1 Hz, 3H), 4.05 (d, J = 5.8 Hz, 3H), 3.74 (d, J = 7.9 Hz, 3H), 3.26 (s, 2H), 2.97 - 2.88 (m, 1H), 2.79 - 2.70 (m, 1H), 2.69 - 2.62 (m, 1H), 2.57 - 2.53 (m, 2H), 2.42 (s, 3H), 2.27 - 2.15 (m, 1H), 2.03 - 1.97 (m, 2H), 1.94 (s, 3H), 1.75 (d, J = 5.6 Hz, 1H).

### [Preparation Example 17]

### Preparation of Compounds 67 to 70

### 17-1. Preparation of Compound 67, (3R)-1-((7-cyano-2-(2,2'-dichloro-3'-(4-hydroxy-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid

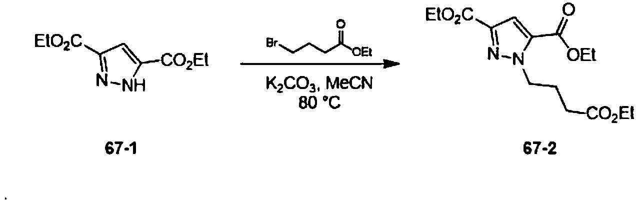

A solution of Compound 67-1 (25 g, 117.81 mmol, 1 eq) in MeCN (200 mL) was treated with K₂CO₃ (17.00 g, 123.00 mmol, 1.04 eq) and ethyl 4-bromobutanoate (16.9 mL, 117.92 mmol, 1 eq), and the reaction mixture was stirred at 80 °C for 4 hours. After completion of the reaction, the reaction mixture was filtered, and the filtrate was concentrated under reduced pressure to afford Compound 67-2 (60.3 g, 74.5% yield) as a white solid.

LCMS m/z 327.2 [M+1]⁺.

¹H NMR (400 MHz, CDCl3) δ 7.33 (s, 1H), 4.69 (t, J = 6.9 Hz, 2H), 4.42 - 4.32 (m, 4H), 4.10 (q, J = 7.2 Hz, 2H), 2.36 - 2.29 (m, 2H), 2.23 - 2.15 (m, 2H), 1.38 (dt, J = 4.1, 7.1 Hz, 6H), 1.23 (t, J = 7.2 Hz, 3H).

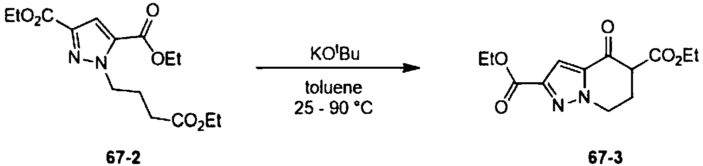

A solution of Compound 67-2 (30 g, 91.93 mmol, 1 eq) in toluene (280 mL) was slowly treated with KOtBu (11.35 g, 101.12 mmol, 1.1 eq) at room temperature over 5 minutes. The reaction mixture was stirred at room temperature for 25 minutes and then stirred at 90 °C for 3 hours. To the reaction mixture were sequentially added EA (600 mL), aq. NH₄Cl (700 mL), and aq. 1 M HCl (350 mL), followed by extraction with EA (400 mL * 3). The organic layer was washed with aq. NH₄Cl (700 mL * 2), dried over Na₂SO₄, and concentrated under reduced pressure. To the residue was added a Petroleum ether: EA = 10:1 (100 mL) solution, and the mixture was stirred at room temperature for 20 minutes to precipitate a solid. The solid was collected by filtration to afford Compound 67-3 (23.9 g, 92.7% yield) as a white solid.

LCMS m/z 281.1 [M+1]⁺.

¹H NMR (400 MHz, CDCl3) δ 12.03 (s, 1H), 7.15 (s, 1H), 4.42 (q, J = 7.2 Hz, 2H), 4.35 - 4.29 (m, 4H), 2.91 (t, J = 7.5 Hz, 2H), 1.43 - 1.34 (m, 6H).

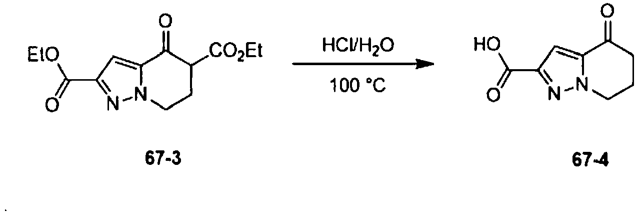

A mixture of Compound 67-3 (23.9 g, 85.27 mmol, 1 eq) and HCl (306.00 g, 3.11 mol, 300 mL, 36.42 eq) in H₂O (150 mL) was stirred at 100 °C for 6 hours under a nitrogen atmosphere. After completion of the reaction, the reaction mixture was concentrated under reduced pressure. MeCN (30 mL) was added to the residue, and the mixture was stirred at room temperature for 20 minutes to precipitate a solid. The solid was collected by filtration to afford Compound 67-4 (12.9 g, 83.9% yield) as a white solid.

¹H NMR (400 MHz, DMSO-d6) δ 13.02 (br s, 1H), 7.13 (s, 1H), 4.43 - 4.38 (m, 2H), 2.71 - 2.66 (m, 2H), 2.34 - 2.27 (m, 2H).

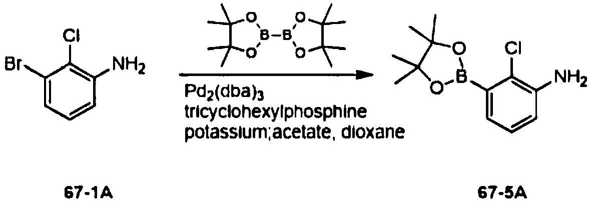

A mixture of Compound 67-1A (10 g, 48.43 mmol, 1 eq), 4,4,5,5-tetramethyl-2-(4,4,5,5-tetramethyl-1,3,2-dioxaborolan-2-yl)-1,3,2-dioxaborolane (14.76 g, 58.12 mmol, 1.2 eq), potassium acetate (7.13 g, 72.65 mmol, 1.5 eq), tricyclohexylphosphane (2.72 g, 9.69 mmol, 3.14 mL, 0.2 eq), and Pd₂(dba)₃ (4.44 g, 4.84 mmol, 0.1 eq) in dioxane (100 mL) was stirred at 120 °C for 4 hours under a nitrogen atmosphere. After completion of the reaction, the reaction mixture was filtered and concentrated under reduced pressure. The residue was purified by column chromatography (SiO₂, Petroleum ether/Ethyl acetate = 1/0 to 1/1) to afford Compound 67-5A (14.7 g, 89.78% yield) as a yellow solid.

LCMS m/z 254.1 [M+1]⁺.

¹H NMR (400 MHz, CDCl3) δ 7.64 - 7.61 (m, 1H), 7.06 (s, 1H), 6.83 (dd, J = 2.5, 6.9 Hz, 1H), 4.71 - 3.49 (m, 2H), 1.37 (s, 12H).

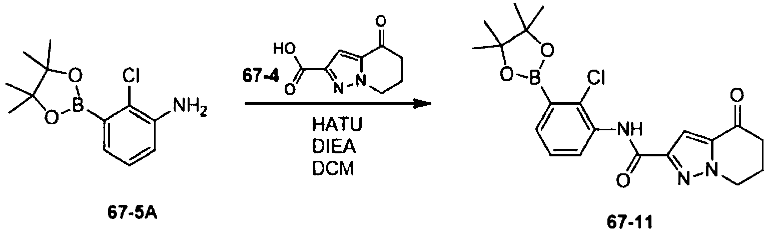

A mixture of Compound 67-5A (7.8 g, 23.07 mmol, 75% purity, 1 eq), Compound 67-4 (4.20 g, 23.30 mmol, 1.01 eq), HATU (10.53 g, 27.69 mmol, 1.2 eq), and DIPEA (11.93 g, 92.30 mmol, 16.08 mL, 4 eq) in DCM (150 mL) was stirred at room temperature for 18 hours under a nitrogen atmosphere. After completion of the reaction, the reaction mixture was diluted with DCM (200 mL) and washed with water (150 mL * 4). The organic layer was washed with brine (200 mL * 2), dried over Na₂SO₄, and concentrated under reduced pressure. The residue was purified by column chromatography (SiO₂, Petroleum ether/Ethyl acetate = 1/0 to 0/1) to afford Compound 67-11 (4.86 g, 40.5% yield) as a white solid.

LCMS m/z 416.0 [M+1]⁺.

¹H NMR (400 MHz, CDCl3) δ 9.47 (s, 1H), 8.65 (dd, J = 1.6, 8.2 Hz, 1H), 7.49 - 7.42 (m, 2H), 7.31 (t, J = 7.7 Hz, 1H), 4.52 - 4.45 (m, 2H), 2.79 - 2.72 (m, 2H), 2.49 - 2.41 (m, 2H), 1.39 (s, 12H).

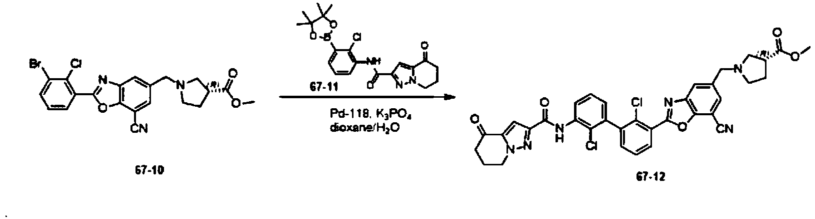

A mixture of Compound 67-10 (1 g, 2.11 mmol, 1 eq) and Compound 67-11 (1.14 g, 2.74 mmol, 1.3 eq) in dioxane (15 mL)/H₂O (1.5 mL) was treated with K₃PO₄ (894.25 mg, 4.21 mmol, 2 eq) and Pd-118 (137.29 mg, 210.64 µmol, 0.1 eq), and the reaction mixture was stirred at 80 °C for 18 hours under a nitrogen atmosphere. After completion of the reaction, the reaction mixture was diluted with H₂O (30 mL) and extracted with EA (50 mL * 3). The organic layer was washed with brine (50 mL * 2), dried over Na₂SO₄, and concentrated under reduced pressure. The residue was purified by column chromatography (SiO₂, Petroleum ether/Ethyl acetate = 3/1 to 0/1) to afford Compound 67-12 (500 mg, 30.9% yield) as a light yellow solid.

LCMS m/z 683.3 [M+1]⁺.

¹H NMR (400 MHz, DMSO-d6) δ 9.85 (s, 1H), 8.25 - 8.20 (m, 1H), 8.17 (s, 1H), 8.11 (dd, J = 1.4, 8.1 Hz, 1H), 7.94 (d, J = 1.2 Hz, 1H), 7.76 - 7.66 (m, 2H), 7.54 (t, J = 7.9 Hz, 1H), 7.32 - 7.26 (m, 2H), 4.51 - 4.44 (m, 2H), 3.82 - 3.72 (m, 2H), 3.13 - 3.02 (m, 1H), 2.76 - 2.66 (m, 4H), 2.58 - 2.53 (m, 2H), 2.38 - 2.29 (m, 3H), 1.99 (s, 3H), 1.22 - 1.12 (m, 2H).

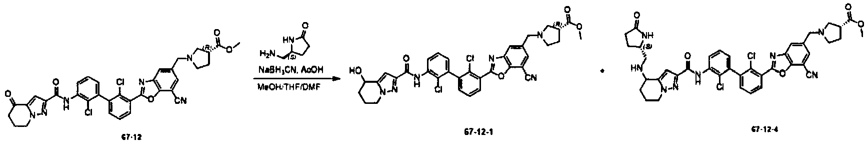

A mixture of Compound 67-12 (100 mg, 146.30 µmol, 1 eq), (5S)-5-(aminomethyl)pyrrolidin-2-one (83.50 mg, 731.49 µmol, 5 eq), and AcOH (175.70 mg, 2.93 mmol, 167.34 µL, 20 eq) in MeOH (2 mL)/THF (2 mL)/DMF (1 mL) was stirred at 60 °C for 30 minutes. NaBH₃CN (275.80 mg, 4.39 mmol, 30 eq) was then added, and the reaction mixture was stirred at 60 °C for 16 hours. After completion of the reaction, H₂O (1 mL) was added to the reaction mixture, followed by concentration under reduced pressure. The residue was purified by prep-HPLC (column: Welch Xtimate C18 150*25 mm*5 um; mobile phase: [water (NH₄HCO₃)-ACN]; B%: 45%-75%, 11 min) to afford Compound 67-12-1 (67 mg, 66.0% yield) and Compound 67-12-4 (23 mg, 19.9% yield), each as a white solid.

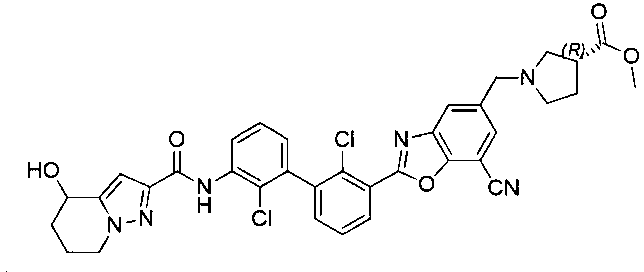

### (Compound 67-12-1)

LCMS m/z 685.3 [M+1]⁺.

¹H NMR (400 MHz, DMSO-d6) δ 9.58 (s, 1H), 8.33 - 8.16 (m, 3H), 7.94 (d, J = 1.2 Hz, 1H), 7.76 - 7.66 (m, 2H), 7.52 (t, J = 7.9 Hz, 1H), 7.24 (dd, J = 1.3, 7.6 Hz, 1H), 6.75 (s, 1H), 5.61 (d, J = 5.5 Hz, 1H), 4.77 (br d, J = 6.3 Hz, 1H), 4.19 - 4.11 (m, 2H), 3.77 (d, J = 2.9 Hz, 2H), 3.60 (s, 3H), 3.10 - 3.04 (m, 1H), 2.80 - 2.71 (m, 2H), 2.70 - 2.65 (m, 3H), 2.20 - 2.11 (m, 1H), 2.04 - 1.95 (m, 3H), 1.77 - 1.69 (m, 1H).

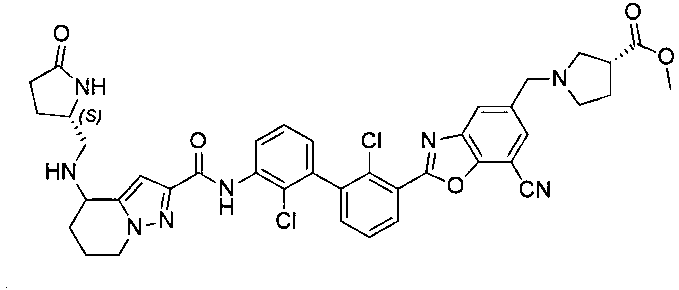

### (Compound 67-12-4)

LCMS m/z 783.3 [M+1]⁺.

¹H NMR (400 MHz, DMSO-d6) δ 9.57 (s, 1H), 8.33 - 8.14 (m, 3H), 7.94 (d, J = 1.2 Hz, 1H), 7.76 - 7.65 (m, 3H), 7.52 (t, J = 7.9 Hz, 1H), 7.24 (dd, J = 1.4, 7.6 Hz, 1H), 6.84 (d, J = 11.9 Hz, 1H), 4.23 - 4.09 (m, 2H), 3.95 - 3.83 (m, 1H), 3.77 (d, J = 2.9 Hz, 2H), 3.60 (s, 4H), 3.10 - 3.02 (m, 1H), 2.74 (br d, J = 8.5 Hz, 1H), 2.70 - 2.65 (m, 3H), 2.19 - 1.86 (m, 9H), 1.75 - 1.64 (m, 2H).

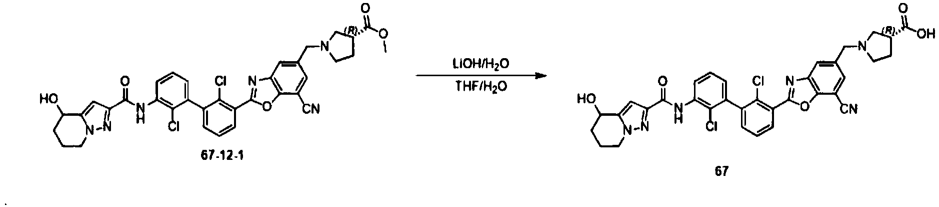

A solution of Compound 67-12-1 (67 mg, 97.73 µmol, 1 eq) and LiOH·H₂O (8.93 mg, 212.88 µmol, 2.18 eq) in THF (2 mL)/H₂O (0.4 mL) was stirred at room temperature for 2 hours. The pH of the reaction mixture was adjusted to a range of 6-7 using an aq. 1N HCl solution, followed by concentration under reduced pressure. The residue was purified by prep-HPLC (column: Welch Xtimate C18 150*25 mm*5 um; mobile phase: [water (NH₄HCO₃)-ACN]; B%: 23%-53%, 11 min) to afford Compound 67 (48 mg, 71.7% yield) as a white solid.

LCMS m/z 671.3 [M+1]⁺.

¹H NMR (400 MHz, DMSO-d6) δ 9.58 (s, 1H), 8.29 (d, J = 8.1 Hz, 1H), 8.22 (dd, J = 1.9, 7.6 Hz, 1H), 8.17 (s, 1H), 7.94 (d, J = 1.2 Hz, 1H), 7.76 - 7.66 (m, 2H), 7.52 (t, J = 7.9 Hz, 1H), 7.28 - 7.21 (m, 1H), 6.75 (s, 1H), 5.60 (br s, 1H), 4.77 (br s, 1H), 4.17 - 4.11 (m, 2H), 3.76 (br d, J = 8.6 Hz, 2H), 2.98 - 2.88 (m, 2H), 2.77 - 2.63 (m, 3H), 2.20 - 2.12 (m, 1H), 2.03 - 1.90 (m, 4H), 1.77 - 1.68 (m, 1H).

### 17-2. Preparation of Compound 68, (3R)-1-((7-cyano-2-(2,2'-dichloro-3'-(4-((((S)-5-oxopyrrolidin-2-yl)methyl)amino)-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid

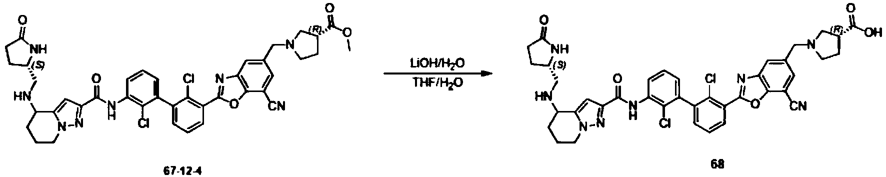

A solution of Compound 67-12-4 (51 mg, 65.24 µmol, 1 eq) in THF (4 mL)/H₂O (1 mL) was treated with LiOH·H₂O (5.48 mg, 130.49 µmol, 2 eq), and the reaction mixture was stirred at room temperature for 2 hours. The pH of the reaction mixture was adjusted to a range of 6-7 using an aq. 1N HCl solution, followed by concentration under reduced pressure. The residue was purified by prep-HPLC (column: Welch Xtimate C18 150*25 mm*5 um; mobile phase: [water (NH₄HCO₃)-ACN]; B%: 28%-58%, 11 min) to afford Compound 68 (50 mg, 98.3% yield) as a white solid.

LCMS m/z 769.3 [M+1]⁺.

¹H NMR (400 MHz, DMSO-d6) δ 9.57 (s, 1H), 8.30 (br d, J = 8.1 Hz, 1H), 8.22 (dd, J = 1.5, 7.6 Hz, 1H), 8.17 (s, 1H), 7.94 (s, 1H), 7.76 - 7.64 (m, 3H), 7.52 (t, J = 8.0 Hz, 1H), 7.24 (br d, J = 7.4 Hz, 1H), 6.84 (d, J = 11.9 Hz, 1H), 4.15 (br d, J = 5.2 Hz, 2H), 3.88 (td, J = 6.2, 12.5 Hz, 1H), 3.80 - 3.71 (m, 2H), 3.58 (br d, J = 2.0 Hz, 2H), 2.96 - 2.90 (m, 1H), 2.71 (br d, J = 8.3 Hz, 1H), 2.65 (br dd, J = 5.8, 14.5 Hz, 2H), 2.59 (br d, J = 6.1 Hz, 1H), 2.54 (br d, J = 3.8 Hz, 2H), 2.21 - 1.89 (m, 9H), 1.74 - 1.64 (m, 2H).

### 17-3. Preparation of Compound 69, (3R)-1-((2-(3'-(4-((2-acetamidoethyl)amino)-4,5,6,7-tetrahydropyrazolo[1,5-a]pyridine-2-carboxamido)-2,2'-dichloro-[1,1'-biphenyl]-3-yl)-7-cyanobenzo[d]oxazol-5-yl)methyl)pyrrolidine-3-carboxylic acid

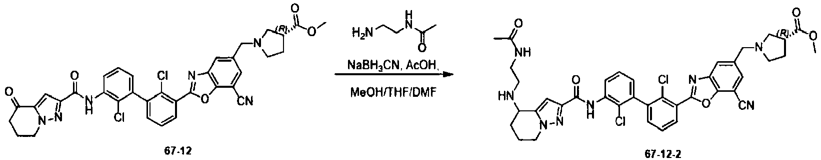

A mixture of Compound 67-12 (120 mg, 175.56 µmol, 1 eq), N-(2-aminoethyl)acetamide (89.65 mg, 877.78 µmol, 83.79 µL, 5 eq), and AcOH (210.85 mg, 3.51 mmol, 200.81 µL, 20 eq) in MeOH (2 mL)/THF (2 mL)/DMF (1 mL) was stirred at 60 °C for 30 minutes. NaBH₃CN (330.97 mg, 5.27 mmol, 30 eq) was then added, and the reaction mixture was stirred at 60 °C for 16 hours. After completion of the reaction, H₂O (1 mL) was added to the reaction mixture, followed by concentration under reduced pressure. The residue was purified by prep-HPLC (column: Welch Xtimate C18 150*25 mm*5 um; mobile phase: [water (NH₄HCO₃)-ACN]; B%: 45%-75%, 11 min) to afford Compound 67-12-2 (89 mg, 65.8% yield) as a white solid.

LCMS m/z 771.3 [M+1]⁺.

¹H NMR (400 MHz, DMSO-d6) δ 9.57 (s, 1H), 8.34 - 8.27 (m, 1H), 8.22 (dd, J = 1.8, 7.6 Hz, 1H), 8.17 (s, 1H), 7.94 (d, J = 0.8 Hz, 1H), 7.84 (br t, J = 5.2 Hz, 1H), 7.76 - 7.67 (m, 2H), 7.52 (t, J = 7.9 Hz, 1H), 7.24 (dd, J = 1.3, 7.6 Hz, 1H), 6.79 (s, 1H), 4.20 - 4.09 (m, 2H), 3.88 (br t, J = 6.1 Hz, 1H), 3.77 (d, J = 2.7 Hz, 2H), 3.60 (s, 3H), 3.21 - 3.01 (m, 4H), 2.82 - 2.71 (m, 2H), 2.68 - 2.66 (m, 2H), 2.62 (br s, 2H), 2.28 - 2.13 (m, 2H), 2.06 - 1.90 (m, 4H), 1.80 (s, 3H), 1.72 - 1.62 (m, 1H).

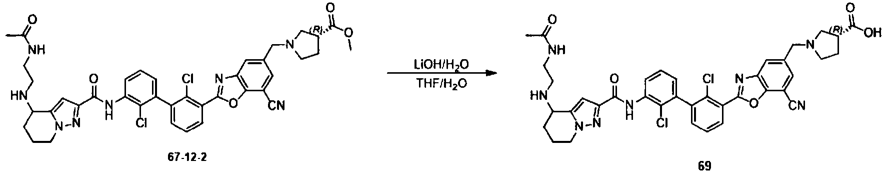

A solution of Compound 67-12-2 (89 mg, 115.63 µmol, 1 eq) in THF (4 mL)/H₂O (1 mL) was treated with LiOH·H₂O (9.70 mg, 231.27 µmol, 2 eq) and stirred at room temperature for 2 hours. The pH of the reaction mixture was adjusted to a range of 6-7 using an aq. 1N HCl solution, followed by concentration under reduced pressure. The residue was purified by pre-HPLC (column: Welch Xtimate C18 150*25 mm*5 um; mobile phase: [water (NH₄HCO₃)-ACN]; B%: 23%-53%, 11 min) to afford Compound 69 (80 mg, 89.4% yield) as a white solid.

LCMS m/z 757.3 [M+1]⁺.

¹H NMR (400 MHz, DMSO-d6) δ 9.56 (s, 1H), 8.30 (dd, J = 1.2, 8.2 Hz, 1H), 8.22 (dd, J = 1.8, 7.6 Hz, 1H), 8.17 (s, 1H), 7.94 (d, J = 1.1 Hz, 1H), 7.83 (br t, J = 5.2 Hz, 1H), 7.75 - 7.65 (m, 2H), 7.52 (t, J = 7.9 Hz, 1H), 7.24 (dd, J = 1.4, 7.6 Hz, 1H), 6.79 (s, 1H), 4.19 - 4.11 (m, 2H), 3.88 (br t, J = 5.9 Hz, 1H), 3.76 (br d, J = 8.5 Hz, 2H), 3.13 (br d, J = 6.2 Hz, 2H), 3.01 - 2.89 (m, 2H), 2.72 (br d, J = 8.3 Hz, 1H), 2.70 - 2.59 (m, 4H), 2.55 (br s, 1H), 2.22 - 2.14 (m, 1H), 2.03 - 1.90 (m, 4H), 1.80 (s, 3H), 1.71 - 1.62 (m, 1H).

### 17-4. Preparation of Compound 70, (3R)-1-((7-cyano-2-(2,2'-dichloro-3'-(4-((2-hydroxyethyl)amino)-4,5,6,7-tetrahydropyrazolo[1,5-a]pyridine-2-carboxamido)-[1,1'-biphenyl]-3-yl)benzo[d]oxazol-5-yl)methyl)pyrrolidine-3-carboxylic acid

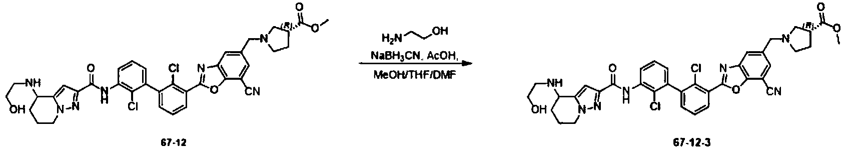

A mixture of Compound 67-12 (120 mg, 175.56 µmol, 1 eq), 2-aminoethanol (53.62 mg, 877.78 µmol, 53.09 µL, 5 eq), and AcOH (210.84 mg, 3.51 mmol, 200.80 µL, 20 eq) in MeOH (2 mL)/THF (2 mL)/DMF (1 mL) was stirred at 60 °C for 30 minutes. NaBH₃CN (330.96 mg, 5.27 mmol, 30 eq) was then added, and the reaction mixture was stirred at 60 °C for 16 hours. After completion of the reaction, H₂O (1 mL) was added to the reaction mixture, followed by concentration under reduced pressure. The residue was purified by prep-HPLC (column: Welch Xtimate C18 150*25 mm*5 um; mobile phase: [water (NH₄HCO₃)-ACN]; B%: 45%-75%, 11 min) to afford Compound 67-12-3 (71 mg, 54.9% yield) as a white solid.

LCMS m/z 730.3 [M+1]⁺.

¹H NMR (400 MHz, DMSO-d6) δ 9.57 (s, 1H), 8.32 - 8.16 (m, 3H), 7.94 (s, 1H), 7.78 - 7.65 (m, 2H), 7.52 (br t, J = 7.9 Hz, 1H), 7.24 (br d, J = 7.0 Hz, 1H), 6.78 (s, 1H), 4.52 (br t, J = 5.3 Hz, 1H), 4.15 (br s, 2H), 3.89 (br t, J = 5.8 Hz, 1H), 3.77 (br d, J = 2.1 Hz, 2H), 3.60 (s, 4H), 3.52 - 3.44 (m, 4H), 3.09 - 3.03 (m, 1H), 2.74 (br d, J = 8.6 Hz, 2H), 2.23 - 2.12 (m, 2H), 2.07 - 1.85 (m, 5H), 1.75 - 1.63 (m, 1H).

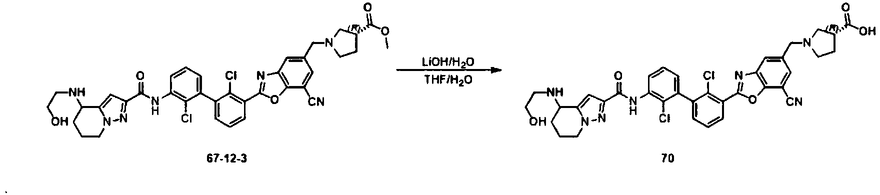

A solution of Compound 67-12-3 (71 mg, 97.44 µmol, 1 eq) in THF (4 mL)/H₂O (1 mL) was treated with LiOH·H₂O (8.18 mg, 194.89 µmol, 2 eq) and stirred at room temperature for 2 hours. The pH of the reaction mixture was adjusted to a range of 6-7 using an aq. 1N HCl solution, followed by concentration under reduced pressure. The residue was purified by prep-HPLC (column: Welch Xtimate C18 15025 mm5 um; mobile phase: [water (NH₄HCO₃)-ACN]; B%: 18%-48%, 11 min) to afford Compound 70 (50 mg, 71.30% yield) as a white solid.

LCMS m/z 716.5 [M+1]⁺.

¹H NMR (400 MHz, DMSO-d6) δ 9.57 (s, 1H), 8.30 (d, J = 7.5 Hz, 1H), 8.22 (dd, J = 1.8, 7.6 Hz, 1H), 8.17 (s, 1H), 7.94 (d, J = 1.0 Hz, 1H), 7.76 - 7.64 (m, 2H), 7.52 (t, J = 8.0 Hz, 1H), 7.24 (dd, J = 1.3, 7.5 Hz, 1H), 6.78 (s, 1H), 4.64 - 4.38 (m, 1H), 4.18 - 4.11 (m, 2H), 3.89 (br t, J = 6.1 Hz, 1H), 3.76 (br d, J = 8.5 Hz, 2H), 3.50 - 3.45 (m, 5H), 2.97 - 2.92 (m, 1H), 2.75 - 2.65 (m, 4H), 2.57 (br d, J = 1.4 Hz, 1H), 2.22 - 2.14 (m, 1H), 2.05 - 1.90 (m, 4H), 1.74 - 1.65 (m, 1H).

### [Preparation Example 18]

### Preparation of Compounds 71 to 80

### 18-1. Preparation of Compound 71, 4-hydroxy-N-(3'-(5-(((2-hydroxyethyl)amino)methyl)picolinamido)-2,2'-dimethyl-[1,1'-biphenyl]-3-yl)-4,5,6,7-tetrahydropyrazolo[1,5-a]pyridine-2-carboxamide

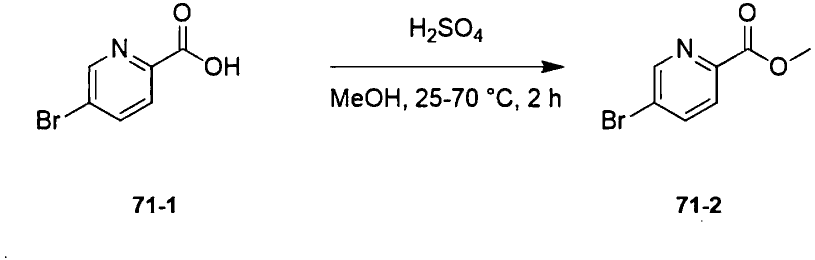

A solution of 5-bromopyridine-2-carboxylic acid (10 g, 49.50 mmol, 1 eq) in MeOH (79.18 g, 2.47 mol, 100.00 mL, 49.92 eq) was treated with c-H₂SO₄ (9.20 g, 93.80 mmol, 5.00 mL, 1.89 eq), and the reaction mixture was stirred at 70 °C for 2 hours. Water (600 mL) was then added to the mixture, followed by extraction with EtOAc (600 mL * 2). The organic layer was washed with brine (600 mL), dried over Na₂SO₄, and concentrated under reduced pressure. The residue was purified by flash silica gel chromatography (Petroleum ether/Ethyl acetate = 100/1 to 1/1) to afford Compound 71-2 (10.2 g, 85.8% yield) as a white solid.

¹H NMR (400 MHz, DMSO-d6) δ = 8.86 (d, J = 1.9 Hz, 1H), 8.27 (dd, J = 2.4, 8.4 Hz, 1H), 7.99 (d, J = 8.4 Hz, 1H), 3.89 (s, 3H).

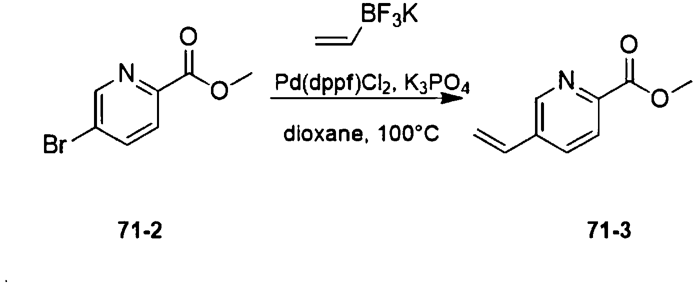

A mixture of methyl 5-bromopicolinate (9.8 g, 45.36 mmol, 1 eq), potassium trifluoro(vinyl)boranuide (6.08 g, 45.36 mmol, 1 eq), K₃PO₄ (28.89 g, 136.09 mmol, 3 eq), and Pd(dppf)Cl₂ (1.85 g, 2.27 mmol, 0.05 eq) in dioxane (100 mL) was stirred at 100 °C for 2 hours under a nitrogen atmosphere. Water (600 mL) was then added to the mixture, followed by extraction with EtOAc (600 mL * 2). The organic layer was washed with brine (600 mL), dried over Na₂SO₄, and concentrated under reduced pressure. The residue was purified by flash silica gel chromatography (Petroleum ether/Ethyl acetate = 100/1 to 1/1) to afford Compound 71-3 (6.2 g, 75.8% yield).

¹H NMR (400 MHz, DMSO-d6) δ = 8.78 (d, J = 1.9 Hz, 1H), 8.12 - 8.06 (m, 1H), 8.04 - 7.96 (m, 1H), 6.85 (dd, J = 11.1, 17.8 Hz, 1H), 6.13 (d, J = 17.6 Hz, 1H), 5.54 (d, J = 11.1 Hz, 1H), 3.87 (s, 3H).

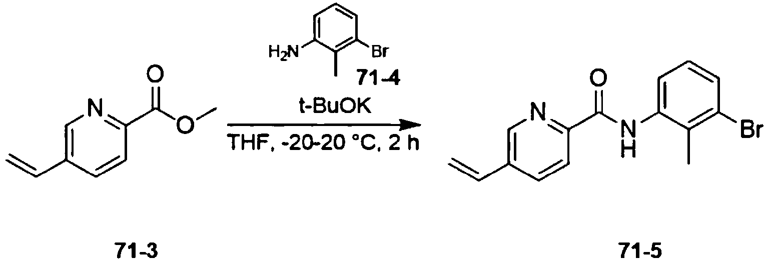

A solution of methyl 5-vinylpyridine-2-carboxylate (5 g, 30.64 mmol, 1 eq) and 3-bromo-2-methyl-aniline (5.70 g, 30.64 mmol, 3.78 mL, 1 eq) in THF (80 mL) was treated with t-BuOK (6.88 g, 61.28 mmol, 2 eq) at -20 °C, and the mixture was then stirred at 20 °C for 2 hours. Water (1000 mL) was added to the mixture, and the organic layer was extracted with ethyl acetate (500 mL * 3). The organic layer was washed with brine (600 mL), dried over Na₂SO₄, and concentrated under reduced pressure. The residue was purified by flash silica gel chromatography (Petroleum ether/Ethyl acetate = 100/1 to 1/1) to afford Compound 71-5 (6.8 g, 55.9% yield) as a white solid.

¹H NMR (400 MHz, DMSO-d6) δ = 10.45 (s, 1H), 8.82 (d, J = 1.6 Hz, 1H), 8.33 - 8.17 (m, 1H), 8.15 - 8.06 (m, 1H), 7.69 (d, J = 7.9 Hz, 1H), 7.49 (d, J = 7.9 Hz, 1H), 7.19 (t, J = 8.0 Hz, 1H), 6.90 (dd, J = 11.0, 17.8 Hz, 1H), 6.16 (d, J = 17.8 Hz, 1H), 5.56 (d, J = 11.1 Hz, 1H), 2.34 (s, 3H).

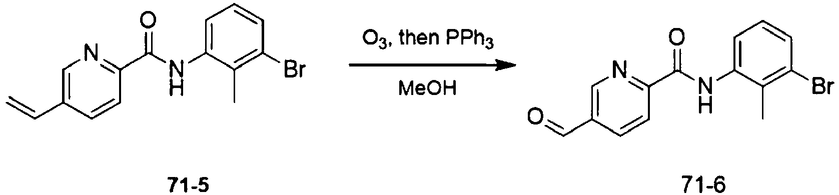

A solution of N-(3-bromo-2-methyl-phenyl)-5-vinyl-pyridine-2-carboxamide (1.8 g, 5.68 mmol, 1 eq) in MeOH (20 mL) was subjected to bubbling with ozone for 30 minutes. PPh3 (2.98 g, 11.35 mmol, 2 eq) was then added, and the mixture was stirred at room temperature for 2 hours. Water (40 mL) was added to the mixture, followed by extraction with ethyl acetate (30 mL x 3). The organic layer was washed with brine (60 mL), dried over Na₂SO₄, and concentrated under reduced pressure. The residue was purified by flash silica gel chromatography (Petroleum ether/Ethyl acetate = 100/1 to 3/1) to afford Compound 71-6 (92 mg, 54.8% yield) as a white solid.

LCMS m/z 318.9 [M+1]⁺.

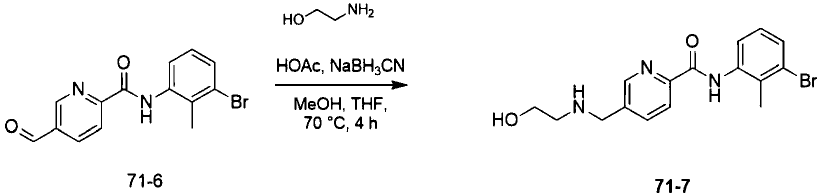

A solution of N-(3-bromo-2-methyl-phenyl)-5-formyl-pyridine-2-carboxamide (1.6 g, 5.01 mmol, 1 eq), 2-aminoethanol (612.45 mg, 10.03 mmol, 606.39 µL, 2 eq), and HOAc (361.27 mg, 6.02 mmol, 344.07 µL, 1.2 eq) in THF (15 mL)/MeOH (15 mL) was stirred at 70 °C for 2 hours. NaBH₃CN (472.57 mg, 7.52 mmol, 1.5 eq) was then added, and the mixture was stirred at 70 °C for 2 hours. Water (40 mL) was added to the mixture, followed by extraction with ethyl acetate (30 mL x 3). The organic layer was washed with brine (60 mL), dried over Na₂SO₄, and concentrated under reduced pressure. The residue was purified by flash silica gel chromatography (Petroleum ether/Ethyl acetate = 100/1 to 1/1) to afford Compound 71-7 (1.78 g, 87.7% yield) as a white solid.

¹H NMR (400 MHz, DMSO-d6) δ = 10.48 (s, 1H), 8.69 (d, J = 1.3 Hz, 1H), 8.23 (s, 1H), 8.16 - 8.07 (m, 1H), 8.03 (d, J = 1.9 Hz, 1H), 7.73 - 7.60 (m, 1H), 7.49 (d, J = 7.5 Hz, 1H), 7.34 - 7.10 (m, 1H), 5.53 - 5.50 (m, 1H), 3.90 (s, 2H), 3.50 (t, J = 5.8 Hz, 2H), 2.62 (t, J = 5.8 Hz, 2H), 2.34 (s, 3H).

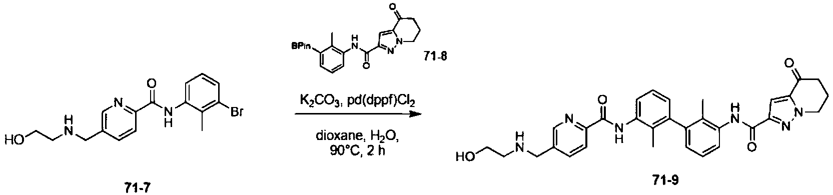

A mixture of N-(3-bromo-2-methyl-phenyl)-5-[(2-hydroxyethylamino)methyl]pyridine-2-carboxamide (500 mg, 1.37 mmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (813.88 mg, 2.06 mmol, 1.5 eq), K₂CO₃ (531.23 mg, 3.84 mmol, 2.8 eq), and Pd(dppf)Cl₂ (56.05 mg, 68.64 µmol, 0.05 eq) in dioxane (15 mL)/H₂O (4 mL) was stirred at 90 °C for 2 hours under a nitrogen atmosphere. Water (40 mL) was then added to the mixture, followed by extraction with ethyl acetate (30 mL x 3). The organic layer was washed with brine (60 mL), dried over Na₂SO₄, and concentrated under reduced pressure. The residue was purified by flash silica gel chromatography (Dichloromethane:Methanol = 100/1 to 10/1) to afford Compound 71-9 (330 mg, 39.1% yield) as a white solid.

¹H NMR (400 MHz, DMSO-d6) δ = 10.34 (s, 1H), 9.87 (s, 1H), 8.68 (s, 1H), 8.20 - 8.13 (m, 1H), 8.03 (d, J = 1.5 Hz, 1H), 7.87 (d, J = 7.7 Hz, 1H), 7.49 (s, 1H), 7.34 - 7.23 (m, 3H), 7.05 - 6.96 (m, 2H), 4.53 - 4.40 (m, 3H), 3.88 (s, 2H), 3.48 (t, J = 5.7 Hz, 4H), 2.76 - 2.65 (m, 3H), 2.60 (t, J = 5.8 Hz, 2H), 2.38 - 2.30 (m, 4H), 2.01 (s, 4H), 1.94 (s, 3H).

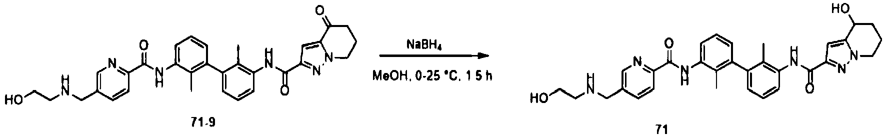

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (50 mg, 90.48 µmol, 1 eq) in MeOH (3 mL) was treated with NaBH₄ (6.85 mg, 180.96 µmol, 2 eq) at 0 °C, and the mixture was then stirred at 25 °C for 2 hours. The pH of the mixture was adjusted to 7 using an aq. 1N HCl solution, and the precipitated solid was collected by filtration. The collected solid was purified by prep-HPLC (column: Waters Xbridge C18 150*50 mm*10 um; mobile phase: [water (NH₄HCO₃)-ACN]; B%: 20%-50%, 10 min) to afford Compound 71 (21 mg, 39.7% yield) as a white solid.

LCMS m/z 555.1 [M+1]⁺.

¹H NMR (400 MHz, DMSO-d6) δ = 10.33 (s, 1H), 9.53 (s, 1H), 8.67 (s, 1H), 8.13 (d, J = 7.9 Hz, 1H), 8.01 (br d, J = 7.5 Hz, 1H), 7.87 (d, J = 8.0 Hz, 1H), 7.57 (d, J = 7.9 Hz, 1H), 7.30 (td, J = 7.8, 15.5 Hz, 2H), 6.97 (d, J = 7.4 Hz, 2H), 6.69 (s, 1H), 5.57 (d, J = 5.4 Hz, 1H), 4.76 (d, J = 5.8 Hz, 1H), 4.51 (s, 1H), 4.20 - 4.07 (m, 2H), 3.85 (s, 2H), 3.48 (d, J = 4.6 Hz, 2H), 2.56 (d, J = 8.9 Hz, 2H), 2.20 - 2.10 (m, 1H), 2.01 (s, 3H), 1.94 (s, 3H), 1.76 - 1.67 (m, 1H).

### 18-2. Preparation of Compound 72, 4-((2-acetamidoethyl)amino)-N-(3'-(5-(((2-hydroxyethyl)amino)methyl)picolinamido)-2,2'-dimethyl-[1,1'-biphenyl]-3-yl)-4,5,6,7-tetrahydropyrazolo[1,5-a]pyridine-2-carboxamide

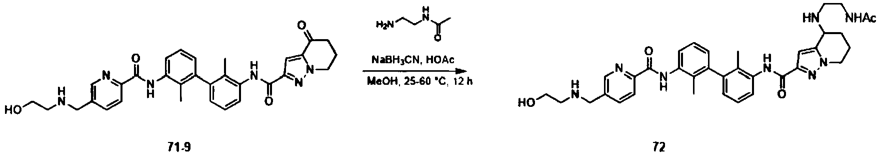

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (50 mg, 90.48 µmol, 1 eq) and N-(2-aminoethyl)acetamide (92.41 mg, 904.78 µmol, 86.36 µL, 10 eq) in MeOH (3 mL) was treated with HOAc (217.33 mg, 3.62 mmol, 206.99 µL, 40 eq) and stirred at room temperature for 2 hours. NaBH₃CN (11.37 mg, 180.96 µmol, 2 eq) was then added, and the mixture was stirred at 60 °C for 10 hours. After completion of the reaction, the mixture was concentrated under reduced pressure, and the residue was purified by prep-HPLC (column: Waters Xbridge C18 150*50 mm *10 um; mobile phase: [water (NH₄HCO₃)-ACN]; B%: 20%-50%, 10 min) to afford Compound 72 (32 mg, 52.6% yield) as a white solid.

LCMS m/z 639.3 [M+1]⁺.

¹H NMR (400 MHz, DMSO-d6) δ = 10.32 (s, 1H), 9.52 (d, J = 2.0 Hz, 1H), 8.67 (d, J = 1.4 Hz, 1H), 8.12 (d, J = 8.0 Hz, 1H), 8.06 - 7.98 (m, 1H), 7.94 - 7.78 (m, 2H), 7.57 (d, J = 7.9 Hz, 1H), 7.29 (td, J = 7.8, 15.6 Hz, 2H), 6.97 (d, J = 7.5 Hz, 2H), 6.72 (s, 1H), 4.64 - 4.50 (m, 1H), 4.40 (t, J = 5.1 Hz, 1H), 4.19 - 4.06 (m, 2H), 3.84 (s, 3H), 3.13 (q, J = 6.3 Hz, 2H), 2.68 - 2.62 (m, 2H), 2.57 (s, 2H), 2.28 - 2.12 (m, 2H), 2.00 (s, 3H), 1.94 (s, 3H), 1.80 (s, 3H), 1.72 - 1.56 (m, 1H), 1.05 (t, J = 7.0 Hz, 4H).

### 18-3. Preparation of Compound 73, N-(3'-(5-(((2-hydroxyethyl)amino)methyl)picolinamido)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide

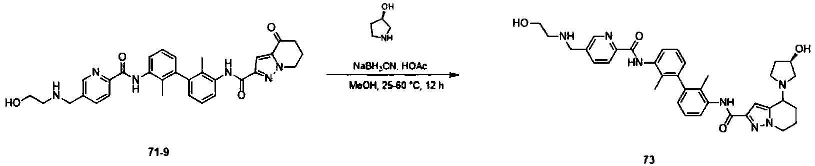

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (50 mg, 90.48 µmol, 1 eq) and (R)-pyrrolidin-3-ol (78.82 mg, 904.78 µmol, 75.07 µL, 10 eq) in MeOH (30 mL) was treated with HOAc (305.27 mg, 5.08 mmol, 290.74 µL, 56.19 eq) at room temperature, and the mixture was stirred at room temperature for 2 hours. NaBH₃CN (28.43 mg, 452.39 µmol, 5 eq) was then added, and the mixture was stirred at 60 °C for 2 hours. After completion of the reaction, the mixture was concentrated under reduced pressure, and the residue was purified by prep-HPLC (column: Phenomenex Luna C18 150*25 mm *10 um; mobile phase: [water (FA)-ACN]; B%: 8%-50%, 14 min) to afford Compound 73 (31 mg, 52.1% yield) as a white solid.

LCMS m/z 624.7 [M+1]⁺.

¹H NMR (400 MHz, DMSO-d6) δ = 10.33 (s, 1H), 9.53 (d, J = 3.0 Hz, 1H), 8.67 (d, J = 1.1 Hz, 1H), 8.13 (d, J = 8.0 Hz, 1H), 8.02 (d, J = 1.8 Hz, 1H), 7.87 (d, J = 8.0 Hz, 1H), 7.57 (dd, J = 2.9, 7.9 Hz, 1H), 7.29 (td, J = 7.8, 15.8 Hz, 2H), 6.97 (d, J = 7.5 Hz, 2H), 6.67 (d, J = 7.1 Hz, 1H), 4.71 (t, J = 4.9 Hz, 1H), 4.52 (t, J = 5.1 Hz, 1H), 4.23 - 4.09 (m, 3H), 3.85 (s, 2H), 3.81 - 3.72 (m, 1H), 3.48 (q, J = 5.5 Hz, 2H), 2.95 - 2.74 (m, 2H), 2.73 - 2.64 (m, 1H), 2.62 - 2.53 (m, 3H), 2.28 - 2.18 (m, 1H), 2.01 (s, 3H), 1.94 (s, 4H), 1.89 (s, 2H), 1.56 (d, J = 3.4 Hz, 1H).

### 18-4. Preparation of Compound 74, N-(3'-(5-(((2-hydroxyethyl)amino)methyl)picolinamido)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide

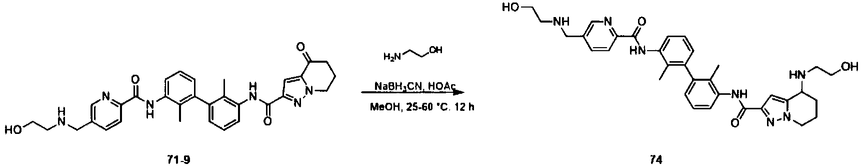

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (50 mg, 90.48 µmol, 1 eq) and 2-aminoethanol (55.27 mg, 904.78 µmol, 54.72 µL, 10 eq) in MeOH (5 mL) was treated with HOAc (305.28 mg, 5.08 mmol, 290.75 µL, 56.19 eq), and the mixture was stirred at 60 °C for 2 hours. NaBH₃CN (11.37 mg, 180.96 µmol, 2 eq) was then added, and the mixture was stirred at 60 °C for an additional 2 hours. After completion of the reaction, the mixture was concentrated under reduced pressure, and the residue was purified by prep-HPLC (column: Waters Xbridge C18 150*50 mm*10 um; mobile phase: [water (NH₄HCO₃)-ACN]; B%: 20%-50%, 10 min) to afford Compound 74 (30 mg, 52.7% yield) as a yellow solid.

LCMS m/z 598.1 [M+1]⁺.

¹H NMR (400 MHz, DMSO-d6) δ = 10.33 (s, 1H), 9.52 (s, 1H), 8.67 (d, J = 1.3 Hz, 1H), 8.13 (d, J = 7.9 Hz, 1H), 8.01 (dd, J = 1.9, 7.9 Hz, 1H), 7.87 (d, J = 7.9 Hz, 1H), 7.58 (d, J = 8.1 Hz, 1H), 7.30 (td, J = 7.8, 15.8 Hz, 2H), 6.98 (d, J = 7.5 Hz, 2H), 6.72 (s, 1H), 4.53 (d, J = 5.4 Hz, 2H), 4.21 - 4.05 (m, 2H), 3.94 - 3.77 (m, 3H), 3.48 (q, J = 5.5 Hz, 5H), 2.72 - 2.65 (m, 2H), 2.58 (t, J = 5.8 Hz, 2H), 2.24 - 2.15 (m, 1H), 2.01 (s, 3H), 1.94 (s, 3H), 1.76 - 1.61 (m, 1H).

### 18-5. Preparation of Compound 75, N-(3'-(5-(((2-hydroxyethyl)amino)methyl)picolinamido)-2,2'-dimethyl-[1,1'-biphenyl]-3-yl)-4-(((5-oxopyrrolidin-2-yl)methyl)amino)-4,5,6,7-tetrahydropyrazolo[1,5-a]pyridine-2-carboxamide

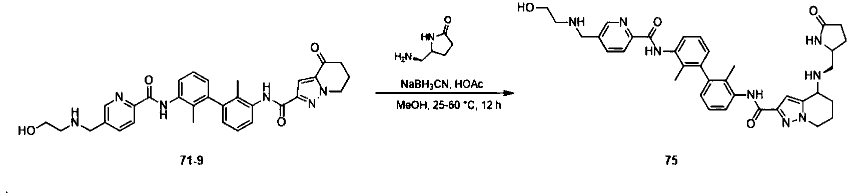

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (100 mg, 180.96 µmol, 1 eq), (5S)-5-(aminomethyl)pyrrolidin-2-one (206.55 mg, 1.81 mmol, 297.04 µL, 10 eq), and DIPEA (233.87 mg, 1.81 mmol, 315.18 µL, 10 eq) in MeOH (5 mL) was treated with HOAc (217.33 mg, 3.62 mmol, 206.99 µL, 20 eq) and stirred at 25 °C for 2 hours. NaBH₃CN (56.86 mg, 904.78 µmol, 5 eq) was then added, and the mixture was stirred at 60 °C for 2 hours. After completion of the reaction, the mixture was concentrated under reduced pressure, and the residue was purified by prep-HPLC (column: Waters Xbridge 150*25 mm* 5 µm; mobile phase: [water (NH₄HCO₃)-ACN]; B%: 20%-50%, 10 min) to afford Compound 75 (22 mg, 17.7% yield) as a white solid.

LCMS m/z 651.7 [M+1]⁺.

¹H NMR (400 MHz, DMSO-d6) δ = 10.33 (s, 1H), 9.52 (br s, 1H), 8.68 (s, 1H), 8.14 (br d, J = 7.8 Hz, 1H), 8.03 (br d, J = 8.3 Hz, 1H), 7.86 (br d, J = 8.3 Hz, 1H), 7.69 (br d, J = 19.4 Hz, 1H), 7.57 (br d, J = 7.8 Hz, 1H), 7.33 - 7.21 (m, 2H), 6.97 (br d, J = 7.6 Hz, 2H), 6.76 (d, J = 9.3 Hz, 1H), 4.67 - 4.51 (m, 1H), 4.13 (br d, J = 4.0 Hz, 3H), 3.89 (s, 4H), 2.63 - 2.57 (m, 4H), 2.11 (br s, 5H), 2.00 (s, 3H), 1.94 (s, 3H), 1.75 - 1.64 (m, 2H), 1.23 (br s, 2H).

### 18-6. Preparation of Compound 76, 2-((2-((3'-(5-(((2-hydroxyethyl)amino)methyl)picolinamido)-2,2'-dimethyl-[1,1'-biphenyl]-3-yl)carbamoyl)-4,5,6,7-tetrahydropyrazolo[1,5-a]pyridin-4-yl)amino)acetic acid

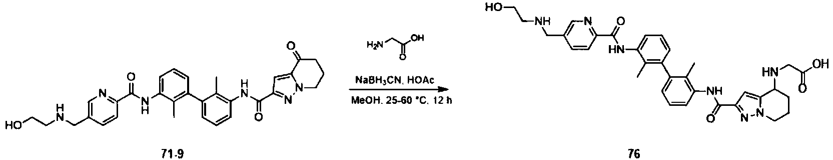

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (100 mg, 180.96 µmol, 1 eq) and 2-aminoacetic acid (135.84 mg, 1.81 mmol, 297.04 µL, 10 eq) in MeOH (5 mL) was treated with HOAc (217.33 mg, 3.62 mmol, 206.99 µL, 20 eq) and stirred at 60 °C for 2 hours. NaBH₃CN (56.86 mg, 904.78 µmol, 5 eq) was then added, and the mixture was stirred at 60 °C for 10 hours. After completion of the reaction, the mixture was concentrated under reduced pressure, and the residue was purified by prep-HPLC (column: Waters Xbridge 150*25 mm* 5 um; mobile phase: [water (NH₄HCO₃)-ACN]; B%: 10%-40%, 10 min) to afford Compound 76 (32 mg, 27.4% yield) as a white solid.

LCMS m/z 612.6 [M+1]⁺.

¹H NMR (400 MHz, DMSO-d6) δ = 10.33 (s, 1H), 9.56 (s, 1H), 8.68 (s, 1H), 8.13 (d, J = 8.0 Hz, 1H), 8.02 (dd, J = 1.5, 8.0 Hz, 1H), 7.87 (d, J = 8.0 Hz, 1H), 7.56 (br d, J = 7.9 Hz, 1H), 7.29 (td, J = 7.8, 15.8 Hz, 2H), 6.97 (br d, J = 7.6 Hz, 2H), 6.77 (s, 1H), 4.14 (br t, J = 6.0 Hz, 2H), 4.08 - 4.02 (m, 1H), 3.88 (s, 2H), 3.49 - 3.47 (m, 4H), 3.28 (s, 2H), 2.60 (t, J = 5.8 Hz, 2H), 2.30 - 2.12 (m, 1H), 2.01 (s, 3H), 1.94 (s, 3H), 1.86 - 1.63 (m, 1H).

### 18-7. Preparation of Compound 77, (3R)-1-(2-((3'-(5-(((2-hydroxyethyl)amino)methyl)picolinamido)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid

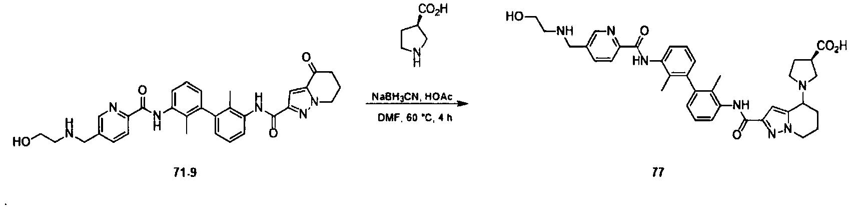

A solution of N-[3-[3-[[5-[(2-hydroxyethylamino)methyl]pyridine-2-carbonyl]amino]-2-methylphenyl]-2-methyl-phenyl]-4-oxo-6,7-dihydro-5H-pyrazolo[1,5-a]pyridine-2-carboxamide (220 mg, 398.10 µmol, 1 eq) and (3R)-pyrrolidine-3-carboxylic acid (320.83 mg, 2.79 mmol, 7 eq) in DMF (15 mL) was treated with HOAc (239.06 mg, 3.98 mmol, 227.89 µL, 10 eq) and stirred at 60 °C for 2 hours. NaBH₃CN (125.08 mg, 1.99 mmol, 5 eq) was then added, and the mixture was stirred at 60 °C for 2 hours. After completion of the reaction, the mixture was concentrated under reduced pressure, and the residue was purified by prep-HPLC (column: Waters Xbridge 150*25 mm* 5 um; mobile phase: [water (NH₄HCO₃)-ACN]; gradient: 10%-40% B over 9 min) to afford Compound 77 (62 mg, 22.7% yield) as a white solid.

LCMS m/z 652.5 [M+1]⁺.

¹H NMR (400 MHz, DMSO-d6) δ = 10.34 (s, 1H), 9.55 (br s, 1H), 8.68 (s, 1H), 8.14 (d, J = 8.0 Hz, 1H), 8.03 (br d, J = 8.0 Hz, 1H), 7.88 (br d, J = 8.0 Hz, 1H), 7.58 (br dd, J = 3.3, 7.8 Hz, 1H), 7.40 - 7.20 (m, 2H), 6.98 (br d, J = 7.4 Hz, 2H), 6.67 (d, J = 4.3 Hz, 1H), 4.31 - 4.04 (m, 3H), 3.87 (s, 2H), 3.81 (br s, 2H), 3.49 (br t, J = 5.7 Hz, 3H), 2.95 - 2.82 (m, 2H), 2.78 - 2.67 (m, 2H), 2.65 - 2.55 (m, 3H), 2.25 (br s, 1H), 2.02 (s, 3H), 1.95 (s, 6H).

### 18-8. Preparation of Compound 78, N-(3'-(5-(((2-hydroxyethyl)amino)methyl)picolinamido)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide

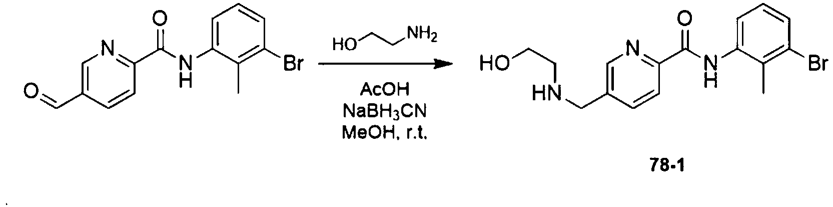

A solution of N-(3-bromo-2-methyl-phenyl)-5-formyl-pyridine-2-carboxamide (1 g, 3.13 mmol, 1 eq) in MeOH (20 mL) was treated with 2-aminoethanol (1.91 g, 31.33 mmol, 1.89 mL, 10 eq), NaBH₃CN (984.48 mg, 15.67 mmol, 5 eq), and AcOH (940.77 mg, 15.67 mmol, 896.83 µL, 5 eq), and the mixture was stirred at room temperature for 12 hours. The mixture was filtered, and the filtrate was concentrated under reduced pressure. The residue was purified by reversed-phase HPLC (column: Waters XBridge 150 * 25 mm * 5 um; mobile phase: [water (NH₄HCO₃) - ACN]; gradient: 14 % - 44 % B over 9 min) to afford Compound 78-1 (473 mg, 38.5% yield) as a white solid.

LCMS m/z 363.06 [M+3]⁺.

¹H NMR (400 MHz, CHLOROFORM-d) δ ppm 2.12 (br s, 2 H) 2.51 (s, 3 H) 2.87 (t, J=5.07 Hz, 2 H) 3.66 - 3.81 (m, 2 H) 3.98 (s, 2 H) 7.14 (t, J=8.07 Hz, 1 H) 7.41 (d, J=8.00 Hz, 1 H) 7.93 (br d, J=7.63 Hz, 1 H) 8.12 - 8.24 (m, 1 H) 8.27 (d, J=7.88 Hz, 1 H) 8.62 (s, 1 H) 10.10 (br s, 1 H).

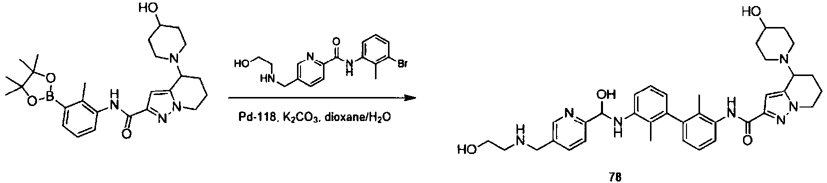

A mixture of 4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (100 mg, 208.16 µmol, 1 eq), N-(3-bromo-2-methyl-phenyl)-5-[(2-hydroxyethylamino)methyl]pyridine-2-carboxamide (75.82 mg, 208.16 µmol, 1 eq), ditert-butyl(cyclopentyl)phosphane;dichloropalladium iron (13.57 mg, 20.82 µmol, 0.1 eq), and K₂CO₃ (86.31 mg, 624.47 µmol, 3 eq) in dioxane (2.5 mL)/H₂O (0.5 mL) was stirred at 90 °C for 4 hours under a nitrogen atmosphere. After completion of the reaction, the mixture was filtered and the filtrate was concentrated under reduced pressure. The residue was purified by reversed-phase HPLC (column: Waters XBridge 150 * 25 mm * 5 um; mobile phase: [water (NH₄HCO₃)-ACN]; gradient: 25 % - 55 % B over 9 min) to afford Compound 78 (17 mg, 12.7% yield) as a yellow solid.

LCMS m/z 637.84 [M+1]⁺.

¹H NMR (400 MHz, METHANOL-d4) δ ppm 1.54 - 1.66 (m, 2 H) 1.77 - 1.85 (m, 1 H) 1.86 - 1.94 (m, 3 H) 2.06 (s, 3 H) 2.12 (s, 4 H) 2.21 - 2.29 (m, 1 H) 2.38 - 2.47 (m, 1 H) 2.53 - 2.61 (m, 1 H) 2.76 - 2.90 (m, 5 H) 3.63 (dt, J=8.82, 4.47 Hz, 1 H) 3.70 - 3.74 (m, 2 H) 3.96 - 4.01 (m, 2 H) 4.02 - 4.15 (m, 3 H) 4.23 - 4.32 (m, 1 H) 6.84 (s, 1 H) 7.07 (dd, J=7.88, 3.63 Hz, 3 H) 7.34 (q, J=8.30 Hz, 3 H) 7.67 (d, J=7.63 Hz, 1 H) 7.93 (d, J=7.63 Hz, 1 H) 8.03 - 8.08 (m, 1 H) 8.21 - 8.25 (m, 1 H) 8.72 (br d, J=1.38 Hz, 1 H).

### 18-9. Preparation of Compound 79, 1-(2-((3'-(5-(((2-hydroxyethyl)amino)methyl)picolinamido)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

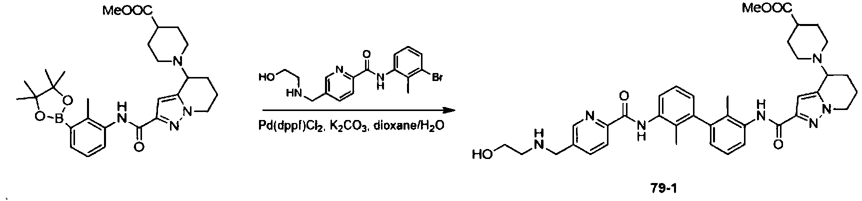

A mixture of methyl 1-[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (100 mg, 191.41 µmol, 1 eq), N-(3-bromo-2-methyl-phenyl)-5-[(2-hydroxyethylamino)methyl]pyridine-2-carboxamide (69.72 mg, 191.41 µmol, 1 eq), ditert-butyl(cyclopentyl)phosphane;dichloropalladium iron (12.47 mg, 19.14 µmol, 0.1 eq), and K₂CO₃ (79.36 mg, 574.23 µmol, 3 eq) in dioxane (2.5 mL)/H₂O (0.5 mL) was stirred at 90 °C for 6 hours under a nitrogen atmosphere. After completion of the reaction, the mixture was concentrated under reduced pressure and purified by column chromatography (SiO₂, Petroleum ether/Ethyl acetate = 10/1), followed by further purification by reversed-phase HPLC (column: Phenomenex Luna C18 150 * 25 mm * 10 um; mobile phase: [water (TFA)-ACN]; gradient: 13%-43% B over 9 min) to afford Compound 79-1 (100 mg, 67.6% yield) as a white solid.

LCMS m/z 680.6 [M+1]⁺.

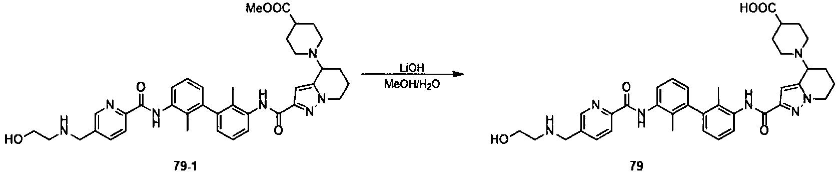

A mixture of methyl 1-[2-[[3-[3-[[5-[(2-hydroxyethylamino)methyl]pyridine-2-carbonyl]amino]-2-methyl-phenyl]-2-methyl-phenyl]carbamoyl]-4,5,6,7-tetrahydropyrazolo[1,5-a]pyridin-4-yl]piperidine-4-carboxylate (100 mg, 147.10 µmol, 1.49 µL, 1 eq) and LiOH (30.86 mg, 735.50 µmol, 5 eq) in H₂O (4 mL)/MeOH (20 mL) was stirred at room temperature for 12 hours. After completion of the reaction, the mixture was concentrated under reduced pressure, and the residue was purified by reversed-phase HPLC (column: Phenomenex Luna C18 150 * 25 mm * 10 um; mobile phase: [water (NH₄HCO₃)-ACN]; gradient: 15%-45% B over 10 min) to afford Compound 79 (52 mg, 52.5% yield) as a yellow solid.

LCMS m/z 666.4 [M+1]⁺.

¹H NMR (400 MHz, DMSO-d6) δ ppm 1.46 - 1.73 (m, 4 H) 1.79 - 1.91 (m, 3 H) 1.96 (s, 3 H) 2.03 (s, 3 H) 2.12 - 2.36 (m, 5 H) 2.59 - 2.61 (m, 2 H) 2.67 - 2.74 (m, 2 H) 2.77 - 2.85 (m, 1 H) 3.47 - 3.50 (m, 2 H) 3.87 (s, 2 H) 3.97 (br dd, J=10.51, 4.28 Hz, 1 H) 4.05 - 4.11 (m, 1 H) 4.18 - 4.24 (m, 1 H) 4.49 - 4.56 (m, 1 H) 6.64 (s, 1 H) 6.99 (d, J=7.58 Hz, 2 H) 7.31 (dt, J=15.98, 7.84 Hz, 2 H) 7.58 (t, J=6.48 Hz, 1 H) 7.89 (d, J=7.58 Hz, 1 H) 8.01 - 8.05 (m, 1 H) 8.15 (d, J=7.95 Hz, 1 H) 8.69 (s, 1 H) 9.55 (d, J=5.75 Hz, 1 H) 10.34 (s, 1 H).

### 18-10. Preparation of Compound 80, 2-((2-((3'-(5-(((2-hydroxyethyl)amino)methyl)picolinamido)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid

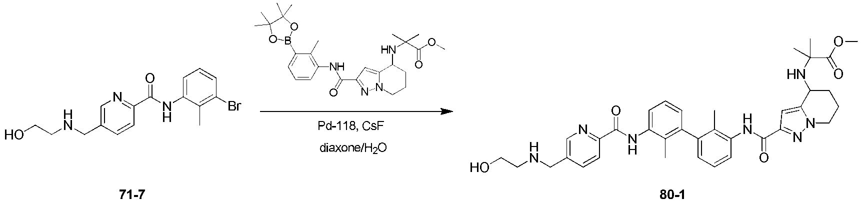

A mixture of N-(3-bromo-2-methyl-phenyl)-5-[(2-hydroxyethylamino)methyl]pyridine-2-carboxamide 71-7 (121.07 mg, 332.39 µmol, 1.1 eq), methyl 2-methyl-2-[[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]propanoate (150 mg, 302.17 µmol, 1 eq), CsF (91.80 mg, 604.34 µmol, 22.31 µL, 2 eq), and ditert-butyl(cyclopentyl)phosphane;dichloropalladium;iron (19.69 mg, 30.22 µmol, 0.1 eq) in dioxane (5 mL)/H₂O (1 mL) was stirred at 80 °C for 4 hours under an N2 atmosphere. After completion of the reaction, the mixture was cooled to room temperature, filtered, and the filtrate was concentrated under reduced pressure. The residue was purified by prep-TLC (SiO₂, DCM:MeOH = 0:1) to afford Compound 80-1 (100 mg, 38.9% yield) as a yellow solid.

LCMS m/z 654.4 [M+1]⁺.

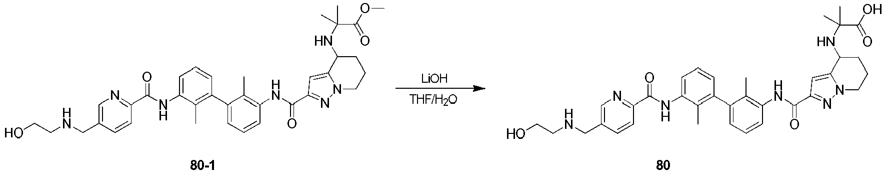

A solution of methyl 2-[[2-[[3-[3-[[5-[(2-hydroxyethylamino)methyl]pyridine-2-carbonyl]amino]-2-methyl-phenyl]-2-methyl-phenyl]carbamoyl]-4,5,6,7-tetrahydropyrazolo[1,5-a]pyridin-4-yl]amino]-2-methyl-propanoate 80-1 (100 mg, 152.96 µmol, 1 eq) in THF (20 mL)/H₂O (10 mL) was treated with LiOH·H₂O (19.26 mg, 458.88 µmol, 3 eq), and the mixture was stirred at room temperature for 12 hours. After completion of the reaction, the mixture was concentrated under reduced pressure, and the residue was purified by reversed-phase HPLC ((0.1% NH₃·H₂O condition); column: Phenomenex Luna C18 150 * 25 mm * 10 um; mobile phase: [water (NH₄HCO₃)-ACN]; gradient: 12%-42% B over 10 min) to afford Compound 80 (17 mg, 17.2% yield) as a white solid.

LCMS m/z 640.4 [M+1]⁺.

¹H NMR (400 MHz, DMSO-d6) δ ppm 1.29 (d, J=3.75 Hz, 6 H) 1.59 - 1.69 (m, 1 H) 1.94 (s, 4 H) 2.02 (s, 4 H) 2.08 (s, 3 H) 2.11 - 2.18 (m, 1 H) 2.55 - 2.62 (m, 3 H) 3.86 (s, 3 H) 3.90 - 3.96 (m, 2 H) 4.08 - 4.14 (m, 2 H) 6.75 (s, 1 H) 6.98 (d, J=7.50 Hz, 2 H) 7.25 - 7.35 (m, 2 H) 7.58 (d, J=7.88 Hz, 1 H) 7.88 (d, J=8.13 Hz, 1 H) 7.98 - 8.06 (m, 1 H) 8.14 (d, J=7.88 Hz, 1 H) 8.68 (s, 1 H) 9.52 (s, 1 H) 10.28 - 10.38 (m, 1 H)

### [Preparation Example 19]

### Preparation of Compounds 81 to 90

### 19-1. Preparation of Compound 81, N-(2,2'-dichloro-3'-(5-(((2-hydroxyethyl)amino)methyl)picolinamido)-[1,1'-biphenyl]-3-yl)-4-hydroxy-4,5,6,7-tetrahydropyrazolo[1,5-a]pyridine-2-carboxamide

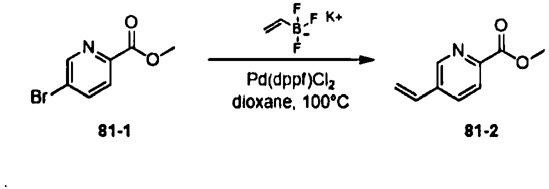

A mixture of compound 81-1 (25g, 115.72 mmol, 1eq), potassium;trifluoro(vinyl)boranuide (20.00 g, 149.28 mmol, 1.29 eq) in dioxane (400 mL)/H₂O (80 mL) was treated with Pd(dppf)Cl₂ (8.5 g, 11.62 mmol, 0.1 eq) and Na₂CO₃ (37 g, 349.09 mmol, 3.02 eq), and the reaction mixture was stirred at 90 °C for 18 hours under a nitrogen atmosphere. After completion of the reaction, the mixture was filtered, and the filtrate was diluted with H₂O (200 mL) and extracted with EA (300 mL * 2). The organic layer was washed with brine (200 mL * 3), dried over Na₂SO₄, and concentrated under reduced pressure. The residue was purified by flash silica gel chromatography (Eluent of 0-30% Ethyl acetate/Petroleum) to afford Compound 81-2 (16.4 g, 86.8% yield) as a yellow liquid.

LCMS m/z 163.8 [M+1]⁺.

¹H NMR (400MHz, Choroform-d) δ 8.72 (d, J =2.0 Hz, 1H), 8.10 (d, J =8.0 Hz, 1H), 7.86 (dd, J =2.1, 8.2 Hz, 1H), 6.76 (dd, J =10.9, 17.7 Hz, 1H), 5.95 (d, J =17.6 Hz, 1H), 5.53 (d, J =11.0 Hz, 1H), 4.00 (s, 3H).

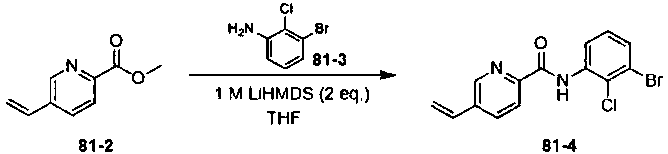

A solution of Compound 81-2 (3 g, 18.39 mmol, 1 eq) and Compound 81-3 (4.5 g, 21.80 mmol, 1.19 eq) in THF (100 mL) was treated with LiHMDS (1 M, 36.77 mL, 2 eq) at 0 °C, and the mixture was then stirred at room temperature for 16 hours. The mixture was sequentially treated with aq. saturated NH₄Cl solution (100 mL) and H₂O (50 mL), followed by extraction with EA (100 mL * 2). The organic layer was washed with aq. saturated NaCl solution (200 mL * 2), dried over Na₂SO₄, and concentrated under reduced pressure. The residue was separated by flash silica gel chromatography (Eluent of 0-30% Ethyl acetate/Petroleum) and further triturated with Petroleum ether/EA (10:1, 80 mL) to afford Compound 81-4 (4.8 g, 77.3% yield) as a white solid.

LCMS (m/z 338.7 [M+1]⁺.

¹H NMR (400MHz, DMSO-d6) δ 10.68 (s, 1H), 8.86 (d, J =1.5 Hz, 1H), 8.41 - 8.32 (m, 1H), 8.25 - 8.19 (m, 1H), 8.19 - 8.12 (m, 1H), 7.60 (dd, J =1.0, 8.0 Hz, 1H), 7.37 (t, J =8.2 Hz, 1H), 6.90 (dd, J =11.0, 17.8 Hz, 1H), 6.19 (d, J =17.6 Hz, 1H), 5.58 (d, J =11.0 Hz, 1H).

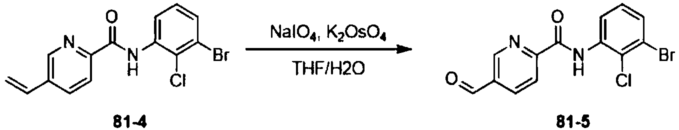

A mixture of Compound 81-4 (3.8 g, 11.26 mmol, 1 eq) in THF (90 mL)/H₂O (45 mL) was cooled to 0 °C, and dipotassium; dioxide (dioxo) osmium; dihydrate (24.88 mg, 67.54 µmol, 0.006 eq) and NaIO4 (7.22 g, 33.77 mmol, 1.87 mL, 3 eq) were slowly added. The reaction mixture was stirred at room temperature for 2 hours. After completion of the reaction, the mixture was filtered, and the filtrate was concentrated under reduced pressure to afford Compound 81-5 (4.4 g, 89% yield) as a white solid, which was used in the next reaction without further purification.

¹H NMR (400MHz, DMSO-d6) δ 10.76 (s, 1H), 10.26 - 10.17 (m, 1H), 9.22 (d, J =1.5 Hz, 1H), 8.52 (dd, J=2.0, 8.0 Hz, 1H), 8.36 (d, J =8.0 Hz, 1H), 8.26 (dd, J =1.2, 8.2 Hz, 1H), 7.63 (dd, J =1.2, 8.1 Hz, 1H), 7.38 (t, J =8.2 Hz, 1H).

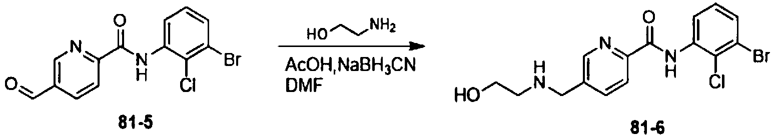

A solution of Compound 81-5 (2.9 g, 8.54 mmol, 1 eq) and 2-aminoethanol (725.00 mg, 11.87 mmol, 717.82 µL, 1.39 eq) in DMF (80 mL) was treated with HOAc (1.22 g, 20.28 mmol, 1.16 mL, 2.38 eq) and stirred at 60 °C for 30 minutes. NaBH₃CN (1.9 g, 30.24 mmol, 3.54 eq) was then added, and the mixture was stirred at 60 °C for 3 hours. After completion of the reaction, the mixture was diluted with H₂O (200 mL), and the pH was adjusted to 8 using aq. saturated NaHCO₃ solution, followed by extraction with EA (200 mL * 2). The organic layer was dried over Na₂SO₄ and concentrated under reduced pressure. The residue was purified by flash silica gel chromatography (Eluent of 0-20% MeOH/DCM) to afford Compound 81-6 (1.4 g, 39.8% yield) as a yellow solid.

LCMS m/z 396.0 [M+1]⁺.

¹H NMR (400MHz, DMSO-d6) δ 10.72 (s, 1H), 8.71 (d, J =1.4 Hz, 1H), 8.37 (dd, J =1.3, 8.2 Hz, 1H), 8.16 (d, J =8.0 Hz, 1H), 8.06 (dd, J =2.0, 8.0 Hz, 1H), 7.61 (dd, J =1.4, 8.1 Hz, 1H), 7.38 (t, J =8.2 Hz, 1H), 4.53 (br s, 1H), 3.88 (s, 2H), 3.54 - 3.46 (m, 2H), 2.60 (t, J =5.8 Hz, 2H).

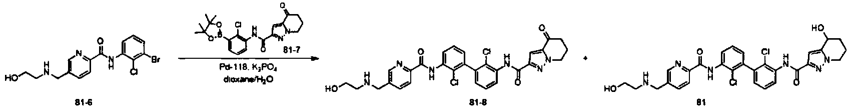

A mixture of Compound 81-6 (500 mg, 1.30 mmol, 1 eq) and Compound 81-7 (800.00 mg, 1.92 mmol, 1.48 eq) in dioxane (20 mL)/H₂O (4 mL) was treated with K₃PO₄ (850 mg, 4.00 mmol, 3.08 eq) and diitert-butyl(cyclopentyl)phosphane;dichloropalladium iron (83.33 mg, 127.86 µmol, 9.84e-2 eq), and the reaction mixture was stirred at 70 °C for 5 hours under a nitrogen atmosphere. After completion of the reaction, the mixture was diluted with H₂O (50 mL) and extracted with EA (50 mL * 2). The organic layer was washed with aq. saturated NaCl solution (50 mL * 3), dried over Na₂SO₄, and concentrated under reduced pressure. The residue was purified by flash silica gel chromatography (Eluent of 0-20% DCM/MeOH) to afford Compound 81-8 (450 mg, 55.4% yield) as a yellow solid and Compound 81 (209.5 mg, 68.5% yield) as a white solid.

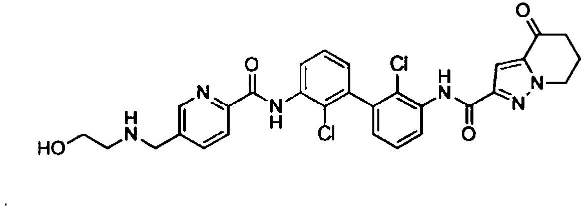

LCMS 594.9 m/z [M+1]⁺.

¹H NMR (400MHz, DMSO-d6) δ 10.73 (s, 1H), 9.83 (s, 1H), 8.70 (s, 1H), 8.53 (d, J =8.0 Hz, 1H), 8.19 (d, J =8.0 Hz, 1H), 8.11 (d, J =8.0 Hz, 1H), 8.06 (br d, J =8.0 Hz, 1H), 7.53 (td, J =8.0, 11.0 Hz, 2H), 7.30 (s, 1H), 7.24 (d, J =7.5 Hz, 1H), 7.19 (d, J =7.4 Hz, 1H), 4.49 (td, J =5.7, 13.4 Hz, 3H), 3.86 (s, 2H), 3.48 (q, J =5.6 Hz, 2H), 2.72 (t, J =6.3 Hz, 2H), 2.68 (s, 1H), 2.58 (t, J =5.8 Hz, 2H), 2.38 - 2.31 (m, 3H).

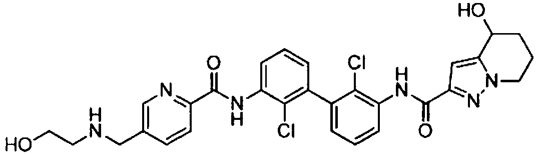

LCMS 594.9m/z [M+1]⁺.

¹H NMR (400MHz, DMSO-d6) δ 10.73 (s, 1H), 9.56 (s, 1H), 8.70 (s, 1H), 8.53 (br d, J =8.0 Hz, 1H), 8.35 - 8.27 (m, 1H), 8.19 (d, J =8.0 Hz, 1H), 8.06 (br d, J =7.8 Hz, 1H), 7.52 (td, J =7.8, 15.4 Hz, 2H), 7.18 (br d, J =7.5 Hz, 2H), 6.75 (s, 1H), 5.61 (br d, J =5.0 Hz, 1H), 4.77 (br d, J =5.3 Hz, 1H), 4.56 - 4.45 (m, 1H), 4.15 (br s, 2H), 3.86 (s, 2H), 3.53 - 3.43 (m, 2H), 2.58 (br t, J=5.8 Hz, 3H), 2.20 - 2.14 (m, 1H), 2.16 (br s, 1H), 2.06 - 1.99 (m, 1H), 1.93 (br d, J =6.5 Hz, 1H), 2.08 - 1.87 (m, 1H), 1.78 - 1.71 (m, 1H).

### 19-2. Preparation of Compound 82, 4-((2-acetamidoethyl)amino)-N-(2,2'-dichloro-3'-(5-(((2-hydroxyethyl)amino)methyl)picolinamido)-[1,1'-biphenyl]-3-yl)-4,5,6,7-tetrahydropyrazolo[1,5-a]pyridine-2-carboxamide

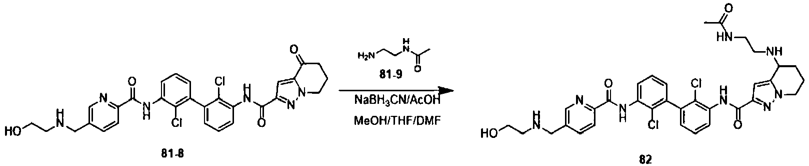

A mixture of Compound 81-8 (200 mg, 337.01 µmol, 1 eq), Compound 81-9 (200.00 mg, 1.96 mmol, 186.92 µL, 5.81 eq), and AcOH (210.00 mg, 3.50 mmol, 200.00 µL, 10.38 eq) in MeOH (4 mL)/THF (4 mL)/DMF (2 mL) was stirred at 60 °C for 30 minutes. NaBH₃CN (120.00 mg, 1.91 mmol, 5.67 eq) was then added, and the reaction mixture was stirred at 60 °C for 18 hours. After completion of the reaction, the mixture was diluted with H₂O (25 mL) and extracted with EA (50 mL * 2). The organic layer was washed with aq. saturated NaCl solution (50 mL * 3), dried over Na₂SO₄, and concentrated under reduced pressure. The residue was purified by prep-HPLC (column: Phenomenex C18 80*40 mm*3 um; mobile phase: [water (NH₄HCO₃)-ACN]; B%: 36%-66%, 8 min) to afford Compound 82 (105.65 mg, 44.3% yield) as a white solid.

LCMS 681.3 m/z [M+1]⁺.

¹H NMR (400MHz, DMSO-d6) δ 10.73 (s, 1H), 9.55 (s, 1H), 8.70 (s, 1H), 8.54 (br d, J =8.0 Hz, 1H), 8.35 - 8.28 (m, 1H), 8.19 (d, J =7.8 Hz, 1H), 8.06 (br d, J =7.8 Hz, 1H), 7.84 (br s, 1H), 7.52 (td, J =7.9, 15.5 Hz, 2H), 7.19 (br d, J =7.5 Hz, 2H), 6.80 (s, 1H), 4.50 (br t, J =5.3 Hz, 1H), 4.21 - 4.10 (m, 2H), 3.92 - 3.85 (m, 3H), 3.48 (q, J =5.4 Hz, 2H), 3.14 (q, J =6.2 Hz, 2H), 2.64 (br t, J =6.4 Hz, 2H), 2.58 (br t, J =5.8 Hz, 3H), 2.19 (br d, J =8.0 Hz, 1H), 2.32 (br d, J =15.6 Hz, 1H), 2.00 (br d, J =4.8 Hz, 1H), 1.91 (br d, J =7.0 Hz, 1H), 1.81 (s, 3H), 1.72 - 1.62 (m, 1H).

### 19-3. Preparation of Compound 83, N-(2,2'-dichloro-3'-(5-(((2-hydroxyethyl)amino)methyl)picolinamido)-[1,1'-biphenyl]-3-yl)-4-((2-hydroxyethyl)amino)-4,5,6,7-tetrahydropyrazolo[1,5-a]pyridine-2-carboxamide

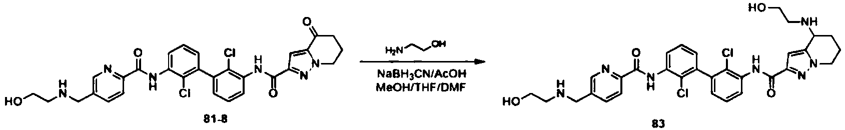

A solution of Compound 81-8 (150 mg, 252.76 µmol, 1 eq), 2-aminoethanol (60.00 mg, 982.29 µmol, 59.41 µL, 3.89 eq), and AcOH (157.50 mg, 2.62 mmol, 150.00 µL, 10.38 eq) in MeOH (3 mL)/THF (3 mL)/DMF (1.5 mL) was stirred at 60 °C for 30 minutes. NaBH₃CN (90.00 mg, 1.43 mmol, 5.67 eq) was then added, and the reaction mixture was stirred at 60 °C for 18 hours. After completion of the reaction, the reaction mixture was diluted with H₂O (25 mL) and extracted with EA (20 mL * 2). The organic layer was washed with aq. saturated NaCl solution (50 mL * 3), dried over Na₂SO₄, and concentrated under reduced pressure. The residue was purified by prep-HPLC (column: Phenomenex C18 80*40 mm*3 um; mobile phase: [water (NH₄HCO₃)-ACN]; B%: 36%-66%, 8 min) to afford Compound 83 (34.94 mg, 21.4% yield) as a white solid.

LCMS 640.3 m/z [M+1]⁺.

¹H NMR (400MHz, DMSO-d6) δ 10.73 (s, 1H), 9.55 (s, 1H), 8.71 (s, 1H), 8.58 - 8.49 (m, 1H), 8.35 - 8.27 (m, 1H), 8.19 (d, J =8.0 Hz, 1H), 8.09 - 8.02 (m, 1H), 7.52 (td, J =8.0, 16.1 Hz, 2H), 7.19 (br d, J =7.5 Hz, 2H), 6.79 (s, 1H), 4.51 (q, J =5.5 Hz, 2H), 4.20 - 4.11 (m, 2H), 3.89 (br d, J =6.3 Hz, 1H), 3.86 (s, 2H), 3.48 (q, J =5.7 Hz, 4H), 2.68 (br d, J =2.0 Hz, 2H), 2.58 (br t, J =5.8 Hz, 3H), 2.18 (br s, 1H), 2.24 - 2.11 (m, 1H), 2.03 (br d, J =7.3 Hz, 1H), 1.91 (br s, 1H), 1.69 (br d, J =11.3 Hz, 1H).

### 19-4. Preparation of Compound 84, N-(2,2'-dichloro-3'-(5-(((2-hydroxyethyl)amino)methyl)picolinamido)-[1,1'-biphenyl]-3-yl)-4-((((S)-5-oxopyrrolidin-3-yl)methyl)amino)-4,5,6,7-tetrahydropyrazolo[1,5-a]pyridine-2-carboxamide

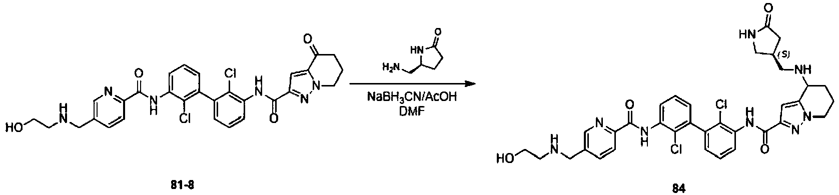

A mixture of Compound 81-8 (120 mg, 202.20 µmol, 1 eq), (5S)-5-(aminomethyl)pyrrolidin-2-one (180.00 mg, 1.20 mmol, 5.91 eq, HCl), and AcOH (126.00 mg, 2.10 mmol, 120.00 µL, 10.38 eq) in DMF (3 mL) was treated with NaBH₃CN (72.00 mg, 1.15 mmol, 5.67 eq), and the reaction mixture was stirred at 60 °C for 18 hours. After completion of the reaction, the mixture was diluted with H₂O (25 mL) and extracted with EA (20 mL * 2). The organic layer was washed with aq. saturated NaCl solution (50 mL * 3), dried over Na₂SO₄, and concentrated under reduced pressure. The residue was purified by prep-HPLC (column: Phenomenex C18 80*40 mm*3 um; mobile phase: [water (NH₄HCO₃)-ACN]; B%: 34%-64%, 8 min) to afford Compound 84 (43.8 mg, 30.9% yield) as a white solid.

LCMS 692.8 m/z [M+1]⁺.

¹H NMR (400MHz, DMSO-d6) δ 10.73 (s, 1H), 9.55 (s, 1H), 8.70 (s, 1H), 8.53 (d, J =6.8 Hz, 1H), 8.34 - 8.27 (m, 1H), 8.19 (d, J =8.0 Hz, 1H), 8.11 - 8.02 (m, 1H), 7.70 (br d, J =18.8 Hz, 1H), 7.52 (td, J =7.9, 15.5 Hz, 2H), 7.19 (br d, J =7.5 Hz, 2H), 6.84 (d, J =11.8 Hz, 1H), 4.50 (t, J =5.4 Hz, 1H), 4.21 - 4.10 (m, 2H), 3.86 (s, 3H), 3.58 (br s, 1H), 3.48 (q, J =5.6 Hz, 2H), 2.68 (br s, 1H), 2.62 - 2.55 (m, 4H), 2.22 - 1.98 (m, 6H), 1.91 (br d, J =7.0 Hz, 1H), 1.77 - 1.65 (m, 2H).

### 19-5. Preparation of Compound 85, N-(2,2'-dichloro-3'-(5-(((2-hydroxyethyl)amino)methyl)picolinamido)-[1,1'-biphenyl]-3-yl)-4-((R)-3-hydroxypyrrolidin-1-yl)-4,5,6,7-tetrahydropyrazolo[1,5-a]pyridine-2-carboxamide

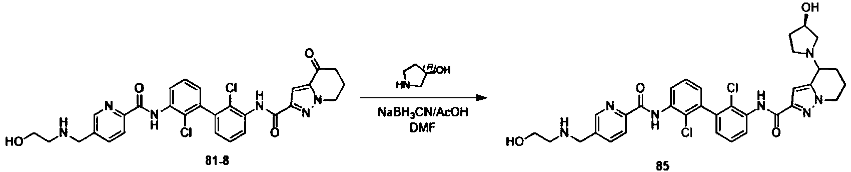

A mixture of Compound 81-8 (250 mg, 421.26 µmol, 1 eq), (3R)-pyrrolidin-3-ol (250.01 mg, 2.87 mmol, 238.10 µL, 6.81 eq), and AcOH (262.50 mg, 4.37 mmol, 250 µL, 10.38 eq) in DMF (6 mL) was stirred at 60 °C for 30 minutes. NaBH₃CN (150.00 mg, 2.39 mmol, 5.67 eq) was then added, and the reaction mixture was stirred at 60 °C for 18 hours. After completion of the reaction, the mixture was diluted with H₂O (20 mL), and the pH was adjusted to 8 using aq. saturated NaHCO₃ solution, followed by extraction with EA (50 mL * 2). The organic layer was dried over Na₂SO₄ and concentrated under reduced pressure. The residue was purified by prep-HPLC (column: Phenomenex C18 80*40 mm*3 um; mobile phase: [water (NH₃H₂O+NH₄HCO₃)-ACN]; B%: 36%-66%, 8 min) to afford Compound 85 (82.65 mg, 28.7% yield) as a white solid.

LCMS 664.4 m/z [M+1]⁺.

¹H NMR (400MHz, DMSO-d6) δ 10.73 (s, 1H), 9.56 (s, 1H), 8.71 (s, 1H), 8.53 (br d, J =8.0 Hz, 1H), 8.28 (br d, J =7.5 Hz, 1H), 8.19 (d, J =8.0 Hz, 1H), 8.07 (br d, J =7.5 Hz, 1H), 7.52 (td, J =7.7, 15.9 Hz, 2H), 7.19 (br d, J =7.5 Hz, 2H), 6.74 (d, J =8.5 Hz, 1H), 4.76 - 4.67 (m, 1H), 4.53 (br s, 1H), 4.18 (br s, 3H), 3.88 (s, 2H), 3.81 (br s, 1H), 3.48 (br d, J =5.3 Hz, 1H), 2.92 - 2.87 (m, 1H), 2.78 (br dd, J =6.4, 14.2 Hz, 2H), 2.71 (br d, J =7.5 Hz, 1H), 2.59 (br t, J =5.8 Hz, 3H), 2.44 (br d, J =6.5 Hz, 1H), 2.24 (br s, 1H), 1.96 (br s, 1H), 1.89 (br s, 3H), 1.57 (br s, 1H).

### 19-6. Preparation of Compound 86, (3R)-1-(2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

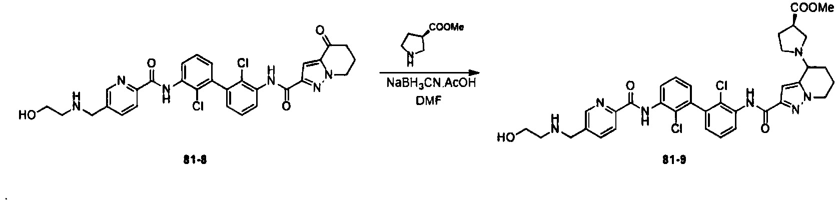

A solution of Compound 81-8 (250 mg, 421.26 µmol, 1 eq), (3R)-pyrrolidine-3-carboxylic acid (250.01 mg, 2.17 mmol, 5.15 eq), and AcOH (262.51 mg, 4.37 mmol, 250.01 µL, 10.38 eq) in DMF (6 mL) was stirred at 60 °C for 30 minutes. NaBH₃CN (150.00 mg, 2.39 mmol, 5.67 eq) was then added, and the reaction mixture was stirred at 60 °C for 30 minutes. After completion of the reaction, the mixture was diluted with H₂O (25 mL) and extracted with EA (20 mL * 2). The organic layer was washed with aq. saturated NaCl solution (50 mL * 3), dried over Na₂SO₄, and concentrated under reduced pressure. The residue was purified by flash silica gel chromatography (Eluent of 0-40% MeOH/EA) to afford Compound 81-9 (110 mg, 35.1% yield) as a white solid.

LCMS 706.4 m/z [M+1]⁺.

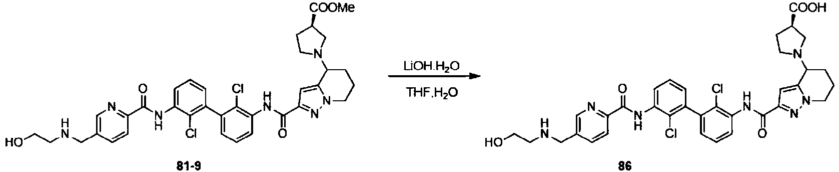

A mixture of Compound 81-9 (110 mg, 155.67 µmol, 1 eq) in THF (2 mL)/H₂O (0.5 mL) was treated with LiOH·H₂O (50 mg, 2.09 mmol, 13.41 eq) and stirred at room temperature for 5 hours. The pH of the reaction mixture was adjusted to 7 using an aq. 1N HCl solution, followed by concentration under reduced pressure. The residue was purified by prep-HPLC (column: Phenomenex C18 80*40mm*3um; mobile phase: [water (NH₃H₂O+NH₄HCO₃)-ACN]; B%: 17%-47%, 8 min) to afford Compound 86 (41.11 mg, 37.99% yield) as a white solid.

LCMS 692.4 m/z [M+1]⁺.

¹H NMR (400MHz, DMSO-d6) δ 10.72 (s, 1H), 9.56 (s, 1H), 8.69 (s, 1H), 8.52 (d, J =8.0 Hz, 1H), 8.28 (br s, 1H), 8.18 (d, J =8.0 Hz, 1H), 8.05 (d, J =7.5 Hz, 1H), 7.51 (td, J =8.0, 15.7 Hz, 2H), 7.18 (d, J =7.5 Hz, 2H), 6.72 (d, J =4.5 Hz, 1H), 4.49 (br s, 1H), 4.17 (br d, J =10.8 Hz, 2H), 3.87 - 3.79 (m, 3H), 3.47 (br t, J =5.4 Hz, 2H), 2.90 (br s, 2H), 2.86 - 2.77 (m, 1H), 2.72 (br s, 2H), 2.62 - 2.54 (m, 3H), 2.24 (br s, 1H), 2.00 - 1.86 (m, 5H).

### 19-7. Preparation of Compound 87, N-(2,2'-dichloro-3'-(5-(((2-hydroxyethyl)amino)methyl)picolinamido)-[1,1'-biphenyl]-3-yl)-4-(4-hydroxypiperidin-1-yl)-4,5,6,7-tetrahydropyrazolo[1,5-a]pyridine-2-carboxamide

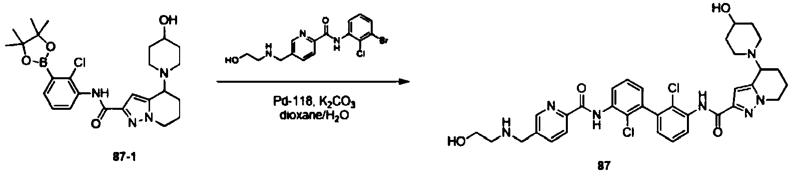

A mixture of N-(3-bromo-2-chloro-phenyl)-5-[(2-hydroxyethylamino)methyl]pyridine-2-carboxamide (69.82 mg, 181.52 µmol, 1 eq), N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (100 mg, 199.67 µmol, 1.1 eq), ditert-butyl(cyclopentyl)phosphane;dichloropalladium iron (11.83 mg, 18.15 µmol, 0.1 eq), and K₂CO₃ (75.26 mg, 544.56 µmol, 3 eq) in dioxane (5 mL)/H₂O (1 mL) was stirred at 90 °C for 12 hours under a nitrogen atmosphere. After completion of the reaction, the mixture was concentrated under reduced pressure and purified by reversed-phase HPLC (column: Waters XBridge 150 * 25 mm * 5 um; mobile phase: [water (NH₄HCO₃) - ACN]; gradient: 32% - 62% B over 9 min) to afford Compound 87 (26 mg, 20.9% yield) as a white solid.

LCMS m/z 678.3 [M+1]⁺.

¹H NMR (400 MHz, DMSO-d6) δ ppm 1.30 - 1.51 (m, 2 H) 1.61 - 1.80 (m, 3 H) 1.82 - 2.02 (m, 2 H) 2.10 - 2.45 (m, 4 H) 2.58 (t, J=5.75 Hz, 2 H) 2.62 - 2.79 (m, 2 H) 3.48 (q, J=5.71 Hz, 3 H) 3.86 (s, 2 H) 3.97 (br dd, J=9.76, 5.13 Hz, 1 H) 4.01 - 4.12 (m, 1 H) 4.16 - 4.25 (m, 1 H) 4.47 - 4.61 (m, 2 H) 6.66 (s, 1 H) 7.19 (d, J=7.50 Hz, 2 H) 7.52 (dt, J=15.54, 7.93 Hz, 2 H) 8.06 (dd, J=8.00, 2.00 Hz, 1 H) 8.19 (d, J=8.00 Hz, 1 H) 8.28 (ddd, J=8.16, 4.53, 1.44 Hz, 1 H) 8.53 (dd, J=8.32, 1.44 Hz, 1 H) 8.70 (d, J=1.38 Hz, 1 H) 9.55 (d, J=1.63 Hz, 1 H) 10.73 (s, 1 H).

### 19-8. Preparation of Compound 89, 2-((2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)amino)acetic acid

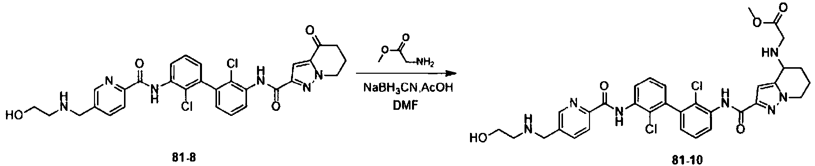

A solution of Compound 81-8 (500 mg, 842.52 µmol, 1 eq), methyl 2-aminoacetate; hydrochloride (400 mg, 3.19 mmol, 3.78 eq), and AcOH (525.01 mg, 8.75 mmol, 500.01 µL, 10.38 eq) in DMF (6 mL) was stirred at 60 °C for 30 minutes. NaBH₃CN (300.00 mg, 4.78 mmol, 5.67 eq) was then added, and the reaction mixture was stirred at 60 °C for 18 hours. After completion of the reaction, the mixture was diluted with H₂O (25 mL) and extracted with EA (20 mL * 2). The organic layer was washed with aq. saturated NaCl solution (50 mL * 3), dried over Na₂SO₄, and concentrated under reduced pressure. The residue was purified by flash silica gel chromatography (Eluent of 0-30% DCM/MeOH) to afford Compound 81-10 (160 mg, 13.3% yield) as a brown solid.

LCMS 666.4 m/z [M+1]⁺.

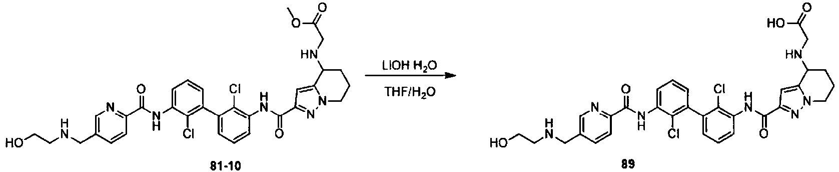

A solution of Compound 81-10 (180 mg, 270.05 µmol, 1 eq) in THF (4 mL)/H₂O (1 mL) was treated with LiOH·H₂O (70 mg, 2.92 mmol, 10.82 eq) and stirred at room temperature for 5 hours. The pH of the reaction mixture was adjusted to 7 using an aq. 1N HCl solution, followed by concentration under reduced pressure. The residue was purified by prep-HPLC (column: Phenomenex C18 80*40 mm*3 um; mobile phase: [water (NH₃H₂O+NH₄HCO₃)-ACN]; B%: 16%-46%, 8 min) to afford Compound 89 (53.19 mg, 30.1% yield) as a white solid.

LCMS 652.3 m/z [M+1]⁺.

¹H NMR (400MHz, DMSO-d6) δ 10.73 (s, 1H), 9.57 (s, 1H), 8.70 (s, 1H), 8.53 (d, J =8.0 Hz, 1H), 8.27 (d, J =8.0 Hz, 1H), 8.19 (d, J =7.8 Hz, 1H), 8.06 (br d, J =8.0 Hz, 1H), 7.51 (td, J =8.0, 15.7 Hz, 2H), 7.18 (d, J =7.8 Hz, 2H), 6.82 (s, 1H), 4.15 (br t, J =6.0 Hz, 2H), 4.03 (br t, J =5.4 Hz, 1H), 3.87 (s, 2H), 3.49 - 3.46 (m, 6H), 3.30 (s, 2H), 2.58 (t, J =5.8 Hz, 2H), 2.18 (br s, 1H), 2.00 (br s, 1H), 1.90 (br d, J =4.3 Hz, 1H), 1.74 (br s, 1H).

### 19-9. Preparation of Compound 88, 1-(2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)piperidine-4-carboxylic acid

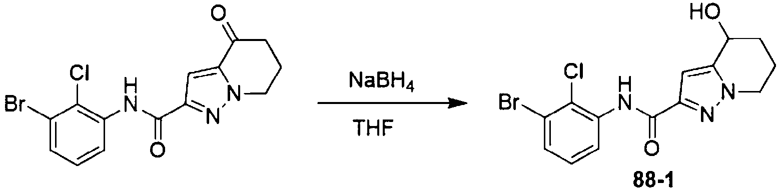

A solution of N-(3-bromo-2-chloro-phenyl)-4-oxo-6,7-dihydro-5H-pyrazolo[1,5-a]pyridine-2-carboxamide (7 g, 18.99 mmol, 1 eq) in THF (80 mL) was treated with NaBH₄ (3.59 g, 94.95 mmol, 5 eq) at 0 °C, and the reaction mixture was stirred at room temperature for 16 hours. After completion of the reaction, aq. 4 M HCl (50 mL) was added, followed by concentration under reduced pressure. The residue was purified by column chromatography (SiO₂, Petroleum ether/Ethyl acetate = 1/1 to 0/1) to afford Compound 88-1 (2.5 g, 33.3% yield) as a yellow solid.

LCMS m/z 368.99 [M+3]⁺.

¹H NMR (400 MHz, DMSO-d6) δ ppm 1.66 - 1.77 (m, 2 H) 2.11 - 2.21 (m, 2 H) 4.13 - 4.18 (m, 2 H) 5.49 - 5.54 (m, 1 H) 6.72 (s, 1 H) 6.69 - 6.79 (m, 1 H) 7.32 (t, J=8.13 Hz, 1 H) 7.58 (dd, J=8.07, 1.19 Hz, 1 H) 8.11 (d, J=7.68 Hz, 1 H) 9.63 (s, 1 H).

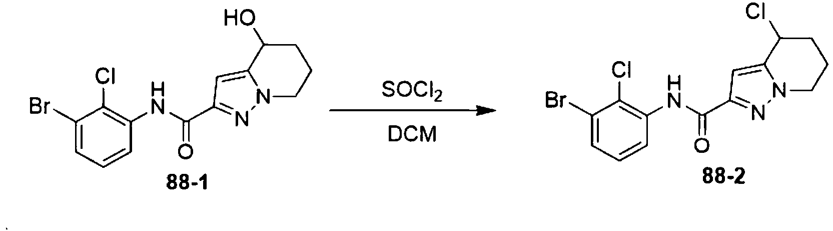

A solution of N-(3-bromo-2-chloro-phenyl)-4-hydroxy-4,5,6,7-tetrahydropyrazolo[1,5-a]pyridine-2-carboxamide (2.5 g, 6.75 mmol, 1 eq) in DCM (30 mL) was treated with SOCl₂ (4.01 g, 33.73 mmol, 2.45 mL, 5 eq) at 0 °C, and the reaction mixture was stirred at room temperature for 12 hours. After completion of the reaction, aq. sat'd NH₄Cl solution (200 mL) was added, and the mixture was extracted with EtOAc (200 mL * 2). The organic layer was washed with brine (200 mL), dried over Na₂SO₄, and concentrated under reduced pressure to afford Compound 88-2 (2.2 g, 79.6% yield) as a yellow solid, which was used in the next reaction without further purification.

LCMS m/z 386.95 [M+3]⁺.

¹H NMR (400 MHz, DMSO-d6) δ ppm 2.10 - 2.20 (m, 2 H) 2.28 - 2.37 (m, 2 H) 4.03 (q, J=7.13 Hz, 1 H) 4.37 (dt, J=13.04, 4.61 Hz, 1 H) 5.67 (t, J=4.13 Hz, 1 H) 6.84 (s, 1 H) 7.33 (t, J=8.07 Hz, 1 H) 7.61 (dd, J=8.07, 1.31 Hz, 1 H) 8.01 (dd, J=8.19, 1.19 Hz, 1 H) 9.73 (s, 1 H).

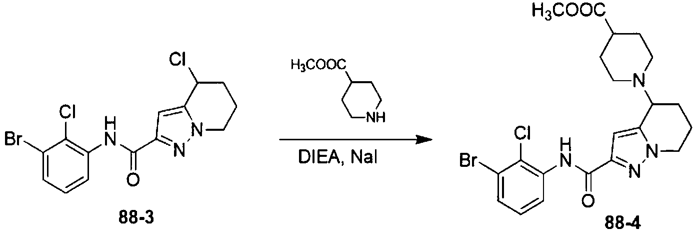

A solution of N-(3-bromo-2-chloro-phenyl)-4-chloro-4,5,6,7-tetrahydropyrazolo[1,5-a]pyridine-2-carboxamide (1 g, 2.57 mmol, 1 eq) and methyl piperidine-4-carboxylate (1.10 g, 7.71 mmol, 3 eq) was treated with DIPEA (996.54 mg, 7.71 mmol, 1.34 mL, 3 eq) and Nal (115.58 mg, 771.06 µmol, 0.3 eq), and the reaction mixture was stirred at 40 °C for 12 hours. Methanol (40 mL) was added to the mixture to dissolve the precipitated solid, followed by addition of H₂O (500 mL) to re-precipitate the solid. The precipitated solid was collected by filtration to afford Compound 88-4 (1.2 g, 60.2% yield) as a yellow solid, which was used in the next reaction without further purification.

LCMS m/z 494.07 [M+3]⁺.

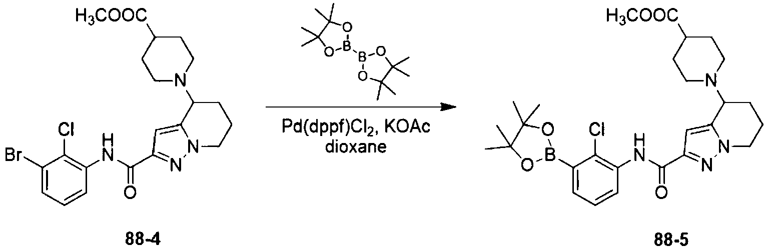

A mixture of methyl 1-[2-[(3-bromo-2-chloro-phenyl)carbamoyl]-4,5,6,7-tetrahydropyrazolo[1,5-a]pyridin-4-yl]piperidine-4-carboxylate (300 mg, 605.09 µmol, 1 eq), 4,4,5,5-tetramethyl-2-(4,4,5,5-tetramethyl-1,3,2-dioxaborolan-2-yl)-1,3,2-dioxaborolane (460.96 mg, 1.82 mmol, 3 eq), AcOK (178.15 mg, 1.82 mmol, 3 eq), and Pd(dppf)Cl₂ (44.27 mg, 60.51 µmol, 0.1 eq) in dioxane (4 mL) was stirred at 90 °C for 12 hours under a nitrogen atmosphere. After completion of the reaction, the mixture was concentrated under reduced pressure and purified by prep-TLC (SiO₂, DCM:MeOH = 10:1) to afford Compound 88-5 (500 mg, 91.3% yield) as a yellow solid.

LCMS m/z 542.25 [M+1]⁺.

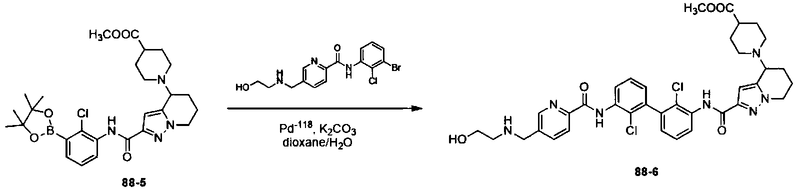

A mixture of methyl 1-[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (359.88 mg, 662.93 µmol, 1.5 eq), N-(3-bromo-2-chloro-phenyl)-5-[(2-hydroxyethylamino)methyl]pyridine-2-carboxamide (170 mg, 441.95 µmol, 1 eq), ditert-butyl(cyclopentyl)phosphane;dichloropalladium;iron (28.80 mg, 44.20 µmol, 0.1 eq), and K₂CO₃ (183.24 mg, 1.33 mmol, 3 eq) in dioxane (5 mL)/H₂O (1 mL) was stirred at 90 °C for 12 hours under a nitrogen atmosphere. After completion of the reaction, the mixture was concentrated under reduced pressure and purified by prep-TLC (SiO₂, DCM:MeOH = 0:1) to afford Compound 88-6 (200 mg, 44.5% yield) as a white solid.

LCMS m/z 719.24 [M+1]⁺.

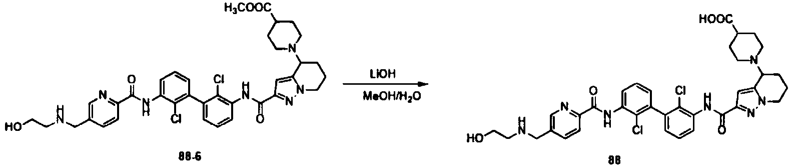

A mixture of methyl 1-[2-[[2-chloro-3-[2-chloro-3-[[5-[(2-hydroxyethylamino)methyl]pyridine-2-carbonyl]amino]phenyl]phenyl]carbamoyl]-4,5,6,7-tetrahydropyrazolo[1,5-a]pyridin-4-yl]piperidine-4-carboxylate (200 mg, 277.53 µmol, 1 eq) in H₂O (4 mL)/MeOH (4 mL) was treated with LiOH·H₂O (34.94 mg, 832.59 µmol, 3 eq) and stirred at room temperature for 3 hours. After completion of the reaction, the mixture was concentrated under reduced pressure, and the residue was purified by reversed-phase HPLC (column: Waters XBridge Prep OBD C18 150 * 40 mm * 10 um; mobile phase: [water (NH₄HCO₃) - ACN]; gradient: 5% - 35% B over 15 min) to afford Compound 88 (22 mg, 11.0% yield) as a white solid.

LCMS m/z 705.22 [M+3]⁺.

¹H NMR (400 MHz, DMSO-d6) δ ppm 1.47 - 1.72 (m, 3 H) 1.80 (br t, J=11.63 Hz, 2 H) 1.88 - 2.00 (m, 2 H) 2.07 - 2.30 (m, 3 H) 2.38 - 2.47 (m, 1 H) 2.59 (br s, 2 H) 2.66 (br d, J=10.51 Hz, 1 H) 2.78 (br d, J=10.13 Hz, 1 H) 3.48 (br t, J=5.57 Hz, 2 H) 3.91 - 4.08 (m, 3 H) 4.19 (br d, J=11.38 Hz, 2 H) 4.49 (br s, 3 H) 6.66 (s, 1 H) 7.17 (br d, J=7.50 Hz, 2 H) 7.43 - 7.57 (m, 2 H) 8.05 (br d, J=7.63 Hz, 1 H) 8.18 (d, J=8.00 Hz, 1 H) 8.28 (br dd, J=7.07, 4.32 Hz, 1 H) 8.53 (d, J=7.50 Hz, 1 H) 8.69 (s, 1 H) 9.53 (s, 1 H) 10.72 (s, 1 H).

### 19-10. Preparation of Compound 90, 2-((2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)amino)-2-methylpropanoic acid

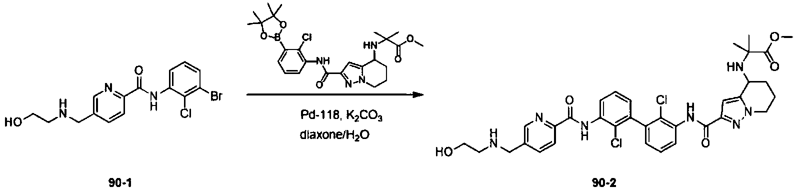

A mixture of N-(3-bromo-2-chloro-phenyl)-5-[(2-hydroxyethylamino)methyl]pyridine-2-carboxamide (110 mg, 285.97 µmol, 1 eq), methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (162.58 mg, 314.57 µmol, 1.1 eq), ditert-butyl(cyclopentyl)phosphane;dichloropalladium iron (18.64 mg, 28.60 µmol, 0.1 eq), and K₂CO₃ (118.57 mg, 857.91 µmol, 3 eq) in dioxane (5 mL)/H₂O (1 mL) was stirred at 90 °C for 12 hours under a nitrogen atmosphere. After completion of the reaction, the mixture was concentrated under reduced pressure, and the residue was purified by prep-TLC (SiO₂, Ethyl acetate:Methanol = 3:1) to afford Compound 90-2 (200 mg, 80.5% yield) as a white solid.

LCMS m/z 694.2 [M+1]⁺.

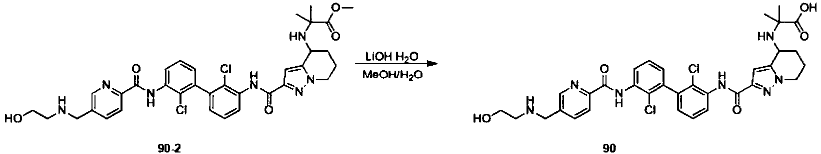

A solution of methyl 2-[[2-[[2-chloro-3-[2-chloro-3-[[5-[(2-hydroxyethylamino)methyl]pyridine-2-carbonyl]amino]phenyl]phenyl]carbamoyl]-4,5,6,7-tetrahydropyrazolo[1,5-a]pyridin-4-yl]amino]-2-methyl-propanoate (200 mg, 287.93 µmol, 1 eq) in H₂O (3 mL)/MeOH (3 mL) was treated with LiOH·H₂O (36.25 mg, 863.80 µmol, 3 eq) and stirred at room temperature for 12 hours. After completion of the reaction, the mixture was concentrated under reduced pressure, and the residue was purified by reversed-phase HPLC (column: Waters XBridge 150 * 25 mm * 5 um; mobile phase: [water (NH₄HCO₃) - ACN]; gradient: 15% - 45% B over 9 min) to afford Compound 90 (70 mg, 35.3% yield) as a white solid.

LCMS m/z 680.3 [M+3]⁺.

¹H NMR (400 MHz, DMSO-d6) δ ppm 1.29 (d, J=3.75 Hz, 7 H) 1.58 - 1.69 (m, 1 H) 1.85 - 2.19 (m, 4 H) 2.59 (t, J=5.82 Hz, 2 H) 3.87 (s, 2 H) 3.91 - 3.97 (m, 1 H) 4.07 - 4.17 (m, 2 H) 6.80 (s, 1 H) 7.18 (dt, J=7.63, 1.75 Hz, 2 H) 7.52 (dt, J=15.70, 7.91 Hz, 2 H) 8.06 (dd, J=8.00, 2.00 Hz, 1 H) 8.19 (d, J=8.00 Hz, 1 H) 8.27 - 8.32 (m, 1 H) 8.53 (dd, J=8.25, 1.38 Hz, 1 H) 8.70 (d, J=1.38 Hz, 1 H) 9.54 (s, 1 H) 10.73 (s, 1 H).

### [Preparation Example 20]

### Preparation of Compounds 91 to 99

### 20-1. Preparation of Compound 91, 4-((2-hydroxyethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

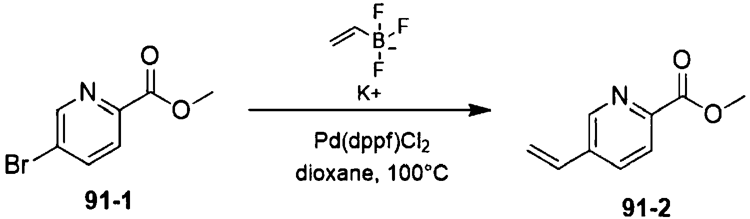

A mixture of methyl 5-bromopicolinate (15 g, 69.43 mmol, 1 eq), potassium trifluoro(vinyl)boranuide (18.60 g, 138.87 mmol, 2 eq), K₃PO₄ (44.22 g, 208.30 mmol, 3 eq), and Pd(dppf)Cl₂ (2.84 g, 3.47 mmol, 0.05 eq) in dioxane (150 mL) was stirred at 100 °C for 2 hours under a nitrogen atmosphere. After completion of the reaction, the mixture was diluted with H₂O (400 mL) and extracted with EtOAc (200 mL * 3). The organic layer was washed with brine (400 mL), dried over Na₂SO₄, and concentrated under reduced pressure. The residue was purified by flash silica gel chromatography (Petroleum ether/Ethyl acetate = 100/1 to 3/1) to afford Compound 91-2 (10.2 g, 81.0% yield) as a yellow liquid.

LCMS m/z 164.1 [M+1]⁺.

¹H NMR (400 MHz, CHLOROFORM-d) δ = 8.64 (d, J = 2.0 Hz, 1H), 8.01 (d, J = 8.1 Hz, 1H), 7.78 (dd, J = 2.3, 8.1 Hz, 1H), 6.68 (dd, J = 11.0, 17.6 Hz, 1H), 5.88 (d, J = 17.6 Hz, 1H), 5.44 (d, J = 11.0 Hz, 1H), 3.92 (s, 3H).

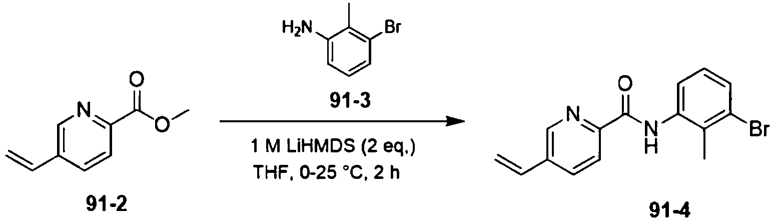

A solution of methyl 5-vinylpyridine-2-carboxylate (9.2 g, 56.38 mmol, 1 eq) and 3-bromo-2-methyl-aniline (10.49 g, 56.38 mmol, 6.95 mL, 1 eq) in THF (150 mL) was treated with LiHMDS (1 M, 112.76 mL, 2 eq) at 0 °C, and the mixture was then stirred at 25 °C for 2 hours. After completion of the reaction, the mixture was diluted with H₂O (200 mL) and extracted with EtOAc (200 mL * 3). The organic layer was washed with brine (600 mL), dried over Na₂SO₄, and concentrated under reduced pressure. The residue was purified by flash silica gel chromatography (Petroleum ether/Ethyl acetate = 100/1 to 3/1) to afford Compound 91-4 (11.2 g, 56.3% yield) as a colorless liquid.

LCMS m/z 317.0 [M+1]⁺.

¹H NMR (400 MHz, DMSO-d6) δ = 10.46 (s, 1H), 8.81 (d, J = 1.8 Hz, 1H), 8.21 - 7.97 (m, 2H), 7.69 (d, J = 7.8 Hz, 1H), 7.48 (d, J = 7.3 Hz, 1H), 7.18 (t, J = 8.1 Hz, 1H), 6.89 (dd, J = 11.1, 17.8 Hz, 1H), 6.80 - 6.71 (m, 1H), 6.16 (d, J = 17.8 Hz, 1H), 5.55 (d, J = 11.3 Hz, 1H), 2.34 (s, 3H)

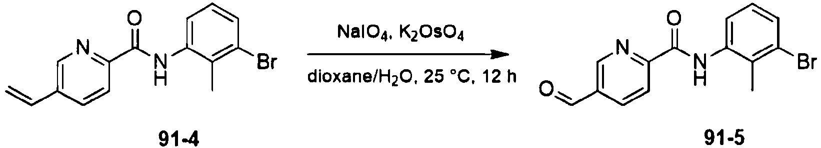

A mixture of N-(3-bromo-2-methyl-phenyl)-5-vinyl-pyridine-2-carboxamide (3 g, 9.46 mmol, 1 eq) and 6-dimethylpyridine (2.03 g, 18.92 mmol, 2.20 mL, 2 eq) in dioxane (80 mL)/H₂O (5 mL) was treated with dipotassium dioxido(dioxo)osmium dihydrate (697.00 mg, 1.89 mmol, 0.2 eq) and sodium periodate (8.09 g, 37.83 mmol, 2.10 mL, 4 eq), which were slowly added over 30 minutes at room temperature. The reaction mixture was then stirred at room temperature for 12 hours. After completion of the reaction, the mixture was diluted with H₂O (20 mL) and extracted with EtOAc (10 mL * 3). The organic layer was washed with brine (20 mL), dried over Na₂SO₄, and concentrated under reduced pressure. The residue was purified by flash silica gel chromatography (Petroleum ether/Ethyl acetate = 100/1 to 3/1) to afford Compound 91-5 (1.5 g, 47.2% yield) as a white solid.

LCMS m/z 319.0 [M+1]⁺.

¹H NMR (400 MHz, DMSO-d6) δ = 10.65 (s, 1H), 10.23 (s, 1H), 9.33 - 9.09 (m, 1H), 8.49 (dd, J = 2.0, 8.0 Hz, 1H), 8.32 (d, J = 8.0 Hz, 1H), 7.67 - 7.47 (m, 2H), 7.20 (t, J = 8.0 Hz, 1H), 2.33 (s, 3H).

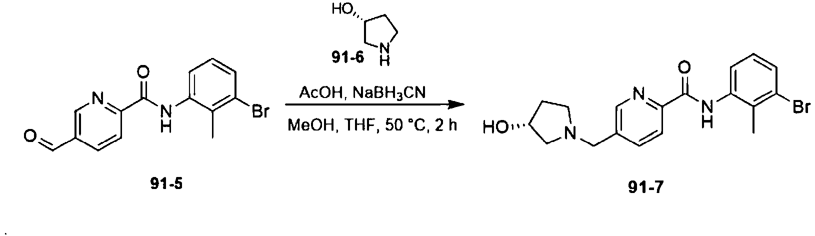

A solution of N-(3-bromo-2-methyl-phenyl)-5-formyl-pyridine-2-carboxamide (1.5 g, 4.70 mmol, 1 eq) and (3R)-pyrrolidin-3-ol (409.46 mg, 4.70 mmol, 389.96 µL, 1 eq) in MeOH (60 mL)/THF (10 mL) was treated with HOAc (15.86 g, 264.08 mmol, 15.10 mL, 56.19 eq) and stirred at 50 °C for 30 minutes. NaBH₃CN (1.48 g, 23.50 mmol, 5 eq) was then added, and the reaction mixture was stirred at 50 °C for 2 hours. After completion of the reaction, the mixture was concentrated under reduced pressure, and the residue was purified by prep-HPLC (column: Phenomenex Luna C18 (250 * 70 mm *10 µm); mobile phase: [water (FA) - ACN]; B%: 10%-40%, 20 min) to afford Compound 91-7 (1.2 g, 58.8% yield) as a white solid.

LCMS m/z 392.0 [M+2]⁺.

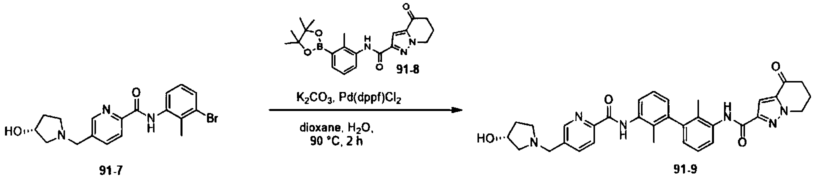

A mixture of N-(3-bromo-2-methyl-phenyl)-5-[[(3R)-3-hydroxypyrrolidin-1-yl]methyl]pyridine-2-carboxamide (800 mg, 2.05 mmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (1.22 g, 3.07 mmol, 1.5 eq), K₂CO₃ (793.27 mg, 5.74 mmol, 2.8 eq), and Pd(dppf)Cl₂ (83.70 mg, 102.49 µmol, 0.05 eq) in dioxane (10 mL)/H₂O (1 mL) was stirred at 90 °C for 2 hours under a nitrogen atmosphere. After completion of the reaction, the mixture was diluted with H₂O (20 mL) and extracted with EtOAc (10 mL * 3). The organic layer was washed with brine (20 mL), dried over Na₂SO₄, and concentrated under reduced pressure. The residue was purified by flash silica gel chromatography (Dichloromethane:Methanol = 100/1 to 5/1) to afford Compound 91-9 (1.02 g, 60.3% yield) as a white solid.

LCMS m/z 579.1 [M+2]⁺.

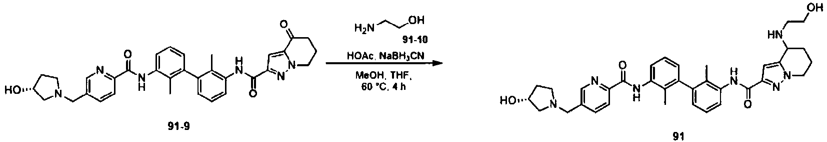

A solution of N-[3-[3-[[5-[[(3R)-3-hydroxypyrrolidin-1-yl]methyl]pyridine-2-carbonyl]amino]-2-methyl-phenyl]-2-methyl-phenyl]-4-oxo-6,7-dihydro-5H-pyrazolo[1,5-a]pyridine-2-carboxamide (200 mg, 345.63 µmol, 1 eq), 2-aminoethanol (211.12 mg, 3.46 mmol, 209.03 µL, 10 eq), and HOAc (207.55 mg, 3.46 mmol, 197.67 µL, 10 eq) in MeOH (10 mL)/THF (10 mL) was stirred at 60 °C for 2 hours. NaBH₃CN (65.16 mg, 1.04 mmol, 3 eq) was then added, and the reaction mixture was stirred at 60 °C for an additional 2 hours. After completion of the reaction, the mixture was concentrated under reduced pressure, and the residue was purified by prep-HPLC (column: Waters XBridge 150 * 25 mm * 5 µm; mobile phase: [water (ammonia hydroxide v/v) - ACN]; B%: 20%-50%, 9 min) to afford Compound 91 (32 mg, 14.1% yield) as a white solid.

LCMS m/z 624.4 [M+1]⁺.

¹H NMR (400 MHz, DMSO-d6) δ = 10.32 (br s, 1H), 9.51 (br s, 1H), 8.64 (br s, 1H), 8.14 (br d, J = 8.1 Hz, 1H), 7.98 (br d, J = 7.8 Hz, 1H), 7.87 (br d, J = 7.8 Hz, 1H), 7.58 (br d, J = 8.0 Hz, 1H), 7.30 (td, J = 7.6, 15.6 Hz, 2H), 6.98 (br d, J = 7.4 Hz, 2H), 6.71 (s, 1H), 4.72 (br d, J = 3.0 Hz, 1H), 4.51 (br s, 1H), 4.28 - 4.08 (m, 3H), 3.88 (br s, 1H), 3.71 (br d, J = 10.9 Hz, 2H), 3.48 (br d, J = 5.1 Hz, 2H), 2.76 - 2.61 (m, 4H), 2.42 - 2.30 (m, 2H), 2.27 - 2.07 (m, 2H), 2.01 (br s, 3H), 1.94 (br s, 3H), 1.76 - 1.52 (m, 2H).

### 20-2. Preparation of Compound 92, 4-((R)-3-hydroxypyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

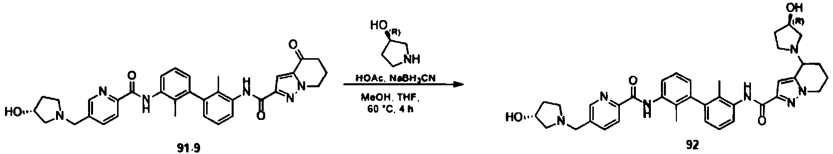

A solution of N-[3-[3-[[5-[[(3R)-3-hydroxypyrrolidin-1-yl]methyl]pyridine-2-carbonyl]amino]-2-methyl-phenyl]-2-methyl-phenyl]-4-oxo-6,7-dihydro-5H-pyrazolo[1,5-a]pyridine-2-carboxamide (200 mg, 345.63 µmol, 1 eq), (3R)-pyrrolidin-3-ol (301.11 mg, 3.46 mmol, 286.77 µL, 10 eq), and HOAc (207.55 mg, 3.46 mmol, 197.67 µL, 10 eq) in MeOH (10 mL)/THF (10 mL) was stirred at 60 °C for 2 hours. NaBH₃CN (65.16 mg, 1.04 mmol, 3 eq) was then added, and the reaction mixture was stirred at 60 °C for an additional 2 hours. After completion of the reaction, the mixture was concentrated under reduced pressure, and the residue was purified by prep-HPLC (column: Waters XBridge 150 * 25 mm * 5 µm; mobile phase: [water (NH₄HCO₃) - ACN]; B%: 24%-54%, 9 min) to afford Compound 92 (21 mg, 8.88% yield) as a white solid.

LCMS m/z 650.4 [M+1]⁺.

¹H NMR (400 MHz, DMSO-d6) δ = 10.32 (s, 1H), 9.53 (br s, 1H), 8.64 (s, 1H), 8.14 (br d, J = 8.0 Hz, 1H), 7.98 (br d, J = 8.1Hz, 1H), 7.87 (br d, J = 8.0 Hz, 1H), 7.58 (br d, J = 7.6 Hz, 1H), 7.30 (td, J = 7.7, 15.7 Hz, 2H), 6.98 (br d, J = 7.4 Hz, 2H),6.67 (br d, J = 7.1 Hz, 1H), 4.87 - 4.55 (m, 2H), 4.30 - 4.08 (m, 4H), 3.84 - 3.61 (m, 3H), 2.72 (br s, 2H), 2.68 - 2.58 (m, 2H), 2.46 - 2.39 (m, 2H), 2.39 - 2.14 (m, 3H), 2.01 (s, 3H), 1.94 (s, 3H), 1.89 (br s, 2H), 1.56 (br d, J = 3.1 Hz, 2H).

### 20-3. Preparation of Compound 93, N-(3'-(5-(((R)-3-hydroxypyrrolidin-1-yl)methyl)picolinamido)-2,2'-dimethyl-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

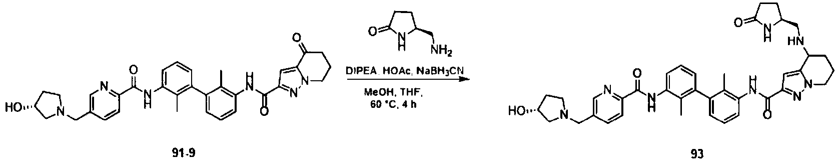

A mixture of N-[3-[3-[[5-[[(3R)-3-hydroxypyrrolidin-1-yl]methyl]pyridine-2-carbonyl]amino]-2-methyl-phenyl]-2-methyl-phenyl]-4-oxo-6,7-dihydro-5H-pyrazolo[1,5-a]pyridine-2-carboxamide (200 mg, 345.63 µmol, 1 eq), (5S)-5-(aminomethyl)pyrrolidin-2-one (394.52 mg, 3.46 mmol, 297.04 µL, 10 eq), and DIPEA (446.69 mg, 3.46 mmol, 602.00 µL, 10 eq) in MeOH (5 mL)/THF (1 mL) was treated with HOAc (415.10 mg, 6.91 mmol, 395.33 µL, 20 eq) and stirred at 60 °C for 2 hours. NaBH₃CN (108.60 mg, 1.73 mmol, 5 eq) was then added, and the reaction mixture was stirred at 60 °C for an additional 2 hours. After completion of the reaction, the mixture was concentrated under reduced pressure, and the residue was purified by prep-HPLC (column: Waters XBridge 150 * 25 mm * 5 µm; mobile phase: [water (ammonia hydroxide v/v) - ACN]; B%: 20%-50%, 9 min) to afford Compound 93 (18 mg, 7.31% yield) as a white solid.

LCMS m/z 678.4 [M+1]⁺.

¹H NMR (400 MHz, DMSO-d6) δ = 10.32 (s, 1H), 9.51 (br s, 1H), 8.64 (br s, 1H), 8.13 (br t, J = 6.8 Hz, 1H), 7.98 (br d, J = 7.6 Hz, 1H), 7.86 (br t, J = 8.4 Hz, 1H), 7.76 - 7.48 (m, 2H), 7.36 - 7.20 (m, 3H), 7.19 - 7.04 (m, 1H), 6.98 (br d, J = 7.1 Hz, 2H), 6.76 (br d, J = 9.5 Hz, 1H), 4.72 (br d, J = 2.8 Hz, 1H), 4.23 - 4.08 (m, 3H), 3.95 - 3.82 (m, 1H), 3.71 (br d, J = 10.9 Hz, 2H), 3.58 (br s, 1H), 2.78 - 2.67 (m, 2H), 2.62 (br d, J = 6.5 Hz, 2H), 2.44 (br d, J = 6.5 Hz, 2H), 2.37 - 2.27 (m, 4H), 2.11 (br s, 2H), 2.01 (br s, 3H), 1.94 (br s, 3H), 1.76 - 1.63 (m, 2H), 1.56 (br s, 1H).

### 20-4. Preparation of Compound 94, 4-((2-acetamidoethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

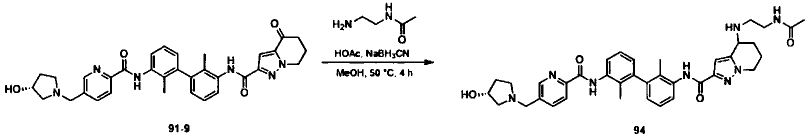

A solution of N-[3-[3-[[5-[[(3R)-3-hydroxypyrrolidin-1-yl]methyl]pyridine-2-carbonyl]amino]-2-methyl-phenyl]-2-methyl-phenyl]-4-oxo-6,7-dihydro-5H-pyrazolo[1,5-a]pyridine-2-carboxamide (200 mg, 345.63 µmol, 1 eq) and N-(2-aminoethyl)acetamide (353.00 mg, 3.46 mmol, 329.91 µL, 10 eq) in MeOH (20 mL) was treated with HOAc (207.56 mg, 3.46 mmol, 197.67 µL, 10 eq) and NaBH₃CN (43.44 mg, 691.25 µmol, 2 eq), and the reaction mixture was stirred at 60 °C for 2 hours. After completion of the reaction, the mixture was concentrated under reduced pressure, and the residue was purified by prep-HPLC (column: Waters XBridge 150 * 25 mm * 5 µm; mobile phase: [water (ammonia hydroxide v/v) - ACN]; B%: 20%-50%, 9 min) to afford Compound 94 (22 mg, 9.10% yield) as a white solid.

LCMS m/z 665.6 [M+1]⁺.

¹H NMR (400 MHz, DMSO-d6) δ = 10.32 (br s, 1H), 9.51 (br s, 1H), 8.64 (br s, 1H), 8.14 (br d, J = 7.5 Hz, 1H), 7.98 (br d, J = 7.3 Hz, 1H), 7.92 - 7.75 (m, 2H), 7.58 (br d, J = 7.3 Hz, 1H), 7.42 - 7.18 (m, 2H), 6.98 (br d, J = 7.3 Hz, 2H), 6.72 (s, 1H), 4.71 (br d, J = 3.6 Hz, 1H), 4.31 - 4.06 (m, 3H), 3.94 - 3.81 (m, 1H), 3.71 (br d, J = 11.1 Hz, 2H), 3.13 (br d, J = 5.4 Hz, 2H), 2.73 - 2.58 (m, 5H), 2.34 (br d, J = 9.6 Hz, 2H), 2.26 - 2.10 (m, 2H), 2.01 (br s, 4H), 1.94 (br s, 3H), 1.80 (s, 3H), 1.71 - 1.51 (m, 2H).

### 20-5. Preparation of Compound 95, 2-((2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)amino)acetic acid

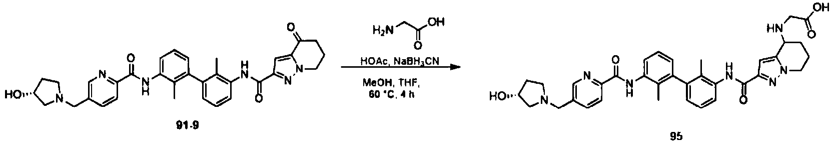

A solution of N-[3-[3-[[5-[[(3R)-3-hydroxypyrrolidin-1-yl]methyl]pyridine-2-carbonyl]amino]-2-methyl-phenyl]-2-methyl-phenyl]-4-oxo-6,7-dihydro-5H-pyrazolo[1,5-a]pyridine-2-carboxamide (200 mg, 345.63 µmol, 1 eq) and 2-aminoacetic acid (259.45 mg, 3.46 mmol, 297.04 µL, 10 eq) in MeOH (5 mL)/THF (1 mL) was treated with HOAc (415.10 mg, 6.91 mmol, 395.33 µL, 20 eq) and stirred at 60 °C for 2 hours. NaBH₃CN (108.60 mg, 1.73 mmol, 5 eq) was then added, and the reaction mixture was stirred at 60 °C for an additional 2 hours. After completion of the reaction, the mixture was concentrated under reduced pressure, and the residue was purified by prep-HPLC (column: Waters XBridge 150 * 25 mm * 5 µm; mobile phase: [water (NH₄HCO₃) - ACN]; B%: 13%-43%, 9 min) to afford Compound 95 (31 mg, 13.3% yield) as a white solid.

LCMS m/z 638.4 [M+1]⁺.

¹H NMR (400 MHz, DMSO-d6) δ = 10.33 (s, 1H), 9.56 (s, 1H), 8.64 (s, 1H), 8.14 (d, J = 7.9 Hz, 1H), 7.98 (dd, J = 1.7, 8.1 Hz, 1H), 7.86 (d, J = 8.0 Hz, 1H), 7.56 (br d, J = 8.0 Hz, 1H), 7.30 (td, J = 7.8, 15.4 Hz, 2H), 6.98 (br d, J = 7.5 Hz, 2H), 6.77 (s, 1H), 4.25 - 4.05 (m, 4H), 3.71 (br d, J = 10.9 Hz, 4H), 2.78 - 2.58 (m, 3H), 2.46 - 2.30 (m, 3H), 2.25 - 2.14 (m, 1H), 2.01 (s, 3H), 1.94 (s, 3H), 1.84 - 1.69 (m, 1H), 1.56 (br dd, J = 4.6, 8.3 Hz, 1H), 1.05 (t, J = 7.0 Hz, 1H).

### 20-6. Preparation of Compound 96, (3R)-1-(2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid

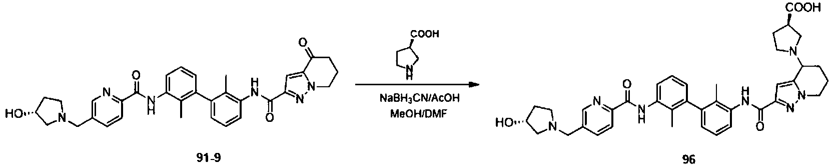

A solution of N-[3-[3-[[5-[[(3R)-3-hydroxypyrrolidin-1-yl]methyl]pyridine-2-carbonyl]amino]-2-methyl-phenyl]-2-methyl-phenyl]-4-oxo-6,7-dihydro-5H-pyrazolo[1,5-a]pyridine-2-carboxamide (200 mg, 345.63 µmol, 1 eq) and (3R)-pyrrolidine-3-carboxylic acid (397.92 mg, 3.46 mmol, 10 eq) in DMF (10 mL)/MeOH (10 mL) was treated with NaBH₃CN (217.20 mg, 3.46 mmol, 10 eq) and AcOH (207.56 mg, 3.46 mmol, 197.86 µL, 10 eq), and the reaction mixture was stirred at 60 °C for 12 hours. After completion of the reaction, the mixture was concentrated under reduced pressure, and the residue was purified by reversed-phase HPLC (column: Waters XBridge 150 * 25 mm * 5 um; mobile phase: [water (NH₄HCO₃) - ACN]; gradient: 14%-44% B over 9 min) to afford Compound 96 (50 mg, 21.1% yield) as a white solid.

LCMS m/z 678.6 [M+1]⁺.

¹H NMR (400 MHz, DMSO-d6) δ = 10.32 (s, 1H), 9.54 (br d, J = 2.8 Hz, 1H), 8.65 (s, 1H), 8.14 (d, J = 7.9 Hz, 1H), 7.98 (dd, J = 2.1, 7.9 Hz, 1H), 7.87 (d, J = 7.8 Hz, 1H), 7.57 (dd, J = 3.2, 7.4 Hz, 1H), 7.34 - 7.25 (m, 2H), 6.98 (d, J = 7.1 Hz, 2H), 6.66 (d, J = 4.3 Hz, 1H), 4.24 - 4.12 (m, 3H), 3.81 - 3.66 (m, 3H), 3.32 (br s, 6H), 2.94 - 2.84 (m, 2H), 2.74 - 2.67 (m, 3H), 2.63 - 2.58 (m, 2H), 2.48 - 2.39 (m, 2H), 2.34 (br dd, J = 3.8, 9.6 Hz, 1H), 2.25 (br s, 1H), 2.01 (s, 3H), 1.94 (s, 4H), 1.61 - 1.52 (m, 1H).

### 20-7. Preparation of Compound 97, N-(2,2'-dichloro-3'-((3-(((S)-3-hydroxypyrrolidin-1-yl)methyl)-1,7-naphthyridin-8-yl)amino)-[1,1'-biphenyl]-3-yl)-4-(4-hydroxypiperidin-1-yl)-4,5,6,7-tetrahydropyrazolo[1,5-a]pyridine-2-carboxamide

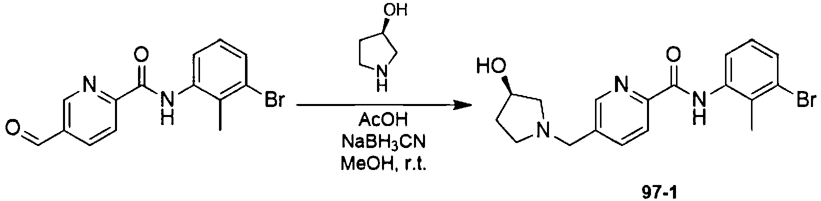

A solution of N-(3-bromo-2-methyl-phenyl)-5-formyl-pyridine-2-carboxamide (1 g, 3.13 mmol, 1 eq) in MeOH (20 mL) was treated with (3R)-pyrrolidin-3-ol (2.73 g, 31.33 mmol, 2.60 mL, 10 eq), NaBH₃CN (984.48 mg, 15.67 mmol, 5 eq), and AcOH (940.77 mg, 15.67 mmol, 896.83 µL, 5 eq), and the mixture was stirred at room temperature for 12 hours. After completion of the reaction, the mixture was concentrated under reduced pressure and purified by reversed-phase HPLC (column: Waters XBridge 150 * 25 mm * 5 um; mobile phase: [water (NH₄HCO₃) - ACN]; gradient: 14%-44% B over 9 min) to afford Compound 97-1 (740 mg, 54.4% yield) as a white solid.

LCMS m/z 389.07 [M+1]⁺.

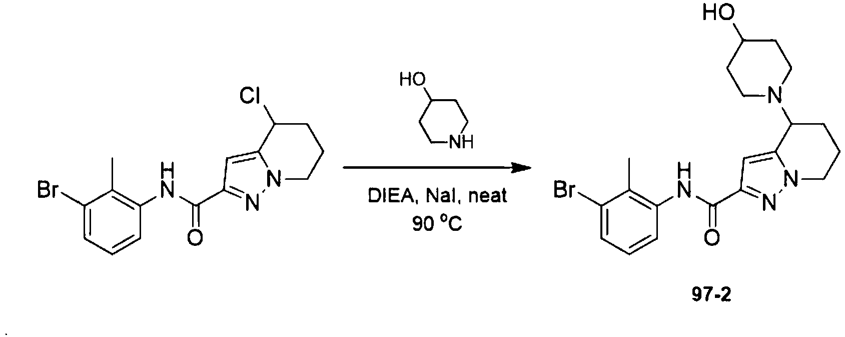

A mixture of N-(3-bromo-2-methylphenyl)-4-chloro-4,5,6,7-tetrahydropyrazolo[1,5-a]pyridine-2-carboxamide (2 g, 5.43 mmol, 1 eq), piperidin-4-ol (548.73 mg, 5.43 mmol, 1 eq), DIPEA (2.10 g, 16.28 mmol, 2.83 mL, 3 eq), and Nal (243.95 mg, 1.63 mmol, 0.3 eq) was stirred at 90 °C for 12 hours. After completion of the reaction, the mixture was filtered and the filtrate was concentrated under reduced pressure. The residue was purified by reversed-phase HPLC (column: Phenomenex Luna C18 (250 * 70 mm, 10 um); mobile phase: [water (NH₄HCO₃) - ACN]; gradient: 30%-60% B over 20 min) to afford Compound 97-2 (1.66 g, 69.9% yield) as a yellow solid.

LCMS m/z 432.12 [M+1]⁺.

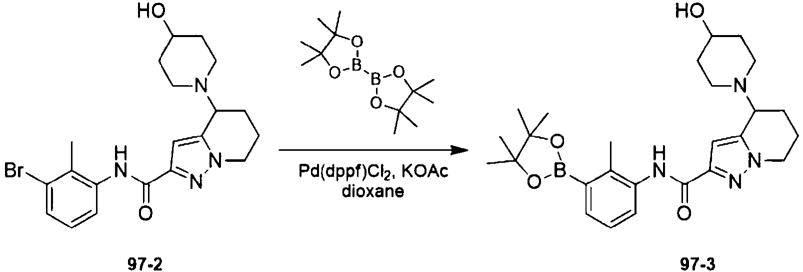

A mixture of N-(3-bromo-2-methyl-phenyl)-4-(4-hydroxy-1-piperidyl)-4,5,6,7-tetrahydropyrazolo[1,5-a]pyridine-2-carboxamide (200 mg, 461.53 µmol, 1 eq), 4,4,5,5-tetramethyl-2-(4,4,5,5-tetramethyl-1,3,2-dioxaborolan-2-yl)-1,3,2-dioxaborolane (351.60 mg, 1.38 mmol, 3 eq), AcOK (135.88 mg, 1.38 mmol, 3 eq), and Pd(dppf)Cl₂ (33.77 mg, 46.15 µmol, 0.1 eq) in dioxane (4 mL) was stirred at 90 °C for 12 hours under a nitrogen atmosphere. After completion of the reaction, the mixture was concentrated under reduced pressure, and the residue was purified by column chromatography (SiO₂, Ethyl acetate:Methanol = 10/1) to afford Compound 97-3 (120 mg, 48.7% yield) as a black liquid.

LCMS m/z 480.29 [M+1]⁺.

¹H NMR (400 MHz, CHLOROFORM-d) δ ppm 1.36 (s, 12 H) 1.49 - 1.66 (m, 4 H) 1.71 - 2.01 (m, 5 H) 2.10 (br s, 1 H) 2.25 (br s, 1 H) 2.40 (br s, 1 H) 2.56 (s, 3 H) 2.89 (s, 2 H) 3.95 (br s, 1 H) 4.06 (br s, 1 H) 4.23 (br s, 1 H) 6.86 (br s, 1 H) 7.19 - 7.25 (m, 1 H) 7.59 (d, J=7.38 Hz, 1 H) 8.17 (br d, J=8.00 Hz, 1 H) 8.67 (br s, 1 H).

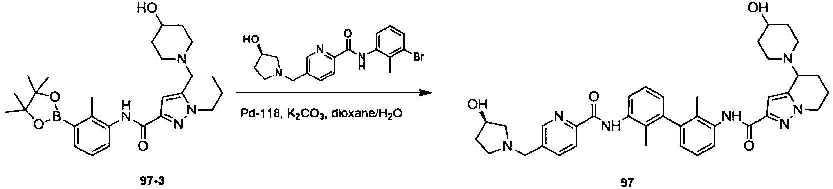

A mixture of 4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (80 mg, 166.53 µmol, 1 eq), N-(3-bromo-2-methyl-phenyl)-5-[[(3R)-3-hydroxypyrrolidin-1-yl]methyl]pyridine-2-carboxamide (64.99 mg, 166.53 µmol, 1 eq), ditert-butyl(cyclopentyl)phosphane;dichloropalladium iron (10.85 mg, 16.65 µmol, 0.1 eq), and K₂CO₃ (69.05 mg, 499.58 µmol, 3 eq) in dioxane (2.5 mL)/H₂O (0.5 mL) was stirred at 90 °C for 2 hours under a nitrogen atmosphere. After completion of the reaction, the mixture was filtered and the filtrate was concentrated under reduced pressure. The residue was purified by reversed-phase HPLC (column: Waters XBridge 150 * 25 mm * 5 um; mobile phase: [water (NH₄HCO₃) - ACN]; gradient: 28%-58% B over 9 min) to afford Compound 97 (23 mg, 20.8% yield) as a yellow solid.

LCMS m/z 663.35 [M+1]⁺.

¹H NMR (400 MHz, DMSO-d6) δ ppm 1.31 - 1.48 (m, 2 H) 1.52 - 1.61 (m, 1 H) 1.65 - 1.80 (m, 3 H) 1.82 - 1.91 (m, 1 H) 1.95 (s, 3 H) 2.01 - 2.03 (m, 3 H) 2.11 - 2.20 (m, 1 H) 2.11 - 2.20 (m, 1 H) 2.28 (td, J=9.44, 1.25 Hz, 1 H) 2.35 (dd, J=9.57, 3.69 Hz, 1 H) 2.33 - 2.37 (m, 1 H) 2.38 - 2.47 (m, 2 H) 2.38 - 2.47 (m, 1 H) 2.60 - 2.76 (m, 4 H) 3.42 - 3.52 (m, 1 H) 3.66 - 3.78 (m, 2 H) 3.93 - 3.99 (m, 1 H) 4.00 - 4.09 (m, 1 H) 4.20 (br dd, J=8.19, 3.69 Hz, 2 H) 4.55 (d, J=4.13 Hz, 1 H) 4.72 (d, J=4.50 Hz, 1 H) 6.62 (s, 1 H) 6.99 (d, J=7.63 Hz, 2 H) 7.31 (dt, J=16.13, 7.82 Hz, 2 H) 7.55 - 7.60 (m, 1 H) 7.87 (d, J=8.25 Hz, 1 H) 7.97 - 8.01 (m, 1 H) 8.15 (d, J=8.00 Hz, 1 H) 8.65 (s, 1 H) 9.43 - 9.59 (m, 1 H) 10.31 - 10.35 (m, 1 H)

### 20-8. Preparation of Compound 98, 1-(2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

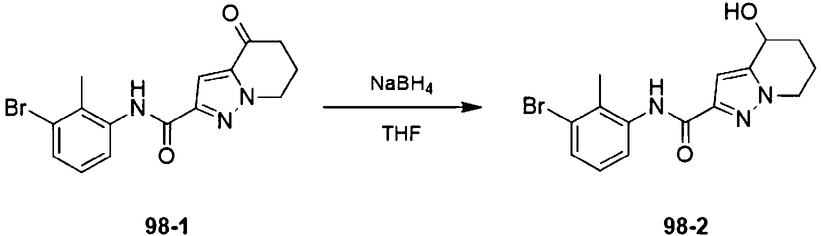

A solution of N-(3-bromo-2-methyl-phenyl)-4-oxo-6,7-dihydro-5H-pyrazolo[1,5-a]pyridine-2-carboxamide (10 g, 28.72 mmol, 1 eq) in THF (20 mL) was treated with NaBH₄ (3.26 g, 86.16 mmol, 3 eq), and the reaction mixture was stirred at room temperature for 16 hours. After completion of the reaction, aq. sat'd NH₄Cl solution (200 mL) was added, and the mixture was extracted with EtOAc (200 mL * 2). The organic layer was washed with brine (200 mL), dried over Na₂SO₄, and concentrated under reduced pressure to afford Compound 98-2 (9 g, 78.7% yield), which was used in the next reaction without further purification.

LCMS m/z 352.1 [M+3]⁺.

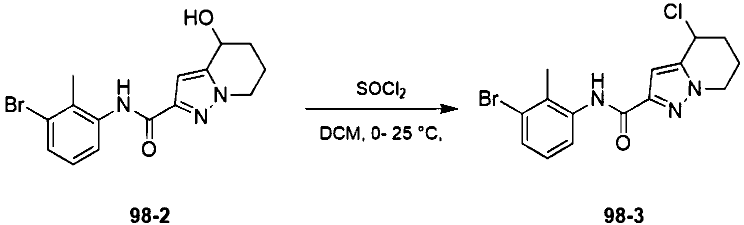

A solution of N-(3-bromo-2-methyl-phenyl)-4-hydroxy-4,5,6,7-tetrahydropyrazolo[1,5-a]pyridine-2-carboxamide (4.6 g, 13.13 mmol, 1 eq) in DCM (30 mL) was treated with SOCl₂ (7.81 g, 65.67 mmol, 4.77 mL, 5 eq), and the reaction mixture was stirred at room temperature for 12 hours. After completion of the reaction, aq. sat'd NH₄Cl solution (200 mL) was added, and the mixture was extracted with EtOAc (200 mL * 2). The organic layer was washed with brine (200 mL), dried over Na₂SO₄, and concentrated under reduced pressure to afford Compound 98-3 (9 g, 78.7% yield), which was used in the next reaction without further purification.

LCMS m/z 369.7 [M+3]⁺.

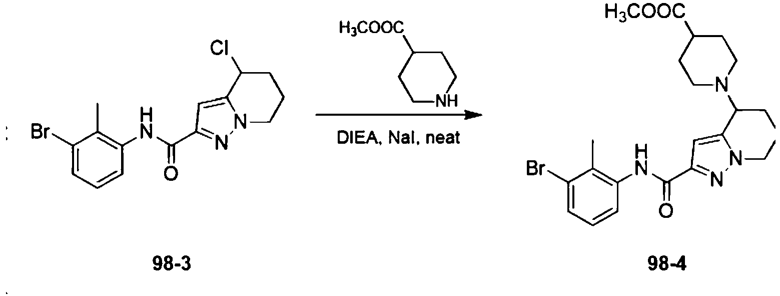

A mixture of N-(3-bromo-2-methylphenyl)-4-chloro-4,5,6,7-tetrahydropyrazolo[1,5-a]pyridine-2-carboxamide (2 g, 5.43 mmol, 1 eq), methyl piperidine-4-carboxylate (2.33 g, 16.28 mmol, 3 eq), DIPEA (2.10 g, 16.28 mmol, 2.83 mL, 3 eq), and Nal (243.95 mg, 1.63 mmol, 0.3 eq) was stirred at 40 °C for 12 hours. After completion of the reaction, the mixture was concentrated under reduced pressure, and the residue was purified by reversed-phase HPLC (column: Phenomenex Luna C18 (250 * 70 mm, 10 um); mobile phase: [water (NH₄HCO₃) - ACN]; gradient: 50%-80% B over 21 min) to afford Compound 98-4 (1.73 g, 66.4% yield) as a yellow solid.

LCMS m/z 477.2 [M+3]⁺.

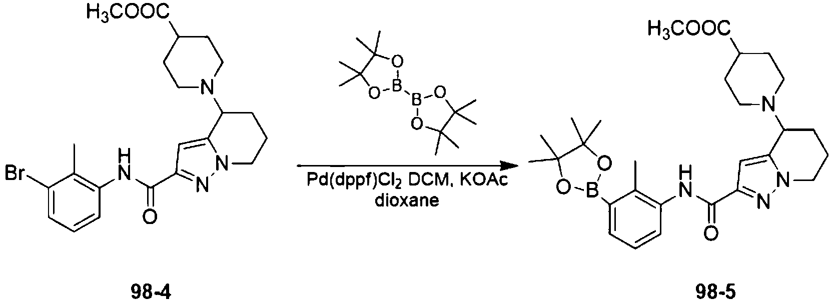

A mixture of methyl 1-[2-[(3-bromo-2-methyl-phenyl)carbamoyl]-4,5,6,7-tetrahydropyrazolo[1,5-a]pyridin-4-yl]piperidine-4-carboxylate (200 mg, 420.72 µmol, 1 eq), 4,4,5,5-tetramethyl-2-(4,4,5,5-tetramethyl-1,3,2-dioxaborolan-2-yl)-1,3,2-dioxaborolane (320.51 mg, 1.26 mmol, 3 eq), AcOK (123.87 mg, 1.26 mmol, 3 eq), and Pd(dppf)Cl₂ (30.78 mg, 42.07 µmol, 0.1 eq) in dioxane (4 mL) was stirred at 90 °C for 12 hours under a nitrogen atmosphere. After completion of the reaction, the mixture was concentrated under reduced pressure, and the residue was purified by column chromatography (SiO₂, Ethyl acetate:Methanol = 10/1) to afford Compound 98-5 (215 mg, 89.99% yield) as a black liquid.

LCMS m/z 522.3 [M+1]⁺.

¹H NMR (400 MHz, CHLOROFORM-d) δ ppm 1.36 (s, 12 H) 1.93 (br s, 7 H) 2.29 - 2.42 (m, 2 H) 2.56 (s, 3 H) 3.71 (d, J=5.63 Hz, 4 H) 3.92 (br s, 1 H) 4.23 (br s, 2 H) 6.85 (br s, 1 H) 7.21 - 7.25 (m, 1 H) 7.59 (d, J=7.38 Hz, 1 H) 8.11 - 8.21 (m, 1 H) 8.62 - 8.71 (m, 1 H).

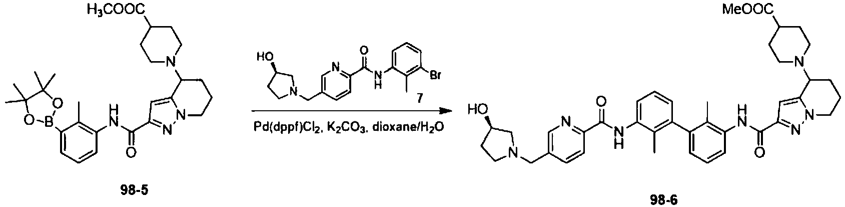

A mixture of methyl 1-[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (100 mg, 191.41 µmol, 1.25 eq), N-(3-bromo-2-methyl-phenyl)-5-[[(3R)-3-hydroxypyrrolidin-1-yl]methyl]pyridine-2-carboxamide (59.76 mg, 153.13 µmol, 1 eq), ditert-butyl(cyclopentyl)phosphane;dichloropalladium iron (9.98 mg, 15.31 µmol, 0.1 eq), and K₂CO₃ (63.49 mg, 459.38 µmol, 3 eq) in H₂O (3 mL)/dioxane (15 mL) was stirred at 90 °C for 12 hours. After completion of the reaction, the mixture was concentrated under reduced pressure and purified by reversed-phase HPLC (column: Waters XBridge Prep OBD C18 150 * 40 mm * 10 um; mobile phase: [water (NH₄HCO₃) - ACN]; B: 20%, isocratic elution mode) to afford Compound 98-6 (100 mg, 74.0% yield) as a black solid.

LCMS m/z 706.5 [M+1]⁺.

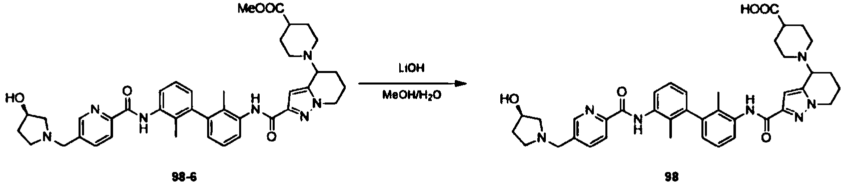

A mixture of methyl 1-[2-[[3-[3-[[5-[[(3R)-3-hydroxypyrrolidin-1-yl]methyl]pyridine-2-carbonyl]amino]-2-methyl-phenyl]-2-methyl-phenyl]carbamoyl]-4,5,6,7-tetrahydropyrazolo[1,5-a]pyridin-4-yl]piperidine-4-carboxylate (100 mg, 141.67 µmol, 1 eq) and LiOH (29.72 mg, 708.37 µmol, 5 eq) in H₂O (4 mL)/MeOH (20 mL) was stirred at room temperature for 12 hours. After completion of the reaction, the mixture was concentrated under reduced pressure, and the residue was purified by reversed-phase HPLC (column: Phenomenex Luna C18 150 * 25 mm * 10 um; mobile phase: [water (NH₄HCO₃) - ACN]; gradient: 18%-48% B over 10 min) to afford Compound 98 (60 mg, 60.60% yield) as a yellow solid.

LCMS m/z 692.6 [M+1]⁺.

¹H NMR (400 MHz, DMSO-d6) δ ppm 1.44 - 1.62 (m, 3 H) 1.63 - 1.74 (m, 1 H) 1.77 - 1.86 (m, 2 H) 1.94 (s, 3 H) 2.01 (s, 3 H) 2.10 - 2.30 (m, 3 H) 2.34 (dd, J=9.66, 3.42 Hz, 1 H) 2.39 - 2.48 (m, 2 H) 2.57 - 2.65 (m, 2 H) 2.70 (br dd, J=9.47, 6.17 Hz, 3 H) 2.75 - 2.85 (m, 1 H) 3.62 - 3.78 (m, 3 H) 3.96 (br dd, J=9.54, 5.13 Hz, 1 H) 4.02 - 4.09 (m, 1 H) 4.19 (br d, J=7.21 Hz, 2 H) 4.66 - 4.78 (m, 1 H) 6.62 (s, 1 H) 6.98 (d, J=7.34 Hz, 2 H) 7.29 (dt, J=15.86, 7.78 Hz, 2 H) 7.56 (t, J=7.03 Hz, 1 H) 7.86 (d, J=8.19 Hz, 1 H) 7.98 (dd, J=8.01, 1.89 Hz, 1 H) 8.14 (d, J=8.07 Hz, 1 H) 8.64 (d, J=1.34 Hz, 1 H) 9.53 (d, J=5.62 Hz, 1 H) 10.33 (s, 1 H).

### 20-9. Preparation of Compound 99, 2-((2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid

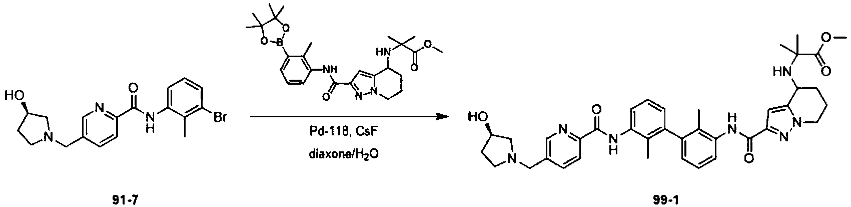

A mixture of N-(3-bromo-2-methyl-phenyl)-5-[[(3R)-3-hydroxypyrrolidin-1-yl]methyl]pyridine-2-carboxamide (130 mg, 333.10 µmol, 1 eq), methyl 2-methyl-2-[[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]propanoate (181.89 mg, 366.41 µmol, 1.1 eq), CsF (101.20 mg, 666.20 µmol, 24.59 µL, 2 eq), and ditert-butyl(cyclopentyl)phosphane;dichloropalladium iron (21.71 mg, 33.31 µmol, 0.1 eq) in dioxane (5 mL)/H₂O (1 mL) was stirred at 80 °C for 4 hours under a nitrogen atmosphere. After completion of the reaction, the mixture was filtered and the filtrate was concentrated under reduced pressure. The residue was purified by prep-TLC (SiO₂, Ethyl acetate:Methanol = 10:1) to afford Compound 99-1 (170 mg, 49.5% yield) as a brown solid.

LCMS m/z 680.4 [M+1]⁺.

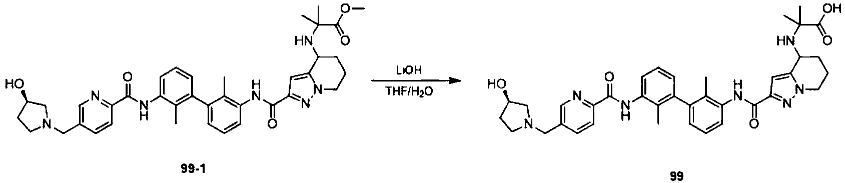

A solution of methyl 2-[[2-[[3-[3-[[5-[[(3R)-3-hydroxypyrrolidin-1-yl]methyl]pyridine-2-carbonyl]amino]-2-methyl-phenyl]-2-methyl-phenyl]carbamoyl]-4,5,6,7-tetrahydropyrazolo[1,5-a]pyridin-4-yl]amino]-2-methyl-propanoate (170 mg, 250.07 µmol, 1 eq) in THF (30 mL)/H₂O (15 mL) was treated with LiOH (17.94 mg, 750.21 µmol, 3 eq), and the resulting mixture was stirred at room temperature for 12 hours. After completion of the reaction, the mixture was concentrated under reduced pressure, and the residue was purified by reversed-phase HPLC ((0.1% NH₃·H₂O condition); column: Phenomenex Luna C18, 150 * 25 mm * 10 µm; mobile phase: [water (NH₄HCO₃)-ACN]; gradient: 14%-44% B over 10 min) to afford Compound 99 (75 mg, 44.6% yield) as a white solid.

LCMS m/z 666.7 [M+1]⁺.

¹H NMR (400 MHz, DMSO-d6) δ ppm 1.29 (d, J=3.38 Hz, 6 H) 1.52 - 1.60 (m, 1 H) 1.61 - 1.68 (m, 1 H) 1.94 (s, 3 H) 2.01 (s, 3 H) 2.14 (br dd, J=13.38, 5.75 Hz, 1 H) 2.35 (dd, J=9.63, 3.50 Hz, 1 H) 2.40 - 2.48 (m, 2 H) 2.59 - 2.65 (m, 1 H) 2.70 (dd, J=9.57, 6.19 Hz, 1 H) 3.66 - 3.78 (m, 4 H) 3.93 (br dd, J=7.44, 5.19 Hz, 1 H) 4.08 - 4.14 (m, 2 H) 4.17 - 4.24 (m, 1 H) 4.63 - 4.82 (m, 1 H) 6.74 (s, 1 H) 6.97 (d, J=7.50 Hz, 2 H) 7.29 (dt, J=15.79, 7.80 Hz, 2 H) 7.57 (d, J=7.75 Hz, 1 H) 7.87 (d, J=7.75 Hz, 1 H) 7.98 (dd, J=8.00, 2.00 Hz, 1 H) 8.14 (d, J=8.13 Hz, 1 H) 8.64 (d, J=1.38 Hz, 1 H) 9.51 (s, 1 H) 10.32 (s, 1 H)

### [Preparation Example 21]

### Preparation of Compounds 100 to 108

### 21-1. Preparation of Compound 100, N-(2,2'-dichloro-3'-(5-(((R)-3-hydroxypyrrolidin-1-yl)methyl)picolinamido)-[1,1'-biphenyl]-3-yl)-4-((2-hydroxyethyl)amino)-4,5,6,7-tetrahydropyrazolo[1,5-a]pyridine-2-carboxamide

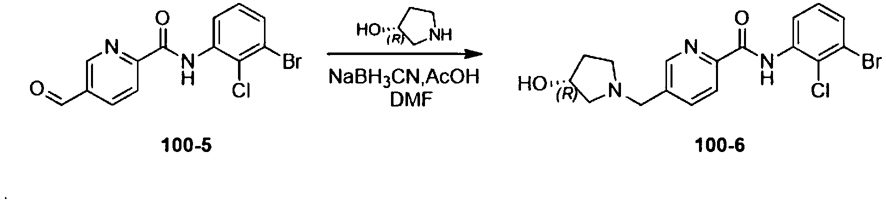

A solution of compound 100-5 (2.0 g, 5.89 mmol, 1.0 eq), (3R)-pyrrolidin-3-ol (800 mg, 9.18 mmol, 1.56 eq), and acetic acid (1.05 g, 17.49 mmol, 1.0 mL, 2.97 eq) in DMF (30 mL) was stirred at 60 °C for 30 minutes. Sodium cyanoborohydride (NaBH₃CN, 1.5 g, 23.87 mmol, 4.05 eq) was then added, and the reaction mixture was further stirred at 60 °C for 30 minutes. After completion of the reaction, the mixture was diluted with water (50 mL) and adjusted to pH 8 using a aq. saturated sodium bicarbonate solution. The aqueous phase was extracted with ethyl acetate (50 mL * 2). The combined organic layers were dried over anhydrous sodium sulfate, filtered, and concentrated under reduced pressure. The residue was purified by flash silica gel chromatography (eluent: ethyl acetate/petroleum ether, 0-100%) to afford Compound 100-6 (1.2 g, 47.3% yield) as a yellow solid. The obtained compound was used in the next step without further purification.

LCMS m/z 411.7 [M+1]⁺.

¹H NMR ( 400MHz, DMSO-d6) δ 10.72 (s, 1H), 8.68 (d, J =1.4 Hz, 1H), 8.37 (dd, J =1.3, 8.2 Hz, 1H), 8.17 (d, J =8.0 Hz, 1H), 8.02 (dd, J =2.0, 8.0 Hz, 1H), 7.61 (dd, J =1.4, 8.0 Hz, 1H), 7.38 (t, J =8.2 Hz, 1H), 4.73 (d, J =4.5 Hz, 1H), 4.26 - 4.17 (m, 1H), 3.82 - 3.65 (m, 2H), 2.75 - 2.67 (m, 1H), 2.63 (q, J =7.8 Hz, 1H), 2.45 (dt, J =5.7, 8.3 Hz, 1H), 2.35 (dd, J =3.6, 9.6 Hz, 1H), 2.08 - 1.95 (m, 1H), 1.62 - 1.51 (m, 1H), 1.62 - 1.51 (m, 1H).

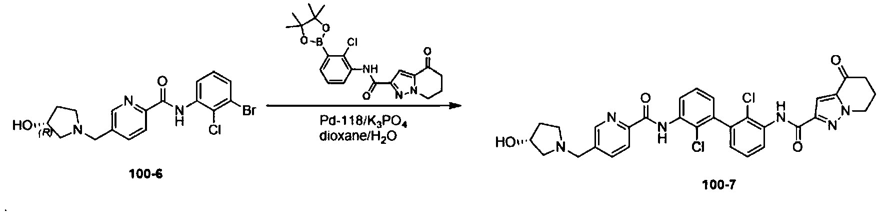

A mixture of compound 100-6 (500 mg, 1.22 mmol, 1.0 eq) and N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (750 mg, 1.80 mmol, 1.48 eq) in dioxane/H₂O (10 mL/2 mL) was prepared. To the reaction mixture were added K₃PO₄ (750 mg, 3.53 mmol, 2.90 eq) and ditert-butyl(cyclopentyl)phosphane·dichloropalladium·iron (84.29 mg, 129.34 µmol, 0.106 eq), and the resulting mixture was stirred at 70 °C for 5 hours under a nitrogen atmosphere. After completion of the reaction, the mixture was filtered, and the filtrate was diluted with water (50 mL) and extracted with ethyl acetate (50 mL * 2). The combined organic layers were washed with brine (50 mL x 3), dried over anhydrous sodium sulfate, filtered, and concentrated under reduced pressure. The residue was purified by flash silica gel chromatography (eluent: ethyl acetate/petroleum ether, 0-100%, followed by MeOH/ethyl acetate, 0-30%) to afford Compound 100-7 (600 mg, 72.2% yield) as a yellow solid.

LCMS m/z 620.9[M+1]⁺.

¹H NMR (400MHz, DMSO-d6) δ 10.73 (s, 1H), 9.83 (s, 1H), 8.68 (s, 1H), 8.53 (dd, J =1.3, 8.3 Hz, 1H), 8.20 (d, J =8.0 Hz, 1H), 8.11 (dd, J =1.2, 8.1 Hz, 1H), 8.03 (dd, J =1.7, 8.0 Hz, 1H), 7.53 (td, J =7.9, 11.1 Hz, 2H), 7.30 (s, 1H), 7.27 - 7.15 (m, 2H), 4.74 (d, J =4.5 Hz, 1H), 4.48 (t, J =5.8 Hz, 2H), 4.27 - 4.17 (m, 1H), 3.80 - 3.62 (m, 2H), 2.76 - 2.68 (m, 3H), 2.62 (q, J =7.9 Hz, 1H), 2.48 - 2.41 (m, 1H), 2.39 - 2.31 (m, 3H), 2.07 - 1.96 (m, 1H), 1.63 - 1.48 (m, 1H).

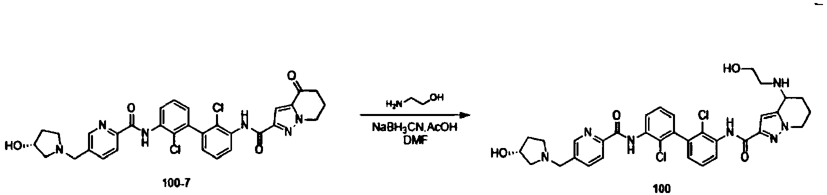

A solution of compound 100-7 (190 mg, 306.70 µmol, 1 eq), 2-aminoethanol (150 mg, 2.46 mmol, 148.51 µL, 8.01 eq), and AcOH (210.00 mg, 3.50 mmol, 200 µL, 11.40 eq) in DMF (3 mL) was stirred at 60°C for 30 minutes, after which NaBH₃CN (120 mg, 1.91 mmol, 6.23 eq) was added, and the mixture was stirred at 60°C for 18 hours. The mixture was diluted with H₂O (20 mL), adjusted to pH 8 with aq. saturated NaHCO₃ solution, and extracted with EA (50 mL * 2). The organic layer was dried over Na₂SO₄, concentrated under reduced pressure, and the residue was purified by prep-HPLC (column: Phenomenex C18 80 * 40mm * 3um; mobile phase: [water (NH₃H₂O + NH₄HCO₃)-ACN]; B%: 34%-64%, 8 min) to afford Compound 100 (55.03 mg, 26.7% yield) as a white solid.

LCMS m/z 664.4 [M+1]⁺.

¹H NMR (400MHz, DMSO-d6) δ 10.73 (s, 1H), 9.83 (s, 1H), 8.68 (s, 1H), 8.53 (dd, J =1.3, 8.3 Hz, 1H), 8.20 (d, J =8.0 Hz, 1H), 8.11 (dd, J =1.2, 8.1 Hz, 1H), 8.03 (dd, J =1.7, 8.0 Hz, 1H), 7.53 (td, J =7.9, 11.1 Hz, 2H), 7.30 (s, 1H), 7.27 - 7.15 (m, 2H), 4.74 (d, J =4.5 Hz, 1H), 4.48 (t, J =5.8 Hz, 2H), 4.27 - 4.17 (m, 1H), 3.80 - 3.62 (m, 2H), 2.76 - 2.68 (m, 3H), 2.62 (q, J =7.9 Hz, 1H), 2.48 - 2.41 (m, 1H), 2.39 - 2.31 (m, 3H), 2.07 - 1.96 (m, 1H), 1.63 - 1.48 (m, 1H).

### 21-2. Preparation of Compound 101, N-(2,2'-dichloro-3'-(5-(((R)-3-hydroxypyrrolidin-1-yl)methyl)picolinamido)-[1,1'-biphenyl]-3-yl)-4-((R)-3-hydroxypyrrolidin-1-yl)-4,5,6,7-tetrahydropyrazolo[1,5-a]pyridine-2-carboxamide

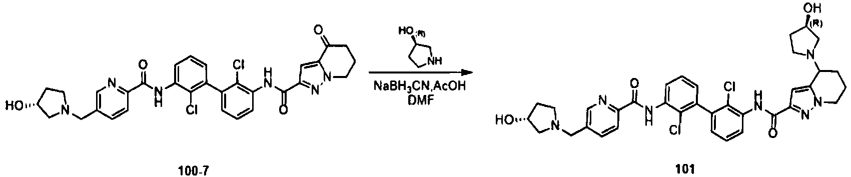

A solution of compound 100-7 (150 mg, 242.13 µmol, 1 eq), (3R)-pyrrolidin-3-ol (150 mg, 1.72 mmol, 142.86 µL, 7.11 eq), and AcOH (157.50 mg, 2.62 mmol, 150 µL, 10.83 eq) in DMF (3 mL) was stirred at 60°C for 30 minutes, after which NaBH₃CN (100 mg, 1.59 mmol, 6.57 eq) was added, and the mixture was stirred at 60°C for 18 hours. The mixture was diluted with H₂O (20 mL), adjusted to pH 8 with aq. saturated NaHCO₃ solution, and extracted with EA (50 mL * 2). The organic layer was dried over Na₂SO₄, concentrated under reduced pressure, and the residue was purified by prep-HPLC (column: Phenomenex C18 80 * 40mm * 3um; mobile phase: [water (NH₃H₂O+NH₄HCO₃)-ACN]; B%: 40%-70%, 8 min) to afford Compound 101 (91.19 mg, 54.4% yield) as a white solid.

LCMS m/z 690.4 [M+1]⁺.

¹H NMR (400MHz, DMSO-d6) δ 10.72 (s, 1H), 9.56 (s, 1H), 8.68 (s, 1H), 8.52 (dd, J =1.3, 8.3 Hz, 1H), 8.32 - 8.25 (m, 1H), 8.20 (d, J =8.0 Hz, 1H), 8.03 (dd, J =1.9, 8.2 Hz, 1H), 7.52 (td, J =8.0, 15.9 Hz, 2H), 7.19 (br d, J =7.5 Hz, 2H), 6.74 (d, J =8.8 Hz, 1H), 4.76 - 4.67 (m, 2H), 4.25 - 4.13 (m, 4H), 3.81 (br d, J =4.3 Hz, 1H), 3.78 - 3.66 (m, 2H), 2.94 - 2.67 (m, 3H), 2.66 - 2.52 (m, 2H), 2.49 - 2.30 (m, 3H), 2.23 (br s, 1H), 2.06 - 1.83 (m, 5H), 1.62 - 1.50 (m, 2H)

### 21-3. Preparation of Compound 102, N-(2,2'-dichloro-3'-(5-(((R)-3-hydroxypyrrolidin-1-yl)methyl)picolinamido)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

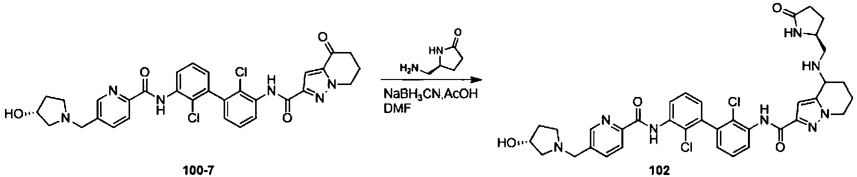

A solution of compound 100-7 (150 mg, 242.13 µmol, 1 eq), (5S)-5-(aminomethyl)pyrrolidin-2-one (200 mg, 1.33 mmol, 5.48 eq, HCl), and AcOH (157.50 mg, 2.62 mmol, 150.00 µL, 10.83 eq) in DMF (3 mL) was stirred at 60 °C for 30 minutes, after which NaBH₃CN (100 mg, 1.59 mmol, 6.57 eq) was added, and the mixture was stirred at 60 °C for 18 hours. The mixture was diluted with H₂O (20 mL), adjusted to pH 8 with aq. saturated NaHCO₃ solution, and extracted with EA (50 mL * 2). The organic layer was dried over Na₂SO₄, concentrated under reduced pressure, and the residue was purified by prep-HPLC (column: Phenomenex C18 80 * 40mm * 3um; mobile phase: [water (NH₃H₂O+NH₄HCO₃)-ACN]; B%: 37%-67%, 8 min) to afford Compound 102 (72.37 mg, 41.65% yield) as a white solid.

LCMS m/z 917.4 [M+1]⁺.

¹H NMR (400MHz, DMSO-d6) δ 10.72 (s, 1H), 9.55 (s, 1H), 8.68 (s, 1H), 8.56 - 8.48 (m, 1H), 8.30 (d, J =7.8 Hz, 1H), 8.20 (d, J =8.0 Hz, 1H), 8.03 (d, J =7.8 Hz, 1H), 7.70 (d, J =18.3 Hz, 1H), 7.52 (td, J =7.8, 15.7 Hz, 2H), 7.19 (br d, J =7.8 Hz, 2H), 6.84 (d, J =11.8 Hz, 1H), 4.73 (d, J =4.8 Hz, 1H), 4.26 - 4.12 (m, 3H), 3.88 (br s, 1H), 3.78 - 3.66 (m, 2H), 3.58 (br s, 1H), 2.75 - 2.55 (m, 5H), 2.49 - 2.39 (m, 1H), 2.35 (dd, J =3.5, 9.8 Hz, 1H), 2.21 - 2.07 (m, 4H), 2.02 (td, J =7.0, 13.7 Hz, 2H), 1.97 - 1.86 (m, 1H), 1.78 - 1.63 (m, 2H), 1.57 (br s, 1H).

### 21-4. Preparation of Compound 103, 4-((2-acetamidoethyl)amino)-N-(2,2'-dichloro-3'-(5-(((R)-3-hydroxypyrrolidin-1-yl)methyl)picolinamido)-[1,1'-biphenyl]-3-yl)-4,5,6,7-tetrahydropyrazolo[1,5-a]pyridine-2-carboxamide

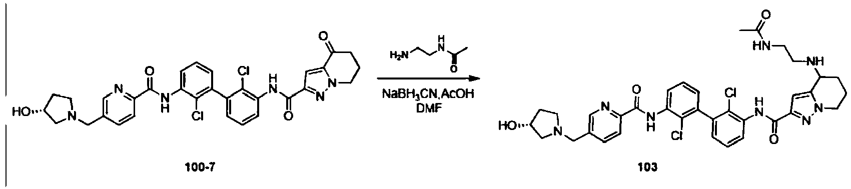

A solution of compound 100-7 (150 mg, 242.13 µmol, 1 eq), N-(2-aminoethyl)acetamide (150 mg, 1.47 mmol, 140.19 µL, 6.07 eq), and AcOH (157.50 mg, 2.62 mmol, 150.00 µL, 10.83 eq) in DMF (3 mL) was stirred at 60 °C for 30 minutes, after which NaBH₃CN (100 mg, 1.59 mmol, 6.57 eq) was added, and the mixture was stirred at 60 °C for 18 hours. The mixture was diluted with H₂O (20 mL), adjusted to pH 8 with aq. saturated NaHCO₃ solution, and extracted with EA (50 mL * 2). The organic layer was dried over Na₂SO₄, concentrated under reduced pressure, and the residue was purified by prep-HPLC (column: Phenomenex C18 80 * 40mm * 3um; mobile phase: [water (NH₃H₂O + NH₄HCO₃)-ACN]; B%: 36%-66%, 8 min) to afford Compound 103 (91.47 mg, 53.5% yield) as a white solid.

LCMS m/z 707.3 [M+1]⁺.

¹H NMR (400MHz, DMSO-d6) δ 10.72 (s, 1H), 9.54 (s, 1H), 8.67 (s, 1H), 8.52 (d, J =8.3 Hz, 1H), 8.30 (d, J =8.0 Hz, 1H), 8.19 (d, J =7.8 Hz, 1H), 8.03 (br d, J =7.8 Hz, 1H), 7.83 (br s, 1H), 7.52 (td, J =8.0, 15.9 Hz, 2H), 7.18 (br d, J =7.8 Hz, 2H), 6.79 (s, 1H), 4.72 (d, J =4.5 Hz, 1H), 4.25 - 4.09 (m, 3H), 3.88 (br s, 1H), 3.79 - 3.63 (m, 2H), 3.18 - 3.09 (m, 2H), 2.74 - 2.58 (m, 4H), 2.47 - 2.40 (m, 1H), 2.34 (dd, J =3.3, 9.5 Hz, 1H), 2.47 - 2.30 (m, 1H), 2.17 (br s, 2H), 2.00 (br dd, J =6.9, 12.7 Hz, 2H), 1.92 (br s, 1H), 1.81 (s, 3H), 1.66 (br d, J =10.5 Hz, 1H), 1.55 (br d, J =4.5 Hz, 1H).

### 21-5. Preparation of Compound 104, 2-((2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid

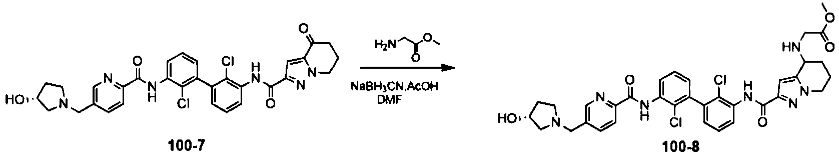

A solution of compound 100-7 (150 mg, 242.13 µmol, 1 eq), methyl 2-aminoacetate; hydrochloride (150 mg, 1.19 mmol, 4.93 eq), and AcOH (157.50 mg, 2.62 mmol, 150.00 µL, 10.83 eq) in DMF (3 mL) was stirred at 60 °C for 30 minutes, after which NaBH₃CN (100.00 mg, 1.59 mmol, 6.57 eq) was added, and the mixture was stirred at 60 °C for 18 hours. The mixture was diluted with H₂O (20 mL), adjusted to pH 8 with aq. saturated NaHCO₃ solution, and extracted with EA (50 mL * 2). The organic layer was dried over Na₂SO₄, concentrated under reduced pressure, and the residue was purified by flash silica gel chromatography (eluent: 0-40% MeOH/EA) to afford Compound 100-8 (150 mg, 63.8% yield) as a yellow solid.

LCMS m/z 692.4 [M+1]⁺.

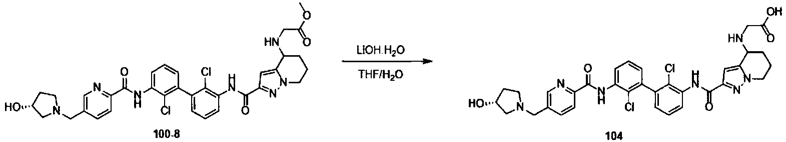

A solution of compound 100-8 (150 mg, 216.58 µmol, 1 eq) in THF (2 mL) and H₂O (0.5 mL) was treated with LiOH·H₂O (70 mg, 2.92 mmol, 13.50 eq), and the mixture was stirred at room temperature for 5 hours. The mixture was adjusted to pH 7 with aq. 1N HCl solution, concentrated under reduced pressure, and purified by prep-HPLC (column: Phenomenex C18 80 * 40mm * 3um; mobile phase: [water (NH₃H₂O+NH₄HCO₃)-ACN]; B%: 27%-57%, 8 min) to afford Compound 104 (61.69 mg, 40.1% yield) as a white solid.

LCMS m/z 678.3[M+1]⁺.

¹H NMR (400MHz, DMSO-d6) δ 10.72 (s, 1H), 9.57 (s, 1H), 8.68 (s, 1H), 8.53 (d, J =8.3 Hz, 1H), 8.28 (d, J =8.3 Hz, 1H), 8.20 (d, J =8.0 Hz, 1H), 8.03 (dd, J =1.8, 8.0 Hz, 1H), 7.52 (td, J=7.9, 15.6 Hz, 2H), 7.19 (d, J =7.5 Hz, 2H), 6.82 (s, 1H), 4.27 - 4.11 (m, 4H), 4.04 (br t, J =5.6 Hz, 1H), 3.78 - 3.65 (m, 2H), 3.30 (s, 3H), 2.70 (dd, J =6.1, 9.7 Hz, 1H), 2.62 (q, J =7.7 Hz, 1H), 2.49 - 2.41 (m, 1H), 2.35 (dd, J =3.6, 9.7 Hz, 1H), 2.20 (br dd, J =5.6, 13.4 Hz, 1H), 2.01 (br dd, J =6.9, 12.9 Hz, 2H), 1.91 (br dd, J =6.5, 13.8 Hz, 1H), 1.80 - 1.68 (m, 1H), 1.62 - 1.50 (m, 1H).

### 21-6. Preparation of Compound 105, (3R)-1-(2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

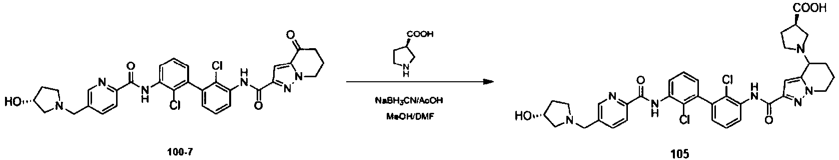

A solution of compound 100-7 (150 mg, 242.13 µmol, 1 eq), methyl 2-aminoacetate; hydrochloride (150 mg, 1.19 mmol, 4.93 eq), and AcOH (157.50 mg, 2.62 mmol, 150.00 µL, 10.83 eq) in DMF (3 mL) was stirred at 60 °C for 30 minutes, after which NaBH₃CN (100.00 mg, 1.59 mmol, 6.57 eq) was added, and the mixture was stirred at 60 °C for 18 hours. The mixture was diluted with H₂O (20 mL), adjusted to pH 8 with aq. saturated NaHCO₃ solution, and extracted with EA (50 mL * 2). The organic layer was dried over Na₂SO₄, concentrated under reduced pressure, and the residue was purified by flash silica gel chromatography (eluent: 0-40% MeOH/EA) to afford Compound 100-8 (150 mg, 63.8% yield) as a yellow solid.

LCMS m/z 718.2 [M+1]⁺.

¹H NMR (400 MHz, DMSO-d6) δ ppm 1.53 - 1.63 (m, 1 H) 1.86 - 2.02 (m, 6 H) 2.32 - 2.38 (m, 2 H) 2.58 - 2.65 (m, 2 H) 2.66 - 2.76 (m, 3 H) 2.88 - 2.98 (m, 2 H) 3.18 (s, 2 H) 3.67 - 3.79 (m, 2 H) 3.83 (br s, 1 H) 4.14 - 4.24 (m, 3 H) 4.62 - 4.83 (m, 1 H) 6.73 (d, J =4.63 Hz, 1 H) 7.19 (br d, J =7.63 Hz, 2 H) 7.47 - 7.59 (m, 1 H) 7.52 (dt, J =15.88, 7.94 Hz, 1 H) 8.03 (br d, J=8.13 Hz, 1 H) 8.16 - 8.32 (m, 2 H) 8.53 (d, J =8.25 Hz, 1 H) 8.68 (s, 1 H) 9.56 (s, 1 H) 10.72 (s, 1 H).

### 21-7. Preparation of Compound 107, 1-(2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)piperidine-4-carboxylic acid

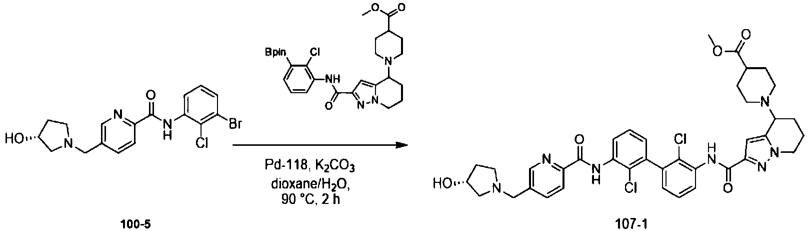

N-(3-bromo-2-chloro-phenyl)-5-[[(3R)-3-hydroxypyrrolidin-1-yl]methyl]pyridine-2-carboxamide (100 mg, 243.49 µmol, 1 eq), methyl 1-[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (158.62 mg, 292.19 µmol, 1.2 eq), K₂CO₃ (94.23 mg, 681.78 µmol, 2.8 eq), and di-tert-butyl(cyclopentyl)phosphane;dichloropalladium iron (15.87 mg, 24.35 µmol, 0.1 eq) were dissolved in a mixed solvent of dioxane (6 mL) and H₂O (1.5 mL), and the resulting mixture was stirred at 90 °C for 2 hours under a nitrogen atmosphere. After completion of the reaction, the mixture was filtered, and the filtrate was concentrated under reduced pressure. The residue was purified by prep-TLC (dichloromethane:methanol = 10:1) to afford Compound 107-1 (161 mg, 70.8% yield) as a white solid.

LCMS m/z 748.3 [M+1]⁺.

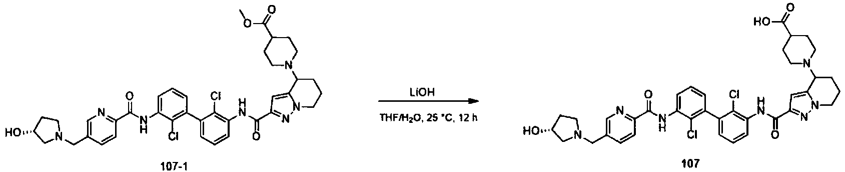

Methyl 1-[2-[[2-chloro-3-[2-chloro-3-[[5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]piperidine-4-carboxylate (150 mg, 200.89 µmol, 1 eq) was dissolved in a mixed solvent of THF (6 mL) and H₂O (2 mL), and LiOH (25.29 mg, 602.67 µmol, 3 eq) was added thereto. The reaction mixture was stirred at room temperature for 12 hours. After completion of the reaction, the mixture was concentrated under reduced pressure, and the residue was purified by prep-HPLC (column: Waters XBridge, 150 * 25 mm * 5 µm; mobile phase: [water (NH₄HCO₃)-ACN]; gradient: 20-50% B over 9 min) to afford Compound 107 (68 mg, 43.9% yield) as a white solid.

LCMS m/z 734.0 [M+1]⁺.

¹H NMR (400 MHz, DMSO-d6 ) δ = 10.72 (s, 1H), 9.55 (s, 1H), 8.67 (s, 1H), 8.52 (d, J = 8.3 Hz, 1H), 8.31 - 8.14 (m, 2H), 8.02 (br d, J = 8.0 Hz, 1H), 7.52 (td, J = 7.9, 16.0 Hz, 2H), 7.18 (br d, J = 7.4 Hz, 2H), 6.67 (s, 1H), 4.73 (br d, J = 4.6 Hz, 1H), 4.36 (br t, J = 4.9 Hz, 2H), 4.20 (br d, J = 6.1 Hz, 2H), 4.07 - 3.91 (m, 2H), 3.72 (br d, J = 10.6 Hz, 2H), 3.46 - 3.42 (m, 4H), 2.71 - 2.65 (m, 3H), 2.62 (br s, 2H), 2.39 - 2.29 (m, 2H), 2.05 - 1.92 (m, 2H), 1.70 - 1.47 (m, 4H).

### 21-8. Preparation of Compound 106, N-(2,2'-dichloro-3'-(5-(((R)-3-hydroxypyrrolidin-1-yl)methyl)picolinamido)-[1,1'-biphenyl]-3-yl)-4-(4-hydroxypiperidin-1-yl)-4,5,6,7-tetrahydropyrazolo[1,5-a]pyridine-2-carboxamide

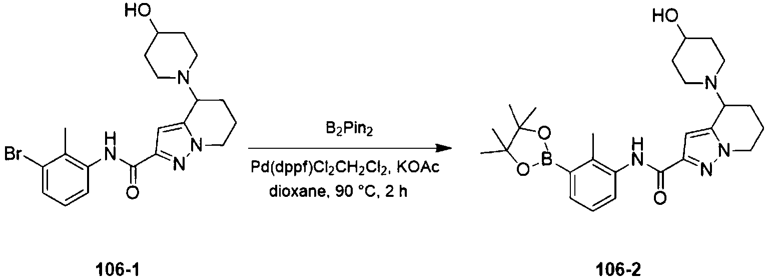

N-(3-bromo-2-chloro-phenyl)-4-(4-hydroxy-1-piperidyl)-4,5,6,7-tetrahydropyrazolo[1,5-a]pyridine-2-carboxamide (300 mg, 661.14 µmol, 1 eq), 4,4,5,5-tetramethyl-2-(4,4,5,5-tetramethyl-1,3,2-dioxaborolan-2-yl)-1,3,2-dioxaborolane (503.67 mg, 1.98 mmol, 3 eq), KOAc (194.65 mg, 1.98 mmol, 3 eq), and Pd(dppf)Cl₂·CH₂Cl₂ (53.99 mg, 66.11 µmol, 0.1 eq) were dissolved in dioxane (10 mL), and the resulting mixture was stirred at 90 °C for 2 hours under a nitrogen atmosphere. After completion of the reaction, the mixture was filtered, and the filtrate was concentrated under reduced pressure. The residue was purified by flash silica gel chromatography (dichloromethane:methanol = 100:1 to 10:1) to afford Compound 106-2 (460 mg, 97.2% yield) as a white solid.

LCMS m/z 501.3 [M+1]⁺.

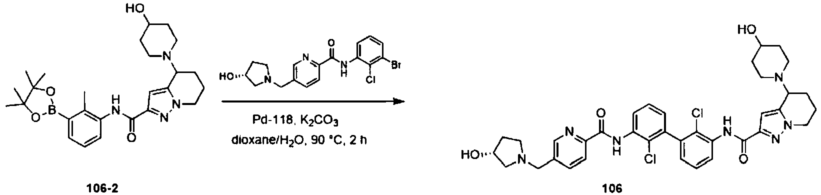

N-(3-bromo-2-chloro-phenyl)-5-[[(3R)-3-hydroxypyrrolidin-1-yl]methyl]pyridine-2-carboxamide (90 mg, 219.14 µmol, 1 eq), N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (131.70 mg, 262.97 µmol, 1.2 eq), K₂CO₃ (84.81 mg, 613.60 µmol, 2.8 eq), and di-tert-butyl(cyclopentyl)phosphane;dichloropalladium iron (14.28 mg, 21.91 µmol, 0.1 eq) were dissolved in a mixed solvent of dioxane (8 mL) and H₂O (2 mL). The resulting mixture was stirred at 90 °C for 2 hours under a nitrogen atmosphere. After completion of the reaction, the mixture was filtered, and the filtrate was concentrated under reduced pressure. The residue was purified by prep-HPLC (column: Waters XBridge Prep OBD C18, 150 * 40 mm * 10 µm; mobile phase: [water (NH₄HCO₃)-ACN]; gradient: 30-60% B over 15 min) to afford Compound 106 (16 mg, 9.8% yield) as a white solid.

LCMS m/z 706.1 [M+1]⁺.

¹H NMR (400 MHz, DMSO-d6) δ = 10.72 (s, 1H), 9.55 (s, 1H), 8.67 (s, 1H), 8.58 - 8.48 (m, 1H), 8.31 - 8.24 (m, 1H), 8.19 (d, J = 8.0 Hz, 1H), 8.02 (dd, J = 1.6, 7.9 Hz, 1H), 7.65 - 7.44 (m, 2H), 7.29 - 7.08 (m, 2H), 6.65 (s, 1H), 4.83 - 4.52 (m, 2H), 4.23 - 4.16 (m, 2H), 4.14 - 3.93 (m, 2H), 3.77 - 3.66 (m, 2H), 3.51 - 3.38 (m, 1H), 2.73 - 2.59 (m, 3H), 2.43 - 2.31 (m, 3H), 2.20 - 2.12 (m, 2H), 2.05 - 1.82 (m, 4H), 1.72 (dt, J = 4.9, 8.9 Hz, 2H), 1.60 - 1.31 (m, 4H).

### 21-9. Preparation of Compound 108, 2-((2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)-2-methylpropanoic acid

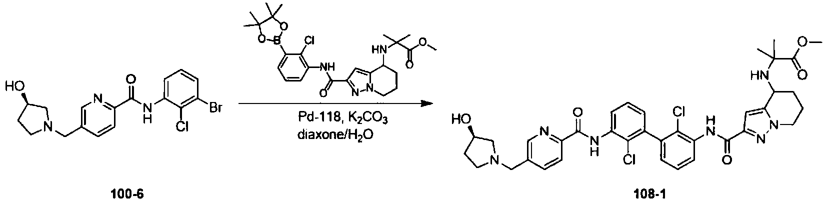

N-(3-bromo-2-chloro-phenyl)-5-[[(3R)-3-hydroxypyrrolidin-1-yl]methyl]pyridine-2-carboxamide (120 mg, 292.19 µmol, 1 eq), methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methylpropanoate (492.43 mg, 438.28 µmol, 1.5 eq), di-tert-butyl(cyclopentyl)phosphane;dichloropalladium iron (19.04 mg, 29.22 µmol, 0.1 eq), and K₂CO₃ (121.15 mg, 876.57 µmol, 3 eq) were dissolved in a mixed solvent of dioxane (5 mL) and H₂O (1 mL). The resulting mixture was stirred at 90 °C for 12 hours under a nitrogen atmosphere. After completion of the reaction, the mixture was cooled to room temperature, filtered, and the filtrate was concentrated under reduced pressure. The residue was purified by prep-TLC (SiO₂, Ethyl acetate: Methanol = 3:1) to afford Compound 108-1 (200 mg, 54.1% yield) as a white solid.

LCMS m/z 722.2 [M+3]⁺.

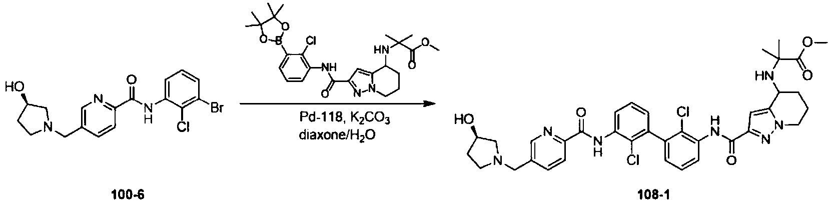

Methyl 2-[[2-[[2-chloro-3-[2-chloro-3-[[5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]amino]-2-methylpropanoate (200 mg, 277.53 µmol, 1 eq) was dissolved in a mixed solvent of H₂O (3 mL) and MeOH (3 mL), and LiOH (19.92 mg, 832.59 µmol, 3 eq) was added thereto. The reaction mixture was stirred at room temperature for 12 hours. After completion of the reaction, the mixture was concentrated under reduced pressure, and the residue was purified by reversed-phase HPLC (column: Waters XBridge, 150 * 25 mm * 5 um; mobile phase: [water (NH₄HCO₃)-ACN]; gradient: 18-48% B over 9 min) to afford Compound 108 (32 mg, 16.1% yield) as a white solid.

LCMS m/z 706.2 [M+1]⁺.

¹H NMR (400 MHz, DMSO-d6) δ ppm 1.30 (br d, J=2.88 Hz, 6 H) 1.52 - 1.69 (m, 2 H) 1.87 - 2.20 (m, 6 H) 2.32 - 2.39 (m, 2 H) 2.61 - 2.73 (m, 2 H) 3.67 - 3.80 (m, 2 H) 3.91 - 3.96 (m, 1 H) 4.07 - 4.25 (m, 3 H) 4.71 - 4.78 (m, 1 H) 6.79 (s, 1 H) 7.15 - 7.22 (m, 2 H) 7.52 (dt, J=15.88, 7.94 Hz, 2 H) 8.04 (br d, J=8.13 Hz, 1 H) 8.20 (d, J=7.88 Hz, 1 H) 8.30 (br d, J=8.13 Hz, 1 H) 8.53 (br d, J=8.51 Hz, 1 H) 8.68 (s, 1 H) 9.55 (s, 1 H) 10.72 (s, 1 H).

### [Preparation Example 22]

Preparation of Compound 109, N-(2,2'-dimethyl-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide

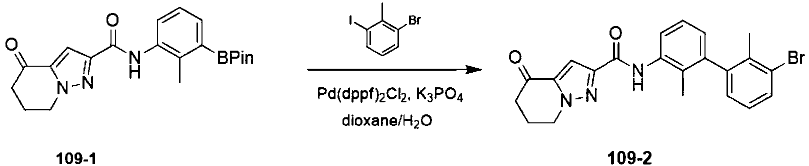

Compound 109-1 (2 g, 5.06 mmol, 1 eq), 1-bromo-3-iodo-2-methylbenzene (1.65 g, 5.57 mmol, 1.1 eq), Pd(dppf)Cl₂ (370.24 mg, 506.00 µmol, 0.1 eq), and K₃PO₄ (1.07 g, 5.06 mmol, 1 eq) were dissolved in a mixed solvent of dioxane (20 mL) and H₂O (4 mL). The resulting mixture was stirred at 60 °C for 16 hours under a nitrogen atmosphere. After completion of the reaction, the mixture was filtered, and the filtrate was diluted with EtOAc (50 mL), washed with brine (40 mL * 3), dried over Na₂SO₄, and concentrated under reduced pressure. The residue was purified by column chromatography (SiO₂, Petroleum ether/Ethyl acetate = 100/1 to 1/1) to afford Compound 109-2 (1.90 g, 85.5% yield) as a white solid.

LCMS m/z 439.9 [M+1]⁺.

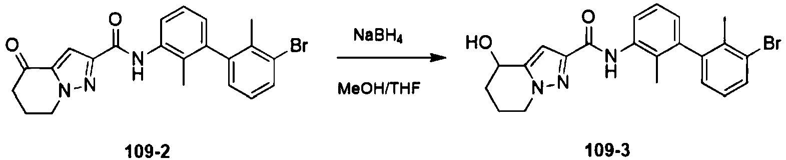

Compound 109-2 (1.9 g, 4.33 mmol, 1 eq) was dissolved in a mixed solvent of THF (20 mL) and MeOH (20 mL), and NaBH₄ (328.62 mg, 8.66 mmol, 2 eq) was slowly added at 0 °C. The reaction mixture was then stirred at room temperature for 2 hours. Thereafter, H₂O (20 mL * 3) was slowly added to the mixture at 0 °C, and the mixture was extracted with EA (50 mL * 2). The organic layer was washed with brine (30 mL), dried over Na₂SO₄, and concentrated under reduced pressure. The residue was purified by column chromatography (SiO₂, Petroleum ether/Ethyl acetate = 100:1 to 2:1) to afford Compound 109-3 (1.6 g, 74.9% yield) as a white solid.

LCMS m/z 441.9 [M+1]⁺.

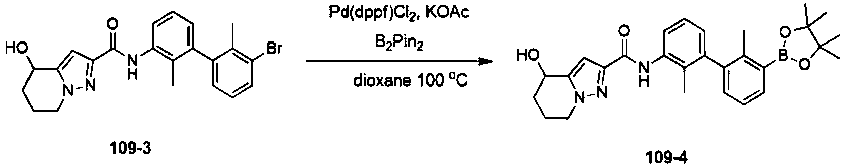

Compound 109-3 (1.6 g, 3.25 mmol, 89.438% purity, 1 eq), Pd(dppf)Cl₂ (237.79 mg, 324.98 µmol, 0.1 eq), KOAc (956.84 mg, 9.75 mmol, 3 eq), and 4,4,5,5-tetramethyl-2-(4,4,5,5-tetramethyl-1,3,2-dioxaborolan-2-yl)-1,3,2-dioxaborolane (2.48 g, 9.75 mmol, 3 eq) were dissolved in dioxane (20 mL). The resulting mixture was stirred at 100 °C for 4 hours under a nitrogen atmosphere. After completion of the reaction, the mixture was filtered, and the filtrate was diluted with EtOAc (100 mL). The organic layer was washed with brine (50 mL x 3), dried over Na₂SO₄, and concentrated under reduced pressure. The residue was purified by column chromatography (SiO₂, Petroleum ether/Ethyl acetate = 10:1 to 1:1) to afford Compound 109-4 (1.66 g, 93.4% yield) as a yellow solid.

LCMS m/z 488.2 [M+1]⁺.

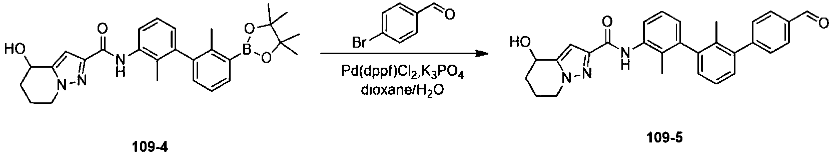

Compound 109-4 (300 mg, 548.66 µmol, 89.139% purity, 1 eq), 4-bromobenzaldehyde (111.66 mg, 603.53 µmol, 1.1 eq), K₃PO₄ (349.39 mg, 1.65 mmol, 3 eq), and Pd(dppf)Cl₂ (40.15 mg, 54.87 µmol, 0.1 eq) were dissolved in a mixed solvent of dioxane (5 mL) and H₂O (1 mL). The resulting mixture was stirred at 60 °C for 15 hours under a nitrogen atmosphere. After completion of the reaction, the mixture was filtered, and the filtrate was diluted with EtOAc (100 mL). The organic layer was washed with brine (50 mL x 3), dried over Na₂SO₄, and concentrated under reduced pressure. The residue was purified by prep-TLC (SiO₂, petroleum ether:ethyl acetate = 1:1) to afford Compound 109-5 (230 mg, 80.1% yield) as a white solid.

LCMS m/z 466.2 [M+1]⁺.

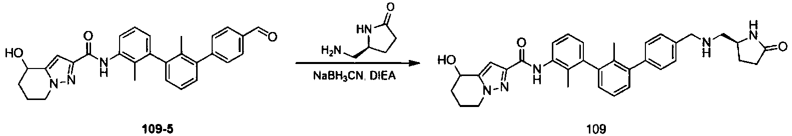

Compound 109-5 (400 mg, 859.21 µmol, 1 eq) and (5S)-5-(aminomethyl)pyrrolidin-2-one (117.69 mg, 1.03 mmol, 1.2 eq) were dissolved in MeOH (2 mL), and DIPEA (111.05 mg, 859.21 µmol, 149.66 µL, 1 eq) was added thereto. The reaction mixture was stirred at room temperature for 30 minutes. NaBH₃CN (269.97 mg, 4.30 mmol, 5 eq) was then added, and the mixture was stirred at room temperature for an additional 2 hours. After completion of the reaction, the mixture was concentrated under reduced pressure, and the residue was purified by reversed-phase HPLC (column: Waters XBridge C18, 150 * 50 mm * 10 um; mobile phase: [water (NH₄HCO₃)-ACN]; gradient: 28-58% B over 10 min) to afford Compound 109 (62 mg, 12.5% yield) as a white solid.

LCMS m/z 564.3 [M+1]⁺.

¹H NMR (400 MHz, DMSO-d6) δ = 9.56 (s, 1H), 8.34 (s, 1H), 7.71 (s, 1H), 7.57 (d, J = 8.3 Hz, 1H), 7.51 - 7.41 (m, 2H), 7.42 - 7.28 (m, 2H), 4.12 (br s, 2H), 3.95 (s, 2H), 3.88 (s, 1H), 3.62 (s, 2H), 2.68 - 2.56 (m, 5H), 2.33 (s, 1H), 2.23 - 2.00 (m, 6H), 1.96 (s, 3H), 1.72 (d, J = 5.5 Hz, 1H).

### [Preparation Example 23]

Preparation of Compound 110, 4-hydroxy-N-(3'-(6-methoxy-5-(((((S)-5-oxopyrrolidin-2-yl)methyl)amino)methyl)pyrazin-2-yl)-2,2'-dimethyl-[1,1'-biphenyl]-3-yl)-4,5,6,7-tetrahydropyrazolo[1,5-a]pyridine-2-carboxamide

To a solution of 5-Bromo-2-iodo-3-methoxypyrazine (2 g, 6.35 mmol, 1 eq) in THF (20 mL), i-PrMgCl-LiCl (1.3 M in THF, 5.86 mL, 1.2 eq) was added at -78 °C. The reaction mixture was stirred at -78 °C for 30 minutes. DMF (1.86 g, 25.40 mmol, 1.95 mL, 4 eq) was then slowly added at -78 °C, and the mixture was stirred at -20 °C for 30 minutes. The reaction mixture was diluted with an aqueous citric acid solution (5%, 50 mL) and stirred at 20 °C for 10 minutes, followed by extraction with EtOAc (30 mL * 2). The organic layer was washed with brine (50 mL), dried over Na₂SO₄, and concentrated under reduced pressure. The residue was purified by column chromatography (SiO₂, Petroleum ether/Ethyl acetate = 100:1 to 5:1) to afford Compound 110-6 (2.1 g) as a yellow liquid.

¹H NMR (400 MHz, DMSO-d6) delta 10.08 (s, 1H), 8.65 (s, 1H), 4.05 (s, 3H).

To a solution of Compound 110-4 (300 mg, 615.51 µmol, 1 eq) and Compound 110-6 (146.94 mg, 677.07 µmol, 1.1 eq) in dioxane (5 mL)/H₂O (1 mL), Pd(dppf)Cl₂ (45.04 mg, 61.55 µmol, 0.1 eq) and K₃PO₄ (391.96 mg, 1.85 mmol, 3 eq) were added, and the resulting mixture was stirred at 60 °C for 16 hours under a nitrogen atmosphere. After completion of the reaction, the mixture was filtered, and the filtrate was diluted with EtOAc (20 mL). The organic layer was washed with brine (20 mL * 3), dried over Na₂SO₄, and concentrated under reduced pressure. The residue was purified by prep-TLC (SiO₂, PE:EA = 1:1) to afford Compound 110-7 (370 mg, 79.2% yield) as a white solid.

LCMS m/z 498.2 [M+1]⁺.

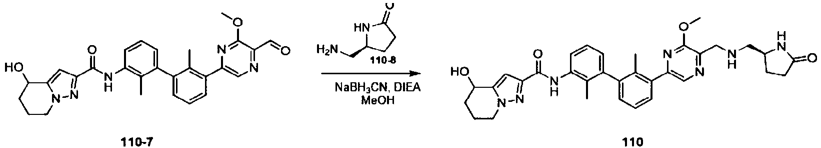

To a solution of Compound 110-8 (91.77 mg, 803.95 µmol, 2 eq) in MeOH (3 mL), DIPEA (51.95 mg, 401.97 µmol, 70.02 µL, 1 eq) was added. The reaction mixture was stirred at room temperature for 30 minutes. Compound 110-7 (200 mg, 401.97 µmol, 1 eq) and NaBH₃CN (252.61 mg, 4.02 mmol, 10 eq) were then added, and the mixture was stirred at 60 °C for 5 hours. After completion of the reaction, the mixture was concentrated under reduced pressure, and the residue was purified by reversed-phase HPLC (column: Waters XBridge C18, 150 * 50 mm * 10 um; mobile phase: [water (NH₄HCO₃)-ACN]; B%: 26%-56% B, 10 min) to afford Compound 110 (82 mg, 34.2% yield) as a white solid.

LCMS m/z 596.3 [M+1]⁺.

¹H NMR (400 MHz, DMSO-d6) δ = 9.55 (s, 1H), 8.34 (s, 1H), 7.70 (s, 1H), 7.57 (d, J = 8.3 Hz, 1H), 7.51 - 7.47 (m, 1H), 7.42 - 7.37 (m, 1H), 7.28 (t, J = 7.8 Hz, 1H), 4.76 (d, J = 6.5 Hz, 1H), 4.12 (br s, 2H), 3.95 (s, 2H), 3.88 (s, 1H), 3.62 (s, 2H), 3.37 (s, 3H), 2.68 - 2.56 (m, 5H), 2.33 (s, 1H), 2.23 - 2.00 (m, 6H), 1.96 (s, 3H), 1.72 (d, J = 5.5 Hz, 1H).

### [Preparation Example 24]

Preparation of Compound 111, N-(2,2'-dichloro-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide

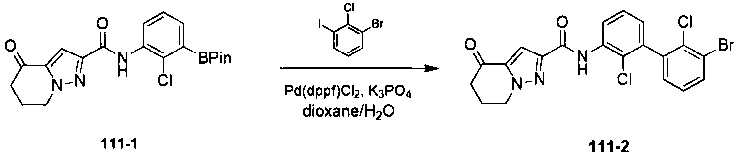

To a solution of N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (4 g, 9.62 mmol, 1 eq) and 1-bromo-2-chloro-3-iodobenzene (3.05 g, 9.62 mmol, 1 eq) in dioxane (50 mL)/H₂O (10 mL), Pd(dppf)Cl₂ (704.11 mg, 962.28 µmol, 0.1 eq) and K₃PO₄ (6.13 g, 28.87 mmol, 3 eq) were added, and the resulting mixture was stirred at 60 °C for 12 hours. After completion of the reaction, the mixture was concentrated under reduced pressure, and the residue was purified by column chromatography (SiO₂, Petroleum ether/Ethyl acetate = 100/1 to 1/1) to afford Compound 111-2 (3.5 g, 64.6% yield) as a yellow solid.

LCMS m/z 480.0 [M+1]⁺.

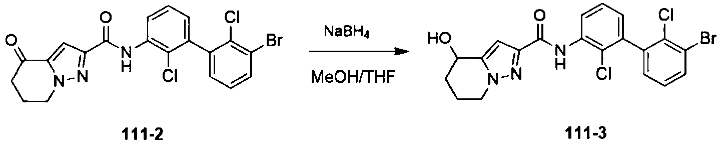

To a solution of N-[3-(3-bromo-2-chloro-phenyl)-2-chlorophenyl]-4-oxo-6,7-dihydro-5H-pyrazolo[1,5-a]pyridine-2-carboxamide (3.5 g, 7.30 mmol, 1 eq) in THF (30 mL)/MeOH (15 mL), NaBH₄ (552.68 mg, 14.60 mmol, 2 eq) was added and the reaction mixture was stirred at room temperature for 3 hours. Thereafter, an aq. saturated NH₄Cl solution (100 mL) was added to the mixture at 0 °C, followed by extraction with EA (50 mL). The organic layer was washed with brine (100 mL), dried over Na₂SO₄, and concentrated under reduced pressure. The residue was purified by column chromatography (SiO₂, Petroleum ether/Ethyl acetate = 100:1 to 1:1) to afford Compound 111-3 (3.2 g, 77.4% yield) as a white solid.

LCMS m/z 482.0 [M+1]⁺.

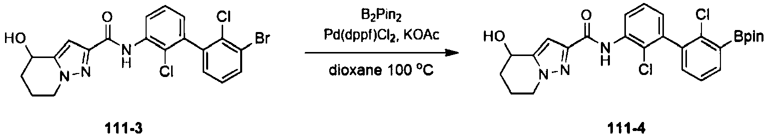

A mixture of N-[3-(3-bromo-2-chloro-phenyl)-2-chlorophenyl]-4-hydroxy-4,5,6,7-tetrahydropyrazolo[1,5-a]pyridine-2-carboxamide (3.2 g, 6.65 mmol, 1 eq), 4,4,5,5-tetramethyl-2-(4,4,5,5-tetramethyl-1,3,2-dioxaborolan-2-yl)-1,3,2-dioxaborolane (1.86 g, 7.32 mmol, 1.1 eq), Pd(dppf)Cl₂ (486.62 mg, 665.05 µmol, 0.1 eq), and KOAc (979.04 mg, 9.98 mmol, 1.5 eq) in dioxane (50 mL) was stirred at 100 °C for 16 hours under a nitrogen atmosphere. After completion of the reaction, the mixture was concentrated under reduced pressure, and the residue was purified by column chromatography (SiO₂, Petroleum ether/Ethyl acetate = 100:1 to 1:1) to afford Compound 111-4 (2.7 g, 38.8% yield) as a yellow solid.

LCMS m/z 528.2 [M+1]⁺.

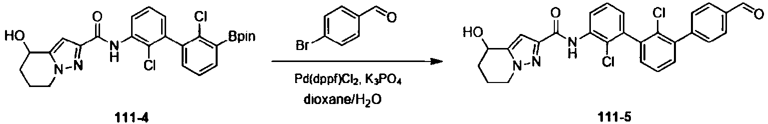

A mixture of N-[2-chloro-3-[2-chloro-3-(4,4,5,5-tetramethyl-1,3,2-dioxaborolan-2-yl)phenyl]phenyl]-4-hydroxy-4,5,6,7-tetrahydropyrazolo[1,5-a]pyridine-2-carboxamide (300 mg, 567.93 µmol, 1 eq), 4-bromobenzaldehyde (126.09 mg, 681.52 µmol, 1.2 eq), K₃PO₄ (361.66 mg, 1.70 mmol, 3 eq), and Pd(dppf)Cl₂ (41.56 mg, 56.79 µmol, 0.1 eq) in dioxane (5 mL)/H₂O (1 mL) was stirred at 80 °C for 16 hours under a nitrogen atmosphere. After completion of the reaction, the mixture was concentrated under reduced pressure, and the residue was purified by column chromatography (SiO₂, Petroleum ether/Ethyl acetate = 100:1 to 1:1) to afford Compound 111-5 (260 mg, 55.6% yield) as a yellow solid.

LCMS m/z 506.1 [M+1]⁺.

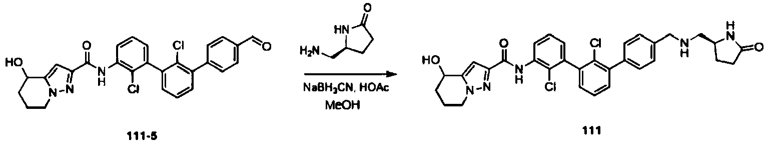

To a solution of N-[2-chloro-3-[2-chloro-3-(4-formylphenyl)phenyl]phenyl]-4-hydroxy-4,5,6,7-tetrahydropyrazolo[1,5-a]pyridine-2-carboxamide (200 mg, 394.96 µmol, 1 eq) and (5S)-5-(aminomethyl)pyrrolidin-2-one (450.83 mg, 3.95 mmol, 10 eq) in MeOH (5 mL), HOAc (237.18 mg, 3.95 mmol, 225.89 µL, 10 eq) and NaBH₃CN (248.20 mg, 3.95 mmol, 10 eq) were added, and the resulting mixture was stirred at room temperature for 4 hours. After completion of the reaction, the mixture was concentrated under reduced pressure, and the residue was purified by reversed-phase HPLC (column: Waters XBridge, 150 * 25 mm, 5 um; mobile phase: [water (NH₄HCO₃)-ACN]; B%: 38%-68%, 8 min) to afford Compound 111 (120 mg, 49.7% yield) as a yellow solid.

LCMS m/z 604.2 [M+1]⁺.

¹H NMR (400 MHz, DMSO-d6) δ = 9.55 (s, 1H), 8.27 (d, J = 8.2 Hz, 1H), 7.68 (s, 1H), 7.53 - 7.40 (m, 7H), 7.36 (dd, J = 1.7, 7.3 Hz, 1H), 7.19 (dd, J = 1.5, 7.6 Hz, 1H), 6.74 (s, 1H), 5.60 (d, J = 5.5 Hz, 1H), 4.76 (q, J = 5.5 Hz, 1H), 4.18 - 4.10 (m, 2H), 3.81 - 3.71 (m, 2H), 3.63 (s, 1H), 2.55 - 2.52 (m, 2H), 2.40 - 2.26 (m, 1H), 2.19 - 1.90 (m, 6H), 1.77 - 1.63 (m, 2H).

### [Preparation Example 25]

### Preparation of Compounds 112 to 115

### 25-1. Preparation of Compound 112, N-(3"-fluoro-5"-methoxy-2,2'-dimethyl-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide

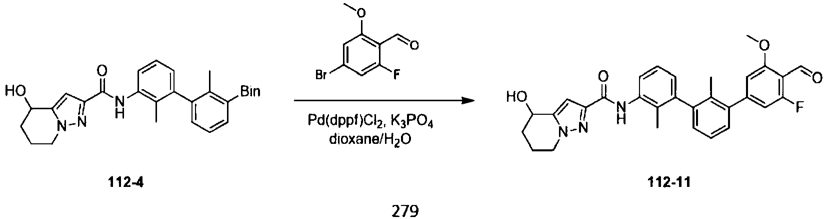

To a mixture of Compound 112-4 (500 mg, 1.03 mmol, 1 eq) and 4-bromo-2-fluoro-6-methoxybenzaldehyde (262.97 mg, 1.13 mmol, 1.1 eq) in dioxane (5 mL)/H₂O (1 mL), Pd(dppf)Cl₂ (75.06 mg, 102.59 µmol, 0.1 eq) and K₃PO₄ (653.26 mg, 3.08 mmol, 3 eq) were added, and the resulting mixture was stirred at 60 °C for 16 hours under a nitrogen atmosphere. After completion of the reaction, the mixture was filtered, and the filtrate was diluted with EtOAc (50 mL). The organic layer was washed with brine (50 mL x 3), dried over Na₂SO₄, and concentrated under reduced pressure. The residue was purified by prep-TLC (SiO₂, PE:EA = 1:1) to afford Compound 112-11 (370 mg, 67.5% yield) as a yellow liquid.

LCMS m/z 514.3 [M+1]⁺.

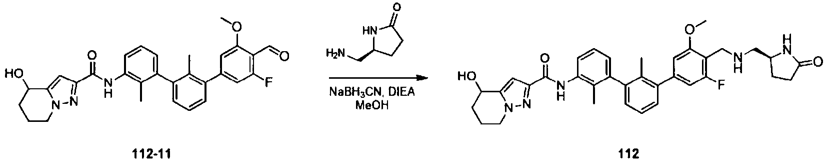

To a solution of (5S)-5-(aminomethyl)pyrrolidin-2-one (366.73 mg, 3.21 mmol, 5 eq) in MeOH (5 mL), DIPEA (83.05 mg, 642.58 µmol, 111.93 µL, 1 eq) was added. The reaction mixture was stirred at room temperature for 20 minutes. Compound 112-11 (330 mg, 642.58 µmol, 1 eq) and NaBH₃CN (403.81 mg, 6.43 mmol, 10 eq) were then added, and the mixture was stirred at 60 °C for 4 hours. After completion of the reaction, the mixture was concentrated under reduced pressure, and the residue was purified by reversed-phase HPLC (column: Waters XBridge C18, 150 * 50 mm * 10 um; mobile phase: [water (NH₄HCO₃)-ACN]; B%: 32%-62%, 10 min) to afford Compound 112 (93 mg, 23.7% yield) as a white solid.

LCMS m/z 612.3 [M+1]⁺.

¹H NMR (400 MHz, DMSO-d6) δ = 9.52 (s, 1H), 7.66 (s, 1H), 7.57 (d, J = 7.9 Hz, 1H), 7.36 - 7.25 (m, 3H), 7.12 (d, J = 7.3 Hz, 1H), 7.01 (d, J = 7.3 Hz, 1H), 6.85 - 6.79 (m, 2H), 6.68 (s, 1H), 5.55 (d, J = 5.5 Hz, 1H), 4.78 - 4.73 (m, 1H), 4.17 - 4.08 (m, 2H), 3.86 (s, 3H), 3.74 (s, 2H), 3.58 (s, 1H), 3.31 - 3.29 (m, 6H), 2.52 - 2.52 (m, 4H), 2.18 - 2.14 (m, 1H), 1.97 (s, 3H), 1.92 (s, 3H)

### 25-2. Preparation of Compound 112-1, N-(4"-(((2-acetamidoethyl)amino)methyl)-3"-fluoro-5"-methoxy-2,2'-dimethyl-[1,1':3',1"-terphenyl]-3-yl)-4-hydroxy-4,5,6,7-tetrahydropyrazolo[1,5-a]pyridine-2-carboxamide

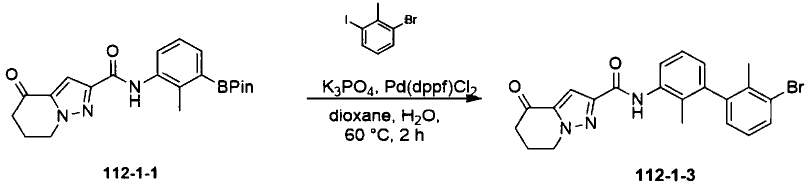

A mixture of N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (1.5 g, 3.79 mmol, 1 eq), 1-bromo-3-iodo-2-methylbenzene (1.13 g, 3.79 mmol, 1 eq), K₃PO₄ (2.42 g, 11.38 mmol, 3 eq), and Pd(dppf)Cl₂ (154.96 mg, 189.75 µmol, 0.05 eq) in dioxane (15 mL)/H₂O (3 mL) was stirred at 60 °C for 2 hours under a nitrogen atmosphere. After completion of the reaction, the mixture was filtered, and the filtrate was concentrated under reduced pressure. The residue was purified by flash silica gel chromatography (Petroleum ether/Ethyl acetate = 100/1 to 1/1) to afford Compound 112-1-3 (1.32 g, 71.42% yield) as a white solid.

LCMS m/z 438.2 [M+1]⁺.

¹H NMR (400 MHz, DMSO-d6) δ = 9.87 (s, 1H), 7.62 (dd, J = 0.8, 7.8 Hz, 1H), 7.50 (d, J = 7.6 Hz, 1H), 7.33 - 7.24 (m, 2H), 7.23 - 7.17 (m, 1H), 7.12 (d, J = 6.8 Hz, 1H), 6.99 (d, J = 7.0 Hz, 1H), 4.46 (t, J = 5.8 Hz, 2H), 2.76 - 2.67 (m, 2H), 2.39 - 2.31 (m, 2H), 2.08 (s, 3H), 1.91 (s, 3H).

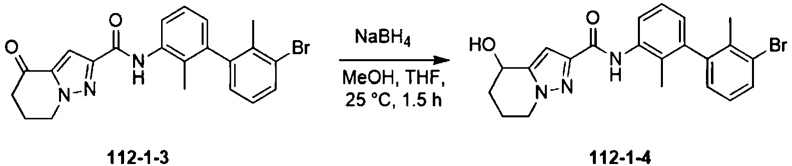

To a solution of N-[3-(3-bromo-2-methylphenyl)-2-methylphenyl]-4-oxo-6,7-dihydro-5H-pyrazolo[1,5-a]pyridine-2-carboxamide 3 (1.5 g, 3.42 mmol, 1 eq) in MeOH (30 mL)/THF (30 mL), NaBH₄ (430.11 mg, 6.84 mmol, 2 eq) was added. The reaction mixture was stirred at room temperature for 2 hours. Thereafter, the mixture was adjusted to pH 7 with an aq. saturated NH₄Cl solution, and the precipitated solid was collected by filtration. The solid was dried under vacuum and purified by flash silica gel chromatography (Petroleum ether/Ethyl acetate = 100/1 to 5/1) to afford Compound 112-1-4 (1.22 g, 76.9% yield) as a white solid.

LCMS m/z 442.0 [M+1]⁺.

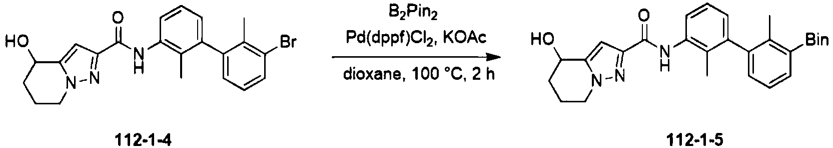

A mixture of N-[3-(3-bromo-2-methylphenyl)-2-methylphenyl]-4-hydroxy-4,5,6,7-tetrahydropyrazolo[1,5-a]pyridine-2-carboxamide (1.1 g, 2.50 mmol, 1 eq), 4,4,5,5-tetramethyl-2-(4,4,5,5-tetramethyl-1,3,2-dioxaborolan-2-yl)-1,3,2-dioxaborolane (697.80 mg, 2.75 mmol, 1.1 eq), Pd(dppf)Cl₂ (204.01 mg, 249.81 µmol, 0.1 eq), and KOAc (367.75 mg, 3.75 mmol, 1.5 eq) in dioxane (15 mL) was stirred at 100 °C for 2 hours under a nitrogen atmosphere. After completion of the reaction, the mixture was filtered, and the filtrate was concentrated under reduced pressure. The residue was purified by flash silica gel chromatography (Petroleum ether/Ethyl acetate = 100/1 to 1/1) to afford Compound 112-1-5 (1.12 g, 87.3% yield) as a white solid.

LCMS m/z 488.3 [M+1]⁺.

¹H NMR (400 MHz, DMSO-d6) δ = 9.49 (s, 1H), 7.69 - 7.63 (m, 1H), 7.57 (d, J = 7.9 Hz, 1H), 7.24 (dt, J = 1.7, 7.7 Hz, 2H), 7.15 (s, 1H), 6.91 (d, J = 7.5 Hz, 1H), 6.69 (s, 1H), 5.56 (d, J = 5.5 Hz, 1H), 4.86 - 4.68 (m, 1H), 4.30 - 4.07 (m, 2H), 2.16 (s, 3H), 1.88 (s, 3H), 1.31 (s, 11H).

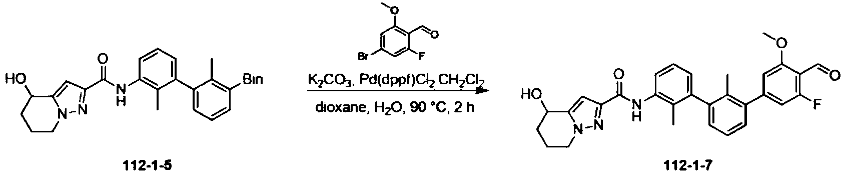

A mixture of 4-Hydroxy-N-[2-methyl-3-[2-methyl-3-(4,4,5,5-tetramethyl-1,3,2-dioxaborolan-2-yl)phenyl]phenyl]-4,5,6,7-tetrahydropyrazolo[1,5-a]pyridine-2-carboxamide (1.01 g, 2.07 mmol, 1.01 eq), 4-bromo-2-fluoro-6-methoxybenzaldehyde (1.1 g, 4.72 mmol, 2.30 eq), K₂CO₃ (794.22 mg, 5.75 mmol, 2.8 eq), and Pd(dppf)Cl₂·CH₂Cl₂ (83.80 mg, 102.62 µmol, 0.05 eq) in dioxane (15 mL)/ H₂O (0.5 mL) was stirred at 90 °C for 2 hours under a nitrogen atmosphere. After completion of the reaction, the mixture was diluted with H₂O (40 mL) and extracted with ethyl acetate (40 mL x 3). The organic layer was washed with brine (60 mL), dried over Na₂SO₄, and concentrated under reduced pressure. The residue was purified by flash silica gel chromatography (Petroleum ether/Ethyl acetate = 100/1 to 1/1) to afford Compound 112-1-7 (820 mg, 70.0% yield) as a white solid.

LCMS m/z 514.2 [M+1]⁺.

¹H NMR (400 MHz, DMSO-d6) δ = 10.34 (s, 1H), 9.53 (s, 1H), 7.58 (br d, J = 7.9 Hz, 2H), 7.41 - 7.35 (m, 1H), 7.34 - 7.25 (m, 2H), 7.17 (br d, J = 7.1 Hz, 1H), 7.07 - 6.88 (m, 3H), 6.69 (s, 1H), 5.56 (d, J = 5.4 Hz, 1H), 4.76 (q, J = 5.6 Hz, 1H), 4.17 - 4.05 (m, 2H), 3.97 (s, 3H), 3.17 (d, J = 5.3 Hz, 1H), 2.25 - 2.11 (m, 1H), 2.09 - 2.00 (m, 1H), 1.97 (s, 3H), 1.94 (s, 3H), 1.79 - 1.69 (m, 1H).

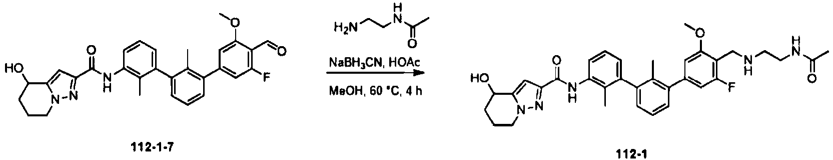

To a solution of N-[3-[3-(3-fluoro-4-formyl-5-methoxyphenyl)-2-methylphenyl]-2-methylphenyl]-4-hydroxy-4,5,6,7-tetrahydropyrazolo[1,5-a]pyridine-2-carboxamide (120 mg, 233.66 µmol, 1 eq) and N-(2-aminoethyl)acetamide (238.65 mg, 2.34 mmol, 223.04 µL, 10 eq) in MeOH (3 mL), HOAc (210.47 mg, 3.50 mmol, 200.45 µL, 15 eq) was added. The reaction mixture was stirred at 60 °C for 2 hours. NaBH₃CN (29.37 mg, 467.33 µmol, 2 eq) was then added, and the mixture was stirred at 60 °C for an additional 2 hours. After completion of the reaction, the mixture was filtered, and the filtrate was concentrated under reduced pressure. The residue was purified by prep-HPLC (column: Waters XBridge C18, 150 * 50 mm * 10 um; mobile phase: [water (NH₄HCO₃)-ACN]; B%: 34%-64%, 10 min) to afford Compound 112-1 (47 mg, 31.8% yield) as a white solid.

LCMS m/z 600.3 [M+1]⁺.

¹H NMR (400 MHz, DMSO-d6) δ = 9.53 (s, 1H), 7.80 (br s, 1H), 7.57 (br d, J = 7.8 Hz, 1H), 7.43 - 7.21 (m, 3H), 7.12 (br d, J = 7.1 Hz, 1H), 7.01 (br d, J = 7.4 Hz, 1H), 6.87 - 6.76 (m, 2H), 6.69 (s, 1H), 5.58 (br s, 1H), 4.76 (br s, 1H), 4.12 (br s, 2H), 3.85 (s, 3H), 3.72 (br s, 2H), 3.12 (br d, J = 5.8 Hz, 2H), 2.53 (br t, J = 6.4 Hz, 2H), 2.17 (br d, J = 6.1 Hz, 1H), 2.07 (s, 1H), 1.97 (s, 3H), 1.92 (s, 3H), 1.78 (s, 3H), 1.75 - 1.64 (m, 1H).

### 25-3. Preparation of Compound 113, N-(3"-fluoro-4"-(((R)-3-hydroxypyrrolidin-1-yl)methyl)-5"-methoxy-2,2'-dimethyl-[1,1':3',1"-terphenyl]-3-yl)-4-hydroxy-4,5,6,7-tetrahydropyrazolo[1,5-a]pyridine-2-carboxamide

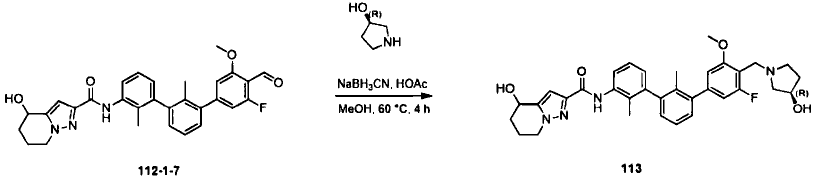

To a solution of N-[3-[3-(3-fluoro-4-formyl-5-methoxyphenyl)-2-methylphenyl]-2-methylphenyl]-4-hydroxy-4,5,6,7-tetrahydropyrazolo[1,5-a]pyridine-2-carboxamide (120 mg, 233.66 µmol, 1 eq) and (3R)-pyrrolidin-3-ol (203.56 mg, 2.34 mmol, 193.87 µL, 10 eq) in MeOH (3 mL), HOAc (210.47 mg, 3.50 mmol, 200.45 µL, 15 eq) was added. The reaction mixture was stirred at 60 °C for 2 hours. NaBH₃CN (29.37 mg, 467.32 µmol, 2 eq) was then added, and the mixture was stirred at 60 °C for an additional 2 hours. After completion of the reaction, the mixture was concentrated under reduced pressure, and the residue was purified by prep-HPLC (column: Waters XBridge C18, 150 * 50 mm * 10 um; mobile phase: [water (NH₄HCO₃)-ACN]; B%: 40%-70%, 10 min) to afford Compound 113 (31 mg, 21.5% yield) as a white solid.

LCMS m/z 585.3 [M+1]⁺.

¹H NMR (400 MHz, DMSO-d6) δ = 9.52 (br s, 1H), 7.57 (br d, J = 7.6 Hz, 1H), 7.40 - 7.23 (m, 3H), 7.12 (br d, J = 7.0 Hz, 1H), 7.01 (br d, J = 7.3 Hz, 1H), 6.87 - 6.76 (m, 2H), 6.69 (s, 1H), 5.58 (br d, J = 4.6 Hz, 1H), 4.81 - 4.60 (m, 2H), 4.13 (br s, 3H), 3.84 (s, 3H), 3.63 (br s, 2H), 2.79 - 2.54 (m, 2H), 2.46 - 2.27 (m, 2H), 2.23 - 2.10 (m, 1H), 1.97 (br s, 4H), 1.92 (br s, 4H), 1.83 - 1.66 (m, 1H), 1.47 (br d, J = 3.5 Hz, 1H).

### 25-4. Preparation of Compound 114, N-(3"-fluoro-4"-(((2-hydroxyethyl)amino)methyl)-5"-methoxy-2,2'-dimethyl-[1,1':3',1 "-terphenyl]-3-yl)-4-hydroxy-4,5,6,7-tetrahydropyrazolo[1,5-a]pyridine-2-carboxamide

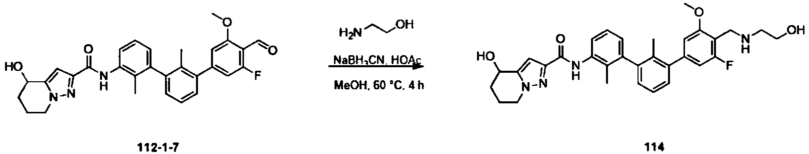

To a solution of N-[3-[3-(3-fluoro-4-formyl-5-methoxyphenyl)-2-methylphenyl]-2-methylphenyl]-4-hydroxy-4,5,6,7-tetrahydropyrazolo[1,5-a]pyridine-2-carboxamide (120 mg, 233.66 µmol, 1 eq) and 2-aminoethanol (142.73 mg, 2.34 mmol, 141.31 µL, 10 eq) in MeOH (3 mL), HOAc (210.47 mg, 3.50 mmol, 200.45 µL, 15 eq) was added. The reaction mixture was stirred at 60 °C for 2 hours. NaBH₃CN (29.37 mg, 467.32 µmol, 2 eq) was then added, and the mixture was stirred at 60 °C for an additional 2 hours. After completion of the reaction, the mixture was concentrated under reduced pressure, and the residue was purified by prep-HPLC (column: Waters XBridge C18, 150 * 50 mm * 10 um; mobile phase: [water (NH₄HCO₃)-ACN]; B%: 32%-62%, 10 min) to afford Compound 114 (42 mg, 30.5% yield) as a white solid.

LCMS m/z 559.3 [M+1]⁺.

¹H NMR (400 MHz, DMSO-d6) δ = 9.52 (br s, 1H), 7.56 (br s, 1H), 7.41 - 7.22 (m, 3H), 7.12 (br s, 1H), 7.01 (br d, J = 1.8 Hz, 1H), 6.82 (br s, 2H), 6.69 (br d, J = 1.3 Hz, 1H), 5.58 (br s, 1H), 4.76 (br d, J = 1.7 Hz, 1H), 4.49 (br s, 1H), 4.12 (br s, 2H), 3.85 (br s, 3H), 3.74 (br s, 2H), 2.56 (br s, 2H), 2.22 - 2.11 (m, 1H), 1.97 (br s, 3H), 1.92 (br s, 3H), 1.73 (br d, J = 2.2 Hz, 1H).

### 25-5. Preparation of Compound 115, 2-(((3-fluoro-3"-(4-((R)-3-hydroxypyrrolidin-1-yl)-4,5,6,7-tetrahydropyrazolo[1,5-a]pyridine-2-carboxamido)-5-methoxy-2',2"-dimethyl-[1,1':3',1"-terphenyl]-4-yl)methyl)amino)-2-methylpropanoic acid

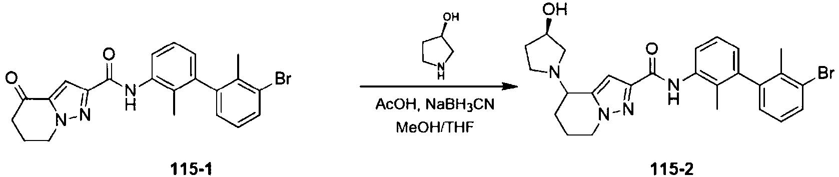

To a solution of N-[3-(3-bromo-2-methylphenyl)-2-methylphenyl]-4-oxo-6,7-dihydro-5H-pyrazolo[1,5-a]pyridine-2-carboxamide (650 mg, 1.48 mmol, 1 eq) and (3R)-pyrrolidin-3-ol (1.29 g, 14.83 mmol, 1.23 mL, 10 eq) in MeOH (20 mL)/THF (20 mL), HOAc (89.05 mg, 1.48 mmol, 84.89 µL, 1 eq) and NaBH₃CN (465.96 mg, 7.41 mmol, 5 eq) were added, and the reaction mixture was stirred at 50 °C for 12 hours. After completion of the reaction, the mixture was concentrated under reduced pressure, and the residue was purified by prep-HPLC (column: Waters XBridge Prep OBD C18, 150 * 40 mm * 10 um; mobile phase: [water (NH₄HCO₃)-ACN]; B%: 42%-72%, 15 min) to afford Compound 115-2 (503 mg, 63.9% yield) as a white solid.

LCMS m/z 511.2 [M+3]⁺.

¹H NMR (400 MHz, CHLOROFORM-d) δ 8.70 (s, 1H), 8.11 (t, J = 6.6 Hz, 1H), 7.58 (t, J = 5.0 Hz, 1H), 7.32 - 7.28 (m, 1H), 7.14 - 7.06 (m, 2H), 6.93 (d, J = 7.5 Hz, 1H), 6.85 (br s, 1H), 4.41 (br s, 1H), 4.30 - 4.12 (m, 2H), 3.94 (br s, 1H), 3.19 - 2.94 (m, 1H), 2.85 (br s, 1H), 2.66 (br s, 1H), 2.49 (br s, 1H), 2.22 (br d, J = 5.1 Hz, 2H), 2.16 - 2.13 (m, 3H), 2.05 (s, 6H), 1.87 (br s, 1H), 1.26 (s, 1H).

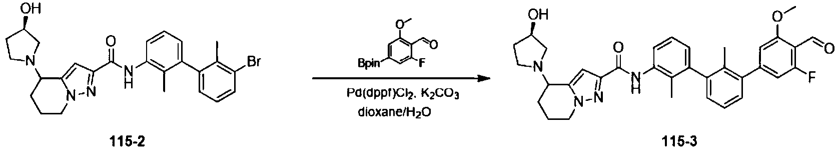

A mixture of N-[3-(3-bromo-2-methylphenyl)-2-methylphenyl]-4-[(3R)-3-hydroxypyrrolidin-1-yl]-4,5,6,7-tetrahydropyrazolo[1,5-a]pyridine-2-carboxamide (200 mg, 392.59 µmol, 1 eq), 2-fluoro-6-methoxy-4-(4,4,5,5-tetramethyl-1,3,2-dioxaborolan-2-yl)benzaldehyde (131.96 mg, 471.11 µmol, 1.2 eq), K₂CO₃ (162.77 mg, 1.18 mmol, 3 eq), and Pd(dppf)Cl₂ (28.73 mg, 39.26 µmol, 0.1 eq) in dioxane (2 mL)/H₃O (0.4 mL) was stirred at 90 °C for 3 hours under a nitrogen atmosphere. After completion of the reaction, the mixture was concentrated under reduced pressure, and the residue was purified by prep-TLC (SiO₂, Ethyl acetate:Methanol = 3:1) to afford Compound 115-3 (230 mg, 98.5% yield) as a white solid.

LCMS m/z 583.5 [M+1]⁺.

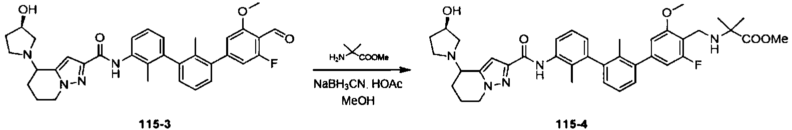

To a solution of N-[3-[3-(3-fluoro-4-formyl-5-methoxyphenyl)-2-methylphenyl]-2-methylphenyl]-4-[(3R)-3-hydroxypyrrolidin-1-yl]-4,5,6,7-tetrahydropyrazolo[1,5-a]pyridine-2-carboxamide (230 mg, 394.74 µmol, 1 eq) and methyl 2-amino-2-methylpropanoate (231.21 mg, 1.97 mmol, 5 eq) in MeOH (5 mL), NaBH₃CN (124.03 mg, 1.97 mmol, 5 eq) and AcOH (23.70 mg, 394.74 µmol, 22.60 µL, 1 eq) were added, and the reaction mixture was stirred at 60 °C for 12 hours. After completion of the reaction, the mixture was concentrated under reduced pressure, and the residue was purified by prep-TLC (SiO₂, Ethyl acetate: Methanol = 3:1) to afford Compound 115-4 (171 mg, 60.8% yield) as a white solid.

LCMS m/z 684.6 [M+1]⁺.

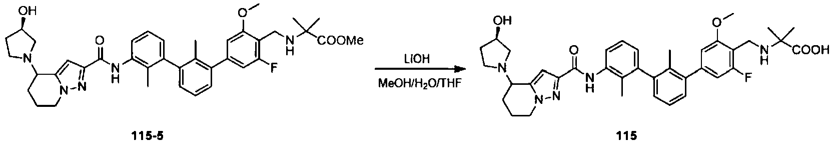

To a solution of methyl 2-[[2-fluoro-4-[3-[3-[[4-[(3R)-3-hydroxypyrrolidin-1-yl]-4,5,6,7-tetrahydropyrazolo[1,5-a]pyridine-2-carbonyl]amino]-2-methylphenyl]-2-methylphenyl]-6-methoxyphenyl]methylamino]-2-methylpropanoate (251 mg, 367.06 µmol, 1 eq) in MeOH (12 mL)/H₂O (1 mL)/THF (3 mL), LiOH·H₂O (46.21 mg, 1.10 mmol, 3 eq) was added. The reaction mixture was stirred at room temperature for 3 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 22-52% B over 9 min) to afford Compound 115 (184 mg, 74.0% yield) as a white solid.

LCMS m/z 670.6 [M+1]⁺.
¹H NMR (400 MHz, CHLOROFORM-d) δ 8.72 - 8.68 (m, 1H), 8.11 - 8.05 (m, 1H), 7.30 (s, 1H), 7.23 - 7.18 (m, 1H), 7.16 - 7.07 (m, 2H), 6.95 (br d, J = 7.9 Hz, 1H), 6.81 (s, 1H), 6.73 - 6.63 (m, 2H), 4.36 (br s, 1H), 4.26 - 4.08 (m, 2H), 3.98 (s, 3H), 3.91 (br s, 2H), 3.83 (br d, J = 6.1 Hz, 1H), 3.09 - 2.91 (m, 2H), 2.87 - 2.67 (m, 3H), 2.64 - 2.46 (m, 2H), 2.40 (br s, 1H), 2.17 (br d, J = 7.3 Hz, 1H), 2.05 (s, 3H), 2.03 - 1.94 (m, 3H), 1.89 (s, 3H), 1.83 - 1.72 (m, 1H), 1.48 (br s, 6H).

### [Preparation Example 26]

### Preparation of Compounds 116 to 119

### 26-1. Preparation of Compound 116, N-(4"-(((2-acetamidoethyl)amino)methyl)-2,2'-dichloro-3"-fluoro-5"-methoxy-[1,1':3',1"-terphenyl]-3-yl)-4-hydroxy-4,5,6,7-tetrahydropyrazolo[1,5-a]pyridine-2-carboxamide

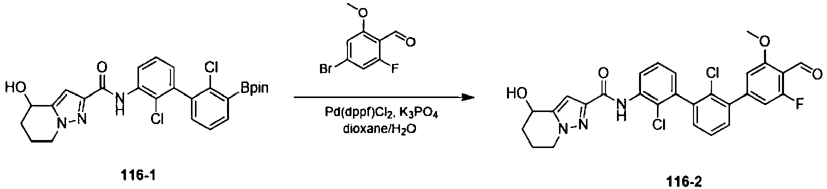

A mixture of N-[2-chloro-3-[2-chloro-3-(4,4,5,5-tetramethyl-1,3,2-dioxaborolan-2-yl)phenyl]phenyl]-4-hydroxy-4,5,6,7-tetrahydropyrazolo[1,5-a]pyridine-2-carboxamide (800 mg, 1.51 mmol, 1 eq), 4-bromo-2-fluoro-6-methoxybenzaldehyde (423.51 mg, 1.82 mmol, 1.2 eq), K₃PO₄ (964.42 mg, 4.54 mmol, 3 eq), and Pd(dppf)Cl₂ (110.82 mg, 151.45 µmol, 0.1 eq) in dioxane (10 mL)/H₂O (2 mL) was stirred at 60 °C for 16 hours under a nitrogen atmosphere. After completion of the reaction, the mixture was concentrated under reduced pressure, and the residue was purified by column chromatography (SiO₂, Petroleum ether/Ethyl acetate = 100/1 to 1/1) to afford Compound 116-2 (700 mg, 79.7% yield) as a yellow solid.

LCMS m/z 554.1 [M+1]⁺.

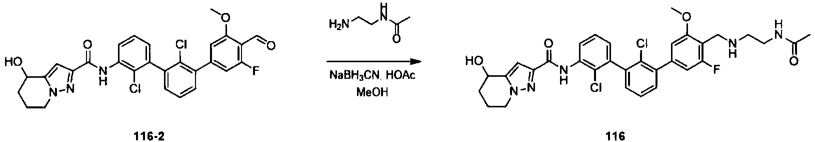

To a solution of N-[2-chloro-3-[2-chloro-3-(3-fluoro-4-formyl-5-methoxyphenyl)phenyl]phenyl]-4-hydroxy-4,5,6,7-tetrahydropyrazolo[1,5-a]pyridine-2-carboxamide (150 mg, 270.56 µmol, 1 eq) and N-(2-aminoethyl)acetamide (276.34 mg, 2.71 mmol, 258.26 µL, 10 eq) in MeOH (5 mL), HOAc (162.48 mg, 2.71 mmol, 154.74 µL, 10 eq) was added. The reaction mixture was stirred at room temperature for 30 minutes. NaBH₃CN (170.03 mg, 2.71 mmol, 10 eq) was then added, and the mixture was stirred at room temperature for 4 hours. After completion of the reaction, the mixture was concentrated under reduced pressure, and the residue was purified by reversed-phase HPLC (column: Waters XBridge C18, 150 * 50 mm, 10 um; mobile phase: [water (NH₄HCO₃)-ACN]; gradient: 35-65% B over 10 min) to afford Compound 116 (42 mg, 23.8% yield) as a yellow solid.

LCMS m/z 640.2 [M+1]⁺.

¹H NMR (400 MHz, DMSO-d6) δ = 9.55 (s, 1H), 8.27 (d, J = 8.3 Hz, 1H), 7.79 (s, 1H), 7.56 - 7.45 (m, 3H), 7.40 (t, J = 4.5 Hz, 1H), 7.19 (d, J = 7.5 Hz, 1H), 6.94 - 6.88 (m, 2H), 6.74 (s, 1H), 5.60 (d, J = 5.3 Hz, 1H), 4.76 (d, J = 5.5 Hz, 1H), 4.14 (s, 2H), 3.86 (s, 3H), 3.72 (s, 2H), 3.31 - 3.30 (m, 3H), 3.11 (d, J = 6.1 Hz, 2H), 2.33 (s, 2H), 2.16 (s, 1H), 2.01 (s, 1H), 1.77 (s, 3H).

### 26-2. Preparation of Compound 117, N-(2,2'-dichloro-3"-fluoro-4"-(((R)-3-hydroxypyrrolidin-1-yl)methyl)-5"-methoxy-[1,1':3',1"-terphenyl]-3-yl)-4-hydroxy-4,5,6,7-tetrahydropyrazolo[1,5-a]pyridine-2-carboxamide

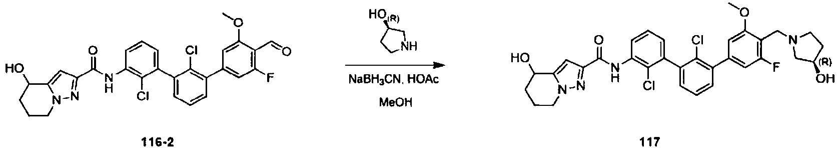

To a solution of N-[2-chloro-3-[2-chloro-3-(3-fluoro-4-formyl-5-methoxyphenyl)phenyl]phenyl]-4-hydroxy-4,5,6,7-tetrahydropyrazolo[1,5-a]pyridine-2-carboxamide (150 mg, 270.56 µmol, 1 eq) and (3R)-pyrrolidin-3-ol (235.72 mg, 2.71 mmol, 224.49 µL, 10 eq) in MeOH (5 mL), HOAc (162.48 mg, 2.71 mmol, 154.74 µL, 10 eq) was added. The reaction mixture was stirred at room temperature for 30 minutes. NaBH₃CN (170.03 mg, 2.71 mmol, 10 eq) was then added, and the mixture was stirred at room temperature for 4 hours. After completion of the reaction, the mixture was concentrated under reduced pressure, and the residue was purified by reversed-phase HPLC (column: Waters XBridge C18, 150 * 50 mm * 10 um; mobile phase: [water (NH₄HCO₃)-ACN]; gradient: 35-65% B over 10 min) to afford Compound 117 (46 mg, 26.8% yield) as a yellow solid.

LCMS m/z 625.2 [M+1]⁺.

¹H NMR (400 MHz, DMSO-d6) δ = 9.56 (s, 1H), 8.29 - 8.25 (m, 1H), 7.58 - 7.46 (m, 3H), 7.40 (dd, J = 3.0, 5.8 Hz, 1H), 7.19 (d, J = 7.5 Hz, 1H), 6.94 - 6.89 (m, 2H), 6.74 (s, 1H), 5.60 (d, J = 5.4 Hz, 1H), 4.76 (d, J = 5.5 Hz, 1H), 4.64 (d, J = 4.6 Hz, 1H), 4.14 (s, 2H), 3.85 (s, 3H), 3.63 (s, 2H), 2.74 - 2.66 (m, 1H), 2.62 - 2.56 (m, 1H), 2.33 (s, 2H), 2.17 (s, 1H), 2.04 - 1.86 (m, 4H), 1.73 (s, 1H), 1.47 (d, J = 3.8 Hz, 1H).

### 26-3. Preparation of Compound 118, N-(2,2'-dichloro-3"-fluoro-4"-(((2-hydroxyethyl)amino)methyl)-5"-methoxy-[1,1':3',1"-terphenyl]-3-yl)-4-hydroxy-4,5,6,7-tetrahydropyrazolo[1,5-a]pyridine-2-carboxamide

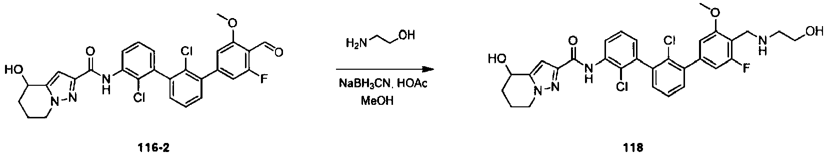

To a solution of N-[2-chloro-3-[2-chloro-3-(3-fluoro-4-formyl-5-hydroxyphenyl)phenyl]phenyl]-4-hydroxy-4,5,6,7-tetrahydropyrazolo[1,5-a]pyridine-2-carboxamide (100 mg, 185.06 µmol, 1 eq) and 2-aminoethanol (113.04 mg, 1.85 mmol, 111.92 µL, 10 eq) in MeOH (5 mL), HOAc (111.13 mg, 1.85 mmol, 105.84 µL, 10 eq) was added. The reaction mixture was stirred at room temperature for 30 minutes. NaBH₃CN (116.29 mg, 1.85 mmol, 10 eq) was then added, and the mixture was stirred at room temperature for 4 hours. After completion of the reaction, the mixture was concentrated under reduced pressure, and the residue was purified by reversed-phase HPLC (column: Waters XBridge C18, 150 * 50 mm, 10 um; mobile phase: [water (NH₄HCO₃)-ACN]; gradient: 35-65% B over 10 min) to afford Compound 118 (62 mg, 57.2% yield) as a yellow solid.

LCMS m/z 599.2 [M+1]⁺.

¹H NMR (400 MHz, DMSO-d6) δ = 9.56 (s, 1H), 8.28 (d, J = 7.7 Hz, 1H), 7.57 - 7.46 (m, 3H), 7.43 - 7.37 (m, 1H), 7.19 (dd, J = 1.4, 7.6 Hz, 1H), 6.95 - 6.89 (m, 2H), 6.74 (s, 1H), 5.60 (d, J = 5.3 Hz, 1H), 4.79 - 4.73 (m, 1H), 4.47 (s, 1H), 4.18 - 4.09 (m, 2H), 3.87 (s, 3H), 3.74 (s, 2H), 3.48 - 3.42 (m, 2H), 2.58 - 2.54 (m, 2H), 2.39 - 2.27 (m, 1H), 2.16 (dd, J = 5.5, 13.2 Hz, 1H), 2.06 - 1.87 (m, 2H), 1.80 - 1.65 (m, 1H).

### 26-4. Preparation of Compound 119, N-(2,2'-dichloro-3"-fluoro-5"-methoxy-4"-(((((S)-5-oxopyrrolidin-2-yl)methyl)amino)methyl)-[1,1':3',1"-terphenyl]-3-yl)-4-hydroxy-4,5,6,7-tetrahydropyrazolo[1,5-a]pyridine-2-carboxamide

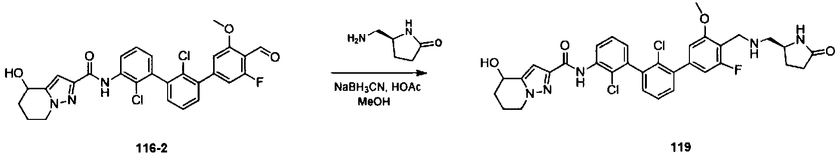

To a solution of N-[2-chloro-3-[2-chloro-3-(3-fluoro-4-formyl-5-methoxyphenyl)phenyl]phenyl]-4-hydroxy-4,5,6,7-tetrahydropyrazolo[1,5-a]pyridine-2-carboxamide (150 mg, 270.56 µmol, 1 eq) and 5-(aminomethyl)pyrrolidin-2-one (308.84 mg, 2.71 mmol, 10 eq) in MeOH (5 mL), HOAc (162.48 mg, 2.71 mmol, 154.74 µL, 10 eq) was added. The reaction mixture was stirred at room temperature for 30 minutes. NaBH₃CN (170.03 mg, 2.71 mmol, 10 eq) was then added, and the mixture was stirred at room temperature for 4 hours. After completion of the reaction, the mixture was concentrated under reduced pressure, and the residue was purified by reversed-phase HPLC (column: Waters XBridge C18, 150 * 50 mm * 10 um; mobile phase: [water (NH₄HCO₃)-ACN]; gradient: 35-65% B over 10 min) to afford Compound 119 (36 mg, 20.3% yield) as a yellow solid.

LCMS m/z 652.2 [M+1]⁺.

¹H NMR (400 MHz, DMSO-d6) δ = 9.56 (s, 1H), 8.28 (d, J = 8.1 Hz, 1H), 7.68 (s, 1H), 7.58 - 7.46 (m, 3H), 7.41 (t, J = 4.5 Hz, 1H), 7.20 (d, J = 7.3 Hz, 1H), 6.98 - 6.87 (m, 2H), 6.75 (s, 1H), 5.61 (d, J = 5.3 Hz, 1H), 4.77 (d, J = 5.4 Hz, 1H), 4.15 (s, 2H), 3.88 (s, 3H), 3.82 - 3.69 (m, 2H), 3.59 (s, 1H), 2.23 - 2.14 (m, 1H), 2.13 - 2.03 (m, 3H), 1.93 (d, J = 5.9 Hz, 2H), 1.78 - 1.59 (m, 2H).

### [Preparation Example 27]

### Preparation of Compounds 120, 122, 124, and 126

### 27-1. Preparation of Compound 120, N-(4"-(((2-acetamidoethyl)amino)methyl)-2,2'-dichloro-3"-fluoro-5"-methoxy-[1,1':3',1"-terphenyl]-3-yl)-4-((2-hydroxyethyl)amino)-4,5,6,7-tetrahydropyrazolo[1,5-a]pyridine-2-carboxamide

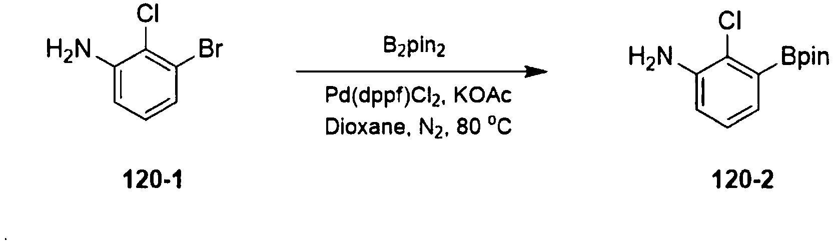

A mixture of 3-Bromo-2-chloroaniline (10 g, 48.43 mmol, 1 eq), 4,4,5,5-tetramethyl-2-(4,4,5,5-tetramethyl-1,3,2-dioxaborolan-2-yl)-1,3,2-dioxaborolane (24.60 g, 96.87 mmol, 2 eq), Pd(dppf)Cl₂ (3.54 g, 4.84 mmol, 0.1 eq), and KOAc (9.51 g, 96.87 mmol, 2 eq) in dioxane (150 mL) was stirred at 80 °C for 24 hours under a nitrogen atmosphere. After completion of the reaction, the mixture was filtered, and the filtrate was concentrated under reduced pressure. The residue was purified by column chromatography (SiO₂, Petroleum ether/Ethyl acetate = 100/1 to 10/1) to afford Compound 120-2 (10.1 g, 74.6% yield) as a white solid.

LCMS m/z 254.1 [M+1]⁺.

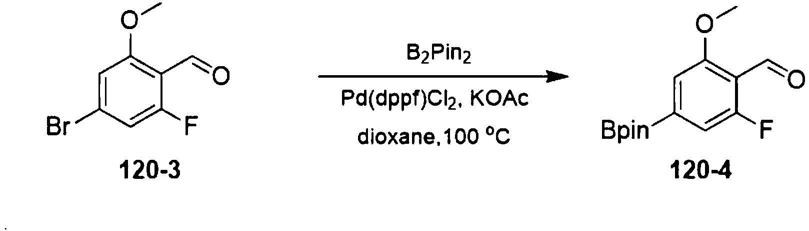

A mixture of 4,4,5,5-Tetramethyl-2-(4,4,5,5-tetramethyl-1,3,2-dioxaborolan-2-yl)-1 ,3,2-dioxaborolane (655.16 mg, 2.58 mmol, 2 eq), 4-bromo-2-fluoro-6-methoxybenzaldehyde (300 mg, 1.29 mmol, 1 eq), KOAc (253.20 mg, 2.58 mmol, 2 eq), and Pd(dppf)Cl₂ CH₂Cl₂ (105.35 mg, 129.00 µmol, 0.1 eq) in dioxane (10 mL) was stirred at 80 °C for 12 hours under a nitrogen atmosphere. After completion of the reaction, the mixture was filtered, and the filtrate was concentrated under reduced pressure. The residue was purified by prep-TLC (SiO₂, DMC:MeOH = 3:1) to afford Compound 120-4 (264 mg, 73.0% yield) as a brown solid.

¹H NMR (400 MHz, CHLOROFORM-d) δ 10.57 - 10.56 (m, 1H), 7.37 (s, 1H), 7.26 - 7.21 (m, 1H), 4.09 - 4.08 (m, 3H), 1.47 (s, 12H).

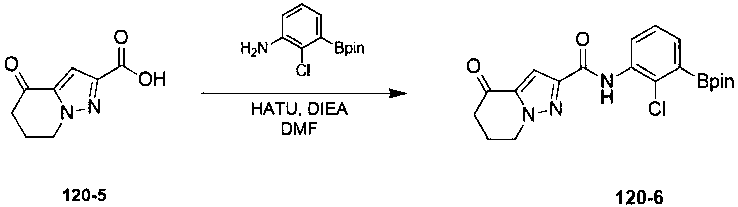

To a solution of 2-Chloro-3-(4,4,5,5-tetramethyl-1,3,2-dioxaborolan-2-yl)aniline (6.40 g, 25.23 mmol, 1 eq) and 4-oxo-6,7-dihydro-5H-pyrazolo[1,5-a]pyridine-2-carboxylic acid (5 g, 27.75 mmol, 1.1 eq) in DMF (110 mL), HATU (14.39 g, 37.85 mmol, 1.5 eq) and DIPEA (9.78 g, 75.69 mmol, 13.18 mL, 3 eq) were added, and the resulting mixture was stirred at room temperature for 12 hours. After completion of the reaction, the mixture was diluted with water (1000 mL), and the precipitated solid was collected by filtration to afford Compound 120-6 (5 g, 28.61% yield), which was used in the next reaction without further purification.

LCMS m/z 416.3 [M+1]⁺.

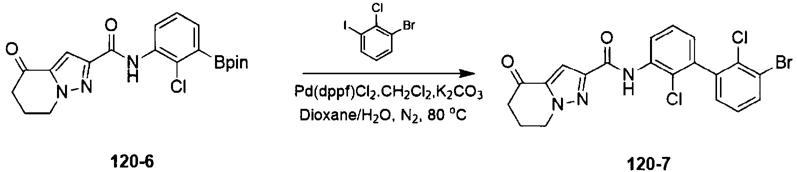

To a solution of N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (3 g, 7.22 mmol, 1 eq) and 1-bromo-2-chloro-3-iodobenzene (2.29 g, 7.22 mmol, 1 eq) in dioxane (60 mL)/H₂O (5 mL), Pd(dppf)Cl₂·CH₂Cl₂ (589.38 mg, 721.71 µmol, 0.1 eq) and K₂CO₃ (2.99 g, 21.65 mmol, 3 eq) were added, and the resulting mixture was stirred at 80 °C for 12 hours under a nitrogen atmosphere. After completion of the reaction, the mixture was diluted with H₂O (200 mL) and extracted with ethyl acetate (120 mL x 2). The organic layer was washed with brine (60 mL), dried over Na₂SO₄, and concentrated under reduced pressure to afford Compound 120-7 (1.5 g, 36.0% yield) as a brown solid, which was used in the next reaction without further purification.

LCMS m/z 479.8 [M+1]⁺.

¹H NMR (400 MHz, DMSO-d6) δ 9.83 - 9.80 (m, 1H), 8.11 - 8.06 (m, 1H), 7.87 (dd, J = 3.1, 6.3 Hz, 1H), 7.52 - 7.47 (m, 1H), 7.41 - 7.39 (m, 2H), 7.30 - 7.28 (m, 1H), 7.24 - 7.20 (m, 1H), 4.47 (br t, J = 5.9 Hz, 2H), 2.73 - 2.69 (m, 2H), 2.36 (br s, 2H).

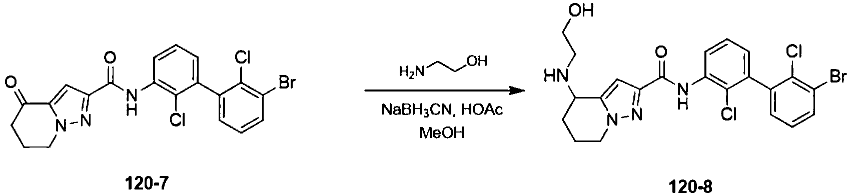

To a solution of N-[3-(3-bromo-2-chloro-phenyl)-2-chlorophenyl]-4-oxo-6,7-dihydro-5H-pyrazolo[1,5-a]pyridine-2-carboxamide (500 mg, 1.04 mmol, 1 eq) and 2-aminoethanol (637.40 mg, 10.44 mmol, 631.09 µL, 10 eq) in MeOH (80 mL), AcOH (1.25 g, 20.87 mmol, 1.19 mL, 20 eq) and NaBH₃CN (327.88 mg, 5.22 mmol, 5 eq) were added, and the reaction mixture was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 44-74% B over 9 min) to afford Compound 120-8 (375 mg, 68.5% yield) as a white solid.

LCMS m/z 525.1 [M+3]⁺.

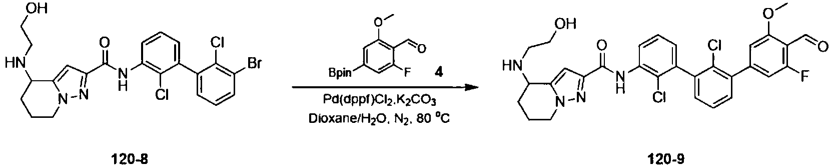

A mixture of N-[3-(3-bromo-2-chloro-phenyl)-2-chlorophenyl]-4-(2-hydroxyethylamino)-4,5,6,7-tetrahydropyrazolo[1,5-a]pyridine-2-carboxamide (350 mg, 667.64 µmol, 1 eq), 2-fluoro-6-methoxy-4-(4,4,5,5-tetramethyl-1,3,2-dioxaborolan-2-yl)benzaldehyde (243.11 mg, 867.93 µmol, 1.3 eq), Pd(dppf)Cl₂ (48.85 mg, 66.76 µmol, 0.1 eq), and K₂CO₃ (276.82 mg, 2.00 mmol, 3 eq) in H₂O (15 mL)/dioxane (60 mL) was stirred at 90 °C for 12 hours under a nitrogen atmosphere. After completion of the reaction, the mixture was concentrated under reduced pressure, and the residue was purified by column chromatography (SiO₂, Ethyl acetate:Methanol = 100/1 to 1/1) to afford Compound 120-9 (340 mg, 85.2% yield) as a brown solid.

LCMS m/z 597.2 [M+1]⁺.

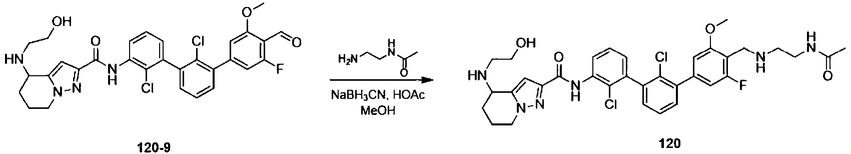

A mixture of N-[2-chloro-3-[2-chloro-3-(3-fluoro-4-formyl-5-methoxyphenyl)phenyl]phenyl]-4-(2-hydroxyethylamino)-4,5,6,7-tetrahydropyrazolo[1,5-a]pyridine-2-carboxamide (80 mg, 133.90 µmol, 1 eq), N-(2-aminoethyl)acetamide (136.76 mg, 1.34 mmol, 127.81 µL, 10 eq), NaBH₃CN (42.07 mg, 669.50 µmol, 5 eq), and HOAc (160.82 mg, 2.68 mmol, 153.16 µL, 20 eq) in MeOH (15 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 30-60% B over 9 min) to afford Compound 120 (39 mg, 42.18% yield) as a white solid.

LCMS m/z 683.3 [M+1]⁺.

¹H NMR (400 MHz, DMSO-d6) δ 9.54 (s, 1H), 8.27 (d, J = 8.4 Hz, 1H), 7.78 (t, J = 5.8 Hz, 1H), 7.56 - 7.45 (m, 3H), 7.40 (t, J = 4.6 Hz, 1H), 7.19 (dd, J = 1.5, 7.6 Hz, 1H), 6.95 - 6.87 (m, 2H), 6.77 (s, 1H), 4.51 (br s, 1H), 4.19 - 4.09 (m, 2H), 3.91 - 3.87 (m, 1H), 3.86 (s, 2H), 3.72 (s, 2H), 3.50 - 3.44 (m, 2H), 3.15 - 3.09 (m, 2H), 2.71 - 2.65 (m, 2H), 2.56 - 2.53 (m, 2H), 2.39 - 2.27 (m, 1H), 2.23 - 2.14 (m, 1H), 2.06 - 1.97 (m, 1H), 1.91 (br d, J = 7.0 Hz, 1H), 1.78 (s, 3H), 1.73 - 1.64 (m, 1H).

### 27-2. Preparation of Compound 122, N-(2,2'-dichloro-3"-fluoro-4"-(((R)-3-hydroxypyrrolidin-1-yl)methyl)-5"-methoxy-[1,1':3',1"-terphenyl]-3-yl)-4-((2-hydroxyethyl)amino)-4,5,6,7-tetrahydropyrazolo[1,5-a]pyridine-2-carboxamide

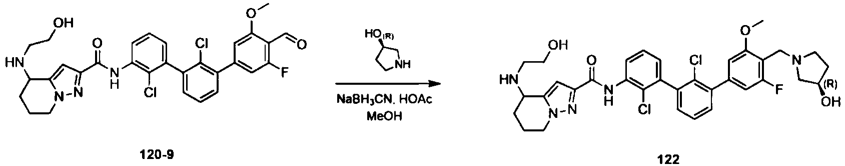

A mixture of N-[2-chloro-3-[2-chloro-3-(3-fluoro-4-formyl-5-methoxyphenyl)phenyl]phenyl]-4-(2-hydroxyethylamino)-4,5,6,7-tetrahydropyrazolo[1,5-a]pyridine-2-carboxamide (80 mg, 133.90 µmol, 1 eq), (3R)-pyrrolidin-3-ol (116.65 mg, 1.34 mmol, 111.10 µL, 10 eq), NaBH₃CN (42.07 mg, 669.50 µmol, 5 eq), and HOAc (160.81 mg, 2.68 mmol, 153.16 µL, 20 eq) in MeOH (15 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 35-65% B over 9 min) to afford Compound 122 (11 mg, 11.9% yield) as a white solid.

LCMS m/z 668.3 [M+1]⁺.

¹H NMR (400 MHz, DMSO-d6) δ 9.54 (s, 1H), 8.27 (d, J = 7.7 Hz, 1H), 7.55 - 7.52 (m, 2H), 7.51 - 7.45 (m, 1H), 7.40 (dd, J = 3.1, 6.1 Hz, 1H), 7.19 (dd, J = 1.6, 7.7 Hz, 1H), 6.94 - 6.88 (m, 2H), 6.77 (s, 1H), 4.63 (d, J = 4.5 Hz, 1H), 4.51 (s, 1H), 4.14 (br s, 3H), 3.85 (s, 2H), 3.63 (br s, 1H), 3.47 (q, J = 5.2 Hz, 2H), 3.37 - 3.35 (m, 2H), 2.67 (br d, J = 1.9 Hz, 3H), 2.59 (br d, J = 7.3 Hz, 1H), 2.43 (br s, 1H), 2.35 - 2.30 (m, 3H), 1.99 - 1.85 (m, 3H).

### 27-3. Preparation of Compound 124, N-(2,2'-dichloro-3"-fluoro-4"-(((2-hydroxyethyl)amino)methyl)-5"-methoxy-[1,1':3',1"-terphenyl]-3-yl)-4-((2-hydroxyethyl)amino)-4,5,6,7-tetrahydropyrazolo[1,5-a]pyridine-2-carboxamide

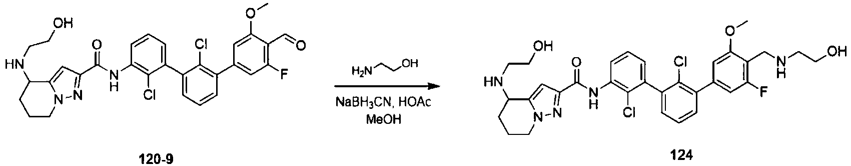

A mixture of N-[2-chloro-3-[2-chloro-3-(3-fluoro-4-formyl-5-methoxyphenyl)phenyl]phenyl]-4-(2-hydroxyethylamino)-4,5,6,7-tetrahydropyrazolo[1,5-a]pyridine-2-carboxamide (80 mg, 133.90 µmol, 1 eq), 2-aminoethanol (81.79 mg, 1.34 mmol, 80.98 µL, 10 eq), NaBH₃CN (42.07 mg, 669.50 µmol, 5 eq), and HOAc (160.81 mg, 2.68 mmol, 153.16 µL, 20 eq) in MeOH (15 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 30-60% B over 9 min) to afford Compound 124 (36 mg, 41.4% yield) as a white solid.

LCMS m/z 642.3 [M+1]⁺.

¹H NMR (400 MHz, DMSO-d6) δ 9.54 (s, 1H), 8.27 (dd, J = 1.4, 8.3 Hz, 1H), 7.55 - 7.46 (m, 3H), 7.40 (t, J = 4.6 Hz, 1H), 7.19 (dd, J = 1.5, 7.6 Hz, 1H), 6.94 - 6.88 (m, 2H), 6.77 (s, 1H), 4.51 (br s, 2H), 4.19 - 4.11 (m, 2H), 3.87 (s, 3H), 3.75 (s, 1H), 3.50 - 3.43 (m, 4H), 2.70 - 2.64 (m, 3H), 2.57 (t, J = 5.6 Hz, 2H), 2.34 - 2.31 (m, 1H), 2.19 (br d, J = 7.9 Hz, 1H), 2.01 (br s, 1H), 1.95 - 1.86 (m, 1H), 1.74 - 1.63 (m, 1H).

### 27-4. Preparation of Compound 126, N-(2,2'-dichloro-3"-fluoro-5"-methoxy-4"-(((((S)-5-oxopyrrolidin-2-yl)methyl)amino)methyl)-[1,1':3',1"-terphenyl]-3-yl)-4-((2-hydroxyethyl)amino)-4,5,6,7-tetrahydropyrazolo[1,5-a]pyridine-2-carboxamide

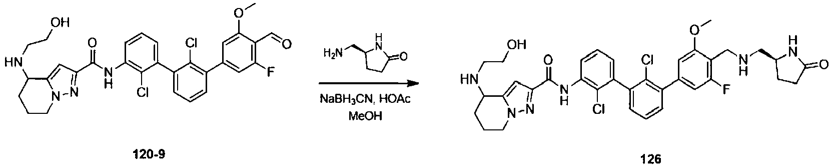

A mixture of N-[2-chloro-3-[2-chloro-3-(3-fluoro-4-formyl-5-methoxyphenyl)phenyl]phenyl]-4-(2-hydroxyethylamino)-4,5,6,7-tetrahydropyrazolo[1,5-a]pyridine-2-carboxamide (80 mg, 133.90 µmol, 1 eq), (5S)-5-(aminomethyl)pyrrolidin-2-one (152.84 mg, 1.34 mmol, 10 eq), NaBH₃CN (42.07 mg, 669.50 µmol, 5 eq), and HOAc (160.81 mg, 2.68 mmol, 153.16 µL, 20 eq) in MeOH (15 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 30-60% B over 9 min) to afford Compound 126 (60 mg, 63.1% yield) as a white solid.

LCMS m/z 695.4 [M+1]⁺.

¹H NMR (400 MHz, DMSO-d6) δ 9.54 (s, 1H), 8.27 (d, J = 7.8 Hz, 1H), 7.66 (s, 1H), 7.55 - 7.46 (m, 3H), 7.40 (t, J = 4.6 Hz, 1H), 7.19 (d, J = 7.8 Hz, 1H), 6.94 - 6.88 (m, 2H), 6.77 (s, 1H), 4.51 (t, J = 5.4 Hz, 1H), 4.18 - 4.09 (m, 2H), 3.87 (s, 3H), 3.75 (br s, 1H), 3.59 (br s, 1H), 3.47 (q, J = 5.5 Hz, 2H), 2.67 (br d, J = 1.9 Hz, 3H), 2.34 - 2.32 (m, 1H), 2.17 (br s, 1H), 2.13 - 2.01 (m, 5H), 1.95 - 1.87 (m, 1H), 1.73 - 1.62 (m, 2H).

### [Preparation Example 28]

### Preparation of Compounds 121, 123, 125, 127, and 128

### 28-1. Preparation of Compound 121, N-(4"-(((2-acetamidoethyl)amino)methyl)-2,2'-dichloro-3"-fluoro-5"-methoxy-[1,1':3',1"-terphenyl]-3-yl)-4-((R)-3-hydroxypyrrolidin-1-yl)-4,5,6,7-tetrahydropyrazolo[1,5-a]pyridine-2-carboxamide

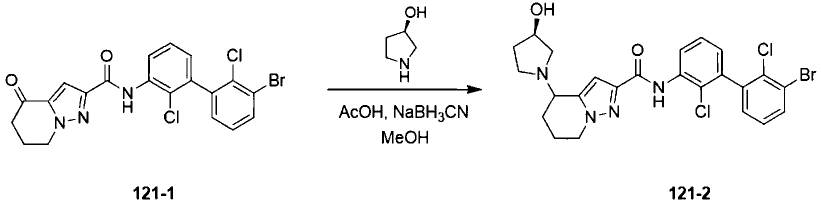

To a solution of N-[3-(3-bromo-2-chloro-phenyl)-2-chlorophenyl]-4-oxo-6,7-dihydro-5H-pyrazolo[1,5-a]pyridine-2-carboxamide (1 g, 2.09 mmol, 1 eq) and (3R)-pyrrolidin-3-ol (909.10 mg, 10.44 mmol, 865.81 µL, 5 eq) in MeOH (15 mL), AcOH (1.25 g, 20.87 mmol, 1.19 mL, 10 eq) and NaBH₃CN (655.76 mg, 10.44 mmol, 5 eq) were added, and the reaction mixture was stirred at 60 °C for 12 hours. After completion of the reaction, the mixture was concentrated under reduced pressure, and the residue was purified by reversed-phase HPLC (0.1% FA condition; column: Phenomenex Luna C18, 250 * 70 mm, 10 um; mobile phase: water (FA)-ACN; gradient: 35-65% B over 13 min) to afford Compound 121-2 (674 mg, 56.3% yield) as a white solid.

LCMS m/z 551.2 [M+1]⁺.

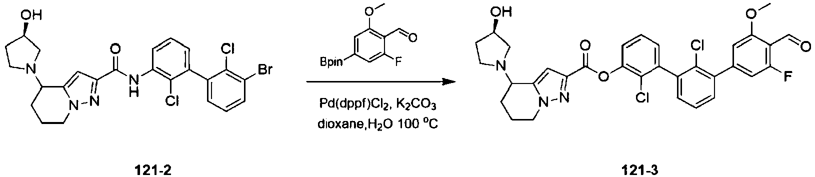

To a solution of N-[3-(3-bromo-2-chloro-phenyl)-2-chlorophenyl]-4-[(3R)-3-hydroxypyrrolidin-1-yl]-4,5,6,7-tetrahydropyrazolo[1,5-a]pyridine-2-carboxamide (670 mg, 1.22 mmol, 1 eq) and 2-fluoro-6-methoxy-4-(4,4,5,5-tetramethyl-1,3,2-dioxaborolan-2-yl)benzaldehyde (545.67 mg, 1.95 mmol, 1.6 eq) in dioxane (10 mL)/H₂O (1.5 mL), K₂CO₃ (504.84 mg, 3.65 mmol, 3 eq) and Pd(dppf)Cl₂ (44.55 mg, 60.88 µmol, 0.05 eq) were added, and the resulting mixture was stirred at 90 °C for 4 hours. After completion of the reaction, the mixture was concentrated under reduced pressure, and the residue was purified by column chromatography (SiO₂, Ethyl acetate:Methanol = 2:1) to afford Compound 121-3 (633 mg, 65.0% yield) as a yellow liquid.

LCMS m/z 623.2 [M+1]⁺.

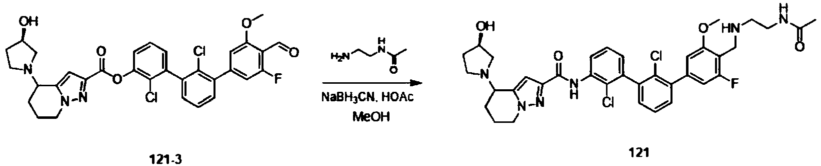

To a solution of [2-chloro-3-[2-chloro-3-(3-fluoro-4-formyl-5-methoxyphenyl)phenyl]phenyl] 4-[(3R)-3-hydroxypyrrolidin-1-yl]-4,5,6,7-tetrahydropyrazolo[1,5-a]pyridine-2-carboxylate (150 mg, 240.20 µmol, 1 eq) and N-(2-aminoethyl)acetamide (245.33 mg, 2.40 mmol, 230.14 µL, 10 eq) in MeOH (15 mL), NaBH₃CN (75.47 mg, 1.20 mmol, 5 eq) and AcOH (144.24 mg, 2.40 mmol, 137.50 µL, 10 eq) were added, and the resulting mixture was stirred at 60 °C for 12 hours. After completion of the reaction, the mixture was concentrated under reduced pressure, and the residue was purified by reversed-phase HPLC (0.1% NH₃·H₂O condition; column: Waters XBridge, 150 * 25 mm * 5 um; mobile phase: [water (NH₄HCO₃)-ACN]; gradient: 35-65% B over 9 min) to afford Compound 121 (60 mg, 35.0% yield) as a white solid.

LCMS m/z 709.3 [M+1]⁺.

¹H NMR (400 MHz, DMSO-d6) δ ppm 1.49 - 1.62 (m, 1 H) 1.72 - 1.79 (m, 3 H) 1.84 - 1.91 (m, 3 H) 2.15 - 2.27 (m, 1 H) 2.38 - 2.45 (m, 1 H) 2.55 - 2.62 (m, 1 H) 2.63 - 2.71 (m, 1 H) 2.72 - 2.80 (m, 1 H) 2.81 - 2.92 (m, 1 H) 3.07 - 3.17 (m, 5 H) 3.67 - 3.74 (m, 2 H) 3.81 - 3.87 (m, 3 H) 4.06 - 4.20 (m, 4 H) 4.66 - 4.74 (m, 1 H) 6.68 - 6.74 (m, 1 H) 6.85 - 6.92 (m, 2 H) 7.14 - 7.19 (m, 1 H) 7.35 - 7.40 (m, 1 H) 7.43 - 7.48 (m, 1 H) 7.49 - 7.53 (m, 2 H) 7.73 - 7.80 (m, 1 H) 8.22 - 8.27 (m, 1 H) 9.50 - 9.55 (m, 1 H)

### 28-2. Preparation of Compound 123, N-(2,2'-dichloro-3"-fluoro-4"-(((R)-3-hydroxypyrrolidin-1-yl)methyl)-5"-methoxy-[1,1':3',1 "-terphenyl]-3-yl)-4-((R)-3-hydroxypyrrolidin-1-yl)-4,5,6,7-tetrahydropyrazolo[1,5-a]pyridine-2-carboxamide

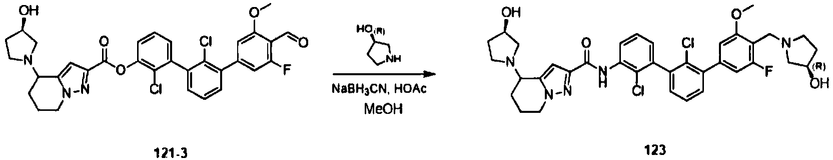

To a solution of [2-chloro-3-[2-chloro-3-(3-fluoro-4-formyl-5-methoxyphenyl)phenyl]phenyl] 4-[(3R)-3-hydroxypyrrolidin-1-yl]-4,5,6,7-tetrahydropyrazolo[1,5-a]pyridine-2-carboxylate (150 mg, 240.20 µmol, 1 eq) and (3R)-pyrrolidin-3-ol (209.26 mg, 2.40 mmol, 199.68 µL, 10 eq) in MeOH (15 mL), NaBH₃CN (75.47 mg, 1.20 mmol, 5 eq) and AcOH (144.24 mg, 2.40 mmol, 137.50 µL, 10 eq) were added, and the resulting mixture was stirred at 50 °C for 12 hours. After completion of the reaction, the mixture was concentrated under reduced pressure, and the residue was purified by reversed-phase HPLC (0.1% NH₃·H₂O condition; column: Waters XBridge, 150 * 25 mm * 5 um; mobile phase: [water (NH₄HCO₃)-ACN]; gradient: 42-72% B over 9 min) to afford Compound 123 (17 mg, 10.0% yield) as a white solid.

LCMS m/z 694.3 [M+1]⁺.

¹H NMR (400 MHz, DMSO-d6) δ ppm 1.43 - 1.51 (m, 1 H) 1.52 - 1.61 (m, 1 H) 1.86 - 1.90 (m, 2 H) 1.91 - 1.99 (m, 2 H) 2.05 - 2.08 (m, 1 H) 2.18 - 2.28 (m, 1 H) 2.30 - 2.35 (m, 1 H) 2.40 - 2.47 (m, 2 H) 2.55 - 2.65 (m, 2 H) 2.66 - 2.73 (m, 2 H) 2.74 - 2.81 (m, 1 H) 2.82 - 2.94 (m, 1 H) 3.55 - 3.68 (m, 2 H) 3.82 - 3.87 (m, 3 H) 4.07 - 4.12 (m, 1 H) 4.13 - 4.19 (m, 3 H) 4.61 - 4.66 (m, 1 H) 4.68 - 4.74 (m, 1 H) 6.69 - 6.76 (m, 1 H) 6.86 - 6.94 (m, 2 H) 7.16 - 7.22 (m, 1 H) 7.37 - 7.43 (m, 1 H) 7.45 - 7.50 (m, 1 H) 7.52 - 7.57 (m, 2 H) 8.22 - 8.30 (m, 1 H) 9.50 - 9.59 (m, 1 H)

### 28-3. Preparation of Compound 125, N-(2,2'-dichloro-3"-fluoro-4"-(((2-hydroxyethyl)amino)methyl)-5"-methoxy-[1,1':3',1"-terphenyl]-3-yl)-4-((R)-3-hydroxypyrrolidin-1-yl)-4,5,6,7-tetrahydropyrazolo[1,5-a]pyridine-2-carboxamide

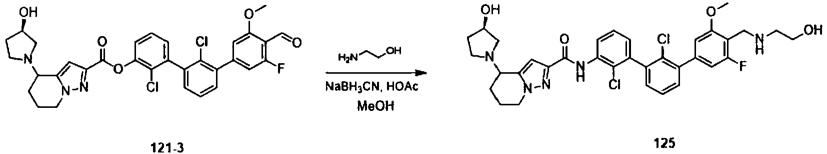

To a solution of [2-chloro-3-[2-chloro-3-(3-fluoro-4-formyl-5-methoxyphenyl)phenyl]phenyl] 4-[(3R)-3-hydroxypyrrolidin-1-yl]-4,5,6,7-tetrahydropyrazolo[1,5-a]pyridine-2-carboxylate (150 mg, 240.20 µmol, 1 eq) and 2-aminoethanol (146.72 mg, 2.40 mmol, 144.98 µL, 10 eq) in MeOH (15 mL), NaBH₃CN (75.47 mg, 1.20 mmol, 5 eq) and AcOH (144.24 mg, 2.40 mmol, 137.50 µL, 10 eq) were added, and the resulting mixture was stirred at 50 °C for 12 hours. After completion of the reaction, the mixture was concentrated under reduced pressure, and the residue was purified by reversed-phase HPLC (0.1% NH₃·H₂O condition; column: Waters XBridge, 150 * 25 mm * 5 um; mobile phase: [water (NH₄HCO₃)-ACN]; gradient: 35-65% B over 9 min) to afford Compound 125 (34 mg, 20.7% yield) as a white solid.

LCMS m/z 668.3 [M+1]⁺.

¹H NMR (400 MHz, DMSO-d6) δ ppm 1.51 - 1.61 (m, 1 H) 1.84 - 1.92 (m, 3 H) 2.04 - 2.08 (m, 1 H) 2.17 - 2.28 (m, 1 H) 2.38 - 2.46 (m, 1 H) 2.63 (br s, 1 H) 2.73 - 2.81 (m, 1 H) 2.83 - 2.94 (m, 1 H) 3.14 - 3.19 (m, 3 H) 3.42 - 3.48 (m, 3 H) 3.71 - 3.76 (m, 2 H) 3.78 - 3.82 (m, 1 H) 3.83 - 3.88 (m, 3 H) 4.14 - 4.18 (m, 2 H) 4.42 - 4.55 (m, 1 H) 4.66 - 4.75 (m, 1 H) 6.69 - 6.75 (m, 1 H) 6.87 - 6.94 (m, 2 H) 7.16 - 7.21 (m, 1 H) 7.36 - 7.42 (m, 1 H) 7.45 - 7.50 (m, 1 H) 7.51 - 7.54 (m, 2 H) 8.23 - 8.29 (m, 1 H) 9.52 - 9.56 (m, 1 H).

### 28-4. Preparation of Compound 127, N-(2,2'-dichloro-3"-fluoro-5"-methoxy-4"-(((((S)-5-oxopyrrolidin-2-yl)methyl)amino)methyl)-[1,1':3',1"-terphenyl]-3-yl)-4-((R)-3-hydroxypyrrolidin-1-yl)-4,5,6,7-tetrahydropyrazolo[1,5-a]pyridine-2-carboxamide

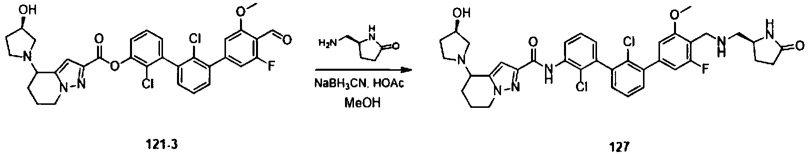

To a solution of [2-chloro-3-[2-chloro-3-(3-fluoro-4-formyl-5-methoxyphenyl)phenyl]phenyl] 4-[(3R)-3-hydroxypyrrolidin-1-yl]-4,5,6,7-tetrahydropyrazolo[1,5-a]pyridine-2-carboxylate (80 mg, 128.11 µmol, 1 eq) and (5S)-5-(aminomethyl)pyrrolidin-2-one (146.23 mg, 1.28 mmol, 10 eq) in MeOH (15 mL), NaBH₃CN (40.25 mg, 640.55 µmol, 5 eq) and AcOH (76.93 mg, 1.28 mmol, 73.34 µL, 10 eq) were added, and the resulting mixture was stirred at 50 °C for 12 hours. After completion of the reaction, the mixture was concentrated under reduced pressure, and the residue was purified by reversed-phase HPLC (0.1% NH₃·H₂O condition; column: Waters XBridge, 150 * 25 mm * 5 um; mobile phase: [water (NH₄HCO₃)-ACN]; gradient: 35-65% B over 9 min) to afford Compound 127 (11 mg, 11.5% yield) as a white solid.

LCMS m/z 721.4 [M+1]⁺.

¹H NMR (400 MHz, DMSO-d6) δ ppm 1.57 (br s, 1 H) 1.61 - 1.71 (m, 1 H) 1.90 (br d, J=9.38 Hz, 3 H) 1.93 - 1.99 (m, 1 H) 2.03 - 2.14 (m, 3 H) 2.24 (br s, 1 H) 2.41 - 2.45 (m, 1 H) 2.57 - 2.64 (m, 1 H) 2.65 - 2.73 (m, 1 H) 2.74 - 2.82 (m, 1 H) 2.85 - 2.92 (m, 1 H) 3.07 - 3.26 (m, 1 H) 3.53 - 3.64 (m, 1 H) 3.72 - 3.76 (m, 2 H) 3.77 - 3.83 (m, 1 H) 3.87 (s, 3 H) 4.17 (br s, 3 H) 4.71 (br s, 1 H) 6.73 (br d, J=8.75 Hz, 1 H) 6.85 - 6.98 (m, 2 H) 7.19 (br d, J=7.50 Hz, 1 H) 7.40 (br t, J=4.50 Hz, 1 H) 7.48 (br t, J=7.94 Hz, 1 H) 7.53 (br d, J=4.50 Hz, 2 H) 7.66 (s, 1 H) 8.22 - 8.31 (m, 1 H) 9.55 (br s, 1 H).

### 28-5. Preparation of Compound 128, 2-(((2',2"-dichloro-3-fluoro-3"-(4-((R)-3-hydroxypyrrolidin-1-yl)-4,5,6,7-tetrahydropyrazolo[1,5-a]pyridine-2-carboxamido)-5-methoxy-[1,1':3',1"-terphenyl]-4-yl)methyl)amino)-2-methylpropanoic acid

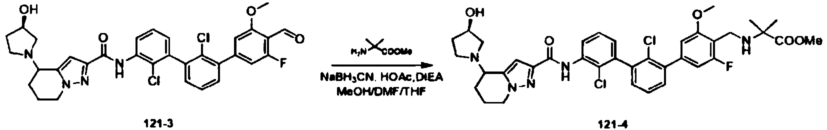

To a solution of N-[2-chloro-3-[2-chloro-3-(3-fluoro-4-formyl-5-methoxyphenyl)phenyl]phenyl]-4-[(3R)-3-hydroxypyrrolidin-1-yl]-4,5,6,7-tetrahydropyrazolo[1,5-a]pyridine-2-carboxamide (121 mg, 194.07 µmol, 1 eq) and methyl 2-amino-2-methylpropanoate hydrochloride (149.05 mg, 970.33 µmol, 5 eq) in MeOH (7 mL)/DMF (0.5 mL)/THF (0.5 mL), NaBH₃CN (60.98 mg, 970.33 µmol, 5 eq), DIPEA (125.41 mg, 970.33 µmol, 169.01 µL, 5 eq), and AcOH (116.54 mg, 1.94 mmol, 111.09 µL, 10 eq) were added, and the resulting mixture was stirred at 60 °C for 12 hours. After completion of the reaction, the mixture was concentrated under reduced pressure, and the residue was purified by prep-TLC (SiO₂, Ethyl acetate: Methanol = 3:1) to afford Compound 121-4 (100 mg, 68.9% yield) as a white solid.

LCMS m/z 724.2 [M+1]⁺.

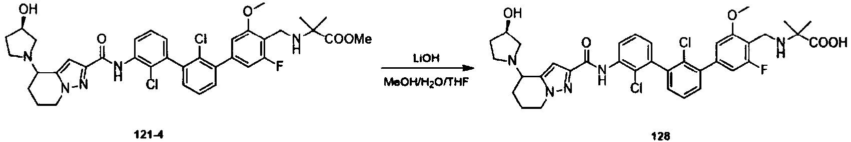

To a solution of methyl 2-[[4-[2-chloro-3-[2-chloro-3-[[4-[(3R)-3-hydroxypyrrolidin-1-yl]-4,5,6,7-tetrahydropyrazolo[1,5-a]pyridine-2-carbonyl]amino]phenyl]phenyl]-2-fluoro-6-methoxyphenyl]methylamino]-2-methylpropanoate (100 mg, 138.00 µmol, 1 eq) in MeOH (4 mL)/THF (1 mL)/H₂O (1 mL), LiOH·H₂O (17.37 mg, 413.99 µmol, 3 eq) was added. The reaction mixture was stirred at room temperature for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 25-55% B over 9 min) to afford Compound 128 (61 mg, 61.5% yield) as a white solid.

LCMS m/z 710.3 [M+1]⁺.

¹H NMR (400 MHz, CHLOROFORM-d) δ 9.45 (s, 1H), 8.65 - 8.61 (m, 1H), 7.42 - 7.32 (m, 2H), 7.28 - 7.24 (m, 2H), 7.04 (d, J = 7.6 Hz, 1H), 6.88 - 6.79 (m, 3H), 4.37 (br s, 1H), 4.27 - 4.14 (m, 2H), 3.99 (s, 3H), 3.94 - 3.81 (m, 3H), 3.04 (br d, J = 5.9 Hz, 1H), 2.96 (br d, J = 6.5 Hz, 1H), 2.90 - 2.68 (m, 3H), 2.65 - 2.50 (m, 2H), 2.41 (br s, 1H), 2.25 - 2.10 (m, 1H), 2.00 (br d, J = 5.0 Hz, 3H), 1.84 - 1.74 (m, 1H), 1.49 (br s, 6H).

### [Preparation Example 29]

### Preparation of Compounds 129 to 137

### 29-1. Preparation of Compound 129, N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-hydroxy-4,5,6,7-tetrahydropyrazolo[1,5-a]pyridine-2-carboxamide

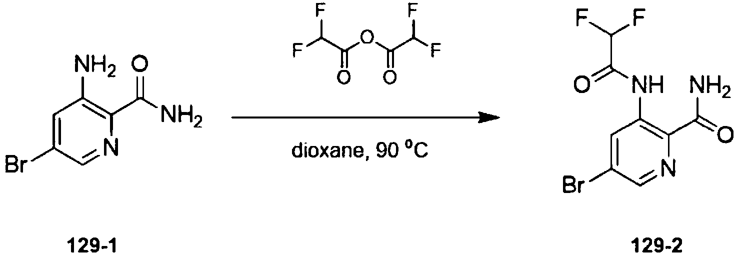

A mixture of 3-amino-5-bromopyridine-2-carboxamide (10 g, 46.29 mmol, 1 eq) and (2,2-difluoroacetyl) 2,2-difluoroacetate (12.08 g, 69.43 mmol, 1.5 eq) in dioxane (100 mL) was stirred at 90 °C for 12 hours. After completion of the reaction, water (300 mL) was added to the mixture, and the precipitated solid was collected by filtration to afford Compound 129-2 (14 g), which was used in the next reaction without further purification.

LCMS m/z 293.9 [M+1]⁺.

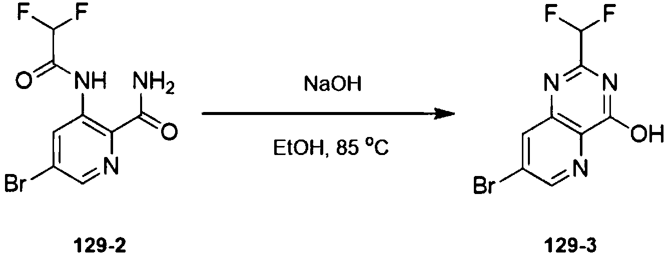

A mixture of 5-bromo-3-[(2,2-difluoroacetyl)amino]pyridine-2-carboxamide (14 g, 47.61 mmol, 1 eq) and NaOH (3.81 g, 95.22 mmol, 2 eq) in EtOH (200 mL) was stirred at 85 °C for 3 hours. After completion of the reaction, the mixture was diluted with H₂O (100 mL) and neutralized with an aqueous 12 N HCl solution. The precipitated solid was collected by filtration to afford Compound 129-3 (11 g, 83.70% yield) as a yellow solid.

LCMS m/z 275.8 [M+1]⁺.

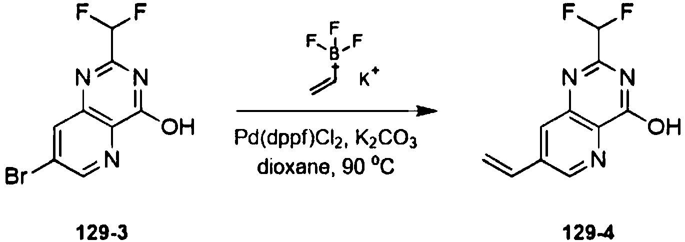

A mixture of 7-bromo-2-(difluoromethyl)pyrido[3,2-d]pyrimidin-4-ol (5 g, 18.11 mmol, 1 eq), potassium trifluoro(vinyl)boranuide (3.64 g, 27.17 mmol, 1.5 eq), Pd(dppf)Cl₂ (662.69 mg, 905.67 µmol, 0.05 eq), and K₂CO₃ (7.51 g, 54.34 mmol, 3 eq) in dioxane (50 mL) was stirred at 90 °C for 1 hour under a nitrogen atmosphere. After completion of the reaction, the mixture was diluted with H₂O (100 mL), and the pH was adjusted to 6-7 with an aqueous 12 N HCl solution, followed by extraction with ethyl acetate (100 mL * 3). The organic layer was dried over Na₂SO₄ and concentrated under reduced pressure to afford Compound 129-4 (2.1 g, 46.7% yield) as a brown solid.

LCMS m/z 224.2 [M+1]⁺.

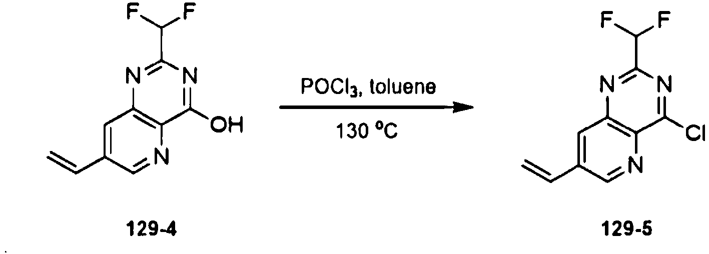

A solution of 2-(difluoromethyl)-7-vinylpyrido[3,2-d]pyrimidin-4-ol (1.6 g, 7.17 mmol, 1 eq) and POCl₃ (5.50 g, 35.85 mmol, 3.33 mL, 5 eq) in toluene (100 mL) was stirred at 130 °C for 6 hours. After completion of the reaction, an aqueous NaHCO₃ solution (200 mL) was slowly added to the mixture at 0 °C, followed by extraction with ethyl acetate (100 mL * 3). The organic layer was dried over Na₂SO₄ and concentrated under reduced pressure. The residue was purified by flash silica gel chromatography (ISCO; 25 g SepaFlash^{®} Silica Flash Column; eluent: 0-25% ethyl acetate/petroleum ether gradient @ 60 mL/min) to afford Compound 129-5 (450 mg, 22.1% yield) as a yellow solid.

LCMS m/z 242.1 [M+1]⁺.

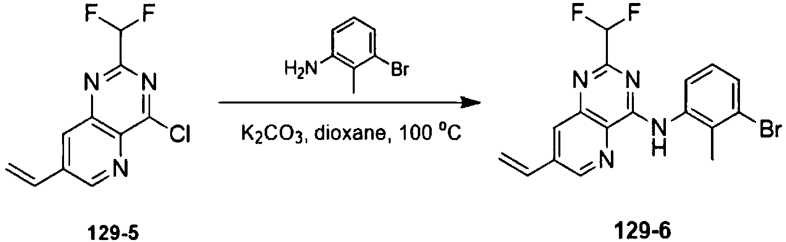

A solution of 3-bromo-2-methylaniline (415.80 mg, 2.23 mmol, 275.36 µL, 1.2 eq), 4-chloro-2-(difluoromethyl)-7-vinylpyrido[3,2-d]pyrimidine (450 mg, 1.86 mmol, 1 eq) and K₂CO₃ (308.87 mg, 2.23 mmol, 1.2 eq) in dioxane (20 mL) was stirred at 100 °C for 12 hours. After completion of the reaction, the mixture was filtered, and the filtrate was concentrated under reduced pressure. The residue was purified by flash silica gel chromatography (ISCO^{®}; 25 g SepaFlash^{®} Silica Flash Column; eluent: 0-20% ethyl acetate/petroleum ether gradient @ 100 mL/min) to afford Compound 129-6 (320 mg, 43.9% yield) as a yellow liquid.

LCMS m/z 391.1 [M+1]⁺.

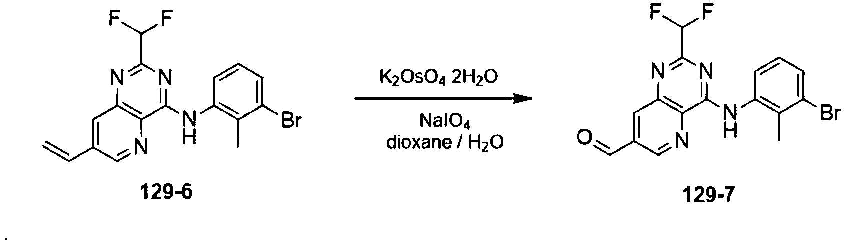

A mixture of N-(3-bromo-2-methylphenyl)-2-(difluoromethyl)-7-vinylpyrido[3,2-d]pyrimidin-4-amine (300 mg, 766.85 µmol, 1 eq), dipotassium dioxido(dioxo)osmium dihydrate (56.51 mg, 153.37 µmol, 0.2 eq), and sodium periodate (656.09 mg, 3.07 mmol, 169.97 µL, 4 eq) in dioxane (20 mL)/H₂O (5 mL) was stirred at 60 °C for 12 hours. After completion of the reaction, the mixture was filtered, and the filtrate was concentrated under reduced pressure. The residue was purified by flash silica gel chromatography (ISCO^{®}; 25 g SepaFlash^{®} Silica Flash Column; eluent: 0-40% ethyl acetate/petroleum ether gradient @ 100 mL/min) to afford Compound 129-7 (250 mg, 53.8% yield) as a yellow solid.

LCMS m/z 393.1 [M+1]⁺.

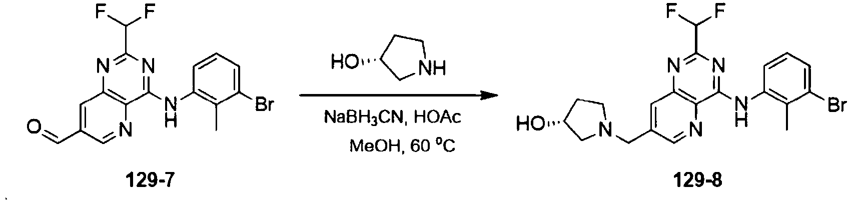

A solution of (3R)-pyrrolidin-3-ol (553.94 mg, 6.36 mmol, 527.56 µL, 10 eq), 4-(3-bromo-2-methyl-anilino)-2-(difluoromethyl)pyrido[3,2-d]pyrimidine-7-carbaldehyde (250 mg, 635.83 µmol, 1 eq), NaBH₃CN (399.56 mg, 6.36 mmol, 10 eq), and HOAc (38.18 mg, 635.83 µmol, 36.36 µL, 1 eq) in MeOH (10 mL) was stirred at 60 °C for 12 hours. After completion of the reaction, the mixture was concentrated under reduced pressure, and the residue was purified by prep-HPLC (column: Waters Xbridge Prep OBD C18 150*40 mm*10 um; mobile phase: [water (NH₄HCO₃)-ACN]; B%: 32%-62%, 10 min) to afford Compound 129-8 (200 mg, 67.7% yield) as a yellow solid.

LCMS m/z 464.2 [M+1]⁺.

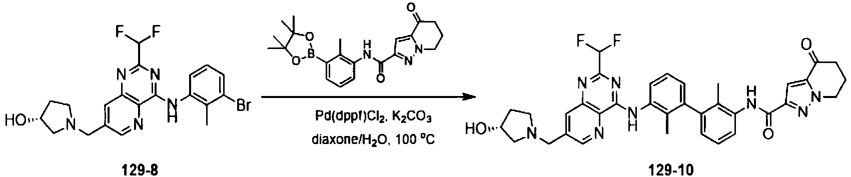

A mixture of (3R)-1-[[4-(3-bromo-2-methylanilino)-2-(difluoromethyl)pyrido[3,2-d]pyrimidin-7-yl]methyl]pyrrolidin-3-ol (100 mg, 215.38 µmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (212.82 mg, 323.06 µmol, 1.5 eq), Pd(dppf)Cl₂ (23.64 mg, 32.31 µmol, 0.15 eq), and K₂CO₃ (89.30 mg, 646.13 µmol, 3 eq) in dioxane (10 mL)/H₂O (2 mL) was stirred at 100 °C for 12 hours under a nitrogen atmosphere. After completion of the reaction, the mixture was filtered, and the filtrate was concentrated under reduced pressure. The residue was purified by flash silica gel chromatography (ISCO^{®}; 12 g SepaFlash^{®} Silica Flash Column; eluent: 0-30% MeOH/ethyl acetate gradient @ 80 mL/min) to afford Compound 129-10 (120 mg, 29.0% yield) as a yellow solid.

LCMS m/z 653.5 [M+1]⁺.

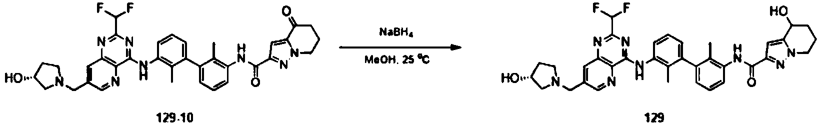

A solution of N-[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]-4-oxo-6,7-dihydro-5H-pyrazolo[1,5-a]pyridine-2-carboxamide (120 mg, 183.85 µmol, 1 eq) and NaBH₄ (34.78 mg, 919.27 µmol, 5 eq) in MeOH (20 mL) was stirred at room temperature for 12 hours. After completion of the reaction, an aq. saturated NH₄Cl solution (1 mL) was added, and the mixture was filtered. The filtrate was concentrated under reduced pressure, and the residue was purified by prep-HPLC (column: Welch Xtimate C18, 150 * 25 mm * 5 um; mobile phase: [water (HCl)-ACN]; gradient: 18-48% B over 8 min) to afford Compound 129 (25 mg, 20.1% yield) as a yellow solid.

LCMS m/z 655.5 [M+1]⁺.

¹H NMR (400 MHz, DMSO-d6) δ = 10.40 (s, 1H), 9.54 (s, 1H), 8.96 (s, 1H), 8.17 (s, 1H), 7.66 (s, 1H), 7.58 (s, 1H), 7.35 (br s, 1H), 7.30 (br s, 1H), 7.07 (s, 1H), 7.02 (s, 1H), 6.85 (s, 1H), 6.70 (s, 2H), 5.58 (s, 1H), 4.76 (s, 2H), 4.14 (s, 2H), 3.87 (d, J = 14.0 Hz, 2H), 2.69 (s, 3H), 2.17 (s, 2H), 1.98 (d, J = 17.4 Hz, 8H), 1.73 (s, 1H), 1.59 (s, 2H).

### 29-2. Preparation of Compound 130, 4-((2-acetamidoethyl)amino)-N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4,5,6,7-tetrahydropyrazolo[1,5-a]pyridine-2-carboxamide

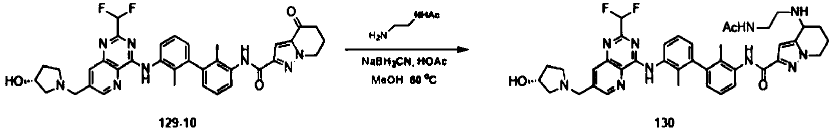

A solution of N-(2-aminoethyl)acetamide (125.19 mg, 1.23 mmol, 117.00 µL, 10 eq), NaBH₃CN (38.51 mg, 612.85 µmol, 5 eq), N-[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]-4-oxo-6,7-dihydro-5H-pyrazolo[1,5-a]pyridine-2-carboxamide (80 mg, 122.57 µmol, 1 eq), and HOAc (73.84 mg, 1.23 mmol, 70.32 µL, 10 eq) in MeOH (10 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 30-60% B over 9 min) to afford Compound 130 (16 mg, 16.9% yield) as a yellow solid.

LCMS m/z 739.5 [M+1]⁺.

¹H NMR (400 MHz, METHANOL-d4) δ = 9.00 (d, J = 1.6 Hz, 1H), 8.20 (s, 1H), 7.95 (d, J = 7.6 Hz, 1H), 7.68 (d, J = 8.0 Hz, 1H), 7.41 - 7.31 (m, 2H), 7.14 - 7.09 (m, 2H), 6.86 (s, 1H), 6.56 (s, 1H), 4.60 (br s, 1H), 4.44 - 4.36 (m, 1H), 4.29 - 4.16 (m, 2H), 4.06 - 3.89 (m, 3H), 3.37 - 3.35 (m, 2H), 2.92 - 2.80 (m, 4H), 2.66 - 2.57 (m, 2H), 2.36 - 2.27 (m, 1H), 2.26 - 2.15 (m, 2H), 2.10 (d, J = 3.5 Hz, 6H), 1.98 (s, 3H), 1.86 - 1.74 (m, 2H), 1.38 - 1.24 (m, 2H).

### 29-3. Preparation of Compound 131, N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide

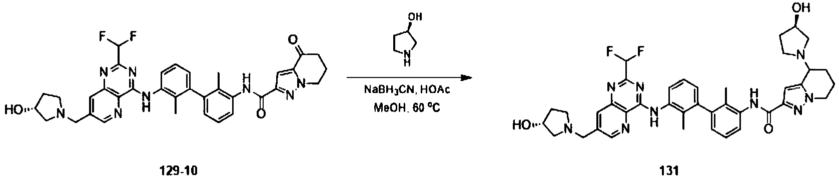

A mixture of N-[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]-4-oxo-6,7-dihydro-5H-pyrazolo[1,5-a]pyridine-2-carboxamide (80 mg, 122.57 µmol, 1 eq), NaBH₃CN (38.51 mg, 612.85 µmol, 5 eq), (3R)-pyrrolidin-3-ol (106.78 mg, 1.23 mmol, 101.70 µL, 10 eq), and HOAc (73.84 mg, 1.23 mmol, 70.32 µL, 10 eq) in MeOH (10 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 28-58% B over 9 min) to afford Compound 131 (15 mg, 16.0% yield) as a yellow solid.

LCMS m/z 724.5 [M+1]⁺.

¹H NMR (400 MHz, METHANOL-d4) δ = 9.00 (d, J = 1.8 Hz, 1H), 8.21 (s, 1H), 7.95 (d, J = 7.8 Hz, 1H), 7.68 (d, J = 7.6 Hz, 1H), 7.41 - 7.31 (m, 2H), 7.14 - 7.09 (m, 2H), 6.84 (s, 1H), 6.74 - 6.36 (m, 1H), 4.86 - 4.86 (m, 1H), 4.61 (s, 2H), 4.39 (s, 2H), 4.24 (s, 2H), 4.00 - 3.87 (m, 3H), 3.07 - 2.92 (m, 2H), 2.87 (dd, J = 5.9, 10.0 Hz, 2H), 2.79 - 2.67 (m, 2H), 2.62 (dd, J = 2.9, 9.8 Hz, 2H), 2.45 - 2.33 (m, 1H), 2.20 (s, 2H), 2.14 - 2.03 (m, 9H), 1.82 - 1.73 (m, 2H), 1.31 (m, 2H).

### 29-4. Preparation of Compound 132, N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-((2-hydroxyethyl)amino)-4,5,6,7-tetrahydropyrazolo[1,5-a]pyridine-2-carboxamide

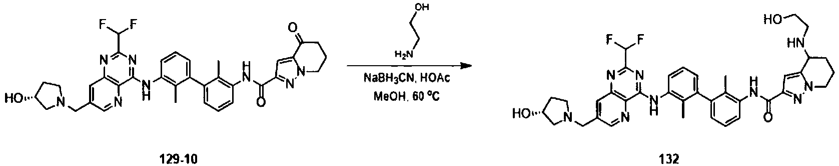

A mixture of N-[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]-4-oxo-6,7-dihydro-5H-pyrazolo[1,5-a]pyridine-2-carboxamide (70 mg, 107.25 µmol, 1 eq), NaBH₃CN (33.70 mg, 536.24 µmol, 5 eq), 2-aminoethanol (65.51 mg, 1.07 mmol, 64.86 µL, 10 eq), and HOAc (73.84 mg, 1.23 mmol, 70.32 µL, 10 eq) in MeOH (10 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 28-58% B over 9 min) to afford Compound 132 (12 mg, 14.4% yield) as a yellow solid.

LCMS m/z 698.6 [M+1]⁺.

¹H NMR (400 MHz, METHANOL-d4) δ = 9.00 (d, J = 1.8 Hz, 1H), 8.20 (s, 1H), 7.95 (d, J = 7.5 Hz, 1H), 7.68 (d, J = 7.6 Hz, 1H), 7.39 (t, J = 7.9 Hz, 1H), 7.33 (t, J = 7.8 Hz, 1H), 7.15 - 7.08 (m, 2H), 6.88 (s, 1H), 6.77 - 6.37 (m, 1H), 4.61 (br s, 1H), 4.44 - 4.37 (m, 1H), 4.29 - 4.18 (m, 2H), 4.09 - 4.03 (m, 1H), 4.01 - 3.89 (m, 2H), 3.72 (t, J = 5.6 Hz, 2H), 2.91 - 2.83 (m, 4H), 2.65 - 2.58 (m, 2H), 2.37 - 2.14 (m, 4H), 2.10 (d, J = 3.1 Hz, 7H), 1.88 - 1.74 (m, 2H), 1.37 - 1.28 (m, 1H).

### 29-5. Preparation of Compound 133, (3R)-1-(2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid

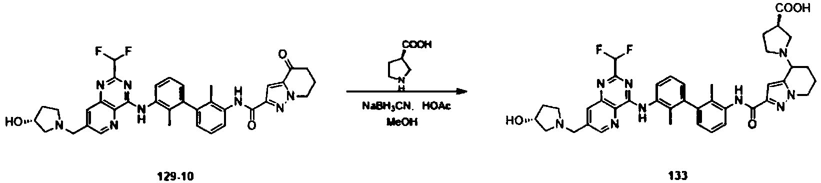

A solution of N-[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]-4-oxo-6,7-dihydro-5H-pyrazolo[1,5-a]pyridine-2-carboxamide (120 mg, 183.85 µmol, 1 eq), (3R)-pyrrolidine-3-carboxylic acid (211.67 mg, 1.84 mmol, 10 eq), HOAc (110.41 mg, 1.84 mmol, 105.25 µL, 10 eq), and NaBH₃CN (57.77 mg, 919.27 µmol, 5 eq) in MeOH (40 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 30-60% B over 9 min) to afford Compound 133 (30 mg, 21.0% yield) as a yellow solid.

LCMS m/z 752.6 [M+1]⁺.

¹H NMR (400 MHz, DMSO-d6) δ = 10.39 (br s, 1H), 9.54 (br s, 1H), 8.95 (d, J = 1.6 Hz, 1H), 8.17 (s, 1H), 7.58 (br d, J = 7.8 Hz, 1H), 7.39 - 7.26 (m, 2H), 7.10 - 7.00 (m, 2H), 6.71 (t, J = 54.5 Hz, 1H), 4.27 - 4.11 (m, 4H), 3.93 - 3.79 (m, 4H), 2.96 - 2.82 (m, 3H), 2.80 - 2.58 (m, 6H), 2.44 - 2.39 (m, 1H), 2.26 (br s, 1H), 2.09 - 1.88 (m, 13H), 1.64 - 1.54 (m, 1H).

### 29-6. Preparation of Compound 134, 2-((2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetic acid

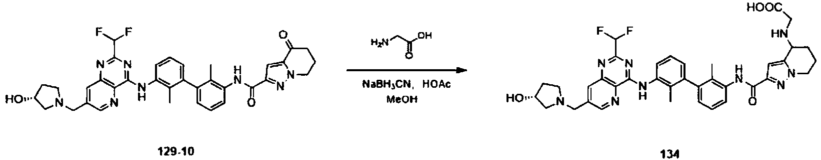

A solution of N-[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]-4-oxo-6,7-dihydro-5H-pyrazolo[1,5-a]pyridine-2-carboxamide (120 mg, 183.85 µmol, 1 eq), 2-aminoacetic acid (138.01 mg, 1.84 mmol, 10 eq), HOAc (110.41 mg, 1.84 mmol, 105.25 µL, 10 eq), and NaBH₃CN (57.77 mg, 919.25 µmol, 5 eq) in MeOH (40 mL) was stirred at 60 °C for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 30-60% B over 9 min) to afford Compound 134 (51 mg, 38.1% yield) as a yellow solid.

LCMS m/z 712.6 [M+1]⁺.

¹H NMR (400 MHz, DMSO-d6) δ = 10.45 - 10.35 (m, 1H), 9.62 - 9.52 (m, 1H), 9.01 - 8.92 (m, 1H), 8.24 - 8.13 (m, 1H), 7.72 - 7.63 (m, 1H), 7.60 - 7.52 (m, 1H), 7.40 - 7.25 (m, 2H), 7.12 - 7.01 (m, 2H), 6.86 - 6.56 (m, 2H), 4.27 - 4.19 (m, 1H), 4.18 - 4.13 (m, 2H), 4.08 - 4.03 (m, 1H), 3.93 - 3.82 (m, 2H), 3.82 - 3.81 (m, 1H), 2.78 - 2.66 (m, 3H), 2.44 - 2.40 (m, 1H), 2.35 - 2.32 (m, 1H), 2.26 - 2.17 (m, 1H), 2.09 - 1.87 (m, 11H), 1.80 - 1.71 (m, 1H), 1.65 - 1.54 (m, 1H).

### 29-7. Preparation of Compound 135, N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide

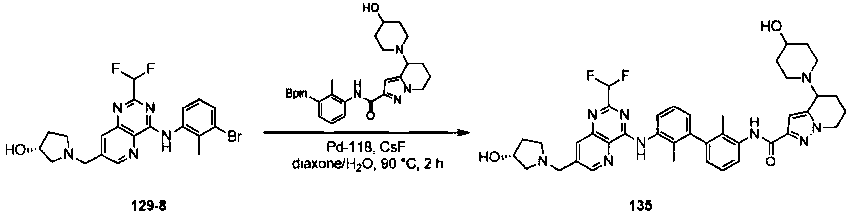

A mixture of (3R)-1-[[4-(3-bromo-2-methylanilino)-2-(difluoromethyl)pyrido[3,2-d]pyrimidin-7-yl]methyl]pyrrolidin-3-ol (70 mg, 150.76 µmol, 1 eq), 4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (86.91 mg, 180.92 µmol, 1.2 eq), CsF (68.70 mg, 452.29 µmol, 16.70 µL, 3 eq), and ditert-butyl(cyclopentyl)phosphane;dichloropalladium iron (9.83 mg, 15.08 µmol, 0.1 eq) in dioxane (3 mL)/H₂O (0.5 mL) was stirred at 90 °C for 2 hours under a nitrogen atmosphere. After completion of the reaction, the mixture was cooled to room temperature and concentrated under reduced pressure. The residue was purified by prep-HPLC (column: Waters XBridge Prep OBD C18, 150 * 40 mm * 10 um; mobile phase: [water (NH₄HCO₃)-ACN]; gradient: 25-55% B over 15 min) to afford Compound 135 (12 mg, 10.2% yield) as a white solid.

LCMS m/z 738.4 [M+1]⁺.

¹H NMR (400 MHz, DMSO-d6) δ = 10.42 (s, 1H), 9.53 (br d, J = 4.4 Hz, 1H), 8.99 (br s, 1H), 8.23 (br s, 1H), 7.64 (d, J = 8.0 Hz, 1H), 7.56 (dd, J = 4.6, 7.6 Hz, 1H), 7.35 (t, J = 7.8 Hz, 1H), 7.29 (t, J = 7.8 Hz, 1H), 7.07 (d, J = 7.4 Hz, 1H), 7.01 (d, J = 6.9 Hz, 1H), 6.71 (s, 1H), 6.65 - 6.56 (m, 1H), 4.88 (br s, 1H), 4.57 (br s, 1H), 4.30 - 4.15 (m, 2H), 4.15 - 3.92 (m, 3H), 3.51 - 3.39 (m, 2H), 3.27 - 3.06 (m, 1H), 2.84 (br s, 1H), 2.74 (br s, 1H), 2.70 - 2.63 (m, 1H), 2.47 - 2.38 (m, 1H), 2.37 - 2.23 (m, 1H), 2.15 (br d, J = 14.0 Hz, 1H), 2.09 - 2.01 (m, 1H), 1.99 (s, 3H), 1.94 (s, 3H), 1.88 (br d, J = 5.3 Hz, 1H), 1.73 (br s, 2H), 1.67 (br s, 2H), 1.51 - 1.32 (m, 2H).

### 29-8. Preparation of Compound 136, 1-(2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

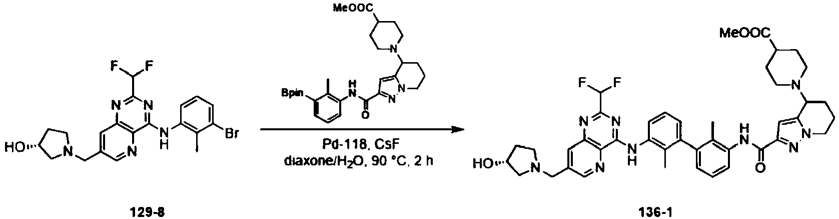

A mixture of (3R)-1-[[4-(3-bromo-2-methylanilino)-2-(difluoromethyl)pyrido[3,2-d]pyrimidin-7-yl]methyl]pyrrolidin-3-ol (70 mg, 150.76 µmol, 1 eq), methyl 1-[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (94.52 mg, 180.92 µmol, 1.2 eq), CsF (68.70 mg, 452.29 µmol, 16.70 µL, 3 eq), and ditert-butyl(cyclopentyl)phosphane;dichloropalladium iron (9.83 mg, 15.08 µmol, 0.1 eq) in dioxane (3 mL)/H₂O (0.5 mL) was stirred at 90 °C for 2 hours under a nitrogen atmosphere. After completion of the reaction, the mixture was cooled to room temperature and concentrated under reduced pressure. The residue was purified by prep-HPLC (column: Waters XBridge Prep OBD C18, 150 * 40 mm * 10 um; mobile phase: [water (NH₄HCO₃)-ACN]; gradient: 40-70% B over 15 min) to afford Compound 136-1 (42 mg, 33.9% yield) as a white solid.

LCMS m/z 780.4 [M+1]⁺.

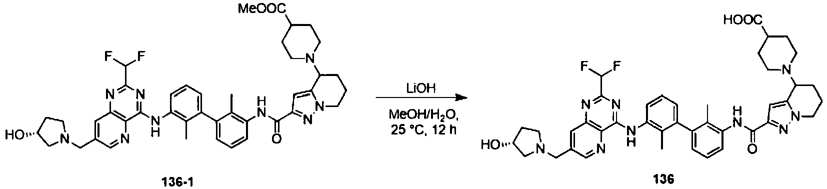

To a solution of methyl 1-[2-[[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]carbamoyl]-4,5,6,7-tetrahydropyrazolo[1,5-a]pyridin-4-yl]piperidine-4-carboxylate (30 mg, 38.47 µmol, 1 eq) in H₂O (3 mL)/MeOH (10 mL), LiOH (2.76 mg, 115.40 µmol, 3 eq) was added. The reaction mixture was stirred at room temperature for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 18-48% B over 9 min) to afford Compound 136 (18 mg, 58.0% yield) as a white solid.

LCMS m/z 766.5 [M+1]⁺.

¹H NMR (400 MHz, DMSO-d6) δ = 10.39 (br s, 1H), 9.52 (d, J = 4.1 Hz, 1H), 8.96 (s, 1H), 8.19 (br d, J = 1.6 Hz, 1H), 7.72 (s, 1H), 7.56 (dd, J = 4.3, 7.4 Hz, 1H), 7.40 - 7.24 (m, 2H), 7.04 (dd, J = 7.6, 21.8 Hz, 2H), 6.74 - 6.54 (m, 2H), 4.77 (br dd, J = 4.2, 5.3 Hz, 1H), 4.32 - 4.15 (m, 2H), 4.13 - 3.96 (m, 2H), 3.91 - 3.74 (m, 1H), 3.51 - 3.41 (m, 3H), 2.84 - 2.76 (m, 2H), 2.73 - 2.57 (m, 3H), 2.45 (br s, 2H), 2.34 - 2.11 (m, 4H), 1.99 (s, 3H), 1.94 (s, 3H), 1.89 - 1.76 (m, 3H), 1.72 - 1.49 (m, 4H).

### 29-9. Preparation of Compound 137, 2-((2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid

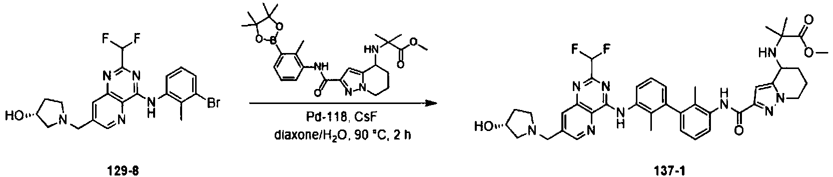

A mixture of (3R)-1-[[4-(3-bromo-2-methylanilino)-2-(difluoromethyl)pyrido[3,2-d]pyrimidin-7-yl]methyl]pyrrolidin-3-ol (70 mg, 150.76 µmol, 1 eq), methyl 2-methyl-2-[[2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]propanoate (89.81 mg, 180.92 µmol, 1.2 eq), CsF (68.70 mg, 452.29 µmol, 16.70 µL, 3 eq), and ditert-butyl(cyclopentyl)phosphane;dichloropalladium iron (9.83 mg, 15.08 µmol, 0.1 eq) in dioxane (3 mL)/H₂O (0.5 mL) was stirred at 90 °C for 2 hours under a nitrogen atmosphere. After completion of the reaction, the mixture was cooled to room temperature and concentrated under reduced pressure. The residue was purified by prep-HPLC (column: Phenomenex Luna C18, 150 * 40 mm * 15 um; mobile phase: [water (TFA)-ACN]; gradient: 15-45% B over 15 min) to afford Compound 137-1 (68 mg, 53.85% yield) as a white solid.

LCMS m/z 754.4 [M+1]⁺.

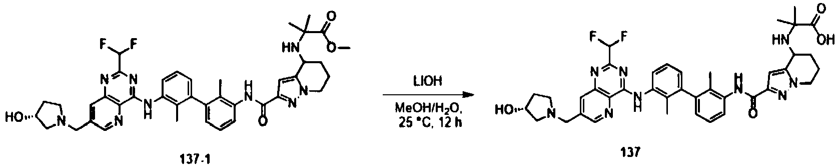

To a solution of methyl 2-[[2-[[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]carbamoyl]-4,5,6,7-tetrahydropyrazolo[1,5-a]pyridin-4-yl]amino]-2-methylpropanoate (60 mg, 79.59 µmol, 1 eq) in H₂O (3 mL)/MeOH (10 mL), LiOH (5.71 mg, 238.78 µmol, 3 eq) was added. The reaction mixture was stirred at room temperature for 12 hours. After completion of the reaction, the mixture was concentrated under reduced pressure, and the residue was purified by prep-HPLC (column: Waters XBridge, 150 * 25 mm * 5 um; mobile phase: [water (NH₄HCO₃)-ACN]; gradient: 20-50% B over 9 min) to afford Compound 137 (10 mg, 16.1% yield) as a white solid.

LCMS m/z 740.6 [M+1]⁺.

¹H NMR (400 MHz, DMSO-d6 ) δ = 10.38 (s, 1H), 9.51 (s, 1H), 8.95 (d, J = 1.9 Hz, 1H), 8.16 (d, J = 1.6 Hz, 1H), 7.74 - 7.63 (m, 1H), 7.57 (d, J = 7.8 Hz, 1H), 7.41 - 7.32 (m, 1H), 7.28 (t, J = 7.9 Hz, 1H), 7.13 - 7.05 (m, 1H), 7.01 (d, J = 7.6 Hz, 1H), 6.87 - 6.52 (m, 2H), 4.85 - 4.69 (m, 1H), 4.39 - 4.19 (m, 2H), 4.16 - 4.08 (m, 3H), 3.98 - 3.90 (m, 2H), 2.78 - 2.71 (m, 2H), 2.69 - 2.64 (m, 2H), 2.44 - 2.36 (m, 2H), 2.09 - 2.03 (m, 2H), 1.98 (s, 3H), 1.94 (s, 3H), 1.71 - 1.54 (m, 3H), 1.28 (d, J = 3.5 Hz, 5H).

### [Preparation Example 30]

### Preparation of Compounds 138 to 146

### 30-1. Preparation of Compound 138, N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-hydroxy-4,5,6,7-tetrahydropyrazolo[1,5-a]pyridine-2-carboxamide

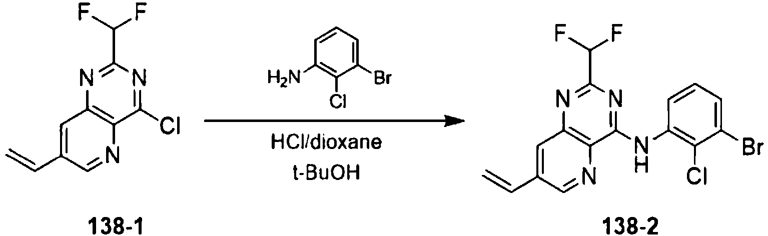

To a solution of 4-Chloro-2-(difluoromethyl)-7-vinylpyrido[3,2-d]pyrimidine (250 mg, 1.03 mmol, 1 eq) and 3-bromo-2-chloroaniline (427.25 mg, 2.07 mmol, 2 eq) in t-BuOH (13 mL), a solution of HCl/dioxane (4 M, 517.33 µL, 2 eq) was added. The resulting mixture was stirred at 120 °C for 12 hours. After completion of the reaction, water (20 mL) was added, and the mixture was extracted with EtOAc (20 mL * 2). The organic layer was washed with brine (10 mL), dried over Na₂SO₄, and concentrated under reduced pressure. The residue was purified by column chromatography (SiO₂, Petroleum ether/Ethyl acetate = 100:1 to 5:1) to afford Compound 138-2 (280 mg, 63.1% yield) as a white solid.

LCMS m/z 411.1 [M+1]⁺.

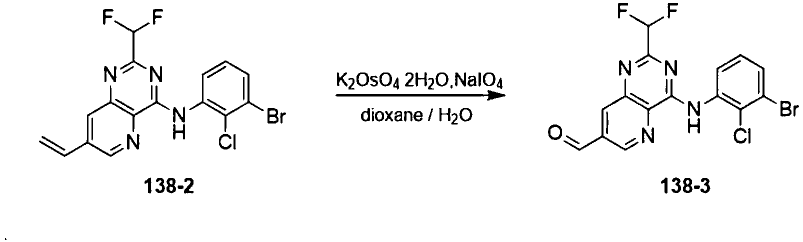

To a mixture of N-(3-bromo-2-chloro-phenyl)-2-(difluoromethyl)-7-vinylpyrido[3,2-d]pyrimidin-4-amine (830 mg, 2.02 mmol, 1 eq) in dioxane (60 mL)/H₂O (12 mL), K₂OsO₄·2H₂O (148.59 mg, 403.27 µmol, 0.2 eq) and NalO₄ (1.73 g, 8.08 mmol, 4 eq) were added, and the resulting mixture was stirred at room temperature for 12 hours. After completion of the reaction, an aq. saturated NH₄Cl solution (50 mL) was added at 0 °C, followed by dilution with H₂O (50 mL). The mixture was extracted with EtOAc (60 mL). The organic layer was dried over Na₂SO₄ and concentrated under reduced pressure. The residue was purified by column chromatography (SiO₂, Petroleum ether/Ethyl acetate = 100/1 to 2/1) to afford Compound 138-3 (596 mg, 55.0% yield) as a white solid.

LCMS m/z 415.0 [M+3]⁺.

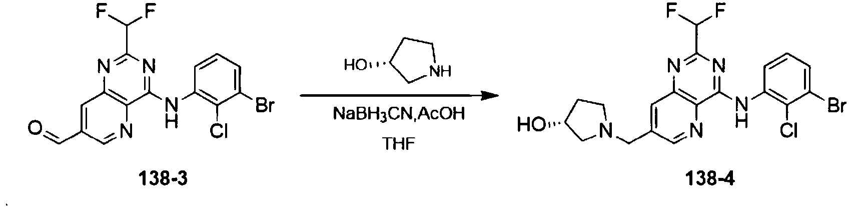

To a solution of 4-(3-bromo-2-chloroanilino)-2-(difluoromethyl)pyrido[3,2-d]pyrimidine-7-carbaldehyde (596 mg, 1.44 mmol, 1 eq) and (3R)-pyrrolidin-3-ol (2.51 g, 28.82 mmol, 2.40 mL, 20 eq) in MeOH (80 mL), NaBH₃CN (452.77 mg, 7.20 mmol, 5 eq) and AcOH (43.27 mg, 720.50 µmol, 41.25 µL, 0.5 eq) were then added, and the resulting mixture was stirred at 60 °C for 12 hours. After completion of the reaction, the mixture was concentrated under reduced pressure. The residue was purified by prep-HPLC (column: Waters Xbridge Prep OBD C18, 150 * 40 mm * 10 um; mobile phase: [water (NH₄HCO₃)-ACN]; gradient: 40%-70% B over 15 min) to afford Compound 138-4 (425 mg, 59.4% yield) as a white solid.

LCMS m/z 485.9 [M+3]⁺.

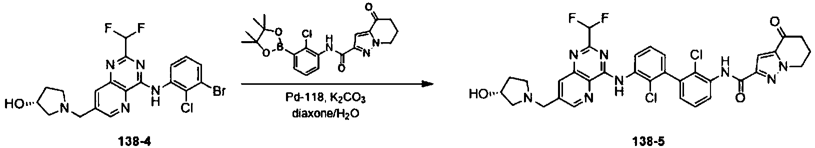

A mixture of (3R)-1-[[4-(3-bromo-2-chloroanilino)-2-(difluoromethyl)pyrido[3,2-d]pyrimidin-7-yl]methyl]pyrrolidin-3-ol (365 mg, 753.01 µmol, 1 eq), N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (469.51 mg, 1.13 mmol, 1.5 eq), K₂CO₃ (312.21 mg, 2.26 mmol, 3 eq), and ditert-butyl(cyclopentyl)phosphane-dichloropalladium-iron (73.62 mg, 112.95 µmol, 0.15 eq) in dioxane (80 mL)/H₂O (16 mL) was stirred under a nitrogen atmosphere at 90 °C for 12 hours. After completion of the reaction, the mixture was concentrated under reduced pressure. The residue was purified by flash silica gel chromatography (ISCO^{®}; 12 g SepaFlash^{®} Silica Flash Column; eluent: 0-23% ethyl acetate/methanol gradient @ 50 mL/min) to afford Compound 138-5 (441 mg, 78.5% yield) as a white solid.

LCMS m/z 693.2 [M+1]⁺.

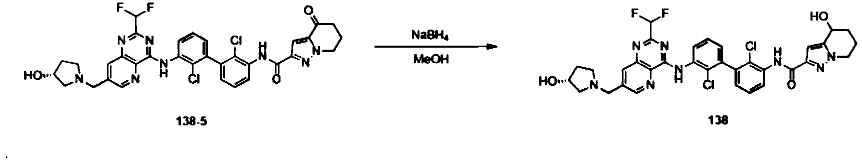

To a solution of N-[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]-4-oxo-6,7-dihydro-5H-pyrazolo[1,5-a]pyridine-2-carboxamide (48 mg, 69.21 µmol, 1 eq) in MeOH (10 mL), NaBH₄ (13.05 mg, 207.63 µmol, 3 eq) was added, and the reaction mixture was stirred at 40 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 40-70% B over 9 min) to afford Compound 138 (28 mg, 57.5% yield) as a white solid.

LCMS m/z 695.2 [M+1]⁺.

¹H NMR (400 MHz, DMSO-d6) δ 10.27 (br s, 1H), 9.55 (s, 1H), 9.01 (s, 1H), 8.57 (br d, J = 7.8 Hz, 1H), 8.30 (d, J = 8.4 Hz, 1H), 8.25 (s, 1H), 7.59 (t, J = 8.0 Hz, 1H), 7.51 (t, J = 7.9 Hz, 1H), 7.24 (br d, J = 7.5 Hz, 1H), 7.20 (br d, J = 7.6 Hz, 1H), 7.04 - 6.73 (m, 2H), 5.59 (d, J = 5.4 Hz, 1H), 4.79 - 4.72 (m, 2H), 4.22 (br s, 1H), 4.14 (br s, 2H), 3.88 (q, J = 13.9 Hz, 2H), 2.77 - 2.66 (m, 2H), 2.41 (dd, J = 3.4, 9.4 Hz, 1H), 2.16 (br s, 1H), 2.08 - 1.97 (m, 2H), 1.93 (br d, J = 6.9 Hz, 1H), 1.72 (br d, J = 7.8 Hz, 1H), 1.59 (br s, 1H), 1.23 (br s, 1H).

### 30-2. Preparation of Compound 139, 4-((2-acetamidoethyl)amino)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4,5,6,7-tetrahydropyrazolo[1,5-a]pyridine-2-carboxamide

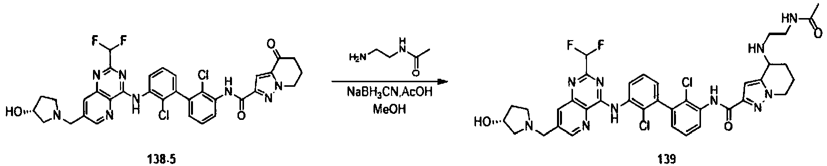

To a solution of N-[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]-4-oxo-6,7-dihydro-5H-pyrazolo[1,5-a]pyridine-2-carboxamide (60 mg, 86.51 µmol, 1 eq) and N-(2-aminoethyl)acetamide (119.90 mg, 1.17 mmol, 13.57 eq) in MeOH (10 mL), NaBH₃CN (27.18 mg, 432.57 µmol, 5 eq) and AcOH (51.95 mg, 865.14 µmol, 10 eq) were added, and the reaction mixture was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 35-65% B over 9 min) to afford Compound 139 (34 mg, 48.8% yield) as a white solid.

LCMS m/z 779.4 [M+1]⁺.

¹H NMR (400 MHz, DMSO-d6) δ = 10.47 - 10.10 (m, 1H), 9.54 (s, 1H), 9.00 (s, 1H), 8.57 (br d, J = 6.8 Hz, 1H), 8.30 (d, J = 8.1 Hz, 1H), 8.25 (s, 1H), 7.84 - 7.79 (m, 1H), 7.59 (t, J = 7.9 Hz, 1H), 7.51 (t, J = 7.9 Hz, 1H), 7.24 (d, J = 6.1 Hz, 1H), 7.19 (dd, J = 1.6, 7.6 Hz, 1H), 6.79 (s, 1H), 7.05 - 6.72 (m, 1H), 7.06 - 6.71 (m, 1H), 4.73 (d, J = 4.6 Hz, 1H), 4.23 (br s, 1H), 4.18 - 4.10 (m, 2H), 3.88 (q, J = 14.2 Hz, 3H), 3.13 (q, J = 6.3 Hz, 2H), 2.76 - 2.61 (m, 4H), 2.41 (br dd, J = 3.6, 9.5 Hz, 2H), 2.20 (br d, J = 6.1 Hz, 2H), 2.07 - 1.98 (m, 2H), 1.81 (s, 3H), 1.72 - 1.55 (m, 2H).

### 30-3. Preparation of Compound 140, N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide

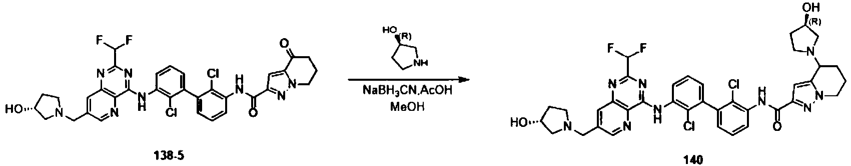

To a solution of N-[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]-4-oxo-6,7-dihydro-5H-pyrazolo[1,5-a]pyridine-2-carboxamide (60 mg, 86.51 µmol, 1 eq) and (3R)-pyrrolidin-3-ol (75.37 mg, 865.14 µmol, 10 eq) in MeOH (10 mL), NaBH₃CN (27.18 mg, 432.57 µmol, 5 eq) and AcOH (51.95 mg, 865.14 µmol, 10 eq) were added, and the reaction mixture was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 40-70% B over 9 min) to afford Compound 140 (35 mg, 51.1% yield) as a white solid.

LCMS m/z 764.4 [M+1]⁺.

¹H NMR (400 MHz, DMSO-d6) δ = 10.42 - 10.11 (m, 1H), 9.55 (s, 1H), 9.01 (s, 1H), 8.58 (br d, J = 8.9 Hz, 1H), 8.31 - 8.24 (m, 2H), 7.60 (t, J = 7.9 Hz, 1H), 7.51 (t, J = 8.1 Hz, 1H), 7.25 (d, J = 7.1 Hz, 1H), 7.20 (d, J = 6.4 Hz, 1H), 7.05 - 6.71 (m, 2H), 4.75 - 4.68 (m, 2H), 4.18 (br s, 4H), 3.94 - 3.81 (m, 3H), 2.80 - 2.65 (m, 5H), 2.47 - 2.38 (m, 3H), 2.02 (s, 2H), 1.95 - 1.85 (m, 4H), 1.58 (br s, 2H).

### 30-4. Preparation of Compound 141, N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-((2-hydroxyethyl)amino)-4,5,6,7-tetrahydropyrazolo[1,5-a]pyridine-2-carboxamide

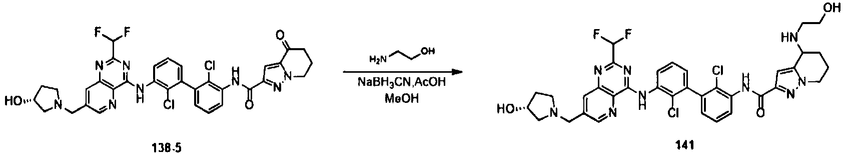

To a solution of N-[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]-4-oxo-6,7-dihydro-5H-pyrazolo[1,5-a]pyridine-2-carboxamide (60 mg, 86.51 µmol, 1 eq) and 2-aminoethanol (52.85 mg, 865.14 µmol, 10 eq) in MeOH (10 mL), NaBH₃CN (27.18 mg, 432.57 µmol, 5 eq) and AcOH (51.95 mg, 865.14 µmol, 10 eq) were added, and the reaction mixture was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 35-65% B over 9 min) to afford Compound 141 (32 mg, 48.4% yield) as a white solid.

LCMS m/z 738.3 [M+1]⁺.

¹H NMR (400 MHz, DMSO-d6) δ 10.34 - 10.21 (m, 1H), 9.55 (s, 1H), 9.01 (s, 1H), 8.61 - 8.53 (m, 1H), 8.30 (d, J = 8.3 Hz, 1H), 8.25 (s, 1H), 7.59 (t, J = 7.1 Hz, 1H), 7.50 (t, J = 7.9 Hz, 1H), 7.24 (br d, J = 8.6 Hz, 1H), 7.19 (d, J = 7.3 Hz, 1H), 7.04 - 6.72 (m, 2H), 4.73 (d, J = 4.6 Hz, 1H), 4.50 (t, J = 5.4 Hz, 1H), 4.15 (br s, 3H), 3.88 (br d, J = 14.3 Hz, 3H), 3.50 - 3.44 (m, 2H), 2.76 - 2.65 (m, 5H), 2.40 (br d, J = 3.8 Hz, 1H), 2.24 - 1.98 (m, 5H), 1.72 - 1.54 (m, 2H).

### 30-5. Preparation of Compound 142, (3R)-1-(2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)pyrrolidine-3-carboxylic acid

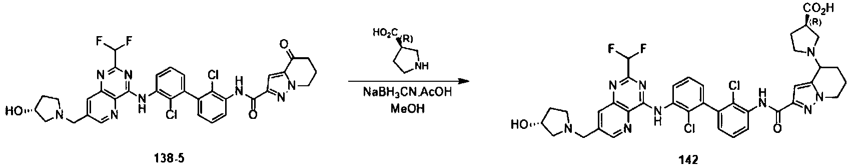

To a solution of N-[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]-4-oxo-6,7-dihydro-5H-pyrazolo[1,5-a]pyridine-2-carboxamide (60 mg, 86.51 µmol, 1 eq) and (3R)-pyrrolidine-3-carboxylic acid (99.60 mg, 865.14 µmol, 10 eq) in MeOH (15 mL)/DMF (15 mL), NaBH₃CN (27.18 mg, 432.57 µmol, 5 eq) and AcOH (51.95 mg, 865.14 µmol, 10 eq) were added, and the reaction mixture was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 20-50% B over 9 min) to afford Compound 142 (48 mg, 68.3% yield) as a white solid.

LCMS m/z 792.5 [M+1]⁺.

¹H NMR (400 MHz, DMSO-d6) δ 10.28 (br s, 1H), 9.56 (s, 1H), 9.01 (d, J = 1.4 Hz, 1H), 8.58 (d, J = 8.1 Hz, 1H), 8.30 - 8.23 (m, 2H), 7.60 (t, J = 7.9 Hz, 1H), 7.51 (t, J = 8.0 Hz, 1H), 7.25 (d, J = 7.6 Hz, 1H), 7.20 (d, J = 6.6 Hz, 1H), 6.88 (t, J = 54.5 Hz, 1H), 6.72 (d, J = 4.4 Hz, 1H), 4.73 (br s, 1H), 4.26 - 4.12 (m, 3H), 3.95 - 3.80 (m, 3H), 2.96 - 2.81 (m, 3H), 2.78 - 2.57 (m, 6H), 2.41 (dd, J = 3.3, 9.8 Hz, 1H), 2.22 (br s, 1H), 2.03 (br dd, J = 6.6, 12.9 Hz, 1H), 1.97 - 1.88 (m, 4H), 1.58 (br d, J = 3.6 Hz, 1H).

### 30-6. Preparation of Compound 143, 2-((2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetic acid

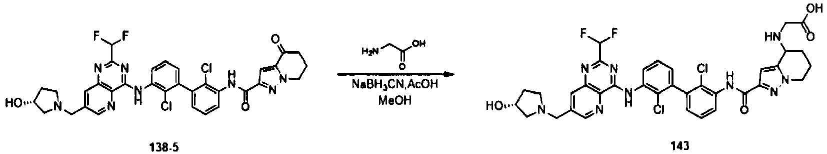

To a solution of N-[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]-4-oxo-6,7-dihydro-5H-pyrazolo[1,5-a]pyridine-2-carboxamide (60 mg, 86.51 µmol, 1 eq) and 2-aminoacetic acid (64.94 mg, 865.14 µmol, 10 eq) in MeOH (10 mL)/DMF (10 mL), NaBH₃CN (27.18 mg, 432.57 µmol, 5 eq) and AcOH (51.95 mg, 865.14 µmol, 10 eq) were added, and the reaction mixture was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 20-50% B over 9 min) to afford Compound 143 (34 mg, 50.5% yield) as a white solid.

LCMS m/z 752.4 [M+1]⁺.

¹H NMR (400 MHz, DMSO-d6) δ 10.28 (s, 1H), 9.57 (s, 1H), 9.01 (d, J = 1.6 Hz, 1H), 8.57 (d, J = 8.3 Hz, 1H), 8.31 - 8.24 (m, 2H), 7.60 (t, J = 7.9 Hz, 1H), 7.51 (t, J = 7.9 Hz, 1H), 7.25 (d, J = 7.5 Hz, 1H), 7.19 (d, J = 7.1 Hz, 1H), 6.82 (s, 1H), 6.88 (t, J = 54.4 Hz, 1H), 4.84 - 4.64 (m, 1H), 4.22 (br s, 1H), 4.15 (br t, J = 5.8 Hz, 2H), 4.03 (br t, J = 5.9 Hz, 1H), 3.88 (q, J = 14.2 Hz, 2H), 2.77 - 2.64 (m, 2H), 2.42 (br dd, J = 3.3, 9.6 Hz, 2H), 2.33 (br s, 1H), 2.19 (br s, 1H), 2.09 - 1.96 (m, 2H), 1.92 (br s, 1H), 1.75 (br s, 1H), 1.58 (br d, J = 3.0 Hz, 1H).

### 30-7. Preparation of Compound 144, N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

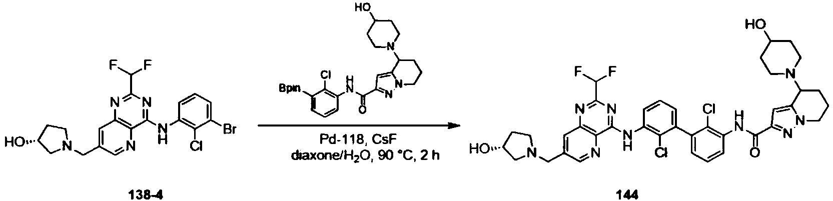

A mixture of (3R)-1-[[4-(3-bromo-2-chloroanilino)-2-(difluoromethyl)pyrido[3,2-d]pyrimidin-7-yl]methyl]pyrrolidin-3-ol (100 mg, 206.30 µmol, 1 eq), N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (154.98 mg, 309.45 µmol, 1.5 eq), CsF (94.01 mg, 618.91 µmol, 3 eq), and ditert-butyl(cyclopentyl)phosphane-dichloropalladium-iron (13.45 mg, 20.63 µmol, 0.1 eq) in dioxane (3 mL)/water (0.5 mL) was stirred at 90 °C for 2 hours under a nitrogen atmosphere. After completion of the reaction, the mixture was filtered and the filtrate was concentrated under reduced pressure. The residue was purified by prep-HPLC (column: Waters XBridge, 150 * 25 mm * 5 µm; mobile phase: water (NH₄HCO₃)-ACN; gradient: 42-72% B over 9 min) to afford Compound 144 (18 mg, 10.6% yield) as a white solid.

LCMS m/z 778.3 [M+3]⁺.

¹H NMR (400 MHz, DMSO-d6) δ = 10.29 (br s, 1H), 9.56 (d, J = 1.6 Hz, 1H), 9.00 (d, J = 1.8 Hz, 1H), 8.55 (dd, J = 1.4, 8.3 Hz, 1H), 8.29 - 8.23 (m, 2H), 7.59 (t, J = 7.9 Hz, 1H), 7.50 (t, J = 7.9 Hz, 1H), 7.27 - 7.22 (m, 1H), 7.19 (dd, J = 1.2, 7.6 Hz, 1H), 7.03 - 6.71 (m, 1H), 6.65 (s, 1H), 4.76 (d, J = 4.6 Hz, 1H), 4.57 (d, J = 4.1 Hz, 1H), 4.24 - 4.15 (m, 2H), 4.09 - 3.84 (m, 4H), 3.45 (br d, J = 4.4 Hz, 2H), 2.75 - 2.64 (m, 4H), 2.43 - 2.36 (m, 2H), 2.26 (br t, J = 9.3 Hz, 1H), 2.13 (br d, J = 13.4 Hz, 1H), 2.07 - 1.96 (m, 2H), 1.92 - 1.82 (m, 1H), 1.80 - 1.67 (m, 3H), 1.61 - 1.53 (m, 1H), 1.47 - 1.33 (m, 2H).

### 30-8. Preparation of Compound 145, 1-(2-((2,2'-dichloro-3'-((2-(difluoromethyl)-7-({(R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid

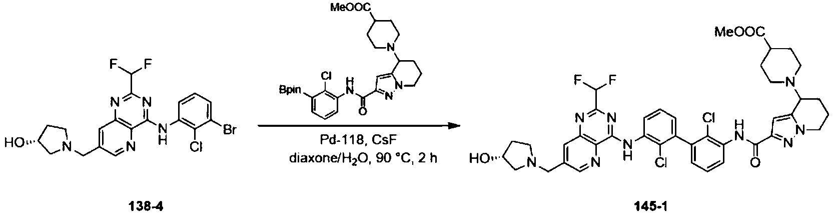

A mixture of (3R)-1-[[4-(3-bromo-2-chloroanilino)-2-(difluoromethyl)pyrido[3,2-d]pyrimidin-7-yl]methyl]pyrrolidin-3-ol (80 mg, 165.04 µmol, 1 eq), methyl 1-[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (107.51 mg, 198.05 µmol, 1.2 eq), CsF (75.21 mg, 495.13 µmol, 3 eq), and ditert-butyl(cyclopentyl)phosphane-dichloropalladium-iron (10.76 mg, 16.50 µmol, 0.1 eq) in dioxane (3 mL)/water (0.5 mL) was stirred at 90 °C for 2 hours under a nitrogen atmosphere. After completion of the reaction, the mixture was filtered and the filtrate was concentrated under reduced pressure. The residue was purified by prep-HPLC (column: Phenomenex Luna C18, 150 * 40 mm * 15 µm; mobile phase: water (TFA)-ACN; gradient: 22-52% B over 15 min) to afford Compound 145-1 (73 mg, 43.1% yield) as a white solid.

LCMS m/z 820.3 [M+1]⁺.

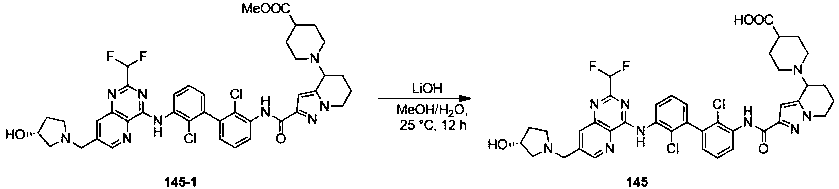

To a solution of methyl 4-[2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]cyclohexanecarboxylate (70 mg, 85.39 µmol, 1 eq) in water (3 mL)/MeOH (10 mL), LiOH (10.2 mg, 426.97 µmol, 5 eq) was added, and the reaction mixture was stirred at room temperature for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 22-52% B over 9 min) to afford Compound 145 (16 mg, 22.0% yield) as a white solid.

LCMS m/z 806.2 [M+1]⁺.

¹H NMR (400 MHz, DMSO-d6 ) δ = 10.13 (s, 1H), 9.67 - 9.49 (m, 1H), 9.01 (d, J = 1.8 Hz, 1H), 8.63 - 8.51 (m, 1H), 8.36 - 8.21 (m, 2H), 7.66 - 7.57 (m, 1H), 7.51 (t, J = 7.9 Hz, 1H), 7.29 - 6.99 (m, 3H), 6.91 - 6.62 (m, 2H), 4.81 - 4.69 (m, 1H), 4.29 - 4.19 (m, 2H), 4.13 - 3.94 (m, 2H), 2.87 - 2.74 (m, 2H), 2.73 - 2.65 (m, 3H), 2.45 - 2.40 (m, 2H), 2.26 - 2.11 (m, 3H), 2.10 - 1.91 (m, 3H), 1.87 - 1.79 (m, 2H), 1.73 - 1.47 (m, 5H).

### 30-9. Preparation of Compound 146, 2-((2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid

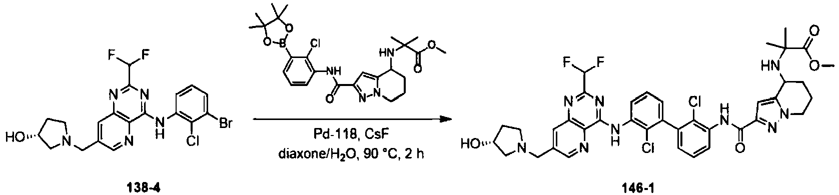

A mixture of (3R)-1-[[4-(3-bromo-2-chloroanilino)-2-(difluoromethyl)pyrido[3,2-d]pyrimidin-7-yl]methyl]pyrrolidin-3-ol (100 mg, 206.30 µmol, 1 eq), methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methylpropanoate (138.61 mg, 268.19 µmol, 1.3 eq), CsF (94.01 mg, 618.91 µmol, 3 eq), and ditert-butyl(cyclopentyl)phosphane-dichloropalladium-iron (13.45 mg, 20.63 µmol, 0.1 eq) in dioxane (8 mL)/water (2 mL) was stirred at 90 °C for 2 hours under a nitrogen atmosphere. After completion of the reaction, the mixture was filtered and the filtrate was concentrated under reduced pressure. The residue was purified by prep-HPLC (column: Phenomenex Luna C18, 150 * 40 mm * 15 µm; mobile phase: water (TFA)-ACN; gradient: 20-50% B over 15 min) to afford Compound 146-1 (82 mg, 47.5% yield) as a white solid.

LCMS m/z 794.4 [M+1]⁺.

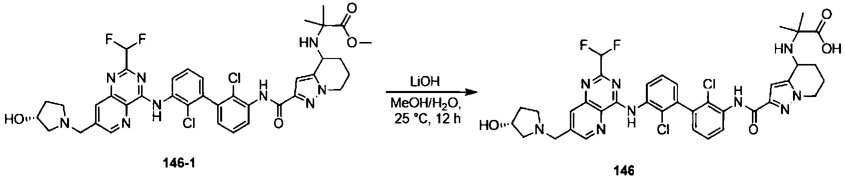

To a solution of methyl 2-[[2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]amino]-2-methylpropanoate (70 mg, 88.09 µmol, 1 eq) in water (3 mL)/MeOH (10 mL), LiOH (10.53 mg, 440.43 µmol, 5 eq) was added, and the reaction mixture was stirred at room temperature for 12 hours. After completion of the reaction, the mixture was concentrated under reduced pressure. The residue was purified by prep-HPLC (column: Waters XBridge Prep OBD C18, 150 * 40 mm * 10 µm; mobile phase: water (NH₄HCO₃)-ACN; gradient: 18-48% B over 15 min) to afford Compound 146 (46 mg, 63.5% yield) as a white solid.

LCMS m/z 780.3 [M+1]⁺.

¹H NMR (400 MHz, DMSO-d6) δ = 10.28 (br s, 1H), 9.54 (s, 1H), 9.11 - 8.95 (m, 1H), 8.57 (br d, J = 7.9 Hz, 1H), 8.33 - 8.23 (m, 2H), 7.66 - 7.48 (m, 2H), 7.29 - 7.17 (m, 2H), 6.91 - 6.72 (m, 2H), 4.79 (br dd, J = 2.4, 4.4 Hz, 1H), 4.31 - 4.19 (m, 1H), 4.12 (br s, 2H), 3.99 - 3.88 (m, 3H), 2.85 - 2.66 (m, 3H), 2.21 - 2.11 (m, 1H), 2.08 - 1.99 (m, 2H), 1.92 (br dd, J = 3.4, 7.9 Hz, 1H), 1.78 - 1.49 (m, 3H), 1.29 (br s, 6H).

### [Preparation Example 31]

### Preparation of Compounds 147 to 150

### 31-1. Preparation of Compound 147, N-(3'-(4-((2-hydroxyethyl)amino)-4,5,6,7-tetrahydropyrazolo[1,5-a]pyridine-2-carboxamido)-2,2'-dimethyl-[1,1'-biphenyl]-3-yl)-5-methyl-4,5,6,7-tetrahydrothiazolo[5,4-c]pyridine-2-carboxamide

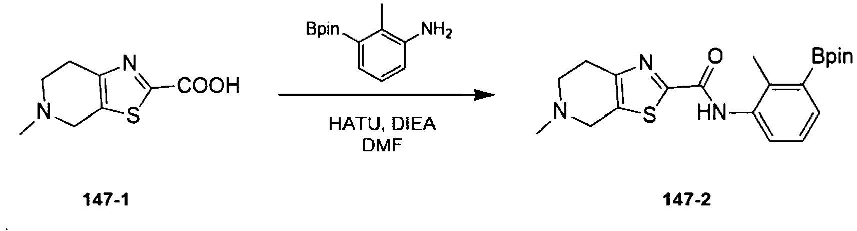

A solution of 5-methyl-6,7-dihydro-4H-thiazolo[5,4-c]pyridine-2-carboxylic acid (2.5 g, 12.61 mmol, 1 eq), 2-methyl-3-(4,4,5,5-tetramethyl-1,3,2-dioxaborolan-2-yl)aniline (2.94 g, 12.61 mmol, 1 eq), HATU (7.19 g, 18.92 mmol, 1.5 eq), and DIPEA (5.70 g, 44.14 mmol, 7.69 mL, 3.5 eq) in DMF (50 mL) was stirred at room temperature for 12 hours. After completion of the reaction, water (250 mL) was added to the mixture, and the resulting precipitate was collected by filtration to afford Compound 147-2 (3 g, 46.0% yield) as a white solid, which was used in the next step without further purification.

LCMS m/z 414.2 [M+1]⁺.

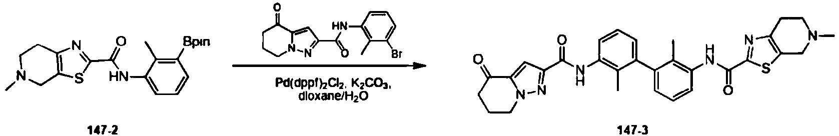

A mixture of N-(3-bromo-2-methylphenyl)-4-oxo-6,7-dihydro-5H-pyrazolo[1,5-a]pyridine-2-carboxamide (2 g, 5.74 mmol, 1 eq), 5-methyl-N-[2-methyl-3-(4,4,5,5-tetramethyl-1,3,2-dioxaborolan-2-yl)phenyl]-6,7-dihydro-4H-thiazolo[5,4-c]pyridine-2-carboxamide (2.61 g, 6.32 mmol, 1.1 eq), Pd(dppf)Cl₂ (420.29 mg, 574.39 µmol, 0.1 eq), and K₂CO₃ (2.38 g, 17.23 mmol, 3 eq) in dioxane (50 mL)/water (10 mL) was stirred at 90 °C for 12 hours under a nitrogen atmosphere. After completion of the reaction, the mixture was concentrated under reduced pressure. The residue was purified by flash silica gel chromatography (ISCO^{®}; 25 g SepaFlash^{®} Silica Flash Column; eluent: 0-10% ethyl acetate/methanol gradient @ 80 mL/min) to afford Compound 147-3 (2.39 g, 75.1% yield) as a yellow solid.

LCMS m/z 555.3 [M+1]⁺.

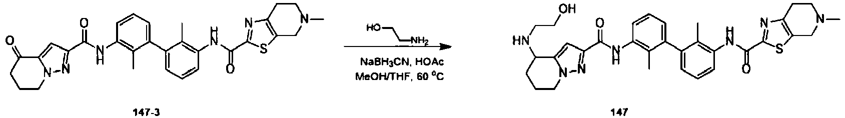

A mixture of 5-methyl-N-[2-methyl-3-[2-methyl-3-[(4-oxo-6,7-dihydro-5H-pyrazolo[1,5-a]pyridine-2-carbonyl)amino]phenyl]phenyl]-6,7-dihydro-4H-thiazolo[5,4-c]pyridine-2-carboxamide (200 mg, 360.58 µmol, 1 eq), 2-aminoethanol (220.25 mg, 3.61 mmol, 218.07 µL, 10 eq), and AcOH (21.65 mg, 360.58 µmol, 20.62 µL, 10 eq) in MeOH (10 mL)/THF (5 mL) was stirred at 60 °C for 1 hour. NaBH₃CN (113.30 mg, 1.80 mmol, 5 eq) was then slowly added, and the reaction mixture was further stirred at 60 °C for 11 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 35-65% B over 8 min) to afford Compound 147 (120 mg, 54.3% yield) as a white solid.

LCMS m/z 600.5 [M+1]⁺.

¹H NMR (400 MHz, DMSO-d6) δ = 10.24 (s, 1H), 9.52 (s, 1H), 7.57 (d, J = 7.9 Hz, 1H), 7.49 (d, J = 7.9 Hz, 1H), 7.28 (q, J = 7.6 Hz, 2H), 7.02 (d, J = 7.3 Hz, 1H), 6.96 (d, J = 7.2 Hz, 1H), 6.71 (s, 1H), 4.51 (t, J = 5.4 Hz, 1H), 4.14 (br t, J = 6.5 Hz, 2H), 3.88 (br s, 1H), 3.70 (s, 2H), 3.48 (q, J = 5.9 Hz, 2H), 2.92 - 2.87 (m, 2H), 2.80 - 2.75 (m, 2H), 2.68 (br s, 2H), 2.41 (s, 3H), 2.23 - 2.14 (m, 1H), 2.08 (br s, 1H), 2.01 (br s, 1H), 1.94 (d, J = 5.1 Hz, 7H), 1.69 (br d, J = 13.0 Hz, 1H)

### 31-2. Preparation of Compound 148, N-(3'-(4-((2-acetamidoethyl)amino)-4,5,6,7-tetrahydropyrazolo[1,5-a]pyridine-2-carboxamido)-2,2'-dimethyl-[1,1'-biphenyl]-3-yl)-5-methyl-4,5,6,7-tetrahydrothiazolo[5,4-c]pyridine-2-carboxamide

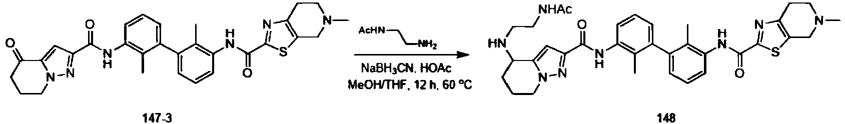

A mixture of 5-methyl-N-[2-methyl-3-[2-methyl-3-[(4-oxo-6,7-dihydro-5H-pyrazolo[1,5-a]pyridine-2-carbonyl)amino]phenyl]phenyl]-6,7-dihydro-4H-thiazolo[5,4-c]pyridine-2-carboxamide (200 mg, 360.58 µmol, 1 eq), N-(2-aminoethyl)acetamide (368.28 mg, 3.61 mmol, 344.18 µL, 10 eq), NaBH₃CN (113.30 mg, 1.80 mmol, 5 eq), and AcOH (21.65 mg, 360.58 µmol, 20.62 µL, 1 eq) in MeOH (10 mL)/THF (5 mL) was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 36-66% B over 8 min) to afford Compound 148 (120 mg, 50.9% yield) as a white solid.

LCMS m/z 641.5 [M+1]⁺.

¹H NMR (400 MHz, DMSO-d6) δ = 10.24 (s, 1H), 9.56 (s, 1H), 7.56 (d, J = 7.9 Hz, 1H), 7.49 (d, J = 7.7 Hz, 1H), 7.28 (q, J = 7.5 Hz, 2H), 7.02 (d, J = 7.3 Hz, 1H), 6.97 (d, J = 7.2 Hz, 1H), 6.75 (s, 1H), 4.14 (t, J = 6.4 Hz, 2H), 4.03 (t, J = 5.6 Hz, 1H), 3.70 (s, 3H), 3.29 (s, 4H), 2.90 (d, J = 5.3 Hz, 2H), 2.82 - 2.74 (m, 2H), 2.46 - 2.35 (m, 3H), 2.19 (d, J = 6.2 Hz, 1H), 2.00 (s, 1H), 1.94 (d, J = 5.5 Hz, 7H), 1.75 (s, 1H).

### 31-3. Preparation of Compound 149, 2-((2-((2,2'-dimethyl-3'-(5-methyl-4,5,6,7-tetrahydrothiazolo[5,4-c]pyridine-2-carboxamido)-[1,1'-biphenyl]-3-yl)carbamoyl)-4,5,6,7-tetrahydropyrazolo[1,5-a]pyridin-4-yl)amino)acetic acid

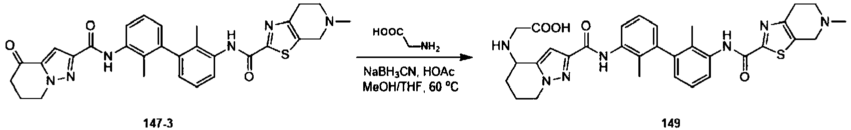

A mixture of 5-Methyl-N-[2-methyl-3-[2-methyl-3-[(4-oxo-6,7-dihydro-5H-pyrazolo[1,5-a]pyridine-2-carbonyl)amino]phenyl]phenyl]-6,7-dihydro-4H-thiazolo[5,4-c]pyridine-2-carboxamide (200 mg, 360.58 µmol, 1 eq), 2-aminoacetic acid (27.07 mg, 360.58 µmol, 10 eq), NaBH₃CN (113.30 mg, 1.80 mmol, 5 eq), and AcOH (21.65 mg, 360.58 µmol, 20.62 µL, 10 eq) in MeOH (10 mL)/THF (5 mL) was stirred at 60 °C for 12 hours. After completion of the reaction, the mixture was concentrated under reduced pressure. The residue was purified by prep-HPLC (column: Waters XBridge C18, 150 * 50 mm * 10 µm; mobile phase: water (NH₄HCO₃)-ACN; gradient: 20-50% B over 9 min) to afford Compound 149 (150 mg, 66.4% yield) as a white solid.

LCMS m/z 614.4 [M+1]⁺.

¹H NMR (400 MHz, DMSO-d6) δ = 10.24 (s, 1H), 9.53 (d, J = 2.6 Hz, 1H), 7.59 - 7.53 (m, 1H), 7.49 (d, J = 7.8 Hz, 1H), 7.28 (q, J = 7.8 Hz, 2H), 7.02 (d, J = 7.2 Hz, 1H), 6.97 (d, J = 7.3 Hz, 1H), 6.67 (d, J = 7.3 Hz, 1H), 4.70 (t, J = 5.0 Hz, 1H), 4.16 (d, J = 4.0 Hz, 3H), 3.79 (d, J = 4.8 Hz, 1H), 3.70 (s, 2H), 2.93 - 2.86 (m, 3H), 2.83 - 2.60 (m, 4H), 2.41 (s, 3H), 2.24 (s, 1H), 2.01 - 1.86 (m, 10H), 1.62 - 1.51 (m, 1H)

### 31-4. Preparation of Compound 150, N-(3'-(4-((R)-3-hydroxypyrrolidin-1-yl)-4,5,6,7-tetrahydropyrazolo[1,5-a]pyridine-2-carboxamido)-2,2'-dimethyl-[1,1'-biphenyl]-3-yl)-5-methyl-4,5,6,7-tetrahydrothiazolo[5,4-c]pyridine-2-carboxamide

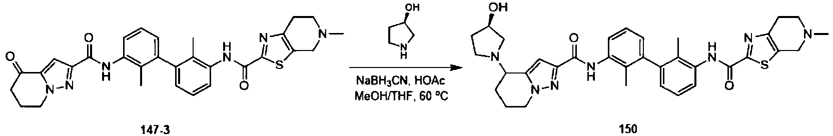

A mixture of 5-Methyl-N-[2-methyl-3-[2-methyl-3-[(4-oxo-6,7-dihydro-5H-pyrazolo[1,5-a]pyridine-2-carbonyl)amino]phenyl]phenyl]-6,7-dihydro-4H-thiazolo[5,4-c]pyridine-2-carboxamide (200 mg, 360.58 µmol, 1 eq), (3R)-pyrrolidin-3-ol (314.14 mg, 3.61 mmol, 299.18 µL, 10 eq), NaBH₃CN (113.30 mg, 1.80 mmol, 5 eq), and AcOH (21.65 mg, 360.58 µmol, 20.62 µL, 10 eq) in MeOH (10 mL)/THF (5 mL) was stirred at 60 °C for 12 hours. After completion of the reaction, the mixture was concentrated under reduced pressure. The residue was purified by prep-HPLC (column: Waters XBridge C18, 150 * 50 mm * 10 µm; mobile phase: water (NH₄HCO₃)-ACN; gradient: 40-70% B over 9 min) to afford Compound 150 (160 mg, 69.9% yield) as a white solid.

LCMS m/z 626.4 [M+1]⁺.

¹H NMR (400 MHz, DMSO-d6) δ = 10.24 (s, 1H), 9.52 (s, 1H), 7.83 (s, 1H), 7.57 (d, J = 7.9 Hz, 1H), 7.49 (d, J = 7.9 Hz, 1H), 7.28 (q, J = 7.5 Hz, 2H), 7.02 (d, J = 7.5 Hz, 1H), 6.96 (d, J = 7.5 Hz, 1H), 6.73 (s, 1H), 4.18 - 4.07 (m, 2H), 3.87 (s, 1H), 3.70 (s, 2H), 3.21 - 3.06 (m, 2H), 2.94 - 2.86 (m, 2H), 2.82 - 2.73 (m, 2H), 2.64 (s, 2H), 2.40 (s, 3H), 2.20 (s, 2H), 1.94 (d, J = 4.9 Hz, 7H), 1.80 (s, 3H), 1.74 - 1.62 (m, 1H)

### 31-5. Preparation of Compound 150-1, N-(2,2'-dichloro-3'-(4-((2-hydroxyethyl)amino)-4,5,6,7-tetrahydropyrazolo[1,5-a]pyridine-2-carboxamido)-[1,1'-biphenyl]-3-yl)-5-(2-fluoroethyl)-4,5,6,7-tetrahydrothiazolo[5,4-c]pyridine-2-carboxamide

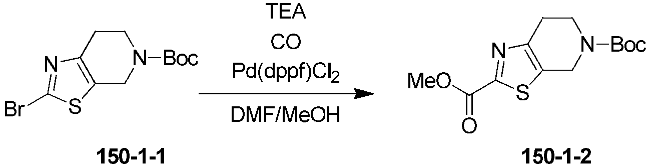

To a solution of tert-Butyl 2-bromo-6,7-dihydro-4H-thiazolo[5,4-c]pyridine-5-carboxylate (4.50 g, 14.10 mmol, 1 eq) in DMF (25 mL)/MeOH (25 mL), TEA (4.28 g, 42.29 mmol, 5.89 mL, 3 eq) and Pd(dppf)Cl₂ (515.74 mg, 704.85 µmol, 0.05 eq) were added, and the reaction mixture was stirred at 80 °C for 12 hours under CO atmosphere (45 psi). After completion of the reaction, the mixture was concentrated under reduced pressure. The residue was purified by column chromatography on silica gel (Petroleum ether/Ethyl acetate = 1/0 to 1/1) to afford Compound 150-1-2 (3.58 g, 68.0% yield) as a yellow solid.

LCMS m/z 299.1 [M+1]⁺.

¹H NMR (400 MHz, DMSO-d6) δ ppm 1.42 (s, 9 H) 2.84 (br t, J=5.75 Hz, 2 H) 3.65 - 3.72 (m, 2 H) 3.89 (s, 3 H) 4.70 (s, 2 H)

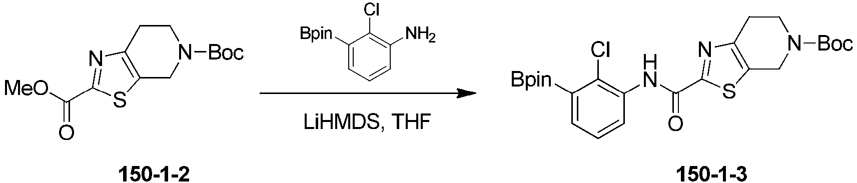

A mixture of 5-tert-butyl 2-methyl 6,7-dihydrothiazolo[5,4-c]pyridine-2,5(4H)-dicarboxylate (2.0 g, 6.70 mmol, 1 eq), 2-chloro-3-(4,4,5,5-tetramethyl-1,3,2-dioxaborolan-2-yl)aniline (4.59 g, 18.10 mmol, 2.7 eq), LiHMDS, (1 M, 16.76 mL, 2.5 eq) in THF (50 mL) was stirred under a nitrogen atmosphere at room temperature for 3 hours. After completion of the reaction, water (100 mL) was added, and the mixture was extracted with EtOAc (100 mL * 2). The combined organic layers were washed with brine (100 mL), dried over Na₂SO₄, filtered, and concentrated under reduced pressure. The residue was purified by reversed-phase prep-HPLC (column: Waters XBridge Prep OBD C18, 150 * 40 mm * 10 µm; mobile phase: water (NH₄HCO₃)-ACN; gradient: 65-95% B over 20 min) to afford Compound 150-1-3 (1.64 g, 46.5% yield) as a black solid.

LCMS m/z 520.1 [M+1]⁺.

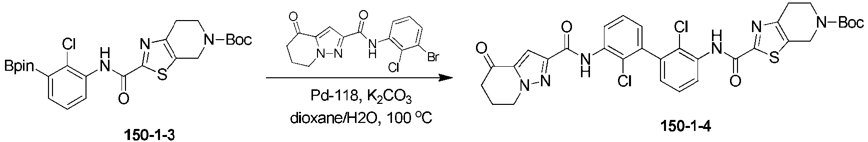

A mixture of N-(3-bromo-2-chloro-phenyl)-4-oxo-6,7-dihydro-5H-pyrazolo[1,5-a]pyridine-2-carboxamide (1.0 g, 2.71 mmol, 1 eq), tert-butyl 2-[[2-chloro-3-(4,4,5,5-tetramethyl-1,3,2-dioxaborolan-2-yl)phenyl]carbamoyl]-6,7-dihydro-4H-thiazolo[5,4-c]pyridine-5-carboxylate (1.55 g, 2.98 mmol, 1.1 eq), K₂CO₃ (1.12 g, 8.14 mmol, 3 eq), and ditert-butyl(cyclopentyl)phosphane-dichloropalladium-iron (176.81 mg, 271.29 µmol, 0.1 eq) in dioxane (30 mL)/water (6 mL) was stirred at 100 °C for 12 hours under a nitrogen atmosphere. After completion of the reaction, the mixture was cooled to room temperature and concentrated under reduced pressure. The residue was purified by column chromatography on silica gel (Petroleum ether/Ethyl acetate = 1/0 to 0/1) to afford Compound 150-1-4 (1.51 g, 73.5% yield) as a yellow solid.

LCMS m/z 681.1 [M+1]⁺.

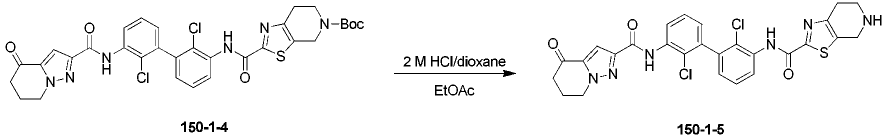

To a solution of tert-Butyl 2-[[2-chloro-3-[2-chloro-3-[(4-oxo-6,7-dihydro-5H-pyrazolo[1,5-a]pyridine-2-carbonyl)amino]phenyl]phenyl]carbamoyl]-6,7-dihydro-4H-thiazolo[5,4-c]pyridine-5-carboxylate (1.0 g, 1.47 mmol, 1 eq) in EtOAc (30 mL), HCl/dioxane (2 M, 366.79 µL, 0.50 eq) was added, and the reaction mixture was stirred at room temperature for 8 hours. After completion of the reaction, the mixture was concentrated under reduced pressure to afford Compound 150-1-5 (788 mg, 83.1% yield), which was used in the next step without further purification.

LCMS m/z 581.1 [M+1]⁺.

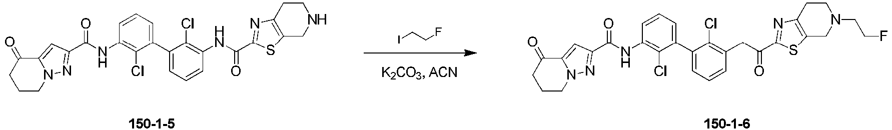

To a solution of N-[2-chloro-3-[2-chloro-3-[(4-oxo-6,7-dihydro-5H-pyrazolo[1,5-a]pyridine-2-carbonyl)amino]phenyl]phenyl]-4,5,6,7-tetrahydrothiazolo[5,4-c]pyridine-2-carboxamide (500 mg, 859.89 µmol, 1 eq) and 1-fluoro-2-iodoethane (2.99 g, 17.20 mmol, 20 eq) in CH₃CN (10 mL), K₂CO₃ (K₂CO₃; 594.22 mg, 4.30 mmol, 5 eq) was added, and the reaction mixture was stirred at 80 °C for 12 hours. After completion of the reaction, the mixture was cooled to room temperature and concentrated under reduced pressure. The residue was purified by column chromatography on silica gel (Petroleum ether/Ethyl acetate = 1/0 to 0/1) to afford Compound 150-1-6 (635 mg, 98.8% yield) as a white solid.

LCMS m/z 627.2 [M+1]⁺.

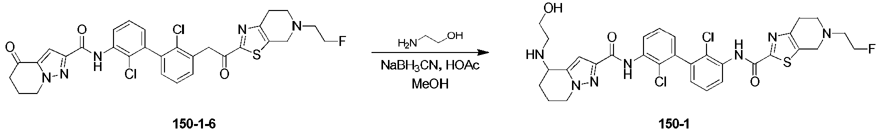

To a solution of N-[2-chloro-3-[2-chloro-3-[(4-oxo-6,7-dihydro-5H-pyrazolo[1,5-a]pyridine-2-carbonyl)amino]phenyl]phenyl]-5-(2-fluoroethyl)-6,7-dihydro-4H-thiazolo[5,4-c]pyridine-2-carboxamide (200 mg, 318.72 µmol, 1 eq) and 2-aminoethanol (194.68 mg, 3.19 mmol, 192.37 µL, 10 eq) in MeOH (3 mL), NaBH₃CN (100.14 mg, 1.59 mmol, 5 eq) and AcOH (95.70 mg, 1.59 mmol, 91.23 µL, 5 eq) were added, and the reaction mixture was stirred at 60 °C for 12 hours. After completion of the reaction, the mixture was cooled to room temperature and concentrated under reduced pressure. The residue was purified by reversed-phase HPLC (column: Waters XBridge, 150 * 25 mm * 5 µm; mobile phase: water (NH₄HCO₃)-ACN; gradient: 42-72% B over 9 min) to afford Compound 150-1 (66 mg, 30.48% yield) as a white solid.

LCMS m/z 672.3 [M+1]⁺.

¹H NMR (400 MHz, DMSO-d6) δ ppm 1.19 - 1.27 (m, 1 H) 1.63 - 1.75 (m, 1 H) 1.86 - 2.06 (m, 2 H) 2.13 - 2.23 (m, 1 H) 2.63 - 2.74 (m, 2 H) 2.79 - 2.99 (m, 6 H) 3.49 (br s, 2 H) 3.85 - 3.95 (m, 3 H) 4.08 - 4.21 (m, 2 H) 4.46 - 4.60 (m, 2 H) 4.68 (t, J=4.82 Hz, 1 H) 6.78 (s, 1 H) 7.11 - 7.18 (m, 1 H) 7.24 (dd, J=7.63, 1.38 Hz, 1 H) 7.50 (q, J=8.17 Hz, 2 H) 8.10 (dd, J=8.19, 1.31 Hz, 1 H) 8.30 (dd, J=8.19, 1.06 Hz, 1 H) 9.54 (s, 1 H) 10.12 (br s, 1 H).

### 31-6. Preparation of Compound 150-2, 2-((2-((2,2'-dichloro-3'-(5-(2-fluoroethyl)-4,5,6,7-tetrahydrothiazolo[5,4-c]pyridine-2-carboxamido)-[1,1'-biphenyl]-3-yl)carbamoyl)-4,5,6,7-tetrahydropyrazolo[1,5-a]pyridin-4-yl)amino)acetic acid

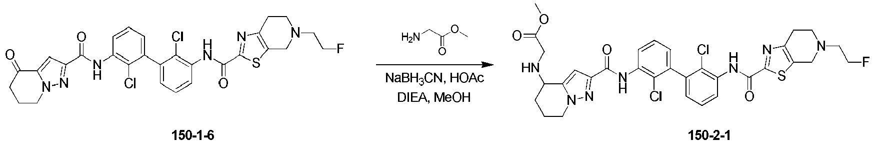

To a solution of N-[2-chloro-3-[2-chloro-3-[(4-oxo-6,7-dihydro-5H-pyrazolo[1,5-a]pyridine-2-carbonyl)amino]phenyl]phenyl]-5-(2-fluoroethyl)-6,7-dihydro-4H-thiazolo[5,4-c]pyridine-2-carboxamide (200 mg, 318.72 µmol, 1 eq), and methyl 2-aminoacetate hydrochloride (400.16 mg, 3.19 mmol, 10 eq) in MeOH (5 mL), NaBH₃CN (100.14 mg, 1.59 mmol, 5 eq), AcOH (95.69 mg, 1.59 mmol, 91.22 µL, 5 eq), and DIPEA (411.92 mg, 3.19 mmol, 555.15 µL, 10 eq) were added, and the reaction mixture was stirred at 60 °C for 12 hours. After completion of the reaction, the mixture was cooled to room temperature and concentrated under reduced pressure. The residue was purified by column chromatography on silica gel (Petroleum ether/Ethyl acetate = 1/0 to 0/1) to afford Compound 150-2-1 (243 mg, 96.8% yield) as a white solid.

LCMS m/z 702.2 [M+3]⁺.

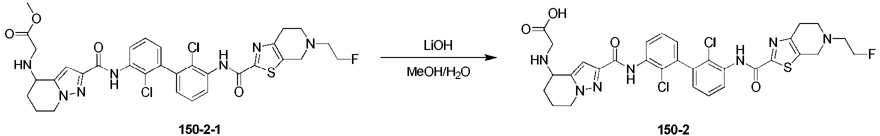

To a solution of methyl 2-[[2-[[2-chloro-3-[2-chloro-3-[[5-(2-fluoroethyl)-6,7-dihydro-4H-thiazolo[5,4-c]pyridine-2-carbonyl]amino]phenyl]phenyl]carbamoyl]-4,5,6,7-tetrahydropyrazolo[1,5-a]pyridin-4-yl]amino]acetate (200 mg, 285.47 µmol, 1 eq) in MeOH (5 mL)/water (1 mL), LiOH·H₂O (35.94 mg, 856.40 µmol, 3 eq) was added, and the reaction mixture was stirred at room temperature for 12 hours. After completion of the reaction, the mixture was concentrated under reduced pressure. The residue was purified by reversed-phase HPLC (column: Waters XBridge, 150 * 25 mm, 5 µm; mobile phase: water (NH₄HCO₃)-ACN; gradient: 22-52% B over 9 min) to afford Compound 150-2 (41 mg, 20.7% yield) as a white solid.

LCMS m/z 688.2 [M+3]⁺.

¹H NMR (400 MHz, DMSO-d6) δ ppm 1.69 - 1.79 (m, 1 H) 1.85 - 1.96 (m, 1 H) 1.96 - 2.06 (m, 1 H) 2.15 - 2.26 (m, 1 H) 2.85 (br t, J=4.75 Hz, 1 H) 2.92 (br dd, J=6.44, 4.31 Hz, 5 H) 3.29 (s, 3 H) 3.89 (s, 2 H) 4.05 (br t, J=5.75 Hz, 1 H) 4.15 (br t, J=5.94 Hz, 2 H) 4.56 (t, J=4.82 Hz, 1 H) 4.68 (t, J=4.75 Hz, 1 H) 6.82 (s, 1 H) 7.16 (dd, J=7.63, 1.38 Hz, 1 H) 7.24 (d, J=7.50 Hz, 1 H) 7.50 (q, J=8.09 Hz, 2 H) 8.09 (dd, J=8.13, 1.25 Hz, 1 H) 8.26 (br d, J=8.13 Hz, 1 H) 9.57 (s, 1 H).

### [Preparation Example 32]

### Preparation of Compounds 151 to 154

### 32-1. Preparation of Compound 151, N-(2,2'-dimethyl-3'-(5-methyl-4,5,6,7-tetrahydrothiazolo[4,5-c]pyridin-2-yl)-[1,1'-biphenyl]-3-yl)-4-((2-hydroxyethyl)amino)-4,5,6,7-tetrahydropyrazolo[1,5-a]pyridine-2-carboxamide

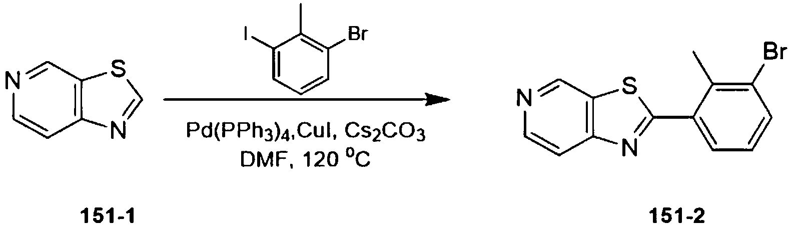

A mixture of thiazolo[4,5-c]pyridine (200 mg, 1.47 mmol, 1 eq), 1-bromo-3-iodo-2-methylbenzene (479.71 mg, 1.62 mmol, 1.1 eq), Pd(PPh₃)₄ (169.72 mg, 146.87 µmol, 0.1 eq), Cul (27.97 mg, 146.87 µmol, 0.1 eq), and Cs₂CO₃ (1.44 g, 4.41 mmol, 3 eq) in DMF (10 mL) was stirred at 120 °C for 12 hours under a nitrogen atmosphere. After completion of the reaction, the mixture was filtered and the filtrate was concentrated under reduced pressure. The residue was purified by flash silica gel chromatography (ISCO^{®}; 25 g SepaFlash^{®} Silica Flash Column; eluent: 0-50% ethyl acetate/petroleum ether gradient @ 100 mL/min) to afford Compound 151-2 (250 mg, 39.0% yield) as a yellow oil.

LCMS m/z 305.0 [M+1]⁺.

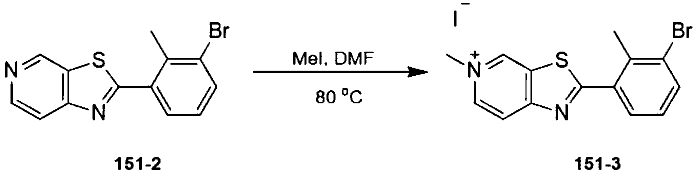

A solution of 2-(3-Bromo-2-methylphenyl)thiazolo[4,5-c]pyridine (250 mg, 819.15 µmol, 1 eq) and iodomethane (232.54 mg, 1.64 mmol, 101.99 µL, 2 eq) in DMF (10 mL) was stirred at 80 °C for 4 hours. After completion of the reaction, the mixture was concentrated under reduced pressure to afford Compound 151-3, which was used in the next step without further purification.

LCMS m/z 319.1 [M-127]⁺.

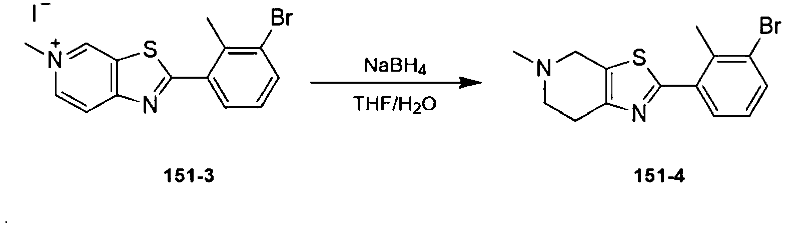

To a solution of 2-(3-Bromo-2-methylphenyl)-5-iodo-5-methylthiazolo[4,5-c]pyridine (250 mg, 559.12 µmol, 1 eq) in THF (8 mL)/water (8 mL), NaBH₄ solution (84.74 mg, 2.24 mmol, 4 eq) was slowly added, and the reaction mixture was stirred at room temperature for 4 hours. After completion of the reaction, an aq. saturated NH₄Cl solution (10 mL) was added, and the mixture was extracted with ethyl acetate (10 mL * 3). The combined organic layers were dried over Na₂SO₄ and concentrated under reduced pressure. The residue was purified by flash silica gel chromatography (ISCO^{®}; 12 g SepaFlash^{®} Silica Flash Column; eluent: 0-50% ethyl acetate/petroleum ether gradient @ 80 mL/min) to afford Compound 151-4 (130 mg, 71.9% yield) as a yellow oil.

LCMS m/z 323.0 [M+1]⁺.

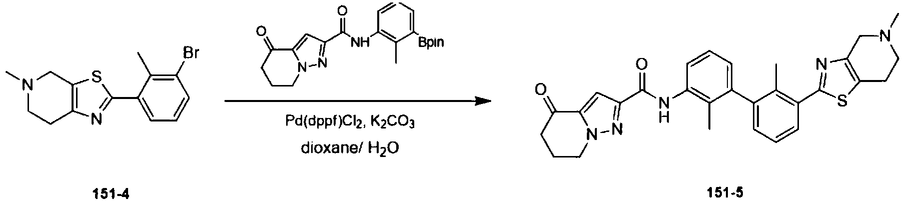

A mixture of 2-(3-bromo-2-methylphenyl)-5-methyl-6,7-dihydro-4H-thiazolo[4,5-c]pyridine (130 mg, 402.16 µmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (190.75 mg, 482.60 µmol, 1.2 eq), Pd(dppf)Cl₂ (29.43 mg, 40.22 µmol, 0.1 eq), and K₂CO₃ (166.74 mg, 1.21 mmol, 3 eq) in dioxane (20 mL)/water (5 mL) was stirred at 80 °C for 4 hours under a nitrogen atmosphere. After completion of the reaction, the mixture was concentrated under reduced pressure. The residue was purified by flash silica gel chromatography (ISCO^{®}; 25 g SepaFlash^{®} Silica Flash Column; eluent: 0-50% methanol/ethyl acetate gradient @ 100 mL/min) to afford Compound 151-5 (200 mg, 48.6% yield) as a yellow oil.

LCMS m/z 512.2 [M+1]⁺.

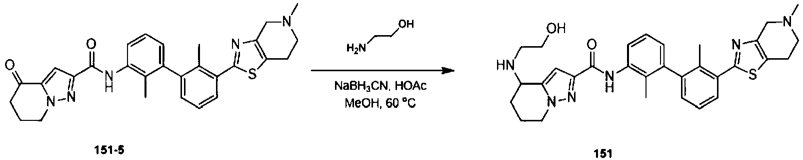

A solution of N-[2-Methyl-3-[2-methyl-3-(5-methyl-6,7-dihydro-4H-thiazolo[4,5-c]pyridin-2-yl)phenyl]phenyl]-4-oxo-6,7-dihydro-5H-pyrazolo[1,5-a]pyridine-2-carboxamide (100 mg, 195.45 µmol, 1 eq), 2-aminoethanol (119.39 mg, 1.95 mmol, 118.21 µL, 10 eq), and HOAc (11.74 mg, 195.45 µmol, 11.18 µL, 1 eq) in MeOH (15 mL) was stirred at 60 °C for 1 hour. NaBH₃CN (61.41 mg, 977.26 µmol, 5 eq) was then slowly added, and the mixture was further stirred at 60 °C for 3 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 30-60% B over 9 min) to afford Compound 151 (10 mg, 6.0% yield) as a yellow solid.

LCMS m/z 557.4 [M+1]⁺.

¹H NMR (400 MHz, DMSO-d6) δ = 9.53 (s, 1H), 7.67 - 7.55 (m, 2H), 7.37 (t, J = 7.6 Hz, 1H), 7.28 (t, J = 7.8 Hz, 1H), 7.20 (d, J = 7.4 Hz, 1H), 6.99 (d, J = 7.4 Hz, 1H), 6.73 (s, 1H), 4.56 (s, 1H), 4.21 - 4.07 (m, 2H), 3.89 (t, J = 5.9 Hz, 1H), 3.57 (s, 2H), 3.52 - 3.46 (m, 2H), 2.90 (s, 2H), 2.77 - 2.63 (m, 4H), 2.41 (s, 3H), 2.16 (s, 4H), 2.12 - 1.97 (m, 2H), 1.94 (s, 4H), 1.78 - 1.61 (m, 1H).

### 32-2. Preparation of Compound 152, 4-((2-acetamidoethyl)amino)-N-(2,2'-dimethyl-3'-(5-methyl-4,5,6,7-tetrahydrothiazolo[4,5-c]pyridin-2-yl)-[1,1'-biphenyl]-3-yl)-4,5,6,7-tetrahydropyrazolo[1,5-a]pyridine-2-carboxamide

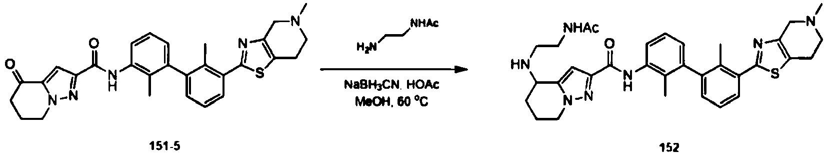

A mixture of N-(2-Aminoethyl)acetamide (299.43 mg, 2.93 mmol, 279.85 µL, 10 eq), N-[2-methyl-3-[2-methyl-3-(5-methyl-6,7-dihydro-4H-thiazolo[4,5-c]pyridin-2-yl)phenyl]phenyl]-4-oxo-6,7-dihydro-5H-pyrazolo[1,5-a]pyridine-2-carboxamide (150 mg, 293.18 µmol, 1 eq), NaBH₃CN (92.12 mg, 1.47 mmol, 5 eq), and HOAc (17.61 mg, 293.18 µmol, 16.77 µL, 1 eq) in methanol (25 mL) was stirred at 60 °C for 4 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 28-58% B over 9 min) to afford Compound 152 (35 mg, 19.5% yield) as a yellow solid.

LCMS m/z 598.5 [M+1]⁺.

¹H NMR (400 MHz, DMSO-d6) δ = 9.52 (s, 1H), 7.84 (s, 1H), 7.61 (t, J = 7.1 Hz, 2H), 7.38 (t, J = 7.6 Hz, 1H), 7.29 (t, J = 7.7 Hz, 1H), 7.20 (d, J = 7.5 Hz, 1H), 6.99 (d, J = 7.4 Hz, 1H), 6.74 (s, 1H), 4.14 (s, 2H), 3.88 (t, J = 5.7 Hz, 1H), 3.64 - 3.51 (m, 2H), 3.23 - 3.05 (m, 3H), 2.91 (s, 2H), 2.80 - 2.57 (m, 4H), 2.41 (s, 3H), 2.16 (s, 4H), 2.04 - 1.96 (m, 1H), 1.94 (s, 4H), 1.81 (s, 3H), 1.76 - 1.59 (m, 1H).

### 32-3. Preparation of Compound 153, 2-((2-((2,2'-dimethyl-3'-(5-methyl-4,5,6,7-tetrahydrothiazolo[4,5-c]pyridin-2-yl)-[1,1'-biphenyl]-3-yl)carbamoyl)-4,5,6,7-tetrahydropyrazolo[1,5-a]pyridin-4-yl)amino)acetic acid

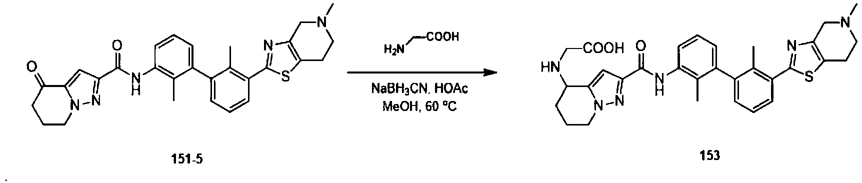

A mixture of N-[2-Methyl-3-[2-methyl-3-(5-methyl-6,7-dihydro-4H-thiazolo[4,5-c]pyridin-2-yl)phenyl]phenyl]-4-oxo-6,7-dihydro-5H-pyrazolo[1,5-a]pyridine-2-carboxamide (150 mg, 293.18 µmol, 1 eq), 2-aminoacetic acid (220.08 mg, 2.93 mmol, 10 eq), NaBH₃CN (92.12 mg, 1.47 mmol, 5 eq), HOAc (17.61 mg, 293.18 µmol, 16.77 µL, 1 eq) in MeOH (15 mL) was stirred at 60 °C for 12 hours. After completion of the reaction, the mixture was concentrated under reduced pressure. The residue was purified by prep-HPLC (column: Waters XBridge, 150 * 25 mm * 5 µm; mobile phase: water (NH₄HCO₃)-ACN; gradient: 15-45% B over 9 min) to afford Compound 153 (54 mg, 31.9% yield) as a white solid.

LCMS m/z 571.5 [M+1]⁺.

¹H NMR (400 MHz, DMSO-d6) δ = 9.57 (s, 1H), 7.65 - 7.56 (m, 2H), 7.38 (t, J = 7.7 Hz, 1H), 7.28 (t, J = 7.8 Hz, 1H), 7.20 (d, J = 6.8 Hz, 1H), 7.00 (d, J = 6.9 Hz, 1H), 6.78 (s, 1H), 4.14 (t, J = 6.1 Hz, 2H), 4.07 (t, J = 5.8 Hz, 1H), 3.59 - 3.56 (m, 4H), 3.31 (s, 2H), 2.91 (t, J = 5.5 Hz, 2H), 2.78 - 2.65 (m, 2H), 2.41 (s, 3H), 2.24 - 2.18 (m, 1H), 2.16 (s, 3H), 2.11 - 1.97 (m, 1H), 1.95 - 1.86 (m, 4H), 1.84 - 1.65 (m, 1H).

### 32-4. Preparation of Compound 154, N-(2,2'-dimethyl-3'-(5-methyl-4,5,6,7-tetrahydrothiazolo[4,5-c]pyridin-2-yl)-[1,1'-biphenyl]-3-yl)-4-((R)-3-hydroxypyrrolidin-1-yl)-4,5,6,7-tetrahydropyrazolo[1,5-a]pyridine-2-carboxamide

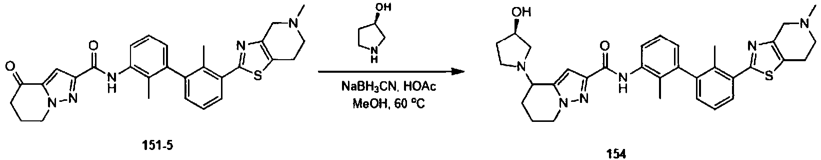

A mixture of (3R)-pyrrolidin-3-ol (255.42 mg, 2.93 mmol, 243.25 µL, 10 eq), N-[2-methyl-3-[2-methyl-3-(5-methyl-6,7-dihydro-4H-thiazolo[4,5-c]pyridin-2-yl)phenyl]phenyl]-4-oxo-6,7-dihydro-5H-pyrazolo[1,5-a]pyridine-2-carboxamide (150 mg, 293.18 µmol, 1 eq), NaBH₃CN (92.12 mg, 1.47 mmol, 5 eq), and HOAc (17.61 mg, 293.18 µmol, 16.77 µL, 1 eq) in methanol (25 mL) was stirred at 60 °C for 4 hours. After completion of the reaction, the mixture was concentrated under reduced pressure. The residue was purified by prep-HPLC (column: Waters XBridge, 150 * 25 mm * 5 um; mobile phase: water (NH₄HCO₃)-ACN; gradient: 36-66% B over 9 min) to afford Compound 154 (30 mg, 16.6% yield) as a yellow solid.

LCMS m/z 583.28 [M+1]⁺.

¹H NMR (400 MHz, DMSO-d6) δ = 9.55 (d, J = 3.1 Hz, 1H), 7.64 - 7.55 (m, 2H), 7.38 (t, J = 7.6 Hz, 1H), 7.28 (t, J = 7.8 Hz, 1H), 7.20 (d, J = 6.8 Hz, 1H), 6.99 (d, J = 7.3 Hz, 1H), 6.68 (d, J = 7.1 Hz, 1H), 4.79 - 4.70 (m, 1H), 4.23 - 4.10 (m, 3H), 3.79 (d, J = 4.6 Hz, 1H), 3.57 (s, 2H), 2.98 - 2.56 (m, 7H), 2.45 - 2.37 (m, 4H), 2.24 (s, 1H), 2.16 (s, 3H), 1.96 - 1.85 (m, 6H), 1.57 (dd, J = 4.4, 7.8 Hz, 1H).

### [Preparation Example 33]

### Preparation of Compounds 155 to 157

### 33-1. Preparation of Compound 155, 2,2'-((2,2'-(2,2'-dimethyl-[1,1'-biphenyl]-3,3'-diyl)bis(4,5,6,7-tetrahydropyrazolo[1,5-a]pyridine-4,2-diyl))bis(azanediyl))diethanol

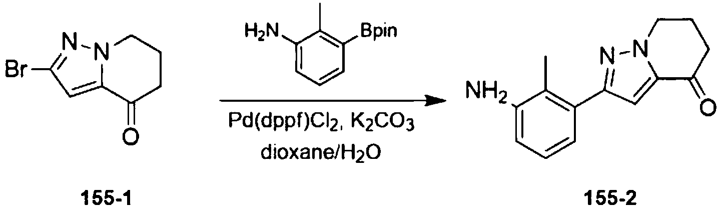

A mixture of 2-bromo-6,7-dihydro-5H-pyrazolo[1,5-a]pyridin-4-one (5 g, 23.25 mmol, 1 eq), 2-methyl-3-(4,4,5,5-tetramethyl-1,3,2-dioxaborolan-2-yl)aniline (5.96 g, 25.58 mmol, 1.1 eq), K₂CO₃ (9.00 g, 65.10 mmol, 2.8 eq), and Pd(dppf)Cl₂ (850.63 mg, 1.16 mmol, 0.05 eq) in dioxane (50 mL)/H₂O (5 mL) was stirred at 90 °C for 12 hours under a nitrogen atmosphere. After completion of the reaction, the reaction mixture was concentrated under reduced pressure. The residue was purified by silica gel column chromatography (Petroleum ether/Ethyl acetate = 1/1 to 0/1) to afford Compound 155-2 (2.10 g, 37.4% yield) as a yellow liquid.

LCMS m/z 242.2 [M+1]⁺.

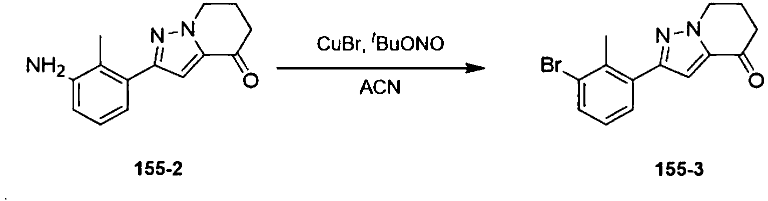

A mixture of 2-(3-amino-2-methylphenyl)-6,7-dihydro-5H-pyrazolo[1,5-a]pyridin-4-one (2.1 g, 8.70 mmol, 1 eq), CuBr (2.50 g, 17.41 mmol, 2 eq), and tert-butylnitrite (1.08 g, 10.44 mmol, 1.2 eq) in ACN (50 mL) was stirred at 0 °C for 12 hours under a nitrogen atmosphere. After completion of the reaction, the mixture was diluted with water (250 mL) and extracted with ethyl acetate (250 mL). The organic layer was concentrated under reduced pressure, and the residue was purified by silica gel column chromatography (Petroleum ether/Ethyl acetate = 3/1 to 2/1) to afford Compound 155-3 (720 mg, 27.1% yield) as a yellow solid.

LCMS m/z 304.02 [M+1]⁺.

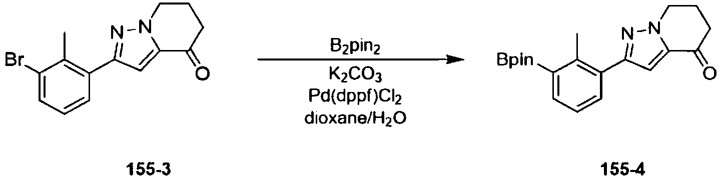

A mixture of 2-(3-bromo-2-methylphenyl)-6,7-dihydro-5H-pyrazolo[1,5-a]pyridin-4-one (300 mg, 983.06 µmol, 1 eq), 4,4,5,5-tetramethyl-2-(4,4,5,5-tetramethyl-1,3,2-dioxaborolan-2-yl)-1,3,2-dioxaborolane (499.27 mg, 1.97 mmol, 2 eq), Pd(dppf)Cl₂ (71.93 mg, 98.31 µmol, 0.1 eq), and KOAc (289.44 mg, 2.95 mmol, 3 eq) in dioxane (10 mL) was stirred at 80 °C for 12 hours under a nitrogen atmosphere. After completion of the reaction, the mixture was concentrated under reduced pressure, and the residue was purified by silica gel column chromatography (Petroleum ether/Ethyl acetate = 3/1 to 2/1) to afford Compound 155-4 (460 mg, 98.3% yield) as a yellow liquid.

LCMS m/z 352.23 [M+1]⁺.

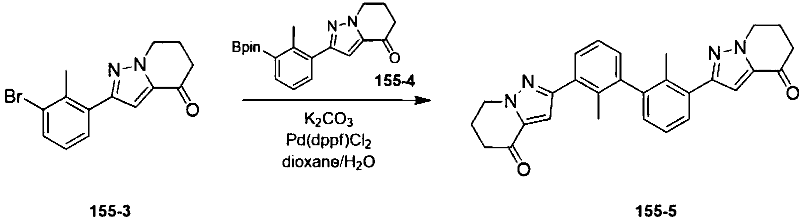

A mixture of 2-(3-bromo-2-methylphenyl)-6,7-dihydro-5H-pyrazolo[1,5-a]pyridin-4-one (400 mg, 1.31 mmol, 1 eq), 2-[2-methyl-3-(4,4,5,5-tetramethyl-1,3,2-dioxaborolan-2-yl)phenyl]-6,7-dihydro-5H-pyrazolo[1,5-a]pyridin-4-one (461.69 mg, 1.31 mmol, 1 eq), K₂CO₃ (543.48 mg, 3.93 mmol, 3 eq), and Pd(dppf)Cl₂ (95.91 mg, 131.07 µmol, 0.1 eq) in dioxane (8 mL)/H₂O (2 mL) was stirred at 80 °C for 12 hours under a nitrogen atmosphere. After completion of the reaction, the mixture was concentrated under reduced pressure, and the residue was purified by silica gel column chromatography (Petroleum ether/Ethyl acetate = 1/1 to 0/1) to afford Compound 155-5 (248 mg, 42.0% yield) as a yellow liquid.

LCMS m/z 451.2 [M+1]⁺.

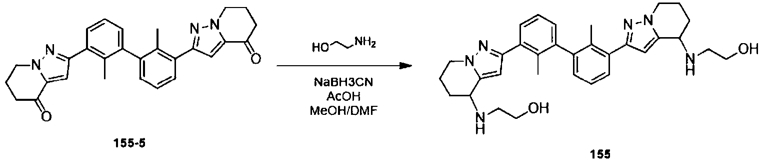

To a solution of 2-[2-methyl-3-[2-methyl-3-(4-oxo-6,7-dihydro-5H-pyrazolo[1,5-a]pyridin-2-yl)phenyl]phenyl]-6,7-dihydro-5H-pyrazolo[1,5-a]pyridin-4-one (100 mg, 221.96 µmol, 1 eq) and 2-aminoethanol (203.37 mg, 3.33 mmol, 200.96 µL, 15 eq) in MeOH (10 mL)/DMF (2 mL), NaBH₃CN (139.48 mg, 2.22 mmol, 10 eq) and AcOH (133.29 mg, 2.22 mmol, 127.06 µL, 10 eq) were added, and the resulting mixture was stirred at 60 C for 4 hours. After completion of the reaction, the mixture was concentrated under reduced pressure, and the residue was purified by reversed-phase HPLC (column: Waters Xbridge, 150 * 25 mm * 5 um; mobile phase: [water (NH₄HCO₃)-ACN]; gradient: 20-50% B over 9 min) to afford Compound 155 (14 mg, 11.5% yield) as a white solid.

LCMS m/z 541.4 [M+1]⁺.

¹H NMR (400 MHz, DMSO-d6) δ = 7.49 (br d, J = 7.8 Hz, 2H), 7.28 (t, J = 7.6 Hz, 2H), 7.06 (br d, J = 7.6 Hz, 2H), 6.43 (s, 2H), 4.53 (br t, J = 4.9 Hz, 2H), 4.08 (br t, J = 6.0 Hz, 4H), 3.89 (br t, J = 5.9 Hz, 2H), 3.52 - 3.46 (m, 4H), 2.77 - 2.66 (m, 4H), 2.22 - 2.00 (m, 12H), 1.96 - 1.84 (m, 2H), 1.76 - 1.62 (m, 2H).

### 33-2. Preparation of Compound 156, (3R,3'R)-1,1'-(2,2'-(2,2'-dimethyl-[1,1'-biphenyl]-3,3'-diyl)bis(4,5,6,7-tetrahydropyrazolo[1,5-a]pyridine-4,2-diyl))bis(pyrrolidin-3-ol)

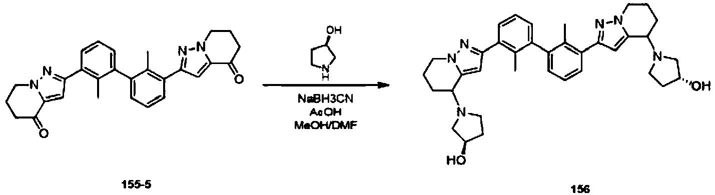

To a solution of 2-[2-methyl-3-[2-methyl-3-(4-oxo-6,7-dihydro-5H-pyrazolo[1,5-a]pyridin-2-yl)phenyl]phenyl]-6,7-dihydro-5H-pyrazolo[1,5-a]pyridin-4-one (100 mg, 221.96 µmol, 1 eq) and (3R)-pyrrolidin-3-ol (290.06 mg, 3.33 mmol, 276.77 µL, 15 eq) in MeOH (10 mL)/DMF (2 mL), NaBH₃CN (139.48 mg, 2.22 mmol, 10 eq) and AcOH (133.29 mg, 2.22 mmol, 127.07 µL, 10 eq) were added, and the resulting mixture was stirred at 60 C for 3 hours. After completion of the reaction, the mixture was concentrated under reduced pressure, and the residue was purified by reversed-phase HPLC (column: Waters Xbridge, 150 * 25 mm * 5 um; mobile phase: [water (NH₄HCO₃)-ACN]; gradient: 28-58% B over 9 min) to afford Compound 156 (25 mg, 18.2% yield) as a white solid.

LCMS m/z 592.35 [M+1]⁺.

¹H NMR (400 MHz, DMSO-d6) δ = 7.49 (d, J = 7.6 Hz, 2H), 7.27 (t, J = 7.6 Hz, 2H), 7.06 (dd, J = 3.1, 7.1 Hz, 2H), 6.40 (d, J = 7.2 Hz, 2H), 4.69 (br s, 2H), 4.22 - 4.03 (m, 6H), 3.76 (br s, 2H), 2.91 (dd, J = 6.2, 9.5 Hz, 1H), 2.85 - 2.60 (m, 4H), 2.56 - 2.52 (m, 2H), 2.48 - 2.41 (m, 1H), 2.25 (br s, 2H), 2.12 (d, J = 3.5 Hz, 6H), 2.01 - 1.84 (m, 8H), 1.56 (br dd, J = 4.4, 7.7 Hz, 2H)

### 33-3. Preparation of Compound 157, (3R)-1-(2-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridin-4-yl)pyrrolidin-3-ol

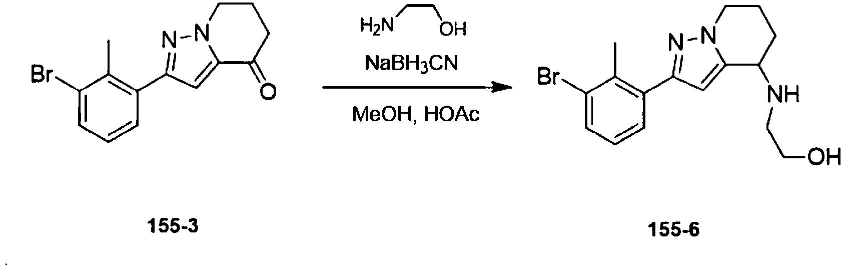

To a solution of 2-(3-bromo-2-methyl-phenyl)-6,7-dihydro-5H-pyrazolo[1,5-a]pyridin-4-one (200 mg, 655.37 µmol, 1 eq) and 2-aminoethanol (200.16 mg, 3.28 mmol, 197.79 µL, 5 eq) in MeOH (5 mL), HOAc (393.57 mg, 6.55 mmol, 375.18 µL, 10 eq) and NaBH₃CN (411.85 mg, 6.55 mmol, 10 eq) were added, and the resulting mixture was stirred at 50 C for 4 hours. After completion of the reaction, the mixture was concentrated under reduced pressure, and the residue was purified by prep-TLC (SiO₂, DCM:MeOH = 10:1) to afford Compound 155-6 (150 mg, 55.2% yield) as a yellow solid.

LCMS m/z 250.1 [M+1]⁺.

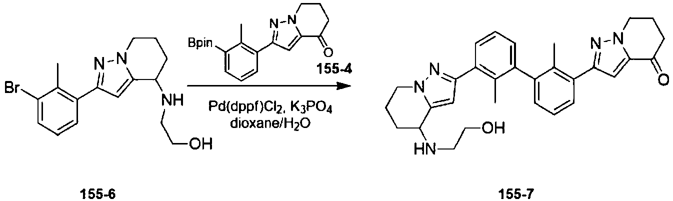

To a mixture of 2-[2-methyl-3-(4,4,5,5-tetramethyl-1,3,2-dioxaborolan-2-yl)phenyl]-6,7-dihydro-5H-pyrazolo[1,5-a]pyridin-4-one (200 mg, 567.80 µmol, 1.1 eq) and 2-[[2-(3-bromo-2-methylphenyl)-4,5,6,7-tetrahydropyrazolo[1,5-a]pyridin-4-yl]amino]ethanol (150 mg, 428.26 umol) in dioxane (5 mL)/H₂O (1 mL), Pd(dppf)Cl₂ (37.77 mg, 51.62 µmol, 0.1 eq) and K₃PO₄ (328.71 mg, 1.55 mmol, 3 eq) were added, and the resulting mixture was stirred at 80 C for 16 hours under a nitrogen atmosphere. After completion of the reaction, the mixture was concentrated under reduced pressure, and the residue was purified by prep-TLC (SiO₂, DCM:MeOH = 8:1) to afford Compound 155-7 (180 mg, 70.3% yield) as a white solid.

LCMS m/z 496.3 [M+1]⁺.

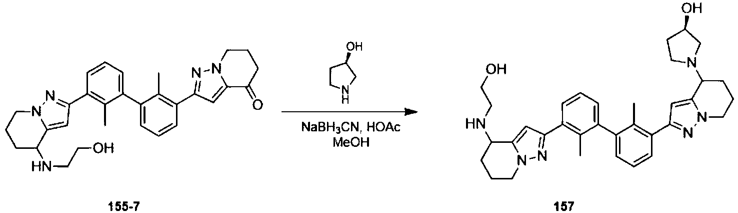

To a solution of (3R)-pyrrolidin-3-ol (316.41 mg, 3.63 mmol, 301.92 µL, 10 eq) and 2-[3-[3-[4-(2-hydroxyethylamino)-4,5,6,7-tetrahydropyrazolo[1,5-a]pyridin-2-yl]-2-methyl-phenyl]-2-methylphenyl]-6,7-dihydro-5H-pyrazolo[1,5-a]pyridin-4-one (180 mg, 363.19 µmol, 1 eq) in MeOH (20 mL), NaBH₃CN (182.58 mg, 2.91 mmol, 8 eq) and AcOH (218.09 mg, 3.63 mmol, 207.91 µL, 10 eq) were added, and the resulting mixture was stirred at 60 °C for 3 hours. After completion of the reaction, the mixture was concentrated under reduced pressure, and the residue was purified by reversed-phase HPLC (column: Waters Xbridge, 150 * 25 mm * 5 um; mobile phase: water (NH₄HCO₃)-ACN; B%: 22% to 52% over 9 min) to afford Compound 157 (186 mg, 87.6% yield) as a white solid.

LCMS m/z 567.3 [M+1]⁺.

¹H NMR (400 MHz, DMSO-d6) δ = 7.51 - 7.47 (m, J = 7.6 Hz, 2H), 7.28 (t, J = 7.5 Hz, 2H), 7.09 - 7.05 (m, J = 7.6 Hz, 2H), 6.48 (br s, 1H), 6.40 (br d, J = 7.6 Hz, 1H), 4.68 (br s, 2H), 4.19 (br s, 1H), 4.11 (br d, J = 6.0 Hz, 4H), 3.78 (br s, 1H), 3.53 (br s, 2H), 2.97 - 2.85 (m, 1H), 2.79 (br s, 3H), 2.47 - 2.40 (m, 1H), 2.21 (br s, 2H), 2.11 (t, J = 3.2 Hz, 6H), 2.08 - 2.02 (m, 1H), 1.90 (br s, 7H), 1.63 - 1.49 (m, 1H).

### [Preparation Example 34]

### Preparation of Compounds 158 to 161

### 34-1. Preparation of Compound 158, 4-((2-hydroxyethyl)amino)-N-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridine-2-carboxamide

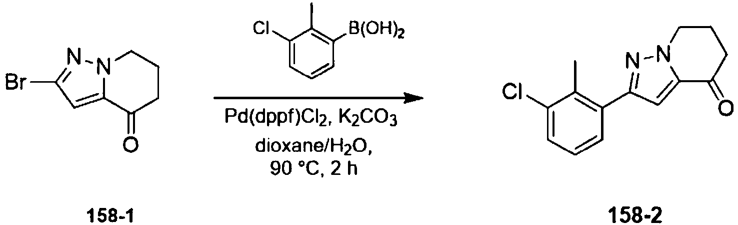

A mixture of 2-bromo-6,7-dihydro-5H-pyrazolo[1,5-a]pyridin-4-one (1 g, 4.65 mmol, 1 eq), (3-chloro-2-methylphenyl)boronic acid (871.63 mg, 5.12 mmol, 1.1 eq), K₂CO₃ (1.80 g, 13.02 mmol, 2.8 eq), and Pd(dppf)Cl₂ (340.25 mg, 465.01 µmol, 0.1 eq) in dioxane (10 mL)/water (2 mL) was stirred at 90 °C for 2 hours under a nitrogen atmosphere. After completion of the reaction, the mixture was diluted with water (40 mL) and extracted with ethyl acetate (30 mL x 3). The combined organic layers were washed with brine (60 mL), dried over Na₂SO₄, and concentrated under reduced pressure. The resulting residue was purified by flash silica gel chromatography (Petroleum ether/Ethyl acetate = 100:1 to 3:1) to afford Compound 158-2 (682 mg, 50.6% yield) as a white solid.

¹H NMR (400 MHz, DMSO-d6) δ = 7.47 (dd, J = 3.6, 7.8 Hz, 2H), 7.32 - 7.24 (m, 1H), 7.06 (s, 1H), 4.47 - 4.37 (m, 2H), 2.75 -2.65 (m, 2H), 2.46 (s, 3H), 2.37 - 2.29 (m, 2H).

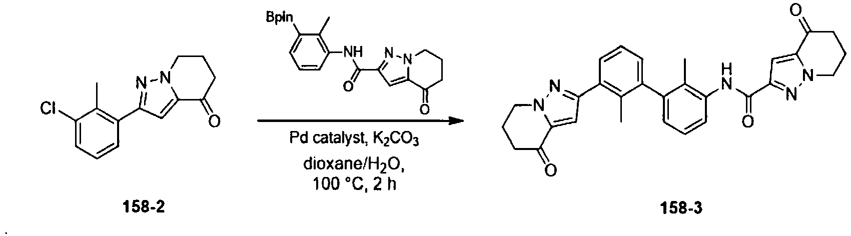

A mixture of 2-(3-chloro-2-methylphenyl)-6,7-dihydro-5H-pyrazolo[1,5-a]pyridin-4-one (630 mg, 2.42 mmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (1.15 g, 2.90 mmol, 1.2 eq), K₂CO₃ (935.12 mg, 6.77 mmol, 2.8 eq), and [2-(2-aminophenyl)phenyl]chloro-palladium dicyclohexyl[3-(2,4,6-triisopropylphenyl)phenyl]phosphane (190.12 mg, 241.64 µmol, 0.1 eq) in dioxane (10 mL)/water (2 mL) was stirred at 100 °C for 2 hours under a nitrogen atmosphere. After completion of the reaction, the mixture was diluted with water (40 mL) and extracted with ethyl acetate (30 mL x 3). The combined organic layers were washed with brine (60 mL), dried over Na₂SO₄, and concentrated under reduced pressure. The resulting residue was purified by flash silica gel chromatography (ethyl acetate/methanol = 100/1 to 3/1) to afford Compound 158-3 (1.1 g, 83.0% yield) as a white solid.

LCMS m/z 494.2 [M+1]⁺.

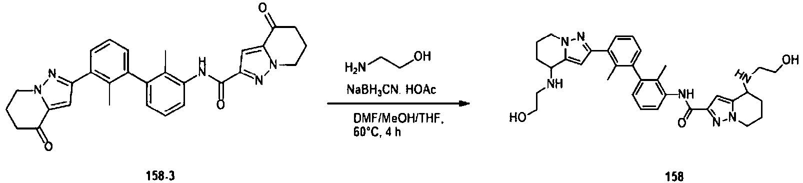

A mixture of N-[2-methyl-3-[2-methyl-3-(4-oxo-6,7-dihydro-5H-pyrazolo[1,5-a]pyridin-2-yl)phenyl]phenyl]-4-oxo-6,7-dihydro-5H-pyrazolo[1,5-a]pyridine-2-carboxamide (300 mg, 607.83 µmol, 1 eq), 2-aminoethanol (556.92 mg, 9.12 mmol, 551.41 µL, 15 eq), and HOAc (365.00 mg, 6.08 mmol, 347.95 µL, 10 eq) in MeOH (10 mL)/DMF (3 mL)/THF (5 mL) was stirred at 60 °C for 2 hours. NaBH₃CN (114.59 mg, 1.82 mmol, 3 eq) was then added, and the reaction mixture was further stirred at 60 °C for 2 hours. After completion of the reaction, the mixture was concentrated under reduced pressure. The resulting residue was purified by prep-HPLC (column: Waters Xbridge Prep OBD C18, 150 * 40 mm * 10 um; mobile phase: [water (NH₄HCO₃)-ACN]; gradient: 12% to 42% B over 15 min) to afford Compound 158 (46 mg, 12.3% yield) as a white solid.

LCMS m/z 584.5 [M+1]⁺.

¹H NMR (400 MHz, DMSO-d6) δ = 9.50 (d, J = 1.4 Hz, 1H), 7.68 - 7.40 (m, 2H), 7.27 (dt, J = 3.1, 7.6 Hz, 2H), 7.12 - 6.91 (m, 2H), 6.71 (s, 1H), 6.42 (s, 1H), 4.52 (q, J = 5.9 Hz, 2H), 4.21 - 4.03 (m, 4H), 3.89 (br t, J = 5.5 Hz, 2H), 3.48 (br d, J = 5.3 Hz, 4H), 3.17 (d, J = 4.8 Hz, 1H), 2.77 - 2.61 (m, 4H), 2.23 - 2.14 (m, 2H), 2.12 - 2.07 (m, 3H), 2.05 - 1.98 (m, 2H), 1.93 (d, J = 3.0 Hz, 4H), 1.77 - 1.61 (m, 2H).

### 34-2. Preparation of Compound 161, 4-((R)-3-hydroxypyrrolidin-1-yl)-N-(3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridine-2-carboxamide

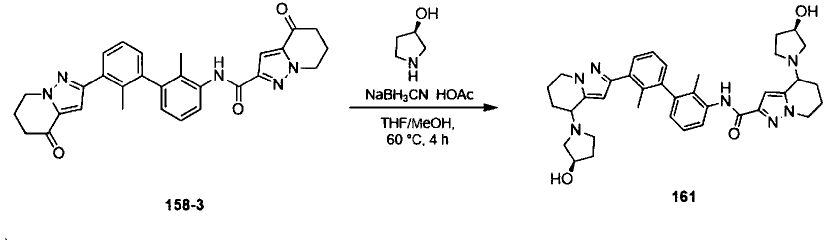

A solution of N-[2-methyl-3-[2-methyl-3-(4-oxo-6,7-dihydro-5H-pyrazolo[1,5-a]pyridin-2-yl)phenyl]phenyl]-4-oxo-6,7-dihydro-5H-pyrazolo[1,5-a]pyridine-2-carboxamide (300 mg, 607.83 µmol, 1 eq), (3R)-pyrrolidin-3-ol (529.55 mg, 6.08 mmol, 504.33 µL, 10 eq), and HOAc (365.00 mg, 6.08 mmol, 347.95 µL, 10 eq) in MeOH (10 mL)/THF (10 mL) was stirred at 60 °C for 2 hours. NaBH₃CN (114.59 mg, 1.82 mmol, 3 eq) was then added, and the reaction mixture was further stirred at 60 °C for 2 hours. After completion of the reaction, the mixture was concentrated under reduced pressure. The resulting residue was purified by prep-HPLC (column: Waters Xbridge Prep OBD C18, 150 * 40 mm * 10 um; mobile phase: [water (NH₄HCO₃)-ACN]; gradient: 15% to 45% B over 15 min) to afford Compound 161 (38 mg, 9.34% yield) as a white solid.

LCMS m/z 636.5 [M+1]⁺.

¹H NMR (400 MHz, DMSO-d6) δ = 9.52 (d, J = 3.6 Hz, 1H), 7.65 (s, 2H), 7.27 (dt, J = 3.8, 7.6 Hz, 2H), 7.11 - 6.91 (m, 2H), 6.67 (d, J = 7.3 Hz, 1H), 6.39 (d, J = 7.4 Hz, 1H), 4.83 - 4.59 (m, 2H), 4.25 - 4.14 (m, 3H), 4.12 - 3.94 (m, 2H), 3.84 - 3.71 (m, 2H), 2.96 - 2.75 (m, 3H), 2.74 - 2.58 (m, 2H), 2.54 (br s, 1H), 2.47 - 2.40 (m, 1H), 2.32 - 2.18 (m, 2H), 2.13 - 2.05 (m, 3H), 1.94 (br d, J = 2.9 Hz, 4H), 1.89 (br s, 5H), 1.55 (br dd, J = 4.4, 7.8 Hz, 2H).

### 34-3. Preparation of Compound 159, 4-((2-hydroxyethyl)amino)-N-(3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)-4,5,6,7-tetrahydropyrazolo[1,5-a]pyridine-2-carboxamide

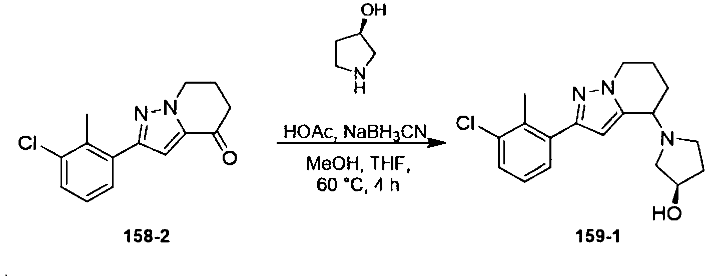

A mixture of 2-(3-chloro-2-methyl-phenyl)-6,7-dihydro-5H-pyrazolo[1,5-a]pyridin-4-one (500 mg, 1.92 mmol, 1 eq), (3R)-pyrrolidin-3-ol (334.15 mg, 3.84 mmol, 318.24 µL, 2 eq), and HOAc (1.15 g, 19.18 mmol, 1.10 mL, 10 eq) in MeOH (10 mL)/THF (10 mL) was stirred at 60 °C for 2 hours. NaBH₃CN (361.54 mg, 5.75 mmol, 3 eq) was then added, and the reaction mixture was further stirred at 60 °C for 2 hours. After completion of the reaction, the mixture was concentrated under reduced pressure. The resulting residue was purified by flash silica gel chromatography (DCM:MeOH = 100/1 to 10/1) to afford Compound 159-1 (620 mg, 87.6% yield) as a colorless liquid.

LCMS m/z 332.3 [M+1]⁺.

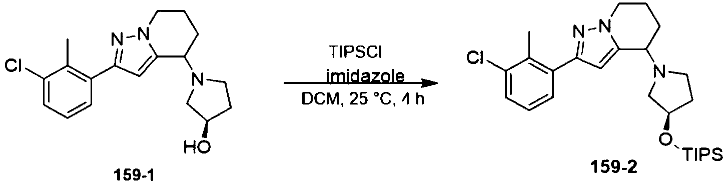

To a solution of (3R)-1-[2-(3-chloro-2-methyl-phenyl)-4,5,6,7-tetrahydropyrazolo[1,5-a]pyridin-4-yl]pyrrolidin-3-ol (620 mg, 1.87 mmol, 1 eq) and chloro(triisopropyl)silane (720.45 mg, 3.74 mmol, 799.61 µL, 2 eq) in DMC (10 mL), imidazole (254.40 mg, 3.74 mmol, 2 eq) was added, and the reaction mixture was stirred at room temperature for 4 hours. After completion of the reaction, the mixture was diluted with water (40 mL) and extracted with ethyl acetate (30 mL x 3). The combined organic layers were washed with brine (60 mL), dried over Na₂SO₄, and concentrated under reduced pressure. The resulting residue was purified by flash silica gel chromatography (Petroleum ether/Ethyl acetate = 100/1 to 1/1) to afford Compound 159-2 (720 mg, 71.0% yield) as a white solid.

LCMS m/z 488.5 [M+1]⁺.

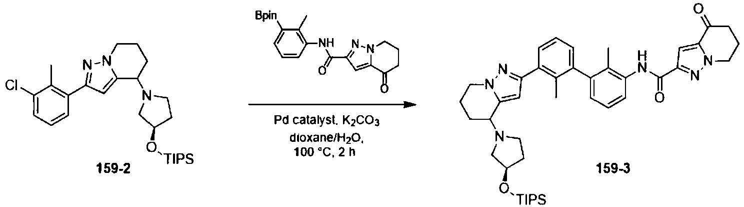

A mixture of [(3R)-1-[2-(3-chloro-2-methyl-phenyl)-4,5,6,7-tetrahydropyrazolo[1,5-a]pyridin-4-yl]pyrrolidin-3-yl]oxy-triisopropyl-silane (400 mg, 819.37 µmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (388.64 mg, 983.25 µmol, 1.2 eq), K₂CO₃ (317.09 mg, 2.29 mmol, 2.8 eq), and [2-(2-aminophenyl)phenyl]-chloro-palladium;dicyclohexyl-[3-(2,4,6-triisopropylphenyl)phenyl]phosphane (64.47 mg, 81.94 µmol, 0.1 eq) in dioxane (6 mL) / water (1 mL) was stirred under a nitrogen atmosphere at 100 °C for 2 hours. After completion of the reaction, the mixture was filtered, and the filtrate was concentrated under reduced pressure. The resulting residue was purified by flash silica gel chromatography (Petroleum ether/Ethyl acetate = 100/1 to 1/1) to afford Compound 159-3 (372 mg, 56.6% yield) as a white solid.

LCMS m/z 721.7 [M+1]⁺.

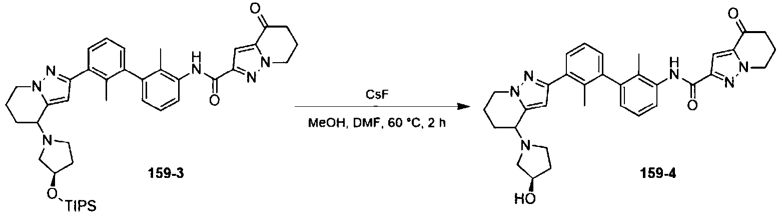

To a solution of N-[2-methyl-3-[2-methyl-3-[4-[(3R)-3-triisopropylsilyloxypyrrolidin-1-yl]-4,5,6,7-tetrahydropyrazolo[1,5-a]pyridin-2-yl]phenyl]phenyl]-4-oxo-6,7-dihydro-5H-pyrazolo[1,5-a]pyridine-2-carboxamide (300 mg, 416.08 µmol, 1 eq) in MeOH (2 mL)/DMF (2 mL), CsF (189.61 mg, 1.25 mmol, 3 eq) was added, and the reaction mixture was stirred at 60 °C for 2 hours. After completion of the reaction, the mixture was filtered, and the filtrate was concentrated under reduced pressure. The residue was purified by prep-HPLC (column: Phenomenex Luna C18, 150 * 25 mm * 10 µm; mobile phase: water (FA)/ACN; gradient: 10-40% B over 10 min) to afford Compound 159-4 (82 mg, 33.1% yield) as a white solid.

LCMS m/z 565.4 [M+1]⁺.

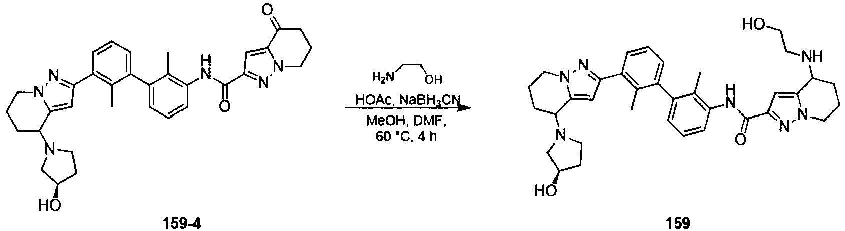

A mixture of N-[3-[3-[4-[(3R)-3-hydroxypyrrolidin-1-yl]-4,5,6,7-tetrahydropyrazolo[1,5-a]pyridin-2-yl]-2-methylphenyl]-2-methylphenyl]-4-oxo-6,7-dihydro-5H-pyrazolo[1,5-a]pyridine-2-carboxamide (80 mg, 141.67 µmol, 1 eq), 2-aminoethanol (86.54 mg, 1.42 mmol, 10 eq), and HOAc (85.08 mg, 1.42 mmol, 10 eq) in DMF (5 mL)/MeOH (15 mL) was stirred at 60 °C for 2 hours. NaBH₃CN (26.71 mg, 425.02 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm, 5 µm; mobile phase: [water (NH₄HCO₃)-ACN]; gradient: 22-52% B over 9 min) to afford Compound 159 (22 mg, 24.1 % yield) as a white solid.

LCMS m/z 610.6 [M+1]⁺.

¹H NMR (400 MHz, DMSO-d6 ) δ = 9.50 (d, J = 1.8 Hz, 1H), 7.57 (br d, J = 8.0 Hz, 1H), 7.48 (d, J = 7.3 Hz, 1H), 7.27 (dt, J = 3.8, 7.7 Hz, 2H), 7.09 - 7.03 (m, 1H), 6.97 (dd, J = 2.9, 7.1 Hz, 1H), 6.71 (s, 1H), 6.39 (d, J = 7.3 Hz, 1H), 4.76 - 4.49 (m, 2H), 4.18 - 4.02 (m, 5H), 3.92 - 3.72 (m, 2H), 3.47 (br t, J = 5.7 Hz, 3H), 2.97 - 2.76 (m, 2H), 2.75 - 2.57 (m, 4H), 2.54 (br d, J = 3.5 Hz, 1H), 2.33 - 2.16 (m, 2H), 2.10 (s, 3H), 2.00 (br dd, J = 7.3, 12.5 Hz, 2H), 1.93 (br d, J = 3.1 Hz, 4H), 1.90 (br d, J = 5.9 Hz, 2H), 1.72 - 1.49 (m, 2H).

### 34-4. Preparation of Compound 160, N-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-4-((R)-3-hydroxypyrrolidin-1-yl)-4,5,6,7-tetrahydropyrazolo[1,5-a]pyridine-2-carboxamide

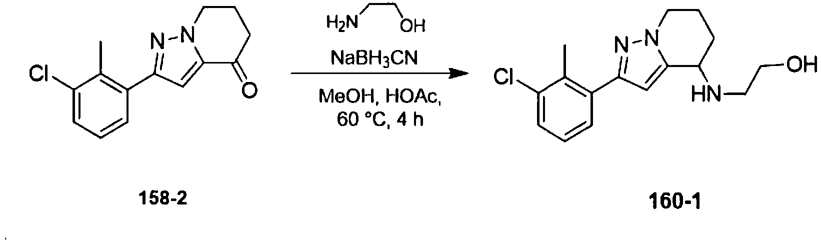

A solution of 2-(3-chloro-2-methylphenyl)-6,7-dihydro-5H-pyrazolo[1,5-a]pyridin-4-one (1.2 g, 4.60 mmol, 1 eq), 2-aminoethanol (562.29 mg, 9.21 mmol, 556.72 µL, 2 eq), and HOAc (829.17 mg, 13.81 mmol, 3 eq) in MeOH (20 mL) was stirred at 60 °C for 2 hours. NaBH₃CN (867.70 mg, 13.81 mmol, 3 eq) was then added to the reaction mixture, followed by stirring at 60 °C for an additional 2 hours. The reaction mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by flash silica gel chromatography (0-10%, Petroleum ether/Ethyl acetate) to afford Compound 160-2 (1.52 g, 86.3% yield) as a white solid.

LCMS m/z 306.3 [M+1]⁺.

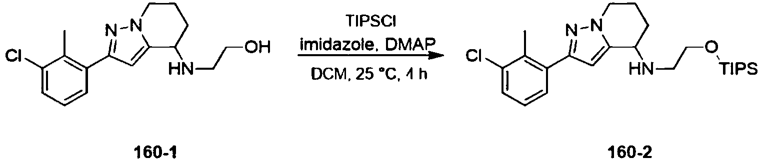

A solution of 2-[[2-(3-chloro-2-methylphenyl)-4,5,6,7-tetrahydropyrazolo[1,5-a]pyridin-4-yl]amino]ethanol (1.5 g, 4.91 mmol, 1 eq), chloro(triisopropyl)silane (1.89 g, 9.81 mmol, 2 eq), imidazole (667.88 mg, 9.81 mmol, 2 eq), and DMAP (119.85 mg, 981.03 µmol, 0.2 eq) in DCM (30 mL) was stirred under a nitrogen atmosphere at room temperature for 4 hours. The reaction mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by prep-HPLC (column: Phenomenex Luna C18, 150 * 40 mm * 15 µm; mobile phase: water (FA)/ACN; gradient: 30-60% B over 15 min) to afford Compound 160-2 (1.62 g, 64.3% yield) as a white solid.

LCMS m/z 462.5 [M+1]⁺.

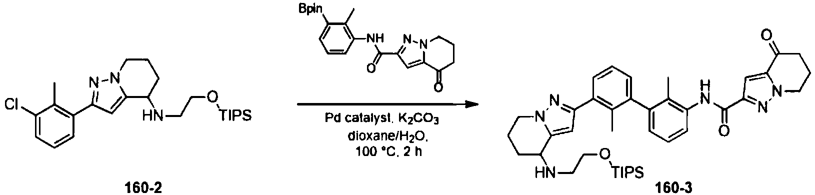

A mixture of 2-(3-chloro-2-methylphenyl)-N-(2-triisopropylsilyloxyethyl)-4,5,6,7-tetrahydropyrazolo[1,5-a]pyridin-4-amine (440 mg, 952.09 µmol, 1 eq), N-[2-methyl-3-(4,4,5,5-tetramethyl-1,3,2-dioxaborolan-2-yl)phenyl]-4-oxo-6,7-dihydro-5H-pyrazolo[1,5-a]pyridine-2-carboxamide (451.59 mg, 1.14 mmol, 1.2 eq), K₂CO₃ (368.45 mg, 2.67 mmol, 2.8 eq), and [2-(2-aminophenyl)phenyl]-chloro-palladium;dicyclohexyl-[3-(2,4,6-triisopropylphenyl)phenyl]phosphane (74.91 mg, 95.21 µmol, 0.1 eq) in dioxane (10 mL)/H₂O (2 mL) was stirred under a nitrogen atmosphere at 100 °C for 2 hours. After completion of the reaction, the mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by flash silica gel chromatography (Petroleum ether/Ethyl acetate = 100/1 to 1/1) to afford Compound 160-3 (520 mg, 62.8% yield) as a white solid.

LCMS m/z 695.7 [M+1]⁺.

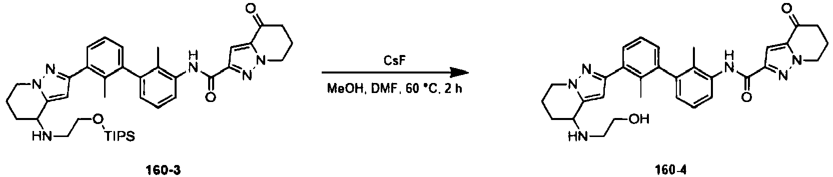

To a solution of N-[2-methyl-3-[2-methyl-3-[4-(2-triisopropylsilyloxyethylamino)-4,5,6,7-tetrahydropyrazolo[1,5-a]pyridin-2-yl]phenyl]phenyl]-4-oxo-6,7-dihydro-5H-pyrazolo[1,5-a]pyridine-2-carboxamide (330 mg, 474.83 µmol, 1 eq) in MeOH (10 mL)/DMF (10 mL), CsF (216.39 mg, 1.42 mmol, 52.58 µL, 3 eq) was added. The reaction mixture was stirred at 60 °C for 2 hours. After completion of the reaction, the mixture was filtered, and the filtrate was concentrated under reduced pressure. The residue was purified by prep-HPLC (column: Waters XBridge Prep OBD C18, 150 * 40 mm * 10 µm; mobile phase: [water (NH₄HCO₃)-ACN]; gradient: 18-48% B over 15 min) to afford Compound 160-4 (122 mg, 45.3% yield) as a white solid.

LCMS m/z 539.3 [M+1]⁺.

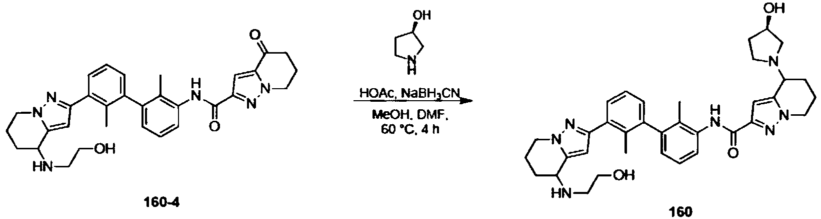

A solution of N-[3-[3-[4-(2-hydroxyethylamino)-4,5,6,7-tetrahydropyrazolo[1,5-a]pyridin-2-yl]-2-methylphenyl]-2-methylphenyl]-4-oxo-6,7-dihydro-5H-pyrazolo[1,5-a]pyridine-2-carboxamide (100 mg, 185.65 µmol, 1 eq), (3R)-pyrrolidin-3-ol (161.74 mg, 1.86 mmol, 10 eq), and HOAc (111.48 mg, 1.86 mmol, 10 eq) in DMF (5 mL)/MeOH (15 mL) was stirred at 60 °C for 2 hours. NaBH₃CN (35.00 mg, 556.96 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; gradient: 22-52% B over 9 min) to afford Compound 160 (62 mg, 52.0% yield) as a white solid.

LCMS m/z 610.5 [M+1]⁺.

¹H NMR (400 MHz, DMSO-d6) δ = 9.52 (d, J = 3.3 Hz, 1H), 7.57 (br dd, J = 3.4, 7.8 Hz, 1H), 7.49 (br d, J = 7.6 Hz, 1H), 7.27 (dt, J = 2.7, 7.6 Hz, 2H), 7.10 - 6.95 (m, 2H), 6.68 (d, J = 7.3 Hz, 1H), 6.42 (s, 1H), 4.72 (t, J = 4.9 Hz, 1H), 4.51 (br t, J = 4.9 Hz, 1H), 4.26 - 4.12 (m, 3H), 4.08 (br t, J = 6.0 Hz, 2H), 3.94 - 3.86 (m, 1H), 3.79 (br d, J = 4.9 Hz, 1H), 3.48 (br d, J = 5.1 Hz, 2H), 2.76 (br s, 2H), 2.74 - 2.58 (m, 3H), 2.31 - 2.15 (m, 2H), 2.11 (s, 3H), 2.07 (s, 1H), 1.94 (br d, J = 2.9 Hz, 4H), 1.89 (br s, 3H), 1.76 - 1.51 (m, 2H).

### [Preparation Example 35]

### Preparation of Compounds 162 to 167

### 35-1. Preparation of Compound 162, (5S)-5-((((5-(3'-(4-((2-hydroxyethyl)amino)-4,5,6,7-tetrahydropyrazolo[1,5-a]pyridin-2-yl)-2,2'-dimethyl-[1,1'-biphenyl]-3-yl)-3-methoxypyrazin-2-yl)methyl)amino)methyl)pyrrolidin-2-one

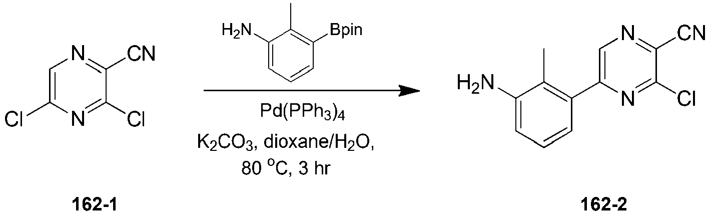

A mixture of 3,5-dichloropyrazine-2-carbonitrile (4.5 g, 25.86 mmol, 1 eq), 2-methyl-3-(4,4,5,5-tetramethyl-1,3,2-dioxaborolan-2-yl)aniline (6.33 g, 27.16 mmol, 1.05 eq), Pd(PPh₃)₄ (1.20 g, 1.03 mmol, 0.04 eq), and K₂CO₃ (10.72 g, 77.59 mmol, 3 eq) in dioxane (50 mL)/H₂O (5 mL) was stirred under a nitrogen atmosphere at 80 °C for 2 hours. After completion of the reaction, the mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by flash silica gel chromatography (Petroleum ether/Ethyl acetate = 100/1 to 3/1) to afford Compound 162-2 (4.6 g, 58.1% yield) as a white solid.

LCMS m/z 245.2 [M+1]⁺

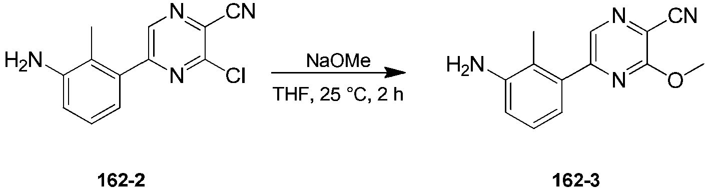

To a solution of 5-(3-amino-2-methylphenyl)-3-chloropyrazine-2-carbonitrile (4.5 g, 18.39 mmol, 1 eq) in THF (100 mL), NaOMe (3.97 g, 22.07 mmol, 1.2 eq) was added at room temperature. The resulting mixture was stirred at room temperature for 2 hours. After completion of the reaction, the mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by flash silica gel chromatography (Petroleum ether/Ethyl acetate = 100/1 to 3/1) to afford Compound 162-3 (4.26 g, 77.1% yield) as a white solid.

LCMS m/z 241.3 [M+1]⁺

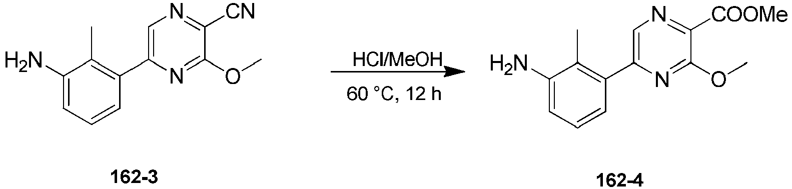

A solution of 5-(3-bromo-2-methylphenyl)-3-methoxypyrazine-2-carbonitrile (3.5 g, 11.51 mmol, 1 eq) in HCl/MeOH (4 M, 60 mL, 20.86 eq) was stirred at 60 °C for 12 hours. After completion of the reaction, the mixture was diluted with H₂O (40 mL) and extracted with ethyl acetate (30 mL x 3). The combined organic layers were washed with brine (60 mL), dried over Na₂SO₄, and concentrated under reduced pressure to afford Compound 162-4 (3.86 g, 79.5% yield) as a white solid, which was used in the subsequent reaction without further purification.

LCMS m/z 337.1 [M+1]⁺

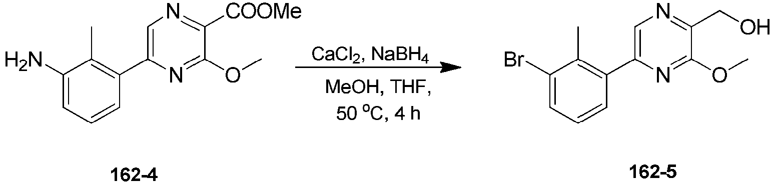

To a solution of methyl 5-(3-bromo-2-methylphenyl)-3-methoxypyrazine-2-carboxylate (3.82 g, 11.33 mmol, 1 eq) in MeOH (40 mL)/THF (40 mL), CaCl₂ (3.77 g, 33.99 mmol, 3 eq) and NaBH₄ (2.57 g, 67.98 mmol, 6 eq) were added. The reaction mixture was stirred at room temperature for 2 hours and then at 50 °C for an additional 2 hours. After completion of the reaction, the mixture was diluted with H₂O (200 mL) and extracted with ethyl acetate (100 mL x 2). The combined organic layers were washed with brine (50 mL), dried over Na₂SO₄, and concentrated under reduced pressure to afford Compound 162-5 (3.62 g, 82.6% yield) as a white solid, which was used in the subsequent reaction without further purification.

LCMS m/z 311.2 [M+1]⁺

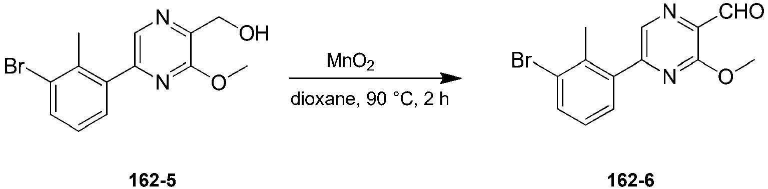

To a solution of [5-(3-bromo-2-methylphenyl)-3-methoxypyrazin-2-yl]methanol (3.5 g, 11.32 mmol, 1 eq) in dioxane (100 mL), MnO₂ (4.92 g, 5 eq) was added. The reaction mixture was stirred at 90 °C for 2 hours. After completion of the reaction, the mixture was filtered, and the filtrate was concentrated under reduced pressure to afford Compound 162-6 (3.2 g, 73.6% yield), which was used in the subsequent reaction without further purification.

LCMS m/z 309.2 [M+1]⁺

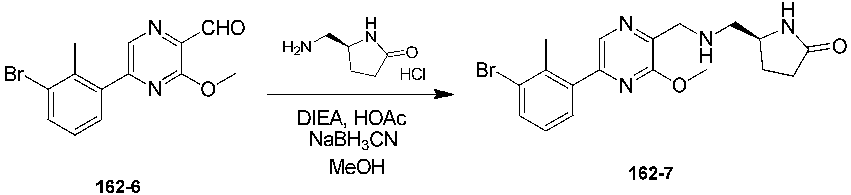

To a solution of 5-(3-bromo-2-methylphenyl)-3-methoxypyrazine-2-carbaldehyde (1.25 g, 4.07 mmol, 1 eq), (5S)-5-(aminomethyl)pyrrolidin-2-one (3.06 g, 20.35 mmol, 5 eq, HCl salt), and DIPEA (3.16 g, 24.42 mmol, 6 eq) in MeOH (30 mL), HOAc (4.89 g, 81.40 mmol, 20 eq) was added. The resulting mixture was stirred at 50 °C for 2 hours. NaBH₃CN (1.28 g, 20.35 mmol, 5 eq) was then added to the reaction mixture, followed by stirring at 50 °C for an additional 2 hours. The reaction mixture was concentrated under reduced pressure, and the residue was purified by prep-HPLC (column: Kromasil Eternity XT, 250 * 80 mm * 10 µm; mobile phase: [water (NH₄HCO₃)-ACN]; gradient: 30-60% B over 20 min) to afford Compound 162-7 (820 mg, 44.7% yield) as a white solid.

LCMS m/z 405.3 [M+1]⁺

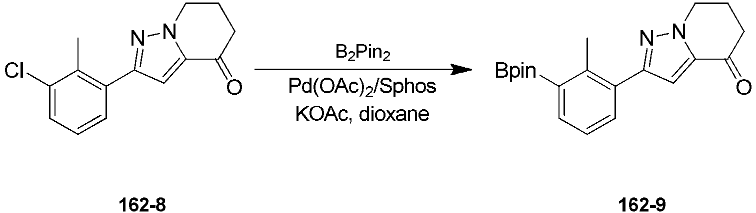

A mixture of 2-(3-chloro-2-methylphenyl)-6,7-dihydro-5H-pyrazolo[1,5-a]pyridin-4-one (600 mg, 2.30 mmol, 1 eq), 4,4,5,5-tetramethyl-2-(4,4,5,5-tetramethyl-1,3,2-dioxaborolan-2-yl)-1,3,2-dioxaborolane (876.59 mg, 3.45 mmol, 1.5 eq), KOAc (451.72 mg, 4.60 mmol, 2 eq), Pd(OAc)₂ (51.67 mg, 230.13 µmol, 0.1 eq), and SPhos (188.95 mg, 460.27 µmol, 0.2 eq) in dioxane (10 mL) was stirred under a nitrogen atmosphere at 90 °C for 2 hours. After completion of the reaction, the mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by flash silica gel chromatography (Petroleum ether/Ethyl acetate = 100/1 to 3/1) to afford Compound 162-9 (652 mg, 64.3% yield) as a white solid.

LCMS m/z 353.3 [M+1]⁺

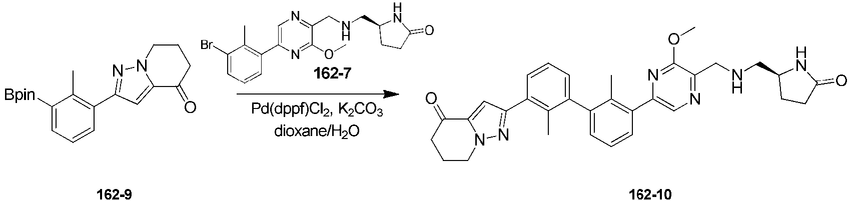

A mixture of (5S)-5-[[[5-(3-bromo-2-methylphenyl)-3-methoxypyrazin-2-yl]methylamino]methyl]pyrrolidin-2-one (350 mg, 863.58 µmol, 1 eq), 2-[2-methyl-3-(4,4,5,5-tetramethyl-1,3,2-dioxaborolan-2-yl)phenyl]-6,7-dihydro-5H-pyrazolo[1,5-a]pyridin-4-one (456.28 mg, 1.30 mmol, 1.5 eq), Pd(dppf)Cl₂ (63.19 mg, 86.36 µmol, 0.1 eq), and K₂CO₃ (358.06 mg, 2.59 mmol, 3 eq) in dioxane (8 mL) / H₂O (2 mL) was stirred under a nitrogen atmosphere at 90 °C for 2 hours. After completion of the reaction, the mixture was concentrated under reduced pressure, and the residue was purified by prep-TLC (dichloromethane/methanol = 10/1) to afford Compound 162-10 (472 mg, 79.4% yield) as a white solid.

LCMS m/z 551.4[M+1]⁺

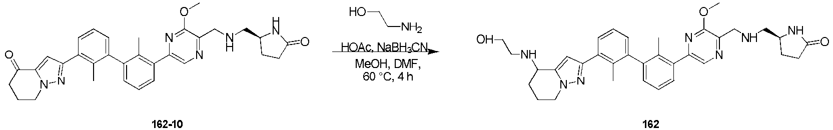

A mixture of 2-[3-[3-[6-methoxy-5-[[[(2S)-5-oxopyrrolidin-2-yl]methylamino]methyl]pyrazin-2-yl]-2-methylphenyl]-2-methylphenyl]-6,7-dihydro-5H-pyrazolo[1,5-a]pyridin-4-one (100 mg, 181.60 µmol, 1 eq), 2-aminoethanol (110.93 mg, 1.82 mmol, 109.83 µL, 10 eq), and HOAc (109.05 mg, 1.82 mmol, 103.96 µL, 10 eq) in MeOH (10 mL)/THF (10 mL) was stirred at 60 °C for 2 hours. NaBH₃CN (34.24 mg, 544.81 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; gradient: 26-56% B over 9 min) to afford Compound 162 (18 mg, 15.8% yield) as a white solid.

LCMS m/z 596.6 [M+1]⁺

¹H NMR (400 MHz, DMSO-*d₆*) δ = 8.34 (s, 1H), 7.69 (s, 1H), 7.54 - 7.45 (m, 2H), 7.40 (t, *J* = 7.6 Hz, 1H), 7.29 (t, *J* = 7.6 Hz, 1H), 7.26 - 7.20 (m, 1H), 7.10 (d, *J* = 7.2 Hz, 1H), 6.43 (s, 1H), 4.50 (br s, 1H), 4.08 (br t, *J* = 6.1 Hz, 2H), 3.96 (s, 3H), 3.95 - 3.77 (m, 3H), 3.67 - 3.56 (m, 1H), 3.52 - 3.45 (m, 2H), 2.78 - 2.67 (m, 2H), 2.62 (br t, *J* = 5.4 Hz, 2H), 2.24 - 2.10 (m, 6H), 2.09 - 1.99 (m, 5H), 1.96 - 1.86 (m, 1H), 1.78 - 1.62 (m, 2H).

### 35-2. Preparation of Compound 163, 2-((2-(3'-(6-methoxy-5-(((((S)-5-oxopyrrolidin-2-yl)methyl)amino)methyl)pyrazin-2-yl)-2,2'-dimethyl-[1,1'-biphenyl]-3-yl)-4,5,6, 7-tetrahydropyrazolo[1,5-a]pyridin-4-yl)amino)acetic acid

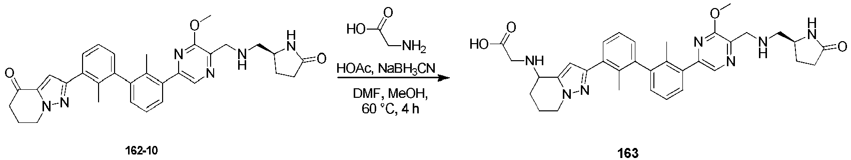

To a solution of 2-[3-[3-[6-methoxy-5-[[[(2S)-5-oxopyrrolidin-2-yl]methylamino]methyl]pyrazin-2-yl]-2-methylphenyl]-2-methylphenyl]-6,7-dihydro-5H-pyrazolo[1,5-a]pyridin-4-one (150 mg, 272.41 µmol, 1 eq) and 2-aminoacetic acid (204.49 mg, 2.72 mmol, 297.04 µL, 10 eq) in MeOH (20 mL)/DMF (5 mL), HOAc (327.16 mg, 5.45 mmol, 311.88 µL, 20 eq) was added. The resulting mixture was stirred at 60 °C for 2 hours. NaBH₃CN (85.59 mg, 1.36 mmol, 5 eq) was then added to the reaction mixture, followed by stirring at 60 °C for an additional 2 hours. The reaction mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 8-38% B over 15 min) to afford Compound 163 (36 mg, 20.5% yield) as a white solid.

LCMS m/z 610.4 [M+1]⁺

¹H NMR (400 MHz, DMSO-*d₆*) δ = 8.34 (s, 1H), 7.69 (s, 1H), 7.54 - 7.46 (m, 2H), 7.39 (t, *J* = 7.6 Hz, 1H), 7.29 (t, *J* = 7.6 Hz, 1H), 7.25 - 7.20 (m, 1H), 7.10 (dd, *J* = 1.1, 7.5 Hz, 1H), 6.50 (s, 1H), 4.15 - 4.06 (m, 3H), 3.95 (s, 3H), 3.88 (s, 2H), 3.68 - 3.59 (m, 3H), 3.30 (s, 4H), 2.64 - 2.58 (m, 2H), 2.23 - 2.17 (m, 1H), 2.13 (s, 3H), 2.11 - 2.08 (m, 2H), 2.05 (d, *J* = 2.1 Hz, 3H), 1.91 (br dd, *J* = 6.3, 13.4 Hz, 1H), 1.80 - 1.65 (m, 2H).

### 35-3. Preparation of Compound 164, (5S)-5-((((5-(3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)-3-methoxypyrazin-2-yl)methyl)amino)methyl)pyrrolidin-2-one

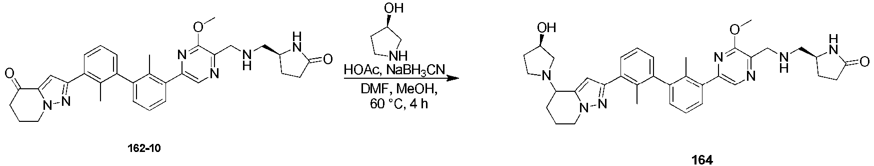

A solution of 2-[3-[3-[6-methoxy-5-[[[(2S)-5-oxopyrrolidin-2-yl]methylamino]methyl]pyrazin-2-yl]-2-methylphenyl]-2-methylphenyl]-6,7-dihydro-5H-pyrazolo[1,5-a]pyridin-4-one (150 mg, 272.41 µmol, 1 eq), (3R)-pyrrolidin-3-ol (237.32 mg, 2.72 mmol, 226.02 µL, 10 eq), and HOAc (163.58 mg, 2.72 mmol, 155.94 µL, 10 eq) in DMF (5 mL)/MeOH (20 mL) was stirred at 60 °C for 2 hours. NaBH₃CN (51.35 mg, 817.22 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 18-48% B over 15 min) to afford Compound 164 (41 mg, 23.0% yield) as a white solid.

LCMS m/z 622.5 [M+1]⁺

¹H NMR (400 MHz, DMSO-*d₆*) δ = 8.34 (s, 1H), 7.69 (s, 1H), 7.53 - 7.46 (m, 2H), 7.39 (t, *J* = 7.6 Hz, 1H), 7.29 (t, *J* = 7.6 Hz, 1H), 7.22 (dd, *J* = 3.9, 6.7 Hz, 1H), 7.10 (d, *J* = 7.0 Hz, 1H), 6.43 - 6.37 (m, 1H), 4.74 - 4.64 (m, 1H), 4.23 - 4.14 (m, 1H), 4.14 - 4.04 (m, 2H), 3.96 (s, 3H), 3.90 (s, 2H), 3.76 (br s, 1H), 3.64 (quin, *J* = 5.9 Hz, 1H), 2.95 - 2.75 (m, 2H), 2.74 - 2.61 (m, 3H), 2.54 (br dd, *J* = 3.3, 8.9 Hz, 1H), 2.32 - 2.16 (m, 2H), 2.13 (s, 3H), 2.11 - 2.08 (m, 2H), 2.05 (d, *J* = 3.3 Hz, 3H), 1.97 - 1.83 (m, 4H), 1.77 - 1.66 (m, 1H), 1.61 - 1.50 (m, 1H).

### 35-4. Preparation of Compound 165, (3R)-1-(2-(3'-(6-methoxy-5-(((((S)-5-oxopyrrolidin-2-yl)methyl)amino)methyl)pyrazin-2-yl)-2,2'-dimethyl-[1,1'-biphenyl]-3-yl)-4,5,6,7-tetrahydropyrazolo[1,5-a]pyridin-4-yl)pyrrolidine-3-carboxylic acid

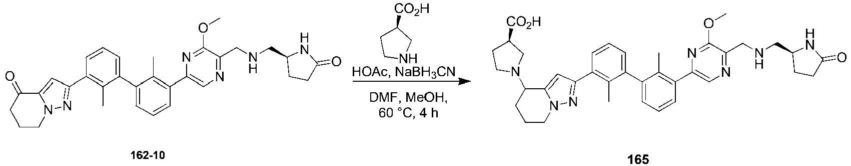

To a solution of 2-[3-[3-[6-methoxy-5-[[[(2S)-5-oxopyrrolidin-2-yl]methylamino]methyl]pyrazin-2-yl]-2-methylphenyl]-2-methylphenyl]-6,7-dihydro-5H-pyrazolo[1,5-a]pyridin-4-one (150 mg, 272.41 µmol, 1 eq) and (3R)-pyrrolidine-3-carboxylic acid (313.62 mg, 2.72 mmol, 10 eq) in MeOH (20 mL)/DMF (5 mL), HOAc (327.16 mg, 5.45 mmol, 311.88 µL, 20 eq) was added. The resulting mixture was stirred at 60 °C for 2 hours. NaBH₃CN (85.59 mg, 1.36 mmol, 5 eq) was then added to the reaction mixture, followed by stirring at 60 °C for an additional 2 hours. The reaction mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 10-40% B over 15 min) to afford Compound 165 (42 mg, 22.5% yield) as a white solid.

¹H NMR (400 MHz, DMSO-*d₆*) δ = 8.34 (s, 1H), 7.69 (s, 1H), 7.55 - 7.46 (m, 2H), 7.39 (br t, *J* = 7.5 Hz, 1H), 7.29 (t, *J* = 7.5 Hz, 1H), 7.25 - 7.18 (m, 1H), 7.10 (br d, *J* = 7.1 Hz, 1H), 6.39 (dd, J = 2.3, 6.6 Hz, 1H), 4.15 - 4.01 (m, 2H), 3.96 (s, 3H), 3.89 (s, 2H), 3.78 (br s, 2H), 3.62 (br d, *J* = 5.9 Hz, 2H), 2.99 - 2.81 (m, 3H), 2.80 - 2.70 (m, 2H), 2.67 - 2.58 (m, 3H), 2.30 - 2.20 (m, 1H), 2.13 (s, 3H), 2.09 (br s, 2H), 2.05 (br d, *J* = 3.4 Hz, 3H), 1.99 - 1.85 (m, 5H), 1.77 - 1.65 (m, 1H).

### 35-5. Preparation of Compound 166, (5S)-5-((((5-(3'-(4-(4-hydroxypiperidin-1-yl)-4,5,6,7-tetrahydropyrazolo[1,5-a]pyridin-2-yl)-2,2'-dimethyl-[1,1'-biphenyl]-3-yl)-3-methoxypyrazin-2-yl)methyl)amino)methyl)pyrrolidin-2-one

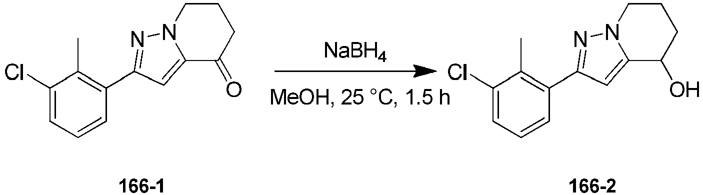

To a solution of 2-(3-chloro-2-methylphenyl)-6,7-dihydro-5H-pyrazolo[1,5-a]pyridin-4-one (2.5 g, 9.59 mmol, 1 eq) in MeOH (35 mL), NaBH₄ (1.21 g, 19.18 mmol, 2 eq) was added at 0 °C. The resulting mixture was stirred at 0 °C for 90 minutes. After completion of the reaction, the mixture was diluted with H₂O (100 mL) and extracted with ethyl acetate (50 mL x 3). The combined organic layers were washed with brine (120 mL), dried over Na₂SO₄, and concentrated under reduced pressure. The residue was purified by flash silica gel chromatography (Petroleum ether/Ethyl acetate = 100/1 to 1/1) to afford Compound 166-2 (2.32 g, 78.7% yield) as a white solid.

LCMS m/z 263.3 [M+1]⁺

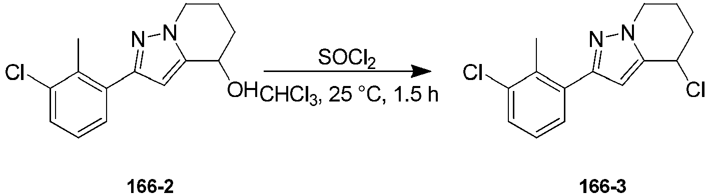

To a solution of 2-(3-chloro-2-methylphenyl)-4,5,6,7-tetrahydropyrazolo[1,5-a]pyridin-4-ol (1.8 g, 6.85 mmol, 1 eq) in CHCl₃ (25 mL), SOCl₂ (8.15 g, 68.51 mmol, 4.98 mL, 10 eq) was added at 0 °C. The resulting mixture was stirred at room temperature for 2 hours. After completion of the reaction, the mixture was concentrated under reduced pressure, and aq. saturated NaHCO₃ (80 mL) was slowly added. The mixture was then extracted with EtOAc (40 mL * 2). The combined organic layers were washed with brine (100 mL), dried over Na₂SO₄, and concentrated under reduced pressure to afford Compound 166-3 (1.81 g, 84.5% yield) as a white solid, which was used in the subsequent reaction without further purification.

¹H NMR EW35827-524-P1B3 (400 MHz, DMSO-*d₆*) δ = 7.42 (d, *J* = 8.1 Hz, 2H), 7.26 - 7.22 (m, 1H), 6.55 (s, 1H), 5.67 (t, *J* = 4.0 Hz, 1H), 4.29 (td, J = 4.5, 12.8 Hz, 1H), 4.09 (dt, J = 4.8, 8.7 Hz, 1H), 2.46 (s, 3H), 2.37 - 2.27 (m, 2H), 2.21 - 2.14 (m, 1H), 2.11 - 2.04 (m, 1H).

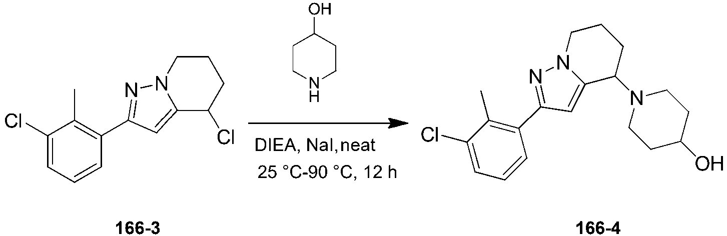

To mixture of 4-chloro-2-(3-chloro-2-methylphenyl)-4,5,6,7-tetrahydropyrazolo[1,5-a]pyridine (300 mg, 1.07 mmol, 1 eq) and piperidin-4-ol (323.75 mg, 3.20 mmol, 3 eq), DIPEA (413.67 mg, 3.20 mmol, 557.51 µL, 3 eq) and Nal (47.98 mg, 320.08 µmol, 0.3 eq) were added. The resulting mixture was stirred at 90 °C for 12 hours. After completion of the reaction, the mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 25-55% B over 15 min) to afford Compound 166-4 (402 mg, 87.5% yield) as a white solid.

LCMS m/z 346.3 [M+1]⁺

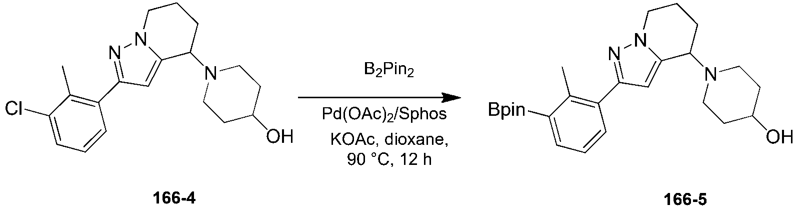

To a solution of 1-[2-(3-chloro-2-methylphenyl)-4,5,6,7-tetrahydropyrazolo[1,5-a]pyridin-4-yl]piperidin-4-ol (230 mg, 665.00 µmol, 1 eq) and 4,4,5,5-tetramethyl-2-(4,4,5,5-tetramethyl-1,3,2-dioxaborolan-2-yl)-1,3,2-dioxaborolane (337.74 mg, 1.33 mmol, 2 eq) in dioxane (6 mL), KOAc (130.53 mg, 1.33 mmol, 2 eq), Pd(OAc)₂ (14.93 mg, 66.50 µmol, 0.1 eq), and SPhos (54.60 mg, 133.00 µmol, 0.2 eq) were added. The resulting mixture was stirred at 90 °C for 12 hours. After completion of the reaction, the mixture was concentrated under reduced pressure, and the residue was purified by flash silica gel chromatography (dichloromethane/methanol = 10/1) to afford Compound 166-5 (282 mg, 77.5% yield) as a white solid.

LCMS m/z 438.2 [M+1]⁺

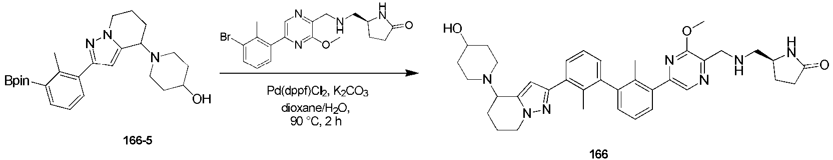

A solution of 1-[2-[2-methyl-3-(4,4,5,5-tetramethyl-1,3,2-dioxaborolan-2-yl)phenyl]-4,5,6,7-tetrahydropyrazolo[1,5-a]pyridin-4-yl]piperidin-4-ol (210.44 mg, 481.14 µmol, 1.5 eq), (5S)-5-[[[5-(3-bromo-2-methylphenyl)-3-methoxypyrazin-2-yl]methylamino]methyl]pyrrolidin-2-one (130 mg, 320.76 µmol, 1 eq), K₂CO₃ (124.13 mg, 898.13 µmol, 2.8 eq), and ditert-butyl(cyclopentyl)phosphane;dichloropalladium;iron (20.91 mg, 32.08 µmol, 0.1 eq) in dioxane (6 mL)/H₂O (2 mL) was stirred under a nitrogen atmosphere at 90 °C for 2 hours. After completion of the reaction, the mixture was filtered, and the filtrate was concentrated under reduced pressure. The residue was purified by prep-HPLC (column: Waters XBridge, 150 * 25 mm * 5 µm; mobile phase: [water (NH₄HCO₃)-ACN]; gradient: 30-60% B over 9 min) to afford Compound 166 (16 mg, 7.5% yield) as a white solid.

LCMS m/z 636.4 [M+1]⁺

¹H NMR (400 MHz, DMSO-*d₆*) δ = 8.34 (s, 1H), 7.68 (s, 1H), 7.57 - 7.46 (m, 2H), 7.39 (t, *J* = 7.4 Hz, 1H), 7.32 - 7.26 (m, 1H), 7.22 (t, *J* = 7.5 Hz, 1H), 7.10 (d, *J* = 7.4 Hz, 1H), 6.34 (d, *J* = 4.0 Hz, 1H), 4.56 - 4.51 (m, 1H), 4.17 - 4.09 (m, 1H), 3.97 - 3.92 (m, 4H), 3.89 (s, 2H), 3.67 - 3.60 (m, 1H), 3.50 - 3.39 (m, 2H), 2.78 - 2.69 (m, 2H), 2.65 - 2.58 (m, 2H), 2.43 (br t, *J* = 9.6 Hz, 1H), 2.26 (br t, *J* = 10.3 Hz, 1H), 2.16 - 2.12 (m, 4H), 2.08 - 2.03 (m, 4H), 2.03 - 1.78 (m, 3H), 1.78 - 1.64 (m, 4H), 1.49 - 1.30 (m, 2H).

### 35-6. Preparation of Compound 167, 1-(2-(3'-(6-methoxy-5-(((((S)-5-oxopyrrolidin-2-yl)methyl)amino)methyl)pyrazin-2-yl)-2,2'-dimethyl-[1,1'-biphenyl]-3-yl)-4,5,6,7-tetrahydropyrazolo[1,5-a]pyridin-4-yl)piperidine-4-carboxylic acid

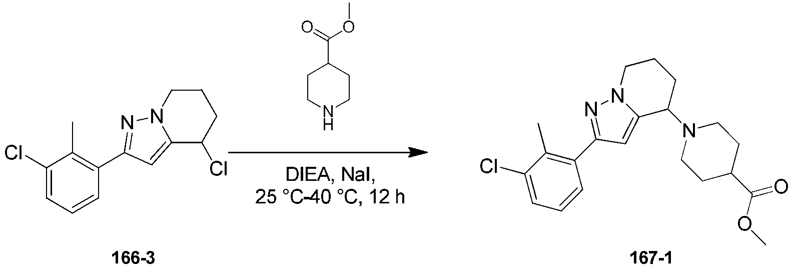

To a mixture of 4-chloro-2-(3-chloro-2-methylphenyl)-4,5,6,7-tetrahydropyrazolo[1,5-a]pyridine (100 mg, 355.64 µmol, 1 eq) and methyl piperidine-4-carboxylate (152.77 mg, 1.07 mmol, 3 eq), DIPEA (137.89 mg, 1.07 mmol, 185.84 µL, 3 eq) and Nal (15.99 mg, 106.69 µmol, 0.3 eq) were added at room temperature. The resulting mixture was stirred at 40 °C for 12 hours. After completion of the reaction, the mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 48-78% B over 15 min) to afford Compound 167-1 (132 mg, 86.1% yield) as a white solid.

LCMS m/z 388.3 [M+1]⁺

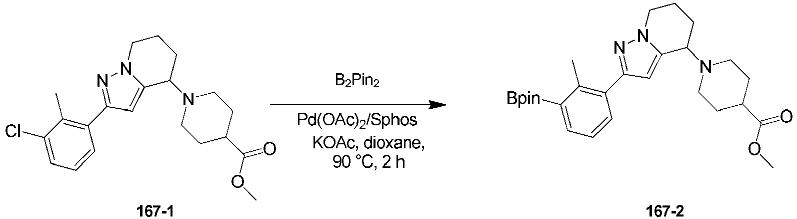

A solution of methyl 1-[2-(3-chloro-2-methylphenyl)-4,5,6,7-tetrahydropyrazolo[1,5-a]pyridin-4-yl]piperidine-4-carboxylate (280 mg, 721.83 µmol, 1 eq), 4,4,5,5-tetramethyl-2-(4,4,5,5-tetramethyl-1,3,2-dioxaborolan-2-yl)-1,3,2-dioxaborolane (366.60 mg, 1.44 mmol, 2 eq), KOAc (141.68 mg, 1.44 mmol, 2 eq), Pd(OAc)₂ (16.21 mg, 72.18 µmol, 0.1 eq), and SPhos (59.27 mg, 144.37 µmol, 0.2 eq) in dioxane (10 mL) was stirred under a nitrogen atmosphere at 90 °C for 2 hours. After completion of the reaction, the mixture was filtered, and the filtrate was concentrated under reduced pressure. The residue was purified by prep-TLC (dichloromethane/methanol = 10/1) to afford Compound 167-2 (460 mg, 93.5% yield) as a white solid.

LCMS m/z 480.3 [M+1]⁺

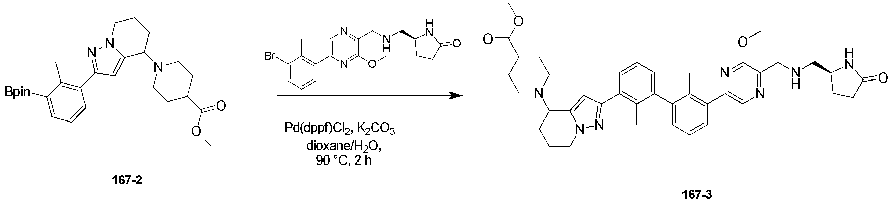

A mixture of methyl 1-[2-[2-methyl-3-(4,4,5,5-tetramethyl-1,3,2-dioxaborolan-2-yl)phenyl]-4,5,6,7-tetrahydropyrazolo[1,5-a]pyridin-4-yl]piperidine-4-carboxylate (212.92 mg, 444.13 µmol, 1.5 eq), (5S)-5-[[[5-(3-bromo-2-methylphenyl)-3-methoxypyrazin-2-yl]methylamino]methyl]pyrrolidin-2-one (120 mg, 296.09 µmol, 1 eq), K₂CO₃ (114.58 mg, 829.04 µmol, 2.8 eq), and ditert-butyl(cyclopentyl)phosphane;dichloropalladium;iron (19.30 mg, 29.61 µmol, 0.1 eq) in dioxane (8 mL)/H₂O (2 mL) was stirred under a nitrogen atmosphere at 90 °C for 2 hours. After completion of the reaction, the mixture was filtered, and the filtrate was concentrated under reduced pressure. The residue was purified by prep-TLC (dichloromethane/methanol = 10/1) to afford Compound 167-3 (186 mg, 83.4% yield) as a white solid.

LCMS m/z 678.5 [M+1]⁺

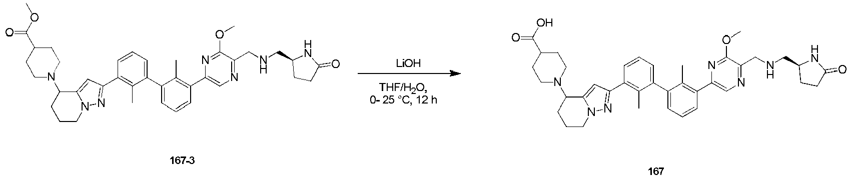

To a solution of methyl 1-[2-[3-[3-[6-methoxy-5-[[[(2S)-5-oxopyrrolidin-2-yl]methylamino]methyl]pyrazin-2-yl]-2-methylphenyl]-2-methylphenyl]-4,5,6,7-tetrahydropyrazolo[1,5-a]pyridin-4-yl]piperidine-4-carboxylate (180 mg, 265.55 µmol, 1 eq) in THF (10 mL)/H₂O (3 mL), LiOH (55.71 mg, 1.33 mmol, 5 eq) was added at 0 °C. The resulting mixture was stirred at room temperature for 12 hours. After completion of the reaction, the mixture was concentrated under reduced pressure, and the residue was purified by prep-HPLC (column: Phenomenex Luna C18, 150 * 25 mm * 10 µm; mobile phase: [water (HCl)-ACN]; gradient: 5-35% B over 10 min) to afford Compound 167 (36 mg, 19.4% yield) as a white solid.

LCMS m/z 644.6 [M+1]⁺

¹H NMR (400 MHz, DMSO-*d₆*) δ = 11.22 - 10.83 (m, 1H), 9.66 - 9.31 (m, 2H), 8.49 (s, 1H), 7.80 (s, 1H), 7.58 - 7.51 (m, 2H), 7.48 - 7.41 (m, 1H), 7.34 (t, J = 7.7 Hz, 1H), 7.30 - 7.19 (m, 2H), 7.15 (d, *J* = 6.6 Hz, 1H), 4.90 (br s, 1H), 4.43 (br t, *J* = 4.6 Hz, 2H), 4.25 - 4.12 (m, 2H), 4.02 (d, *J* = 0.9 Hz, 3H), 3.99 (br d, *J* = 4.6 Hz, 1H), 3.58 - 3.47 (m, 2H), 3.31 - 3.23 (m, 2H), 3.22 - 3.12 (m, 3H), 2.71 - 2.55 (m, 2H), 2.36 - 2.30 (m, 1H), 2.28 - 2.23 (m, 2H), 2.19 (br t, *J* = 3.8 Hz, 2H), 2.15 (s, 3H), 2.07 (d, *J =* 5.3 Hz, 4H), 2.03 - 1.96 (m, 2H), 1.87 - 1.73 (m, 1H).

### [Preparation Example 36]

### Preparation of Compounds 168 to 171

### 36-1. Preparation of Compound 168, (5S)-5-((((5-(2,2'-dichloro-3'-(4-((2-hydroxyethyl)amino)-4,5,6,7-tetrahydropyrazolo[1,5-a]pyridin-2-yl)-[1,1'-biphenyl]-3-yl)-3-methoxypyrazin-2-yl)methyl)amino)methyl)pyrrolidin-2-one

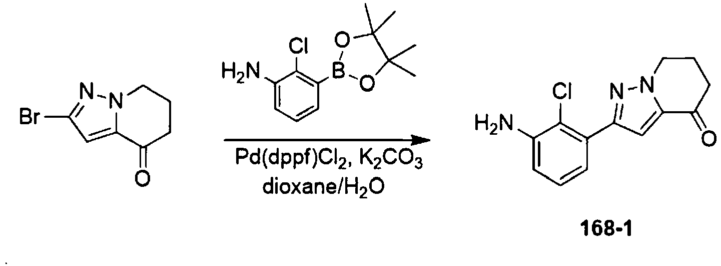

A mixture of 2-bromo-6,7-dihydro-5H-pyrazolo[1,5-a]pyridin-4-one (3 g, 13.95 mmol, 1 eq), 2-chloro-3-(4,4,5,5-tetramethyl-1,3,2-dioxaborolan-2-yl)aniline (4.24 g, 16.74 mmol, 1.2 eq), K₂CO₃ (3.86 g, 27.90 mmol, 2 eq), and Pd(dppf)Cl₂ (510.38 mg, 697.52 µmol, 0.05 eq) in dioxane (30 mL) / H₂O (5 mL) was stirred under a nitrogen atmosphere at 80 °C for 12 hours. After completion of the reaction, the mixture was concentrated under reduced pressure, and the residue was purified by column chromatography (SiO₂, Petroleum ether/Ethyl acetate = 3/1) to afford Compound 168-1 (3.7 g, 85.3% yield) as a white solid.

LCMS m/z 262.1 [M+1]⁺.

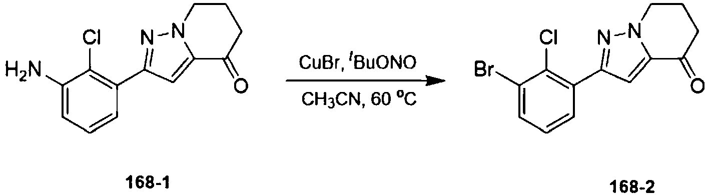

To a solution of 2-(3-amino-2-chloro-phenyl)-6,7-dihydro-5H-pyrazolo[1,5-a]pyridin-4-one (3.7 g, 14.14 mmol, 1 eq) in ACN (35 mL), CuBr (4.06 g, 28.28 mmol, 861.19 µL, 2 eq) and tert-butyl nitrite (2.92 g, 28.28 mmol, 3.36 mL, 2 eq) were added. The resulting mixture was stirred at 60 °C for 1 hour. After completion of the reaction, the mixture was concentrated under reduced pressure, and the residue was purified by column chromatography (SiO₂, Petroleum ether/Ethyl acetate = 1/1) to afford Compound 168-2 (3.9 g, 73.8% yield) as a white solid.

LCMS m/z 327.0 [M+1]⁺.

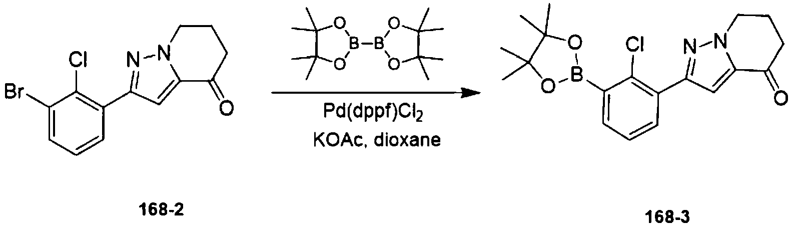

A mixture of 2-(3-bromo-2-chloro-phenyl)-6,7-dihydro-5H-pyrazolo[1,5-a]pyridin-4-one (3.9 g, 11.98 mmol, 1 eq), 4,4,5,5-tetramethyl-2-(4,4,5,5-tetramethyl-1,3,2-dioxaborolan-2-yl)-1,3,2-dioxaborolane (3.65 g, 14.37 mmol, 1.2 eq), Pd(dppf)Cl₂ (438.23 mg, 598.92 µmol, 0.05 eq), and AcOK (2.35 g, 23.96 mmol, 2 eq) in dioxane (40 mL) was stirred under a nitrogen atmosphere at 80 °C for 12 hours. After completion of the reaction, the mixture was concentrated under reduced pressure, and the residue was purified by column chromatography (SiO₂, Petroleum ether/Ethyl acetate = 1/1) to afford Compound 168-3 (2.2 g, 24.6% yield) as a brown liquid.

LCMS m/z 373.2 [M+1]⁺.

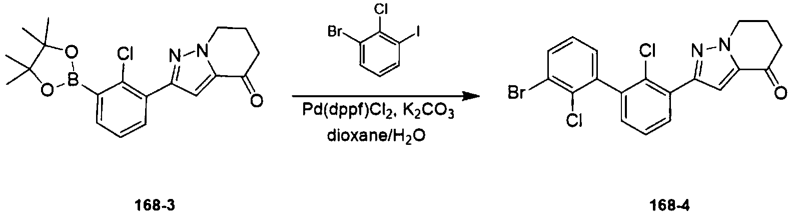

A mixture of 2-[2-chloro-3-(4,4,5,5-tetramethyl-1,3,2-dioxaborolan-2-yl)phenyl]-6,7-dihydro-5H-pyrazolo[1,5-a]pyridin-4-one (2.2 g, 5.90 mmol, 1 eq), 1-bromo-2-chloro-3-iodobenzene (2.25 g, 7.08 mmol, 1.2 eq), K₃PO₄ (3.76 g, 17.71 mmol, 3 eq), and Pd(dppf)Cl₂·CH₂Cl₂ (241.06 mg, 295.18 µmol, 0.05 eq) in dioxane (20 mL)/H₂O (4 mL) was stirred under a nitrogen atmosphere at 60 °C for 12 hours. After completion of the reaction, the mixture was concentrated under reduced pressure, and the residue was purified by prep-HPLC under basic conditions (column: Kromasil Eternity XT, 250 * 80 mm * 10 µm; mobile phase: water (NH₄OAc)/ACN; gradient: 60-90% B over 20 min) to afford Compound 168-4 (700 mg, 27.9% yield) as a white solid.

LCMS m/z 437.0 [M+1]⁺.

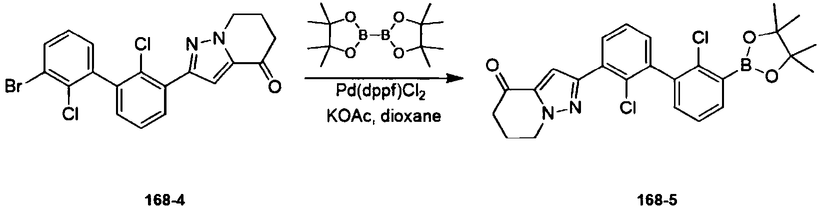

A mixture of 2-[3-(3-bromo-2-chloro-phenyl)-2-chlorophenyl]-6,7-dihydro-5H-pyrazolo[1,5-a]pyridin-4-one (700 mg, 1.61 mmol, 1 eq), 4,4,5,5-tetramethyl-2-(4,4,5,5-tetramethyl-1,3,2-dioxaborolan-2-yl)-1,3,2-dioxaborolane (815.16 mg, 3.21 mmol, 2 eq), AcOK (315.04 mg, 3.21 mmol, 2 eq), and Pd(dppf)Cl₂ (58.72 mg, 80.25 µmol, 0.05 eq) in dioxane (10 mL) was stirred under a nitrogen atmosphere at 90 °C for 12 hours. After completion of the reaction, the mixture was concentrated under reduced pressure, and the residue was purified by column chromatography (SiO₂, Petroleum ether/Ethyl acetate = 3/1 to 2/1) to afford Compound 168-5 (900 mg, 76.5% yield) as a yellow liquid.

LCMS m/z 483.2 [M+1]⁺.

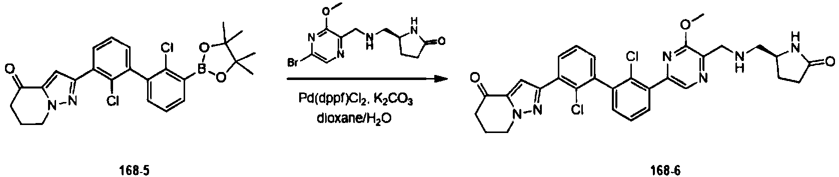

A mixture of (5S)-5-[[(5-bromo-3-methoxypyrazin-2-yl)methylamino]methyl]pyrrolidin-2-one (100 mg, 317.29 µmol, 1 eq), 2-[2-chloro-3-[2-chloro-3-(4,4,5,5-tetramethyl-1,3,2-dioxaborolan-2-yl)phenyl]phenyl]-6,7-dihydro-5H-pyrazolo[1,5-a]pyridin-4-one (183.98 mg, 380.75 µmol, 1.2 eq), K₂CO₃ (87.71 mg, 634.59 µmol, 2 eq), and [2-(2-aminophenyl)phenyl]-chloro-palladium;dicyclohexyl-[3-(2,4,6-triisopropylphenyl)phenyl]phosphane (24.96 mg, 31.73 µmol, 0.1 eq) in dioxane (5 mL) / H₂O (1 mL) was stirred under a nitrogen atmosphere at 80 °C for 4 hours. After completion of the reaction, the mixture was concentrated under reduced pressure, and the residue was purified by prep-TLC (SiO₂, DCM:MeOH = 20:1) to afford Compound 168-6 (80 mg, 35.8% yield) as a white solid.

LCMS m/z 591.2 [M+1]⁺.

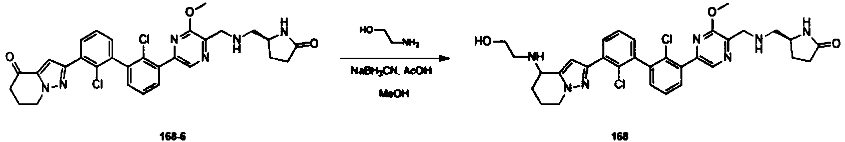

To a solution of 2-[2-chloro-3-[2-chloro-3-[6-methoxy-5-[[[(2S)-5-oxopyrrolidin-2-yl]methylamino]methyl]pyrazin-2-yl]phenyl]phenyl]-6,7-dihydro-5H-pyrazolo[1,5-a]pyridin-4-one (40 mg, 67.63 µmol, 1 eq) and 2-aminoethanol (41.31 mg, 676.26 µmol, 40.82 µL, 10 eq) in MeOH (10 mL), AcOH (8.12 mg, 135.25 µmol, 7.74 µL, 2 eq) and NaBH₃CN (21.25 mg, 338.13 µmol, 5 eq) were added. The resulting mixture was stirred at 60 °C for 12 hours. After completion of the reaction, the mixture was concentrated under reduced pressure, and the residue was purified by reversed-phase HPLC (0.1% NH₃·H₂O; column: Waters XBridge, 150 * 25 mm * 5 µm; mobile phase: [water (NH₄HCO₃)-ACN]; gradient: 25-55% B over 9 min) to afford Compound 168 (10 mg, 23.0% yield) as a white solid.

LCMS m/z 636.3 [M+1]⁺.

¹H NMR (400 MHz, METHANOL-d4) δ ppm 1.77 - 1.91 (m, 2 H) 1.98 - 2.10 (m, 1 H) 2.14 - 2.42 (m, 5 H) 2.70 - 2.83 (m, 2 H) 2.84 - 2.96 (m, 2 H) 3.58 - 3.79 (m, 2 H) 3.79 - 3.91 (m, 1 H) 3.97 - 4.03 (m, 2 H) 4.11 (br s, 4 H) 4.14 - 4.24 (m, 2 H) 6.76 (d, J=2.38 Hz, 1 H) 7.32 (dd, J=7.50, 1.38 Hz, 1 H) 7.37 - 7.47 (m, 2 H) 7.52 (t, J=7.63 Hz, 1 H) 7.60 - 7.76 (m, 2 H) 8.41 (s, 1 H).

### 36-2. Preparation of Compound 169, 2-((2-(2,2'-dichloro-3'-(6-methoxy-5-(((((S)-5-oxopyrrolidin-2-yl)methyl)amino)methyl)pyrazin-2-yl)-[1,1'-biphenyl]-3-yl)-4,5,6,7-tetrahydropyrazolo[1,5-a]pyridin-4-yl)amino)acetic acid

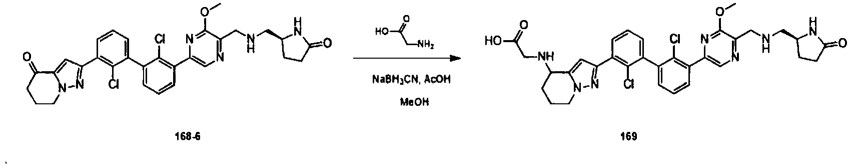

To a solution of 2-[2-chloro-3-[2-chloro-3-[6-methoxy-5-[[[(2S)-5-oxopyrrolidin-2-yl]methylamino]methyl]pyrazin-2-yl]phenyl]phenyl]-6,7-dihydro-5H-pyrazolo[1,5-a]pyridin-4-one (80 mg, 135.25 µmol, 1 eq) and 2-aminoacetic acid (101.53 mg, 1.35 mmol, 10 eq) in MeOH (15 mL), AcOH (16.24 mg, 270.50 µmol, 15.49 µL, 2 eq) and NaBH₃CN (42.50 mg, 676.26 µmol, 5 eq) were added. The resulting mixture was stirred at 60 °C for 12 hours. After completion of the reaction, the mixture was concentrated under reduced pressure, and the residue was purified by reversed-phase HPLC under acidic conditions (0.1% TFA; column: Phenomenex Luna C18, 150 * 40 mm * 15 µm; mobile phase: [water (TFA)-ACN]; gradient: 10-40% B over 15 min), followed by further purification by reversed-phase HPLC under basic conditions (0.1% NH₃·H₂O; column: Waters XBridge, 150 * 25 mm * 5 µm; mobile phase: [water (NH₄HCO₃)-ACN]; gradient: 15-45% B over 9 min), to afford Compound 169 (19 mg, 21.5% yield) as a white solid.

LCMS m/z 650.3 [M+1]⁺.

¹H NMR (400 MHz, DMSO-d6) δ ppm 1.70 (br s, 2 H) 2.06 - 2.14 (m, 3 H) 2.57 - 2.66 (m, 3 H) 3.62 (br s, 5 H) 3.90 (s, 3 H) 3.98 (s, 4 H) 4.06 - 4.16 (m, 4 H) 6.74 (d, J=2.50 Hz, 1 H) 7.36 (d, J=7.50 Hz, 1 H) 7.45 - 7.51 (m, 2 H) 7.59 (t, J=7.63 Hz, 1 H) 7.69 (s, 1 H) 7.72 (d, J=7.36 Hz, 1 H) 7.77 - 7.80 (m, 1 H) 8.44 (s, 1 H).

### 36-3. Preparation of Compound 170, (5S)-5-((((5-(2,2'-dichloro-3'-(4-((R)-3-hydroxypyrrolidin-1-yl)-4,5,6,7-tetrahydropyrazolo[1,5-a]pyridin-2-yl)-[1,1'-biphenyl]-3-yl)-3-methoxypyrazin-2-yl)methyl)amino)methyl)pyrrolidin-2-one

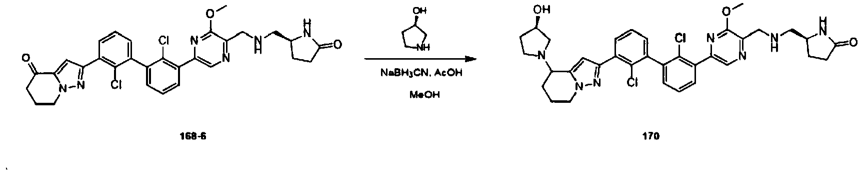

To a solution of 2-[2-chloro-3-[2-chloro-3-[6-methoxy-5-[[[(2S)-5-oxopyrrolidin-2-yl]methylamino]methyl]pyrazin-2-yl]phenyl]phenyl]-6,7-dihydro-5H-pyrazolo[1,5-a]pyridin-4-one (40 mg, 67.63 µmol, 1 eq) and (3R)-pyrrolidin-3-ol (58.92 mg, 676.26 µmol, 56.22 µL, 10 eq) in MeOH (10 mL), AcOH (8.12 mg, 135.25 µmol, 7.74 µL, 2 eq) and NaBH₃CN (21.25 mg, 338.13 µmol, 5 eq) were added. The resulting mixture was stirred at 50 °C for 12 hours. After completion of the reaction, the mixture was concentrated under reduced pressure, and the residue was purified by reversed-phase HPLC (0.1% NH₃·H₂O; column: Waters XBridge, 150 * 25 mm * 5 µm; mobile phase: [water (NH₄HCO₃)-ACN]; gradient: 28-58% B over 9 min) to afford Compound 170 (8 mg, 16.9% yield) as a white solid.

LCMS m/z 662.4 [M+1]⁺.

¹H NMR (400 MHz, METHANOL-d4) δ ppm 1.67 - 1.89 (m, 2 H) 1.95 - 2.16 (m, 4 H) 2.22 - 2.43 (m, 4 H) 2.61 - 2.84 (m, 4 H) 2.85 - 3.05 (m, 2 H) 3.80 - 3.92 (m, 2 H) 4.00 (d, J=1.88 Hz, 2 H) 4.05 (s, 3 H) 4.12 - 4.24 (m, 2 H) 4.33 (br s, 1 H) 6.71 (d, J=2.75 Hz, 1 H) 7.26 - 7.35 (m, 1 H) 7.39 (dd, J=7.57, 1.19 Hz, 1 H) 7.43 (t, J=7.63 Hz, 1 H) 7.51 (t, J=7.63 Hz, 1 H) 7.63 - 7.75 (m, 2 H) 8.41 (s, 1 H).

### 36-4. Preparation of Compound 171, (3R)-1-(2-(2,2'-dichloro-3'-(6-methoxy-5-(((((S)-5-oxopyrrolidin-2-yl)methyl)amino)methyl)pyrazin-2-yl)-[1,1'-biphenyl]-3-yl)-4,5,6,7-tetrahydropyrazolo[1,5-a]pyridin-4-yl)pyrrolidine-3-carboxylic acid

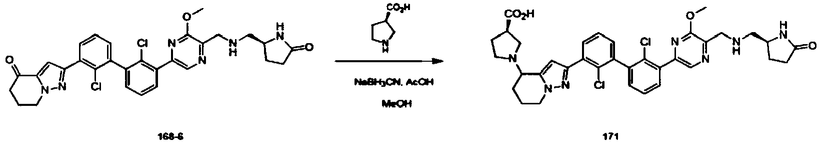

To a solution of 2-[2-chloro-3-[2-chloro-3-[6-methoxy-5-[[[(2S)-5-oxopyrrolidin-2-yl]methylamino]methyl]pyrazin-2-yl]phenyl]phenyl]-6,7-dihydro-5H-pyrazolo[1,5-a]pyridin-4-one (80 mg, 135.25 µmol, 1 eq) and (3R)-pyrrolidine-3-carboxylic acid (155.72 mg, 1.35 mmol, 10 eq) in MeOH (15 mL), AcOH (16.24 mg, 270.50 µmol, 15.49 µL, 2 eq) and NaBH₃CN (42.50 mg, 676.26 µmol, 5 eq) were added. The resulting mixture was stirred at 60 °C for 12 hours. After completion of the reaction, the mixture was concentrated under reduced pressure, and the residue was purified by reversed-phase HPLC (0.1% NH₃·H₂O; column: Waters XBridge, 150 * 25 mm * 5 µm; mobile phase: [water (NH₄HCO₃)-ACN]; gradient: 12-42% B over 9 min) to afford Compound 171 (26 mg, 26.6% yield) as a white solid.

LCMS m/z 662.4 [M+1]⁺.

¹H NMR (400 MHz, DMSO-d6) δ ppm 1.23 (br s, 1 H) 1.70 (br s, 2 H) 1.93 (br s, 6 H) 2.07 - 2.10 (m, 2 H) 2.24 (br s, 2 H) 2.29 - 2.36 (m, 2 H) 2.90 (br d, J=15.63 Hz, 3 H) 3.63 (br s, 2 H) 3.80 (br s, 1 H) 3.92 (s, 2 H) 3.98 (s, 3 H) 4.06 - 4.19 (m, 2 H) 6.58 - 6.63 (m, 1 H) 7.36 (d, J=7.11 Hz, 1 H) 7.43 - 7.52 (m, 2 H) 7.59 (t, J=7.12 Hz, 1 H) 7.67 - 7.74 (m, 2 H) 7.76 - 7.82 (m, 1 H) 8.44 (s, 1 H).

### [Preparation Example 37]

### Preparation of Compounds 174 to 179

### 37-1. Preparation of Compound 174, (3R)-1-((8-((3'-(4-((2-hydroxyethyl)amino)-4,5,6,7-tetrahydropyrazolo[1,5-a]pyridin-2-yl)-2,2'-dimethyl-[1,1'-biphenyl]-3-yl)amino)-1,7-naphthyridin-3-yl)methyl)pyrrolidin-3-ol

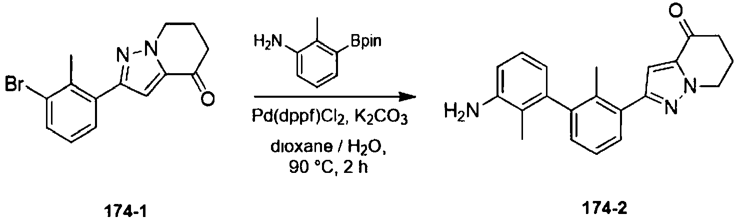

A mixture of 2-(3-bromo-2-methylphenyl)-6,7-dihydro-5H-pyrazolo[1,5-a]pyridin-4-one (1.2 g, 3.93 mmol, 1 eq), 2-methyl-3-(4,4,5,5-tetramethyl-1,3,2-dioxaborolan-2-yl)aniline (1.01 g, 4.33 mmol, 1.1 eq), K₂CO₃ (1.52 g, 11.01 mmol, 2.8 eq), and Pd(dppf)Cl₂ (287.73 mg, 393.22 µmol, 0.1 eq) in dioxane (15 mL) / H₂O (2 mL) was stirred under a nitrogen atmosphere at 90 °C for 2 hours. After completion of the reaction, the mixture was diluted with H₂O (40 mL) and extracted with ethyl acetate (30 mL x 3). The combined organic layers were washed with brine (60 mL), dried over Na₂SO₄, and concentrated under reduced pressure. The residue was purified by flash silica gel chromatography (Petroleum ether/Ethyl acetate = 100/1 to 1/1) to afford Compound 174-2 (820 mg, 56.6% yield) as a yellow liquid.

LCMS m/z 332.4 [M+1]⁺.

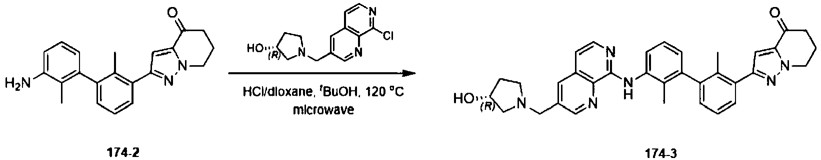

To a solution of (3R)-1-[(8-chloro-1,7-naphthyridin-3-yl)methyl]pyrrolidin-3-ol (596.82 mg, 2.26 mmol, 1.5 eq) and 2-[3-(3-amino-2-methylphenyl)-2-methylphenyl]-6,7-dihydro-5H-pyrazolo[1,5-a]pyridin-4-one (500 mg, 1.51 mmol, 1 eq) in t-BuOH (10 mL), HCl/dioxane (4 M, 754.35 µL, 2 eq) was added. The resulting mixture was stirred at 120 °C for 12 hours. After completion of the reaction, the pH of the mixture was adjusted to 12 by addition of an aqueous 1 N NaOH solution (40 mL), and the precipitated solid was collected by filtration to afford Compound 174-3 (720 mg, 68.3% yield) as a white solid, which was used in the subsequent reaction without further purification.

LCMS m/z 559.5 [M+1]⁺.

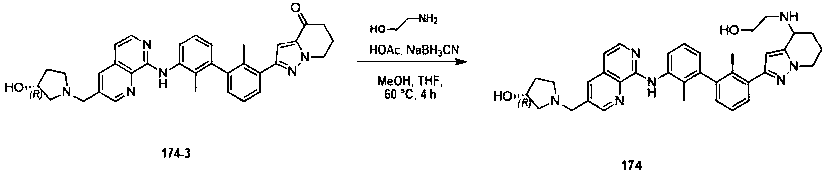

A mixture of 2-[3-[3-[[3-[[(3R)-3-hydroxypyrrolidin-1-yl]methyl]-1,7-naphthyridin-8-yl]amino]-2-methylphenyl]-2-methylphenyl]-6,7-dihydro-5H-pyrazolo[1,5-a]pyridin-4-one (100 mg, 179.00 µmol, 1 eq), 2-aminoethanol (109.34 mg, 1.79 mmol, 108.25 µL, 10 eq), and HOAc (107.49 mg, 1.79 mmol, 102.47 µL, 10 eq) in MeOH (10 mL) / THF (10 mL) was stirred at 60 °C for 2 hours. NaBH₃CN (33.74 mg, 536.99 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; B%: 45-75% over 9 min) to afford Compound 174 (42 mg, 36.9% yield) as a white solid.

LCMS m/z 604.6 [M+1]⁺.

¹H NMR (400 MHz, DMSO-d6) δ = 9.30 (s, 1H), 8.86 (d, J = 1.8 Hz, 1H), 8.44 (d, J = 8.0 Hz, 1H), 8.18 (d, J = 1.3 Hz, 1H), 8.05 (d, J = 5.8 Hz, 1H), 7.49 (br d, J = 7.8 Hz, 1H), 7.36 - 7.25 (m, 2H), 7.17 (d, J = 5.9 Hz, 1H), 7.09 (d, J = 7.4 Hz, 1H), 6.88 (br d, J = 8.0 Hz, 1H), 6.47 (s, 1H), 4.74 (d, J = 4.5 Hz, 1H), 4.58 (br s, 1H), 4.29 - 4.17 (m, 1H), 4.09 (br t, J = 6.0 Hz, 2H), 3.97 (br s, 1H), 3.90 - 3.71 (m, 2H), 3.50 (br d, J = 3.1 Hz, 2H), 3.17 (d, J = 4.4 Hz, 2H), 2.83 - 2.72 (m, 3H), 2.66 (q, J = 7.5 Hz, 1H), 2.38 (dd, J = 3.5, 9.8 Hz, 1H), 2.29 - 2.17 (m, 1H), 2.14 (s, 3H), 2.08 (d, J = 3.6 Hz, 3H), 2.05 - 1.86 (m, 3H), 1.78 - 1.52 (m, 2H).

### 37-2. Preparation of Compound 175, N-(2-((2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)amino)ethyl)acetamide

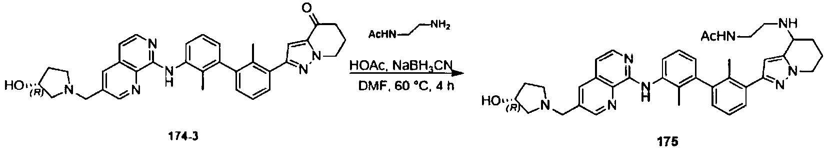

A solution of 2-[3-[3-[[3-[[(3R)-3-hydroxypyrrolidin-1-yl]methyl]-1,7-naphthyridin-8-yl]amino]-2-methylphenyl]-2-methylphenyl]-6,7-dihydro-5H-pyrazolo[1,5-a]pyridin-4-one (100 mg, 179.00 µmol, 1 eq), N-(2-aminoethyl)acetamide (182.82 mg, 1.79 mmol, 171.50 µL, 10 eq), and HOAc (107.49 mg, 1.79 mmol, 102.47 µL, 10 eq) in DMF (5 mL) was stirred at 60 °C for 2 hours. NaBH₃CN (33.74 mg, 536.99 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; gradient: 32-62% B over 9 min) to afford Compound 175 (16 mg, 13.1% yield) as a white solid.

LCMS m/z 645.4 [M+1]⁺.

¹H NMR (400 MHz, DMSO-d6) δ = 9.29 (s, 1H), 8.86 (d, J = 1.5 Hz, 1H), 8.43 (d, J = 7.8 Hz, 1H), 8.17 (d, J = 1.5 Hz, 1H), 8.05 (d, J = 5.8 Hz, 1H), 7.81 (br t, J = 5.3 Hz, 1H), 7.49 (br d, J = 7.8 Hz, 1H), 7.30 (td, J = 7.8, 10.6 Hz, 2H), 7.17 (d, J = 5.8 Hz, 1H), 7.08 (d, J = 6.8 Hz, 1H), 6.91 - 6.84 (m, 1H), 6.44 (s, 1H), 4.73 (br d, J = 4.1 Hz, 1H), 4.27 - 4.17 (m, 1H), 4.08 (br t, J = 6.1 Hz, 2H), 3.93 - 3.85 (m, 1H), 3.80 (d, J = 11.0 Hz, 2H), 3.21 - 3.04 (m, 4H), 2.73 (dd, J = 6.1, 9.7 Hz, 1H), 2.69 - 2.60 (m, 3H), 2.47 (br d, J = 6.1 Hz, 1H), 2.38 (dd, J = 3.5, 9.6 Hz, 1H), 2.19 (br dd, J = 2.1, 7.9 Hz, 1H), 2.14 (s, 3H), 2.09 - 2.06 (m, 3H), 2.05 - 1.97 (m, 2H), 1.94 - 1.84 (m, 1H), 1.78 (d, J = 1.1 Hz, 3H), 1.72 - 1.52 (m, 2H).

### 37-3. Preparation of Compound 176, (5S)-5-(((2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)amino)methyl)pyrrolidin-2-one

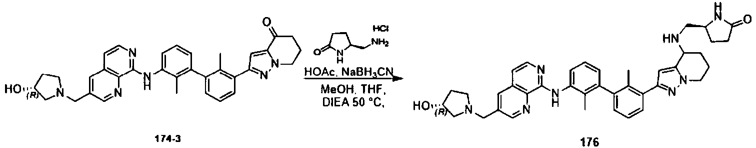

A solution of 2-[3-[3-[[3-[[(3R)-3-hydroxypyrrolidin-1-yl]methyl]-1,7-naphthyridin-8-yl]amino]-2-methyl-phenyl]-2-methyl-phenyl]-6,7-dihydro-5H-pyrazolo[1,5-a]pyridin-4-one (100 mg, 179.00 µmol, 1 eq), (5S)-5-(aminomethyl)pyrrolidin-2-one (204.32 mg, 1.79 mmol, 297.14 µL, 10 eq), and DIPEA (231.33 mg, 1.79 mmol, 311.77 µL, 10 eq) in MeOH (5 mL) / THF (1 mL) was stirred at 60 °C for 2 hours. NaBH₃CN (56.24 mg, 894.98 µmol, 5 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; gradient: 32-62% B over 9 min) to afford Compound 176 (28 mg, 22.6% yield) as a white solid.

LCMS m/z 657.4 [M+1]⁺.

¹H NMR (400 MHz, DMSO-d6 ) δ = 9.30 (s, 1H), 8.86 (d, J = 1.6 Hz, 1H), 8.43 (d, J = 8.0 Hz, 1H), 8.18 (d, J = 1.6 Hz, 1H), 8.05 (d, J = 5.8 Hz, 1H), 7.67 (d, J = 16.5 Hz, 1H), 7.49 (d, J = 7.4 Hz, 1H), 7.37 - 7.26 (m, 2H), 7.18 (d, J = 5.9 Hz, 1H), 7.09 (d, J = 7.3 Hz, 1H), 6.88 (dd, J = 2.9, 6.9 Hz, 1H), 6.47 (d, J = 9.0 Hz, 1H), 4.73 (d, J = 4.5 Hz, 1H), 4.32 - 4.18 (m, 1H), 4.11 - 4.07 (m, 2H), 3.94 - 3.72 (m, 3H), 3.67 - 3.54 (m, 1H), 3.17 (d, J = 5.3 Hz, 3H), 2.74 (dd, J = 6.1, 9.6 Hz, 1H), 2.66 - 2.61 (m, 2H), 2.48 - 2.30 (m, 2H), 2.28 - 2.16 (m, 2H), 2.14 (d, J = 1.4 Hz, 3H), 2.08 (br d, J = 4.0 Hz, 4H), 2.02 (br dd, J = 6.8, 13.1 Hz, 2H), 1.96 - 1.85 (m, 1H), 1.77 - 1.65 (m, 2H), 1.63 - 1.53 (m, 1H).

### 37-4. Preparation of Compound 177, (3R)-1-((8-((3'-(4-((R)-3-hydroxypyrrolidin-1-yl)-4,5,6,7-tetrahydropyrazolo[1,5-a]pyridin-2-yl)-2,2'-dimethyl-[1,1'-biphenyl]-3-yl)amino)-1,7-naphthyridin-3-yl)methyl)pyrrolidin-3-ol

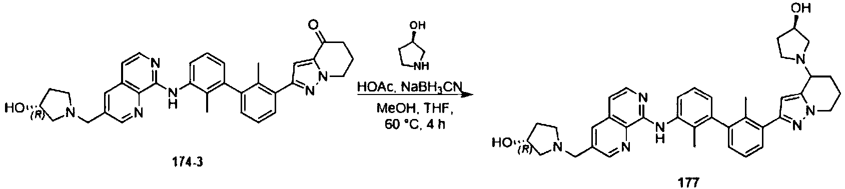

A solution of 2-[3-[3-[[3-[[(3R)-3-hydroxypyrrolidin-1-yl]methyl]-1,7-naphthyridin-8-yl]amino]-2-methyl-phenyl]-2-methyl-phenyl]-6,7-dihydro-5H-pyrazolo[1,5-a]pyridin-4-one (100 mg, 179.00 µmol, 1 eq), (3R)-pyrrolidin-3-ol (155.94 mg, 1.79 mmol, 148.52 µL, 10 eq), and HOAc (107.49 mg, 1.79 mmol, 102.47 µL, 10 eq) in MeOH (10 mL) / THF (10 mL) was stirred at 60 °C for 2 hours. NaBH₃CN (33.74 mg, 536.99 µmol, 3 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; B%: 38-68% over 9 min) to afford Compound 177 (46 mg, 38.7% yield) as a white solid.

LCMS m/z 630.6 [M+1]⁺.

¹H NMR (400 MHz, DMSO-d6) δ = 9.29 (s, 1H), 8.85 (d, J = 1.1 Hz, 1H), 8.44 (d, J = 8.2 Hz, 1H), 8.17 (s, 1H), 8.05 (d, J = 5.6 Hz, 1H), 7.49 (d, J = 7.8 Hz, 1H), 7.36 - 7.24 (m, 2H), 7.17 (d, J = 5.7 Hz, 1H), 7.08 (d, J = 7.3 Hz, 1H), 6.87 (dd, J = 3.4, 7.3 Hz, 1H), 6.40 (dd, J = 2.1, 7.3 Hz, 1H), 4.79 - 4.64 (m, 2H), 4.29 - 4.16 (m, 2H), 4.15 - 3.90 (m, 3H), 3.85 - 3.71 (m, 3H), 3.17 (d, J = 5.1 Hz, 2H), 2.96 - 2.74 (m, 2H), 2.72 - 2.58 (m, 2H), 2.46 - 2.35 (m, 2H), 2.23 (br d, J = 8.2 Hz, 1H), 2.14 (s, 3H), 2.08 (d, J = 3.7 Hz, 3H), 1.95 (br s, 2H), 1.92 - 1.80 (m, 3H), 1.67 - 1.45 (m, 2H).

### 37-5. Preparation of Compound 178, (3R)-1-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)pyrrolidine-3-carboxylic acid

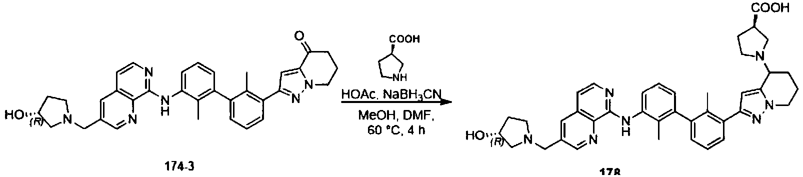

To a solution of 2-[3-[3-[[3-[[(3R)-3-hydroxypyrrolidin-1-yl]methyl]-1,7-naphthyridin-8-yl]amino]-2-methylphenyl]-2-methylphenyl]-6,7-dihydro-5H-pyrazolo[1,5-a]pyridin-4-one (100 mg, 179.00 µmol, 1 eq) and (3R)-pyrrolidine-3-carboxylic acid (206.08 mg, 1.79 mmol, 10 eq) in MeOH (5 mL) / DMF (5 mL), HOAc (214.97 mg, 3.58 mmol, 204.93 µL, 20 eq) was added. The resulting mixture was stirred at 60 °C for 2 hours. NaBH₃CN (56.24 mg, 894.98 µmol, 5 eq) was then added to the reaction mixture, followed by stirring at 60 °C for an additional 2 hours. The reaction mixture was concentrated under reduced pressure, and the residue was purified by prep-HPLC (column: Waters XBridge Prep OBD C18, 150 * 40 mm, 10 µm; mobile phase: [water (NH₄HCO₃)-ACN]; gradient: 10-40% B over 15 min) to afford Compound 178 (32 mg, 25.8% yield) as a white solid.

LCMS m/z 658.4 [M+1]⁺.

¹H NMR (400 MHz, DMSO-d6) δ = 9.29 (s, 1H), 8.86 (d, J = 1.7 Hz, 1H), 8.44 (d, J = 8.2 Hz, 1H), 8.18 (d, J = 1.6 Hz, 1H), 8.06 (d, J = 5.7 Hz, 1H), 7.50 (d, J = 6.8 Hz, 1H), 7.35 - 7.26 (m, 2H), 7.17 (d, J = 5.9 Hz, 1H), 7.09 (d, J = 7.5 Hz, 1H), 6.88 (dd, J = 3.4, 7.0 Hz, 1H), 6.39 (dd, J = 2.9, 6.7 Hz, 1H), 4.26 - 4.19 (m, 1H), 4.14 - 4.04 (m, 2H), 3.82 - 3.73 (m, 3H), 3.17 (s, 2H), 2.99 - 2.84 (m, 3H), 2.80 - 2.69 (m, 3H), 2.64 (br t, J = 7.9 Hz, 2H), 2.38 (dd, J = 3.6, 9.6 Hz, 1H), 2.31 - 2.20 (m, 1H), 2.14 (s, 3H), 2.11 - 2.06 (m, 4H), 2.05 - 1.99 (m, 1H), 1.98 - 1.89 (m, 4H), 1.69 - 1.47 (m, 1H).

### 37-6. Preparation of Compound 179, 2-((2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,5,6,7-tetrahydropyrazolo[1,5-a]pyridin-4-yl)amino)acetic acid

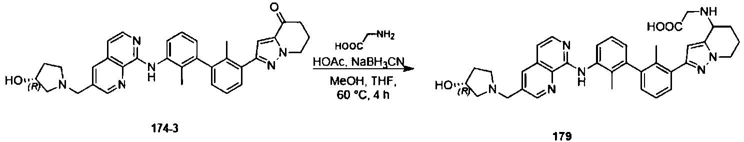

To a solution of 2-[3-[3-[[3-[[(3R)-3-hydroxypyrrolidin-1-yl]methyl]-1,7-naphthyridin-8-yl]amino]-2-methylphenyl]-2-methylphenyl]-6,7-dihydro-5H-pyrazolo[1,5-a]pyridin-4-one (200 mg, 357.99 µmol, 1 eq) and 2-aminoacetic acid (268.73 mg, 3.58 mmol, 297.04 µL, 10 eq) in MeOH (5 mL) / THF (1 mL), HOAc (429.95 mg, 7.16 mmol, 409.87 µL, 20 eq) was added. The resulting mixture was stirred at 60 °C for 2 hours. NaBH₃CN (112.48 mg, 1.79 mmol, 5 eq) was then added to the reaction mixture, followed by stirring at 60 °C for an additional 2 hours. The reaction mixture was concentrated under reduced pressure, and the residue was purified by prep-HPLC (column: Waters XBridge, 150 * 25 mm * 5 µm; mobile phase: [water (NH₄HCO₃)-ACN]; gradient: 15-45% B over 9 min) to afford Compound 179 (43 mg, 18.4% yield) as a white solid.

LCMS m/z 618.5 [M+1]⁺.

¹H NMR (400 MHz, DMSO-d6) δ = 11.66 (br s, 1H), 9.94 (br d, J = 3.9 Hz, 2H), 9.54 - 9.36 (m, 1H), 8.90 - 8.72 (m, 1H), 7.70 (d, J = 7.0 Hz, 1H), 7.63 - 7.46 (m, 3H), 7.40 - 7.27 (m, 3H), 7.19 (d, J = 7.4 Hz, 1H), 6.96 (s, 1H), 4.80 - 4.71 (m, 3H), 4.49 - 4.41 (m, 3H), 4.14 (br d, J = 5.8 Hz, 3H), 3.98 (br s, 2H), 3.47 (br s, 2H), 3.42 - 3.23 (m, 2H), 3.16 (s, 2H), 2.35 - 2.25 (m, 2H), 2.19 (s, 3H), 2.14 - 2.00 (m, 2H), 1.96 (s, 3H).

### [Preparation Example 38]

### Preparation of Compounds 180 to 185

### 38-1. Preparation of Compound 180, (3R)-1-((8-((2,2'-dichloro-3'-(4-((2-hydroxyethyl)amino)-4,5,6,7-tetrahydropyrazolo[1,5-a]pyridin-2-yl)-[1,1'-biphenyl]-3-yl)amino)-1,7-naphthyridin-3-yl)methyl)pyrrolidin-3-ol

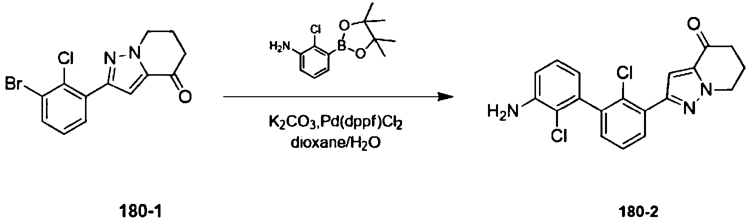

To a solution of 2-chloro-3-(4,4,5,5-tetramethyl-1,3,2-dioxaborolan-2-yl)aniline (2.38 g, 9.38 mmol, 1 eq) and 2-(3-bromo-2-chloro-phenyl)-6,7-dihydro-5H-pyrazolo[1,5-a]pyridin-4-one (3.36 g, 10.32 mmol, 1.1 eq) in dioxane (15 mL) / H₂O (3 mL), K₂CO₃ (2.59 g, 18.76 mmol, 2 eq) and ditert-butyl(cyclopentyl)phosphane;dichloropalladium;iron (611.42 mg, 938.13 µmol, 0.1 eq) were added, and the resulting mixture was stirred at 90 °C for 12 hours. After completion of the reaction, the mixture was concentrated under reduced pressure, and the residue was purified by column chromatography (SiO₂, Petroleum ether/Ethyl acetate = 1/1) to afford Compound 180-2 (1 g, 26.8% yield) as a yellow solid.

LCMS m/z 372.1 [M+1]⁺.

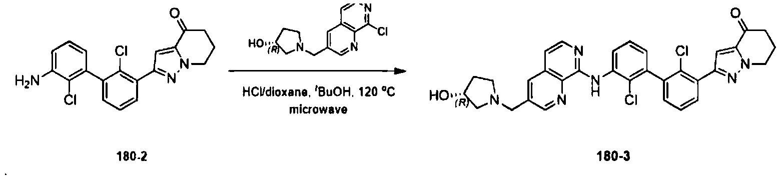

To a solution of 2-[3-(3-amino-2-chloro-phenyl)-2-chlorophenyl]-6,7-dihydro-5H-pyrazolo[1,5-a]pyridin-4-one (500 mg, 1.34 mmol, 1 eq) and (3R)-1-[(8-chloro-1,7-naphthyridin-3-yl)methyl]pyrrolidin-3-ol (885.57 mg, 3.36 mmol, 2.5 eq) in t-BuOH (10 mL), HCl/dioxane (4 M, 671.60 µL, 2 eq) was added. The resulting mixture was stirred at 120 °C for 12 hours. After completion of the reaction, the mixture was concentrated under reduced pressure to afford Compound 180-3 (880 mg, 89.3% yield) as a brown solid, which was used in the subsequent reaction without further purification.

LCMS m/z 599.3 [M+1]⁺.

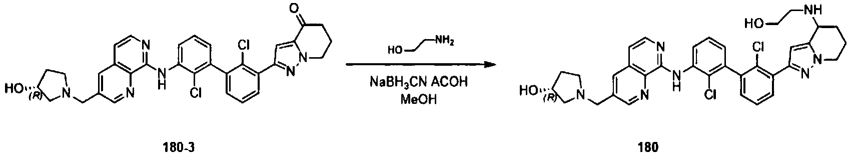

To a solution of 2-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-5H-pyrazolo[1,5-a]pyridin-4-one (250 mg, 417.01 µmol, 1 eq) and 2-aminoethanol (254.72 mg, 4.17 mmol, 251.70 µL, 10 eq) in MeOH (10 mL), AcOH (50.08 mg, 834.02 µmol, 47.74 µL, 2 eq) and NaBH₃CN (131.03 mg, 2.09 mmol, 5 eq) were added. The resulting mixture was stirred at 60 °C for 12 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 38-68% B over 9 min) to afford Compound 180 (25 mg, 9.30% yield) as a brown solid.

LCMS m/z 644.2 [M+1]⁺.

¹H NMR (400 MHz, DMSO-d6) δ ppm 1.53 - 1.74 (m, 2 H) 1.86 - 2.07 (m, 4 H) 2.20 (br dd, J = 13.51, 5.38 Hz, 1 H) 2.38 (dd, J = 9.63, 3.50 Hz, 1 H) 2.60 - 2.78 (m, 5 H) 3.47 (q, J = 5.34 Hz, 2 H) 3.75 - 3.84 (m, 2 H) 3.89 (br dd, J = 11.76, 6.00 Hz, 1 H) 4.11 (br t, J = 6.07 Hz, 2 H) 4.19 - 4.25 (m, 1 H) 4.43 - 4.53 (m, 1 H) 4.72 (d, J = 4.50 Hz, 1 H) 6.68 (d, J = 2.75 Hz, 1 H) 7.03 (ddd, J = 7.47, 3.72, 1.44 Hz, 1 H) 7.33 (d, J = 5.88 Hz, 2 H) 7.43 - 7.54 (m, 2 H) 7.79 (ddd, J = 7.79, 3.35, 1.63 Hz, 1 H) 8.19 (d, J = 5.88 Hz, 1 H) 8.24 (s, 1 H) 8.87 - 8.94 (m, 1 H) 9.12 (dd, J = 8.38, 1.25 Hz, 1 H) 9.86 - 9.91 (m, 1 H)

### 38-2. Preparation of Compound 181, N-(2-((2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)amino)ethyl)acetamide

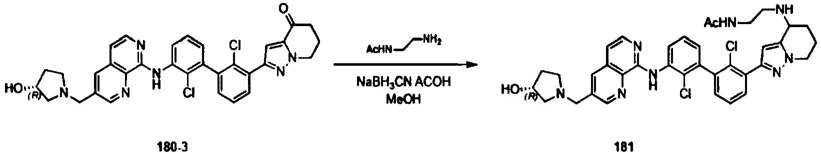

To a solution of 2-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-5H-pyrazolo[1,5-a]pyridin-4-one (220 mg, 366.97 µmol, 1 eq) and N-(2-aminoethyl)acetamide (374.80 mg, 3.67 mmol, 10 eq) in MeOH (10 mL), AcOH (44.07 mg, 733.93 µmol, 42.02 µL, 2 eq) and NaBH₃CN (115.30 mg, 1.83 mmol, 5 eq) were added. The resulting mixture was stirred at 60 °C for 4 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 35-65% B over 9 min) to afford Compound 181 (32 mg, 12.5% yield) as a white solid.

LCMS m/z 685.5 [M+1]⁺.

¹H NMR (400 MHz, DMSO-d6) δ ppm 1.53 - 1.62 (m, 1 H) 1.64 - 1.73 (m, 1 H) 1.77 (s, 3 H) 1.85 - 1.95 (m, 1 H) 1.96 - 2.09 (m, 3 H) 2.20 (br dd, J=13.82, 5.75 Hz, 2 H) 2.38 (dd, J=9.60, 3.48 Hz, 1 H) 2.62 - 2.67 (m, 3 H) 2.73 (dd, J = 9.54, 6.24 Hz, 1 H) 3.09 - 3.16 (m, 2 H) 3.78 - 3.91 (m, 3 H) 4.10 (br t, J=6.17 Hz, 2 H) 4.22 (br s, 1 H) 4.70 - 4.77 (m, 1 H) 6.68 (d, J=2.45 Hz, 1 H) 7.03 (ddd, J=7.55, 4.19, 1.47 Hz, 1 H) 7.31 - 7.35 (m, 2 H) 7.44 - 7.53 (m, 2 H) 7.77 - 7.82 (m, 2 H) 8.18 (d, J=5.87 Hz, 1 H) 8.23 (s, 1 H) 8.89 - 8.92 (m, 1 H) 9.12 (dd, J=8.44, 1.34 Hz, 1 H) 9.88 (d, J=1.96 Hz, 1 H)

### 38-3. Preparation of Compound 182, (5S)-5-(((2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)amino)methyl)pyrrolidin-2-one

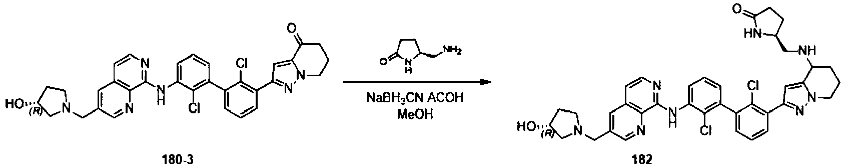

To a solution of 2-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-5H-pyrazolo[1,5-a]pyridin-4-one (220 mg, 366.97 µmol, 1 eq) and (5S)-5-(aminomethyl)pyrrolidin-2-one (418.88 mg, 3.67 mmol, 10 eq) in MeOH (10 mL), AcOH (44.07 mg, 733.93 µmol, 40.02 µL, 2 eq), DIPEA (237.14 mg, 1.83 mmol, 319.60 µL, 5 eq), and NaBH₃CN (115.30 mg, 1.83 mmol, 5 eq) were added. The resulting mixture was stirred at 60 °C for 12 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 38-68% B over 9 min) to afford Compound 182 (46 mg, 17.7% yield) as a white solid.

LCMS m/z 697.3[M+1]⁺.

¹H NMR (400 MHz, DMSO-d6) δ ppm 1.53 - 1.77 (m, 3 H) 1.92 (br dd, J=13.51, 6.75 Hz, 1 H) 1.97 - 2.07 (m, 3 H) 2.07 - 2.13 (m, 2 H) 2.15 - 2.25 (m, 2 H) 2.39 (dd, J=9.51, 3.50 Hz, 1 H) 2.44 - 2.49 (m, 1 H) 2.59 - 2.69 (m, 3 H) 2.74 (dd, J=9.51, 6.13 Hz, 1 H) 3.53 - 3.61 (m, 1 H) 3.76 - 3.83 (m, 2 H) 3.85 - 3.93 (m, 1 H) 4.08 - 4.16 (m, 2 H) 4.18 - 4.26 (m, 1 H) 4.74 (d, J=4.38 Hz, 1 H) 6.72 (d, J=7.25 Hz, 1 H) 6.95 - 7.12 (m, 1 H) 7.34 (d, J=10.48 Hz, 1 H) 7.33 (s, 1 H) 7.43 - 7.55 (m, 2 H) 7.64 - 7.72 (m, 1 H) 7.79 (dt, J=7.82, 1.91 Hz, 1 H) 8.19 (d, J=5.75 Hz, 1 H) 8.23 (s, 1 H) 8.90 (s, 1 H) 9.13 (dd, J=8.32, 1.31 Hz, 1 H) 9.86 - 9.92 (m, 1 H)

### 38-4. Preparation of Compound 183, (3R)-1-(2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)pyrrolidin-3-ol

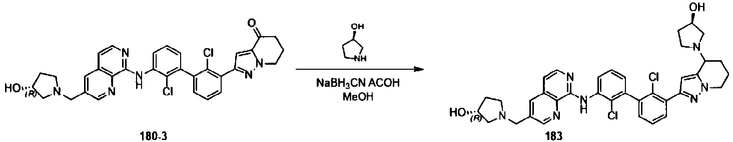

To a solution of 2-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-5H-pyrazolo[1,5-a]pyridin-4-one (250 mg, 417.01 µmol, 1 eq) and (3R)-pyrrolidin-3-ol (363.30 mg, 4.17 mmol, 346.66 µL, 10 eq) in MeOH (10 mL), AcOH (50.08 mg, 834.02 µmol, 47.74 µL, 2 eq) and NaBH₃CN (131.03 mg, 2.09 mmol, 5 eq) were added. The resulting mixture was stirred at 60 °C for 4 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 42-72% B over 9 min) to afford Compound 183 (32 mg, 11.1% yield) as a white solid.

LCMS m/z 670.2[M+1]⁺.

¹H NMR (400 MHz, DMSO-d6) δ ppm 1.56 (br s, 2 H) 1.89 (br s, 3 H) 1.98 - 2.08 (m, 1 H) 2.25 (br s, 1 H) 2.36 - 2.46 (m, 2 H) 2.62 - 2.81 (m, 4 H) 2.82 - 2.92 (m, 1 H) 3.29 - 3.31 (m, 1 H) 3.34 - 3.38 (m, 1 H) 3.76 - 3.89 (m, 3 H) 4.09 - 4.24 (m, 4 H) 4.66 (br d, J=4.13 Hz, 1 H) 4.72 (d, J=4.38 Hz, 1 H) 6.63 (d, J=6.38 Hz, 1 H) 7.00 - 7.06 (m, 1 H) 7.34 (d, J=6.00 Hz, 2 H) 7.44 - 7.54 (m, 2 H) 7.77 - 7.82 (m, 1 H) 8.19 (d, J=5.75 Hz, 1 H) 8.25 (s, 1 H) 8.91 (s, 1 H) 9.13 (d, J=8.25 Hz, 1 H) 9.89 (d, J=3.13 Hz, 1 H)

### 38-5. Preparation of Compound 184, (3R)-1-(2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)pyrrolidine-3-carboxylic acid

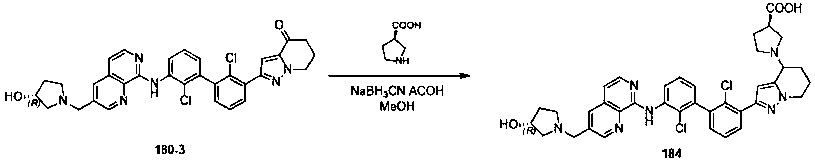

To a solution of 2-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-5H-pyrazolo[1,5-a]pyridin-4-one (220 mg, 366.97 µmol, 1 eq) and (3R)-pyrrolidine-3-carboxylic acid (422.49 mg, 3.67 mmol, 10 eq) in MeOH (10 mL), AcOH (44.07 mg, 733.93 µmol, 42.02 µL, 2 eq) and NaBH₃CN (115.30 mg, 1.83 mmol, 5 eq) were added. The resulting mixture was stirred at 60 °C for 4 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 22-52% B over 9 min) to afford Compound 184 (42 mg, 16.5% yield) as a white solid.

LCMS m/z 698.5 [M+1]⁺.

¹H NMR (400 MHz, DMSO-d6) δ ppm 1.51 - 1.64 (m, 1 H) 1.86 - 1.97 (m, 5 H) 1.98 - 2.06 (m, 1 H) 2.23 (br s, 1 H) 2.39 (dd, J=9.60, 3.24 Hz, 1 H) 2.60 - 2.77 (m, 5 H) 2.79 - 2.99 (m, 3 H) 3.75 - 3.88 (m, 5 H) 4.12 (br dd, J=13.57, 7.21 Hz, 2 H) 4.19 - 4.23 (m, 1 H) 6.60 - 6.64 (m, 1 H) 7.03 (t, J=6.42 Hz, 1 H) 7.29 - 7.36 (m, 2 H) 7.44 - 7.53 (m, 2 H) 7.80 (br d, J=7.70 Hz, 1 H) 8.18 (d, J=5.87 Hz, 1 H) 8.23 (s, 1 H) 8.90 (s, 1 H) 9.12 (d, J=8.31 Hz, 1 H) 9.88 (d, J=3.18 Hz, 1 H).

### 38-6. Preparation of Compound 185, 2-((2-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridin-4-yl)amino)acetic acid

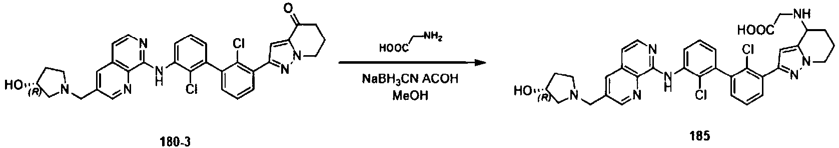

To a solution of 2-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-5H-pyrazolo[1,5-a]pyridin-4-one (220 mg, 366.97 µmol, 1 eq) and 2-aminoacetic acid (275.47 mg, 3.67 mmol, 10 eq) in MeOH (10 mL), AcOH (44.07 mg, 733.93 µmol, 42.02 µL, 2 eq) and NaBH₃CN (115.30 mg, 1.83 mmol, 5 eq) were added. The resulting mixture was stirred at 60 °C for 4 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 20-50% B over 9 min) to afford Compound 185 (45 mg, 18.6% yield) as a white solid.

LCMS m/z 658.4[M+1]⁺.

¹H NMR (400 MHz, DMSO-d6) δ ppm 1.53 - 1.63 (m, 1 H) 1.80 (br d, J=7.13 Hz, 1 H) 1.88 - 1.97 (m, 1 H) 1.99 - 2.08 (m, 2 H) 2.17 - 2.24 (m, 1 H) 2.34 - 2.42 (m, 1 H) 2.66 (q, J=7.88 Hz, 1 H) 2.74 (br dd, J=9.51, 6.25 Hz, 1 H) 3.31 (br d, J=1.75 Hz, 2 H) 3.77 - 3.87 (m, 5 H) 4.12 (br s, 3 H) 4.18 - 4.27 (m, 2 H) 6.77 (s, 1 H) 7.01 - 7.07 (m, 1 H) 7.34 (d, J=11.07 Hz, 1 H) 7.34 (s, 1 H) 7.45 - 7.53 (m, 2 H) 7.80 (br d, J=7.75 Hz, 1 H) 8.18 (d, J=5.75 Hz, 1 H) 8.24 (s, 1 H) 8.91 (s, 1 H) 9.12 (d, J=8.25 Hz, 1 H) 9.88 (s, 1 H).

### [Preparation Example 39]

### Preparation of Compounds 186 to 189

### 39-1. Preparation of Compound 186, 2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetic acid

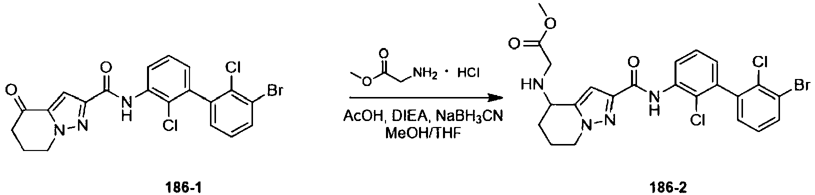

To a solution of N-[3-(3-bromo-2-chloro-phenyl)-2-chlorophenyl]-4-oxo-6,7-dihydro-5H-pyrazolo[1,5-a]pyridine-2-carboxamide (2.17 g, 4.54 mmol, 1 eq) and methyl 2-aminoacetate hydrochloride (2.28 g, 18.15 mmol, 4 eq) in MeOH (20 mL) / THF (20 mL), AcOH (272.46 mg, 4.54 mmol, 259.73 µL, 1 eq), DIPEA (2.35 g, 18.15 mmol, 3.16 mL, 4 eq), and NaBH₃CN (855.35 mg, 13.61 mmol, 3 eq) were added, and the resulting mixture was stirred at 50 °C for 12 hours. After completion of the reaction, the mixture was concentrated under reduced pressure, and the residue was purified by prep-HPLC (column: Phenomenex Luna C18, 250 * 70 mm * 10 µm; mobile phase: [water (NH₄HCO₃)-ACN]; gradient: 60-90% B over 20 min) to afford Compound 186-2 (1.23 g, 48.5% yield) as a white solid.

LCMS m/z 553.0 [M+3]⁺.

¹H NMR (400 MHz, CHLOROFORM-d) δ 9.42 (s, 1H), 8.63 (d, J = 8.5 Hz, 1H), 7.70 (dd, J = 2.6, 7.0 Hz, 1H), 7.42 - 7.35 (m, 1H), 7.26 - 7.19 (m, 2H), 6.99 (dd, J = 1.5, 7.5 Hz, 1H), 6.87 (s, 1H), 4.28 - 4.05 (m, 3H), 3.78 (s, 3H), 3.55 (s, 2H), 2.43 - 2.31 (m, 1H), 2.12 - 1.96 (m, 2H), 1.88 (br dd, J = 7.1, 14.1 Hz, 1H).

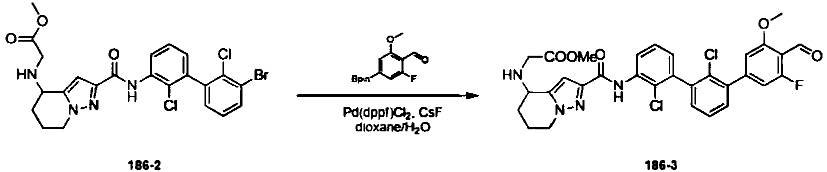

A solution of methyl 2-[[2-[[3-(3-bromo-2-chloro-phenyl)-2-chlorophenyl]carbamoyl]-4,5,6,7-tetrahydropyrazolo[1,5-a]pyridin-4-yl]amino]acetate (800 mg, 1.45 mmol, 1 eq), 2-fluoro-6-methoxy-4-(4,4,5,5-tetramethyl-1,3,2-dioxaborolan-2-yl)benzaldehyde (608.64 mg, 2.17 mmol, 1.5 eq), Pd(dppf)Cl₂ (106.00 mg, 144.86 µmol, 0.1 eq), and CsF (660.15 mg, 4.35 mmol, 160.43 µL, 3 eq) in dioxane (10 mL) / H₂O (2 mL) was stirred under a nitrogen atmosphere at 90 °C for 12 hours. After completion of the reaction, the mixture was concentrated under reduced pressure, and the residue was purified by flash silica gel chromatography (ISCO^{®}; 12 g SepaFlash^{®} Silica Flash Column; eluent: 0-10% methanol/dichloromethane @ 50 mL/min) to afford Compound 186-3 (1.29 g, 92.6% yield) as a red solid.

LCMS m/z 625.4 [M+1]⁺.

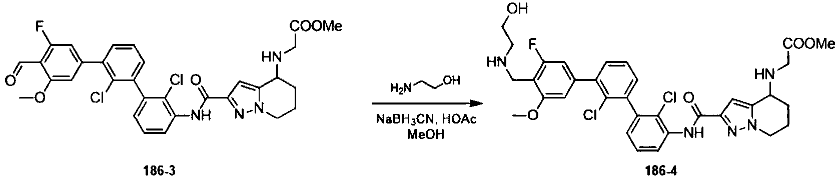

To a solution of methyl 2-[[2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (323 mg, 516.41 µmol, 1 eq) and 2-aminoethanol (126.17 mg, 2.07 mmol, 124.68 µL, 4 eq) in MeOH (3 mL), NaBH₃CN (97.36 mg, 1.55 mmol, 3 eq) and AcOH (31.01 mg, 516.41 µmol, 29.56 µL, 1 eq) were added. The resulting mixture was stirred at 60 °C for 12 hours. After completion of the reaction, the mixture was concentrated under reduced pressure, and the residue was purified by prep-HPLC (column: Waters XBridge, 150 * 25 mm * 5 µm; mobile phase: [water (NH₄HCO₃)-ACN]; gradient: 40-70% B over 9 min) to afford Compound 186-4 (92 mg, 26.5% yield) as a white solid.

LCMS m/z 670.3 [M+1]⁺.

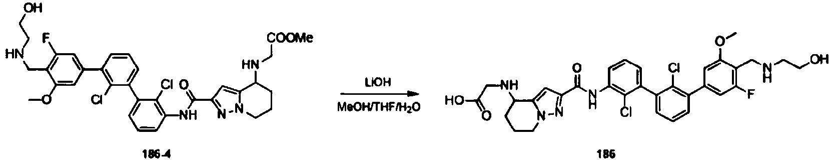

To a solution of methyl 2-[[2-[[2-chloro-3-[2-chloro-3-[3-fluoro-4-[(2-hydroxyethylamino)methyl]-5-methoxyphenyl]phenyl]phenyl]carbamoyl]-4,5,6,7-tetrahydropyrazolo[1,5-a]pyridin-4-yl]amino]acetate (80 mg, 119.30 µmol, 1 eq) in MeOH (5 mL)/THF (1 mL)/H₂O (3 mL), LiOH·H₂O (15.02 mg, 357.91 µmol, 3 eq) was added. The resulting mixture was stirred at room temperature for 3 hours. After completion of the reaction, the mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 10-40% B over 15 min) to afford Compound 186 (26 mg, 32.5% yield) as a white solid.

LCMS m/z 656.3 [M+1]⁺.

¹H NMR (400 MHz, DMSO-d6) δ 9.56 (s, 1H), 8.26 (d, J = 8.3 Hz, 1H), 7.56 - 7.46 (m, 3H), 7.42 - 7.38 (m, 1H), 7.19 (dd, J = 1.5, 7.6 Hz, 1H), 6.96 - 6.88 (m, 2H), 6.81 (s, 1H), 4.15 (br t, J = 6.1 Hz, 2H), 4.03 (br t, J = 5.9 Hz, 1H), 3.87 (s, 3H), 3.77 (s, 2H), 3.46 (br s, 2H), 3.30 (s, 2H), 2.61 - 2.57 (m, 2H), 2.25 - 2.15 (m, 1H), 2.04 - 1.97 (m, 1H), 1.95 - 1.86 (m, 1H), 1.78 - 1.69 (m, 1H).

### 39-2. Preparation of Compound 187, 2-((2-((2,2'-dichloro-3"-fluoro-4"-(((R)-3-hydroxypyrrolidin-1-yl)methyl)-5"-methoxy-[1,1':3',1"-terphenyl]-3-yl)carbamoyl)-4,5,6,7-tetrahydropyrazolo[1,5-a]pyridin-4-yl)amino)acetic acid

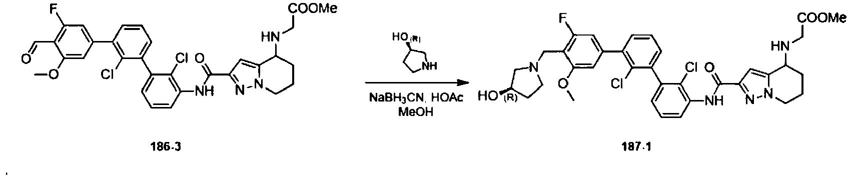

To a solution of methyl 2-[[2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (323 mg, 516.41 µmol, 1 eq) and (3R)-pyrrolidin-3-ol (179.96 mg, 2.07 mmol, 171.72 µL, 4 eq) in MeOH (3 mL), NaBH₃CN (97.35 mg, 1.55 mmol, 3 eq) and AcOH (31.01 mg, 516.41 µmol, 29.56 µL, 1 eq) were added. The resulting mixture was stirred at 60 °C for 12 hours. After completion of the reaction, the mixture was concentrated under reduced pressure, and the residue was purified by prep-TLC (SiO₂, dichloromethane/methanol = 10/1) to afford Compound 187-1 (130 mg, 33.6% yield) as a white solid.

LCMS m/z 696.3 [M+1]⁺.

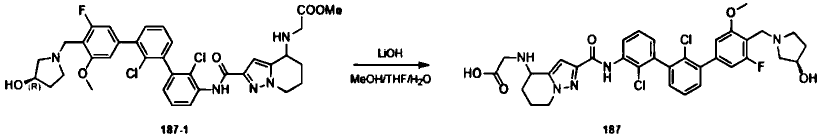

To a mixture of methyl 2-[[2-[[2-chloro-3-[2-chloro-3-[3-fluoro-4-[[(3R)-3-hydroxypyrrolidin-1-yl]methyl]-5-methoxyphenyl]phenyl]phenyl]carbamoyl]-4,5,6,7-tetrahydropyrazolo[1,5-a]pyridin-4-yl]amino]acetate (112 mg, 160.78 µmol, 1 eq) in MeOH (4 mL) / H₂O (2 mL), LiOH·H₂O (20.24 mg, 482.35 µmol, 3 eq) was added. The resulting mixture was stirred at room temperature for 12 hours. After completion of the reaction, the mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 20-50% B over 15 min) to afford Compound 187 (52 mg, 46.9% yield) as a white solid.

LCMS m/z 682.3 [M+1]⁺.

¹H NMR (400 MHz, DMSO-d6) δ 9.56 (s, 1H), 8.26 (d, J = 8.3 Hz, 1H), 7.57 - 7.46 (m, 3H), 7.40 (dd, J = 3.1, 6.1 Hz, 1H), 7.19 (d, J = 7.1 Hz, 1H), 6.95 - 6.88 (m, 2H), 6.81 (s, 1H), 4.80 - 4.49 (m, 1H), 4.18 - 4.11 (m, 3H), 4.03 (br s, 1H), 3.85 (s, 3H), 3.66 - 3.63 (m, 2H), 2.72 (dd, J = 6.4, 9.5 Hz, 1H), 2.63 - 2.56 (m, 1H), 2.46 - 2.41 (m, 1H), 2.33 (br dd, J = 3.6, 9.2 Hz, 1H), 2.18 (br d, J = 4.3 Hz, 1H), 2.02 - 1.88 (m, 3H), 1.75 (br s, 1H), 1.47 (br dd, J = 5.0, 8.5 Hz, 1H).

### 39-3. Preparation of Compound 188, 2-((2-((2,2'-dichloro-3"-fluoro-5"-methoxy-4"-(((((S)-5-oxopyrrolidin-2-yl)methyl)amino)methyl)-[1,1':3',1"-terphenyl]-3-yl)carbamoyl)-4,5,6,7-tetrahydropyrazolo[1,5-a]pyridin-4-yl)amino)acetic acid

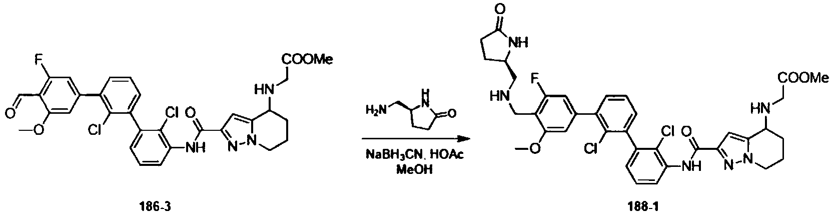

To a solution of methyl 2-[[2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (323 mg, 516.41 µmol, 1 eq) and (5S)-5-(aminomethyl)pyrrolidin-2-one (311.10 mg, 2.07 mmol, 4 eq, HCl) in MeOH (3 mL), NaBH₃CN (97.35 mg, 1.55 mmol, 3 eq), DIPEA (266.97 mg, 2.07 mmol, 359.80 µL, 4 eq), and AcOH (31.01 mg, 516.41 µmol, 29.56 µL, 1 eq) were added. The resulting mixture was stirred at 60 °C for 12 hours. After completion of the reaction, the mixture was concentrated under reduced pressure, and the residue was purified by prep-TLC (SiO₂, dichloromethane/methanol = 10/1) to afford Compound 188-1 (131 mg, 34.3% yield) as a white solid.

LCMS m/z 723.5 [M+1]⁺.

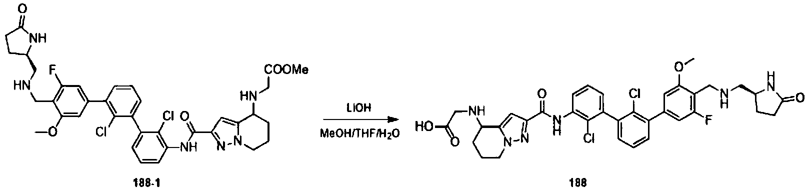

To a mixture of Methyl 2-[[2-[[2-chloro-3-[2-chloro-3-[3-fluoro-5-methoxy-4-[[[(2S)-5-oxopy To a solution of rrolidin-2-yl]methylamino]methyl]phenyl]phenyl]phenyl]carbamoyl]-4,5,6,7-tetrahydropyrazolo[1,5-a]pyridin-4-yl]amino]acetate (125 mg, 172.74 µmol, 1 eq) in MeOH (4 mL) / H₂O (2 mL), LiOH·H₂O (21.74 mg, 518.23 µmol, 3 eq) was added. The resulting mixture was stirred at room temperature for 12 hours. After completion of the reaction, the mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 20-50% B over 15 min) to afford Compound 188 (35 mg, 27.9% yield) as a white solid.

LCMS m/z 709.4 [M+1]⁺.

¹H NMR (400 MHz, DMSO-d6) δ = 9.56 (s, 1H), 8.26 (d, J = 8.1 Hz, 1H), 7.65 (s, 1H), 7.56 - 7.46 (m, 3H), 7.43 - 7.37 (m, 1H), 7.19 (d, J = 7.3 Hz, 1H), 6.96 - 6.88 (m, 2H), 6.81 (s, 1H), 4.15 (br t, J = 5.9 Hz, 2H), 4.03 (br t, J = 5.8 Hz, 1H), 3.87 (s, 3H), 3.75 (br s, 2H), 3.58 (br d, J = 6.1 Hz, 2H), 3.17 (s, 2H), 2.20 (br d, J = 6.5 Hz, 1H), 2.17 - 1.98 (m, 5H), 1.91 (br s, 1H), 1.77 - 1.57 (m, 2H).

### 39-4. Preparation of Compound 189, 2-((2-((4"-(((2-acetamidoethyl)amino)methyl)-2,2'-dichloro-3"-fluoro-5"-methoxy-[1,1':3',1"-terphenyl]-3-yl)carbamoyl)-4,5,6,7-tetrahydropyrazolo[1,5-a]pyridin-4-yl)amino)acetic acid

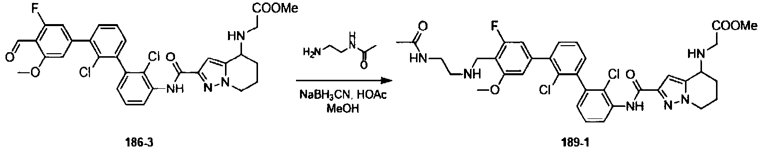

To a solution of methyl 2-[[2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (323 mg, 516.41 µmol, 1 eq) and N-(2-aminoethyl)acetamide (210.97 mg, 2.07 mmol, 197.91 µL, 4 eq) in MeOH (3 mL), NaBH₃CN (97.36 mg, 1.55 mmol, 3 eq) and AcOH (31.01 mg, 516.41 µmol, 29.56 µL, 1 eq) were added. The resulting mixture was stirred at 60 °C for 12 hours. After completion of the reaction, the mixture was concentrated under reduced pressure, and the residue was purified by prep-HPLC (column: Waters XBridge, 150 * 25 mm * 5 µm; mobile phase: [water (NH₄HCO₃)-ACN]; gradient: 34-64% B over 9 min) to afford Compound 189-1 (163 mg, 44.3% yield) as a white solid.

LCMS m/z 711.5 [M+1]⁺.

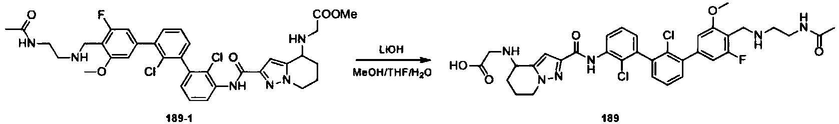

To a mixture of methyl 2-[[2-[[3-[3-[4-[(2-acetamidoethylamino)methyl]-3-fluoro-5-methoxyphenyl]-2-chlorophenyl]-2-chlorophenyl]carbamoyl]-4,5,6,7-tetrahydropyrazolo[1,5-a]pyridin-4-yl]amino]acetate (150 mg, 210.79 µmol, 1 eq) in MeOH (4 mL)/THF (1 mL)/H₂O (2 mL), LiOH·H₂O (26.54 mg, 632.37 µmol, 3 eq) was added. The resulting mixture was stirred at room temperature for 2 hours. After completion of the reaction, the mixture was concentrated under reduced pressure, and the residue was purified by prep-HPLC (column: Waters XBridge Prep OBD C18, 150 * 40 mm * 10 µm; mobile phase: [water (NH₄HCO₃)-ACN]; gradient: 10-40% B over 15 min) to afford Compound 189 (100 mg, 66.6% yield) as a white solid.

LCMS m/z 697.3 [M+1]⁺.

¹H NMR (400 MHz, DMSO-d6) δ 9.56 (s, 1H), 8.26 (d, J = 8.1 Hz, 1H), 7.80 (br t, J = 5.3 Hz, 1H), 7.56 - 7.51 (m, 2H), 7.51 - 7.46 (m, 1H), 7.43 - 7.36 (m, 1H), 7.19 (d, J = 7.9 Hz, 1H), 6.95 - 6.88 (m, 2H), 6.81 (s, 1H), 4.15 (br t, J = 6.1 Hz, 2H), 4.03 (br t, J = 5.8 Hz, 1H), 3.87 (s, 3H), 3.75 - 3.72 (m, 2H), 3.31 (s, 2H), 3.13 (q, J = 6.0 Hz, 2H), 2.58 - 2.53 (m, 2H), 2.25 - 2.15 (m, 1H), 2.04 - 1.96 (m, 1H), 1.96 - 1.85 (m, 1H), 1.78 (s, 3H), 1.76 - 1.70 (m, 1H).

### [Preparation Example 40]

### Preparation of Compounds 190 to 197

### 40-1. Preparation of Compound 190, 2-((2-((2,2'-dichloro-3'-(3-fluoro-4-(((2-hydroxyethyl)amino)methyl)-5-methoxybenzamido)-[1,1'-biphenyl]-3-yl)carbamoyl)-4,5,6,7-tetrahydropyrazolo[1,5-a]pyridin-4-yl)amino)acetic acid

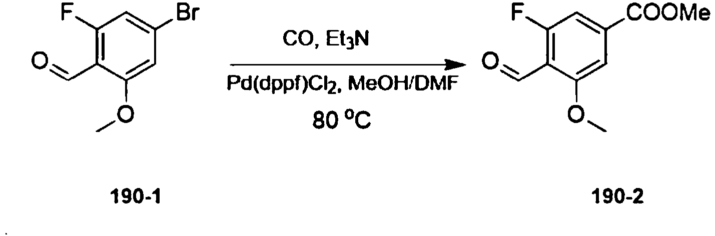

To a solution of 4-bromo-2-fluoro-6-methoxybenzaldehyde (7 g, 30.04 mmol, 1 eq) in DMF (35 mL) / MeOH (35 mL), Pd(dppf)Cl₂ (1.10 g, 1.50 mmol, 0.05 eq) and TEA (9.12 g, 90.12 mmol, 12.54 mL, 3 eq) were added. The resulting mixture was stirred under CO atmosphere (50 psi) at 80 °C for 4 hours. After completion of the reaction, the mixture was concentrated under reduced pressure, and the residue was purified by column chromatography (SiO₂, Petroleum ether/Ethyl acetate = 3/1 to 2/1) to afford Compound 190-2 (6.2 g, 97.2% yield) as a yellow solid.

LCMS m/z 213.2 [M+1]⁺.

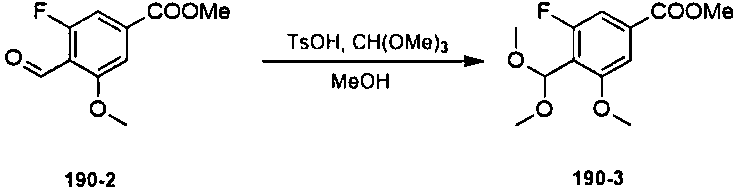

To a solution of methyl 3-fluoro-4-formyl-5-methoxybenzoate (3 g, 14.14 mmol, 1 eq) in MeOH (30 mL), trimethoxymethane (4.50 g, 42.42 mmol, 4.65 mL, 3 eq) and 4-methylbenzenesulfonic acid hydrate (268.95 mg, 1.41 mmol, 0.1 eq) were added. The resulting mixture was stirred at 50 °C for 12 hours. After completion of the reaction, the mixture was concentrated under reduced pressure, and the residue was diluted with ethyl acetate (300 mL) and washed with water (500 mL). H₂O layer was extracted with ethyl acetate (300 mL * 3). The combined organic layers were washed with brine (60 mL), dried over Na₂SO₄, and concentrated under reduced pressure to afford Compound 190-3 (3.7 g, 91.2% yield) as a white solid, which was used in the subsequent reaction without further purification.

¹H NMR (400 MHz, CHLOROFORM-d) δ ppm 3.32 - 3.39 (m, 6 H) 3.79 - 3.84 (m, 6 H) 5.61 - 5.64 (m, 1 H) 7.27 (s, 2 H).

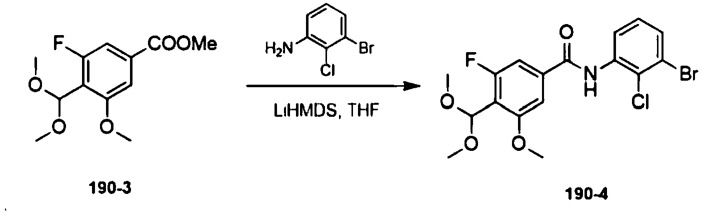

To a solution of methyl 4-(dimethoxymethyl)-3-fluoro-5-methoxybenzoate (3.2 g, 12.39 mmol, 1 eq) and 3-bromo-2-chloroaniline (3.07 g, 14.87 mmol, 1.2 eq) in THF (35 mL), LiHMDS (1 M, 37.17 mL, 3 eq) was added under a nitrogen atmosphere, and the resulting mixture was stirred at room temperature for 4 hours. After completion of the reaction, the mixture was poured into H₂O (300 mL) and extracted with EA (200 mL * 3). The combined organic layers were washed with brine (50 mL), dried over Na₂SO₄, and concentrated under reduced pressure to afford Compound 190-4 (7.4 g, 96.6% yield) as a brown solid, which was used in the subsequent reaction without further purification.

¹H NMR (400 MHz, DMSO-d6) δ ppm 3.33 (s, 9 H) 5.60 - 5.70 (m, 1 H) 7.31 - 7.41 (m, 2 H) 7.43 - 7.48 (m, 1 H) 7.53 - 7.60 (m, 1 H) 7.67 - 7.77 (m, 1 H).

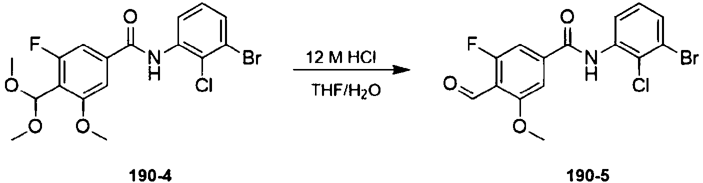

To a solution of N-(3-bromo-2-chloro-phenyl)-4-(dimethoxymethyl)-3-fluoro-5-methoxybenzamide (7.4 g, 17.10 mmol, 1 eq) in THF (70 mL) / H₂O (20 mL), concentrated HCl (12 M, 2.85 mL, 2 eq) was slowly added. The resulting mixture was stirred at room temperature for 12 hours. After completion of the reaction, the mixture was poured into H₂O (500 mL) and extracted with EA (300 mL * 3). The combined organic layers were washed with brine (50 mL), dried over Na₂SO₄, and concentrated under reduced pressure to afford Compound 190-5 (6.6 g, 84.8% yield) as a yellow solid.

LCMS m/z 386.0 [M+1]⁺.

¹H NMR (400 MHz, DMSO-d6) δ ppm 3.93 - 4.11 (m, 3 H) 7.33 - 7.40 (m, 1 H) 7.42 - 7.47 (m, 1 H) 7.55 (br s, 2 H) 7.71 - 7.78 (m, 1 H) 10.27 - 10.43 (m, 1 H) 10.46 - 10.60 (m, 1 H).

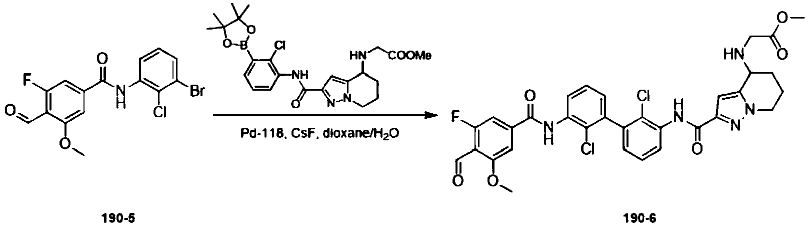

A mixture of N-(3-bromo-2-chloro-phenyl)-3-fluoro-4-formyl-5-methoxybenzamide (700 mg, 1.81 mmol, 1 eq), methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (973.50 mg, 1.99 mmol, 1.1 eq), CsF (550.09 mg, 3.62 mmol, 133.68 µL, 2 eq), and ditert-butyl(cyclopentyl)phosphane;dichloropalladium iron (118.01 mg, 181.07 µmol, 0.1 eq) in dioxane (20 mL) / H₂O (4 mL) was stirred under a nitrogen atmosphere at 70 °C for 12 hours. After completion of the reaction, the mixture was concentrated under reduced pressure, and the residue was purified by column chromatography (SiO₂, ethyl acetate/MeOH = 10/1) to afford Compound 190-6 (960 mg, 55.5% yield) as a white solid.

LCMS m/z 668.2 [M+1]⁺.

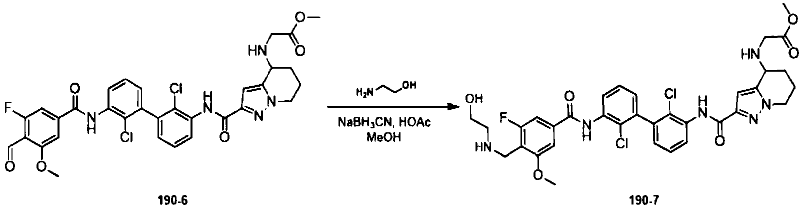

To a solution of methyl 2-[[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]amino]acetate (80 mg, 119.67 µmol, 1 eq) and 2-aminoethanol (73.10 mg, 1.20 mmol, 72.23 µL, 10 eq) in MeOH (10 mL), AcOH (14.37 mg, 239.34 µmol, 13.70 µL, 2 eq) and NaBH₃CN (37.60 mg, 598.36 µmol, 5 eq) were added. The resulting mixture was stirred at 60 °C for 12 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 32-62% B over 9 min) to afford Compound 190-7 (34 mg, 37.8% yield) as a white solid.

LCMS m/z 713.3 [M+1]⁺.

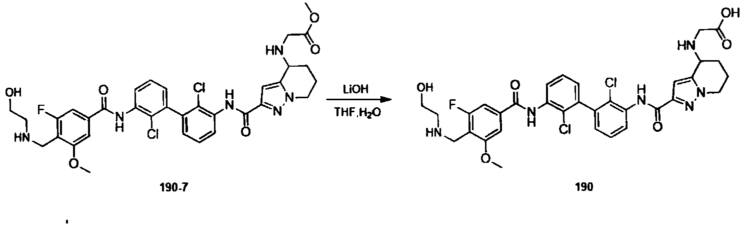

To a solution of methyl 2-[[2-[[2-chloro-3-[2-chloro-3-[[3-fluoro-4-[(2-hydroxyethylamino)methyl]-5-methoxybenzoyl]amino]phenyl]phenyl]carbamoyl]-4,5,6,7-tetrahydropyrazolo[1,5-a]pyridin-4-yl]amino]acetate (34 mg, 47.65 µmol, 1 eq) in THF (15 mL) / H₂O (5 mL), LiOH (6.00 mg, 142.94 µmol, 3 eq) was added. The resulting mixture was stirred at room temperature for 12 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 15-45% B over 9 min) to afford Compound 190 (16 mg, 47.5% yield) as a white solid.

LCMS m/z 699.3 [M+1]⁺.

¹H NMR (400 MHz, DMSO-d6) δ ppm 1.75 (br s, 1 H) 1.91 (br s, 1 H) 2.00 (br s, 1 H) 2.19 (br s, 1 H) 2.52 - 2.55 (m, 2 H) 3.42 - 3.45 (m, 5 H) 3.77 (s, 2 H) 3.92 (s, 3 H) 4.03 (br t, J=5.82 Hz, 1 H) 4.15 (br t, J=6.00 Hz, 2 H) 6.81 (s, 1 H) 7.15 (d, J=6.38 Hz, 1 H) 7.30 (br d, J=7.38 Hz, 1 H) 7.42 - 7.53 (m, 4 H) 7.65 (d, J=7.88 Hz, 1 H) 8.25 (br d, J=8.26 Hz, 1 H) 9.56 (s, 1 H) 10.23 (s, 1 H).

### 40-2. Preparation of Compound 191, 2-((4-((3'-(4-((carboxymethyl)amino)-4,5,6,7-tetrahydropyrazolo[1,5-a]pyridine-2-carboxamido)-2,2'-dichloro-[1,1'-biphenyl]-3-yl)carbamoyl)-2-fluoro-6-methoxybenzyl)amino)acetic acid

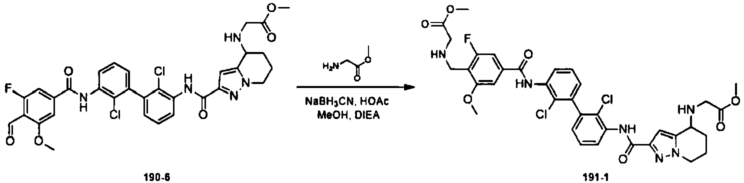

To a solution of methyl 2-[[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]amino]acetate (110 mg, 164.55 µmol, 1 eq) and methyl 2-aminoacetate (206.60 mg, 1.65 mmol, 10 eq, HCl) in MeOH (20 mL), AcOH (19.76 mg, 329.10 µmol, 18.84 µL, 2 eq), DIPEA (212.67 mg, 1.65 mmol, 286.61 µL, 10 eq), and NaBH₃CN (51.70 mg, 822.74 µmol, 5 eq) were added, and the resulting mixture was stirred at 60 °C for 12 hours. After completion of the reaction, the mixture was concentrated under reduced pressure, and the residue was purified by column chromatography (SiO₂, ethyl acetate/MeOH = 5/1) to afford Compound 191-1 (30 mg, 24.4% yield) as a white solid.

LCMS m/z 741.2 [M+1]⁺.

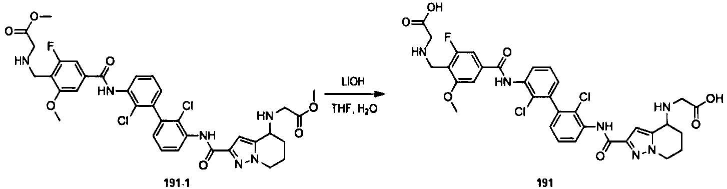

To a solution of methyl 2-[[4-[[2-chloro-3-[2-chloro-3-[[4-[(2-methoxy-2-oxoethyl)amino]-4,5,6,7-tetrahydropyrazolo[1,5-a]pyridine-2-carbonyl]amino]phenyl]phenyl]carbamoyl]-2-fluoro-6-methoxyphenyl]methylamino]acetate (30 mg, 40.45 µmol, 1 eq) in THF (20 mL) / H₂O (5 mL), LiOH (5.09 mg, 121.36 µmol, 3 eq) was added. The resulting mixture was stirred at room temperature for 4 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 10-40% B over 9 min) to afford Compound 191 (28 mg, 97.0% yield) as a white solid.

LCMS m/z 713.2 [M+1]⁺.

¹H NMR (400 MHz, DMSO-d6) δ ppm 1.75 (br d, J=2.13 Hz, 1 H) 1.91 (br d, J=6.88 Hz, 1 H) 1.96 - 2.05 (m, 1 H) 2.20 (br d, J=7.63 Hz, 1 H) 2.33 (br s, 1 H) 2.84 - 2.92 (m, 1 H) 3.14 (s, 2 H) 3.29 - 3.29 (m, 2 H) 3.93 (s, 3 H) 4.04 (br t, J=5.88 Hz, 1 H) 4.15 (br t, J=6.07 Hz, 2 H) 6.81 (s, 1 H) 7.15 (dd, J=7.63, 1.38 Hz, 1 H) 7.30 (d, J=7.13 Hz, 1 H) 7.43 - 7.54 (m, 4 H) 7.65 (dd, J=8.00, 1.50 Hz, 1 H) 8.25 (d, J=8.25 Hz, 1 H) 9.56 (s, 1 H) 10.28 (s, 1 H).

### 40-3. Preparation of Compound 192, 2-((2-((2,2'-dichloro-3'-(3-fluoro-4-(((R)-3-hydroxypyrrolidin-1-yl)methyl)-5-methoxybenzamido)-[1,1'-biphenyl]-3-yl)carbamoyl)-4,5,6,7-tetrahydropyrazolo[1,5-a]pyridin-4-yl)amino)acetic acid

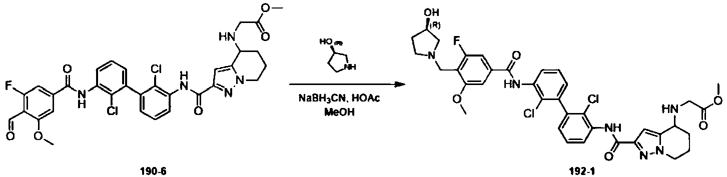

To a solution of methyl 2-[[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]amino]acetate (320 mg, 478.69 µmol, 1 eq) and (3R)-pyrrolidin-3-ol (417.03 mg, 4.79 mmol, 397.93 µL, 10 eq) in MeOH (20 mL), AcOH (57.49 mg, 957.37 µmol, 54.81 µL, 2 eq) and NaBH₃CN (150.41 mg, 2.39 mmol, 5 eq) were then added, and the resulting mixture was stirred at 60 °C for 12 hours. After completion of the reaction, the mixture was concentrated under reduced pressure, and the residue was purified by column chromatography on silica gel (ethyl acetate/MeOH = 5/1) to afford Compound 192-1 (173 mg, 48.8% yield) as a white solid.

LCMS m/z 739.3 [M+1]⁺.

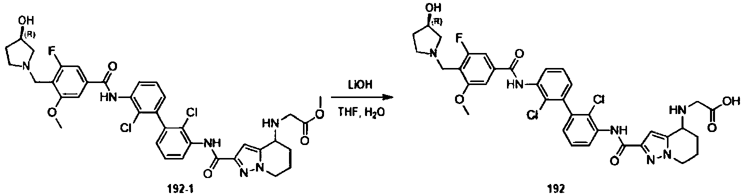

To a solution of methyl 2-[[2-[[2-chloro-3-[2-chloro-3-[[3-fluoro-4-[[(3R)-3-hydroxypyrrolidin-1-yl]methyl]-5-methoxybenzoyl]amino]phenyl]phenyl]carbamoyl]-4,5,6,7-tetrahydropyrazolo[1,5-a]pyridin-4-yl]amino]acetate (173 mg, 233.90 µmol, 1 eq) in THF (30 mL)/H₂O (10 mL), LiOH (29.45 mg, 701.71 µmol, 3 eq) was added. The resulting mixture was stirred at room temperature for 4 hours. After completion of the reaction, the mixture was concentrated under reduced pressure, and the residue was purified by reversed-phase HPLC under basic conditions (0.1% NH₃·H₂O; column: Waters XBridge, 150 * 25 mm * 5 µm; mobile phase: [water (NH₄HCO₃)-ACN]; gradient: 15-45% B over 9 min) to afford Compound 192 (63 mg, 37.1% yield) as a white solid.

LCMS m/z 725.3 [M+1]⁺.

¹H NMR (400 MHz, DMSO-d6) δ ppm 1.40 - 1.52 (m, 1 H) 1.68 - 1.79 (m, 1 H) 1.88 - 2.04 (m, 3 H) 2.20 (br dd, J=13.70, 5.44 Hz, 1 H) 2.29 (dd, J=9.57, 3.81 Hz, 1 H) 2.39 - 2.46 (m, 1 H) 2.53 - 2.59 (m, 1 H) 2.70 (dd, J=9.51, 6.25 Hz, 1 H) 3.30 - 3.33 (m, 3 H) 3.66 (br s, 2 H) 3.90 (s, 3 H) 4.04 (br t, J=5.88 Hz, 1 H) 4.10 - 4.17 (m, 3 H) 6.82 (s, 1 H) 7.15 (dd, J=7.63, 1.38 Hz, 1 H) 7.30 (d, J=7.38 Hz, 1 H) 7.39 - 7.55 (m, 4 H) 7.65 (dd, J=8.00, 1.38 Hz, 1 H) 8.25 (d, J=8.13 Hz, 1 H) 9.56 (s, 1 H) 10.24 (s, 1 H)

### 40-4. Preparation of Compound 193, (3R)-1-(4-((3'-(4-((carboxymethyl)amino)-4,5,6,7-tetrahydropyrazolo[1,5-a]pyridine-2-carboxamido)-2,2'-dichloro-[1,1'-biphenyl]-3-yl)carbamoyl)-2-fluoro-6-methoxybenzyl)pyrrolidine-3-carboxylic acid

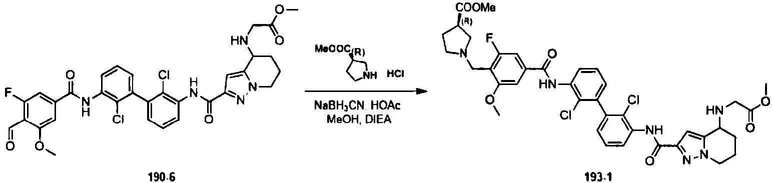

To a solution of methyl 2-[[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]amino]acetate (110 mg, 164.55 µmol, 1 eq) and methyl (3R)-pyrrolidine-3-carboxylate (272.52 mg, 1.65 mmol, 10 eq, HCl) in MeOH (20 mL), AcOH (19.76 mg, 329.10 µmol, 18.84 µL, 2 eq), DIPEA (212.67 mg, 1.65 mmol, 286.61 µL, 10 eq) and NaBH₃CN (51.70 mg, 822.74 µmol, 5 eq) were added, and the resulting mixture was stirred at 60 °C for 12 hours. After completion of the reaction, the mixture was concentrated under reduced pressure, and the residue was purified by column chromatography on silica gel (ethyl acetate/MeOH = 5/1) to afford Compound 193-1 (80 mg, 60.9% yield) as a white solid.

LCMS m/z 781.3 [M+1]⁺.

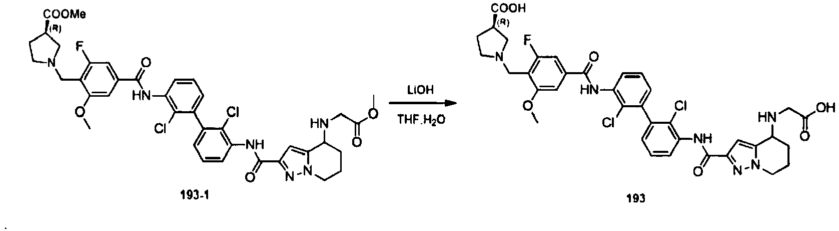

To a solution of methyl (3R)-1-[[4-[[2-chloro-3-[2-chloro-3-[[4-[(2-methoxy-2-oxoethyl)amino]-4,5,6,7-tetrahydropyrazolo[1,5-a]pyridine-2-carbonyl]amino]phenyl]phenyl]carbamoyl]-2-fluoro-6-methoxyphenyl]methyl]pyrrolidine-3-carboxylate (80 mg, 102.35 µmol, 1 eq) in THF (20 mL) / H₂O (5 mL), LiOH (12.88 mg, 307.04 µmol, 3 eq) was added, and the resulting mixture was stirred at room temperature for 4 hours. After completion of the reaction, the mixture was concentrated under reduced pressure, and the residue was purified by reversed-phase HPLC (0.1% NH₃·H₂O condition; column: Waters XBridge, 150 x 25 mm, 5 um; mobile phase: [water (NH₄HCO₃)-ACN]; gradient: 10-40% B over 9 min) to afford Compound 193 (54 mg, 70.0% yield) as a white solid.

LCMS m/z 753.2 [M+1]⁺.

¹H NMR (400 MHz, DMSO-d6) δ ppm 1.68 - 1.79 (m, 1 H) 1.85 - 1.94 (m, 3 H) 1.97 - 2.05 (m, 1 H) 2.15 - 2.24 (m, 1 H) 2.59 (br dd, J=8.88, 6.63 Hz, 1 H) 2.72 (br t, J=8.76 Hz, 1 H) 2.81 - 2.91 (m, 2 H) 3.29 (s, 3 H) 3.68 (br s, 2 H) 3.90 (s, 2 H) 4.04 (br t, J=6.00 Hz, 1 H) 4.15 (br t, J=6.07 Hz, 2 H) 6.82 (s, 1 H) 7.15 (dd, J=7.63, 1.38 Hz, 1 H) 7.29 (d, J=7.38 Hz, 1 H) 7.42 - 7.53 (m, 4 H) 7.65 (dd, J=7.94, 1.44 Hz, 1 H) 8.25 (d, J=8.26 Hz, 1 H) 9.56 (s, 1 H) 10.25 (s, 1 H)

### 40-5. Preparation of Compound 194, (3R)-1-(2-((2,2'-dichloro-3'-(3-fluoro-4-(((2-hydroxyethyl)amino)methyl)-5-methoxybenzamido)-[1,1'-biphenyl]-3-yl)carbamoyl)-4,5,6,7-tetrahydropyrazolo[1,5-a]pyridin-4-yl)pyrrolidine-3-carboxylic acid

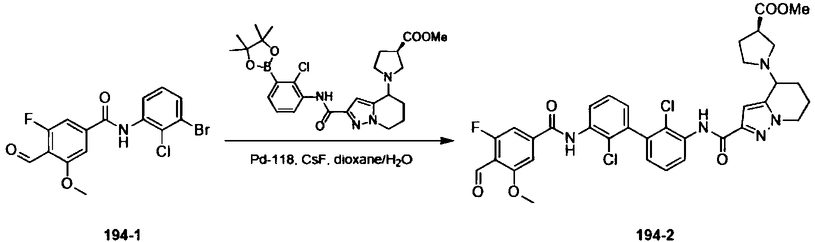

A mixture of N-(3-bromo-2-chloro-phenyl)-3-fluoro-4-formyl-5-methoxybenzamide (723.73 mg, 1.87 mmol, 1.1 eq), methyl (3R)-1-[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (900 mg, 1.70 mmol, 1 eq), CsF (517.04 mg, 3.40 mmol, 125.65 µL, 2 eq), and ditert-butyl(cyclopentyl)phosphane;dichloropalladium iron (55.46 mg, 85.09 µmol, 0.05 eq) in dioxane (10 mL) / H₂O (2 mL) was stirred at 80 °C for 4 hours under a nitrogen atmosphere. After completion, the mixture was concentrated under reduced pressure. The residue was purified by column chromatography (SiO₂, Petroleum ether/Ethyl acetate = 0/1), followed by additional purification using reversed-phase HPLC (0.1% NH₃·H₂O condition; column: Waters XBridge Prep OBD C18, 150 * 40 mm * 10 um; mobile phase: [water (NH₄HCO₃)-ACN]; gradient: 48-78% B over 15 min) to afford Compound 194-2 (700 mg, 46.4% yield) as a white solid.

LCMS m/z 708.2 [M+1]⁺.

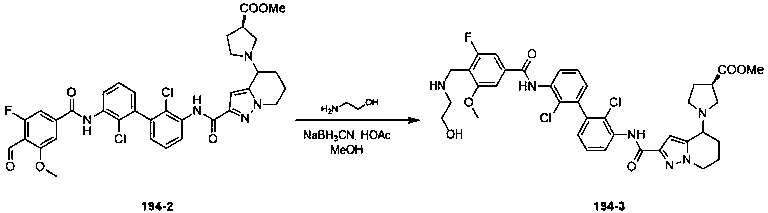

To a solution of methyl (3R)-1-[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]pyrrolidine-3-carboxylate (200 mg, 282.26 µmol, 1 eq) and 2-aminoethanol (172.41 mg, 2.82 mmol, 170.37 µL, 10 eq) in MeOH (20 mL), AcOH (33.90 mg, 564.52 µmol, 32.32 µL, 2 eq) and NaBH₃CN (88.69 mg, 1.41 mmol, 5 eq) were added, and the reaction mixture was stirred at 60 °C for 12 hours. After completion of the reaction, the mixture was concentrated under reduced pressure. The residue was purified by reversed-phase HPLC (0.1% TFA condition; column: Phenomenex Luna C18, 150 * 40 mm * 15 um; mobile phase: [water (TFA)-ACN]; gradient: 13-43% B over 15 min) to afford Compound 194-3 (73 mg, 33.6% yield) as a yellow solid.

LCMS m/z 753.3 [M+1]⁺.

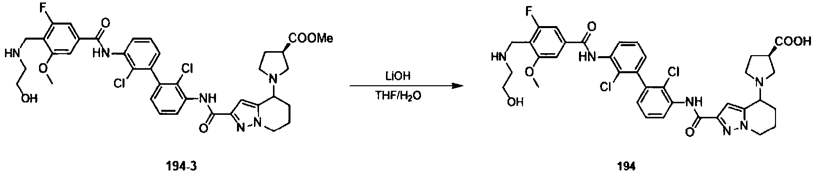

To a solution of methyl (3R)-1-[2-[[2-chloro-3-[2-chloro-3-[[3-fluoro-4-[(2-hydroxyethylamino)methyl]-5-methoxybenzoyl]amino]phenyl]phenyl]carbamoyl]-4,5,6,7-tetrahydropyrazolo[1,5-a]pyridin-4-yl]pyrrolidine-3-carboxylate (70 mg, 92.88 µmol, 1 eq) in THF (20 mL) / H₂O (5 mL), LiOH (11.69 mg, 278.65 µmol, 3 eq) was added, and the reaction mixture was stirred at room temperature for 4 hours. After completion of the reaction, the mixture was concentrated under reduced pressure. The residue was purified by reversed-phase HPLC (0.1% NH₃·H₂O condition; column: Waters Xbridge, 150 * 25 mm * 5 um; mobile phase: [water (NH₄HCO₃)-ACN]; gradient: 15-45% B over 9 min) to afford Compound 194 (60 mg, 86.4% yield) as a white solid.

LCMS m/z 739.3 [M+1]⁺.

¹H NMR (400 MHz, DMSO-d6) δ ppm 1.93 (br d, J=5.13 Hz, 4 H) 2.60 (br d, J=8.13 Hz, 1 H) 2.68 - 2.74 (m, 2 H) 2.91 (br s, 4 H) 3.61 (br s, 4 H) 3.82 (br s, 2 H) 3.96 (s, 3 H) 4.09 (br s, 2 H) 4.17 (br d, J=11.51 Hz, 2 H) 6.72 (d, J=4.50 Hz, 1 H) 7.15 (d, J=6.25 Hz, 1 H) 7.31 (d, J=7.50 Hz, 1 H) 7.46 - 7.58 (m, 4 H) 7.61 - 7.69 (m, 1 H) 8.24 (br d, J=6.88 Hz, 1 H) 9.56 (s, 1 H) 10.32 (s, 1 H).

### 40-6. Preparation of Compound 195, (3R)-1-(2-((3'-(4-(((carboxymethyl)amino)methyl)-3-fluoro-5-methoxybenzamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid

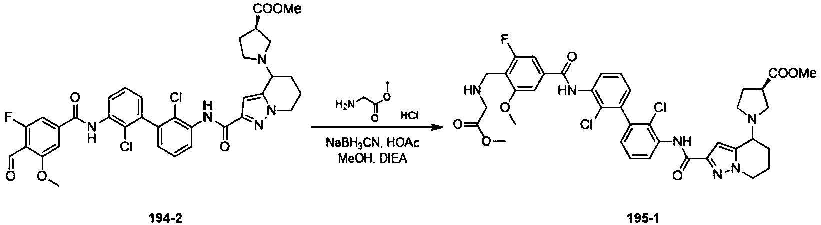

To a solution of methyl (3R)-1-[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]pyrrolidine-3-carboxylate (200 mg, 282.26 µmol, 1 eq) and methyl 2-aminoacetate hydrochloride (354.39 mg, 2.82 mmol, 10 eq) in MeOH (20 mL), AcOH (33.90 mg, 564.52 µmol, 32.32 µL, 2 eq), DIPEA (364.80 mg, 2.82 mmol, 491.65 µL, 10 eq), and NaBH₃CN (88.69 mg, 1.41 mmol, 5 eq) were added, and the reaction mixture was stirred at 60 °C for 12 hours. After completion of the reaction, the mixture was concentrated under reduced pressure. The residue was purified by reversed-phase HPLC (0.1% TFA condition; column: Phenomenex Luna C18, 150 * 40 mm * 15 um; mobile phase: [water (TFA)-ACN]; gradient: 15-45% B over 15 min) to afford Compound 195-1 (120 mg, 43.5% yield) as a white solid.

LCMS m/z 781.3 [M+1]⁺.

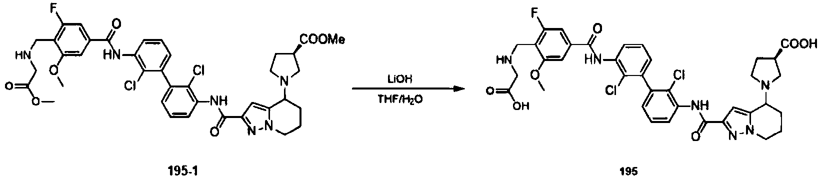

To a solution of methyl (3R)-1-[2-[[2-chloro-3-[2-chloro-3-[[3-fluoro-5-methoxy-4-[[(2-methoxy-2-oxo-ethyl)amino]methyl]benzoyl]amino]phenyl]phenyl]carbamoyl]-4,5,6,7-tetrahydropyrazolo[1,5-a]pyridin-4-yl]pyrrolidine-3-carboxylate (120 mg, 153.52 µmol, 1 eq) in THF (25 mL) / H₂O (5 mL), LiOH (19.33 mg, 460.56 µmol, 3 eq) was added, and the reaction mixture was stirred at room temperature for 12 hours. After completion of the reaction, the mixture was concentrated under reduced pressure. The residue was purified by reversed-phase HPLC (0.1% NH₃·H₂O condition; column: Waters Xbridge, 150 * 25 mm * 5 um; mobile phase: [water (NH₄HCO₃)-ACN]; gradient: 8-38% B over 9 min) to afford Compound 195 (32 mg, 27.3% yield) as a white solid.

LCMS m/z 753.2 [M+1]⁺.

¹H NMR (400 MHz, DMSO-d6) δ ppm 1.88 - 1.97 (m, 4 H) 2.19 - 2.27 (m, 1 H) 2.56 - 2.63 (m, 2 H) 2.67 - 2.74 (m, 2 H) 2.77 (br d, J=8.63 Hz, 1 H) 2.81 - 2.86 (m, 1 H) 2.91 (br d, J=5.00 Hz, 2 H) 3.13 (s, 2 H) 3.82 (br s, 2 H) 3.93 (s, 4 H) 4.14 - 4.22 (m, 2 H) 6.71 (d, J=4.63 Hz, 1 H) 7.15 (d, J=7.50 Hz, 1 H) 7.30 (br d, J=7.50 Hz, 1 H) 7.42 - 7.56 (m, 4 H) 7.65 (d, J=7.33 Hz, 1 H) 8.24 (dd, J=8.13, 3.38 Hz, 1 H) 9.56 (s, 1 H) 10.19 - 10.36 (m, 1 H)

### 40-7. Preparation of Compound 196, (3R)-1-(2-((2,2'-dichloro-3'-(3-fluoro-4-(((R)-3-hydroxypyrrolidin-1-yl)methyl)-5-methoxybenzamido)-[1,1'-biphenyl]-3-yl)carbamoyl)-4,5,6,7-tetrahydropyrazolo[1,5-a]pyridin-4-yl)pyrrolidine-3-carboxylic acid

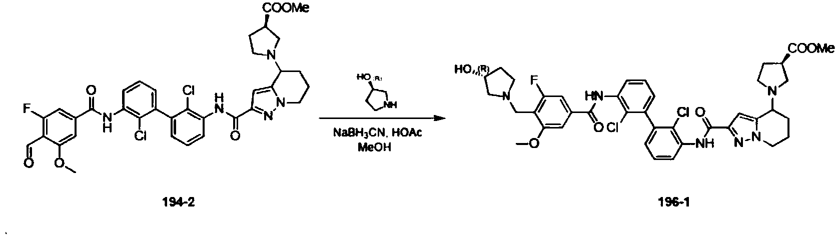

To a solution of methyl (3R)-1-[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]pyrrolidine-3-carboxylate (200 mg, 282.26 µmol, 1 eq) and (3R)-pyrrolidin-3-ol (245.91 mg, 2.82 mmol, 234.64 µL, 10 eq) in MeOH (20 mL), AcOH (33.90 mg, 564.52 µmol, 32.32 µL, 2 eq) and NaBH₃CN (88.69 mg, 1.41 mmol, 5 eq) were added, and the reaction mixture was stirred at 60 °C for 12 hours. After completion of the reaction, the mixture was concentrated under reduced pressure. The residue was purified by reversed-phase HPLC (0.1% TFA condition; column: Phenomenex Luna C18, 150 * 40 mm * 15 um; mobile phase: [water (TFA)-ACN]; gradient: 13-43% B over 15 min) to afford Compound 196-1 (73 mg, 32.5% yield) as a white solid.

LCMS m/z 779.3 [M+1]⁺.

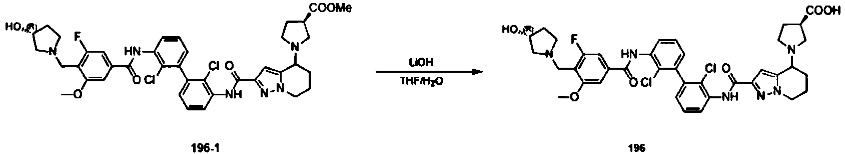

To a solution of methyl (3R)-1-[2-[[2-chloro-3-[2-chloro-3-[[3-fluoro-4-[[(3R)-3-hydroxypyrrolidin-1-yl]methyl]-5-methoxy-benzoyl]amino]phenyl]phenyl]carbamoyl]-4,5,6,7-tetrahydropyrazolo[1,5-a]pyridin-4-yl]pyrrolidine-3-carboxylate (70 mg, 89.78 µmol, 1 eq) in THF (20 mL) / H₂O (5 mL), LiOH (11.30 mg, 269.34 µmol, 3 eq) was added, and the reaction mixture was stirred at room temperature for 4 hours. After completion of the reaction, the mixture was concentrated under reduced pressure. The residue was purified by reversed-phase HPLC (0.1% NH₃·H₂O condition; column: Waters XBridge, 150 * 25 mm * 5 um; mobile phase: [water (NH₄HCO₃)-ACN]; gradient: 15-45% B over 9 min) to afford Compound 196 (35 mg, 49.9% yield) as a white solid.

LCMS m/z 765.3 [M+1]⁺.

¹H NMR (400 MHz, DMSO-d6) δ ppm 1.93 (br s, 6 H) 2.24 (br s, 1 H) 2.33 (br s, 1 H) 2.67 (br s, 2 H) 2.76 (br s, 2 H) 2.94 (br s, 2 H) 3.85 (br s, 1 H) 3.97 (s, 3 H) 4.10 - 4.25 (m, 3 H) 4.37 (br s, 2 H) 6.73 (br s, 1 H) 7.15 (d, J=7.38 Hz, 1 H) 7.32 (d, J=7.50 Hz, 1 H) 7.47 - 7.56 (m, 3 H) 7.59 (s, 1 H) 7.65 (dd, J=7.94, 1.44 Hz, 1 H) 8.24 (br d, J=8.25 Hz, 1 H) 9.57 (s, 1 H) 10.36 (s, 1 H).

### 40-8. Preparation of Compound 197, (3R)-1-(4-((3'-(4-((R)-3-carboxypyrrolidin-1-yl)-4,5,6,7-tetrahydropyrazolo[1,5-a]pyridine-2-carboxamido)-2,2'-dichloro-[1,1'-biphenyl]-3-yl)carbamoyl)-2-fluoro-6-methoxybenzyl)pyrrolidine-3-carboxylic acid

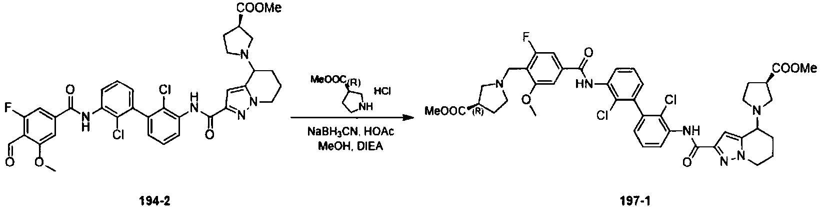

To a solution of methyl (3R)-1-[2-[[2-chloro-3-[2-chloro-3-[(3-fluoro-4-formyl-5-methoxybenzoyl)amino]phenyl]phenyl]carbamoyl]-4,5,6,7-tetrahydropyrazolo[1,5-a]pyridin-4-yl]pyrrolidine-3-carboxylate (200 mg, 282.26 µmol, 1 eq) and methyl (3R)-pyrrolidine-3-carboxylate (467.48 mg, 2.82 mmol, 10 eq, HCl) in MeOH (20 mL), AcOH (33.90 mg, 564.52 µmol, 32.32 µL, 2 eq), DIPEA (364.80 mg, 2.82 mmol, 491.65 µL, 10 eq), and NaBH₃CN (88.69 mg, 1.41 mmol, 5 eq) were added, and the reaction mixture was stirred at 60 °C for 12 hours. After completion of the reaction, the mixture was concentrated under reduced pressure. The residue was purified by reversed-phase HPLC (0.1% TFA condition; column: Phenomenex Luna C18, 150 * 40 mm * 15 um; mobile phase: [water (TFA)-ACN]; gradient: 18-48% B over 15 min) to afford Compound 197-1 (100 mg, 28.0% yield) as a white solid.

LCMS m/z 821.4 [M+1]⁺.

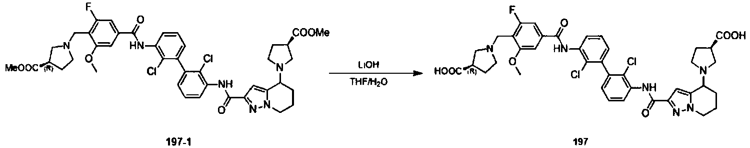

To a solution of methyl (3R)-1-[[4-[[2-chloro-3-[2-chloro-3-[[4-[(3R)-3-methoxycarbonylpyrrolidin-1-yl]-4,5,6,7-tetrahydropyrazolo[1,5-a]pyridine-2-carbonyl]amino]phenyl]phenyl]carbamoyl]-2-fluoro-6-methoxy-phenyl]methyl]pyrrolidine-3-carboxylate (100 mg, 121.70 µmol, 1 eq) in THF (25 mL) / H₂O (5 mL), LiOH (15.32 mg, 365.09 µmol, 3 eq) was added, and the reaction mixture was stirred at room temperature for 12 hours. After completion of the reaction, the mixture was concentrated under reduced pressure. The residue was purified by reversed-phase HPLC (0.1% NH₃·H₂O condition; column: Waters XBridge, 150 * 25 mm * 5 um; mobile phase: [water (NH₄HCO₃)-ACN]; gradient: 8-38% B over 9 min) to afford Compound 197 (80 mg, 81.1% yield) as a white solid.

LCMS m/z 793.3 [M+1]⁺.

¹H NMR (400 MHz, DMSO-d6) δ ppm 1.87 - 1.94 (m, 5 H) 2.24 (br s, 1 H) 2.33 (br s, 1 H) 2.56 - 2.64 (m, 3 H) 2.69 - 2.77 (m, 3 H) 2.81 - 2.86 (m, 2 H) 2.88 - 2.93 (m, 2 H) 3.68 (br s, 3 H) 3.82 (br s, 2 H) 3.90 (s, 3 H) 4.13 - 4.21 (m, 2 H) 6.71 (d, J=4.50 Hz, 1 H) 7.15 (d, J=7.50 Hz, 1 H) 7.29 (br d, J=7.63 Hz, 1 H) 7.41 - 7.53 (m, 4 H) 7.66 (dd, J=8.07, 1.44 Hz, 1 H) 8.25 (dd, J=8.13, 3.63 Hz, 1 H) 9.55 (s, 1 H) 10.24 (s, 1 H).

### [Preparation Example 41]

### Preparation of Compounds 198 to 199

### 41-1. Preparation of Compound 198, 2-((2-((2,2'-dichloro-3'-(6-fluoro-5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)amino)acetic acid

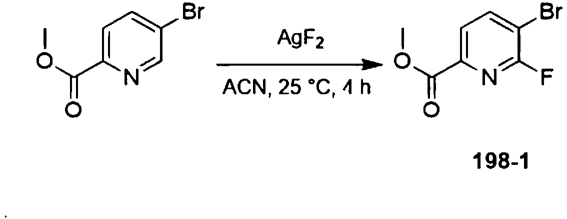

To a solution of methyl 5-bromopyridine-2-carboxylate (7 g, 32.40 mmol, 1 eq) in ACN (80 mL), difluorosilver (14.18 g, 97.21 mmol, 3 eq) was added. The reaction mixture was stirred at room temperature for 4 hours. After completion of the reaction, the mixture was filtered and concentrated under reduced pressure. The residue was purified by flash silica gel chromatography (Petroleum ether/Ethyl acetate = 100/1 to 5/1) to afford Compound 198-1 (7.2 g, 85.4% yield) as a white solid.

¹H NMR (400 MHz, DMSO-d6) δ = 8.57 - 8.40 (m, 1H), 7.92 (dd, J = 1.4, 7.9 Hz, 1H), 3.89 (s, 3H).

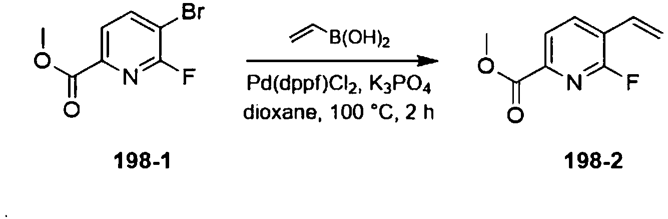

A mixture of methyl 5-bromo-6-fluoropyridine-2-carboxylate (6 g, 25.64 mmol, 1 eq), potassium trifluoro(vinyl)boranide (6.87 g, 51.28 mmol, 2 eq), K₃PO₄ (16.33 g, 76.92 mmol, 3 eq), and Pd(dppf)Cl₂·CH₂Cl₂ (1.05 g, 1.28 mmol, 0.05 eq) in dioxane (150 mL) was stirred under a nitrogen atmosphere at 100 °C for 2 hours. After completion of the reaction, the mixture was concentrated under reduced pressure. The residue was purified by flash silica gel chromatography (Petroleum ether/Ethyl acetate = 100/1 to 5/1) to afford Compound 198-2 (4.2 g, 81.8% yield) as a white solid.

¹H NMR (400 MHz, DMSO-d6) δ = 8.37 (dd, J = 7.8, 9.7 Hz, 1H), 8.00 (dd, J = 1.4, 7.7 Hz, 1H), 6.83 (dd, J = 11.3, 17.8 Hz, 1H), 6.18 (d, J = 17.6 Hz, 1H), 5.81 - 5.63 (m, 1H), 3.88 (s, 3H).

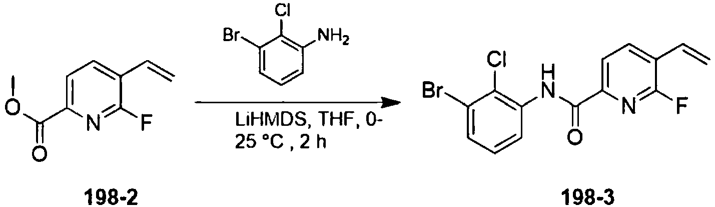

To a solution of methyl 6-fluoro-5-vinylpyridine-2-carboxylate (3.5 g, 19.32 mmol, 1 eq) and 3-bromo-2-chloroaniline (3.99 g, 19.32 mmol, 1 eq) in THF (120 mL), LiHMDS (1 M, 38.64 mL, 2 eq) was added at 0 °C, and the reaction mixture was stirred at room temperature for 2 hours. After completion of the reaction, H₂O (200 mL) was added, and the mixture was extracted with EA (200 mL x 3). The combined organic layers were washed with brine (600 mL), dried over Na₂SO₄, and concentrated under reduced pressure. The residue was purified by flash silica gel chromatography (Petroleum ether/Ethyl acetate = 100/1 to 3/1) to afford Compound 198-3 (3.2 g, 42.8% yield) as a white solid.

¹H NMR (400 MHz, DMSO-d6) δ = 10.34 (s, 1H), 8.47 (dd, J = 7.9, 9.5 Hz, 1H), 8.22 - 8.04 (m, 2H), 7.64 (dd, J = 1.4, 8.1 Hz, 1H), 7.37 (t, J = 8.1 Hz, 1H), 6.87 (dd, J = 11.3, 17.7 Hz, 1H), 6.21 (d, J = 17.8 Hz, 1H), 5.71 (d, J = 11.4 Hz, 1H).

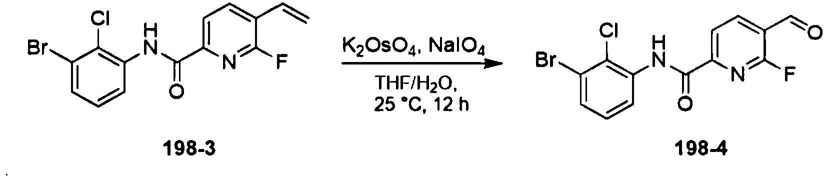

To a solution of N-(3-bromo-2-chloro-phenyl)-6-fluoro-5-vinyl-pyridine-2-carboxamide (1.5 g, 4.22 mmol, 1 eq) and sodium periodate (2.71 g, 12.66 mmol, 701.24 µL, 3 eq) in H₂O (6 mL) / THF (60 mL), dipotassium dioxido(dioxo)osmium dihydrate (310.85 mg, 843.67 µmol, 0.2 eq) was slowly added at room temperature over 30 minutes, and the reaction mixture was stirred at room temperature for 12 hours. After completion of the reaction, H₂O (40 mL) was added, and the mixture was extracted with EA (40 mL x 3). The combined organic layers were washed with brine (60 mL), dried over Na₂SO₄, and concentrated under reduced pressure to afford Compound 198-4 (1.42 g, 84.7% yield) as a yellow solid, which was used in the subsequent reaction without further purification.

LCMS m/z 359.1 [M+2]⁺.

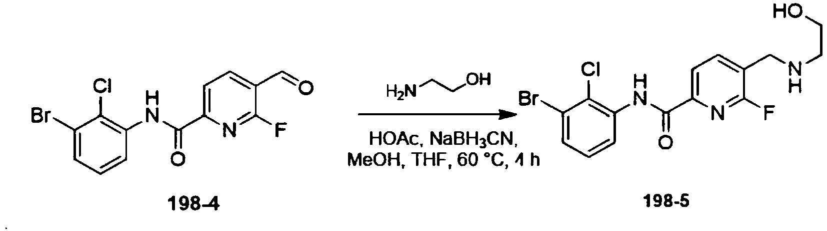

A solution of N-(3-bromo-2-chloro-phenyl)-6-fluoro-5-formyl-pyridine-2-carboxamide (700 mg, 1.96 mmol, 1 eq), 2-aminoethanol (956.66 mg, 15.66 mmol, 947.18 µL, 8 eq), and HOAc (1.18 g, 19.58 mmol, 1.12 mL, 10 eq) in MeOH (10 mL) / THF (10 mL) was stirred at 60 °C for 2 hours. NaBH₃CN (369.07 mg, 5.87 mmol, 3 eq) was then added, and the mixture was further stirred at 60 °C for 2 hours. After completion of the reaction, the mixture was concentrated under reduced pressure. The residue was purified by prep-HPLC (column: Kromasil Eternity XT, 250 * 80 mm * 10 um; mobile phase: [water (NH₄HCO₃)-ACN]; gradient: 30-60% B over 20 min) to afford Compound 198-5 (420 mg, 47.9% yield) as a white solid.

LCMS m/z 403.7 [M+1]⁺.

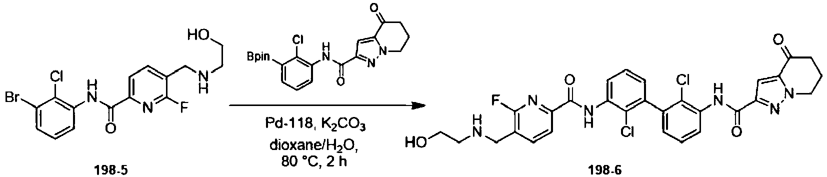

A mixture of N-(3-bromo-2-chloro-phenyl)-6-fluoro-5-[(2-hydroxyethylamino)methyl]pyridine-2-carboxamide (300 mg, 745.07 µmol, 1 eq) and N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (464.56 mg, 1.12 mmol, 1.5 eq), ditert-butyl(cyclopentyl)phosphane;dichloropalladium iron (48.56 mg, 74.51 µmol, 0.1 eq) and K₂CO₃ (308.92 mg, 2.24 mmol, 3 eq) in dioxane (6 mL) / H₂O (1.5 mL) was stirred under a nitrogen atmosphere at 80 °C for 2 hours. After completion of the reaction, the mixture was filtered and the filtrate was concentrated under reduced pressure. The residue was purified by prep-TLC (DCM:MeOH = 10/1) to afford Compound 198-6 (421 mg, 73.9% yield) as a white solid.

LCMS m/z 611.2 [M+1]⁺.

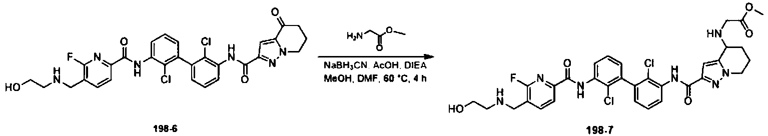

To a mixture of N-[2-chloro-3-[2-chloro-3-[[6-fluoro-5-[(2-hydroxyethylamino)methyl]pyridine-2-carbonyl]amino]phenyl]phenyl]-4-oxo-6,7-dihydro-5H-pyrazolo[1,5-a]pyridine-2-carboxamide (100 mg, 163.55 µmol, 1 eq), methyl 2-aminoacetate (145.71 mg, 1.64 mmol, 10 eq), and DIPEA (211.37 mg, 1.64 mmol, 284.87 µL, 10 eq) in MeOH (20 mL) / DMF (5 mL), HOAc (196.42 mg, 3.27 mmol, 187.24 µL, 20 eq) was added, and the reaction mixture was stirred at 60 °C for 2 hours. NaBH₃CN (51.39 mg, 817.73 µmol, 5 eq) was then added, and the mixture was further stirred at 60 °C for 2 hours. After completion of the reaction, the mixture was concentrated under reduced pressure. The residue was purified by prep-HPLC (column: Waters XBridge Prep OBD C18, 150 * 40 mm * 10 um; mobile phase: [water (NH₄HCO₃)-ACN]; gradient: 28-58% B over 15 min) to afford Compound 198-7 (81 mg, 66.5% yield) as a white solid.

LCMS m/z 684.3 [M+1]⁺.

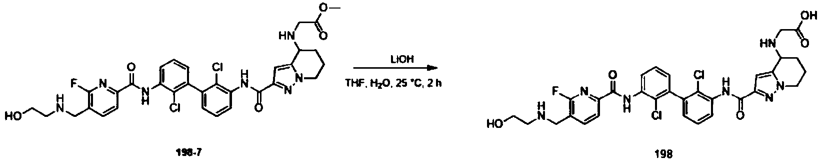

To a solution of methyl 2-[[2-[[2-chloro-3-[2-chloro-3-[[6-fluoro-5-[(2-hydroxyethylamino)methyl]pyridine-2-carbonyl]amino]phenyl]phenyl]carbamoyl]-4,5,6,7-tetrahydropyrazolo[1,5-a]pyridin-4-yl]amino]acetate (80 mg, 116.87 µmol, 1 eq) in THF (10 mL) / H₂O (3 mL), LiOH (14.71 mg, 350.60 µmol, 3 eq) was added, and the reaction mixture was stirred at room temperature for 2 hours. After completion of the reaction, the mixture was concentrated under reduced pressure. The residue was purified by prep-HPLC (column: Waters XBridge, 150 * 25 mm * 5 um; mobile phase: [water (NH₄HCO₃)-ACN]; gradient: 18-48% B over 9 min) to afford Compound 198 (36 mg, 43.6% yield) as a white solid.

LCMS m/z 670.4 [M+1]⁺.

¹H NMR (400 MHz, DMSO-d6 ) δ = 10.30 (s, 1H), 9.57 (s, 1H), 8.41 - 8.32 (m, 1H), 8.31 - 8.21 (m, 2H), 8.14 (dd, J = 1.5, 7.5 Hz, 1H), 7.51 (td, J = 7.9, 14.4 Hz, 2H), 7.26 - 7.12 (m, 2H), 6.82 (s, 1H), 4.67 - 4.34 (m, 1H), 4.22 - 4.12 (m, 2H), 4.09 - 4.01 (m, 1H), 3.88 (s, 2H), 3.49 (br d, J = 5.9 Hz, 2H), 2.67 - 2.62 (m, 2H), 2.37 - 2.30 (m, 2H), 2.26 - 2.09 (m, 2H), 2.07 (s, 3H), 1.81 - 1.65 (m, 1H).

### 41-2. Preparation of Compound 199, (3R)-1-(2-((2,2'-dichloro-3'-(6-fluoro-5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

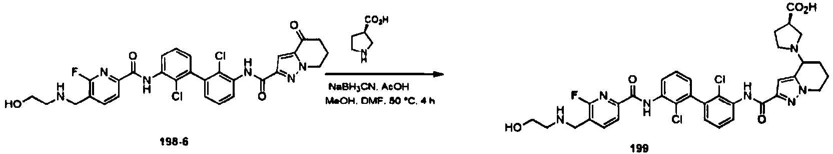

To a solution of N-[2-chloro-3-[2-chloro-3-[[6-fluoro-5-[(2-hydroxyethylamino)methyl]pyridine-2-carbonyl]amino]phenyl]phenyl]-4-oxo-6,7-dihydro-5H-pyrazolo[1,5-a]pyridine-2-carboxamide (100 mg, 163.55 µmol, 1 eq) and (3R)-pyrrolidine-3-carboxylic acid (94.15 mg, 817.73 µmol, 5 eq) in MeOH (20 mL) / DMF (5 mL), HOAc (196.42 mg, 3.27 mmol, 187.24 µL, 20 eq) was added, and the reaction mixture was stirred at 50 °C for 2 hours. NaBH₃CN (51.39 mg, 817.73 µmol, 5 eq) was then added, and the mixture was further stirred at 50 °C for 2 hours. After completion of the reaction, the mixture was filtered, and the filtrate was concentrated under reduced pressure. The residue was purified by prep-HPLC (column: Waters XBridge Prep OBD C18, 150 * 40 mm * 10 um; mobile phase: [water (NH₄HCO₃)-ACN]; gradient: 15-45% B over 15 min) to afford Compound 199 (56 mg, 45.7% yield) as a white solid.

LCMS m/z 710.4 [M+1]⁺.

¹H NMR (400 MHz, DMSO-d6 ) δ = 10.30 (s, 1H), 9.57 (s, 1H), 8.34 (br d, J = 7.9 Hz, 1H), 8.31 - 8.23 (m, 2H), 8.20 - 8.08 (m, 1H), 7.62 - 7.44 (m, 2H), 7.19 (dd, J = 7.5, 17.1 Hz, 2H), 6.72 (d, J = 4.5 Hz, 1H), 4.23 - 4.12 (m, 2H), 3.90 - 3.81 (m, 3H), 3.51 - 3.47 (m, 3H), 2.98 - 2.80 (m, 3H), 2.79 - 2.66 (m, 3H), 2.60 - 2.57 (m, 2H), 2.34 - 2.15 (m, 1H), 1.98 - 1.84 (m, 5H).

### [Preparation Example 42]

### Preparation of Compounds 200 to 201

### 42-1. Preparation of Compound 200, 2-((2-((2,2'-dichloro-3'-(3-fluoro-5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)amino)acetic acid

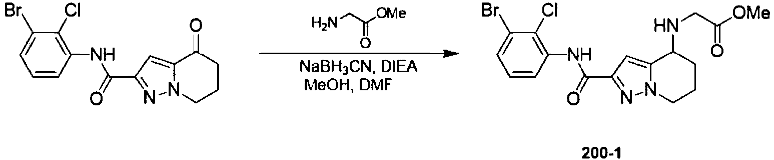

To a solution of N-(3-bromo-2-chloro-phenyl)-4-oxo-6,7-dihydro-5H-pyrazolo[1,5-a]pyridine-2-carboxamide (1 g, 2.71 mmol, 1 eq) and methyl 2-aminoacetate (1.21 g, 13.56 mmol, 5 eq) in DMF (10 mL) / MeOH (10 mL), NaBH₃CN (852.39 mg, 13.56 mmol, 5 eq) and DIPEA (1.75 g, 13.56 mmol, 2.36 mL, 5 eq) were added, and the reaction mixture was stirred at 60 °C for 12 hours. After completion of the reaction, the mixture was concentrated under reduced pressure. The residue was purified by column chromatography (SiO₂, Petroleum ether/Ethyl acetate = 1/1 to 0/1), followed by additional purification by reversed-phase HPLC (column: Waters XBridge Prep OBD C18, 150 * 40 mm * 10 um; mobile phase: [water (NH₄HCO₃)-ACN]; gradient: 38-68% B over 15 min) to afford Compound 200-1 (315 mg, 25.7% yield) as a yellow liquid.

LCMS m/z 440.03 [M+2]⁺.

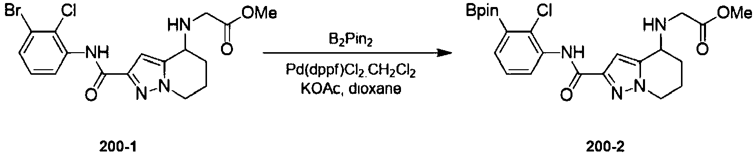

A solution of methyl 2-[[2-[(3-bromo-2-chlorophenyl)carbamoyl]-4,5,6,7-tetrahydropyrazolo[1,5-a]pyridin-4-yl]amino]acetate (200 mg, 452.79 µmol, 1 eq), 4,4,5,5-tetramethyl-2-(4,4,5,5-tetramethyl-1,3,2-dioxaborolan-2-yl)-1,3,2-dioxaborolane (344.94 mg, 1.36 mmol, 3 eq), AcOK (133.31 mg, 1.36 mmol, 3 eq), and Pd(dppf)Cl₂·CH₂Cl₂ (36.98 mg, 45.28 µmol, 0.1 eq) in dioxane (2 mL) was stirred under a nitrogen atmosphere at 90 °C for 12 hours. After completion of the reaction, the mixture was concentrated under reduced pressure. The residue was purified by prep-TLC (SiO₂, Petroleum ether:Ethyl acetate = 0:1) to afford Compound 200-2 (174 mg, 55.4% yield) as a black solid.

LCMS m/z 488.2 [M+1]⁺.

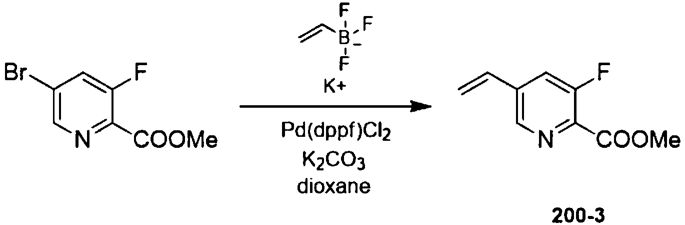

A mixture of methyl 5-bromo-3-fluoropyridine-2-carboxylate (5 g, 21.37 mmol, 1 eq), potassium trifluoro(vinyl)boranide (2.86 g, 21.37 mmol, 1 eq), Pd(dppf)Cl₂ (1.56 g, 2.14 mmol, 0.1 eq), and K₂CO₃ (8.86 g, 64.10 mmol, 3 eq) in dioxane (60 mL) was stirred under a nitrogen atmosphere at 100 °C for 12 hours. After completion of the reaction, the mixture was filtered and the filtrate was concentrated under reduced pressure. The residue was purified by prep-TLC (SiO₂, Petroleum ether:Ethyl acetate = 2:1) to afford Compound 200-3 (3.23 g, 82.6% yield) as a pink solid.

LCMS m/z 181.05 [M+1]⁺.

¹H NMR (EW41301-123-P1A1, 400 MHz, DMSO-d6) δ ppm 3.89 (s, 3 H) 5.64 (d, *J* =11.01 Hz, 1 H) 6.25 (d, *J* =17.76 Hz, 1 H) 6.86 (dd, *J* =17.64, 11.13 Hz, 1 H) 8.11 (br d, *J* =12.01 Hz, 1 H) 8.64 (s, 1 H).

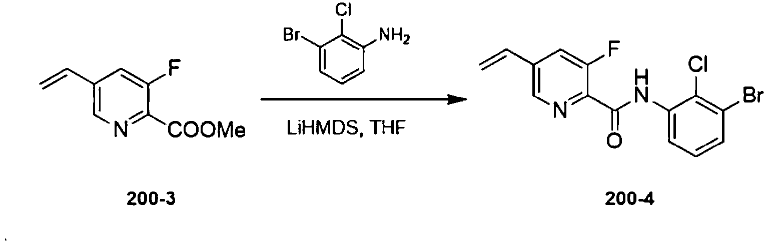

A mixture of methyl 3-fluoro-5-vinyl-pyridine-2-carboxylate (3.2 g, 17.66 mmol, 1 eq), 3-bromo-2-chloroaniline (3.65 g, 17.66 mmol, 1 eq), and LiHMDS (1 M in THF, 35.33 mL, 2 eq) in THF (40 mL) was stirred under a nitrogen atmosphere at room temperature for 12 hours. After completion of the reaction, an aq. saturated NH₄Cl solution (100 mL) was added, and the mixture was extracted with EtOAc (100 mL x 2). The combined organic layers were washed with brine (100 mL), dried over Na₂SO₄, and concentrated under reduced pressure. The residue was purified by column chromatography (SiO₂, Petroleum ether/Ethyl acetate = 2/1 to 0/1) to afford Compound 200-4 (6 g, 76.4% yield) as a pink solid.

LCMS m/z 353.96 [M+3]⁺.

¹H NMR (EW41301-130-P1A1, 400 MHz, DMSO-d6) δ ppm 5.55 - 5.69 (m, 1 H) 6.20 - 6.33 (m, 1 H) 6.79 - 6.93 (m, 1 H) 7.29 - 7.41 (m, 1 H) 7.53 - 7.65 (m, 1 H) 8.15 (d, *J* =12.38 Hz, 1 H) 8.19 - 8.30 (m, 1 H) 8.70 (s, 1 H) 10.47 - 10.60 (m, 1 H).

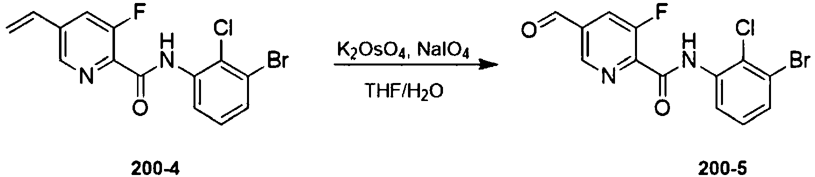

To a solution of N-(3-bromo-2-chloro-phenyl)-3-fluoro-5-vinyl-pyridine-2-carboxamide (3 g, 8.44 mmol, 1 eq) in THF (80 mL) / H₂O (5 mL), NaIO₄ (7.22 g, 33.75 mmol, 1.87 mL, 4 eq) and dipotassium dioxido(dioxo)osmium dihydrate (310.85 mg, 843.67 µmol, 0.1 eq) were added, and the reaction mixture was stirred at room temperature for 12 hours. After completion of the reaction, H₂O (100 mL) was added, and the mixture was extracted with ethyl acetate (50 mL x 3). The combined organic layers were washed with brine (100 mL), dried over Na₂SO₄, and concentrated under reduced pressure to afford Compound 200-5 (2.1 g, 34.8% yield), which was used in the subsequent reaction without further purification.

LCMS m/z 355.94 [M+3]⁺.

¹H NMR (400 MHz, DMSO-d6) δ ppm 7.38 (br t, *J* =8.13 Hz, 1 H) 7.56 - 7.77 (m, 1 H) 7.99 - 8.16 (m, 1 H) 8.38 (br d, *J* =10.26 Hz, 1 H) 9.07 (s, 1 H) 10.20 (s, 1 H) 10.62 - 10.75 (m, 1 H).

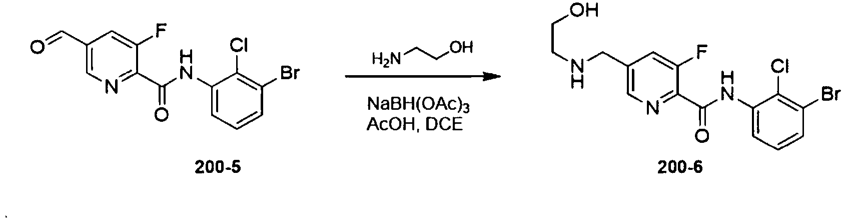

To a solution of N-(3-bromo-2-chloro-phenyl)-3-fluoro-5-formyl-pyridine-2-carboxamide (1 g, 2.80 mmol, 1 eq) and 2-aminoethanol (1.71 g, 27.97 mmol, 1.69 mL, 10 eq) in DCE (20 mL), AcOH (839.71 mg, 13.98 mmol, 800.49 µL, 5 eq) and NaBH(OAc)₃ (1.54 g, 7.27 mmol, 2.6 eq) were added, and the reaction mixture was stirred at 40 °C for 12 hours. After completion of the reaction, the mixture was concentrated under reduced pressure. The residue was purified by reversed-phase HPLC (column: Kromasil Eternity XT, 250 * 80 mm * 10 um; mobile phase: [water (NH₄HCO₃)-ACN]; gradient: 30-60% B over 20 min) to afford Compound 200-6 (480 mg, 38.3% yield) as a white solid.

LCMS m/z 400.99 [M+3]⁺.

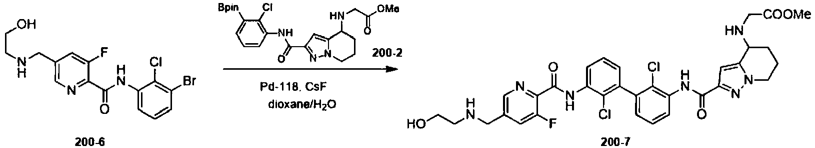

A mixture of N-(3-bromo-2-chloro-phenyl)-3-fluoro-5-[(2-hydroxyethylamino)methyl]pyridine-2-carboxamide (100 mg, 248.36 µmol, 1 eq) and methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (121.39 mg, 248.36 µmol, 1 eq), CsF (75.45 mg, 496.71 µmol, 18.34 µL, 2 eq) and ditert-butyl(cyclopentyl)phosphane;dichloropalladium iron (16.19 mg, 24.84 µmol, 0.1 eq) in dioxane (4 mL) / H₂O (1 mL) was stirred under a nitrogen atmosphere at 70 °C for 12 hours. After completion of the reaction, the mixture was concentrated under reduced pressure. The residue was purified by prep-TLC (SiO₂, ethyl acetate: methanol = 3:1) to afford Compound 200-7 (103 mg, 54.5% yield) as a brown solid.

LCMS m/z 683.18 [M+1]⁺.

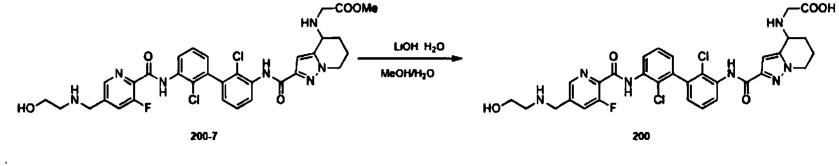

To a mixture of methyl 2-[[2-[[2-chloro-3-[2-chloro-3-[[3-fluoro-5-[(2-hydroxyethylamino)methyl]pyridine-2-carbonyl]amino]phenyl]phenyl]carbamoyl]-4,5,6,7-tetrahydropyrazolo[1,5-a]pyridin-4-yl]amino]acetate (103 mg, 150.47 µmol, 1 eq) in H₂O (2 mL) / MeOH (2 mL), LiOH·H₂O (18.94 mg, 451.40 µmol, 3 eq) was added, and the reaction mixture was stirred at room temperature for 12 hours. After completion of the reaction, the mixture was concentrated under reduced pressure. The residue was purified by reversed-phase HPLC (column: Phenomenex Luna C18, 150 * 25 mm * 10 um; mobile phase: [water (HCl)-ACN]; gradient: 5-35% B over 10 min) to afford Compound 200 (22 mg, 21.5% yield) as a white solid.

LCMS m/z 669.17 [M+1]⁺.

¹H NMR (400 MHz, DMSO-d6) δ ppm 1.97 - 2.16 (m, 2 H) 2.27 (br s, 2 H) 2.99 - 3.09 (m, 4 H) 3.72 (br t, *J* =5.19 Hz, 2 H) 3.92 - 4.07 (m, 2 H) 4.22 (br s, 2 H) 4.37 (br s, 2 H) 4.74 (br s, 1 H) 7.18 - 7.25 (m, 3 H) 7.48 - 7.58 (m, 2 H) 8.19 (d, *J*=8.13 Hz, 1 H) 8.24 - 8.34 (m, 2 H) 8.75 (s, 1 H) 9.59 (br s, 2 H) 9.66 (s, 1 H) 9.83 (br s, 1 H) 10.58 (s, 1 H).

### 42-2. Preparation of Compound 201, (3R)-1-(2-((2,2'-dichloro-3'-(3-fluoro-5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

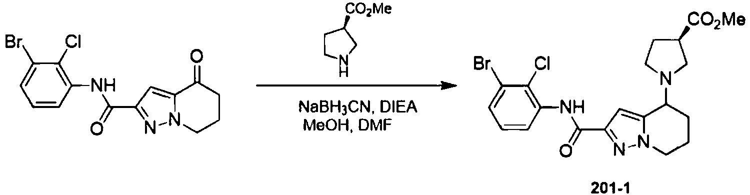

To a solution of N-(3-bromo-2-chloro-phenyl)-4-oxo-6,7-dihydro-5H-pyrazolo[1,5-a]pyridine-2-carboxamide (1.0 g, 2.71 mmol, 1 eq) and methyl (3R)-pyrrolidine-3-carboxylate (1.75 g, 13.56 mmol, 5 eq) in DMF (10 mL) / MeOH (10 mL), NaBH₃CN (852.39 mg, 13.56 mmol, 5 eq) and DIPEA (1.75 g, 13.56 mmol, 2.36 mL, 5 eq) were added, and the reaction mixture was stirred at 60 °C for 12 hours. After completion of the reaction, the mixture was concentrated under reduced pressure. The residue was purified by column chromatography on silica gel (Petroleum ether/Ethyl acetate = 1/1 to 0/1) to afford Compound 201-1 (1.1 g, 71.5% yield) as a yellow liquid.

LCMS m/z 480.06 [M+3]⁺.

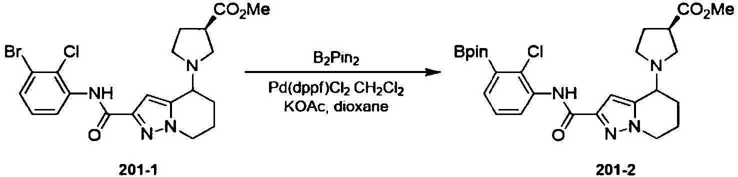

A mixture of methyl (3R)-1-[2-(3-bromo-2-chloro-phenyl)carbamoyl-4,5,6,7-tetrahydropyrazolo[1,5-a]pyridin-4-yl]pyrrolidine-3-carboxylate (1.0 g, 2.08 mmol, 1 eq), 4,4,5,5-tetramethyl-2-(4,4,5,5-tetramethyl-1,3,2-dioxaborolan-2-yl)-1,3,2-dioxaborolane (1.58 g, 6.23 mmol, 3 eq), AcOK (611.14 mg, 6.23 mmol, 3 eq), and Pd(dppf)Cl₂ (151.88 mg, 207.57 µmol, 0.1 eq) in dioxane (20 mL) was stirred under a nitrogen atmosphere at 90 °C for 12 hours. After completion of the reaction, the mixture was concentrated under reduced pressure. The residue was purified by column chromatography on silica gel (Petroleum ether/Ethyl acetate = 1/1 to 0/1) to afford Compound 201-2 (1.21 g, 33.1% yield) as a black solid.

LCMS m/z 528.23 [M+1]⁺.

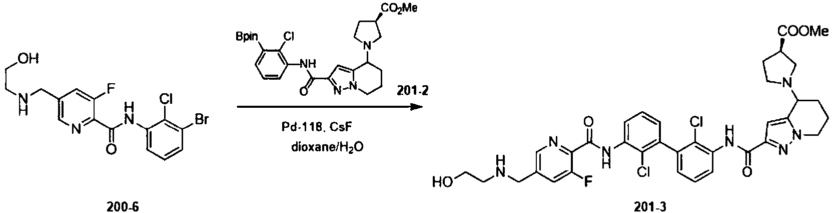

A mixture of N-(3-bromo-2-chloro-phenyl)-3-fluoro-5-[(2-hydroxyethylamino)methyl]pyridine-2-carboxamide (100 mg, 248.36 µmol, 1 eq), methyl (3R)-1-[2-[2-chloro-3-(4,4,5,5-tetramethyl-1,3,2-dioxaborolan-2-yl)phenyl]carbamoyl-4,5,6,7-tetrahydropyrazolo[1,5-a]pyridin-4-yl]pyrrolidine-3-carboxylate (394.0 mg, 745.07 µmol, 3 eq), CsF (75.45 mg, 496.71 µmol, 18.34 µL, 2 eq), and ditert-butyl(cyclopentyl)phosphane;dichloropalladium iron (16.19 mg, 24.84 µmol, 0.1 eq) in dioxane (4 mL) / water (1 mL) was stirred under a nitrogen atmosphere at 70 °C for 12 hours. After completion of the reaction, the mixture was concentrated under reduced pressure. The residue was purified by prep-TLC (SiO₂, Ethyl acetate:Methanol = 3:1) to afford Compound 201-3 (113 mg, 56.51% yield) as a light brown solid.

LCMS m/z 723.21 [M+1]⁺.

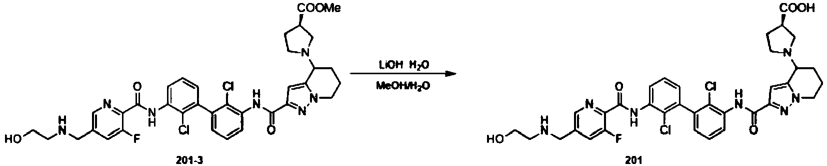

To a solution of methyl (3R)-1-[2-[2-chloro-3-[2-chloro-3-[[3-fluoro-5-[(2-hydroxyethylamino)methyl]pyridine-2-carbonyl]amino]phenyl]phenyl]carbamoyl-4,5,6,7-tetrahydropyrazolo[1,5-a]pyridin-4-yl]pyrrolidine-3-carboxylate (113 mg, 155.95 µmol, 1 eq) in H₂O (3 mL)/methanol (3 mL), LiOH·H₂O (19.63 mg, 467.84 µmol, 3 eq) was added, and the reaction mixture was stirred at room temperature for 12 hours. After completion of the reaction, the mixture was concentrated under reduced pressure. The residue was purified by reversed-phase HPLC (column: Phenomenex Luna C18, 150 * 25 mm * 10 µm; mobile phase: [H₂O(HCl)-ACN]; gradient: 5% to 35% B over 10 min) to afford Compound 201 (46 mg, 40.7% yield) as a white solid.

LCMS m/z 709.2 [M+1]⁺.

¹H NMR (400 MHz, DMSO-d6) δ ppm 2.02 (br s, 1 H) 2.25 (br s, 2 H) 3.04 (br s, 2 H) 3.25 - 3.33 (m, 2 H) 3.45 (br dd, *J* =14.01, 7.00 Hz, 5 H) 3.66 - 3.79 (m, 4 H) 3.97 (s, 1 H) 4.23 (br d, *J* =6.25 Hz, 1 H) 4.26 - 4.33 (m, 1 H) 4.37 (br s, 2 H) 4.89 (br s, 1 H) 7.22 (t, *J* =6.82 Hz, 2 H) 7.30 (br d, *J* =8.50 Hz, 1 H) 7.46 - 7.60 (m, 2 H) 8.14 - 8.22 (m, 1 H) 8.28 - 8.34 (m, 1 H) 8.76 (s, 1 H) 9.60 (br s, 2 H) 9.68 (br s, 1 H) 10.58 (s, 1 H) 11.86 (br s, 1 H).

### [Preparation Example 43]

### Preparation of Compounds 202 to 203

### 43-1. Preparation of Compound 202, 2-((2-((2,2'-dichloro-3'-(6-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid

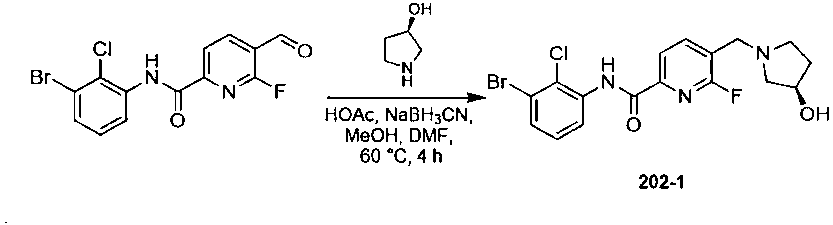

A mixture of N-(3-bromo-2-chloro-phenyl)-6-fluoro-5-formyl-pyridine-2-carboxamide (700 mg, 1.96 mmol, 1 eq), (3R)-pyrrolidin-3-ol (1.36 g, 15.66 mmol, 1.30 mL, 8 eq), and HOAc (1.18 g, 19.58 mmol, 10 eq) in DMF (5 mL) / MeOH (20 mL) was stirred at 60 °C for 2 hours, and then NaBH₃CN (369.07 mg, 5.87 mmol, 3 eq) was added. The mixture was further stirred at 60 °C for an additional 2 hours. After completion of the reaction, the mixture was concentrated under reduced pressure. The residue was purified by prep-HPLC (column: Kromasil Eternity XT, 250 * 80 mm * 10 µm; mobile phase: [water (NH₄HCO₃)-ACN]; gradient: 30% to 60% B over 20 min) to afford Compound 202-1 (360 mg, 38.6% yield) as a white solid.

LCMS m/z 429.7 [M+2]⁺.

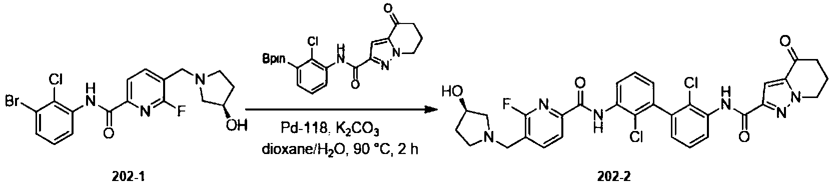

A mixture of N-(3-bromo-2-chloro-phenyl)-6-fluoro-5-[[(3R)-3-hydroxypyrrolidin-1-yl]methyl]pyridine-2-carboxamide (120 mg, 279.93 µmol, 1 eq), N-[2-chloro-3-(4,4,5,5-tetramethyl-1,3,2-dioxaborolan-2-yl)phenyl]-4-oxo-6,7-dihydro-5H-pyrazolo[1,5-a]pyridine-2-carboxamide (139.63 mg, 335.91 µmol, 1.2 eq), ditert-butyl(cyclopentyl)phosphane;dichloropalladium iron (18.24 mg, 27.99 µmol, 0.1 eq), and K₂CO₃ (116.06 mg, 839.78 µmol, 3 eq) in dioxane (4 mL) / H₂O (1.5 mL) was stirred under a nitrogen atmosphere at 90 °C for 2 hours. After completion of the reaction, the mixture was filtered and the filtrate was concentrated under reduced pressure. The residue was purified by prep-TLC (DCM:MeOH = 10:1) to afford Compound 202-2 (172 mg, 86.7% yield) as a white solid.

LCMS m/z 637.4 [M+1]⁺.

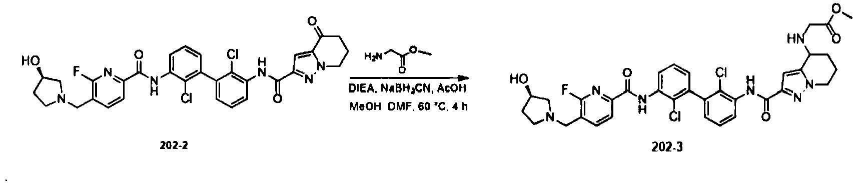

To a mixture of N-[2-chloro-3-[2-chloro-3-[[6-fluoro-5-[[(3R)-3-hydroxypyrrolidin-1-yl]methyl]pyridine-2-carbonyl]amino]phenyl]phenyl]-4-oxo-6,7-dihydro-5H-pyrazolo[1,5-a]pyridine-2-carboxamide (70 mg, 109.81 µmol, 1 eq), methyl 2-aminoacetate (97.83 mg, 1.10 mmol, 10 eq), and DIPEA (141.92 mg, 1.10 mmol, 191.26 µL, 10 eq) in MeOH (20 mL) / DMF (5 mL), HOAc (131.88 mg, 2.20 mmol, 125.72 µL, 20 eq) was added, and the reaction mixture was stirred at 60 °C for 2 hours. NaBH₃CN (34.50 mg, 549.03 µmol, 5 eq) was then added, and the reaction mixture was further stirred at 60 °C for 2 hours. After completion of the reaction, the mixture was concentrated under reduced pressure. The residue was purified by prep-HPLC (column: Waters Xbridge Prep OBD C18, 150 * 40 mm * 10 µm; mobile phase: [water (NH₄HCO₃)-ACN]; gradient: 33% to 63% B over 15 min) to afford Compound 202-3 (82 mg, 89.3% yield) as a white solid.

LCMS m/z 710.3 [M+1]⁺.

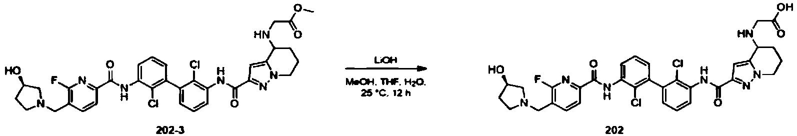

To a solution of methyl 2-[[2-[[2-chloro-3-[2-chloro-3-[[6-fluoro-5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]amino]acetate (50 mg, 70.36 µmol, 1 eq) in THF (10 mL) / water (3 mL), LiOH (8.86 mg, 211.09 µmol, 3 eq) was added, and the reaction mixture was stirred at room temperature for 12 hours. After completion of the reaction, the mixture was concentrated under reduced pressure. The residue was purified by prep-HPLC (column: Waters Xbridge, 150 * 25 mm * 5 µm; mobile phase: [water (NH₄HCO₃)-ACN]; gradient: 18% to 48% B over 9 min) to afford Compound 202 (18 mg, 34.8% yield) as a white solid.

LCMS m/z 696.4 [M+1]⁺.

¹H NMR (400 MHz, DMSO-d6 ) δ = 10.33 (s, 1H), 9.59 (s, 1H), 8.40 - 8.28 (m, 2H), 8.25 (d, J = 8.3 Hz, 2H), 7.60 - 7.47 (m, 2H), 7.29 - 7.15 (m, 2H), 6.89 (s, 1H), 4.36 - 4.24 (m, 1H), 4.16 (br t, J = 5.8 Hz, 3H), 4.04 - 3.93 (m, 1H), 3.53 - 3.47 (m, 2H), 3.03 - 2.87 (m, 2H), 2.79 - 2.60 (m, 1H), 2.37 - 2.17 (m, 4H), 2.13 - 2.00 (m, 3H), 1.97 - 1.88 (m, 1H), 1.86 - 1.57 (m, 3H).

### 43-2. Preparation of Compound 203, (3R)-1-(2-((2,2'-dichloro-3'-(6-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

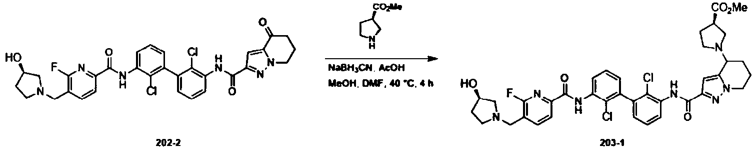

To a solution of N-[2-chloro-3-[2-chloro-3-[[6-fluoro-5-[[(3R)-3-hydroxypyrrolidin-1-yl]methyl]pyridine-2-carbonyl]amino]phenyl]phenyl]-4-oxo-6,7-dihydro-5H-pyrazolo[1,5-a]pyridine-2-carboxamide (70 mg, 109.81 µmol, 1 eq) and methyl (3R)-pyrrolidine-3-carboxylate (141.82 mg, 1.10 mmol, 10 eq) in MeOH (20 mL) / DMF (5 mL), HOAc (131.88 mg, 2.20 mmol, 125.72 µL, 20 eq) was added, and the reaction mixture was stirred at 40 °C for 2 hours. NaBH₃CN (34.50 mg, 549.03 µmol, 5 eq) was then added, and the mixture was further stirred at 40 °C for 2 hours. After completion of the reaction, the mixture was concentrated under reduced pressure. The residue was purified by prep-HPLC (column: Waters Xbridge Prep OBD C18, 150 * 40 mm * 10 µm; mobile phase: [water (NH₄HCO₃)-ACN]; gradient: 45% to 75% B over 15 min) to afford Compound 203-1 (46 mg, 50.2% yield) as a white solid.

LCMS m/z 750.4 [M+1]⁺.

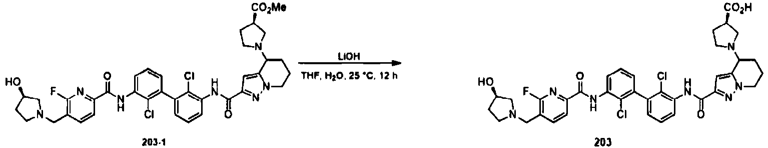

To a solution of methyl (3R)-1-[2-[[2-chloro-3-[2-chloro-3-[[6-fluoro-5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]pyrrolidine-3-carboxylate (46 mg, 61.28 µmol, 1 eq) in THF (10 mL) / H₂O (3 mL), LiOH·H₂O (7.71 mg, 183.84 µmol, 3 eq) was added, and the reaction mixture was stirred at room temperature for 12 hours. After completion of the reaction, the mixture was filtered, and the filtrate was concentrated under reduced pressure. The residue was purified by prep-HPLC (column: Waters Xbridge, 150 * 25 mm * 5 µm; mobile phase: [water (NH₄HCO₃)-ACN]; gradient: 18% to 48% B over 9 min) to afford Compound 203 (36 mg, 75.7% yield) as a white solid.

LCMS m/z 736.4 [M+1]⁺.

¹H NMR (400 MHz, DMSO-d6 ) δ = 10.31 (s, 1H), 9.57 (s, 1H), 8.40 - 8.30 (m, 1H), 8.27 (br dd, J = 3.4, 7.7 Hz, 2H), 8.18 - 8.06 (m, 1H), 7.61 - 7.46 (m, 2H), 7.29 - 7.14 (m, 2H), 6.78 - 6.67 (m, 1H), 4.94 - 4.54 (m, 1H), 4.31 - 4.12 (m, 3H), 3.86 - 3.67 (m, 3H), 3.54 - 3.46 (m, 2H), 2.98 - 2.89 (m, 2H), 2.83 - 2.68 (m, 4H), 2.62 - 2.57 (m, 1H), 2.30 - 2.21 (m, 1H), 2.10 - 2.03 (m, 3H), 1.98 - 1.90 (m, 4H), 1.69 - 1.40 (m, 1H).

### [Preparation Example 44]

### Preparation of Compounds 204 to 205

### 44-1. Preparation of Compound 204, 2-((2-((2,2'-dichloro-3'-(3-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid

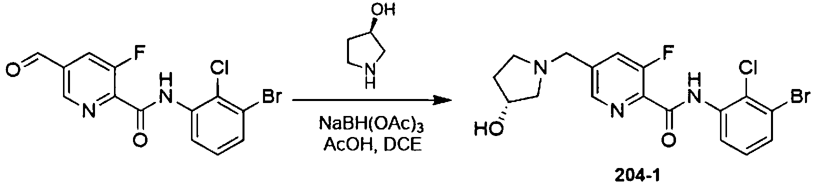

To a solution of N-(3-bromo-2-chloro-phenyl)-3-fluoro-5-formyl-pyridine-2-carboxamide (900 mg, 2.52 mmol, 1 eq) and (3R)-pyrrolidin-3-ol (1.10 g, 12.59 mmol, 1.05 mL, 5 eq) in DCE (20 mL), NaBH(OAc)₃ (1.60 g, 7.55 mmol, 3 eq), and AcOH (755.74 mg, 12.59 mmol, 720.44 µL, 5 eq) were added, and the reaction mixture was stirred at room temperature for 4 hours. After completion of the reaction, the mixture was filtered and the filtrate was concentrated under reduced pressure. The residue was purified by reversed-phase HPLC (column: Kromasil Eternity XT, 250 * 80 mm * 10 µm; mobile phase: [water (NH₄HCO₃)-ACN]; gradient: 40% to 70% B over 20 min) to afford Compound 204-1 (327 mg, 27.2% yield) as a white solid.

LCMS m/z 427.01 [M+3]⁺.

¹H NMR (400 MHz, DMSO-d6) δ ppm 1.91 (s, 3 H) 2.33 (dd, J=9.69, 3.44 Hz, 1 H) 3.61 - 3.73 (m, 2 H) 3.96 (q, J=7.13 Hz, 4 H) 7.28 (t, J=8.13 Hz, 1 H) 7.52 (dd, J=8.00, 1.25 Hz, 1 H) 7.78 (d, J=11.88 Hz, 1 H) 8.20 (d, J=7.50 Hz, 1 H) 8.45 (s, 1 H) 10.51 (s, 1 H).

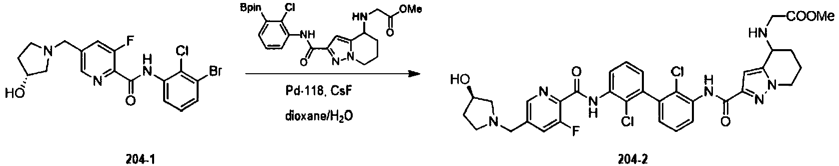

A mixture of N-(3-bromo-2-chloro-phenyl)-3-fluoro-5-[(3R)-3-hydroxypyrrolidin-1-ylmethyl]pyridine-2-carboxamide (100 mg, 233.27 µmol, 1 eq), methyl 2-[[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (114.02 mg, 233.27 µmol, 1 eq), CsF (70.87 mg, 466.55 µmol, 17.22 µL, 2 eq), and ditert-butyl(cyclopentyl)phosphane;dichloropalladium iron (15.20 mg, 23.33 µmol, 0.1 eq) in dioxane (3 mL) / H₂O (0.5 mL) was stirred at 70 °C for 12 hours under a nitrogen atmosphere. After completion of the reaction, the mixture was filtered and the filtrate was concentrated under reduced pressure. The residue was purified by prep-TLC (SiO₂, Ethyl acetate: Methanol = 5:1) to afford Compound 204-2 (110 mg, 51.7% yield) as a black solid.

LCMS m/z 709.2 [M+1]⁺.

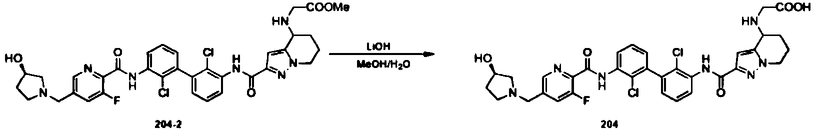

To a solution of methyl 2-[[2-[[2-chloro-3-[2-chloro-3-[[3-fluoro-5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]amino]acetate (110 mg, 154.80 µmol, 1 eq) in MeOH (2 mL) / H₂O (2 mL), LiOH (19.49 mg, 464.41 µmol, 3 eq) was added, and the reaction mixture was stirred at room temperature for 12 hours. After completion of the reaction, the mixture was concentrated under reduced pressure. The residue was purified by reversed-phase HPLC (column: Waters Xbridge 150 * 25 mm * 5 µm; mobile phase: [water (NH₄HCO₃)-CAN]; gradient: 26% - 56% B over 9 min) to afford Compound 204 (28 mg, 24.9% yield) as a yellow solid.

LCMS m/z 695.18 [M+1]⁺.

¹H NMR (400 MHz, DMSO-d6) δ ppm 1.97 (br s, 4 H) 2.15 (br s, 4 H) 3.73 (br s, 2 H) 4.20 (br s, 2 H) 4.43 (br s, 4 H) 5.29 - 5.58 (m, 1 H) 7.04 (s, 1 H) 7.22 (ddd, *J* =9.60, 7.85, 1.44 Hz, 2 H) 7.49 - 7.57 (m, 2 H) 8.22 (br d, *J* =8.13 Hz, 2 H) 8.31 (br d, *J* =8.13 Hz, 1 H) 8.75 (br s, 1 H) 9.64 (s, 1 H) 10.58 (s, 1 H).

### 44-2. Preparation of Compound 205, (3R)-1-(2-((2,2'-dichloro-3'-(3-fluoro-5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

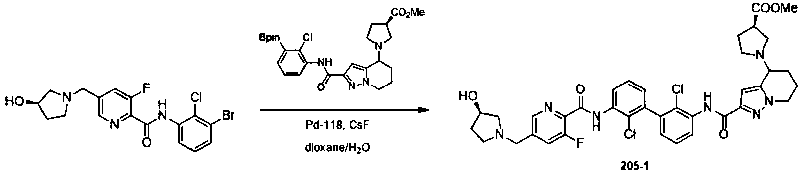

A mixture of N-(3-bromo-2-chloro-phenyl)-3-fluoro-5-[[(3R)-3-hydroxypyrrolidin-1-yl]methyl]pyridine-2-carboxamide (100 mg, 233.27 µmol, 1 eq) and methyl (3R)-1-[2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (370.09 mg, 699.82 µmol, 3 eq), CsF (70.87 mg, 466.55 µmol, 17.22 µL, 2 eq), and ditert-butyl(cyclopentyl)phosphane;dichloropalladium iron (15.20 mg, 23.33 µmol, 0.1 eq) in dioxane (4 mL) / water (1 mL) was stirred at 70 °C for 12 hours under a nitrogen atmosphere. After completion of the reaction, the mixture was filtered, and the filtrate was concentrated under reduced pressure. The residue was purified by prep-TLC (SiO₂, Ethyl acetate:Methanol = 3:1) to afford Compound 205-1 (160 mg, 82.2% yield) as a brown solid.

LCMS m/z 749.23 [M+1]⁺.

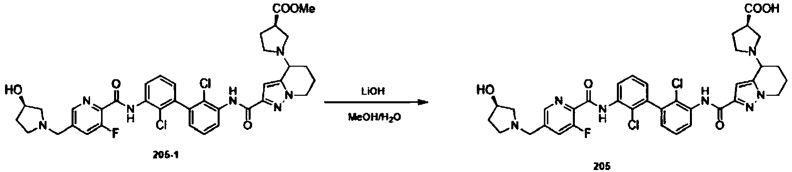

To a solution of methyl (3R)-1-[2-[[2-chloro-3-[2-chloro-3-[[3-fluoro-5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]pyrrolidine-3-carboxylate (160 mg, 213.15 µmol, 1 eq) in H₂O (2 mL) /MeOH (2 mL), LiOH (26.83 mg, 639.45 µmol, 3 eq) was added, and the reaction mixture was stirred at room temperature for 12 hours. After completion of the reaction, the mixture was concentrated under reduced pressure. The residue was purified by reversed-phase HPLC (column: Phenomenex Luna C18 150 * 25 mm * 10 µm, mobile phase: [water (HCl) -CAN]; gradient: 5% - 35% B over 10 min) to afford Compound 205 (20 mg, 12.3% yield) as a white solid.

LCMS m/z 735.2 [M+1]⁺.

¹H NMR (400 MHz, DMSO-d6) δ ppm 1.53 - 1.62 (m, 1 H) 1.89 - 2.07 (m, 6 H) 2.32 - 2.40 (m, 2 H) 2.58 - 2.75 (m, 6 H) 2.92 (br d, *J* =3.50 Hz, 2 H) 3.69 - 3.80 (m, 2 H) 4.14 - 4.24 (m, 3 H) 4.74 (br s, 1 H) 6.73 (d, *J* =4.75 Hz, 1 H) 7.19 (t, *J*=8.04 Hz, 2 H) 7.48 - 7.56 (m, 2 H) 7.89 (d, *J* =11.63 Hz, 1 H) 8.25 - 8.29 (m, 1 H) 8.39 (d, *J* =9.13 Hz, 1 H) 8.54 (s, 1 H) 9.57 (s, 1 H) 10.58 (s, 1 H).

### [Preparation Example 45]

### Preparation of Compounds 206 to 207

### 45-1. Preparation of Compound 206, (S)-N-(2,2'-dichloro-3'-(5-(((2-hydroxyethyl)amino)methyl)picolinamido)-[1,1'-biphenyl]-3-yl)-4-((2-hydroxyethyl)amino)-4,5,6,7-tetrahydropyrazolo[1,5-a]pyridine-2-carboxamide

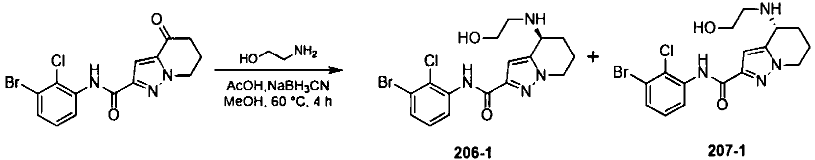

A solution of N-(3-bromo-2-chloro-phenyl)-4-oxo-6,7-dihydro-5H-pyrazolo[1,5-a]pyridine-2-carboxamide (1.0 g, 2.71 mmol, 1 eq), 2-aminoethanol (1.66 g, 27.13 mmol, 1.64 mL, 10 eq), and HOAc (1.63 g, 27.13 mmol, 1.55 mL, 10 eq) in MeOH (30 mL) was stirred at 60 °C for 2 hours, and then NaBH₃CN (511.43 mg, 8.14 mmol, 3 eq) was added. The mixture was further stirred at 60 °C for 2 hours. After completion of the reaction, the mixture was filtered and the filtrate was concentrated under reduced pressure. The residue was purified by prep-HPLC (column: Waters Xbridge 150 * 25 mm * 5 µm; mobile phase: [water (NH₄HCO₃)-CAN]; gradient: 30% - 60% B over 9 min) to afford N-(3-bromo-2-chloro-phenyl)-4-((2-hydroxyethyl)amino)-4,5,6,7-tetrahydropyrazolo[1,5-a]pyridine-2-carboxamide (400 mg, 90% yield) as a white solid. The obtained product was further separated by SFC (column: DAICEL CHIRALPAK AS (250 * 30 mm * 10 µm); mobile phase: [CO₂-EtOH (0.1% NH₃H₂O)]; B%: 40%, isocratic elution ode) to afford Compound 206-1 (156 mg, 13.2% yield) and Compound 207-1 (162 mg, 13.7% yield), each as a white solid.

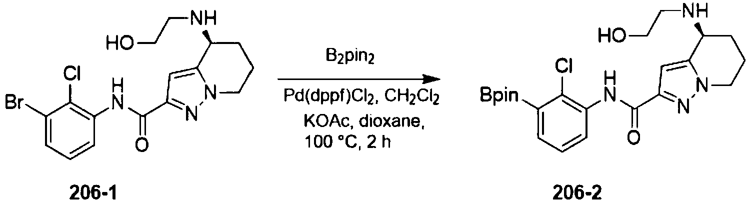

A mixture of (4S)-N-(3-bromo-2-chloro-phenyl)-4-(2-hydroxyethylamino)-4,5,6,7-tetrahydropyrazolo[1,5-a]pyridine-2-carboxamide (150.0 mg, 362.59 µmol, 1 eq), 4,4,5,5-tetramethyl-2-(4,4,5,5-tetramethyl-1,3,2-dioxaborolan-2-yl)-1,3,2-dioxaborolane (276.22 mg, 1.09 mmol, 3 eq), KOAc (106.75 mg, 1.09 mmol, 3 eq), and Pd(dppf)Cl₂·CH₂Cl₂ (29.61 mg, 36.26 µmol, 0.1 eq) in dioxane (5 mL) was stirred at 100 °C for 2 hours under a nitrogen atmosphere. After completion of the reaction, water (40 mL) was added and the mixture was extracted with ethyl acetate (40 mL x 3). The combined organic layers were washed with brine (60 mL), dried over Na₂SO₄, filtered, and concentrated under reduced pressure. The resulting residue was purified by flash silica gel chromatography (petroleum ether/ethyl acetate = 100:1 to 1:1) to afford Compound 206-2 (136 mg, 73.2% yield) as a white solid.

LCMS m/z 461.4 [M+1]⁺.

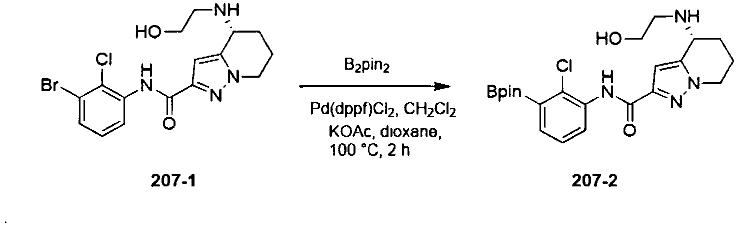

A mixture of (4R)-N-(3-bromo-2-chloro-phenyl)-4-(2-hydroxyethylamino)-4,5,6,7-tetrahydropyrazolo[1,5-a]pyridine-2-carboxamide (150.0 mg, 362.59 µmol, 1 eq), 4,4,5,5-tetramethyl-2-(4,4,5,5-tetramethyl-1,3,2-dioxaborolan-2-yl)-1,3,2-dioxaborolane (276.22 mg, 1.09 mmol, 3 eq), KOAc (71.17 mg, 725.17 µmol, 2 eq), and Pd(dppf)Cl₂·CH₂Cl₂ (29.61 mg, 36.26 µmol, 0.1 eq) in dioxane (5 mL) was stirred at 100 °C for 2 hours under a nitrogen atmosphere. After completion of the reaction, water (40 mL) was added, and the mixture was extracted with ethyl acetate (40 mL x 3). The combined organic layers were washed with brine (60 mL), dried over Na₂SO₄, filtered, and concentrated under reduced pressure. The resulting residue was purified by prep-TLC (petroleum ether/ethyl acetate = 1:1) to afford Compound 207-2 (168 mg, 95.5% yield) as a white solid.

LCMS m/z 461.4 [M+1]⁺.

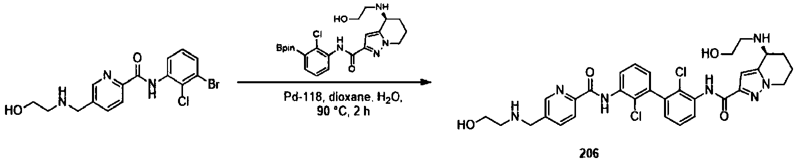

A mixture of N-(3-bromo-2-chloro-phenyl)-5-[(2-hydroxyethylamino)methyl]pyridine-2-carboxamide (100 mg, 259.97 µmol, 1 eq), (4S)-N-[2-chloro-3-(4,4,5,5-tetramethyl-1,3,2-dioxaborolan-2-yl)phenyl]-4-(2-hydroxyethylamino)-4,5,6,7-tetrahydropyrazolo[1,5-a]pyridine-2-carboxamide (119.79 mg, 259.97 µmol, 1 eq), K₂CO₃ (100.61 mg, 727.93 µmol, 2.8 eq), and ditert-butyl(cyclopentyl)phosphane;dichloropalladium iron (16.94 mg, 26.00 µmol, 0.1 eq) in dioxane (3 mL) / water (0.5 mL) was stirred at 90 °C for 2 hours under a nitrogen atmosphere. After completion of the reaction, water (10 mL) was added, and the mixture was extracted with ethyl acetate (10 mL X 3). The combined organic layers were washed with brine (20 mL), dried over Na₂SO₄, filtered, and concentrated under reduced pressure. The resulting residue was purified by prep-HPLC (column: Waters Xbridge (150 * 25 mm * 5 µm) with a mobile phase: [water (NH₄HCO₃)-ACN]; gradient: 28%-58% B over 9 min) to afford Compound 206 (16 mg, 9.16% yield) as a white solid.

LCMS m/z 638.5 [M+1]⁺.

¹H NMR (400 MHz, DMSO-d6 ) δ = 10.73 (s, 1H), 9.55 (s, 1H), 8.70 (s, 1H), 8.53 (d, J = 8.4 Hz, 1H), 8.30 (br d, J = 8.1 Hz, 1H), 8.19 (d, J = 7.8 Hz, 1H), 8.06 (br d, J = 7.6 Hz, 1H), 7.57 - 7.47 (m, 2H), 7.19 (br d, J = 7.6 Hz, 2H), 6.79 (s, 1H), 4.54 - 4.48 (m, 2H), 4.15 (br s, 2H), 3.86 (s, 2H), 3.48 (br d, J = 5.6 Hz, 4H), 2.68 (br s, 2H), 2.61 - 2.56 (m, 2H), 2.34 (br s, 1H), 2.18 (br s, 2H), 2.02 (br s, 1H), 1.92 (br s, 1H), 1.68 (br s, 1H).

### 45-2. Preparation of Compound 207, (R)-N-(2,2'-dichloro-3'-(5-(((2-hydroxyethyl)amino)methyl)picolinamido)-[1,1'-biphenyl]-3-yl)-4-((2-hydroxyethyl)amino)-4,5,6,7-tetrahydropyrazolo[1,5-a]pyridine-2-carboxamide

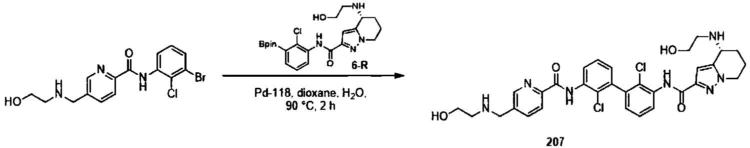

A mixture of N-(3-bromo-2-chloro-phenyl)-5-[(2-hydroxyethylamino)methyl]pyridine-2-carboxamide (100 mg, 259.97 µmol, 1 eq), (4R)-N-[2-chloro-3-(4,4,5,5-tetramethyl-1,3,2-dioxaborolan-2-yl)phenyl]-4-(2-hydroxyethylamino)-4,5,6,7-tetrahydropyrazolo[1,5-a]pyridine-2-carboxamide (119.79 mg, 259.97 µmol, 1 eq), K₂CO₃ (100.61 mg, 727.93 µmol, 2.8 eq), and ditert-butyl(cyclopentyl)phosphane;dichloropalladium iron (16.94 mg, 26.00 µmol, 0.1 eq) in dioxane (3 mL) /water (0.5 mL) was stirred at 90 °C for 2 hours under a nitrogen atmosphere. After completion of the reaction, water (10 mL) was added, and the mixture was extracted with ethyl acetate (10 mL * 3). The combined organic layers were washed with brine (20 mL), dried over Na₂SO₄, filtered, and concentrated under reduced pressure. The resulting residue was purified by prep-HPLC (column: Waters Xbridge 150 * 25 mm * 5 µm: mobile phase: [water (NH₄HCO₃)-ACN]; gradient: 25%-55% B over 9 min) to afford Compound 207 (12 mg, 6.86% yield) as a white solid.

LCMS m/z 638.5 [M+1]⁺.

¹H NMR (400 MHz, DMSO-d6 ) δ = 10.73 (s, 1H), 9.55 (s, 1H), 8.70 (s, 1H), 8.54 (br d, J = 8.3 Hz, 1H), 8.30 (br d, J = 8.3 Hz, 1H), 8.19 (br d, J = 7.8 Hz, 1H), 8.06 (br d, J = 7.9 Hz, 1H), 7.61 - 7.44 (m, 2H), 7.19 (br d, J = 7.0 Hz, 2H), 6.79 (s, 1H), 4.51 (br d, J = 6.8 Hz, 2H), 4.15 (br s, 2H), 3.89 - 3.80 (m, 2H), 3.52 - 3.43 (m, 4H), 2.68 (br s, 3H), 2.62 - 2.54 (m, 2H), 2.18 (br s, 2H), 2.02 (br s, 1H), 1.92 (br s, 1H), 1.70 (br s, 1H).

### [Preparation Example 46]

### Preparation of Compound 208, (R)-1-((S)-2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid, and Compounds 209, (R)-1-((R)-2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylic acid

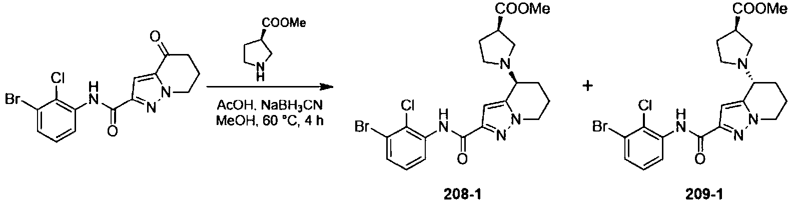

A solution of N-(3-bromo-2-chloro-phenyl)-4-oxo-6,7-dihydro-5H-pyrazolo[1,5-a]pyridine-2-carboxamide (1 g, 2.71 mmol, 1 eq), methyl (3R)-pyrrolidine-3-carboxylate (1.05 g, 8.14 mmol, 3 eq), HOAc (1.63 g, 27.13 mmol, 1.55 mL, 10 eq), and DIPEA (1.05 g, 8.14 mmol, 1.42 mL, 3 eq) in MeOH (40 mL) was stirred at 60 °C for 2 hours. NaBH₃CN (511.43 mg, 8.14 mmol, 3 eq) was then added, and the reaction mixture was further stirred at 60 °C for 2 hours. The mixture was filtered, and the filtrate was concentrated under reduced pressure. The residue was purified by prep-HPLC (column: Phenomenex Luna C18, 250 * 70 mm * 10 µm; mobile phase: [water (NH₄HCO₃)-CAN]; gradient: 55-85% B over 20 min) to afford (3R)-methyl 1-(2-((3-bromo-2-chlorophenyl)carbamoyl)-4,5,6,7-tetrahydropyrazolo[1,5-a]pyridin-4-yl)pyrrolidine-3-carboxylate (1.2 g, 94% yield) as a white solid. The product was further separated by SFC (column: Phenomenex Cellulose-2, 250 * 30 mm * 10 µm; mobile phase: [CO₂-EtOH(0.1% NH₃·H₂O)]; B = 55%, isocratic elution mode) to give Compound 208-1 (520 mg, 37.8% yield) and Compound 209-1 (518 mg, 37.6% yield), each obtained as a yellow oil.

LCMS m/z 483.1 [M+1]⁺.

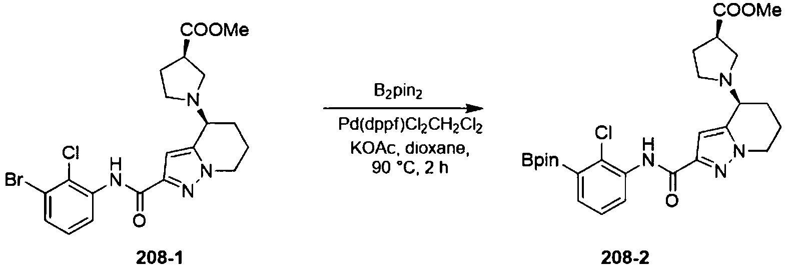

A mixture of methyl (3R)-1-[(4S)-2-[(3-bromo-2-chlorophenyl)carbamoyl]-4,5,6,7-tetrahydropyrazolo[1,5-a]pyridin-4-yl]pyrrolidine-3-carboxylate (450 mg, 934.06 µmol, 1 eq), 4,4,5,5-tetramethyl-2-(4,4,5,5-tetramethyl-1,3,2-dioxaborolan-2-yl)-1,3,2-dioxaborolane (711.58 mg, 2.80 mmol, 3 eq), KOAc (275.00 mg, 2.80 mmol, 3 eq), and Pd(dppf)Cl₂·CH₂Cl₂ (76.28 mg, 93.41 µmol, 0.1 eq) in dioxane (10 mL) was stirred at 100 °C for 2 hours under a nitrogen atmosphere. After completion of the reaction, the mixture was filtered, and the filtrate was concentrated under reduced pressure. The residue was purified by flash silica gel chromatography (Petroleum ether/Ethyl acetate = 100/1 to 1/1) to afford Compound 208-2 (482 mg, 87.8% yield) as a yellow solid.

LCMS m/z 529.1 [M+1]⁺.

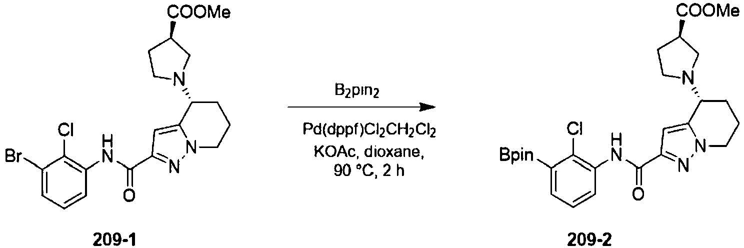

A mixture of methyl (3R)-1-[(4R)-2-[(3-bromo-2-chlorophenyl)carbamoyl]-4,5,6,7-tetrahydropyrazolo[1,5-a]pyridin-4-yl]pyrrolidine-3-carboxylate (450 mg, 934.06 µmol, 1 eq), 4,4,5,5-tetramethyl-2-(4,4,5,5-tetramethyl-1,3,2-dioxaborolan-2-yl)-1,3,2-dioxaborolane (711.58 mg, 2.80 mmol, 3 eq), KOAc (183.34 mg, 1.87 mmol, 2 eq), and Pd(dppf)Cl₂·CH₂Cl₂ (76.28 mg, 93.41 µmol, 0.1 eq) in dioxane (10 mL) was stirred at 100 °C for 2 hours under a nitrogen atmosphere. After completion of the reaction, the mixture was filtered, and the filtrate was concentrated under reduced pressure. The residue was purified by flash silica gel chromatography (Petroleum ether/Ethyl acetate = 100/1 to 1/1) to afford Compound 208-2 (482 mg, 87.8% yield) as a white solid.

LCMS m/z 529.1 [M+1]⁺.

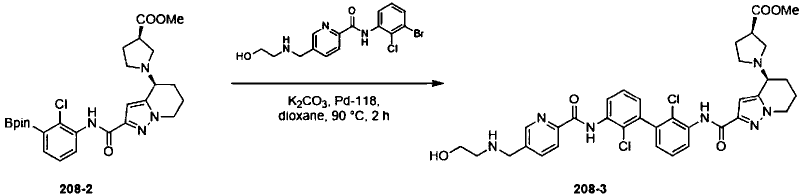

A mixture of N-(3-bromo-2-chloro-phenyl)-5-[(2-hydroxyethylamino)methyl]pyridine-2-carboxamide (130 mg, 337.97 µmol, 1 eq), methyl (3R)-1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (232.35 mg, 439.35 µmol, 1.3 eq), K₂CO₃ (K₂CO₃, 130.79 mg, 946.30 µmol, 2.8 eq), and ditert-butyl(cyclopentyl)phosphane;dichloropalladium iron (22.03 mg, 33.80 µmol, 0.1 eq) in dioxane (6 mL) / water (0.5 mL) was stirred at 90 °C for 2 hours under a nitrogen atmosphere. After completion of the reaction, the mixture was filtered and the filtrate was concentrated under reduced pressure. The residue was purified by prep-HPLC (column: Phenomenex Luna C18, 150 * 40 mm * 15 µm; mobile phase: [water (FA)-ACN]; gradient: 5% to 35% B over 15 min) to afford Compound 208-3 (62 mg, 24.6% yield) as a white solid.

LCMS m/z 706.4 [M+1]⁺.

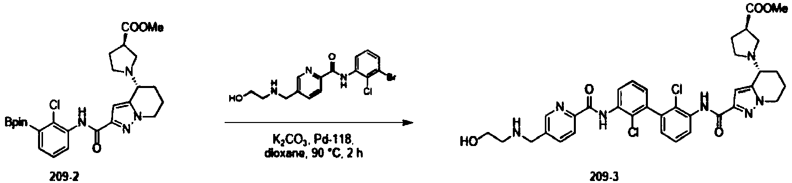

A mixture of N-(3-bromo-2-chloro-phenyl)-5-[(2-hydroxyethylamino)methyl]pyridine-2-carboxamide (150 mg, 389.96 µmol, 1 eq), methyl (3R)-1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (309.34 mg, 584.94 µmol, 1.5 eq), K₂CO₃ (150.91 mg, 1.09 mmol, 2.8 eq), and ditert-butyl(cyclopentyl)phosphane;dichloropalladium iron (25.42 mg, 39.00 µmol, 0.1 eq) in dioxane (6 mL) / water (0.5 mL) was stirred at 90 °C for 2 hours under a nitrogen atmosphere. After completion of the reaction, the mixture was filtered and the filtrate was concentrated under reduced pressure. The residue was purified by prep-HPLC (column: Phenomenex Luna C18, 150 * 25 mm * 10 µm; mobile phase: [water (FA)-ACN]; gradient: 15% to 45% B over 7 min) to afford Compound 209-3 (92 mg, 30.5% yield) as a white solid.

LCMS m/z 708.3 [M+1]⁺.

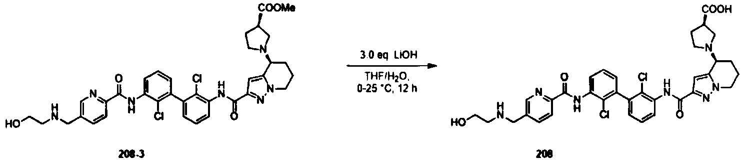

To a solution of methyl (3R)-1-[(4S)-2-[[2-chloro-3-[2-chloro-3-[[5-[(2-hydroxyethylamino)methyl]pyridine-2-carbonyl]amino]phenyl]phenyl]carbamoyl]-4,5,6,7-tetrahydropyrazolo[1,5-a]pyridin-4-yl]pyrrolidine-3-carboxylate 7-S (60 mg, 84.91 µmol, 1 eq) in THF (3 mL) / water (1 mL), LiOH·H₂O (8.91 mg, 212.28 µmol, 2.5 eq) was added at 0 °C, and the reaction mixture was stirred at room temperature for 12 hours. After completion of the reaction, the mixture was concentrated under reduced pressure. The residue was purified by prep-HPLC (column: Waters XBridge, 150 * 25 mm * 5 µm; mobile phase: [water (NH₄HCO₃)-ACN]; gradient: 15% to 45% B over 9 min) to afford Compound 208 (32 mg, 51.9% yield) as a white solid.

LCMS m/z 694.3 [M+1]⁺.

¹H NMR (400 MHz, DMSO-d6 ) δ = 10.73 (s, 1H), 9.65 - 9.47 (m, 1H), 8.70 (s, 1H), 8.53 (d, J = 8.3 Hz, 1H), 8.35 - 8.25 (m, 1H), 8.18 (d, J = 8.1 Hz, 1H), 8.05 (dd, J = 1.6, 8.1 Hz, 1H), 7.52 (td, J = 8.0, 15.9 Hz, 2H), 7.18 (d, J = 7.5 Hz, 2H), 6.71 (s, 1H), 4.51 (dt, J = 2.9, 5.8 Hz, 1H), 4.36 (br s, 3H), 4.15 (br dd, J = 6.3, 13.0 Hz, 2H), 3.86 (s, 2H), 3.50 - 3.47 (m, 3H), 2.98 - 2.85 (m, 2H), 2.76 - 2.67 (m, 2H), 2.57 (br t, J = 5.8 Hz, 4H), 1.97 - 1.88 (m, 4H).

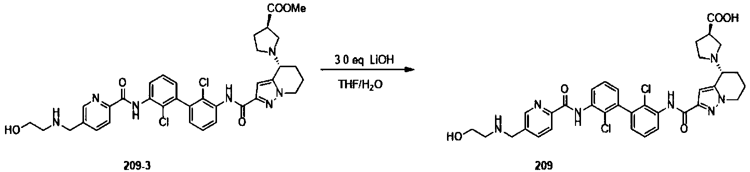

To a solution of methyl (3R)-1-[(4R)-2-[[2-chloro-3-[2-chloro-3-[[5-[(2-hydroxyethylamino)methyl]pyridine-2-carbonyl]amino]phenyl]phenyl]carbamoyl]-4,5,6,7-tetrahydropyrazolo[1,5-a]pyridin-4-yl]pyrrolidine-3-carboxylate (60.00 mg, 84.91 µmol, 1 eq) in THF (3 mL) / water (1 mL), LiOH·H₂O (8.91 mg, 212.28 µmol, 2.5 eq) was added at 0 °C, and the reaction mixture was stirred at room temperature for 12 hours. After completion of the reaction, the mixture was concentrated under reduced pressure. The residue was purified by prep-TLC (dichloromethane/methanol = 10/1) to afford Compound 209 (27 mg, 43.6% yield) as a white solid.

LCMS m/z 694.3 [M+1]⁺.

¹H NMR (400 MHz, DMSO-d6 ) δ = 10.73 (s, 1H), 9.57 (s, 1H), 8.70 (s, 1H), 8.52 (dd, J = 1.4, 8.4 Hz, 1H), 8.32 - 8.24 (m, 1H), 8.19 (d, J = 7.9 Hz, 1H), 8.11 - 7.98 (m, 1H), 7.52 (td, J = 7.9, 15.9 Hz, 2H), 7.18 (d, J = 7.6 Hz, 2H), 6.72 (s, 1H), 4.37 (br s, 3H), 4.27 - 4.10 (m, 3H), 3.87 (s, 2H), 2.97 - 2.87 (m, 3H), 2.73 - 2.67 (m, 2H), 2.58 (br t, J = 5.6 Hz, 4H), 2.38 - 2.15 (m, 2H), 1.98 - 1.89 (m, 4H).

### [Preparation Example 47]

### Preparation of Compound 210, (R)-1-((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid, and Compounds 211, (R)-1-((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylic acid

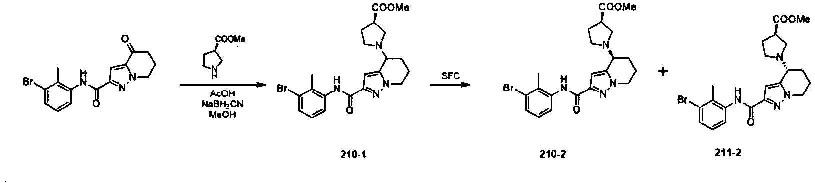

A solution of N-(3-bromo-2-methylphenyl)-4-oxo-6,7-dihydro-5H-pyrazolo[1,5-a]pyridine-2-carboxamide (2.0 g, 5.74 mmol, 1 eq), methyl (3R)-pyrrolidine-3-carboxylate (3.71 g, 28.72 mmol, 5 eq), HOAc (3.45 g, 57.44 mmol, 3.29 mL, 10 eq), and DIPEA (3.71 g, 28.72 mmol, 5.00 mL, 5 eq) in MeOH (60 mL) was stirred at 60 °C for 2 hours. NaBH₃CN (1.08 g, 17.23 mmol, 3 eq) was then added, and the reaction mixture was stirred at 60 °C for an additional 2 hours. After completion of the reaction, the mixture was filtered, and the filtrate was concentrated under reduced pressure. The residue was purified by prep-HPLC (column: Phenomenex Luna C18, 250 * 70 mm * 10 µm; mobile phase: [water (NH₄HCO₃)-ACN]; gradient: 45%-75% B over 20 min) to afford Compound 210-1 (1.32 g, 95% yield) as a white solid. The product was further separated by SFC (column: REGIS (S,S) Whelk-O1, 250 * 25 mm * 10 µm; mobile phase: CO₂-i-PrOH (0.1% NH₃·H₂O); B%: 50%, isocratic elution) to give Compound 210-2 (722 mg, 25.8% yield) and Compound 211-2 (760 mg, 27.2% yield), each as a white solid.

LCMS 463.2 [M+1]⁺.

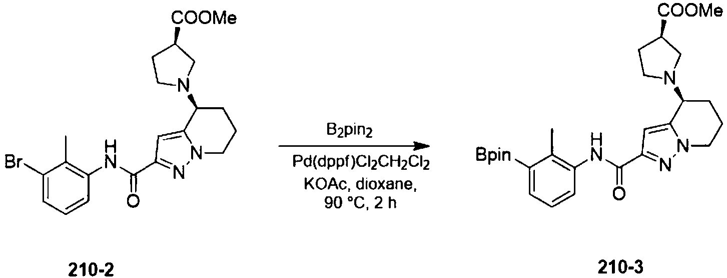

A mixture of methyl (3R)-1-[(4S)-2-[(3-bromo-2-methylphenyl)carbamoyl]-4,5,6,7-tetrahydropyrazolo[1,5-a]pyridin-4-yl]pyrrolidine-3-carboxylate (300 mg, 650.26 µmol, 1 eq), 4,4,5,5-tetramethyl-2-(4,4,5,5-tetramethyl-1,3,2-dioxaborolan-2-yl)-1,3,2-dioxaborolane (495.38 mg, 1.95 mmol, 3 eq), KOAc (191.45 mg, 1.95 mmol, 3 eq), and Pd(dppf)Cl₂·CH₂Cl₂ (53.10 mg, 65.03 µmol, 0.1 eq) in dioxane (8 mL) was stirred at 90 °C for 2 hours under nitrogen atmosphere. After completion of the reaction, the mixture was filtered, and the filtrate was concentrated under reduced pressure. The residue was purified by flash silica gel chromatography (dichloromethane/methanol = 10:1) to afford Compound 210-3 (312 mg, 84.9% yield) as a white solid.

LCMS m/z 509.4 [M+1]⁺.

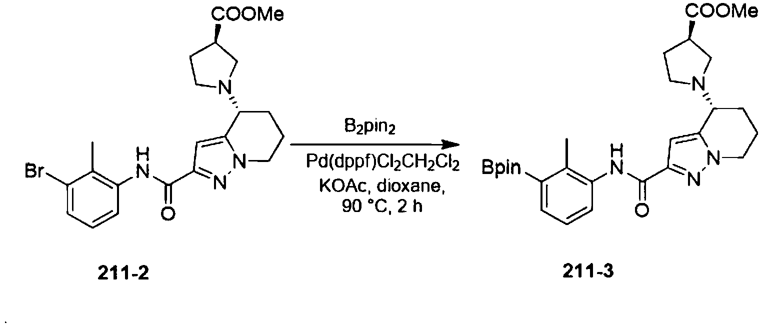

A mixture of methyl (3R)-1-[(4S)-2-[(3-bromo-2-methylphenyl)carbamoyl]-4,5,6,7-tetrahydropyrazolo[1,5-a]pyridin-4-yl]pyrrolidine-3-carboxylate (300 mg, 650.26 µmol, 1 eq), 4,4,5,5-tetramethyl-2-(4,4,5,5-tetramethyl-1,3,2-dioxaborolan-2-yl)-1,3,2-dioxaborolane (495.38 mg, 1.95 mmol, 3 eq), KOAc (191.45 mg, 1.95 mmol, 3 eq), and Pd(dppf)Cl₂·CH₂Cl₂ (53.10 mg, 65.03 µmol, 0.1 eq) in dioxane (3 mL) was stirred at 90 °C for 2 hours under a nitrogen atmosphere. After completion of the reaction, the mixture was filtered, and the filtrate was concentrated under reduced pressure. The residue was purified by prep-TLC (dichloromethane/methanol = 10:1) to afford Compound 211-3 (280 mg, 76.2% yield) as a white solid.

LCMS m/z 509.4 [M+1]⁺.

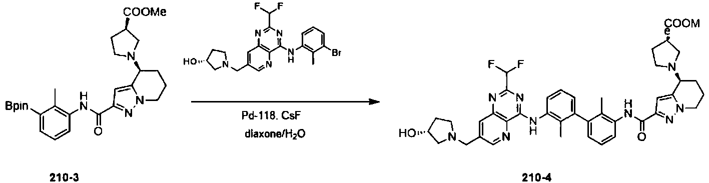

A mixture of (3R)-1-[[4-(3-bromo-2-methyl-anilino)-2-(difluoromethyl)pyrido[3,2-d]pyrimidin-7-yl]methyl]pyrrolidin-3-ol (80 mg, 172.30 µmol, 1 eq), methyl (3R)-1-[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (105.12 mg, 206.76 µmol, 1.2 eq), CsF (78.52 mg, 516.90 µol, 1.2 eq), , 3 eq), ditert-butyl(cyclopentyl)phosphane;dichloropalladium;iron (11.23 mg, 17.23 µmol, 0.1 eq) in dioxane (3 mL) / H₂O (0.5 mL) was stirred at 90 °C for 2 hours under a nitrogen atmosphere. After completion of the reaction, the mixture was filtered and the filtrate was concentrated under reduced pressure. The residue was purified by prep-HPLC (column: Phenomenex luna C18 150*40mm* 15µm; mobile phase: [water (FA)-ACN]; gradient: 5%-35% B over 15 min) to afford Compound 210-4 (82 mg, 59.0% yield) as a white solid.

LCMS m/z 766.5 [M+1]⁺.

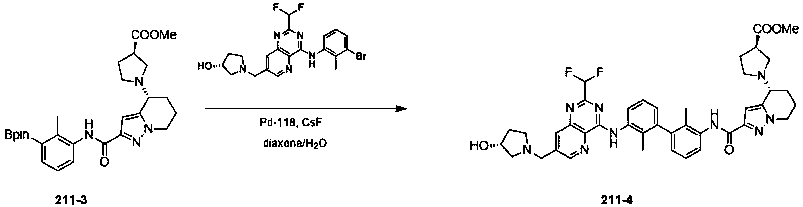

A mixture of (3R)-1-[[4-(3-bromo-2-methyl-anilino)-2-(difluoromethyl)pyrido[3,2-d]pyrimidin-7-yl]methyl]pyrrolidin-3-ol (80 mg, 172.30 µmol, 1 eq), methyl (3R)-1-[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (105.12 mg, 206.76 µmol, 1.2 eq), CsF (CsF, 78.52 mg, 516.90 µmol, 3 eq), and di-tert-butyl(cyclopentyl)phosphane;dichloropalladium;iron (11.23 mg, 17.23 µmol, 0.1 eq) in dioxane (3 mL) / water (0.5 mL) was stirred at 90 °C for 2 hours under a nitrogen atmosphere. After completion of the reaction, the mixture was filtered and the filtrate was concentrated under reduced pressure. The residue was purified by prep-HPLC (column: Phenomenex Luna C18, 150 * 40 mm * 15 µm; mobile phase: [water (FA)-ACN]; gradient: 5-35% B over 15 min) to afford Compound 211-4 (86 mg, 61.9% yield) as a white solid.

LCMS m/z 766.5 [M+1]⁺.

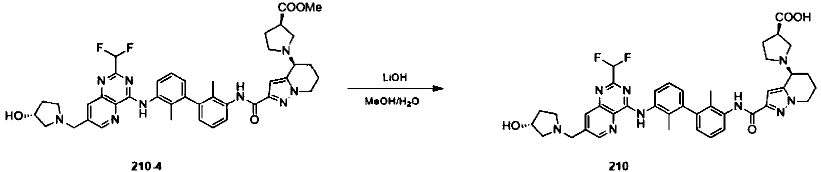

To a mixture of methyl (3R)-1-[(4S)-2-[[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]carbamoyl]-4,5,6,7-tetrahydropyrazolo[1,5-a]pyridin-4-yl]pyrrolidine-3-carboxylate (70 mg, 91.40 µmol, 1 eq) in THF (10 mL) / water (3 mL), LiOH·H₂O (11.51 mg, 274.21 µmol, 3 eq) was added at 0 °C, and the reaction mixture was stirred at room temperature for 12 hours. After completion of the reaction, the mixture was concentrated under reduced pressure. The residue was purified by prep-HPLC (column: Waters XBridge, 150 * 25 mm * 5 µm; mobile phase: [water (NH₄HCO₃)-ACN]; gradient: 18-48% B over 9 min) to afford Compound 210 (63 mg, 79.61 µmol, 87.10% yield, 95% purity) as a white solid.

LCMS m/z 752.5 [M+1]⁺.

¹H NMR (400 MHz, DMSO-d6 ) δ = 10.49 (br s, 1H), 9.56 (br s, 1H), 9.10 (br s, 1H), 8.55 - 8.35 (m, 1H), 7.59 (br dd, J = 7.9, 24.1 Hz, 2H), 7.32 (td, J = 7.8, 27.9 Hz, 2H), 7.05 (dd, J = 7.2, 25.6 Hz, 2H), 6.89 - 6.53 (m, 2H), 5.47 - 4.96 (m, 1H), 4.48 - 4.30 (m, 2H), 4.27 - 4.07 (m, 3H), 4.01 - 3.76 (m, 2H), 3.54 - 3.47 (m, 3H), 3.17 (br s, 2H), 3.06 - 2.87 (m, 4H), 2.82 - 2.60 (m, 3H), 2.38 - 2.22 (m, 1H), 2.21 - 2.05 (m, 1H), 1.99 (s, 4H), 1.94 (s, 4H), 1.88 - 1.69 (m, 1H).

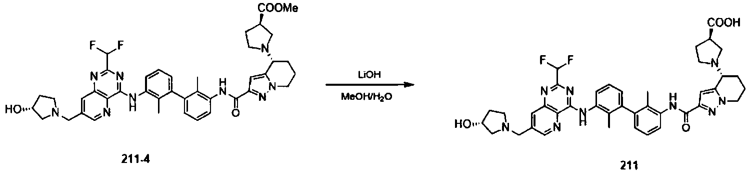

To a mixture of methyl (3R)-1-[(4R)-2-[[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]carbamoyl]-4,5,6,7-tetrahydropyrazolo[1,5-a]pyridin-4-yl]pyrrolidine-3-carboxylate (70.00 mg, 91.40 µmol, 1 eq) in THF (10 mL) / H₂O (3 mL), LiOH H₂O (11.51 mg, 274.21 µmol, 3 eq) was added at 0 C, and the reaction mixture was stirred at room temperature for 12 hours. After completion of the reaction, the mixture was concentrated under reduced pressure. The residue was purified by prep-HPLC (column: Waters Xbridge 150 * 25 mm * 5 um; mobile phase: [water (NH₄HCO₃)-ACN]; gradient: 18% to 48% B over 9 min) to afford Compound 211 (62 mg, 85.7% yield) as a white solid.

LCMS m/z 752.5 [M+1]⁺.

¹H NMR (400 MHz, DMSO-d6 ) δ = 10.51 (s, 1H), 9.58 (br s, 1H), 9.15 (br s, 1H), 8.69 - 8.35 (m, 1H), 7.70 - 7.51 (m, 2H), 7.32 (td, J = 7.8, 27.6 Hz, 2H), 7.05 (dd, J = 7.1, 25.5 Hz, 2H), 6.90 - 6.55 (m, 2H), 5.47 - 5.10 (m, 1H), 4.39 (br s, 3H), 4.27 - 4.12 (m, 2H), 4.07 - 3.73 (m, 2H), 3.64 - 3.50 (m, 3H), 3.13 - 2.95 (m, 4H), 2.92 - 2.67 (m, 3H), 2.41 - 2.22 (m, 1H), 2.20 - 2.05 (m, 2H), 1.99 (s, 4H), 1.94 (s, 4H), 1.88 - 1.73 (m, 1H).

### [Preparation Example 48]

### Preparation of Compound 212, 2-(((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetic acid, and Compound 213, 2-(((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)acetic acid

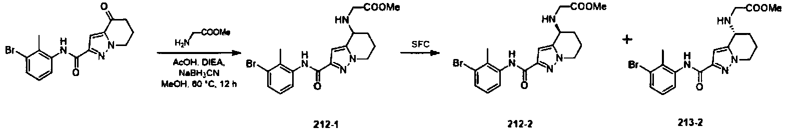

To a mixture of N-(3-bromo-2-methylphenyl)-4-oxo-6,7-dihydro-5H-pyrazolo[1,5-a]pyridine-2-carboxamide (2 g, 5.74 mmol, 1 eq) and methyl 2-aminoacetate hydrochloride (7.21 g, 57.44 mmol, 10 eq) in MeOH (40 mL), AcOH (689.87 mg, 11.49 mmol, 657.64 µL, 2 eq), DIPEA (7.42 g, 57.44 mmol, 10 mL, 10 eq), and NaBH₃CN (1.80 g, 28.72 mmol, 5 eq) were added, and the reaction mixture was stirred at 60 °C for 12 hours. After completion of the reaction, the mixture was concentrated under reduced pressure. The residue was purified by prep-HPLC (column: Kromasil Eternity XT 250 * 80 mm * 10 um; mobile phase: [water (NH₄HCO₃)-ACN]; gradient: 25% to 58% B over 20 min) to afford Compound 212-1 (1.5 g, 59.5% yield) as a white solid. The product was further purified by SFC (column: DAICEL CHIRALPAK AD, 250 * 30 mm * 10 um; mobile phase: [CO₂-CAN/i-PrOH(0.1% NH₃H₂O)]; B = 50%, isocratic elution mode) to give Compound 212-2 (650 mg, 25.8% yield) and Compound 213-2 (675 mg, 26.8% yield) as yellow solids.

LCMS m/z 421.1 [M+1]⁺.

¹H NMR (400 MHz, DMSO-d6) δ ppm 1.62 - 1.80 (m, 1 H) 1.85 - 2.04 (m, 2 H) 2.18 - 2.24 (m, 1 H) 2.27 (s, 3 H) 3.32 (s, 1 H) 3.40 - 3.48 (m, 2 H) 3.62 - 3.67 (m, 3 H) 3.94 (br t, J=5.69 Hz, 1 H) 4.08 - 4.20 (m, 2 H) 6.71 (s, 1 H) 7.15 (t, J=8.00 Hz, 1 H) 7.42 (d, J=7.75 Hz, 1 H) 7.48 (d, J=7.75 Hz, 1 H) 9.79 (s, 1 H).

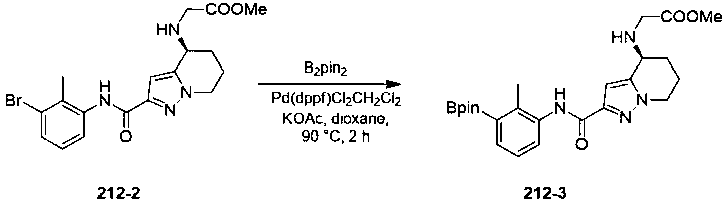

A mixture of methyl 2-[[(4S)-2-[(3-bromo-2-methylphenyl)carbamoyl]-4,5,6,7-tetrahydropyrazolo[1,5-a]pyridin-4-yl]amino]acetate 3-S (300 mg, 712.10 µmol, 1 eq), 4,4,5,5-tetramethyl-2-(4,4,5,5-tetramethyl-1,3,2-dioxaborolan-2-yl)-1,3,2-dioxaborolane (542.49 mg, 2.14 mmol, 3 eq), KOAc (209.66 mg, 2.14 mmol, 3 eq), and Pd(dppf)Cl₂·CH₂Cl₂ (58.15 mg, 71.21 µmol, 0.1 eq) in dioxane (10 mL) was stirred at 90 °C for 2 hours under a nitrogen atmosphere. After completion of the reaction, the mixture was filtered and the filtrate was concentrated under reduced pressure. The residue was purified by prep-TLC (dichloromethane:methanol = 10:1) to afford Compound 212-3 (236 mg, 67.2% yield) as a white solid.

LCMS m/z 469.3[M+1]⁺.

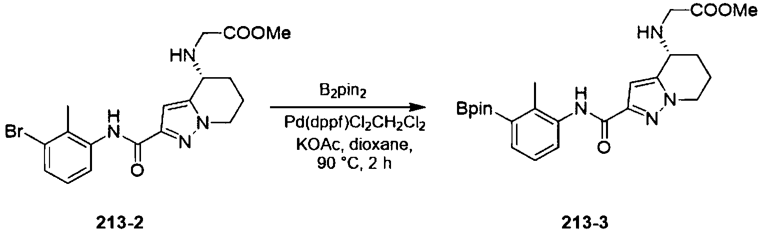

A mixture of methyl 2-[[(4R)-2-[(3-bromo-2-methylphenyl)carbamoyl]-4,5,6,7-tetrahydropyrazolo[1,5-a]pyridin-4-yl]amino]acetate (300 mg, 712.10 µmol, 1 eq), 4,4,5,5-tetramethyl-2-(4,4,5,5-tetramethyl-1,3,2-dioxaborolan-2-yl)-1,3,2-dioxaborolane (542.49 mg, 2.14 mmol, 3 eq), KOAc (209.66 mg, 2.14 mmol, 3 eq), and Pd(dppf)Cl₂·CH₂Cl₂ (58.15 mg, 71.21 µmol, 0.1 eq) in dioxane (3 mL) was stirred at 90 °C for 2 hours under a nitrogen atmosphere. After completion, the mixture was filtered and the filtrate was concentrated under reduced pressure. The residue was purified by prep-TLC (dichloromethane:methanol = 10:1) to afford Compound 213-3 (241 mg, 57.8 percent yield) as a white solid.

LCMS m/z 469.5[M+1]⁺.

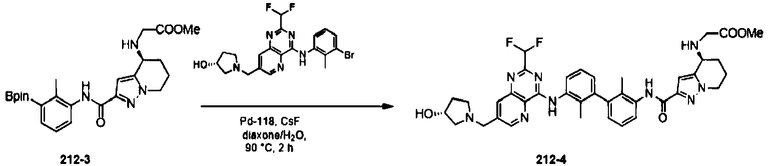

A mixture of (3R)-1-[[4-(3-bromo-2-methylanilino)-2-(difluoromethyl)pyrido[3,2-d]pyrimidin-7-yl]methyl]pyrrolidin-3-ol (80 mg, 172.30 µmol, 1 eq), methyl 2-[[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (161.39 mg, 344.60 µmol, 2 eq), CsF (78.52 mg, 516.90 µmol, 19.08 µL, 3 eq), and Pd(dppf)Cl₂ (12.61 mg, 17.23 µmol, 0.1 eq) in dioxane (10 mL) / water (2 mL) was stirred at 90 °C for 2 hours under nitrogen. The reaction mixture was filtered and the filtrate was concentrated under reduced pressure. The residue was purified by prep-HPLC (Phenomenex Luna C18, 150 * 25 mm * 10 µm; mobile phase: [water(HCl)-ACN]e; gradient: 5% to 35% B over 10 min) to give Compound 212-4 (46 mg, 33.1 percent yield) as a white solid.

LCMS m/z 726.4 [M+1]⁺.

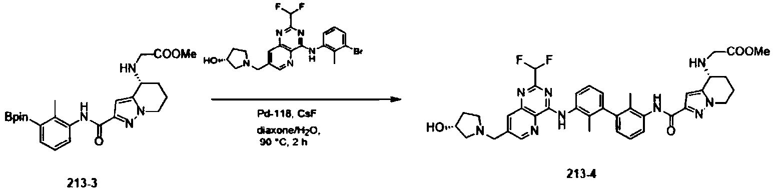

A mixture of (3R)-1-[[4-(3-bromo-2-methyl-anilino)-2-(difluoromethyl)pyrido[3,2-d]pyrimidin-7-yl]methyl]pyrrolidin-3-ol (80 mg, 172.30 µmol, 1 eq), methyl 2-[[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (96.84 mg, 206.76 µmol, 1.2 eq), CsF (78.52 mg, 516.90 µmol, 19.08 µL, 3 eq), and ditert-butyl(cyclopentyl)phosphane;dichloropalladium iron (11.23 mg, 17.23 µmol, 0.1 eq) in dioxane (8 mL) / water (2 mL) was stirred at 90 °C for 2 hours under nitrogen. After filtration and concentration, the residue was purified by prep-HPLC (column: C18 150*30mm; mobile phase: [water (FA)-ACN]; gradient: 15% to 45% B over 7 min) to afford Compound 213-4 (82 mg, 62.2 percent yield) as a yellow solid.

LCMS m/z 726.4 [M+1]⁺.

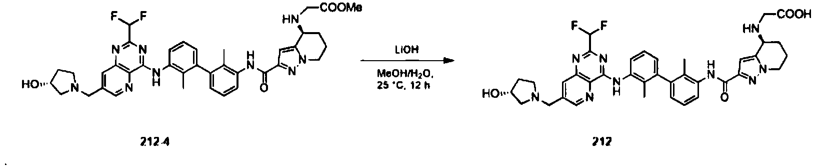

To a solution of methyl 2-[[(4S)-2-[[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]carbamoyl]-4,5,6,7-tetrahydropyrazolo[1,5-a]pyridin-4-yl]amino]acetate (55 mg, 75.78 µmol, 1 eq) in THF (10 mL) / water (3 mL), LiOH·H₂O (9.54 mg, 227.34 µmol, 3 eq) was added, and the reaction mixture was stirred at room temperature for 12 hours. The mixture was concentrated under reduced pressure and purified by prep-HPLC (column: Waters Xbridge, 150 * 25 mm * 5 µm; mobile phase: [water (NH₄HCO₃)-ACN]; gradient: 15 to 45 percent B over 9 min) to give Compound 212 (23 mg, 40.5 percent yield) as a white solid.

LCMS m/z 712.4 [M+1]⁺.

¹H NMR (400 MHz, DMSO-d6 ) δ = 10.39 (s, 1H), 9.56 (br s, 1H), 8.95 (d, J = 1.8 Hz, 1H), 8.16 (s, 1H), 7.65 (d, J = 7.9 Hz, 1H), 7.56 (br d, J = 7.9 Hz, 1H), 7.35 (t, J = 7.8 Hz, 1H), 7.29 (br t, J = 7.3 Hz, 1H), 7.06 (br d, J = 7.8 Hz, 1H), 7.01 (br d, J = 7.5 Hz, 1H), 6.76 (br s, 1H), 6.70 (t, J = 54.5 Hz, 1H), 4.25 - 4.04 (m, 4H), 3.90 - 3.81 (m, 2H), 2.79 - 2.64 (m, 3H), 2.41 (br dd, J = 3.4, 9.6 Hz, 3H), 2.34 - 2.29 (m, 1H), 2.20 (br s, 2H), 2.08 - 2.00 (m, 2H), 1.99 (br s, 3H), 1.94 (s, 3H), 1.80 - 1.50 (m, 3H).

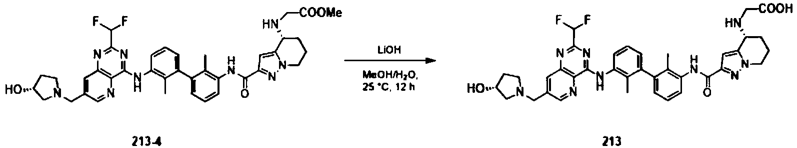

To a mixture of methyl 2-[[(4R)-2-[[3-[3-[[2-(difluoromethyl)-7-[[(3R)-3-hydroxypyrrolidin-1-yl]methyl]pyrido[3,2-d]pyrimidin-4-yl]amino]-2-methylphenyl]-2-methylphenyl]carbamoyl]-4,5,6,7-tetrahydropyrazolo[1,5-a]pyridin-4-yl]amino]acetate (60.00 mg, 82.67 µmol, 1 eq) in THF (10 mL) / water (3 mL), LiOH·H₂O (10.41 mg, 248.01 µmol, 3 eq) was added, and the reaction mixture was stirred at room temperature for 12 hours. After completion, the mixture was concentrated under reduced pressure. The residue was purified by prep-HPLC (column: Waters Xbridge 150 * 25mm* 5µm; mobile phase: [water (NH₄HCO₃)-ACN]; gradient: 15 to 45 percent B over 9 min) to afford Compound 213 (46 mg, 74.27 percent yield) as a white solid.

LCMS m/z 712.5 [M+1]⁺.

¹H NMR (400 MHz, DMSO-d6 ) δ = 10.47 (s, 1H), 9.58 (s, 1H), 9.05 (br s, 1H), 8.34 (br s, 1H), 7.71 - 7.53 (m, 2H), 7.33 (td, J = 7.8, 26.9 Hz, 2H), 7.05 (dd, J = 7.4, 23.1 Hz, 2H), 6.87 - 6.55 (m, 2H), 4.33 (br s, 2H), 4.20 - 4.08 (m, 4H), 3.40 (s, 3H), 3.14 - 2.92 (m, 4H), 2.85 - 2.63 (m, 2H), 2.31 - 2.19 (m, 1H), 2.15 - 2.03 (m, 2H), 1.99 (s, 3H), 1.95 (s, 3H), 1.87 - 1.70 (m, 2H).

### [Preparation Example 49]

### Preparation of Compounds 214 to 217

### 49-1. Preparation of Compound 214, (S)-4-((2-hydroxyethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide, and Compound 215, (R)-4-((2-hydroxyethyl)amino)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

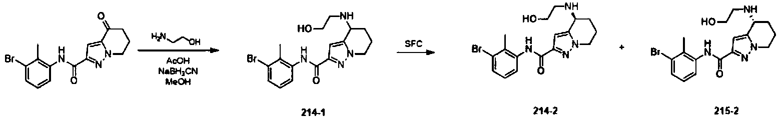

To a solution of N-(3-bromo-2-methylphenyl)-4-oxo-6,7-dihydro-5H-pyrazolo[1,5-a]pyridine-2-carboxamide (2.5 g, 7.18 mmol, 1 eq) and 2-aminoethanol (4.39 g, 71.80 mmol, 4.33 mL, 10 eq) in MeOH (20 mL), NaBH₃CN (4.51 g, 71.80 mmol, 10 eq) and AcOH (4.31 g, 71.80 mmol, 4.11 mL, 10 eq) were added, and the reaction mixture was stirred at 60 °C for 12 hours. After completion, the mixture was concentrated under reduced pressure. The residue was purified by reversed-phase HPLC (column: Phenomenex Luna C18, 250 * 70 mm * 10 um; mobile phase: [water (NH₄HCO₃)-ACN]; gradient: 20%-50% B over 20 min). The resulting material was further separated by SFC (column: DAICEL CHIRALPAK IC, 250 mm * 30 mm * 10 um; mobile phase: CO₂-i-PrOH/ACN; B%: 40%, isocratic elution mode) to afford Compound 214-2 (379 mg, 26.7% yield) and compound 215-2 (391 mg, 27.5% yield) as white solids.

LCMS m/z 392.08 [M+3]⁺.

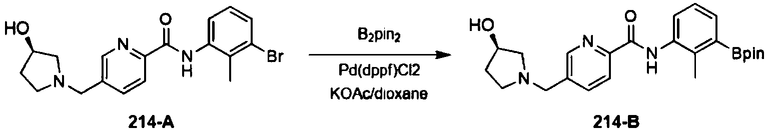

A mixture of N-(3-bromo-2-methylphenyl)-5-[[(3R)-3-hydroxypyrrolidin-1-yl]methyl]pyridine-2-carboxamide (400 mg, 1.02 mmol, 1 eq), 4,4,5,5-tetramethyl-2-(4,4,5,5-tetramethyl-1,3,2-dioxaborolan-2-yl)-1,3,2-dioxaborolane (780.80 mg, 3.07 mmol, 3 eq), KOAc (301.76 mg, 3.07 mmol, 3 eq), and Pd(dppf)Cl₂ (74.99 mg, 102.49 µmol, 0.1 eq) in dioxane (6 mL) was stirred at 90 °C for 3 hours under a nitrogen atmosphere. After completion, the mixture was concentrated under reduced pressure. The residue was purified by prep-TLC (SiO₂, ethyl acetate/methanol = 5:1) to afford Compound 214-B (320 mg, 57.1% yield) as a white solid.

LCMS m/z 437.25 [M+1]⁺.

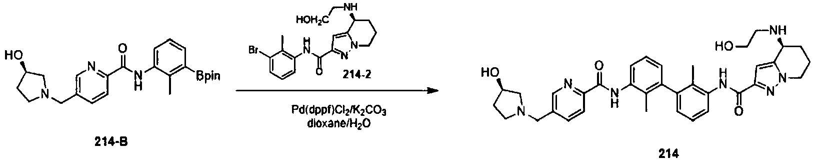

A mixture of 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (160 mg, 365.85 µmol, 1.1 eq), (4S)-N-(3-bromo-2-methylphenyl)-4-(2-hydroxyethylamino)-4,5,6,7-tetrahydropyrazolo[1,5-a]pyridine-2-carboxamide (130.80 mg, 332.59 µmol, 1 eq), Pd(dppf)Cl₂ (24.34 mg, 33.26 µmol, 0.1 eq), and K₂CO₃ (45.97 mg, 332.59 µmol, 1 eq) in dioxane (2 mL) / water (0.5 mL) was stirred at 90 °C for 12 hours under a nitrogen atmosphere. After completion, the mixture was concentrated under reduced pressure. The residue was purified by reversed-phase HPLC (column: Waters XBridge 150 * 25 mm * 5 µm; mobile phase: [water (NH₄HCO₃)-ACN]; gradient: 26-56% B over 9 min) to afford Compound 214 (24 mg, 11.1% yield) as a yellow solid.

LCMS m/z 623.32 [M+1]⁺.

¹H NMR (EW41301-99-P1A1, 400 MHz, DMSO-d6) δ ppm 1.51 - 1.63 (m, 1 H) 1.63 - 1.76 (m, 1 H) 1.95 (s, 3 H) 1.98 - 2.08 (m, 6 H) 2.14 - 2.23 (m, 1 H) 2.31 - 2.38 (m, 1 H) 2.41 - 2.48 (m, 1 H) 2.58 - 2.65 (m, 1 H) 2.66 - 2.74 (m, 3 H) 3.46 - 3.50 (m, 2 H) 3.64 - 3.79 (m, 2 H) 3.89 (br t, J=5.94 Hz, 1 H) 4.11 - 4.18 (m, 2 H) 4.21 (br d, J=2.63 Hz, 1 H) 4.54 (t, J=5.38 Hz, 1 H) 4.74 (d, J=4.63 Hz, 1 H) 6.72 (s, 1 H) 6.98 (d, J=7.38 Hz, 2 H) 7.22 - 7.38 (m, 2 H) 7.58 (br d, J=8.00 Hz, 1 H) 7.87 (d, J=7.75 Hz, 1 H) 7.98 (d, J=8.38 Hz, 1 H) 8.14 (d, J=8.00 Hz, 1 H) 8.65 (s, 1 H) 9.53 (s, 1 H) 10.34 (s, 1 H).

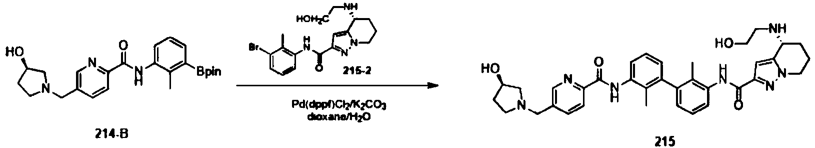

A mixture of 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide 5 (160 mg, 365.85 µmol, 1.1 eq), (4R)-N-(3-bromo-2-methylphenyl)-4-(2-hydroxyethylamino)-4,5,6,7-tetrahydropyrazolo[1,5-a]pyridine-2-carboxamide (130.80 mg, 332.59 µmol, 1 eq), Pd(dppf)Cl₂ (24.34 mg, 33.26 µmol, 0.1 eq), and K₂CO₃ (45.97 mg, 332.59 µmol, 1 eq) in dioxane (2.5 mL) / water (0.5 mL) was stirred at 90 °C for 12 hours under a nitrogen atmosphere. After completion, the mixture was concentrated under reduced pressure. The residue was purified by reversed-phase HPLC (column: Waters XBridge 150 * 25 mm * 5 µm; mobile phase: [water (NH₄HCO₃)-ACN]; gradient: 26-56% B over 9 min) to afford Compound 215 (36 mg, 17.1% yield) as a yellow solid.

LCMS m/z 623.32 [M+1]⁺.

¹H NMR (400 MHz, DMSO-*d*6) δ ppm 1.52 - 1.61 (m, 1 H) 1.63 - 1.76 (m, 1 H) 1.95 (s, 3 H) 1.98 - 2.13 (m, 6 H) 2.14 - 2.24 (m, 1 H) 2.35 (dd, *J* =9.57, 3.44 Hz, 1 H) 2.40 - 2.48 (m, 1 H) 2.58 - 2.76 (m, 4 H) 3.46 - 3.49 (m, 2 H) 3.64 - 3.79 (m, 2 H) 3.89 (br t, *J* =5.88 Hz, 1 H) 4.10 - 4.18 (m, 2 H) 4.21 (br d, *J* =2.63 Hz, 1 H) 4.54 (t, *J* =5.44 Hz, 1 H) 4.74 (d, *J* =4.50 Hz, 1 H) 6.72 (s, 1 H) 6.98 (d, *J* =7.50 Hz, 2 H) 7.21 - 7.39 (m, 2 H) 7.58 (br d, *J*=8.00 Hz, 1 H) 7.87 (d, *J* =8.00 Hz, 1 H) 7.99 (dd, *J* =7.94, 1.69 Hz, 1 H) 8.14 (d, *J* =7.88 Hz, 1 H) 8.65 (s, 1 H) 9.54 (s, 1 H) 10.34 (s, 1 H).

### 49-2. Preparation of Compound 216, (S)-4-((R)-3-hydroxypyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide, and Cmpound 217, ((R)-4-((R)-3-hydroxypyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

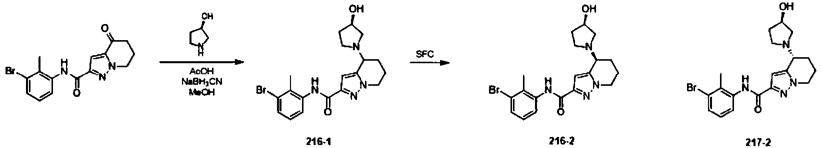

To a solution of N-(3-bromo-2-methylphenyl)-4-oxo-6,7-dihydro-5H-pyrazolo[1,5-a]pyridine-2-carboxamide (2.5 g, 7.18 mmol, 1 eq) and (3R)-pyrrolidin-3-ol (6.26 g, 71.80 mmol, 5.97 mL, 10 eq) in methanol (20 mL), NaBH₃CN (4.51 g, 71.80 mmol, 10 eq) and acetic acid (4.31 g, 71.80 mmol, 4.11 mL, 10 eq) were added, and the reaction mixture was stirred at 60 °C for 12 hours. After completion, the mixture was concentrated under reduced pressure. The residue was purified by reversed-phase HPLC (column: Phenomenex Luna C18, 250 * 70 mm * 10 µm; mobile phase: [water (NH₄HCO₃)-ACN]; gradient: 30-60% B over 20 min, followed by Phenomenex Luna C18, 150 * 25 mm * 10 µm; mobile phase: [water (HCl)-ACN]; gradient: 5-35% B over 10 min). The resulting fractions were further separated by SFC (column: DAICEL CHIRALPAK IG, 250 * 30 mm, 10 µm; mobile phase: CO₂-ACN/MeOH (0.1% NH₃·H₂O); B = 62%, isocratic elution mode) to afford Compound 217-2 (395 mg, 24.5% yield) and compound 216-2 (351 mg, 21.8% yield), each obtained as a white solid.

LCMS m/z 418.1 [M+3]⁺.

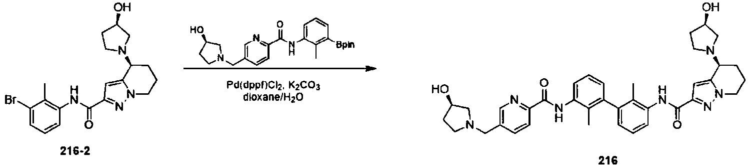

To a solution of 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (140 mg, 320.12 µmol, 1 eq) and (4S)-N-(3-bromo-2-methylphenyl)-4-[(3R)-3-hydroxypyrrolidin-1-yl]-4,5,6,7-tetrahydropyrazolo[1,5-a]pyridine-2-carboxamide (134.23 mg, 320.12 µmol, 1 eq) in dioxane (5 mL) and water (1 mL), Pd(dppf)Cl₂ (23.42 mg, 32.01 µmol, 0.1 eq) and K₂CO₃ (K₂CO₃, 132.73 mg, 960.35 µmol, 3 eq) were added, and the reaction mixture was stirred under a nitrogen atmosphere at 90 °C for 12 hours. After completion, the mixture was concentrated under reduced pressure, and the residue was purified by prep-TLC (silica gel, ethyl acetate/methanol = 1:2). The obtained material was further purified by reversed-phase HPLC (column: Waters Xbridge Prep OBD C18, 150 * 40 mm * 10 µm; mobile phase: [water (NH₄HCO₃)-ACN]; gradient: 18-48% B over 15 min) to afford Compound 216 (30 mg, 15.0% yield) as a light brown solid.

LCMS m/z 649.34 [M+1]⁺.

¹H NMR (400 MHz, DMSO-d6) δ ppm 1.51 - 1.63 (m, 2 H) 1.78 - 2.01 (m, 8 H) 2.02 (s, 3 H) 2.25 (br s, 1 H) 2.33 - 2.35 (m, 1 H) 2.41 - 2.48 (m, 1 H) 2.57 - 2.66 (m, 2 H) 2.66 - 2.81 (m, 3 H) 3.64 - 3.82 (m, 3 H) 4.09 - 4.27 (m, 4 H) 4.74 (br s, 2 H) 6.67 (s, 1 H) 6.98 (br d, *J* =7.50 Hz, 2 H) 7.20 - 7.40 (m, 2 H) 7.49 - 7.68 (m, 1 H) 7.88 (br d, *J* =8.00 Hz, 1 H) 7.98 (br d, *J* =6.88 Hz, 1 H) 8.15 (d, *J* =8.00 Hz, 1 H) 8.65 (s, 1 H) 9.54 (br d, *J* =2.63 Hz, 1 H) 10.34 (s, 1 H).

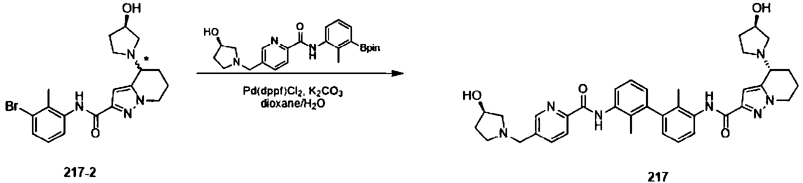

A mixture of 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (140 mg, 320.12 µmol, 1 eq), (4R)-N-(3-bromo-2-methyl-phenyl)-4-[(3R)-3-hydroxypyrrolidin-1-yl]-4,5,6,7-tetrahydropyrazolo[1,5-a]pyridine-2-carboxamide (134.23 mg, 320.12 µmol, 1 eq), Pd(dppf)Cl₂ (23.42 mg, 32.01 µmol, 0.1 eq), and K₂CO₃ (132.73 mg, 960.35 µmol, 3 eq) in dioxane (5 mL) and H₂O (1 mL) was stirred under a nitrogen atmosphere at 90 °C for 12 hours. After completion of the reaction, the mixture was concentrated under reduced pressure, and the residue was purified by prep-TLC (SiO₂, Ethyl acetate:Methanol = 1:2), followed by further purification by reversed-phase HPLC (column: Waters Xbridge Prep OBD C18 150 * 40 mm * 10 µm; mobile phase: [water (NH₄HCO₃)-ACN]; gradient: 18% to 48% B over 15 min), to afford Compound 217 (52 mg, 23.1% yield) as a light brown solid.

LCMS m/z 649.34 [M+3]⁺.

¹H NMR (400 MHz, DMSO-d6) δ ppm 1.51 - 1.63 (m, 2 H) 1.85 - 1.97 (m, 6 H) 1.99 - 2.05 (m, 4 H) 2.17 - 2.29 (m, 1 H) 2.32 - 2.47 (m, 3 H) 2.58 - 2.73 (m, 2 H) 2.76 - 2.94 (m, 2 H) 3.64 - 3.81 (m, 3 H) 4.10 - 4.27 (m, 4 H) 4.73 (dd, *J* =9.26, 4.38 Hz, 2 H) 6.69 (s, 1 H) 6.98 (d, *J* =7.38 Hz, 2 H) 7.21 - 7.39 (m, 2 H) 7.58 (dd, *J* =7.75, 2.88 Hz, 1 H) 7.88 (d, *J* =7.88 Hz, 1 H) 7.94 - 8.03 (m, 1 H) 8.15 (d, *J* =8.00 Hz, 1 H) 8.65 (s, 1 H) 9.54 (d, *J* =2.88 Hz, 1 H) 10.34 (s, 1 H)

### [Preparation Example 50]

### Preparation of Compound 128, 2-(((S)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetic acid, and Compound 129, 2-(((R)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetic acid

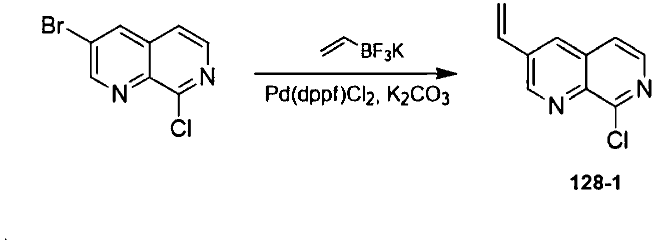

A mixture of 3-bromo-8-chloro-1,7-naphthyridine (5 g, 20.53 mmol, 1 eq), potassium hydride trifluoro(vinyl)boron (5.50 g, 41.07 mmol, 2 eq), Pd(dppf)Cl₂ (1.50 g, 2.05 mmol, 0.1 eq), and K₃PO₄ (8.72 g, 41.07 mmol, 2 eq) in dioxane (150 mL) was stirred under a nitrogen atmosphere at 75 °C for 36 hours. After completion of the reaction, the mixture was concentrated under reduced pressure, and the residue was purified by column chromatography (SiO₂, Petroleum ether/Ethyl acetate = 3/1) to afford Compound 128-1 (2 g, 51.0% yield) as a white solid.

LCMS m/z 191.1 [M+1]⁺.

¹H NMR (400 MHz, DMSO-d6) δ ppm 5.69 (d, J=11.13 Hz, 1 H) 6.32 (d, J=17.76 Hz, 1 H) 6.94 - 7.09 (m, 1 H) 7.91 (d, J=5.50 Hz, 1 H) 8.39 (d, J=5.50 Hz, 1 H) 8.53 (d, J=2.13 Hz, 1 H) 9.33 (d, J=2.00 Hz, 1 H).

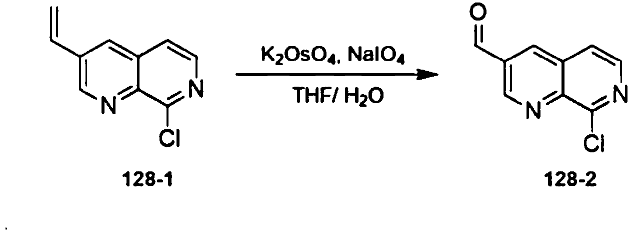

To a solution of 8-chloro-3-vinyl-1,7-naphthyridine (4.2 g, 22.03 mmol, 1 eq) in THF (50 mL) / H₂O (5 mL), K₂OsO₄ (14.14 g, 66.10 mmol, 3 eq) and NaIO₄ (1.22 g, 3.30 mmol, 0.15 eq) were added. The resulting mixture was stirred at room temperature for 12 hours. An aq. saturated Na₂SO₃ solution (100 mL) was added to the mixture, followed by stirring at room temperature for 15 minutes. The mixture was extracted with ethyl acetate (50 mL * 3), and the combined organic layers were dried over Na₂SO₄ and concentrated under reduced pressure. The residue was purified by column chromatography (SiO₂, Petroleum ether/Ethyl acetate = 1/1) to afford Compound 128-2 (4.2 g, 97.9% yield) as a white solid.

LCMS m/z 193.0 [M+1]⁺.

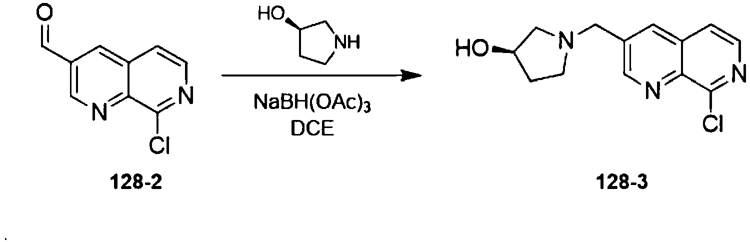

To a solution of 8-chloro-1,7-naphthyridine-3-carbaldehyde (6.8 g, 35.31 mmol, 1 eq) and (3R)-pyrrolidin-3-ol (3.69 g, 42.37 mmol, 1.2 eq) in DCE (70 mL), NaBH(OAc)₃ (22.45 g, 105.92 mmol, 3 eq) was added. The resulting mixture was stirred at room temperature for 12 hours. After completion of the reaction, the mixture was concentrated under reduced pressure, and the residue was purified by column chromatography (SiO₂, ethyl acetate/methanol = 10/1) to afford Compound 128-3 (7.2 g, 76.5% yield) as a brown liquid.

LCMS m/z 264.1 [M+1]⁺.

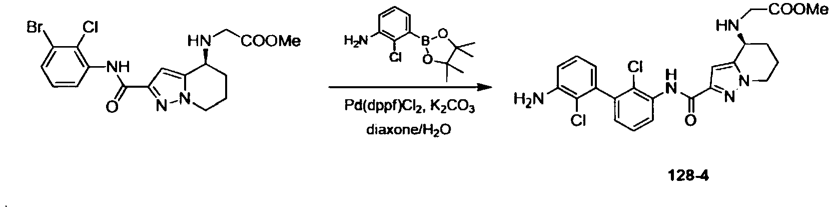

A mixture of methyl 2-[[(4S)-2-[(3-bromo-2-chloro-phenyl)carbamoyl]-4,5,6,7-tetrahydropyrazolo[1,5-a]pyridin-4-yl]amino]acetate (120.00 mg, 271.67 µmol, 1 eq), 2-chloro-3-(4,4,5,5-tetramethyl-1,3,2-dioxaborolan-2-yl)aniline (137.76 mg, 543.35 µmol, 2 eq), Pd(dppf)Cl₂ (19.88 mg, 27.17 µmol, 0.1 eq), and K₂CO₃ (112.64 mg, 815.02 µmol, 3 eq) in of dioxane (2 mL) and H₂O (0.4 mL) was stirred under a nitrogen atmosphere at 90 °C for 3 hours. After completion of the reaction, the mixture was concentrated under reduced pressure, and the residue was purified by prep-TLC (SiO₂, DCM:methanol = 20:1) to afford Compound 128-4 (100 mg, 75.3% yield) as a yellow solid.

LCMS m/z 488.3 [M+1]⁺.

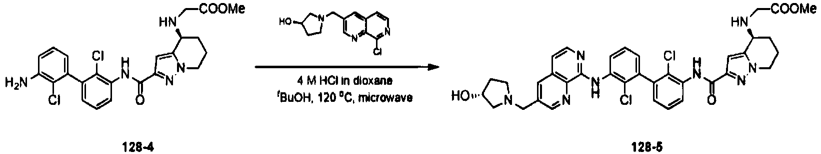

To a solution of methyl 2-[[(4S)-2-[[3-(3-amino-2-chloro-phenyl)-2-chloro-phenyl]carbamoyl]-4,5,6,7-tetrahydropyrazolo[1,5-a]pyridin-4-yl]amino]acetate (90 mg, 184.29 µmol, 1 eq) and (3R)-1-[(8-chloro-1,7-naphthyridin-3-yl)methyl]pyrrolidin-3-ol (145.80 mg, 552.86 µmol, 3 eq) in t-BuOH (20 mL), HCl/dioxane (4 M, 92.14 µL, 2 eq) was added. The resulting mixture was subjected to microwave irradiation at 120 °C for 8 hours. After completion of the reaction, the mixture was concentrated under reduced pressure to afford Compound 128-5 (160 mg, 61.8% yield) as a pink solid, which was used in the next reaction without further purification.

LCMS m/z 715.3 [M+1]⁺.

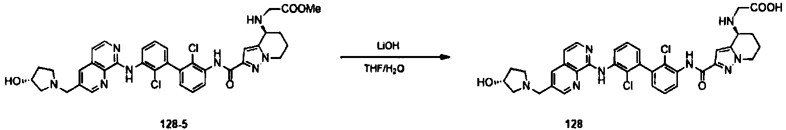

To a solution of methyl 2-[[(4S)-2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]amino]acetate (160 mg, 223.58 µmol, 1 eq) inof THF (30 mL) / H₂O (15 mL), LiOH·H₂O (28.15 mg, 670.74 µmol, 3 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Waters Xbridge 150 * 25 mm * 5 µm; mobile phase: [water (NH₄HCO₃)-ACN]; gradient: 22% to 52% B over 9 min) to afford Compound 128 (14 mg, 8.48% yield) as a white solid.

LCMS (m/z 701.3 [M+1]⁺.

¹H NMR (400 MHz, DMSO-d6) δ ppm 1.58 (br s, 1 H) 1.75 (br s, 1 H) 1.91 (br s, 1 H) 2.02 (br dd, J=12.88, 7.13 Hz, 2 H) 2.20 (br s, 1 H) 2.32 - 2.43 (m, 2 H) 2.62 - 2.69 (m, 2 H) 2.74 (br dd, J=9.76, 6.00 Hz, 2 H) 3.75 - 3.89 (m, 4 H) 4.04 (br s, 1 H) 4.15 (br t, J=6.19 Hz, 2 H) 4.22 (br s, 1 H) 6.83 (s, 1 H) 7.03 (d, J=6.75 Hz, 1 H) 7.17 - 7.23 (m, 1 H) 7.35 (d, J=6.00 Hz, 1 H) 7.51 (q, J=8.00 Hz, 2 H) 8.19 (d, J=5.75 Hz, 1 H) 8.23 - 8.31 (m, 2 H) 8.92 (d, J=1.88 Hz, 1 H) 9.14 (dd, J=8.32, 1.44 Hz, 1 H) 9.53 - 9.62 (m, 1 H) 9.86 - 9.92 (m, 1 H).

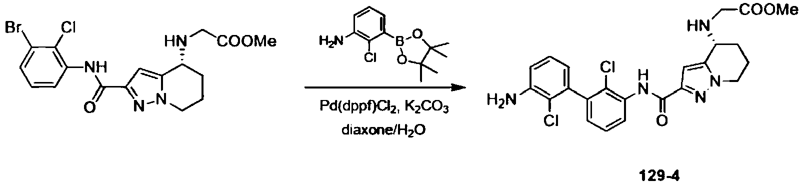

A mixture of methyl 2-[[(4R)-2-[(3-bromo-2-chloro-phenyl)carbamoyl]-4,5,6,7-tetrahydropyrazolo[1,5-a]pyridin-4-yl]amino]acetate (120.00 mg, 271.67 µmol, 1 eq), 2-chloro-3-(4,4,5,5-tetramethyl-1,3,2-dioxaborolan-2-yl)aniline (137.76 mg, 543.35 µmol, 2 eq), Pd(dppf)Cl₂ (19.88 mg, 27.17 µmol, 0.1 eq), and K₂CO₃ (112.64 mg, 815.02 µmol, 3 eq) in dioxane (2 mL) and H₂O (0.4 mL) was stirred under a nitrogen atmosphere at 90 °C for 3 hours. After completion of the reaction, the mixture was concentrated under reduced pressure, and the residue was purified by prep-TLC (SiO₂, DCM:methanol = 20:1) to afford Compound 129-4 (110 mg, 82.9% yield) as a yellow solid.

LCMS m/z 488.2 [M+1]⁺.

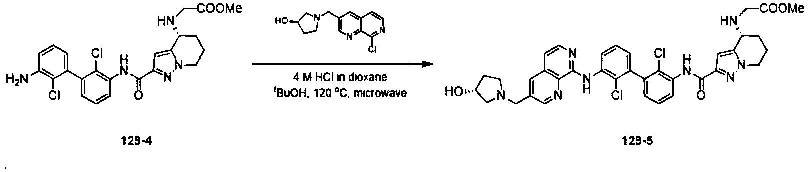

To a solution of methyl 2-[[(4R)-2-[[3-(3-amino-2-chloro-phenyl)-2-chloro-phenyl]carbamoyl]-4,5,6,7-tetrahydropyrazolo[1,5-a]pyridin-4-yl]amino]acetate (100 mg, 204.76 µmol, 1 eq) and (3R)-1-[(8-chloro-1,7-naphthyridin-3-yl)methyl]pyrrolidin-3-ol (162.00 mg, 614.29 µmol, 3 eq) in t-BuOH (20 mL), HCl/dioxane (4 M, 102.38 µL, 2 eq) was added. The resulting mixture was subjected to microwave irradiation at 120 °C for 8 hours. After completion of the reaction, the mixture was concentrated under reduced pressure to afford Compound 129-5 (130 mg, 38.1% yield) as a pink solid, which was used in the next reaction without further purification.

LCMS m/z 715.4 [M+1]⁺.

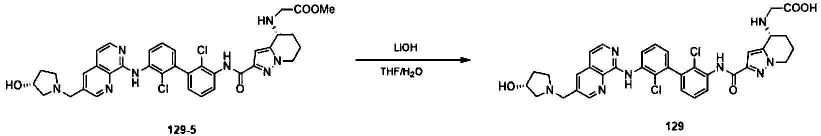

To a solution of methyl 2-[[(4R)-2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]amino]acetate (130 mg, 181.66 µmol, 1 eq) in THF (30 mL) / H₂O (15 mL), LiOH·H₂O (22.87 mg, 544.98 µmol, 3 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Waters Xbridge 150 * 25 mm * 5 µm; mobile phase: [water (NH₄HCO₃)-ACN]; gradient: 22% to 52% B over 9 min) to afford Compound 129 (12 mg, 8.94% yield) as a white solid.

LCMS m/z 701.3 [M+1]⁺.

¹H NMR (400 MHz, DMSO-d6) δ ppm 1.53 - 1.62 (m, 1 H) 1.70 - 1.78 (m, 1 H) 1.88 - 1.94 (m, 1 H) 2.02 (br d, J=6.13 Hz, 2 H) 2.30 - 2.34 (m, 1 H) 2.35 - 2.41 (m, 2 H) 2.66 (br d, J=9.13 Hz, 2 H) 2.71 - 2.77 (m, 2 H) 3.77 - 3.88 (m, 4 H) 4.04 (br s, 1 H) 4.15 (br t, J=6.25 Hz, 2 H) 4.22 (br s, 1 H) 6.82 (s, 1 H) 7.03 (d, J=7.75 Hz, 1 H) 7.20 (d, J=6.13 Hz, 1 H) 7.35 (d, J=5.88 Hz, 1 H) 7.46 - 7.55 (m, 2 H) 8.19 (d, J=5.75 Hz, 1 H) 8.23 - 8.31 (m, 2 H) 8.92 (d, J=2.00 Hz, 1 H) 9.13 (d, J=8.38 Hz, 1 H) 9.57 (s, 1 H) 9.86 - 9.94 (m, 1 H).

### [Preparation Example 51]

### Preparation of Compound 220, (S)-2-((2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)amino)acetic acid, and Compound 221, (R)-2-((2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)amino)acetic acid

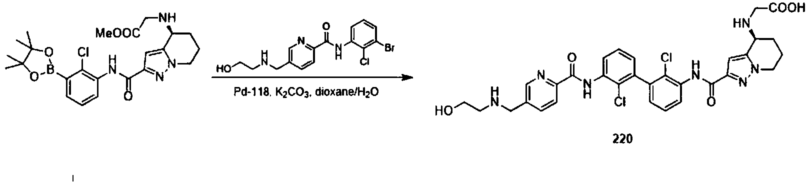

A mixture of methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (100 mg, 204.59 µmol, 1.2 eq), N-(3-bromo-2-chloro-phenyl)-5-[(2-hydroxyethylamino)methyl]pyridine-2-carboxamide (65.58 mg, 170.50 µmol, 1 eq), K₂CO₃ (47.13 mg, 340.99 µmol, 2 eq), and ditert-butyl(cyclopentyl)phosphane·dichloropalladium iron (11.11 mg, 17.05 µmol, 0.1 eq) in dioxane (5 mL) and H₂O (1 mL) was stirred under a nitrogen atmosphere at 90 °C for 12 hours. After completion of the reaction, the mixture was concentrated under reduced pressure, and the residue was purified by reversed-phase HPLC (0.1% NH₃·H₂O; column: Waters Xbridge 150 * 25 mm * 5 µm; mobile phase: [water (NH₄HCO₃)-ACN]; gradient: 18% to 48% B over 9 min) to afford Compound 220 (17 mg, 14.8% yield) as a white solid.

LCMS m/z 652.3 [M+1]⁺.

¹H NMR (400 MHz, DMSO-d6) δ ppm 1.66 - 1.79 (m, 1 H) 1.91 (br s, 1 H) 1.96 - 2.07 (m, 1 H) 2.19 (br d, J=7.63 Hz, 2 H) 2.55 - 2.61 (m, 2 H) 3.30 (br s, 2 H) 3.48 (br s, 2 H) 3.87 (s, 2 H) 4.03 (br t, J=5.75 Hz, 1 H) 4.15 (br t, J=6.07 Hz, 2 H) 6.81 (s, 1 H) 7.18 (d, J=7.63 Hz, 2 H) 7.46 - 7.56 (m, 2 H) 8.06 (dd, J=8.07, 1.81 Hz, 1 H) 8.19 (d, J=8.00 Hz, 1 H) 8.27 (d, J=8.25 Hz, 1 H) 8.52 (dd, J=8.19, 1.31 Hz, 1 H) 8.68 - 8.72 (m, 1 H) 9.56 (s, 1 H) 10.72 (s, 1 H).

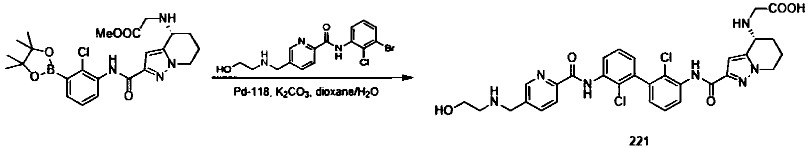

A mixture of mAthyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (130 mg, 265.97 µmol, 1.2 eq), N-(3-bromo-2-chloro-phenyl)-5-[(2-hydroxyethylamino)methyl]pyridine-2-carboxamide (85.26 mg, 221.64 µmol, 1 eq), K₂CO₃ (61.27 mg, 443.29 µmol, 2 eq), and ditert-butyl(cyclopentyl)phosphane·dichloropalladium iron (14.45 mg, 22.16 µmol, 0.1 eq) in dioxane (5 mL) and H₂O (1 mL) was stirred under a nitrogen atmosphere at 90 °C for 12 hours. After completion of the reaction, the mixture was concentrated under reduced pressure, and the residue was purified by reversed-phase HPLC (0.1% NH₃·H₂O; column: Waters Xbridge 150 * 25 mm * 5 µm; mobile phase: [water (NH₄HCO₃)-ACN]; gradient: 18% to 48% B over 9 min) to afford Compound 221 (17 mg, 11.5% yield) as a white solid.

LCMS m/z 652.3 [M+1]⁺.

¹H NMR (400 MHz, DMSO-d6) δ ppm 1.74 (br s, 1 H) 1.90 (br d, J=6.75 Hz, 1 H) 2.01 (br d, J=7.88 Hz, 1 H) 2.19 (br d, J=6.00 Hz, 1 H) 2.55 - 2.63 (m, 2 H) 3.29 (s, 2 H) 3.48 (br t, J=5.69 Hz, 2 H) 3.87 (s, 2 H) 4.01 - 4.05 (m, 1 H) 4.15 (br t, J=5.88 Hz, 2 H) 6.81 (s, 1 H) 7.18 (d, J=7.38 Hz, 2 H) 7.46 - 7.57 (m, 2 H) 8.06 (br d, J=7.88 Hz, 1 H) 8.18 (d, J=8.00 Hz, 1 H) 8.28 (br d, J=8.00 Hz, 1 H) 8.52 (d, J=8.25 Hz, 1 H) 8.70 (s, 1 H) 9.56 (s, 1 H) 10.72 (s, 1 H).

### [Preparation Example 52]

### Preparation of Compound 222, 2-(((S)-2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid, and Compound 223, 2-(((R)-2-((2,2'-dichloro-3'-(5-(((R)-3-hydroxypyrrolidin-1-yl)methyl)picolinamido)-[1,1'-biphenyl]-3-yl)carbamoyl)-4,5,6,7-tetrahydropyrazolo[1,5-a]pyridin-4-yl)amino)acetic acid

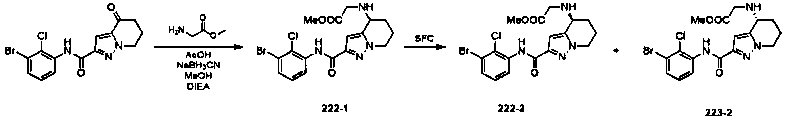

To a solution of N-(3-bromo-2-chloro-phenyl)-4-oxo-6,7-dihydro-5H-pyrazolo[1,5-a]pyridine-2-carboxamide (2.2 g, 5.97 mmol, 1 eq), methyl 2-aminoacetate (7.49 g, 59.68 mmol, 10 eq, HCl) in MeOH (40 mL), AcOH (716.80 mg, 11.94 mmol, 683.31 µL, 2 eq), DIPEA (7.71 g, 59.68 mmol, 10.40 mL, 10 eq), and NaBH₃CN (1.88 g, 29.84 mmol, 5 eq) were added. The resulting mixture was stirred at 60 °C for 12 hours. After completion of the reaction, the mixture was concentrated under reduced pressure, and the residue was purified by prep-HPLC (basic condition; column: Kromasil Eternity XT 250 * 80 mm * 10 µm; mobile phase: [water (NH₄HCO₃)-ACN]; gradient: 45% to 75% B over 20 min) to afford Compound 222-1 (1.5 g, 75.7% yield) as a white solid. The product was further purified by SFC (column: DAICEL CHIRALPAK IG, 250 * 30 mm * 10 µm; mobile phase: CO₂-ACN/i-PrOH (0.1% NH₃·H₂O); B%: 57.5%, isocratic elution mode) to separate compound 222-2 (655 mg, 43.6% yield) and 223-2 (630 mg, 42.0% yield) as white solids.

LCMS m/z 442.1 [M+1]⁺.

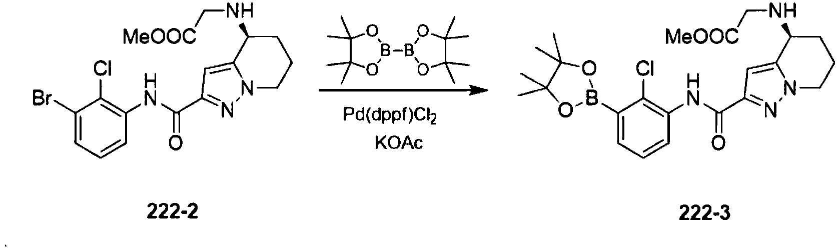

A mixture of methyl 2-[[(4S)-2-[(3-bromo-2-chloro-phenyl)carbamoyl]-4,5,6,7-tetrahydropyrazolo[1,5-a]pyridin-4-yl]amino]acetate (220 mg, 498.07 µmol, 1 eq), 4,4,5,5-tetramethyl-2-(4,4,5,5-tetramethyl-1,3,2-dioxaborolan-2-yl)-1,3,2-dioxaborolane (379.44 mg, 1.49 mmol, 3 eq), KOAc (146.64 mg, 1.49 mmol, 3 eq), and Pd(dppf)Cl₂·CH₂Cl₂ (61.01 mg, 74.71 µmol, 0.15 eq) in dioxane (7 mL) was stirred under a nitrogen atmosphere at 90 °C for 12 hours. After completion of the reaction, the mixture was concentrated under reduced pressure, and the residue was purified by prep-TLC (SiO₂, Petroleum ether/Ethyl acetate = 0/1) to afford Compound 222-3 (220 mg, 71.3% yield) as a yellow solid.

LCMS m/z 489.3 [M+1]⁺.

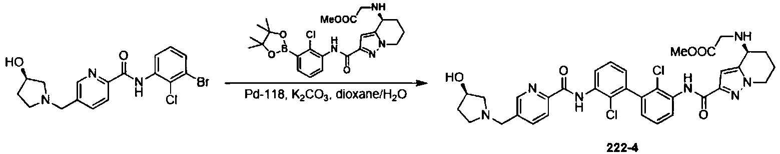

A mixture of N-(3-bromo-2-chloro-phenyl)-5-[[(3R)-3-hydroxypyrrolidin-1-yl]methyl]pyridine-2-carboxamide (125 mg, 304.36 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (178.52 mg, 365.24 µmol, 1.2 eq), CsF (138.70 mg, 913.09 µmol, 33.71 µL, 3 eq), and ditert-butyl(cyclopentyl)phosphane·dichloropalladium iron (19.84 mg, 30.44 µmol, 0.1 eq) in dioxane (5 mL) and H₂O (1 mL) was stirred under a nitrogen atmosphere at 90 °C for 12 hours. After completion of the reaction, the mixture was concentrated under reduced pressure, and the residue was purified by prep-TLC (SiO₂, ethyl acetate/methanol = 0/1) to afford Compound 222-4 (120 mg, 45.5% yield) as a white solid.

LCMS m/z 692.4 [M+1]⁺.

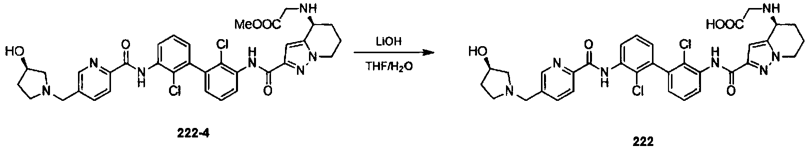

To a solution of methyl 2-[[(4S)-2-[[2-chloro-3-[2-chloro-3-[[5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]amino]acetate (120 mg, 173.26 µmol, 1 eq) inf THF (30 mL) / H₂O (15 mL), LiOH·H₂O (6.22 mg, 259.89 µmol, 1.5 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Waters Xbridge 150 * 25 mm * 5 µm; mobile phase: [water (NH₄HCO₃)-ACN]; gradient: 18% to 48% B over 9 min) to afford Compound 222 (97 mg, 79.2% yield) as a white solid.

LCMS m/z 678.3 [M+1]⁺.

¹H NMR (400 MHz, DMSO-d6) δ ppm 1.50 - 1.61 (m, 1 H) 1.68 - 1.79 (m, 1 H) 1.90 (br dd, J=13.70, 6.32 Hz, 1 H) 1.97 - 2.06 (m, 2 H) 2.13 - 2.25 (m, 1 H) 2.34 (dd, J=9.63, 3.63 Hz, 1 H) 2.40 - 2.47 (m, 2 H) 2.58 - 2.65 (m, 1 H) 2.69 (dd, J=9.63, 6.13 Hz, 1 H) 3.31 (s, 2 H) 3.66 - 3.78 (m, 3 H) 4.04 (br t, J=5.94 Hz, 1 H) 4.12 - 4.24 (m, 3 H) 6.82 (s, 1 H) 7.18 (d, J=7.50 Hz, 2 H) 7.46 - 7.57 (m, 2 H) 8.02 (dd, J=8.00, 1.88 Hz, 1 H) 8.19 (d, J=8.00 Hz, 1 H) 8.27 (d, J=8.25 Hz, 1 H) 8.52 (dd, J=8.25, 1.25 Hz, 1 H) 8.67 (s, 1 H) 9.57 (s, 1 H) 10.72 (s, 1 H).

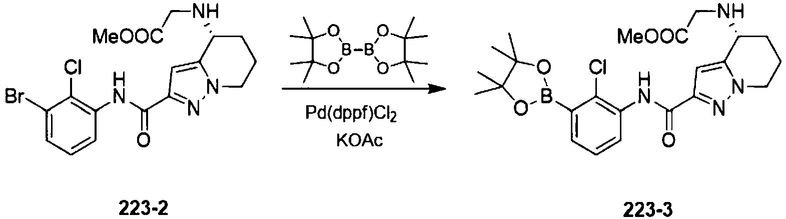

A mixture of methyl 2-[[(4R)-2-[(3-bromo-2-chloro-phenyl)carbamoyl]-4,5,6,7-tetrahydropyrazolo[1,5-a]pyridin-4-yl]amino]acetate (230 mg, 520.71 µmol, 1 eq), 4,4,5,5-tetramethyl-2-(4,4,5,5-tetramethyl-1,3,2-dioxaborolan-2-yl)-1,3,2-dioxaborolane (396.68 mg, 1.56 mmol, 3 eq), KOAc (153.31 mg, 1.56 mmol, 3 eq), and Pd(dppf)Cl₂·CH₂Cl₂ (63.78 mg, 78.11 µmol, 0.15 eq) in dioxane (7 mL) was stirred under a nitrogen atmosphere at 90 °C for 12 hours. After completion of the reaction, the mixture was concentrated under reduced pressure, and the residue was purified by prep-TLC (SiO₂, Petroleum ether/Ethyl acetate = 0/1) to afford Compound 223-3 (250 mg, 76.6% yield) as a white solid.

LCMS m/z 489.3 [M+1]⁺.

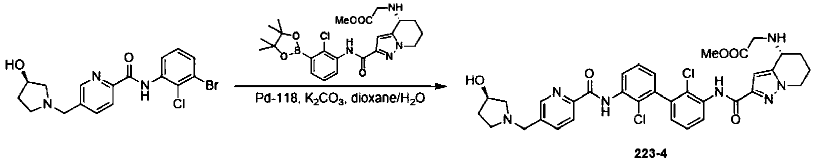

A mixture of N-(3-bromo-2-chloro-phenyl)-5-[[(3R)-3-hydroxypyrrolidin-1-yl]methyl]pyridine-2-carboxamide (125 mg, 304.36 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (178.52 mg, 365.24 µmol, 1.2 eq), CsF (138.70 mg, 913.09 µmol, 33.71 µL, 3 eq), and ditert-butyl(cyclopentyl)phosphane·dichloropalladium iron (19.84 mg, 30.44 µmol, 0.1 eq) in dioxane (5 mL) and H₂O (1 mL) was stirred under a nitrogen atmosphere at 90 °C for 12 hours. After completion of the reaction, the mixture was concentrated under reduced pressure, and the residue was purified by prep-TLC (SiO₂, ethyl acetate/methanol = 0/1) to afford Compound 223-4 (100 mg, 39.8% yield) as a white solid.

LCMS m/z 692.4 [M+1]⁺.

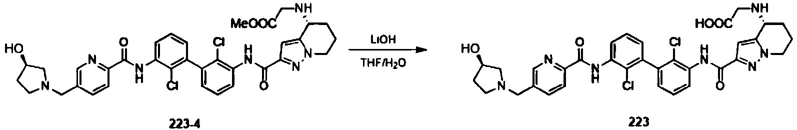

To a solution of methyl 2-[[(4R)-2-[[2-chloro-3-[2-chloro-3-[[5-[[(3R)-3-hydroxypyrrolidin-1-yl]methyl]pyridine-2-carbonyl]amino]phenyl]phenyl]carbamoyl]-4,5,6,7-tetrahydropyrazolo[1,5-a]pyridin-4-yl]amino]acetate (100 mg, 144.39 µmol, 1 eq) in THF (30 mL) / H₂O (15 mL), LiOH·H₂O (10.37 mg, 433.16 µmol, 3 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Waters Xbridge 150 * 25 mm * 5 µm; mobile phase: [water (NH₄HCO₃)-ACN]; gradient: 18% to 48% B over 9 min) to afford Compound 223 (78 mg, 78.8% yield) as a white solid.

LCMS m/z 678.3 [M+1]⁺.

¹H NMR (400 MHz, DMSO-d6) δ ppm 1.56 (br dd, J=8.19, 4.82 Hz, 1 H) 1.76 (br d, J=7.38 Hz, 1 H) 1.90 (br d, J=13.63 Hz, 1 H) 2.01 (br dd, J=12.69, 6.82 Hz, 2 H) 2.19 (br s, 1 H) 2.35 (dd, J=9.63, 3.63 Hz, 1 H) 2.41 - 2.48 (m, 2 H) 2.60 - 2.66 (m, 1 H) 2.70 (dd, J=9.94, 6.19 Hz, 1 H) 3.33 (s, 2 H) 3.66 - 3.78 (m, 3 H) 4.05 (br t, J=5.88 Hz, 1 H) 4.11 - 4.23 (m, 3 H) 6.83 (s, 1 H) 7.19 (d, J=7.50 Hz, 2 H) 7.48 - 7.57 (m, 2 H) 8.03 (dd, J=8.00, 1.88 Hz, 1 H) 8.20 (d, J=8.13 Hz, 1 H) 8.28 (d, J=8.25 Hz, 1 H) 8.53 (dd, J=8.25, 1.38 Hz, 1 H) 8.66 - 8.70 (m, 1 H) 9.58 (s, 1 H) 10.72 (s, 1 H).

### [Preparation Example 53]

### Preparation of Compound 224, (S)-N-(2,2'-dichloro-3'-(5-(((2-hydroxyethyl)amino)methyl)picolinamido)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide, and Compound 225, (R)-N-(2,2'-dichloro-3'-(5-(((2-hydroxyethyl)amino)methyl)picolinamido)-[1,1'-biphenyl]-3-yl)-4-((((S)-5-oxopyrrolidin-2-yl)methyl)amino)-4,5,6,7-tetrahydropyrazolo[1,5-a]pyridine-2-carboxamide

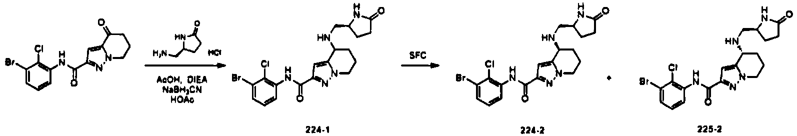

To a solution of N-(3-bromo-2-chloro-phenyl)-4-oxo-6,7-dihydro-5H-pyrazolo[1,5-a]pyridine-2-carboxamide (1 g, 2.71 mmol, 1 eq), and (5S)-5-(aminomethyl)pyrrolidin-2-one·HCl (1.55 g, 10.28 mmol, 3.79 eq) in MeOH (20 mL), AcOH (325.83 mg, 5.43 mmol, 310.61 µL, 2 eq), DIPEA (1.75 g, 13.56 mmol, 2.36 mL, 3.79 eq), and NaBH₃CN (852.41 mg, 13.56 mmol, 5 eq) were added. The resulting mixture was stirred at 60 °C for 12 hours. After completion of the reaction, the mixture was concentrated under reduced pressure, and the residue was purified by prep-HPLC (basic condition; column: Phenomenex Luna C18, 250 * 70 mm * 10 µm; mobile phase: [water (NH₄HCO₃)-ACN]; gradient: 40% to 70% B over 20 min) to afford Compound 224-1 (1 g, 78.9% yield) as a white solid. The product was further purified by SFC (column: DAICEL CHIRALPAK AD, 250 * 30 mm * 10 µm; mobile phase: CO₂-ACN/i-PrOH (0.1% NH₃·H₂O); B%: 55%, isocratic elution) to separate compound 224-2 (477 mg, 47.7% yield) and 225-2 (450 mg, 45.0% yield) as white solids.

LCMS m/z 467.1 [M+1]⁺.

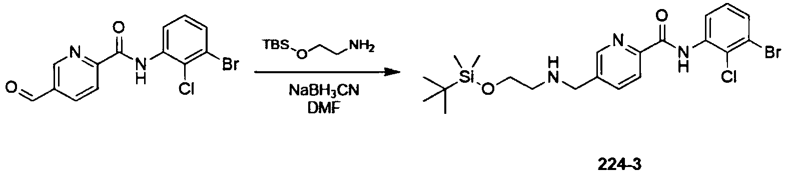

To a solution of N-(3-bromo-2-chloro-phenyl)-5-formyl-pyridine-2-carboxamide (1.2 g, 3.53 mmol, 1 eq) and 2-[tert-butyl(dimethyl)silyl]oxyethanamine (3.10 g, 17.67 mmol, 5 eq) in DMF (20 mL), AcOH (424.42 mg, 7.07 mmol, 404.59 µL, 2 eq) and NaBH₃CN (1.11 g, 17.67 mmol, 5 eq) were added. The resulting mixture was stirred at room temperature for 12 hours. After completion of the reaction, the mixture was concentrated under reduced pressure, and the residue was purified by column chromatography (SiO₂, Petroleum ether/Ethyl acetate = 3/1) to afford Compound 224-3 (1.4 g, 63.5% yield) as a white solid.

LCMS m/z 499.1 [M+1]⁺.

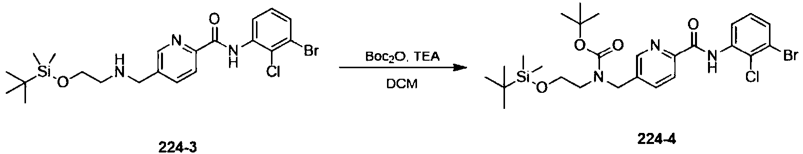

To a solution of N-(3-bromo-2-chloro-phenyl)-5-[[2-[tert-butyl(dimethyl)silyl]oxyethylamino]methyl]pyridine-2-carboxamide (1.2 g, 2.41 mmol, 1 eq) and tert-butoxycarbonyl tert-butyl carbonate (1.05 g, 4.81 mmol, 1.11 mL, 2 eq) in DCM (15 mL), TEA (486.77 mg, 4.81 mmol, 669.56 µL, 2 eq) was added. The resulting mixture was stirred at room temperature for 12 hours. After completion of the reaction, the mixture was concentrated under reduced pressure, and the residue was purified by column chromatography (SiO₂, Petroleum ether/Ethyl acetate = 5/1) to afford Compound 224-4 (790 mg, 53.1% yield) as a white solid.

LCMS m/z 600.2 [M+1]⁺.

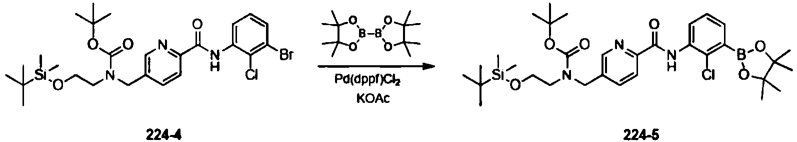

A mixture of tert-Butyl N-[[6-[(3-bromo-2-chloro-phenyl)carbamoyl]-3-pyridyl]methyl]-N-[2-[tert-butyl(dimethyl)silyl]oxyethyl]carbamate (500 mg, 834.68 µmol, 1 eq), 4,4,5,5-tetramethyl-2-(4,4,5,5-tetramethyl-1,3,2-dioxaborolan-2-yl)-1,3,2-dioxaborolane (317.94 mg, 1.25 mmol, 1.5 eq), AcOK (245.75 mg, 2.50 mmol, 3 eq), and Pd(dppf)Cl₂ (61.07 mg, 83.47 µmol, 0.1 eq) in dioxane (10 mL) was stirred under a nitrogen atmosphere at 90 °C for 12 hours. The mixture was then filtered, and the filtrate was concentrated under reduced pressure. The residue was purified by prep-TLC (SiO₂, Petroleum ether/Ethyl acetate = 5/1) to afford Compound 224-5 (300 mg, 47.2% yield) as a white solid.

LCMS m/z 646.4 [M+1]⁺.

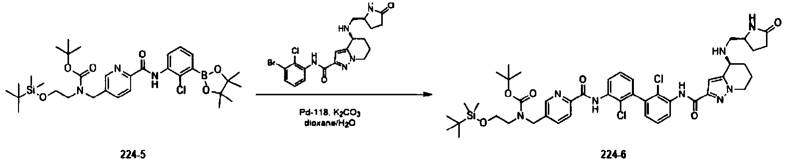

A mixture of (4S)-N-(3-bromo-2-chloro-phenyl)-4-[[(2S)-5-oxopyrrolidin-2-yl]methylamino]-4,5,6,7-tetrahydropyrazolo[1,5-a]pyridine-2-carboxamide (110 mg, 235.67 µmol, 1 eq) and tert-butyl N-[2-[tert-butyl(dimethyl)silyl]oxyethyl]-N-[[6-[[2-chloro-3-(4,4,5,5-tetramethyl-1,3,2-dioxaborolan-2-yl)phenyl]carbamoyl]-3-pyridyl]methyl]carbamate (182.72 mg, 282.80 µmol, 1.2 eq), K₂CO₃ (65.14 mg, 471.34 µmol, 2 eq), and ditert-butyl(cyclopentyl)phosphane·dichloropalladium iron (15.36 mg, 23.57 µmol, 0.1 eq) in dioxane (5 mL) and H₂O (1 mL) was stirred under a nitrogen atmosphere at 80 °C for 4 hours. The mixture was then filtered, and the filtrate was concentrated under reduced pressure. The residue was purified by prep-TLC (SiO₂, ethyl acetate/methanol = 5:1) to afford Compound 224-6 (170 mg, 55.7% yield) as a brown solid.

LCMS m/z 907.4 [M+1]⁺.

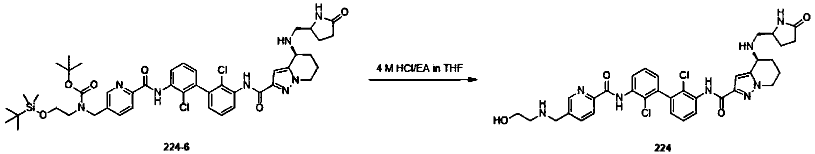

To a solution of tert-Butyl N-[2-[tert-butyl(dimethyl)silyl]oxyethyl]-N-[[6-[[2-chloro-3-[2-chloro-3-[[(4S)-4-[[(2S)-5-oxopyrrolidin-2-yl]methylamino]-4,5,6,7-tetrahydropyrazolo[1,5-a]pyridine-2-carbonyl]amino]phenyl]phenyl]carbamoyl]-3-pyridyl]methyl]carbamate (155 mg, 171.09 µmol, 1 eq) in THF (10 mL), HCl/EtOAc (4 M, 427.71 µL, 10 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Phenomenex Luna C18 150 * 25 mm * 10 µm; mobile phase: [water (HCl)-ACN]; gradient: 5% to 35% B over 10 min) to afford Compound 224 (72 mg, 60.8% yield) as a yellow solid.

LCMS m/z 691.4 [M+1]⁺.

¹H NMR (400 MHz, DMSO-d6) δ ppm 1.78 - 1.89 (m, 1 H) 1.97 - 2.05 (m, 1 H) 2.13 - 2.26 (m, 4 H) 2.35 - 2.44 (m, 1 H) 2.96 - 3.11 (m, 3 H) 3.18 (br s, 1 H) 3.72 (t, J=5.25 Hz, 2 H) 4.22 (br t, J=5.75 Hz, 2 H) 4.34 (br t, J=5.25 Hz, 2 H) 4.72 (br s, 1 H) 7.20 (d, J=7.50 Hz, 2 H) 7.32 (s, 1 H) 7.47 - 7.57 (m, 2 H) 8.03 (s, 1 H) 8.21 (br d, J=8.13 Hz, 1 H) 8.27 (d, J=8.00 Hz, 1 H) 8.34 (dd, J=8.13, 1.63 Hz, 1 H) 8.47 (d, J=7.13 Hz, 1 H) 8.92 (s, 1 H) 9.62 (br s, 1 H) 9.64 (s, 1 H) 9.92 (br s, 2 H) 10.71 (s, 1 H).

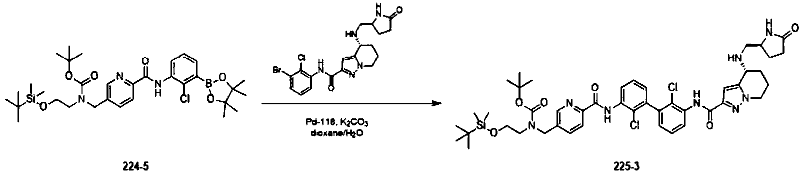

A mixture of (4R)-N-(3-bromo-2-chloro-phenyl)-4-[[(2S)-5-oxopyrrolidin-2-yl]methylamino]-4,5,6,7-tetrahydropyrazolo[1,5-a]pyridine-2-carboxamide (180 mg, 385.64 µmol, 1 eq) and tert-butyl N-[2-[tert-butyl(dimethyl)silyl]oxyethyl]-N-[[6-[[2-chloro-3-(4,4,5,5-tetramethyl-1,3,2-dioxaborolan-2-yl)phenyl]carbamoyl]-3-pyridyl]methyl]carbamate (298.99 mg, 462.77 µmol, 1.2 eq), K₂CO₃ (106.60 mg, 771.28 µmol, 2 eq), and ditert-butyl(cyclopentyl)phosphane·dichloropalladium iron (25.13 mg, 38.56 µmol, 0.1 eq) in dioxane (5 mL) and H₂O (1 mL) was stirred under a nitrogen atmosphere at 80 °C for 4 hours. The mixture was then filtered, and the filtrate was concentrated under reduced pressure. The residue was purified by prep-TLC (SiO₂, ethyl acetate/methanol = 5:1) to afford Compound 225-3 (180 mg, 36.0% yield) as a brown solid.

LCMS m/z 466.1 [M+1]⁺.

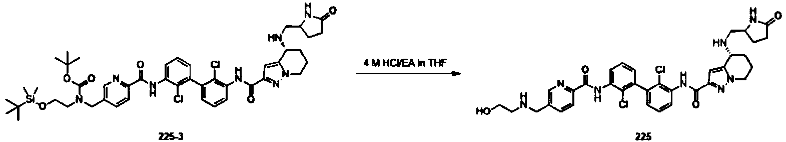

To a solution of tert-Butyl N-[2-[tert-butyl(dimethyl)silyl]oxyethyl]-N-[[6-[[2-chloro-3-[2-chloro-3-[[(4R)-4-[[(2S)-5-oxopyrrolidin-2-yl]methylamino]-4,5,6,7-tetrahydropyrazolo[1,5-a]pyridine-2-carbonyl]amino]phenyl]phenyl]carbamoyl]-3-pyridyl]methyl]carbamate (179 mg, 197.58 µmol, 1 eq) in THF (10 mL), HCl/EtOAc (4 M, 493.94 µL, 10 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Phenomenex Luna C18 150 * 25 mm * 10 µm; mobile phase: [water (HCl)-ACN]; gradient: 5% to 35% B over 10 min) to afford Compound 225 (62 mg, 45.3% yield) as a yellow solid.

LCMS m/z 693.3 [M+1]⁺.

¹H NMR (400 MHz, DMSO-d6) δ ppm 1.74 - 1.84 (m, 1 H) 1.96 - 2.08 (m, 1 H) 2.16 - 2.26 (m, 4 H) 2.32 - 2.39 (m, 1 H) 2.98 - 3.11 (m, 3 H) 3.16 (br s, 1 H) 3.72 (t, J=5.19 Hz, 2 H) 4.22 (br t, J=5.75 Hz, 2 H) 4.35 (br t, J=5.19 Hz, 2 H) 4.74 (br s, 1 H) 7.20 (d, J=6.88 Hz, 2 H) 7.30 (s, 1 H) 7.47 - 7.57 (m, 2 H) 7.99 (s, 1 H) 8.21 (br d, J=8.13 Hz, 1 H) 8.27 (d, J=8.00 Hz, 1 H) 8.34 (dd, J=8.13, 1.63 Hz, 1 H) 8.47 (d, J=7.38 Hz, 1 H) 8.92 (s, 1 H) 9.54 - 9.68 (m, 3 H) 9.89 (br s, 1 H) 10.10 (br s, 1 H) 10.71 (s, 1 H).

### [Preparation Example 54]

### Preparation of Compound 226, 1-((S)-2-((2,2'-dichloro-3'-((3-(((S)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid, and Compound 227, 1-((R)-2-((2,2'-dichloro-3'-((3-(((S)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid

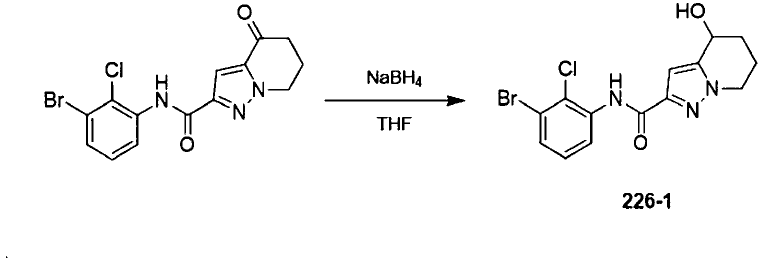

To a solution of N-(3-bromo-2-chloro-phenyl)-4-oxo-6,7-dihydro-5H-pyrazolo[1,5-a]pyridine-2-carboxamide (3 g, 8.14 mmol, 1 eq) in THF (50 mL), NaBH₄ (1.02 g, 16.28 mmol, 2 eq) was added. The resulting mixture was stirred at room temperature for 16 hours. Aqueous 4 N HCl (50 mL) was then added, and the mixture was extracted with EA (50 mL * 3). The combined organic layers were dried over Na₂SO₄ and concentrated under reduced pressure to afford Compound 226-1 (2.9 g, 76.9% yield) as a yellow solid, which was used in the next reaction without further purification.

LCMS m/z 368.99 [M+2]⁺.

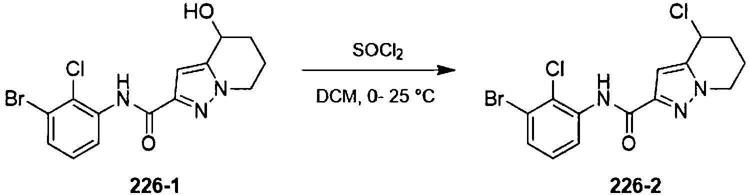

To a solution of N-(3-bromo-2-chloro-phenyl)-4-hydroxy-4,5,6,7-tetrahydropyrazolo[1,5-a]pyridine-2-carboxamide (2.9 g, 7.82 mmol, 1 eq) in DCM (30 mL), SOCl₂ (4.65 g, 39.12 mmol, 2.84 mL, 5 eq) was slowly added. The resulting mixture was stirred at room temperature for 12 hours. Aq. saturated NH₄Cl (200 mL) was added, and the mixture was extracted with EtOAc (200 mL * 2). The combined organic layers were washed with brine (200 mL), dried over Na₂SO₄, and concentrated under reduced pressure to afford Compound 226-2 (2.6 g, 68.3% yield) as a yellow solid, which was used in the next reaction without further purification.

LCMS m/z 386.95 [M+2]⁺.

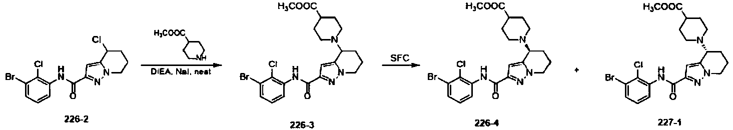

A mixture of N-(3-bromo-2-chloro-phenyl)-4-chloro-4,5,6,7-tetrahydropyrazolo[1,5-a]pyridine-2-carboxamide (1 g, 2.57 mmol, 1 eq), methyl piperidine-4-carboxylate (1.10 g, 7.71 mmol, 3 eq), DIPEA (996.54 mg, 7.71 mmol, 1.34 mL, 3 eq), and Nal (115.58 mg, 771.06 µmol, 0.3 eq) was stirred at 40 °C for 12 hours. After completion of the reaction, the mixture was concentrated under reduced pressure, and the residue was purified by reversed-phase HPLC (column: Kromasil Eternity XT 250 * 80 mm * 10 µm; mobile phase: [water (NH₄HCO₃)-ACN]; gradient: 55% to 85% B over 20 min), followed by SFC (column: DAICEL CHIRALPAK AS, 250 * 30 mm * 10 µm; mobile phase: CO₂/i-PrOH (0.1% NH₃·H₂O); B%: 40%, isocratic elution) to afford Compound 226-4 (Rt = 71 min, 370 mg, 28.5% yield) and compound 227-1 (Rt = 2.053 min, 320 mg, 24.6% yield) as yellow solids.

LCMS m/z 494.07 [M+2]⁺.

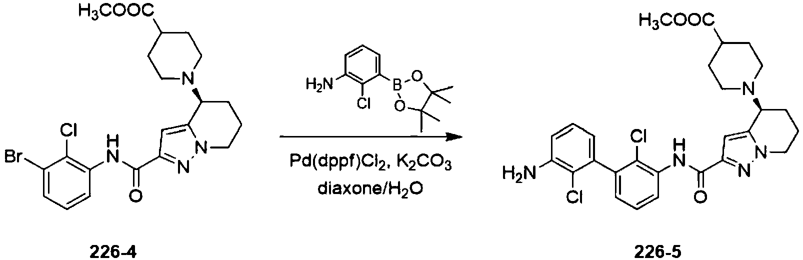

A mixture of methyl 1-[(4S)-2-[(3-bromo-2-chloro-phenyl)carbamoyl]-4,5,6,7-tetrahydropyrazolo[1,5-a]pyridin-4-yl]piperidine-4-carboxylate (226-4, 370 mg, 746.27 µmol, 1 eq), 2-chloro-3-(4,4,5,5-tetramethyl-1,3,2-dioxaborolan-2-yl)aniline (283.81 mg, 1.12 mmol, 1.5 eq), Pd(dppf)Cl₂ (54.61 mg, 74.63 µmol, 0.1 eq), and K₂CO₃ (309.43 mg, 2.24 mmol, 3 eq) in dioxane (2.5 mL) and H₂O (0.5 mL) was stirred under a nitrogen atmosphere at 90 °C for 4 hours. After completion of the reaction, the mixture was concentrated under reduced pressure, and the residue was purified by column chromatography (SiO₂, Petroleum ether/Ethyl acetate = 10/1) to afford Compound 226-5 (320 mg, 77.4% yield) as a yellow solid.

LCMS m/z 541.16 [M+1]⁺.

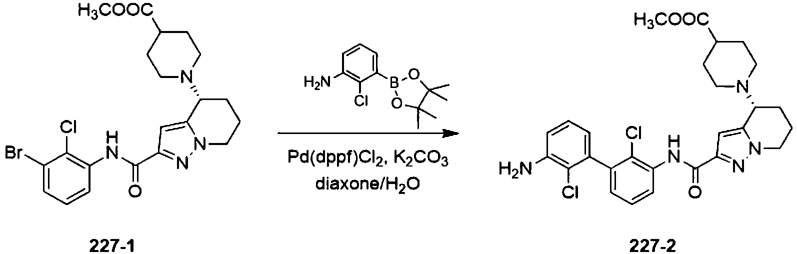

A mixture of methyl 1-[(4R)-2-[(3-bromo-2-chloro-phenyl)carbamoyl]-4,5,6,7-tetrahydropyrazolo[1,5-a]pyridin-4-yl]piperidine-4-carboxylate (227-1, 200.00 mg, 403.39 µmol, 1 eq), 2-chloro-3-(4,4,5,5-tetramethyl-1,3,2-dioxaborolan-2-yl)aniline (153.41 mg, 605.09 µmol, 1.5 eq), Pd(dppf)Cl₂ (29.52 mg, 40.34 µmol, 0.1 eq), and K₂CO₃ (167.26 mg, 1.21 mmol, 3 eq) in dioxane (2.5 mL) and H₂O (0.5 mL) was stirred under a nitrogen atmosphere at 90 °C for 4 hours. After completion of the reaction, the mixture was concentrated under reduced pressure, and the residue was purified by column chromatography (SiO₂, Petroleum ether/Ethyl acetate = 10/1) to afford Compound 227-2 (205 mg, 89.9% yield) as a yellow solid.

LCMS m/z 541.16 [M+1]⁺.

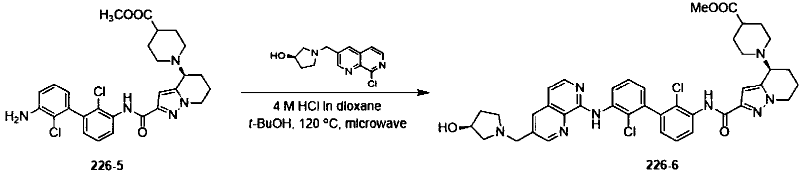

To a solution of methyl 1-[(4S)-2-[[3-(3-amino-2-chloro-phenyl)-2-chloro-phenyl]carbamoyl]-4,5,6,7-tetrahydropyrazolo[1,5-a]pyridin-4-yl]piperidine-4-carboxylate (320 mg, 589.91 µmol, 1 eq) in t-BuOH (10 mL), (3R)-1-[(8-chloro-1,7-naphthyridin-3-yl)methyl]pyrrolidin-3-ol (466.72 mg, 1.77 mmol, 3 eq) and 4 M HCl in dioxane (0.5 mL) were added. The resulting mixture was subjected to microwave irradiation at 120 °C for 8 hours. After completion of the reaction, the mixture was concentrated under reduced pressure, and the precipitated solid was dissolved in MeOH and concentrated again under reduced pressure to afford Compound 226-6 (400 mg, 88.0% yield) as a brown solid, which was used in the next reaction without further purification.

LCMS m/z 768.27 [M+1]⁺.

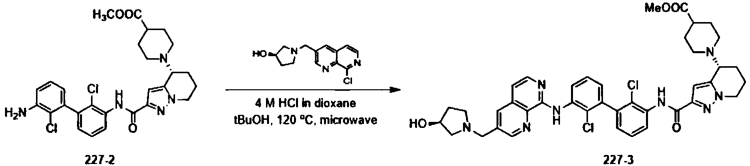

To a solution of methyl 1-[(4R)-2-[[3-(3-amino-2-chloro-phenyl)-2-chloro-phenyl]carbamoyl]-4,5,6,7-tetrahydropyrazolo[1,5-a]pyridin-4-yl]piperidine-4-carboxylate (205 mg, 377.91 µmol, 1 eq) in t-BuOH (10 mL), (3R)-1-[(8-chloro-1,7-naphthyridin-3-yl)methyl]pyrrolidin-3-ol (466.72 mg, 1.77 mmol, 3 eq) and 4 M HCl in dioxane (0.5 mL) were added. The resulting mixture was subjected to microwave irradiation at 120 °C for 8 hours. After completion of the reaction, the mixture was concentrated under reduced pressure, and the precipitated solid was dissolved in MeOH and concentrated again under reduced pressure to afford Compound 227-3 (200 mg, 68.76% yield) as a brown solid, which was used in the next reaction without further purification.

LCMS m/z 768.27 [M+1]⁺.

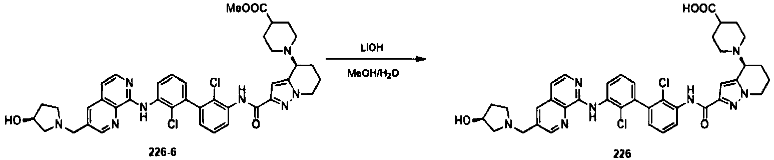

A mixture of Methyl 1-[(4S)-2-[[2-chloro-3-[2-chloro-3-[[3-[[(3S)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]piperidine-4-carboxylate (120 mg, 155.90 µmol, 1 eq) and LiOH (32.71 mg, 779.51 µmol, 5 eqin H₂O (4 mL) / MeOH (20 mL) was stirred under a nitrogen atmosphere at room temperature for 12 hours. After completion of the reaction, the mixture was concentrated under reduced pressure, and the residue was purified by reversed-phase HPLC (column: Waters XBridge Prep OBD C18 150 * 40 mm * 10 µm; mobile phase: [water (NH₄HCO₃)-ACN]; B: 20%, isocratic elution) to afford Compound 226 (30 mg, 25.2% yield) as a yellow solid.

LCMS m/z 754.25 [M+1]⁺.

¹H NMR (400 MHz, CHLOROFORM-d) δ ppm 1.63 - 2.09 (m, 9 H) 2.16 - 2.42 (m, 4 H) 2.45 - 2.59 (m, 2 H) 2.71 - 2.77 (m, 1 H) 2.78 - 2.85 (m, 2 H) 2.88 - 2.95 (m, 1 H) 3.06 - 3.13 (m, 2 H) 3.84 - 4.06 (m, 4 H) 4.13 - 4.29 (m, 1 H) 4.38 - 4.49 (m, 1 H) 6.86 (s, 1 H) 6.95 (d, J=7.50 Hz, 1 H) 7.06 (d, J=5.88 Hz, 2 H) 7.41 (dt, J=16.20, 8.04 Hz, 2 H) 8.01 (br d, J=9.26 Hz, 1 H) 8.20 (d, J=5.88 Hz, 1 H) 8.63 (t, J=7.88 Hz, 1 H) 8.81 - 8.95 (m, 1 H) 9.12 (d, J=8.38 Hz, 1 H) 9.46 (d, J=15.26 Hz, 1 H) 9.93 (s, 1 H).

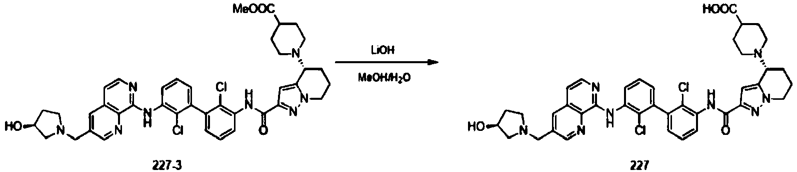

A mixture of methyl 1-[(4R)-2-[[2-chloro-3-[2-chloro-3-[[3-[[(3S)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]piperidine-4-carboxylate (200 mg, 259.84 µmol, 1 eq) and LiOH (32.71 mg, 779.51 µmol, 3 eq) in H₂O (4 mL) and MeOH (20 mL) was stirred under a nitrogen atmosphere at room temperature for 12 hours. After completion of the reaction, the mixture was concentrated under reduced pressure, and the residue was purified by reversed-phase HPLC (column: Waters XBridge Prep OBD C18 150 * 40 mm * 10 µm; mobile phase: [water (NH₄HCO₃)-ACN]; B: 20%, isocratic elution) to afford Compound 227 (68 mg, 33.9% yield) as a yellow solid.

LCMS m/z 754.25 [M+1]⁺.

¹H NMR (400 MHz, DMSO-d6) δ ppm 1.50 - 1.72 (m, 4 H) 1.78 - 1.87 (m, 2 H) 1.87 - 1.93 (m, 1 H) 1.95 - 2.06 (m, 2 H) 2.12 - 2.35 (m, 4 H) 2.39 (dd, J=9.76, 3.63 Hz, 1 H) 2.49 (br s, 2 H) 2.65 - 2.70 (m, 2 H) 2.72 - 2.83 (m, 2 H) 3.77 - 3.90 (m, 2 H) 3.98 (br dd, J=9.88, 4.63 Hz, 1 H) 4.02 - 4.13 (m, 1 H) 4.16 - 4.27 (m, 2 H) 4.73 (br d, J=3.63 Hz, 1 H) 6.68 (s, 1 H) 7.04 (d, J=7.63 Hz, 1 H) 7.21 (d, J=7.75 Hz, 1 H) 7.36 (d, J=5.88 Hz, 1 H) 7.46 - 7.57 (m, 2 H) 8.20 (d, J=5.88 Hz, 1 H) 8.24 - 8.33 (m, 2 H) 8.93 (d, J=1.38 Hz, 1 H) 9.11 - 9.18 (m, 1 H) 9.56 (s, 1 H) 9.90 (s, 1 H).

### [Preparation Example 55]

### Preparation of Compounds 228 to 240

### 55-1. Preparation of Compound 228, (3R)-1-(2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)pyrrolidine-3-carboxylic acid

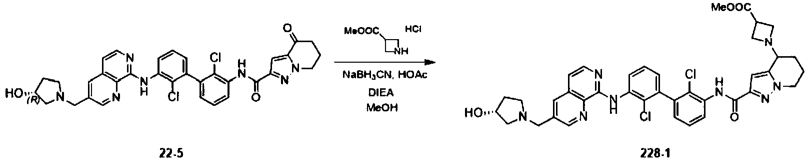

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (60 mg, 93.38 µmol, 1 eq) and methyl azetidine-3-carboxylate·HCl (283.11 mg, 1.87 mmol, 20 eq) in MeOH (20 mL), AcOH (8.97 mg, 149.41 µmol, 8.55 µL, 1.60 eq), DIPEA (241.38 mg, 1.87 mmol, 325.30 µL, 20 eq), and NaBH₃CN (29.34 mg, 466.90 µmol, 5 eq). The resulting mixture was stirred at 60 °C for 4 hours. The mixture was cooled to room temperature, concentrated under reduced pressure, and the residue was purified by reversed-phase HPLC (0.1% NH₃·H₂O; column: Waters XBridge 150 * 25 mm * 5 µm; mobile phase: [water (NH₄HCO₃)-ACN]; gradient: 55% to 85% B over 9 min) to afford Compound 228-1 (32 mg, 45.7% yield) as a white solid.

LCMS m/z 741.3 [M+1]⁺.

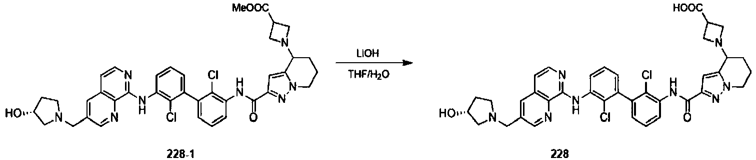

To a solution of methyl 1-[2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]azetidine-3-carboxylate (32 mg, 43.15 µmol, 1 eq) in THF (25 mL) / H₂O (10 mL), LiOH (3.10 mg, 129.44 µmol, 3 eq) was added. The resulting mixture was stirred at room temperature for 12 hours. After completion of the reaction, the mixture was concentrated under reduced pressure, and the residue was purified by reversed-phase HPLC (0.1% NH₃·H₂O; column: Phenomenex Luna C18 150 * 25 mm * 10 µm; mobile phase: [water (NH₄HCO₃)-ACN]; gradient: 24% to 54% B over 10 min) to afford Compound 228 (30 mg, 91.7% yield) as a white solid.

LCMS m/z 727.2 [M+1]⁺.

¹H NMR (400 MHz, DMSO-d6) δ ppm 1.52 - 1.62 (m, 1 H) 1.74 (br s, 2 H) 1.80 - 1.89 (m, 1 H) 1.95 - 2.09 (m, 1 H) 2.13 - 2.24 (m, 1 H) 2.38 (dd, J=9.57, 3.56 Hz, 1 H) 2.43 - 2.48 (m, 1 H) 2.62 - 2.69 (m, 1 H) 2.74 (br dd, J=9.51, 6.25 Hz, 1 H) 3.15 (br d, J=7.13 Hz, 1 H) 3.19 - 3.24 (m, 3 H) 3.77 - 3.88 (m, 3 H) 4.04 - 4.12 (m, 1 H) 4.17 - 4.25 (m, 2 H) 4.74 (br s, 1 H) 6.75 (s, 1 H) 7.02 (dt, J=7.50, 1.38 Hz, 1 H) 7.20 (dd, J=7.57, 1.44 Hz, 1 H) 7.34 (d, J=5.88 Hz, 1 H) 7.51 (q, J=7.84 Hz, 2 H) 8.19 (d, J=5.88 Hz, 1 H) 8.23 - 8.30 (m, 2 H) 8.92 (d, J=1.63 Hz, 1 H) 9.13 (dd, J=8.38, 1.38 Hz, 1 H) 9.55 (s, 1 H) 9.88 (s, 1 H)

### 55-2. Preparation of Compound 229, 4-(bis(2-hydroxyethyl)amino)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridine-2-carboxamide

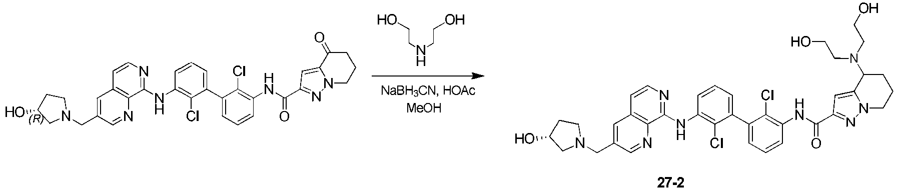

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (50 mg, 77.82 µmol, 1 eq) and 2-aminopropane-1,3-diol (141.80 mg, 1.56 mmol, 20 eq) in MeOH (20 mL), AcOH (9.35 mg, 155.63 µmol, 8.91 µL, 2 eq) and NaBH₃CN (24.45 mg, 389.08 µmol, 5 eq) were added. The resulting mixture was stirred at 60 °C for 36 hours. The mixture was cooled to room temperature, concentrated under reduced pressure, and the residue was purified by reversed-phase HPLC (0.1% NH₃·H₂O; column: Waters XBridge 150 * 25 mm * 5 µm; mobile phase: [water (NH₄HCO₃)-ACN]; gradient: 38% to 68% B over 9 min) to afford Compound 229 (34 mg, 59.6% yield) as a white solid.

LCMS m/z 717.3 [M+1]⁺.

¹H NMR (EW40143-351-P1H1, 400 MHz, DMSO-d6) δ ppm 1.60 (br d, *J*=8.00 Hz, 2 H) 1.92 (br s, 2 H) 2.01 (br d, *J*=13.01 Hz, 2 H) 2.16 (br s, 2 H) 2.38 (br d, *J*=9.63 Hz, 2 H) 2.66 (br d, *J*=8.13 Hz, 2 H) 2.71 - 2.81 (m, 4 H) 3.82 (br d, *J*=9.26 Hz, 2 H) 4.03 (br s, 1 H) 4.14 (br s, 2 H) 4.22 (br s, 1 H) 4.49 - 4.54 (m, 2 H) 4.74 (br d, *J*=4.25 Hz, 1 H) 6.80 - 6.86 (m, 1 H) 7.03 (br d, *J*=7.38 Hz, 1 H) 7.20 (br d, *J*=7.50 Hz, 1 H) 7.35 (br d, *J*=5.63 Hz, 1 H) 7.51 (q, *J*=8.05 Hz, 2 H) 8.19 (br d, *J*=5.75 Hz, 1 H) 8.25 (br s, 1 H) 8.30 (br d, *J*=8.38 Hz, 1 H) 8.92 (s, 1 H) 9.13 (br d, *J*=7.88 Hz, 1 H) 9.55 (s, 1 H) 9.89 (s, 1 H)

### 55-3. Preparation of Compound 230, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((1,3-dihydroxypropan-2-yl)amino)-4,5,6,7-tetrahydropyrazolo[1,5-a]pyridine-2-carboxamide

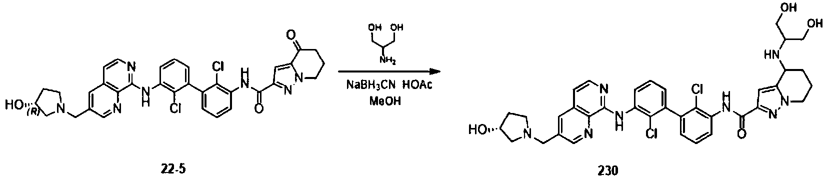

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (50 mg, 77.82 µmol, 1 eq) and 2-aminopropane-1,3-diol (141.80 mg, 1.56 mmol, 20 eq) in MeOH (20 mL), AcOH (9.35 mg, 155.63 µmol, 8.91 µL, 2 eq) and NaBH₃CN (24.45 mg, 389.08 µmol, 5 eq) were added. The resulting mixture was stirred at 60 °C for 36 hours. After completion of the reaction, the mixture was filtered, and the filtrate was concentrated under reduced pressure. The residue was purified by reversed-phase HPLC (0.1% NH₃·H₂O; column: Waters XBridge 150 * 25 mm * 5 µm; mobile phase: [water (NH₄HCO₃)-ACN]; gradient: 38% to 68% B over 9 min) to afford Compound 230 (34 mg, 59.6% yield) as a white solid.

LCMS m/z 717.3 [M+1]⁺.

¹H NMR (400 MHz, DMSO-d6) δ ppm 1.60 (br d, J=8.00 Hz, 2 H) 1.92 (br s, 2 H) 2.01 (br d, J=13.01 Hz, 2 H) 2.16 (br s, 2 H) 2.38 (br d, J=9.63 Hz, 2 H) 2.66 (br d, J=8.13 Hz, 2 H) 2.71 - 2.81 (m, 4 H) 3.82 (br d, J=9.26 Hz, 2 H) 4.03 (br s, 1 H) 4.14 (br s, 2 H) 4.22 (br s, 1 H) 4.49 - 4.54 (m, 2 H) 4.74 (br d, J=4.25 Hz, 1 H) 6.80 - 6.86 (m, 1 H) 7.03 (br d, J=7.38 Hz, 1 H) 7.20 (br d, J=7.50 Hz, 1 H) 7.35 (br d, J=5.63 Hz, 1 H) 7.51 (q, J=8.05 Hz, 2 H) 8.19 (br d, J=5.75 Hz, 1 H) 8.25 (br s, 1 H) 8.30 (br d, J=8.38 Hz, 1 H) 8.92 (s, 1 H) 9.13 (br d, J=7.88 Hz, 1 H) 9.55 (s, 1 H) 9.89 (s, 1 H).

### 55-4. Preparation of Compound 231, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((tetrahydro-2H-pyran-4-yl)amino)-4,5,6,7-tetrahydropyrazolo[1,5-a]pyridine-2-carboxamide

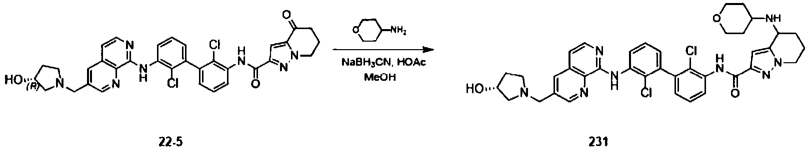

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (100 mg, 155.63 µmol, 1 eq) and tetrahydropyran-4-amine (157.42 mg, 1.56 mmol, 20 eq) in MeOH (20 mL), AcOH (18.69 mg, 311.27 µmol, 17.82 µL, 2 eq) and NaBH₃CN (48.90 mg, 778.17 µmol, 5 eq) were added. The resulting mixture was stirred under a nitrogen atmosphere at 60 °C for 24 hours. After completion of the reaction, the mixture was filtered, and the filtrate was concentrated under reduced pressure. The residue was purified by reversed-phase HPLC (0.1% NH₃·H₂O; column: Phenomenex Luna C18 150 * 25 mm * 10 µm; mobile phase: [water (NH₄HCO₃)-ACN]; gradient: 56% to 86% B over 10 min) to afford Compound 231 (73 mg, 61.8% yield) as a white solid.

LCMS m/z 727.3 [M+1]⁺.

¹H NMR (400 MHz, DMSO-d6) δ ppm 1.20 - 1.36 (m, 2 H) 1.52 - 1.68 (m, 2 H) 1.79 (br d, J=12.13 Hz, 2 H) 1.90 (br dd, J=13.63, 7.00 Hz, 1 H) 1.96 - 2.09 (m, 3 H) 2.13 - 2.23 (m, 1 H) 2.30 - 2.41 (m, 1 H) 2.65 (q, J=7.63 Hz, 1 H) 2.73 (dd, J=9.63, 6.13 Hz, 1 H) 2.78 - 2.88 (m, 1 H) 3.75 - 3.89 (m, 4 H) 4.01 (br t, J=5.88 Hz, 1 H) 4.09 - 4.16 (m, 2 H) 4.21 (br d, J=2.63 Hz, 1 H) 4.74 (d, J=4.50 Hz, 1 H) 6.77 (s, 1 H) 7.02 (dd, J=7.63, 1.38 Hz, 1 H) 7.19 (dd, J=7.57, 1.31 Hz, 1 H) 7.33 (d, J=5.88 Hz, 1 H) 7.50 (q, J=7.63 Hz, 2 H) 8.18 (d, J=5.75 Hz, 1 H) 8.24 (s, 1 H) 8.30 (d, J=8.26 Hz, 1 H) 8.91 (d, J=1.75 Hz, 1 H) 9.13 (dd, J=8.44, 1.31 Hz, 1 H) 9.54 (s, 1 H) 9.88 (s, 1 H)

### 55-5. Preparation of Compound 232, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(3-hydroxyazetidin-1-yl)-4,5,6,7-tetrahydropyrazolo[1,5-a]pyridine-2-carboxamide

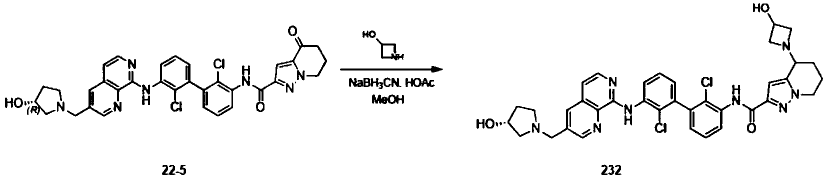

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (60 mg, 93.38 µmol, 1 eq) and azetidin-3-ol (136.51 mg, 1.87 mmol, 20 eq) in MeOH (20 mL), AcOH (11.21 mg, 186.76 µmol, 10.69 µL, 2 eq) and NaBH₃CN (29.34 mg, 466.90 µmol, 5 eq) were added. The resulting mixture was stirred under a nitrogen atmosphere at 60 °C for 4 hours. After completion of the reaction, the mixture was filtered, and the filtrate was concentrated under reduced pressure. The residue was purified by reversed-phase HPLC (0.1% NH₃·H₂O; column: Waters XBridge 150 * 25 mm * 5 µm; mobile phase: [water (NH₄HCO₃)-ACN]; gradient: 42% to 72% B over 9 min) to afford Compound 232 (49 mg, 73.5% yield) as a white solid.

LCMS m/z 699.2 [M+1]⁺.

¹H NMR (400 MHz, DMSO-d6) δ ppm 1.52 - 1.61 (m, 1 H) 1.67 - 1.79 (m, 2 H) 1.80 - 1.89 (m, 1 H) 1.96 - 2.04 (m, 1 H) 2.14 - 2.25 (m, 1 H) 2.38 (dd, J=9.63, 3.50 Hz, 1 H) 2.65 (q, J=7.84 Hz, 1 H) 2.74 (dd, J=9.63, 6.13 Hz, 1 H) 2.81 (t, J=6.44 Hz, 1 H) 3.01 (t, J=6.44 Hz, 1 H) 3.40 - 3.44 (m, 2 H) 3.48 - 3.54 (m, 2 H) 3.77 - 3.87 (m, 2 H) 4.04 - 4.11 (m, 1 H) 4.11 - 4.16 (m, 1 H) 4.18 - 4.25 (m, 2 H) 4.74 (d, J=4.50 Hz, 1 H) 5.35 (d, J=6.25 Hz, 1 H) 6.73 (s, 1 H) 7.02 (d, J=7.25 Hz, 1 H) 7.20 (dd, J=7.57, 1.31 Hz, 1 H) 7.34 (d, J=5.88 Hz, 1 H) 7.51 (q, J=7.63 Hz, 2 H) 8.19 (d, J=5.88 Hz, 1 H) 8.23 - 8.30 (m, 2 H) 8.91 (d, J=1.50 Hz, 1 H) 9.13 (dd, J=8.38, 1.38 Hz, 1 H) 9.55 (s, 1 H) 9.88 (s, 1 H)

### 55-6. Preparation of Compound 233, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((4-hydroxycyclohexyl)amino)-4,5,6,7-tetrahydropyrazolo[1,5-a]pyridine-2-carboxamideide

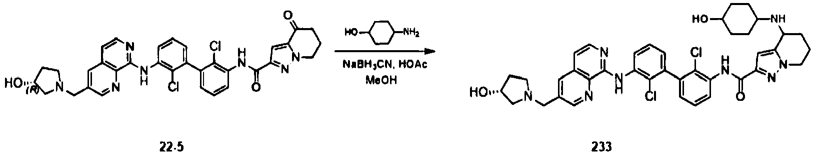

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (50 mg, 77.82 µmol, 1 eq) and 4-aminocyclohexanol (179.25 mg, 1.56 mmol, 20 eq) in MeOH (20 mL), AcOH (9.35 mg, 155.63 µmol, 8.91 µL, 2 eq) and NaBH₃CN (24.45 mg, 389.08 µmol, 5 eq) were added. The resulting mixture was stirred under a nitrogen atmosphere at 60 °C for 2 hours. After completion of the reaction, the mixture was filtered, and the filtrate was concentrated under reduced pressure. The residue was purified by reversed-phase HPLC (0.1% NH₃·H₂O; column: Waters XBridge 150 * 25 mm * 5 µm; mobile phase: [water (NH₄HCO₃)-ACN]; gradient: 48% to 78% B over 9 min) to afford Compound 233 (4 mg, 6.58% yield) as a white solid.

LCMS m/z 741.3 [M+1]⁺.

¹H NMR (400 MHz, DMSO-d6) δ ppm 1.07 - 1.24 (m, 3 H) 1.58 (br s, 2 H) 1.79 - 1.91 (m, 4 H) 2.07 (s, 4 H) 2.32 - 2.40 (m, 2 H) 2.62 - 2.69 (m, 2 H) 2.74 (br dd, J=9.51, 6.13 Hz, 2 H) 3.77 - 3.88 (m, 3 H) 3.95 (br s, 1 H) 4.09 - 4.17 (m, 2 H) 4.21 (br s, 1 H) 4.49 (d, J=4.38 Hz, 1 H) 4.74 (d, J=4.50 Hz, 1 H) 6.72 (s, 1 H) 7.03 (d, J=6.63 Hz, 1 H) 7.19 (d, J=7.75 Hz, 1 H) 7.35 (d, J=5.88 Hz, 1 H) 7.51 (q, J=8.34 Hz, 2 H) 8.19 (d, J=5.88 Hz, 1 H) 8.24 - 8.33 (m, 2 H) 8.92 (s, 1 H) 9.13 (d, J=8.25 Hz, 1 H) 9.55 (s, 1 H) 9.89 (s, 1 H)

### 55-7. Preparation of Compound 234, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((2-methoxyethyl)amino)-4,5,6,7-tetrahydropyrazolo[1,5-a]pyridine-2-carboxamide

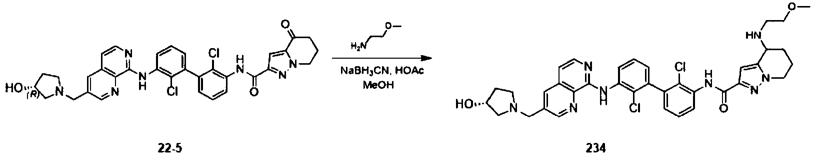

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (100 mg, 155.63 µmol, 1 eq) and 2-methoxyethanamine (233.79 mg, 3.11 mmol, 270.59 µL, 20 eq) in MeOH (20 mL), AcOH (18.69 mg, 311.27 µmol, 17.82 µL, 2 eq) and NaBH₃CN (48.90 mg, 778.17 µmol, 5 eq) were added. The resulting mixture was stirred under a nitrogen atmosphere at 60 °C for 2 hours. After completion of the reaction, the mixture was filtered, and the filtrate was concentrated under reduced pressure. The residue was purified by reversed-phase HPLC (0.1% NH₃·H₂O; column: Phenomenex Luna C18 150 * 25 mm * 10 µm; mobile phase: [water (NH₄HCO₃)-ACN]; gradient: 58% to 88% B over 10 min) to afford Compound 234 (58 mg, 52.5% yield) as a white solid.

LCMS m/z 701.3 [M+1]⁺.

¹H NMR (400 MHz, DMSO-d6) δ ppm 1.53 - 1.62 (m, 1 H) 1.63 - 1.74 (m, 1 H) 1.90 (br dd, J=13.76, 7.13 Hz, 1 H) 1.95 - 2.05 (m, 2 H) 2.07 (s, 1 H) 2.13 - 2.21 (m, 1 H) 2.38 (dd, J=9.57, 3.56 Hz, 1 H) 2.65 (q, J=7.75 Hz, 1 H) 2.71 - 2.79 (m, 3 H) 3.25 (s, 3 H) 3.40 - 3.44 (m, 3 H) 3.76 - 3.85 (m, 2 H) 3.89 (dd, J=11.69, 5.69 Hz, 1 H) 4.10 - 4.16 (m, 2 H) 4.18 - 4.26 (m, 1 H) 4.74 (d, J=4.63 Hz, 1 H) 6.76 (s, 1 H) 7.02 (dd, J=7.57, 1.44 Hz, 1 H) 7.19 (dd, J=7.57, 1.44 Hz, 1 H) 7.34 (d, J=5.88 Hz, 1 H) 7.50 (q, J=8.00 Hz, 2 H) 8.19 (d, J=5.75 Hz, 1 H) 8.25 (d, J=1.63 Hz, 1 H) 8.29 (d, J=8.13 Hz, 1 H) 8.91 (d, J=1.88 Hz, 1 H) 9.13 (dd, J=8.38, 1.38 Hz, 1 H) 9.55 (s, 1 H) 9.88 (s, 1 H).

### 55-8. Preparation of Compound 235, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-((2-(methylsulfonamido)ethyl)amino)-4,5,6,7-tetrahydropyrazolo[1,5-a]pyridine-2-carboxamide

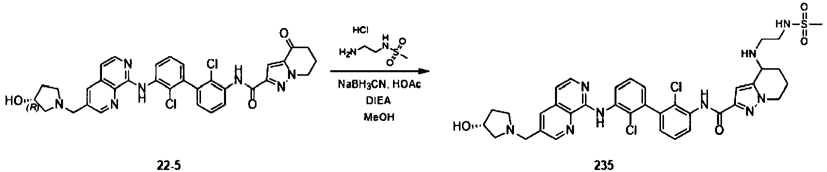

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (70 mg, 108.94 µmol, 1 eq) and N-(2-aminoethyl)methanesulfonamide (380.54 mg, 2.18 mmol, 20 eq, HCl) in MeOH (20 mL), AcOH (10.47 mg, 174.31 µmol, 9.98 µL, 1.60 eq), DIPEA (281.60 mg, 2.18 mmol, 379.52 µL, 20 eq), and NaBH₃CN (34.23 mg, 544.72 µmol, 5 eq) were added. The resulting mixture was stirred under a nitrogen atmosphere at 60 °C for 12 hours. After completion of the reaction, the mixture was filtered, and the filtrate was concentrated under reduced pressure. The residue was purified by reversed-phase HPLC (0.1% NH₃·H₂O; column: Phenomenex Luna C18 150 * 25 mm * 10 µm; mobile phase: [water (NH₄HCO₃)-ACN]; gradient: 48% to 78% B over 10 min) to afford Compound 235 (32 mg, 36.8% yield) as a white solid.

LCMS m/z 764.2 [M+1]⁺.

¹H NMR (400 MHz, DMSO-d6) δ ppm 1.52 - 1.62 (m, 1 H) 1.63 - 1.73 (m, 1 H) 1.90 (br dd, J=14.20, 7.32 Hz, 1 H) 1.98 - 2.08 (m, 2 H) 2.12 - 2.23 (m, 1 H) 2.38 (dd, J=9.57, 3.56 Hz, 1 H) 2.43 - 2.48 (m, 1 H) 2.61 - 2.69 (m, 1 H) 2.71 - 2.76 (m, 3 H) 2.91 (s, 3 H) 3.01 - 3.09 (m, 2 H) 3.74 - 3.92 (m, 3 H) 4.08 - 4.24 (m, 3 H) 4.74 (br d, J=4.13 Hz, 1 H) 6.81 (s, 1 H) 6.95 - 7.04 (m, 2 H) 7.19 (dd, J=7.63, 1.50 Hz, 1 H) 7.32 (d, J=5.88 Hz, 1 H) 7.46 - 7.54 (m, 2 H) 8.18 (d, J=5.75 Hz, 1 H) 8.23 (s, 1 H) 8.30 (d, J=7.80 Hz, 1 H) 8.90 (d, J=1.88 Hz, 1 H) 9.12 (dd, J=8.38, 1.38 Hz, 1 H) 9.54 (s, 1 H) 9.87 (s, 1 H).

### 55-9. Preparation of Compound 236, 4-(6-acetyl-2,6-diazaspiro[3.3]heptan-2-yl)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4,5,6,7-tetrahydropyrazolo[1,5-a]pyridine-2-carboxamide

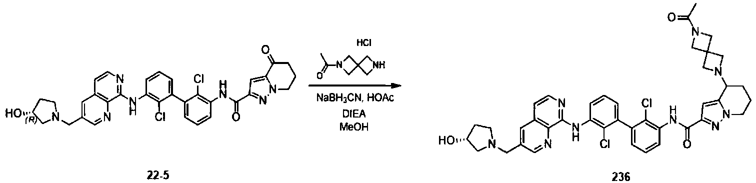

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (70 mg, 108.94 µmol, 1 eq) and 1-(2,6-diazaspiro[3.3]heptan-2-yl)ethanone·HCl (384.88 mg, 2.18 mmol, 20 eq) in MeOH (20 mL), AcOH (10.47 mg, 174.31 µmol, 9.98 µL, 1.60 eq), DIPEA (281.60 mg, 2.18 mmol, 379.52 µL, 20 eq), and NaBH₃CN (34.23 mg, 544.72 µmol, 5 eq) were added. The resulting mixture was stirred under a nitrogen atmosphere at 60 °C for 12 hours. After completion of the reaction, the mixture was filtered, and the filtrate was concentrated under reduced pressure. The residue was purified by reversed-phase HPLC (0.1% NH₃·H₂O; column: Phenomenex Luna C18 150 * 25 mm * 10 µm; mobile phase: [water (NH₄HCO₃)-ACN]; gradient: 46% to 76% B over 10 min) to afford Compound 236 (45 mg, 51.7% yield) as a white solid.

LCMS m/z 766.3 [M+1]⁺.

¹H NMR (400 MHz, DMSO-d6) δ ppm 1.52 - 1.62 (m, 1 H) 1.71 (s, 4 H) 1.80 - 1.89 (m, 1 H) 1.96 - 2.08 (m, 1 H) 2.13 - 2.24 (m, 1 H) 2.38 (dd, J=9.63, 3.63 Hz, 1 H) 2.44 - 2.48 (m, 1 H) 2.65 (q, J=7.71 Hz, 1 H) 2.73 (dd, J=9.63, 6.13 Hz, 1 H) 3.26 (br d, J=7.13 Hz, 2 H) 3.49 (t, J=4.32 Hz, 1 H) 3.76 - 3.88 (m, 4 H) 4.02 - 4.11 (m, 1 H) 4.15 (s, 2 H) 4.17 - 4.24 (m, 2 H) 4.75 (br d, J=3.88 Hz, 1 H) 6.72 (s, 1 H) 7.02 (d, J=7.38 Hz, 1 H) 7.19 (dd, J=7.57, 1.44 Hz, 1 H) 7.33 (d, J=5.88 Hz, 1 H) 7.46 - 7.54 (m, 2 H) 8.18 (d, J=5.75 Hz, 1 H) 8.22 - 8.29 (m, 2 H) 8.90 (s, 1 H) 9.12 (dd, J=8.38, 1.38 Hz, 1 H) 9.54 (s, 1 H) 9.88 (s, 1 H).

### 55-10. Preparation of Compound 237, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(3-(2-hydroxypropan-2-yl)azetidin-1-yl)-4,5,6,7-tetrahydropyrazolo[1,5-a]pyridine-2-carboxamide

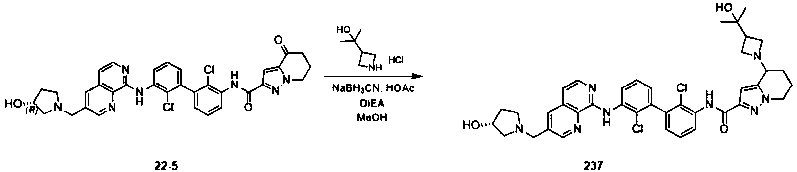

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (50 mg, 77.82 µmol, 1 eq) and 2-(azetidin-3-yl)propan-2-ol·HCl (235.99 mg, 1.56 mmol, 20 eq in MeOH (20 mL), AcOH (7.48 mg, 124.50 µmol, 7.13 µL, 1.60 eq), DIPEA (201.15 mg, 1.56 mmol, 271.09 µL, 20 eq), and NaBH₃CN (24.45 mg, 389.08 µmol, 5 eq) were added. The resulting mixture was stirred under a nitrogen atmosphere at 60 °C for 4 hours. After completion of the reaction, the mixture was filtered, and the filtrate was concentrated under reduced pressure. The residue was purified by reversed-phase HPLC (0.1% NH₃·H₂O; column: Waters XBridge 150 * 25 mm * 5 µm; mobile phase: [water (NH₄HCO₃)-ACN]; gradient: 52% to 82% B over 9 min) to afford Compound 237 (34 mg, 57.7% yield) as a white solid.

LCMS m/z 741.3 [M+1]⁺.

¹H NMR (400 MHz, DMSO-d6) δ ppm 1.02 (d, J=4.38 Hz, 6 H) 1.52 - 1.62 (m, 1 H) 1.69 - 1.77 (m, 2 H) 1.83 (br d, J=5.75 Hz, 1 H) 1.96 - 2.06 (m, 1 H) 2.14 - 2.25 (m, 1 H) 2.30 - 2.40 (m, 2 H) 2.61 - 2.69 (m, 1 H) 2.73 (dd, J=9.57, 6.19 Hz, 1 H) 3.02 (br t, J=7.07 Hz, 1 H) 3.13 (br t, J=7.19 Hz, 1 H) 3.20 - 3.26 (m, 2 H) 3.46 (br t, J=4.13 Hz, 2 H) 3.76 - 3.87 (m, 2 H) 4.02 - 4.13 (m, 1 H) 4.20 (br dd, J=7.69, 4.69 Hz, 2 H) 4.30 (s, 1 H) 4.74 (d, J=4.63 Hz, 1 H) 6.72 (s, 1 H) 7.02 (d, J=7.25 Hz, 1 H) 7.19 (dd, J=7.57, 1.31 Hz, 1 H) 7.34 (d, J=5.88 Hz, 1 H) 7.50 (q, J=7.63 Hz, 2 H) 8.18 (d, J=5.75 Hz, 1 H) 8.22 - 8.31 (m, 2 H) 8.91 (d, J=1.63 Hz, 1 H) 9.13 (dd, J=8.32, 1.31 Hz, 1 H) 9.55 (s, 1 H) 9.88 (s, 1 H).

### 55-11. Preparation of Compound 238, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(6-oxo-2,5-diazaspiro[3.4]octan-2-yl)-4,5,6,7-tetrahydropyrazolo[1,5-a]pyridine-2-carboxamide

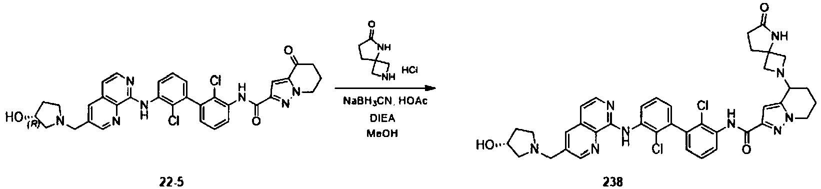

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (70 mg, 108.94 µmol, 1 eq) and 2,5-diazaspiro[3.4]octan-6-one (354.32 mg, 2.18 mmol, 20 eq, HCl) in MeOH (20 mL), AcOH (10.47 mg, 174.31 µmol, 9.98 µL, 1.60 eq), DIPEA (281.60 mg, 2.18 mmol, 379.52 µL, 20 eq), and NaBH₃CN (34.23 mg, 544.72 µmol, 5 eq) were added. The resulting mixture was stirred under a nitrogen atmosphere at 60 °C for 12 hours. After completion of the reaction, the mixture was filtered, and the filtrate was concentrated under reduced pressure. The residue was purified by reversed-phase HPLC (0.1% NH₃·H₂O; column: Phenomenex Luna C18 150 * 25 mm * 10 µm; mobile phase: [water (NH₄HCO₃)-ACN]; gradient: 44% to 74% B over 10 min) to afford Compound 238 (35 mg, 41.8% yield) as a white solid.

LCMS m/z 752.2 [M+1]⁺.

¹H NMR (400 MHz, DMSO-d6) δ ppm 1.49 - 1.64 (m, 1 H) 1.66 - 1.74 (m, 1 H) 1.78 (br dd, J=10.26, 3.50 Hz, 1 H) 1.82 - 1.90 (m, 1 H) 1.98 - 2.07 (m, 2 H) 2.12 - 2.24 (m, 5 H) 2.37 (dd, J=9.57, 3.56 Hz, 1 H) 2.43 - 2.48 (m, 1 H) 2.60 - 2.69 (m, 1 H) 2.73 (dd, J=9.57, 6.19 Hz, 1 H) 3.10 (d, J=6.88 Hz, 1 H) 3.30 (s, 2 H) 3.49 (br t, J=4.44 Hz, 1 H) 3.74 - 3.86 (m, 2 H) 4.02 - 4.15 (m, 1 H) 4.17 - 4.25 (m, 2 H) 4.69 - 4.82 (m, 1 H) 6.71 (s, 1 H) 7.01 (d, J=6.88 Hz, 1 H) 7.19 (dd, J=7.57, 1.44 Hz, 1 H) 7.32 (d, J=5.88 Hz, 1 H) 7.50 (td, J=7.94, 5.88 Hz, 2 H) 8.12 (s, 1 H) 8.17 (d, J=5.88 Hz, 1 H) 8.21 - 8.28 (m, 2 H) 8.89 (d, J=1.75 Hz, 1 H) 9.12 (dd, J=8.38, 1.38 Hz, 1 H) 9.54 (s, 1 H) 9.87 (s, 1 H).

### 55-12. Preparation of Compound 239, 4-((2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)butanoic acid

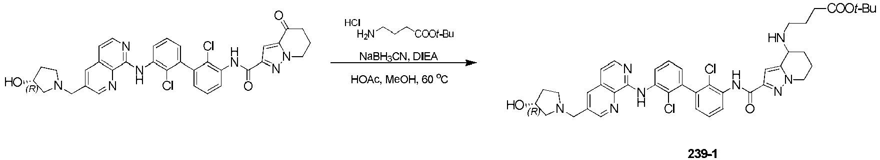

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (100 mg, 155.63 µmol, 1 eq) and tert-butyl 4-aminobutanoate·HCl (609.11 mg, 3.11 mmol, 20 eq) in MeOH (20 mL), AcOH (18.69 mg, 311.27 µmol, 17.82 µL, 2 eq), DIPEA (402.28 mg, 3.11 mmol, 542.16 µL, 20 eq), and NaBH₃CN (48.90 mg, 778.17 µmol, 5 eq) were added. The resulting mixture was stirred at 60 °C for 12 hours. After completion of the reaction, the mixture was concentrated under reduced pressure, and the residue was purified by reversed-phase HPLC (0.1% NH₃·H₂O; column: Waters XBridge 150 * 25 mm * 5 µm; mobile phase: [water (NH₄HCO₃)-ACN]; gradient: 70% to 100% B over 9 min) to afford Compound 239-1 (73 mg, 59.1% yield) as a white solid.

LCMS m/z 785.3 [M+1]⁺.

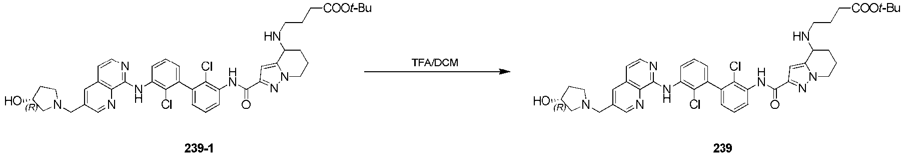

To a solution of tert-Butyl 4-[[2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-4,5,6,7-tetrahydropyrazolo[1,5-a]pyridin-4-yl]amino]butanoate (239-1, 63 mg, 80.18 µmol, 1 eq) in DCM (15 mL), TFA (27.43 mg, 240.53 µmol, 17.87 µL, 3 eq) was added. The resulting mixture was stirred at room temperature for 12 hours. After completion of the reaction, the mixture was concentrated under reduced pressure, and the residue was purified by reversed-phase HPLC (0.1% HCl; column: Waters XBridge 150 * 25 mm * 5 µm; mobile phase: [water (NH₄HCO₃)-ACN]; gradient: 25% to 55% B over 9 min) to afford Compound 239 (14 mg, 23.2% yield) as a white solid.

LCMS m/z 729.2 [M+1]⁺

¹H NMR (400 MHz, DMSO-d6) δ ppm 1.24 (br s, 1 H) 1.60 (br dd, J=8.13, 4.50 Hz, 1 H) 1.67 - 1.80 (m, 3 H) 1.95 (br s, 1 H) 2.00 - 2.13 (m, 2 H) 2.15 - 2.24 (m, 1 H) 2.33 (t, *J*=7.19 Hz, 2 H) 2.40 - 2.45 (m, 1 H) 2.66 - 2.81 (m, 4 H) 3.81 - 3.92 (m, 2 H) 4.07 (br s, 1 H) 4.14 - 4.20 (m, 2 H) 4.24 (br s, 1 H) 4.76 (br s, 1 H) 6.88 (s, 1 H) 7.04 (dd, *J*=7.63, 1.50 Hz, 1 H) 7.21 (dd, *J*=7.63, 1.38 Hz, 1 H) 7.36 (d, *J*=5.88 Hz, 1 H) 7.52 (q, *J*=7.63 Hz, 2 H) 8.20 (d, *J*=5.88 Hz, 1 H) 8.26 - 8.31 (m, 2 H) 8.93 (d, *J*=1.88 Hz, 1 H) 9.14 (dd, *J*=8.38, 1.50 Hz, 1 H) 9.57 (s, 1 H) 9.90 (s, 1 H)

### 55-13. Preparation of Compound 240, N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(2-oxopyrrolidin-1-yl)-4,5,6,7-tetrahydropyrazolo[1,5-a]pyridine-2-carboxamide

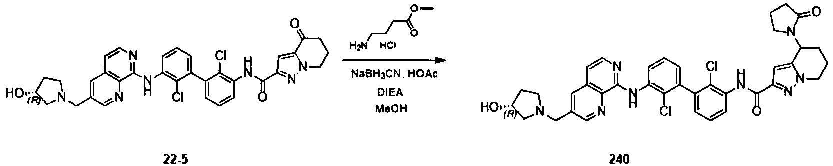

To a solution of N-[2-chloro-3-[2-chloro-3-[[3-[[(3R)-3-hydroxypyrrolidin-1-yl]methyl]-1,7-naphthyridin-8-yl]amino]phenyl]phenyl]-4-oxo-6,7-dihydro-5H-pyrazolo[1,5-a]pyridine-2-carboxamide (60.00 mg, 93.38 µmol, 1 eq) and methyl 4-aminobutanoate·HCl (286.88 mg, 1.87 mmol, 20 eq) in MeOH (20 mL), AcOH (8.97 mg, 149.41 µmol, 8.55 µL, 1.60 eq), DIPEA (241.38 mg, 1.87 mmol, 325.30 µL, 20 eq), and NaBH₃CN (29.34 mg, 466.90 µmol, 5 eq) were added. The resulting mixture was stirred under a nitrogen atmosphere at 60 °C for 2 hours. After completion of the reaction, the mixture was filtered, and the filtrate was concentrated under reduced pressure. The residue was purified by reversed-phase HPLC (0.1% NH₃·H₂O; column: Waters XBridge 150 * 25 mm * 5 µm; mobile phase: [water (NH₄HCO₃)-ACN]; gradient: 50% to 80% B over 9 min) to afford Compound 240 (34 mg, 50.1% yield) as a white solid.

LCMS m/z 711.2 [M+1]⁺.

¹H NMR (400 MHz, DMSO-d6) δ ppm 1.53 - 1.62 (m, 1 H) 1.88 - 2.01 (m, 4 H) 2.07 (s, 2 H) 2.14 (br s, 1 H) 2.26 - 2.42 (m, 3 H) 2.65 (q, J=7.75 Hz, 1 H) 2.74 (dd, J=9.63, 6.13 Hz, 1 H) 3.09 - 3.16 (m, 1 H) 3.25 - 3.33 (m, 2 H) 3.76 - 3.88 (m, 2 H) 4.07 - 4.16 (m, 1 H) 4.18 - 4.30 (m, 2 H) 4.74 (d, J=4.63 Hz, 1 H) 5.28 (dd, J=10.13, 6.38 Hz, 1 H) 6.57 (s, 1 H) 7.02 (dd, J=7.63, 1.50 Hz, 1 H) 7.20 (dd, J=7.57, 1.19 Hz, 1 H) 7.34 (d, J=5.88 Hz, 1 H) 7.50 (q, J=7.75 Hz, 2 H) 8.19 (d, J=5.75 Hz, 1 H) 8.21 - 8.26 (m, 2 H) 8.91 (d, J=1.88 Hz, 1 H) 9.13 (d, J=8.52 Hz, 1 H) 9.57 (d, J=1.88 Hz, 1 H) 9.88 (s, 1 H)

### [Preparation Example 56]

### Preparation of Compounds 241 to 244

### 56-1. Preparation of Compound 241, (S)-4-((2-acetamidoethyl)amino)-N-(2,2'-dichloro-3'-(5-(((2-hydroxyethyl)amino)methyl)picolinamido)-[1,1'-biphenyl]-3-yl)-4,5,6,7-tetrahydropyrazolo[1,5-a]pyridine-2-carboxamide, and Compound 224, (R)-4-((2-acetamidoethyl)amino)-N-(2,2'-dichloro-3'-(5-(((2-hydroxyethyl)amino)methyl)picolinamido)-[1,1'-biphenyl]-3-yl)-4,5,6,7-tetrahydropyrazolo[1,5-a]pyridine-2-carboxamide

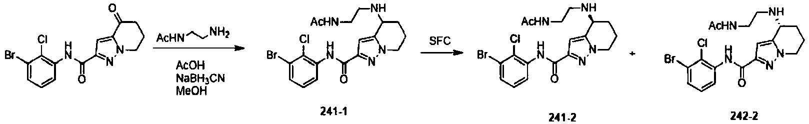

A solution of N-(3-bromo-2-chloro-phenyl)-4-oxo-6,7-dihydro-5H-pyrazolo[1,5-a]pyridine-2-carboxamide (500 mg, 1.36 mmol, 1 eq), N-(2-aminoethyl)acetamide (692.70 mg, 6.78 mmol, 649.81 µL, 5 eq), and HOAc (814.54 mg, 13.56 mmol, 776.49 µL, 10 eq) in MeOH (20 mL) was stirred at 60 °C for 2 hours. NaBH₃CN (255.72 mg, 4.07 mmol, 3 eq) was then added, and the mixture was stirred at 60 °C for an additional 2 hours. The reaction mixture was cooled to room temperature, concentrated under reduced pressure, and the residue was purified by prep-HPLC (column: Waters XBridge Prep OBD C18 150 * 40 mm * 10 µm; mobile phase: [water (NH₄HCO₃)-ACN]; gradient: 25% to 55% B over 15 min) to afford Compound 241-1 (608 mg, 95% yield) as a white solid. Further separation by SFC (column: Phenomenex Cellulose-2, 250 * 30 mm * 10 µm; mobile phase: CO₂/ACN-iPrOH (0.1% NH₃·H₂O); B%: 40%, isocratic elution) provided compounds 241-2 (302 mg, 46.5% yield) and 241-2 (306 mg, 47.1% yield) as white solids.

LCMS m/z 456.2 [M+3]⁺.

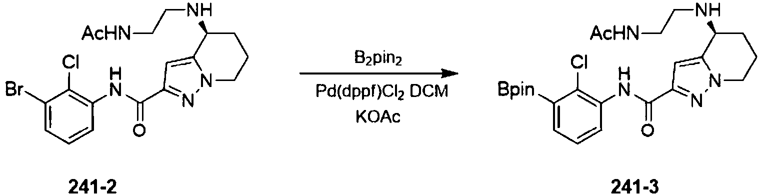

A mixture of (4S)-4-(2-acetamidoethylamino)-N-(3-bromo-2-chloro-phenyl)-4,5,6,7-tetrahydropyrazolo[1,5-a]pyridine-2-carboxamide (3, 200 mg, 439.80 µmol, 1 eq), 4,4,5,5-tetramethyl-2-(4,4,5,5-tetramethyl-1,3,2-dioxaborolan-2-yl)-1,3,2-dioxaborolane (335.05 mg, 1.32 mmol, 3 eq), KOAc (129.49 mg, 1.32 mmol, 3 eq), and Pd(dppf)Cl₂·CH₂Cl₂ (35.92 mg, 43.98 µmol, 0.1 eq) in dioxane (6 mL) was stirred under a nitrogen atmosphere at 90 °C for 12 hours. The reaction mixture was cooled to room temperature, concentrated under reduced pressure, and the residue was purified by prep-TLC (SiO₂, ethyl acetate/methanol = 3:1) to afford Compound 241-3 (187 mg, 68.6% yield) as a white solid.

LCMS m/z 502.2 [M+1]⁺.

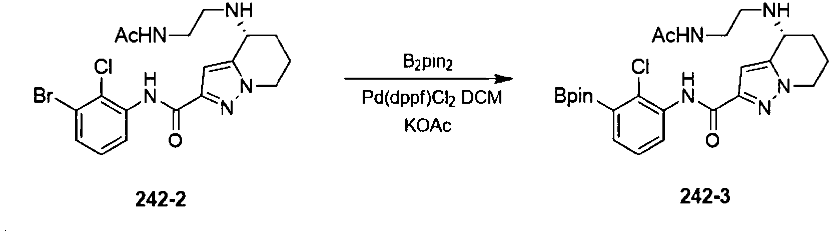

A mixture of (4R)-4-(2-acetamidoethylamino)-N-(3-bromo-2-chloro-phenyl)-4,5,6,7-tetrahydropyrazolo[1,5-a]pyridine-2-carboxamide (200 mg, 439.80 µmol, 1 eq), 4,4,5,5-tetramethyl-2-(4,4,5,5-tetramethyl-1,3,2-dioxaborolan-2-yl)-1,3,2-dioxaborolane (335.05 mg, 1.32 mmol, 3 eq), KOAc (129.49 mg, 1.32 mmol, 3 eq), and Pd(dppf)Cl₂·CH₂Cl₂ (35.92 mg, 43.98 µmol, 0.1 eq) in dioxane (6 mL) was stirred under a nitrogen atmosphere at 90 °C for 12 hours. The reaction mixture was cooled to room temperature, concentrated under reduced pressure, and the residue was purified by prep-TLC (SiO₂, ethyl acetate/methanol = 3:1) to afford Compound 242-3 (200 mg, 73.4% yield) as a white solid.

LCMS m/z 502.4 [M+1]⁺.

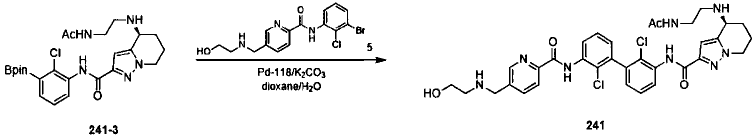

A mixture of (4S)-4-(2-acetamidoethylamino)-N-[2-chloro-3-(4,4,5,5-tetramethyl-1,3,2-dioxaborolan-2-yl)phenyl]-4,5,6,7-tetrahydropyrazolo[1,5-a]pyridine-2-carboxamide (180 mg, 358.70 µmol, 1.2 eq), N-(3-bromo-2-chloro-phenyl)-5-[(2-hydroxyethylamino)methyl]pyridine-2-carboxamide (114.98 mg, 298.92 µmol, 1 eq), ditert-butyl(cyclopentyl)phosphane;dichloropalladium iron (19.48 mg, 29.89 µmol, 0.1 eq), and K₂CO₃ (123.94 mg, 896.75 µmol, 3 eq) in dioxane (5 mL) and H₂O (1 mL) was stirred under a nitrogen atmosphere at 90 °C for 12 hours. The reaction mixture was cooled to room temperature, concentrated under reduced pressure, and the residue was purified by prep-TLC (SiO₂, ethyl acetate/methanol = 0:1) and further purified by reversed-phase HPLC (column: Waters XBridge 150 * 25 mm * 5 µm; mobile phase: [water (NH₄HCO₃)-ACN]; gradient: 24% to 54% B over 9 min) to afford Compound 241 (31 mg, 14.8% yield) as a white solid.

LCMS m/z 681.2 [M+3]⁺.

¹H NMR (400 MHz, DMSO-d6) δ ppm 1.61 - 1.72 (m, 1 H) 1.81 (s, 3 H) 1.85 - 1.95 (m, 1 H) 1.96 - 2.05 (m, 1 H) 2.18 (br dd, *J*=13.45, 5.69 Hz, 2 H) 2.58 (t, *J*=5.75 Hz, 2 H) 2.63 (br t, *J*=6.38 Hz, 2 H) 3.05 - 3.21 (m, 3 H) 3.44 - 3.51 (m, 2 H) 3.83 - 3.91 (m, 1 H) 3.86 (s, 2 H) 4.08 - 4.20 (m, 2 H) 4.49 (t, *J*=5.25 Hz, 1 H) 6.79 (s, 1 H) 7.18 (br d, *J*=7.50 Hz, 2 H) 7.42 - 7.61 (m, 2 H) 7.82 (br t, *J*=5.32 Hz, 1 H) 8.05 (dd, *J*=8.07, 1.69 Hz, 1 H) 8.18 (d, *J*=8.00 Hz, 1 H) 8.30 (d, *J*=7.38 Hz, 1 H) 8.53 (dd, *J*=8.25, 1.13 Hz, 1 H) 8.69 (s, 1 H) 9.54 (s, 1 H) 10.72 (s, 1 H).

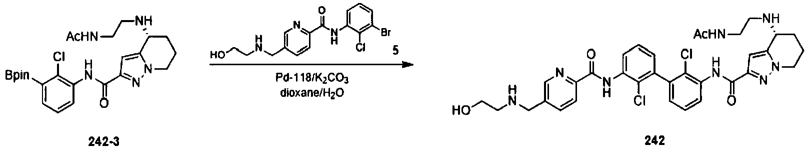

A mixture of (4R)-4-(2-acetamidoethylamino)-N-[2-chloro-3-(4,4,5,5-tetramethyl-1,3,2-dioxaborolan-2-yl)phenyl]-4,5,6,7-tetrahydropyrazolo[1,5-a]pyridine-2-carboxamide (200 mg, 398.56 µmol, 1.2 eq), N-(3-bromo-2-chloro-phenyl)-5-[(2-hydroxyethylamino)methyl]pyridine-2-carboxamide (127.76 mg, 332.13 µmol, 1 eq), ditert-butyl(cyclopentyl)phosphane;dichloropalladium iron (21.65 mg, 33.21 µmol, 0.1 eq), and K₂CO₃ (137.71 mg, 996.39 µmol, 3 eq) in dioxane (5 mL) and H₂O (1 mL) was stirred under a nitrogen atmosphere at 90 °C for 12 hours. The reaction mixture was cooled to room temperature, concentrated under reduced pressure, and the residue was purified by prep-TLC (SiO₂, ethyl acetate/methanol = 0:1) and further purified by reversed-phase HPLC (column: Waters XBridge 150 * 25 mm * 5 µm; mobile phase: [water (NH₄HCO₃)-ACN]; gradient: 24% to 54% B over 9 min) to afford Compound 242 (16 mg, 6.81% yield) as a white solid.

LCMS m/z 681.3 [M+3]⁺.

¹H NMR (400 MHz, DMSO-d6) δ ppm 1.66 (br d, *J*=9.76 Hz, 1 H) 1.81 (s, 3 H) 1.91 (br s, 1 H) 2.00 (br s, 1 H) 2.17 (br s, 1 H) 2.54 - 2.67 (m, 4 H) 3.02 - 3.24 (m, 3 H) 3.41 - 3.63 (m, 3 H) 3.87 (br s, 3 H) 4.14 (br s, 2 H) 4.52 (br s, 1 H) 6.79 (s, 1 H) 7.18 (br d, *J*=7.25 Hz, 2 H) 7.41 - 7.62 (m, 2 H) 7.83 (br s, 1 H) 8.06 (br d, *J*=7.88 Hz, 1 H) 8.19 (br d, *J*=7.88 Hz, 1 H) 8.30 (br d, *J*=8.13 Hz, 1 H) 8.52 (br d, *J*=7.88 Hz, 1 H) 8.70 (br s, 1 H) 9.54 (s, 1 H) 10.72 (s, 1 H).

### 56-2. Preparation of Compound 243, (S)-N-(2,2'-dichloro-3'-(5-(((2-hydroxyethyl)amino)methyl)picolinamido)-[1,1'-biphenyl]-3-yl)-4-((R)-3-hydroxypyrrolidin-1-yl)-4,5,6,7-tetrahydropyrazolo[1,5-a]pyridine-2-carboxamide, and Compound 244, (R)-N-(2,2'-dichloro-3'-(5-(((2-hydroxyethyl)amino)methyl)picolinamido)-[1,1'-biphenyl]-3-yl)-4-((R)-3-hydroxypyrrolidin-1-yl)-4,5,6,7-tetrahydropyrazolo[1,5-a]pyridine-2-carboxamide

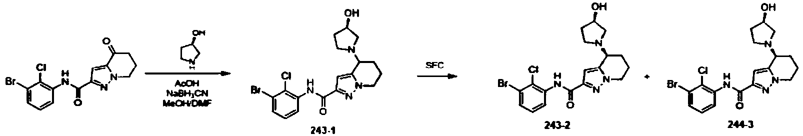

To a solution of N-(3-bromo-2-chloro-phenyl)-4-oxo-6,7-dihydro-5H-pyrazolo[1,5-a]pyridine-2-carboxamide (500 mg, 1.36 mmol, 1 eq) and (3R)-pyrrolidin-3-ol (1.18 g, 13.56 mmol, 1.13 mL, 10 eq) in DMF (5 mL) / MeOH (15 mL), NaBH₃CN (255.72 mg, 4.07 mmol, 3 eq) and AcOH (407.28 mg, 6.78 mmol, 388.26 µL, 5 eq) were added. The resulting mixture was stirred at 60 °C for 2 hours. The reaction mixture was cooled to room temperature, concentrated under reduced pressure, and the residue was purified by reversed-phase HPLC (column: Waters XBridge Prep OBD C18 150 * 40 mm * 10 µm; mobile phase: [water (NH₄HCO₃)-ACN]; gradient: 35% to 65% B over 15 min). Further separation by SFC (column: DAICEL CHIRALPAK AD 250 * 30 mm, 10 µm; mobile phase: CO₂/EtOH (0.1% NH₃·H₂O); B: 60%, isocratic) provided compounds 243-2 (185 mg, 63.0% yield) and 244-2 (180 mg, 61.3% yield) as white solids.

LCMS m/z 441.0 [M+3]⁺.

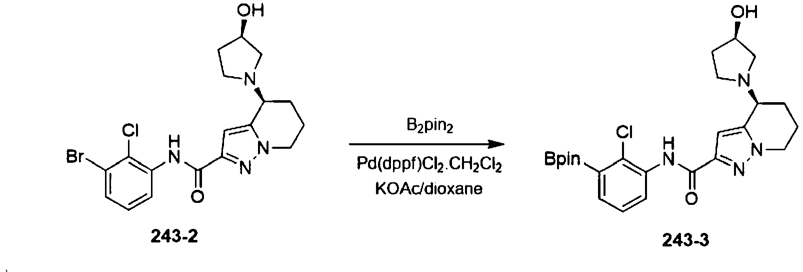

A mixture of (4S)-N-(3-bromo-2-chloro-phenyl)-4-[(3R)-3-hydroxypyrrolidin-1-yl]-4,5,6,7-tetrahydropyrazolo[1,5-a]pyridine-2-carboxamide (150.00 mg, 341.12 µmol, 1 eq), 4,4,5,5-tetramethyl-2-(4,4,5,5-tetramethyl-1,3,2-dioxaborolan-2-yl)-1,3,2-dioxaborolane (259.87 mg, 1.02 mmol, 3 eq), KOAc (100.43 mg, 1.02 mmol, 3 eq), and Pd(dppf)Cl₂·CH₂Cl₂ (27.86 mg, 34.11 µmol, 0.1 eq) in dioxane (10 mL) was stirred under a nitrogen atmosphere at 90 °C for 12 hours. The reaction mixture was cooled to room temperature, concentrated under reduced pressure, and the residue was purified by column chromatography (SiO₂, ethyl acetate/methanol = 10:1 to 5:1) to afford Compound 243-3 (200 mg, 66.2% yield) as a white solid.

LCMS m/z 487.3 [M+1]⁺.

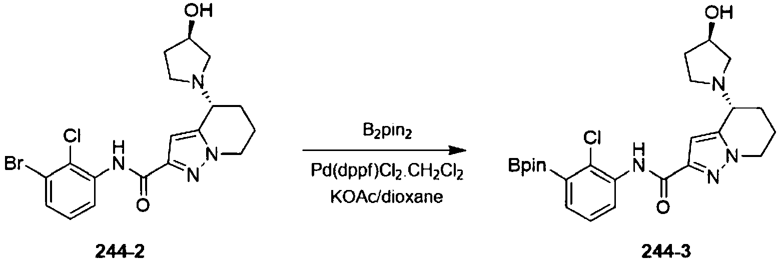

To a solution of (4R)-N-(3-bromo-2-chloro-phenyl)-4-[(3R)-3-hydroxypyrrolidin-1-yl]-4,5,6,7-tetrahydropyrazolo[1,5-a]pyridine-2-carboxamide (180 mg, 409.34 µmol, 1 eq) and 4,4,5,5-tetramethyl-2-(4,4,5,5-tetramethyl-1,3,2-dioxaborolan-2-yl)-1,3,2-dioxaborolane (311.84 mg, 1.23 mmol, 3 eq) in dioxane (10 mL), KOAc (120.52 mg, 1.23 mmol, 3 eq) and Pd(dppf)Cl₂·CH₂Cl₂ (33.43 mg, 40.93 µmol, 0.1 eq) were added. The resulting mixture was stirred under a nitrogen atmosphere at 100 °C for 12 hours. The reaction mixture was cooled to room temperature, concentrated under reduced pressure, and the residue was purified by column chromatography (SiO₂, ethyl acetate/methanol = 10/1 to 5/1) to afford Compound 244-3 (180 mg, 54.2% yield) as a white solid.

LCMS m/z 487.3 [M+1]⁺.

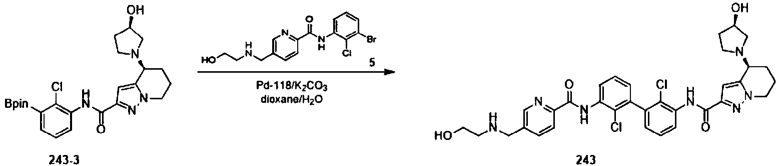

A mixture of (4S)-N-[2-chloro-3-(4,4,5,5-tetramethyl-1,3,2-dioxaborolan-2-yl)phenyl]-4-[(3S)-3-hydroxypyrrolidin-1-yl]-4,5,6,7-tetrahydropyrazolo[1,5-a]pyridine-2-carboxamide (151.87 mg, 311.97 µmol, 1.2 eq), N-(3-bromo-2-chloro-phenyl)-5-[(2-hydroxyethylamino)methyl]pyridine-2-carboxamide (100 mg, 259.97 µmol, 1 eq), ditert-butyl(cyclopentyl)phosphane;dichloropalladium iron (16.94 mg, 26.00 µmol, 0.1 eq), and K₂CO₃ (107.79 mg, 779.92 µmol, 3 eq) in dioxane (5 mL) and H₂O (1 mL) was stirred under a nitrogen atmosphere at 90 °C for 12 hours. The reaction mixture was cooled to room temperature, concentrated under reduced pressure, and the residue was purified by reversed-phase HPLC (column: Waters XBridge 150 * 25 mm * 5 µm; mobile phase: [water (NH₄HCO₃)-ACN]; gradient: 30% to 60% B over 9 min) to afford Compound 243 (39 mg, 22.1% yield) as a white solid.

LCMS m/z 664.5 [M+1]⁺.

¹H NMR (400 MHz, DMSO-d6) δ ppm 1.47 - 1.64 (m, 1 H) 1.82 - 2.03 (m, 4 H) 2.23 (br s, 1 H) 2.54 - 2.64 (m, 3 H) 2.65 - 2.72 (m, 1 H) 2.76 (dd, *J*=9.32, 6.19 Hz, 1 H) 3.41 - 3.60 (m, 3 H) 3.86 (s, 3 H) 3.81 (br s, 1 H) 4.16 (br s, 3 H) 4.51 (br s, 1 H) 4.65 - 4.76 (m, 1 H) 6.72 (s, 1 H) 7.17 (br d, *J*=7.63 Hz, 2 H) 7.43 - 7.59 (m, 2 H) 7.98 - 8.11 (m, 1 H) 8.18 (d, *J*=8.00 Hz, 1 H) 8.28 (dt, *J*=6.69, 1.59 Hz, 1 H) 8.52 (d, *J*=8.51 Hz, 1 H) 8.69 (s, 1 H) 9.54 (s, 1 H) 10.72 (s, 1 H).

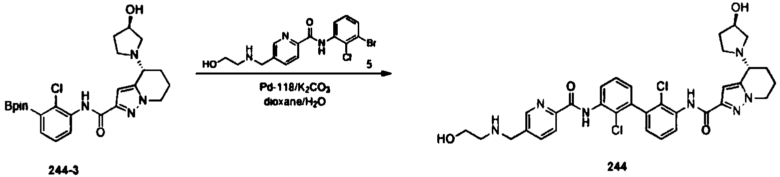

A mixture of (4R)-N-[2-chloro-3-(4,4,5,5-tetramethyl-1,3,2-dioxaborolan-2-yl)phenyl]-4-[(3S)-3-hydroxypyrrolidin-1-yl]-4,5,6,7-tetrahydropyrazolo[1,5-a]pyridine-2-carboxamide (151.87 mg, 311.97 µmol, 1.2 eq), N-(3-bromo-2-chloro-phenyl)-5-[(2-hydroxyethylamino)methyl]pyridine-2-carboxamide (100 mg, 259.97 µmol, 1 eq), ditert-butyl(cyclopentyl)phosphane;dichloropalladium;iron (16.94 mg, 26.00 µmol, 0.1 eq), and K₂CO₃ (107.79 mg, 779.92 µmol, 3 eq) in dioxane (5 mL) and H₂O (1 mL) was stirred under a nitrogen atmosphere at 90 °C for 12 hours. The reaction mixture was cooled to room temperature, concentrated under reduced pressure, and the residue was purified by reversed-phase HPLC (column: Waters XBridge 150 * 25 mm * 5 µm; mobile phase: [water (NH₄HCO₃)-ACN]; gradient: 30% to 60% B over 9 min) to afford Compound 244 (40 mg, 22.6% yield) as a white solid.

LCMS m/z 664.5 [M+1]⁺.

¹H NMR (400 MHz, DMSO-d6) δ ppm 1.51 - 1.64 (m, 1 H) 1.83 - 2.00 (m, 4 H) 2.13 - 2.30 (m, 1 H) 2.32 - 2.47 (m, 2 H) 2.52 - 2.61 (m, 3 H) 2.75 - 2.94 (m, 2 H) 3.41 - 3.54 (m, 2 H) 3.80 (br s, 1 H) 3.86 (s, 2 H) 4.09 - 4.23 (m, 3 H) 4.50 (br s, 1 H) 4.70 (br d, *J*=4.00 Hz, 1 H) 6.74 (s, 1 H) 7.18 (d, *J*=7.63 Hz, 2 H) 7.45 - 7.58 (m, 2 H) 8.01 - 8.08 (m, 1 H) 8.18 (d, *J*=8.00 Hz, 1 H) 8.28 (ddd, *J*=8.19, 3.38, 1.31 Hz, 1 H) 8.53 (dd, *J*=8.19, 1.19 Hz, 1 H) 8.69 (s, 1 H) 9.55 (s, 1 H) 10.72 (s, 1 H).

### [Preparation Example 57]

### Preparation of Compounds 245 to 250

### 57-1. Preparation of Compound 245, (S)-2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetic acid, and Compound 246, (R)-2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetic acid

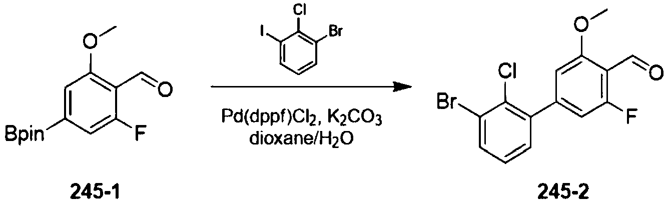

A mixture of 2-fluoro-6-methoxy-4-(4,4,5,5-tetramethyl-1,3,2-dioxaborolan-2-yl)benzaldehyde (1.4 g, 5.00 mmol, 1 eq), 1-bromo-2-chloro-3-iodo-benzene (1.59 g, 5.00 mmol, 1 eq), Pd(dppf)Cl₂ (365.73 mg, 499.82 µmol, 0.1 eq), and K₂CO₃ (2.07 g, 14.99 mmol, 3 eq) in dioxane (25 mL) and H₂O (5 mL) was stirred under a nitrogen atmosphere at 60 °C for 12 hours. The reaction mixture was cooled to room temperature, concentrated under reduced pressure, and the residue was purified by column chromatography (SiO₂, Petroleum ether/Ethyl acetate = 0:1 to 10:1) to afford Compound 245-2 (1.6 g, 80.1% yield) as a yellow solid.

LCMS m/z 343.0 [M+3]⁺.

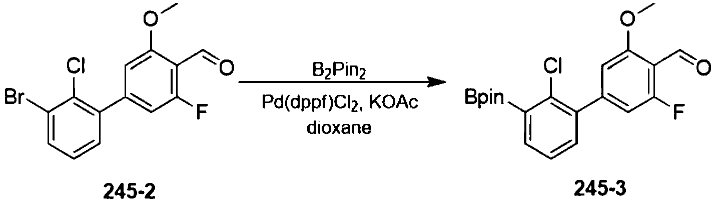

A mixture of 4-(3-bromo-2-chloro-phenyl)-2-fluoro-6-methoxy-benzaldehyde (1.5 g, 4.37 mmol, 1 eq), 4,4,5,5-tetramethyl-2-(4,4,5,5-tetramethyl-1,3,2-dioxaborolan-2-yl)-1,3,2-dioxaborolane (3.33 g, 13.10 mmol, 3 eq), Pd(dppf)Cl₂ (319.45 mg, 436.59 µmol, 0.1 eq), and KOAc (1.29 g, 13.10 mmol, 3 eq) in dioxane (25 mL) was stirred under a nitrogen atmosphere at 100 °C for 12 hours. The reaction mixture was cooled to room temperature, concentrated under reduced pressure, and the residue was purified by column chromatography (SiO₂, Petroleum ether/Ethyl acetate = 0:1 to 5:1) to afford Compound 245-3 (1.58 g, 74.1% yield) as a yellow solid.

LCMS m/z 391.1 [M+1]⁺.

¹H NMR (400 MHz, DMSO-d6) δ ppm 1.17 (s, 12 H) 3.95 (s, 3 H) 6.97 (d, *J*=11.26 Hz, 1 H) 7.07 (s, 1 H) 7.43 - 7.49 (m, 1 H) 7.53 - 7.57 (m, 1 H) 7.68 (dd, *J*=7.32, 1.69 Hz, 1 H) 10.34 (s, 1 H).

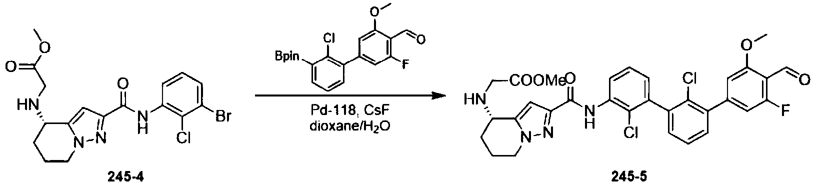

A mixture of 4-[2-chloro-3-(4,4,5,5-tetramethyl-1,3,2-dioxaborolan-2-yl)phenyl]-2-fluoro-6-methoxy-benzaldehyde (424.51 mg, 1.09 mmol, 1 eq) and methyl 2-[[(4S)-2-[(3-bromo-2-chlorophenyl)carbamoyl]-4,5,6,7-tetrahydropyrazolo[1,5-a]pyridin-4-yl]amino]acetate (480 mg, 1.09 mmol, 1 eq), Ditert-butyl(cyclopentyl)phosphane;dichloropalladium iron (70.82 mg, 108.67 µmol, 0.1 eq) and CsF (330.14 mg, 2.17 mmol, 80.23 µL, 2 eq) in dioxane (10 mL) / H₂O (2 mL) was stirred under a nitrogen atmosphere at 70 °C for 12 hours. The reaction mixture was cooled to room temperature, concentrated under reduced pressure, and the residue was purified by column chromatography (SiO₂, Petroleum ether/Ethyl acetate = 1:0 to 0:1) to afford Compound 245-5 (380 mg, 41.3% yield) as a yellow solid.

LCMS m/z 625.2 [M+1]⁺.

¹H NMR (400 MHz, DMSO-d6) δ ppm 1.61 - 1.78 (m, 2 H) 2.20 (br s, 2 H) 3.33 (s, 6 H) 3.65 (s, 4 H) 4.15 (br t, *J*=5.50 Hz, 2 H) 6.79 (s, 1 H) 7.05 (d, *J*=11.01 Hz, 1 H) 7.13 - 7.22 (m, 2 H) 7.47 - 7.49 (m, 1 H) 7.57 - 7.61 (m, 2 H) 8.28 (d, *J*=8.13 Hz, 1 H) 9.54 - 9.58 (m, 1 H) 10.35 (s, 1 H).

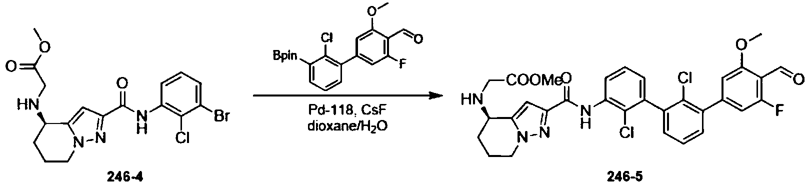

A mixture of methyl 2-[[(4R)-2-[(3-bromo-2-chloro-phenyl)carbamoyl]-4,5,6,7-tetrahydropyrazolo[1,5-a]pyridin-4-yl]amino]acetate (480 mg, 1.09 mmol, 1 eq) and 4-[2-chloro-3-(4,4,5,5-tetramethyl-1,3,2-dioxaborolan-2-yl)phenyl]-2-fluoro-6-methoxy-benzaldehyde (424.51 mg, 1.09 mmol, 1 eq), Ditert-butyl(cyclopentyl)phosphane;dichloropalladium iron (70.82 mg, 108.67 µmol, 0.1 eq) and CsF (330.14 mg, 2.17 mmol, 80.23 µL, 2 eq) in dioxane (10 mL) / H₂O (2 mL) was stirred under a nitrogen atmosphere at 70 °C for 12 hours. The reaction mixture was cooled to room temperature, concentrated under reduced pressure, and the residue was purified by column chromatography (SiO₂, Petroleum ether/Ethyl acetate = 1:0 to 0:1) to afford Compound 246-5 (250 mg, 32.7% yield) as a yellow solid.

LCMS m/z 625.2 [M+1]⁺.

¹H NMR (400 MHz, DMSO-d6) δ ppm 1.59 - 1.79 (m, 2 H) 2.11 - 2.30 (m, 2 H) 3.33 (s, 6 H) 3.66 (s, 4 H) 4.15 (br t, *J*=6.38 Hz, 2 H) 6.79 (s, 1 H) 7.05 (d, *J*=11.51 Hz, 1 H) 7.15 (s, 1 H) 7.21 (dd, *J*=7.63, 1.38 Hz, 1 H) 7.47 - 7.49 (m, 1 H) 7.58 - 7.61 (m, 2 H) 8.28 (d, *J*=8.00 Hz, 1 H) 9.57 (s, 1 H) 10.35 (s, 1 H).

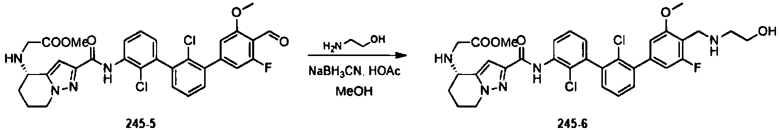

A mixture of methyl 2-[[(4S)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (100 mg, 159.88 µmol, 1 eq), 2-aminoethanol (48.83 mg, 799.39 µmol, 48.25 µL, 5 eq), NaBH₃CN (50.24 mg, 799.39 µmol, 5 eq), and AcOH (96.01 mg, 1.60 mmol, 91.53 µL, 10 eq) in MeOH (4 mL) was stirred at 60 °C for 2 hours. The reaction mixture was cooled to room temperature, concentrated under reduced pressure, and the residue was purified by reversed-phase HPLC (column: Phenomenex Luna C18 150 * 25 mm * 10 µm; mobile phase: [water (HCl)-ACN]; gradient: 8% to 38% B over 10 min) to afford Compound 245-6 (40 mg, 33.5% yield) as a white solid.

LCMS /z 670.3 [M+1]⁺.

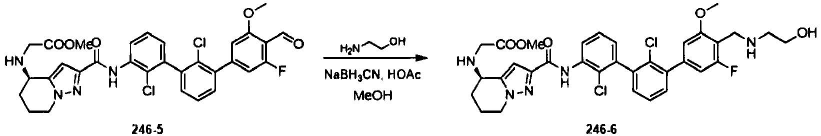

A mixture of methyl 2-[[(4R)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (80.00 mg, 127.90 µmol, 1 eq), 2-aminoethanol (78.13 mg, 1.28 mmol, 77.20 µL, 10 eq), NaBH₃CN (40.19 mg, 639.52 µmol, 5 eq) and AcOH (76.81 mg, 1.28 mmol, 73.22 µL, 10 eq) in MeOH (5 mL) was stirred at 60 °C for 2 hours. The reaction mixture was cooled to room temperature, concentrated under reduced pressure, and the residue was purified by prep-TLC (SiO₂, DCM/MeOH = 10:1) to afford Compound 246-6 (81 mg, 87.8% yield) as a white solid.

LCMS m/z 670.3 [M+1]⁺.

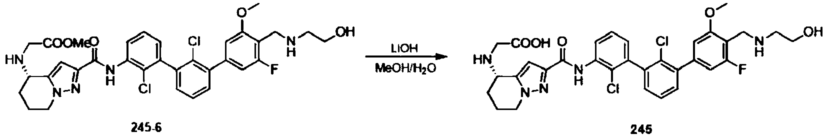

A solution of methyl 2-[[(4S)-2-[[2-chloro-3-[2-chloro-3-[3-fluoro-4-[(2-hydroxyethylamino)methyl]-5-methoxy-phenyl]phenyl]phenyl]carbamoyl]-4,5,6,7-tetrahydropyrazolo[1,5-a]pyridin-4-yl]amino]acetate (40.00 mg, 59.65 µmol, 1 eq) in MeOH (4 mL) and H₂O (2 mL) was treated with LiOH (7.51 mg, 178.96 µmol, 3 eq) and stirred at room temperature for 12 hours. The reaction mixture was concentrated under reduced pressure, and the residue was purified by reversed-phase HPLC (column: Waters XBridge 150 * 25 mm * 5 µm; mobile phase: [water (NH₄HCO₃)-ACN]; gradient: 18% to 48% B over 9 min) to afford Compound 245 (26 mg, 66.3% yield) as a white solid.

LCMS m/z 656.3 [M+1]⁺.

¹H NMR (400 MHz, DMSO-d6) δ ppm 1.69 - 1.79 (m, 1 H) 1.86 - 1.96 (m, 1 H) 1.96 - 2.06 (m, 1 H) 2.21 (br dd, *J*=13.45, 5.57 Hz, 1 H) 2.57 - 2.63 (m, 2 H) 3.30 (s, 2 H) 3.78 (s, 3 H) 3.88 (s, 3 H) 4.04 (br t, *J*=5.88 Hz, 1 H) 4.15 (br t, *J*=6.07 Hz, 2 H) 6.82 (s, 1 H) 6.89 - 6.96 (m, 2 H) 7.20 (dd, *J*=7.63, 1.25 Hz, 1 H) 7.41 (t, *J*=4.63 Hz, 1 H) 7.49 (t, *J*=7.94 Hz, 1 H) 7.54 (d, *J*=4.63 Hz, 2 H) 8.26 (d, *J*=8.25 Hz, 1 H) 9.57 (s, 1 H).

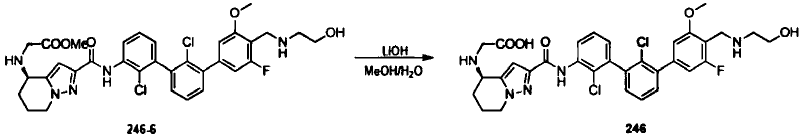

A solution of methyl 2-[[(4R)-2-[[2-chloro-3-[2-chloro-3-[3-fluoro-4-[(2-hydroxyethylamino)methyl]-5-methoxy-phenyl]phenyl]phenyl]carbamoyl]-4,5,6,7-tetrahydropyrazolo[1,5-a]pyridin-4-yl]amino]acetate (81.00 mg, 120.80 µmol, 1 eq) in MeOH (2 mL) and H₂O (2 mL) was treated with LiOH (15.21 mg, 362.39 µmol, 3 eq) and stirred at room temperature for 12 hours. The reaction mixture was concentrated under reduced pressure, and the residue was purified by reversed-phase HPLC (column: Waters XBridge 150 * 25 mm * 5 µm; mobile phase: [water (NH₄HCO₃)-ACN]; gradient: 18% to 48% B over 9 min) to afford Compound 246 (19 mg, 23.9% yield) as a white solid.

LCMS m/z 656.2 [M+1]⁺.

¹H NMR (400 MHz, DMSO-d6) δ ppm 1.68 - 1.80 (m, 1 H) 1.86 - 2.06 (m, 2 H) 2.14 - 2.27 (m, 1 H) 2.62 (t, *J*=5.57 Hz, 2 H) 3.28 (s, 2 H) 3.47 - 3.51 (m, 2 H) 3.79 - 3.81 (m, 2 H) 3.88 (s, 3 H) 4.04 (br t, *J*=5.82 Hz, 1 H) 4.15 (br t, *J*=6.07 Hz, 2 H) 6.82 (s, 1 H) 6.89 - 6.98 (m, 2 H) 7.20 (d, *J*=7.38 Hz, 1 H) 7.41 (t, *J*=4.50 Hz, 1 H) 7.49 (t, *J*=7.88 Hz, 1 H) 7.54 (d, *J*=4.50 Hz, 2 H) 8.27 (br d, J=8.25 Hz, 1 H) 9.57 (s, 1 H).

### 57-2. Preparation Compound 247, of 2-(((S)-2-((2,2'-dichloro-3"-fluoro-4"-(((R)-3-hydroxypyrrolidin-1-yl)methyl)-5"-methoxy-[1,1':3',1"-terphenyl]-3-yl)carbamoyl)-4,5,6,7-tetrahydropyrazolo[1,5-a]pyridin-4-yl)amino)acetic acid, and Compound 248, 2-(((R)-2-((2,2'-dichloro-3"-fluoro-4"-(((R)-3-hydroxypyrrolidin-1-yl)methyl)-5"-methoxy-[1,1':3',1"-terphenyl]-3-yl)carbamoyl)-4,5,6,7-tetrahydropyrazolo[1,5-a]pyridin-4-yl)amino)acetic acid,

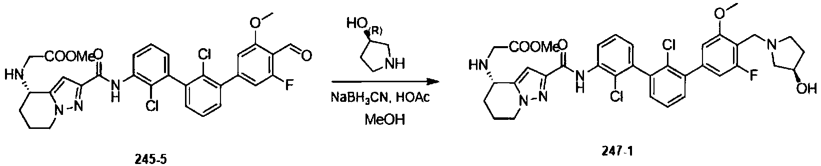

To a solution of methyl 2-[[(4S)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (100 mg, 159.88 µmol, 1 eq) and (3R)-pyrrolidin-3-ol (69.64 mg, 799.39 µmol, 66.45 µL, 5 eq) in MeOH (4 mL), NaBH₃CN (50.24 mg, 799.39 µmol, 5 eq) and AcOH (96.01 mg, 1.60 mmol, 91.53 µL, 10 eq) were added, and the mixture was stirred at 60 °C for 2 hours. The reaction mixture was cooled to room temperature, concentrated under reduced pressure, and the residue was purified by reversed-phase HPLC (column: Phenomenex Luna C18 150 * 25 mm * 10 µm; mobile phase: [water (HCl)-ACN]; gradient: 8% to 38% B over 10 min) to afford Compound 247-1 (40 mg, 32.3% yield) as a white solid.

LCMS m/z 696.3 [M+1]⁺.

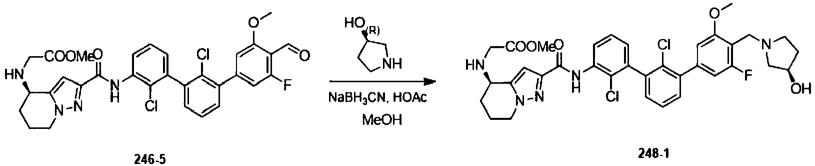

To a solution of methyl 2-[[(4R)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (80 mg, 127.90 µmol, 1 eq) and (3R)-pyrrolidin-3-ol (111.43 mg, 1.28 mmol, 106.33 µL, 10 eq) in MeOH (4 mL), NaBH₃CN (40.19 mg, 639.52 µmol, 5 eq) and AcOH (76.81 mg, 1.28 mmol, 73.22 µL, 10 eq) were added, and the mixture was stirred at 60 °C for 2 hours. The reaction mixture was cooled to room temperature, concentrated under reduced pressure, and the residue was purified by prep-TLC (SiO₂, DCM/MeOH = 10:1) to afford Compound 248-1 (95 mg, 98.1% yield) as a white solid.

LCMS m/z 696.3 [M+1]⁺.

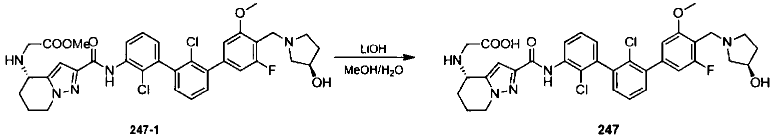

A solution of methyl 2-[[(4S)-2-[[2-chloro-3-[2-chloro-3-[3-fluoro-4-[[(3R)-3-hydroxypyrrolidin-1-yl]methyl]-5-methoxy-phenyl]phenyl]phenyl]carbamoyl]-4,5,6,7-tetrahydropyrazolo[1,5-a]pyridin-4-yl]amino]acetate (40.00 mg, 57.42 µmol, 1 eq) in MeOH (4 mL) and H₂O (2 mL) was treated with LiOH (7.23 mg, 172.27 µmol, 3 eq) and stirred at room temperature for 12 hours. The reaction mixture was concentrated under reduced pressure, and the residue was purified by reversed-phase HPLC (column: Waters XBridge 150 * 25 mm * 5 µm; mobile phase: [water (NH₄HCO₃)-ACN]; gradient: 20% to 50% B over 9 min) to afford Compound 247 (32 mg, 81.6% yield) as a white solid.

LCMS m/z 682.3 [M+1]⁺.

¹H NMR (400 MHz, DMSO-d6) δ ppm 1.42 - 1.54 (m, 1 H) 1.76 (br s, 1 H) 1.87 - 2.06 (m, 3 H) 2.20 (br s, 1 H) 2.31 - 2.38 (m, 1 H) 2.43 - 2.48 (m, 1 H) 2.61 (q, *J*=7.50 Hz, 1 H) 2.70 - 2.77 (m, 1 H) 3.32 (s, 2 H) 3.65 (br s, 3 H) 3.86 (s, 3 H) 4.05 (br s, 1 H) 4.16 (br s, 3 H) 6.82 (s, 1 H) 6.87 - 6.96 (m, 2 H) 7.20 (br d, *J*=7.50 Hz, 1 H) 7.40 (br dd, *J*=5.75, 2.88 Hz, 1 H) 7.46 - 7.57 (m, 3 H) 8.27 (br d, *J*=8.13 Hz, 1 H) 9.57 (s, 1 H).

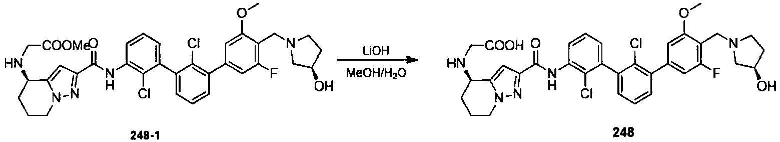

A solution of methyl 2-[[(4R)-2-[[2-chloro-3-[2-chloro-3-[3-fluoro-4-[[(3R)-3-hydroxypyrrolidin-1-yl]methyl]-5-methoxy-phenyl]phenyl]phenyl]carbamoyl]-4,5,6,7-tetrahydropyrazolo[1,5-a]pyridin-4-yl]amino]acetate (95.00 mg, 136.38 µmol, 1 eq) in MeOH (2 mL) and H₂O (2 mL) was treated with LiOH (17.17 mg, 409.13 µmol, 3 eq) and stirred at room temperature for 12 hours. The reaction mixture was concentrated under reduced pressure, and the residue was purified by reversed-phase HPLC (column: Waters XBridge 150 * 25 mm * 5 µm; mobile phase: [water (NH₄HCO₃)-ACN]; gradient: 18% to 48% B over 9 min) to afford Compound 248 (24 mg, 25.7% yield) as a white solid.

LCMS m/z 682.3 [M+1]⁺.

¹H NMR (400 MHz, DMSO-d6) δ ppm 1.49 (br s, 1 H) 1.76 (br s, 1 H) 1.86 - 2.06 (m, 3 H) 2.20 (br s, 1 H) 2.35 (br d, *J*=6.75 Hz, 1 H) 2.46 (br d, *J*=6.50 Hz, 1 H) 2.57 - 2.65 (m, 1 H) 2.70 - 2.78 (m, 1 H) 3.32 (br s, 2 H) 3.66 (br s, 3 H) 3.86 (s, 3 H) 4.06 (br s, 1 H) 4.15 (br s, 3 H) 6.83 (s, 1 H) 6.86 - 6.98 (m, 2 H) 7.20 (br d, *J*=7.25 Hz, 1 H) 7.41 (br s, 1 H) 7.44 - 7.60 (m, 3 H) 8.27 (br d, *J*=7.88 Hz, 1 H) 9.57 (s, 1 H).

### 57-3. Preparation of Compound 249, 2-(((S)-2-((2,2'-dichloro-3"-fluoro-5"-methoxy-4"-(((((S)-5-oxopyrrolidin-2-yl)methyl)amino)methyl)-[1,1':3',1"-terphenyl]-3-yl)carbamoyl)-4,5,6,7-tetrahydropyrazolo[1,5-a]pyridin-4-yl)amino)acetic acid, and Compound 250, 2-(((R)-2-((2,2'-dichloro-3"-fluoro-5"-methoxy-4"-(((((S)-5-oxopyrrolidin-2-yl)methyl)amino)methyl)-[1,1':3',1 "-terphenyl]-3-yl)carbamoyl)-4,5,6,7-tetrahydropyrazolo[1,5-a]pyridin-4-yl)amino)acetic acid

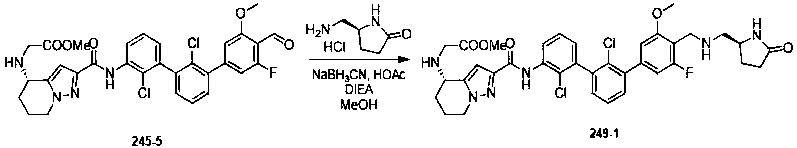

To a solution of methyl 2-[[(4S)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (100 mg, 159.88 µmol, 1 eq) and (5S)-5-(aminomethyl)pyrrolidin-2-one (120.39 mg, 799.39 µmol, 5 eq, HCl) in MeOH (4 mL), NaBH₃CN (50.23 mg, 799.39 µmol, 5 eq), DIPEA (103.32 mg, 799.39 µmol, 139.24 µL, 5 eq), and AcOH (96.01 mg, 1.60 mmol, 91.52 µL, 10 eq) were added, and the mixture was stirred at 60 °C for 12 hours. The reaction mixture was cooled to room temperature, concentrated under reduced pressure, and the residue was purified by reversed-phase HPLC (column: Phenomenex Luna C18 150 * 25 mm * 10 µm; mobile phase: [water (HCl)-ACN]; gradient: 8% to 38% B over 10 min) to afford Compound 249-1 (70 mg, 60.5% yield) as a white solid.

LCMS m/z 723.4 [M+1]⁺.

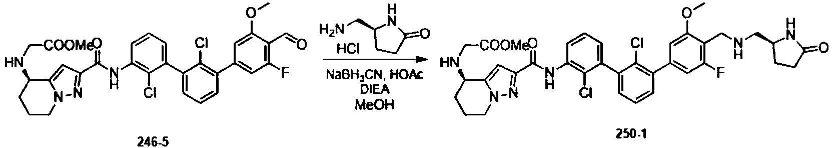

To a solution of methyl 2-[[(4R)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (80 mg, 127.90 µmol, 1 eq) and (5S)-5-(aminomethyl)pyrrolidin-2-one (192.63 mg, 1.28 mmol, 10 eq, HCl) in MeOH (4 mL), NaBH₃CN (40.19 mg, 639.52 µmol, 5 eq), DIPEA (165.31 mg, 1.28 mmol, 222.78 µL, 10 eq), and AcOH (76.81 mg, 1.28 mmol, 73.22 µL, 10 eq) were added, and the mixture was stirred at 60 °C for 2 hours. The reaction mixture was cooled to room temperature, concentrated under reduced pressure, and the residue was purified by prep-TLC (SiO₂, DCM/MeOH = 10:1) to afford Compound 250-1 (90 mg, 88.4% yield) as a white solid.

LCMS m/z 723.3 [M+1]⁺.

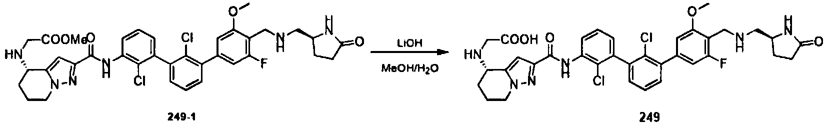

A solution of methyl 2-[[(4S)-2-[[2-chloro-3-[2-chloro-3-[3-fluoro-5-methoxy-4-[[[(2S)-5-oxopyrrolidin-2-yl]methylamino]methyl]phenyl]phenyl]phenyl]carbamoyl]-4,5,6,7-tetrahydropyrazolo[1,5-a]pyridin-4-yl]amino]acetate (70.00 mg, 96.74 µmol, 1 eq) in MeOH (4 mL) and H₂O (2 mL) was treated with LiOH (12.18 mg, 290.21 µmol, 3 eq) and stirred at room temperature for 12 hours. The reaction mixture was concentrated under reduced pressure, and the residue was purified by reversed-phase HPLC (column: Waters XBridge 150 * 25 mm * 5 µm; mobile phase: [water (NH₄HCO₃)-ACN]; gradient: 18% to 48% B over 9 min) to afford Compound 249 (20 mg, 29.1% yield) as a white solid.

LCMS m/z 709.3 [M+1]⁺.

¹H NMR (400 MHz, DMSO-d6) δ ppm 1.62 - 1.69 (m, 1 H) 1.70 - 1.78 (m, 1 H) 1.92 (br s, 1 H) 1.98 - 2.14 (m, 4 H) 2.16 - 2.26 (m, 1 H) 3.29 - 3.29 (m, 2 H) 3.59 (br s, 3 H) 3.69 - 3.80 (m, 3 H) 3.88 (s, 3 H) 4.03 (br s, 1 H) 4.15 (br t, *J*=5.75 Hz, 2 H) 6.81 (s, 1 H) 6.89 - 6.95 (m, 2 H) 7.20 (d, *J*=7.26 Hz, 1 H) 7.41 (t, *J*=4.63 Hz, 1 H) 7.49 (t, *J*=8.00 Hz, 1 H) 7.52 - 7.56 (m, 2 H) 7.67 (s, 1 H) 8.27 (d, *J*=8.13 Hz, 1 H) 9.57 (s, 1 H).

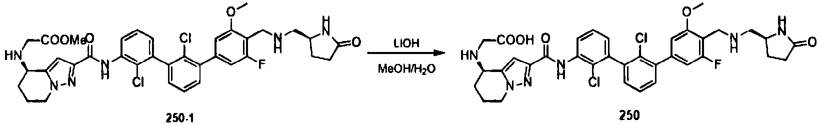

A solution of methyl 2-[[(4R)-2-[[2-chloro-3-[2-chloro-3-[3-fluoro-5-methoxy-4-[[[(2S)-5-oxopyrrolidin-2-yl]methylamino]methyl]phenyl]phenyl]phenyl]carbamoyl]-4,5,6,7-tetrahydropyrazolo[1,5-a]pyridin-4-yl]amino]acetate (90.00 mg, 124.37 µmol, 1 eq) in MeOH (2 mL) and H₂O (2 mL) was treated with LiOH (15.66 mg, 373.12 µmol, 3 eq) and stirred at room temperature for 12 hours. The reaction mixture was concentrated under reduced pressure, and the residue was purified by reversed-phase HPLC (column: Waters XBridge 150 * 25 mm * 5 µm; mobile phase: [water (NH₄HCO₃)-ACN]; gradient: 18% to 48% B over 9 min) to afford Compound 250 (55 mg, 62.3% yield) as a white solid.

LCMS m/z 709.4 [M+1]⁺.

¹H NMR (400 MHz, DMSO-d6) δ ppm 1.59 - 1.80 (m, 2 H) 1.92 (br dd, *J*=13.57, 6.44 Hz, 1 H) 1.97 - 2.15 (m, 4 H) 2.16 - 2.26 (m, 1 H) 3.29 - 3.32 (m, 2 H) 3.72 - 3.80 (m, 3 H) 3.88 (s, 3 H) 4.05 (br t, *J*=5.82 Hz, 1 H) 4.15 (br t, *J*=6.00 Hz, 2 H) 6.82 (s, 1 H) 6.86 - 6.97 (m, 2 H) 7.20 (d, *J*=7.38 Hz, 1 H) 7.41 (t, *J*=4.57 Hz, 1 H) 7.44 - 7.58 (m, 3 H) 7.67 (s, 1 H) 8.26 (br d, *J*=8.13 Hz, 1 H) 9.57 (s, 1 H).

### [Preparation Example 58]

### Preparation of Compounds 251 to 252

### 58-1. Preparation of Compound 251, isopropyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate

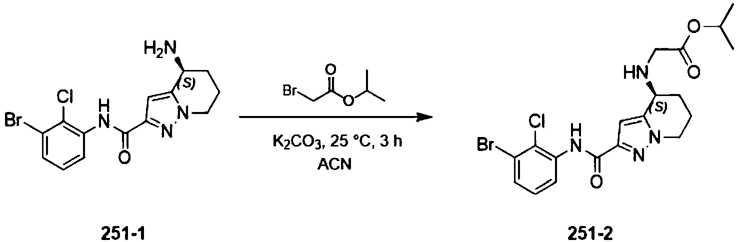

To a solution of (4S)-4-amino-N-(3-bromo-2-chloro-phenyl)-4,5,6,7-tetrahydropyrazolo[1,5-a]pyridine-2-carboxamide (300 mg, 811.59 µmol, 1 eq) and isopropyl 2-bromoacetate (161.61 mg, 892.75 µmol, 115.52 µL, 1.1 eq in ACN (5 mL), K₂CO₃ (336.51 mg, 2.43 mmol, 3 eq) was added, and the mixture was stirred at room temperature for 3 hours. Water (40 mL) was added, and the mixture was extracted with ethyl acetate (30 mL x 3). The combined organic layers were washed with brine (60 mL), dried over Na₂SO₄, concentrated under reduced pressure, and the residue was purified by flash silica gel chromatography (Petroleum ether/Ethyl acetate = 100/1 to 2/1) to afford Compound 251-2 (260 mg, 61.3% yield) as a white solid.

¹H NMR (400 MHz, DMSO-d6) δ = 9.65 (s, 1H), 8.08 (dd, J = 1.4, 8.3 Hz, 1H), 7.59 (dd, J = 1.4, 8.0 Hz, 1H), 7.33 (t, J = 8.1 Hz, 1H), 6.75 (s, 1H), 4.99 - 4.89 (m, 1H), 4.23 - 4.08 (m, 2H), 3.94 (br s, 1H), 3.40 - 3.35 (m, 2H), 2.28 - 2.15 (m, 1H), 2.03 - 1.85 (m, 2H), 1.77 - 1.65 (m, 1H), 1.20 (d, J = 6.3 Hz, 6H).

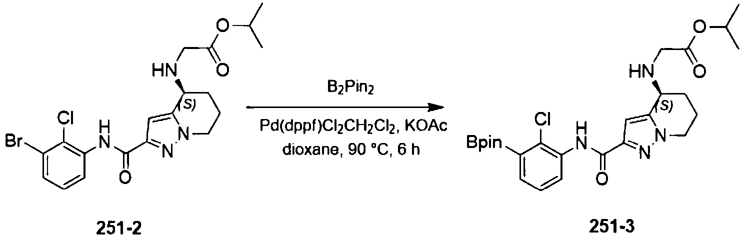

A mixture of isopropyl 2-[[(4S)-2-[(3-bromo-2-chloro-phenyl)carbamoyl]-4,5,6,7-tetrahydropyrazolo[1,5-a]pyridin-4-yl]amino]acetate (200 mg, 425.75 µmol, 1 eq), 4,4,5,5-tetramethyl-2-(4,4,5,5-tetramethyl-1,3,2-dioxaborolan-2-yl)-1,3,2-dioxaborolane (324.34 mg, 1.28 mmol, 3 eq), KOAc (125.35 mg, 1.28 mmol, 3 eq), and cyclopentyl(diphenyl)phosphane;dichloromethane; dichloropalladium iron (34.77 mg, 42.58 µmol, 0.1 eq) in dioxane (8 mL) was stirred under a nitrogen atmosphere at 90 °C for 5 hours. The reaction mixture was cooled to room temperature, filtered, and the filtrate was concentrated under reduced pressure. The residue was purified by flash silica gel chromatography (Petroleum ether/Ethyl acetate = 100/1 to 2/1) to afford Compound 251-3 (239 mg, 86.8% yield) as a white solid.

LCMS m/z 517.3 [M+1]⁺.

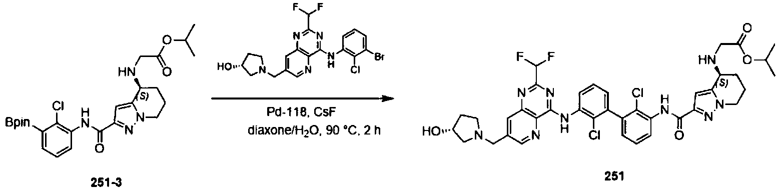

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (159.93 mg, 309.45 µmol, 1.5 eq), CsF (94.01 mg, 618.91 µmol, 3 eq, 22.85 µL), and ditert-butyl(cyclopentyl)phosphane;dichloropalladium;iron (13.45 mg, 20.63 µmol, 0.1 eq) were dissolved in dioxane (5 mL) and H₂O (1 mL) and stirred under a nitrogen atmosphere at 90 °C for 2 hours. The reaction mixture was cooled to room temperature, filtered, and the filtrate was concentrated under reduced pressure. The residue was purified by prep-HPLC (column: Waters XBridge 150 * 25 mm * 5 µm; mobile phase: [water (NH₄HCO₃)-ACN]; gradient: 50%-80% B over 9 min) to afford Compound 251 (9 mg, 5.27% yield) as a white solid.

LCMS m/z 794.3 [M+1]⁺.

¹H NMR (400 MHz, METHANOL-d4) δ = 8.95 - 8.90 (m, 2H), 8.39 (dd, J = 1.3, 8.3 Hz, 1H), 8.19 (s, 1H), 7.49 (t, J = 8.0 Hz, 1H), 7.42 (t, J = 7.9 Hz, 1H), 7.12 (dd, J = 1.6, 7.6 Hz, 2H), 6.99 - 6.90 (m, 1H), 6.83 (d, J = 1.8 Hz, 1H), 6.80 - 6.61 (m, 1H), 5.04 - 4.98 (m, 1H), 4.33 (br t, J = 6.6 Hz, 1H), 4.17 - 4.13 (m, 2H), 4.01 (br t, J = 5.9 Hz, 1H), 3.96 - 3.81 (m, 2H), 3.45 - 3.43 (m, 2H), 2.85 - 2.76 (m, 2H), 2.61 - 2.50 (m, 2H), 2.26 (br s, 1H), 2.16 - 2.04 (m, 2H), 1.96 (br dd, J = 6.7, 14.2 Hz, 1H), 1.83 - 1.70 (m, 2H), 1.23 (d, J = 6.3 Hz, 9H).

### 58-2. Preparation of Compound 252, tert-butyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate

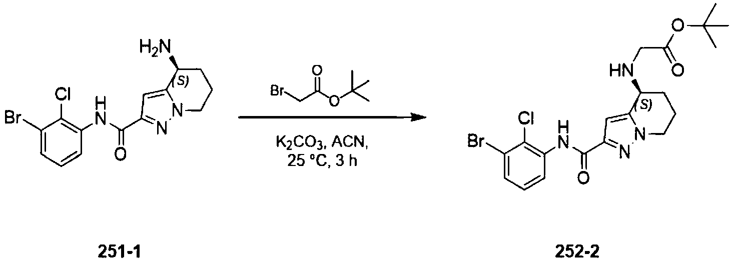

To a solution of (4S)-4-amino-N-(3-bromo-2-chloro-phenyl)-4,5,6,7-tetrahydropyrazolo[1,5-a]pyridine-2-carboxamide (200 mg, 541.06 µmol, 1 eq) and tert-butyl 2-bromoacetate (116.09 mg, 595.17 µmol, 87.88 µL, 1.1 eq) in acetonitrile (5 mL), K₂CO₃ (224.33 mg, 1.62 mmol, 3 eq) was added. The reaction mixture was stirred at room temperature for 3 hours. The mixture was then filtered, and the filtrate was concentrated under reduced pressure. The residue was purified by prep-HPLC (column: Waters XBridge 150 * 25 mm * 5 µm; mobile phase: [water (NH₄HCO₃)-ACN]; gradient: 58%-88% B over 9 min) to afford Compound 252-2 (220 mg, 79.8% yield) as a white solid.

LCMS m/z 967.2 [2M+1]⁺.

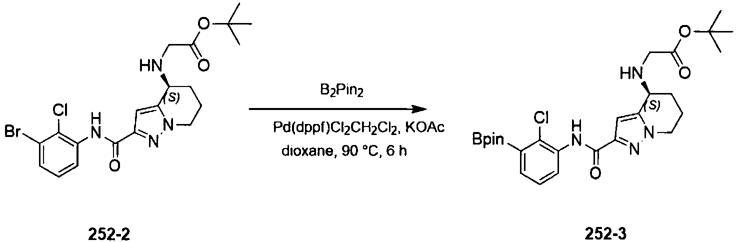

A mixture of tert-Butyl 2-[[(4S)-2-[(3-bromo-2-chloro-phenyl)carbamoyl]-4,5,6,7-tetrahydropyrazolo[1,5-a]pyridin-4-yl]amino]acetate (150 mg, 310.05 µmol, 1 eq), 4,4,5,5-tetramethyl-2-(4,4,5,5-tetramethyl-1,3,2-dioxaborolan-2-yl)-1,3,2-dioxaborolane (236.20 mg, 930.16 µmol, 3 eq), KOAc (91.29 mg, 930.16 µmol, 3 eq), and cyclopentyl(diphenyl)phosphane;dichloromethane;dichloropalladium;iron (25.32 mg, 31.01 µmol, 0.1 eq) in dioxane (8 mL) was stirred under a nitrogen atmosphere at 90 °C for 3 hours. The mixture was cooled to room temperature, and water (40 mL) was added. The mixture was extracted with ethyl acetate (30 mL x 3), and the combined organic layers were washed with brine (60 mL), dried over Na₂SO₄, and concentrated under reduced pressure. The residue was purified by prep-TLC (Petroleum ether/Ethyl acetate = 2/1) to afford Compound 252-3 (152 mg, 73.8% yield) as a white solid.

LCMS m/z 531.3 [M+1]⁺.

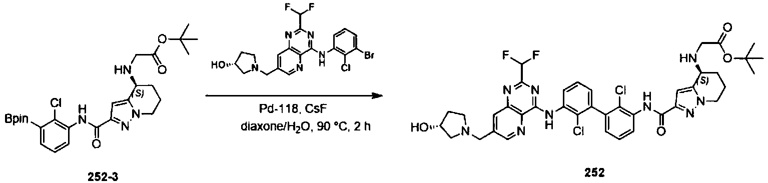

A mixture of (3R)-1-[[4-(3-bromo-2-chloro-anilino)-2-(difluoromethyl)pyrido[3,2-d]pyrimidin-7-yl]methyl]pyrrolidin-3-ol (70 mg, 144.41 µmol, 1 eq), tert-butyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (91.99 mg, 173.29 µmol, 1.2 eq), CsF (65.81 mg, 433.24 µmol, 15.99 µL, 3 eq), and ditert-butyl(cyclopentyl)phosphane;dichloropalladium;iron (9.41 mg, 14.44 µmol, 0.1 eq) in dioxane (5 mL) / H₂O (1 mL) was stirred under a nitrogen atmosphere at 90 °C for 3 hours. The mixture was cooled to room temperature, filtered, and the filtrate was concentrated under reduced pressure. The residue was purified by prep-HPLC (column: Waters Xbridge 150 * 25 mm * 5 µm; mobile phase: [water (NH₄HCO₃)-ACN]; gradient: 50%-80% B over 9 min) to afford Compound 252 (3 mg, 2.47% yield) as a white solid.

LCMS m/z 808.5 [M+1]⁺.

¹H NMR (400 MHz, METHANOL-d4 ) δ = 9.00 (s, 1H), 8.98 (d, J = 8.4 Hz, 1H), 8.43 (d, J = 8.4 Hz, 1H), 8.25 (s, 1H), 7.54 (t, J = 7.9 Hz, 1H), 7.46 (t, J = 7.9 Hz, 1H), 7.17 (dd, J = 5.4, 6.8 Hz, 2H), 7.51 - 7.01 (m, 1H), 6.88 - 6.56 (m, 2H), 4.56 (br s, 4H), 4.41 - 4.35 (m, 1H), 4.24 - 4.15 (m, 2H), 4.07 - 4.02 (m, 1H), 4.02 - 3.89 (m, 2H), 3.41 (s, 2H), 2.84 (dd, J = 5.9, 9.8 Hz, 2H), 2.62 - 2.56 (m, 2H), 2.23 - 2.06 (m, 2H), 2.01 (br dd, J = 6.2, 13.7 Hz, 1H), 1.84 - 1.72 (m, 2H), 1.49 (s, 9H).

### [Preparation Example 59]

### Preparation of Compound 253, (4S,4'S)-N,N'-(2,2'-dimethyl-[1,1'-biphenyl]-3,3'-diyl)bis(4-((2-hydroxyethyl)amino)-4,5,6,7-tetrahydropyrazolo[1,5-a]pyridine-2-carboxamide)

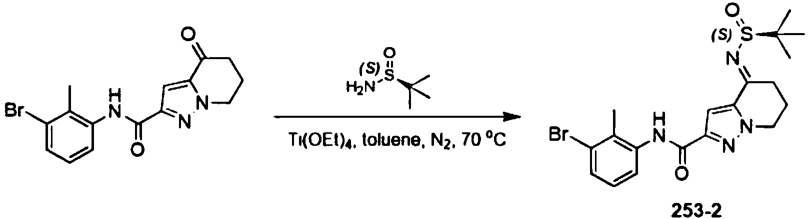

A mixture of N-(3-bromo-2-methyl-phenyl)-4-oxo-6,7-dihydro-5H-pyrazolo[1,5-a]pyridine-2-carboxamide (6 g, 17.23 mmol, 1 eq), (S)-2-methylpropane-2-sulfinamide (6.27 g, 51.70 mmol, 3 eq), and Ti(OEt)₄ (7.86 g, 34.46 mmol, 7.15 mL, 2 eq) in toluene (100 mL) was stirred under a nitrogen atmosphere at 70 °C for 12 hours. The mixture was cooled to room temperature, concentrated under reduced pressure, and the residue was poured into H₂O (500 mL) and extracted with ethyl acetate (300 mL * 3). The combined organic layers were washed with brine (30 mL), dried over Na₂SO₄, and concentrated under reduced pressure to afford Compound 253-2 (10 g, 90.0% yield) as a brown solid, which was used directly in the next reaction without further purification.

LCMS m/z 451.0 [M+1]⁺.

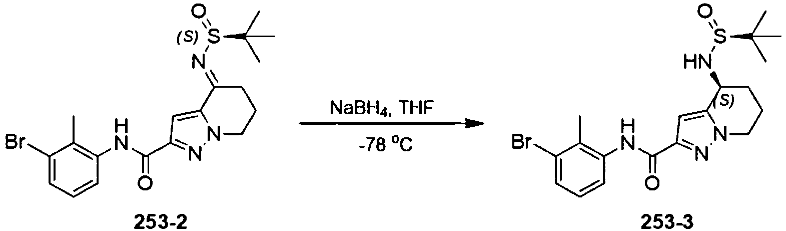

A solution of (4E)-N-(3-bromo-2-methyl-phenyl)-4-[(S)-tert-butylsulfinyl]imino-6,7-dihydro-5H-pyrazolo[1,5-a]pyridine-2-carboxamide (10 g, 22.15 mmol, 1 eq) in THF (150 mL) was cooled to - 78 °C, and NaBH₄ (3.35 g, 88.62 mmol, 4 eq) was added. The reaction mixture was stirred at -78 °C for 12 hours. The reaction was quenched by pouring into H₂O (500 mL) and extracted with ethyl acetate (300 mL * 3). The combined organic layers were washed with brine (50 mL), dried over Na₂SO₄, and concentrated under reduced pressure. The residue was purified by column chromatography on silica gel (Petroleum ether/Ethyl acetate = 1/1 to 0/1) to afford Compound 253-3 (5 g, 46.2% yield) as a white solid.

LCMS m/z 455.1 [M+1]⁺.

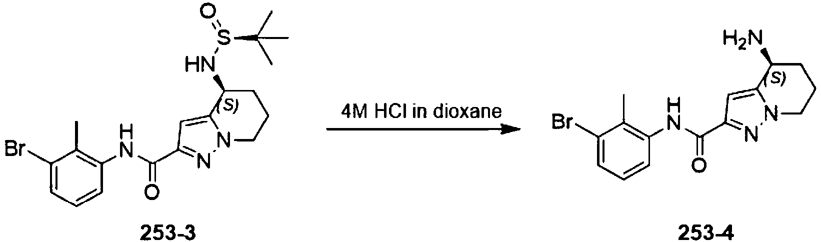

To a solution of (4S)-N-(3-bromo-2-methyl-phenyl)-4-[[(S)-tert-butylsulfinyl]amino]-4,5,6,7-tetrahydropyrazolo[1,5-a]pyridine-2-carboxamide (5 g, 11.03 mmol, 1 eq) in dioxane (50 mL), HCl/dioxane (4 M, 5.51 mL, 2 eq) was added. The reaction mixture was stirred at room temperature for 12 hours. The mixture was concentrated under reduced pressure, and the residue was poured into H₂O (500 mL) and extracted with ethyl acetate (300 mL * 3). The combined organic layers were washed with brine (30 mL), dried over Na₂SO₄, and concentrated under reduced pressure. The residue was purified by column chromatography on silica gel (Ethyl acetate/MeOH = 10/1 to 5/1) to afford Compound 253-4 (2 g, 51.4% yield) as a white solid.

LCMS m/z 349.1 [M+1]⁺.

¹H NMR (400 MHz, DMSO-d6) δ ppm 1.46 - 1.57 (m, 1 H) 1.86 - 1.96 (m, 1 H) 1.98 - 2.07 (m, 2 H) 2.13 (br dd, J=11.32, 6.82 Hz, 1 H) 2.26 (s, 3 H) 3.15 - 3.19 (m, 1 H) 3.91 (dd, J=8.76, 5.38 Hz, 1 H) 4.01 - 4.10 (m, 1 H) 4.13 - 4.21 (m, 1 H) 6.74 (s, 1 H) 7.11 - 7.18 (m, 1 H) 7.45 (dd, J=18.70, 7.82 Hz, 2 H) 9.77 (s, 1 H):

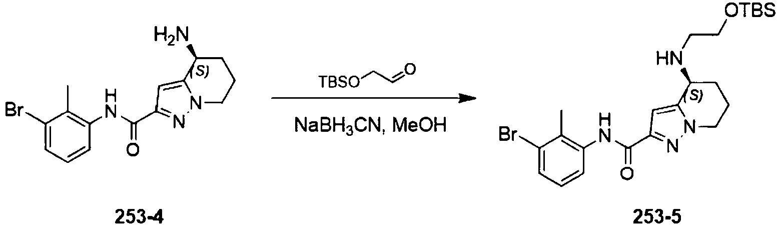

To a solution of (4S)-4-amino-N-(3-bromo-2-methyl-phenyl)-4,5,6,7-tetrahydropyrazolo[1,5-a]pyridine-2-carboxamide (1 g, 2.86 mmol, 1 eq) and 2-[tert-butyl(dimethyl)silyl]oxyacetaldehyde (598.97 mg, 3.44 mmol, 654.61 µL, 1.2 eq) in MeOH (10 mL), NaBH₃CN (899.74 mg, 14.32 mmol, 5 eq) was added at room temperature, and the reaction mixture was stirred at room temperature for 12 hours. The mixture was concentrated under reduced pressure, and the residue was purified by reversed-phase HPLC (0.1% NH₃·H₂O condition, column: Waters Xbridge Prep OBD C18 150 * 40 mm * 10 µm; mobile phase: [water (NH₄HCO₃)-ACN]; gradient: 58%-88% B over 15 min) to afford Compound 253-5 (880 mg, 59.3% yield) as a brown liquid.

LCMS m/z 509.2 [M+1]⁺.

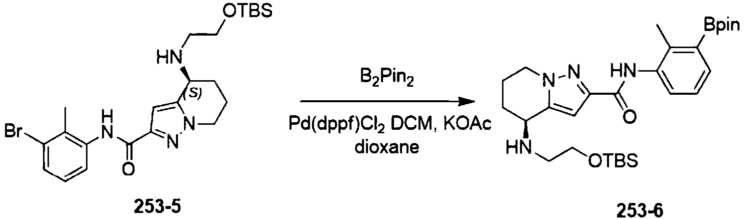

A mixture of (4S)-N-(3-bromo-2-methyl-phenyl)-4-[2-[tert-butyl(dimethyl)silyl]oxyethylamino]-4,5,6,7-tetrahydropyrazolo[1,5-a]pyridine-2-carboxamide (400 mg, 788.12 µmol, 1 eq), 4,4,5,5-tetramethyl-2-(4,4,5,5-tetramethyl-1,3,2-dioxaborolan-2-yl)-1,3,2-dioxaborolane (400.27 mg, 1.58 mmol, 2 eq), and KOAc (154.70 mg, 1.58 mmol, 2 eq) in dioxane (5 mL) was treated with Pd(dppf)Cl₂·CH₂Cl₂ (32.18 mg, 39.41 µmol, 0.05 eq) under a nitrogen atmosphere. The mixture was stirred at 90 °C for 12 hours. After cooling to room temperature, the reaction mixture was concentrated under reduced pressure, and the residue was purified by column chromatography (SiO₂, Petroleum ether/Ethyl acetate = 1/1 to 0/1) to afford Compound 253-6 (373 mg, 70.8% yield) as a brown liquid.

LCMS m/z 555.4 [M+1]⁺.

¹H NMR (400 MHz, DMSO-d6) δ ppm 0.02 - 0.07 (m, 6 H) 0.87 (s, 9 H) 1.31 (s, 11 H) 1.61 - 1.71 (m, 1 H) 1.85 - 1.95 (m, 1 H) 1.99 (s, 1 H) 2.00 - 2.06 (m, 1 H) 2.14 - 2.24 (m, 1 H) 2.37 (s, 3 H) 2.68 - 2.75 (m, 2 H) 3.65 (t, J=6.00 Hz, 2 H) 3.90 (br dd, J=7.00, 5.38 Hz, 1 H) 4.03 (q, J=7.13 Hz, 1 H) 4.11 - 4.19 (m, 2 H) 6.63 - 6.68 (m, 1 H) 7.18 (t, J=7.63 Hz, 1 H) 7.46 - 7.56 (m, 2 H) 9.48 (s, 1 H).

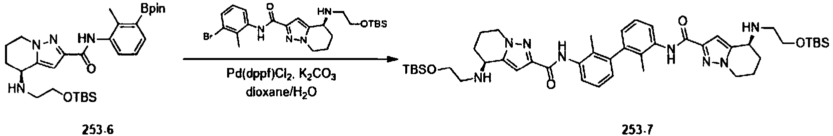

A mixture of (4S)-N-(3-bromo-2-methyl-phenyl)-4-[2-[tert-butyl(dimethyl)silyl]oxyethylamino]-4,5,6,7-tetrahydropyrazolo[1,5-a]pyridine-2-carboxamide (384.36 mg, 757.30 µmol, 1.2 eq), (4S)-4-[2-[tert-butyl(dimethyl)silyl]oxyethylamino]-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (350 mg, 631.08 µmol, 1 eq), and K₂CO₃ (174.44 mg, 1.26 mmol, 2 eq) in dioxane (5 mL)/H₂O (1 mL) was treated with Pd(dppf)Cl₂ (23.09 mg, 31.55 µmol, 0.05 eq) under a nitrogen atmosphere. The mixture was stirred at 80 °C for 4 hours. After cooling to room temperature, the reaction mixture was concentrated under reduced pressure, and the residue was purified by column chromatography (SiO₂, Petroleum ether/Ethyl acetate = 3/1) to afford Compound 253-7 (390 mg, 68.6% yield) as a brown solid.

LCMS m/z 856.6 [M+1]⁺.

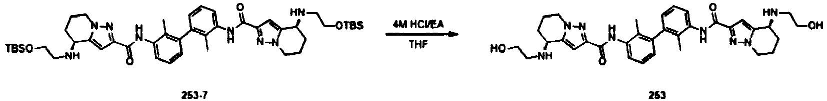

A solution of (4S)-4-[2-[tert-butyl(dimethyl)silyl]oxyethylamino]-N-[3-[3-[[(4S)-4-[2-[tert-butyl(dimethyl)silyl]oxyethylamino]-4,5,6,7-tetrahydropyrazolo[1,5-a]pyridine-2-carbonyl]amino]-2-methyl-phenyl]-2-methyl-phenyl]-4,5,6,7-tetrahydropyrazolo[1,5-a]pyridine-2-carboxamide (200 mg, 233.84 µmol, 1 eq) in THF (10 mL) was treated with HCl/EtOAc (4 M, 584.61 µL, 10 eq). The mixture was stirred at room temperature for 12 hours. The reaction mixture was concentrated under reduced pressure, and the residue was purified by reversed-phase HPLC (0.1% NH₃·H₂O condition, column: Waters Xbridge 150 * 25 mm * 5 µm; mobile phase: [water (NH₄HCO₃) -ACN]; gradient: 18%-48% B over 9 min) to afford Compound 253 (96 mg, 65.5% yield) as a white solid.

LCMS m/z 627.6 [M+1]⁺.

¹H NMR (400 MHz, DMSO-d6) δ ppm 1.62 - 1.75 (m, 2 H) 1.88 - 1.96 (m, 8 H) 1.97 - 2.04 (m, 2 H) 2.07 (br d, J=3.50 Hz, 2 H) 2.14 - 2.23 (m, 2 H) 2.60 - 2.76 (m, 4 H) 3.44 - 3.52 (m, 4 H) 3.88 (br t, J=6.07 Hz, 2 H) 4.08 - 4.20 (m, 4 H) 4.53 (t, J=5.38 Hz, 2 H) 6.71 (s, 2 H) 6.96 (d, J=7.25 Hz, 2 H) 7.27 (t, J=7.75 Hz, 2 H) 7.56 (d, J=7.88 Hz, 2 H) 9.52 (s, 2 H).

### [Preparation Example 60]

### Preparation of Compound 254, (S)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid, and Compound 255, (R)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid

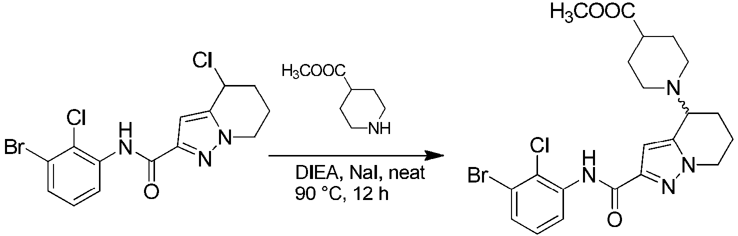

To a solution of N-(3-bromo-2-chloro-phenyl)-4-chloro-4,5,6,7-tetrahydropyrazolo[1,5-a]pyridine-2-carboxamide (2.5 g, 6.43 mmol, 1 eq) and methyl piperidine-4-carboxylate (3.68 g, 25.70 mmol, 4 eq), DIPEA (2.49 g, 19.28 mmol, 3.36 mL, 3 eq) and NaI (288.94 mg, 1.93 mmol, 0.3 eq) were added. The reaction mixture was stirred at 90 °C for 12 hours. After cooling to room temperature, the mixture was diluted with H₂O (100 mL) and extracted with ethyl acetate (100 mL * 3). The combined organic layers were washed with brine (60 mL), dried over Na₂SO₄, filtered, and concentrated under reduced pressure. The residue was purified by prep-HPLC (Phenomenex Luna C18, 250 × 70 mm, 10 µm; mobile phase: H₂O (FA)-ACN; gradient: 15%-35% B over 25 min) and further separated by SFC (DAICEL CHIRALPAK AS, 250 × 30 mm, 10 µm; mobile phase: CO₂-i-PrOH (0.1% NH₃·H₂O); B%: 35%, isocratic elution mode) to afford the individual stereoisomers methyl 1-[(4S)-2-[(3-bromo-2-chlorophenyl)carbamoyl]-4,5,6,7-tetrahydropyrazolo[1,5-a]pyridin-4-yl]piperidine-4-carboxylate (1.42 g, 42.3% yield) and methyl 1-[(4R)-2-[(3-bromo-2-chloro-phenyl)carbamoyl]-4,5,6,7-tetrahydropyrazolo[1,5-a]pyridin-4-yl]piperidine-4-carboxylate (1.4 g, 41.7% yield) as a white solid.

LCMS m/z 497.0 [M+2]⁺

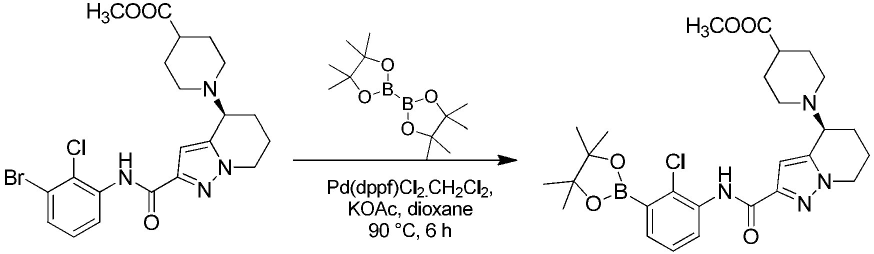

A mixture of methyl 1-[(4S)-2-[(3-bromo-2-chloro-phenyl)carbamoyl]-4,5,6,7-tetrahydropyrazolo[1,5-a]pyridin-4-yl]piperidine-4-carboxylate (200 mg, 403.39 µmol, 1 eq), 4,4,5,5-tetramethyl-2-(4,4,5,5-tetramethyl-1,3,2-dioxaborolan-2-yl)-1,3,2-dioxaborolane (307.31 mg, 1.21 mmol, 3 eq), KOAc (118.77 mg, 1.21 mmol, 3 eq), and Pd(dppf)Cl₂·CH₂Cl₂ (32.94 mg, 40.34 µmol, 0.1 eq) in dioxane (5 mL) was stirred under a nitrogen atmosphere at 90 °C for 6 hours. After cooling to room temperature, the mixture was diluted with H₂O (50 mL) and extracted with ethyl acetate (40 mL * 3). The combined organic layers were washed with brine (60 mL), dried over Na₂SO₄, filtered, and concentrated under reduced pressure. The residue was purified by flash silica gel chromatography (Petroleum ether/Ethyl acetate = 100/1 to 2/1) to afford methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (220 mg, 80.3% yield) as a white solid.

LCMS m/z 543.1 [M+1]⁺

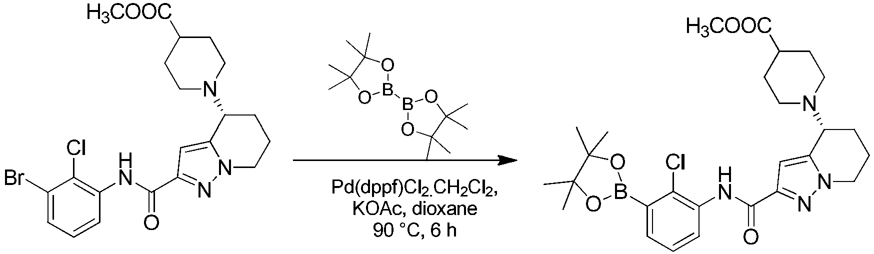

A mixture of methyl 1-[(4R)-2-[(3-bromo-2-chloro-phenyl)carbamoyl]-4,5,6,7-tetrahydropyrazolo[1,5-a]pyridin-4-yl]piperidine-4-carboxylate (200.00 mg, 403.39 µmol, 1 eq), 4,4,5,5-tetramethyl-2-(4,4,5,5-tetramethyl-1,3,2-dioxaborolan-2-yl)-1,3,2-dioxaborolane (307.31 mg, 1.21 mmol, 3 eq), KOAc (118.77 mg, 1.21 mmol, 3 eq), and Pd(dppf)Cl₂·CH₂Cl₂ (32.94 mg, 40.34 µmol, 0.1 eq) in dioxane (5 mL) was stirred under nitrogen at 90 °C for 6 hours. After cooling to room temperature, the mixture was diluted with H₂O (50 mL) and extracted with ethyl acetate (40 mL * 3). The combined organic layers were washed with brine (60 mL), dried over Na₂SO₄, filtered, and concentrated under reduced pressure. The residue was purified by flash silica gel chromatography (Petroleum ether/Ethyl acetate = 100/1 to 2/1) to afford methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (235 mg, 88.0% yield) as a white solid.

LCMS m/z 543.3 [M+1]⁺

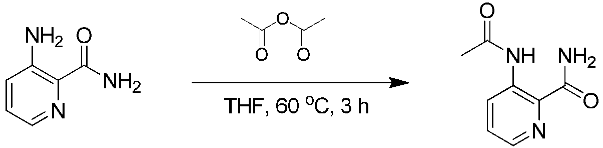

A mixture of 3-aminopyridine-2-carboxamide (5 g, 36.46 mmol, 1 eq) and acetyl acetate (37.22 g, 364.59 mmol, 34.24 mL, 10 eq) in THF (150 mL) was stirred at 60 °C for 3 hours. After cooling to room temperature, the mixture was filtered, and the filtrate was concentrated under reduced pressure to afford 3-acetamidopyridine-2-carboxamide (6.1 g, 92.4% yield), which was used in the next reaction without further purification.

¹H NMR (400 MHz, DMSO-*d₆*) δ ppm 2.14 (s, 3 H) 7.57 (dd, J=8.57, 4.44 Hz, 1 H) 7.99 (br s, 1 H) 8.29 (dd, J=4.44, 1.44 Hz, 1 H) 8.49 (br s, 1 H) 8.93 (dd, J=8.51, 1.38 Hz, 1 H) 12.13 (s, 1 H).

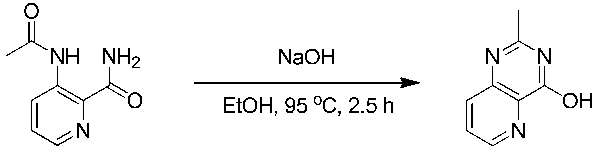

A mixture of 3-acetamidopyridine-2-carboxamide (5.6 g, 31.25 mmol, 1 eq) in EtOH (50 mL) was treated with NaOH (2.50 g, 62.51 mmol, 2 eq), and the mixture was stirred at 95 °C for 3 hours. After cooling to room temperature, the mixture was filtered, and the filtrate was concentrated under reduced pressure. The residue was purified by prep-HPLC (Phenomenex Luna C18 column, 250 × 70 mm, 10 µm; mobile phase: [water (HCl)-ACN]; gradient: 1%-15% B over 25 min) to afford 2-methylpyrido[3,2-d]pyrimidin-4-ol (5.02 g, 89.7% yield) as a white solid.

¹H NMR (400 MHz, DMSO-*d₆*) δ = 8.74 (br d, *J* = 3.6 Hz, 1H), 8.01 (dd, *J* = 1.1, 8.3 Hz, 1H), 7.78 (dd, *J* = 4.3, 8.4 Hz, 1H), 2.40 (s, 3H).

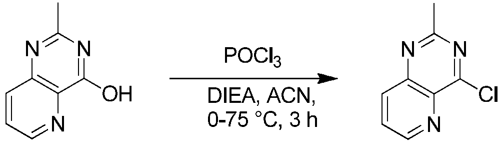

To a mixture of 2-methylpyrido[3,2-d]pyrimidin-4-ol (2 g, 12.41 mmol, 1 eq) and DIPEA (2.41 g, 18.61 mmol, 3.24 mL, 1.5 eq) in ACN (30 mL), POCl₃ (5.71 g, 37.23 mmol, 3.47 mL, 3 eq) was added 0 °C. The mixture was stirred at 75 °C for 3 hours. After cooling to room temperature, the mixture was diluted with H₂O (50 mL) and extracted with ethyl acetate (40 mL × 3). The combined organic layers were washed with brine (60 mL), dried over Na₂SO₄, filtered, and concentrated under reduced pressure. The residue was purified by flash silica gel chromatography (Petroleum ether/Ethyl acetate = 100/1 to 3/1) to afford 4-chloro-2-methyl-pyrido[3,2-d]pyrimidine (620 mg, 22.2% yield) as a white solid.

¹H NMR (400 MHz, DMSO-*d₆)* δ = 8.87 - 8.80 (m, 1H), 8.20 (br d, *J* = 8.1 Hz, 1H), 7.90 (dd, J = 4.4, 8.4 Hz, 1H), 2.54 (s, 3H).

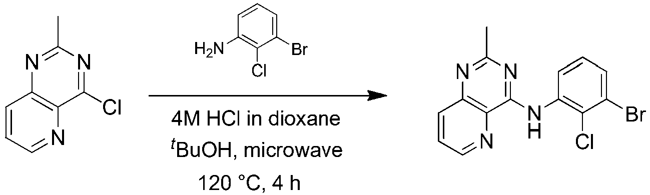

A mixture of 4-chloro-2-methyl-pyrido[3,2-d]pyrimidine (300 mg, 1.67 mmol, 1 eq) and 3-bromo-2-chloro-aniline (379.35 mg, 1.84 mmol, 1.1 eq) in t-BuOH (8 mL) was treated with HCl/dioxane (2 M, 1.50 mL, 1.80 eq) and stirred at 120 °C for 4 hours in a microwave reactor. After cooling to room temperature, the mixture was diluted with H₂O (50 mL) and extracted with ethyl acetate (40 mL × 3). The combined organic layers were washed with brine (60 mL), dried over Na₂SO₄, filtered, and concentrated under reduced pressure. The residue was purified by flash silica gel chromatography (Petroleum ether/Ethyl acetate = 100/1 to 2/1) to afford N-(3-bromo-2-chloro-phenyl)-2-methyl-pyrido[3,2-d]pyrimidin-4-amine (282 mg, 38.6% yield) as a white solid.

LCMS m/z 351.0 [M+3]⁺

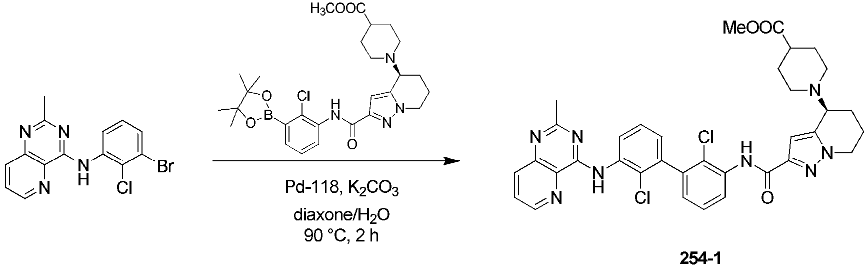

A mixture of N-(3-bromo-2-chloro-phenyl)-2-methyl-pyrido[3,2-d]pyrimidin-4-amine (80 mg, 228.82 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (149.06 mg, 274.59 µmol, 1.2 eq), ditert-butyl(cyclopentyl)phosphane;dichloropalladium;iron (14.91 mg, 22.88 µmol, 0.1 eq), and K₂CO₃ (94.87 mg, 686.47 µmol, 3 eq) in dioxane (8 mL) / H₂O (1.5 mL) was stirred under nitrogen at 90 °C for 2 hours. The mixture was cooled to room temperature, filtered, and the filtrate concentrated under reduced pressure. The residue was purified by prep-TLC (Petroleum ether/Ethyl acetate = 1/2) to afford Compound 254-1 (102 mg, 45.5% yield) as a white solid.

LCMS m/z 685.2 [M+1]⁺

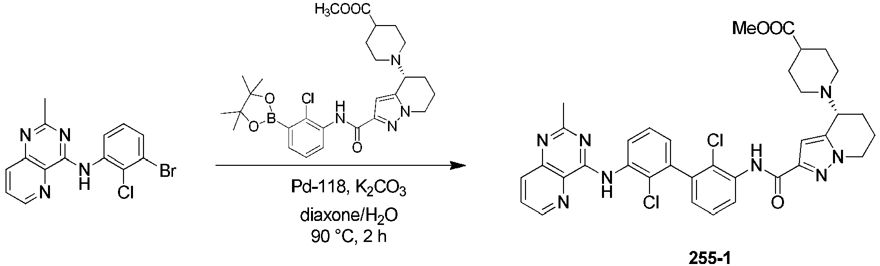

A mixture of N-(3-bromo-2-chloro-phenyl)-2-methyl-pyrido[3,2-d]pyrimidin-4-amine (80 mg, 228.82 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate 5-R (186.33 mg, 343.24 µmol, 1.5 eq), ditert-butyl(cyclopentyl)phosphane;dichloropalladium;iron (14.91 mg, 22.88 µmol, 0.1 eq), and K₂CO₃ (94.87 mg, 686.47 µmol, 3 eq) in dioxane (8 mL) / H₂O (1.5 mL) was stirred under nitrogen at 90 °C for 2 hours. The reaction mixture was cooled to room temperature, filtered, and the filtrate concentrated under reduced pressure. The residue was purified by prep-HPLC (Phenomenex Luna C18 150 * 25 mm * 10 µm; mobile phase: [H₂O (TFA)-ACN]; gradient: 16%-46% B over 15 min) to afford Compound 255-1 (62 mg, 28.0% yield) as a white solid.

LCMS m/z 685.3[M+1]⁺

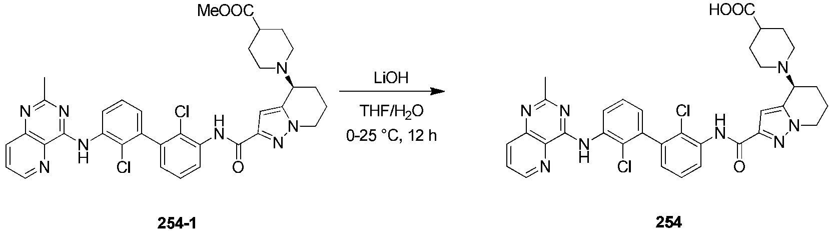

A solution of methyl 1-[(4S)-2-[[2-chloro-3-[2-chloro-3-[(2-methylpyrido[3,2-d]pyrimidin-4-yl)amino]phenyl]phenyl]carbamoyl]-4,5,6,7-tetrahydropyrazolo[1,5-a]pyridin-4-yl]piperidine-4-carboxylate 254-1 (102 mg, 148.77 µmol, 1 eq) in H₂O (3 mL) / THF (6 mL) was treated with LiOH·H₂O (18.73 mg, 446.32 µmol, 3 eq) at 0 °C. The mixture was stirred at room temperature for 12 hours. The reaction mixture was filtered, and the filtrate concentrated under reduced pressure. The residue was purified by prep-HPLC (Phenomenex Luna C18 150 * 25 mm * 10 µm; mobile phase: [water (HCl)-ACN]; gradient: 13%-43% B over 10 min) to afford Compound 254 (25 mg, 22.5% yield) as a white solid.

LCMS m/z 671.2 [M+1]⁺

¹H NMR (400 MHz, DMSO-*d₆* ) δ = 10.94 - 10.73 (m, 1H), 9.66 (d, *J =* 3.0 Hz, 1H), 8.98 (d, *J* = 3.5 Hz, 1H), 8.55 - 8.43 (m, 1H), 8.35 - 8.24 (m, 1H), 8.19 (t, *J* = 7.3 Hz, 1H), 8.01 (dd, *J* = 4.2, 8.6 Hz, 1H), 7.67 - 7.56 (m, 1H), 7.55 - 7.49 (m, 1H), 7.48 - 7.40 (m, 1H), 7.31 - 7.21 (m, 2H), 5.01 - 4.85 (m, 1H), 4.32 - 4.18 (m, 2H), 2.65 (s, 3H), 2.54 (s, 4H), 2.35 - 2.21 (m, 3H), 2.19 - 2.06 (m, 4H), 2.02 - 1.84 (m, 3H).

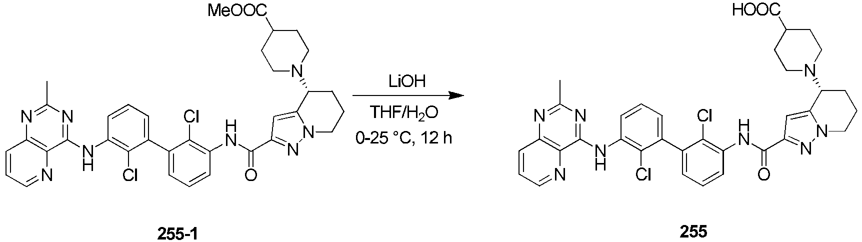

A solution of methyl 1-[(4R)-2-[[2-chloro-3-[2-chloro-3-(2-methylpyrido[3,2-d]pyrimidin-4-yl)amino]phenyl]phenyl]carbamoyl]-4,5,6,7-tetrahydropyrazolo[1,5-a]pyridin-4-yl]piperidine-4-carboxylate (30 mg, 43.76 µmol, 1 eq) in H₂O (3 mL) / THF (10 mL) was treated with LiOH·H₂O (5.51 mg, 131.27 µmol, 3 eq) at 0 °C. The mixture was stirred at room temperature for 12 hours. The reaction mixture was filtered, and the filtrate concentrated under reduced pressure. The residue was purified by prep-HPLC (Waters Xbridge 150 * 25 mm * 5 µm; mobile phase: [water (NH₄HCO₃)-ACN]; gradient: 45%-75% B over 9 min) to give Compound 255 (8 mg, 25.8% yield) as a white solid.

LCMS m/z 671.2 [M+1]⁺

¹H NMR (400 MHz, DMSO-*d₆* ) δ = 9.55 (s, 1H), 8.91 (d, *J* = 4.1 Hz, 1H), 8.82 (d, *J* = 8.4 Hz, 1H), 8.34 - 8.24 (m, 1H), 8.20 (d, *J* = 8.0 Hz, 1H), 7.92 (dd, *J* = 4.3, 8.5 Hz, 1H), 7.57 (t, *J* = 7.9 Hz, 1H), 7.50 (t, *J* = 7.9 Hz, 1H), 7.18 (t, *J =* 7.1 Hz, 2H), 6.66 (s, 1H), 4.27 - 4.17 (m, 1H), 4.08 (br s, 1H), 4.08 - 3.87 (m, 2H), 3.17 (s, 2H), 2.82 - 2.75 (m, 1H), 2.64 (s, 3H), 2.40 - 2.24 (m, 2H), 2.13 (br dd, *J* = 2.3, 6.2 Hz, 2H), 2.02 - 1.88 (m, 2H), 1.86 - 1.74 (m, 2H), 1.71 - 1.47 (m, 3H).

### [Preparation Example 61]

### Preparation of Compound 256, (R)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)piperidine-4-carboxylic acid

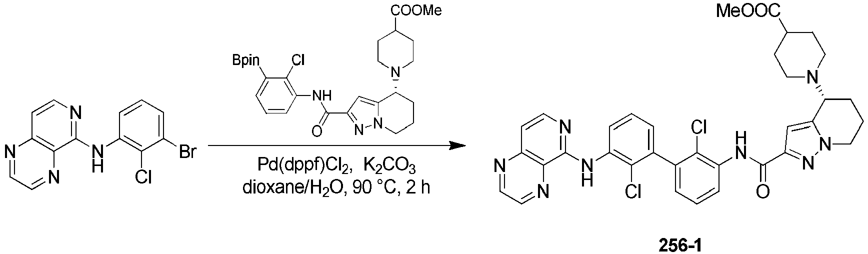

N-(3-bromo-2-chloro-phenyl)pyrido[3,4-b]pyrazin-5-amine (100 mg, 297.99 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (539.22 mg, 297.99 µmol, 1 eq), K₂CO₃ (123.55 mg, 893.96 µmol, 3 eq), and Pd(dppf)Cl₂ (21.80 mg, 29.80 µmol, 0.1 eq) were added to a mixture of dioxane (2 mL) and H₂O (0.5 mL), and the reaction mixture was stirred at 90 °C for 2 hours under a nitrogen atmosphere. After completion of the reaction, the mixture was filtered, and the filtrate was concentrated under reduced pressure. The resulting residue was purified by column chromatography (SiO₂, Petroleum ether/Ethyl acetate = 1/1) to afford Compound 256-1 (110 mg, 46.7% yield) as a yellow solid.

LCMS m/z 671.4 [M+1] ⁺.

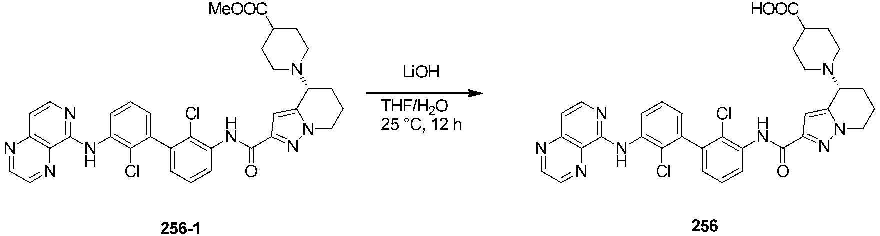

Methyl 1-[(4R)-2-[[2-chloro-3-[2-chloro-3-(pyrido[3,4-b]pyrazin-5-ylamino)phenyl]phenyl]carbamoyl]-4,5,6,7-tetrahydropyrazolo[1,5-a]pyridin-4-yl]piperidine-4-carboxylate 256-1 (40.00 mg, 59.56 µmol, 1 eq) and LiOH (7.50 mg, 178.68 µmol, 3 eq) were added to a mixture of MeOH (6 mL) and H₂O (1 mL), and the reaction mixture was stirred at room temperature for 12 hours. The reaction mixture was concentrated under reduced pressure, and the resulting residue was purified by reversed-phase HPLC (column: Phenomenex Luna C18, 150 * 25 mm * 10 µm; mobile phase: [water (HCl)-ACN]; gradient: 25% to 55% B over 10 min) to afford Compound 256 (28 mg, 70.7% yield) as a yellow solid.

LCMS m/z 657.2 [M+1] ⁺.

¹H NMR (400 MHz, DMSO-d6) δ ppm 1.23 (s, 2 H) 2.00 (br d, *J*=7.70 Hz, 2 H) 2.03 - 2.15 (m, 4 H) 2.21 - 2.31 (m, 3 H) 4.20 - 4.30 (m, 2 H) 4.93 (br d, *J*=1.59 Hz, 1 H) 7.08 - 7.12 (m, 1 H) 7.25 (s, 1 H) 7.39 (s, 1 H) 7.44 (d, *J*=5.99 Hz, 1 H) 7.53 (dt, *J*=13.39, 7.98 Hz, 2 H) 8.19 (t, *J*=8.07 Hz, 1 H) 8.41 (d, *J*=6.11 Hz, 1 H) 8.97 - 9.01 (m, 1 H) 9.02 (s, 1 H) 9.21 (d, *J*=1.71 Hz, 1 H) 9.66 (d, *J*=2.81 Hz, 1 H) 9.78 (s, 1 H).

### [Preparation Example 62]

### Preparation of Compound 257, (S)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)piperidine-4-carboxylic acid

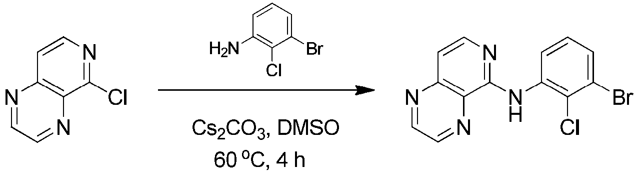

To a solution of 5-chloropyrido[3,4-b]pyrazine (1 g, 6.04 mmol, 1 eq) and 3-bromo-2-chloroaniline (1.87 g, 9.06 mmol, 1.5 eq) in DMSO (40 mL), Cs₂CO₃ (3.67 g, 11.27 mmol, 1.87 eq) was added. The reaction mixture was stirred at 60 °C for 4 hours. After cooling to room temperature, H₂O (40 mL) was added, and the mixture was extracted with ethyl acetate (30 mL x 3). The combined organic layers were washed with brine (60 mL), dried over Na₂SO₄, filtered, and concentrated under reduced pressure. The resulting residue was purified by flash silica gel chromatography (Petroleum ether/Ethyl acetate = 100/1 to 3/1) to afford N-(3-bromo-2-chloro-phenyl)pyrido[3,4-b]pyrazin-5-amine (1.1 g, 53.1% yield) as a yellow solid.

LCMS m/z 336.9 [M+1] ⁺.

¹H NMR (400 MHz, CHLOROFORM-d) δ ppm 7.21 - 7.26 (m, 1 H) 7.34 - 7.40 (m, 2 H) 8.39 (d, J=5.99 Hz, 1 H) 8.83 (s, 1 H) 9.03 (d, J=1.71 Hz, 1 H) 9.08 (d, J=8.19 Hz, 1 H) 9.73 (br s, 1 H).

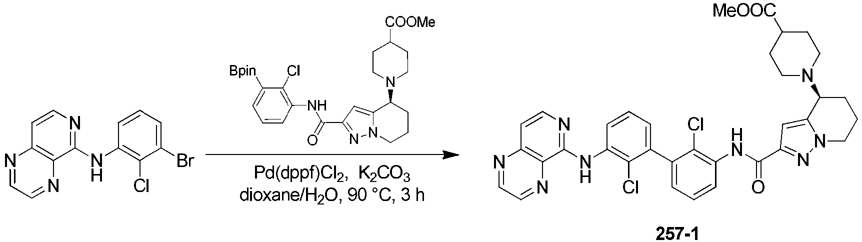

N-(3-bromo-2-chloro-phenyl)pyrido[3,4-b]pyrazin-5-amine (60 mg, 178.79 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (106.76 mg, 196.67 µmol, 1.1 eq), K₂CO₃ (74.13 mg, 536.38 µmol, 3 eq), and Pd(dppf)Cl₂ (13.08 mg, 17.88 µmol, 0.1 eq) were added to a mixture of dioxane (2 mL) and H₂O (0.5 mL), and the reaction mixture was stirred at 90 °C for 3 hours under a nitrogen atmosphere. After cooling to room temperature, the reaction mixture was concentrated under reduced pressure, and the resulting residue was purified by column chromatography (SiO₂, Petroleum ether/Ethyl acetate = 1/1) to afford Compound 257-1 (50 mg, 38.3% yield) as a yellow solid.

LCMS m/z 671.2 [M+1] ⁺.

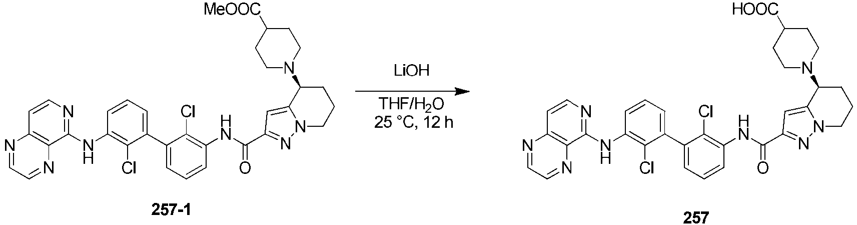

Methyl 1-[(4S)-2-[[2-chloro-3-[2-chloro-3-(pyrido[3,4-b]pyrazin-5-ylamino)phenyl]phenyl]carbamoyl]-4,5,6,7-tetrahydropyrazolo[1,5-a]pyridin-4-yl]piperidine-4-carboxylate 257-1 (40 mg, 59.56 µmol, 1 eq) and LiOH (7.50 mg, 178.68 µmol, 3 eq) were added to a mixture of H₂O (2 mL) and THF (10 mL), and the reaction mixture was stirred at room temperature for 12 hours. The reaction mixture was concentrated under reduced pressure, and the resulting residue was purified by reversed-phase HPLC (column: Phenomenex Luna C18, 150 * 25 mm * 10 µm; mobile phase: [water (HCl)-ACN]; gradient: 40% to 70% B over 10 min) to afford Compound 257 (28 mg, 70.0% yield) as a yellow solid.

LCMS m/z 657.2 [M+1] ⁺.

¹H NMR (400 MHz, DMSO-d6) δ ppm 1.84 - 2.16 (m, 4 H) 2.18 - 2.37 (m, 4 H) 4.15 - 4.32 (m, 2 H) 7.09 (d, *J*=7.21 Hz, 1 H) 7.23 (d, *J*=7.09 Hz, 1 H) 7.27 - 7.39 (m, 1 H) 7.44 (d, *J*=5.99 Hz, 1 H) 7.53 (br d, *J*=12.96 Hz, 2 H) 8.19 (s, 1 H) 8.41 (d, *J*=5.87 Hz, 1 H) 9.02 (s, 2 H) 9.20 (s, 1 H) 9.66 (d, *J*=2.81 Hz, 1 H) 9.74 (s, 1 H)

### [Preparation Example 63]

### Preparation of Compound 258, 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid, and Compound 259, 2-(((R)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid,

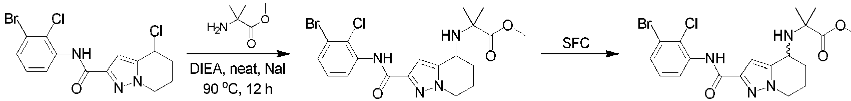

To a solution of N-(3-bromo-2-chloro-phenyl)-4-chloro-4,5,6,7-tetrahydropyrazolo[1,5-a]pyridine-2-carboxamide (3 g, 7.71 mmol, 1 eq) and methyl 2-amino-2-methyl-propanoate (9.03 g, 77.11 mmol, 10 eq), DIPEA (2.99 g, 23.13 mmol, 4.03 mL, 3 eq) and NaI (115.57 mg, 771.06 µmol, 0.1 eq) were added. The reaction mixture was stirred at 90 °C for 12 hours. After cooling to room temperature, the reaction mixture was filtered, and the filtrate was concentrated under reduced pressure. The resulting residue was purified by column chromatography (SiO₂, Petroleum ether/Ethyl acetate = 3/1 to 1/1) and further purified by reversed-phase HPLC (column: Waters Xbridge BEH C18, 250 × 50 mm, 10 µm; mobile phase: [water (NH₄HCO₃)-ACN]; gradient: 55% to 85% B over 20 min). The obtained racemate was separated by SFC (column: DAICEL CHIRALPAK IG, 250 mm × 30 mm, 10 µm; mobile phase: CO₂/ACN/i-PrOH (0.1% NH₃·H₂O); B%: 0%, isocratic elution mode) to afford the isomer methyl 2-[[(4S)-2-[(3-bromo-2-chloro-phenyl)carbamoyl]-4,5,6,7-tetrahydropyrazolo[1,5-a]pyridin-4-yl]amino]-2-methyl-propanoate (466 mg, 12.61% yield) and the isomer methyl 2-[[(4R)-2-[(3-bromo-2-chloro-phenyl)carbamoyl]-4,5,6,7-tetrahydropyrazolo[1,5-a]pyridin-4-yl]amino]-2-methyl-propanoate (471 mg, 12.8% yield), each obtained as a white solid.

LCMS m/z 470.9 [M+3]⁺.

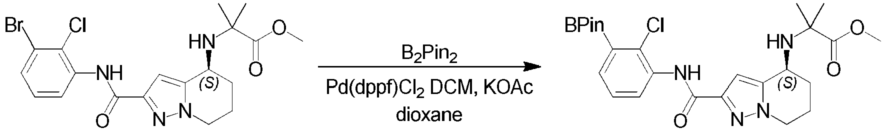

Methyl 2-[[(4S)-2-[(3-bromo-2-chloro-phenyl)carbamoyl]-4,5,6,7-tetrahydropyrazolo[1,5-a]pyridin-4-yl]amino]-2-methyl-propanoate (320 mg, 681.20 µmol, 1 eq), 4,4,5,5-tetramethyl-2-(4,4,5,5-tetramethyl-1,3,2-dioxaborolan-2-yl)-1,3,2-dioxaborolane (518.95 mg, 2.04 mmol, 3 eq), AcOK (200.56 mg, 2.04 mmol, 3 eq), and Pd(dppf)Cl₂·CH₂Cl₂ (55.63 mg, 68.12 µmol, 0.1 eq) were added to dioxane (6 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 1/0 to 0/1) to afford methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (494 mg, 98.2% yield) as a yellow solid. The product was confirmed by LCMS (EW41301-454-P1C1).

LCMS (EW41301-454-P1C1), m/z 517.2 [M+1]⁺.

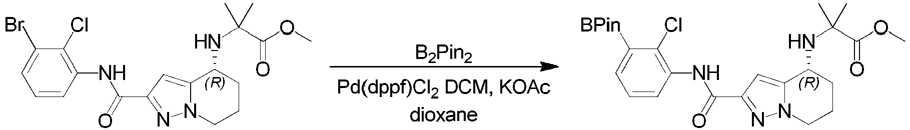

Methyl 2-[[(4R)-2-[(3-bromo-2-chloro-phenyl)carbamoyl]-4,5,6,7-tetrahydropyrazolo[1,5-a]pyridin-4-yl]amino]-2-methyl-propanoate (320.00 mg, 681.20 µmol, 1 eq), 4,4,5,5-tetramethyl-2-(4,4,5,5-tetramethyl-1,3,2-dioxaborolan-2-yl)-1,3,2-dioxaborolane (518.95 mg, 2.04 mmol, 3 eq), AcOK (200.56 mg, 2.04 mmol, 3 eq), and Pd(dppf)Cl₂·CH₂Cl₂ (55.63 mg, 68.12 µmol, 0.1 eq) were added to dioxane (6 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 1/0 to 0/1) to afford methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (510 mg, 99.9% yield) as a yellow solid.

LCMS m/z 517.2 [M+1]⁺.

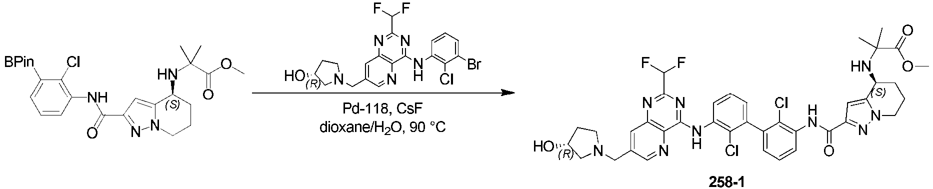

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (138.61 mg, 268.19 µmol, 1.3 eq), ditert-butyl(cyclopentyl)phosphane;dichloropalladium;iron (13.45 mg, 20.63 µmol, 0.1 eq), and CsF (94.01 mg, 618.91 µmol, 3 eq) were added to a mixture of dioxane (8 mL) and H₂O (2 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 10:1) to afford Compound 258-1 (246 mg, 97.5% yield) as a yellow solid.

LCMS m/z 794.3 [M+1]⁺.

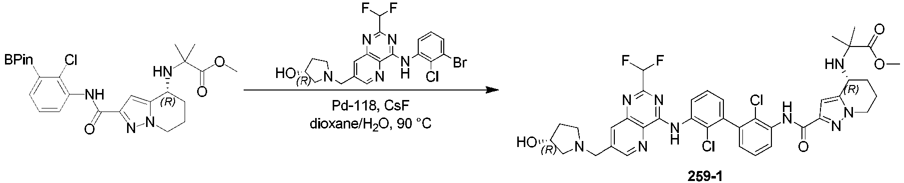

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (138.61 mg, 268.19 µmol, 1.3 eq), CsF (94.01 mg, 618.91 µmol, 3 eq), and ditert-butyl(cyclopentyl)phosphane;dichloropalladium;iron (13.45 mg, 20.63 µmol, 0.1 eq) were added to a mixture of dioxane (8 mL) and H₂O (2 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 10:1) to afford Compound 259-1 (220 mg, 93.9% yield) as a yellow solid.

LCMS m/z 794.3 [M+1]⁺.

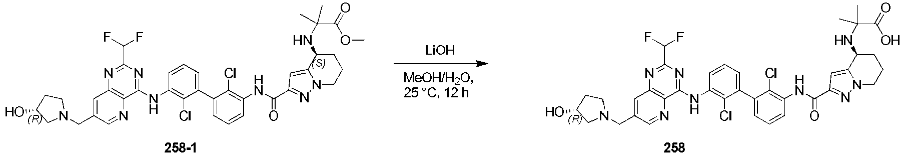

Methyl 2-[[(4S)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]amino]-2-methyl-propanoate 258-1 (240 mg, 302.01 µmol, 1 eq) and LiOH·H₂O (38.02 mg, 906.03 µmol, 3 eq) were added to a mixture of H₂O (1 mL) and MeOH (5 mL), and the reaction mixture was stirred at room temperature for 12 hours. The reaction mixture was concentrated under reduced pressure, and the resulting residue was purified by reversed-phase HPLC (column: Waters Xbridge, 150 * 25 mm * 5 µm; mobile phase: [water (NH₄HCO₃)-ACN]; gradient: 22% to 52% B over 9 min) to afford Compound 258 (29 mg, 12.1% yield) as a yellow solid.

LCMS m/z 780.3 [M+1]⁺.

¹H NMR (400 MHz, DMSO-*d*₆) δ ppm 1.30 (d, *J*=3.13 Hz, 6 H) 1.54 - 1.69 (m, 2 H) 1.87 - 1.97 (m, 1 H) 1.98 - 2.07 (m, 2 H) 2.09 - 2.18 (m, 1 H) 2.31 - 2.45 (m, 2 H) 2.64 - 2.80 (m, 2 H) 3.90 - 3.97 (m, 2 H) 4.08 - 4.17 (m, 2 H) 4.20 - 4.29 (m, 1 H) 6.73 - 6.82 (m, 1 H) 6.87 - 7.04 (m, 1 H) 7.17 - 7.29 (m, 2 H) 7.51 (t, *J*=7.82 Hz, 1 H) 7.60 (t, *J*=7.94 Hz, 1 H) 8.23 - 8.33 (m, 2 H) 8.57 (d, *J*=8.38 Hz, 1 H) 9.02 (s, 1 H) 9.55 (s, 1 H) 10.29 (s, 1 H).

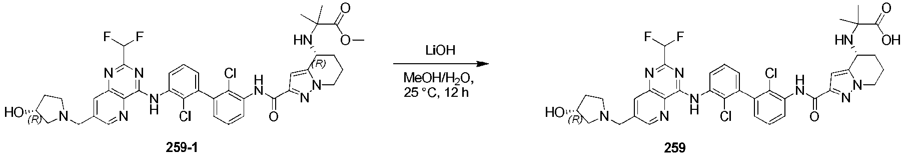

Methyl 2-[[(4R)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]amino]-2-methyl-propanoate 259-1 (220 mg, 276.84 µmol, 1 eq) and LiOH·H₂O (34.85 mg, 830.53 µmol, 3 eq) were added to a mixture of H₂O (1 mL) and MeOH (5 mL), and the reaction mixture was stirred at room temperature for 12 hours. The reaction mixture was concentrated under reduced pressure, and the resulting residue was purified by reversed-phase HPLC (column: Waters Xbridge, 150 * 25 mm * 5 µm; mobile phase: [water (NH₄HCO₃)-ACN]; gradient: 22% to 52% B over 9 min) to afford Compound 259 (46 mg, 21.0% yield) as a yellow solid.

LCMS m/z 782.3 [M+3]⁺.

¹H NMR (400 MHz, DMSO-*d*₆) δ ppm 1.34 (br s, 6 H) 1.59 - 1.72 (m, 2 H) 1.91 - 2.01 (m, 1 H) 2.03 - 2.12 (m, 2 H) 2.13 - 2.24 (m, 1 H) 2.46 (dd, *J*=9.51, 3.38 Hz, 1 H) 2.69 - 2.81 (m, 3 H) 3.86 - 4.00 (m, 4 H) 4.16 (br s, 2 H) 4.23 - 4.30 (m, 1 H) 6.76 - 6.86 (m, 1 H) 6.89 - 7.09 (m, 1 H) 7.20 - 7.26 (m, 1 H) 7.26 - 7.32 (m, 1 H) 7.55 (t, *J*=7.88 Hz, 1 H) 7.64 (t, *J*=7.88 Hz, 1 H) 8.29 (s, 1 H) 8.34 (br d, *J*=8.25 Hz, 1 H) 8.58 - 8.65 (m, 1 H) 9.05 (d, *J*=1.50 Hz, 1 H) 9.59 (s, 1 H) 10.25 - 10.37 (m, 1 H).

### [Preparation Example 64]

### Preparation of Compound 259-1, (R)-2-((2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)amino)-2-methylpropanoic acid

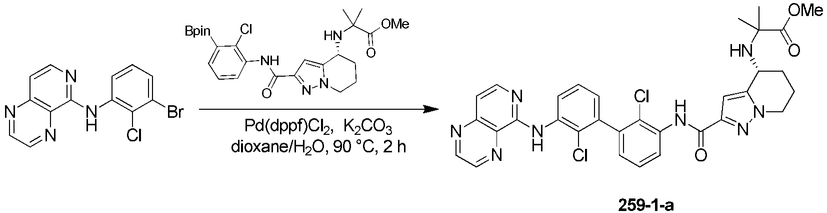

N-(3-bromo-2-chloro-phenyl)pyrido[3,4-b]pyrazin-5-amine (50 mg, 148.99 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (96.25 mg, 148.99 µmol, 1 eq), K₂CO₃ (61.78 mg, 446.98 µmol, 3 eq), and Pd(dppf)Cl₂ (10.90 mg, 14.90 µmol, 0.1 eq) were added to a mixture of dioxane (2 mL) and H₂O (0.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 2/1) to afford Compound 259-1-a (50 mg, 41.59% yield) as a yellow solid.

LCMS m/z 645.2 [M+1] ⁺.

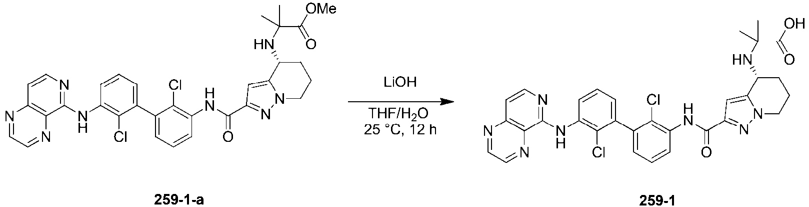

Methyl 2-[[(4R)-2-[[2-chloro-3-[2-chloro-3-(pyrido[3,4-b]pyrazin-5-ylamino)phenyl]phenyl]carbamoyl]-4,5,6,7-tetrahydropyrazolo[1,5-a]pyridin-4-yl]amino]-2-methyl-propanoate 259-1-a (50 mg, 46.47 µmol, 1 eq) and LiOH (5.85 mg, 139.42 µmol, 3 eq) were added to a mixture of H₂O (1 mL) and THF (5 mL), and the reaction mixture was stirred at room temperature for 12 hours. The reaction mixture was concentrated under reduced pressure, and the resulting residue was purified by reversed-phase HPLC (column: Phenomenex Luna C18, 150 × 25 mm, 10 µm; mobile phase: [water (HCl)-ACN]; gradient: 25% to 55% B over 10 min) to afford Compound 259-1 (15 mg, 50.6% yield) as a yellow solid.

LCMS m/z 631.3 [M+1] ⁺.

¹H NMR (400 MHz, DMSO-*d*6) δ ppm 1.43 - 1.71 (m, 7 H) 1.94 - 2.28 (m, 6 H) 4.12 - 4.21 (m, 1 H) 4.22 - 4.33 (m, 1 H) 7.02 (br dd,*J*=2.75, 1.38 Hz, 1 H) 7.08 (br d,*J*=7.63 Hz, 1 H) 7.22 (d,*J*=7.50 Hz, 1 H) 7.44 (d,*J*=5.88 Hz, 1 H) 7.47 - 7.59 (m, 2 H) 8.22 (br d,*J*=7.63 Hz, 1 H) 8.41 (d,*J*=6.00 Hz, 1 H) 8.97 - 9.04 (m, 2 H) 9.20 (d,*J*=1.75 Hz, 1 H) 9.64 (s, 1 H) 9.73 (s, 1 H).

### [Preparation Example 65]

### Preparation of Compound 260, (S)-2-((2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)amino)-2-methylpropanoic acid

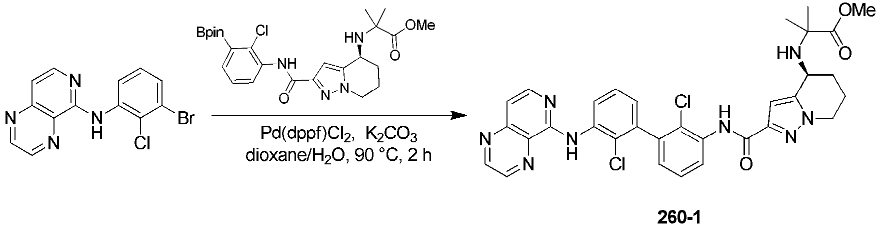

N-(3-bromo-2-chlorophenyl)pyrido[3,4-b]pyrazin-5-amine (107.80 mg, 208.59 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (186.90 mg, 208.59 µmol, 1 eq), K₂CO₃ (86.49 mg, 625.77 µmol, 3 eq), and Pd(dppf)Cl₂ (15.26 mg, 20.86 µmol, 0.1 eq) were added to a mixture of dioxane (2 mL) and H₂O (0.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 2/1) to afford Compound 260-1 (40 mg, 29.1% yield) as a yellow solid.

LCMS m/z 645.2 [M+1] ⁺.

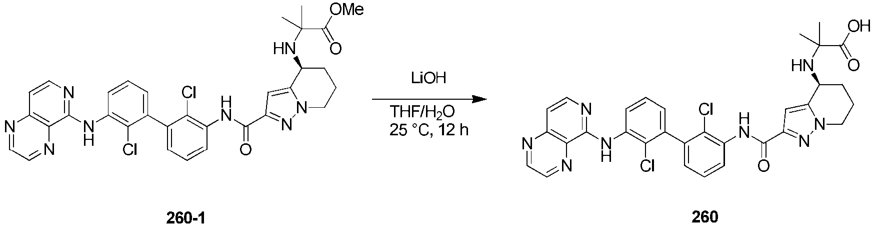

(S)-Methyl 2-((2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)amino)-2-methylpropanoate 260-1 (40 mg, 61.96 µmol, 1 eq) and LiOH (7.80 mg, 185.89 µmol, 3 eq) were added to a mixture of H₂O (1 mL) and THF (5 mL), and the reaction mixture was stirred at room temperature for 12 hours. The reaction mixture was concentrated under reduced pressure, and the resulting residue was purified by reversed-phase HPLC (column: Phenomenex Luna C18, 150 * 25 mm * 10 µm; mobile phase: [water (HCl)-ACN]; gradient: 25% to 55% B over 10 min) to afford Compound 260 (15 mg, 37.5% yield) as a yellow solid.

LCMS m/z 631.52 [M+1] ⁺.

¹H NMR (400 MHz, DMSO-*d*6) δ ppm 1.61 (br d, *J*=12.76 Hz, 6 H) 1.95 - 2.11 (m, 2 H) 2.13 - 2.21 (m, 2 H) 2.22 - 2.35 (m, 2 H) 4.14 - 4.24 (m, 1 H) 4.26 - 4.35 (m, 1 H) 7.04 - 7.12 (m, 2 H) 7.24 (d, *J*=7.50 Hz, 1 H) 7.45 (d, *J*=6.00 Hz, 1 H) 7.54 (dt, *J*=14.23, 7.83 Hz, 2 H) 8.18 - 8.24 (m, 1 H) 8.43 (d, *J*=6.00 Hz, 1 H) 8.99 - 9.05 (m, 2 H) 9.21 (d, *J*=1.88 Hz, 1 H) 9.66 (s, 1 H) 9.74 (s, 1 H)

### [Preparation Example 66]

### Preparation of Compound 261, 1-((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid, and , Compound 262 and 1-((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid,

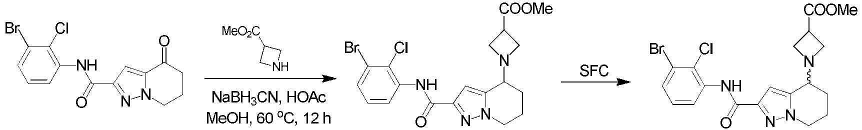

To a solution of N-(3-bromo-2-chloro-phenyl)-4-oxo-6,7-dihydro-5H-pyrazolo[1,5-a]pyridine-2-carboxamide (4 g, 10.85 mmol, 1 eq) and methyl azetidine-3-carboxylate hydrochloride (1.97 g, 13.02 mmol, 1.2 eq) in MeOH (80 mL), AcOH (3.26 g, 54.26 mmol, 3.11 mL, 5 eq) and NaBH₃CN (3.41 g, 54.26 mmol, 5 eq) were added. The reaction mixture was stirred at 60 °C for 12 hours. After cooling to room temperature, the reaction mixture was concentrated under reduced pressure. The resulting residue was purified by column chromatography (SiO₂, Petroleum ether/Ethyl acetate = 1/1 to 0/1) and further separated by SFC (EW41301-417-P1A1) (column: DAICEL CHIRALCEL OJ, 250 mm * 30 mm * 10 µm; mobile phase: CO₂-ACN/i-PrOH (0.1% NH₃·H₂O); B%: 40%, isocratic elution mode) to afford the enantiomer methyl 1-[(4S)-2-[(3-bromo-2-chloro-phenyl)carbamoyl]-4,5,6,7-tetrahydropyrazolo[1,5-a]pyridin-4-yl]azetidine-3-carboxylate (978 mg, 17.3% yield) and the enantiomer methyl 1-[(4R)-2-[(3-bromo-2-chloro-phenyl)carbamoyl]-4,5,6,7-tetrahydropyrazolo[1,5-a]pyridin-4-yl]azetidine-3-carboxylate (784 mg, 15.1% yield), each obtained as a white solid.

LCMS m/z 466.9 [M+1]⁺.

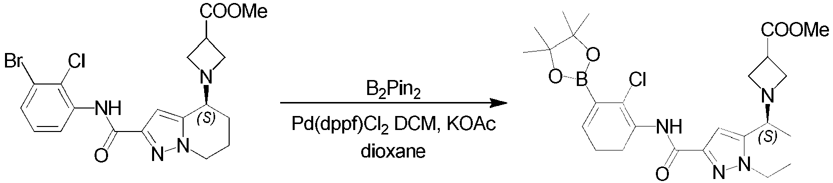

Methyl 1-[(4S)-2-[(3-bromo-2-chloro-phenyl)carbamoyl]-4,5,6,7-tetrahydropyrazolo[1,5-a]pyridin-4-yl]azetidine-3-carboxylate (330 mg, 705.52 µmol, 1 eq), 4,4,5,5-tetramethyl-2-(4,4,5,5-tetramethyl-1,3,2-dioxaborolan-2-yl)-1,3,2-dioxaborolane (537.47 mg, 2.12 mmol, 3 eq), Pd(dppf)Cl₂·CH₂Cl₂ (57.62 mg, 70.55 µmol, 0.1 eq), and KOAc (207.72 mg, 2.12 mmol, 3 eq) were added to dioxane (6 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 1/0 to 1/2) to afford methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (425 mg, 93.6% yield) as a yellow solid.

LCMS m/z 515.5 [M+1]⁺.

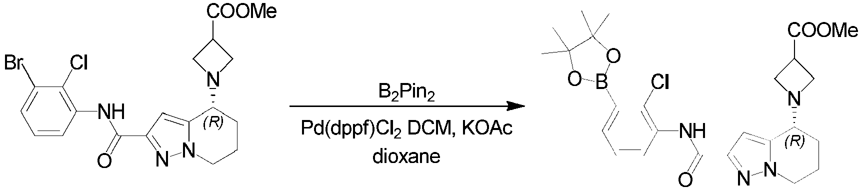

Methyl 1-[(4R)-2-[(3-bromo-2-chloro-phenyl)carbamoyl]-4,5,6,7-tetrahydropyrazolo[1,5-a]pyridin-4-yl]azetidine-3-carboxylate (600 mg, 1.28 mmol, 1 eq), 4,4,5,5-tetramethyl-2-(4,4,5,5-tetramethyl-1,3,2-dioxaborolan-2-yl)-1,3,2-dioxaborolane (977.22 mg, 3.85 mmol, 3 eq), Pd(dppf)Cl₂·CH₂Cl₂ (104.75 mg, 128.28 µmol, 0.1 eq), and KOAc (377.67 mg, 3.85 mmol, 3 eq) were added to dioxane (10 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 1/0 to 1/2) to afford methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (748 mg, 90.6% yield) as a yellow solid.

LCMS m/z 515.2 [M+1]⁺.

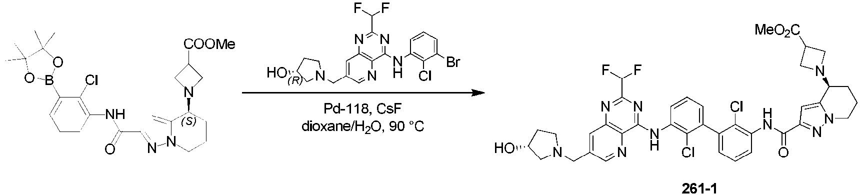

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (138.07 mg, 268.19 µmol, 1.3 eq), CsF (94.01 mg, 618.91 µmol, 3 eq), and ditert-butyl(cyclopentyl)phosphane;dichloropalladium;iron (13.45 mg, 20.63 µmol, 0.1 eq) were added to a mixture of dioxane (8 mL) and H₂O (2 mL), and the reaction mixture was stirred at 90 °C for 8 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 10:1) to afford Compound 261-1 (254 mg, 94.7% yield) as a yellow solid.

LCMS m/z 792.3 [M+1]⁺.

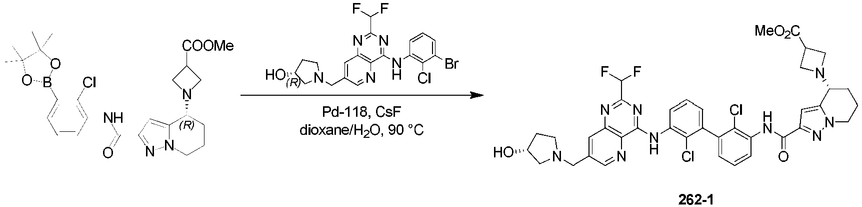

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate R-4 (138.07 mg, 268.19 µmol, 1.3 eq), CsF (94.01 mg, 618.91 µmol, 3 eq), and ditert-butyl(cyclopentyl)phosphane;dichloropalladium;iron (13.45 mg, 20.63 µmol, 0.1 eq) were added to a mixture of dioxane (8 mL) and H₂O (2 mL), and the reaction mixture was stirred at 90 °C for 8 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 10:1) to afford Compound 261-1 (233 mg, 91.1% yield) as a yellow solid.

LCMS /z 792.3 [M+1]⁺.

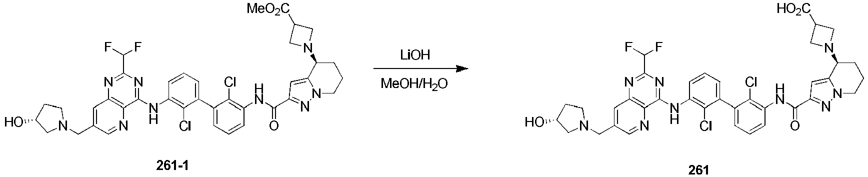

To a mixture of Methyl 1-[(4S)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]azetidine-3-carboxylate 261-1 (254 mg, 320.44 µmol, 1 eq) in H₂O (2 mL) and MeOH (10 mL), LiOH·H₂O (40.34 mg, 961.32 µmol, 3 eq) was added, and the reaction mixture was stirred at room temperature for 4 hours. The reaction mixture was concentrated under reduced pressure, and the resulting residue was purified by reversed-phase HPLC (column: Waters Xbridge, 150 * 25 mm * 5 µm; mobile phase: [water (NH₄HCO₃)-ACN]; gradient: 20% to 50% B over 9 min) to afford Compound 261 (76 mg, 28.9% yield) as a yellow solid.

*LCMS m/z 778.3 [M+1]⁺.

¹H NMR (400 MHz, DMSO-*d*₆) δ ppm 1.10 - 1.25 (m, 1 H) 1.52 - 1.62 (m, 1 H) 1.68 - 1.78 (m, 2 H) 1.79 - 1.89 (m, 1 H) 1.98 - 2.06 (m, 1 H) 2.12 - 2.24 (m, 1 H) 2.38 - 2.43 (m, 1 H) 2.64 - 2.78 (m, 3 H) 3.11 - 3.16 (m, 2 H) 3.21 (br s, 2 H) 3.83 - 3.93 (m, 2 H) 4.04 - 4.12 (m, 1 H) 4.22 (br dd, *J=*7.07, 4.69 Hz, 2 H) 4.70 - 4.81 (m, 1 H) 6.73 - 6.76 (m, 1 H) 6.85 - 7.01 (m, 1 H) 7.19 (dd, *J*=7.57, 1.19 Hz, 1 H) 7.24 (d, *J*=7.63 Hz, 1 H) 7.50 (t, *J*=7.94 Hz, 1 H) 7.59 (t, *J*=7.94 Hz, 1 H) 8.24 - 8.29 (m, 2 H) 8.57 (d, *J*=8.13 Hz, 1 H) 9.00 (d, *J*=1.50 Hz, 1 H) 9.56 (s, 1 H) 10.20 - 10.34 (m, 1 H).

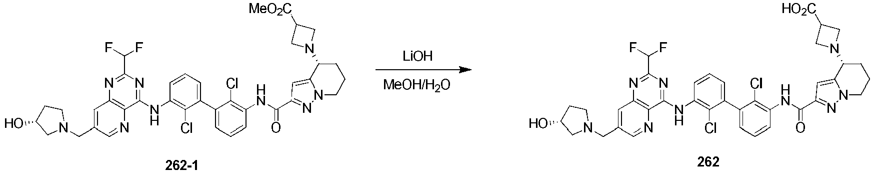

To a mixture of Methyl 1-[(4R)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]azetidine-3-carboxylate (233 mg, 293.95 µmol, 1 eq) in H₂O (2 mL) and MeOH (10 mL), LiOH·H₂O (37.01 mg, 881.84 µmol, 3 eq) was added, and the reaction mixture was stirred at room temperature for 4 hours. The reaction mixture was concentrated under reduced pressure, and the resulting residue was purified by reversed-phase HPLC (column: Waters Xbridge, 150 * 25 mm * 5 µm; mobile phase: [water (NH₄HCO₃)-ACN]; gradient: 20% to 50% B over 9 min) to afford Compound 262 (76 mg, 31.5% yield) as a yellow solid.

LCMS m/z 778.3 [M+1]⁺.

¹H NMR (400 MHz, DMSO-*d*₆) δ ppm 1.15 - 1.25 (m, 1 H) 1.55 - 1.62 (m, 1 H) 1.71 - 1.79 (m, 2 H) 1.82 - 1.89 (m, 1 H) 1.98 - 2.08 (m, 1 H) 2.15 - 2.25 (m, 1 H) 2.42 (dd, *J*=9.69, 3.44 Hz, 1 H) 2.63 - 2.78 (m, 3 H) 3.15 (br d, *J*=7.38 Hz, 2 H) 3.22 (br s, 2 H) 3.84 - 3.94 (m, 2 H) 4.05 - 4.12 (m, 1 H) 4.22 (br dd, *J*=7.69, 4.69 Hz, 2 H) 4.70 - 4.83 (m, 1 H) 6.74 - 6.77 (m, 1 H) 6.86 - 7.03 (m, 1 H) 7.20 (dd, *J*=7.63, 1.25 Hz, 1 H) 7.25 (d, *J*=7.63 Hz, 1 H) 7.51 (t, J*=*7.94 Hz, 1 H) 7.60 (t, *J*=7.94 Hz, 1 H) 8.23 - 8.30 (m, 2 H) 8.58 (d, *J*=8.00 Hz, 1 H) 9.01 (s, 1 H) 9.56 (s, 1 H) 10.27 (br d, *J*=8.00 Hz, 1 H).

### [Preparation Example 66]

### Preparation of Compound 262, 1-((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid

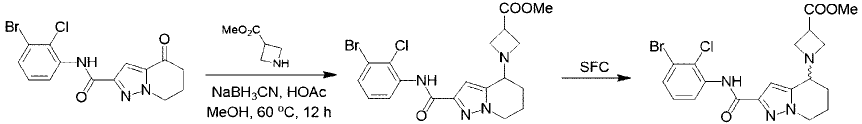

To a solution of N-(3-bromo-2-chloro-phenyl)-4-oxo-6,7-dihydro-5H-pyrazolo[1,5-a]pyridine-2-carboxamide (4 g, 10.85 mmol, 1 eq) and methyl azetidine-3-carboxylate hydrochloride (1.97 g, 13.02 mmol, 1.2 eq) in MeOH (80 mL), AcOH (3.26 g, 54.26 mmol, 3.11 mL, 5 eq) and NaBH₃CN (3.41 g, 54.26 mmol, 5 eq) were added. The reaction mixture was stirred at 60 °C for 12 hours. After cooling to room temperature, the reaction mixture was concentrated under reduced pressure. The resulting residue was purified by column chromatography (SiO₂, Petroleum ether/Ethyl acetate = 1/1 to 0/1) and further separated by SFC (EW41301-417-P1A1) (column: DAICEL CHIRALCEL OJ (250 mm * 30 mm * 10 µm); mobile phase: [CO₂-ACN/i-PrOH (0.1% NH₃·H₂O)]; B%: 40%, isocratic elution mode) to afford the enantiomer methyl 1-[(4S)-2-[(3-bromo-2-chloro-phenyl)carbamoyl]-4,5,6,7-tetrahydropyrazolo[1,5-a]pyridin-4-yl]azetidine-3-carboxylate (978 mg, 17.3% yield) and the enantiomer methyl 1-[(4R)-2-[(3-bromo-2-chloro-phenyl)carbamoyl]-4,5,6,7-tetrahydropyrazolo[1,5-a]pyridin-4-yl]azetidine-3-carboxylate (784 mg, 15.1% yield) each as a white solid.

LCMS m/z 466.9 [M+1]⁺.

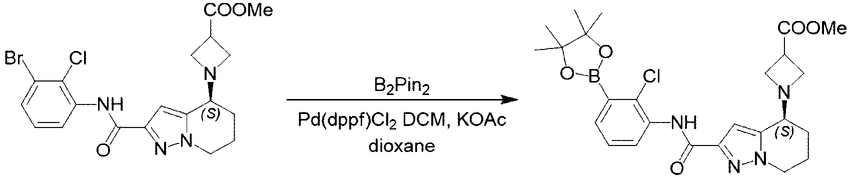

Methyl 1-[(4S)-2-[(3-bromo-2-chloro-phenyl)carbamoyl]-4,5,6,7-tetrahydropyrazolo[1,5-a]pyridin-4-yl]azetidine-3-carboxylate (330 mg, 705.52 µmol, 1 eq), 4,4,5,5-tetramethyl-2-(4,4,5,5-tetramethyl-1,3,2-dioxaborolan-2-yl)-1,3,2-dioxaborolane (537.47 mg, 2.12 mmol, 3 eq), Pd(dppf)Cl₂·CH₂Cl₂ (57.62 mg, 70.55 µmol, 0.1 eq), and KOAc (207.72 mg, 2.12 mmol, 3 eq) were added to dioxane (6 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 1/0 to 1/2) to afford methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (425 mg, 93.6% yield) as a yellow solid.

LCMS m/z 515.5 [M+1]⁺.

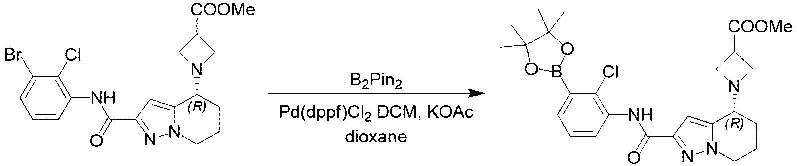

Methyl 1-[(4R)-2-[(3-bromo-2-chloro-phenyl)carbamoyl]-4,5,6,7-tetrahydropyrazolo[1,5-a]pyridin-4-yl]azetidine-3-carboxylate (600 mg, 1.28 mmol, 1 eq), 4,4,5,5-tetramethyl-2-(4,4,5,5-tetramethyl-1,3,2-dioxaborolan-2-yl)-1,3,2-dioxaborolane (977.22 mg, 3.85 mmol, 3 eq), Pd(dppf)Cl₂·CH₂Cl₂ (104.75 mg, 128.28 µmol, 0.1 eq), and KOAc (377.67 mg, 3.85 mmol, 3 eq) were added to dioxane (10 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by column chromatography (SiO₂, Petroleum ether/Ethyl acetate = 1/0 to 1/2) to afford methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (748 mg, 90.6% yield) as a yellow solid.

LCMS m/z 515.2 [M+1]⁺.

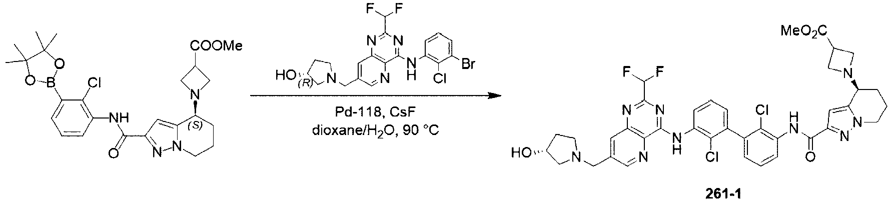

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (138.07 mg, 268.19 µmol, 1.3 eq), CsF (94.01 mg, 618.91 µmol, 3 eq), and ditert-butyl(cyclopentyl)phosphane;dichloropalladium;iron (13.45 mg, 20.63 µmol, 0.1 eq) were added to a mixture of dioxane (8 mL) and H₂O (2 mL), and the reaction mixture was stirred at 90 °C for 8 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 10:1) to afford Compound 261-1 (254 mg, 94.7% yield) as a yellow solid.

LCMS m/z 792.3 [M+1]⁺.

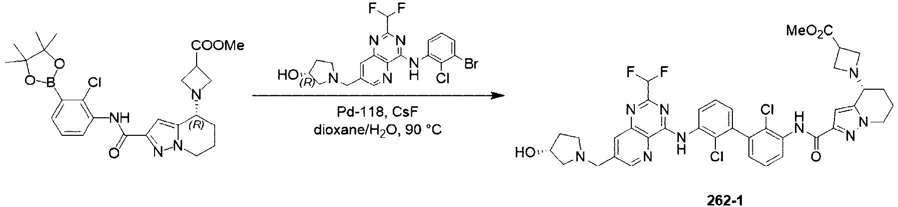

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate R-4 (138.07 mg, 268.19 µmol, 1.3 eq), CsF (94.01 mg, 618.91 µmol, 3 eq), and ditert-butyl(cyclopentyl)phosphane;dichloropalladium;iron (13.45 mg, 20.63 µmol, 0.1 eq) were added to a mixture of dioxane (8 mL) and H₂O (2 mL), and the reaction mixture was stirred at 90 °C for 8 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 10:1) to afford Compound 261-1 (233 mg, 91.1% yield) as a yellow solid.

LCMS /z 792.3 [M+1]⁺.

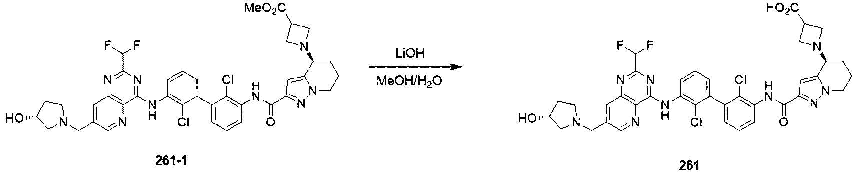

To a mixture of methyl 1-[(4S)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]azetidine-3-carboxylate 261-1 (254 mg, 320.44 µmol, 1 eq) in H₂O (2 mL) and MeOH (10 mL), LiOH·H₂O (40.34 mg, 961.32 µmol, 3 eq) were added, and the reaction mixture was stirred at room temperature for 4 hours. The reaction mixture was concentrated under reduced pressure, and the resulting residue was purified by reversed-phase HPLC (column: Waters Xbridge, 150 * 25 mm * 5 µm; mobile phase: [water (NH₄HCO₃)-ACN]; gradient: 20% to 50% B over 9 min) to afford Compound 261 (76 mg, 28.9% yield) as a yellow solid.

LCMS m/z 778.3 [M+1]⁺.

¹H NMR (400 MHz, DMSO-d₆) δ ppm 1.10 - 1.25 (m, 1 H) 1.52 - 1.62 (m, 1 H) 1.68 - 1.78 (m, 2 H) 1.79 - 1.89 (m, 1 H) 1.98 - 2.06 (m, 1 H) 2.12 - 2.24 (m, 1 H) 2.38 - 2.43 (m, 1 H) 2.64 - 2.78 (m, 3 H) 3.11 - 3.16 (m, 2 H) 3.21 (br s, 2 H) 3.83 - 3.93 (m, 2 H) 4.04 - 4.12 (m, 1 H) 4.22 (br dd, J=7.07, 4.69 Hz, 2 H) 4.70 - 4.81 (m, 1 H) 6.73 - 6.76 (m, 1 H) 6.85 - 7.01 (m, 1 H) 7.19 (dd, J=7.57, 1.19 Hz, 1 H) 7.24 (d, J=7.63 Hz, 1 H) 7.50 (t, J=7.94 Hz, 1 H) 7.59 (t, J=7.94 Hz, 1 H) 8.24 - 8.29 (m, 2 H) 8.57 (d, J=8.13 Hz, 1 H) 9.00 (d, J=1.50 Hz, 1 H) 9.56 (s, 1 H) 10.20 - 10.34 (m, 1 H).

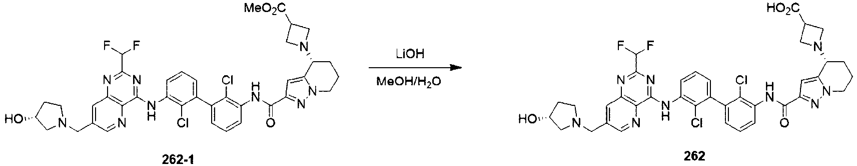

To a mixture of methyl 1-[(4R)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]azetidine-3-carboxylate (233 mg, 293.95 µmol, 1 eq) in H₂O (2 mL)/MeOH (10 mL), LiOH·H₂O (37.01 mg, 881.84 µmol, 3 eq) were added, and the reaction mixture was stirred at room temperature for 4 hours. The reaction mixture was concentrated under reduced pressure, and the resulting residue was purified by reversed-phase HPLC (column: Waters Xbridge, 150 * 25 mm * 5 µm; mobile phase: [water (NH₄HCO₃)-ACN]; gradient: 20% to 50% B over 9 min) to afford Compound 262 (76 mg, 31.5% yield) as a yellow solid.

LCMS m/z 778.3 [M+1]⁺.

¹H NMR (400 MHz, DMSO-d₆) δ ppm 1.15 - 1.25 (m, 1 H) 1.55 - 1.62 (m, 1 H) 1.71 - 1.79 (m, 2 H) 1.82 - 1.89 (m, 1 H) 1.98 - 2.08 (m, 1 H) 2.15 - 2.25 (m, 1 H) 2.42 (dd, J=9.69, 3.44 Hz, 1 H) 2.63 - 2.78 (m, 3 H) 3.15 (br d, J=7.38 Hz, 2 H) 3.22 (br s, 2 H) 3.84 - 3.94 (m, 2 H) 4.05 - 4.12 (m, 1 H) 4.22 (br dd, J=7.69, 4.69 Hz, 2 H) 4.70 - 4.83 (m, 1 H) 6.74 - 6.77 (m, 1 H) 6.86 - 7.03 (m, 1 H) 7.20 (dd, J=7.63, 1.25 Hz, 1 H) 7.25 (d, J=7.63 Hz, 1 H) 7.51 (t, J=7.94 Hz, 1 H) 7.60 (t, J=7.94 Hz, 1 H) 8.23 - 8.30 (m, 2 H) 8.58 (d, J=8.00 Hz, 1 H) 9.01 (s, 1 H) 9.56 (s, 1 H) 10.27 (br d, J=8.00 Hz, 1 H).

### [Preparation Example 67]

### Preparation of Compound 263, (R)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)azetidine-3-carboxylic acid

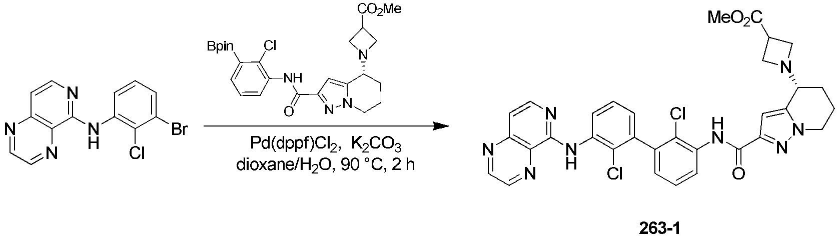

N-(3-bromo-2-chloro-phenyl)pyrido[3,4-b]pyrazin-5-amine 3 (120 mg, 357.58 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (289.28 mg, 393.34 µmol, 1.1 eq), K₂CO₃ (148.26 mg, 1.07 mmol, 3 eq), and ditert-butyl(cyclopentyl)phosphane;dichloropalladium;iron (23.31 mg, 35.76 µmol, 0.1 eq) were added to a mixture of dioxane (2 mL) and H₂O (0.5 mL), and the reaction mixture was stirred at 90 °C for 5 hours under a nitrogen atmosphere. After cooling to room temperature, the reaction mixture was concentrated under reduced pressure, and the resulting residue was purified by column chromatography (SiO₂, petroleum ether:dichloromethane = 10/1) to afford Compound 263-1 (100 mg, 34.7% yield) as a black solid.

LCMS m/z 643.1 [M+1] ⁺.

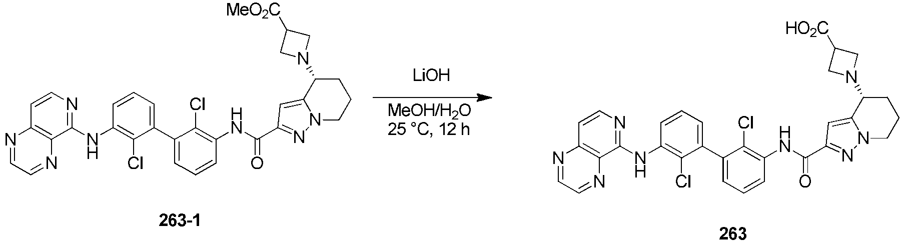

Methyl 1-[(4R)-2-[[2-chloro-3-[2-chloro-3-(pyrido[3,4-b]pyrazin-5-ylamino)phenyl]phenyl]carbamoyl]-4,5,6,7-tetrahydropyrazolo[1,5-a]pyridin-4-yl]azetidine-3-carboxylate 263-1 (100.00 mg, 155.39 µmol, 1 eq) and LiOH (19.56 mg, 466.18 µmol, 3 eq) were added to a mixture of THF (5 mL) and H₂O (1 mL), and the reaction mixture was stirred at room temperature for 2 hours. The reaction mixture was concentrated under reduced pressure, and the resulting residue was purified by reversed-phase HPLC (column: Waters XBridge, 150 * 25 mm * 5 µm; mobile phase: [water (NH₄HCO₃)-ACN]; gradient: 24% to 54% B over 9 min) to afford Compound 263 (15 mg, 9.20% yield) as a yellow solid.

LCMS m/z 629.1 [M+1] ⁺.

¹H NMR (400 MHz, DMSO-*d*6) δ ppm 1.74 (br s, 1 H) 1.80 - 1.92 (m, 1 H) 2.18 (br s, 1 H) 3.04 - 3.11 (m, 1 H) 3.19 (br t, *J*=6.60 Hz, 2 H) 3.43 - 3.48 (m, 2 H) 3.51 (br t, *J*=4.34 Hz, 1 H) 4.04 - 4.12 (m, 1 H) 4.17 - 4.25 (m, 1 H) 6.75 (s, 1 H) 7.09 (d, *J*=7.58 Hz, 1 H) 7.16 - 7.22 (m, 1 H) 7.44 (d, *J*=5.87 Hz, 1 H) 7.47 - 7.60 (m, 2 H) 8.28 (dd, *J*=7.27, 3.24 Hz, 1 H) 8.42 (d, *J*=6.11 Hz, 1 H) 8.98 - 9.06 (m, 2 H) 9.20 (d, *J*=1.83 Hz, 1 H) 9.56 (s, 1 H) 9.74 (s, 1 H).

### [Preparation Example 68]

### Preparation of Compound 264, (S)-1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)azetidine-3-carboxylic acid

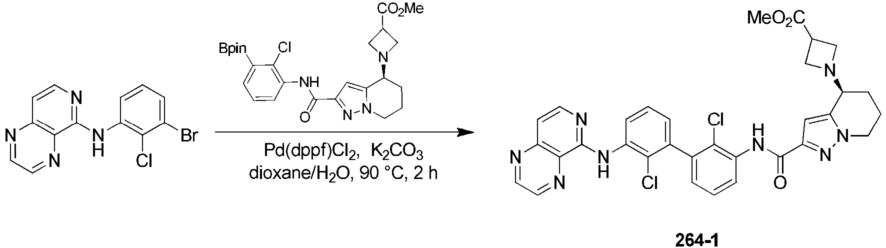

N-(3-bromo-2-chlorophenyl)pyrido[3,4-b]pyrazin-5-amine (120 mg, 357.58 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (184.09 mg, 357.58 µmol, 1 eq), K₂CO₃ (148.26 mg, 1.07 mmol, 3 eq), and ditert-butyl(cyclopentyl)phosphane;dichloropalladium;iron (23.31 mg, 35.76 µmol, 0.1 eq) were added to a mixture of dioxane (2 mL) and H₂O (0.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by column chromatography (SiO₂, petroleum ether:dichloromethane = 10/1) to afford Compound 264-1 (200 mg, 43.4% yield) as a black solid.

LCMS m/z 643.1 [M+1] ⁺.

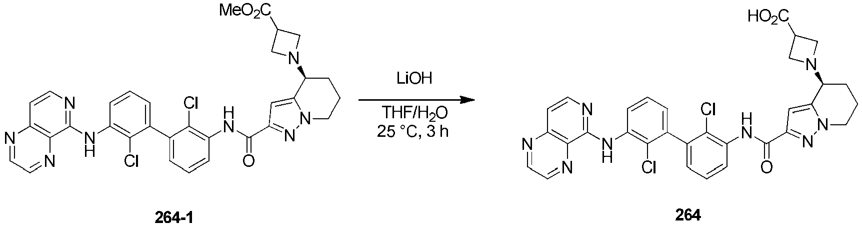

(S)-Methyl 1-(2-((2,2'-dichloro-3'-(pyrido[3,4-b]pyrazin-5-ylamino)-[1,1'-biphenyl]-3-yl)carbamoyl)-4,5,6,7-tetrahydropyrazolo[1,5-a]pyridin-4-yl)azetidine-3-carboxylate 264-1 (100 mg, 155.39 µmol, 1 eq) and LiOH (13.04 mg, 310.79 µmol, 2 eq) were added to a mixture of H₂O (1 mL) and THF (5 mL), and the reaction mixture was stirred at room temperature for 3 hours. The reaction mixture was concentrated under reduced pressure, and the resulting residue was purified by reversed-phase HPLC (column: Waters XBridge, 150 * 25 mm * 5 µm; mobile phase: [water (NH₄HCO₃)-ACN]; gradient: 24% to 54% B over 9 min) to afford Compound 264 (22 mg, 20.2% yield) as a yellow solid.

LCMS m/z 629.1 [M+1] ⁺.

¹H NMR (400 MHz, DMSO-*d*6) δ ppm 1.69 - 1.79 (m, 2 H) 1.80 - 1.90 (m, 1 H) 2.08 - 2.27 (m, 1 H) 3.09 (br d, *J*=7.09 Hz, 1 H) 3.19 (s, 2 H) 3.45 (br d, *J*=7.21 Hz, 2 H) 3.51 (br s, 1 H) 4.02 - 4.14 (m, 1 H) 4.16 - 4.28 (m, 1 H) 6.75 (s, 1 H) 7.09 (d, *J*=7.34 Hz, 1 H) 7.20 (d, *J*=6.72 Hz, 1 H) 7.44 (d, *J*=5.99 Hz, 1 H) 7.52 (dt, *J*=17.57, 8.02 Hz, 2 H) 8.24 - 8.30 (m, 1 H) 8.42 (d, *J*=5.99 Hz, 1 H) 8.98 - 9.05 (m, 2 H) 9.20 (d, *J*=1.71 Hz, 1 H) 9.56 (s, 1 H) 9.74 (s, 1 H).

### [Preparation Example 69]

### Preparation of Compound 265, 1-((S)-2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)azetidine-3-carboxylic acid

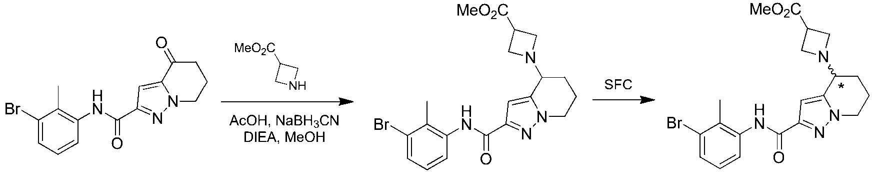

To a solution of N-(3-bromo-2-methyl-phenyl)-4-oxo-6,7-dihydro-5H-pyrazolo[1,5-a]pyridine-2-carboxamide (2 g, 5.74 mmol, 1 eq) and methyl azetidine-3-carboxylate hydrochloride (3.24 g, 17.23 mmol, 3 equivalents, HCl) in MeOH (40 mL), AcOH (689.87 mg, 11.49 mmol, 657.64 µL, 2 eq), DIPEA (2.23 g, 17.23 mmol, 3.00 mL, 3 eq), and NaBH₃CN (1.80 g, 28.72 mmol, 5 eq) were added. The reaction mixture was stirred at 60 °C for 12 hours. After cooling to room temperature, the reaction mixture was concentrated under reduced pressure and purified by prep-HPLC (column: UniSil 10-120 C18, 70 * 250 mm; mobile phase: [water (NH₄HCO₃)-ACN]; gradient: 45% to 75% B over 20 min), followed by separation by SFC (column: DAICEL CHIRALPAK AS, 250 mm * 30 mm * 10 µm; mobile phase: CO₂/i-PrOH; B%: 60%, isocratic elution mode) to afford the enantiomer methyl 1-[(4S)-2-[(3-bromo-2-methyl-phenyl)carbamoyl]-4,5,6,7-tetrahydropyrazolo[1,5-a]pyridin-4-yl]azetidine-3-carboxylate (520 mg, 45.8% yield) and the enantiomer methyl 1-[(4R)-2-[(3-bromo-2-methylphenyl)carbamoyl]-4,5,6,7-tetrahydropyrazolo[1,5-a]pyridin-4-yl]azetidine-3-carboxylate (600 mg, 53.4% yield), each obtained as a white solid.

LCMS m/z 449.0 [M+1]⁺.

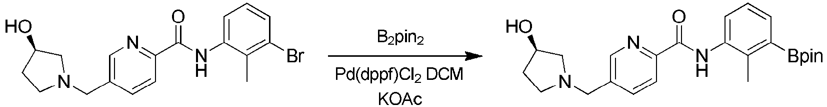

N-(3-bromo-2-methyl-phenyl)-5-[[(3R)-3-hydroxypyrrolidin-1-yl]methyl]pyridine-2-carboxamide (900 mg, 2.31 mmol, 1 eq), 4,4,5,5-tetramethyl-2-(4,4,5,5-tetramethyl-1,3,2-dioxaborolan-2-yl)-1,3,2-dioxaborolane (1.76 g, 6.92 mmol, 3 eq), AcOK (452.65 mg, 4.61 mmol, 2 eq), and Pd(dppf)Cl₂·CH₂Cl₂ (94.16 mg, 115.30 µmol, 0.05 eq) were added to dioxane (20 mL), and the reaction mixture was stirred at 100 °C for 12 hours under a nitrogen atmosphere. After cooling to room temperature, the reaction mixture was filtered, and the filtrate was concentrated under reduced pressure. The resulting residue was purified by column chromatography (SiO₂, Ethyl acetate:Methanol = 3/1) to afford 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (900 mg, 66.9% yield) as a black solid.

LCMS m/z 438.2 [M+1]⁺.

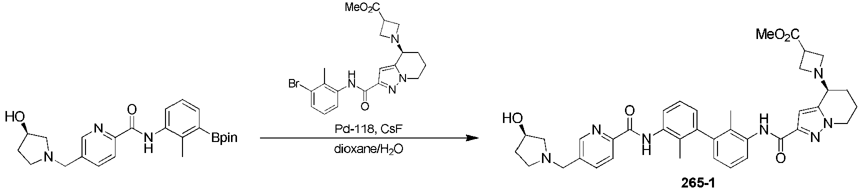

Methyl 1-[(4S)-2-[(3-bromo-2-methyl-phenyl)carbamoyl]-4,5,6,7-tetrahydropyrazolo[1,5-a]pyridin-4-yl]azetidine-3-carboxylate (200 mg, 447.10 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (293.30 mg, 670.65 µmol, 1.5 eq), CsF (135.83 mg, 894.21 µmol, 2 eq), and ditert-butyl(cyclopentyl)phosphane;dichloropalladium;iron (29.14 mg, 44.71 µmol, 0.1 eq) were added to a mixture of dioxane (5 mL) and H₂O (1 mL), and the reaction mixture was stirred at 80 °C for 12 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 5:1) to afford methyl 1-[(4S)-2-[[3-[3-[[5-[[(3R)-3-hydroxypyrrolidin-1-yl]methyl]pyridine-2-carbonyl]amino]-2-methyl-phenyl]-2-methylphenyl]carbamoyl]-4,5,6,7-tetrahydropyrazolo[1,5-a]pyridin-4-yl]azetidine-3-carboxylate 265-1 (270 mg, 60.5% yield) as a black solid.

LCMS m/z 678.4 [M+1]⁺.

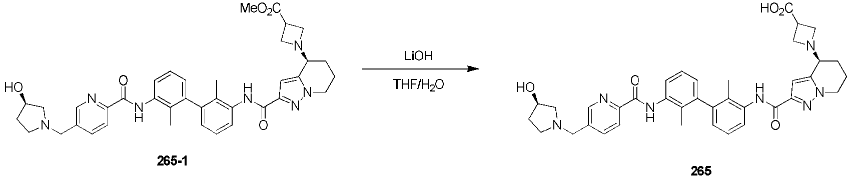

To a mixture of methyl 1-[(4S)-2-[[3-[3-[[5-[[(3R)-3-hydroxypyrrolidin-1-yl]methyl]pyridine-2-carbonyl]amino]-2-methyl-phenyl]-2-methyl-phenyl]carbamoyl]-4,5,6,7-tetrahydropyrazolo[1,5-a]pyridin-4-yl]azetidine-3-carboxylate (160 mg, 236.06 µmol, 1 eq) in THF (20 mL) and H₂O (10 mL), LiOH·H₂O (29.72 mg, 708.18 µmol, 3 eq) was added, and the reaction mixture was stirred at room temperature for 12 hours. The reaction mixture was concentrated under reduced pressure, and the resulting residue was purified by reversed-phase HPLC (0.1% NH₃·H₂O condition; column: Waters XBridge, 150 * 25 mm * 5 µm; mobile phase: [water (NH₄HCO₃)-ACN]; gradient: 12% to 42% B over 9 min) to afford Compound 265 (68 mg, 41.2% yield) as a white solid.

LCMS m/z 678.4 [M+1]⁺.

¹H NMR (400 MHz, DMSO-*d*6) δ ppm 1.52 - 1.62 (m, 1 H) 1.70 - 1.80 (m, 2 H) 1.86 (br d, *J*=5.63 Hz, 1 H) 1.95 (s, 3 H) 2.02 (s, 3 H) 2.16 - 2.24 (m, 1 H) 2.35 (dd, *J*=9.63, 3.50 Hz, 1 H) 2.42 - 2.48 (m, 1 H) 2.59 - 2.66 (m, 1 H) 2.67 - 2.74 (m, 1 H) 3.12 - 3.18 (m, 1 H) 3.20 - 3.24 (m, 1 H) 3.38 (br d, *J*=7.13 Hz, 2 H) 3.48 (br d, *J*=7.13 Hz, 1 H) 3.51 - 3.54 (m, 1 H) 3.67 - 3.77 (m, 2 H) 4.03 - 4.13 (m, 1 H) 4.21 (br dd, *J*=7.57, 5.07 Hz, 2 H) 4.72 (br s, 1 H) 6.69 (s, 1 H) 6.98 (d, *J*=7.50 Hz, 2 H) 7.25 - 7.35 (m, 2 H) 7.57 (br d, *J*=7.88 Hz, 1 H) 7.88 (d, *J*=8.00 Hz, 1 H) 7.99 (dd, *J*=8.00, 1.88 Hz, 1 H) 8.15 (d, *J*=8.00 Hz, 1 H) 8.65 (s, 1 H) 9.53 (s, 1 H) 10.33 (s, 1 H)

### [Preparation Example 70]

### Preparation of Compound 266, 1-((R)-2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)azetidine-3-carboxylic acid

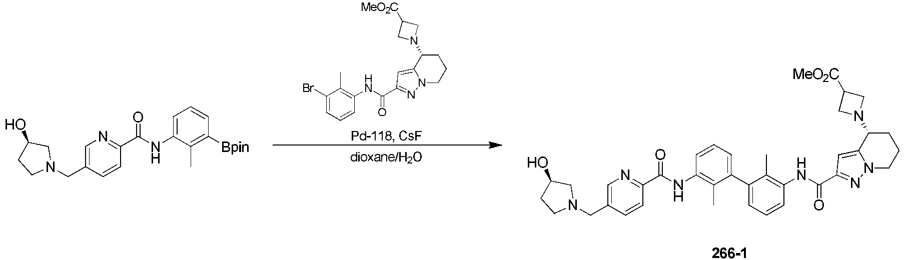

Methyl 1-[(4R)-2-[(3-bromo-2-methyl-phenyl)carbamoyl]-4,5,6,7-tetrahydropyrazolo[1,5-a]pyridin-4-yl]azetidine-3-carboxylate (200 mg, 447.10 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (293.30 mg, 670.65 µmol, 1.5 eq), CsF (135.83 mg, 894.21 µmol, 2 eq), and ditert-butyl(cyclopentyl)phosphane;dichloropalladium;iron (29.14 mg, 44.71 µmol, 0.1 eq) were added to a mixture of dioxane (5 mL) and H₂O (1 mL), and the reaction mixture was stirred at 80 °C for 12 hours under a nitrogen atmosphere. After cooling to room temperature, the reaction mixture was filtered, and the filtrate was concentrated under reduced pressure. The resulting residue was purified by prep-TLC (SiO₂, ethyl acetate/methanol = 5/1) to afford Compound 266-1 (215 mg, 55.3% yield) as a black solid.

LCMS m/z 678.4 [M+1]⁺.

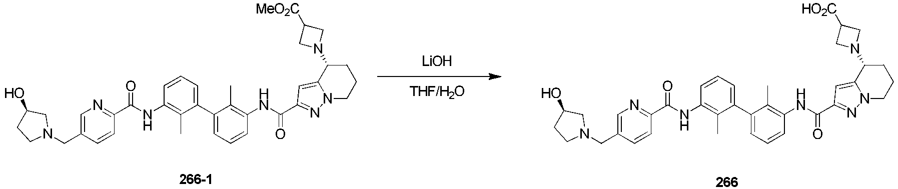

To a solution of methyl 1-[(4R)-2-[[3-[3-[[5-[[(3R)-3-hydroxypyrrolidin-1-yl]methyl]pyridine-2-carbonyl]amino]-2-methyl-phenyl]-2-methyl-phenyl]carbamoyl]-4,5,6,7-tetrahydropyrazolo[1,5-a]pyridin-4-yl]azetidine-3-carboxylate 266-1 (160 mg, 236.06 µmol, 1 eq) in THF (20 mL)/H₂O (10 mL), LiOH·H₂O (29.72 mg, 708.18 µmol, 3 eq) was added, and the reaction mixture is stirred at room temperature for 12 hours. After completion, the reaction mixture was concentrated under reduced pressure, and the resulting residue was purified by reversed-phase HPLC((0.1% NH₃•H₂O condition) column: Waters Xbridge 150 * 25 mm * 5 um; mobile phase: [water (NH₄HCO₃)-ACN]; gradient: 12%-42% B over 9 min) to afford Compound 266 (94 mg, 56.9% yield) as a white solid.

LCMS m/z 678.4 [M+1]⁺.

¹H NMR (400 MHz, DMSO-*d*6) δ ppm 1.51 - 1.62 (m, 1 H) 1.71 - 1.80 (m, 2 H) 1.85 (br d, *J*=4.88 Hz, 1 H) 1.94 (s, 3 H) 2.01 (s, 3 H) 2.19 (dt, *J*=9.35, 4.64 Hz, 1 H) 2.34 (dd, *J*=9.76, 3.63 Hz, 1 H) 2.41 - 2.47 (m, 1 H) 2.62 (q, *J*=7.80 Hz, 1 H) 2.67 - 2.72 (m, 1 H) 3.11 - 3.17 (m, 1 H) 3.21 (br t, *J*=6.69 Hz, 1 H) 3.35 - 3.38 (m, 2 H) 3.47 (br d, *J*=7.25 Hz, 1 H) 3.49 - 3.53 (m, 1 H) 3.66 - 3.76 (m, 2 H) 4.02 - 4.12 (m, 1 H) 4.17 - 4.24 (m, 2 H) 4.72 (br s, 1 H) 6.68 (s, 1 H) 6.98 (d, *J*=7.63 Hz, 2 H) 7.25 - 7.34 (m, 2 H) 7.57 (br d, *J*=8.00 Hz, 1 H) 7.87 (d, *J*=7.88 Hz, 1 H) 7.98 (dd, *J*=8.00, 1.88 Hz, 1 H) 8.14 (d, *J*=8.00 Hz, 1 H) 8.63 - 8.66 (m, 1 H) 9.53 (s, 1 H) 10.32 (s, 1 H)

### [Preparation Example 71]

### Preparation of Compound 267, 1-((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

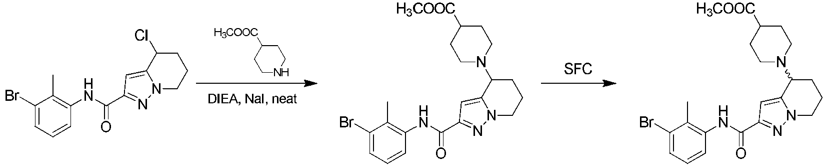

To a solution of N-(3-bromo-2-methylphenyl)-4-chloro-4,5,6,7-tetrahydropyrazolo[1,5-a]pyridine-2-carboxamide (1.5 g, 4.07 mmol, 1 eq) and methyl piperidine-4-carboxylate (5.83 g, 40.69 mmol, 10 eq) in methyl piperidine-4-carboxylate (10 mL), DIPEA (1.58 g, 12.21 mmol, 2.13 mL, 3 eq) and NaI (182.97 mg, 1.22 mmol, 0.3 eq) were added. The reaction mixture was stirred at 40 °C for 12 hours. After cooling to room temperature, the reaction mixture was concentrated under reduced pressure. The resulting residue was purified by prep-HPLC (column: Phenomenex Luna C18, 250 × 70 mm, 10 µm; mobile phase: [water (HCl)-ACN]; gradient: 0% to 30% B over 30 min) and further separated by SFC (column: DAICEL CHIRALPAK AS, 250 mm × 30 mm, 10 µm; mobile phase: CO₂/i-PrOH (0.1% NH₃·H₂O); B%: 50%, isocratic elution mode) to afford the enantiomer (S)-methyl 1-(2-((3-bromo-2-methylphenyl)carbamoyl)-4,5,6,7-tetrahydropyrazolo[1,5-a]pyridin-4-yl)piperidine-4-carboxylate (777 mg, 39.2% yield) and the enantiomer (R)-methyl 1-(2-((3-bromo-2-methylphenyl)carbamoyl)-4,5,6,7-tetrahydropyrazolo[1,5-a]pyridin-4-yl)piperidine-4-carboxylate (740 mg, 39.8% yield), each obtained as a white solid.

LCMS m/z 475.1 [M+1]⁺.

¹H NMR (400 MHz, DMSO-d6) δ ppm 2.27 (s, 5 H) 2.52 - 2.55 (m, 1 H) 2.65 - 2.74 (m, 1 H) 3.16 (s, 3 H) 3.64 (s, 3 H) 4.16 - 4.29 (m, 2 H) 4.88 (br s, 1 H) 7.16 (t, J=7.94 Hz, 1 H) 7.38 (d, J=7.88 Hz, 2 H) 7.50 (d, J=8.00 Hz, 1 H) 9.97 (s, 1 H)

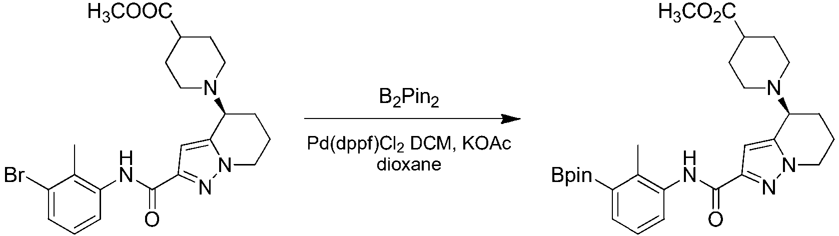

(S)-Methyl 1-(2-((3-bromo-2-methylphenyl)carbamoyl)-4,5,6,7-tetrahydropyrazolo[1,5-a]pyridin-4-yl)piperidine-4-carboxylate (200 mg, 420.72 µmol, 1 eq), 4,4,5,5-tetramethyl-2-(4,4,5,5-tetramethyl-1,3,2-dioxaborolan-2-yl)-1,3,2-dioxaborolane (213.67 mg, 841.44 µmol, 2 eq), AcOK (82.58 mg, 841.44 µmol, 2 eq), and Pd(dppf)Cl₂·CH₂Cl₂ (34.36 mg, 42.07 µmol, 0.1 eq) were added to dioxane (5 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 10:1) to afford methyl 1-[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (213 mg, 86.2% yield) as a black solid.

LCMS m/z 523.2 [M+1]⁺.

### [Preparation Example 72]

### Preparation of Compound 268, 1-((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

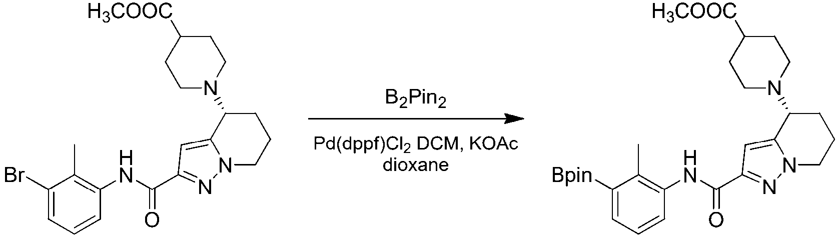

Methyl 1-[2-[(3-bromo-2-methyl-phenyl)carbamoyl]-4,5,6,7-tetrahydropyrazolo[1,5-a]pyridin-4-yl]piperidine-4-carboxylate (200 mg, 420.72 µmol, 1 eq), 4,4,5,5-tetramethyl-2-(4,4,5,5-tetramethyl-1,3,2-dioxaborolan-2-yl)-1,3,2-dioxaborolane (213.67 mg, 841.44 µmol, 2 eq), AcOK (82.58 mg, 841.44 µmol, 2 eq), and Pd(dppf)Cl₂·CH₂Cl₂ (34.36 mg, 42.07 µmol, 0.1 eq) were added to dioxane (5 mL), and the reaction mixture was stirred at 90 °C for 4 hours under a nitrogen atmosphere. After cooling to room temperature, the reaction mixture was filtered, and the filtrate was concentrated under reduced pressure. The resulting residue was purified by prep-TLC (SiO₂, Petroleum ether/Ethyl acetate = 0/1) to afford methyl 1-[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (237 mg, 95.9% yield) as a black solid.

LCMS m/z 523.3 [M+1]⁺.

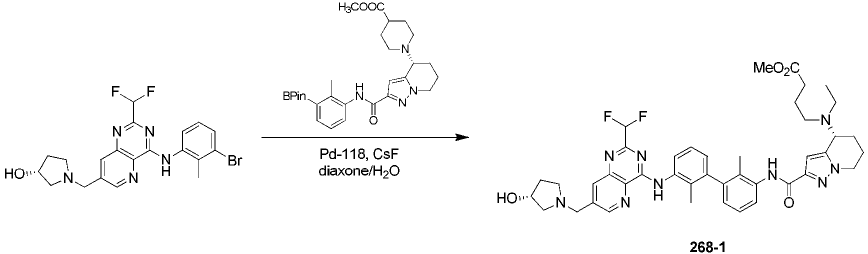

(3R)-1-[[4-(3-bromo-2-methyl-anilino)-2-(difluoromethyl)pyrido[3,2-d]pyrimidin-7-yl]methyl]pyrrolidin-3-ol (110 mg, 236.91 µmol, 1 eq), methyl 1-[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (148.53 mg, 284.30 µmol, 1.2 eq), CsF (71.97 mg, 473.83 µmol, 2 eq), and ditert-butyl(cyclopentyl)phosphane;dichloropalladium;iron (15.44 mg, 23.69 µmol, 0.1 eq) were added to a mixture of dioxane (5 mL) and H₂O (1 mL), and the reaction mixture was stirred at 80 °C for 8 hours under a nitrogen atmosphere. After cooling to room temperature, the reaction mixture was filtered, and the filtrate was concentrated under reduced pressure. The resulting residue was purified by prep-TLC (SiO₂, ethyl acetate/methanol = 15/1) to afford Compound 268-1 (78 mg, 29.5% yield) as a yellow solid.

LCMS m/z 780.4 [M+1]⁺.

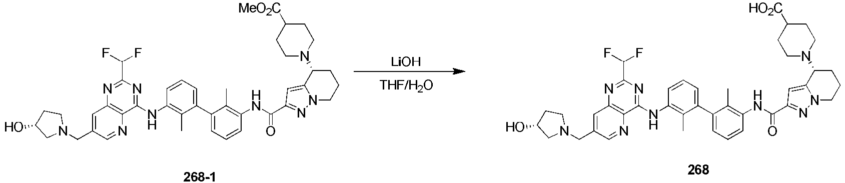

To a solution of methyl 1-[(4R)-2-[[3-[3-[[2-(difluoromethyl)-7-[[(3R)-3-hydroxypyrrolidin-1-yl]methyl]pyrido[3,2-d]pyrimidin-4-yl]amino]-2-methyl-phenyl]-2-methyl-phenyl]carbamoyl]-4,5,6,7-tetrahydropyrazolo[1,5-a]pyridin-4-yl]piperidine-4-carboxylate 268-1 (78 mg, 100.02 µmol, 1 eq) in THF (30 mL) and H₂O (10 mL), LiOH·H₂O (12.59 mg, 300.05 µmol, 3 eq) was added, and the reaction mixture was stirred at room temperature for 12 hours. The reaction mixture was concentrated under reduced pressure, and the resulting residue was purified by reversed-phase HPLC (0.1% NH₃·H₂O condition; column: Waters XBridge, 150 * 25 mm * 5 µm; mobile phase: [water (NH₄HCO₃)-ACN]; gradient: 18% to 48% B over 9 min) to afford Compound 268 (39 mg, 48.3% yield) as a yellow solid.

LCMS m/z 766.7 [M+1]⁺.

¹H NMR (400 MHz, DMSO-d6) δ ppm 1.14 - 1.24 (m, 1 H) 1.47 - 1.54 (m, 1 H) 1.56 - 1.62 (m, 2 H) 1.64 - 1.73 (m, 1 H) 1.81 (br t, *J*=11.76 Hz, 2 H) 1.95 (s, 3 H) 1.99 (s, 3 H) 2.01 - 2.08 (m, 1 H) 2.13 - 2.21 (m, 2 H) 2.24 - 2.33 (m, 1 H) 2.38 - 2.46 (m, 2 H) 2.65 - 2.72 (m, 2 H) 2.73 - 2.82 (m, 2 H) 3.81 - 3.89 (m, 2 H) 3.91 - 3.99 (m, 1 H) 4.00 - 4.10 (m, 1 H) 4.16 - 4.25 (m, 2 H) 4.73 (br d, *J*=3.50 Hz, 1 H) 6.70 (t, *J*=54.59 Hz, 1 H) 6.62 (s, 1 H) 7.01 (d, *J*=7.25 Hz, 1 H) 7.06 (d, *J*=7.50 Hz, 1 H) 7.28 (t, *J*=7.82 Hz, 1 H) 7.35 (t, *J*=7.69 Hz, 1 H) 7.57 (dd, *J*=7.69, 4.44 Hz, 1 H) 7.66 (d, *J*=7.88 Hz, 1 H) 8.16 (s, 1 H) 8.95 (d, *J*=1.63 Hz, 1 H) 9.51 (d, *J*=4.13 Hz, 1 H) 10.38 (s, 1 H)

### [Preparation Example 73]

### Preparation of Compound 269, (S)-N-(2,2'-dichloro-3'-((2-(difluoromethyl)-7-((3-fluoroazetidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

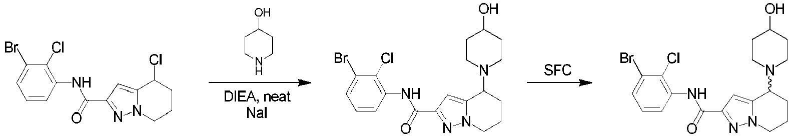

N-(3-bromo-2-chloro-phenyl)-4-chloro-4,5,6,7-tetrahydropyrazolo[1,5-a]pyridine-2-carboxamide (1 g, 2.57 mmol, 1 eq) and piperidin-4-ol (2.60 g, 25.70 mmol, 10 eq) were combined in piperidin-4-ol (10 mL), and DIPEA (996.54 mg, 7.71 mmol, 1.34 mL, 3 eq) and NaI (115.58 mg, 771.06 µmol, 0.3 eq) were added. The reaction mixture was stirred at 90 °C for 5 hours. After cooling to room temperature, the reaction mixture was filtered, and the filtrate was concentrated under reduced pressure. The resulting residue was purified by prep-HPLC (column: UniSil 10-120 C18, 70 × 250 mm; mobile phase: [water (NH₄HCO₃)-ACN]; gradient: 45% to 75% B over 20 min) and further separated by SFC (column: DAICEL CHIRALPAK AD, 250 mm * 30 mm * 10 µm; mobile phase: CO₂/EtOH (0.1% NH₃H₃O); B%: 40%, isocratic elution mode) to afford the enantiomer (4S)-N-(3-bromo-2-chloro-phenyl)-4-(4-hydroxy-1-piperidyl)-4,5,6,7-tetrahydropyrazolo[1,5-a]pyridine-2-carboxamide (230 mg, 43.7% yield) and the enantiomer (4R)-N-(3-bromo-2-chloro-phenyl)-4-(4-hydroxy-1-piperidyl)-4,5,6,7-tetrahydropyrazolo[1,5-a]pyridine-2-carboxamide (230 mg, 43.7% yield), each obtained as a yellow solid.

LCMS m/z 455.1 [M+1]⁺.

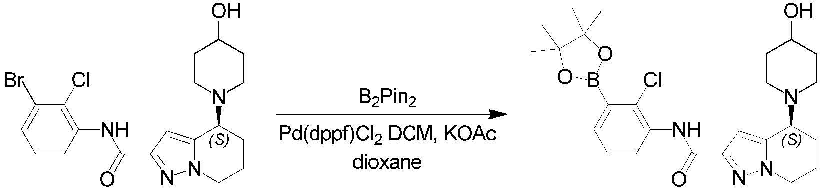

(4S)-N-(3-bromo-2-chloro-phenyl)-4-(4-hydroxy-1-piperidyl)-4,5,6,7-tetrahydropyrazolo[1,5-a]pyridine-2-carboxamide (150 mg, 330.57 µmol, 1 eq), 4,4,5,5-tetramethyl-2-(4,4,5,5-tetramethyl-1,3,2-dioxaborolan-2-yl)-1,3,2-dioxaborolane (251.83 mg, 991.71 µmol, 3 eq), AcOK (64.88 mg, 661.14 µmol, 2 eq), and Pd(dppf)Cl₂·CH₂Cl₂ (27.00 mg, 33.06 µmol, 0.1 eq) were added to dioxane (10 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by prep-TLC (SiO₂, Ethyl acetate: Methanol = 20:1) to afford (4S)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (120 mg, 62.3% yield) as a black solid.

LCMS m/z 501.3 [M+1]⁺.

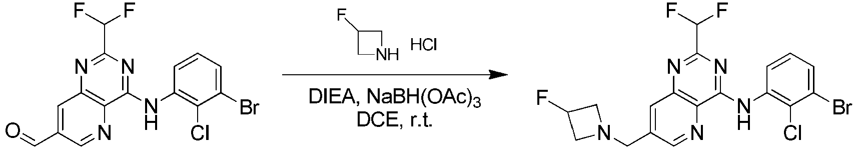

To a solution of 4-(3-bromo-2-chloro-anilino)-2-(difluoromethyl)pyrido[3,2-d]pyrimidine-7-carbaldehyde (1 g, 2.42 mmol, 1 eq) and 3-fluoroazetidine hydrochloride (1.35 g, 12.09 mmol, 5 eq) in DCE (10 mL), NaBH(OAc)₃ (1.54 g, 7.25 mmol, 3 eq) was added. The reaction mixture was stirred at room temperature for 12 hours. The reaction mixture was filtered, and the filtrate was concentrated under reduced pressure. The resulting residue was purified by column chromatography (SiO₂, Petroleum ether/Ethyl acetate = 2/1 to 0/1) to afford N-(3-bromo-2-chloro-phenyl)-2-(difluoromethyl)-7-[(3-fluoroazetidin-1-yl)methyl]pyrido[3,2-d]pyrimidin-4-amine (350 mg, 25.4% yield) as a white solid.

LCMS m/z 474.0 [M+1]⁺.

¹H NMR (400 MHz, DMSO-d6) δ ppm 3.22 - 3.27 (m, 1 H) 3.28 - 3.32 (m, 1 H) 3.59 - 3.72 (m, 2 H) 3.94 (s, 2 H) 5.22 (dquin, J=57.72, 5.08, 5.08, 5.08, 5.08 Hz, 1 H) 6.82 (t, J=54.28 Hz, 1 H) 7.42 (t, J=8.13 Hz, 1 H) 7.67 (dd, J=8.07, 1.31 Hz, 1 H) 8.18 - 8.23 (m, 1 H) 8.32 (d, J=7.87 Hz, 1 H) 8.96 (d, J=1.88 Hz, 1 H) 10.35 (s, 1 H)

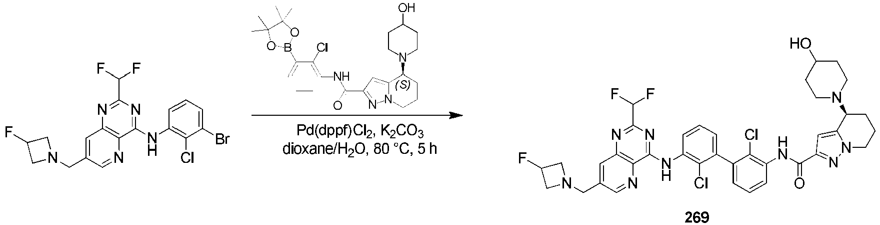

N-(3-bromo-2-chloro-phenyl)-2-(difluoromethyl)-7-[(3-fluoroazetidin-1-yl)methyl]pyrido[3,2-d]pyrimidin-4-amine (35 mg, 74.04 µmol, 1 eq), (4S)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (48.21 mg, 96.26 µmol, 1.3 eq), K₂CO₃ (20.47 mg, 148.09 µmol, 2 eq), and Pd(dppf)Cl₂ (5.42 mg, 7.40 µmol, 0.1 eq) were added to a mixture of dioxane (3 mL) and H₂O (0.5 mL), and the reaction mixture was stirred at 80 °C for 5 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 20:1) and further purified by reversed-phase HPLC (0.1% NH₃·H₂O condition; column: Waters XBridge, 150 * 25 mm * 5 µm; mobile phase: [water (NH₄HCO₃)-ACN]; gradient: 50% to 80% B over 9 min) to afford Compound 269 (6 mg, 10.3% yield) as a white solid.

LCMS m/z 766.3 [M+1]⁺.

¹H NMR (400 MHz, DMSO-d6) δ ppm 1.33 - 1.40 (m, 1 H) 1.41 - 1.47 (m, 1 H) 1.62 - 1.70 (m, 1 H) 1.72 (br s, 1 H) 1.86 - 1.92 (m, 1 H) 1.93 - 1.99 (m, 1 H) 2.10 - 2.18 (m, 1 H) 2.24 - 2.30 (m, 1 H) 2.33 (br s, 1 H) 2.40 (br s, 1 H) 2.44 (br dd, J=4.44, 2.44 Hz, 1 H) 2.63 - 2.68 (m, 1 H) 2.71 - 2.77 (m, 1 H) 3.26 (br d, J=4.50 Hz, 1 H) 3.42 - 3.51 (m, 1 H) 3.61 - 3.70 (m, 2 H) 3.94 (s, 2 H) 4.01 - 4.10 (m, 1 H) 4.17 - 4.24 (m, 1 H) 4.54 (d, J=4.13 Hz, 1 H) 5.11 - 5.18 (m, 1 H) 5.26 - 5.32 (m, 1 H) 6.65 (s, 1 H) 6.74 - 7.02 (m, 1 H) 7.19 (d, J=7.13 Hz, 1 H) 7.25 (br d, J=7.75 Hz, 1 H) 7.50 (t, J=7.86 Hz, 1 H) 7.59 (t, J=7.94 Hz, 1 H) 8.22 (s, 1 H) 8.28 (dd, J=7.50, 3.38 Hz, 1 H) 8.55 (d, J=8.25 Hz, 1 H) 8.97 (s, 1 H) 9.55 (s, 1 H) 10.28 (s, 1 H)

### [Preparation Example 74]

### Preparation of Compound 270, (R)-N-(2,2'-dichloro-3'-((2-(difluoromethyl)-7-((3-fluoroazetidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

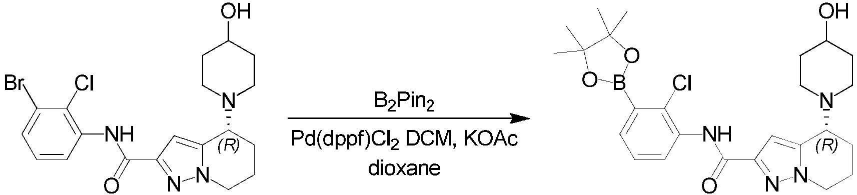

A mixture of (4R)-N-(3-bromo-2-chloro-phenyl)-4-(4-hydroxy-1-piperidyl)-4,5,6,7-tetrahydropyrazolo[1,5-a]pyridine-2-carboxamide (150 mg, 330.57 µmol, 1 eq), 4,4,5,5-tetramethyl-2-(4,4,5,5-tetramethyl-1,3,2-dioxaborolan-2-yl)-1,3,2-dioxaborolane (251.83 mg, 991.71 µmol, 3 eq), AcOK (64.88 mg, 661.14 µmol, 2 eq), and Pd(dppf)Cl₂·CH₂Cl₂ (27.00 mg, 33.06 µmol, 0.1 eq) in dioxane (3 mL) was stirred at 100 °C for 12 hours under a nitrogen atmosphere. After cooling to room temperature, the reaction mixture was filtered, and the filtrate was concentrated under reduced pressure. The resulting residue was purified by prep-TLC (SiO₂, ethyl acetate/methanol = 20/1) to afford (4R)-N-[2-chloro-3-(4,4,5,5-tetramethyl-1,3,2-dioxaborolan-2-yl)phenyl]-4-(4-hydroxy-1-piperidy)-4,5,6,7-tetrahydropyrazolo[1,5-a]pyridine-2-carboxamide (150 mg, 45.3% yield) as a black solid.

LCMS m/z 501.2 [M+1]⁺.

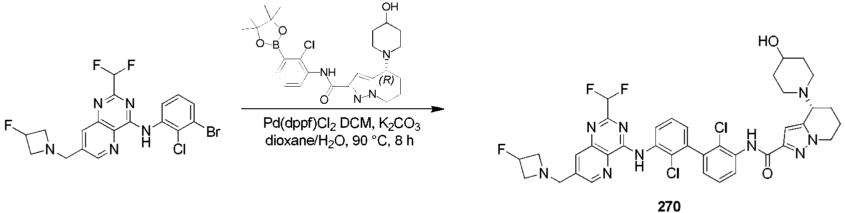

N-(3-bromo-2-chloro-phenyl)-2-(difluoromethyl)-7-[(3-fluoroazetidin-1-yl)methyl]pyrido[3,2-d]pyrimidin-4-amine 6 (50 mg, 105.78 µmol, 1 eq), (4R)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide 4-R (63.57 mg, 126.93 µmol, 1.2 eq), K₂CO₃ (29.24 mg, 211.56 µmol, 2 eq), and Pd(dppf)Cl₂·CH₂Cl₂ (8.64 mg, 10.58 µmol, 0.1 eq) were added to a mixture of dioxane (3 mL) and H₂O (0.5 mL), and the reaction mixture was stirred at 90 °C for 8 hours under a nitrogen atmosphere. After cooling to room temperature, the reaction mixture was filtered, and the filtrate was concentrated under reduced pressure. The resulting residue was purified by reversed-phase HPLC (0.1% NH₃·H₃O condition; column: Waters XBridge, 150 * 25 mm * 5 µm; mobile phase: [water (NH₄HCO₃)-ACN]; gradient: 55% to 85% B over 9 min) to afford Compound 270 (13 mg, 15.3% yield) as a white solid.

LCMS m/z 766.3 [M+1]⁺.

¹H NMR (400 MHz, DMSO-d6) δ ppm 1.14 - 1.31 (m, 1 H) 1.35 - 1.49 (m, 2 H) 1.65 - 1.78 (m, 3 H) 1.88 (br d, J=14.26 Hz, 1 H) 1.95 (br s, 1 H) 2.12 (br s, 1 H) 2.24 - 2.33 (m, 1 H) 2.37 - 2.44 (m, 1 H) 2.62 - 2.69 (m, 1 H) 2.74 (br d, J=10.88 Hz, 1 H) 3.24 (br dd, J=9.32, 4.57 Hz, 1 H) 3.44 - 3.51 (m, 1 H) 3.60 - 3.70 (m, 2 H) 3.94 (s, 2 H) 4.01 - 4.10 (m, 1 H) 4.20 (br d, J=12.51 Hz, 1 H) 4.57 (d, J=4.13 Hz, 1 H) 5.15 (s, 1 H) 5.29 (s, 1 H) 6.65 (s, 1 H) 6.88 (t, J=54.28 Hz, 1 H) 7.19 (d, J=6.38 Hz, 1 H) 7.25 (d, J=7.63 Hz, 1 H) 7.50 (t, J=7.94 Hz, 1 H) 7.59 (t, J=7.88 Hz, 1 H) 8.22 (s, 1 H) 8.27 (dd, J=7.50, 3.50 Hz, 1 H) 8.54 (d, J=7.50 Hz, 1 H) 8.97 (d, J=1.63 Hz, 1 H) 9.56 (s, 1 H) 10.28 (br s, 1 H).

### [Preparation Example 75]

### Preparation of Compound 271, (S)-N-(2,2'-dichloro-3'-((7-((3,3-difluoroazetidin-1-yl)methyl)-2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

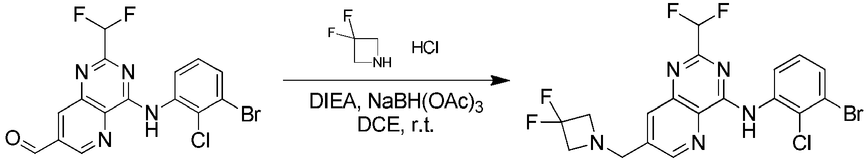

To a solution of 4-(3-bromo-2-chloro-anilino)-2-(difluoromethyl)pyrido[3,2-d]pyrimidine-7-carbaldehyde (1 g, 2.42 mmol, 1 eq) and 3,3-difluoroazetidine hydrochloride (1.57 g, 12.09 mmol, 5 eq) in DCE (10 mL), NaBH(OAc)₃ (1.54 g, 7.25 mmol, 3 eq) was added. The reaction mixture was stirred at room temperature for 12 hours. The reaction mixture was filtered, and the filtrate was concentrated under reduced pressure. The resulting residue was purified by column chromatography (SiO₂, Petroleum ether/Ethyl acetate = 2/1 to 0/1) to afford N-(3-bromo-2-chloro-phenyl)-7-[(3,3-difluoroazetidin-1-yl)methyl]-2-(difluoromethyl)pyrido[3,2-d]pyrimidin-4-amine (339 mg, 23.4% yield) as a red solid.

LCMS m/z 492.0 [M+1]⁺.

¹H NMR (400 MHz, DMSO-d6) δ ppm 3.74 (t, J=12.51 Hz, 4 H) 4.05 (s, 2 H) 6.82 (t, J=54.34 Hz, 1 H) 7.40 (t, J=7.69 Hz, 1 H) 7.66 (dd, J=8.07, 1.19 Hz, 1 H) 8.24 (s, 1 H) 8.32 (dd, J=8.19, 1.19 Hz, 1 H) 8.98 (d, J=1.63 Hz, 1 H) 10.35 (br s, 1 H).

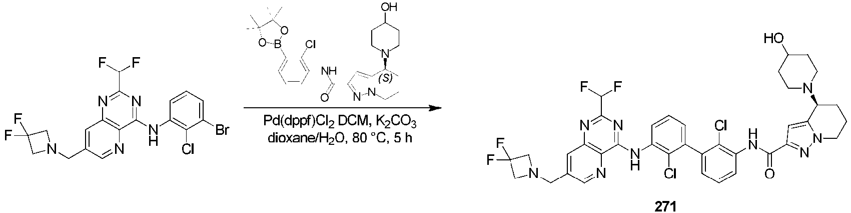

N-(3-bromo-2-chloro-phenyl)-7-[(3,3-difluoroazetidin-1-yl)methyl]-2-(difluoromethyl)pyrido[3,2-d]pyrimidin-4-amine (50 mg, 101.90 µmol, 1 eq), (4S)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (61.24 mg, 122.28 µmol, 1.2 eq), K₂CO₃ (28.17 mg, 203.80 µmol, 2 eq), and Pd(dppf)Cl₂·CH₂Cl₂ (8.32 mg, 10.19 µmol, 0.1 eq) were added to a mixture of dioxane (3 mL) and H₂O (0.5 mL), and the reaction mixture was stirred at 80 °C for 5 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 20:1) and further purified by reversed-phase HPLC (0.1% NH₃·H₂O condition; column: Waters XBridge, 150 * 25 mm * 5 µm; mobile phase: [water (NH₄HCO₃)-ACN]; gradient: 55% to 85% B over 9 min) to afford Compound 271 (8 mg, 9.91% yield) as a white solid.

LCMS m/z 784.3 [M+1]⁺.

¹H NMR (400 MHz, DMSO-d6) δ ppm 1.23 (s, 1 H) 1.32 - 1.49 (m, 2 H) 1.71 (br d, J=14.51 Hz, 2 H) 1.88 (br d, J=13.51 Hz, 1 H) 1.97 (br d, J=11.01 Hz, 1 H) 2.12 (br s, 1 H) 2.23 - 2.35 (m, 1 H) 2.37 - 2.44 (m, 1 H) 2.62 - 2.68 (m, 1 H) 2.74 (br d, J=12.26 Hz, 1 H) 3.46 (br s, 1 H) 3.74 (t, J=12.51 Hz, 4 H) 3.96 (br dd, J=10.57, 4.94 Hz, 1 H) 4.05 (s, 3 H) 4.19 (br s, 1 H) 4.55 (d, J=4.13 Hz, 1 H) 6.65 (s, 1 H) 6.88 (t, J=54.28 Hz, 1 H) 7.19 (d, J=6.38 Hz, 1 H) 7.25 (d, J=7.50 Hz, 1 H) 7.51 (t, J=7.94 Hz, 1 H) 7.59 (t, J=7.94 Hz, 1 H) 8.25 - 8.30 (m, 2 H) 8.54 (d, J=7.00 Hz, 1 H) 9.00 (d, J=1.63 Hz, 1 H) 9.55 (s, 1 H) 10.30 (s, 1 H)

### [Preparation Example 76]

### Preparation of Compoun 272, (R)-N-(2,2'-dichloro-3'-((7-((3,3-difluoroazetidin-1-yl)methyl)-2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

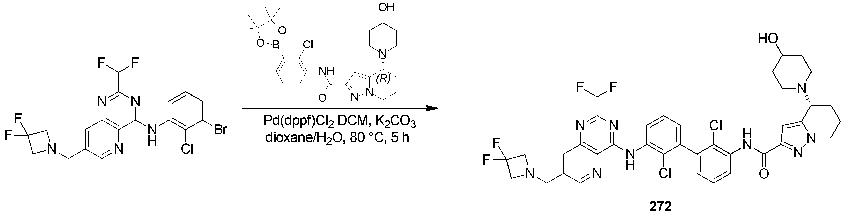

N-(3-bromo-2-chloro-phenyl)-7-[(3,3-difluoroazetidin-1-yl)methyl]-2-(difluoromethyl)pyrido[3,2-d]pyrimidin-4-amine (50 mg, 101.90 µmol, 1 eq), (4R)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (66.34 mg, 132.47 µmol, 1.3 eq), K₂CO₃ (28.17 mg, 203.80 µmol, 2 eq), and Pd(dppf)Cl₂·CH₂Cl₂ (8.32 mg, 10.19 µmol, 0.1 eq) were added to a mixture of dioxane (3 mL) and H₂O (0.5 mL), and the reaction mixture was stirred at 80 °C for 5 hours under a nitrogen atmosphere. After cooling to room temperature, the reaction mixture was filtered, and the filtrate was concentrated under reduced pressure. The resulting residue was purified by prep-TLC (SiO₂, Ethyl acetate:Methanol = 20:1) and further purified by reversed-phase HPLC (0.1% NH₃·H₂O condition; column: Waters XBridge, 150 * 25 mm * 5 µm; mobile phase: [water (NH₄HCO₃)-ACN]; gradient: 50% to 80% B over 15 min) to afford Compound 272 (12 mg, 14.1% yield) as a white solid.

LCMS m/z 784.3 [M+1]⁺.

¹H NMR (400 MHz, DMSO-d6) δ ppm 1.32 - 1.48 (m, 2 H) 1.65 - 1.78 (m, 3 H) 1.89 (br d, J=8.63 Hz, 1 H) 1.95 (br s, 1 H) 2.14 (br d, J=13.38 Hz, 1 H) 2.24 - 2.34 (m, 1 H) 2.36 - 2.44 (m, 1 H) 2.62 - 2.69 (m, 1 H) 2.74 (br d, J=11.26 Hz, 1 H) 3.43 - 3.51 (m, 1 H) 3.74 (t, J=12.51 Hz, 4 H) 3.96 (br dd, J=9.94, 5.44 Hz, 1 H) 4.01 - 4.10 (m, 3 H) 4.20 (br d, J=13.26 Hz, 1 H) 4.55 (d, J=4.00 Hz, 1 H) 6.65 (s, 1 H) 6.73 - 7.03 (m, 1 H) 7.19 (dd, J=7.63, 1.25 Hz, 1 H) 7.25 (d, J=7.50 Hz, 1 H) 7.50 (t, J=7.94 Hz, 1 H) 7.59 (t, J=7.94 Hz, 1 H) 8.24 - 8.30 (m, 2 H) 8.54 (dd, J=8.25, 1.38 Hz, 1 H) 8.99 (d, J=1.75 Hz, 1 H) 9.55 (s, 1 H) 10.29 (s, 1 H).

### [Preparation Example 77]

### Preparation of Compound 273, (S)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid

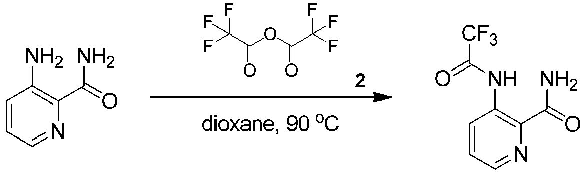

3-Aminopyridine-2-carboxamide (2 g, 14.58 mmol, 1 eq) and (2,2,2-trifluoroacetyl) 2,2,2-trifluoroacetate (3.68 g, 17.50 mmol, 2.43 mL, 1.2 eq) were added to dioxane (30 mL), and the reaction mixture was stirred at 90 °C for 12 hours. After completion of the reaction, the mixture was concentrated under reduced pressure to afford 3-[(2,2,2-trifluoroacetyl)amino]pyridine-2-carboxamide (5.7 g, 83.8% yield) as a black solid, which was used in the subsequent reaction without further purification.

LCMS m/z 234.1 [M+1]⁺.

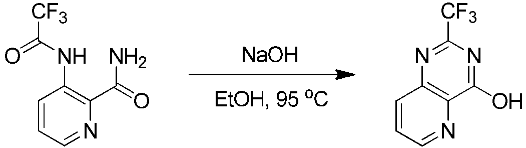

To a solution of 3-[(2,2,2-trifluoroacetyl)amino]pyridine-2-carboxamide (5.7 g, 24.45 mmol, 1 eq) in EtOH (60 mL), NaOH (3.91 g, 97.79 mmol, 4 eq) was added. The reaction mixture was stirred at 95 °C for 12 hours. After cooling to room temperature, the reaction mixture was concentrated under reduced pressure, and the resulting residue was purified by reversed-phase HPLC (0.1% NH₃·H₂O condition; column: Kromasil Eternity XT, 250 * 80 mm * 10 µm; mobile phase: H₂O (NH₄HCO₃)/ACN; gradient: 1% to 20% B over 20 min) to afford 2-(trifluoromethyl)pyrido[3,2-d]pyrimidin-4-ol (9.7 g, 92.2% yield) as a yellow solid.

LCMS m/z 216.0 [M+1]⁺.

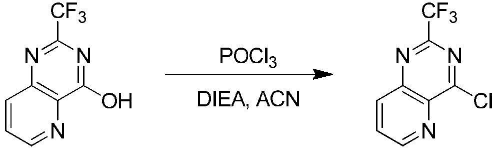

2-(Trifluoromethyl)pyrido[3,2-d]pyrimidin-4-ol (5 g, 23.24 mmol, 1 eq), DIPEA (9.01 g, 69.72 mmol, 12.14 mL, 3 eq), and POCl₃ (10.69 g, 69.72 mmol, 6.50 mL, 3 eq) were added to ACN (100 mL), and the reaction mixture was stirred at room temperature for 12 hours under a nitrogen atmosphere. The reaction mixture was diluted with aq. saturated NaHCO₃ solution (500 mL) and extracted with ethyl acetate (1000 mL * 3). The combined organic layers were washed with brine (500 mL), dried over Na₂SO₄, filtered, and concentrated under reduced pressure. The resulting residue was purified by column chromatography (SiO₂, Petroleum ether/Ethyl acetate = 5/1) to afford 4-chloro-2-(trifluoromethyl)pyrido[3,2-d]pyrimidine (2 g, 28.3% yield) as a brown liquid.

LCMS m/z 233.9 [M+1]⁺.

¹H NMR (400 MHz, DMSO-d6) δ ppm 8.22 - 8.29 (m, 1 H) 8.71 (dd, J=8.63, 1.50 Hz, 1 H) 9.35 (dd, J=4.13, 1.50 Hz, 1 H).

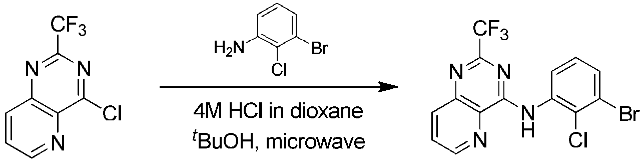

4-Chloro-2-(trifluoromethyl)pyrido[3,2-d]pyrimidine (500 mg, 2.14 mmol, 1 eq) and 3-bromo-2-chloro-aniline (662.95 mg, 3.21 mmol, 1.5 eq) were added to t-BuOH (20 mL), followed by addition of HCl in dioxane (2 M, 2.14 mL, 2 eq). The reaction mixture was stirred at 120 °C for 12 hours. After cooling to room temperature, the reaction mixture was concentrated under reduced pressure, and the resulting residue was purified by reversed-phase HPLC (0.1% NH₃·H₂O condition; column: Phenomenex Luna C18, 250 * 50 mm * 15 µm; mobile phase: H₂O (NH₄HCO₃)/ACN; gradient: 70% to 100% B over 30 min) to afford N-(3-bromo-2-chloro-phenyl)-2-(trifluoromethyl)pyrido[3,2-d]pyrimidin-4-amine (493 mg, 53.6% yield) as a yellow liquid.

LCMS m/z 404.9 [M+1]⁺.

¹H NMR (400 MHz, DMSO-d6) δ ppm 7.41 (t, J=8.13 Hz, 1 H) 7.67 (dd, J=8.00, 1.38 Hz, 1 H) 8.07 (dd, J=8.57, 4.31 Hz, 1 H) 8.20 (dd, J=8.13, 1.38 Hz, 1 H) 8.43 (dd, J=8.51, 1.50 Hz, 1 H) 9.09 (dd, J=4.31, 1.44 Hz, 1 H) 10.54 (s, 1 H)

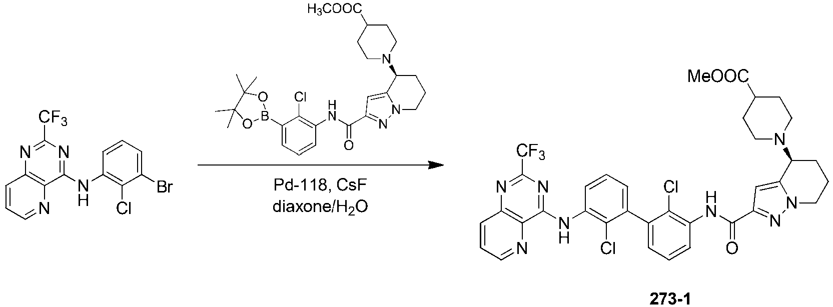

N-(3-bromo-2-chloro-phenyl)-2-(trifluoromethyl)pyrido[3,2-d]pyrimidin-4-amine (50 mg, 123.89 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (87.43 mg, 161.06 µmol, 1.3 eq), CsF (37.64 mg, 247.78 µmol, 2 eq), and ditert-butyl(cyclopentyl)phosphane;dichloropalladium;iron (8.07 mg, 12.39 µmol, 0.1 eq) were added to a mixture of dioxane (3 mL) and H₂O (0.5 mL), and the reaction mixture was stirred at 80 °C for 5 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 2:1) to afford Compound 273-1 (50 mg, 45.8% yield) as a yellow solid.

LCMS m/z 739.2 [M+1]⁺.

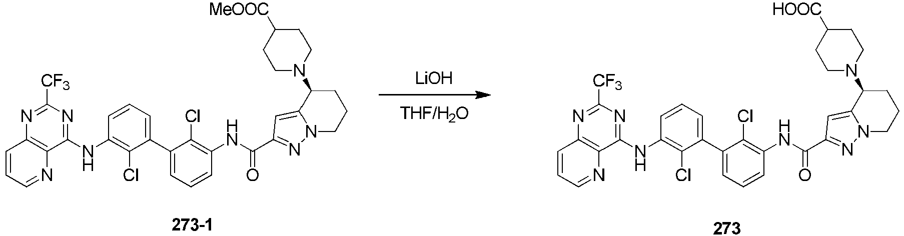

To a mixture of Methyl 1-[(4S)-2-[[2-chloro-3-[2-chloro-3-[[2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]piperidine-4-carboxylate 273-1 (50 mg, 67.61 µmol, 1 eq) in THF (30 mL) and H₂O (10 mL), LiOH·H₂O (8.51 mg, 202.82 µmol, 3 eq) was added, and the reaction mixture was stirred at room temperature for 12 hours. The reaction mixture was concentrated under reduced pressure, and the resulting residue was purified by reversed-phase HPLC (0.1% NH₃·H₂O condition; column: Waters XBridge, 150 * 25 mm * 5 µm; mobile phase: H₂O (NH₄HCO₃)/ACN; gradient: 30% to 60% B over 9 min) to afford Compound 273 (26 mg, 51.9% yield) as a white solid.

LCMS m/z 725.2 [M+1]⁺.

¹H NMR (400 MHz, DMSO-d6) δ ppm 1.49 - 1.70 (m, 3 H) 1.81 (br t, J=12.32 Hz, 2 H) 1.87 - 2.01 (m, 2 H) 2.12 - 2.22 (m, 2 H) 2.23 - 2.34 (m, 2 H) 2.41 - 2.47 (m, 1 H) 2.63 - 2.71 (m, 2 H) 2.79 (br d, J=11.38 Hz, 1 H) 3.97 (br dd, J=9.63, 5.25 Hz, 1 H) 4.08 (br d, J=7.13 Hz, 1 H) 4.21 (br d, J=16.13 Hz, 1 H) 6.67 (s, 1 H) 7.19 (d, J=7.27 Hz, 1 H) 7.29 (d, J=7.63 Hz, 1 H) 7.51 (t, J=7.94 Hz, 1 H) 7.61 (t, J=7.94 Hz, 1 H) 8.09 (dd, J=8.51, 4.38 Hz, 1 H) 8.27 (ddd, J=8.29, 4.16, 1.56 Hz, 1 H) 8.38 (d, J=7.80 Hz, 1 H) 8.46 (dd, J=8.50, 1.38 Hz, 1 H) 9.12 (dd, J=4.31, 1.44 Hz, 1 H) 9.56 (s, 1 H).

### [Preparation Example 78]

### Preparation of Compound 274, (R)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid

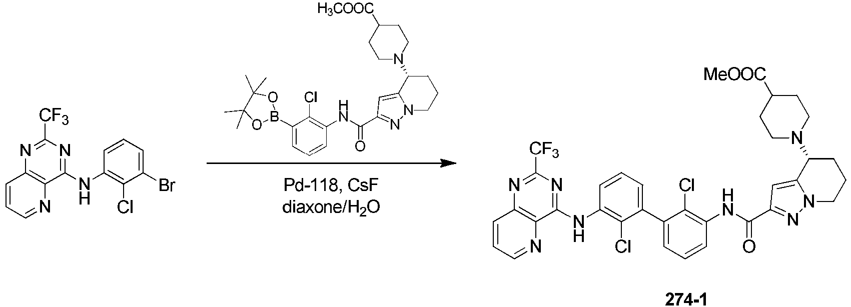

N-(3-bromo-2-chloro-phenyl)-2-(trifluoromethyl)pyrido[3,2-d]pyrimidin-4-amine (50 mg, 123.89 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate 8 (87.43 mg, 161.06 µmol, 1.3 eq), CsF (37.64 mg, 247.78 µmol, 2 eq), and ditert-butyl(cyclopentyl)phosphane;dichloropalladium;iron (8.07 mg, 12.39 µmol, 0.1 eq) were added to a mixture of dioxane (3 mL) and H₂O (0.5 mL), and the reaction mixture was stirred at 80 °C for 4 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 1:1) to afford Compound 274-1 (92 mg, 87.3% yield) as a yellow solid.

LCMS m/z 739.3 [M+1]⁺.

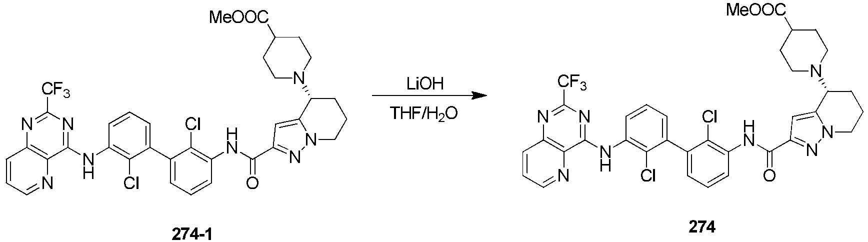

To a mixture of Methyl 1-[(4R)-2-[[2-chloro-3-[2-chloro-3-[[2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]piperidine-4-carboxylate 274-1 (92 mg, 124.40 µmol, 1 eq) in THF (30 mL) and H₂O (10 mL), LiOH·H₂O (15.66 mg, 373.19 µmol, 3 eq) was added, and the reaction mixture was stirred at room temperature for 12 hours. The reaction mixture was concentrated under reduced pressure, and the resulting residue was purified by reversed-phase HPLC (0.1% NH₃·H₂O condition; column: Waters XBridge, 150 × 25 mm, 5 µm; mobile phase: [water (NH₄HCO₃)-ACN]; gradient: 30% to 60% B over 9 min) to afford Compound 274 (35 mg, 38.3% yield) as a white solid.

LCMS m/z 725.3 [M+1]⁺.

¹H NMR (400 MHz, DMSO-d6) δ ppm 1.46 - 1.60 (m, 2 H) 1.61 - 1.74 (m, 2 H) 1.76 - 1.86 (m, 2 H) 1.87 - 2.02 (m, 2 H) 2.11 - 2.18 (m, 1 H) 2.20 - 2.34 (m, 2 H) 2.40 - 2.45 (m, 1 H) 2.67 (br s, 1 H) 2.79 (br d, J=10.63 Hz, 1 H) 3.97 (br dd, J=10.19, 5.32 Hz, 1 H) 4.03 - 4.11 (m, 1 H) 4.21 (br d, J=13.13 Hz, 1 H) 6.67 (s, 1 H) 7.19 (d, J=6.38 Hz, 1 H) 7.29 (d, J=7.63 Hz, 1 H) 7.51 (t, J=7.94 Hz, 1 H) 7.61 (t, J=7.94 Hz, 1 H) 8.08 (dd, J=8.57, 4.32 Hz, 1 H) 8.28 (dd, J=6.88, 4.13 Hz, 1 H) 8.38 (d, J=7.25 Hz, 1 H) 8.46 (dd, J=8.50, 1.25 Hz, 1 H) 9.12 (dd, J=4.19, 1.19 Hz, 1 H) 9.55 (s, 1 H) 10.53 (br s, 1 H).

### [Preparation Example 79]

### Preparation of Compound 275, (S)-2-((2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid, and Compound 276, (R)-2-((2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid

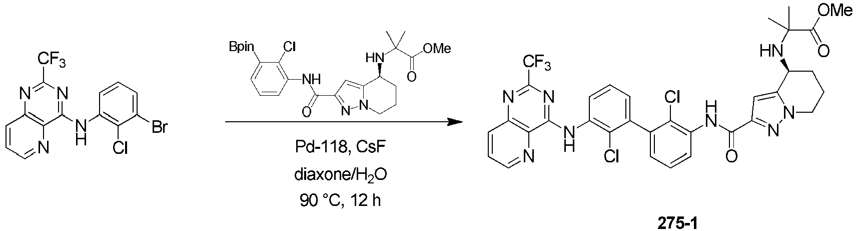

N-(3-bromo-2-chloro-phenyl)-2-(trifluoromethyl)pyrido[3,2-d]pyrimidin-4-amine 1 (100 mg, 247.78 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (166.48 mg, 322.11 µmol, 1.3 eq), ditert-butyl(cyclopentyl)phosphane;dichloropalladium;iron (16.15 mg, 24.78 µmol, 0.1 eq), and CsF (112.91 mg, 743.34 µmol, 3 eq) were added to a mixture of dioxane (8 mL) and H₂O (1.5 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 2:1) to afford Compound 275-1 (104 mg, 48.2% yield) as a yellow solid.

LCMS m/z 712.17 [M+1]⁺.

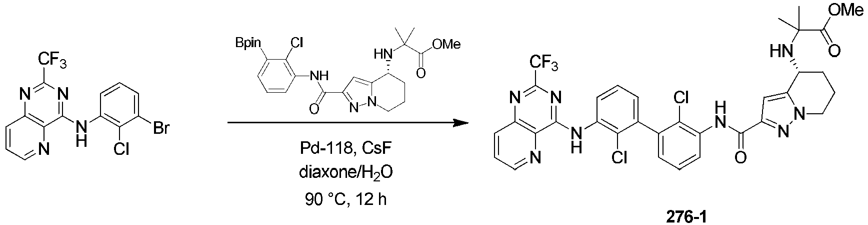

N-(3-bromo-2-chloro-phenyl)-2-(trifluoromethyl)pyrido[3,2-d]pyrimidin-4-amine (100 mg, 247.78 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (166.48 mg, 322.11 µmol, 1.3 eq), ditert-butyl(cyclopentyl)phosphane;dichloropalladium;iron (16.15 mg, 24.78 µmol, 0.1 eq), and CsF (112.91 mg, 743.34 µmol, 3 eq) were added to a mixture of dioxane (8 mL) and H₂O (1.5 mL), and the reaction mixture was stirred at 90 °C for 12 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 2:1) to afford Compound 276-1 (163 mg, 68.2% yield) as a yellow solid.

LCMS m/z 712.17 [M+1]⁺.

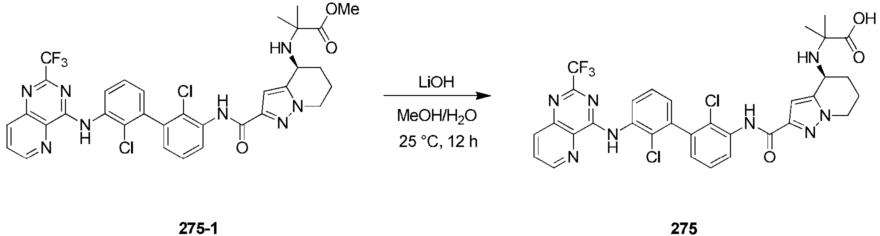

To a mixture of Methyl 2-[[(4S)-2-[[2-chloro-3-[2-chloro-3-[[2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]amino]-2-methyl-propanoate 275-1 (67 mg, 93.90 µmol, 1 eq) in MeOH (5 mL) and H₂O (1 mL), LiOH·H₂O (19.70 mg, 469.49 µmol, 5 eq) was added, and the reaction mixture was stirred at room temperature for 12 hours. The reaction mixture was concentrated under reduced pressure, and the resulting residue was purified by reversed-phase HPLC (column: Waters XBridge, 150 * 25 mm * 5 µm; mobile phase: H₂O (NH₄HCO₃)/ACN; gradient: 32% to 62% B over 9 min) to afford Compound 275 (14 mg, 21.1% yield) as a white solid.

LCMS 698.15[M+1]⁺.

¹H NMR (400 MHz, DMSO-*d*₆) δ ppm 1.29 (d, J=3.50 Hz, 6 H) 1.56 - 1.76 (m, 1 H) 1.85 - 1.98 (m, 1 H) 2.00 - 2.09 (m, 1 H) 2.11 - 2.24 (m, 1 H) 3.88 - 3.99 (m, 1 H) 4.13 (br s, 2 H) 6.80 (s, 1 H) 7.19 (d, *J*=7.35 Hz, 1 H) 7.30 (d, *J*=7.50 Hz, 1 H) 7.51 (t, *J*=7.94 Hz, 1 H) 7.62 (t, *J*=7.88 Hz, 1 H) 8.09 (dd, *J*=8.38, 4.25 Hz, 1 H) 8.30 (d, *J*=8.00 Hz, 1 H) 8.37 (d, *J*=9.01 Hz, 1 H) 8.47 (d, *J*=7.00 Hz, 1 H) 9.13 (d, *J*=4.25 Hz, 1 H) 9.56 (s, 1 H)

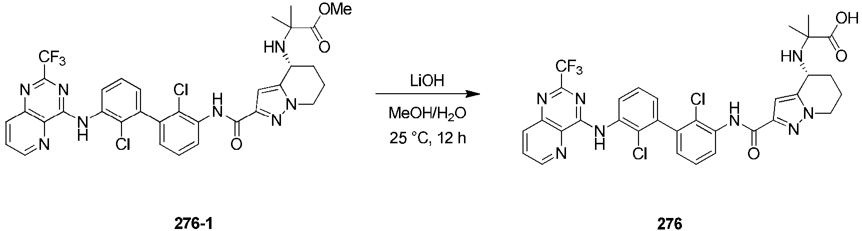

To a mixture of Methyl 2-[[(4R)-2-[[2-chloro-3-[2-chloro-3-[[2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]amino]-2-methyl-propanoate 276-1 (142 mg, 199.01 µmol, 1 eq) in MeOH (5 mL) and H₂O (1 mL), LiOH·H₂O (25.05 mg, 597.03 µmol, 3 eq) was added , and the reaction mixture was stirred at room temperature for 12 hours. The reaction mixture was concentrated under reduced pressure, and the resulting residue was purified by reversed-phase HPLC (column: Waters XBridge, 150 * 25 mm * 5 µm; mobile phase: H₂O (NH₄HCO₃)/ACN; gradient: 30% to 60% B over 9 min) to afford Compound 276 (11 mg, 7.74% yield) as a white solid.

LCMS m/z 698.15 [M+1]⁺.

¹H NMR (400 MHz, DMSO-*d*₆) δ ppm 1.29 (d, *J*=3.88 Hz, 6 H) 1.55 - 1.72 (m, 1 H) 1.87 - 1.98 (m, 1 H) 1.99 - 2.08 (m, 1 H) 2.10 - 2.23 (m, 1 H) 3.93 (br d, *J*=7.75 Hz, 1 H) 4.08 - 4.17 (m, 2 H) 6.80 (s, 1 H) 7.19 (d, *J*=7.50 Hz, 1 H) 7.30 (d, *J*=7.50 Hz, 1 H) 7.51 (t, *J*=8.13 Hz, 1 H) 7.61 (t, *J*=7.94 Hz, 1 H) 8.07 - 8.11 (m, 1 H) 8.30 (d, *J*=7.50 Hz, 1 H) 8.38 (d, *J*=8.25 Hz, 1 H) 8.47 (d, *J*=8.50 Hz, 1 H) 9.13 (d, *J*=3.00 Hz, 1 H) 9.56 (s, 1 H)

### [Preparation Example 80]

### Preparation of Compound 277, (S)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid

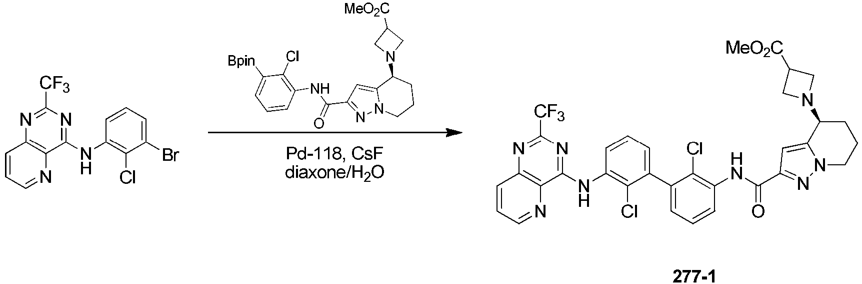

N-(3-bromo-2-chloro-phenyl)-2-(trifluoromethyl)pyrido[3,2-d]pyrimidin-4-amine (60 mg, 148.67 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (99.50 mg, 193.27 µmol, 1.3 eq), CsF (45.17 mg, 297.34 µmol, 2 eq), and ditert-butyl(cyclopentyl)phosphane;dichloropalladium;iron (9.69 mg, 14.87 µmol, 0.1 eq) were added to a mixture of dioxane (3 mL) and H₂O (0.5 mL), and the reaction mixture was stirred at 80 °C for 5 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 1:1) to afford Compound 277-1 (93 mg, 75.6% yield) as a yellow solid.

LCMS m/z 711.2 [M+1]⁺.

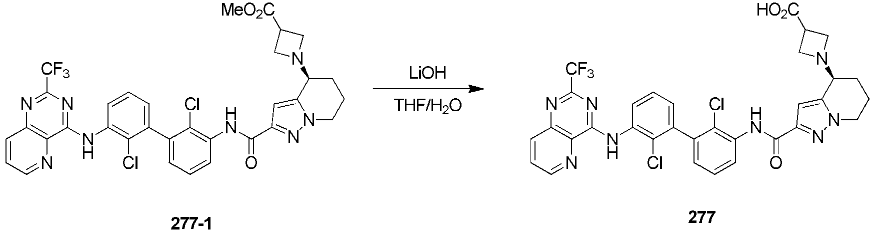

To a mixture of Methyl 1-[(4S)-2-[[2-chloro-3-[2-chloro-3-[[2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]azetidine-3-carboxylate 277-1 (93 mg, 130.71 µmol, 1 eq) in THF (25 mL) and H₂O (10 mL), LiOH·H₂O (16.45 mg, 392.12 µmol, 3 eq) was added, and the reaction mixture was stirred at room temperature for 12 hours. The reaction mixture was concentrated under reduced pressure, and the resulting residue was purified by reversed-phase HPLC (0.1% NH₃·H₂O condition; column: Waters XBridge, 150 * 25 mm * 5 µm; mobile phase: [water (NH₄HCO₃)-ACN]; gradient: 28% to 58% B over 9 min) to afford Compound 277 (45 mg, 47.8% yield) as a white solid.

LCMS m/z 697.2 [M+1]⁺.

¹H NMR (400 MHz, DMSO-*d*6) δ ppm 1.74 (br s, 2 H) 1.81 - 1.89 (m, 1 H) 2.13 - 2.25 (m, 1 H) 3.14 (br dd, *J*=14.82, 7.32 Hz, 2 H) 3.19 - 3.23 (m, 2 H) 3.46 - 3.50 (m, 2 H) 3.53 (br t, *J*=4.13 Hz, 1 H) 4.04 - 4.13 (m, 1 H) 4.18 - 4.27 (m, 1 H) 6.75 (s, 1 H) 7.19 (dd, *J*=7.63, 1.25 Hz, 1 H) 7.29 (dt, *J*=7.63, 1.44 Hz, 1 H) 7.50 (t, *J*=7.94 Hz, 1 H) 7.61 (t, *J*=7.94 Hz, 1 H) 8.08 (dd, J=8.57, 4.31 Hz, 1 H) 8.27 (ddd, *J*=8.22, 3.78, 1.50 Hz, 1 H) 8.38 (d, *J*=8.13 Hz, 1 H) 8.46 (dd, *J*=8.51, 1.50 Hz, 1 H) 9.12 (dd, *J=*4.25, 1.25 Hz, 1 H) 9.55 (s, 1 H) 10.35 - 10.68 (m, 1 H)

### [Preparation Example 81]

### Preparation of Compound 278, (R)-1-(2-((2,2'-dichloro-3'-((2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid

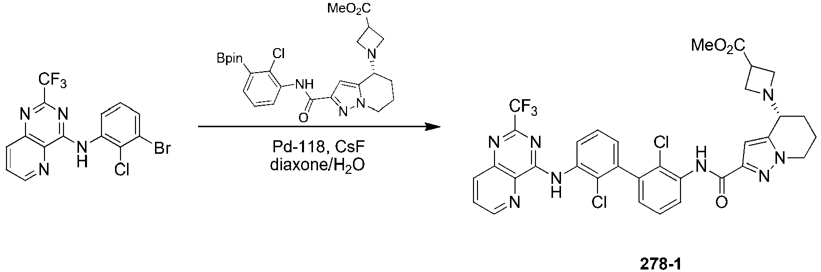

N-(3-bromo-2-chloro-phenyl)-2-(trifluoromethyl)pyrido[3,2-d]pyrimidin-4-amine (60 mg, 148.67 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (99.50 mg, 193.27 µmol, 1.3 eq), CsF (45.17 mg, 297.34 µmol, 2 eq), and ditert-butyl(cyclopentyl)phosphane;dichloropalladium;iron (9.69 mg, 14.87 µmol, 0.1 eq) were added to a mixture of dioxane (3 mL) and H₂O (0.5 mL), and the reaction mixture was stirred at 80 °C for 5 hours under a nitrogen atmosphere. After cooling to room temperature, the reaction mixture was filtered, and the filtrate was concentrated under reduced pressure. The resulting residue was purified by prep-TLC (SiO₂, petroleum ether:ethyl acetate = 1:1) to afford Compound 278-1 (80 mg, 71.8% yield) as a yellow solid.

LCMS m/z 711.2 [M+1]⁺.

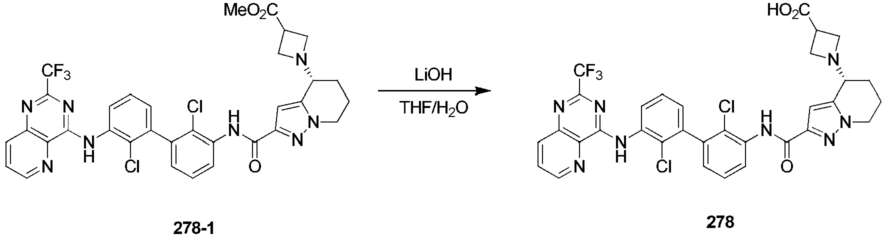

To a mixture of Methyl 1-[(4R)-2-[[2-chloro-3-[2-chloro-3-[[2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]azetidine-3-carboxylate 278-1 (80 mg, 112.44 µmol, 1 eq) in THF (25 mL) and H₂O (10 mL), LiOH·H₂O (14.15 mg, 337.31 µmol, 3 eq) was added, and the reaction mixture was stirred at room temperature for 12 hours. The reaction mixture was concentrated under reduced pressure, and the resulting residue was purified by reversed-phase HPLC (0.1% NH₃·H₂O condition; column: Waters XBridge, 150 * 25 mm * 5 µm; mobile phase: [water (NH₄HCO₃)-ACN]; gradient: 28% to 58% B over 9 min) to afford Compound 278 (34 mg, 41.6% yield) as a white solid.

LCMS m/z 697.2 [M+1]⁺.

¹H NMR (400 MHz, DMSO-*d*6) δ ppm 1.74 (br s, 2 H) 1.80 - 1.91 (m, 1 H) 2.13 - 2.25 (m, 1 H) 3.10 - 3.18 (m, 2 H) 3.19 - 3.23 (m, 2 H) 3.46 - 3.51 (m, 2 H) 3.53 (br t, *J*=4.25 Hz, 1 H) 4.04 - 4.13 (m, 1 H) 4.18 - 4.26 (m, 1 H) 6.75 (s, 1 H) 7.18 (d, *J*=7.63 Hz, 1 H) 7.29 (d, *J*=7.63 Hz, 1 H) 7.50 (t, *J*=7.94 Hz, 1 H) 7.61 (t, *J=*7.88 Hz, 1 H) 8.08 (dd, *J*=8.50, 4.25 Hz, 1 H) 8.24 - 8.30 (m, 1 H) 8.38 (d, *J*=8.13 Hz, 1 H) 8.46 (dd, *J*=8.44, 1.31 Hz, 1 H) 9.12 (d, *J*=3.13 Hz, 1 H) 9.55 (s, 1 H) 10.53 (br s, 1 H).

### [Preparation Example 82]

### Preparation of Compound 279, (S)-2-((2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid, and Compound 280, (R)-2-((2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid

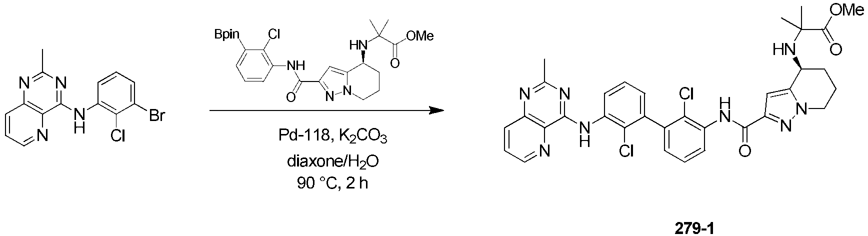

N-(3-bromo-2-chloro-phenyl)-2-methyl-pyrido[3,2-d]pyrimidin-4-amine (80 mg, 228.82 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (130.09 mg, 251.71 µmol, 1.1 eq), ditert-butyl(cyclopentyl)phosphane;dichloropalladium;iron (14.91 mg, 22.88 µmol, 0.1 eq), and K₂CO₃ (94.88 mg, 686.47 µmol, 3 eq) were added to a mixture of dioxane (6 mL) and H₂O (1.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by prep-TLC (Petroleum ether/Ethyl acetate = 100/1 to 1/1) to afford Compound 279-1 (102 mg, 52.7% yield) as a white solid.

LCMS m/z 659.2 [M+1]⁺.

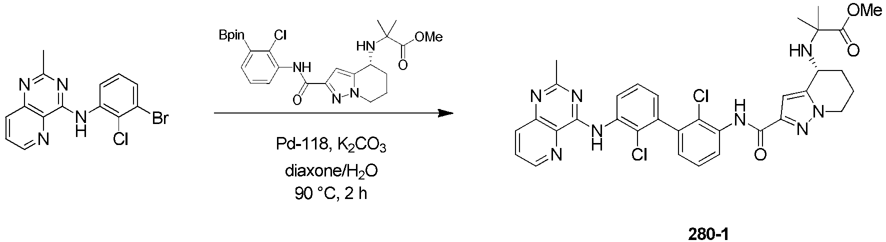

N-(3-bromo-2-chloro-phenyl)-2-methyl-pyrido[3,2-d]pyrimidin-4-amine (100 mg, 286.03 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (162.61 mg, 314.63 µmol, 1.1 eq), ditert-butyl(cyclopentyl)phosphane;dichloropalladium;iron (18.64 mg, 28.60 µmol, 0.1 eq), and K₂CO₃ (118.60 mg, 858.09 µmol, 3 eq) were added to a mixture of dioxane (6 mL) and H₂O (1.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by prep-TLC (Petroleum ether/Ethyl acetate = 1/1) to afford Compound 280-1 (122 mg, 51.7% yield) as a white solid.

LCMS m/z 659.2 [M+1]⁺

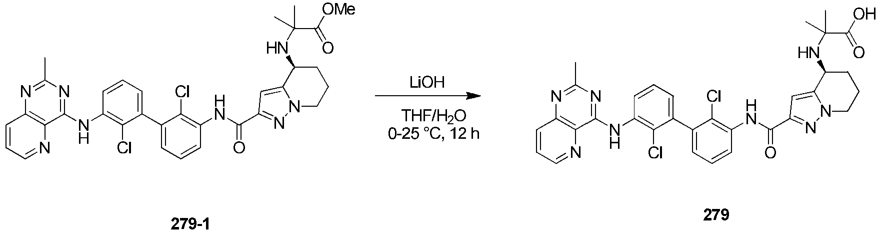

To a mixture of Methyl 2-[[(4S)-2-[[2-chloro-3-[2-chloro-3-[(2-methylpyrido[3,2-d]pyrimidin-4-yl)amino]phenyl]phenyl]carbamoyl]-4,5,6,7-tetrahydropyrazolo[1,5-a]pyridin-4-yl]amino]-2-methyl-propanoate 279-1 (82 mg, 124.32 µmol, 1 eq) in H₂O (1.5 mL) and THF (5 mL), LiOH·H₂O (15.65 mg, 372.97 µmol, 3 eq) was added at 0 °C, and the reaction mixture was stirred at room temperature for 12 hours. The reaction mixture was concentrated under reduced pressure, and the resulting residue was purified by prep-HPLC (column: Waters XBridge, 150 * 25 mm * 5 µm; mobile phase: [water (NH₄HCO₃)-ACN]; gradient: 25% to 55% B over 9 min) to afford Compound 279 (26 mg, 30.7% yield) as a white solid.

LCMS m/z 645.2 [M+1]⁺

¹H NMR (400 MHz, DMSO-*d₆* ) δ = 9.98 (s, 1H), 9.54 (s, 1H), 8.91 (dd, *J* = 1.4, 4.3 Hz, 1H), 8.83 (dd, *J* = 1.4, 8.3 Hz, 1H), 8.30 (dd, *J* = 1.4, 8.3 Hz, 1H), 8.20 (dd, *J* = 1.4, 8.5 Hz, 1H), 7.92 (dd, *J* = 4.2, 8.4 Hz, 1H), 7.65 - 7.56 (m, 1H), 7.50 (t, *J* = 8.0 Hz, 1H), 7.26 - 7.13 (m, 2H), 6.79 (s, 1H), 4.21 - 4.06 (m, 2H), 3.93 (br dd, *J* = 5.6, 7.1 Hz, 1H), 3.17 (s, 2H), 2.64 (s, 3H), 2.21 - 2.10 (m, 1H), 2.07 - 1.99 (m, 1H), 1.98 - 1.87 (m, 1H), 1.70 - 1.58 (m, 1H), 1.29 (d, *J* = 4.1 Hz, 6H).

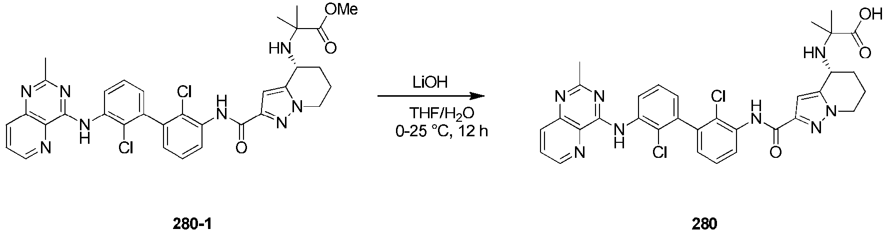

To a mixture of Methyl 2-[[(4R)-2-[[2-chloro-3-[2-chloro-3-[(2-methylpyrido[3,2-d]pyrimidin-4-yl)amino]phenyl]phenyl]carbamoyl]-4,5,6,7-tetrahydropyrazolo[1,5-a]pyridin-4-yl]amino]-2-methyl-propanoate 281-1 (98 mg, 148.58 µmol, 1 eq) in H₂O (1.5 mL) and THF (5 mL), LiOH·H₂O (18.70 mg, 445.75 µmol, 3 eq) was added at 0 °C, and the reaction mixture was stirred at room temperature for 12 hours. The reaction mixture was concentrated under reduced pressure, and the resulting residue was purified by prep-HPLC (column: Waters XBridge, 150 * 25 mm * 5 µm; mobile phase: [water (NH₄HCO₃)-ACN]; gradient: 5% to 55% B over 9 min) to afford Compound 280 (18 mg, 17.8% yield) as a white solid.

LCMS m/z 645.2 [M+1]⁺

¹H NMR (400 MHz, DMSO-*d₆* ) δ = 9.98 (s, 1H), 9.54 (s, 1H), 8.91 (d, *J* = 3.0 Hz, 1H), 8.83 (d, *J* = 7.3 Hz, 1H), 8.30 (d, *J* = 8.1 Hz, 1H), 8.20 (d, *J* = 8.6 Hz, 1H), 7.93 (dd, *J* = 4.3, 8.5 Hz, 1H), 7.58 (t, *J = 7.9* Hz, 1H), 7.50 (t, *J* = 7.9 Hz, 1H), 7.19 (t, *J* = 6.8 Hz, 2H), 6.79 (s, 1H), 4.18 - 4.04 (m, 2H), 3.98 - 3.87 (m, 1H), 3.20 - 3.12 (m, 2H), 2.64 (s, 3H), 2.16 - 1.96 (m, 2H), 1.92 (br s, 1H), 1.67 - 1.59 (m, 1H), 1.28 (d, *J* = 4.1 Hz, 6H).

### [Preparation Example 83]

### Preparation of Compound 281, (S)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid, and Compound 282, (R)-1-(2-((2,2'-dichloro-3'-((2-methylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid

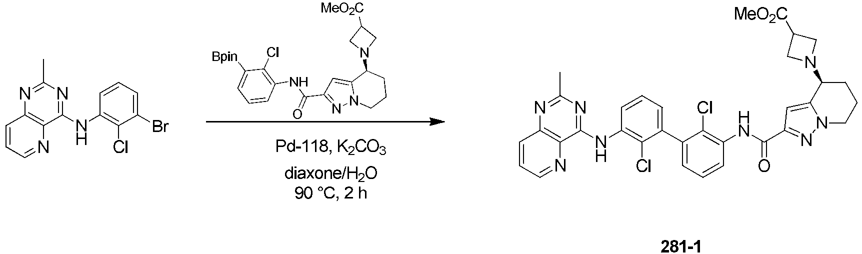

N-(3-bromo-2-chloro-phenyl)-2-methyl-pyrido[3,2-d]pyrimidin-4-amine (80 mg, 228.82 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (141.36 mg, 274.59 µmol, 1.2 eq), ditert-butyl(cyclopentyl)phosphane;dichloropalladium;iron (14.91 mg, 22.88 µmol, 0.1 eq), and K₂CO₃ (94.87 mg, 686.47 µmol, 3 eq) were added to a mixture of dioxane (8 mL) and H₂O (1.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by prep-TLC (Petroleum ether/Ethyl acetate = 1/1) to afford Compound 281-1 (82 mg, 43.6% yield) as a white solid.

LCMS m/z 657.2 [M+1]⁺.

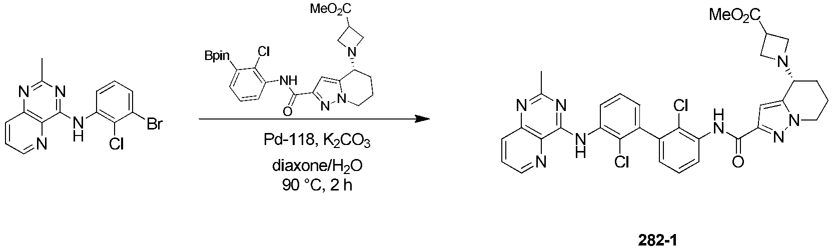

N-(3-bromo-2-chloro-phenyl)-2-methyl-pyrido[3,2-d]pyrimidin-4-amine 1 (80 mg, 228.82 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate 2R (176.70 mg, 343.24 µmol, 1.5 eq), ditert-butyl(cyclopentyl)phosphane;dichloropalladium;iron (14.91 mg, 22.88 µmol, 0.1 eq), and K₂CO₃ (94.87 mg, 686.47 µmol, 3 eq) were added to a mixture of dioxane (2 mL) and H₂O (0.5 mL), and the reaction mixture was stirred at 90 °C for 2 hours under a nitrogen atmosphere. After cooling to room temperature, the reaction mixture was filtered, and the filtrate was concentrated under reduced pressure. The resulting residue was purified by prep-TLC (Petroleum ether/Ethyl acetate = 1/1) to afford Compound 281-1 (93 mg, 51.3% yield) as a white solid.

LCMS m/z 657.3 [M+1]⁺

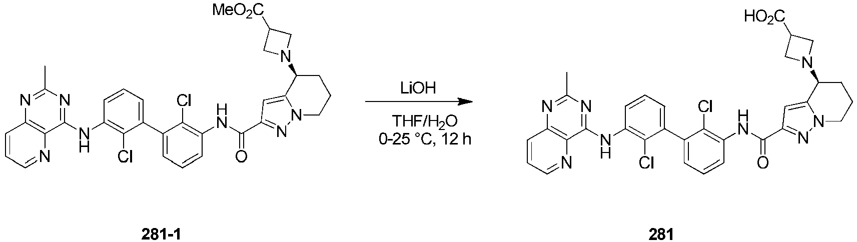

To a mixture of methyl 1-[(4S)-2-[[2-chloro-3-[2-chloro-3-[(2-methylpyrido[3,2-d]pyrimidin-4-yl)amino]phenyl]phenyl]carbamoyl]-4,5,6,7-tetrahydropyrazolo[1,5-a]pyridin-4-yl]azetidine-3-carboxylate 281-1 (76.73 mg, 116.69 µmol, 1 eq) in H₂O (1.5 mL) / THF (5 mL), LiOH·H₂O (14.69 mg, 350.06 µmol, 3 eq) was added at 0 °C. The reaction mixture was stirred at room temperature for 12 hours. The reaction mixture was then concentrated under reduced pressure, and the resulting residue was purified by prep-HPLC (column: Waters Xbridge 150 * 25 mm * 5 µm; mobile phase: [water (NH₄HCO₃)-ACN]; gradient: 25%-55% B over 9 min) to afford Compound 281 (21 mg, 25.1% yield) as a white solid.

LCMS m/z 643.1 [M+1]⁺

¹H NMR (400 MHz, DMSO-*d₆* ) δ = 9.73 - 9.64 (m, 1H), 9.04 (d, J = 3.5 Hz, 1H), 8.32 (d, J = 8.4 Hz, 1H), 8.26 - 8.18 (m, 1H), 8.15 (ddd, J = 1.3, 4.2, 8.1 Hz, 1H), 8.07 (dd, J = 4.3, 8.6 Hz, 1H), 7.67 - 7.58 (m, 1H), 7.52 (t, J = 7.9 Hz, 1H), 7.33 (br d, J = 7.6 Hz, 1H), 7.27 - 7.18 (m, 1H), 7.14 (s, 1H), 4.93 - 4.74 (m, 1H), 4.41 - 4.15 (m, 4H), 4.11 - 4.00 (m, 1H), 3.16 (s, 2H), 2.65 (s, 3H), 2.32 (s, 2H), 2.22 - 2.12 (m, 1H), 2.06 - 1.93 (m, 2H).

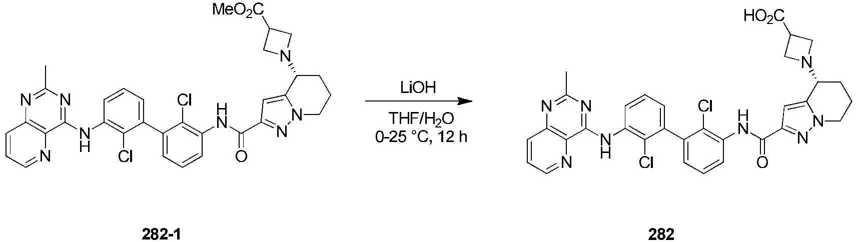

A mixture of methyl 1-[(4R)-2-[[2-chloro-3-[2-chloro-3-[(2-methylpyrido[3,2-d]pyrimidin-4-yl)amino]phenyl]phenyl]carbamoyl]-4,5,6,7-tetrahydropyrazolo[1,5-a]pyridin-4-yl]azetidine-3-carboxylate 3R (100 mg, 152.08 µmol, 1 eq) in H₂O (1.5 mL) and THF (5 mL) was cooled to 0 °C, and LiOH·H₃O (19.14 mg, 456.24 µmol, 3 eq) was added. The reaction mixture was stirred at room temperature for 12 hours. After completion of the reaction, the mixture was concentrated under reduced pressure, and the resulting residue was purified by prep-HPLC (column: Waters Xbridge 150 * 25 mm * 5 µm; mobile phase: [water (NH₄HCO₃)-ACN]; gradient: 25%-55% B over 9 min) to afford Compound 282 (41 mg, 37.6% yield) as a white solid.

LCMS m/z 645.2 [M+1]⁺

¹H NMR (400 MHz, DMSO-d₆ ) δ = 12.09 (br d, J = 2.5 Hz, 1H), 11.77 - 11.46 (m, 1H), 9.69 (d, J = 3.0 Hz, 1H), 9.08 (d, J = 3.3 Hz, 1H), 8.42 (d, J = 8.5 Hz, 1H), 8.19 - 8.10 (m, 2H), 8.00 (br d, J = 7.4 Hz, 1H), 7.66 - 7.58 (m, 1H), 7.52 (t, J = 7.9 Hz, 1H), 7.39 (d, J = 7.6 Hz, 1H), 7.26 - 7.21 (m, 1H), 7.14 (br s, 1H), 4.96 - 4.76 (m, 1H), 4.73 - 4.53 (m, 1H), 4.27 - 4.12 (m, 4H), 3.61 (br d, J = 8.8 Hz, 3H), 2.67 (s, 3H), 2.45 - 2.30 (m, 1H), 2.24 - 2.12 (m, 1H), 2.09 - 1.92 (m, 2H).

### [Preparation Example 84]

### Preparation of Compound 283, (S)-1-(2-((2,2'-dichloro-3'-((3-((3-fluoroazetidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid, Compound 283 and (R)-1-(2-((2,2'-dichloro-3'-((3-((3-fluoroazetidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)piperidine-4-carboxylic acid, Compound 284

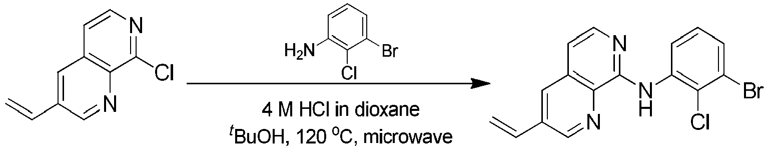

A mixture of 8-chloro-3-vinyl-1,7-naphthyridine (1.0 g, 5.25 mmol, 1 eq) and 3-bromo-2-chloroaniline (2.71 g, 13.11 mmol, 2.5 eq) in tert-butanol (20 mL) was treated with HCl in dioxane (2 M, 11.38 mL, 4.34 eq). The resulting reaction mixture was stirred at 120 °C for 12 hours. After completion of the reaction, the mixture was cooled to room temperature and concentrated under reduced pressure. The residue was purified by column chromatography on silica gel (Petroleum ether/Ethyl acetate = 1:0 to 0:1) to afford N-(3-bromo-2-chloro-phenyl)-3-vinyl-1,7-naphthyridin-8-amine (2.0 g, 67.6% yield) as a yellow solid.

LCMS m/z 361.9 [M+3]⁺.

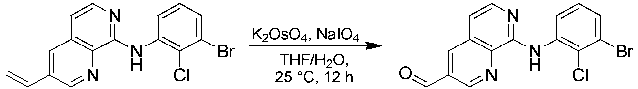

A solution of N-(3-bromo-2-chloro-phenyl)-3-vinyl-1,7-naphthyridin-8-amine (2.0 g, 5.55 mmol, 1 eq) in THF (20 mL) and water (4 mL) was treated with dipotassium osmate dihydrate (408.67 mg, 1.11 mmol, 0.2 eq) and sodium periodate (4.74 g, 22.18 mmol, 4 eq). The resulting reaction mixture was stirred at room temperature for 12 hours. After completion of the reaction, the mixture was diluted with water (50 mL) and extracted with ethyl acetate (30 mL × 3). The combined organic layers were dried over Na₂SO₄, filtered, and concentrated under reduced pressure. The residue was purified by column chromatography on silica gel (Petroleum ether/Ethyl acetate = 1/0 to 1/1) to afford 8-(3-bromo-2-chloro-anilino)-1,7-naphthyridine-3-carbaldehyde (1.12 g, 27.8% yield) as a white solid.

LCMS m/z 363.9 [M+3]⁺.

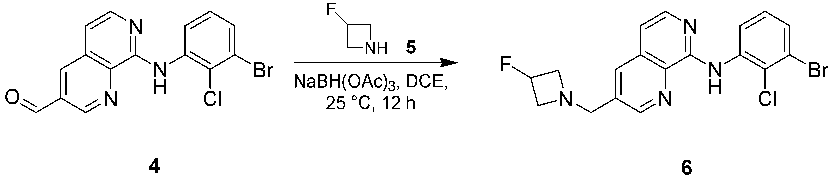

A mixture of 8-(3-bromo-2-chloro-anilino)-1,7-naphthyridine-3-carbaldehyde (1.0 g, 2.76 mmol, 1 eq) and 3-fluoroazetidine (621.20 mg, 8.27 mmol, 3 eq) in 1,2-dichloroethane (20 mL) was treated with sodium triacetoxyborohydride (876.73 mg, 4.14 mmol, 1.5 eq). The resulting reaction mixture was stirred at room temperature for 12 hours. After completion of the reaction, the mixture was concentrated under reduced pressure. The residue was purified by column chromatography on silica gel (Petroleum ether/Ethyl acetate = 1/0 to 0/1) to afford N-(3-bromo-2-chloro-phenyl)-3-[(3-fluoroazetidin-1-yl)methyl]-1,7-naphthyridin-8-amine (804 mg, 55.3% yield) as a black solid.

LCMS m/z 420.9 [M+1]⁺.

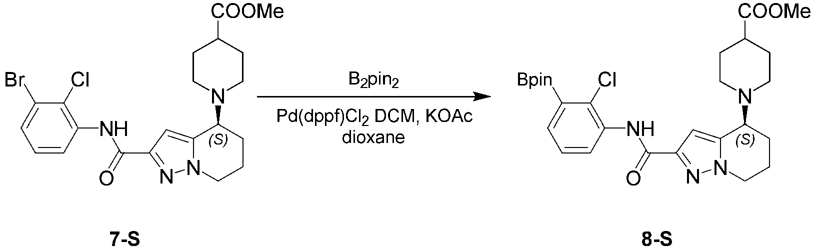

A mixture of methyl 1-[(4S)-2-[(3-bromo-2-chloro-phenyl)carbamoyl]-4,5,6,7-tetrahydropyrazolo[1,5-a]pyridin-4-yl]piperidine-4-carboxylate (580 mg, 1.17 mmol, 1 eq), 4,4,5,5-tetramethyl-2-(4,4,5,5-tetramethyl-1,3,2-dioxaborolan-2-yl)-1,3,2-dioxaborolane (891.20 mg, 3.51 mmol, 3 eq), palladium(ll) chloride complexed with 1,1'-bis(diphenylphosphino)ferrocene dichloromethane adduct (95.53 mg, 116.98 µmol, 0.1 eq), and potassium acetate (344.42 mg, 3.51 mmol, 3 eq) in dioxane (10 mL) was stirred under a nitrogen atmosphere at 90 °C for 12 hours. After completion of the reaction, the mixture was cooled to room temperature and filtered. The filtrate was concentrated under reduced pressure, and the residue was purified by prep-TLC on silica gel (Petroleum ether/Ethyl acetate = 1:1) to afford methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (635 mg, 80.9% yield) as a yellow solid.

LCMS m/z 543.2 [M+3]⁺.

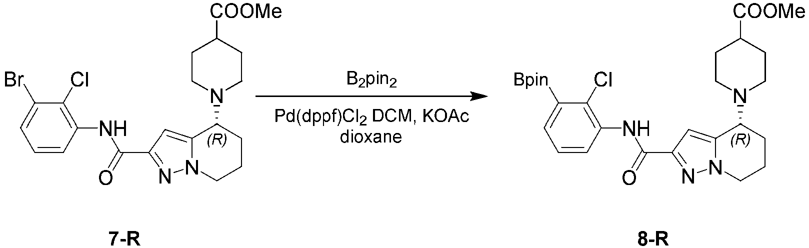

A mixture of methyl 1-[(4R)-2-[(3-bromo-2-chloro-phenyl)carbamoyl]-4,5,6,7-tetrahydropyrazolo[1,5-a]pyridin-4-yl]piperidine-4-carboxylate (640.00 mg, 1.29 mmol, 1 eq), 4,4,5,5-tetramethyl-2-(4,4,5,5-tetramethyl-1,3,2-dioxaborolan-2-yl)-1,3,2-dioxaborolane (983.39 mg, 3.87 mmol, 3 eq), Pd(dppf)Cl₂·CH₂Cl₂ (105.42 mg, 129.09 µmol, 0.1 eq), and potassium acetate (380.05 mg, 3.87 mmol, 3 eq) in dioxane (10 mL) was stirred under a nitrogen atmosphere at 90 °C for 12 hours. After completion of the reaction, the mixture was cooled to room temperature and filtered. The filtrate was concentrated under reduced pressure, and the residue was purified by prep-TLC on silica gel (Petroleum ether/Ethyl acetate = 1:1) to afford methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (1.24 g, 53.0% yield) as a black solid.

LCMS /z 543.2 [M+1]⁺.

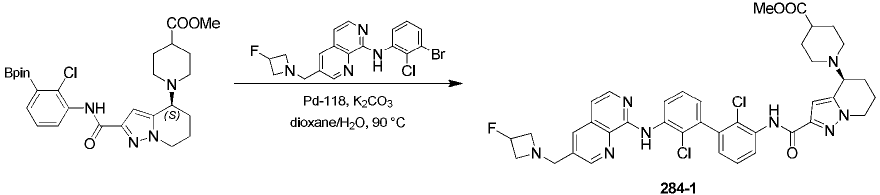

A mixture of N-(3-bromo-2-chloro-phenyl)-3-[(3-fluoroazetidin-1-yl)methyl]-1,7-naphthyridin-8-amine (100 mg, 237.14 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (128.73 mg, 237.14 µmol, 1 eq), and di-tert-butyl(cyclopentyl)phosphine;dichloropalladium;iron (15.46 mg, 23.71 µmol, 0.1 eq), and K₂CO₃ (98.32 mg, 711.42 µmol, 3 eq) in dioxane (8 mL) and water (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by prep-TLC on silica gel (Ethyl acetate/Methanol = 10:1) to afford Compound 284-1 (205 mg, 68.4% yield) as a yellow solid.

LCMS m/z 757.4 [M+1]⁺.

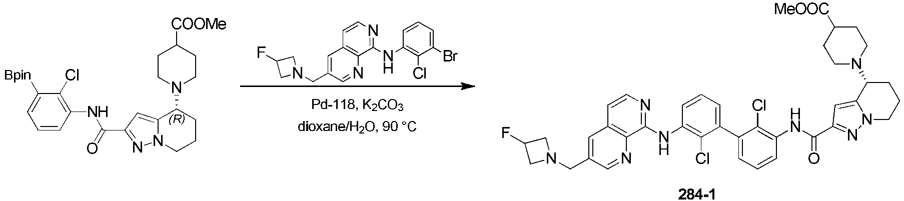

A mixture of N-(3-bromo-2-chloro-phenyl)-3-[(3-fluoroazetidin-1-yl)methyl]-1,7-naphthyridin-8-amine (100 mg, 237.14 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (429.11 mg, 237.14 µmol, 1 eq), di-tert-butyl(cyclopentyl)phosphine;dichloropalladium;iron (15.46 mg, 23.71 µmol, 0.1 eq), and K₂CO₃ (98.33 mg, 711.42 µmol, 3 eq) in dioxane (8 mL) and water (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by prep-TLC on silica gel (Ethyl acetate/Methanol = 10:1) to afford Compound 284-1 (234 mg, 78.14% yield) as a yellow solid.

LCMS m/z 757.2 [M+1]⁺.

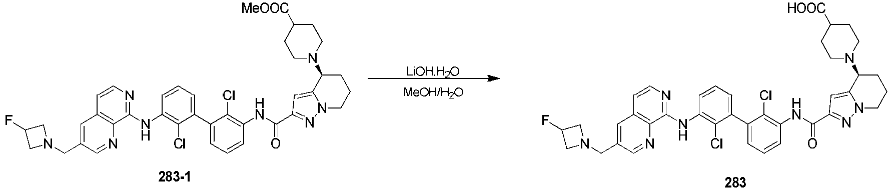

A mixture of methyl 1-[(4S)-2-[[2-chloro-3-[2-chloro-3-[[3-[(3-fluoroazetidin-1-yl)methyl]-1,7-naphthyridin-8-yl]amino]phenyl]phenyl]carbamoyl]-4,5,6,7-tetrahydropyrazolo[1,5-a]pyridin-4-yl]piperidine-4-carboxylate (200 mg, 263.96 µmol, 1 eq) and LiOH·H₃O (33.23 mg, 791.89 µmol, 3 eq) in a mixture of water (4 mL) and methanol (6 mL) was stirred at room temperature for 12 hours. After completion of the reaction, the mixture was concentrated under reduced pressure. The resulting residue was purified by reversed-phase HPLC (column: Waters XBridge, 150 * 25 mm * 5 µm; mobile phase: [water (NH₄HCO₃)-ACN]; gradient: 36% to 66% acetonitrile over 20 min) to afford Compound 283 (35 mg, 17.6% yield) as a yellow solid.

LCMS m/z 745.2 [M+3]⁺.

¹H NMR (400 MHz, DMSO-*d*₆) δ ppm 1.48 - 1.74 (m, 4 H) 1.77 - 2.03 (m, 5 H) 2.11 - 2.35 (m, 5 H) 2.64 - 2.69 (m, 1 H) 2.80 (br dd, *J*=8.94, 5.44 Hz, 1 H) 3.60 - 3.69 (m, 2 H) 3.90 (s, 2 H) 3.94 - 4.11 (m, 2 H) 4.18 - 4.26 (m, 1 H) 5.11 - 5.32 (m, 1 H) 6.68 (s, 1 H) 7.04 (d, *J*=7.50 Hz, 1 H) 7.18 - 7.23 (m, 1 H) 7.36 (d, *J*=5.88 Hz, 1 H) 7.52 (q, *J*=8.05 Hz, 2 H) 8.20 (d, *J*=5.75 Hz, 1 H) 8.25 (d, *J*=1.75 Hz, 1 H) 8.27 - 8.32 (m, 1 H) 8.89 (d, *J*=1.88 Hz, 1 H) 9.14 (dd, *J*=8.32, 1.31 Hz, 1 H) 9.55 (s, 1 H) 9.88 (s, 1 H).

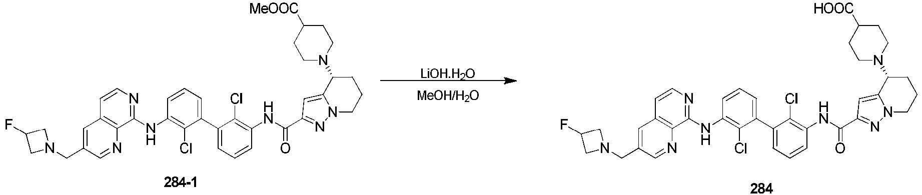

To a solotion of methyl 1-[(4R)-2-[[2-chloro-3-[2-chloro-3-[[3-[(3-fluoroazetidin-1-yl)methyl]-1,7-naphthyridin-8-yl]amino]phenyl]phenyl]carbamoyl]-4,5,6,7-tetrahydropyrazolo[1,5-a]pyridin-4-yl]piperidine-4-carboxylate (200 mg, 263.96 µmol, 1 eq) in water (4 mL) and methanol (6 mL), LiOH·H₂O (33.23 mg, 791.89 µmol, 3 eq) was added, and the reaction mixutre was stirred at room temperature for 12 hours. After completion of the reaction, the mixture was concentrated under reduced pressure. The resulting residue was purified by reversed-phase HPLC (column: Waters XBridge, 150 * 25 mm * 5 µm; mobile phase: [water (NH₄HCO₃)-ACN]; gradient: 36% to 66% acetonitrile over 20 min) to afford Compound 284 (33 mg, 16.6% yield) as a yellow solid.

LCMS (m/z 743.1 [M+1]⁺.

¹H NMR (400 MHz, DMSO-*d*₆) δ ppm 1.47 - 1.74 (m, 4 H) 1.77 - 2.03 (m, 5 H) 2.10 - 2.35 (m, 5 H) 2.66 - 2.69 (m, 1 H) 2.76 - 2.84 (m, 1 H) 3.59 - 3.70 (m, 2 H) 3.90 (s, 2 H) 3.93 - 4.11 (m, 2 H) 4.17 - 4.26 (m, 1 H) 5.12 - 5.33 (m, 1 H) 6.68 (s, 1 H) 7.04 (d, *J*=7.63 Hz, 1 H) 7.20 (d, *J*=7.75 Hz, 1 H) 7.36 (d, *J*=5.75 Hz, 1 H) 7.46 - 7.56 (m, 2 H) 8.20 (d, *J*=5.88 Hz, 1 H) 8.24 (s, 1 H) 8.26 - 8.33 (m, 1 H) 8.89 (d, *J*=1.75 Hz, 1 H) 9.13 (d, *J*=7.25 Hz, 1 H) 9.55 (s, 1 H) 9.88 (s, 1 H).

### [Preparation Example 85]

### Preparation of Compound 285, (S)-N-(2,2'-dimethyl-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide, amd Compound 286, (S)-N-(2,2'-dimethyl-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

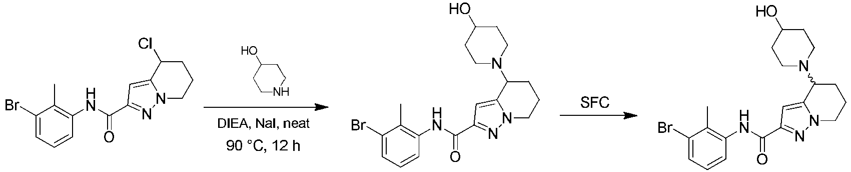

A mixture of N-(3-bromo-2-methylphenyl)-4-chloro-4,5,6,7-tetrahydropyrazolo[1,5-a]pyridine-2-carboxamide (6.68 g, 18.12 mmol, 1 eq) and piperidin-4-ol (9.16 g, 90.60 mmol, 5 eq) was treated with N,N-diisopropylethylamine (7.03 g, 54.36 mmol, 9.47 mL, 3 eq) and sodium iodide (543.21 mg, 3.62 mmol, 0.2 eq). The resulting mixture was stirred at 90 °C for 12 hours. After completion of the reaction, the mixture was cooled to room temperature and diluted with aq. saturated ammonium chloride solution (100 mL). The aqueous layer was extracted with ethyl acetate (150 mL × 3). The combined organic layers were washed with brine (50 mL), dried over Na2SO4, filtered, and concentrated under reduced pressure. The resulting residue was purified by column chromatography (SiO₂, Petroleum ether/Ethyl acetate = 0:1 to 1:0), followed by separation by SFC (column: DAICEL CHIRALPAK AS, 250 mm × 30 mm, 10 µm; mobile phase: carbon dioxide and ethanol containing 0.1% aqueous ammonia; B = 45%, isocratic elution mode) to afford methyl (4S)-N-(3-bromo-2-methylphenyl)-4-(4-hydroxy-1-piperidyl)-4,5,6,7-tetrahydropyrazolo[1,5-a]pyridine-2-carboxamide (1.4 g, 44.71% yield) and methyl (4R)-N-(3-bromo-2-methylphenyl)-4-(4-hydroxy-1-piperidyl)-4,5,6,7-tetrahydropyrazolo[1,5-a]pyridine-2-carboxamide (1.3 g, 41.5% yield), each obtained as a pink solid.

LCMS m/z 470.9 [M+3]⁺,

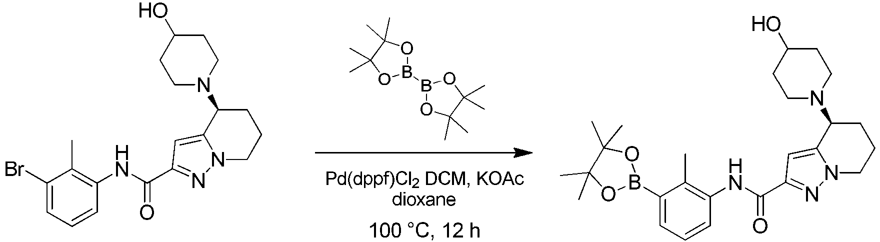

A mixture of (4S)-N-(3-bromo-2-methylphenyl)-4-(4-hydroxy-1-piperidyl)-4,5,6,7-tetrahydropyrazolo[1,5-a]pyridine-2-carboxamide (400 mg, 923.06 µmol, 1 eq), 4,4,5,5-tetramethyl-2-(4,4,5,5-tetramethyl-1,3,2-dioxaborolan-2-yl)-1,3,2-dioxaborolane (703.20 mg, 2.77 mmol, 3 eq), Pd(dppf)Cl₂·CH₂Cl₂ (75.38 mg, 92.31 µmol, 0.1 eq), and potassium acetate (271.77 mg, 2.77 mmol, 3 eq) in dioxane (15 mL) was stirred under a nitrogen atmosphere at 100 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 1:0 to 0:1) to afford (4S)-4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (400 mg, 67.6% yield) as a black oil.

LCMS m/z 480.29 [M+1]⁺.

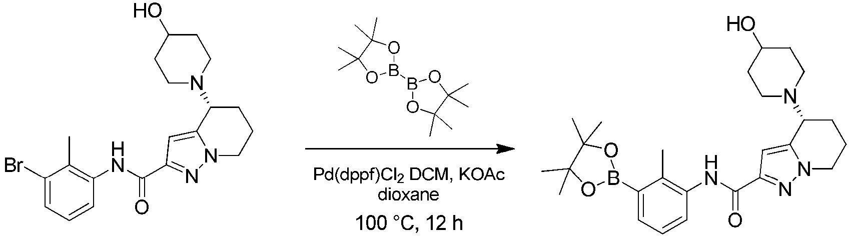

A mixture of (4R)-N-(3-bromo-2-methylphenyl)-4-(4-hydroxy-1-piperidyl)-4,5,6,7-tetrahydropyrazolo[1,5-a]pyridine-2-carboxamide (400 mg, 923.06 µmol, 1 eq), 4,4,5,5-tetramethyl-2-(4,4,5,5-tetramethyl-1,3,2-dioxaborolan-2-yl)-1,3,2-dioxaborolane (703.20 mg, 2.77 mmol, 3 eq), Pd(dppf)Cl₂·CH₂Cl₂ (75.38 mg, 92.31 µmol, 0.1 eq), and potassium acetate (271.77 mg, 2.77 mmol, 3 eq) in dioxane (15 mL) was stirred under a nitrogen atmosphere at 100 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 1/0 to 0/1) to afford (4R)-4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (400 mg, 63.1% yield) as a black oil.

LCMS (m/z 480.29 [M+1]⁺.

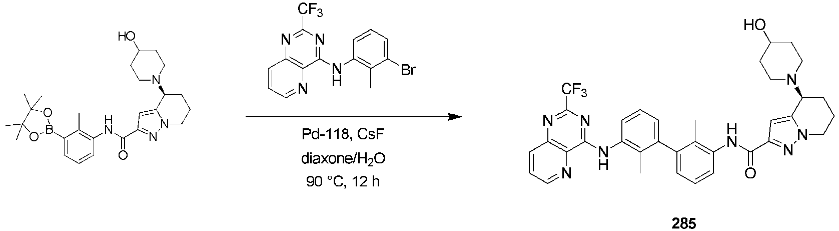

A mixture of N-(3-bromo-2-methylphenyl)-2-(trifluoromethyl)pyrido[3,2-d]pyrimidin-4-amine (130 mg, 339.28 µmol, 1 eq), (4R)-4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (211.89 mg, 441.06 µmol, 1.3 eq), CsF (154.61 mg, 1.02 mmol, 3 eq), and di-tert-butyl(cyclopenta-1,4-dien-1-yl)phosphane;palladium;iron (22.11 mg, 33.93 µmol, 0.1 eq) in a mixture of dioxane (8 mL) and water (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 0/1 to 1/0), followed by further purification by reversed-phase HPLC (column: Waters XBridge, 150 * 25 mm * 5 µm; mobile phase: [water (NH₄HCO₃)-ACN]; gradient: 40% to 70% acetonitrile over 15 min) to afford Compound 285 (21 mg, 60.0% yield) as a white solid.

LCMS m/z 656.28 [M+1]⁺.

¹H NMR (400 MHz, DMSO-d₆) δ = 11.81 (br s, 1H), 10.66 (br s, 1H), 10.21 (br d, J = 2.4 Hz, 1H), 9.45 (s, 1H), 9.20 - 9.13 (m, 1H), 8.78 - 8.69 (m, 2H), 8.55 - 8.47 (m, 1H), 8.46 - 8.38 (m, 1H), 8.23 (br d, J = 7.5 Hz, 1H), 8.15 (br d, J = 7.2 Hz, 1H), 7.75 (s, 1H), 5.68 (br d, J = 2.0 Hz, 1H), 5.34 (br d, J = 12.1 Hz, 1H), 5.19 (br t, J = 9.7 Hz, 1H), 5.09 (br d, J = 4.3 Hz, 1H), 4.65 - 4.56 (m, 1H), 4.46 (s, 4H), 3.92 - 3.77 (m, 2H), 3.59 - 3.51 (m, 1H), 3.47 - 3.37 (m, 1H), 3.28 (br s, 1H), 3.15 - 3.15 (m, 1H), 3.14 - 3.11 (m, 3H), 3.07 (s, 3H), 2.60 - 2.50 (m, 2H)

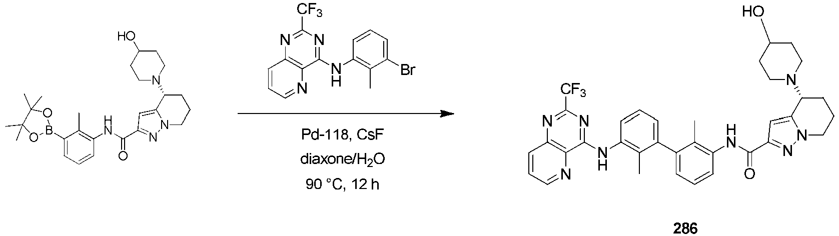

A mixture of N-(3-bromo-2-methylphenyl)-2-(trifluoromethyl)pyrido[3,2-d]pyrimidin-4-amine (130 mg, 339.28 µmol, 1 eq), (4R)-4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (211.89 mg, 441.06 µmol, 1.3 eq), CsF (154.61 mg, 1.02 mmol, 3 eq), and di-tert-butyl(cyclopenta-1,4-dien-1-yl)phosphane;palladium;iron (22.11 mg, 33.93 µmol, 0.1 eq) in a mixture of dioxane (8 mL) and water (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 0/1 to 1/0), followed by further purification by reversed-phase HPLC (column: Waters XBridge, 150 * 25 mm * 5 µm; mobile phase: [water (NH₄HCO₃)-ACN]; gradient: 40% to 70% acetonitrile over 15 min) to afford Compound 286 (33 mg, 14.6% yield) as a yellow solid.

LCMS m/z 656.28 [M+1]⁺.

¹H NMR (400 MHz, DMSO-d6) δ = 11.43 (br s, 1H), 10.30 (br s, 1H), 9.86 - 9.80 (m, 1H), 9.17 - 9.13 (m, 1H), 8.80 (br dd, J = 3.9, 8.1 Hz, 1H), 8.38 - 8.32 (m, 2H), 8.14 (t, J = 7.7 Hz, 1H), 8.06 (t, J = 7.7 Hz, 1H), 7.89 - 7.83 (m, 1H), 7.78 (br d, J = 7.5 Hz, 1H), 7.43 - 7.33 (m, 1H), 5.32 (br d, J = 3.3 Hz, 1H), 4.97 (br d, J = 12.5 Hz, 1H), 4.87 - 4.77 (m, 1H), 4.73 (br dd, J = 5.1, 9.5 Hz, 1H), 4.24 (br d, J = 3.3 Hz, 1H), 4.10 (s, 4H), 3.56 - 3.40 (m, 2H), 3.18 (br t, J = 10.0 Hz, 1H), 3.05 (br t, J = 10.1 Hz, 1H), 2.92 (br d, J = 12.1 Hz, 1H), 2.76 (s, 4H), 2.71 (s, 3H), 2.23 - 2.13 (m, 2H).

### [Preparation Example 86]

### Preparation of Compound 287, (S)-N-(3'-((2-(difluoromethyl)pyrido[3,2-d]pyrimidin-4-yl)amino)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide, and Compound 288, (R)-N-(3'-((2-(difluoromethyl)pyrido[3,2-d]pyrimidin-4-yl)amino)-2,2'-dimethyl-[1,1'-biphenyl]-3-yl)-4-(4-hydroxypiperidin-1-yl)-4,5,6,7-tetrahydropyrazolo[1,5-a]pyridine-2-carboxamide

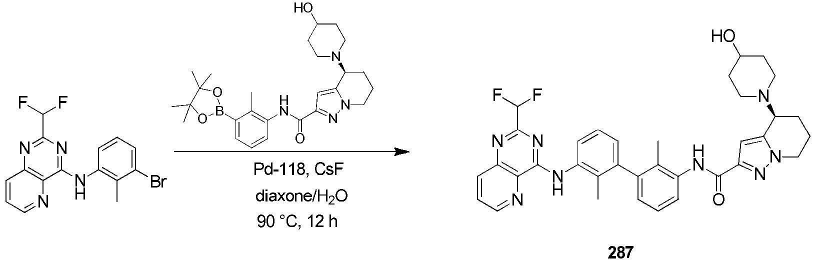

A mixture of N-(3-bromo-2-methylphenyl)-2-(difluoromethyl)pyrido[3,2-d]pyrimidin-4-amine (120 mg, 328.61 µmol, 1 eq), (4S)-4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (205.23 mg, 427.19 µmol, 1.3 eq), CsF (149.75 mg, 985.83 µmol, 3 eq), and di-tert-butyl(cyclopenta-1,4-dien-1-yl)phosphane;palladium;iron (21.42 mg, 32.86 µmol, 0.1 eq) in a mixture of dioxane (4 mL)/ H₂O (1 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate=3/1 to 1/1), followed by further purification by reversed-phase HPLC (column: Waters XBridge, 150 * 25 mm * 5 µm; mobile phase: [water (NH₄HCO₃)-ACN]; gradient: 40% to 70% acetonitrile over 9 min) to afford Compound 287 (70 mg, 33.0% yield) as a white solid.

LCMS m/z 638.29 [M+1]⁺

¹H NMR (400 MHz, DMSO-*d₆*) δ = 10.42 (s, 1H), 9.53 (d, *J* = 4.4 Hz, 1H), 9.03 (dd, *J* = 1.4, 4.3 Hz, 1H), 8.33 (dd, *J* = 1.4, 8.5 Hz, 1H), 8.02 - 7.98 (m, 1H), 7.69 (d, *J* = 8.0 Hz, 1H), 7.58 (dd, *J =* 4.8, 7.3 Hz, 1H), 7.40 - 7.33 (m, 1H), 7.32 - 7.25 (m, 1H), 7.08 (d, *J* = 7.3 Hz, 1H), 7.02 (d, *J* = 6.9 Hz, 1H), 6.88 - 6.72 (m, 1H), 6.63 - 6.59 (m, 1H), 4.56 (d, *J* = 4.1 Hz, 1H), 4.25 - 4.16 (m, 1H), 4.10 - 4.01 (m, 1H), 3.99 - 3.91 (m, 1H), 3.52 - 3.42 (m, 1H), 2.79 - 2.62 (m, 2H), 2.48 - 2.38 (m, 2H), 2.31 - 2.23 (m, 1H), 2.21 - 2.09 (m, 1H), 2.00 (s, 3H), 1.96 (s, 3H), 1.93 - 1.83 (m, 1H), 1.79 - 1.65 (m, 3H), 1.47 (br s, 2H).

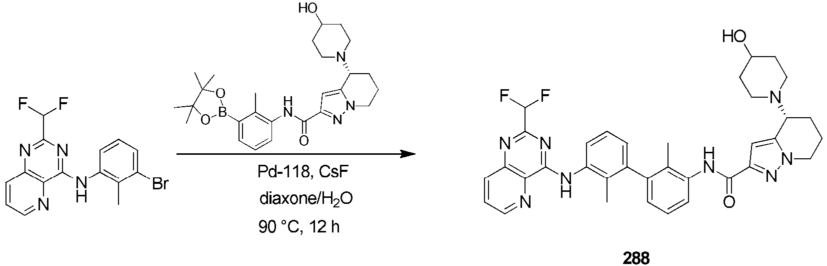

A mixture of N-(3-bromo-2-methylphenyl)-2-(difluoromethyl)pyrido[3,2-d]pyrimidin-4-amine (100 mg, 273.84 µmol, 1 eq), (4R)-4-(4-hydroxy-1-piperidyl)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (171.02 mg, 355.99 µmol, 1.3 eq), CsF (124.79 mg, 821.52 µmol, 3 eq), and di-tert-butyl(cyclopenta-1,4-dien-1-yl)phosphane;palladium;iron (17.85 mg, 27.38 µmol, 0.1 eq) in a mixture of dioxane (8 mL) and water (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 3/1 to 1/1), followed by further purification by reversed-phase HPLC (column: Waters XBridge, 150 * 25 mm * 5 µm; mobile phase: [water (NH₄HCO₃)-ACN]; gradient: 40% to 70% acetonitrile over 9 min) to afford Compound 288 (70 mg, 39.6% yield) as a white solid.

LCMS m/z 638.29 [M+1]⁺.

¹H NMR (400 MHz, DMSO-*d₆*) δ = 10.42 (s, 1H), 9.56 - 9.48 (m, 1H), 9.05 - 9.00 (m, 1H), 8.37 - 8.28 (m, 1H), 8.02 - 7.98 (m, 1H), 7.69 (d, *J* = 8.0 Hz, 1H), 7.58 (dd, *J* = 4.8, 7.6 Hz, 1H), 7.40 - 7.33 (m, 1H), 7.31 - 7.26 (m, 1H), 7.10 - 7.05 (m, 1H), 7.01 (s, 1H), 6.90 - 6.71 (m, 1H), 6.65 - 6.57 (m, 1H), 4.56 (d, *J* = 4.0 Hz, 1H), 4.24 - 4.18 (m, 1H), 4.10 - 4.01 (m, 1H), 3.99 - 3.93 (m, 1H), 3.47 (br dd, *J =* 3.9, 8.0 Hz, 1H), 2.78 - 2.64 (m, 2H), 2.45 - 2.37 (m, 1H), 2.32 - 2.23 (m, 1H), 2.20 - 2.12 (m, 1H), 2.00 (s, 4H), 1.98 - 1.93 (m, 4H), 1.90 - 1.83 (m, 1H), 1.74 - 1.65 (m, 2H), 1.47 - 1.33 (m, 2H)

### [Preparation Example 87]

### Preparation of Compound 289, (S)-1-(2-((2,2'-dichloro-3'-((2-(difluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid, and Compound 290, (S)-1-(2-((2,2'-dichloro-3'-((2-(difluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid

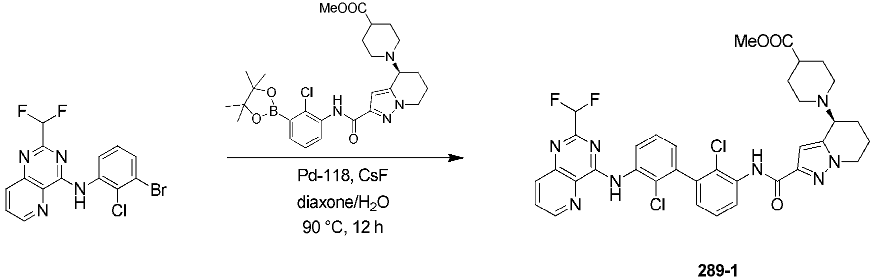

A mixture of (S)-methyl 1-(2-((2-chloro-3-(4,4,5,5-tetramethyl-1,3,2-dioxaborolan-2-yl)phenyl)carbamoyl)-4,5,6,7-tetrahydropyrazolo[1,5-a]pyridin-4-yl)piperidine-4-carboxylate (183.02 mg, 337.14 µmol, 1.3 eq), N-(3-bromo-2-chloro-phenyl)-2-(difluoromethyl)pyrido[3,2-d]pyrimidin-4-amine (100 mg, 259.34 µmol, 1 eq), CsF (118.18 mg, 778.02 µmol, 3 eq), and di-tert-butyl(cyclopentyl)phosphane;palladium;iron (16.90 mg, 25.93 µmol, 0.1 eq) in a mixture of dioxane (8 mL)/H₂O (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 1:0 to 0:1) to afford Compound 289-1 (115 mg, 40.56% yield) as a white solid.

LCMS m/z 720.19 [M+1]⁺. 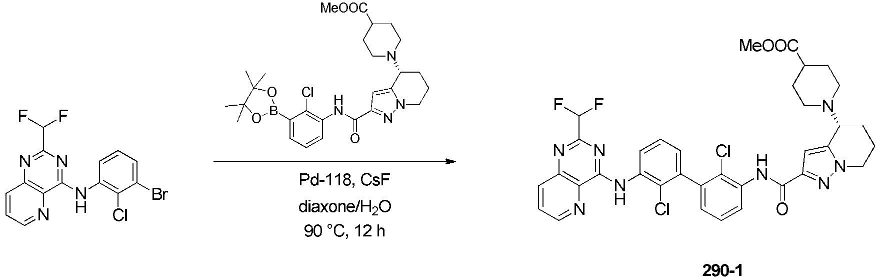 A mixture of N-(3-bromo-2-chloro-phenyl)-2-(difluoromethyl)pyrido[3,2-d]pyrimidin-4-amine (100 mg, 259.34 µmol, 1 eq), (R)-methyl 1-(2-((2-chloro-3-(4,4,5,5-tetramethyl-1,3,2-dioxaborolan-2-yl)phenyl)carbamoyl)-4,5,6,7-tetrahydropyrazolo[1,5-a]pyridin-4-yl)piperidine-4-carboxylate (183.02 mg, 337.14 µmol, 1.3 eq), CeF (118.18 mg, 778.02 µmol, 3 eq), and di-tert-butyl(cyclopentyl)phosphane;palladium;iron (16.90 mg, 25.93 µmol, 0.1 eq) in a mixture of dioxane (4 mL) / H₂O (1 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 1:0 to 0:1) to afford Compound 290-1 (85 mg, 37.7% yield) as a white solid.

LCMS m/z 720.19 [M+1]⁺.

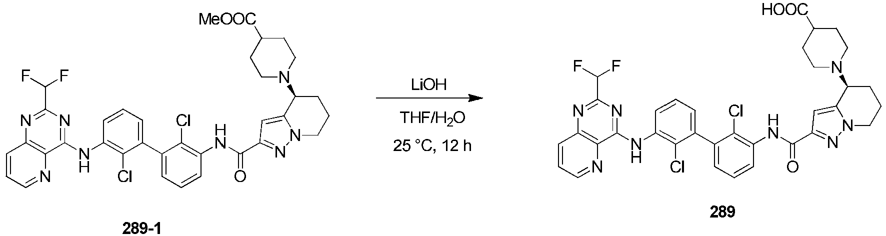

A mixture of (S)-methyl 1-(2-((2,2'-dichloro-3'-((2-(difluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylate (100 mg, 138.58 µmol, 1 eq) and lithium hydroxide (9.96 mg, 415.75 µmol, 3 eq) in THF (2.5 mL) / H₂O (0.5 mL) was stirred at room temperature for 12 hours. After completion of the reaction, the mixture was concentrated under reduced pressure. The resulting residue was purified by reversed-phase HPLC (column: Waters XBridge, 150 * 25 mm * 5 µm; mobile phase: [H₂O (NH₄HCO₃)-ACN]; gradient: 25% to 55% B over 9 min) to afford Compound 289 (23 mg, 23.2% yield) as a white solid.

LCMS m/z 706.18[M+1]⁺.

¹H NMR (400 MHz, DMSO-d₆) δ = 9.58 (s, 1H), 9.10 (dd, J = 1.3, 4.3 Hz, 1H), 8.64 - 8.61 (m, 1H), 8.44 (dd, J = 1.4, 8.5 Hz, 1H), 8.31 - 8.28 (m, 1H), 8.07 (dd, J = 4.2, 8.5 Hz, 1H), 7.64 - 7.62 (m, 1H), 7.53 (t, J = 7.9 Hz, 1H), 7.27 (d, J = 7.7 Hz, 1H), 7.22 (d, J = 6.5 Hz, 1H), 6.92 (t, J = 54.3 Hz, 1H), 6.69 (s, 1H), 4.27 - 4.18 (m, 1H), 4.13 - 4.05 (m, 1H), 3.99 (br dd, J = 5.2, 10.3 Hz, 1H), 4.01 - 3.94 (m, 1H), 2.81 (br d, J = 10.6 Hz, 1H), 2.69 (br s, 1H), 2.35 - 2.24 (m, 2H), 2.23 - 2.12 (m, 2H), 2.03 - 1.91 (m, 2H), 1.91 - 1.79 (m, 3H), 1.71 - 1.52 (m, 3H)

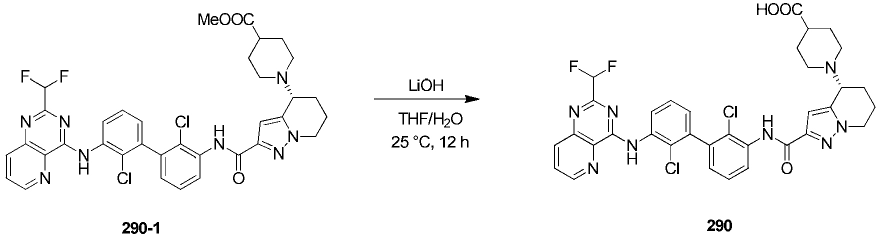

To a mixture of (R)-methyl 1-(2-((2,2'-dichloro-3'-((2-(difluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylate 290-1 (85 mg, 117.80 µmol, 1 eq) in THF (2.5 mL) / H₂O (0.5 mL), lithium hydroxide (8.46 mg, 353.39 µmol, 3 eq) was added, and the reaction mixture was stirred at room temperature for 12 hours. After completion of the reaction, the mixture was concentrated under reduced pressure. The resulting residue was purified by reversed-phase HPLC (column: Waters XBridge, 150 * 25 mm * 5 µm; mobile phase: [H₂O (NH₄HCO₃)-ACN]; gradient: 25% to 55% B over 9 min) to afford Compound 290 (34 mg, 39.9% yield) as a white solid.

LCMS m/z 706.18 [M+1]⁺.

¹H NMR (400 MHz, DMSO-d₆) δ = 9.97 (s, 1H), 9.49 (d, J = 3.3 Hz, 1H), 9.01 (d, J = 7.9 Hz, 1H), 8.83 (dd, J = 1.2, 8.4 Hz, 1H), 8.68 (dd, J = 4.5, 7.0 Hz, 1H), 8.46 (dd, J = 4.3, 8.6 Hz, 1H), 8.01 (t, J = 7.9 Hz, 1H), 7.92 (t, J = 8.0 Hz, 1H), 7.66 (d, J = 7.6 Hz, 1H), 7.61 (d, J = 7.1 Hz, 1H), 7.47 - 7.15 (m, 1H), 7.08 (s, 1H), 4.66 - 4.56 (m, 1H), 4.52 - 4.42 (m, 1H), 4.38 (br dd, J = 5.4, 10.1 Hz, 1H), 3.24 - 3.17 (m, 1H), 3.09 - 3.03 (m, 1H), 2.89 - 2.81 (m, 2H), 2.77 - 2.63 (m, 2H), 2.62 - 2.50 (m, 2H), 2.42 - 2.29 (m, 2H), 2.27 - 2.19 (m, 2H), 2.06 - 1.88 (m, 3H)

### [Preparation Example 88]

### Preparation of Compound 291, (S)-1-(2-((3'-((2-(difluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid, and Compound 292, (R)-1-(2-((3'-((2-(difluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

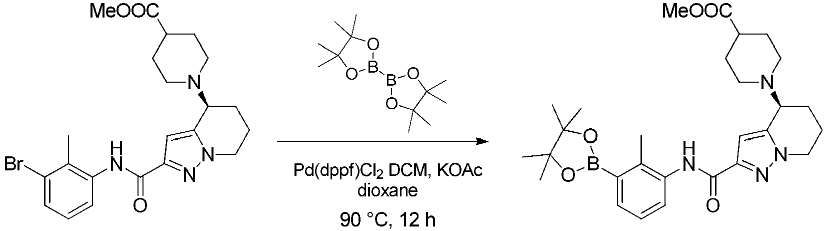

A mixture of 4,4,5,5-tetramethyl-2-(4,4,5,5-tetramethyl-1,3,2-dioxaborolan-2-yl)-1,3,2-dioxaborolane (961.53 mg, 3.79 mmol, 3 eq), (S)-methyl 1-(2-((3-bromo-2-methylphenyl)carbamoyl)-4,5,6,7-tetrahydropyrazolo[1,5-a]pyridin-4-yl)piperidine-4-carboxylate (600 mg, 1.26 mmol, 1 eq), Pd(dppf)Cl•CH₂Cl₂ (103.07 mg, 126.22 µmol, 0.1 eq), and potassium acetate (371.61 mg, 3.79 mmol, 3 eq) in dioxane (15 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 0:1 to 1:0) to afford (S)-methyl 1-(2-((2-methyl-3-(4,4,5,5-tetramethyl-1,3,2-dioxaborolan-2-yl)phenyl)carbamoyl)-4,5,6,7-tetrahydropyrazolo[1,5-a]pyridin-4-yl)piperidine-4-carboxylate (676 mg, 90.2% yield) as a black liquid.

LCMS 522.3 [M+1]⁺.

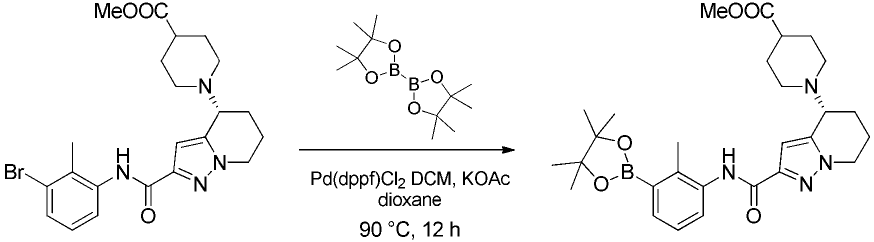

A mixture of 4,4,5,5-tetramethyl-2-(4,4,5,5-tetramethyl-1,3,2-dioxaborolan-2-yl)-1,3,2-dioxaborolane (961.53 mg, 3.79 mmol, 3 eq), (R)-methyl 1-(2-((3-bromo-2-methylphenyl)carbamoyl)-4,5,6,7-tetrahydropyrazolo[1,5-a]pyridin-4-yl)piperidine-4-carboxylate (600 mg, 1.26 mmol, 1 eq), Pd(dppf)Cl•CH₂Cl₂ (103.07 mg, 126.22 µmol, 0.1 eq), and potassium acetate (371.60 mg, 3.79 mmol, 3 eq) in dioxane (15 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 1:0 to 0:1) to afford (R)-methyl 1-(2-((2-methyl-3-(4,4,5,5-tetramethyl-1,3,2-dioxaborolan-2-yl)phenyl)carbamoyl)-4,5,6,7-tetrahydropyrazolo[1,5-a]pyridin-4-yl)piperidine-4-carboxylate (600 mg, 79.1% yield) as a black oil.

LCMS m/z 522.3 [M+1]⁺.

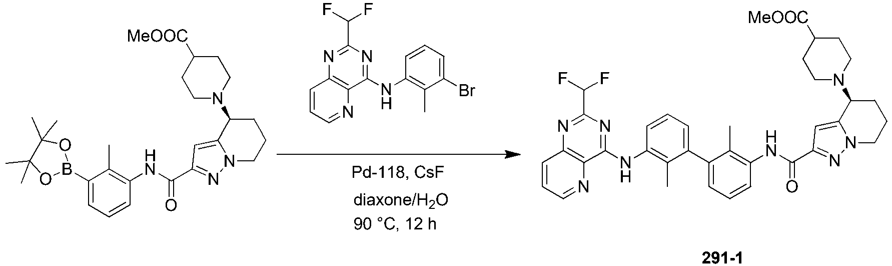

A mixture of (S)-methyl 1-(2-((2-methyl-3-(4,4,5,5-tetramethyl-1,3,2-dioxaborolan-2-yl)phenyl)carbamoyl)-4,5,6,7-tetrahydropyrazolo[1,5-a]pyridin-4-yl)piperidine-4-carboxylate (185.99 mg, 355.99 µmol, 1.3 eq), N-(3-bromo-2-methylphenyl)-2-(difluoromethyl)pyrido[3,2-d]pyrimidin-4-amine (100 mg, 273.84 µmol, 1 eq), CsF (124.79 mg, 821.52 µmol, 3 eq), and di-tert-butyl(cyclopentyl)phosphane;dichloropalladium;iron (17.85 mg, 27.38 µmol, 0.1 eq) in dioxane (8 mL)/H₂O (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 1:0 to 0:1) to afford Compound 291-1 (200 mg, 59.0% yield) as a brown solid.

LCMS m/z 680.3 [M+1]⁺.

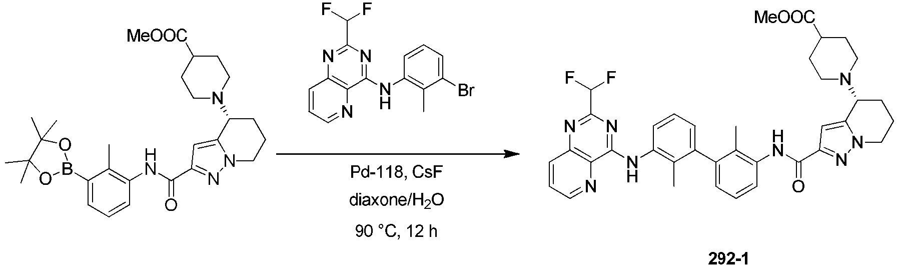

A mixture of (R)-methyl 1-(2-((2-methyl-3-(4,4,5,5-tetramethyl-1,3,2-dioxaborolan-2-yl)phenyl)carbamoyl)-4,5,6,7-tetrahydropyrazolo[1,5-a]pyridin-4-yl)piperidine-4-carboxylate (185.99 mg, 355.99 µmol, 1.3 eq), N-(3-bromo-2-methylphenyl)-2-(difluoromethyl)pyrido[3,2-d]pyrimidin-4-amine (100 mg, 273.84 µmol, 1 eq), CsF (124.79 mg, 821.52 µmol, 3 eq), and di-tert-butyl(cyclopentyl)phosphane;dichloropalladium;iron (17.85 mg, 27.38 µmol, 0.1 eq) in dioxane (8 mL)/H₂O (2 mL) was stirred under a nitrogen atmosphere at 90 °C for 12 hours. After completion of the reaction, the mixture was cooled to room temperature and filtered. The filtrate was concentrated under reduced pressure, and the resulting residue was purified by column chromatography (SiO₂, Petroleum ether/Ethyl acetate = 0:1 to 1:0) to afford Compound 292-1 (170 mg, 74.7% yield) as a brown solid.

LCMS m/z 680.3 [M+1]⁺.

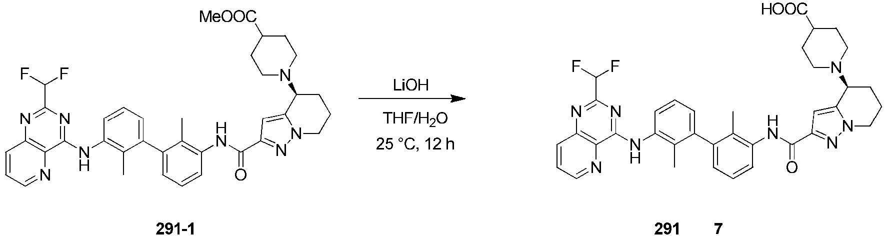

A solution of (S)-methyl 1-(2-((3'-((2-(difluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylate (200 mg, 293.80 µmol, 1 eq) in THF (5 mL) and water (1 mL) was treated with lithium hydroxide (21.11 mg, 881.39 µmol, 3 eq), and the resulting mixture was stirred at room temperature for 12 hours. After completion of the reaction, the mixture was concentrated under reduced pressure, and the resulting residue was purified by prep-HPLC (column: Phenomenex Luna C18, 150 * 25 mm * 10 µms; mobile phase: [H₂O(HCl)-ACN]; gradient: 23% to 53% B over 10 min) to afford Compound 291 (43 mg, 21.5% yield) as a white solid.

LCMS m/z 666.29 [M+1]⁺.

¹H NMR (400 MHz, DMSO-d₆) δ = 11.60 - 11.06 (m, 1H), 10.43 (s, 1H), 9.69 (d, J = 5.6 Hz, 1H), 9.02 (dd, J = 1.3, 4.2 Hz, 1H), 8.32 (dd, J = 1.4, 8.4 Hz, 1H), 7.99 (dd, J = 4.3, 8.4 Hz, 1H), 7.67 (d, J = 7.9 Hz, 1H), 7.55 - 7.49 (m, 1H), 7.45 (br s, 1H), 7.36 (t, J = 7.8 Hz, 1H), 7.30 (t, J = 7.8 Hz, 1H), 7.07 (d, J = 7.3 Hz, 1H), 7.03 (d, J = 7.4 Hz, 1H), 6.72 (s, 1H), 6.72 (d, J = 108.9 Hz, 1H), 4.90 (br s, 2H), 4.29 - 4.21 (m, 3H), 3.52 - 3.41 (m, 1H), 3.53 - 3.38 (m, 1H), 3.27 - 3.05 (m, 3H), 2.55 (br s, 1H), 2.33 - 2.25 (m, 2H), 2.21 - 2.08 (m, 2H), 2.00 (s, 3H), 1.95 (s, 3H)

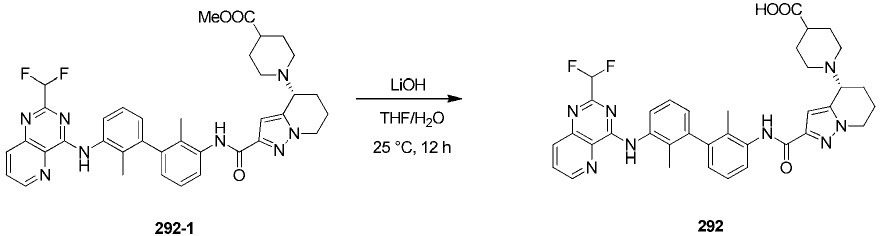

A solution of (R)-methyl 1-(2-((3'-((2-(difluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylate (170 mg, 249.73 µmol, 1 eq) in THF (5 mL) and water (1 mL) was treated with lithium hydroxide (17.94 mg, 749.18 µmol, 3 eq), and the resulting mixture was stirred at room temperature for 12 hours. After completion of the reaction, the mixture was concentrated under reduced pressure, and the resulting residue was purified by prep-HPLC (column: Phenomenex Luna C18, 150 × 25 mm, 10 µms; mobile phase: [H₂O(HCl)-ACN]; gradient: 23% to 53% B over 10 min) to afford Compound 292 (53 mg, 31.5% yield) as a yellow solid.

LCMS m/z 666.29 [M+1]⁺.

¹H NMR (400 MHz, DMSO-d₆) δ = 11.94 - 10.99 (m, 1H), 10.44 (s, 1H), 9.70 (d, J = 5.6 Hz, 1H), 9.03 (dd, J = 1.4, 4.3 Hz, 1H), 8.33 (dd, J = 1.4, 8.4 Hz, 1H), 8.00 (dd, J = 4.3, 8.5 Hz, 1H), 7.69 (d, J = 7.8 Hz, 1H), 7.57 - 7.51 (m, 1H), 7.47 (br s, 1H), 7.37 (t, J = 7.8 Hz, 1H), 7.31 (t, J = 7.7 Hz, 1H), 7.08 (d, J = 7.0 Hz, 1H), 7.04 (d, J = 7.5 Hz, 1H), 6.73 (s, 1H), 6.73 (d, J = 109.1 Hz, 1H), 4.91 (br s, 1H), 4.21 (br s, 1H), 4.27 - 4.21 (m, 2H), 4.30 - 4.17 (m, 1H), 3.55 - 3.46 (m, 1H), 3.31 - 3.09 (m, 4H), 2.65 - 2.56 (m, 1H), 2.34 - 2.27 (m, 2H), 2.04 (br s, 2H), 2.01 (s, 3H), 1.96 (s, 3H)

### [Preparation Example 89]

### Preparation of Compound 293, 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetic acid, and Compound 294, 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetic acid,

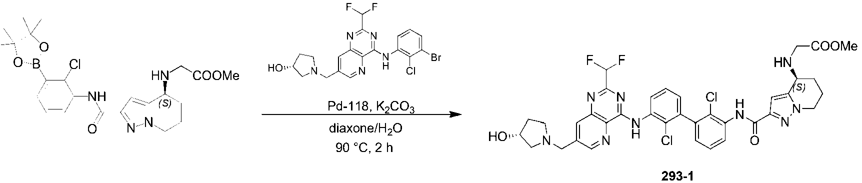

A mixture of methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (181.50 mg, 371.35 µmol, 1.5 eq), (3R)-1-[[4-(3-bromo-2-chloro-anilino)-2-(difluoromethyl)pyrido[3,2-d]pyrimidin-7-yl]methyl]pyrrolidin-3-ol (120 mg, 247.56 µmol, 1 eq), CsF (112.82 mg, 742.69 µmol, 3 eq), and di-tert-butyl(cyclopentyl)phosphane;dichloropalladium;iron (16.13 mg, 24.76 µmol, 0.1 eq) in dioxane (1.6 mL) and water (0.4 mL) was stirred under a nitrogen atmosphere at 90 °C for 12 hours. After completion of the reaction, the mixture was cooled to room temperature and filtered. The filtrate was concentrated under reduced pressure, and the resulting residue was purified by prep-HPLC (column: Phenomenex Luna C18, 150 * 25 mm * 10 µms; mobile phase: [H₂O (HCl)-ACN]; gradient: 13% to 43% B over 10 min) to afford Compound 293-1 (130 mg, 68.5% yield) as a yellow solid.

LCMS m/z 766.4 [M+1]⁺.

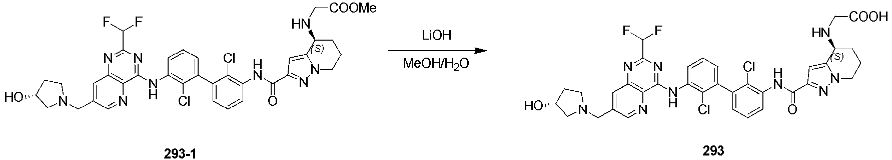

A mixture of methyl 2-[[(4S)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]amino]acetate (293-1, 70 mg, 91.31 µmol, 1 eq) and lithium hydroxide (6.56 mg, 273.93 µmol, 3 eq) in H₂O (1 mL) / MeOH (1 mL) was stirred at room temperature for 12 hours. After completion of the reaction, the mixture was concentrated under reduced pressure. The resulting residue was purified by prep-HPLC (column: Waters Xbridge, 150 * 25 mm * 5 µms; mobile phase: [H₂O (NH₄HCO₃)-ACN]; gradient: 22% to 52% B over 9 min) to afford Compound 293 (52 mg, 74.9% yield) as a yellow solid.

LCMS m/z 752.4 [M+1]⁺.

¹H NMR (400 MHz, METHANOL-d4) δ = 9.04 - 9.00 (m, 2H), 8.47 (br d, J = 7.8 Hz, 1H), 8.29 (s, 1H), 7.58 (t, J = 7.9 Hz, 1H), 7.49 (t, J = 7.9 Hz, 1H), 7.20 (br t, J = 6.1 Hz, 2H), 7.03 (s, 1H), 6.74 (t, J = 54.7 Hz, 1H), 4.62 (br s, 1H), 4.41 (br s, 1H), 4.32 (br s, 1H), 4.28 - 4.24 (m, 2H), 4.03 - 3.93 (m, 2H), 3.45 (s, 2H), 3.40 - 3.37 (m, 1H), 2.92 - 2.86 (m, 2H), 2.64 (br d, J = 9.2 Hz, 2H), 2.35 (br s, 1H), 2.29 - 2.19 (m, 2H), 2.07 (br s, 1H), 1.97 (br s, 1H), 1.82 (br s, 1H)

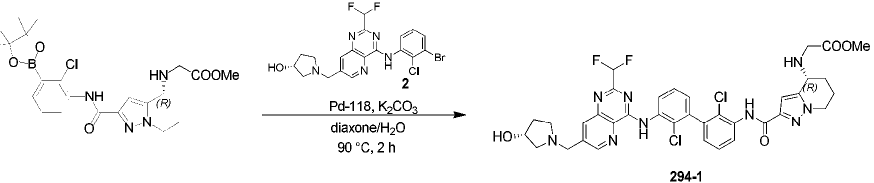

A mixture of methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (151.25 mg, 309.45 µmol, 1.2 eq), (3R)-1-[[4-(3-bromo-2-chloro-anilino)-2-(difluoromethyl)pyrido[3,2-d]pyrimidin-7-yl]methyl]pyrrolidin-3-ol (125 mg, 257.88 µmol, 1 eq), CsF (117.52 mg, 773.64 µmol, 3 eq), and ditert-butyl(cyclopentyl)phosphane;dichloropalladium;iron (16.81 mg, 25.79 µmol, 0.1 eq) in a mixture of dioxane (1.6 mL) / H₂O (0.4 mL) was stirred at 90 °C under a nitrogen atmosphere for 12 hours. After completion of the reaction, the mixture was cooled to room temperature and filtered. The filtrate was concentrated under reduced pressure, and the resulting residue was purified by prep-HPLC (column: Phenomenex Luna C18, 150 * 25 mm * 10 µms; mobile phase: [water (HCl)-ACN]; gradient: 10% to 40% B over 10 min) to afford Compound 294-1 (140 mg, 70.8% yield) as a yellow solid.

LCMS m/z 766.4 [M+1]⁺

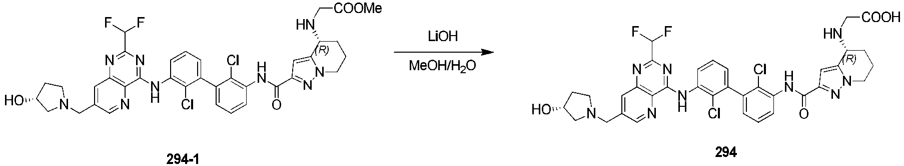

A mixture of methyl 2-[[(4R)-2-[[2-chloro-3-[2-chloro-3-[[2-(difluoromethyl)-7-[[(3R)-3-hydroxypyrrolidin-1-yl]methyl]pyrido[3,2-d]pyrimidin-4-yl]amino]phenyl]phenyl]carbamoyl]-4,5,6,7-tetrahydropyrazolo[1,5-a]pyridin-4-yl]amino]acetate (70 mg, 91.31 µmol, 1 eq) and lithium hydroxide (6.56 mg, 273.93 µmol, 3 eq) in H₂O (1 mL) / MeOH (1 mL) was stirred at room temperature for 12 hours. After completion of the reaction, the mixture was concentrated under reduced pressure. The resulting residue was purified by prep-HPLC (column: Waters Xbridge Prep OBD C18, 150 * 40 mm * 10 µms; mobile phase: [water (NH₄HCO₃)-ACN]; gradient: 15% to 45% B over 15 min) to afford Compound 294 (7 mg, 10.0% yield) as a yellow solid.

LCMS m/z 752.4 [M+1]⁺.

¹H NMR (400 MHz, METHANOL-*d4*) δ = 9.04 - 8.99 (m, 2H), 8.46 (br d, J = 7.7 Hz, 1H), 8.29 (s, 1H), 7.57 (br t, J = 7.8 Hz, 1H), 7.52 - 7.48 (m, 1H), 7.20 (br s, 2H), 7.06 (br s, 1H), 6.74 (br t, J = 55.0 Hz, 1H), 4.62 (br s, 2H), 4.41 (br s, 2H), 4.27 (br s, 2H), 3.99 (br d, J = 8.3 Hz, 2H), 3.49 (br s, 2H), 2.92 - 2.86 (m, 2H), 2.64 (br d, J = 9.2 Hz, 2H), 2.32 (br s, 1H), 2.36 - 2.19 (m, 3H), 2.07 (s, 2H), 2.00 (br s, 1H), 1.81 (br s, 1H).

### [Preparation Example 90]

### Preparation of Compound 295, (S)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide, and Compound 296, (R)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

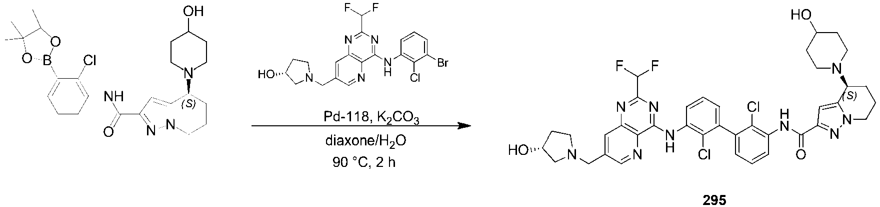

A mixture of (3R)-1-[[4-(3-bromo-2-chloro-anilino)-2-(difluoromethyl)pyrido[3,2-d]pyrimidin-7-yl]methyl]pyrrolidin-3-ol (130 mg, 268.19 µmol, 1 eq), (4S)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (174.61 mg, 348.65 µmol, 1.3 eq), K₂CO₃ (111.20 mg, 804.58 µmol, 3 eq), and di-tert-butyl(cyclopentyl)phosphane;dichloropalladium;iron (17.48 mg, 26.82 µmol, 0.1 eq) in adioxane (6 mL) / H₂O (1 mL) was stirred under a nitrogen atmosphere at 90 °C for 2 hours. After completion of the reaction, the mixture was cooled to room temperature and filtered. The filtrate was concentrated under reduced pressure, and the resulting residue was purified by prep-HPLC (column: Waters Xbridge Prep OBD C18, 150 * 40 mm * 10 µms; mobile phase: [water (NH₄HCO₃)-ACN]; gradient: 40% to 70% B over 20 min) to afford Compound 295 (22 mg, 10.0% yield) as a white solid.

LCMS m/z 778.1 [M+1]⁺.

¹H NMR (400 MHz, DMSO-d₆ ) δ = 10.34 - 10.13 (m, 1H), 9.55 (d, J = 1.5 Hz, 1H), 9.00 (d, J = 1.6 Hz, 1H), 8.63 - 8.51 (m, 1H), 8.35 - 8.19 (m, 2H), 7.63 - 7.56 (m, 1H), 7.53 - 7.49 (m, 1H), 7.25 - 7.15 (m, 2H), 7.07 - 6.78 (m, 1H), 6.77 - 6.62 (m, 1H), 4.75 (d, J = 4.8 Hz, 1H), 4.57 (d, J = 4.0 Hz, 1H), 4.28 - 4.18 (m, 2H), 4.11 - 3.96 (m, 2H), 3.94 - 3.79 (m, 2H), 2.74 (br dd, J = 6.1, 9.6 Hz, 3H), 2.67 (br s, 2H), 2.41 (br dd, J = 3.3, 9.6 Hz, 2H), 2.27 (br t, J = 9.1 Hz, 1H), 2.03 - 1.87 (m, 3H), 1.79 - 1.68 (m, 3H), 1.65 - 1.49 (m, 2H), 1.48 - 1.30 (m, 3H).

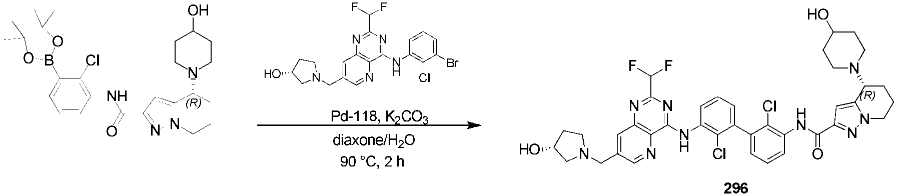

A mixture of (3R)-1-[[4-(3-bromo-2-chloro-anilino)-2-(difluoromethyl)pyrido[3,2-d]pyrimidin-7-yl]methyl]pyrrolidin-3-ol (120 mg, 247.56 µmol, 1 eq), (4R)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (161.18 mg, 321.83 µmol, 1.3 eq), K₂CO₃ (102.65 mg, 742.69 µmol, 3 eq), and Pd(dppf)Cl₂·CH₂Cl₂ (20.22 mg, 24.76 µmol, 0.1 eq) in dioxane (8 mL)/H₂O (2 mL) was stirred under a nitrogen atmosphere at 90 °C for 2 hours. After completion of the reaction, the mixture was cooled to room temperature and filtered. The filtrate was concentrated under reduced pressure, and the resulting residue was purified by prep-HPLC (column: Waters Xbridge, 150 * 25 mm * 5 µms; mobile phase: [water (NH₄HCO₃)-ACN]; gradient: 42% to 72% B over 9 min) to afford Compound 296 (31 mg, 15.2% yield) as a white solid.

LCMS m/z 778.3 [M+1]⁺.

¹H NMR (400 MHz, DMSO-d₆ ) δ = 10.27 (s, 1H), 9.55 (s, 1H), 9.00 (d, J = 1.5 Hz, 1H), 8.57 (dd, J = 1.2, 8.2 Hz, 1H), 8.24 (s, 2H), 7.69 - 7.57 (m, 1H), 7.50 (t, J = 7.9 Hz, 1H), 7.22 (dd, J = 7.1, 20.5 Hz, 2H), 7.04 - 6.56 (m, 2H), 4.75 (d, J = 4.6 Hz, 1H), 4.56 (d, J = 4.1 Hz, 1H), 4.30 - 4.15 (m, 2H), 4.11 - 4.00 (m, 1H), 3.99 - 3.82 (m, 3H), 3.54 - 3.45 (m, 1H), 3.23 - 3.12 (m, 1H), 2.80 - 2.61 (m, 4H), 2.45 - 2.35 (m, 2H), 2.26 (br t, J = 9.4 Hz, 1H), 2.13 (br dd, J = 4.6, 7.9 Hz, 1H), 2.06 - 1.84 (m, 3H), 1.80 - 1.65 (m, 3H), 1.62 - 1.53 (m, 1H), 1.52 - 1.33 (m, 2H).

### [Preparation Example 91]

### Preparation of Compound 297, (S)-1-(2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid, and Compound 298, (S)-1-(2-((2,2'-dichloro-3'-((2-isopropylpyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid,

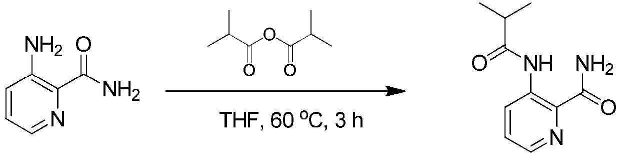

A mixture of 3-aminopyridine-2-carboxamide (4.0 g, 29.17 mmol, 1 eq) and 2-methylpropanoyl 2-methylpropanoate (23.07 g, 145.84 mmol, 24.18 mL, 5 eq) in THF (80 mL) was stirred at 60 °C for 3 hours. After completion of the reaction, the mixture was cooled to room temperature and diluted with water (40 mL). The resulting mixture was extracted with ethyl acetate (30 mL × 3). The combined organic layers were washed with brine (60 mL), dried over Na₂SO₄, filtered, and concentrated under reduced pressure. The resulting residue afforded 3-(2-methylpropanoylamino)pyridine-2-carboxamide (5.2 g, 68.8% yield) as a white solid, which was used directly in the next reaction without further purification.

LCMS m/z 208.0 [M+1]⁺

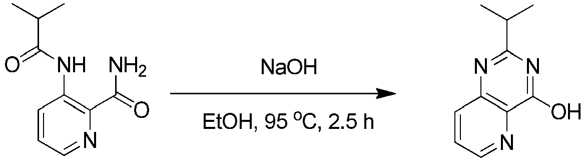

A solution of 3-(2-methylpropanoylamino)pyridine-2-carboxamide (4.0 g, 19.30 mmol, 1 eq) in ethanol (60 mL) was treated with NaOH (1.54 g, 38.60 mmol, 2 eq), and the resulting mixture was stirred at 95 °C for 3 hours. After completion of the reaction, the mixture was cooled to room temperature and diluted with H₂O (40 mL). The aqueous phase was extracted with ethyl acetate (30 mL x 3). The combined organic layers were washed with brine (60 mL), dried over anhydrous sodium sulfate, filtered, and concentrated under reduced pressure. The resulting residue was purified by prep-HPLC (column: Phenomenex Luna C18, 250 * 70 mm, 10 µm; mobile phase: [H₂O (HCl)-ACN]; gradient: 1% to 35% B over 20 min) to afford 2-isopropylpyrido[3,2-d]pyrimidin-4-ol (3.52 g, 91.5% yield) as a white solid.

¹H NMR (400 MHz, DMSO-*d₆*) δ = 12.31 (s, 1H), 8.97 (dd, *J* = 1.4, 8.5 Hz, 1H), 8.29 (dd, *J* = 1.4, 4.4 Hz, 1H), 7.57 (dd, *J* = 4.5, 8.6 Hz, 1H), 2.56 (quin, *J* = 6.9 Hz, 1H), 1.18 (s, 3H), 1.16 (s, 3H).

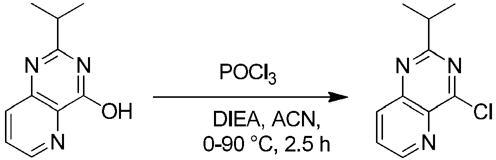

To a solution of 2-isopropylpyrido[3,2-d]pyrimidin-4-ol (500 mg, 2.64 mmol, 1 eq) and N,N-diisopropylethylamine (1.37 g, 10.57 mmol, 4 eq) in acetonitrile (5 mL), POCl₃ (8.10 g, 52.85 mmol, 20 eq) was added at 0 °C, and the resulting mixture was then heated to 90 °C and stirred for 3 hours. After completion of the reaction, the mixture was cooled to room temperature and concentrated under reduced pressure. The residue was diluted with ethyl acetate (30 mL), and the organic phase was washed successively with aq. saturated NaHCO₄ solution (30 mL) and water (30 mL). The organic layer was dried over anhydrous sodium sulfate, filtered, and concentrated under reduced pressure. The resulting residue was purified by flash silica gel column chromatography (Petroleum ether/Ethyl acetate = 100:1 to 3:1) to afford 4-chloro-2-isopropylpyrido[3,2-d]pyrimidine (320 mg, 55.4% yield) as a white solid.

LCMS m/z 208.1 [M+1]⁺

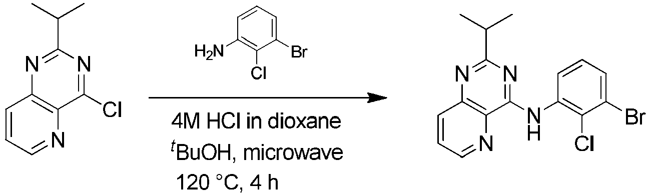

A solution of 4-chloro-2-isopropylpyrido[3,2-d]pyrimidine (300 mg, 1.44 mmol, 1 eq) and 3-bromo-2-chloroaniline (328.11 mg, 1.59 mmol, 1.1 eq) in tert-butanol (8 mL) was treated with hydrogen chloride in dioxane (2 M, 1 mL, 1.38 eq). The resulting mixture was stirred at 120 °C for 4 hours in a microwave reactor. After completion of the reaction, the mixture was cooled to room temperature and diluted with water (40 mL). The aqueous phase was extracted with ethyl acetate (30 mL x 3). The combined organic layers were washed with brine (60 mL), dried over anhydrous sodium sulfate, filtered, and concentrated under reduced pressure. The residue was purified by flash silica gel column chromatography (Petroleum ether/Ethyl acetate = 100/1 to 2/1) to afford N-(3-bromo-2-chloro-phenyl)-2-isopropyl-pyrido[3,2-d]pyrimidin-4-amine (312 mg, 51.4% yield) as a white solid.

LCMS m/z 377.1 [M+2]⁺

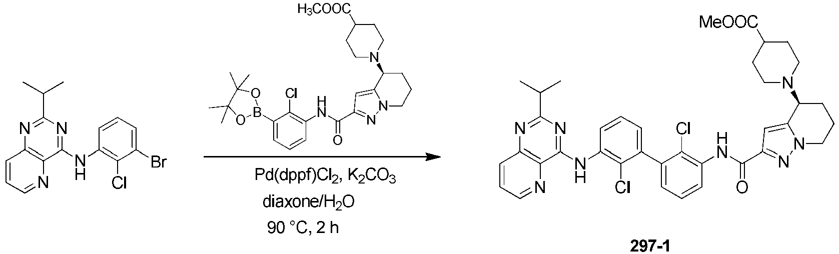

A mixture of N-(3-bromo-2-chloro-phenyl)-2-isopropyl-pyrido[3,2-d]pyrimidin-4-amine (120 mg, 317.74 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (189.74 mg, 349.52 µmol, 1.1 eq), K₂CO₃ (131.74 mg, 953.22 µmol, 3 eq), and di-tert-butyl(cyclopentyl)phosphane;dichloropalladium;iron (20.71 mg, 31.77 µmol, 0.1 eq) in dioxane (8 mL) / H₂O (1.5 mL) was stirred under a nitrogen atmosphere at 90 °C for 2 hours. After completion of the reaction, the mixture was cooled to room temperature and filtered. The filtrate was concentrated under reduced pressure, and the resulting residue was purified by prep-HPLC (column: Phenomenex Luna C18, 150 * 40 mm * 15 µm; mobile phase: [water (FA)-ACN]; gradient: 22% to 52% B over 15 min) to afford Compound 297-1 (102 mg, 35.9% yield) as a white solid.

LCMS m/z 713.3 [M+1]⁺

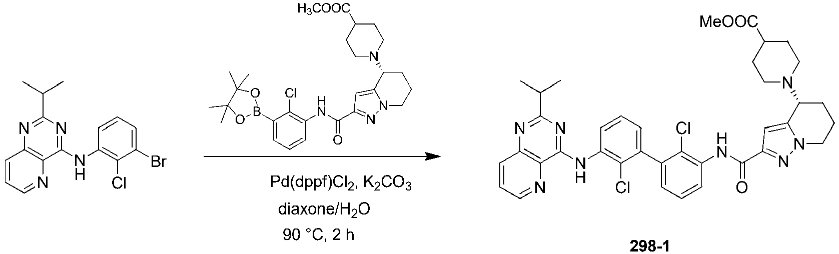

A mixture of N-(3-bromo-2-chloro-phenyl)-2-isopropyl-pyrido[3,2-d]pyrimidin-4-amine (120 mg, 317.74 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (206.99 mg, 381.29 µmol, 1.2 eq), K₂CO₃ (131.75 mg, 953.22 µmol, 3 eq), and cyclopentyl(diphenyl)phosphane-dichloromethane-dichloropalladium-iron complex (25.95 mg, 31.77 µmol, 0.1 eq) in dioxane (6 mL) / H₂O (1.5 mL) was stirred under a nitrogen atmosphere at 90 °C for 2 hours. After completion of the reaction, the mixture was cooled to room temperature and filtered. The filtrate was concentrated under reduced pressure, and the resulting residue was purified by prep-TLC (Petroleum ether/Ethyl acetate = 1/1) to afford compound 298-1 (96 mg, 33.8% yield) as a white solid.

LCMS m/z 713.3 [M+1]⁺

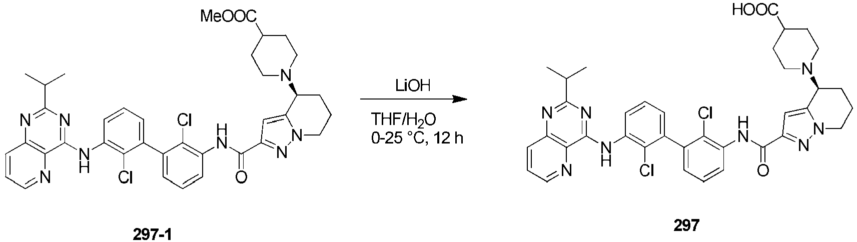

To a solution of methyl 1-[(4S)-2-[[2-chloro-3-[2-chloro-3-[(2-isopropyl-pyrido[3,2-d]pyrimidin-4-yl)amino]phenyl]phenyl]carbamoyl]-4,5,6,7-tetrahydropyrazolo[1,5-a]pyridin-4-yl]piperidine-4-carboxylate (297-1) (80 mg, 112.10 µmol, 1 eq) in H₂O (1.5 mL) / THF (5 mL), LiOH·H₂O (14.11 mg, 336.30 µmol, 3 eq) was added at 0 °C, and the resulting mixture was stirred at room temperature for 12 hours. After completion of the reaction, the mixture was concentrated under reduced pressure. The resulting residue was purified by prep-HPLC (column: Waters Xbridge, 150 * 25 mm * 5 µm; mobile phase: [H₂O (NH₄HCO₃)-ACN]; gradient: 35% to 65% B over 9 min) to afford compound 297 (42 mg, 50.8% yield) as a white solid.

LCMS m/z 699.2 [M+1]⁺

¹H NMR (400 MHz, DMSO-d₆) δ = 9.99 (s, 1H), 9.55 (d, *J* = 1.0 Hz, 1H), 8.97 - 8.85 (m, 2H), 8.33 - 8.23 (m, 2H), 7.99 - 7.88 (m, 1H), 7.66 - 7.56 (m, 1H), 7.51 (t, *J* = 7.9 Hz, 1H), 7.25 - 7.11 (m, 2H), 6.67 (s, 1H), 4.27 - 4.17 (m, 1H), 4.12 - 4.02 (m, 1H), 3.97 (br dd, *J* = 4.9, 10.0 Hz, 1H), 3.22 - 3.10 (m, 1H), 2.84 - 2.75 (m, 1H), 2.72 - 2.64 (m, 1H), 2.47 - 2.38 (m, 1H), 2.35 - 2.10 (m, 3H), 1.90 (br s, 2H), 1.85 - 1.76 (m, 2H), 1.74 - 1.49 (m, 3H), 1.37 (d, *J* = 6.9 Hz, 6H).

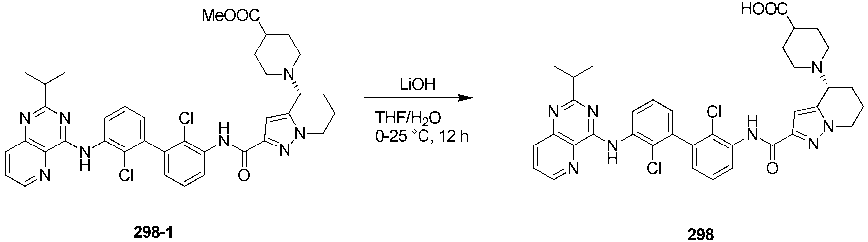

To a solution of methyl 1-[(4R)-2-[[2-chloro-3-[2-chloro-3-[(2-isopropyl-pyrido[3,2-d]pyrimidin-4-yl)amino]phenyl]phenyl]carbamoyl]-4,5,6,7-tetrahydropyrazolo[1,5-a]pyridin-4-yl]piperidine-4-carboxylate (298-1) (80 mg, 112.10 µmol, 1 eq) in H₂O (1.5 mL) / THF (5 mL), LiOH·H₂O (14.11 mg, 336.30 µmol, 3 eq) was added 0 °C, and the resulting mixture was stirred at room temperature for 12 hours. After completion of the reaction, the mixture was concentrated under reduced pressure. The resulting residue was purified by prep-HPLC (column: Waters Xbridge, 150 * 25 mm * 5 µm; mobile phase: [H₂O (NH₄HCO₃)-ACN]; gradient: 32% to 62% B over 9 min) to afford compound 298 (51 mg, 61.7% yield) as a white solid.

LCMS m/z 699.3 [M+1]⁺

¹H NMR (400 MHz, DMSO-d₆) δ = 9.98 (s, 1H), 9.55 (s, 1H), 8.96 - 8.85 (m, 2H), 8.36 - 8.21 (m, 2H), 7.93 (dd, *J* = 4.3, 8.4 Hz, 1H), 7.66 - 7.57 (m, 1H), 7.50 (t, *J* = 7.9 Hz, 1H), 7.25 - 7.12 (m, 2H), 6.67 (s, 1H), 4.27 - 4.17 (m, 1H), 4.12 - 4.02 (m, 1H), 3.97 (br dd, *J* = 5.3, 9.9 Hz, 1H), 3.22 - 3.10 (m, 2H), 2.85 - 2.74 (m, 1H), 2.72 - 2.63 (m, 1H), 2.44 (br s, 1H), 2.34 - 2.20 (m, 2H), 2.17 - 2.09 (m, 1H), 2.03 - 1.94 (m, 1H), 1.92 - 1.74 (m, 3H), 1.71 - 1.49 (m, 3H), 1.36 (d, *J* = 6.9 Hz, 6H).

### [Preparation Example 92]

### Preparation of Compound 299, (S)-2-((2-((2,2'-dichloro-3'-((2-isopropyl-pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid, and Compound 300, (S)-2-((2-((2,2'-dichloro-3'-((2-isopropyl-pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid,

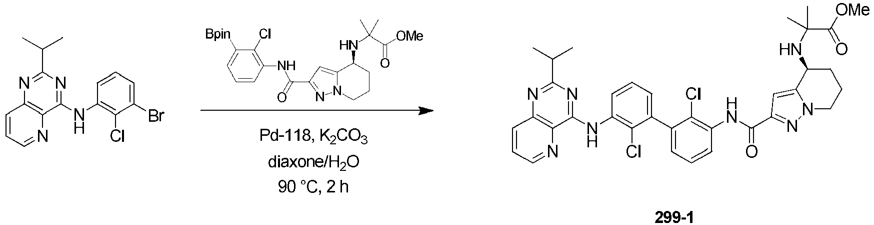

A mixture of N-(3-bromo-2-chloro-phenyl)-2-isopropyl-pyrido[3,2-d]pyrimidin-4-amine (80 mg, 211.83 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methylpropanoate (131.37 mg, 254.19 µmol, 1.2 eq), di-tert-butyl(cyclopentyl)phosphane;dichloropalladium;iron (13.81 mg, 21.18 µmol, 0.1 eq), and K₂CO₃ (87.83 mg, 635.48 µmol, 3 eq) in dioxane (8 mL) / H₂O (1.5 mL) was stirred at 90 °C for 2 hours under a nitrogen atmosphere. After completion of the reaction, the mixture was cooled to room temperature and filtered. The filtrate was concentrated under reduced pressure, and the resulting residue was purified by prep-TLC (Petroleum ether/Ethyl acetate = 1:1) to afford Compound 299-1 (102 mg, 59.5% yield) as a white solid.

LCMS m/z 687.3 [M+1]⁺.

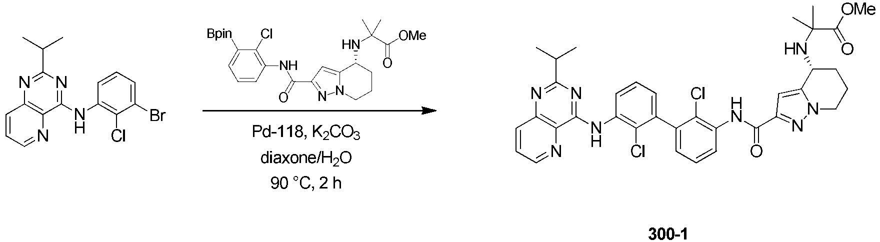

A mixture of N-(3-bromo-2-chloro-phenyl)-2-isopropyl-pyrido[3,2-d]pyrimidin-4-amine (100 mg, 264.78 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methylpropanoate (164.22 mg, 317.74 µmol, 1.2 eq), di-tert-butyl(cyclopentyl)phosphane;dichloropalladium;iron (17.26 mg, 26.48 µmol, 0.1 eq), and K₂CO₃ (109.79 mg, 794.35 µmol, 3 eq) in dioxane (8 mL) / H₂O (1.5 mL) was stirred at 90 °C for 2 hours under a nitrogen atmosphere. After completion of the reaction, the mixture was cooled to room temperature and filtered. The filtrate was concentrated under reduced pressure, and the resulting residue was purified by prep-TLC (Petroleum ether/Ethyl acetate=1/1) to afford Compound 300-4 (101 mg, 44.9% yield) as a white solid.

LCMS m/z 687.3 [M+1]⁺

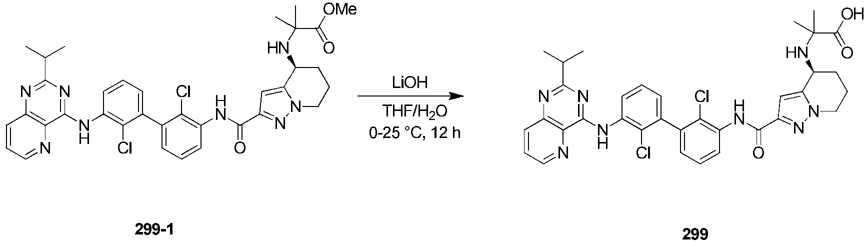

T a solution of methyl 2-[[(4S)-2-[[2-chloro-3-[2-chloro-3-[(2-isopropyl-pyrido[3,2-d]pyrimidin-4-yl)amino]phenyl]phenyl]carbamoyl]-4,5,6,7-tetrahydropyrazolo[1,5-a]pyridin-4-yl]amino]-2-methylpropanoate (102 mg, 148.34 µmol, 1 eq) in H₂O (1.5 mL) / THF (5 mL), LiOH·H₂O (18.67 mg, 445.02 µmol, 3 eq) was added at 0 °C. The reaction mixture was stirred at room temperature for 12 hours. Upon completion of the reaction, the mixture was concentrated under reduced pressure. The resulting residue was purified by prep-HPLC (column: Waters Xbridge, 150 * 25 mm * 5 µm; mobile phase: [H₂O(NH₄HCO₃)-ACN]; gradient: 25% to 55% acetonitrile over 15 min) to afford compound 299 (42 mg, 39.9% yield) as a white solid.

LCMS m/z 673.3 [M+1]⁺

¹H NMR (400 MHz, DMSO-*d₆*) δ = 9.99 (s, 1H), 9.54 (s, 1H), 8.98 - 8.86 (m, 2H), 8.30 (dd, *J* = 1.3, 8.1 Hz, 1H), 8.24 (dd, *J* = 1.5, 8.5 Hz, 1H), 7.93 (dd, *J* = 4.3, 8.5 Hz, 1H), 7.60 (t, *J* = 7.9 Hz, 1H), 7.51 (t, *J* = 7.9 Hz, 1H), 7.18 (ddd, *J* = 1.5, 7.7, 9.4 Hz, 2H), 6.79 (s, 1H), 4.19 - 4.07 (m, 2H), 3.98 - 3.88 (m, 1H), 3.15 (td, *J* = 6.9, 13.7 Hz, 3H), 2.19 - 2.09 (m, 1H), 2.08 - 1.99 (m, 1H), 1.98 - 1.87 (m, 1H), 1.59 (br s, 1H), 1.37 (d, *J* = 6.9 Hz, 6H), 1.29 (d, *J* = 3.6 Hz, 6H).

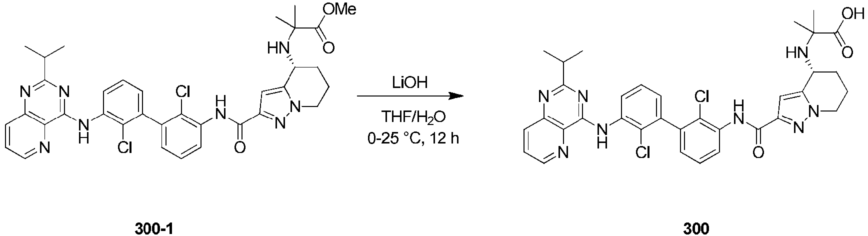

To a solution of methyl 2-[[(4R)-2-[[2-chloro-3-[2-chloro-3-[(2-isopropyl-pyrido[3,2-d]pyrimidin-4-yl)amino]phenyl]phenyl]carbamoyl]-4,5,6,7-tetrahydropyrazolo[1,5-a]pyridin-4-yl]amino]-2-methylpropanoate (80 mg, 116.34 µmol, 1 eq) in H₂O (1.5 mL) / THF (5 mL), LiOH·H₂O (14.65 mg, 349.03 µmol, 3 eq) was added at 0 °C. The reaction mixture was stirred at room temperature for 12 hours. After completion of the reaction, the mixture was concentrated under reduced pressure. The resulting residue was purified by prep-HPLC (column: Waters Xbridge, 150 × 25 mm, 5 µm; mobile phase: [H₂O(NH₄HCO₃)-ACN]; gradient: 25% to 55% B over 15 min) to afford compound 300 (41 mg, 49.7% yield) as a white solid.

LCMS m/z 673.2 [M+1]⁺

¹H NMR (400 MHz, DMSO-*d₆* ) δ = 9.99 (s, 1H), 9.54 (s, 1H), 9.00 - 8.84 (m, 2H), 8.34 - 8.20 (m, 2H), 7.93 (dd, *J* = 4.3, 8.5 Hz, 1H), 7.65 - 7.56 (m, 1H), 7.51 (t, *J* = 7.9 Hz, 1H), 7.25 - 7.13 (m, 2H), 6.79 (s, 1H), 4.20 - 4.08 (m, 2H), 4.01 - 3.85 (m, 1H), 3.15 (td, *J* = 6.9, 13.8 Hz, 2H), 2.23 - 2.10 (m, 1H), 2.07 - 1.86 (m, 2H), 1.72 - 1.53 (m, 1H), 1.37 (d, *J* = 6.9 Hz, 6H), 1.29 (d, *J* = 3.5 Hz, 6H).

### [Preparation Example 93]

### Preparation of Compound 301, (S)-1-(2-((2,2'-dichloro-3'-((2-isopropyl-pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid, and Compound 302, (R)-1-(2-((2,2'-dichloro-3'-((2-isopropyl-pyrido[3,2-d]pyrimidin-4-yl)amino)-[1,1'-biphenyl]-3-yl)carbamoyl)-4,5,6,7-tetrahydropyrazolo[1,5-a]pyridin-4-yl)azetidine-3-carboxylic acid

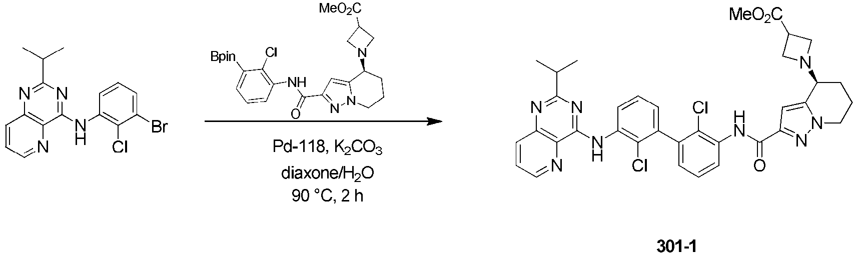

A mixture of N-(3-bromo-2-chloro-phenyl)-2-isopropyl-pyrido[3,2-d]pyrimidin-4-amine (80 mg, 211.83 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (130.86 mg, 254.19 µmol, 1.2 eq), di-tert-butyl(cyclopentyl)phosphane;dichloropalladium;iron (13.81 mg, 21.18 µmol, 0.1 eq), and K₂CO₃ (87.83 mg, 635.48 µmol, 3 eq) in dioxane (8 mL) / H₂O (1.5 mL) was stirred under a nitrogen atmosphere at 90 °C for 2 hours. After completion of the reaction, the mixture was cooled to room temperature, filtered, and the filtrate was concentrated under reduced pressure. The resulting residue was purified by prep-TLC (Petroleum ether/Ethyl acetate = 1/1) to afford compound 300-1 (102 mg, 57.5% yield) as a white solid.

LCMS m/z 685.3 [M+1]⁺.

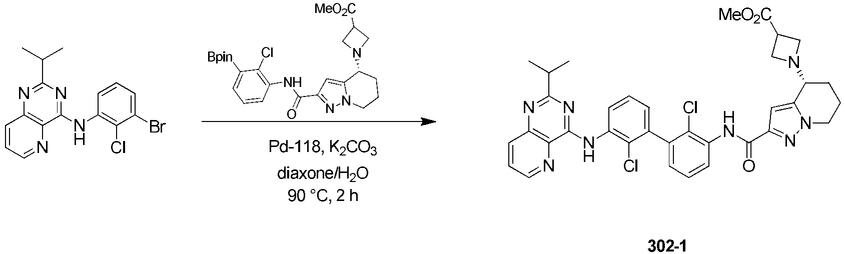

A mixture of N-(3-bromo-2-chloro-phenyl)-2-isopropyl-pyrido[3,2-d]pyrimidin-4-amine (80 mg, 211.83 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]azetidine-3-carboxylate (130.86 mg, 254.19 µmol, 1.2 eq), di-tert-butyl(cyclopentyl)phosphane;dichloropalladium;iron (13.81 mg, 21.18 µmol, 0.1 eq), and K₂CO₃ (87.83 mg, 635.48 µmol, 3 eq) in dioxane (8 mL) / H₂O (1.5 mL) was stirred under a nitrogen atmosphere at 90 °C for 2 hours. After completion of the reaction, the mixture was cooled to room temperature, filtered, and the filtrate was concentrated under reduced pressure. The resulting residue was purified by prep-TLC (Petroleum ether/Ethyl acetate = 1/1) to afford compound 302-1 (92 mg, 50.68% yield) as a white solid.

LCMS m/z 685.2 [M+1]⁺.

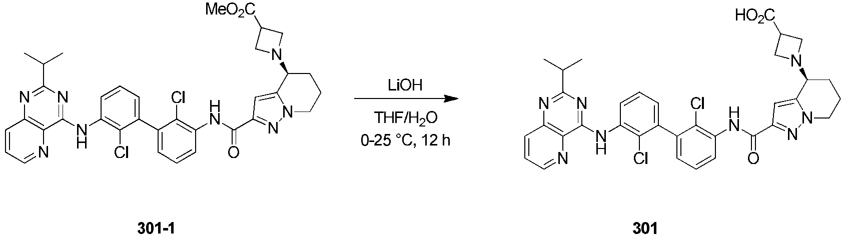

To a solution of methyl 1-[(4S)-2-[[2-chloro-3-[2-chloro-3-[(2-isopropyl-pyrido[3,2-d]pyrimidin-4-yl)amino]phenyl]phenyl]carbamoyl]-4,5,6,7-tetrahydropyrazolo[1,5-a]pyridin-4-yl]azetidine-3-carboxylate (301-1) (80 mg, 116.69 µmol, 1 eq) in H₂O (1.5 mL) / THF (5 mL), LiOH·H₂O (14.69 mg, 350.06 µmol, 3 eq) was added 0 °C,. The reaction mixture was stirred at room temperature for 12 hours. After completion of the reaction, the mixture was concentrated under reduced pressure, and the resulting residue was purified by prep-HPLC (column: Waters Xbridge, 150 * 25 mm * 5 µm; mobile phase: [H₂O (NH₄HCO₃)-ACN]; gradient: 20%-50% B over 15 min) to afford compound 301 (42 mg, 50.9% yield) as a white solid.

LCMS m/z 673.2 [M+1]⁺

¹H NMR (400 MHz, DMSO-*d₆*) δ = 9.99 (s, 1H), 9.59 (br s, 1H), 8.96 - 8.85 (m, 2H), 8.25 (br dd, *J* = 1.3, 8.6 Hz, 2H), 7.93 (dd, *J* = 4.4, 8.5 Hz, 1H), 7.60 (t, *J* = 7.9 Hz, 1H), 7.51 (t, *J* = 7.9 Hz, 1H), 7.19 (dd, *J* = 6.9, 16.4 Hz, 2H), 4.29 (br d, *J* = 4.1 Hz, 2H), 3.86 (s, 4H), 3.19 - 3.10 (m, 3H), 2.30 - 2.07 (m, 2H), 1.99 - 1.72 (m, 3H), 1.37 (d, *J* = 6.9 Hz, 6H).

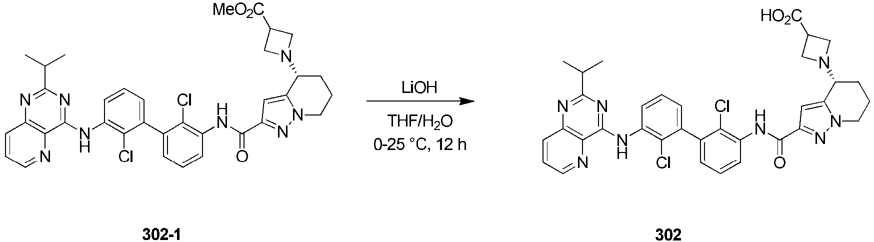

To a solution of methyl 1-[(4R)-2-[[2-chloro-3-[2-chloro-3-[(2-isopropyl-pyrido[3,2-d]pyrimidin-4-yl)amino]phenyl]phenyl]carbamoyl]-4,5,6,7-tetrahydropyrazolo[1,5-a]pyridin-4-yl]azetidine-3-carboxylate (302-1) (80.00 mg, 116.69 µmol, 1 eq) in H₂O (1.5 mL) / THF (5 mL), LiOH·H₂O (14.69 mg, 350.06 µmol, 3 eq) was added at 0 °C. The reaction mixture was stirred at room temperature for 12 hours. After completion of the reaction, the mixture was concentrated under reduced pressure, and the resulting residue was purified by prep-HPLC (column: Waters Xbridge, 150 * 25 mm * 5 µm; mobile phase: [H₂O (NH₄HCO₃)-ACN]; gradient: 30%-60% B over 9 min) to afford compound 302 (46 mg, 55.7% yield) as a white solid.

LCMS m/z 673.2 [M+1]⁺

¹H NMR (400 MHz, DMSO-d₆ ) δ = 9.99 (s, 1H), 9.56 (s, 1H), 8.97 - 8.85 (m, 2H), 8.35 - 8.21 (m, 2H), 7.93 (dd, *J* = 4.2, 8.4 Hz, 1H), 7.70 - 7.56 (m, 1H), 7.51 (t, *J* = 7.9 Hz, 1H), 7.18 (dd, *J* = 7.8, 10.4 Hz, 2H), 6.76 (s, 1H), 4.31 - 4.18 (m, 1H), 4.14 - 4.02 (m, 1H), 3.59 - 3.42 (m, 3H), 3.24 - 3.10 (m, 4H), 2.28 - 2.12 (m, 1H), 1.74 (br s, 3H), 1.37 (d, *J* = 6.9 Hz, 6H).

### [Preparation Example 94]

### Preparation of Compound 303, (S)-N-(2,2'-dichloro-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide, and Compound 304, (R)-N-(2,2'-dichloro-3'-((2-(trifluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

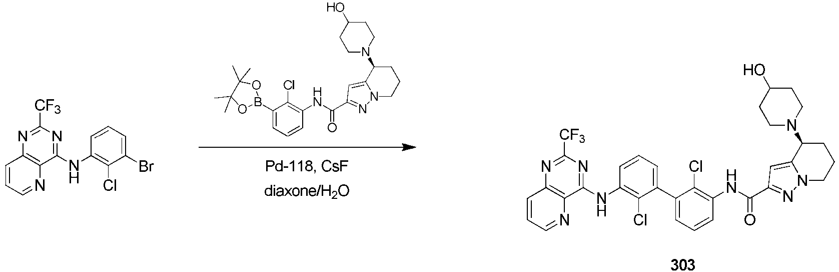

A mixture of N-(3-bromo-2-chloro-phenyl)-2-(trifluoromethyl)pyrido[3,2-d]pyrimidin-4-amine (150 mg, 371.67 µmol, 1 eq), (4S)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (204.76 mg, 408.84 µmol, 1.1 eq), CsF (169.37 mg, 1.12 mmol, 3 eq), and di-tert-butyl(cyclopenta-1,4-dien-1-yl)phosphane;dichloropalladium;iron (24.22 mg, 37.17 µmol, 0.1 eq) in dioxane (8 mL) / H₂O (2 mL) was stirred under a nitrogen atmosphere at 90 °C for 12 hours. After completion of the reaction, the mixture was cooled to room temperature and filtered. The filtrate was concentrated under reduced pressure, and the residue was purified by column chromatography (SiO₂, Petroleum ether/Ethyl acetate = 1/0 to 0/1, Ethyl acetate/Methanol = 1:0 to 3:1) and further purified by reversed-phase HPLC (column: Waters Xbridge 150 * 25 mm * 5 µm; mobile phase: [H₂O (NH₄HCO₃)-ACN]; gradient: 52%-82% B over 15 min) to afford compound 303 (24 mg, 8.79% yield) as a white solid.

LCMS m/z 699.1 [M+3]⁺.

¹H NMR (400 MHz, DMSO-d₆) δ ppm 1.30 - 1.51 (m, 3 H) 1.65 - 1.80 (m, 3 H) 1.82 - 2.03 (m, 2 H) 2.09 - 2.21 (m, 1 H) 2.27 (br t, *J*=9.94 Hz, 1 H) 2.37 - 2.45 (m, 1 H) 2.61 - 2.80 (m, 2 H) 3.97 (br dd, *J*=10.07, 4.94 Hz, 1 H) 4.01 - 4.11 (m, 1 H) 4.17 - 4.26 (m, 1 H) 4.57 (d, *J*=4.13 Hz, 1 H) 6.66 (s, 1 H) 7.16 - 7.23 (m, 1 H) 7.29 (d, *J*=7.63 Hz, 1 H) 7.51 (t, *J*=7.94 Hz, 1 H) 7.61 (t, *J*=7.94 Hz, 1 H) 8.09 (dd, *J*=8.51, 4.38 Hz, 1 H) 8.24 - 8.31 (m, 1 H) 8.38 (d, *J*=8.26 Hz, 1 H) 8.47 (dd, *J*=8.44, 1.19 Hz, 1 H) 9.12 (dd, *J*=4.13, 1.25 Hz, 1 H) 9.56 (d, *J*=0.75 Hz, 1 H) 10.45 - 10.57 (m, 1 H).

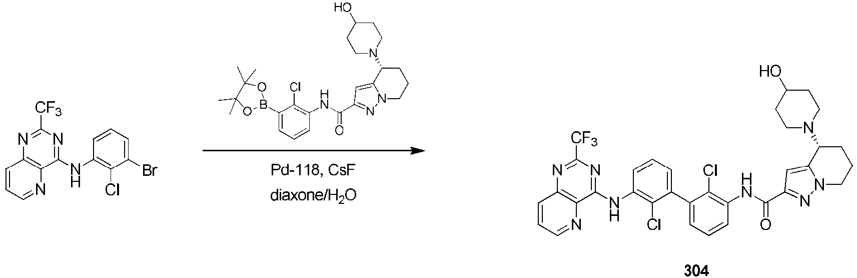

A mixture of N-(3-bromo-2-chloro-phenyl)-2-(trifluoromethyl)pyrido[3,2-d]pyrimidin-4-amine (150 mg, 371.67 µmol, 1 eq), (4R)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (204.76 mg, 408.84 µmol, 1.1 eq), CsF (169.37 mg, 1.12 mmol, 3 eq), and di-tert-butyl(cyclopenta-1,4-dien-1-yl)phosphane;dichloropalladium;iron (24.22 mg, 37.17 µmol, 0.1 eq) in dioxane (8 mL) / H₂O (2 mL) was stirred under a nitrogen atmosphere at 90 °C for 12 hours. After completion, the reaction mixture was cooled to room temperature and filtered. The filtrate was concentrated under reduced pressure, and the residue was purified by column chromatography (SiO₂, Petroleum ether/Ethyl acetate = 1/0 to 0/1, followed by ethyl acetate/methanol = 1:0 to 3:1). The resulting material was further purified by reversed-phase HPLC (column: Phenomenex Luna C18, 150 * 25 mm * 10 µm; mobile phase: [water (HCl)-ACN]; gradient: 30%-60% B over 10 min) to afford Compound 304 (51 mg, 18.8% yield) as a yellow solid.

LCMS m/z 699.1 [M+3]⁺.

¹H NMR (400 MHz, DMSO-d₆) δ ppm 1.07 - 1.22 (m, 2 H) 1.55 - 1.86 (m, 3 H) 1.89 - 2.21 (m, 4 H) 2.21 - 2.42 (m, 3 H) 3.01 - 3.20 (m, 2 H) 3.61 - 3.77 (m, 1 H) 3.98 (br s, 1 H) 4.25 (br s, 2 H) 4.92 (br s, 1 H) 7.14 - 7.33 (m, 2 H) 7.44 - 7.56 (m, 2 H) 7.57 - 7.66 (m, 1 H) 8.08 (br dd, *J*=8.07, 3.94 Hz, 1 H) 8.22 (br t, *J*=7.44 Hz, 1 H) 8.31 - 8.55 (m, 2 H) 9.11 (br d, *J*=3.25 Hz, 1 H) 9.64 (br s, 1 H) 10.50 (s, 1 H) 11.02 - 11.31 (m, 1 H).

### [Preparation Example 95]

### Preparation of Compound 305, (S)-N-(2,2'-dichloro-3'-((2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide, and Compound 306, (S)-N-(2,2'-dichloro-3'-((2-(difluoromethyl)pyrido[3,2-d]pyrimidin-4-yl)amino)-[1,1'-biphenyl]-3-yl)-4-(4-hydroxypiperidin-1-yl)-4,5,6,7-tetrahydropyrazolo[1,5-a]pyridine-2-carboxamide

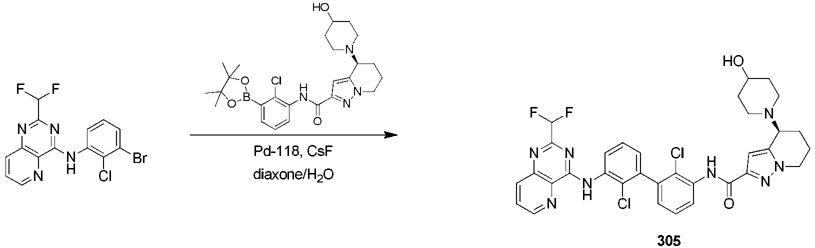

A mixture of N-(3-bromo-2-chloro-phenyl)-2-(difluoromethyl)pyrido[3,2-d]pyrimidin-4-amine (150 mg, 389.01 µmol, 1 eq), (4S)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (214.31 mg, 427.91 µmol, 1.1 eq), CsF (177.27 mg, 1.17 mmol, 3 eq), and di-tert-butyl(cyclopenta-1,4-dien-1-yl)phosphane;dichloropalladium;iron (25.35 mg, 38.90 µmol, 0.1 eq) in dioxane (8 mL) / H₂O (2 mL) was stirred under a nitrogen atmosphere at 90 °C for 12 hours. After completion, the reaction mixture was cooled to room temperature and filtered. The filtrate was concentrated under reduced pressure, and the residue was purified by column chromatography (SiO₂, Petroleum ether/Ethyl acetate = 1/0 to 0/1, followed by ethyl acetate/methanol = 1:0 to 3:1). The resulting material was further purified by reversed-phase HPLC (column: Waters Xbridge, 150 * 25 mm * 5 µm; mobile phase: [H₂O(NH₄HCO₃)-ACN]; gradient: 45%-75% B over 15 min) to afford Compound 305 (82 mg, 30.7% yield) as a white solid.

LCMS m/z 681.1 [M+3]⁺.

¹H NMR (400 MHz, DMSO-d₆) δ ppm 1.73 - 1.86 (m, 1 H) 1.88 - 2.05 (m, 2 H) 2.06 - 2.21 (m, 2 H) 2.25 - 2.37 (m, 2 H) 2.97 - 3.36 (m, 4 H) 3.45 (br d, *J*=11.38 Hz, 1 H) 3.97 (br s, 1 H) 4.14 - 4.33 (m, 2 H) 4.92 (br s, 1 H) 6.90 (t, *J*=54.34 Hz, 1 H) 7.24 (t, *J*=8.50 Hz, 2 H) 7.48 - 7.64 (m, 3 H) 8.05 (ddd, *J=*8.44, 4.25, 0.94 Hz, 1 H) 8.18 - 8.26 (m, 1 H) 8.41 (d, *J*=8.51 Hz, 1 H) 8.61 (d, *J*=8.25 Hz, 1 H) 9.07 (d, *J*=3.63 Hz, 1 H) 9.65 (d, *J*=2.63 Hz, 1 H) 10.31 (s, 1 H) 11.17 - 11.41 (m, 1 H).

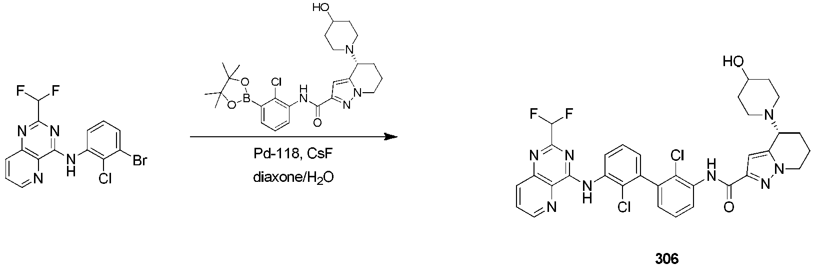

A mixture of N-(3-bromo-2-chloro-phenyl)-2-(difluoromethyl)pyrido[3,2-d]pyrimidin-4-amine (150.00 mg, 389.01 µmol, 1 eq), (4R)-N-[2-chloro-3-(4,4,5,5-tetramethyl-1,3,2-dioxaborolan-2-yl)phenyl]-4-(4-hydroxy-1-piperidyl)-4,5,6,7-tetrahydropyrazolo[1,5-a]pyridine-2-carboxamide (214.31 mg, 427.91 µmol, 1.1 eq), CsF (177.27 mg, 1.17 mmol, 3 eq), and di-tert-butyl(cyclopenta-1,4-dien-1-yl)phosphane;dichloropalladium;iron (25.35 mg, 38.90 µmol, 0.1 eq) in dioxane (10 mL) / H₂O (2.5 mL) was stirred under a nitrogen atmosphere at 90 °C for 12 hours. After completion, the reaction mixture was cooled to room temperature and filtered. The filtrate was concentrated under reduced pressure, and the residue was purified by column chromatography (SiO₂, Petroleum ether/Ethyl acetate = 1:0 to 0:1, followed by ethyl acetate/methanol = 1/0 to 3/1). Further purification was performed by reversed-phase HPLC (column: Phenomenex Luna C18, 150 * 25 mm * 10 µm; mobile phase: [water (HCl)-ACN]; gradient: 28%-58% B over 10 min) to afford Compound 306 (71 mg, 26.3% yield) as a white solid.

LCMS m/z 679.3 [M+1]⁺.

¹H NMR (400 MHz, DMSO-*d₆*) δ ppm 1.77 (br d, *J*=13.63 Hz, 1 H) 1.90 - 2.03 (m, 2 H) 2.05 - 2.21 (m, 2 H) 2.24 - 2.34 (m, 2 H) 3.01 - 3.31 (m, 3 H) 3.40 - 3.49 (m, 1 H) 3.69 (td, *J*=9.94, 4.75 Hz, 1 H) 3.97 (br s, 1 H) 4.20 - 4.39 (m, 6 H) 4.91 (br s, 1 H) 6.90 (t, *J*=54.34 Hz, 1 H) 7.24 (t, *J*=8.50 Hz, 2 H) 7.49 - 7.56 (m, 2 H) 7.61 (t, *J*=7.94 Hz, 1 H) 8.06 (dd, *J*=8.51, 4.25 Hz, 1 H) 8.22 (t, *J*=7.19 Hz, 1 H) 8.42 (dd, *J*=8.44, 0.81 Hz, 1 H) 8.61 (d, *J*=8.25 Hz, 1 H) 9.08 (d, *J*=3.13 Hz, 1 H) 9.65 (br s, 1 H) 10.32 (s, 1 H) 11.01 - 11.27 (m, 1 H).

### [Preparation Example 96]

### Preparation of Compound 307, (R)-1-((S)-2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid, and Compound 308, (R)-1-((R)-2-((3'-(5-(2-(4-carboxybicyclo[2.2.1]heptan-1-yl)ethyl)-1-methyl-4,5,6,7-tetrahydro-1H-imidazo[4,5-c]pyridine-2-carboxamido)-2,2'-dichloro-[1,1'-biphenyl]-3-yl)carbamoyl)-4,5,6,7-tetrahydropyrazolo[1,5-a]pyridin-4-yl)pyrrolidine-3-carboxylic acid,

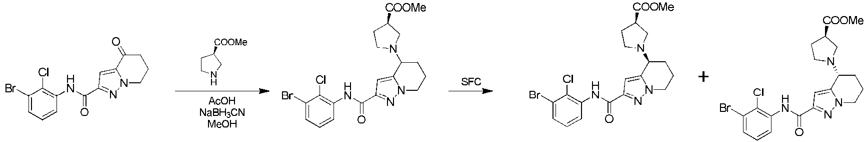

A mixture of N-(3-bromo-2-chloro-phenyl)-4-oxo-6,7-dihydro-5H-pyrazolo[1,5-a]pyridine-2-carboxamide (2.0 g, 5.43 mmol, 1 eq) and methyl (3R)-pyrrolidine-3-carboxylate (3.50 g, 27.13 mmol, 5 eq) in DMF (15 mL) / MeOH (15 mL) was treated with NaBH₃CN (1.70 g, 27.13 mmol, 5 eq) and DIPEA (3.51 g, 27.13 mmol, 4.73 mL, 5 eq). The reaction mixture was stirred at 60 °C for 12 hours. After completion, the mixture was cooled to room temperature and concentrated under reduced pressure. The residue was purified by reversed-phase HPLC (column: Phenomenex Luna C18, 250 * 100 mm * 10 µm; mobile phase: [water (NH₄HCO₃)-ACN]; gradient: 55%-85% B over 20 min). The resulting material was further separated by SFC (column: Daicel Chiralpak IG, 250 * 30 mm * 10 µm; mobile phase: CO₂/EtOH (0.1% NH₃H₂O); B = 55%, isocratic elution mode) as two diastereomers to afford each (R)-methyl 1-((S)-2-((3-bromo-2-chlorophenyl)carbamoyl)-4,5,6,7-tetrahydropyrazolo[1,5-a]pyridin-4-yl)pyrrolidine-3-carboxylate (Rt = 1.161 min, 1.0 g) and (R)-methyl 1-((R)-2-((3-bromo-2-chlorophenyl)carbamoyl)-4,5,6,7-tetrahydropyrazolo[1,5-a]pyridin-4-yl)pyrrolidine-3-carboxylate (Rt = 1.616 min, 1.0 g) as white solids.

LCMS m/z 483.0 [M+1]⁺

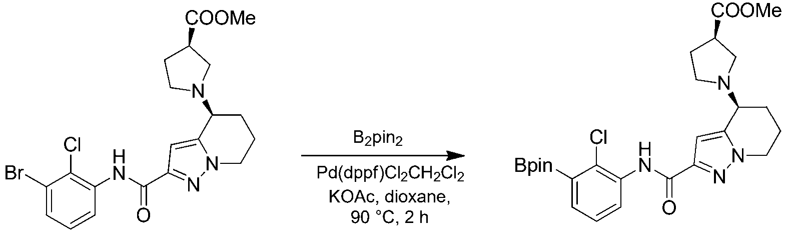

A mixture of methyl (3R)-1-[(4S)-2-[(3-bromo-2-chloro-phenyl)carbamoyl]-4,5,6,7-tetrahydropyrazolo[1,5-a]pyridin-4-yl]pyrrolidine-3-carboxylate (300 mg, 622.70 µmol, 1 eq), 4,4,5,5-tetramethyl-2-(4,4,5,5-tetramethyl-1,3,2-dioxaborolan-2-yl)-1,3,2-dioxaborolane (474.38 mg, 1.87 mmol, 3 eq), KOAc (183.34 mg, 1.87 mmol, 3 eq), and Pd(dppf)Cl₂·CH₂Cl₂ (50.85 mg, 62.27 µmol, 0.1 eq) in dioxane (8 mL) was stirred under a nitrogen atmosphere at 90 °C for 2 hours. After completion, the reaction mixture was cooled to room temperature and filtered. The filtrate was concentrated under reduced pressure, and the residue was purified by flash silica gel chromatography (Petroleum ether/Ethyl acetate = 100/1 to 10/1) to afford methyl (3R)-1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (312 mg, 85.2% yield) as a colorless oil.

LCMS m/z 529.3 [M+1]⁺

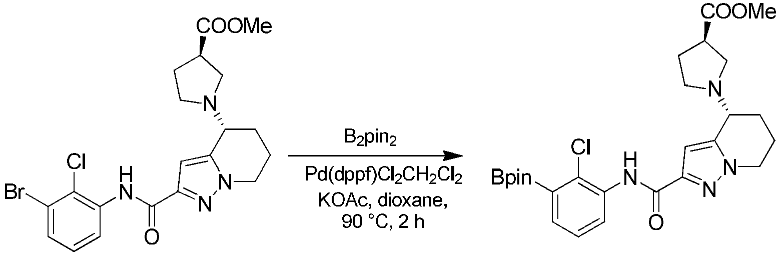

A mixture of methyl (3R)-1-[(4R)-2-[(3-bromo-2-chloro-phenyl)carbamoyl]-4,5,6,7-tetrahydropyrazolo[1,5-a]pyridin-4-yl]pyrrolidine-3-carboxylate (300 mg, 622.70 µmol, 1 eq), 4,4,5,5-tetramethyl-2-(4,4,5,5-tetramethyl-1,3,2-dioxaborolan-2-yl)-1,3,2-dioxaborolane (474.38 mg, 1.87 mmol, 3 eq), KOAc (183.34 mg, 1.87 mmol, 3 eq), and Pd(dppf)Cl₂·CH₂Cl₂ (50.85 mg, 62.27 µmol, 0.1 eq) in dioxane (3 mL) was stirred under a nitrogen atmosphere at 90 °C for 2 hours. After completion, the reaction mixture was cooled to room temperature and filtered. The filtrate was concentrated under reduced pressure, and the residue was purified by flash silica gel chromatography (DCM:MeOH = 10/1) to afford methyl (3R)-1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (318 mg, 86.91% yield) as a colorless oil.

LCMS m/z 529.3 [M+1]⁺

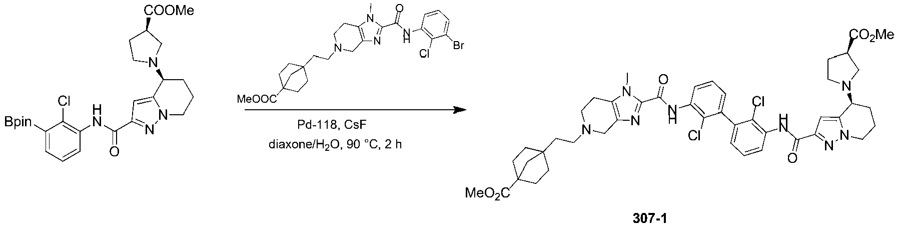

A mixture of methyl 4-[2-[2-[(3-bromo-2-chloro-phenyl)carbamoyl]-1-methyl-6,7-dihydro-4H-imidazo[4,5-c]pyridin-5-yl]ethyl]norbornane-1-carboxylate (150 mg, 272.78 µmol, 1 eq), methyl (3R)-1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (173.11 mg, 327.34 µmol, 1.2 eq), CsF (124.31 mg, 818.35 µmol, 3 eq), and di-tert-butyl(cyclopentyl)phosphane;dichloropalladium;iron (17.78 mg, 27.28 µmol, 0.1 eq) in a mixture of dioxane (8 mL) / H₂O (2 mL) was stirred under a nitrogen atmosphere at 90 °C for 2 hours. After completion, the reaction mixture was cooled to room temperature and filtered. The filtrate was concentrated under reduced pressure, and the residue was purified by prep-HPLC (column: Phenomenex Luna C18, 150 * 40 mm * 15 µm; mobile phase: [water (FA)-ACN]; gradient: 13% to 43% B over 15 min) to afford Compound 307-41 (82 mg, 32.7% yield) as a white solid.

LCMS m/z 871.3 [M+1]⁺

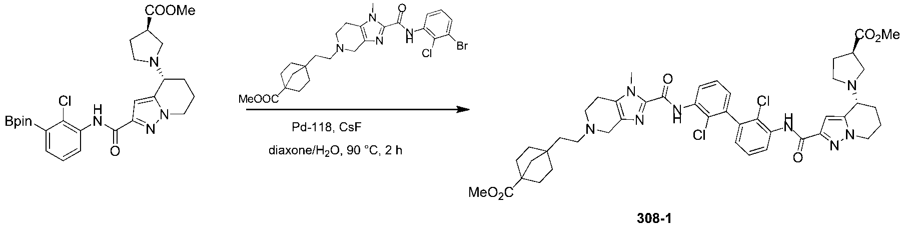

A mixture of methyl 4-[2-[2-[(3-bromo-2-chloro-phenyl)carbamoyl]-1-methyl-6,7-dihydro-4H-imidazo[4,5-c]pyridin-5-yl]ethyl]norbornane-1-carboxylate (150 mg, 272.78 µmol, 1 eq), methyl (3R)-1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (173.11 mg, 327.34 µmol, 1.2 eq), CsF (124.31 mg, 818.35 µmol, 3 eq), and di-tert-butyl(cyclopentyl)phosphane;dichloropalladium;iron (17.78 mg, 27.28 µmol, 0.1 eq) in a mixture of dioxane (8 mL) / H₂O (2 mL) was stirred under a nitrogen atmosphere at 90 °C for 2 hours. After completion, the reaction mixture was cooled to room temperature and filtered. The filtrate was concentrated under reduced pressure, and the residue was purified by prep-HPLC (column: Phenomenex Luna C18, 150 * 40 mm * 15 µm; mobile phase: [water (FA)-ACN]; gradient: 13%-43% B over 15 min) to afford Compound 308-1 (70 mg, 27.9% yield) as a white solid.

LCMS m/z 871.4 [M+1]⁺

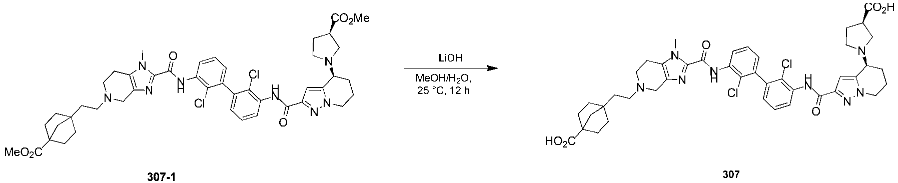

A solution of methyl (3R)-1-[(4S)-2-[[2-chloro-3-[2-chloro-3-[[5-[2-(4-methoxycarbonylnorbornan-1-yl)ethyl]-1-methyl-6,7-dihydro-4H-imidazo[4,5-c]pyridine-2-carbonyl]amino]phenyl]phenyl]carbamoyl]-4,5,6,7-tetrahydropyrazolo[1,5-a]pyridin-4-yl]pyrrolidine-3-carboxylate (80 mg, 91.76 µmol, 1 eq) in a mixture of H₂O (3 mL) / MeOH (10 mL) was treated with LiOH·H₂O (11.55 mg, 275.28 µmol, 3 eq). The reaction mixture was stirred at room temperature for 12 hours. After completion, the mixture was filtered and the filtrate was concentrated under reduced pressure. The residue was purified by prep-HPLC (column: Waters Xbridge, 150 * 25 mm * 5 µm; mobile phase: [water (NH₄HCO₃)-ACN]; gradient: 12%-42% B over 9 min) to afford Compound 307 (48 mg, 58.9% yield) as a white solid.

LCMS m/z 843.4 [M+1]⁺

¹H NMR (400 MHz, DMSO-*d₆* ) δ = 9.89 (s, 1H), 9.55 (s, 1H), 8.36 (d, *J* = 7.6 Hz, 1H), 8.26 (t, *J* = 6.0 Hz, 1H), 7.48 (t, *J* = 7.9 Hz, 2H), 7.15 (t, *J* = 8.4 Hz, 2H), 6.71 (s, 1H), 4.21 - 4.11 (m, 2H), 3.89 (s, 3H), 3.82 (br s, 5H), 2.93 - 2.78 (m, 3H), 2.77 - 2.71 (m, 3H), 2.66 (br d, *J* = 4.0 Hz, 2H), 2.24 (br s, 2H), 1.91 (br d, *J* = 6.6 Hz, 4H), 1.89 - 1.77 (m, 4H), 1.74 - 1.68 (m, 2H), 1.55 - 1.48 (m, 4H), 1.43 (s, 2H), 1.37 (br d, *J* = 11.8 Hz, 2H).

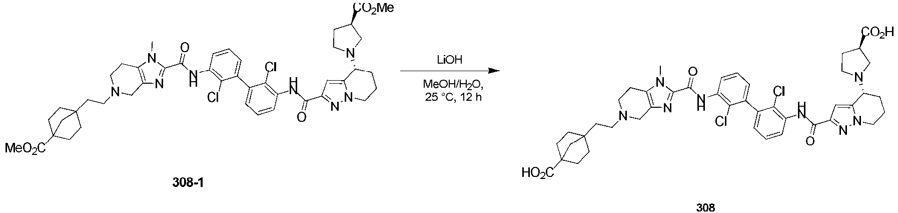

A solution of methyl (3R)-1-[(4R)-2-[[2-chloro-3-[2-chloro-3-[[5-[2-(4-methoxycarbonylnorbornan-1-yl)ethyl]-1-methyl-6,7-dihydro-4H-imidazo[4,5-c]pyridine-2-carbonyl]amino]phenyl]phenyl]carbamoyl]-4,5,6,7-tetrahydropyrazolo[1,5-a]pyridin-4-yl]pyrrolidine-3-carboxylate (80.00 mg, 91.76 µmol, 1 eq) in a mixture of H₂O (3 mL) / MeOH (10 mL) was treated with LiOH·H₂O (11.55 mg, 275.28 µmol, 3 eq). The reaction mixture was stirred at room temperature for 12 hours. After completion, the mixture was filtered and the filtrate was concentrated under reduced pressure. The residue was purified by prep-HPLC (column: Waters Xbridge, 150 * 25 mm * 5 µm; mobile phase: [water (NH₄HCO₃)-ACN]; gradient: 12%-42% B over 9 min) to afford Compound (3R)-1-[(4R)-2-[[3-[3-[[5-[2-(4-carboxynorbornan-1-yl)ethyl]-1-methyl-6,7-dihydro-4H-imidazo[4,5-c]pyridine-2-carbonyl]amino]-2-chlorophenyl]-2-chlorophenyl]carbamoyl]-4,5,6,7-tetrahydropyrazolo[1,5-a]pyridin-4-yl]pyrrolidine-3-carboxylic acid (compound 308, R) (48 mg, 58.9% yield) as a white solid.

LCMS m/z 845.3 [M+1]⁺

¹H NMR (400 MHz, DMSO-*d₆* )) δ = 9.89 (s, 1H), 9.56 (s, 1H), 8.37 (dd, *J* = 1.3, 8.3 Hz, 1H), 8.29 - 8.24 (m, 1H), 7.49 (t, *J* = 7.8 Hz, 2H), 7.15 (dd, *J* = 7.9, 9.3 Hz, 2H), 6.72 (s, 1H), 4.25 - 4.13 (m, 2H), 3.89 (s, 3H), 3.81 (br s, 2H), 3.41 (br s, 4H), 2.97 - 2.86 (m, 3H), 2.79 - 2.69 (m, 4H), 2.69 - 2.60 (m, 3H), 2.22 (br s, 1H), 2.01 - 1.90 (m, 4H), 1.89 - 1.79 (m, 3H), 1.78 - 1.65 (m, 2H), 1.55 - 1.46 (m, 4H), 1.43 (s, 2H), 1.37 (br d, J = 11.4 Hz, 2H).

### [Preparation Example 97]

### 97-1. Preparation of Compound 309, (S)-4-((R)-2-(hydroxymethyl)piperidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide and Compound 309-1, (R)-4-((R)-2-(hydroxymethyl)piperidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

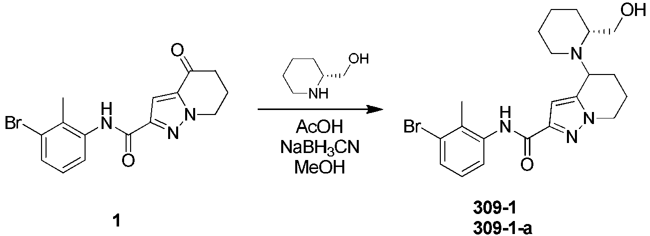

A solution of N-(3-bromo-2-methylphenyl)-4-oxo-6,7-dihydro-5H-pyrazolo[1,5-a]pyridine-2-carboxamide (2.0 g, 5.74 mmol, 1 eq) and (2R)-2-piperidylmethanol (6.62 g, 57.44 mmol, 10 eq) in MeOH (20 mL) was treated with AcOH (1.72 g, 28.72 mmol, 1.64 mL, 5 eq) and NaBH₃CN (1.80 g, 28.72 mmol, 5 eq). The reaction mixture was stirred at 120 °C for 6 hours. After cooling to room temperature, the mixture was purified by reversed-phase HPLC (column: Phenomenex Luna C18, 150 × 40 mm, 15 µm; mobile phase: [water (TFA)-ACN]; gradient: 18%-48% B over 15 min), followed by an additional reversed-phase HPLC purification (column: Waters Xbridge Prep OBD C18, 150 * 40 mm * 10 µm; mobile phase: [water (NH₄HCO₃)-ACN]; gradient: 40%-70% B over 20 min). The resulting material was further purified by chiral SFC (column: Daicel Chiralpak IG, 250 × 30 mm, 10 µm; mobile phase: CO₂-EtOH (0.1% NH₃H₂O)); B%:55%, isocratic elution mode) to afford each (4S)-N-(3-bromo-2-methylphenyl)-4-[(2R)-2-(hydroxymethyl)-1-piperidyl]-4,5,6,7-tetrahydropyrazolo[1,5-a]pyridine-2-carboxamide (Compound 309-1) (141 mg, 5.43% yield) and (4R)-N-(3-bromo-2-methylphenyl)-4-[(2R)-2-(hydroxymethyl)-1-piperidyl]-4,5,6,7-tetrahydropyrazolo[1,5-a]pyridine-2-carboxamide (Compound 309-1-a) (105 mg, 4.05% yield) as a white solid.

LCMS m/z 447.0 [M+1]⁺.

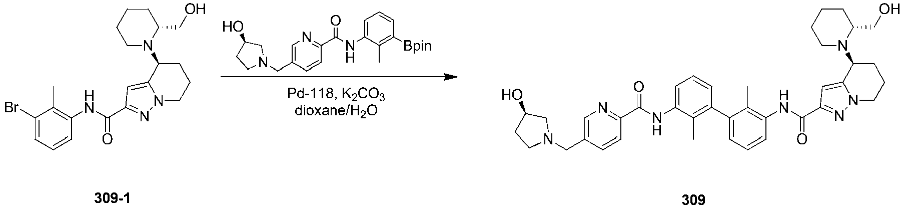

A mixture of (4S)-N-(3-bromo-2-methylphenyl)-4-[(2R)-2-(hydroxymethyl)-1-piperidyl]-4,5,6,7-tetrahydropyrazolo[1,5-a]pyridine-2-carboxamide (100 mg, 223.53 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (127.09 mg, 290.59 µmol, 1.3 eq), K₂CO₃ (92.68 mg, 670.59 µmol, 3 eq), and di-tert-butyl(cyclopentyl)phosphane-dichloropalladium-iron (14.57 mg, 22.35 µmol, 0.1 eq) in dioxane (8 mL) / H₂O (2 mL) was stirred under a nitrogen atmosphere at 90 °C for 5 hours. After cooling to room temperature, the reaction mixture was filtered, and the filtrate was concentrated under reduced pressure. The residue was purified by prep-TLC (SiO₂, Ethyl acetate/Methanol = 5:1), followed by reversed-phase HPLC (column: Waters Xbridge, 150 * 25 mm * 5 µm; mobile phase: [water (NH₄HCO₃)-ACN]; gradient: 25%-55% B over 15 min) to afford Compound 309 (47 mg, 30.4% yield) as a white solid.

LCMS m/z 678.4 [M+1]⁺.

¹H NMR (400 MHz, DMSO-d₆) δ ppm 1.23 - 1.68 (m, 8 H) 1.74 - 1.92 (m, 2 H) 1.95 (s, 3 H) 2.02 (s, 4 H) 2.06 - 2.18 (m, 2 H) 2.30 - 2.39 (m, 1 H) 2.40 - 2.48 (m, 1 H) 2.57 - 2.83 (m, 4 H) 3.51 - 3.60 (m, 1 H) 3.64 - 3.79 (m, 2 H) 3.97 - 4.08 (m, 1 H) 4.19 (br d, *J*=11.*26* Hz, 2 H) 4.39 - 4.49 (m, 1 H) 4.65 (br t, *J*=4.63 Hz, 1 H) 4.75 (br d, *J*=4.13 Hz, 1 H) 6.68 (s, 1 H) 6.98 (br d, *J*=7.38 Hz, 2 H) 7.30 (dt, *J*=15.13, 7.69 Hz, 2 H) 7.59 (br t, *J*=7.00 Hz, 1 H) 7.87 (br d, *J*=7.88 Hz, 1 H) 7.98 (br d, *J*=8.00 Hz, 1 H) 8.14 (br d, *J*=7.88 Hz, 1 H) 8.65 (s, 1 H) 9.51 (br d, *J*=3.75 Hz, 1 H) 10.33 (s, 1 H).

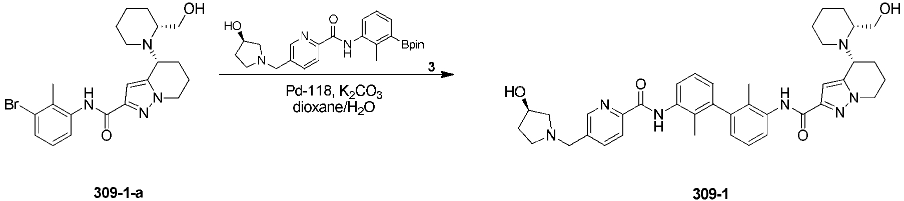

A mixture of (4R)-N-(3-bromo-2-methylphenyl)-4-[(2R)-2-(hydroxymethyl)-1-piperidyl]-4,5,6,7-tetrahydropyrazolo[1,5-a]pyridine-2-carboxamide 309-1-a (100.00 mg, 223.53 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide 3 (127.09 mg, 290.59 µmol, 1.3 eq), K₂CO₃ (92.68 mg, 670.59 µmol, 3 eq), and di-tert-butyl(cyclopentyl)phosphane;dichloropalladium;iron (14.57 mg, 22.35 µmol, 0.1 eq) in dioxane (8 mL) / H₂O (2 mL) was stirred under a nitrogen atmosphere at 90 °C for 5 hours. After cooling to room temperature, the reaction mixture was filtered, and the filtrate was concentrated under reduced pressure. The residue was purified by prep-TLC (SiO₂, Ethyl acetate/Methanol = 5:1), followed by reversed-phase HPLC (column: Waters Xbridge, 150 * 25 mm * 5 µm; mobile phase: [water (NH₄HCO₃)-ACN]; gradient: 28%-58% B over 15 min) to afford Compound 309-1 (23 mg, 14.4% yield) as a white solid.

LCMS m/z 678.4 [M+1]⁺.

¹H NMR (400 MHz, DMSO-d₆) δ ppm 1.13 - 1.37 (m, 5 H) 1.44 - 1.75 (m, 6 H) 1.94 (s, 3 H) 2.01 (s, 3 H) 2.13 (br d, *J*=11.01 Hz, 2 H) 2.35 (br dd, *J*=9.57, 3.19 Hz, 1 H) 2.41 - 2.46 (m, 1 H) 2.57 - 2.74 (m, 3 H) 3.52 - 3.57 (m, 2 H) 3.66 - 3.78 (m, 2 H) 3.98 - 4.07 (m, 1 H) 4.13 - 4.26 (m, 2 H) 4.39 - 4.51 (m, 1 H) 4.67 (br t, *J*=4.69 Hz, 1 H) 4.77 (d, *J*=4.50 Hz, 1 H) 6.61 (s, 1 H) 6.98 (br d, *J*=7.50 Hz, 2 H) 7.30 (dt, *J*=16.29, 7.93 Hz, 2 H) 7.57 (br t, *J*=7.75 Hz, 1 H) 7.86 (br d, *J*=7.88 Hz, 1 H) 7.99 (br d, *J*=8.00 Hz, 1 H) 8.14 (d, J=8.00 Hz, 1 H) 8.65 (s, 1 H) 9.51 (br d, *J*=5.88 Hz, 1 H) 10.34 (s, 1 H).

### 97-2. Preparation of Compound 310, (S)-4-((R)-2-(hydroxymethyl)pyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide, and Compound 310-1, ((R)-4-((R)-2-(hydroxymethyl)pyrrolidin-1-yl)-N-(3'-(5-(((R)-3-hydroxypyrrolidin-1-yl)methyl)picolinamido)-2,2'-dimethyl-[1,1'-biphenyl]-3-yl)-4,5,6,7-tetrahydropyrazolo[1,5-a]pyridine-2-carboxamide

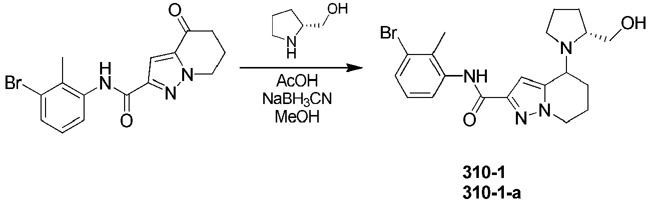

A mixture of N-(3-bromo-2-methylphenyl)-4-oxo-6,7-dihydro-5H-pyrazolo[1,5-a]pyridine-2-carboxamide (1.0 g, 2.87 mmol, 1 eq) and [(2R)-pyrrolidin-2-yl]methanol (2.90 g, 28.72 mmol, 10 eq) in MeOH (20 mL) was treated with AcOH (862.34 mg, 14.36 mmol, 822.05 µL, 5 eq) and NaBH₃CN (902.40 mg, 14.36 mmol, 5 eq). The reaction mixture was stirred at 60 °C for 12 hours. After cooling to room temperature, the mixture was purified by reversed-phase HPLC (column: Waters Xbridge Prep OBD C18, 150 * 40 mm * 10 µm; mobile phase: [water (NH₄HCO₃)-ACN]; gradient: 32%-62% B over 20 min), followed by additional purification by SFC (column: DAICEL CHIRALPAK IG, 250 * 30 mm * 10 µm; mobile phase: CO₂-EtOH (0.1% NH₃H₂O); B = 55%, isocratic elution mode). This afforded the diastereomers (4S)-N-(3-bromo-2-methylphenyl)-4-[(2R)-2-(hydroxymethyl)pyrrolidin-1-yl]-4,5,6,7-tetrahydropyrazolo[1,5-a]pyridine-2-carboxamide 310-1 (352 mg, 27.7% yield) and (4R)-N-(3-bromo-2-methylphenyl)-4-[(2R)-2-(hydroxymethyl)pyrrolidin-1-yl]-4,5,6,7-tetrahydropyrazolo[1,5-a]pyridine-2-carboxamide 310-1-a (407 mg, 31.3% yield), each obtained as a white solid.

LCMS m/z 435.0 [M+3]⁺.

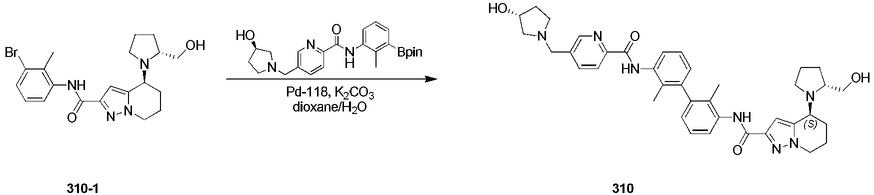

A mixture of (4S)-N-(3-bromo-2-methylphenyl)-4-[(2R)-2-(hydroxymethyl)pyrrolidin-1-yl]-4,5,6,7-tetrahydropyrazolo[1,5-a]pyridine-2-carboxamide 310-1 (150.00 mg, 346.15 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (181.66 mg, 415.38 µmol, 1.2 eq), di-tert-butyl(cyclopentyl)phosphane;dichloropalladium;iron (22.56 mg, 34.61 µmol, 0.1 eq), and K₂CO₃ (143.52 mg, 1.04 mmol, 3 eq) in dioxane (8 mL) / H₂O (2 mL) was stirred at 90 °C for 6 hours under a nitrogen atmosphere. After cooling to room temperature, the reaction mixture was filtered and the filtrate was concentrated under reduced pressure. The residue was purified by prep-TLC (SiO₂, DCM:MeOH = 10:1), followed by further purification by reversed-phase HPLC (column: Waters Xbridge 150 * 25 mm * 5 µm; mobile phase: [water (NH₄HCO₃)-ACN]; gradient: 32%-62% B over 9 min) to afford Compound 310 (42 mg, 17.7% yield) as a white solid.

LCMS m/z 664.6 [M+1]⁺.

¹H NMR (400 MHz, DMSO-d₆) δ ppm 1.53 - 1.72 (m, 4 H) 1.73 - 1.87 (m, 2 H) 1.95 (s, 3 H) 1.98 - 2.05 (m, 5 H) 2.08 (s, 1 H) 2.09 - 2.19 (m, 1 H) 2.35 (dd, *J*=9.57, 3.56 Hz, 1 H) 2.39 - 2.48 (m, 2 H) 2.62 (q, *J*=7.80 Hz, 1 H) 2.58 - 2.66 (m, 1 H) 2.70 (dd, *J*=9.51, 6.13 Hz, 1 H) 2.76 - 2.83 (m, 1 H) 3.14 - 3.25 (m, 2 H) 3.65 - 3.78 (m, 2 H) 4.03 - 4.13 (m, 1 H) 4.14 - 4.25 (m, 2 H) 4.28 (dd, *J*=9.57, 5.57 Hz, 1 H) 4.49 (t, *J*=5.25 Hz, 1 H) 4.74 (d, *J*=4.50 Hz, 1 H) 6.65 (s, 1 H) 6.99 (d, *J*=7.63 Hz, 2 H) 7.30 (dt, *J*=15.23, 7.71 Hz, 2 H) 7.54 - 7.60 (m, 1 H) 7.84 - 7.90 (m, 1 H) 7.96 - 8.01 (m, 1 H) 8.11 - 8.17 (m, 1 H) 8.62 - 8.67 (m, 1 H) 9.56 (d, *J*=3.88 Hz, 1 H) 10.33 (s, 1 H).

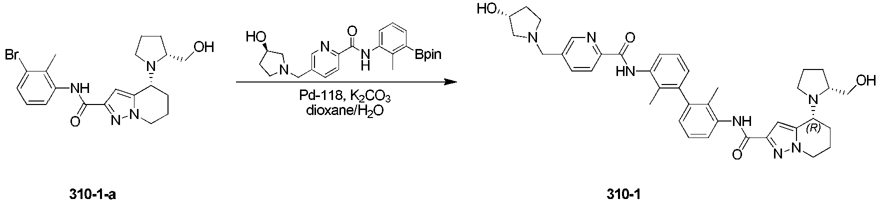

A mixture of (4R)-N-(3-bromo-2-methylphenyl)-4-[(2R)-2-(hydroxymethyl)pyrrolidin-1-yl]-4,5,6,7-tetrahydropyrazolo[1,5-a]pyridine-2-carboxamide 310-1-a (100 mg, 230.77 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (131.20 mg, 299.99 µmol, 1.3 eq), di-tert-butyl(cyclopentyl)phosphane;dichloropalladium;iron (15.04 mg, 23.08 µmol, 0.1 eq), and K₂CO₃ (95.68 mg, 692.30 µmol, 3 eq) in dioxane (8 mL) / H₂O (2 mL) was stirred at 90 °C for 6 hours under a nitrogen atmosphere. After cooling to room temperature, the reaction mixture was filtered and the filtrate was concentrated under reduced pressure. The residue was further purified by prep-TLC (SiO₂, DCM:MeOH = 10:1) to afford Compound 310-1 (29 mg, 18.7% yield) as a white solid.

LCMS m/z 664.4 [M+1]⁺.

¹H NMR (400 MHz, DMSO-*d₆*) δ ppm 1.51 - 1.83 (m, 6 H) 1.95 (s, 3 H) 2.02 (s, 3 H) 2.15 - 2.23 (m, 1 H) 2.35 (dd, *J*=9.57, 3.56 Hz, 1 H) 2.44 (td, *J*=8.29, 5.69 Hz, 1 H) 2.52 - 2.57 (m, 1 H) 2.62 (q, *J*=7.88 Hz, 1 H) 2.67 - 2.81 (m, 2 H) 2.91 - 2.99 (m, 1 H) 3.23 (dt, *J*=10.51, 5.50 Hz, 1 H) 3.28 - 3.34 (m, 1 H) 3.64 - 3.78 (m, 2 H) 4.02 - 4.13 (m, 1 H) 4.15 - 4.26 (m, 3 H) 4.43 (t, *J*=5.32 Hz, 1 H) 4.74 (d, *J*=4.50 Hz, 1 H) 6.70 (s, 1 H) 6.98 (d, *J*=7.50 Hz, 2 H) 7.30 (dt, *J*=16.07, 7.85 Hz, 2 H) 7.59 (dd, *J*=7.50, 5.00 Hz, 1 H) 7.87 (d, *J*=7.75 Hz, 1 H) 7.93 - 8.02 (m, 1 H) 8.14 (d, *J*=8.00 Hz, 1 H) 8.65 (d, *J*=1.38 Hz, 1 H) 9.51 (d, *J*=3.75 Hz, 1 H) 10.33 (s, 1 H).

### [Preparation Example 98]

### Preparation of Compound 311, (4S,4'S)-N,N'-(2,2'-dichloro-[1,1'-biphenyl]-3,3'-diyl)bis(4-(dimethylamino)-4,5,6,7-tetrahydropyrazolo[1,5-a]pyridine-2-carboxamide)

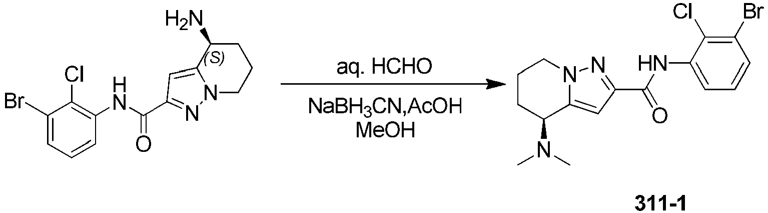

A mixture of (4S)-4-amino-N-(3-bromo-2-chloro-phenyl)-4,5,6,7-tetrahydropyrazolo[1,5-a]pyridine-2-carboxamide (1.0 g, 2.71 mmol, 1 eq) and formaldehyde (263.45 mg, 3.25 mmol, 241.69 µL, 1.2 eq) in MeOH (15 mL) was treated with NaBH₃CN (1.70 g, 27.05 mmol, 10 eq) and AcOH (162.45 mg, 2.71 mmol, 154.87 µL, 1 eq). The reaction mixture was stirred at room temperature for 12 hours. After completion, the mixture was concentrated under reduced pressure, and the residue was purified by reversed-phase HPLC (column: Waters Xbridge Prep OBD C18, 150 * 40 mm * 10 µm; mobile phase: [water (NH₄HCO₃)-ACN]; gradient: 45-75% B over 20 min) to afford Compound 311-1 (657 mg, 54.9% yield) as a white solid.

LCMS m/z 399.0 [M+3]⁺.

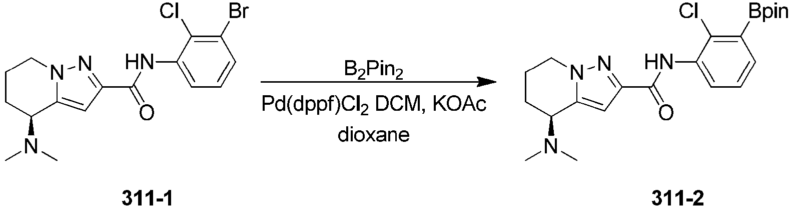

A mixture of (4S)-N-(3-bromo-2-chloro-phenyl)-4-(dimethylamino)-4,5,6,7-tetrahydropyrazolo[1,5-a]pyridine-2-carboxamide 311-1 (300.00 mg, 754.34 µmol, 1 eq), 4,4,5,5-tetramethyl-2-(4,4,5,5-tetramethyl-1,3,2-dioxaborolan-2-yl)-1,3,2-dioxaborolane (383.11 mg, 1.51 mmol, 2 eq), Pd(dppf)Cl₂·CH₂Cl₂ (61.60 mg, 75.43 µmol, 0.1 eq), and KOAc (222.09 mg, 2.26 mmol, 3 eq) in dioxane (6 mL) was stirred under a nitrogen atmosphere at 90 °C for 12 hours. After completion, the reaction mixture was cooled to room temperature, filtered, and the filtrate was concentrated under reduced pressure. The resulting residue was purified by prep-TLC (SiO₂, ethyl acetate/methanol = 10:1) to afford Compound 311-2 (512 mg, 99.1% yield) as a white solid.

LCMS m/z 445.2 [M+1]⁺.

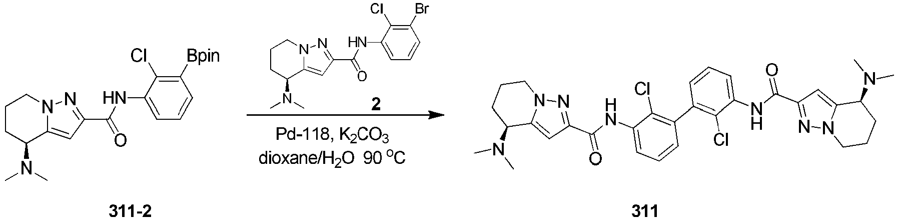

A mixture of (4S)-N-[2-chloro-3-(4,4,5,5-tetramethyl-1,3,2-dioxaborolan-2-yl)phenyl]-4-(dimethylamino)-4,5,6,7-tetrahydropyrazolo[1,5-a]pyridine-2-carboxamide 311-2 (134.20 mg, 301.74 µmol, 1.2 eq), (4S)-N-(3-bromo-2-chloro-phenyl)-4-(dimethylamino)-4,5,6,7-tetrahydropyrazolo[1,5-a]pyridine-2-carboxamide 2 (100.00 mg, 251.45 µmol, 1 eq), di-tert-butyl(cyclopentyl)phosphane;dichloropalladium;iron (16.39 mg, 25.14 µmol, 0.1 eq), and K₂CO₃ (104.25 mg, 754.34 µmol, 3 eq) in dioxane (8 mL) / H₂O (2 mL) was stirred under a nitrogen atmosphere at 90 °C for 12 hours. After completion, the reaction mixture was cooled to room temperature and filtered. The filtrate was concentrated under reduced pressure, and the residue was purified by prep-TLC (SiO₂, Ethyl acetate:Methanol = 10:1), followed by reversed-phase HPLC (column: Phenomenex Luna C18, 150 * 25 mm * 10 µm; mobile phase: [water (NH₄HCO₃)-ACN]; gradient: 56-86% B over 10 min) to afford Compound 311 (41 mg, 25.4% yield) as a white solid.

LCMS m/z 637.2 [M+3]⁺.

¹H NMR (400 MHz, DMSO-d₆) δ ppm 1.63 - 1.75 (m, 2 H) 1.83 - 2.00 (m, 4 H) 2.11 - 2.20 (m, 2 H) 2.25 (s, 12 H) 3.92 (br dd, *J*=9.69, 5.32 Hz, 2 H) 4.02 - 4.13 (m, 2 H) 4.16 - 4.25 (m, 2 H) 6.68 (s, 2 H) 7.16 (dd, *J*=7.57, 1.31 Hz, 2 H) 7.49 (t, *J*=7.88 Hz, 2 H) 8.27 (ddd, *J*=8.19, 3.50, 1.44 Hz, 2 H) 9.55 (s, 2 H).

### [Preparation Example 99]

### Preparation of Compound 312, (4S,4'S)-N,N'-(2,2'-dimethyl-[1,1'-biphenyl]-3,3'-diyl)bis(4-(methylamino)-4,5,6,7-tetrahydropyrazolo[1,5-a]pyridine-2-carboxamide)

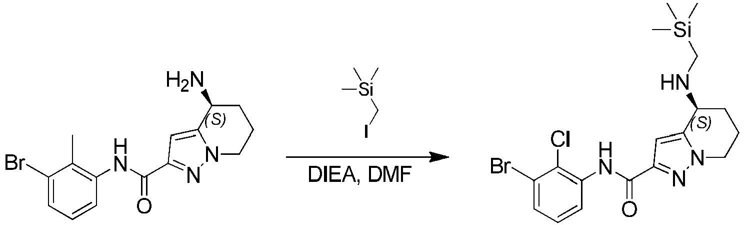

A mixture of 4-amino-N-(3-bromo-2-chloro-phenyl)-4,5,6,7-tetrahydropyrazolo[1,5-a]pyridine-2-carboxamide (440 mg, 1.19 mmol, 1 eq) and iodomethyl(trimethyl)silane (764.62 mg, 3.57 mmol, 530.62 µL, 3 eq) in DMF (10 mL) was treated with DIPEA (307.69 mg, 2.38 mmol, 414.67 µL, 2 eq). The reaction mixture was stirred at 80 °C for 12 hours. After completion, the mixture was cooled to room temperature and concentrated under reduced pressure. The residue was purified by column chromatography (SiO₂, Petroleum ether/Ethyl acetate = 0/1) to afford N-(3-bromo-2-chloro-phenyl)-4-(trimethylsilylmethylamino)-4,5,6,7-tetrahydropyrazolo[1,5-a]pyridine-2-carboxamide (290 mg, 42.7% yield) as a yellow solid.

LCMS m/z 457.0 [M+1]⁺.

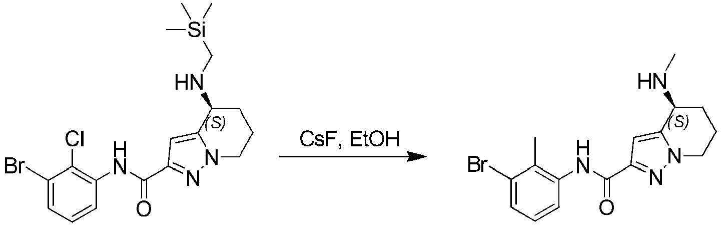

A solution of (4S)-N-(3-bromo-2-methyl-phenyl)-4-(trimethylsilylmethylamino)-4,5,6,7-tetrahydropyrazolo[1,5-a]pyridine-2-carboxamide (364 mg, 835.95 µmol, 1 eq) in ethanol (20 mL) was treated with CsF (2.54 g, 16.72 mmol, 617.18 µL, 20 eq). The reaction mixture was stirred at 90 °C for 12 hours. After completion, the mixture was cooled to room temperature, filtered, and the filtrate was concentrated under reduced pressure. The residue was purified by reversed-phase HPLC (0.1% NH₃·H₂O condition; column: Waters XBridge Prep OBD C18, 150 * 40 mm * 10 µm; mobile phase: [water (NH₄HCO₃)-ACN]; gradient: 40-70% B over 20 min) to afford (4S)-N-(3-bromo-2-methylphenyl)-4-(methylamino)-4,5,6,7-tetrahydropyrazolo[1,5-a]pyridine-2-carboxamide (60 mg, 19.7% yield) as a white solid.

LCMS m/z 363.0 [M+1]⁺.

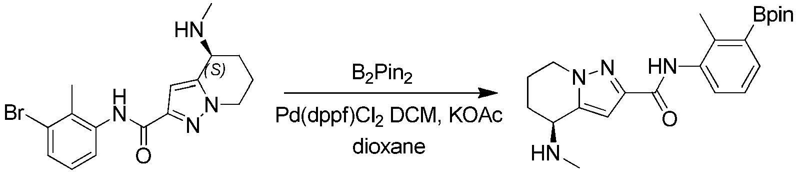

A mixture of (4S)-N-(3-bromo-2-methyl-phenyl)-4-(methylamino)-4,5,6,7-tetrahydropyrazolo[1,5-a]pyridine-2-carboxamide (70 mg, 192.70 µmol, 1 eq), 4,4,5,5-tetramethyl-2-(4,4,5,5-tetramethyl-1,3,2-dioxaborolan-2-yl)-1,3,2-dioxaborolane (146.80 mg, 578.11 µmol, 3 eq), AcOK (37.82 mg, 385.41 µmol, 2 eq), and Pd(dppf)Cl₂·CH₂Cl₂ (15.74 mg, 19.27 µmol, 0.1 eq) in dioxane (3 mL) was stirred under a nitrogen atmosphere at 90 °C for 12 hours. After completion, the reaction mixture was cooled to room temperature, filtered, and the filtrate was concentrated under reduced pressure. The residue was purified by prep-TLC (SiO₂, DCM/MeOH = 0:1) to afford (4S)-4-(methylamino)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (40 mg, 47.5% yield) as a white solid.

LCMS m/z 411.2 [M+1]⁺.

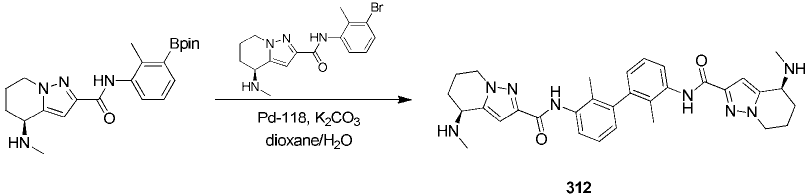

A mixture of (4S)-N-(3-bromo-2-methyl-phenyl)-4-(methylamino)-4,5,6,7-tetrahydropyrazolo[1,5-a]pyridine-2-carboxamide (35.41 mg, 97.49 µmol, 1 eq), (4S)-4-(methylamino)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (40 mg, 97.49 µmol, 1 eq), K₂CO₃ (26.95 mg, 194.97 µmol, 2 eq), and ditert-butyl(cyclopentyl)phosphane;dichloropalladium;iron (6.35 mg, 9.75 µmol, 0.1 eq) in dioxane (3 mL) / H₂O (0.5 mL) was stirred under a nitrogen atmosphere at 80 °C for 4 hours. After completion, the reaction mixture was cooled to room temperature, filtered, and the filtrate was concentrated under reduced pressure. The residue was purified by reversed-phase HPLC (0.1% NH₃·H₂O condition; column: Waters Xbridge 150 * 25 mm * 5 µm; mobile phase: [water (NH₄HCO₃)-can]; gradient: 15%-45% B over 15 min) to afford Compound 312 (14 mg, 24.8% yield) as a white solid.

LCMS m/z 567.5 [M+1]⁺.

¹H NMR (400 MHz, DMSO-d6) δ ppm 1.63 - 1.73 (m, 2 H) 1.93 (s, 6 H) 1.96 - 2.02 (m, 2 H) 2.18 (br dd, J=13.51, 5.75 Hz, 2 H) 2.32 (s, 6 H) 3.73 - 3.78 (m, 2 H) 4.10 - 4.16 (m, 4 H) 6.71 (s, 2 H) 6.95 (d, J=7.50 Hz, 2 H) 7.26 (t, J=7.69 Hz, 2 H) 7.57 (d, J=7.88 Hz, 2 H) 9.50 (s, 2 H)

### [Preparation Example 100]

### Preparation of Compound 313, (S)-N-(2,2'-dichloro-3'-((S)-4-((2-hydroxyethyl)amino)-4,5,6,7-tetrahydropyrazolo[1,5-a]pyridine-2-carboxamido)-[1,1'-biphenyl]-3-yl)-4-(dimethylamino)-4,5,6,7-tetrahydropyrazolo[1,5-a]pyridine-2-carboxamide, and Compound 314, 2-(((S)-2-((2,2'-dichloro-3'-((S)-4-(dimethylamino)-4,5,6,7-tetrahydropyrazolo[1,5-a]pyridine-2-carboxamido)-[1,1'-biphenyl]-3-yl)carbamoyl)-4,5,6,7-tetrahydropyrazolo[1,5-a]pyridin-4-yl)amino)acetic acid

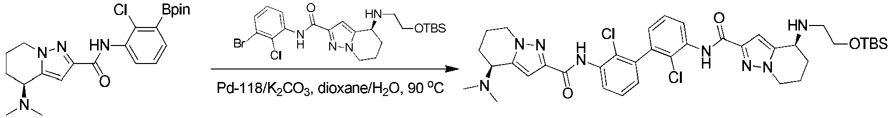

A mixture of (4S)-N-[2-chloro-3-(4,4,5,5-tetramethyl-1,3,2-dioxaborolan-2-yl)phenyl]-4-(dimethylamino)-4,5,6,7-tetrahydropyrazolo[1,5-a]pyridine-2-carboxamide (101.09 mg, 227.29 µmol, 1.2 eq), (4S)-N-(3-bromo-2-chloro-phenyl)-4-[2-[tert-butyl(dimethyl)silyl]oxyethylamino]-4,5,6,7-tetrahydropyrazolo[1,5-a]pyridine-2-carboxamide (100 mg, 189.41 µmol, 1 eq), ditert-butyl(cyclopentyl)phosphane;dichloropalladium;iron (12.34 mg, 18.94 µmol, 0.1 eq), and K₂CO₃ (78.53 mg, 568.23 µmol, 3 eq) in dioxane (8 mL) / H₂O (2 mL) was stirred under a nitrogen atmosphere at 90 °C for 5 hours. After completion, the reaction mixture was cooled to room temperature, filtered, and the filtrate was concentrated under reduced pressure. The residue was purified by prep-TLC (SiO₂, Ethyl acetate:Methanol = 10:1) to afford (4S)-4-[2-[tert-butyl(dimethyl)silyl]oxyethylamino]-N-[2-chloro-3-[2-chloro-3-[[(4S)-4-(dimethylamino)-4,5,6,7-tetrahydropyrazolo[1,5-a]pyridine-2-carbonyl]amino]phenyl]phenyl]-4,5,6,7-tetrahydropyrazolo[1,5-a]pyridine-2-carboxamide (220 mg, 98.5% yield) as a white solid.

LCMS m/z 766.8 [M+3]⁺.

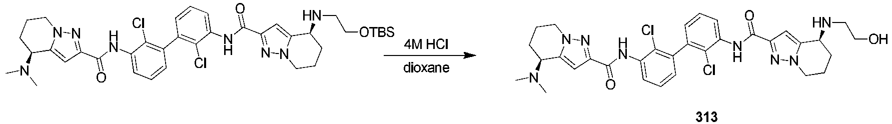

A mixture of (4S)-4-[2-[tert-butyl(dimethyl)silyl]oxyethylamino]-N-[2-chloro-3-[2-chloro-3-[[(4S)-4-(dimethylamino)-4,5,6,7-tetrahydropyrazolo[1,5-a]pyridine-2-carbonyl]amino]phenyl]phenyl]-4,5,6,7-tetrahydropyrazolo[1,5-a]pyridine-2-carboxamide (220 mg, 287.26 µmol, 1 eq) in dioxane (8 mL) was treated with HCl/dioxane (4 M, 143.63 µL, 2 eq), and the reaction mixture was stirred at room temperature for 12 hours. After completion, the mixture was filtered and the filtrate was concentrated under reduced pressure. The residue was purified by reversed-phase HPLC (column: Phenomenex Luna C18, 150 * 25 mm * 10 µm; mobile phase: [water (NH₄HCO₃)-ACN]; gradient: 40-70% B over 10 min) to afford Compound 313 (78 mg, 41.2% yield) as a white solid.

LCMS m/z 651.2 [M+1]⁺.

¹H NMR (400 MHz, DMSO-*d₆*) δ ppm 1.63 - 1.75 (m, 2 H) 1.85 - 2.06 (m, 4 H) 2.08 - 2.21 (m, 3 H) 2.26 (s, 6 H) 2.63 - 2.72 (m, 2 H) 3.43 - 3.52 (m, 2 H) 3.87 - 3.96 (m, 2 H) 4.02 - 4.25 (m, 4 H) 4.53 (t, *J*=5.44 Hz, 1 H) 6.68 (s, 1 H) 6.78 (s, 1 H) 7.16 (dd, *J*=7.63, 1.38 Hz, 2 H) 7.49 (t, *J*=7.94 Hz, 2 H) 8.24 - 8.32 (m, 2 H) 9.55 (d, *J*=1.50 Hz, 2 H).

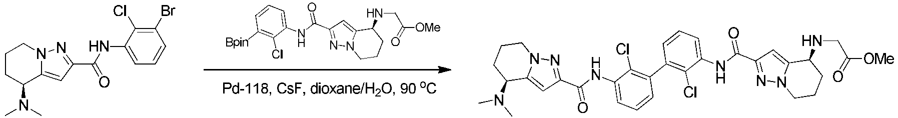

A mixture of (4S)-N-(3-bromo-2-chloro-phenyl)-4-(dimethylamino)-4,5,6,7-tetrahydropyrazolo[1,5-a]pyridine-2-carboxamide (100 mg, 251.45 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (147.48 mg, 301.74 µmol, 1.2 eq), ditert-butyl(cyclopentyl)phosphane;dichloropalladium;iron (16.39 mg, 25.14 µmol, 0.1 eq), and CsF (114.58 mg, 754.34 µmol, 3 eq) in dioxane (8 mL) / H₂O (2 mL) was stirred under a nitrogen atmosphere at 90 °C for 5 hours. After cooling to room temperature, the reaction mixture was filtered and the filtrate was concentrated under reduced pressure. The residue was purified by prep-TLC (SiO₂, Ethyl acetate:Methanol = 10:1) to afford methyl 2-[[(4S)-2-[[2-chloro-3-[2-chloro-3-[[(4S)-4-(dimethylamino)-4,5,6,7-tetrahydropyrazolo[1,5-a]pyridine-2-carbonyl]amino]phenyl]phenyl]carbamoyl]-4,5,6,7-tetrahydropyrazolo[1,5-a]pyridin-4-yl]amino]acetate (86 mg, 45.2% yield) as a white solid.

LCMS m/z 681.2 [M+3]⁺.

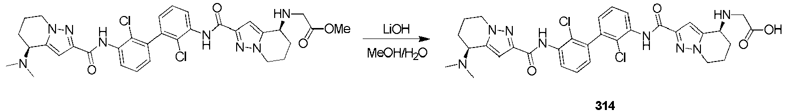

A mixture of methyl 2-[[(4S)-2-[[2-chloro-3-[2-chloro-3-[[(4S)-4-(dimethylamino)-4,5,6,7-tetrahydropyrazolo[1,5-a]pyridine-2-carbonyl]amino]phenyl]phenyl]carbamoyl]-4,5,6,7-tetrahydropyrazolo[1,5-a]pyridin-4-yl]amino]acetate (86 mg, 126.55 µmol, 1 eq) in H₂O (2 mL) and MeOH (4 mL) was treated with LiOH·H₂O (15.93 mg, 379.64 µmol, 3 eq), and the reaction mixture was stirred at room temperature for 12 hours. After completion, the mixture was filtered and the filtrate was concentrated under reduced pressure. The residue was purified by reversed-phase HPLC (column: Phenomenex Luna C18, 150 * 25 mm * 10 µm; mobile phase: [water (NH₄HCO₃)-ACN]; gradient: 20%-50% B over 10 min) to afford Compound S-314 (21 mg, 23.6% yield) as a white solid.

LCMS m/z 667.2 [M+3]⁺.

¹H NMR (400 MHz, DMSO-*d₆*) δ ppm 1.62 - 1.83 (m, 2 H) 1.83 - 2.08 (m, 4 H) 2.10 - 2.22 (m, 2 H) 2.25 (s, 6 H) 3.32 (s, 2 H) 3.92 (br dd, *J*=9.63, 5.38 Hz, 2 H) 4.02 - 4.25 (m, 6 H) 6.68 (s, 1 H) 6.85 (s, 1 H) 7.13 - 7.18 (m, 2 H) 7.49 (t, *J*=7.94 Hz, 2 H) 8.23 - 8.30 (m, 2 H) 9.52 - 9.59 (m, 2 H).

### [Preparation Example 101]

### Preparation of Compound 315, 2-(((S)-2-((2,2'-dichloro-3'-((S)-4-((2-hydroxyethyl)amino)-4,5,6,7-tetrahydropyrazolo[1,5-a]pyridine-2-carboxamido)-[1,1'-biphenyl]-3-yl)carbamoyl)-4,5,6,7-tetrahydropyrazolo[1,5-a]pyridin-4-yl)amino)acetic acid,

### Compound 316, (4S,4'S)-N,N'-(2,2'-dichloro-[1,1'-biphenyl]-3,3'-diyl)bis(4-((2-hydroxyethyl)amino)-4,5,6,7-tetrahydropyrazolo[1,5-a]pyridine-2-carboxamide), and

### Compound 317, 2,2'-(((4S,4'S)-2,2'-(((2,2'-dichloro-[1,1'-biphenyl]-3,3'-diyl)bis(azanediyl))bis(carbonyl))bis(4,5,6,7-tetrahydropyrazolo[1,5-a]pyridine-4,2-diyl))bis(azanediyl))diacetic acid

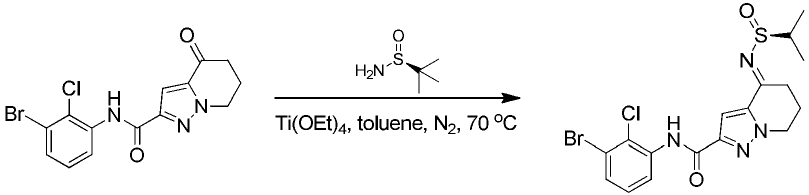

A mixture of N-(3-bromo-2-chloro-phenyl)-4-oxo-6,7-dihydro-5H-pyrazolo[1,5-a]pyridine-2-carboxamide (7.00 g, 18.99 mmol, 1 eq), (S)-2-methylpropane-2-sulfinamide (6.90 g, 56.97 mmol, 3 eq), and Ti(OEt)₄ (17.33 g, 75.96 mmol, 15.75 mL, 4 eq) in toluene (100 mL) was stirred at 70 °C under a nitrogen atmosphere for 4 hours. After completion, the reaction mixture was cooled to room temperature, and ethyl acetate (100 mL) and H₂O (100 mL) were added. The organic layer was separated, and the aqueous layer was extracted with ethyl acetate (60 mL * 2). The combined organic layers were washed with brine (60 mL), dried over Na₂SO₄, filtered, and concentrated under reduced pressure to afford (4E)-N-(3-bromo-2-chloro-phenyl)-4-[(R)-tert-butylsulfinyl]imino-6,7-dihydro-5H-pyrazolo[1,5-a]pyridine-2-carboxamide (10 g, 91.5% yield) as a yellow liquid, which was used directly in the next step without further purification.

LCMS m/z 473.0 [M+3]⁺.

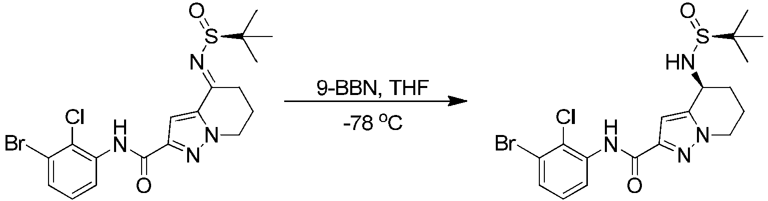

To a solution of (4E)-N-(3-bromo-2-chloro-phenyl)-4-[(R)-tert-butylsulfinyl]imino-6,7-dihydro-5H-pyrazolo[1,5-a]pyridine-2-carboxamide (3.00 g, 6.36 mmol, 1 eq) in THF (30 mL), 9-BBN (0.5 M in THF, 50.87 mL, 4 eq) was added -78 °C under a nitrogen atmosphere. The reaction mixture was then allowed to warm slowly to room temperature and stirred for 12 hours. After completion, ethyl acetate (200 mL) and H₂O (100 mL) were added, and the organic layer was separated. The aqueous layer was extracted with ethyl acetate (100 mL x 2). The combined organic layers were washed with brine (100 mL), dried over Na₂SO₄, filtered, and concentrated under reduced pressure. The residue was purified by column chromatography (SiO₂, Petroleum ether/Ethyl acetate = 1/1 to 0/1) to afford (4S)-N-(3-bromo-2-chloro-phenyl)-4-[[(R)-tert-butylsulfinyl]amino]-4,5,6,7-tetrahydropyrazolo[1,5-a]pyridine-2-carboxamide (1.8 g, 53.7% yield) as a white solid.

LCMS m/z 475.1 [M+3]⁺.

¹H NMR (400 MHz, DMSO-d6) δ ppm 1.16 (s, 8 H) 1.70 - 1.83 (m, 1 H) 2.01 - 2.22 (m, 3 H) 4.01 - 4.13 (m, 2 H) 4.19 - 4.28 (m, 1 H) 4.49 (td, J=8.97, 5.69 Hz, 1 H) 5.93 (d, J=8.76 Hz, 1 H) 6.95 (s, 1 H) 7.34 (t, J=8.13 Hz, 1 H) 7.59 (dd, J=8.00, 1.38 Hz, 1 H) 8.12 (dd, J=8.26, 1.25 Hz, 1 H) 9.64 (s, 1 H).

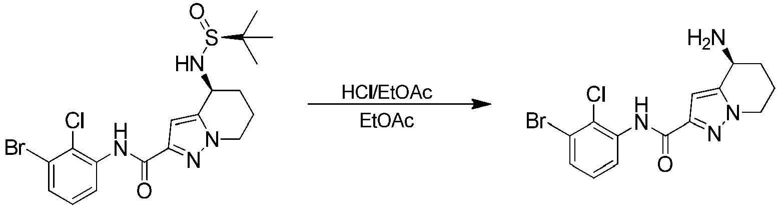

A solution of (4S)-N-(3-bromo-2-chloro-phenyl)-4-[[(R)-tert-butylsulfinyl]amino]-4,5,6,7-tetrahydropyrazolo[1,5-a]pyridine-2-carboxamide (3.0 g, 6.33 mmol, 1 eq) in EtOH (10 mL) was treated with HCl/EtOAc (4 M, 1.58 mL, 1 eq), and the reaction mixture was stirred at room temperature for 12 hours. After completion, ethyl acetate (200 mL) and aq. saturated NaHCO₃ solution (200 mL) were added, and the organic layer was separated. The aqueous layer was extracted with ethyl acetate (100 mL x 2). The combined organic layers were washed with brine (100 mL), dried over Na₂SO₄, filtered, and concentrated under reduced pressure to afford (4S)-4-amino-N-(3-bromo-2-chloro-phenyl)-4,5,6,7-tetrahydropyrazolo[1,5-a]pyridine-2-carboxamide (1.78 g, 68.4% yield) as a white solid, which was used in the next step without further purification.

LCMS m/z 370.9 [M+3]⁺.

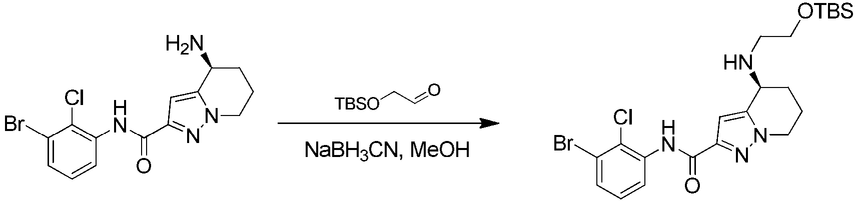

A mixture of 2-[tert-butyl(dimethyl)silyl]oxyacetaldehyde (613.04 mg, 3.52 mmol, 669.99 µL, 1.3 eq) and (4S)-4-amino-N-(3-bromo-2-chloro-phenyl)-4,5,6,7-tetrahydropyrazolo[1,5-a]pyridine-2-carboxamide (1.00 g, 2.71 mmol, 1 eq) in MeOH (20 mL) was treated with NaBH₃CN (1.70 g, 27.05 mmol, 10 eq). The reaction mixture was stirred at room temperature for 12 hours. After completion, the mixture was filtered and the filtrate was concentrated under reduced pressure. The residue was purified by reversed-phase HPLC (column: Waters Xbridge Prep OBD C18, 150 * 40 mm * 10 µm; mobile phase: [water (NH₄HCO₃)-ACN]; gradient: 80-100% B over 20 min) to afford (4S)-N-(3-bromo-2-chloro-phenyl)-4-[2-[tert-butyl(dimethyl)silyl]oxyethylamino]-4,5,6,7-tetrahydropyrazolo[1,5-a]pyridine-2-carboxamide (852 mg, 53.6% yield) as a white solid.

LCMS m/z 529.0 [M+3]⁺.

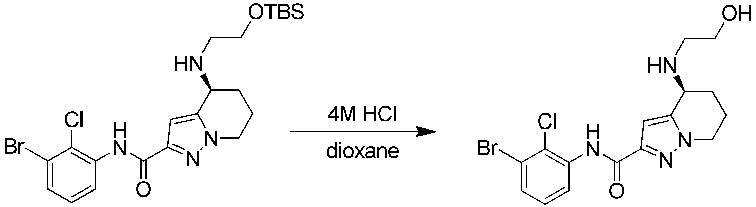

A solution of (4S)-N-(3-bromo-2-chloro-phenyl)-4-[2-[tert-butyl(dimethyl)silyl]oxyethylamino]-4,5,6,7-tetrahydropyrazolo[1,5-a]pyridine-2-carboxamide (540 mg, 1.02 mmol, 1 eq) in dioxane (10 mL) was treated with HCl/dioxane (4 M, 2.56 mL, 10 eq). The reaction mixture was stirred at room temperature for 12 hours. After completion, the mixture was concentrated under reduced pressure to give (4S)-N-(3-bromo-2-chloro-phenyl)-4-(2-hydroxyethylamino)-4,5,6,7-tetrahydropyrazolo[1,5-a]pyridine-2-carboxamide (465 mg, 98.9% yield) as a white solid.

LCMS m/z 415.1 [M+3]⁺.

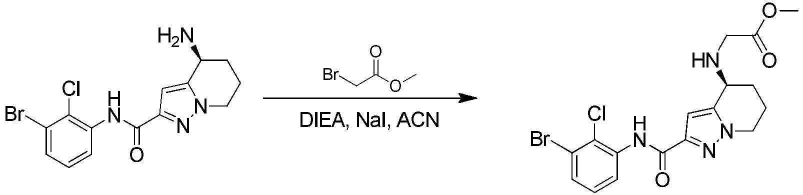

A mixture of (4S)-4-amino-N-(3-bromo-2-chloro-phenyl)-4,5,6,7-tetrahydropyrazolo[1,5-a]pyridine-2-carboxamide (2.0 g, 5.41 mmol, 1 eq) and methyl 2-bromoacetate (993.22 mg, 6.49 mmol, 614.62 µL, 1.2 eq) in ACN (25 mL) was treated with NaI (81.10 mg, 541.06 µmol, 0.1 eq) and DIEA (6.99 g, 54.11 mmol, 9.42 mL, 10 eq). The reaction mixture was stirred at 60 °C for 8 hours. After cooling to room temperature, the mixture was filtered and the filtrate was concentrated under reduced pressure. The residue was purified by column chromatography (SiO₂, Petroleum ether/Ethyl acetate = 3/1 to 1/1) to afford methyl 2-[[(4S)-2-[(3-bromo-2-chloro-phenyl)carbamoyl]-4,5,6,7-tetrahydropyrazolo[1,5-a]pyridin-4-yl]amino]acetate (1.21 g, 47.0% yield) as a white solid.

LCMS m/z 442.9 [M+3]⁺.

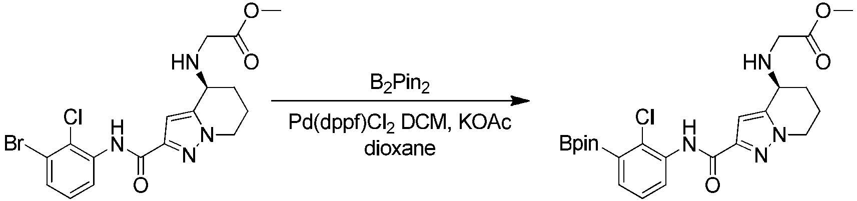

A mixture of methyl 2-[[(4S)-2-[(3-bromo-2-chloro-phenyl)carbamoyl]-4,5,6,7-tetrahydropyrazolo[1,5-a]pyridin-4-yl]amino]acetate (560 mg, 1.27 mmol, 1 eq), 4,4,5,5-tetramethyl-2-(4,4,5,5-tetramethyl-1,3,2-dioxaborolan-2-yl)-1,3,2-dioxaborolane (643.89 mg, 2.54 mmol, 2 eq), KOAc (373.27 mg, 3.80 mmol, 3 eq), and Pd(dppf)Cl₂·CH₂Cl₂ (103.53 mg, 126.78 µmol, 0.1 eq) in dioxane (10 mL) was stirred at 90 °C for 12 hours under a nitrogen atmosphere. After cooling to room temperature, the mixture was filtered and the filtrate was concentrated under reduced pressure. The residue was purified by prep-TLC (SiO₂, Petroleum ether:Ethyl acetate = 0:1) to afford methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (716 mg, 87.8% yield) as a white solid.

LCMS m/z 489.1 [M+1]⁺.

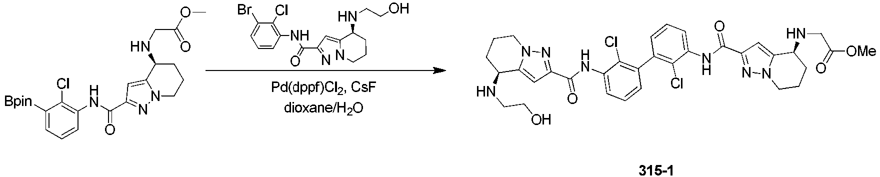

(4S)-N-(3-bromo-2-chloro-phenyl)-4-(2-hydroxyethylamino)-4,5,6,7-tetrahydropyrazolo[1,5-a]pyridine-2-carboxamide (100 mg, 241.72 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (153.59 mg, 314.24 µmol, 1.3 eq), ditert-butyl(cyclopentyl)phosphane;dichloropalladium;iron (15.75 mg, 24.17 µmol, 0.1 eq), and CsF (110.16 mg, 725.17 µmol, 26.77 µL, 3 eq) were added to a mixture of dioxane (8 mL) / H₂O (2 mL), and the resulting mixture was stirred under a nitrogen atmosphere at 90 °C for 12 hours. After completion of the reaction, the mixture was cooled to room temperature, filtered, and the filtrate was concentrated under reduced pressure. The resulting residue was purified by prep-TLC (SiO₂, Ethyl acetate:Methanol = 5:1) to afford Compound 315-1 (210 mg, 99.9% yield) as a white solid.

LCMS m/z 695.5 [M+1]⁺.

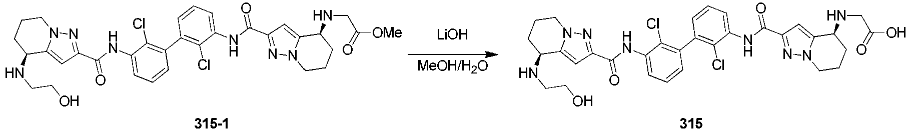

To a solution of methyl 2-[[(4S)-2-[[2-chloro-3-[2-chloro-3-[[(4S)-4-(2-hydroxyethylamino)-4,5,6,7-tetrahydropyrazolo[1,5-a]pyridine-2-carbonyl]amino]phenyl]phenyl]carbamoyl]-4,5,6,7-tetrahydropyrazolo[1,5-a]pyridin-4-yl]amino]acetate 315-1 (210 mg, 301.90 µmol, 1 eq) in H₂O (4 mL) / MeOH (4 mL), LiOH·H₂O (38.01 mg, 905.70 µmol, 3 eq) was added. The resulting mixture was stirred at room temperature for 12 hours. After completion of the reaction, the mixture was concentrated under reduced pressure, and the residue was purified by reversed-phase HPLC (column: Waters Xbridge 150 * 25 mm * 5 um; mobile phase: [water (NH₄HCO₃)-can]; gradient: 12%-42% B over 9 min) to afford Compound 135 (49 mg, 22.6% yield) as a white solid.

LCMS m/z 683.0 [M+3]⁺.

¹H NMR (400 MHz, DMSO-d6) δ ppm 1.64 - 1.80 (m, 2 H) 1.85 - 1.97 (m, 2 H) 1.98 - 2.07 (m, 2 H) 2.13 - 2.25 (m, 2 H) 2.64 - 2.74 (m, 2 H) 3.32 (br s, 2 H) 3.48 (br s, 2 H) 3.87 - 3.96 (m, 2 H) 4.05 (br t, J=5.75 Hz, 1 H) 4.16 (br t, J=5.82 Hz, 4 H) 6.81 (d, J=15.63 Hz, 2 H) 7.13 - 7.21 (m, 2 H) 7.49 (t, J=7.94 Hz, 2 H) 8.24 - 8.33 (m, 2 H) 9.48 - 9.61 (m, 2 H).

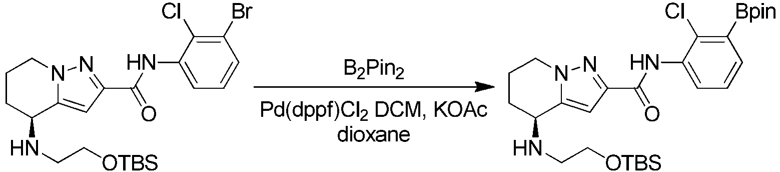

(4S)-N-(3-bromo-2-chloro-phenyl)-4-[2-[tert-butyl(dimethyl)silyl]oxyethylamino]-4,5,6,7-tetrahydropyrazolo[1,5-a]pyridine-2-carboxamide (100 mg, 189.41 µmol, 1 eq), 4,4,5,5-tetramethyl-2-(4,4,5,5-tetramethyl-1,3,2-dioxaborolan-2-yl)-1,3,2-dioxaborolane (96.20 mg, 378.82 µmol, 2 eq), KOAc (55.77 mg, 568.23 µmol, 3 eq), and Pd(dppf)Cl₂·CH₂Cl₂ (15.47 mg, 18.94 µmol, 0.1 eq) were added to dioxane (5 mL), and the resulting mixture was stirred under a nitrogen atmosphere at 90 °C for 12 hours. After completion of the reaction, the mixture was cooled to room temperature, filtered, and the filtrate was concentrated under reduced pressure. The resulting residue was purified by prep-TLC (SiO₂, petroleum ether:ethyl acetate = 1:1) to obtain (4S)-4-[2-[tert-butyl(dimethyl)silyl]oxyethylamino]-N-[2-chloro-3-(4,4,5,5-tetramethyl-1,3,2-dioxaborolan-2-yl)phenyl]-4,5,6,7-tetrahydropyrazolo[1,5-a]pyridine-2-carboxamide (210 mg, 96.4% yield) as a white solid.

LCMS m/z 575.3 [M+1]⁺.

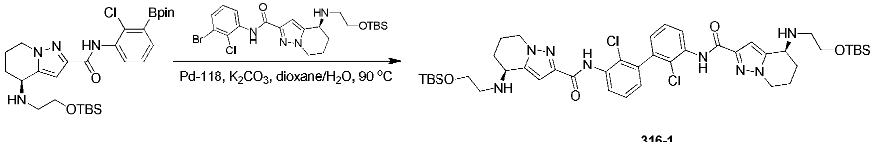

(4S)-4-[2-[tert-butyl(dimethyl)silyl]oxyethylamino]-N-[2-chloro-3-(4,4,5,5-tetramethyl-1,3,2-dioxaborolan-2-yl)phenyl]-4,5,6,7-tetrahydropyrazolo[1,5-a]pyridine-2-carboxamide (141.59 mg, 246.23 µmol, 1.3 eq), (4S)-N-(3-bromo-2-chloro-phenyl)-4-[2-[tert-butyl(dimethyl)silyl]oxyethylamino]-4,5,6,7-tetrahydropyrazolo[1,5-a]pyridine-2-carboxamide (100 mg, 189.41 µmol, 1 eq), ditert-butyl(cyclopentyl)phosphane;dichloropalladium;iron (12.34 mg, 18.94 µmol, 0.1 eq), and K₂CO₃ (78.53 mg, 568.23 µmol, 3 eq) were added to a mixture of dioxane (8 mL) / H₂O (2 mL), and the resulting mixture was stirred under a nitrogen atmosphere at 90 °C for 12 hours. After completion of the reaction, the mixture was cooled to room temperature, filtered, and the filtrate was concentrated under reduced pressure. The resulting residue was purified by prep-TLC (SiO₂, Petroleum ether:Ethyl acetate = 1:1) to afford Compound 316-1 (207 mg, 98.7% yield) as a white solid.

LCMS m/z 897.0 [M+3]⁺.

### [Preparation Example 102]

### Preparation of Compound 318, isopropyl 2-(((S)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetate, and Compound 319, tert-butyl 2-(((S)-2-((2,2'-dichloro-3'-((3-(((R)-3-hydroxypyrrolidin-1-yl)methyl)-1,7-naphthyridin-8-yl)amino)-[1,1'-biphenyl]-3-yl)carbamoyl)-4,5,6,7-tetrahydropyrazolo[1,5-a]pyridin-4-yl)amino)acetate

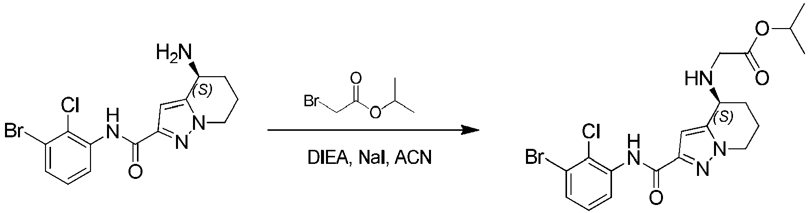

(4S)-4-amino-N-(3-bromo-2-chloro-phenyl)-4,5,6,7-tetrahydropyrazolo[1,5-a]pyridine-2-carboxamide (500 mg, 1.35 mmol, 1 eq) and isopropyl 2-bromoacetate (269.35 mg, 1.49 mmol, 192.53 µL, 1.1 eq) were added to ACN (6 mL), followed by addition of K₂CO₃ (560.83 mg, 4.06 mmol, 3 eq). The resulting mixture was stirred at 60 °C for 12 hours. After completion of the reaction, the mixture was cooled to room temperature, filtered, and the filtrate was concentrated under reduced pressure. The resulting residue was purified by column chromatography (SiO₂, Petroleum ether/Ethyl acetate = 1/1 to 0/1) to obtain isopropyl 2-[[(4S)-2-[(3-bromo-2-chloro-phenyl)carbamoyl]-4,5,6,7-tetrahydropyrazolo[1,5-a]pyridin-4-yl]amino]acetate (240 mg, 35.8% yield) as a white solid.

LCMS m/z 471.1 [M+3]⁺.

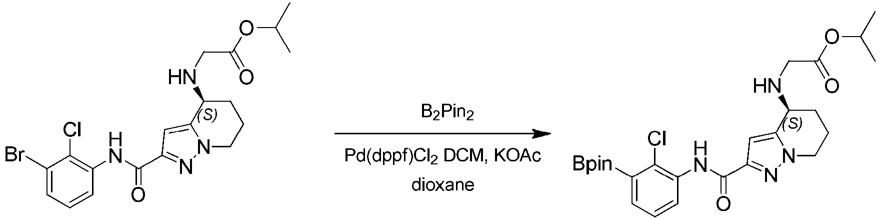

Isopropyl 2-[[(4S)-2-[(3-bromo-2-chloro-phenyl)carbamoyl]-4,5,6,7-tetrahydropyrazolo[1,5-a]pyridin-4-yl]amino]acetate (100 mg, 212.88 µmol, 1 eq), 4,4,5,5-tetramethyl-2-(4,4,5,5-tetramethyl-1,3,2-dioxaborolan-2-yl)-1,3,2-dioxaborolane (162.17 mg, 638.63 µmol, 3 eq), Pd(dppf)Cl₂·CH₂Cl₂ (17.38 mg, 21.29 µmol, 0.1 eq), and AcOK (62.67 mg, 638.63 µmol, 3 eq) were added to dioxane (4 mL), and the resulting mixture was stirred under a nitrogen atmosphere at 90 °C for 5 hours. After completion of the reaction, the mixture was cooled to room temperature, filtered, and the filtrate was concentrated under reduced pressure. The resulting residue was purified by prep-TLC (SiO₂, petroleum ether:ethyl acetate = 1:1) to obtain isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (90 mg, 57.2% yield) as a white solid.

LCMS m/z 517.4 [M+1]⁺.

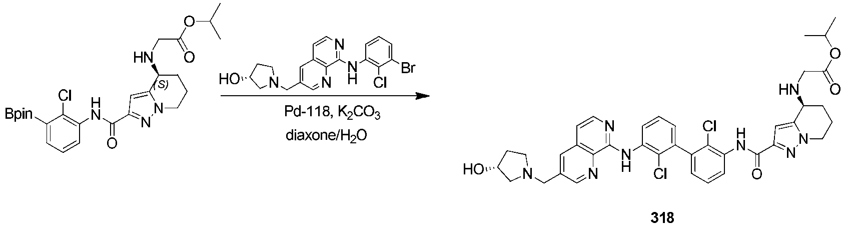

Isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (85.79 mg, 166.00 µmol, 1.2 eq), (3R)-1-[[8-(3-bromo-2-chloro-anilino)-1,7-naphthyridin-3-yl]methyl]pyrrolidin-3-ol (60 mg, 138.34 µmol, 1 eq), K₂CO₃ (57.36 mg, 415.01 µmol, 3 eq), and ditert-butyl(cyclopentyl)phosphane;dichloropalladium;iron (9.02 mg, 13.83 µmol, 0.1 eq) were added to a mixture of dioxane (5 mL) / H₂O (1 mL), and the resulting mixture was stirred under a nitrogen atmosphere at 90 °C for 5 hours. After completion of the reaction, the mixture was cooled to room temperature, filtered, and the filtrate was concentrated under reduced pressure. The resulting residue was purified by prep-TLC (SiO₂, Ethyl acetate:Methanol = 10:1) and further purified by reversed-phase HPLC (column: Waters Xbridge 150 * 25 mm * 5 um; mobile phase: [water (NH₄HCO₃)-can]; gradient: 50%-80% B over 15 min) to afford Compound 318 (37 mg, 35.6% yield) as a white solid.

LCMS m/z 745.2 [M+3]⁺.

¹H NMR (400 MHz, DMSO-d₆) δ ppm 1.22 (d, *J*=6.25 Hz, 6 H) 1.54 - 1.63 (m, 1 H) 1.67 - 1.77 (m, 1 H) 1.84 (s, 3 H) 2.14 - 2.26 (m, 1 H) 2.32 - 2.42 (m, 2 H) 2.63 - 2.79 (m, 3 H) 3.39 (br d, *J*=6.13 Hz, 2 H) 3.77 - 3.90 (m, 2 H) 3.93 - 3.99 (m, 1 H) 4.15 (br t, *J*=7.07 Hz, 2 H) 4.19 - 4.28 (m, 1 H) 4.73 (d, *J*=4.50 Hz, 1 H) 4.91 - 5.00 (m, 1 H) 6.79 (s, 1 H) 7.04 (d, *J*=7.25 Hz, 1 H) 7.21 (dd, *J*=7.69, 1.44 Hz, 1 H) 7.36 (d, *J*=5.75 Hz, 1 H) 7.52 (q, *J*=8.30 Hz, 2 H) 8.18 - 8.22 (m, 1 H) 8.25 - 8.28 (m, 1 H) 8.30 (d, *J*=8.13 Hz, 1 H) 8.93 (d, *J*=1.75 Hz, 1 H) 9.15 (dd, *J*=8.32, 1.31 Hz, 1 H) 9.56 (s, 1 H) 9.90 (s, 1 H).

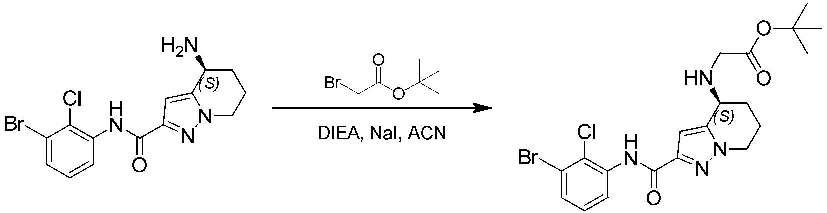

(4S)-4-amino-N-(3-bromo-2-chloro-phenyl)-4,5,6,7-tetrahydropyrazolo[1,5-a]pyridine-2-carboxamide (500 mg, 1.35 mmol, 1 eq) and tert-butyl 2-bromoacetate (290.22 mg, 1.49 mmol, 219.70 µL, 1.1 eq) were added to ACN (6 mL), followed by addition of K₂CO₃ (560.85 mg, 4.06 mmol, 3 eq). The resulting mixture was stirred at 60 °C for 12 hours. After completion of the reaction, the mixture was cooled to room temperature, filtered, and the filtrate was concentrated under reduced pressure. The resulting residue was purified by column chromatography (SiO₂, Petroleum ether/Ethyl acetate = 1/1 to 0/1) to obtain tert-butyl 2-[[(4S)-2-[(3-bromo-2-chloro-phenyl)carbamoyl]-4,5,6,7-tetrahydropyrazolo[1,5-a]pyridin-4-yl]amino]acetate (149 mg, 21.6% yield) as a white solid.

LCMS m/z 483.05 [M+1]⁺.

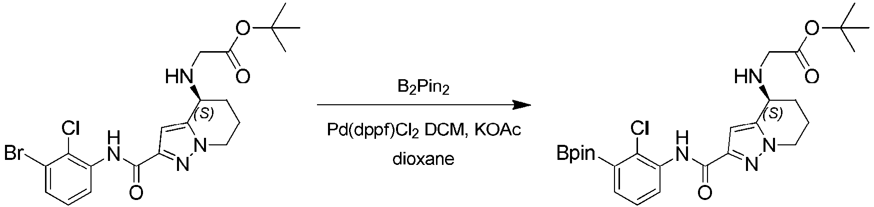

tert-Butyl 2-[[(4S)-2-[(3-bromo-2-chloro-phenyl)carbamoyl]-4,5,6,7-tetrahydropyrazolo[1,5-a]pyridin-4-yl]amino]acetate (140 mg, 289.38 µmol, 1 eq), 4,4,5,5-tetramethyl-2-(4,4,5,5-tetramethyl-1,3,2-dioxaborolan-2-yl)-1,3,2-dioxaborolane (220.46 mg, 868.15 µmol, 3 eq), Pd(dppf)Cl₂·CH₂Cl₂ (23.63 mg, 28.94 µmol, 0.1 eq), and AcOK (85.20 mg, 868.15 µmol, 3 eq) were added to dioxane (4 mL), and the resulting mixture was stirred under a nitrogen atmosphere at 90 °C for 5 hours. After completion of the reaction, the mixture was cooled to room temperature, filtered, and the filtrate was concentrated under reduced pressure. The resulting residue was purified by prep-TLC (SiO₂, Petroleum ether:Ethyl acetate = 1:1) to obtain tert-butyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (134 mg, 61.0% yield) as a white solid.

LCMS m/z 531.2 [M+1]⁺.

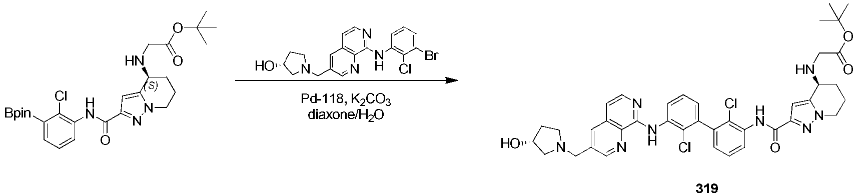

tert-Butyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (117.50 mg, 221.34 µmol, 1.2 eq), (3R)-1-[[8-(3-bromo-2-chloro-anilino)-1,7-naphthyridin-3-yl]methyl]pyrrolidin-3-ol (80 mg, 184.45 µmol, 1 eq), K₂CO₃ (76.48 mg, 553.34 µmol, 3 eq), and ditert-butyl(cyclopentyl)phosphane;dichloropalladium;iron (12.02 mg, 18.44 µmol, 0.1 eq) were added to a mixture of dioxane (5 mL) / H₂O (1 mL), and the resulting mixture was stirred under a nitrogen atmosphere at 90 °C for 5 hours. After completion of the reaction, the mixture was cooled to room temperature, filtered, and the filtrate was concentrated under reduced pressure. The resulting residue was purified by prep-TLC (SiO₂, Ethyl acetate:Methanol = 10:1) and further purified by reversed-phase HPLC (column: Waters Xbridge 150 * 25 mm * 5 um; mobile phase: [water (NH₄HCO₃)-ACN]; gradient: 55%-85% B over 15 min) to afford Compound 319 (27 mg, 19.32% yield) as a white solid.

LCMS m/z 757.3 [M+1]⁺.

¹H NMR (400 MHz, DMSO-d₆) δ ppm 1.44 (s, 9 H) 1.53 - 1.77 (m, 2 H) 1.84 - 2.09 (m, 4 H) 2.14 - 2.26 (m, 1 H) 2.32 - 2.42 (m, 2 H) 2.67 (q, *J*=7.84 Hz, 2 H) 2.75 (dd, *J*=9.69, 6.19 Hz, 1 H) 3.77 - 3.90 (m, 2 H) 3.91 - 3.98 (m, 1 H) 4.10 - 4.19 (m, 2 H) 4.19 - 4.27 (m, 1 H) 4.72 (d, *J*=4.50 Hz, 1 H) 6.78 (s, 1 H) 7.04 (d, *J*=7.25 Hz, 1 H) 7.21 (dd, *J*=7.63, 1.38 Hz, 1 H) 7.36 (d, *J*=5.88 Hz, 1 H) 7.52 (q, *J*=8.30 Hz, 2 H) 8.20 (d, *J*=5.88 Hz, 1 H) 8.25 - 8.33 (m, 2 H) 8.93 (d, *J*=1.75 Hz, 1 H) 9.15 (dd, *J*=8.38, 1.38 Hz, 1 H) 9.56 (s, 1 H) 9.90 (s, 1 H).

### [Preparation Example 103]

### Preparation of Compound 320, diethyl ((R)-1-((S)-2-((3'-(5-(((R)-3-hydroxypyrrolidin-1-yl)methyl)picolinamido)-2,2'-dimethyl-[1,1'-biphenyl]-3-yl)carbamoyl)-4,5,6,7-tetrahydropyrazolo[1,5-a]pyridin-4-yl)pyrrolidin-3-yl) phosphate

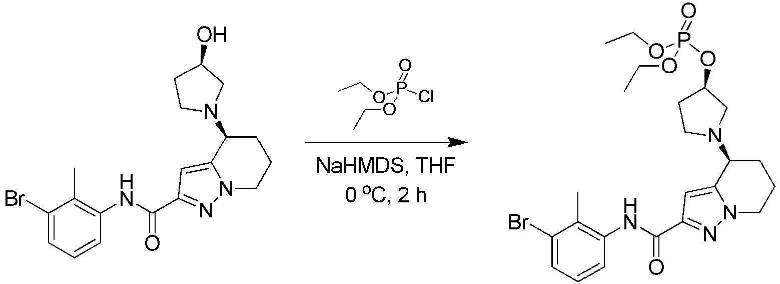

To a solution of 1-[chloro(ethoxy)phosphoryl]oxyethane (990 mg, 5.74 mmol, 829.15 µL, 4.81 eq) and (4S)-N-(3-bromo-2-methyl-phenyl)-4-[(3R)-3-hydroxypyrrolidin-1-yl]-4,5,6,7-tetrahydropyrazolo[1,5-a]pyridine-2-carboxamide (500 mg, 1.19 mmol, 1 eq) in THF (3 mL), NaHMDS (1 M, 3.58 mL, 3 eq) was added. The resulting mixture was stirred at room temperature for 2 hours. After completion of the reaction, the mixture was diluted with aq. saturated NaHCO₃ solution (20 mL) and extracted with ethyl acetate (5 mL * 3). The combined organic layers were dried over Na₂SO₄, filtered, and concentrated under reduced pressure. The resulting residue was purified by column chromatography (SiO₂, DCM:MeOH = 10:1) to obtain [(3R)-1-[(4S)-2-[(3-bromo-2-methylphenyl)carbamoyl]-4,5,6,7-tetrahydropyrazolo[1,5-a]pyridin-4-yl]pyrrolidin-3-yl] diethyl phosphate (200 mg, 30.2% yield) as a yellow liquid.

LCMS m/z 555.0 [M+1]⁺.

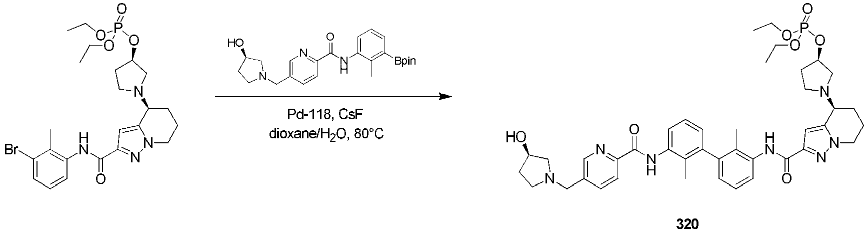

[(3R)-1-[(4S)-2-[(3-bromo-2-methyl-phenyl)carbamoyl]-4,5,6,7-tetrahydropyrazolo[1,5-a]pyridin-4-yl]pyrrolidin-3-yl] diethyl phosphate (75 mg, 135.04 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (118.11 mg, 270.08 µmol, 2 eq), CsF (41.03 mg, 270.08 µmol, 2 eq), and ditert-butyl(cyclopentyl)phosphane;dichloropalladium;iron (8.80 mg, 13.50 µmol, 0.1 eq) were added to a mixture of dioxane (3 mL) / H₂O (0.5 mL), and the resulting mixture was stirred under a nitrogen atmosphere at 80 °C for 5 hours. After completion of the reaction, the mixture was cooled to room temperature, filtered, and the filtrate was concentrated under reduced pressure. The resulting residue was purified by reversed-phase HPLC (0.1% NH₃·H₂O condition; column: Waters Xbridge 150 * 25 mm * 5 um; mobile phase: [water (NH₄HCO₃)-ACN]; gradient: 40%-70% B over 9 min) to afford Compound 320 (33 mg, 30.1% yield) as a white solid.

LCMS m/z 786.8 [M+1]⁺.

¹H NMR (400 MHz, DMSO-d₆) δ ppm 1.23 (t, J=7.07 Hz, 6 H) 1.52 - 1.61 (m, 1 H) 1.80 - 1.91 (m, 3 H) 1.94 (s, 3 H) 2.01 (s, 3 H) 2.09 - 2.18 (m, 1 H) 2.20 - 2.29 (m, 1 H) 2.32 - 2.38 (m, 1 H) 2.42 - 2.48 (m, 1 H) 2.58 - 2.66 (m, 2 H) 2.70 (br dd, J=9.44, 6.44 Hz, 1 H) 2.77 - 2.89 (m, 3 H) 3.64 - 3.78 (m, 2 H) 3.86 - 3.92 (m, 1 H) 3.95 - 4.05 (m, 4 H) 4.12 - 4.25 (m, 3 H) 4.72 (br d, J=4.25 Hz, 1 H) 4.82 (br s, 1 H) 6.67 (s, 1 H) 6.98 (d, J=7.63 Hz, 2 H) 7.25 - 7.35 (m, 2 H) 7.56 (dd, J=7.69, 3.56 Hz, 1 H) 7.87 (d, J=8.00 Hz, 1 H) 7.98 (dd, J=7.94, 1.69 Hz, 1 H) 8.14 (d, J=8.00 Hz, 1 H) 8.64 (s, 1 H) 9.54 (d, J=3.63 Hz, 1 H) 10.33 (s, 1 H).

### [Preparation Example 104]

### Preparation of Compound 321, (S)-isopropyl 2-((2-((2,2'-dichloro-3"-fluoro-4"-(((2-hydroxyethyl)amino)methyl)-5"-methoxy-[1,1':3',1"-terphenyl]-3-yl)carbamoyl)-4,5,6,7-tetrahydropyrazolo[1,5-a]pyridin-4-yl)amino)acetate

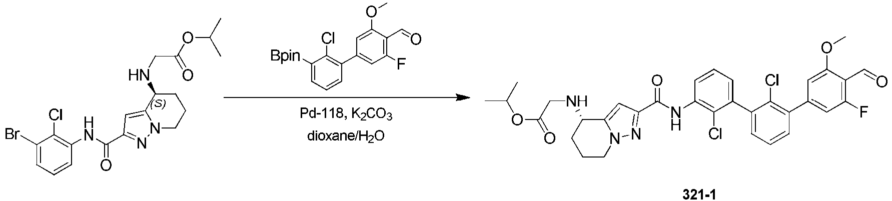

Isopropyl 2-[[(4S)-2-[(3-bromo-2-chloro-phenyl)carbamoyl]-4,5,6,7-tetrahydropyrazolo[1,5-a]pyridin-4-yl]amino]acetate (600 mg, 1.28 mmol, 1 eq), 4-[2-chloro-3-(4,4,5,5-tetramethyl-1,3,2-dioxaborolan-2-yl)phenyl]-2-fluoro-6-methoxy-benzaldehyde (498.94 mg, 1.28 mmol, 1 eq), ditert-butyl(cyclopenta-1,4-dien-1-yl)phosphane;dichloropalladium;iron (832.44 mg, 1.28 mmol, 1 eq), and K₂CO₃ (176.53 mg, 1.28 mmol, 1 eq) were added to a mixture of dioxane (10 mL) / H₂O (2 mL), and the resulting mixture was stirred under a nitrogen atmosphere at 90 °C for 10 hours. After completion of the reaction, the mixture was cooled to room temperature, filtered, and the filtrate was concentrated under reduced pressure. The resulting residue was purified by column chromatography (SiO₂, Petroleum ether/Ethyl acetate = 1/1 to 0/1, Ethyl acetate/Methanol = 1/0 to 3/1) to afford Compound 321 (160 mg, 13.4% yield) as a black solid.

LCMS m/z 655.2 [M+3]⁺.

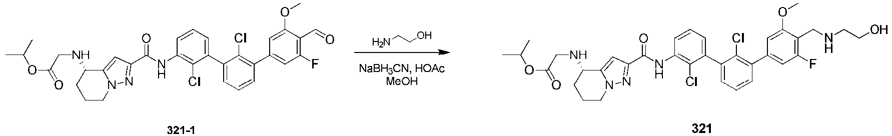

Isopropyl 2-[[(4S)-2-[[2-chloro-3-[2-chloro-3-(3-fluoro-4-formyl-5-methoxyphenyl)phenyl]phenyl]carbamoyl]-4,5,6,7-tetrahydropyrazolo[1,5-a]pyridin-4-yl]amino]acetate (100 mg, 153.02 µmol, 1 eq), 2-aminoethanol (93.47 mg, 1.53 mmol, 92.36 µL, 10 eq), AcOH (45.94 mg, 765.08 µmol, 43.80 µL, 5 eq), and NaBH₃CN (48.08 mg, 765.08 µmol, 5 eq) were added to MeOH (2 mL), and the resulting mixture was stirred under a nitrogen atmosphere at 60 °C for 5 hours. After completion of the reaction, the mixture was cooled to room temperature, filtered, and the filtrate was concentrated under reduced pressure. The resulting residue was purified by reversed-phase HPLC (column: Waters Xbridge 150 * 25 mm * 5 um; mobile phase: [water (NH₄HCO₃)-ACN]; gradient: 48%-78% B over 9 min) to afford Compound 321 (16 mg, 14.0% yield) as a white solid.

LCMS m/z 698.3 [M+1]⁺.

¹H NMR (400 MHz, DMSO-*d*₆) δ ppm 1.13 (br d, *J*=6.13 Hz, 6 H) 1.57 - 1.69 (m, 1 H) 1.78 - 1.93 (m, 2 H) 2.07 - 2.17 (m, 1 H) 2.43 (br d, *J*=1.38 Hz, 5 H) 3.31 (br s, 2 H) 3.38 (br s, 2 H) 3.68 (br s, 2 H) 3.79 (s, 3 H) 3.87 (br s, 1 H) 4.06 (br s, 2 H) 4.41 (br s, 1 H) 4.86 (dt, *J*=12.41, 6.24 Hz, 1 H) 6.70 (s, 1 H) 6.78 - 6.90 (m, 2 H) 7.12 (br d, *J=*7.50 Hz, 1 H) 7.30 - 7.35 (m, 1 H) 7.36 - 7.52 (m, 3 H) 8.19 (br d, *J*=8.00 Hz, 1 H) 9.47 (s, 1 H).

### [Preparation Example 105]

### Preparation of Compound 322, (R)-N-(2,2'-dichloro-3'-((3-((3-hydroxypyrrolidin-1-yl)methyl)-1,7-naphthyridin-8-yl)amino)-[1,1'-biphenyl]-3-yl)-4-(2-hydroxyethyl)-4,5,6,7-tetrahydropyrazolo[1,5-a]pyrimidine-2-carboxamide

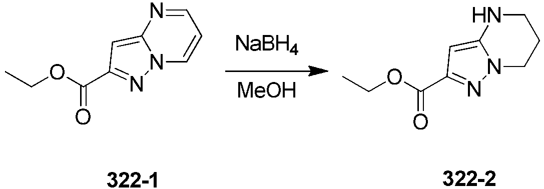

To a solution of Ethyl pyrazolo[1,5-a]pyrimidine-2-carboxylate 322-1 (1 g, 5.23 mmol, 1 eq) in MeOH (20 mL), NaBH₄ (397.48 mg, 10.46 mmol, 2 eq) was added. The resulting mixture was stirred at room temperature for 12 hours. After completion of the reaction, the mixture was poured into aq. saturated NH₄Cl solution (200 mL) and extracted with EA (100 mL * 3). The combined organic layers were washed with brine (20 mL), dried over Na₂SO₄, filtered, and concentrated under reduced pressure to afford Compound 322-2 (980 mg, 94.0% yield), which was used in the next reaction without further purification.

LCMS m/z 196.1 [M+1]⁺.

¹H NMR (400 MHz, DMSO-d6) δ ppm 1.16 - 1.29 (m, 2 H) 1.95 - 2.04 (m, 2 H) 3.12 - 3.19 (m, 2 H) 3.71 (s, 1 H) 4.02 (t, J=6.07 Hz, 2 H) 4.18 (q, J=7.13 Hz, 1 H) 5.60 (d, J=3.25 Hz, 1 H) 6.22 (br d, J=5.63 Hz, 1 H)

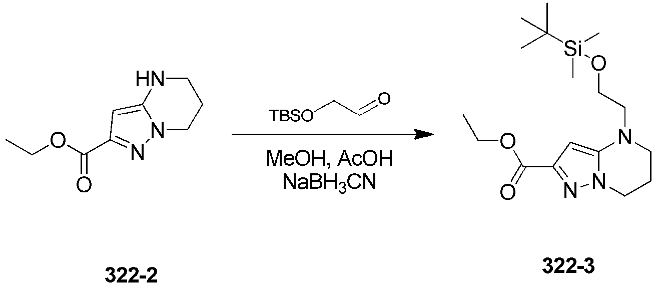

Ethyl 4,5,6,7-tetrahydropyrazolo[1,5-a]pyrimidine-2-carboxylate 322-2 (500 mg, 2.56 mmol, 1 eq) and 2-[tert-butyl(dimethyl)silyl]oxyacetaldehyde (892.91 mg, 5.12 mmol, 975.86 µL, 2 eq) were dissolved in MeOH (20 mL), followed by addition of AcOH (307.62 mg, 5.12 mmol, 293.25 µL, 2 eq) and NaBH₃CN (804.77 mg, 12.81 mmol, 5 eq). The resulting mixture was stirred at 60 °C for 12 hours. After completion of the reaction, the mixture was cooled to room temperature, concentrated under reduced pressure, and purified by reversed-phase HPLC (0.1% NH₃·H₂O condition; column: Waters Xbridge Prep OBD C18 150 * 40 mm * 10 um; mobile phase: [water (NH₄HCO₃)-ACN]; gradient: 50%-80% B over 20 min) to afford Compound 322-3 (140 mg, 13.9% yield) as a brown liquid.

LCMS m/z 354.2 [M+1]⁺.

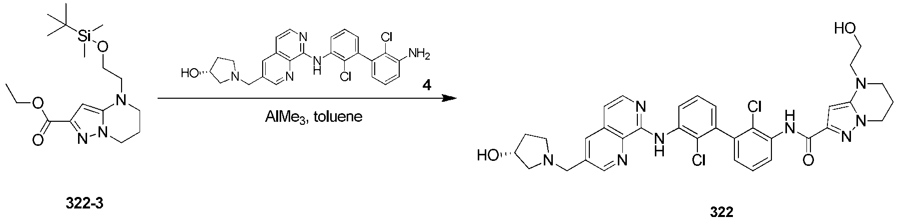

Ethyl 4-[2-[tert-butyl(dimethyl)silyl]oxyethyl]-6,7-dihydro-5H-pyrazolo[1,5-a]pyrimidine-2-carboxylate 322-3 (140 mg, 396.01 µmol, 1 eq), (3R)-1-[[8-[3-(3-amino-2-chloro-phenyl)-2-chloro-anilino]-1,7-naphthyridin-3-yl]methyl]pyrrolidin-3-ol (228.28 mg, 475.21 µmol, 1.2 eq), and AlMe₃ (2 M, 396.01 µL, 2 eq) were added to toluene (10 mL), and the resulting mixture was stirred under a nitrogen atmosphere at 90 °C for 12 hours. After completion of the reaction, the mixture was cooled to room temperature, poured into aq. saturated NH₄Cl solution (20 mL), and extracted with EA (50 mL * 3). The combined organic layers were washed with brine (20 mL), dried over Na₂SO₄, filtered, and concentrated under reduced pressure. The resulting residue was purified by reversed-phase HPLC (0.1% HCl condition; column: Phenomenex Luna C18 150 * 25 mm * 10 um; mobile phase: [water (HCl)-ACN]; gradient: 13%-43% B over 10 min), followed by further purification by reversed-phase HPLC [(0.1% NH₃·H₂O condition) column: Waters Xbridge 150 * 25 mm * 5 um; mobile phase: [water (NH₄HCO₃)-ACN]; gradient: 35%-65% B over 15 min] to afford Compound 322 (7 mg, 2.49% yield) as a white solid.

LCMS m/z 673.3 [M+1]⁺.

¹H NMR (400 MHz, DMSO-d6) δ ppm 1.23 (br s, 1 H) 1.53 - 1.63 (m, 1 H) 2.01 (s, 1 H) 2.08 (br s, 2 H) 2.32 - 2.41 (m, 2 H) 2.62 - 2.70 (m, 2 H) 2.71 - 2.77 (m, 1 H) 3.58 (q, J=5.84 Hz, 2 H) 3.83 (d, J=9.63 Hz, 2 H) 4.06 (t, J=6.19 Hz, 2 H) 4.18 - 4.26 (m, 1 H) 4.67 - 4.73 (m, 2 H) 5.89 (s, 1 H) 7.03 (d, J=7.50 Hz, 1 H) 7.17 (d, J=7.23 Hz, 1 H) 7.35 (d, J=5.88 Hz, 1 H) 7.49 (br t, J=7.94 Hz, 1 H) 7.52 (br t, J=8.00 Hz, 1 H) 8.19 (d, J=5.63 Hz, 1 H) 8.26 (s, 1 H) 8.38 (d, J=7.00 Hz, 1 H) 8.92 (d, J=1.75 Hz, 1 H) 9.14 (d, J=7.00 Hz, 1 H) 9.43 (s, 1 H) 9.89 (s, 1 H)

### [Preparation Example 106]

### 106-1. Preparation of Compound 323, (R)-N-(2,2'-dichloro-3'-((3-((3-hydroxypyrrolidin-1-yl)methyl)-1,7-naphthyridin-8-yl)amino)-[1,1-biphenyl]-3-yl)-5-(2-hydroxyethyl)-4,5,6,7-tetrahydropyrazolo[1,5-a]pyrazine-2-carboxamide

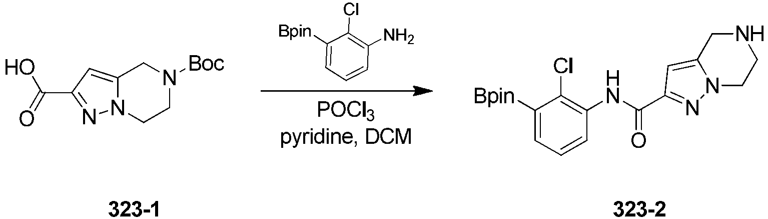

2-Chloro-3-(4,4,5,5-tetramethyl-1,3,2-dioxaborolan-2-yl)aniline 323-1 (1.04 g, 4.12 mmol, 1.1 eq), 5-tert-butoxycarbonyl-6,7-dihydro-4H-pyrazolo[1,5-a]pyrazine-2-carboxylic acid (1 g, 3.74 mmol, 1 eq), POCl₃ (1.72 g, 11.22 mmol, 1.05 mL, 3 eq), and pyridine (1.48 g, 18.71 mmol, 1.51 mL, 5 eq) were added to DCM (20 mL), and the resulting mixture was stirred at room temperature under a nitrogen atmosphere for 12 hours. After completion of the reaction, aq. saturated NaHCO₃ solution (100 mL) was slowly added to the mixture, followed by dilution with H₂O (100 mL), and the mixture was extracted with EtOAc (150 mL). The organic layer was dried over Na₂SO₄, filtered, and concentrated under reduced pressure. The resulting residue was purified by column chromatography (SiO₂, Petroleum ether/Ethyl acetate = 1/0 to 0/1) to afford Compound 323-2 (1.06 g, 56.2% yield) as a yellow solid.

LCMS m/z 403.1 [M+1]⁺.

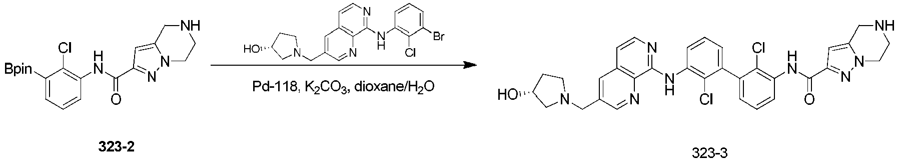

N-[2-chloro-3-(4,4,5,5-tetramethyl-1,3,2-dioxaborolan-2-yl)phenyl]-4,5,6,7-tetrahydropyrazolo[1,5-a]pyrazine-2-carboxamide 323-2 (185.68 mg, 461.12 µmol, 1 eq), (3R)-1-[[8-(3-bromo-2-chloro-anilino)-1,7-naphthyridin-3-yl]methyl]pyrrolidin-3-ol (200 mg, 461.12 µmol, 1 eq), K₂CO₃ (191.19 mg, 1.38 mmol, 3 eq), and ditert-butyl(cyclopentyl)phosphane;dichloropalladium;iron (30.05 mg, 46.11 µmol, 0.1 eq) were added to a mixture of dioxane (8 mL) / H₂O (2 mL), and the resulting mixture was stirred under a nitrogen atmosphere at 90 °C for 6 hours. After completion of the reaction, the mixture was cooled to room temperature, concentrated under reduced pressure, and the resulting residue was purified by column chromatography (SiO₂, Petroleum ether/Ethyl acetate = 1/1 to 0/1, Ethyl acetate/Methanol = 1/0 to 0/1) to afford Compound 323-3 (333 mg, 97.5% yield) as a white solid.

LCMS m/z 629.1 [M+1]⁺.

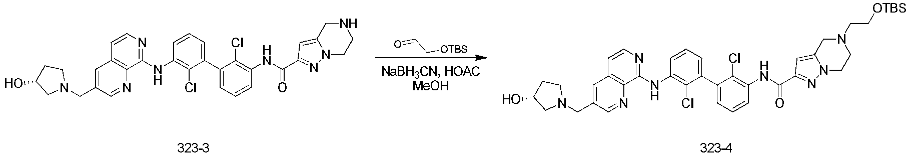

N-[2-chloro-3-[2-chloro-3-[[3-[[(3R)-3-hydroxypyrrolidin-1-yl]methyl]-1,7-naphthyridin-8-yl]amino]phenyl]phenyl]-4,5,6,7-tetrahydropyrazolo[1,5-a]pyrazine-2-carboxamide 323-3 (100 mg, 158.85 µmol, 1 eq) and 2-[tert-butyl(dimethyl)silyl]oxyacetaldehyde (83.07 mg, 476.54 µmol, 90.78 µL, 3 eq) were dissolved in MeOH (4 mL), followed by addition of AcOH (19.08 mg, 317.69 µmol, 18.19 µL, 2 eq) and NaBH₃CN (49.91 mg, 794.23 µmol, 5 eq). The resulting mixture was stirred at 60 °C for 2 hours. After completion of the reaction, the mixture was cooled to room temperature, concentrated under reduced pressure, and the residue was purified by prep-TLC (SiO₂, Ethyl acetate:Methanol = 10:1) to afford Compound 323-4 (162 mg, 98.3% yield) as a white solid.

LCMS m/z 789.2 [M+3]⁺.

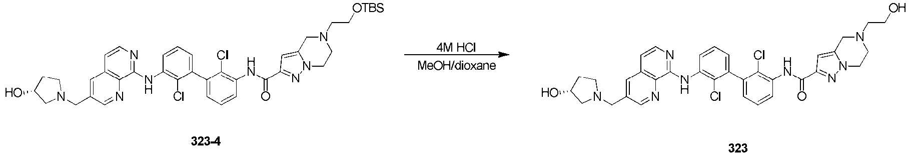

5-[2-[tert-butyl(dimethyl)silyl]oxyethyl]-N-[2-chloro-3-[2-chloro-3-[[3-[[(3R)-3-hydroxypyrrolidin-1-yl]methyl]-1,7-naphthyridin-8-yl]amino]phenyl]phenyl]-6,7-dihydro-4H-pyrazolo[1,5-a]pyrazine-2-carboxamide 323-4 (162 mg, 205.62 µmol, 1 eq) was dissolved in MeOH (4 mL), and HCl/dioxane (2 M, 0.2 mL) was added. The resulting mixture was stirred at room temperature for 12 hours. After completion of the reaction, the mixture was concentrated under reduced pressure, and the residue was purified by reversed-phase HPLC (column: Waters Xbridge 150 * 25 mm * 5 um; mobile phase: [water (NH₄HCO₃)-ACN]; gradient: 45%-75% B over 9 min; column: Phenomenex Luna C18 150 * 25 mm * 10 um; mobile phase: [water (HCl)-ACN]; gradient: 5%-35% B over 10 min) to afford Compound 323 (31 mg, 21.2% yield) as a white solid.

LCMS m/z 673.3 [M+1]⁺.

¹H NMR (400 MHz, METHANOL-*d*₄) δ ppm 1.88 - 2.26 (m, 1 H) 2.26 - 2.60 (m, 1 H) 3.21 (br s, 2 H) 3.36 - 3.53 (m, 4 H) 3.57 - 3.80 (m, 2 H) 3.87 - 4.05 (m, 4 H) 4.56 (br d, *J*=5.13 Hz, 4 H) 4.69 - 4.74 (m, 4 H) 6.79 (s, 1 H) 7.17 (br d, *J*=7.13 Hz, 1 H) 7.33 - 7.43 (m, 2 H) 7.47 (br d, *J*=7.25 Hz, 1 H) 7.56 - 7.67 (m, 2 H) 7.77 (br d, *J*=7.50 Hz, 1 H) 8.26 (br d, *J*=7.63 Hz, 1 H) 8.64 (br s, 1 H) 9.22 (br s, 1 H).

### 106-2. Preparation of Compound 324, (R)-2-(2-((2,2'-dichloro-3'-((3-((3-hydroxypyrrolidin-1-yl)methyl)-1,7-naphthyridin-8-yl)amino)-[1,1'-biphenyl]-3-yl)carbamoyl)-6,7-dihydropyrazolo[1,5-a]pyrazin-5(4H)-yl)acetic acid

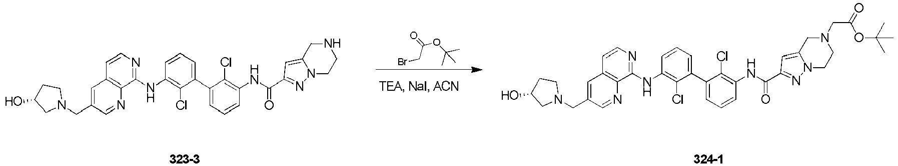

N-[2-chloro-3-[2-chloro-3-[[3-[[(3R)-3-hydroxypyrrolidin-1-yl]methyl]-1,7-naphthyridin-8-yl]amino]phenyl]phenyl]-4,5,6,7-tetrahydropyrazolo[1,5-a]pyrazine-2-carboxamide 323-3 (100 mg, 158.85 µmol, 1 eq) and tert-butyl 2-bromoacetate (154.92 mg, 794.23 µmol, 117.27 µL, 5 eq) were dissolved in ACN (10 mL), followed by addition of TEA (48.22 mg, 476.54 µmol, 66.33 µL, 3 eq). The resulting mixture was stirred at 60 °C for 1 hour. After completion of the reaction, the mixture was concentrated under reduced pressure, and the residue was purified by reversed-phase HPLC (column: Waters Xbridge 150 * 25 mm * 5 um; mobile phase: [water (NH₄HCO₃)-ACN]; gradient: 55%-85% B over 9 min) to afford Compound 324-1 (90 mg, 70.0% yield) as a white solid.

LCMS m/z 743.3 [M+1]⁺.

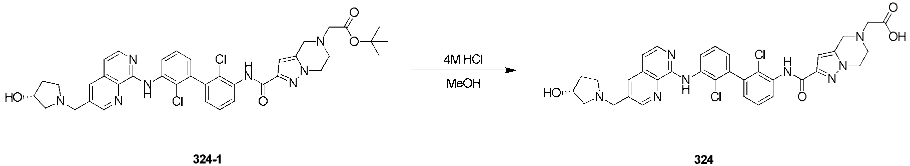

tert-Butyl 2-[2-[[2-chloro-3-[2-chloro-3-[[3-[[(3R)-3-hydroxypyrrolidin-1-yl]methyl]-1,7-naphthyridin-8-yl]amino]phenyl]phenyl]carbamoyl]-6,7-dihydro-4H-pyrazolo[1,5-a]pyrazin-5-yl]acetate 324-1 (90 mg, 121.02 µmol, 1 eq) was dissolved in MeOH (2 mL), and HCl/MeOH (4 M, 0.2 mL) was added. The resulting mixture was stirred at room temperature for 5 hours. After completion of the reaction, the mixture was concentrated under reduced pressure, and the residue was purified by reversed-phase HPLC (column: Phenomenex Luna C18 150 * 25 mm * 10 um; mobile phase: [water (HCl)-ACN]; gradient: 8%-38% B over 10 min; column: Waters Xbridge 150 * 25 mm * 5 um; mobile phase: [water (NH₄HCO₃)-ACN]; gradient: 12%-42% B over 15 min) to afford Compound 324 (11 mg, 12.8% yield) as a yellow solid.

LCMS m/z 689.2 [M+3]⁺.

¹H NMR (400 MHz, DMSO-*d*₆) δ ppm 1.53 - 1.63 (m, 1 H) 1.98 - 2.08 (m, 1 H) 2.39 (dd, *J*=9.63, 3.63 Hz, 1 H) 2.61 - 2.82 (m, 3 H) 3.11 (br dd, *J*=10.26, 6.00 Hz, 3 H) 3.78 - 3.92 (m, 4 H) 4.14 - 4.30 (m, 3 H) 4.74 (br d, *J*=4.13 Hz, 1 H) 6.60 - 6.68 (m, 1 H) 7.03 (dd, *J*=7.50, 1.38 Hz, 1 H) 7.20 (dd, *J*=7.57, 1.31 Hz, 1 H) 7.35 (d, *J*=5.88 Hz, 1 H) 7.47 - 7.56 (m, 2 H) 8.16 - 8.22 (m, 1 H) 8.24 - 8.28 (m, 1 H) 8.31 (br d, *J*=7.88 Hz, 1 H) 8.93 (d, *J*=1.75 Hz, 1 H) 9.14 (dd, *J*=8.44, 1.31 Hz, 1 H) 9.56 (s, 1 H) 9.90 (s, 1 H).

### [Preparation Example 107]

### 107-1. Preparation of Compound 325, (R)-5-(2-hydroxyethyl)-N-(3'-(5-((3-hydroxypyrrolidin-1-yl)methyl)picolinamido)-2,2'-dimethyl-[1,1'-biphenyl]-3-yl)-4,5,6,7-tetrahydropyrazolo[1,5-a]pyrazine-2-carboxamide

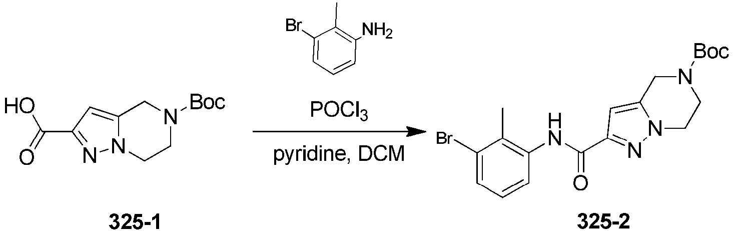

3-Bromo-2-methyl-aniline 2 (765.69 mg, 4.12 mmol, 507.08 µL, 1.1 eq), 5-tert-butoxycarbonyl-6,7-dihydro-4H-pyrazolo[1,5-a]pyrazine-2-carboxylic acid 325-1 (1 g, 3.74 mmol, 1 eq), pyridine (1.48 g, 18.71 mmol, 1.51 mL, 5 eq), and POCl₃ (573.68 mg, 3.74 mmol, 348.74 µL, 1 eq) were added to DCM (20 mL), and the resulting mixture was stirred at room temperature under a nitrogen atmosphere for 5 hours. After completion of the reaction, the mixture was concentrated under reduced pressure, and the residue was added to H₂O (50 mL) and extracted with ethyl acetate (50 mL * 3). The combined organic layers were dried over Na₂SO₄, filtered, and concentrated under reduced pressure. The resulting residue was purified by column chromatography (SiO₂, Petroleum ether/Ethyl acetate = 1/0 to 0/1) to afford Compound 325-2 (1.55 g, 71.3% yield) as a white solid.

LCMS m/z 435.1 [M+1]⁺.

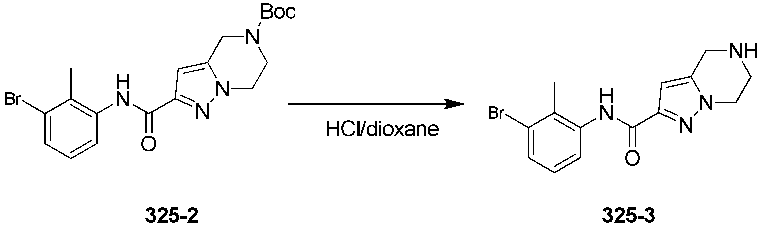

tert-Butyl 2-[(3-bromo-2-methyl-phenyl)carbamoyl]-6,7-dihydro-4H-pyrazolo[1,5-a]pyrazine-5-carboxylate 325-2 (1.55 g, 3.56 mmol, 1 eq) was dissolved in dioxane (30 mL), and HCl/dioxane (2 M, 1.78 mL, 1 eq) was added. The resulting mixture was stirred at room temperature for 12 hours. After completion of the reaction, the mixture was concentrated under reduced pressure, and the residue was poured into H₂O (50 mL) and extracted with ethyl acetate (50 mL x 3). The combined organic layers were dried over Na₂SO₄, filtered, and concentrated under reduced pressure to afford Compound 325-3 (1.23 g, 87.2% yield) as a white solid.

LCMS m/z 335.0 [M+1]⁺.

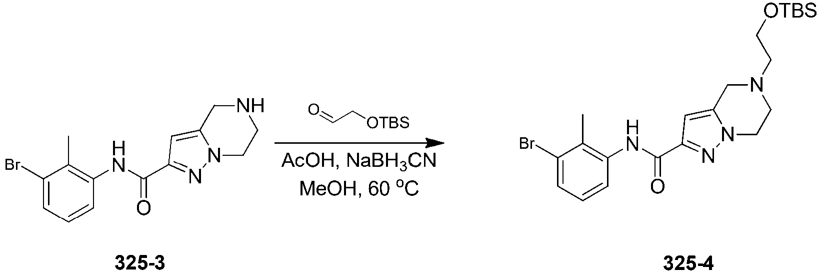

N-(3-bromo-2-methyl-phenyl)-4,5,6,7-tetrahydropyrazolo[1,5-a]pyrazine-2-carboxamide 325-3 (550 mg, 1.64 mmol, 1 eq) and 2-[tert-butyl(dimethyl)silyl]oxyacetaldehyde (858.04 mg, 4.92 mmol, 937.75 µL, 3 eq) were dissolved in MeOH (10 mL), followed by addition of AcOH (492.66 mg, 8.20 mmol, 469.64 µL, 5 eq) and NaBH₃CN (515.55 mg, 8.20 mmol, 5 eq). The resulting mixture was stirred at 60 °C for 12 hours. After completion of the reaction, the mixture was cooled to room temperature, concentrated under reduced pressure, and the residue was purified by column chromatography (SiO₂, Petroleum ether/Ethyl acetate = 1/0 to 1/1) to afford Compound 325-4 (870 mg, 79.5% yield) as a yellow solid.

LCMS m/z 495.0 [M+3]⁺.

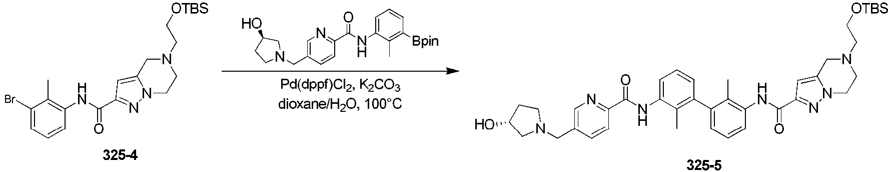

N-(3-bromo-2-methyl-phenyl)-5-[2-[tert-butyl(dimethyl)silyl]oxyethyl]-6,7-dihydro-4H-pyrazolo[1,5-a]pyrazine-2-carboxamide 325-4 (200 mg, 405.26 µmol, 1 eq), N-[2-methyl-3-(4,4,5,5-tetramethyl-1,3,2-dioxaborolan-2-yl)phenyl]-5-[[rac-(3R)-3-hydroxypyrrolidin-1-yl]methyl]pyridine-2-carboxamide 7 (194.96 mg, 445.79 µmol, 1.1 eq), ditert-butyl(cyclopentyl)phosphane;dichloropalladium;iron (26.41 mg, 40.53 µmol, 0.1 eq), and K₂CO₃ (168.03 mg, 1.22 mmol, 3 eq) were added to a mixture of dioxane (8 mL)/H₂O (2 mL), and the resulting mixture was stirred under a nitrogen atmosphere at 90 °C for 12 hours. After completion of the reaction, the mixture was cooled to room temperature, concentrated under reduced pressure, and purified by column chromatography (SiO₂, Petroleum ether/Ethyl acetate = 1/0 to 0/1, Ethyl acetate/Methanol = 1/0 to 2/1) to afford Compound 325-5 (200 mg, 42.2% yield) as a yellow solid.

LCMS m/z 724.8 [M+1]⁺.

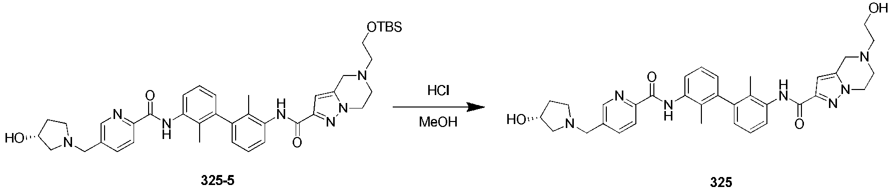

5-[2-[tert-butyl(dimethyl)silyl]oxyethyl]-N-[2-methyl-3-[2-methyl-3-[[5-[[rac-(3R)-3-hydroxypyrrolidin-1-yl]methyl]pyridine-2-carbonyl]amino]phenyl]phenyl]-6,7-dihydro-4H-pyrazolo[1,5-a]pyrazine-2-carboxamide 325-5 (200 mg, 276.25 µmol, 1 eq) was dissolved in MeOH (5 mL), and HCl/MeOH (6 M, 46.04 µL, 1 eq) was added. The resulting mixture was stirred at room temperature for 12 hours. After completion of the reaction, the mixture was concentrated under reduced pressure and purified by reversed-phase HPLC (column: Waters Xbridge Prep OBD C18 150 * 40 mm * 10 um; mobile phase: [water (NH₄HCO₃)-ACN]; gradient: 15%-45% B over 20 min) to afford Compound 325 (84 mg, 48.3% yield) as a white solid.

LCMS m/z 610.4 [M+1]⁺.

¹H NMR (400 MHz, DMSO-*d*₆) δ ppm 1.52 - 1.61 (m, 1 H) 1.90 - 2.07 (m, 6 H) 2.28 - 2.36 (m, 2 H) 2.40 - 2.48 (m, 1 H) 2.56 - 2.78 (m, 4 H) 3.00 (br t, *J*=5.32 Hz, 1 H) 3.59 (br d, *J*=5.13 Hz, 1 H) 3.64 - 3.81 (m, 4 H) 4.15 - 4.22 (m, 2 H) 4.58 (br s, 1 H) 4.67 - 4.78 (m, 1 H) 6.49 - 6.57 (m, 1 H) 6.97 (br d, *J*=7.50 Hz, 1 H) 7.08 - 7.15 (m, 1 H) 7.22 - 7.34 (m, 2 H) 7.59 (br d, *J*=7.88 Hz, 1 H) 7.82 - 7.90 (m, 1 H) 7.98 (br d, *J*=7.88 Hz, 1 H) 8.09 - 8.17 (m, 1 H) 8.64 (s, 1 H) 9.49 - 9.55 (m, 1 H) 10.20 - 10.36 (m, 1 H).

### 107-2. Preparation of Compound 326, (R)-2-(2-((3'-(5-((3-hydroxypyrrolidin-1-yl)methyl)picolinamido)-2,2'-dimethyl-[1,1'-biphenyl]-3-yl)carbamoyl)-6,7-dihydropyrazolo[1,5-a]pyrazin-5(4H)-yl)acetic acid

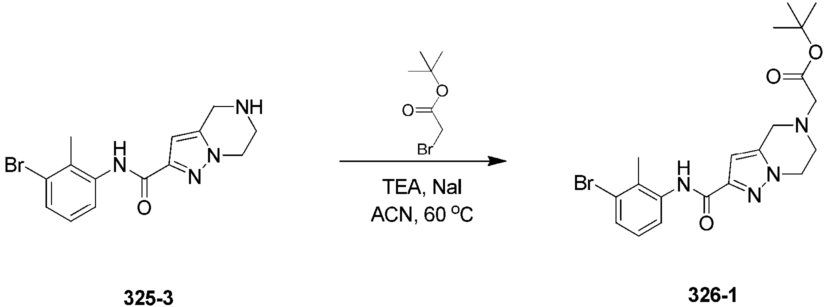

N-(3-bromo-2-methyl-phenyl)-4,5,6,7-tetrahydropyrazolo[1,5-a]pyrazine-2-carboxamide 325-3 (550 mg, 1.64 mmol, 1 eq) and tert-butyl 2-bromoacetate (1.60 g, 8.20 mmol, 1.21 mL, 5 eq) were dissolved in ACN (10 mL), and TEA (498.10 mg, 4.92 mmol, 685.14 µL, 3 eq) was added. The resulting mixture was stirred at 60 °C for 12 hours. After completion of the reaction, the mixture was cooled to room temperature, concentrated under reduced pressure, and purified by column chromatography (SiO₂, Petroleum ether/Ethyl acetate = 1/0 to 1/1) to afford Compound 326-1 (600 mg, 61.0% yield) as a yellow solid.

LCMS m/z 451.1 [M+3]⁺.

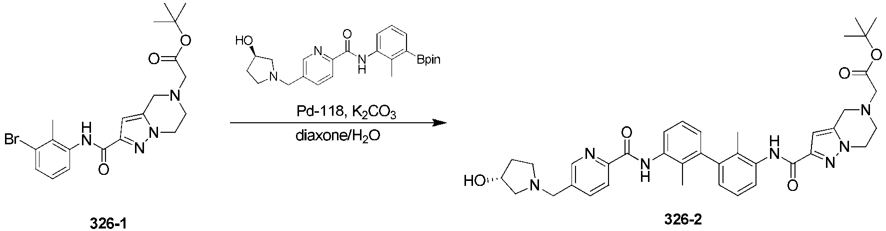

tert-Butyl 2-[2-[(3-bromo-2-methyl-phenyl)carbamoyl]-6,7-dihydro-4H-pyrazolo[1,5-a]pyrazin-5-yl]acetate 326-1 (150 mg, 333.82 µmol, 1 eq), N-[2-methyl-3-(4,4,5,5-tetramethyl-1,3,2-dioxaborolan-2-yl)phenyl]-5-[[rac-(3R)-3-hydroxypyrrolidin-1-yl]methyl]pyridine-2-carboxamide (160.59 mg, 367.20 µmol, 1.1 eq), ditert-butyl(cyclopentyl)phosphane;dichloropalladium;iron (21.76 mg, 33.38 µmol, 0.1 eq), and K₂CO₃ (138.41 mg, 1.00 mmol, 3 eq) were added to a mixture of dioxane (8 mL) / H₂O (2 mL), and the resulting mixture was stirred under a nitrogen atmosphere at 90 °C for 12 hours. After completion of the reaction, the mixture was cooled to room temperature, concentrated under reduced pressure, and the residue was purified by column chromatography (SiO₂, Petroleum ether/Ethyl acetate = 1/0 to 0/1, Ethyl acetate/Methanol = 1/0 to 2/1) to afford Compound 326-2 (50 mg, 8.15% yield) as a yellow solid.

LCMS m/z 680.8 [M+3]⁺.

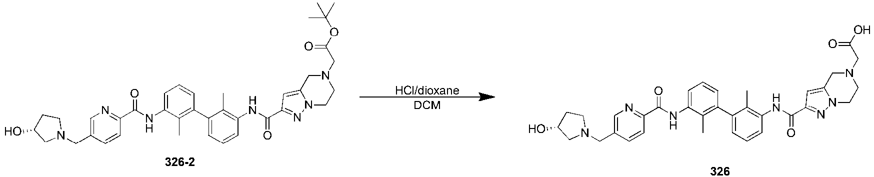

tert-Butyl 2-[2-[[2-methyl-3-[2-methyl-3-[[5-[[rac-(3R)-3-hydroxypyrrolidin-1-yl]methyl]pyridine-2-carbonyl]amino]phenyl]phenyl]carbamoyl]-6,7-dihydro-4H-pyrazolo[1,5-a]pyrazin-5-yl]acetate 326-2 (50 mg, 73.55 µmol, 1 eq) was dissolved in DCM (1 mL), and HCl/dioxane (2 M, 36.78 µL, 1 eq) was added. The resulting mixture was stirred at room temperature for 5 hours. After completion of the reaction, the mixture was concentrated under reduced pressure, and the residue was purified by reversed-phase HPLC (column: Waters Xbridge 150 * 25 mm * 5 um; mobile phase: [water (NH₄HCO₃)-ACN]; gradient: 12%-42% B over 9 min) to afford Compound 326 (22 mg, 47.4% yield) as a white solid

LCMS m/z 624.3 [M+1]⁺.

¹H NMR (400 MHz, DMSO-*d*₆) δ ppm 1.51 - 1.62 (m, 1 H) 1.95 (s, 3 H) 2.02 (s, 3 H) 2.35 (dd, *J*=9.57, 3.56 Hz, 1 H) 2.45 (td, *J*=8.22, 5.69 Hz, 1 H) 2.59 - 2.66 (m, 1 H) 2.71 (dd, *J*=9.57, 6.19 Hz, 1 H) 3.12 (br t, *J*=5.44 Hz, 2 H) 3.43 (br s, 2 H) 3.64 - 3.79 (m, 3 H) 3.88 (s, 2 H) 4.16 - 4.26 (m, 3 H) 4.67 - 4.78 (m, 1 H) 6.56 (s, 1 H) 6.98 (d, *J*=7.38 Hz, 2 H) 7.30 (dt, *J*=15.85, 7.77 Hz, 2 H) 7.59 (d, *J*=7.88 Hz, 1 H) 7.87 (d, *J*=7.50 Hz, 1 H) 7.99 (dd, *J*=8.00, 1.88 Hz, 1 H) 8.15 (d, *J*=8.00 Hz, 1 H) 8.65 (d, *J*=1.50 Hz, 1 H) 9.54 (s, 1 H) 10.33 (s, 1 H).

### [Preparation Example 108]

### Preparation of Compound 327, (R)-N-(3'-((2-(difluoromethyl)-7-((3-hydroxypyrrolidin-1-yl)methyl)pyrido[3,2-d]pyrimidin-4-yl)amino)-2,2'-dimethyl-[1,1'-biphenyl]-3-yl)-5-(2-hydroxyethyl)-4,5,6,7-tetrahydropyrazolo[1,5-a]pyrazine-2-carboxamide

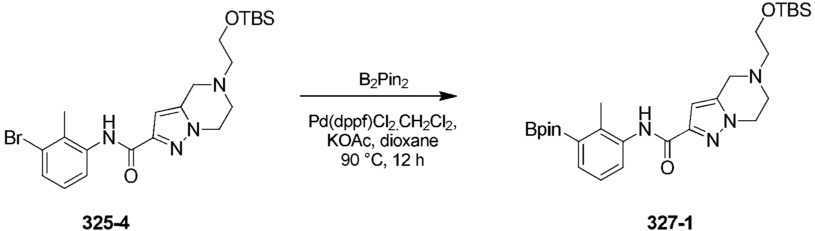

N-(3-bromo-2-methyl-phenyl)-5-[2-[tert-butyl(dimethyl)silyl]oxyethyl]-6,7-dihydro-4H-pyrazolo[1,5-a]pyrazine-2-carboxamide 325-4 (160 mg, 324.21 µmol, 1 eq), 4,4,5,5-tetramethyl-2-(4,4,5,5-tetramethyl-1,3,2-dioxaborolan-2-yl)-1,3,2-dioxaborolane (246.99 mg, 972.62 µmol, 3 eq), KOAc (95.45 mg, 972.62 µmol, 3 eq), and Pd(dppf)Cl₂·CH₂Cl₂ (26.48 mg, 32.42 µmol, 0.1 eq) were added to dioxane (6 mL), and the resulting mixture was stirred under a nitrogen atmosphere at 90 °C for 12 hours. After completion of the reaction, the mixture was filtered and concentrated under reduced pressure. The resulting residue was purified by flash silica gel chromatography (Petroleum ether/Ethyl acetate = 100/1 to 1/1) to afford Compound 327-1 (212 mg, 96.7% yield) as a yellow liquid.

LCMS m/z 541.2 [M+1]⁺

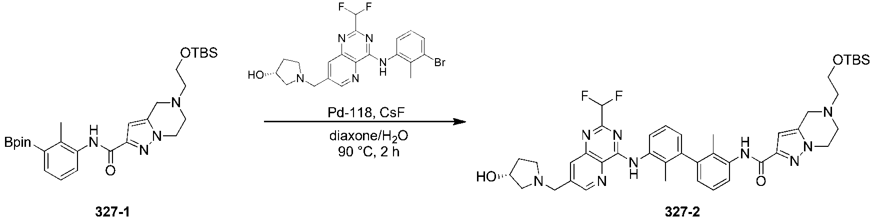

(3R)-1-[[4-(3-bromo-2-methyl-anilino)-2-(difluoromethyl)pyrido[3,2-d]pyrimidin-7-yl]methyl]pyrrolidin-3-ol 327-1 (120 mg, 258.45 µmol, 1 eq), 5-[2-[tert-butyl(dimethyl)silyl]oxyethyl]-N-[2-methyl-3-(4,4,5,5-tetramethyl-1,3,2-dioxaborolan-2-yl)phenyl]-6,7-dihydro-4H-pyrazolo[1,5-a]pyrazine-2-carboxamide 2 (153.68 mg, 284.30 µmol, 1.1 eq), ditert-butyl(cyclopentyl)phosphane;dichloropalladium;iron (16.84 mg, 25.85 µmol, 0.1 eq), and CsF (117.78 mg, 775.35 µmol, 3 eq) were added to a mixture of dioxane (2 mL)/ H₂O (0.5 mL), and the resulting mixture was stirred under a nitrogen atmosphere at 90 °C for 2 hours. After completion of the reaction, the mixture was filtered and concentrated under reduced pressure. The resulting residue was purified by prep-HPLC (column: Phenomenex Luna C18 150 * 40 mm * 15 um; mobile phase: [water (FA)-ACN]; gradient: 22%-52% B over 15 min) to afford Compound 327-2 (142 mg, 61.9% yield) as a white solid.

LCMS m/z 798.5 [M+1]⁺

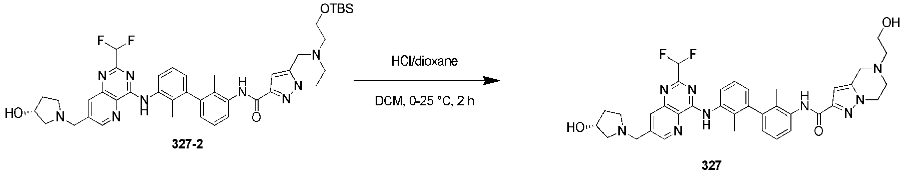

5-[2-[tert-butyl(dimethyl)silyl]oxyethyl]-N-[3-[3-[[2-(difluoromethyl)-7-[[(3R)-3-hydroxypyrrolidin-1-yl]methyl]pyrido[3,2-d]pyrimidin-4-yl]amino]-2-methyl-phenyl]-2-methyl-phenyl]-6,7-dihydro-4H-pyrazolo[1,5-a]pyrazine-2-carboxamide 327-2 (100 mg, 125.31 µmol, 1 eq) was dissolved in DCM (5 mL), and HCl/dioxane (2 M, 1.6 mL, 25.54 eq) was added at 0 °C. The resulting mixture was stirred at room temperature for 2 hours. After completion of the reaction, the mixture was concentrated under reduced pressure, and the residue was purified by flash silica gel chromatography (dichloromethane:methanol = 100/1 to 10/1) to afford Compound 327 (36 mg, 39.9% yield) as a white solid.

LCMS m/z 684.4 [M+1]⁺

¹H NMR (400 MHz, DMSO-*d₆*) δ = 11.99 - 11.23 (m, 2H), 10.57 (s, 1H), 9.80 - 9.68 (m, 1H), 9.34 - 9.18 (m, 1H), 8.62 (dd, *J* = 1.6, 19.8 Hz, 1H), 7.60 (d, *J* = 7.9 Hz, 1H), 7.52 (d, *J* = 7.9 Hz, 1H), 7.40 - 7.34 (m, 1H), 7.30 (t, *J* = 7.8 Hz, 1H), 7.08 (d, *J* = 7.5 Hz, 1H), 7.04 (d, *J* = 7.4 Hz, 1H), 6.88 - 6.57 (m, 2H), 4.76 - 4.66 (m, 2H), 4.58 (br t, *J* = 5.6 Hz, 2H), 4.51 - 4.39 (m, 2H), 3.91 - 3.87 (m, 3H), 3.68 - 3.55 (m, 2H), 3.47 - 3.39 (m, 2H), 3.36 - 3.23 (m, 2H), 3.18 - 2.91 (m, 1H), 2.42 - 2.16 (m, 1H), 1.99 (s, 3H), 1.94 (s, 3H), 1.90 - 1.77 (m, 1H)

### [Preparation Example 109]

### Preparation of Compound 328, (R)-2-(2-((3'-((2-(difluoromethyl)-7-((3-hydroxypyrrolidin-1-yl)methyl)pyrido[3,2-d]pyrimidin-4-yl)amino)-2,2'-dimethyl-[1,1'-biphenyl]-3-yl)carbamoyl)-6,7-dihydropyrazolo[1,5-a]pyrazin-5(4H)-yl)acetic acid

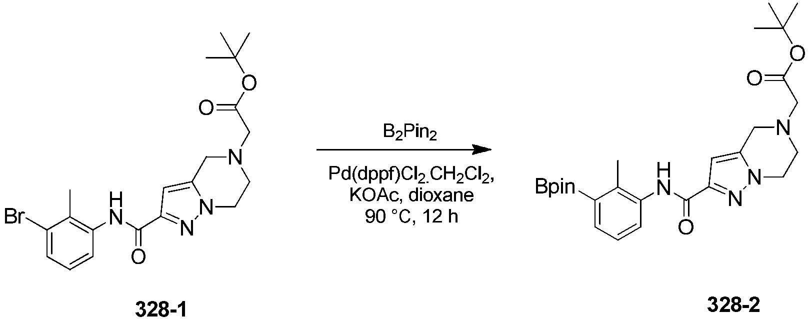

tert-Butyl 2-[2-[(3-bromo-2-methyl-phenyl)carbamoyl]-6,7-dihydro-4H-pyrazolo[1,5-a]pyrazin-5-yl]acetate 328-1 (160 mg, 356.08 µmol, 1 eq), 4,4,5,5-tetramethyl-2-(4,4,5,5-tetramethyl-1,3,2-dioxaborolan-2-yl)-1,3,2-dioxaborolane (271.26 mg, 1.07 mmol, 3 eq), KOAc (104.84 mg, 1.07 mmol, 3 eq), and Pd(dppf)Cl₂·CH₂Cl₂ (29.08 mg, 35.61 µmol, 0.1 eq) were added to dioxane (6 mL), and the resulting mixture was stirred under a nitrogen atmosphere at 90 °C for 12 hours. After completion of the reaction, the mixture was cooled to room temperature, filtered, and the filtrate was concentrated under reduced pressure. The resulting residue was purified by flash silica gel chromatography (0-50%, Petroleum ether/Ethyl acetate) to afford Compound 328-2 (208 mg, 92.9% yield) as a yellow liquid.

LCMS m/z 497.1 [M+1]⁺

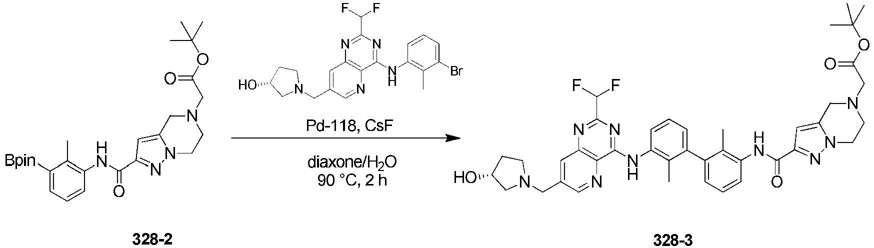

(3R)-1-[[4-(3-bromo-2-methyl-anilino)-2-(difluoromethyl)pyrido[3,2-d]pyrimidin-7-yl]methyl]pyrrolidin-3-ol 328-2 (120.00 mg, 258.45 µmol, 1 eq), tert-butyl 2-[2-[[2-methyl-3-(4,4,5,5-tetramethyl-1,3,2-dioxaborolan-2-yl)phenyl]carbamoyl]-6,7-dihydro-4H-pyrazolo[1,5-a]pyrazin-5-yl]acetate (141.13 mg, 284.30 µmol, 1.1 eq), ditert-butyl(cyclopentyl)phosphane;dichloropalladium;iron (16.84 mg, 25.85 µmol, 0.1 eq), and CsF (117.78 mg, 775.35 µmol, 3 eq) were added to a mixture of dioxane (8 mL) / H₂O (2 mL), and the resulting mixture was stirred under a nitrogen atmosphere at 90 °C for 12 hours. After completion of the reaction, the mixture was cooled to room temperature, filtered, and the filtrate was concentrated under reduced pressure. The resulting residue was purified by flash silica gel chromatography (0-50%, Petroleum ether/Ethyl acetate), followed by further purification by prep-HPLC (column: Phenomenex Luna C18 150 * 40 mm * 15 um; mobile phase: [water (FA)-ACN]; gradient: 22%-52% B over 15 min) to afford Compound 328-2 (132 mg, 61.6% yield) as a white solid.

LCMS m/z 754.4 [M+1]⁺

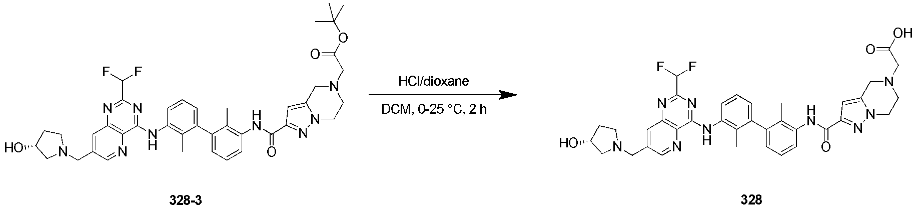

tert-Butyl 2-[2-[[3-[3-[[2-(difluoromethyl)-7-[[(3R)-3-hydroxypyrrolidin-1-yl]methyl]pyrido[3,2-d]pyrimidin-4-yl]amino]-2-methyl-phenyl]-2-methyl-phenyl]carbamoyl]-6,7-dihydro-4H-pyrazolo[1,5-a]pyrazin-5-yl]acetate 328-3 (100 mg, 132.65 µmol, 1 eq) was dissolved in DCM (5 mL), and HCl/dioxane (2 M, 1 mL, 15.08 eq) was added at 0 °C. The resulting mixture was stirred under a nitrogen atmosphere at room temperature for 2 hours. After completion of the reaction, the mixture was concentrated under reduced pressure, and the residue was purified by prep-HPLC (column: Phenomenex Luna C18 150 * 25 mm * 10 um; mobile phase: [water (HCl)-ACN]; gradient: 10%-40% B over 10 min) to afford Compound 328 (32 mg, 31.2% yield) as a white solid.

LCMS m/z 698.4 [M+1]⁺

¹H NMR (400 MHz, DMSO-*d₆*) δ = 12.10 - 11.39 (m, 1H), 10.57 (s, 1H), 9.70 (s, 1H), 9.38 - 9.20 (m, 1H), 8.63 (dd, *J* = 1.6, 18.6 Hz, 1H), 7.64 - 7.57 (m, 1H), 7.52 (d, *J* = 7.9 Hz, 1H), 7.40 - 7.33 (m, 1H), 7.30 (t, *J* = 7.8 Hz, 1H), 7.13 - 7.07 (m, 1H), 7.04 (d, *J* = 7.3 Hz, 1H), 6.88 - 6.57 (m, 2H), 4.76 - 4.67 (m, 2H), 4.60 (br s, 2H), 4.52 (br t, *J* = 5.2 Hz, 2H), 4.45 (br dd, *J* = 2.0, 19.4 Hz, 2H), 4.25 (br s, 3H), 3.83 (br s, 2H), 3.63 - 3.45 (m, 2H), 3.41 - 3.01 (m, 3H), 2.41 - 2.04 (m, 1H), 1.99 (s, 3H), 1.93 (s, 3H)

### [Preparation Example 110]

### Preparation of Compound 329, (R)-isopropyl 1-((S)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylate, and Compound 329-1, (R)-isopropyl 1-((R)-2-((3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)pyrrolidine-3-carboxylate

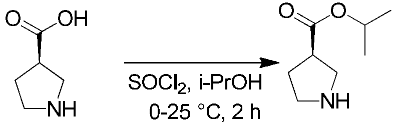

(3R)-Pyrrolidine-3-carboxylic acid (300 mg, 1.98 mmol, 1 eq, HCl salt) was dissolved in propan-2-ol (10 mL), and SOCl₂ (706.33 mg, 5.94 mmol, 431.21 µL, 3 eq) was added at 0 °C. The resulting mixture was stirred at room temperature for 5 hours. After completion of the reaction, the mixture was concentrated under reduced pressure, and the residue was purified by flash silica gel chromatography (Dichloromethane:Methanol = 100:1 to 10:1) to obtain isopropyl (3R)-pyrrolidine-3-carboxylate (162 mg, 46.8% yield) as a yellow liquid.

¹H NMR (400 MHz, CHLOROFORM-d) δ = 9.98 - 9.71 (m, 1H), 5.04 (spt, J = 6.3 Hz, 1H), 3.67 - 3.58 (m, 1H), 3.57 - 3.49 (m, 1H), 3.43 (br s, 2H), 3.32 - 3.08 (m, 1H), 2.45 - 2.19 (m, 2H), 1.26 (dd, J = 2.2, 6.2 Hz, 6H).

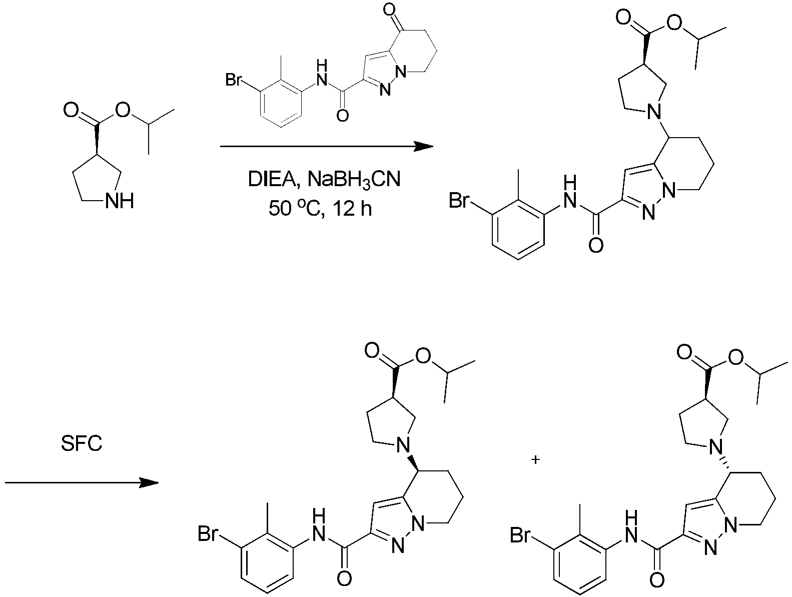

N-(3-bromo-2-methyl-phenyl)-4-oxo-6,7-dihydro-5H-pyrazolo[1,5-a]pyridine-2-carboxamide (1 g, 2.87 mmol, 1 eq), isopropyl (3R)-pyrrolidine-3-carboxylate (2.26 g, 14.36 mmol, 5 eq), DIPEA (1.86 g, 14.36 mmol, 2.50 mL, 5 eq), and HOAc (1.72 g, 28.72 mmol, 1.64 mL, 10 eq) were dissolved in MeOH (10 mL), and NaBH₃CN (541.42 mg, 8.62 mmol, 3 eq) was added. The resulting mixture was stirred at 50 °C for 2 hours. After completion of the reaction, the mixture was cooled to room temperature, H₂O (40 mL) was added, and the mixture was extracted with ethyl acetate (30 mL x 3). The combined organic layers were washed with brine (60 mL), dried over Na₂SO₄, filtered, and concentrated under reduced pressure. The resulting residue was purified by prep-HPLC (column: Waters Xbridge Prep OBD C18 150 * 40 mm * 10 µm; mobile phase: [water (NH₄HCO₃)-ACN]; gradient: 55%-85% B over 52 min). The obtained white solid was further separated by SFC (column: ChiralPak IH, 250 * 50 mm * 10 µm; mobile phase: CO₂-ACN/EtOH (0.1% NH₃·H₂O); B%: 60%, isocratic elution mode) to afford isopropyl (3R)-1-[(4S)-2-[(3-bromo-2-methyl-phenyl)carbamoyl]-4,5,6,7-tetrahydropyrazolo[1,5-a]pyridin-4-yl]pyrrolidine-3-carboxylate (132 mg, 44.6% yield) and isopropyl (3R)-1-[(4R)-2-[(3-bromo-2-methylphenyl)carbamoyl]-4,5,6,7-tetrahydropyrazolo[1,5-a]pyridin-4-yl]pyrrolidine-3-carboxylate (121 mg, 40.9% yield), each obtained as a white solid.

LCMS m/z 490.9 [M+2]⁺

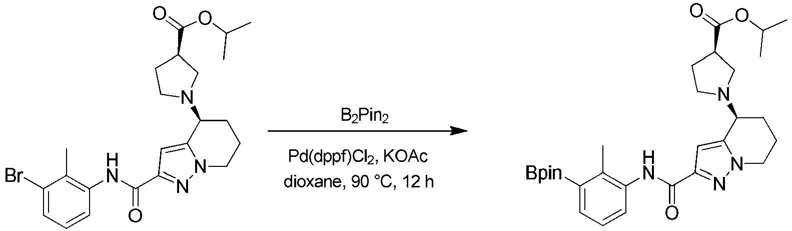

Isopropyl (3R)-1-[(4S)-2-[(3-bromo-2-methyl-phenyl)carbamoyl]-4,5,6,7-tetrahydropyrazolo[1,5-a]pyridin-4-yl]pyrrolidine-3-carboxylate (100 mg, 204.33 µmol, 1 eq), 4,4,5,5-tetramethyl-2-(4,4,5,5-tetramethyl-1,3,2-dioxaborolan-2-yl)-1,3,2-dioxaborolane (155.66 mg, 612.99 µmol, 3 eq), KOAc (60.16 mg, 612.99 µmol, 3 eq), and Pd(dppf)Cl₂·CH₂Cl₂ (16.69 mg, 20.43 µmol, 0.1 eq) were added to dioxane (3 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by flash silica gel chromatography (Petroleum ether/Ethyl acetate = 100/1 to 1/1) to obtain (3R)-1-[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (120 mg, 87.5% yield) as a colorless liquid.

LCMS m/z 537.3 [M+1]⁺

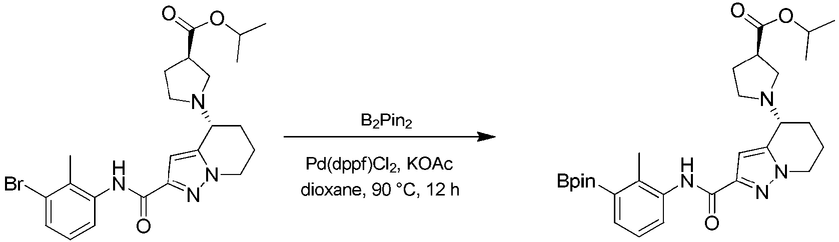

Isopropyl (3R)-1-[(4R)-2-[(3-bromo-2-methyl-phenyl)carbamoyl]-4,5,6,7-tetrahydropyrazolo[1,5-a]pyridin-4-yl]pyrrolidine-3-carboxylate (100 mg, 204.33 µmol, 1 eq), 4,4,5,5-tetramethyl-2-(4,4,5,5-tetramethyl-1,3,2-dioxaborolan-2-yl)-1,3,2-dioxaborolane (155.66 mg, 612.99 µmol, 3 eq), KOAc (60.16 mg, 612.99 µmol, 3 eq), and Pd(dppf)Cl₂·CH₂Cl₂ (16.69 mg, 20.43 µmol, 0.1 eq) were added to dioxane (5 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by flash silica gel chromatography (Petroleum ether/Ethyl acetate = 100/1 to 1/1) to obtain isopropyl (3R)-1-[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (112 mg, 81.7% yield) as a colorless liquid.

LCMS m/z 537.2 [M+1]⁺

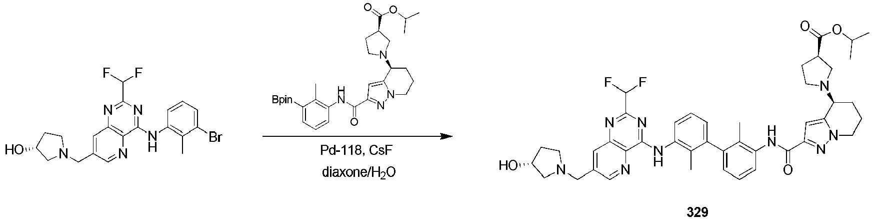

(3R)-1-[[4-(3-bromo-2-methyl-anilino)-2-(difluoromethyl)pyrido[3,2-d]pyrimidin-7-yl]methyl]pyrrolidin-3-ol (70 mg, 150.76 µmol, 1 eq), isopropyl (3R)-1-[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (97.06 mg, 180.92 µmol, 1.2 eq), CsF (68.70 mg, 452.29 µmol, 3 eq), and ditert-butyl(cyclopenta-1,4-dien-1-yl)phosphane;dichloropalladium;iron (9.83 mg, 15.08 µmol, 0.1 eq) were added to a mixture of dioxane (5 mL)/H₂O (1 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by prep-HPLC (column: Waters Xbridge 150 * 25 mm * 5 um; mobile phase: [water (NH₄HCO₃)-ACN]; gradient: 55%-85% B over 15 min) to afford Compound 329 (21 mg, 16.6% yield) as a white solid.

LCMS m/z 794.5 [M+1]⁺

¹H NMR (400 MHz, DMSO-d₆) δ = 10.38 (s, 1H), 9.53 (d, J = 2.0 Hz, 1H), 8.95 (d, J = 1.9 Hz, 1H), 8.17 (s, 1H), 7.66 (d, J = 7.6 Hz, 1H), 7.57 (br d, J = 7.6 Hz, 1H), 7.42 - 7.34 (m, 1H), 7.29 (t, J = 7.8 Hz, 1H), 7.09 - 6.99 (m, 2H), 6.75 - 6.56 (m, 2H), 4.93 - 4.85 (m, 1H), 4.75 (br s, 1H), 4.25 - 4.13 (m, 3H), 3.96 - 3.80 (m, 3H), 2.99 - 2.91 (m, 2H), 2.78 - 2.61 (m, 6H), 2.35 - 2.18 (m, 3H), 2.07 - 2.02 (m, 1H), 1.99 (s, 3H), 1.95 (s, 3H), 1.91 (br d, J = 4.9 Hz, 3H), 1.63 - 1.54 (m, 1H), 1.17 (t, J = 5.8 Hz, 6H).

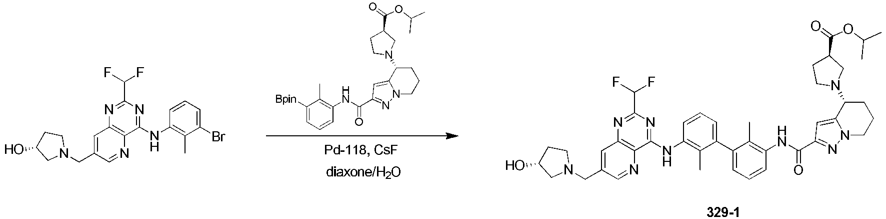

(3R)-1-[[4-(3-bromo-2-methyl-anilino)-2-(difluoromethyl)pyrido[3,2-d]pyrimidin-7-yl]methyl]pyrrolidin-3-ol (60 mg, 129.23 µmol, 1 eq), isopropyl (3R)-1-[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (83.19 mg, 155.07 µmol, 1.2 eq), CsF (58.89 mg, 387.68 µmol, 3 eq), and ditert-butyl(cyclopenta-1,4-dien-1-yl)phosphane;dichloropalladium;iron (8.42 mg, 12.92 µmol, 0.1 eq) were added to a mixture of dioxane (3 mL) and H₂O (0.5 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by prep-HPLC (column: Phenomenex Luna C18 150 * 25 mm * 10 µm; mobile phase: [water (HCl)-ACN]; gradient: 10%-40% B over 18 min) to afford Compound 329-1 (17 mg, 15.05% yield) as a white solid.

LCMS m/z 794.5 [M+1]⁺

¹H NMR (400 MHz, DMSO-d₆) δ = 11.74 - 11.46 (m, 1H), 11.44 - 10.98 (m, 1H), 10.57 (s, 1H), 9.73 (br s, 1H), 9.32 - 9.12 (m, 1H), 8.70 - 8.51 (m, 1H), 7.60 (br d, J = 8.0 Hz, 1H), 7.51 (br d, J = 8.0 Hz, 1H), 7.37 (t, J = 7.8 Hz, 1H), 7.30 (t, J = 7.8 Hz, 1H), 7.22 - 7.16 (m, 1H), 7.07 (dd, J = 7.2, 20.8 Hz, 2H), 6.73 (t, J = 54.6 Hz, 1H), 5.62 - 5.43 (m, 1H), 5.00 - 4.85 (m, 2H), 4.77 - 4.65 (m, 2H), 4.52 - 4.40 (m, 1H), 4.32 - 4.16 (m, 2H), 3.76 - 3.63 (m, 1H), 3.63 - 3.46 (m, 3H), 3.22 - 3.01 (m, 1H), 2.54 (s, 1H), 2.42 - 2.30 (m, 2H), 2.29 - 2.17 (m, 3H), 2.12 - 2.03 (m, 1H), 2.00 (s, 3H), 1.94 (s, 3H), 1.92 - 1.77 (m, 1H), 1.22 (br d, J = 6.0 Hz, 6H).

### [Preparation Example 111]

### Preparation of Compound 330, isopropyl 2-(((S)-2-((3'-((2-(difluoromethyl)-7-((^{®}-3-hydroxypyrrolidin-1-yl)methyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5558yridinedin-4-yl)amino)acetate

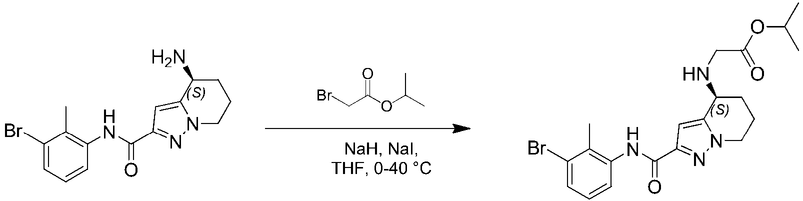

(4S)-4-amino-N-(3-bromo-2-methyl-phenyl)-4,5,6,7-tetrahydropyrazolo[1,5-a]pyridine-2-carboxamide (2 g, 5.73 mmol, 1 eq), isopropyl 2-chloroacetate (782.17 mg, 5.73 mmol, 1 eq), NaH (229.08 mg, 5.73 mmol, 60% purity, 1 eq), and NaI (171.68 mg, 1.15 mmol, 0.2 eq) were dissolved in THF (20 mL), and the resulting mixture was stirred under a nitrogen atmosphere at 40 °C for 12 hours. After completion of the reaction, the mixture was cooled to room temperature, diluted with aq. saturated NH₄Cl solution (50 mL), and extracted with EtOAc (30 mL × 3). The combined organic layers were washed with brine (30 mL), dried over Na₂SO₄, filtered, and concentrated under reduced pressure. The resulting residue was purified by column chromatography (SiO₂, Petroleum ether/Ethyl acetate = 0/1 to 1/0) to obtain isopropyl 2-[[(4S)-2-[(3-bromo-2-methyl-phenyl)carbamoyl]-4,5,6,7-tetrahydropyrazolo[1,5559yridinedin-4-yl]amino]acetate (600 mg, 22.8% yield) as a yellow solid.

LCMS m/z 448.11 [M+1]⁺.

¹H NMR (400 MHz, DMSO-d₆) δ = 9.-7 - 9.63 (m, 1H), 7.48 (d, J = 7.9 Hz, 1H), 7.41 (d, J = 7.9 Hz, 1H), 7.15 (t, J = 8.0 Hz,1H), 6.69 (s, 1H), 4.-9 - 4.89 (m, 1H), 4.-7 - 4.08 (m, 2H), 4.-5 - 3.90 (m, 1H), 2.26 (s, 3H), 2.-3 - 1.86 (m, 2H), 1.-1 - 1.64 (m,1H), 1.20 (d, J = 6.2 Hz, 6H), 1.17 (br s, 1H).

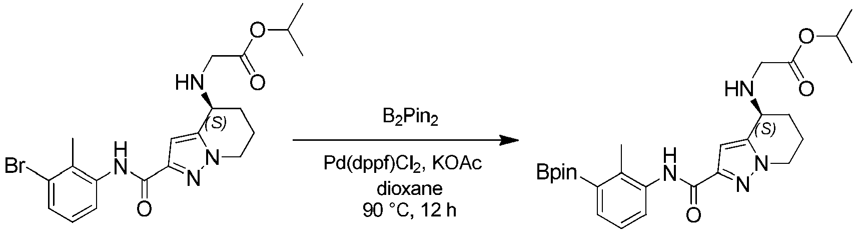

Isopropyl 2-[[(4S)-2-[(3-bromo-2-methyl-phenyl)carbamoyl]-4,5,6,7-tetrahydropyrazolo[1,5 559 yridinedin-4-yl]amino]acetate 3 (300 mg, 667.65 µmol, 1 eq), 4,4,5,5-tetramethyl-2-(4,4,5,5-tetramethyl-1,3,2-dioxaborolan-2-yl)-1,3,2-dioxaborolane (508.62 mg, 2.00 mmol, 3 eq), Pd(dppf)Cl₂ (48.85 mg, 66.76 µmol, 0.1 eq), and KOAc (196.57 mg, 2.00 mmol, 3 eq) were added to dioxane (10 mL), and the resulting mixture was stirred under a nitrogen atmosphere at 90 °C for 12 hours. After completion of the reaction, the mixture was cooled to room temperature, filtered, and the filtrate was concentrated under reduced pressure. The resulting residue was purified by column chromatography (SiO₂, ethyl acetate/methanol = 1/0 to 3/1) to obtain isopropyl 2-[[(4S)-2-[[2-methyl-3-(4,4,5,5-tetramethyl-1,3,2-dioxaborolan-2-yl)phenyl]carbamoyl]-4,5,6,7-tetrahydropyrazolo[1,5559yridinedin-4-yl]amino]acetate (280 mg, 69.2% yield) as a yellow solid.

LCMS m/z 496.29 [M+1]⁺.

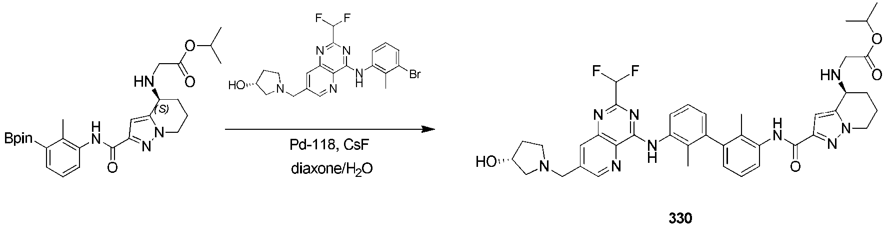

(3R)-1-[[4-(3-bromo-2-methyl-anilino)-2-(difluoromethyl)pyrido[3,2-d]pyrimidin-7-yl]methyl]pyrrolidin-3-ol (80 mg, 172.30 µmol, 1 eq), isopropyl 2-[[(4S)-2-[[2-methyl-3-(4,4,5,5-tetramethyl-1,3,2-dioxaborolan-2-yl)phenyl]carbamoyl]-4,5,6,7-tetrahydropyrazolo[1,5 560yridinedin-4-yl]amino]acetate (111.19 mg, 223.99 µmol, 1.3 eq), ditert-butyl(cyclopenta-1,4-dien-1-yl)phosphane;dichloropalladium;iron (11.23 mg, 17.23 µmol, 0.1 eq), and CsF (78.52 mg, 516.90 µmol, 3 eq) were added to a mixture of dioxane (2 mL) / H₂O (0.5 mL), and the resulting mixture was stirred under a nitrogen atmosphere at 90 °C for 12 hours. After completion of the reaction, the mixture was cooled to room temperature, filtered, and the filtrate was concentrated under reduced pressure. The residue was purified by prep-TLC (SiO₂, Petroleum ether/Ethyl acetate = 1:1), followed by further purification by reversed-phase HPLC (column: Waters Xbridge 150 * 25 mm * 5 um; mobile phase: [water (NH₄HCO₃)-ACN]; gradient: 40%-70% B over 10 min) to afford Compound 330 (10 mg, 7.39% yield) as a yellow solid.

LCMS m/z 753.36[M+1]⁺.

¹H NMR (400 MHz, DMSO-d₆) δ = 10.38 (s, 1H), 9.52 (s, 1H), 8.95 (s, 1H), 8.16 (s, 1H), 7.66 (d, J = 7.8 Hz, 1H), 7.57 (d, J = 7.8 Hz, 1H), 7.35 (t, J = 7.8 Hz, 1H), 7.28 (t, J = 7.8 Hz, 1H), 7.11 - 7.05 (m, 1H), 7.01 (d, J = 7.6 Hz, 1H), 6.87 - 6.53 (m, 2H), 4.94 (td, J = 6.2, 12.5 Hz, 1H), 4.73 (d, J = 4.6 Hz, 1H), 4.30 - 4.05 (m, 3H), 3.95 - 3.77 (m, 3H), 2.80 - 2.60 (m, 3H), 2.47 - 2.02 (m, 5H), 2.01 - 1.92 (m, 7H), 1.90 - 1.48 (m, 3H), 1.28 - 1.14 (m, 7H).

### [Preparation Example 112]

### Preparation of Compound 331, isopropyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoate, and Compound 331-1, isopropyl 2-(((R)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoate

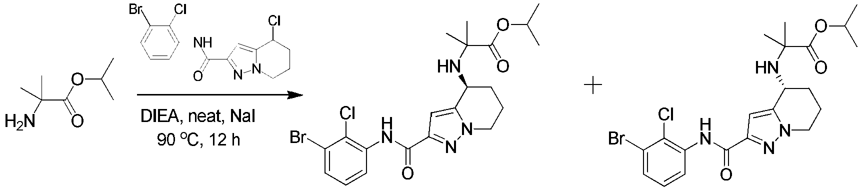

N-(3-bromo-2-chloro-phenyl)-4-chloro-4,5,6,7-tetrahydropyrazolo[1,5-a]pyridine-2-carboxamide (500 mg, 1.29 mmol, 1 eq) and isopropyl 2-amino-2-methyl-propanoate (932.98 mg, 6.43 mmol, 5 eq) were combined, and DIPEA (830.45 mg, 6.43 mmol, 1.12 mL, 5 eq) and NaI (38.53 mg, 257.02 µmol, 0.2 eq) were added. The resulting mixture was stirred at 100 °C for 2 hours. After completion of the reaction, the mixture was cooled to room temperature, diluted with H₂O (20 mL), and extracted with ethyl acetate (10 mL * 3). The combined organic layers were washed with brine (60 mL), dried over Na₂SO₄, filtered, and concentrated under reduced pressure. The residue was purified by prep-HPLC (column: Waters Xbridge Prep OBD C18 150 * 40 mm * 10 µm; mobile phase: [water (NH₄HCO₃)-ACN]; gradient: 60%-90% B over 15 min) and further separated by SFC (column: DAICEL CHIRALPAK AD 250 * 30 mm * 10 µm; mobile phase: CO₂-ACN/EtOH (0.1% NH₃·H₂O); B%: 70%, isocratic elution mode) to afford isopropyl 2-[[(4S)-2-[(3-bromo-2-chloro-phenyl)carbamoyl]-4,5,6,7-tetrahydropyrazolo[1,5-a]pyridin-4-yl]amino]-2-methyl-propanoate (236 mg, 35.0% yield) and isopropyl 2-[[(4R)-2-[(3-bromo-2-chloro-phenyl)carbamoyl]-4,5,6,7-tetrahydropyrazolo[1,5-a]pyridin-4-yl]amino]-2-methyl-propanoate (230 mg, 34.5% yield), each obtained as a white solid.

LCMS m/z 499.1 [M+2]⁺

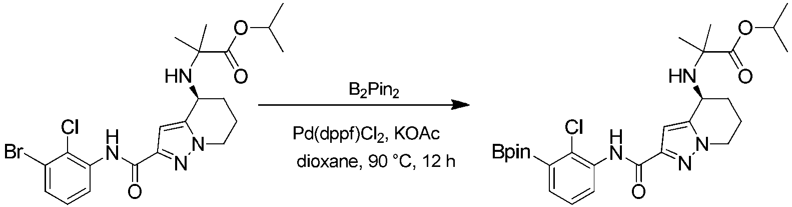

Isopropyl 2-[[(4S)-2-[(3-bromo-2-chloro-phenyl)carbamoyl]-4,5,6,7-tetrahydropyrazolo[1,5-a]pyridin-4-yl]amino]-2-methyl-propanoate (120 mg, 241.05 µmol, 1 eq), 4,4,5,5-tetramethyl-2-(4,4,5,5-tetramethyl-1,3,2-dioxaborolan-2-yl)-1,3,2-dioxaborolane (183.64 mg, 723.16 µmol, 3 eq), KOAc (70.97 mg, 723.16 µmol, 3 eq), and Pd(dppf)Cl₂·CH₂Cl₂ (19.69 mg, 24.11 µmol, 0.1 eq) were added to dioxane (3 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by flash silica gel chromatography (Petroleum ether/Ethyl acetate = 100/1 to 1/1) to obtain isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (152 mg, 92.5% yield) as a white solid.

LCMS m/z 545.3 [M+1]⁺

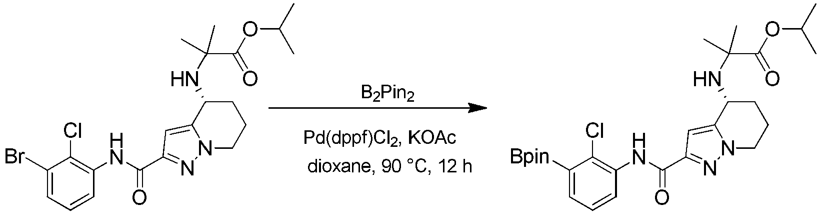

Isopropyl 2-[[(4R)-2-[(3-bromo-2-chloro-phenyl)carbamoyl]-4,5,6,7-tetrahydropyrazolo[1,5-a]pyridin-4-yl]amino]-2-methyl-propanoate (120 mg, 241.05 µmol, 1 eq), 4,4,5,5-tetramethyl-2-(4,4,5,5-tetramethyl-1,3,2-dioxaborolan-2-yl)-1,3,2-dioxaborolane (183.64 mg, 723.16 µmol, 3 eq), KOAc (70.97 mg, 723.16 µmol, 3 eq), and Pd(dppf)Cl₂·CH₂Cl₂ (19.69 mg, 24.11 µmol, 0.1 eq) were added to dioxane (3 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by flash silica gel chromatography (Petroleum ether/Ethyl acetate = 100/1 to 1/1) to obtain isopropyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (146 mg, 88.9% yield) as a white solid.

LCMS m/z 545.2 [M+1]⁺

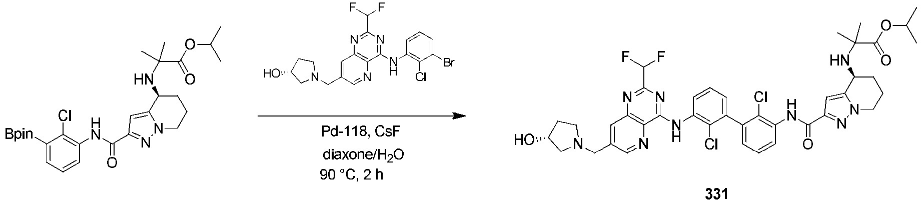

(3R)-1-[[4-(3-bromo-2-chloro-anilino)-2-(difluoromethyl)pyrido[3,2-d]pyrimidin-7-yl]methyl]pyrrolidin-3-ol (80 mg, 165.04 µmol, 1 eq), isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (98.92 mg, 181.55 µmol, 1.1 eq), CsF (75.21 mg, 495.13 µmol, 3 eq), and ditert-butyl(cyclopenta-1,4-dien-1-yl)phosphane;dichloropalladium;iron (10.76 mg, 16.50 µmol, 0.1 eq) were added to a mixture of dioxane (4 mL) / H₂O (1 mL), and the resulting mixture was stirred under a nitrogen atmosphere at 90 °C for 2 hours. After completion of the reaction, the mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 23%-53% B over 20 min) to afford Compound 331 (19 mg, 28.8% yield) as a white solid.

LCMS m/z 822.5 [M+1]⁺

¹H NMR (400 MHz, DMSO-*d₆* ) δ = 11.90 - 11.05 (m, 1H), 10.44 (s, 1H), 10.09 - 9.82 (m, 1H), 9.72 - 9.38 (m, 1H), 9.35 - 9.20 (m, 1H), 8.76 - 8.64 (m, 1H), 8.47 - 8.38 (m, 1H), 8.30 - 8.13 (m, 1H), 7.60 (t, *J* = 7.9 Hz, 1H), 7.52 (t, *J* = 7.9 Hz, 1H), 7.28 (d, *J* = 7.5 Hz, 1H), 7.22 (br d, *J* = 7.9 Hz, 1H), 7.16 - 6.73 (m, 2H), 5.66 - 5.38 (m, 1H), 4.94 (br s, 1H), 4.81 - 4.63 (m, 2H), 4.45 (br d, *J* = 21.0 Hz, 1H), 4.35 - 3.94 (m, 2H), 3.91 - 3.37 (m, 4H), 3.07 (br dd, *J* = 2.3, 10.1 Hz, 1H), 2.39 - 2.22 (m, 2H), 2.13 - 1.99 (m, 2H), 1.95 - 1.82 (m, 1H), 1.68 (br d, *J* = 2.0 Hz, 3H), 1.24 (br s, 8H).

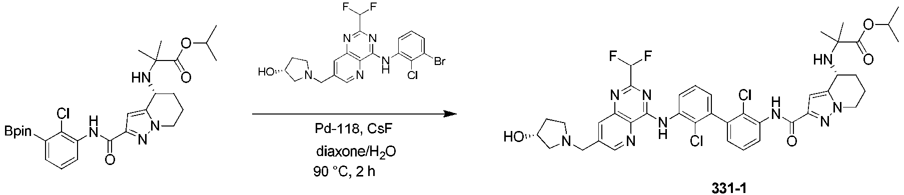

(3R)-1-[[4-(3-bromo-2-chloro-anilino)-2-(difluoromethyl)pyrido[3,2-d]pyrimidin-7-yl]methyl]pyrrolidin-3-ol (80 mg, 165.04 µmol, 1 eq), isopropyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (98.92 mg, 181.55 µmol, 1.1 eq), CsF (75.21 mg, 495.13 µmol, 3 eq), and ditert-butyl(cyclopenta-1,4-dien-1-yl)phosphane;dichloropalladium;iron (10.76 mg, 16.50 µmol, 0.1 eq) were added to a mixture of dioxane (4 mL) / H₂O (1 mL), and the resulting mixture was stirred under a nitrogen atmosphere at 90 °C for 2 hours. After completion of the reaction, the mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 23%-53% B over 20 min) to afford Compound 331-1 (21 mg, 38.2% yield) as a white solid.

LCMS m/z 822.4 [M+1]⁺

¹H NMR (400 MHz, DMSO-*d₆* ) δ = 11.73 - 10.93 (m, 1H), 10.44 (s, 1H), 9.68 (br s, 1H), 9.64 - 9.32 (m, 1H), 9.32 - 9.20 (m, 1H), 8.78 - 8.64 (m, 1H), 8.42 (br dd, *J* = 4.5, 8.1 Hz, 1H), 8.30 - 8.12 (m, 1H), 7.60 (t, *J* = 7.9 Hz, 1H), 7.52 (t, *J* = 7.9 Hz, 1H), 7.28 (br d, *J* = 7.6 Hz, 1H), 7.22 (br d, *J* = 7.4 Hz, 1H), 7.13 - 6.58 (m, 2H), 5.65 - 5.41 (m, 1H), 5.06 - 4.87 (m, 1H), 4.81 - 4.62 (m, 2H), 4.55 - 4.40 (m, 1H), 4.35 - 4.09 (m, 2H), 3.73 - 3.44 (m, 2H), 3.15 - 2.99 (m, 1H), 2.37 - 2.23 (m, 2H), 2.17 - 1.95 (m, 3H), 1.92 - 1.83 (m, 1H), 1.75 - 1.54 (m, 4H), 1.38 - 1.13 (m, 8H).

### [Preparation Example 113]

### Preparation of Compound 332, (R)-isopropyl 1-((S)-2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylate, and Compound 332-1, (R)-isopropyl 1-((R)-2-((2,2'-dichloro-3'-(5-(((2-hydroxyethyl)amino)methyl)picolinamido)-[1,1'-biphenyl]-3-yl)carbamoyl)-4,5,6,7-tetrahydropyrazolo[1,5-a]pyridin-4-yl)pyrrolidine-3-carboxylate

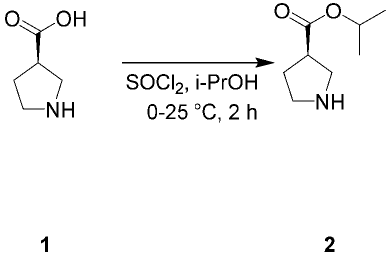

(3R)-Pyrrolidine-3-carboxylic acid (5 g, 32.98 mmol, 1 eq, HCl) was dissolved in propan-2-ol (50 mL), and SOCl₂ (11.77 g, 98.95 mmol, 7.19 mL, 3 eq) was added at 0 °C. The resulting mixture was stirred at room temperature for 5 hours. The mixture was then filtered, and the filtrate was concentrated under reduced pressure. The resulting residue was purified by flash silica gel chromatography (Dichloromethane:Methanol = 100:1 to 10:1) to obtain isopropyl (3R)-pyrrolidine-3-carboxylate (6.12 g, 94.4% yield) as a colorless liquid.

¹H NMR (400 MHz, CHLOROFORM-d) δ = 4.98 (td, *J* = 6.3, 12.5 Hz, 1H), 4.79 - 4.70 (m, 2H), 3.64 - 3.51 (m, 1H), 3.42 - 3.33 (m, 2H), 3.26 - 3.11 (m, 1H), 2.45 - 2.25 (m, 1H), 2.24 - 2.07 (m, 1H), 1.27 (d, *J* = 6.3 Hz, 6H).

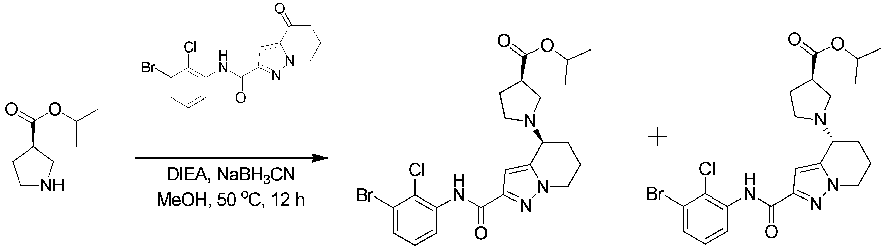

N-(3-bromo-2-chloro-phenyl)-4-oxo-6,7-dihydro-5H-pyrazolo[1,5-a]pyridine-2-carboxamide (500 mg, 1.36 mmol, 1 eq), isopropyl (3R)-pyrrolidine-3-carboxylate (1.07 g, 6.78 mmol, 5 eq), HOAc (488.72 mg, 8.14 mmol, 465.90 µL, 6 eq), and DIPEA (876.53 mg, 6.78 mmol, 1.18 mL, 5 eq) were dissolved in MeOH (10 mL), and the resulting mixture was stirred at 50 °C for 2 hours. After completion of the reaction, the mixture was cooled to room temperature, diluted with H₂O (40 mL), and extracted with ethyl acetate (30 mL × 3). The combined organic layers were washed with brine (60 mL), dried over Na₂SO₄, filtered, and concentrated under reduced pressure. The residue was purified by flash silica gel chromatography (Petroleum ether/Ethyl acetate = 100/1 to 3/1). The obtained white solid was further separated by SFC (column: DAICEL CHIRALCEL OJ 250 * 30 mm * 10 µm; mobile phase: CO₂-i-PrOH (0.1% NH₃·H₂O); B%: 30%, isocratic elution mode) to afford isopropyl (3R)-1-[(4S)-2-[(3-bromo-2-chloro-phenyl)carbamoyl]-4,5,6,7-tetrahydropyrazolo[1,5-a]pyridin-4-yl]pyrrolidine-3-carboxylate (182 mg, 25.0% yield) and isopropyl (3R)-1-[(4R)-2-[(3-bromo-2-chloro-phenyl)carbamoyl]-4,5,6,7-tetrahydropyrazolo[1,5-a]pyridin-4-yl]pyrrolidine-3-carboxylate (179 mg, 24.3% yield), each obtained as a white solid..

LCMS m/z 511.0 [M+2]⁺

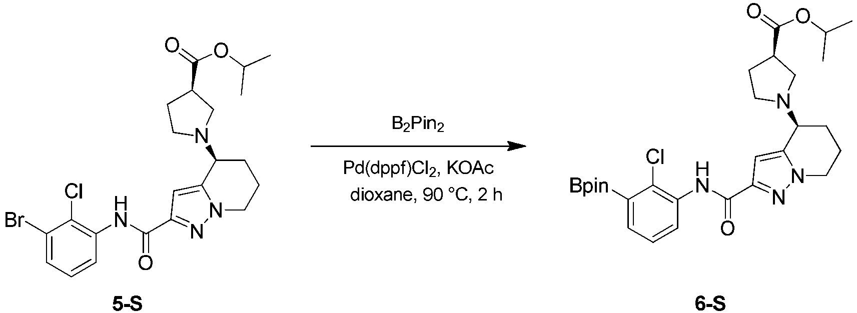

Isopropyl (3R)-1-[(4S)-2-[(3-bromo-2-chloro-phenyl)carbamoyl]-4,5,6,7-tetrahydropyrazolo[1,5-a]pyridin-4-yl]pyrrolidine-3-carboxylate (150 mg, 294.22 µmol, 1 eq), 4,4,5,5-tetramethyl-2-(4,4,5,5-tetramethyl-1,3,2-dioxaborolan-2-yl)-1,3,2-dioxaborolane (224.14 mg, 882.66 µmol, 3 eq), KOAc (86.62 mg, 882.66 µmol, 3 eq), and Pd(dppf)Cl₂·CH₂Cl₂ (24.03 mg, 29.42 µmol, 0.1 eq) were added to dioxane (4 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by flash silica gel chromatography (Petroleum ether/Ethyl acetate = 100/1 to 1/1) to obtain isopropyl (3R)-1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (152 mg, 74.2% yield) as a colorless liquid.

LCMS m/z 557.4 [M+1]⁺

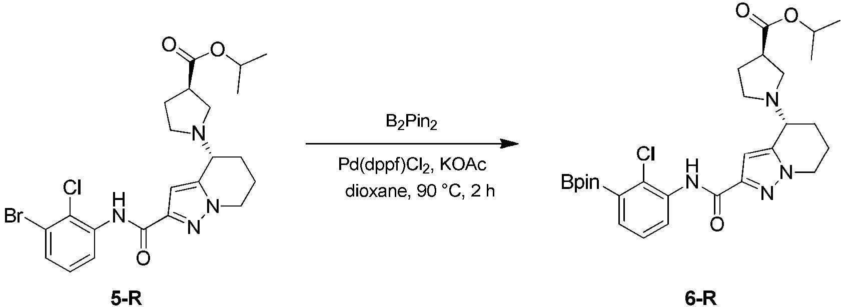

Isopropyl (3R)-1-[(4R)-2-[(3-bromo-2-chloro-phenyl)carbamoyl]-4,5,6,7-tetrahydropyrazolo[1,5-a]pyridin-4-yl]pyrrolidine-3-carboxylate (150 mg, 294.22 µmol, 1 eq), 4,4,5,5-tetramethyl-2-(4,4,5,5-tetramethyl-1,3,2-dioxaborolan-2-yl)-1,3,2-dioxaborolane (224.14 mg, 882.66 µmol, 3 eq), KOAc (86.62 mg, 882.66 µmol, 3 eq), and Pd(dppf)Cl₂·CH₂Cl₂ (24.03 mg, 29.42 µmol, 0.1 eq) were added to dioxane (5 mL), and the resulting mixture was stirred under a nitrogen atmosphere at 90 °C for 2 hours. After completion of the reaction, the mixture was cooled to room temperature, filtered, and the filtrate was concentrated under reduced pressure. The resulting residue was purified by flash silica gel chromatography (Petroleum ether/Ethyl acetate = 100/1 to 1/1) to obtain isopropyl (3R)-1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (152 mg, 74.2% yield) as a colorless liquid.

LCMS m/z 557.3 [M+1]⁺

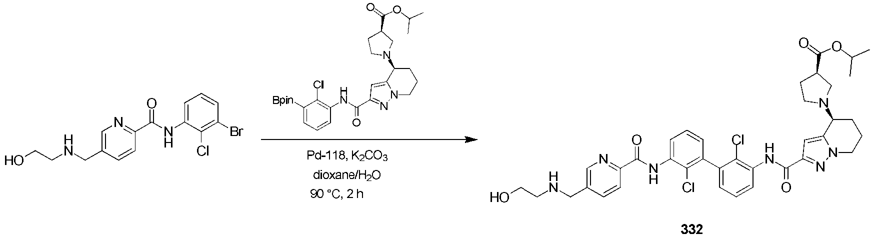

N-(3-bromo-2-chloro-phenyl)-5-[(2-hydroxyethylamino)methyl]pyridine-2-carboxamide (50 mg, 129.99 µmol, 1 eq), isopropyl (3R)-1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (86.87 mg, 155.98 µmol, 1.2 eq), CsF (59.23 mg, 389.96 µmol, 3 eq), and ditert-butyl(cyclopenta-1,4-dien-1-yl)phosphane;dichloropalladium;iron (8.47 mg, 13.00 µmol, 0.1 eq) were added to a mixture of dioxane (3 mL) / H₂O (1 mL), and the resulting mixture was stirred under a nitrogen atmosphere at 90 °C for 2 hours. After completion of the reaction, the mixture was cooled to room temperature, filtered, and the filtrate was concentrated under reduced pressure. The residue was purified by prep-HPLC (column: Waters Xbridge 150 * 25 mm * 5 µm; mobile phase: [water (NH₄HCO₃)-ACN]; gradient: 50%-80% B over 10 min) to afford Compound 332 (16 mg, 15.9% yield) as a white solid.

LCMS m/z 734.4 [M+1]⁺

¹H NMR (400 MHz, DMSO-*d₆*) δ = 10.72 (s, 1H), 9.56 (s, 1H), 8.80 - 8.64 (m, 1H), 8.52 (dd, *J* = 1.3, 8.3 Hz, 1H), 8.27 (ddd, *J* = 1.3, 3.3, 8.2 Hz, 1H), 8.18 (d, *J* = 8.0 Hz, 1H), 8.05 (dd, *J* = 1.8, 8.0 Hz, 1H), 7.51 (td, *J* = 7.8, 15.3 Hz, 2H), 7.18 (d, *J =* 7.5 Hz, 2H), 6.72 (s, 1H), 4.99 - 4.79 (m, 1H), 4.50 (s, 1H), 4.26 - 4.04 (m, 2H), 3.86 (s, 3H), 3.47 (q, *J* = 5.4 Hz, 2H), 3.01 - 2.86 (m, 2H), 2.75 - 2.62 (m, 3H), 2.57 (t, *J* = 5.8 Hz, 2H), 2.29 - 2.12 (m, 1H), 2.01 - 1.80 (m, 5H), 1.17 (t, *J* = 5.8 Hz, 6H).

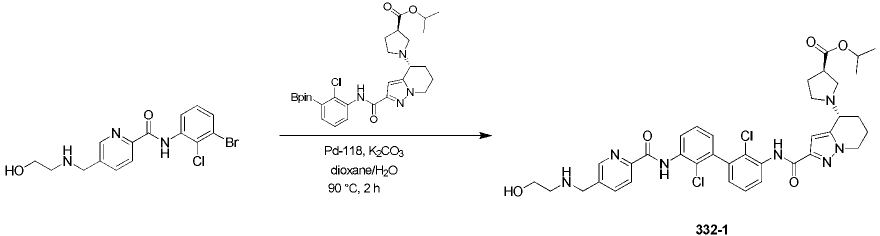

N-(3-bromo-2-chloro-phenyl)-5-[(2-hydroxyethylamino)methyl]pyridine-2-carboxamide (50 mg, 129.99 µmol, 1 eq), isopropyl (3R)-1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]pyrrolidine-3-carboxylate (86.87 mg, 155.98 µmol, 1.2 eq), CsF (59.23 mg, 389.96 µmol, 3 eq), and ditert-butyl(cyclopenta-1,4-dien-1-yl)phosphane;dichloropalladium;iron (8.47 mg, 13.00 µmol, 0.1 eq) were added to a mixture of dioxane (3 mL) / H₂O (1 mL), and the resulting mixture was stirred under a nitrogen atmosphere at 90 °C for 2 hours. After completion of the reaction, the mixture was cooled to room temperature, filtered, and the filtrate was concentrated under reduced pressure. The residue was purified by prep-HPLC (column: Waters Xbridge 150 * 25 mm * 5 µm; mobile phase: [water (NH₄HCO₃)-ACN]; gradient: 50%-80% B over 10 min) to afford Compound 332-1 (12 mg, 11.8% yield) as a white soli

LCMS m/z 734.4 [M+1]⁺

¹H NMR (400 MHz, DMSO-*d₆*) δ = 10.72 (s, 1H), 9.55 (s, 1H), 8.69 (s, 1H), 8.57 - 8.45 (m, 1H), 8.27 (dd, *J* = 1.1, 8.1 Hz, 1H), 8.18 (d, *J* = 7.9 Hz, 1H), 8.05 (br d, *J* = 7.9 Hz, 1H), 7.51 (td, *J* = 7.9, 15.7 Hz, 2H), 7.18 (br d, *J* = 7.6 Hz, 2H), 6.70 (s, 1H), 5.02 - 4.79 (m, 1H), 4.54 - 4.47 (m, 1H), 4.21 - 4.12 (m, 2H), 3.86 (s, 3H), 3.51 - 3.42 (m, 3H), 2.98 - 2.91 (m, 1H), 2.86 (br dd, *J* = 5.7, 8.8 Hz, 1H), 2.76 - 2.70 (m, 1H), 2.69 - 2.64 (m, 2H), 2.57 (br t, *J* = 5.8 Hz, 2H), 2.33 - 2.14 (m, 2H), 1.98 (br s, 1H), 1.89 (br d, *J* = 2.9 Hz, 3H), 1.18 (br dd, *J* = 2.9, 6.1 Hz, 6H).

### [Preparation Example 114]

### Preparation of Compound 333, (S)-N-(2,2'-dichloro-3'-((2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide, and Compound 334, (R)-N-(2,2'-dichloro-3'-((2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide

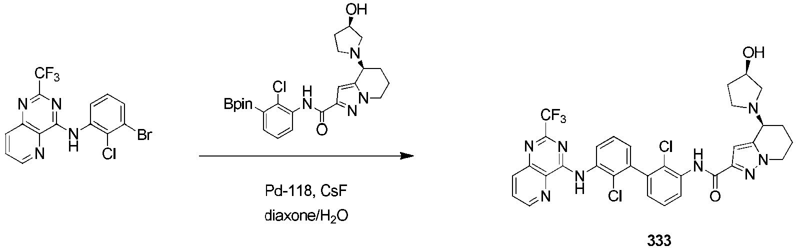

N-(3-bromo-2-chloro-phenyl)-2-(trifluoromethyl)pyrido[3,2-d]pyrimidin-4-amine (100 mg, 247.78 µmol, 1 eq), (4S)-N-[2-chloro-3-(4,4,5,5-tetramethyl-1,3,2-dioxaborolan-2-yl)phenyl]-4-[(3R)-3-hydroxypyrrolidin-1-yl]-4,5,6,7-tetrahydropyrazolo[1,5-a]pyridine-2-carboxamide (120.62 mg, 247.78 µmol, 1 eq), ditert-butyl(cyclopenta-1,4-dien-1-yl)phosphane;dichloropalladium;iron (16.15 mg, 24.78 µmol, 0.1 eq), and CsF (112.91 mg, 743.34 µmol, 3 eq) were added to a mixture of dioxane (8 mL) / H₂O (2 mL), and the resulting mixture was stirred under a nitrogen atmosphere at 90 °C for 12 hours. After completion of the reaction, the mixture was cooled to room temperature, filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 1/1) and further purified by reversed-phase HPLC (column: Agela DuraShell C18 150 * 25 mm * 5 µm; mobile phase: [water (HCl)-ACN]; gradient: 38%-68% B over 20 min; column: Waters Xbridge 150 * 25 mm * 5 µm; mobile phase: [water (NH₄HCO₃)-ACN]; gradient: 52%-82% B over 15 min) to afford Compound 333 (23 mg, 13.3% yield) as a yellow solid.

LCMS m/z 683.3 [M+1]⁺.

¹H NMR (400 MHz, DMSO-*d₆*) δ ppm 1.45 - 1.74 (m, 3 H) 1.76 - 2.04 (m, 4 H) 2.05 - 2.38 (m, 4 H) 2.63 - 2.84 (m, 2 H) 3.88 - 4.32 (m, 3 H) 6.67 (br s, 1 H) 7.11 - 7.36 (m, 2 H) 7.44 - 7.68 (m, 2 H) 8.09 (br s, 1 H) 8.22 - 8.53 (m, 3 H) 9.12 (br s, 1 H) 9.55 (br s, 1 H) 10.39 - 10.66 (m, 1 H) 11.72 - 12.44 (m, 1 H).

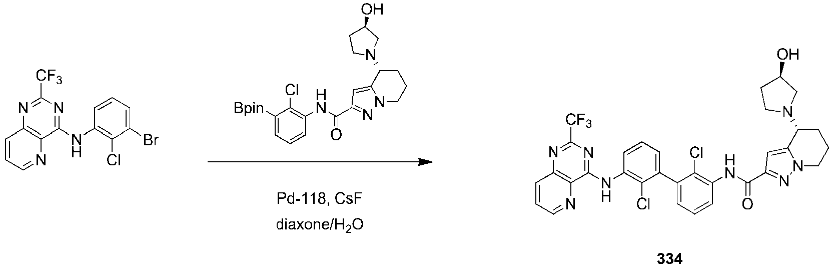

A mixture of N-(3-bromo-2-chloro-phenyl)-2-(trifluoromethyl)pyrido[3,2-d]pyrimidin-4-amine 1 (100.00 mg, 247.78 µmol, 1 eq), (4R)-N-[2-chloro-3-(4,4,5,5-tetramethyl-1,3,2-dioxaborolan-2-yl)phenyl]-4-[(3R)-3-hydroxypyrrolidin-1-yl]-4,5,6,7-tetrahydropyrazolo[1,5-a]pyridine-2-carboxamide 2-R (120.62 mg, 247.78 µmol, 1 eq), ditert-butyl(cyclopenta-1,4-dien-1-yl)phosphane;dichloropalladium;iron (16.15 mg, 24.78 µmol, 0.1 eq), and CsF (112.91 mg, 743.34 µmol, 3 eq) were added to a mixture of dioxane (8 mL) / H₂O (2 mL), and the resulting mixture was stirred under a nitrogen atmosphere at 90 °C for 12 hours. After completion of the reaction, the mixture was cooled to room temperature, filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 1/1) and further purified by reversed-phase HPLC (column: Waters Xbridge 150 * 25 mm * 5 µm; mobile phase: [water (NH₄HCO₃)-ACN]; gradient: 60%-90% B over 9 min; column: Agela DuraShell C18 150 * 25 mm * 5 µm; mobile phase: [water (HCl)-ACN]; gradient: 36%-66% B over 20 min) to afford Compound 334 (37 mg, 21.6% yield) as a yellow solid.

LCMS m/z 683.5 [M+1]⁺.

¹H NMR (400 MHz, DMSO-*d₆*) δ ppm 1.98 (br d, *J*=19.26 Hz, 3 H) 2.09 - 2.19 (m, 1 H) 2.20 - 2.42 (m, 3 H) 3.52 (br s, 1 H) 3.62 (br s, 1 H) 4.26 (br s, 2 H) 4.37 - 4.52 (m, 1 H) 4.83 - 4.97 (m, 1 H) 5.51 (br s, 1 H) 7.20 - 7.36 (m, 3 H) 7.53 (t, *J*=7.88 Hz, 1 H) 7.62 (t, *J*=7.94 Hz, 1 H) 8.10 (dd, *J*=8.51, 4.38 Hz, 1 H) 8.16 - 8.24 (m, 1 H) 8.37 (br d, *J*=8.25 Hz, 1 H) 8.47 (dd, *J*=8.50, 1.38 Hz, 1 H) 9.12 (dd, *J*=4.25, 1.38 Hz, 1 H) 9.68 (br d, *J*=9.01 Hz, 1 H) 10.56 (s, 1 H) 10.79 - 11.37 (m, 1 H).

### [Preparation Example 115]

### Preparation of Compound 335, (S)-N-(2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)-4-((R)-3-hydroxypyrrolidin-1-yl)-4,5,6,7-tetrahydropyrazolo[1,5-a]pyridine-2-carboxamide

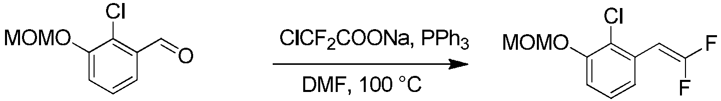

2-Chloro-3-(methoxymethoxy)benzaldehyde (4.5 g, 22.43 mmol, 1 eq) and PPh3 (7.06 g, 26.92 mmol, 1.2 eq) were dissolved in DMF (50 mL), and the mixture was stirred at 100 °C for 30 minutes. A solution of sodium 2-chloro-2,2-difluoroacetate (5.13 g, 33.65 mmol, 1.5 eq) in DMF (15 mL) was then added dropwise. The resulting mixture was stirred at 100 °C for 2 hours, then cooled to room temperature and diluted with H₂O (200 mL). The mixture was extracted with ethyl acetate (100 mL * 3), washed with brine (200 mL), dried over Na₂SO₄, filtered, and concentrated under reduced pressure. The residue was purified by flash silica gel chromatography (0-10%, Petroleum ether/Ethyl acetate) to afford 2-chloro-1-(2,2-difluorovinyl)-3-(methoxymethoxy)benzene (6.12 g, 98.8% yield) as a colorless liquid.

¹H NMR (400 MHz, CHLOROFORM-d) δ = 7.18 - 7.10 (m, 2H), 7.02 (dd, *J* = 2.6, 7.0 Hz, 1H), 5.75 - 5.59 (m, 1H), 5.18 (s, 2H), 3.46 (s, 3H).

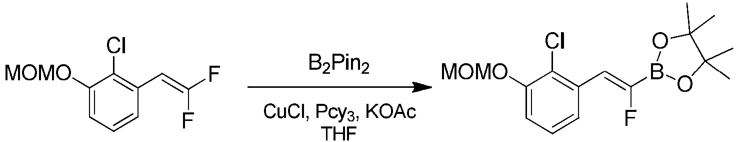

2-Chloro-1-(2,2-difluorovinyl)-3-(methoxymethoxy)benzene (3.5 g, 14.92 mmol, 1 eq), 4,4,5,5-tetramethyl-2-(4,4,5,5-tetramethyl-1,3,2-dioxaborolan-2-yl)-1,3,2-dioxaborolane (7.58 g, 29.83 mmol, 2 eq), KOAc (4.39 g, 44.75 mmol, 3 eq), CuCl (147.68 mg, 1.49 mmol, 35.67 µL, 0.1 eq), and tricyclohexylphosphonium tetrafluoroborate (1.65 g, 4.48 mmol, 0.3 eq) were added to THF (100 mL), and the resulting mixture was stirred under a nitrogen atmosphere at 40 °C for 12 hours. After completion of the reaction, the mixture was cooled to room temperature and diluted with H₂O (40 mL). The mixture was extracted with ethyl acetate (50 mL × 3), washed with brine (60 mL), dried over Na₂SO₄, filtered, and concentrated under reduced pressure. The residue was purified by flash silica gel chromatography (0-30% Petroleum ether/Ethyl acetate) to afford 2-[(Z)-2-[2-chloro-3-(methoxymethoxy)phenyl]-1-fluoro-vinyl]-4,4,5,5-tetramethyl-1,3,2-dioxaborolane (3.8 g, 60.9% yield) as a white solid.

¹H NMR (400 MHz, CHLOROFORM-d) δ = 7.64 (dd, *J* = 1.3, 7.8 Hz, 1H), 7.24 - 7.17 (m, 1H), 7.16 - 7.10 (m, 1H), 6.94 - 6.73 (m, 1H), 5.25 (s, 2H), 3.52 (s, 3H), 1.35 (s, 12H).

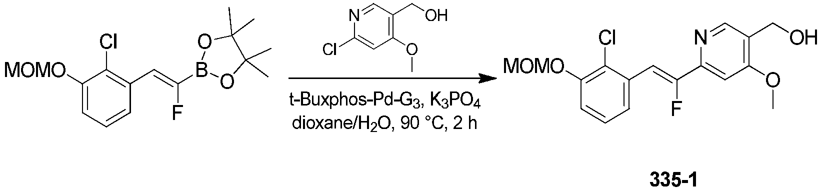

2-[(Z)-2-[2-chloro-3-(methoxymethoxy)phenyl]-1-fluoro-vinyl]-4,4,5,5-tetramethyl-1,3,2-dioxaborolane (2.17 g, 6.34 mmol, 1.1 eq), (6-chloro-4-methoxy-3-pyridyl)methanol (1 g, 5.76 mmol, 1 eq), K₃PO₄ (3.67 g, 17.28 mmol, 3 eq), and [2-(2-aminophenyl)phenyl]-methylsulfonyloxy-palladium; ditert-butyl-[2-(2,4,6-triisopropylphenyl)phenyl]phosphane (457.60 mg, 576.05 µmol, 0.1 eq) were added to a mixture of H₂O (3 mL) / dioxane (15 mL), and the resulting mixture was stirred under a nitrogen atmosphere at 90 °C for 5 hours. After completion of the reaction, the mixture was cooled to room temperature and diluted with H₂O (40 mL). The mixture was extracted with ethyl acetate (50 mL × 3), washed with brine (60 mL), dried over Na₂SO₄, filtered, and concentrated under reduced pressure. The residue was purified by flash silica gel chromatography (Petroleum ether/Ethyl acetate = 100/1 to 1/1) to afford Compound 335-1 (1.32 g, 51.8% yield) as a white solid.

¹H NMR (400 MHz, DMSO-*d₆*) δ = 8.48 (s, 1H), 7.65 - 7.53 (m, 2H), 7.43 - 7.35 (m, 1H), 7.28 - 7.21 (m, 2H), 5.32 (s, 2H), 4.54 (d, *J* = 5.5 Hz, 2H), 3.94 (s, 3H), 3.43 (s, 3H).

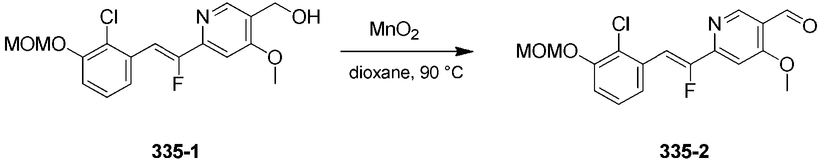

[6-[(Z)-2-[2-chloro-3-(methoxymethoxy)phenyl]-1-fluoro-vinyl]-4-methoxy-3-pyridyl]methanol (1 g, 2.83 mmol, 1 eq) was dissolved in dioxane (20 mL), and MnO₂ (1.23 g, 14.13 mmol, 5 eq) was added. The resulting mixture was stirred at 90 °C for 2 hours. After completion of the reaction, the mixture was cooled to room temperature, filtered, and the filtrate was concentrated under reduced pressure. The residue was purified by flash silica gel chromatography (0-10%, Petroleum ether/Ethyl acetate) to afford Compound 335-2 (820 mg, 74.2% yield) as a white solid.

¹H NMR (400 MHz, DMSO-*d₆*) δ = 10.32 (s, 1H), 8.80 (s, 1H), 7.65 - 7.59 (m, 2H), 7.49 (s, 1H), 7.40 (br t, *J* = 7.9 Hz, 1H), 7.28 (br d, *J* = 8.5 Hz, 1H), 5.33 (s, 2H), 4.09 (s, 3H), 3.43 (br s, 3H).

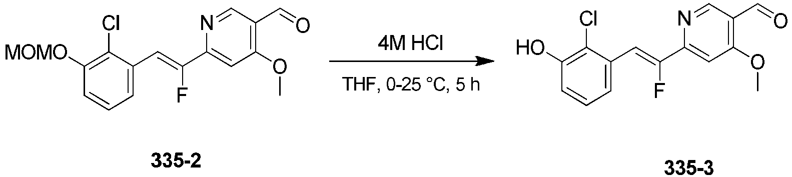

6-[(Z)-2-[2-chloro-3-(methoxymethoxy)phenyl]-1-fluoro-vinyl]-4-methoxy-pyridine-3-carbaldehyde (600 mg, 1.71 mmol, 1 eq) was dissolved in dioxane (6 mL), and HCl/dioxane (2 M, 8.53 mL, 10 eq) was added at 0 °C. The resulting mixture was stirred at room temperature for 5 hours. The mixture was then adjusted to pH 7 with aq. saturated NaHCO₃ solution and extracted with ethyl acetate (50 mL × 3). The combined organic layers were washed with brine (60 mL), dried over Na₂SO₄, filtered, and concentrated under reduced pressure. The residue was purified by flash silica gel chromatography (0-50%, Petroleum ether/Ethyl acetate) to afford Compound 335-3 (520 mg, 79.2% yield) as a white solid.

¹H NMR (400 MHz, DMSO-*d₆*) δ = 10.36 (s, 1H), 10.32 (s, 1H), 8.79 (s, 1H), 7.70 - 7.53 (m, 1H), 7.48 - 7.38 (m, 2H), 7.25 (t, *J* = 7.9 Hz, 1H), 7.00 (dd, *J* = 1.0, 8.1 Hz, 1H), 4.09 (s, 3H).

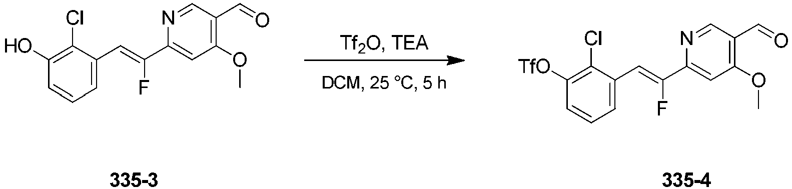

6-[(Z)-2-(2-chloro-3-hydroxy-phenyl)-1-fluoro-vinyl]-4-methoxy-pyridine-3-carbaldehyde 335-3 (350 mg, 1.14 mmol, 1 eq) and trifluoromethylsulfonyl trifluoromethanesulfonate (353.01 mg, 1.25 mmol, 206.44 µL, 1.1 eq) were dissolved in DCM (8 mL), and TEA (345.29 mg, 3.41 mmol, 474.96 µL, 3 eq) was added at 0 °C. The mixture was stirred at room temperature for 5 hours. The reaction mixture was then adjusted to pH 7 with aq. saturated NaHCO₃ solution, extracted with dichloromethane (50 mL * 3), washed with brine (60 mL), dried over Na₂SO₄, filtered, and concentrated under reduced pressure to give 335-4 (560 mg, 89.5% yield) as a white solid, which was used directly in the next reaction without further purification.

LCMS m/z 439.9 [M+1]⁺

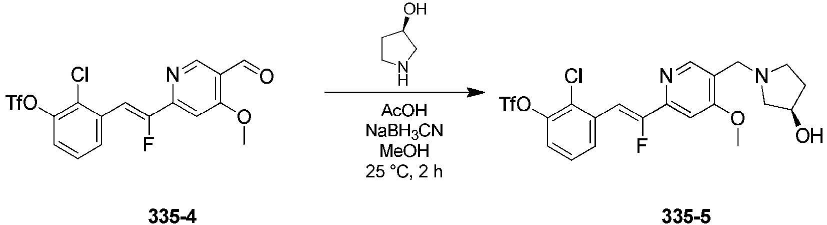

[2-chloro-3-[(Z)-2-fluoro-2-(5-formyl-4-methoxy-2-pyridyl)vinyl]phenyl] trifluoromethanesulfonate 335-4 (400 mg, 909.58 µmol, 1 eq), (3R)-pyrrolidin-3-ol (87.17 mg, 1.00 mmol, 83.17 µL, 1.1 eq), DIPEA (352.67 mg, 2.73 mmol, 475.29 µL, 3 eq), and NaBH₃CN (171.48 mg, 2.73 mmol, 3 eq) were dissolved in MeOH (10 mL), and the mixture was stirred under a nitrogen atmosphere at room temperature for 2 hours. The reaction mixture was diluted with H₂O (50 mL) and extracted with ethyl acetate (50 mL × 3). The combined organic layers were washed with brine (60 mL), dried over Na₂SO₄, filtered, and concentrated under reduced pressure. The residue was purified by prep-HPLC (column: Waters Xbridge Prep OBD C18 150 × 40 mm × 10 µm; mobile phase: [water (NH₄HCO₃)-ACN]; gradient: 45%-75% B over 58 min) to afford Compound 335-5 (402 mg, 77.8% yield) as a white solid.

¹H NMR (400 MHz, CHLOROFORM-d) δ = 8.57 (s, 1H), 8.08 (d, *J* = 7.9 Hz, 1H), 7.65 (s, 1H), 7.55 (s, 1H), 7.51 - 7.45 (m, 1H), 7.40 - 7.35 (m, 1H), 5.40 (s, 2H), 4.50 - 4.45 (m, 1H), 4.06 (s, 3H), 3.88 (s, 2H), 3.57 (s, 1H), 2.92 (br d, *J* = 10.4 Hz, 1H), 2.80 (dd, *J* = 5.3, 10.3 Hz, 1H), 2.64 - 2.55 (m, 1H), 2.37 - 2.28 (m, 1H).

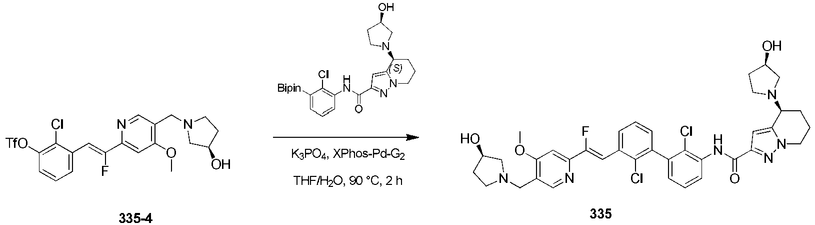

[2-chloro-3-[(Z)-2-fluoro-2-[5-[[(3R)-3-hydroxypyrrolidin-1-yl]methyl]-4-methoxy-2-pyridyl]vinyl]phenyl] trifluoromethanesulfonate 335-4 (80 mg, 156.59 µmol, 1 eq), (4S)-N-[2-chloro-3-(4,4,5,5-tetramethyl-1,3,2-dioxaborolan-2-yl)phenyl]-4-[(3R)-3-hydroxypyrrolidin-1-yl]-4,5,6,7-tetrahydropyrazolo[1,5-a]pyridine-2-carboxamide (99.10 mg, 203.57 µmol, 1.3 eq), K₃PO₄ (99.72 mg, 469.77 µmol, 3 eq), and [2-(2-aminophenyl)phenyl]-chloro-palladium;dicyclohexyl-[3-(2,4,6-triisopropylphenyl)phenyl]phosphane (12.32 mg, 15.66 µmol, 0.1 eq) were added to a mixture of dioxane (5 mL) / H₂O (0.5 mL), and the mixture was stirred under a nitrogen atmosphere at 90 °C for 2 hours. After cooling to room temperature, the mixture was filtered, and the filtrate was concentrated under reduced pressure. The residue was purified by prep-HPLC (column: Waters Xbridge Prep OBD C18 150 × 40 mm × 10 µm; mobile phase: [water (NH₄HCO₃)-ACN]; gradient: 40%-70% B over 15 min) to afford Compound 335 (12 mg, 10.0% yield) as a white solid.

LCMS m/z 721.2 [M+1]⁺.

¹H NMR (400 MHz, DMSO-d6) δ = 12.05 - 11.90 (m, 1H), 11.49 - 11.32 (m, 1H), 11.19 - 11.05 (m, 1H), 10.80 - 10.63 (m, 1H), 9.65 (br d, *J* = 3.4 Hz, 1H), 8.78 - 8.66 (m, 1H), 8.23 - 8.14 (m, 1H), 8.05 - 7.96 (m, 1H), 7.66 - 7.56 (m, 1H), 7.57 - 7.47 (m, 2H), 7.43 (s, 1H), 7.39 - 7.29 (m, 2H), 7.20 (br d, *J* = 7.9 Hz, 1H), 4.87 (br d, *J* = 4.8 Hz, 1H), 4.46 - 4.36 (m, 4H), 4.28 - 4.20 (m, 2H), 4.03 (br d, *J* = 2.0 Hz, 3H), 3.36 - 3.24 (m, 5H), 2.72 (d, *J* = 4.9 Hz, 1H), 2.35 - 2.17 (m, 3H), 2.07 (s, 1H), 2.05 - 1.97 (m, 2H), 1.93 (br d, *J* = 9.0 Hz, 2H).

### [Preparation Example 116]

### 116-1. Preparation of Compound 336, 1-((S)-2-((2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)piperidine-4-carboxylic acid

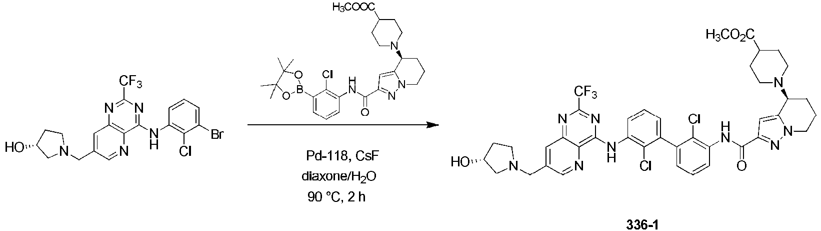

(3R)-1-[[4-(3-bromo-2-chloro-anilino)-2-(trifluoromethyl)pyrido[3,2-d]pyrimidin-7-yl]methyl]pyrrolidin-3-ol (100 mg, 198.92 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (118.78 mg, 218.81 µmol, 1.1 eq), CsF (90.65 mg, 596.76 µmol, 3 eq), and ditert-butyl(cyclopenta-1,4-dien-1-yl)phosphane;dichloropalladium;iron (12.96 mg, 19.89 µmol, 0.1 eq) were added to a mixture of dioxane (6 mL) / H₂O (2 mL), and the resulting mixture was stirred under a nitrogen atmosphere at 90 °C for 2 hours. The mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to afford Compound 336-1 (112 mg, 57.9% yield) as a white solid.

LCMS m/z 838.3 [M+1]⁺.

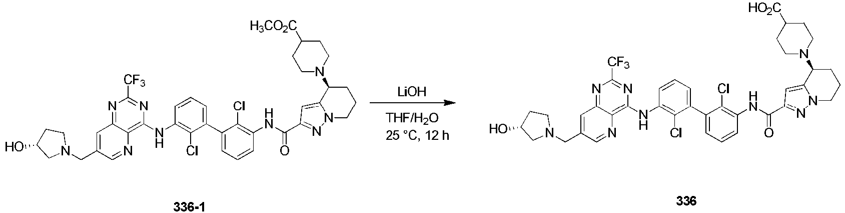

Methyl 1-[(4S)-2-[[2-chloro-3-[2-chloro-3-[[7-[[(3R)-3-hydroxypyrrolidin-1-yl]methyl]-2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]piperidine-4-carboxylate 336-1 (100 mg, 119.23 µmol, 1 eq) was dissolved in a mixture of THF (9 mL) / H₂O (3 mL), and LiOH·H₂O (15.01 mg, 357.70 µmol, 3 eq) was added at 0 °C. The mixture was stirred at room temperature for 12 hours. The reaction mixture was then adjusted to pH 7 with 1 N HCl, and the precipitated solid was collected by filtration. The solid was dried under vacuum and purified by prep-HPLC (column: Waters Xbridge 150 * 25 mm * 5 µm; mobile phase: [water (NH₄HCO₃)-ACN]; gradient: 18%-48% B over 10 min) to afford Compound 336 (52 mg, 50.7% yield) as a white solid.

LCMS m/z 826.4 [M+1]⁺.

¹H NMR (400 MHz, DMSO-*d₆* ) δ = 9.55 (s, 1H), 9.05 (d, *J* = 1.9 Hz, 1H), 8.35 (dd, *J* = 1.4, 8.2 Hz, 1H), 8.32 - 8.25 (m, 2H), 7.60 (t, *J* = 7.9 Hz, 1H), 7.50 (t, *J* = 7.9 Hz, 1H), 7.28 (d, *J* = 7.6 Hz, 1H), 7.18 (dd, *J* = 1.3, 7.6 Hz, 1H), 6.67 (s, 1H), 4.74 (br s, 1H), 4.21 (br d, *J* = 7.8 Hz, 2H), 4.06 (dt, *J* = 4.3, 11.8 Hz, 1H), 4.00 - 3.95 (m, 1H), 3.94 - 3.82 (m, 2H), 2.86 - 2.72 (m, 2H), 2.71 - 2.65 (m, 2H), 2.42 (br dd, *J* = 3.6, 9.8 Hz, 2H), 2.36 - 2.10 (m, 4H), 2.07 (s, 2H), 1.91 - 1.76 (m, 3H), 1.75 - 1.61 (m, 2H), 1.61 - 1.40 (m, 3H).

### 116-2. Preparation of Compound 337, 1-((R)-2-((2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5575yridinedin-4-yl)piperidine-4-carboxylic acid

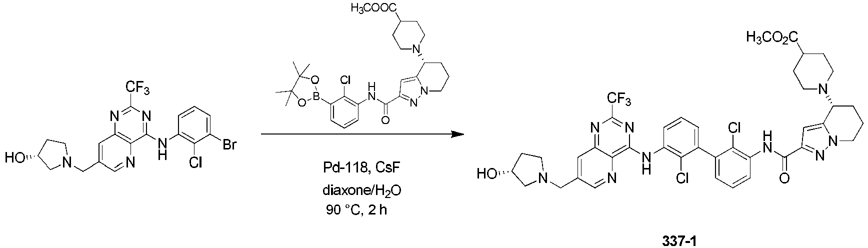

(3R)-1-[[4-(3-bromo-2-chloro-anilino)-2-(trifluoromethyl)pyrido[3,2-d]pyrimidin-7-yl]methyl]pyrrolidin-3-ol (100 mg, 198.92 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5 575 yridinedin-4-yl]piperidine-4-carboxylate (118.78 mg, 218.81 µmol, 1.1 eq), ditert-butyl(cyclopentyl)phosphane;dichloropalladium;iron (12.96 mg, 19.89 µmol, 0.1 eq), and CsF (90.65 mg, 596.76 µmol, 3 eq) were added to a mixture of dioxane (65 mL) / H₂O (1 mL), and the resulting mixture was stirred under a nitrogen atmosphere at 90 °C for 2 hours. The mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to afford Compound 337-1 (116 mg, 59.9% yield) as a white solid.

LCMS m/z 839.9 [M+1]⁺.

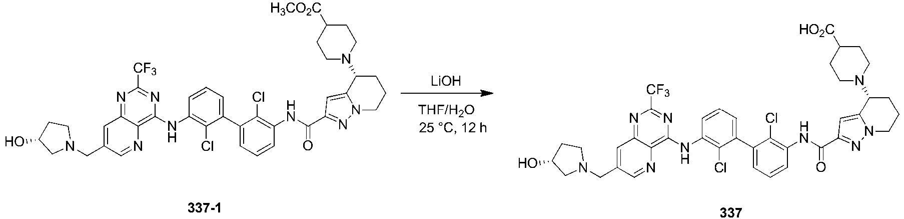

Methyl 1-[(4R)-2-[[2-chloro-3-[2-chloro-3-[[7-[[(3R)-3-hydroxypyrrolidin-1-yl]methyl]-2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]piperidine-4-carboxylate 3 (100.00 mg, 119.23 µmol, 1 eq) was dissolved in a mixture of THF (9 mL) / H₂O (3 mL), and LiOH·H₂O (15.01 mg, 357.70 µmol, 3 eq) was added at 0 °C. The mixture was stirred at room temperature for 12 hours. The reaction mixture was adjusted to pH 7 with 1 N HCl, and the precipitated solid was collected by filtration. The solid was dried under vacuum and purified by prep-HPLC (column: Waters Xbridge 150 * 25 mm * 5 µm; mobile phase: [water (NH₄HCO₃)-ACN]; gradient: 18%-48% B over 10 min) to afford Compound 337 (48 mg, 46.8% yield) as a white solid.

LCMS m/z 825.5 [M+1]⁺.

¹H NMR (400 MHz, DMSO-*d₆*) δ = 9.55 (s, 1H), 9.05 (d, *J* = 1.9 Hz, 1H), 8.34 (br d, *J* = 7.9 Hz, 1H), 8.31 - 8.25 (m, 2H), 7.60 (t, *J* = 7.9 Hz, 1H), 7.50 (t, *J* = 7.9 Hz, 1H), 7.28 (br d, *J* = 7.6 Hz, 1H), 7.18 (dd, *J* = 1.4, 7.6 Hz, 1H), 6.67 (s, 1H), 4.75 (br s, 1H), 4.21 (br d, *J* = 7.5 Hz, 2H), 4.11 - 3.96 (m, 3H), 3.89 (br d, *J* = 13.6 Hz, 2H), 2.83 - 2.76 (m, 2H), 2.71 - 2.64 (m, 3H), 2.43 - 2.40 (m, 2H), 2.30 - 2.22 (m, 2H), 2.20 - 2.12 (m, 2H), 2.08 - 1.98 (m, 2H), 1.89 - 1.76 (m, 3H), 1.68 (br d, *J* = 12.4 Hz, 1H), 1.62 - 1.50 (m, 3H).

### [Preparation Example 117]

### Preparation of Compound 338, (S)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide, and Compound 339, (R)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide

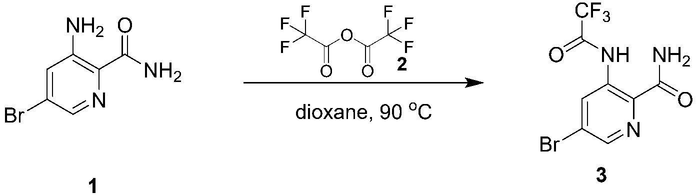

A mixture of 3-amino-5-bromo-pyridine-2-carboxamide (10.00 g, 46.29 mmol, 1 eq) and (2,2,2-trifluoroacetyl) 2,2,2-trifluoroacetate (14.58 g, 69.43 mmol, 9.65 mL, 1.5 eq) in dioxane (100 mL) was stirred at 90 °C for 12 hours. The reaction mixture was cooled to room temperature and concentrated under reduced pressure to afford 5-bromo-3-[(2,2,2-trifluoroacetyl)amino]pyridine-2-carboxamide (14 g, 96.9% yield) as a dark solid, which was used directly in the next reaction without further purification.

LCMS m/z 314.2 [M+3]⁺.

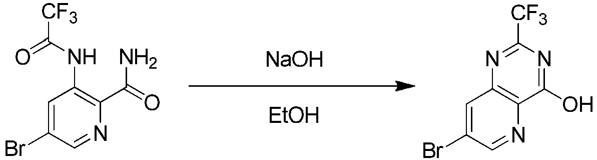

5-Bromo-3-[(2,2,2-trifluoroacetyl)amino]pyridine-2-carboxamide (14 g, 44.87 mmol, 1 eq) was dissolved in EtOH (200 mL), and NaOH (3.59 g, 89.73 mmol, 2 eq) was added. The mixture was stirred at 95 °C for 12 hours. The reaction mixture was cooled to 0 °C, and the precipitated solid was collected by filtration to afford 7-bromo-2-(trifluoromethyl)pyrido[3,2-d]pyrimidin-4-ol (11 g, 66.7% yield) as a dark solid, which was used directly in the next reaction without further purification.

LCMS m/z 293.9 [M+1]⁺.

¹H NMR (400 MHz, DMSO-*d*₆) δ ppm 8.19 - 8.41 (m, 1 H) 9.00 (br s, 1 H) 13.98 (br s, 1 H).

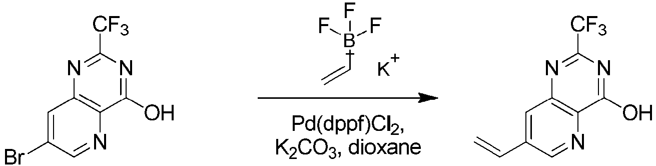

7-Bromo-2-(trifluoromethyl)pyrido[3,2-d]pyrimidin-4-ol (8 g, 27.21 mmol, 1 eq), potassium trifluorovinylboranuide (5.47 g, 40.81 mmol, 1.5 eq), Pd(dppf)Cl₂ (1.99 g, 2.72 mmol, 0.1 eq), and K₂CO₃ (7.52 g, 54.42 mmol, 2 eq) were combined in dioxane (200 mL) and stirred under a nitrogen atmosphere at 90 °C for 12 hours. The mixture was cooled to room temperature, filtered, and the filtrate was concentrated under reduced pressure. The residue was purified by column chromatography (SiO₂, Dichloromethan/Methanol = 1/0 to 1/1) to afford 2-(trifluoromethyl)-7-vinyl-pyrido[3,2-d]pyrimidin-4-ol (7.8 g, 95.1% yield) as a black solid. LCMS m/z 242.0 [M+1]⁺.

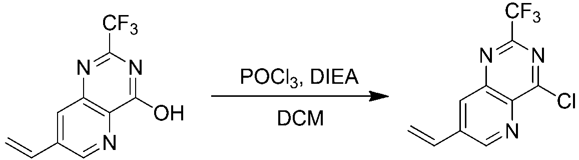

2-(Trifluoromethyl)-7-vinyl-pyrido[3,2-d]pyrimidin-4-ol (9 g, 37.32 mmol, 1 eq) was dissolved in DCM (100 mL), and DIPEA (14.47 g, 111.95 mmol, 19.50 mL, 3 eq) and POCl₃ (17.17 g, 111.95 mmol, 10.44 mL, 3 eq) were added. The mixture was stirred at room temperature for 12 hours. The reaction mixture was concentrated under reduced pressure to afford 4-chloro-2-(trifluoromethyl)-7-vinyl-pyrido[3,2-d]pyrimidine (10.1 g, 72.9% yield) as a dark solid, which was used directly in the next reaction without further purification.

LCMS (m/z 260.0 [M+1]⁺.

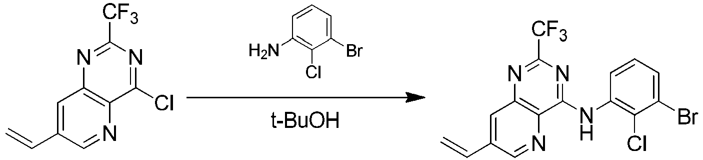

4-Chloro-2-(trifluoromethyl)-7-vinyl-pyrido[3,2-d]pyrimidine (5 g, 19.26 mmol, 1 eq) and 3-bromo-2-chloro-aniline (5.17 g, 25.04 mmol, 1.3 eq) were dissolved in t-BuOH (100 mL) and stirred at 80 °C for 12 hours. The reaction mixture was cooled to room temperature, concentrated under reduced pressure, and the residue was purified by column chromatography (SiO₂, Petroleum ether/Ethyl acetate = 1/0 to 3/1) to afford N-(3-bromo-2-chloro-phenyl)-2-(trifluoromethyl)-7-vinyl-pyrido[3,2-d]pyrimidin-4-amine (10 g, 84.6% yield) as a black solid.

LCMS m/z 430.9 [M+3]⁺.

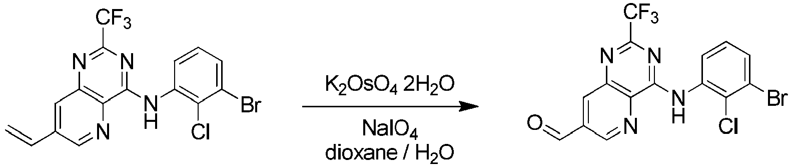

A mixture of N-(3-bromo-2-chloro-phenyl)-2-(trifluoromethyl)-7-vinyl-pyrido[3,2-d]pyrimidin-4-amine (4 g, 9.31 mmol, 1 eq) in dioxane (100 mL) / H₂O (20 mL) was treated with dipotassium dioxido(dioxo)osmium dihydrate (686.10 mg, 1.86 mmol, 0.2 eq) and sodium periodate (7.97 g, 37.24 mmol, 2.06 mL, 4 eq), and the reaction mixture was stirred at room temperature for 12 hours. The mixture was diluted with H₂O (100 mL) and extracted with ethyl acetate (100 mL × 3). The combined organic layers were washed with brine (50 mL), dried over Na₂SO₄, filtered, and concentrated under reduced pressure. The residue was purified by column chromatography on silica gel (Petroleum ether/Ethyl acetate = 1/0 to 2/1) to afford 4-(3-bromo-2-chloro-anilino)-2-(trifluoromethyl)pyrido[3,2-d]pyrimidine-7-carbaldehyde (4.5 g, 67.1% yield) as a white solid.

LCMS m/z 433.0 [M+3]⁺.

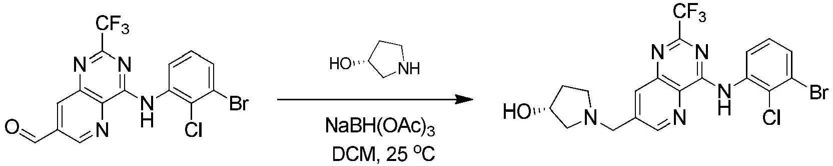

A solution of 4-(3-bromo-2-chloro-anilino)-2-(trifluoromethyl)pyrido[3,2-d]pyrimidine-7-carbaldehyde (4.00 g, 9.27 mmol, 1 eq) and (3R)-pyrrolidin-3-ol (2.42 g, 27.80 mmol, 2.31 mL, 3 eq) in DCM (100 mL) was treated with NaBH(OAc)₃ (5.89 g, 27.80 mmol, 3 eq) and stirred at room temperature for 12 hours. The reaction mixture was concentrated under reduced pressure, and the residue was purified by reversed-phase HPLC (Phenomenex Luna C18, 250 * 70 mm * 10 µm; mobile phase: [water (HCl)-ACN]; gradient: 10%-55% B over 65 min) to afford (3R)-1-[[4-(3-bromo-2-chloro-anilino)-2-(trifluoromethyl)pyrido[3,2-d]pyrimidin-7-yl]methyl]pyrrolidin-3-ol (3.4 g, 72.2% yield) as a yellow solid.

LCMS m/z 504.0 [M+3]⁺.

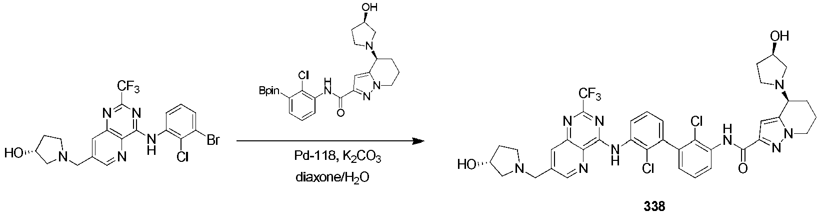

A mixture of (3R)-1-[[4-(3-bromo-2-chloro-anilino)-2-(trifluoromethyl)pyrido[3,2-d]pyrimidin-7-yl]methyl]pyrrolidin-3-ol (100 mg, 198.92 µmol, 1 eq), (4S)-N-[2-chloro-3-(4,4,5,5-tetramethyl-1,3,2-dioxaborolan-2-yl)phenyl]-4-[(3R)-3-hydroxypyrrolidin-1-yl]-4,5,6,7-tetrahydropyrazolo[1,5-a]pyridine-2-carboxamide (106.52 mg, 218.81 µmol, 1.1 eq), K₂CO₃ (82.48 mg, 596.76 µmol, 3 eq), and ditert-butyl(cyclopenta-1,4-dien-1-yl)phosphane;dichloropalladium;iron (12.96 mg, 19.89 µmol, 0.1 eq) in dioxane (8 mL)/H₂O (2 mL) was stirred under N₂ at 90 °C for 12 hours. The reaction mixture was cooled to room temperature, filtered, and concentrated under reduced pressure. The residue was purified by column chromatography (SiO₂, Petroleum ether/Ethyl acetate = 1/0 to 0/1, Ethyl acetate/Methanol = 1/0 to 0/1) and further by reversed-phase HPLC (Waters Xbridge 150 * 25 mm * 5 µm; mobile phase: [water (NH₄HCO₃)-ACN]; gradient: 40%-70% B over 10 min) to afford Compound 338 (51 mg, 32.1% yield) as a yellow solid.

LCMS m/z 782.3 [M+1]⁺.

¹H NMR (400 MHz, DMSO-*d*₆) δ ppm 1.52 - 1.63 (m, 2 H) 1.87 - 2.07 (m, 5 H) 2.13 - 2.38 (m, 2 H) 2.42 (dd, *J*=9.63, 3.50 Hz, 1 H) 2.59 - 2.80 (m, 6 H) 3.80 - 3.96 (m, 3 H) 4.14 - 4.25 (m, 4 H) 4.73 (dd, *J*=13.63, 4.63 Hz, 2 H) 6.73 (s, 1 H) 7.19 (dd, *J*=7.63, 1.50 Hz, 1 H) 7.26 - 7.33 (m, 1 H) 7.51 (t, *J*=7.94 Hz, 1 H) 7.60 (t, *J*=7.94 Hz, 1 H) 8.26 - 8.37 (m, 3 H) 9.05 (d, *J*=1.50 Hz, 1 H) 9.56 (s, 1 H) 10.43 - 10.56 (m, 1 H).

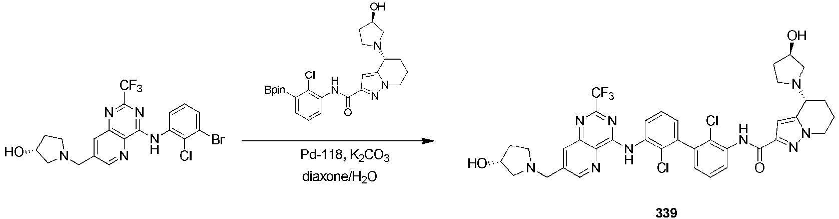

(3R)-1-[[4-(3-bromo-2-chloro-anilino)-2-(trifluoromethyl)pyrido[3,2-d]pyrimidin-7-yl]methyl]pyrrolidin-3-ol (100 mg, 198.92 µmol, 1 eq), (4R)-N-[2-chloro-3-(4,4,5,5-tetramethyl-1,3,2-dioxaborolan-2-yl)phenyl]-4-[(3R)-3-hydroxypyrrolidin-1-yl]-4,5,6,7-tetrahydropyrazolo[1,5-a]pyridine-2-carboxamide (116.20 mg, 238.70 µmol, 1.2 eq), ditert-butyl(cyclopenta-1,4-dien-1-yl)phosphane;dichloropalladium;iron (12.96 mg, 19.89 µmol, 0.1 eq), K₂CO₃ (82.48 mg, 596.76 µmol, 3 eq) in dioxane (8 mL)/H₂O (2 mL) was stirred under N₂ at 90 °C for 12 hours. The mixture was cooled to room temperature, filtered, and concentrated under reduced pressure. The residue was purified by column chromatography (SiO₂, Petroleum ether/Ethyl acetate = 1/0 to 0/1, Ethyl acetate/Methanol = 1/0 to 0/1) and further by reversed-phase HPLC (Waters Xbridge 150 × 25 mm × 5 um; mobile phase: [water (NH₄HCO₃)-ACN]; gradient: 40%-70% B over 10 min) to give compound 339 (55 mg, 34.6% yield) as a yellow solid.

LCMS m/z 782.3 [M+1]⁺.

¹H NMR (400 MHz, DMSO-*d₆*) δ ppm 1.51 - 1.63 (m, 2 H) 1.76 - 2.13 (m, 6 H) 2.18 - 2.27 (m, 1 H) 2.40 - 2.45 (m, 2 H) 2.61 - 2.96 (m, 5 H) 3.78 - 3.95 (m, 3 H) 4.13 - 4.26 (m, 4 H) 4.77 (br dd, *J*=18.20, 4.31 Hz, 2 H) 6.74 (s, 1 H) 7.19 (dd, *J*=7.57, 1.19 Hz, 1 H) 7.28 (d, *J*=7.50 Hz, 1 H) 7.50 (t, *J*=7.94 Hz, 1 H) 7.59 (t, *J*=7.94 Hz, 1 H) 8.24 - 8.37 (m, 3 H) 9.04 (d, *J*=1.63 Hz, 1 H) 9.56 (s, 1 H) 10.34 - 10.60 (m, 1 H).

### [Preparation Example 118]

### 118-1. Preparation of Compound 340, 2-(((S)-2-((2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid

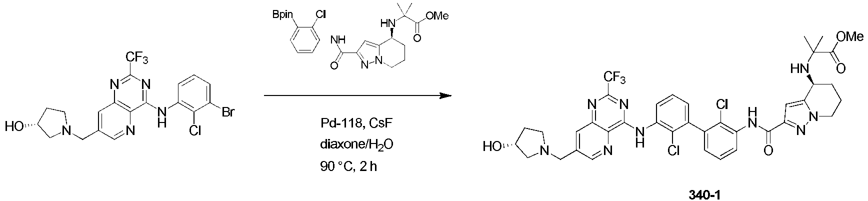

(3R)-1-[[4-(3-bromo-2-chloro-anilino)-2-(trifluoromethyl)pyrido[3,2-d]pyrimidin-7-yl]methyl]pyrrolidin-3-ol (90 mg, 179.03 µmol, 1 eq), methyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (101.78 mg, 196.93 µmol, 1.1 eq), ditert-butyl(cyclopentyl)phosphane;dichloropalladium;iron (11.67 mg, 17.90 µmol, 0.1 eq), K₂CO₃ (74.23 mg, 537.08 µmol, 3 eq) in dioxane (5 mL)/H₂O (1.5 mL) was stirred under N₂ at 90 °C for 2 hours. The mixture was cooled to room temperature, filtered, and concentrated under reduced pressure. The residue was purified by prep-HPLC (Agela DuraShell C18 150 * 25 mm * 5 µm; mobile phase: [water (HCl)-ACN]; gradient: 22%-52% B over 20 min) to give compound 340-1 (102 mg, 63.1% yield) as a white solid.

LCMS m/z 814.3 [M+1]⁺.

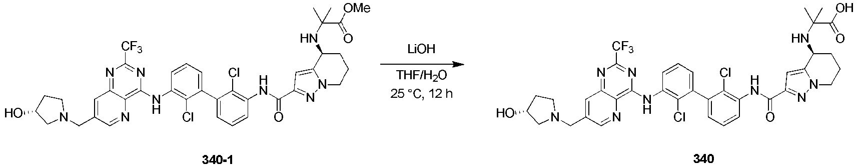

Methyl 2-[[(4S)-2-[[2-chloro-3-[2-chloro-3-[[7-[[(3R)-3-hydroxypyrrolidin-1-yl]methyl]-2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]amino]-2-methyl-propanoate 340-1 (80 mg, 98.44 µmol, 1 eq) in THF (6 mL)/H₂O (2 mL) was treated with LiOH·H₂O (12.39 mg, 295.32 µmol, 3 eq) at 0 °C, and the mixture was stirred at room temperature for 12 hours. The mixture was adjusted to pH 7 with 1N HCl aqueous solution, and the precipitated solid was filtered. The solid was dried under vacuum and purified by prep-HPLC (Waters Xbridge 150 * 25 mm * 5 µm; mobile phase: [water (NH₄HCO₃)-ACN]; gradient: 20%-50% B over 15 min) to give compound 340 (43 mg, 51.9% yield) as a white solid.

LCMS m/z 798.4 [M+1]⁺.

¹H NMR (400 MHz, DMSO-*d₆* ) δ = 9.54 (s, 1H), 9.05 (d, *J* = 1.8 Hz, 1H), 8.34 (dd, *J* = 1.4, 8.2 Hz, 1H), 8.31 - 8.27 (m, 2H), 7.60 (t, *J* = 7.9 Hz, 1H), 7.50 (t, *J* = 7.9 Hz, 1H), 7.28 (dd, *J* = 1.4, 7.6 Hz, 1H), 7.20 - 7.15 (m, 1H), 6.79 (s, 1H), 4.76 (br s, 1H), 4.22 (br s, 1H), 4.15 - 3.97 (m, 3H), 3.95 - 3.83 (m, 3H), 2.77 - 2.64 (m, 3H), 2.44 - 2.41 (m, 1H), 2.17 - 2.01 (m, 3H), 1.98 (s, 1H), 1.92 (br s, 1H), 1.68 - 1.53 (m, 2H), 1.29 (d, *J* = 3.6 Hz, 6H).

### 118-2. Preparation of Compound 341, 2-(((R)-2-((2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)-2-methylpropanoic acid

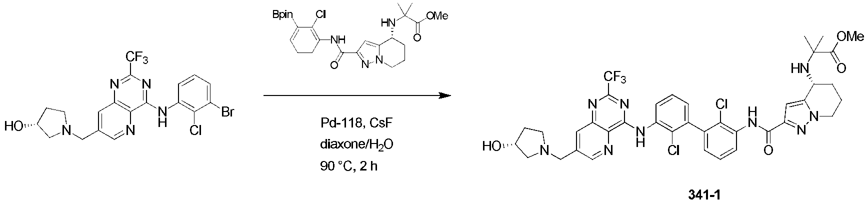

(3R)-1-[[4-(3-bromo-2-chloro-anilino)-2-(trifluoromethyl)pyrido[3,2-d]pyrimidin-7-yl]methyl]pyrrolidin-3-ol (110 mg, 218.81 µmol, 1 eq), methyl 2-[[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]-2-methyl-propanoate (135.70 mg, 262.57 µmol, 1.2 eq), ditert-butyl(cyclopentyl)phosphane;dichloropalladium;iron (14.26 mg, 21.88 µmol, 0.1 eq), and K₂CO₃ (90.72 mg, 656.44 µmol, 3 eq) in dioxane (5 mL)/H₂O (1.5 mL) were stirred under N₂ at 90 °C for 2 hours. The mixture was cooled to room temperature, filtered, and the filtrate was concentrated under reduced pressure. The residue was purified by prep-HPLC (Agela DuraShell C18 150 * 25 mm * 5 µm; mobile phase: [water (HCl)-ACN]; gradient: 22%-52% B over 20 min) to give compound 341-1 (121 mg, 61.2% yield) as a white solid.

LCMS m/z 812.3 [M+1]⁺.

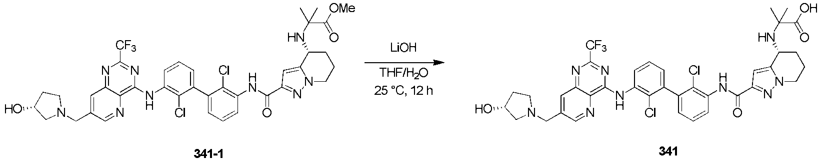

Methyl 2-[[(4R)-2-[[2-chloro-3-[2-chloro-3-[[7-[[(3R)-3-hydroxypyrrolidin-1-yl]methyl]-2-(trifluoromethyl)pyrido[3,2-d]pyrimidin-4-yl]amino]phenyl]phenyl]carbamoyl]-4,5,6,7-tetrahydropyrazolo[1,5-a]pyridin-4-yl]amino]-2-methyl-propanoate 341-1 (80 mg, 98.44 µmol, 1 eq) in THF (6 mL)/H₂O (2 mL) was treated with LiOH·H₂O (12.39 mg, 295.32 µmol, 3 eq) at 0 °C, and the mixture was stirred at room temperature for 12 hours. The mixture was then adjusted to pH 7 with aq. 1N HCl, and the precipitated solid was filtered. The solid was dried under vacuum and further purified by prep-HPLC (Waters Xbridge 150 * 25 mm * 5 µm; mobile phase: [water (NH₄HCO₃)-ACN]; gradient: 20%-50% B over 15 min) to afford Compound 341 (39 mg, 47.1% yield) as a white solid.

LCMS (m/z 798.3 [M+1]⁺.

¹H NMR (400 MHz, DMSO-*d₆*) δ = 10.49 (br s, 1H), 9.54 (s, 1H), 9.05 (d, *J* = 1.8 Hz, 1H), 8.34 (dd, *J* = 1.3, 8.2 Hz, 1H), 8.30 (s, 1H), 8.29 (d, *J* = 7.0 Hz, 2H), 7.60 (t, *J* = 7.9 Hz, 1H), 7.50 (t, *J* = 7.9 Hz, 1H), 7.28 (dd, *J =* 1.3, 7.5 Hz, 1H), 7.18 (dd, J = 1.3, 7.6 Hz, 1H), 6.79 (s, 1H), 4.75 (br s, 1H), 4.22 (br s, 1H), 4.15 - 4.08 (m, 2H), 3.96 - 3.83 (m, 3H), 2.77 - 2.66 (m, 2H), 2.45 - 2.38 (m, 2H), 2.18 - 1.96 (m, 4H), 1.93 (br s, 1H), 1.68 - 1.52 (m, 2H), 1.29 (d, *J* = 3.4 Hz, 6H).

### [Preparation Example 119]

### Preparation of Compound 342, 1-((S)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid, and Compound 343, 1-((R)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)piperidine-4-carboxylic acid

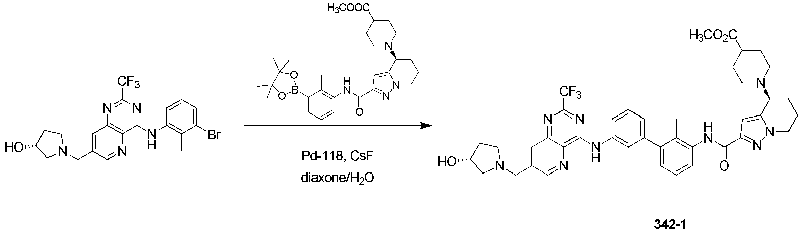

A mixture of (3R)-1-[[4-(3-bromo-2-methyl-anilino)-2-(trifluoromethyl)pyrido[3,2-d]pyrimidin-7-yl]methyl]pyrrolidin-3-ol (100 mg, 207.34 µmol, 1 eq), methyl 1-[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (108.32 mg, 207.34 µmol, 1 eq), ditert-butyl(cyclopenta-1,4-dien-1-yl)phosphane;dichloropalladium;iron (13.51 mg, 20.73 µmol, 0.1 eq), CsF (94.49 mg, 622.02 µmol, 3 eq) in dioxane (10 mL)/H₂O (2 mL) was stirred under N₂ at 90 °C for 12 hours. The mixture was cooled to room temperature, filtered, and the filtrate concentrated under reduced pressure. The residue was purified by column chromatography (SiO₂, Petroleum ether/Ethyl acetate = 1/0 to 0/1, Ethyl acetate/Methanol = 1/0 to 0/1) to give compound 342-1 (250 mg, 98.2% yield) as a brown solid.

LCMS m/z 798.3 [M+1]⁺.

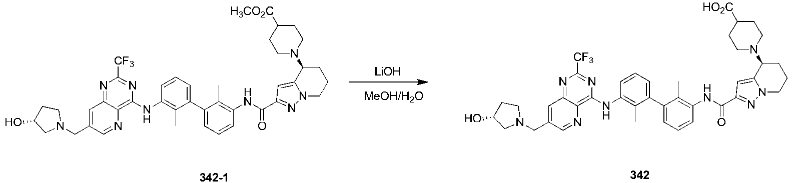

Methyl 1-[(4S)-2-[[3-[3-[[7-[[(3R)-3-hydroxypyrrolidin-1-yl]methyl]-2-(trifluoromethyl)pyrido[3,2-d]pyrimidin-4-yl]amino]-2-methyl-phenyl]-2-methyl-phenyl]carbamoyl]-4,5,6,7-tetrahydropyrazolo[1,5-a]pyridin-4-yl]piperidine-4-carboxylate 342-1 (250 mg, 313.34 µmol, 1 eq) was dissolved in a mixture of H₂O (4 mL) / MeOH (10 mL), and LiOH·H₂O (39.45 mg, 940.01 µmol, 3 eq) was added. The mixture was stirred at room temperature for 12 hours. The reaction mixture was concentrated under reduced pressure, and the residue was purified by reversed-phase HPLC (column: Waters Xbridge 150 * 25 mm * 5 µm; mobile phase: [water (NH₄HCO₃)-ACN]; gradient: 13%-43% B over 10 min) to give compound 342 (49 mg, 19.5% yield) as a yellow solid.

LCMS m/z 784.8 [M+1]⁺.

¹H NMR (400 MHz, DMSO-*d₆*) δ ppm 1.44 - 1.75 (m, 5 H) 1.78 - 1.85 (m, 2 H) 1.91 - 2.08 (m, 9 H) 2.12 - 2.31 (m, 4 H) 2.39 - 2.46 (m, 2 H) 2.67 - 2.81 (m, 4 H) 3.82 - 3.90 (m, 2 H) 3.93 - 3.98 (m, 1 H) 4.01 - 4.08 (m, 1 H) 4.17 - 4.25 (m, 2 H) 4.80 (br s, 1 H) 6.64 (s, 1 H) 7.01 (d, *J*=6.75 Hz, 1 H) 7.09 (d, *J*=7.38 Hz, 1 H) 7.29 (t, *J*=7.69 Hz, 1 H) 7.36 (t, *J*=7.75 Hz, 1 H) 7.52 - 7.63 (m, 2 H) 8.22 (d, *J*=1.63 Hz, 1 H) 9.00 (d, *J*=1.75 Hz, 1 H) 9.54 (d, *J*=2.88 Hz, 1 H) 10.65 (br s, 1 H).

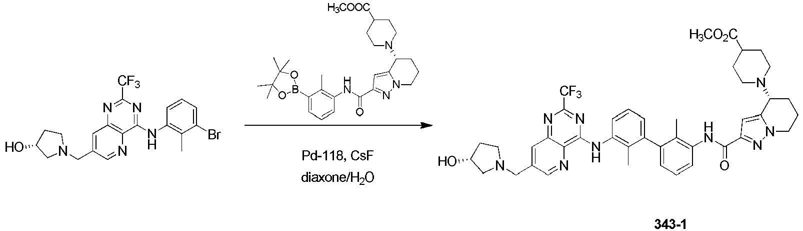

(3R)-1-[[4-(3-bromo-2-methyl-anilino)-2-(trifluoromethyl)pyrido[3,2-d]pyrimidin-7-yl]methyl]pyrrolidin-3-ol (100 mg, 207.34 µmol, 1 eq) and methyl 1-[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (108.32 mg, 207.34 µmol, 1 eq) were dissolved in a mixture of dioxane (10 mL) / H₂O (2 mL). Ditert-butyl(cyclopenta-1,4-dien-1-yl)phosphane;dichloropalladium;iron (13.51 mg, 20.73 µmol, 0.1 eq) and CsF (94.49 mg, 622.02 µmol, 3 eq) were added, and the reaction mixture was stirred under nitrogen at 90 °C for 12 hours. The mixture was cooled to room temperature, filtered, and concentrated under reduced pressure. The residue was purified by column chromatography (SiO₂, Petroleum ether/Ethyl acetate = 1/0 to 0/1, Ethyl acetate/Methanol = 1/0 to 0/1) to afford Compound 343-1 (230 mg, 82.0% yield) as a brown solid.

LCMS m/z 798.6 [M+1]⁺.

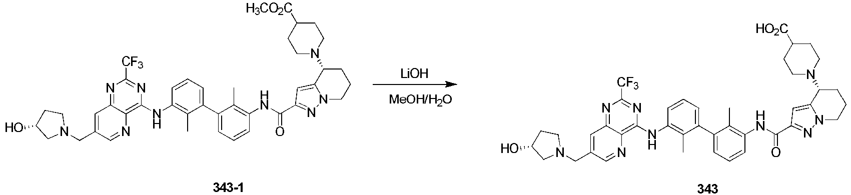

Methyl 1-[(4R)-2-[[3-[3-[[7-[[(3R)-3-hydroxypyrrolidin-1-yl]methyl]-2-(trifluoromethyl)pyrido[3,2-d]pyrimidin-4-yl]amino]-2-methyl-phenyl]-2-methyl-phenyl]carbamoyl]-4,5,6,7-tetrahydropyrazolo[1,5-a]pyridin-4-yl]piperidine-4-carboxylate 343-1 (230 mg, 288.27 µmol, 1 eq) was dissolved in a mixture of H₂O (2 mL) / MeOH (10 mL). LiOH·H₂O (36.29 mg, 864.81 µmol, 3 eq) was added, and the reaction mixture was stirred at room temperature for 12 hours. The mixture was concentrated under reduced pressure, and the residue was purified by reversed-phase HPLC (column: Waters Xbridge 150 * 25 mm * 5 µm; mobile phase: [water (NH₄HCO₃) -ACN]; gradient: 13%-43% B over 10 min) to afford Compound 343 (53 mg, 22.5% yield) as a yellow solid.

LCMS m/z 784.8 [M+1]⁺.

¹H NMR (400 MHz, DMSO-*d₆*) δ ppm 1.48 - 1.73 (m, 4 H) 1.77 - 1.86 (m, 2 H) 1.87 - 2.09 (m, 8 H) 2.13 - 2.30 (m, 3 H) 2.36 - 2.46 (m, 2 H) 2.66 - 2.83 (m, 4 H) 3.82 - 3.91 (m, 2 H) 3.93 - 3.98 (m, 1 H) 4.05 (td, J=11.82, 4.13 Hz, 1 H) 4.17 - 4.27 (m, 2 H) 4.64 - 4.90 (m, 1 H) 6.63 (s, 1 H) 6.93 - 7.14 (m, 2 H) 7.25 - 7.40 (m, 2 H) 7.50 - 7.62 (m, 2 H) 8.22 (d, J=1.50 Hz, 1 H) 9.00 (d, J=1.88 Hz, 1 H) 9.54 (d, J=3.00 Hz, 1 H) 10.56 - 10.73 (m, 1 H).

### [Preparation Example 120]

### Preparation of Compound 344, (S)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-((R)-3-hydroxypyrrolidin-1-yl)-4,5,6,7-tetrahydropyrazolo[1,5-a]pyridine-2-carboxamide, and Compound 345, (R)-4-((R)-3-hydroxypyrrolidin-1-yl)-N-(3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)-4,5,6,7-tetrahydropyrazolo[1,5-a]pyridine-2-carboxamide

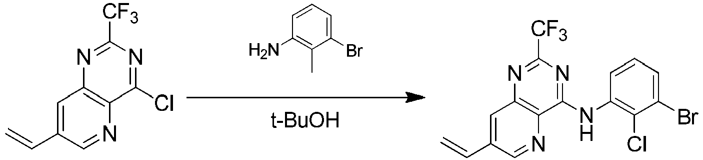

4-chloro-2-(trifluoromethyl)-7-vinyl-pyrido[3,2-d]pyrimidine (5 g, 19.26 mmol, 1 eq) and 3-bromo-2-methyl-aniline (4.66 g, 25.04 mmol, 3.08 mL, 1.3 eq) were dissolved in t-BuOH (100 mL) and stirred at 80 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 3/1) to afford N-(3-bromo-2-methyl-phenyl)-2-(trifluoromethyl)-7-vinyl-pyrido[3,2-d]pyrimidin-4-amine (10 g, 88.8% yield) as a black solid.

LCMS m/z 410.9 [M+3]⁺.

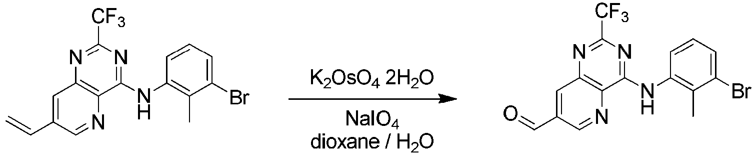

N-(3-bromo-2-methyl-phenyl)-2-(trifluoromethyl)-7-vinyl-pyrido[3,2-d]pyrimidin-4-amine (4 g, 9.78 mmol, 1 eq) was dissolved in a mixture of dioxane (100 mL) / H₂O (20 mL). Dipotassium dioxido(dioxo)osmium dihydrate (720.34 mg, 1.96 mmol, 0.2 eq) and sodium periodate (8.36 g, 39.10 mmol, 2.17 mL, 4 eq) were added, and the reaction was stirred at room temperature for 12 hours. H₂O (100 mL) was added, and the mixture was extracted with ethyl acetate (100 mL * 3). The combined organic layers were washed with brine, dried over Na₂SO₄, filtered, and concentrated under reduced pressure. The residue was purified by column chromatography (SiO₂, Petroleum ether/Ethyl acetate = 1/0 to 2/1) to afford 4-(3-bromo-2-methyl-anilino)-2-(trifluoromethyl)pyrido[3,2-d]pyrimidine-7-carbaldehyde (1.7 g, 33.8% yield) as a white solid.

LCMS m/z 412.9 [M+3]⁺.

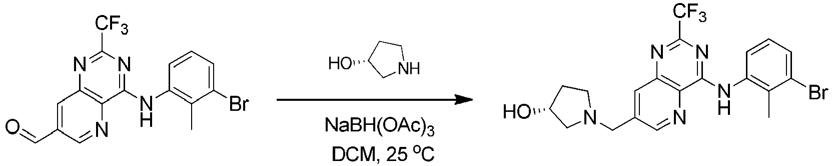

4-(3-bromo-2-methyl-anilino)-2-(trifluoromethyl)pyrido[3,2-d]pyrimidine-7-carbaldehyde (1.6 g, 3.89 mmol, 1 eq) was dissolved in DCM (30 mL) together with (3R)-pyrrolidin-3-ol (1.02 g, 11.67 mmol, 970.44 µL, 3 eq). NaBH(OAc)₃ (2.47 g, 11.67 mmol, 3 eq) was added, and the reaction mixture was stirred at room temperature for 12 hours. The mixture was filtered, and the filtrate was concentrated under reduced pressure. The residue was purified by reversed-phase HPLC (column: Kromasil Eternity XT 250 * 80 mm * 10 µm; mobile phase: [water (NH₄HCO₃)-ACN]; gradient: 35%-70% B over 25 min) to give (3R)-1-[[4-(3-bromo-2-methyl-anilino)-2-(trifluoromethyl)pyrido[3,2-d]pyrimidin-7-yl]methyl]pyrrolidin-3-ol (900 mg, 47.4% yield) as a yellow solid.

LCMS m/z 484.1 [M+3]⁺.

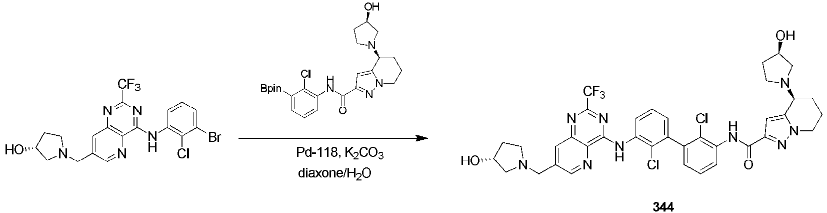

(3R)-1-[[4-(3-bromo-2-methyl-anilino)-2-(trifluoromethyl)pyrido[3,2-d]pyrimidin-7-yl]methyl]pyrrolidin-3-ol (130 mg, 269.54 µmol, 1 eq) was combined with (4S)-4-[(3R)-3-hydroxypyrrolidin-1-yl]-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (150.85 mg, 323.45 µmol, 1.2 eq), ditert-butyl(cyclopenta-1,4-dien-1-yl)phosphane;dichloropalladium;iron (17.57 mg, 26.95 µmol, 0.1 eq), and K₂CO₃ (111.76 mg, 808.63 µmol, 3 eq) in dioxane (10 mL)/H₂O (2 mL). The mixture was stirred under nitrogen at 90 °C for 12 hours. After cooling to room temperature, the mixture was filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 1/1) and further purified by reversed-phase HPLC (column: Waters Xbridge 150 * 25 mm * 5 µm; mobile phase: [water (NH₄HCO₃)-ACN]; gradient: 30%-60% B over 10 min) to afford Compound 344 (30 mg, 14.4% yield) as a yellow solid.

LCMS m/z 742.6 [M+1]⁺.

¹H NMR (400 MHz, DMSO-*d₆*) δ ppm 1.58 (br d, J=4.25 Hz, 2 H) 1.70 - 2.16 (m, 11 H) 2.25 (br s, 1 H) 2.40 - 2.47 (m, 2 H) 2.65 - 2.83 (m, 3 H) 2.85 - 2.94 (m, 1 H) 3.74 - 3.98 (m, 3 H) 4.17 (br s, 4 H) 4.64 - 4.86 (m, 2 H) 6.69 (s, 1 H) 6.95 - 7.13 (m, 2 H) 7.23 - 7.41 (m, 2 H) 7.58 (br d, J=7.63 Hz, 2 H) 8.22 (s, 1 H) 9.00 (s, 1 H) 9.54 (s, 1 H) 10.65 (br s, 1 H).

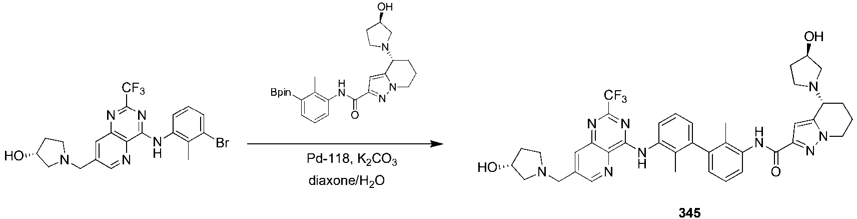

(3R)-1-[[4-(3-bromo-2-methyl-anilino)-2-(trifluoromethyl)pyrido[3,2-d]pyrimidin-7-yl]methyl]pyrrolidin-3-ol (130 mg, 269.54 µmol, 1 eq) was combined with (4R)-4-[(3R)-3-hydroxypyrrolidin-1-yl]-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (150.85 mg, 323.45 µmol, 1.2 eq), ditert-butyl(cyclopenta-1,4-dien-1-yl)phosphane;dichloropalladium;iron (17.57 mg, 26.95 µmol, 0.1 eq), and K₂CO₃ (111.76 mg, 808.63 µmol, 3 eq) in dioxane (10 mL)/H₂O (2 mL). The mixture was stirred under nitrogen at 90 °C for 12 hours. After cooling to room temperature, the mixture was filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 1/1) and further purified by reversed-phase HPLC (column: Waters Xbridge 150 * 25 mm * 5 µm; mobile phase: [water (NH₄HCO₃)-ACN]; gradient: 30%-60% B over 10 min) to give compound 345 (53 mg, 25.1% yield) as a yellow solid.

LCMS m/z 742.7 [M+1]⁺.

¹H NMR (400 MHz, DMSO-*d₆*) δ ppm 1.52 - 1.64 (m, 2 H) 1.85 - 2.09 (m, 11 H) 2.24 (br d, *J*=6.50 Hz, 1 H) 2.42 (br dd, *J*=9.57, 3.31 Hz, 1 H) 2.58 - 2.65 (m, 1 H) 2.66 - 2.81 (m, 4 H) 3.76 - 3.95 (m, 3 H) 4.12 - 4.27 (m, 4 H) 4.68 - 4.84 (m, 2 H) 6.67 (s, 1 H) 7.01 (br d, *J*=7.38 Hz, 1 H) 7.09 (br d, *J*=7.50 Hz, 1 H) 7.29 (t, *J*=7.75 Hz, 1 H) 7.36 (br t, *J*=7.75 Hz, 1 H) 7.58 (br d, *J*=7.75 Hz, 2 H) 8.22 (s, 1 H) 9.00 (d, *J*=1.38 Hz, 1 H) 9.54 (s, 1 H) 10.64 (br s, 1 H).

### [Preparation Example 121]

### Preparation of Compound 346, (S)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4-(methylamino)-4,5,6,7-tetrahydropyrazolo[1,5-a]pyridine-2-carboxamide, and Compound 347, (R)-N-(3'-((3-(((R)-3-hydroxypyrrolidin-1-yl)methyl)-1,7-naphthyridin-8-yl)amino)-2,2'-dimethyl-[1,1'-biphenyl]-3-yl)-4-(methylamino)-4,5,6,7-tetrahydropyrazolo[1,5-a]pyridine-2-carboxamide

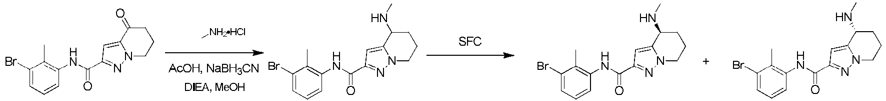

N-(3-bromo-2-methyl-phenyl)-4-oxo-6,7-dihydro-5H-pyrazolo[1,5-a]pyridine-2-carboxamide (5 g, 14.36 mmol, 1 eq) was dissolved in MeOH (100 mL), and DIPEA (9.28 g, 71.80 mmol, 12.51 mL, 5 eq) was added. The mixture was stirred at room temperature for 30 minutes. Methanamine hydrochloride (2.91 g, 43.08 mmol, 3 eq), AcOH (4.31 g, 71.80 mmol, 4.11 mL, 5 eq), and NaBH₃CN (4.51 g, 71.80 mmol, 5 eq) were then added, and the mixture was stirred at 60 °C for 12 hours. The reaction mixture was concentrated under reduced pressure, diluted with water (300 mL), and extracted with ethyl acetate (100 mL * 3). The combined organic layers were dried over Na₂SO₄, filtered, and concentrated under reduced pressure. The residue was purified by flash silica gel chromatography (ISCO^{®}, 120 g SepaFlash^{®} Silica Column, eluent 0-40% ethyl acetate/MeOH at 80 mL/min) and further purified by reversed-phase HPLC (DAICEL CHIRALPAK AS, 250 * 30 mm * 10 µm; mobile phase CO₂/i-PrOH with 0.1% NH₃H₂O; B% 50%, isocratic elution mode) to afford (4S)-N-(3-bromo-2-methyl-phenyl)-4-(methylamino)-4,5,6,7-tetrahydropyrazolo[1,5-a]pyridine-2-carboxamide (1.4 g, 26.3% yield) as a yellow liquid and (4R)-N-(3-bromo-2-methyl-phenyl)-4-(methylamino)-4,5,6,7-tetrahydropyrazolo[1,5-a]pyridine-2-carboxamide (1.4 g, 26.3% yield) as a white solid.

LCMS m/z 362.9 [M+1] ⁺, LCMS (EW41301-1044-P1D2), m/z 362.9 [M+1] ⁺

¹H NMR (400 MHz, DMSO-*d*6) δ ppm 1.84 (br s, 1 H) 1.92 - 1.97 (m, 1 H) 2.11 (br dd, *J*=7.69, 5.32 Hz, 1 H) 2.18 - 2.24 (m, 1 H) 2.26 (s, 3 H) 2.45 (s, 3 H) 4.03 (q, *J*=7.09 Hz, 1 H) 4.10 (br t, *J*=5.69 Hz, 1 H) 4.14 - 4.20 (m, 2 H) 6.85 (s, 1 H) 7.12 - 7.19 (m, 1 H) 7.41 (d, *J*=7.75 Hz, 1 H) 7.49 (d, *J*=7.88 Hz, 1 H) 9.86 (s, 1 H).

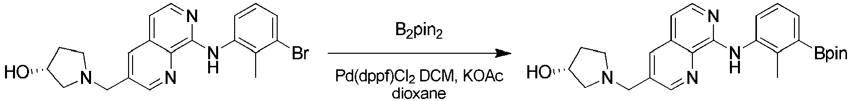

(3R)-1-[[8-(3-bromo-2-methyl-anilino)-1,7-naphthyridin-3-yl]methyl]pyrrolidin-3-ol (300 mg, 725.85 µmol, 1 eq), 4,4,5,5-tetramethyl-2-(4,4,5,5-tetramethyl-1,3,2-dioxaborolan-2-yl)-1,3,2-dioxaborolane (552.96 mg, 2.18 mmol, 3 eq), KOAc (213.70 mg, 2.18 mmol, 3 eq), and Pd(dppf)Cl₂·CH₂Cl₂ (59.28 mg, 72.58 µmol, 0.1 eq) were stirred in dioxane (10 mL) under nitrogen at 100 °C for 12 hours. The mixture was cooled to room temperature, filtered, and the filtrate was concentrated under reduced pressure. The residue was purified by flash silica gel chromatography (ISCO^{®}, 12 g SepaFlash^{®} Silica Column, eluent 0-20% ethyl acetate/ethanol at 30 mL/min) to afford (3R)-1-[[8-[2-methyl-3-(4,4,5,5-tetramethyl-1,3,2-dioxaborolan-2-yl)anilino]-1,7-naphthyridin-3-yl]methyl]pyrrolidin-3-ol (200 mg, 53.8% yield) as a yellow liquid.

LCMS m/z 461.3 [M+1]⁺

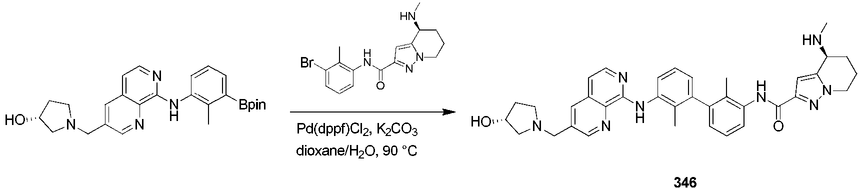

(4S)-N-(3-bromo-2-methyl-phenyl)-4-(methylamino)-4,5,6,7-tetrahydropyrazolo[1,5-a]pyridine-2-carboxamide (110 mg, 302.82 µmol, 1 eq), (3R)-1-[[8-[2-methyl-3-(4,4,5,5-tetramethyl-1,3,2-dioxaborolan-2-yl)anilino]-1,7-naphthyridin-3-yl]methyl]pyrrolidin-3-ol (153.35 mg, 333.10 µmol, 1.1 eq), ditert-butyl(cyclopenta-1,4-dien-1-yl)phosphane;dichloropalladium;iron (19.74 mg, 30.28 µmol, 0.1 eq), and K₂CO₃ (125.56 mg, 908.46 µmol, 3 eq) were stirred in a mixture of dioxane (5 mL) / H₂O (1 mL) under nitrogen at 90 °C for 12 hours. The mixture was cooled to room temperature, filtered, and the filtrate was concentrated under reduced pressure. The residue was purified by flash silica gel chromatography (ISCO^{®}, 12 g SepaFlash^{®} Silica Column, eluent 0-20% DCM:MeOH at 40 mL/min) and further purified by reversed-phase HPLC (column: Waters XBridge 150 * 25 mm * 5 µm; mobile phase: [water (NH₄HCO₃)-ACN]; gradient: 33%-63% B over 15 min) to afford Compound 346 (113 mg, 59.9% yield) as a white solid.

LCMS m/z 617.3 [M+1] ⁺

¹H NMR (400 MHz, DMSO-*d*6) δ ppm 1.54 - 1.61 (m, 1 H) 1.63 - 1.72 (m, 1 H) 1.88 - 1.94 (m, 1 H) 1.97 (s, 3 H) 2.02 (br dd, *J*=13.07, 6.82 Hz, 2 H) 2.08 (s, 3 H) 2.17 (br s, 2 H) 2.32 (s, 4 H) 2.38 (dd, *J*=9.63, 3.63 Hz, 1 H) 2.62 - 2.69 (m, 1 H) 2.74 (dd, *J*=9.69, 6.32 Hz, 1 H) 3.76 (br s, 1 H) 3.78 - 3.86 (m, 2 H) 4.10 - 4.16 (m, 2 H) 4.19 - 4.25 (m, 1 H) 4.73 (d, *J*=4.50 Hz, 1 H) 6.71 (s, 1 H) 6.86 (d, *J*=6.88 Hz, 1 H) 7.00 (d, *J*=6.75 Hz, 1 H) 7.18 (d, *J*=5.88 Hz, 1 H) 7.25 - 7.35 (m, 2 H) 7.59 (d, *J*=8.00 Hz, 1 H) 8.05 (d, *J*=5.88 Hz, 1 H) 8.18 (d, *J*=1.63 Hz, 1 H) 8.43 (d, *J*=8.00 Hz, 1 H) 8.86 (d, *J*=1.88 Hz, 1 H) 9.30 (s, 1 H) 9.51 (s, 1 H)

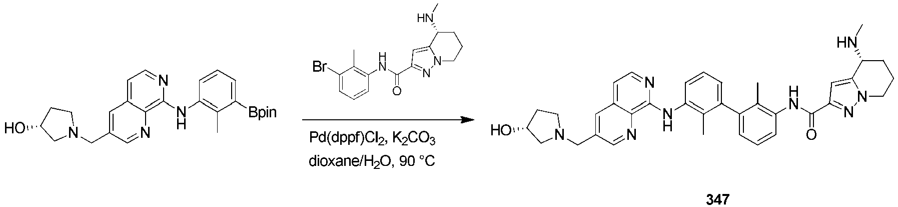

(4R)-N-(3-bromo-2-methyl-phenyl)-4-(methylamino)-4,5,6,7-tetrahydropyrazolo[1,5-a]pyridine-2-carboxamide (110 mg, 302.82 µmol, 1 eq), (3R)-1-[[8-[2-methyl-3-(4,4,5,5-tetramethyl-1,3,2-dioxaborolan-2-yl)anilino]-1,7-naphthyridin-3-yl]methyl]pyrrolidin-3-ol (153.35 mg, 333.10 µmol, 1.1 eq), ditert-butyl(cyclopenta-1,4-dien-1-yl)phosphane;dichloropalladium;iron (19.74 mg, 30.28 µmol, 0.1 eq), and K₂CO₃ (125.56 mg, 908.46 µmol, 3 eq) were stirred in a mixture of dioxane (5 mL) / H₂O (1 mL) under nitrogen at 90 °C for 12 hours. The mixture was cooled to room temperature, filtered, and the filtrate was concentrated under reduced pressure. The residue was purified by flash silica gel chromatography (ISCO^{®}, 12 g SepaFlash^{®} Silica Column, eluent 0-30% DCM:MeOH at 40 mL/min) and further purified by reversed-phase HPLC (column: Waters XBridge 150 * 25 mm * 5 µm; mobile phase: [water (NH₄HCO₃)-ACN]; gradient: 30%-60% B over 10 min) to afford Compound 347 (102 mg, 54.1% yield) as a white solid.

LCMS m/z 617.3 [M+1]⁺

¹H NMR (400 MHz, DMSO-*d*6) δ ppm 1.57 (qd, *J*=7.96, 5.25 Hz, 1 H) 1.64 - 1.72 (m, 1 H) 1.87 - 1.94 (m, 1 H) 1.97 (s, 3 H) 1.99 - 2.05 (m, 2 H) 2.02 - 2.02 (m, 1 H) 2.08 (s, 3 H) 2.14 - 2.24 (m, 2 H) 2.33 (s, 3 H) 2.38 (dd, *J*=9.57, 3.56 Hz, 1 H) 2.65 (q, *J*=7.59 Hz, 1 H) 2.74 (dd, *J*=9.57, 6.32 Hz, 1 H) 3.73 - 3.87 (m, 3 H) 4.10 - 4.17 (m, 2 H) 4.19 - 4.25 (m, 1 H) 4.72 (d, *J*=4.50 Hz, 1 H) 6.71 (s, 1 H) 6.86 (d, *J*=7.38 Hz, 1 H) 7.00 (d, *J*=7.50 Hz, 1 H) 7.17 (d, *J*=5.88 Hz, 1 H) 7.25 - 7.34 (m, 2 H) 7.59 (d, *J*=8.00 Hz, 1 H) 8.05 (d, *J*=5.75 Hz, 1 H) 8.18 (s, 1 H) 8.43 (d, *J*=8.13 Hz, 1 H) 8.86 (d, *J*=1.38 Hz, 1 H) 9.30 (s, 1 H) 9.49 (s, 1 H)

### [Preparation Example 122]

### Preparation of Compound 348, (S)-N-(3'-(5-(((R)-3-hydroxypyrrolidin-1-yl)methyl)picolinamido)-2,2'-dimethyl-[1,1'-biphenyl]-3-yl)-4-(methylamino)-4,5,6,7-tetrahydropyrazolo[1,5-a]pyridine-2-carboxamide, and Compound 349, (R)-N-(3'-(5-(((R)-3-hydroxypyrrolidin-1-yl)methyl)picolinamido)-2,2'-dimethyl-[1,1'-biphenyl]-3-yl)-4-(methylamino)-4,5,6,7-tetrahydropyrazolo[1,5-a]pyridine-2-carboxamide

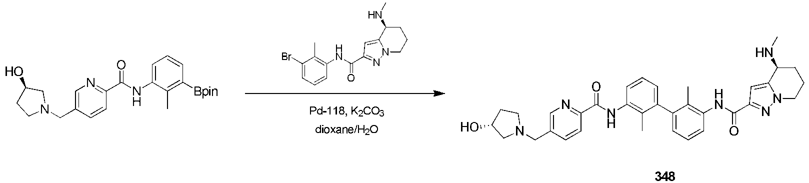

(4S)-N-(3-bromo-2-methyl-phenyl)-4-(methylamino)-4,5,6,7-tetrahydropyrazolo[1,5-a]pyridine-2-carboxamide (100 mg, 275.29 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (132.44 mg, 302.82 µmol, 1.1 eq), ditert-butyl(cyclopenta-1,4-dien-1-yl)phosphane;dichloropalladium;iron (17.94 mg, 27.53 µmol, 0.1 eq), and K₂CO₃ (114.14 mg, 825.87 µmol, 3 eq) were stirred in a mixture of dioxane (10 mL) / H₂O (2 mL) under nitrogen at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 0/1) and further purified by reversed-phase HPLC (column: Waters XBridge 150 * 25 mm * 5 µm; mobile phase: [water (NH₄HCO₃)-ACN]; gradient: 40%-70% B over 10 min) to give compound 348 (36 mg, 21.5% yield) as a yellow solid.

LCMS m/z 594.7 [M+1]⁺.

¹H NMR (400 MHz, DMSO-*d*₆) δ ppm 1.51 - 1.61 (m, 1 H) 1.62 - 1.73 (m, 1 H) 1.86 - 2.06 (m, 9 H) 2.14 - 2.22 (m, 1 H) 2.41 - 2.47 (m, 1 H) 2.51 (br s, 3 H) 2.58 - 2.73 (m, 2 H) 3.63 - 3.79 (m, 3 H) 4.04 - 4.26 (m, 3 H) 4.77 (br d, *J*=2.50 Hz, 1 H) 6.73 (s, 1 H) 6.98 (br d, *J*=7.50 Hz, 2 H) 7.30 (dt, *J*=15.42, 7.74 Hz, 2 H) 7.59 (br d, *J*=7.88 Hz, 1 H) 7.87 (br d, *J*=7.88 Hz, 1 H) 7.98 (br d, *J*=7.88 Hz, 1 H) 8.14 (d, *J*=7.88 Hz, 1 H) 8.64 (s, 1 H) 9.52 (s, 1 H) 10.33 (s, 1 H).

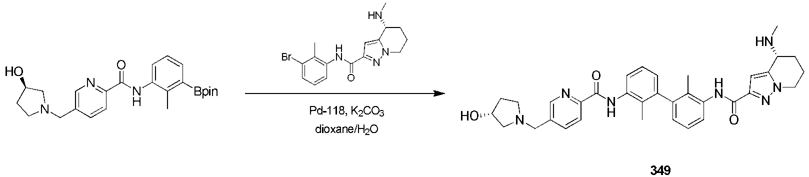

(4R)-N-(3-bromo-2-methyl-phenyl)-4-(methylamino)-4,5,6,7-tetrahydropyrazolo[1,5-a]pyridine-2-carboxamide (100 mg, 275.29 µmol, 1 eq), 5-[[(3R)-3-hydroxypyrrolidin-1-yl]methyl]-N-[2-methyl-3-(4,4,5,5-tetramethyl-1,3,2-dioxaborolan-2-yl)phenyl]pyridine-2-carboxamide (132.44 mg, 302.82 µmol, 1.1 eq), ditert-butyl(cyclopenta-1,4-dien-1-yl)phosphane;dichloropalladium;iron (17.94 mg, 27.53 µmol, 0.1 eq), and K₂CO₃ (114.14 mg, 825.87 µmol, 3 eq) were stirred in a mixture of dioxane (10 mL) / H₂O (2 mL) under nitrogen at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, ethyl acetate/methanol = 1/0 to 0/1) and further purified by reversed-phase HPLC (column: Waters XBridge 150 * 25 mm * 5 µm; mobile phase: [water (NH₄HCO₃)-ACN]; gradient: 23%-53% B over 10 min) to give compound 349 (39 mg, 23.6% yield) as a yellow solid.

LCMS m/z 594.5 [M+1]⁺.

¹H NMR (400 MHz, DMSO-*d*₆) δ ppm 1.57 (br dd, J=8.07, 4.44 Hz, 1 H) 1.62 - 1.73 (m, 1 H) 1.84 - 2.05 (m, 8 H) 2.19 (br dd, *J*=12.44, 6.19 Hz, 1 H) 2.41 - 2.47 (m, 1 H) 2.51 (br s, 3 H) 2.62 (q, *J*=7.59 Hz, 1 H) 2.70 (br dd, *J*=9.38, 6.25 Hz, 1 H) 3.64 - 3.80 (m, 3 H) 4.07 - 4.25 (m, 3 H) 4.75 (br s, 1 H) 6.73 (s, 1 H) 6.98 (br d, *J*=7.38 Hz, 2 H) 7.30 (dt, *J*=15.51, 7.75 Hz, 2 H) 7.59 (br d, *J*=7.88 Hz, 1 H) 7.88 (br d, *J*=7.88 Hz, 1 H) 7.98 (br d, *J*=7.75 Hz, 1 H) 8.14 (d, *J*=8.00 Hz, 1 H) 8.65 (s, 1 H) 9.52 (s, 1 H) 10.33 (s, 1 H).

### [Preparation Example 123]

### Preparation of Compound 350, (S)-N-(2,2'-dichloro-3'-(5-(((R)-3-hydroxypyrrolidin-1-yl)methyl)picolinamido)-[1,1'-biphenyl]-3-yl)-4-(methylamino)-4,5,6,7-tetrahydropyrazolo[1,5-a]pyridine-2-carboxamide, and Compound 351, (R)-N-(2,2'-dichloro-3'-(5-(((R)-3-hydroxypyrrolidin-1-yl)methyl)picolinamido)-[1,1'-biphenyl]-3-yl)-4-(methylamino)-4,5,6,7-tetrahydropyrazolo[1,5-a]pyridine-2-carboxamide

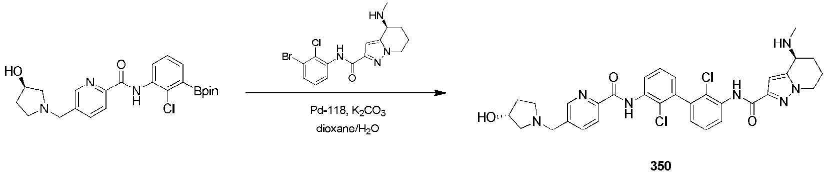

(4S)-N-(3-bromo-2-chloro-phenyl)-4-(methylamino)-4,5,6,7-tetrahydropyrazolo[1,5-a]pyridine-2-carboxamide (100 mg, 260.64 µmol, 1 eq), N-[2-chloro-3-(4,4,5,5-tetramethyl-1,3,2-dioxaborolan-2-yl)phenyl]-5-[[(3R)-3-hydroxypyrrolidin-1-yl]methyl]pyridine-2-carboxamide (238.62 mg, 521.28 µmol, 2 eq), ditert-butyl(cyclopenta-1,4-dien-1-yl)phosphane;dichloropalladium;iron (16.99 mg, 26.06 µmol, 0.1 eq), and K₂CO₃ (108.07 mg, 781.92 µmol, 3 eq) were stirred in a mixture of dioxane (10 mL) / H₂O (2 mL) under nitrogen at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 0/1) and further purified by reversed-phase HPLC (column: Waters XBridge 150 * 25 mm * 5 µm; mobile phase: [water (NH₄HCO₃)-ACN]; gradient: 35%-65% B over 9 min, column: Agela DuraShell C18 150 * 25 mm * 5 µm; mobile phase: [water (HCl)-ACN]; gradient: 12%-42% B over 20 min) to give compound 350 (16 mg, 9.5% yield) as a yellow solid.

LCMS m/z 634.3 [M+1]⁺.

¹H NMR (400 MHz, DMSO-*d*₆) δ ppm 1.81 - 1.93 (m, 1 H) 2.01 - 2.09 (m, 2 H) 2.25 - 2.31 (m, 2 H) 2.65 (s, 3 H) 3.45 (br d, *J*=6.88 Hz, 4 H) 4.23 (br d, *J*=4.63 Hz, 2 H) 4.33 - 4.58 (m, 4 H) 4.64 (br s, 1 H) 5.33 - 5.63 (m, 1 H) 7.18 - 7.24 (m, 3 H) 7.54 (dt, *J*=15.70, 7.91 Hz, 2 H) 8.21 (d, *J*=8.25 Hz, 1 H) 8.28 - 8.32 (m, 1 H) 8.33 - 8.41 (m, 1 H) 8.47 (dd, *J*=8.26, 1.25 Hz, 1 H) 8.89 - 9.01 (m, 1 H) 9.47 - 9.56 (m, 1 H) 9.66 (s, 1 H) 10.72 (s, 1 H).

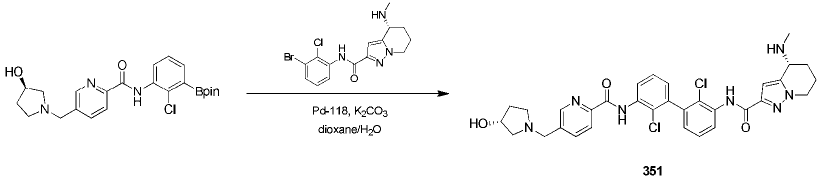

(4R)-N-(3-bromo-2-chloro-phenyl)-4-(methylamino)-4,5,6,7-tetrahydropyrazolo[1,5-a]pyridine-2-carboxamide (100 mg, 260.64 µmol, 1 eq), N-[2-chloro-3-(4,4,5,5-tetramethyl-1,3,2-dioxaborolan-2-yl)phenyl]-5-[[(3R)-3-hydroxypyrrolidin-1-yl]methyl]pyridine-2-carboxamide (238.62 mg, 521.28 µmol, 2 eq), ditert-butyl(cyclopenta-1,4-dien-1-yl)phosphane;dichloropalladium;iron (16.99 mg, 26.06 µmol, 0.1 eq), and K₂CO₃ (108.07 mg, 781.92 µmol, 3 eq) were stirred in a mixture of dioxane (10 mL) / H₂O (2 mL) under nitrogen at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, ethyl acetate/methanol = 1/0 to 0/1) and further purified by reversed-phase HPLC (column: Waters XBridge 150 * 25 mm * 5 µm; mobile phase: [water (NH₄HCO₃)-ACN]; gradient: 35%-65% B over 9 min, column: Agela DuraShell C18 150 * 25 mm * 5 µm; mobile phase: [water (HCl)-ACN]; gradient: 12%-42% B over 20 min) to give compound 351 (57 mg, 33.0% yield) as a yellow solid.

LCMS m/z 636.0 [M+3]⁺.

¹H NMR (400 MHz, DMSO-*d*₆) δ ppm 1.79 - 1.93 (m, 1 H) 1.96 - 2.11 (m, 3 H) 2.23 - 2.33 (m, 2 H) 2.64 (s, 3 H) 4.18 - 4.28 (m, 2 H) 4.35 - 4.58 (m, 3 H) 4.63 (br s, 1 H) 5.31 - 5.60 (m, 1 H) 7.17 - 7.26 (m, 3 H) 7.54 (dt, *J*=15.42, 7.86 Hz, 2 H) 8.20 (d, *J*=8.25 Hz, 1 H) 8.26 - 8.31 (m, 1 H) 8.34 (br s, 1 H) 8.47 (dd, *J*=8.19, 1.31 Hz, 1 H) 8.94 (br s, 1 H) 9.67 (s, 1 H) 10.72 (s, 1 H).

### [Preparation Example 124]

### 124-1. Preparation of Compound 352, (S)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide

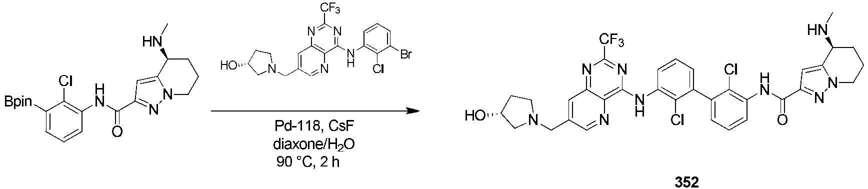

(3R)-1-[[4-(3-bromo-2-chloro-anilino)-2-(trifluoromethyl)pyrido[3,2-d]pyrimidin-7-yl]methyl]pyrrolidin-3-ol (100 mg, 198.92 µmol, 1 eq), (4S)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (102.82 mg, 238.70 µmol, 1.2 eq), ditert-butyl(cyclopentyl)phosphane;dichloropalladium;iron (12.96 mg, 19.89 µmol, 0.1 eq), and CsF (90.65 mg, 596.76 µmol, 3 eq) were stirred in a mixture of dioxane (6 mL) /H₂O (1.5 mL) under nitrogen at 90 °C for 2 hours. The mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to give compound 352 (48 mg, 30.0% yield) as a white solid.

LCMS m/z 728.3 [M+1]⁺.

¹H NMR (400 MHz, DMSO-*d*₆) δ = 10.67 (br s, 1H), 9.65 (br s, 2H), 9.33 (br s, 1H), 8.73 (br s, 1H), 8.21 (br s, 2H), 7.68 - 7.48 (m, 2H), 7.45 - 7.34 (m, 2H), 7.32 - 7.14 (m, 5H), 5.48 (br s, 1H), 4.73 - 4.61 (m, 2H), 4.43 (br s, 1H), 4.23 (br s, 2H), 2.28 (br s, 3H), 2.04 (br s, 2H), 1.23 (br s, 1H), 1.15 (br s, 2H), 1.06 (br s, 1H).

### 124-2. Preparation of Compound 353, (R)-N-(2,2'-dichloro-3'-((7-(((R)-3-hydroxypyrrolidin-1-yl)methyl)-2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide

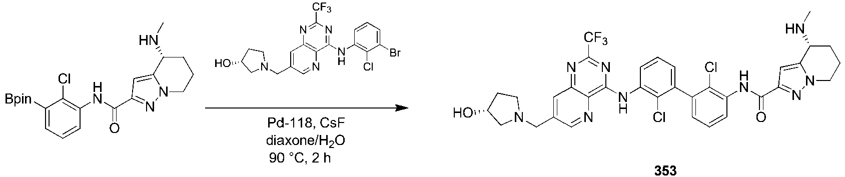

(3R)-1-[[4-(3-bromo-2-chloro-anilino)-2-(trifluoromethyl)pyrido[3,2-d]pyrimidin-7-yl]methyl]pyrrolidin-3-ol (100 mg, 198.92 µmol, 1 eq), (4R)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (102.82 mg, 238.70 µmol, 1.2 eq), ditert-butyl(cyclopentyl)phosphane;dichloropalladium;iron (12.96 mg, 19.89 µmol, 0.1 eq), and CsF (90.65 mg, 596.76 µmol, 3 eq) were stirred in a mixture of dioxane (6 mL) /H₂O (1.5 mL) under nitrogen at 90 °C for 2 hours. The mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 14%-44% B over 20 min) to give compound 353 (52 mg, 32.5% yield) as a white solid.

LCMS m/z 726.1 [M+1]⁺.

¹H NMR (400 MHz, DMSO-*d*₆ ) δ = 11.72 - 10.96 (m, 1H), 10.69 (s, 1H), 9.66 (s, 1H), 9.47 (br s, 1H), 9.39 - 9.25 (m, 1H), 8.73 (br d, *J* = 19.5 Hz, 1H), 8.21 (br d, *J* = 8.1 Hz, 2H), 7.61 (t, *J* = 7.9 Hz, 1H), 7.52 (t, *J* = 7.9 Hz, 1H), 7.32 (d, *J* = 7.4 Hz, 1H), 7.25 - 7.17 (m, 2H), 4.80 - 4.68 (m, 2H), 4.65 (br s, 1H), 4.46 (br d, J = 21.6 Hz, 1H), 4.24 (br d, *J* = 3.9 Hz, 2H), 3.70 - 3.53 (m, 2H), 2.92 - 2.72 (m, 1H), 2.65 (br t, *J* = 5.1 Hz, 3H), 2.36 - 2.22 (m, 3H), 2.10 - 1.96 (m, 3H), 1.88 (br d, *J* = 5.5 Hz, 1H), 1.15 (dd, *J* = 6.1, 14.2 Hz, 2H)

### [Preparation Example 125]

### 125-1. Preparation of Compound 354, (S)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide

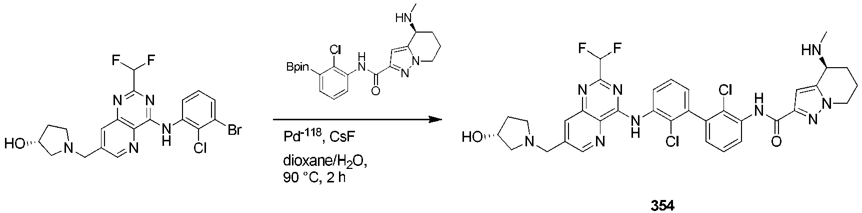

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), (4S)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (97.75 mg, 226.93 µmol, 1.1 eq), ditert-butyl(cyclopentyl)phosphane;dichloropalladium;iron (13.45 mg, 20.63 µmol, 0.1 eq), and CsF (94.01 mg, 618.91 µmol, 3 eq) were stirred in a mixture of dioxane (6 mL) /H₂O (1.5 mL) under nitrogen at 90 °C for 2 hours. The mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 15%-45% B over 20 min) to give compound 354 (32 mg, 19.9% yield) as a white solid.

LCMS m/z 708.2 [M+1]⁺.

¹H NMR (400 MHz, DMSO-*d₆* ) δ = 11.75 - 10.97 (m, 1H), 10.42 (s, 1H), 9.66 (s, 1H), 9.59 - 9.38 (m, 2H), 9.33 - 9.15 (m, 1H), 8.75 - 8.60 (m, 1H), 8.43 (br d, *J* = 7.8 Hz, 1H), 8.28 - 8.12 (m, 1H), 7.66 - 7.46 (m, 2H), 7.33 - 7.15 (m, 3H), 7.07 - 6.72 (m, 1H), 5.63 - 5.41 (m, 1H), 4.78 - 4.60 (m, 3H), 4.50 - 4.41 (m, 1H), 4.23 (br d, *J* = 4.4 Hz, 2H), 3.22 - 3.00 (m, 2H), 2.38 - 2.22 (m, 3H), 2.07 (s, 3H), 2.03 (br d, *J* = 4.6 Hz, 3H).

### 125-2. Preparation of Compound 355, (R)-N-(2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide

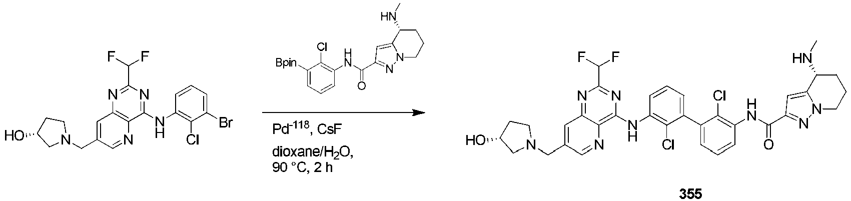

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), (4R)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (97.75 mg, 226.93 µmol, 1.1 eq), ditert-butyl(cyclopentyl)phosphane;dichloropalladium;iron (13.45 mg, 20.63 µmol, 0.1 eq), and CsF (94.01 mg, 618.91 µmol, 3 eq) were stirred in a mixture of dioxane (6 mL) /H₂O (1.5 mL) under nitrogen at 90 °C for 2 hours. The mixture was cooled to room temperature, filtered, and the filtrate was concentrated under reduced pressure. The residue was purified by prep-HPLC (column: Agela DuraShell C18 150 * 25 mm * 5 µm; mobile phase: [water (HCl)-ACN]; gradient: 15%-45% B over 20 min) to give compound 355 (28 mg, 17.6% yield) as a white solid.

LCMS m/z 708.2 [M+1]⁺.

¹H NMR (400 MHz, DMSO-d₆ ) δ = 11.82 - 11.06 (m, 1H), 10.41 (s, 1H), 9.65 (s, 1H), 9.59 - 9.43 (m, 2H), 9.18 (br d, *J* = 3.5 Hz, 1H), 8.79 - 8.62 (m, 1H), 8.44 (br d, *J* = 8.1 Hz, 1H), 8.20 (br d, *J* = 8.1 Hz, 1H), 7.56 (td, *J* = 7.9, 33.5 Hz, 2H), 7.31 - 7.18 (m, 3H), 6.88 (t, *J* = 54.3 Hz, 1H), 5.66 - 5.41 (m, 1H), 4.80 - 4.58 (m, 3H), 4.49 - 4.32 (m, 2H), 4.23 (br d, *J* = 4.4 Hz, 2H), 2.64 (br s, 3H), 2.35 - 2.23 (m, 3H), 2.12 - 2.00 (m, 3H), 1.95 - 1.75 (m, 1H).

### [Preparation Example 126]

### Preparation of Compound 356, (S)-N-(2,2'-dichloro-3'-((2-(trifluoromethyl)pyrido[3,2-d]pyrimid in-4-yl)amino)-[1,1'-biphenyl]-3-yl)-4-(methylamino)-4,5,6,7-tetrahydropyrazolo[1,5-a]pyridine-2-carboxamide, and Compound 357, (R)-N-(2,2'-dichloro-3'-((2-(trifluoromethyl)pyrido[3,2-d]pyrimidin-4-yl)amino)-[1,1'-biphenyl]-3-yl)-4-(methylamino)-4,5,6,7-tetrahydropyrazolo[1,5-a]pyridine-2-carboxamide

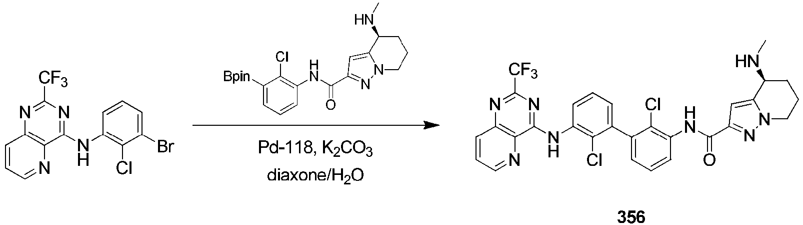

N-(3-bromo-2-chloro-phenyl)-2-(trifluoromethyl)pyrido[3,2-d]pyrimidin-4-amine (100 mg, 247.78 µmol, 1 eq), (4S)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (128.07 mg, 297.34 µmol, 1.2 eq), ditert-butyl(cyclopenta-1,4-dien-1-yl)phosphane;dichloropalladium;iron (16.15 mg, 24.78 µmol, 0.1 eq), and K₂CO₃ (102.74 mg, 743.34 µmol, 3 eq) were stirred in a mixture of dioxane (10 mL) / H₂O (2 mL) under nitrogen at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 0/1) and further purified by reversed-phase HPLC (column: Waters Xbridge 150 * 25 mm * 5 µm; mobile phase: [water (NH₄HCO₃)-ACN]; gradient: 55%-85% B over 10 min) to give compound 356 (16 mg, 9.88% yield) as a yellow solid.

LCMS m/z 627.3 [M+1]⁺.

¹H NMR (400 MHz, DMSO-*d*₆) δ ppm 1.62 - 1.73 (m, 1 H) 1.87 - 1.97 (m, 1 H) 1.97 - 2.06 (m, 1 H) 2.14 - 2.22 (m, 1 H) 2.33 (s, 3 H) 3.75 - 3.80 (m, 1 H) 4.06 - 4.24 (m, 2 H) 6.79 (s, 1 H) 7.13 - 7.24 (m, 1 H) 7.24 - 7.34 (m, 1 H) 7.51 (t, *J*=7.94 Hz, 1 H) 7.61 (t, *J*=7.88 Hz, 1 H) 8.08 (dd, *J*=8.51, 4.25 Hz, 1 H) 8.30 (br d, *J*=8.25 Hz, 1 H) 8.38 (d, *J*=8.13 Hz, 1 H) 8.46 (d, *J*=8.73 Hz, 1 H) 9.08 - 9.15 (m, 1 H) 9.55 (s, 1 H).

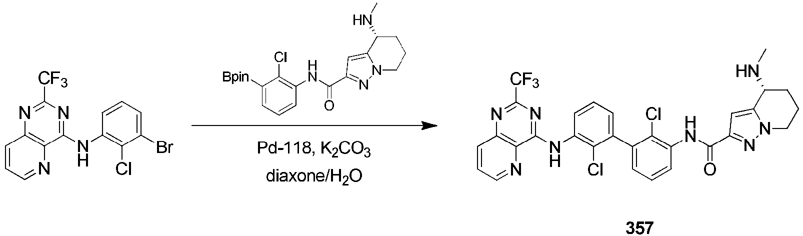

N-(3-bromo-2-chloro-phenyl)-2-(trifluoromethyl)pyrido[3,2-d]pyrimidin-4-amine (100 mg, 247.78 µmol, 1 eq), (4R)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (128.07 mg, 297.34 µmol, 1.2 eq), ditert-butyl(cyclopenta-1,4-dien-1-yl)phosphane;dichloropalladium;iron (16.15 mg, 24.78 µmol, 0.1 eq), and K₂CO₃ (102.74 mg, 743.34 µmol, 3 eq) were stirred in a mixture of dioxane (10 mL) / H₂O (2 mL) under nitrogen at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, Ethyl acetate/Methanol = 1/0 to 0/1) and further purified by reversed-phase HPLC (column: Waters Xbridge 150 * 25 mm * 5 µm; mobile phase: [water (NH₄HCO₃)-ACN]; gradient: 55%-85% B over 10 min) to give compound 357 (24 mg, 14.6% yield) as a yellow solid.

LCMS m/z 627.3 [M+1]⁺.

¹H NMR (400 MHz, DMSO-*d*₆) δ ppm 1.62 - 1.74 (m, 1 H) 1.91 (br dd, *J*=14.01, 7.38 Hz, 1 H) 1.97 - 2.06 (m, 1 H) 2.13 - 2.23 (m, 1 H) 2.33 (s, 3 H) 3.74 - 3.81 (m, 1 H) 4.06 - 4.24 (m, 2 H) 6.79 (s, 1 H) 7.19 (dd, *J*=7.57, 1.31 Hz, 1 H) 7.29 (d, *J*=7.28 Hz, 1 H) 7.51 (t, *J*=7.94 Hz, 1 H) 7.61 (t, *J*=7.94 Hz, 1 H) 8.08 (dd, *J*=8.50, 4.25 Hz, 1 H) 8.30 (d, *J*=8.26 Hz, 1 H) 8.38 (dd, *J*=8.13, 1.25 Hz, 1 H) 8.46 (dd, *J*=8.44, 1.19 Hz, 1 H) 9.12 (dd, *J*=4.19, 1.19 Hz, 1 H) 9.55 (s, 1 H).

### [Preparation Example 127]

### Preparation of Compound 358, isopropyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate, and Compound 359, tert-butyl 2-(((S)-2-((2,2'-dichloro-3'-((2-(difluoromethyl)-7-(((R)-3-hydroxypyrrolidin-1-yl)methyl)pyrido[3,2-d]pyrimidin-4-yl)amino)-[1,1'-biphenyl]-3-yl)carbamoyl)-4,5,6,7-tetrahydropyrazolo[1,5-a]pyridin-4-yl)amino)acetate

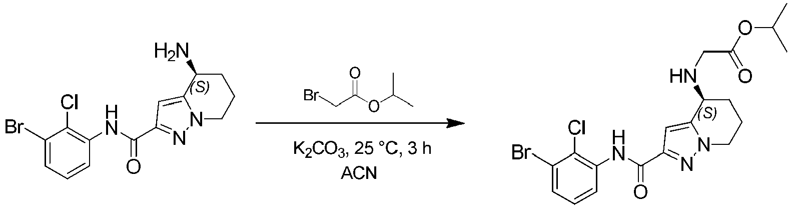

(4S)-4-amino-N-(3-bromo-2-chloro-phenyl)-4,5,6,7-tetrahydropyrazolo[1,5-a]pyridine-2-carboxamide (300 mg, 811.59 µmol, 1 eq) and isopropyl 2-bromoacetate (161.61 mg, 892.75 µmol, 115.52 µL, 1.1 eq) were stirred in acetonitrile (5 mL) with K₂CO₃ (336.51 mg, 2.43 mmol, 3 eq) at room temperature for 3 hours. Water (40 mL) was added and the mixture was extracted with ethyl acetate (40 mL * 3). The combined organic layers were washed with brine (60 mL), dried over Na2SO4, filtered, and concentrated under reduced pressure. The residue was purified by flash silica gel chromatography (Petroleum ether/Ethyl acetate = 100/1 to 2/1) to give isopropyl 2-[[(4S)-2-[(3-bromo-2-chlorophenyl)carbamoyl]-4,5,6,7-tetrahydropyrazolo[1,5-a]pyridin-4-yl]amino]acetate (260 mg, 61.3% yield) as a white solid.

¹H NMR (400 MHz, DMSO-*d*₆) δ = 9.65 (s, 1H), 8.08 (dd, *J* = 1.4, 8.3 Hz, 1H), 7.59 (dd, *J* = 1.4, 8.0 Hz, 1H), 7.33 (t, *J* = 8.1 Hz, 1H), 6.75 (s, 1H), 4.99 - 4.89 (m, 1H), 4.23 - 4.08 (m, 2H), 3.94 (br s, 1H), 3.40 - 3.35 (m, 2H), 2.28 - 2.15 (m, 1H), 2.03 - 1.85 (m, 2H), 1.77 - 1.65 (m, 1H), 1.20 (d, *J =* 6.3 Hz, 6H).

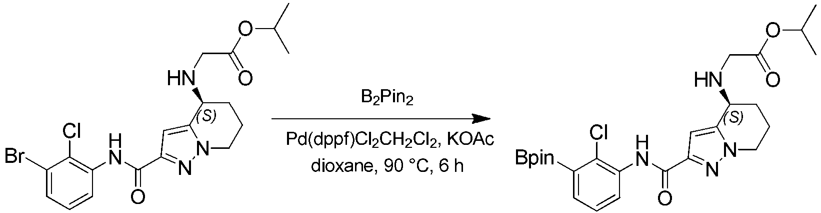

Isopropyl 2-[[(4S)-2-[(3-bromo-2-chloro-phenyl)carbamoyl]-4,5,6,7-tetrahydropyrazolo[1,5-a]pyridin-4-yl]amino]acetate (200 mg, 425.75 µmol, 1 eq), 4,4,5,5-tetramethyl-2-(4,4,5,5-tetramethyl-1,3,2-dioxaborolan-2-yl)-1,3,2-dioxaborolane (324.34 mg, 1.28 mmol, 3 eq), KOAc (125.35 mg, 1.28 mmol, 3 eq), and cyclopentyl(diphenyl)phosphane;dichloromethane;dichloropalladium;iron (34.77 mg, 42.58 µmol, 0.1 eq) were stirred in dioxane (8 mL) under nitrogen at 90 °C for 6 hours. The mixture was cooled to room temperature, filtered, and the filtrate was concentrated under reduced pressure. The residue was purified by flash silica gel chromatography (Petroleum ether/Ethyl acetate = 100/1 to 2/1) to give isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (239 mg, 86.8% yield) as a white solid.

LCMS m/z 517.3 [M+1]⁺

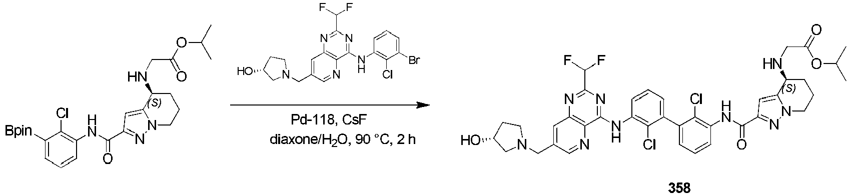

(3R)-1-[[4-(3-bromo-2-chloro-anilino)-2-(difluoromethyl)pyrido[3,2-d]pyrimidin-7-yl]methyl]pyrrolidin-3-ol (100 mg, 206.30 µmol, 1 eq), isopropyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (159.93 mg, 309.45 µmol, 1.5 eq), CsF (94.01 mg, 618.91 µmol, 22.85 µL, 3 eq), and ditert-butyl(cyclopentyl)phosphane;dichloropalladium;iron (13.45 mg, 20.63 µmol, 0.1 eq) were stirred in dioxane (5 mL)/water (1 mL) under nitrogen at 90 °C for 2 hours. The mixture was cooled to room temperature, filtered, and the filtrate was concentrated under reduced pressure. The residue was purified by prep-HPLC (column: Waters Xbridge 15025 *mm5* µm; mobile phase: [water (NH₄HCO₃)-ACN]; gradient: 50%-80% B over 9 min) to give compound 358 (9 mg, 5.27% yield) as a white solid.

LCMS m/z 794.3 [M+1]⁺

¹H NMR (400 MHz, METHANOL-*d₄*) δ = 8.95 - 8.90 (m, 2H), 8.39 (dd, *J* = 1.3, 8.3 Hz, 1H), 8.19 (s, 1H), 7.49 (t, *J* = 8.0 Hz, 1H), 7.42 (t, *J* = 7.9 Hz, 1H), 7.12 (dd, *J* = 1.6, 7.6 Hz, 2H), 6.99 - 6.90 (m, 1H), 6.83 (d, *J* = 1.8 Hz, 1H), 6.80 - 6.61 (m, 1H), 5.04 - 4.98 (m, 1H), 4.33 (br t, *J* = 6.6 Hz, 1H), 4.17 - 4.13 (m, 2H), 4.01 (br t, *J* = 5.9 Hz, 1H), 3.96 - 3.81 (m, 2H), 3.45 - 3.43 (m, 2H), 2.85 - 2.76 (m, 2H), 2.61 - 2.50 (m, 2H), 2.26 (br s, 1H), 2.16 - 2.04 (m, 2H), 1.96 (br dd, *J* = 6.7, 14.2 Hz, 1H), 1.83 - 1.70 (m, 2H), 1.23 (d, *J* = 6.3 Hz, 9H).

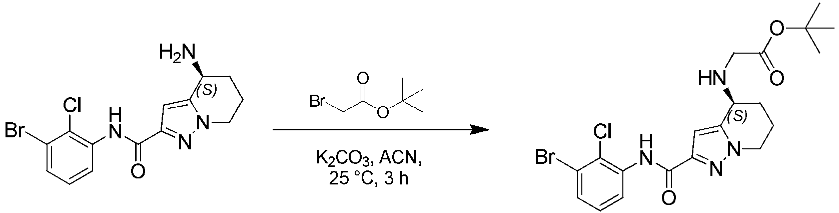

(4S)-4-amino-N-(3-bromo-2-chloro-phenyl)-4,5,6,7-tetrahydropyrazolo[1,5-a]pyridine-2-carboxamide (200 mg, 541.06 µmol, 1 eq) and tert-butyl 2-bromoacetate (116.09 mg, 595.17 µmol, 87.88 µL, 1.1 eq) were stirred in acetonitrile (5 mL) with K₂CO₃ (224.33 mg, 1.62 mmol, 3 eq) at room temperature for 3 hours. The mixture was filtered, and the filtrate was concentrated under reduced pressure. The residue was purified by prep-HPLC (column: Waters Xbridge 150 * 25mm* 5µm; mobile phase: [water (NH₄HCO₃)-ACN]; gradient: 58%-88% B over 9 min) to give tert-butyl 2-[[(4S)-2-[(3-bromo-2-chloro-phenyl)carbamoyl]-4,5,6,7-tetrahydropyrazolo[1,5-a]pyridin-4-yl]amino]acetate (220 mg, 79.8% yield) as a white solid.

LCMS m/z 967.2 [2M+1]⁺

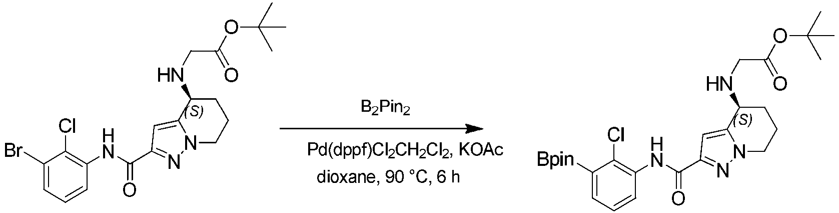

tert-butyl 2-[[(4S)-2-[(3-bromo-2-chloro-phenyl)carbamoyl]-4,5,6,7-tetrahydropyrazolo[1,5-a]pyridin-4-yl]amino]acetate (150 mg, 310.05 µmol, 1 eq), 4,4,5,5-tetramethyl-2-(4,4,5,5-tetramethyl-1,3,2-dioxaborolan-2-yl)-1,3,2-dioxaborolane (236.20 mg, 930.16 µmol, 3 eq), KOAc (91.29 mg, 930.16 µmol, 3 eq), and cyclopentyl(diphenyl)phosphane;dichloromethane;dichloropalladium;iron (25.32 mg, 31.01 µmol, 0.1 eq) were stirred in dioxane (8 mL) under nitrogen at 90 °C for 6 hours. The mixture was cooled to room temperature, filtered, and the filtrate was concentrated under reduced pressure. The residue was purified by prep-TLC (Petroleum ether/Ethyl acetate = 2/1) to give tert-butyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (152 mg, 73.8% yield) as a white solid.

LCMS m/z 531.3 [M+1]⁺

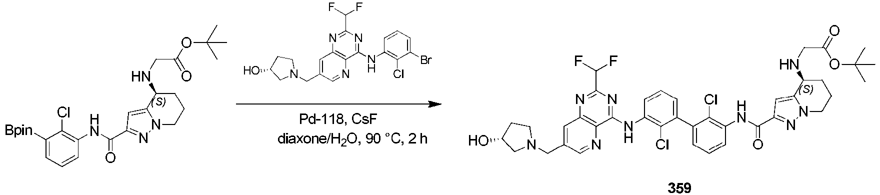

(3R)-1-[[4-(3-bromo-2-chloro-anilino)-2-(difluoromethyl)pyrido[3,2-d]pyrimidin-7-yl]methyl]pyrrolidin-3-ol (70 mg, 144.41 µmol, 1 eq), tert-butyl 2-[[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]acetate (91.99 mg, 173.29 µmol, 1.2 eq), CsF (65.81 mg, 433.24 µmol, 3 eq), and ditert-butyl(cyclopentyl)phosphane;dichloropalladium;iron (9.41 mg, 14.44 µmol, 0.1 eq) were stirred in dioxane (5 mL)/water (1 mL) under nitrogen at 90 °C for 2 hours. The mixture was cooled to room temperature, filtered, and the filtrate was concentrated under reduced pressure. The residue was purified by prep-HPLC (column: Waters Xbridge 150 * 25 mm * 5 µm; mobile phase: [water (NH₄HCO₃)/ACN]; gradient: 50%-80% B over 9 min) to give compound 359 (3 mg, 2.47% yield) as a white solid.

LCMS m/z 808.5 [M+1]⁺

¹H NMR (400 MHz, METHANOL-*d₄* ) δ = 9.00 (s, 1H), 8.98 (d, *J* = 8.4 Hz, 1H), 8.43 (d, *J* = 8.4 Hz, 1H), 8.25 (s, 1H), 7.54 (t, *J* = 7.9 Hz, 1H), 7.46 (t, *J* = 7.9 Hz, 1H), 7.17 (dd, J = 5.4, 6.8 Hz, 2H), 7.51 - 7.01 (m, 1H), 6.88 - 6.56 (m, 2H), 4.56 (br s, 4H), 4.41 - 4.35 (m, 1H), 4.24 - 4.15 (m, 2H), 4.07 - 4.02 (m, 1H), 4.02 - 3.89 (m, 2H), 3.41 (s, 2H), 2.84 (dd, *J* = 5.9, 9.8 Hz, 2H), 2.62 - 2.56 (m, 2H), 2.23 - 2.06 (m, 2H), 2.01 (br dd, *J* = 6.2, 13.7 Hz, 1H), 1.84 - 1.72 (m, 2H), 1.49 (s, 9H).

### [Preparation Example 128]

### Preparation of Compound 360, 2-(((S)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid, and Compound 361, 2-(((R)-2-((3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)carbamoyl)-4,5,6,7-tetrahydropyrazolo[1,5-a]pyridin-4-yl)amino)-2-methylpropanoic acid

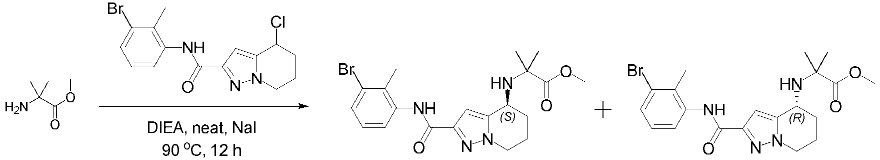

N-(3-bromo-2-methylphenyl)-4-chloro-4,5,6,7-tetrahydropyrazolo[1,5-a]pyridine-2-carboxamide (2 g, 5.43 mmol, 1 eq), methyl 2-amino-2-methylpropanoate (636.12 mg, 5.43 mmol, 1 eq), DIPEA (2.10 g, 16.28 mmol, 2.83 mL, 3 eq), and Nal (243.95 mg, 1.63 mmol, 0.3 eq) were stirred at 90 °C for 12 hours. The mixture was filtered, and the filtrate was concentrated under reduced pressure. The residue was purified by reversed-phase HPLC (column: Phenomenex Luna C18, 250 * 70 mm * 10 µm; mobile phase: [water (NH₄HCO₃)-ACN]; gradient: 30%-60% B over 20 min) to give compound 97-2 (1.55 g, 63.7% yield) as a yellow solid. Further separation by SFC (column: Phenomenex Cellulose-2, 250 * 30 mm * 10 µm; mobile phase: CO₂/EtOH (0.1% NH₃H₃O); B%: 55%, isocratic elution mode) afforded methyl (S)-2-((2-((3-bromo-2-methylphenyl)carbamoyl)-4,5,6,7-tetrahydropyrazolo[1,5-a]pyridin-4-yl)amino)-2-methylpropanoate (586 mg, 37.8% yield) and methyl (R)-2-((2-((3-bromo-2-methylphenyl)carbamoyl)-4,5,6,7-tetrahydropyrazolo[1,5-a]pyridin-4-yl)amino)-2-methylpropanoate (583 mg, 37.6% yield) as yellow liquids.

LCMS m/z 449.12 [M+1]⁺.

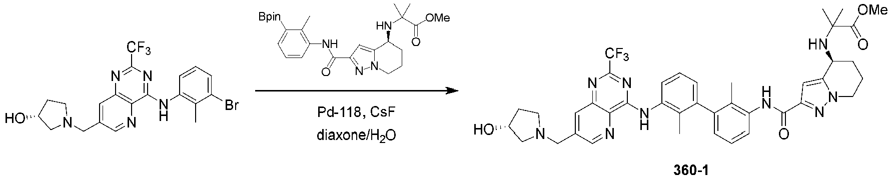

(3R)-1-[[4-(3-bromo-2-methyl-anilino)-2-(trifluoromethyl)pyrido[3,2-d]pyrimidin-7-yl]methyl]pyrrolidin-3-ol (100 mg, 207.34 µmol, 1 eq), methyl 2-methyl-2-[[(4S)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]propanoate (102.93 mg, 207.34 µmol, 1 eq), ditert-butyl(cyclopenta-1,4-dien-1-yl)phosphane;dichloropalladium;iron (13.51 mg, 20.73 µmol, 0.1 eq), and CsF (94.49 mg, 622.02 µmol, 3 eq) were stirred in dioxane (10 mL)/H₂O (2 mL) under N₂ at 90 °C for 12 hours. The mixture was cooled to room temperature, filtered, and the filtrate was concentrated under reduced pressure. The residue was purified by column chromatography (SiO₂, Petroleum ether/Ethyl acetate = 1/0 to 0/1, ethyl acetate/methanol = 1/0 to 0/1) to give compound 360-1 (250 mg, 93.7% yield) as a brown solid.

LCMS m/z 772.5 [M+1]⁺.

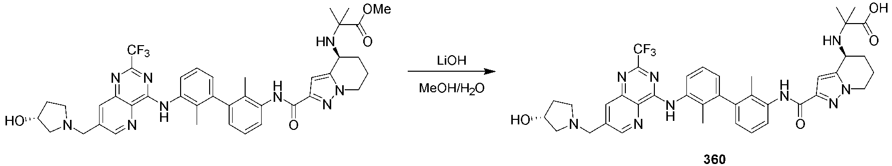

2-[[(4S)-2-[[3-[3-[[7-[[(3R)-3-hydroxypyrrolidin-1-yl]methyl]-2-(trifluoromethyl)pyrido[3,2-d]pyrimidin-4-yl]amino]-2-methyl-phenyl]-2-methyl-phenyl]carbamoyl]-4,5,6,7-tetrahydropyrazolo[1,5-a]pyridin-4-yl]amino]-2-methyl-propanoate (250 mg, 323.91 µmol, 1 eq) was dissolved in H₂O (3 mL)/MeOH (3 mL), and LiOH·H₂O (40.78 mg, 971.72 µmol, 3 eq) was added. The reaction mixture was stirred at room temperature for 12 hours. The mixture was concentrated under reduced pressure, and the residue was purified by reversed-phase HPLC (column: Waters Xbridge Prep OBD C18 150 * 40 mm * 10 µm; mobile phase: [water (NH₄HCO₃)-ACN]; gradient: 8%-38% B over 15 min) to give compound 360 (31 mg, 12.3% yield) as a white solid.

LCMS m/z 758.4 [M+1]⁺.

¹H NMR (400 MHz, DMSO-d6) δ ppm 1.64 (br d, J=11.38 Hz, 6 H) 1.92 (s, 3 H) 1.99 (s, 3 H) 2.00 - 2.11 (m, 2 H) 2.13 - 2.35 (m, 4 H) 4.14 - 4.21 (m, 1 H) 4.26 - 4.33 (m, 1 H) 4.46 (br s, 1 H) 4.67 - 4.82 (m, 3 H) 5.56 (br s, 1 H) 7.01 - 7.07 (m, 2 H) 7.11 (d, J=7.38 Hz, 1 H) 7.31 (t, J=7.69 Hz, 1 H) 7.38 (t, J=7.69 Hz, 1 H) 7.48 - 7.55 (m, 2 H) 8.67 (br s, 1 H) 9.31 (br s, 1 H) 9.72 (s, 1 H) 10.84 (s, 1 H).

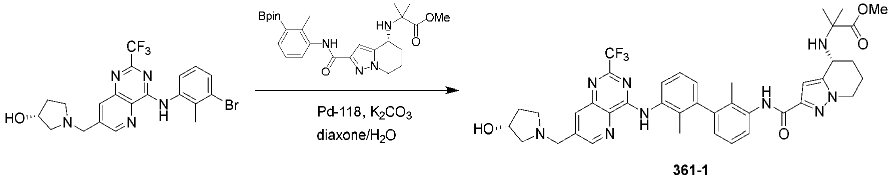

(3R)-1-[[4-(3-bromo-2-methyl-anilino)-2-(trifluoromethyl)pyrido[3,2-d]pyrimidin-7-yl]methyl]pyrrolidin-3-ol (200 mg, 414.68 µmol, 1 eq) and methyl 2-methyl-2-[[(4R)-2-[[2-methyl-3-(4,4,5,5-tetramethyl-1,3,2-dioxaborolan-2-yl)phenyl]carbamoyl]-4,5,6,7-tetrahydropyrazolo[1,5-a]pyridin-4-yl]amino]propanoate (377.39 mg, 456.15 µmol, 1.1 eq) were dissolved in dioxane (10 mL)/H₂O (2 mL) with ditert-butyl(cyclopenta-1,4-dien-1-yl)phosphane;dichloropalladium;iron (27.03 mg, 41.47 µmol, 0.1 eq) and K₂CO₃ (171.93 mg, 1.24 mmol, 3 eq) under N₂. The mixture was stirred at 90 °C for 12 hours. After cooling to room temperature, the mixture was filtered, concentrated under reduced pressure, and the residue was purified by column chromatography (SiO₂, Petroleum ether/Ethyl acetate = 1/0 to 0/1, Ethyl acetate/Methanol = 1/0 to 0/1) to give compound 361-1 (200 mg, 60.0% yield) as a brown solid.

LCMS m/z 772.7 [M+1]⁺.

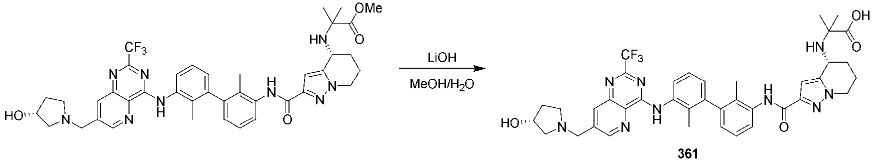

Compound 360-1 (200 mg, 259.12 µmol, 1 eq) was dissolved in H₂O (2 mL)/MeOH (10 mL), and LiOH·H₂O (32.62 mg, 777.37 µmol, 3 eq) was added. The reaction mixture was stirred at room temperature for 12 hours. After concentration under reduced pressure, the residue was purified by reversed-phase HPLC (column: Waters Xbridge Prep OBD C18 150 × 40 mm × 10 µm; mobile phase: [water (NH₄HCO₃) -ACN]; gradient: 8%-38% B over 15 min) to give compound 360 (93 mg, 46.9% yield) as a yellow solid.

LCMS m/z 758.5 [M+1]⁺.

¹H NMR (400 MHz, DMSO-d6) δ ppm 1.62 (br d, J=11.26 Hz, 6 H) 1.92 (s, 3 H) 1.98 (s, 3 H) 2.02 - 2.08 (m, 1 H) 2.18 (br s, 2 H) 2.24 - 2.39 (m, 2 H) 4.16 (br dd, J=12.88, 6.50 Hz, 1 H) 4.24 - 4.33 (m, 1 H) 4.46 (br s, 1 H) 4.71 (br s, 3 H) 5.56 (br s, 1 H) 7.04 (d, J=7.53 Hz, 2 H) 7.11 (d, J=7.00 Hz, 1 H) 7.31 (t, J=7.75 Hz, 1 H) 7.38 (t, J=7.69 Hz, 1 H) 7.52 (dd, J=7.69, 4.19 Hz, 2 H) 8.66 (br s, 1 H) 9.30 (br s, 1 H) 9.71 (s, 1 H) 10.85 (s, 1 H).

### [Preparation Example 129]

### Preparation of Compound 362, (S)-N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide, and Compound 363, (R)-N-(3'-((2-(difluoromethyl)-7-(((R)-3-hydroxypyrrolidin-1-yl)methyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide

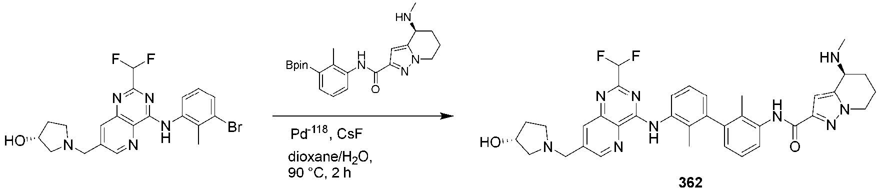

A mixture of (3R)-1-[[4-(3-bromo-2-methyl-anilino)-2-(difluoromethyl)pyrido[3,2-d]pyrimidin-7-yl]methyl]pyrrolidin-3-ol (80 mg, 172.30 µmol, 1 eq), (4S)-4-(methylamino)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (77.77 mg, 189.53 µmol, 1.1 eq), CsF (78.52 mg, 516.90 µmol, 3 eq), and ditert-butyl(cyclopentyl)phosphane;dichloropalladium;iron (11.23 mg, 17.23 µmol, 0.1 eq) in dioxane (6 mL)/H₂O (1.5 mL) was stirred under nitrogen at 90 °C for 2 hours. The mixture was cooled to room temperature, concentrated under reduced pressure, and the residue was purified by prep-HPLC (column: Waters Xbridge Prep OBD C18 150 * 40 mm * 10 µm; mobile phase: [water (NH₄HCO₃)-ACN]; gradient: 25%-55% B over 15 min) to afford Compound 362 (16 mg, 12.5% yield) as a white solid.

LCMS m/z 668.4 [M+1]⁺.

¹H NMR (400 MHz, DMSO-d6 ) δ = 11.52 - 10.93 (m, 1H), 10.57 (s, 1H), 9.71 (s, 1H), 9.40 (br d, J = 1.0 Hz, 2H), 9.27 - 9.14 (m, 1H), 8.59 (br d, J = 19.1 Hz, 1H), 7.59 (d, J = 8.0 Hz, 1H), 7.52 (br d, J = 7.9 Hz, 1H), 7.13 - 7.01 (m, 3H), 6.71 (t, J = 54.5 Hz, 1H), 4.78 - 4.57 (m, 3H), 4.45 (br d, J = 22.5 Hz, 1H), 4.22 (br s, 2H), 3.36 - 3.27 (m, 4H), 3.14 - 3.02 (m, 2H), 3.01 - 2.95 (m, 1H), 2.65 (br t, J = 5.0 Hz, 3H), 2.35 - 2.21 (m, 3H), 1.99 (s, 3H), 1.93 (s, 3H).

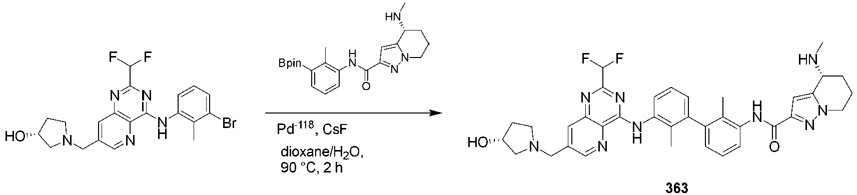

A mixture of (3R)-1-[[4-(3-bromo-2-methyl-anilino)-2-(difluoromethyl)pyrido[3,2-d]pyrimidin-7-yl]methyl]pyrrolidin-3-ol (70 mg, 150.76 µmol, 1 eq), (4R)-4-(methylamino)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (74.23 mg, 180.92 µmol, 1.2 eq), CsF (68.70 mg, 452.29 µmol), and ditert-butyl(cyclopentyl)phosphane;dichloropalladium;iron (9.83 mg, 15.08 µmol, 0.1 eq) in dioxane (6 mL)/H₂O (1.5 mL) was stirred under nitrogen at 90 °C for 2 hours. The reaction mixture was cooled to room temperature, concentrated under reduced pressure, and the residue was purified by prep-HPLC (column: Waters Xbridge Prep OBD C18 150 * 40 mm * 10 µm; mobile phase: [water (NH₄HCO₃)-ACN]; gradient: 25%-55% B over 15 min) to give compound 363 (23 mg, 22.2% yield) as a white solid.

LCMS m/z 668.4 [M+1]⁺

¹H NMR (400 MHz, DMSO-d6) δ = 10.38 (br s, 1H), 9.50 (s, 1H), 8.95 (d, J = 1.9 Hz, 1H), 8.21 - 8.11 (m, 1H), 7.65 (d, J = 7.1 Hz, 1H), 7.58 (d, J = 8.1 Hz, 1H), 7.35 (t, J = 7.8 Hz, 1H), 7.28 (t, J = 7.8 Hz, 1H), 7.06 (d, J = 6.9 Hz, 1H), 7.01 (d, J = 6.9 Hz, 1H), 6.87 - 6.54 (m, 2H), 4.76 (d, J = 4.6 Hz, 1H), 4.29 - 4.19 (m, 1H), 4.18 - 4.08 (m, 2H), 3.96 - 3.81 (m, 2H), 3.76 (dd, J = 5.3, 7.1 Hz, 1H), 3.28 - 3.18 (m, 2H), 2.80 - 2.60 (m, 3H), 2.32 (s, 3H), 2.22 - 2.13 (m, 1H), 2.08 - 2.02 (m, 2H), 1.99 (s, 3H), 1.94 (s, 3H), 1.92 - 1.85 (m, 1H), 1.73 - 1.55 (m, 2H).

### [Preparation Example 130]

### Preparation of Compound 364, 1-((S)-2-((2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)carbamoyl)-4,5,6,7-tetrahydropyrazolo[1,5-a]pyridin-4-yl)piperidine-4-carboxylic acid, and Compound 365, 1-((R)-2-((2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)carbamoyl)-4,5,6,7-tetrahydropyrazolo[1,5-a]pyridin-4-yl)piperidine-4-carboxylic acid

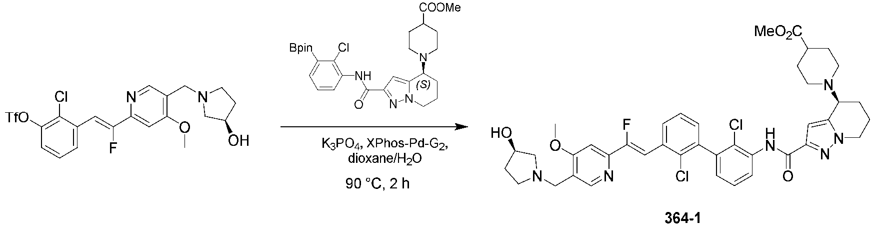

A mixture of [2-chloro-3-[(Z)-2-fluoro-2-[5-[[(3R)-3-hydroxypyrrolidin-1-yl]methyl]-4-methoxy-2-pyridyl]vinyl]phenyl] trifluoromethanesulfonate (100 mg, 195.74 µmol, 1 eq), methyl 1-[(4S)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (138.14 mg, 254.46 µmol, 1.3 eq), K₃PO₄ (124.65 mg, 587.22 µmol, 3 eq), and [2-(2-aminophenyl)phenyl]-chloro-palladium;dicyclohexyl-[3-(2,4,6-triisopropylphenyl)phenyl]phosphane (15.40 mg, 19.57 µmol, 0.1 eq) in dioxane (6 mL)/H₂O (1.5 mL) was stirred under nitrogen at 90 °C for 2 hours. The reaction mixture was cooled to room temperature, filtered, and concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to give compound 364-1 (126 mg, 74.5% yield) as a white solid.

LCMS m/z 779.4 [M+1]⁺

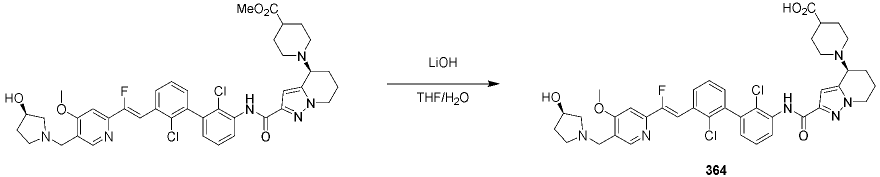

To a mixture of Compound 364-1 (100 mg, 128.58 µmol, 1 eq) in THF (6 mL)/H₂O (2 mL), LiOH·H₂O (16.19 mg, 385.75 µmol, 3 eq) was added at 0 °C, and the reaction mixture was stirred at room temperature for 12 hours. The reaction mixture was adjusted to pH 7 with 1 N HCl, and the precipitated solid was filtered. The solid was dried under vacuum and purified by prep-HPLC (column: Waters Xbridge 150 * 25 mm * 5 µm; mobile phase: [water (NH₄HCO₃)-ACN]; gradient: 18%-48% B over 10 min) to give compound 364 (12 mg, 11.6% yield) as a white solid.

LCMS m/z 765.3 [M+1]⁺

¹H NMR (400 MHz, DMSO-d6) δ = 9.74 - 9.45 (m, 1H), 8.44 (s, 1H), 8.26 (ddd, J = 1.4, 5.2, 8.1 Hz, 1H), 8.01 (dd, J = 1.4, 7.8 Hz, 1H), 7.59 - 7.50 (m, 2H), 7.46 (br d, J = 7.5 Hz, 1H), 7.39 - 7.30 (m, 2H), 7.28 - 7.13 (m, 1H), 6.67 (s, 1H), 4.70 (br d, J = 0.9 Hz, 1H), 4.45 - 4.32 (m, 3H), 4.24 - 4.16 (m, 2H), 4.05 (dt, J = 3.5, 12.0 Hz, 1H), 3.96 (s, 3H), 2.88 - 2.74 (m, 2H), 2.73 - 2.60 (m, 4H), 2.45 - 2.32 (m, 3H), 2.26 - 2.10 (m, 3H), 2.03 - 1.94 (m, 2H), 1.85 - 1.75 (m, 2H), 1.71 - 1.59 (m, 2H), 1.56 - 1.47 (m, 2H).

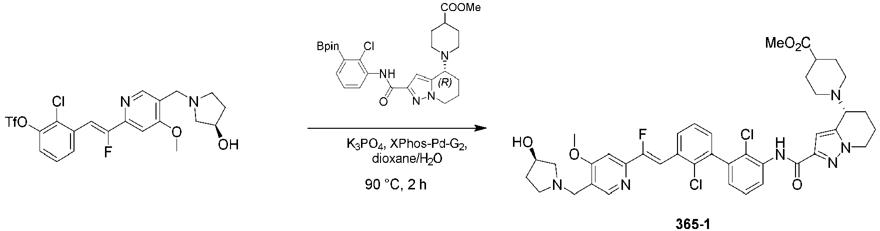

A mixture of [2-chloro-3-[(Z)-2-fluoro-2-[5-[[(3R)-3-hydroxypyrrolidin-1-yl]methyl]-4-methoxy-2-pyridyl]vinyl]phenyl] trifluoromethanesulfonate (100 mg, 195.74 µmol, 1 eq), methyl 1-[(4R)-2-[[2-chloro-3-(4,4,5,5-tetramethyl-1,3,2-dioxaborolan-2-yl)phenyl]carbamoyl]-4,5,6,7-tetrahydropyrazolo[1,5-a]pyridin-4-yl]piperidine-4-carboxylate (138.14 mg, 254.46 µmol, 1.3 eq), K₃PO₄ (124.65 mg, 587.22 µmol, 3 eq), and [2-(2-aminophenyl)phenyl]-chloro-palladium;dicyclohexyl-[3-(2,4,6-triisopropylphenyl)phenyl]phosphane (15.40 mg, 19.57 µmol, 0.1 eq) in dioxane (6 mL)/H₂O (1.5 mL) was stirred under nitrogen at 90 °C for 2 hours. The mixture was cooled to room temperature, filtered, and concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to afford Compound 365-1 (131 mg, 74.0% yield) as a white solid.

LCMS m/z 777.3 [M+1]⁺.

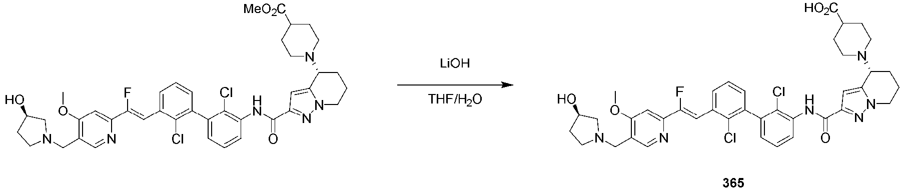

To a mixture of compound 365-1 (100 mg, 128.58 µmol, 1 eq) in THF (6 mL) / H₂O (2 mL), LiOH·H₂O (16.19 mg, 385.75 µmol, 3 eq) was added at 0 °C, and the reaction mixture was stirred at room temperature for 12 hours. The mixture was adjusted to pH 7 with 1 N HCl, and the precipitated solid was collected by filtration. The solid was dried under vacuum and further purified by prep-HPLC (column: Waters Xbridge 150 * 25 mm * 5 µm; mobile phase: [water (NH₄HCO₃)-ACN]; gradient: 18%-48% B over 10 min) to afford Compound 365 (16 mg, 15.8% yield) as a white solid.

LCMS m/z 763.1 [M+1]⁺.

¹H NMR (400 MHz, DMSO-d6) δ = 9.54 (d, J = 1.6 Hz, 1H), 8.44 (s, 1H), 8.25 (ddd, J = 1.4, 5.1, 8.2 Hz, 1H), 8.00 (dd, J = 1.3, 7.8 Hz, 1H), 7.58 - 7.49 (m, 2H), 7.49 - 7.44 (m, 1H), 7.40 - 7.31 (m, 2H), 7.17 (dd, J = 0.9, 7.6 Hz, 1H), 6.67 (d, J = 1.0 Hz, 1H), 4.73 (br d, J = 1.6 Hz, 1H), 4.30 - 4.14 (m, 2H), 4.12 - 3.98 (m, 2H), 3.96 (s, 3H), 3.74 - 3.56 (m, 3H), 2.87 - 2.73 (m, 2H), 2.67 (dt, J = 2.3, 3.9 Hz, 2H), 2.46 - 2.38 (m, 2H), 2.35 - 2.24 (m, 2H), 2.22 - 2.10 (m, 2H), 2.04 - 1.93 (m, 2H), 1.91 - 1.76 (m, 3H), 1.73 - 1.58 (m, 2H), 1.58 - 1.48 (m, 2H).

### [Preparation Example 131]

### Preparation of Compound 366, (S)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(methylamino)-4,5,6,7-tetrahydropyrazolo[1,5-a]pyridine-2-carboxamide, and Compound 367, (R)-N-(2,2'-dichloro-3'-((3-(((R)-3-hydroxypyrrolidin-1-yl)methyl)-1,7-naphthyridin-8-yl)amino)-[1,1'-biphenyl]-3-yl)-4-(methylamino)-4,5,6,7-tetrahydropyrazolo[1,5-a]pyridine-2-carboxamide

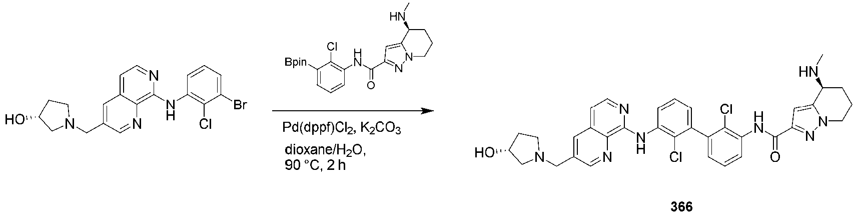

A mixture of (3R)-1-[[8-(3-bromo-2-chloro-anilino)-1,7-naphthyridin-3-yl]methyl]pyrrolidin-3-ol (80 mg, 184.45 µmol, 1 eq), (4S)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (87.39 mg, 202.89 µmol, 1.1 eq), ditert-butyl(cyclopentyl)phosphane;dichloropalladium;iron (12.02 mg, 18.44 µmol, 0.1 eq), and K₂CO₃ (76.47 mg, 553.34 µmol, 3 eq) in dioxane (6 mL) / H₂O (1.5 mL) was stirred under nitrogen at 90 °C for 2 hours. The reaction mixture was cooled to room temperature, filtered, and concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to afford Compound 366 (22 mg, 35.2% yield) as a white solid.

LCMS m/z 657.2 [M+1]⁺.

¹H NMR (400 MHz, DMSO-d6 ) δ = 9.89 (s, 1H), 9.54 (s, 1H), 9.13 (dd, J = 1.4, 8.4 Hz, 1H), 8.92 (d, J = 1.9 Hz, 1H), 8.26 (d, J = 1.8 Hz, 2H), 8.19 (d, J = 5.8 Hz, 1H), 7.51 (q, J = 8.4 Hz, 2H), 7.35 (d, J = 5.9 Hz, 1H), 7.19 (dd, J = 1.5, 7.6 Hz, 1H), 7.03 (dd, J = 1.4, 7.6 Hz, 1H), 6.78 (s, 1H), 4.77 - 4.68 (m, 1H), 4.27 - 4.10 (m, 3H), 3.89 - 3.73 (m, 3H), 2.71 - 2.62 (m, 2H), 2.32 (s, 3H), 2.17 (dt, J = 1.9, 5.8 Hz, 1H), 2.07 (s, 3H), 2.04 - 1.85 (m, 3H), 1.72 - 1.51 (m, 2H).

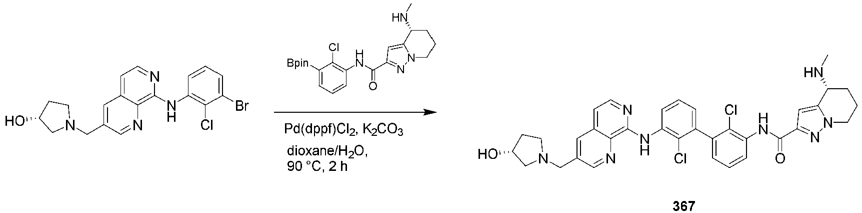

A mixture of (3R)-1-[[8-(3-bromo-2-chloro-anilino)-1,7-naphthyridin-3-yl]methyl]pyrrolidin-3-ol (80 mg, 184.45 µmol, 1 eq), (4R)-N-[2-chloro-3-(4,4,5,5-tetramethyl-1,3,2-dioxaborolan-2-yl)phenyl]-4-(methylamino)-4,5,6,7-tetrahydropyrazolo[1,5-a]pyridine-2-carboxamide (87.39 mg, 202.89 µmol, 1.1 eq), ditert-butyl(cyclopentyl)phosphane;dichloropalladium;iron (12.02 mg, 18.44 µmol, 0.1 eq), and K₂CO₃ (76.47 mg, 553.34 µmol, 3 eq) in dioxane (6 mL) / H₂O (1.5 mL) was stirred under nitrogen at 90 °C for 2 hours. The reaction mixture was cooled to room temperature, filtered, and concentrated under reduced pressure. The residue was purified by prep-HPLC (column: Agela DuraShell C18 150 * 25 mm * 5 µm; mobile phase: [water (HCl)-ACN]; gradient: 22%-52% B over 20 min) to afford Compound 367 (31 mg, 45.3% yield) as a white solid.

LCMS m/z 657.3 [M+1]⁺.

¹H NMR (400 MHz, DMSO-d6) δ = 9.88 (s, 1H), 9.54 (s, 1H), 9.13 (dd, J = 1.5, 8.4 Hz, 1H), 8.91 (d, J = 1.9 Hz, 1H), 8.34 - 8.23 (m, 2H), 8.19 (d, J = 5.8 Hz, 1H), 7.51 (q, J = 8.2 Hz, 2H), 7.34 (d, J = 5.9 Hz, 1H), 7.19 (dd, J = 1.5, 7.6 Hz, 1H), 7.02 (dd, J = 1.4, 7.6 Hz, 1H), 6.78 (s, 1H), 4.74 (d, J = 4.5 Hz, 1H), 4.28 - 4.06 (m, 3H), 3.91 - 3.74 (m, 3H), 2.78 - 2.63 (m, 2H), 2.38 (dd, J = 3.6, 9.5 Hz, 1H), 2.32 (s, 3H), 2.23 - 2.06 (m, 2H), 2.05 - 1.82 (m, 3H), 1.73 - 1.52 (m, 2H).

### [Preparation Example 132]

### Preparation of Compound 368, (S)-N-(3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide, and Compound 369, (R)-N-(3'-((7-(((R)-3-hydroxypyrrolidin-1-yl)methyl)-2-(trifluoromethyl)pyrido[3,2-d]pyrimidin-4-yl)amino)-2,2'-dimethyl-[1,1'-biphenyl]-3-yl)-4-(methylamino)-4,5,6,7-tetrahydropyrazolo[1,5-a]pyridine-2-carboxamide,

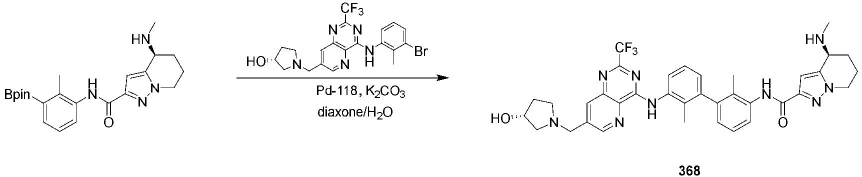

A mixture of (4S)-4-(methylamino)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (140.37 mg, 342.11 µmol, 1.1 eq), (3R)-1-[[4-(3-bromo-2-methyl-anilino)-2-(trifluoromethyl)pyrido[3,2-d]pyrimidin-7-yl]methyl]pyrrolidin-3-ol (150 mg, 311.01 µmol, 1 eq), K₂CO₃ (128.95 mg, 933.04 µmol, 3 eq), and ditert-butyl(cyclopentyl)phosphane;dichloropalladium;iron (20.27 mg, 31.10 µmol, 0.1 eq) in dioxane (8 mL)/H₂O (2 mL) was stirred under nitrogen at 80 °C for 4 hours. The reaction mixture was cooled to room temperature, filtered, and concentrated under reduced pressure. The residue was purified by reversed-phase HPLC (0.1% NH₃·H₂O condition; column: Waters Xbridge 150 * 25 mm * 5 µm; mobile phase: [water (NH₄HCO₃)-ACN]; gradient: 15%-45% B over 15 min) to afford Compound 368 (44 mg, 20.2% yield) as a yellow solid.

LCMS m/z 686.3 [M+1]⁺.

¹H NMR (400 MHz, DMSO-d6) δ ppm 1.55 - 1.72 (m, 2 H) 1.93 (s, 3 H) 1.99 (s, 3 H) 2.01 - 2.08 (m, 2 H) 2.15 - 2.23 (m, 1 H) 2.33 (s, 3 H) 2.42 (dd, J=9.63, 3.38 Hz, 1 H) 2.45 - 2.49 (m, 1 H) 2.66 - 2.77 (m, 2 H) 3.74 - 3.80 (m, 1 H) 3.88 (q, J=14.01 Hz, 2 H) 4.07 - 4.19 (m, 2 H) 4.24 (br s, 1 H) 4.80 (br s, 1 H) 6.73 (s, 1 H) 7.01 (d, J=6.75 Hz, 1 H) 7.09 (d, J=6.75 Hz, 1 H) 7.29 (t, J=7.75 Hz, 1 H) 7.37 (t, J=7.69 Hz, 1 H) 7.58 (br d, J=7.88 Hz, 2 H) 8.19 - 8.25 (m, 1 H) 9.00 (d, J=1.88 Hz, 1 H) 9.53 (s, 1 H) 10.65 (br s, 1 H).

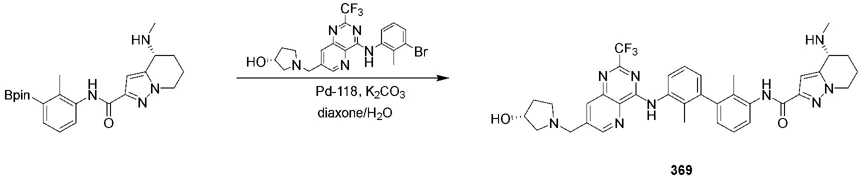

A mixture of (4R)-4-(methylamino)-N-[2-methyl-3-(4,4,5,5-tetramethyl-1,3,2-dioxaborolan-2-yl)phenyl]-4,5,6,7-tetrahydropyrazolo[1,5-a]pyridine-2-carboxamide (140.37 mg, 342.11 µmol, 1.1 eq), (3R)-1-[[4-(3-bromo-2-methyl-anilino)-2-(trifluoromethyl)pyrido[3,2-d]pyrimidin-7-yl]methyl]pyrrolidin-3-ol (150 mg, 311.01 µmol, 1 eq), K₂CO₃ (128.95 mg, 933.04 µmol, 3 eq), and ditert-butyl(cyclopentyl)phosphane;dichloropalladium;iron (20.27 mg, 31.10 µmol, 0.1 eq) in dioxane (8 mL)/H₂O (2 mL) was stirred under nitrogen at 80 °C for 4 hours. The reaction mixture was cooled to room temperature, filtered, and concentrated under reduced pressure. The residue was purified by reversed-phase HPLC (0.1% NH₃·H₂O condition; column: Waters Xbridge 150 * 25 mm * 5 µm; mobile phase: [water (NH₄HCO₃)-ACN]; gradient: 15%-45% B over 15 min) to afford Compound 369 (44 mg, 20.2% yield) as a yellow solid.

LCMS m/z 686.5 [M+1]⁺.

¹H NMR (400 MHz, DMSO-d6) δ ppm 1.55 - 1.73 (m, 2 H) 1.93 (s, 3 H) 1.99 (s, 3 H) 2.01 - 2.11 (m, 2 H) 2.15 - 2.24 (m, 1 H) 2.34 (s, 3 H) 2.42 (dd, J=9.63, 3.38 Hz, 1 H) 2.67 - 2.77 (m, 2 H) 3.74 - 3.81 (m, 1 H) 3.88 (q, J=14.05 Hz, 2 H) 4.06 - 4.20 (m, 2 H) 4.20 - 4.29 (m, 1 H) 4.78 (br d, J=3.63 Hz, 1 H) 6.73 (s, 1 H) 7.01 (d, J=7.00 Hz, 1 H) 7.09 (d, J=7.25 Hz, 1 H) 7.30 (t, J=7.75 Hz, 1 H) 7.37 (t, J=7.75 Hz, 1 H) 7.58 (dd, J=7.82, 2.44 Hz, 2 H) 8.19 - 8.26 (m, 1 H) 9.00 (d, J=1.75 Hz, 1 H) 9.53 (s, 1 H) 10.65 (br s, 1 H).

### [Preparation Example 133]

### Preparation of Compound 370, (R)-N-(2,2'-dichloro-3'-((Z)-2-fluoro-2-(5-(((R)-3-hydroxypyrrolidin-1-yl)methyl)-4-methoxypyridin-2-yl)vinyl)-[1,1'-biphenyl]-3-yl)-4-((R)-3-hydroxypyrrolidin-1-yl)-4,5,6,7-tetrahydropyrazolo[1,5-a]pyridine-2-carboxamide

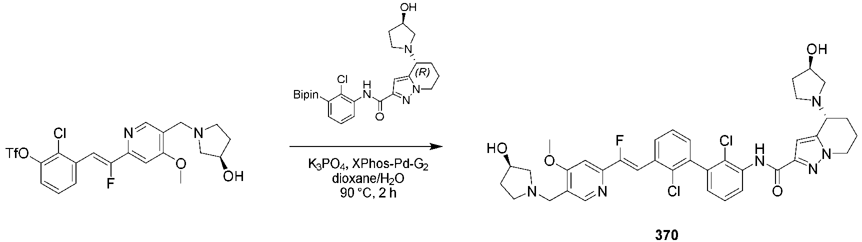

A mixture of [2-chloro-3-[(Z)-2-fluoro-2-[5-[[(3R)-3-hydroxypyrrolidin-1-yl]methyl]-4-methoxy-2-pyridyl]vinyl]phenyl] trifluoromethanesulfonate (100 mg, 195.74 µmol, 1 eq), (4R)-N-[2-chloro-3-(4,4,5,5-tetramethyl-1,3,2-dioxaborolan-2-yl)phenyl]-4-[(3R)-3-hydroxypyrrolidin-1-yl]-4,5,6,7-tetrahydropyrazolo[1,5-a]pyridine-2-carboxamide (104.81 mg, 215.31 µmol, 1.1 eq), K₃PO₄ (124.65 mg, 587.22 µmol, 3 eq), and [2-(2-aminophenyl)phenyl]-chloro-palladium;dicyclohexyl-[3-(2,4,6-triisopropylphenyl)phenyl]phosphane (15.40 mg, 19.57 µmol, 0.1 eq) in dioxane (6 mL)/H₂O (1.5 mL) was stirred under nitrogen at 90 °C for 2 hours. The reaction mixture was cooled to room temperature, filtered, and concentrated under reduced pressure. The residue was purified by prep-HPLC (column: Waters Xbridge Prep OBD C18 150 * 40 mm * 10 µm; mobile phase: [water (NH₄HCO₃)-ACN]; gradient: 40%-70% B over 15 min) to afford Compound 370 (32 mg, 20.4% yield) as a white solid.

LCMS m/z 721.4 [M+1]⁺.

¹H NMR (400 MHz, DMSO-d6) δ = 11.81 - 11.04 (m, 1H), 10.89 - 10.47 (m, 1H), 9.71 - 9.62 (m, 1H), 8.71 (d, J = 16.8 Hz, 1H), 8.23 - 8.15 (m, 1H), 8.00 (d, J = 7.9 Hz, 1H), 7.72 - 7.63 (m, 1H), 7.61 - 7.54 (m, 2H), 7.53 - 7.46 (m, 2H), 7.42 - 7.36 (m, 2H), 7.28 - 7.15 (m, 2H), 4.98 - 4.82 (m, 1H), 4.49 - 4.43 (m, 2H), 4.40 (br s, 2H), 4.24 (br dd, J = 6.1, 8.7 Hz, 2H), 3.57 (br dd, J = 6.0, 12.1 Hz, 2H), 3.54 - 3.46 (m, 2H), 3.44 - 3.34 (m, 1H), 3.32 - 3.25 (m, 2H), 3.09 - 3.01 (m, 1H), 2.39 (br d, J = 4.0 Hz, 1H), 2.35 - 2.25 (m, 2H), 2.21 - 2.12 (m, 1H), 2.08 - 1.97 (m, 2H), 1.92 (br s, 2H).

### [Example 1]

### Confirmation of PD-1/PD-L1 Binding Inhibitory Effect at the Molecular Level

In order to evaluate the inhibitory activity of the compounds synthesized according to the above Preparation Examples against PD-1/PD-L1 molecules in a free protein state,a TR-FRET (Time-Resolved Fluorescence Energy Transfer) experimetn was performed as described below.

Specifically, the compounds were prepared in a 96-well plate by serial dilution starting from 200 nM with 1/2 dilutions to obtain a total of twelve concentrations, and 2 µL of each compound solution was added to a 384-well plate. Thereafter, Tag1-PD-L1 protein and Tag2-PD-1 protein were each prepared at a 1x concentration, mixed at a ratio of 1:1, and 8 µL of the mixed solution was added to each well of the 384-well plate containing the compounds. Anti-Tag1 Eu Cryptate reagent and Anti-Tag2 XL665 antibody were each prepared at a 1* concentration, mixed at a ratio of 1:1, and 10 µL of the mixed solution was added to each well of the 384-well plate containing the compounds and proteins, followed by incubation at room temperature for 2 hours. Upon completion of the reaction, fluorescence signals at wavelengths of 620 nm and 665 nm were measured using a microplate reader.

As shown in Table 2 below, it was confirmed that the compounds of the present invention effectively inhibited the interaction between PD-1 and PD-L1 molecules in a free protein state at the nanomolar level.

**[Table 2]**

| Compound No. | FRET assay (IC₅₀) |
|---|---|
| | A ≤ 10 nM |
| | 10 nM < B ≤ 100 nM |
| | 100 nM < C ≤ 1 µM |
| 1 | A |
| 2 | A |
| 2-1 | A |
| 3 | A |
| 3-1 | A |
| 4 | A |
| 5 | A |
| 6 | A |
| 8 | A |
| 11 | A |
| 12 | A |
| 13 | A |
| 14 | A |
| 15 | A |
| 16 | A |
| 17 | A |
| 18 | B |
| 19 | B |
| 20 | B |
| 21 | B |
| 22 | A |
| 23 | B |
| 24 | B |
| 25 | B |
| 26 | A |
| 27 | B |
| 28 | A |
| 29 | A |
| 30 | A |
| 31 | A |
| 32 | A |
| 33 | A |
| 34 | B |
| 35 | A |
| 36 | B |
| 37 | A |
| 38 | B |
| 39 | A |
| 40 | B |
| 41 | A |
| 42 | A |
| 43 | B |
| 44 | B |
| 45 | A |
| 46 | A |
| 47 | A |
| 48 | A |
| 49 | A |
| 50 | A |
| 51 | A |
| 52 | A |
| 53 | A |
| 54 | B |
| 56 | A |
| 57 | A |
| 58 | B |
| 59 | A |
| 60 | A |
| 61 | A |
| 62 | A |
| 63 | A |
| 64 | A |
| 65 | A |
| 66 | A |
| 67 | A |
| 68 | A |
| 69 | A |
| 70 | A |
| 71 | A |
| 72 | A |
| 73 | A |
| 74 | A |
| 75 | A |
| 76 | A |
| 77 | A |
| 78 | A |
| 79 | A |
| 80 | A |
| 81 | A |
| 82 | A |
| 83 | A |
| 84 | A |
| 85 | A |
| 86 | A |
| 87 | B |
| 88 | A |
| 89 | A |
| 90 | A |
| 91 | A |
| 92 | A |
| 93 | A |
| 94 | A |
| 95 | A |
| 96 | A |
| 97 | A |
| 98 | A |
| 99 | A |
| 100 | A |
| 101 | B |
| 102 | B |
| 103 | A |
| 104 | A |
| 105 | A |
| 106 | A |
| 107 | B |
| 108 | A |
| 109 | A |
| 110 | A |
| 111 | A |
| 112 | A |
| 112-1 | A |
| 113 | A |
| 114 | A |
| 115 | A |
| 116 | B |
| 117 | B |
| 118 | B |
| 119 | B |
| 120 | A |
| 121 | A |
| 122 | A |
| 123 | A |
| 124 | A |
| 125 | A |
| 126 | B |
| 127 | B |
| 128 | A |
| 129 | A |
| 130 | B |
| 131 | A |
| 132 | A |
| 133 | A |
| 134 | B |
| 135 | B |
| 136 | A |
| 137 | A |
| 138 | B |
| 139 | B |
| 140 | B |
| 141 | B |
| 142 | A |
| 143 | A |
| 144 | B |
| 145 | A |
| 146 | A |
| 147 | A |
| 148 | A |
| 149 | A |
| 150 | A |
| 150-1 | A |
| 150-2 | A |
| 151 | B |
| 152 | B |
| 153 | A |
| 154 | B |
| 155 | B |
| 156 | C |
| 157 | D |
| 158 | B |
| 159 | B |
| 160 | B |
| 161 | B |
| 162 | B |
| 163 | B |
| 164 | B |
| 165 | A |
| 166 | B |
| 167 | B |
| 168 | A |
| 169 | A |
| 170 | B |
| 171 | A |
| 174 | A |
| 175 | A |
| 176 | B |
| 177 | A |
| 178 | A |
| 179 | A |
| 180 | A |
| 181 | A |
| 182 | B |
| 183 | A |
| 184 | A |
| 185 | A |
| 186 | A |
| 187 | A |
| 188 | A |
| 189 | A |
| 190 | A |
| 191 | A |
| 192 | A |
| 193 | A |
| 194 | A |
| 195 | A |
| 196 | A |
| 197 | B |
| 198 | A |
| 199 | A |
| 200 | A |
| 201 | A |
| 202 | A |
| 203 | A |
| 204 | A |
| 205 | A |
| 206 | A |
| 207 | A |
| 208 | A |
| 209 | A |
| 210 | A |
| 211 | A |
| 212 | A |
| 213 | A |
| 214 | A |
| 215 | A |
| 216 | A |
| 217 | A |
| 218 | A |
| 219 | A |
| 220 | A |
| 221 | A |
| 222 | A |
| 223 | B |
| 224 | A |
| 225 | B |
| 226 | B |
| 227 | B |
| 228 | B |
| 229 | B |
| 230 | B |
| 231 | B |
| 232 | B |
| 233 | B |
| 234 | B |
| 235 | B |
| 236 | B |
| 237 | B |
| 238 | B |
| 239 | B |
| 240 | B |
| 241 | A |
| 242 | A |
| 243 | A |
| 244 | A |
| 245 | A |
| 246 | A |
| 247 | A |
| 248 | A |
| 249 | B |
| 250 | B |
| 253 | A |
| 254 | B |
| 255 | B |
| 256 | B |
| 257 | B |
| 258 | B |
| 259 | B |
| 259-1 | B |
| 260 | B |
| 261 | A |
| 262 | B |
| 263 | A |
| 264 | A |
| 265 | A |
| 266 | A |
| 267 | A |
| 268 | B |
| 269 | B |
| 270 | B |
| 271 | B |
| 272 | B |
| 273 | B |
| 274 | B |
| 275 | B |
| 276 | A |
| 277 | B |
| 278 | B |
| 279 | B |
| 280 | A |
| 281 | A |
| 282 | A |
| 283 | B |
| 284 | B |
| 285 | B |
| 286 | B |
| 287 | B |
| 288 | B |
| 289 | A |
| 290 | B |
| 291 | B |
| 292 | A |
| 293 | B |
| 294 | B |
| 295 | B |
| 296 | B |
| 297 | C |
| 298 | B |
| 299 | B |
| 300 | B |
| 301 | C |
| 302 | B |
| 303 | B |
| 304 | B |
| 305 | B |
| 306 | B |
| 307 | A |
| 308 | A |
| 309 | A |
| 309-1 | B |
| 310 | A |
| 310-1 | A |
| 311 | A |
| 312 | A |
| 313 | A |
| 314 | A |
| 315 | A |
| 316 | A |
| 317 | A |
| 322 | B |
| 323 | B |
| 324 | A |
| 325 | A |
| 326 | A |
| 327 | A |
| 328 | A |
| 333 | B |
| 334 | B |
| 335 | B |
| 336 | B |
| 337 | B |
| 338 | B |
| 339 | B |
| 340 | B |
| 341 | B |
| 342 | B |
| 343 | B |
| 344 | B |
| 345 | B |
| 346 | A |
| 347 | A |
| 348 | A |
| 349 | B |
| 350 | B |
| 351 | B |
| 352 | B |
| 353 | B |
| 354 | B |
| 355 | B |
| 356 | B |
| 357 | B |

### [Example 2]

### Confirmation of Dimerization Activity of PD-L1 Molecules

In order to evaluate the dimerization of hPD-L1 molecules induced by the compounds of the present invention synthesized according to the above Preparation Examples, a TR-FRET (Time-Resolved Fluorescence Energy Transfer) experiment was performed as described below.

Specifically, the compounds were prepared in a 96-well plate by serial dilution starting from 10 µM with 1/3 dilutions to obtain a total of twelve concentrations. Thereafter, 2 µL of each compound solution was added to a 384-well plate. Fc tag-hPD-L1 protein and His tag-hPD-L1 protein were each prepared at a 1x concentration, mixed at a ratio of 1:1 of above two proteins, and 8 µL of the mixed protein solution was added to each well of the 384-well plate containing the compounds, followed by incubation at room temperature for one day. On the following day, Anti-6His-Tb Cryptate Gold antibody and Anti-human IgG-XL665 antibody were each prepared at a 1x concentration, mixed at a ratio of 1:1, and 10 µL of the mixed antibody solution was added to each well of the 384-well plate containing the compounds and proteins, followed by incubation at room temperature for 2 hours. Upon completion of the reaction, fluorescence signals at wavelengths of 620 nm and 665 nm were measured using a microplate reader.

As shown in Table 3 below, it was confirmed that Compounds 216, 218, and 227 effectively induced dimerization of hPD-L1 molecules at the nanomolar level.

**[Table 3]**

| Compound No. | Dimerization EC₅₀ (nM) |
|---|---|
| 216 | 25.2 |
| 218 | 6.1 |
| 227 | 26.2 |

### [Example 3]

### Confirmation of Internalization Activity of PD-L1 Expressed on the Cell Surface

In order to evaluate the internalization of cell surface PD-L1 induced by the compounds of the present invention synthesized according to the above Preparation Examples, a flow cytometry experiment using fluorescent staining was conducted as described below.

Specifically, a mouse colon cancer cell line expressing human PD-L1 (MC38 hPD-L1) was seeded into a 24-well plate at a density of 5 x 10⁵ cells per well, followed by treatment with Compounds 206 to 227 at a concentration of 1 µM and incubation for 4 hours. After 4 hours, the compound-treated MC38 hPD-L1 cells were collected and stained with PE anti-hPD-L1 fluorecence at room temperature for 30 minutes. The rate of PD-L1 expression on the cell surface was then quantified using flow cytometry.

As shown in Table 4 below, in the absence of treatment with the compounds of the present invention, PD-L1 expressed on the cell surface was observed at a level of 90% to 99%. In contrast, upon treatment with the compounds of the present invention, PD-L1 expressed on the cell surface was significantly reduced, and confirmed to be internalized into the cells.

**[Table 4]**

| Compound No. | Cell surface PD-L1 expression level (%) |
|---|---|
| 206 | 0.9 |
| 207 | 77.5 |
| 208 | 0.5 |
| 209 | 71.0 |
| 210 | 3.2 |
| 211 | 75.3 |
| 212 | 22.1 |
| 213 | 73.9 |
| 214 | 5.7 |
| 215 | 88.7 |
| 216 | 3.0 |
| 217 | 82.8 |
| 218 | 0.3 |
| 219 | 36.5 |
| 220 | 0.6 |
| 221 | 41.3 |
| 222 | 0.3 |
| 223 | 66.4 |
| 224 | 70.8 |
| 225 | 1.6 |
| 226 | 80.8 |
| 227 | 1.6 |

### [Example 4]

### Confirmation of PD-1/PD-L1 Binding Inhibitory Effect at the Cellular Level

In order to evaluate the effect of the compounds of the present invention, synthesized according to the above Preparation Examples, on blocking signal transduction between PD-1/PD-L1-expressing cells, a PD-1/PD-L1 blockade bioassay was conducted as described below.

Specifically, PD-L1 aAPC/CHO-K1 cells engineered to express human PD-L1 were seeded into a 96-well white plate and cultured for one day. On the following day, the compounds were prepared in a 96-well plate by serial dilution at concentrations starting from 10 µM and 5 µM, followed by 1/4 dilutions to obtain a total of ten concentrations. The culture medium in the plate seeded with the PD-L1 aAPC/CHO-K1 cell line was removed, and 40 µL of each prepared compound solution was added to a 96-well plate. Thereafter, 40 µL of PD-1 Effector T cell line engineered to express human PD-1 was additionally added, and then cultured for 6 hours. After 6 hours, 80 µL of Bio-Glo luciferase buffer was added to each well, followed by incubation at room temperature for 10 minutes, and luminescence was measured using a microplate reader.

As shown in Table 5 below, Compounds 206 to 227 were confirmed to effectively inhibit the interaction between PD-1/PD-L1 molecules at the nanomolar level even at the cellular level.

**[Table 5]**

| Compound No. | PD-1/PD-L1 Inhibitory Activity EC₅₀ (nM) |
|---|---|
| 206 | 15.7 |
| 207 | 85.8 |
| 208 | 12.6 |
| 210 | 16.0 |
| 212 | 32.3 |
| 214 | 40.1 |
| 216 | 31.6 |
| 217 | 251.2 |
| 218 | 19.2 |
| 220 | 13.6 |
| 222 | 34.8 |
| 227 | 20.2 |

### [Example 5]

### Confirmation of Tumor Growth Inhibitory Effect of Compound 218 in an Animal Model

In order to evaluate whether the tumor inhibitory effects of the compounds synthesized according to the above Preparation Examples are also effective *in vivo,* animal experiments were conducted as described below.

Specifically, to confirm the tumor growth inhibitory effect upon administration of Compound 218, tumor size was measured *in vivo.* A mouse colon cancer cell line expressing human PD-L1 (MC38 hPD-L1) was prepared at a density of 1.25 x 10⁶ cells in 100 µL of DPBS and inoculated subcutaneously into the flank of 6-week-old female C57BL/6 mice. When the average tumor volume reached approximately 50 mm³, the mice were divided into three experimental groups, with eight mice per group. Vehicle and Compound 218 (50 mg/kg) were administered intraperitoneally once daily, while Durvalumab (anti-PD-L1 mAb) was administered intraperitoneally twice per week. Tumor volume and body weight were measured every three days.

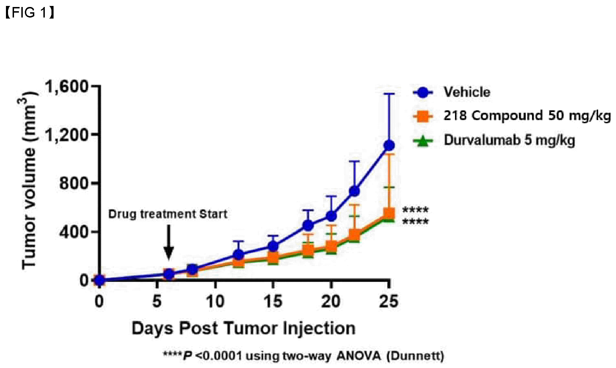

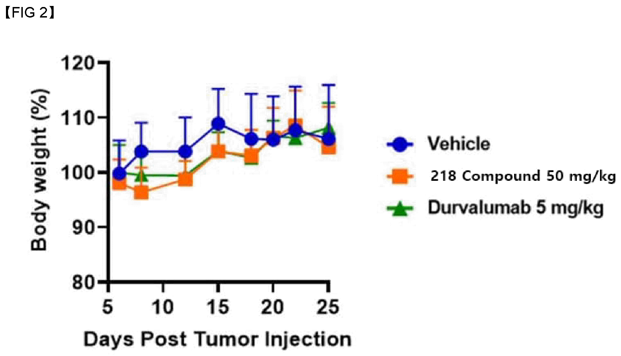

As shown in Figure 1, the group treated with Compound 218 exhibited a significant inhibition of tumor growth compared to the vehicle-treated group. In addition, as shown in Figure 2, no change in body weight of mice was observed, indicating the absence of detectable toxicity. Accordingly, it can be confirmed that Compound 218 of the present invention exhibits an excellent effect in inhibiting tumor growth in an animal model.

## Claims

1. A compound of Formula 1 below or a pharmaceutically acceptable salt thereof: 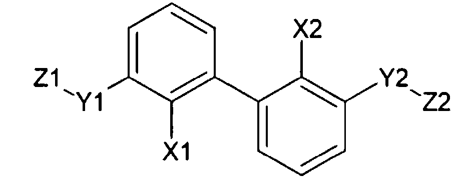 wherein,
X1 and X2 are each independently hydrogen, halo, haloalkyl, cyano, alkyl, alkenyl, alkynyl, - N(Ra)₂, -ORa, -SRa, cycloalkyl, heterocycloalkyl, aryl, or heteroaryl, wherein Ra is each independently hydrogen, halo, alkyl, alkenyl, alkynyl, cycloalkyl, heterocycloalkyl, aryl, or heteroaryl,
Y1 and Y2 are each independently a single bond, or alkyl, alkenyl, alkynyl, -ORb-, -N(Rb)-, - C(=O)Rb-, -C(=O)N(Rb)-, -C(=S)N(Rb)-, -N(Rb)C(=O)-, -N(Rb)S(=O)-, -N(Rb)S(=O)(=NH)-, - CH=C(Rb)-, -N(Rb)CH(OH)-, or -N(Rb)S(=O)₂-, wherein Rb is each independently hydrogen, halo, alkyl, alkenyl, alkynyl, cycloalkyl, heterocycloalkyl, aryl, or heteroaryl, and
Z1 and Z2 are each independently hydrogen, halo, cyano, alkyl, alkenyl, alkynyl, -C(=O)Rc, - C(=O)N(Rc)(Rd), -C(=O)ORc, -N(Rc)(Rd), -N(Rc)C(=O)Rd, -NH(Rc)C(=O)Rd, -NH(Rc)NHC(=O)Rd, - N(Rc)S(=O)Rd, -N(Rc)S(=O)₂Rd, -NO₂, -ORc, -OC(=O)Rc, -SRc, -S(=O)Rc, -S(=O)N(Rc)(Rd), - S(=O)₂Rc, -S(=O)₂N(Rc)(Rd), cycloalkyl, heterocycloalkyl, aryl, or heteroaryl, wherein Rc and Rd are each independently hydrogen, hydroxy, -COOH, alkyl, alkenyl, alkynyl, cycloalkyl, heterocycloalkyl, aryl, or heteroaryl,
wherein the alkyl, alkenyl, alkynyl, cycloalkyl, heterocycloalkyl, aryl, or heteroaryl may be unsubstituted or substituted.

2. The compound of Formula 1 or a pharmaceutically acceptable salt thereof according to Claim 1, **characterized in that**
X1 and X2 are each independently hydrogen, halo, C₁₋₆ alkyl, C₂₋₆ alkenyl, or C₂₋₆ alkynyl,
Y1 and Y2 are each independently a single bond, or C₁₋₆ alkyl, C₂₋₆ alkenyl, C₂₋₆ alkynyl, - ORb-, -N(Rb)-, -C(=O)Rb-, -C(=O)N(Rb)-, -N(Rb)C(=O)-, -N(Rb)S(=O)-, -N(Rb)S(=O)(=NH)-, - CH=C(Rb)-, -N(Rb)CH(OH)-, or -N(Rb)S(=O)₂-, wherein Rb is each independently hydrogen, halo, C₁₋₆ alkyl, C₂₋₆ alkenyl, or C₂₋₆ alkynyl, and
Z1 and Z2 are each independently hydrogen, C₁₋₆ alkyl, C₂₋₆ alkenyl, or C₂₋₆ alkynyl, - C(=O)N(Rc)(Rd), -C(=O)ORc, -N(Rc)(Rd), -N(Rc)C(=O)Rd, -N(Rc)S(=O)Rd, -N(Rc)S(=O)₂Rd, -NO₂, - ORc, -OC(=O)Rc, cycloalkyl, heterocycloalkyl, aryl, or heteroaryl, wherein Rc and Rd are each independently hydrogen, C₁₋₆ alkyl, C₂₋₆ alkenyl, or C₂₋₆ alkynyl, cycloalkyl, heterocycloalkyl, aryl, or heteroaryl,
wherein the alkyl, alkenyl, alkynyl, cycloalkyl, heterocycloalkyl, aryl, or heteroaryl may be unsubstituted or substituted.

3. The compound of Formula 1 or a pharmaceutically acceptable salt thereof according to Claim 2, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, C₁₋₆ alkyl, C₂₋₆ alkenyl, or C₂₋₆ alkynyl, and
Z1 and Z2 are each independently hydrogen; aryl; heterocycloalkyl substituted with one to three heteroatoms selected from the group consisting of N, O, and S; or heteroaryl substituted with one to three heteroatoms selected from the group consisting of N, O, and S,
wherein the aryl, heterocycloalkyl, or heteroaryl may be unsubstituted or substituted with one or more substituents selected from the group consisting of halo, -OH, -CN, -C₁₋₆ alkyl, -OC₁₋₆ alkyl, haloalkyl, -C₁₋₆ alkyl-OH, -C₁₋₆ alkyl-COOH, -C₁₋₆ alkyl-NH-C₁₋₆ alkyl-COOH, -C₁₋₆ alkyl-NH-C₁₋₆ alkyl-NH-COOH, -C₁₋₆ alkyl-NH-C₁₋₆ alkyl-NH-C(=O)-C₁₋₆ alkyl, -C₁₋₆ alkyl-NH-C₁₋₆ alkyl-OH, -NH-C₁₋₆ alkyl, - N(C₁₋₆ alkyl)(C₁₋₆ alkyl), -NH-C₁₋₆ alkyl-OH, -N(C₁₋₆ alkyl-OH)(C₁₋₆ alkyl-OH), -N(C₁₋₆ alkyl-COOH)(C₁₋₆ alkyl-COOH), -NH-C₁₋₆ alkyl-(C₁₋₆ alkyl-OH)₂, -NH-C₁₋₆ alkyl-COOH, -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, -NH-C₁₋₆ alkyl-NH-SO₂-C₁₋₆ alkyl, heterocycloalkyl, -C₁₋₆ alkyl-heterocycloalkyl, -C₁₋₆ alkyl-NH-C₁₋₆ alkyl-heterocycloalkyl, -C₁₋₆ alkyl-bicycloalkyl-COOH, -NH-heterocycloalkyl, -NH-C₁₋₆ alkyl-heterocycloalkyl, and -NH-cycloalkyl-OC₁₋₆ alkyl,
wherein the heterocycloalkyl and cycloalkyl may be unsubstituted or substituted with one or more substituents selected from the group consisting of halo, =O, -OH, -COOH, -C₁₋₆ alkyl-OH, and - C(=O)C₁₋₆ alkyl.

4. The compound of Formula 1 or a pharmaceutically acceptable salt thereof according to Claim 3, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 and Z2 are each independently hydrogen, . 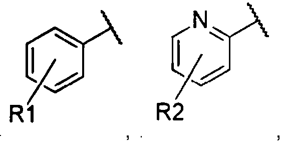 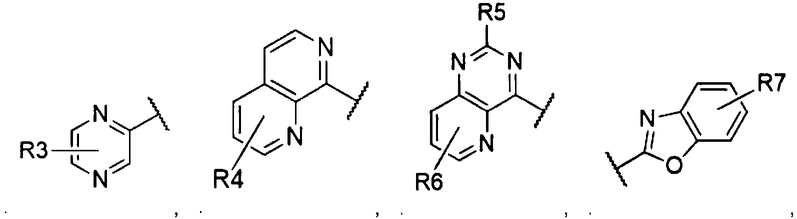 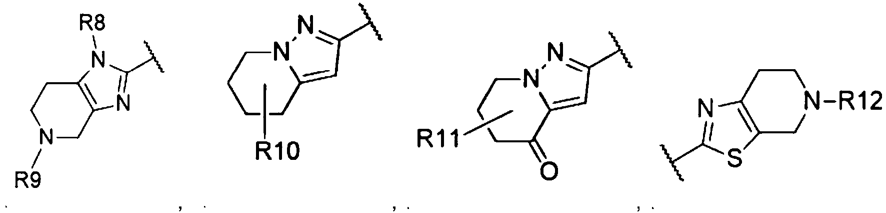 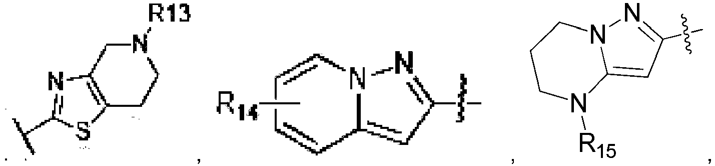 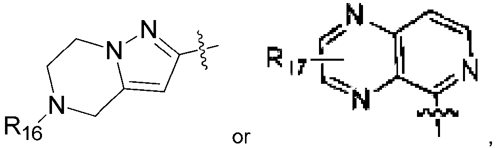
wherein R1 to R17 are each independently hydrogen, halo, -OH, -CN, -C₁₋₆ alkyl, -OC₁₋₆ alkyl, haloalkyl, -C₁₋₆ alkyl-OH, -C₁₋₆ alkyl-COOH, -C₁₋₆ alkyl-NH-C₁₋₆ alkyl-COOH, -C₁₋₆ alkyl-NH-C₁₋₆ alkyl-NH-COOH, -C₁₋₆ alkyl-NH-C₁₋₆ alkyl-NH-C(=O)-C₁₋₆ alkyl, -C₁₋₆ alkyl-NH-C₁₋₆ alkyl-OH, -NH-C₁₋₆ alkyl, -N(C₁₋₆ alkyl)(C₁₋₆ alkyl), -NH-C₁₋₆ alkyl-OH, -N(C₁₋₆ alkyl-OH)(C₁₋₆ alkyl-OH), -N(C₁₋₆ alkyl-COOH)(C₁₋₆ alkyl-COOH), -NH-C₁₋₆ alkyl-(C₁₋₆ alkyl-OH)₂, -NH-C₁₋₆ alkyl-COOH, -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, -NH-C₁₋₆ alkyl-NH-SO₂-C₁₋₆ alkyl, heterocycloalkyl, -C₁₋₆ alkyl-heterocycloalkyl, -C₁₋₆ alkyl-NH-C₁₋₆ alkyl-heterocycloalkyl, -C₁₋₆ alkyl-bicycloalkyl-COOH, -NH-heterocycloalkyl, -NH-C₁₋₆ alkyl-heterocycloalkyl, or -NH-cycloalkyl-OC₁₋₆ alkyl,
wherein the heterocycloalkyl and cycloalkyl may be unsubstituted or substituted with one or more substituents selected from the group consisting of halo, =O, -OH, -COOH, -C₁₋₆ alkyl-OH, and - C(=O)C₁₋₆ alkyl.

5. The compound of Formula 1 or a pharmaceutically acceptable salt thereof according to Claim 4, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 and Z2 are each independently hydrogen, 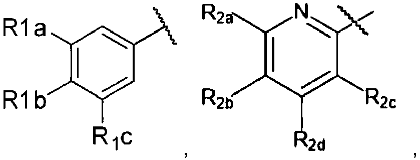 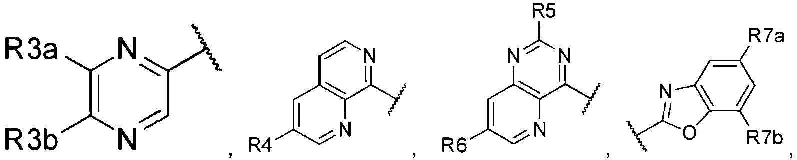 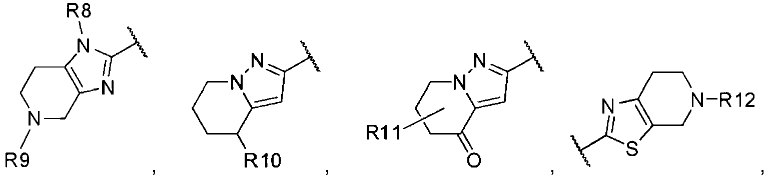 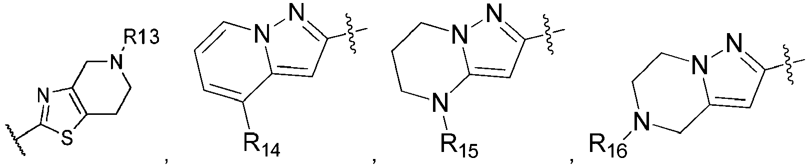 or 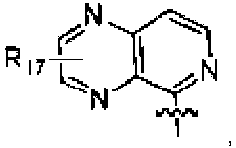
wherein R1a to R1c, R2a to R2d, R3a to R3b, R4 to R6, R7a to R7b, and R8 to R17 are each independently hydrogen, halo, -OH, -CN, -C₁₋₆ alkyl, haloalkyl, -C₁₋₆ alkyl-OH, -C₁₋₆ alkyl-COOH, -C₁₋₆ alkyl-NH-C₁₋₆ alkyl-COOH, -C₁₋₆ alkyl-NH-C₁₋₆ alkyl-NH-C(=O)-C₁₋₆ alkyl, -C₁₋₆ alkyl-NH-C₁₋₆ alkyl-OH, -NH-C₁₋₆ alkyl, -N(C₁₋₆ alkyl)(C₁₋₆ alkyl), -NH-C₁₋₆ alkyl-OH, -N(C₁₋₆ alkyl-OH)(C₁₋₆ alkyl-OH), -NH-C₁₋₆ alkyl-(C₁₋₆ alkyl-OH)₂, -NH-C₁₋₆ alkyl-COOH, -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, -NH-C₁₋₆ alkyl-NH-SO₂-C₁₋₆ alkyl, heterocycloalkyl, -C₁₋₆ alkyl-heterocycloalkyl, -C₁₋₆ alkyl-NH-C₁₋₆ alkyl-heterocycloalkyl, -C₁₋₆ alkyl-bicycloalkyl-COOH, -NH-heterocycloalkyl, -NH-C₁₋₆ alkyl-heterocycloalkyl, or -NH-cycloalkyl-OC₁₋₆ alkyl,
wherein the heterocycloalkyl is pyrrolidine or azetidine, which may be unsubstituted or substituted with one or more substituents selected from the group consisting of halo, =O, -OH, and - COOH,
wherein the cycloalkyl may be unsubstituted or substituted with one or more substituents selected from the group consisting of =O, -OH, -COOH, -C₁₋₆ alkyl-OH, and -C(=O)C₁₋₆ alkyl.

6. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 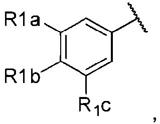
wherein R1a, R1b, and R1c are each independently hydrogen, halo, -C₁₋₆ alkyl, -OC₁₋₆ alkyl, -C₁₋₆ alkyl-NH-C₁₋₆ alkyl-heterocycloalkyl, -C₁₋₆ alkyl-heterocycloalkyl, -C₁₋₆ alkyl-NH-C₁₋₆ alkyl-COOH, -C₁₋₆ alkyl-NH-C₁₋₆ alkyl-NH-C(=O)-C₁₋₆ alkyl, or -C₁₋₆ alkyl-NH-C₁₋₆ alkyl-OH,
wherein the heterocycloalkyl is pyrrolidine, which may be unsubstituted or substituted with one or more substituents selected from the group consisting of =O, -OH, and -COOH.

7. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 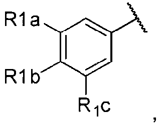
wherein R1a and R1c are each independently hydrogen, halo, -C₁₋₆ alkyl, or -OC₁₋₆ alkyl, and R1b is hydrogen, halo, -C₁₋₆ alkyl, -OC₁₋₆ alkyl, -C₁₋₆ alkyl-NH-C₁₋₆ alkyl-heterocycloalkyl, -C₁₋₆ alkyl-heterocycloalkyl, -C₁₋₆ alkyl-NH-C₁₋₆ alkyl-COOH, -C₁₋₆ alkyl-NH-C₁₋₆ alkyl-NH-C(=O)-C₁₋₆ alkyl, or -C₁₋₆ alkyl-NH-C₁₋₆ alkyl-OH,
wherein the heterocycloalkyl is

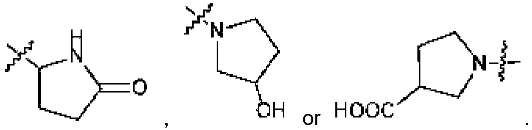

8. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 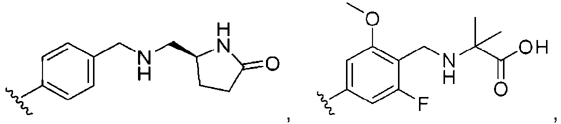 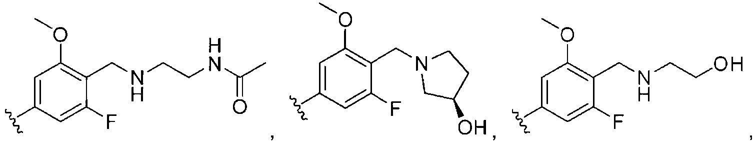 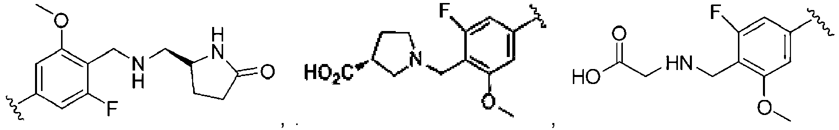 or

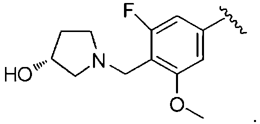

9. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 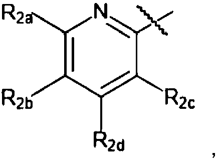
wherein R2a, R2b, R2c, and R2d are each independently hydrogen, halo, -C₁₋₆ alkyl, -OC₁₋₆ alkyl, -C₁₋₆ alkyl-heterocycloalkyl, or -C₁₋₆ alkyl-NH-C₁₋₆ alkyl-OH,
wherein the heterocycloalkyl is pyrrolidine which may be unsubstituted or substituted with OH.

10. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 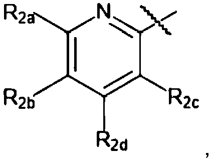
wherein R2a, R2b, R2c, and R2d are each independently hydrogen, halo, -C₁₋₆ alkyl, -OC₁₋₆ alkyl, -C₁₋₆ alkyl-heterocycloalkyl, or -C₁₋₆ alkyl-NH-C₁₋₆ alkyl-OH,
wherein the heterocycloalkyl is

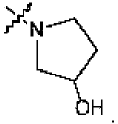

11. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

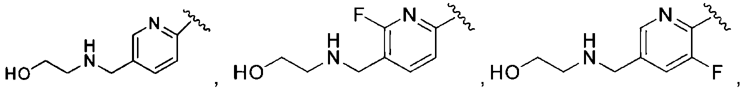

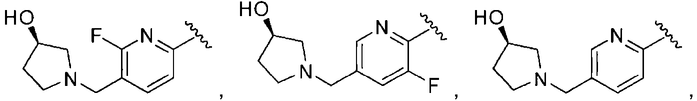

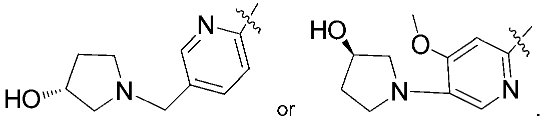

12. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 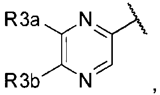
wherein R3a and R3b are each independently hydrogen, -OC₁₋₆ alkyl, or -C₁₋₆ alkyl-NH-C₁₋₆ alkyl-heterocycloalkyl, and
wherein the heterocycloalkyl is pyrrolidine which may be unsubstituted or substituted with =O.

13. The compound of Formula 1 or a pharmaceutically acceptable salt thereof according to Claim 5, **characterized in that**
X1 and X2 are each independently hydrogen, halogen, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halogen, or C1-6 alkyl, and
Z1 or Z2 is 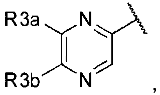
wherein R3a and R3b are each independently hydrogen, -OC₁₋₆ alkyl, or -C₁₋₆ alkyl-NH-C₁₋₆ alkyl-heterocycloalkyl,
wherein the heterocycloalkyl is

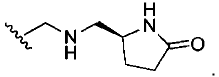

14. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is

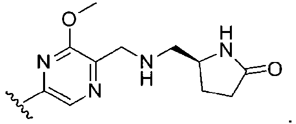

15. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 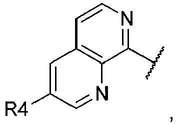
wherein R4 is hydrogen or -C₁₋₆ alkyl-heterocycloalkyl,
wherein the heterocycloalkyl is pyrrolidine or azetidine, which may be unsubstituted or substituted with -OH or halo.

16. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 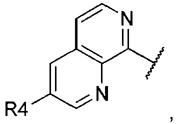
wherein R4 may be one or more selected from the group consisting of hydrogen or -C₁₋₆ alkyl-heterocycloalkyl,
wherein the heterocycloalkyl is

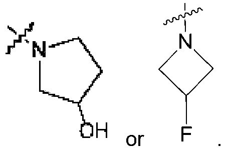

17. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 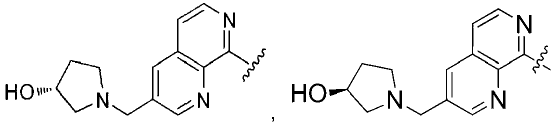 or

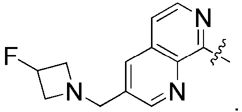

18. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 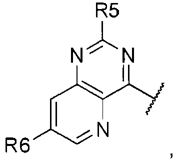
wherein R5 is hydrogen, -C₁₋₆ alkyl, or haloalkyl, and
R6 is hydrogen or C₁₋₆ alkyl-heterocycloalkyl,
wherein the heterocycloalkyl is pyrrolidine or azetidine, which may be unsubstituted or substituted with -OH or halo.

19. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 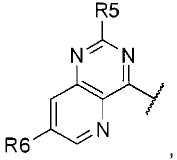
wherein R5 is hydrogen, -C₁₋₆ alkyl, or haloalkyl, and
R6 is hydrogen or -C₁₋₆ alkyl-heterocycloalkyl,
wherein the heterocycloalkyl is

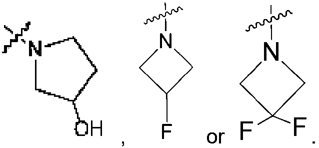

20. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein each Rb is independently hydrogen, halo, or C₁₋₆ alkyl, and
Z1 or Z2 is 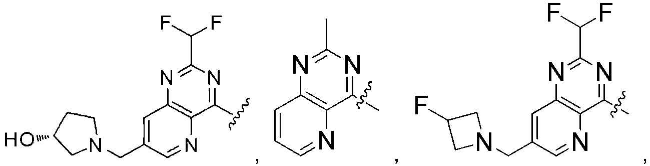 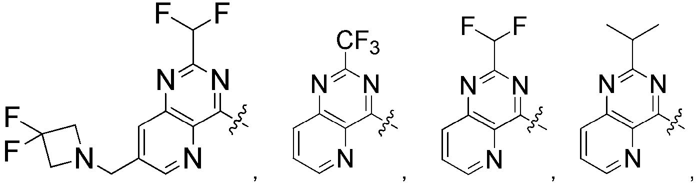 or

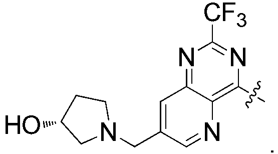

21. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 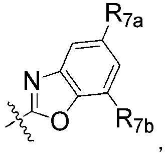
wherein R7a and R7b are each independently hydrogen, -CN, or -C₁₋₆ alkyl-heterocycloalkyl,
wherein the heterocycloalkyl is pyrrolidine which may be unsubstituted or substituted with - COOH.

22. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

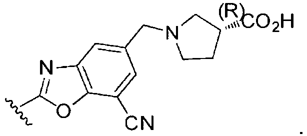

23. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 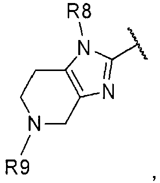
wherein R8 and R9 are each independently hydrogen, -C₁₋₆ alkyl, or -C₁₋₆ alkyl-bicycloalkyl-COOH.

24. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

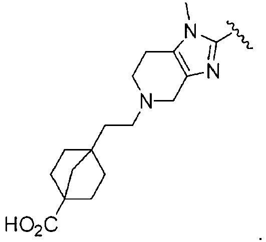

25. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alky,; and
Z1 or Z2 is 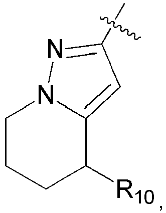
wherein R10 is hydrogen, halo, -OH, -C₁₋₆ alkyl, -NH-C₁₋₆ alkyl, -N(C₁₋₆ alkyl)(C₁₋₆ alkyl), -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, -OC₁₋₆ alkyl, heterocycloalkyl, -NH-heterocycloalkyl, -NH-C₁₋₆ alkyl-heterocycloalkyl, -NH-cycloalkyl, -NH-C₁₋₆ alkyl-COOH, -NH-C₁₋₆ alkyl-OH, -N(C₁₋₆ alkyl-OH)(C₁₋₆ alkyl-OH), -NH-C₁₋₆ alkyl-(C₁₋₆ alkyl-OH)₂, or -NH-C₁₋₆ alkyl-NH-SO₂-C₁₋₆ alkyl,
wherein the heterocycloalkyl and cycloalkyl may be unsubstituted or substituted with one or more substituents selected from the group consisting of =O, -OH, -COOH, -C₁₋₆ alkyl-OH, and - C(=O)C₁₋₆ alkyl.

26. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 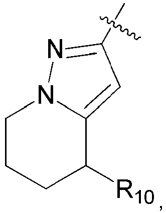
wherein R10 is hydrogen, -OH, -C₁₋₆ alkyl, -NH-C₁₋₆ alkyl, -N(C₁₋₆ alkyl)(C₁₋₆ alkyl), -NH-C₁₋₆ alkyl-NH-C(=O)C₁₋₆ alkyl, -NH-C₁₋₆ alkyl-C(=O)-OC₁₋₆ alkyl, -NH-C₁₋₆ alkyl-OC₁₋₆ alkyl, heterocycloalkyl, -NH-heterocycloalkyl, -NH-C₁₋₆ alkyl-heterocycloalkyl, -NH-cycloalkyl, -NH-C₁₋₆ alkyl-COOH, -NH-C₁₋₆ alkyl-OH, -N(C₁₋₆ alkyl-OH)(C₁₋₆ alkyl-OH), -NH-C₁₋₆ alkyl-(C₁₋₆ alkyl-OH)₂, or -NH-C₁₋₆ alkyl-NH-SO₂-C₁₋₆ alkyl, wherein the heterocycloalkyl and cycloalkyl are

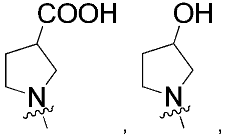

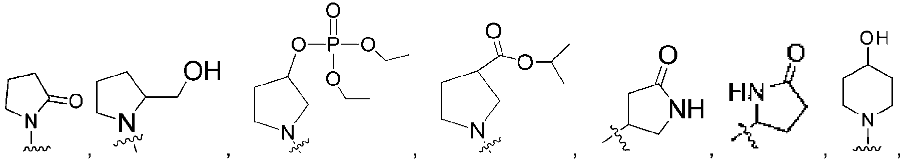

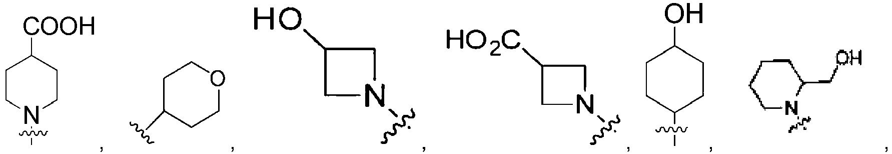

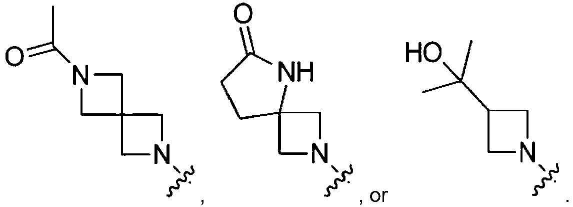

27. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 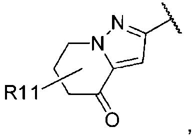
wherein R11 is hydrogen, halo, or -C₁₋₆ alkyl.

28. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

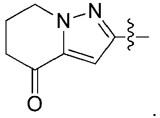

29. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 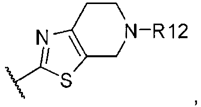
wherein R12 is hydrogen, or -C₁₋₆ alkyl which may be unsubstituted or substituted with halo.

30. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

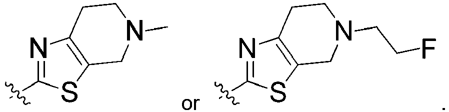

31. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 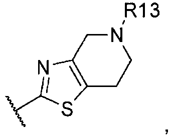
wherein R13 is hydrogen or -C₁₋₆ alkyl.

32. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

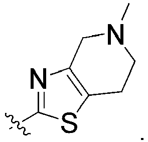

33. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 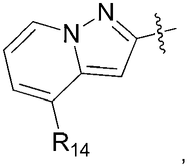
wherein R14 is hydrogen, -C₁₋₆ alkyl, or -NH(C₁₋₆ alkyl)COOH.

34. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

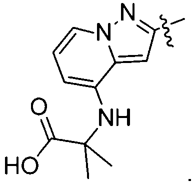

35. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 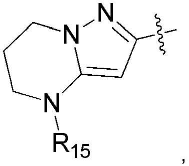
wherein R15 is hydrogen, -C₁₋₆ alkyl, or -C₁₋₆ alkyl-OH.

36. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

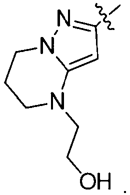

37. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 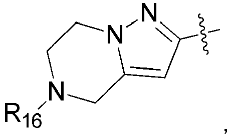
wherein R16 is hydrogen, -C₁₋₆ alkyl, -C₁₋₆ alkyl-OH, or -C₁₋₆ alkyl-COOH.

38. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

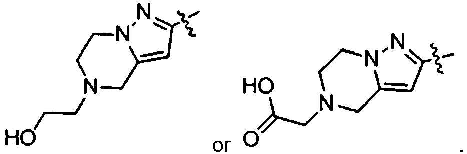

39. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is 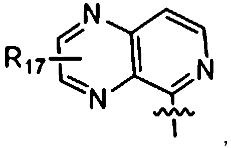
wherein R17 is hydrogen or -C₁₋₆ alkyl.

40. The compound of Formula 1 or a pharmaceutically acceptable salt thereof according to Claim 5, **characterized in that**
X1 and X2 are each independently hydrogen, halo, or C₁₋₆ alkyl,
Y1 and Y2 are each independently a single bond, or -N(Rb)-, -N(Rb)C(=O)-, -N(Rb)CH(OH)-, or -CH=C(Rb)-, wherein Rb is each independently hydrogen, halo, or C₁₋₆ alkyl, and
Z1 or Z2 is

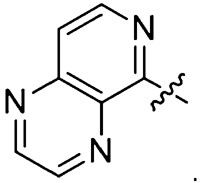

41. A compound selected from the group consisting of the following compounds, or a pharmaceutically acceptable salt thereof:
| No. | Structure of the compound |
|---|---|
| 1 | 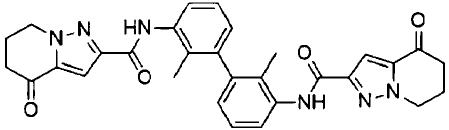 |
| 2 | 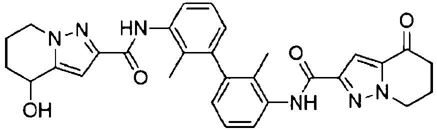 |
| 2-1 | 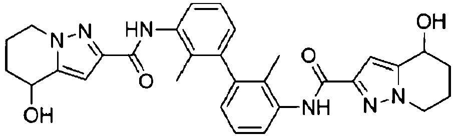 |
| 3 | 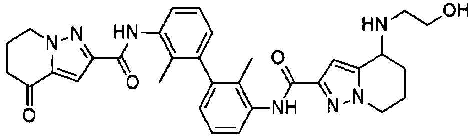 |
| 3-1 | 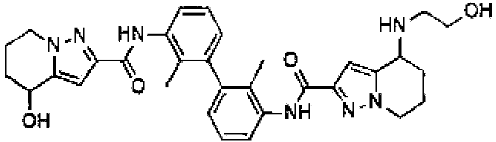 |
| 4 | 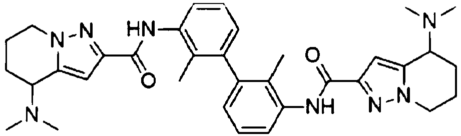 |
| 5 | 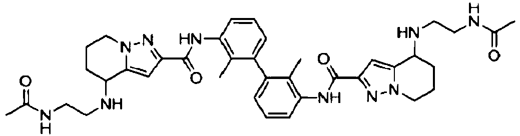 |
| 6 | 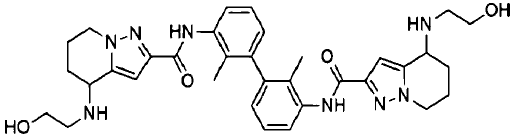 |
| 8 | 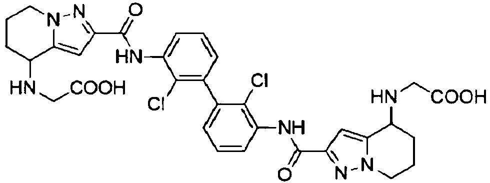 |
| 11 | 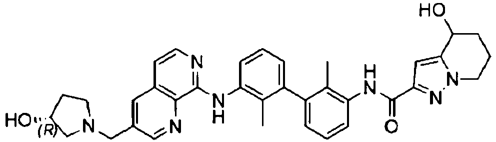 |
| 12 | 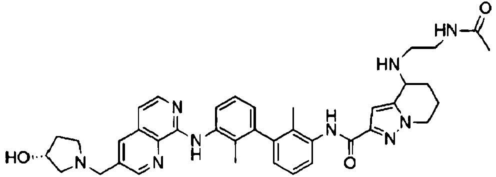 |
| 13 | 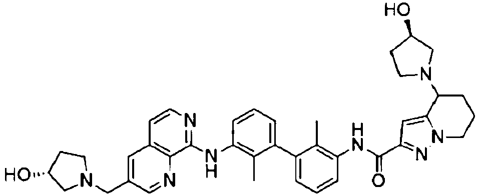 |
| 14 | 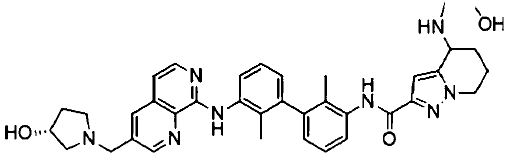 |
| 15 | 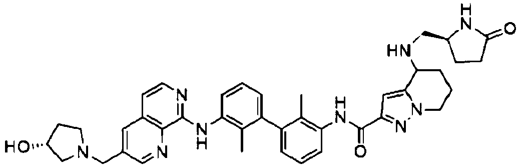 |
| 16 | 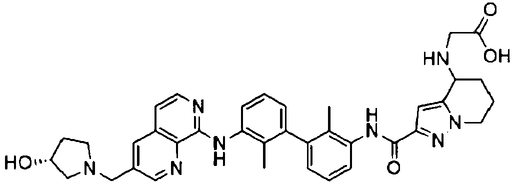 |
| 17 | 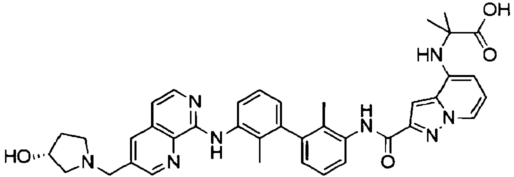 |
| 18 | 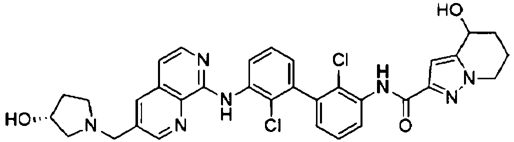 |
| 19 | 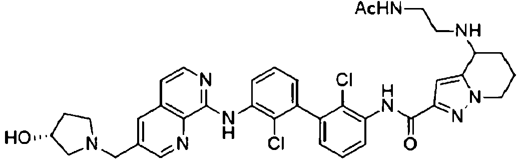 |
| 20 | 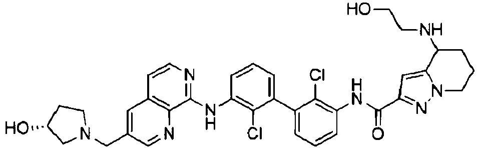 |
| 21 | 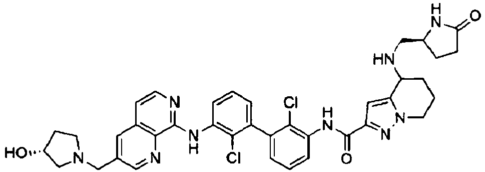 |
| 22 | 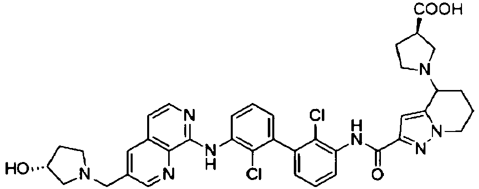 |
| 23 | 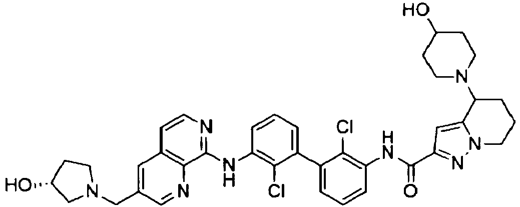 |
| 24 | 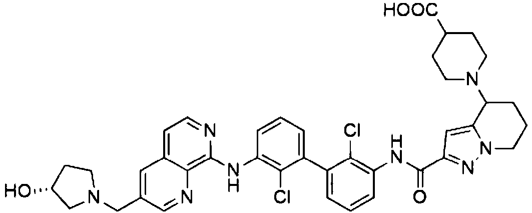 |
| 25 | 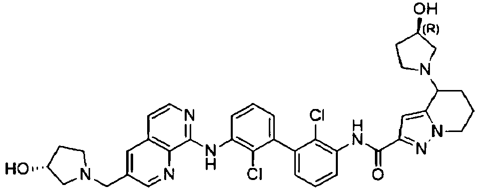 |
| 26 | 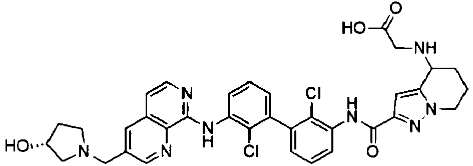 |
| 27 | 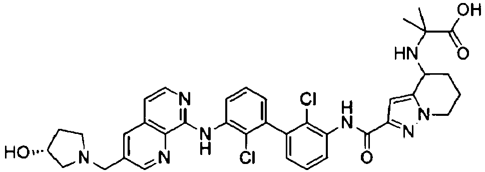 |
| 28 | 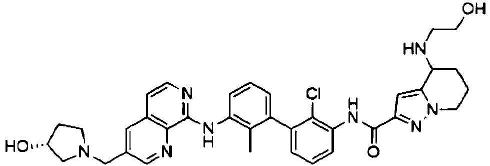 |
| 29 | 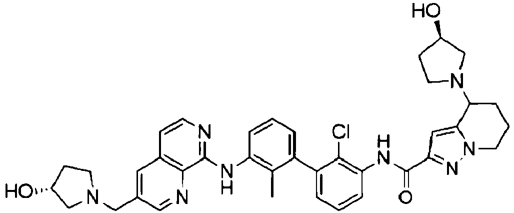 |
| 30 | 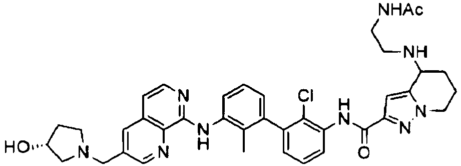 |
| 31 | 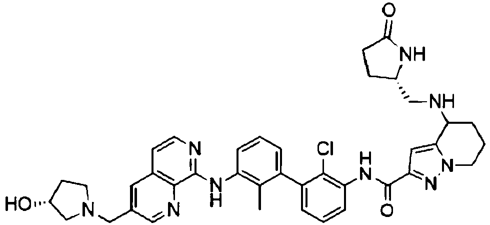 |
| 32 | 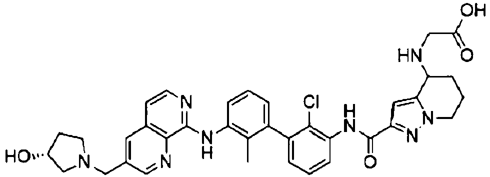 |
| 33 | 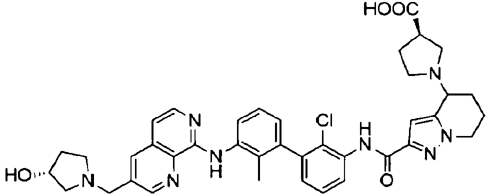 |
| 34 | 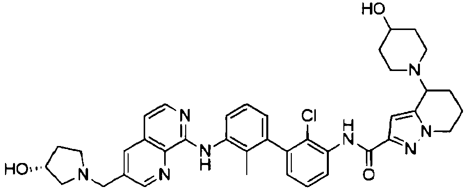 |
| 35 | 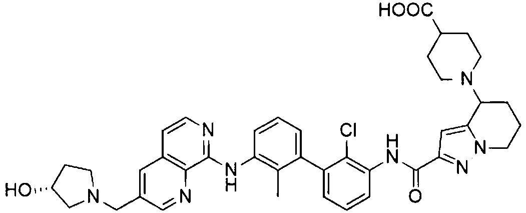 |
| 36 | 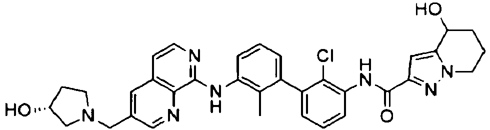 |
| 37 | 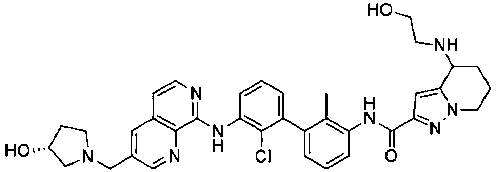 |
| 38 | 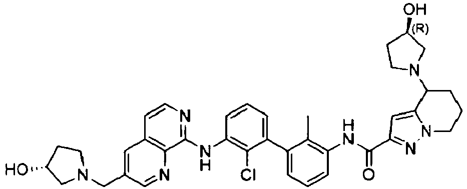 |
| 39 | 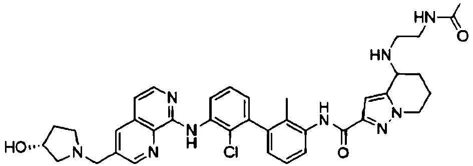 |
| 40 | 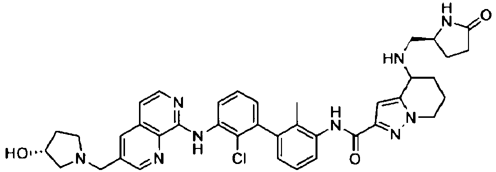 |
| 41 | 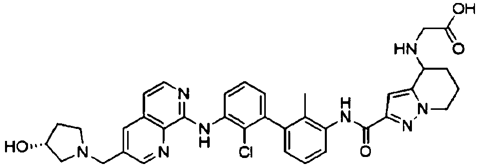 |
| 42 | 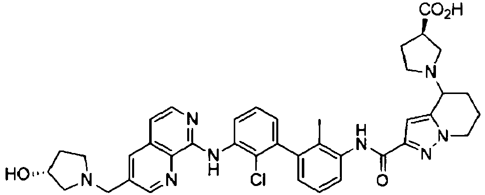 |
| 43 | 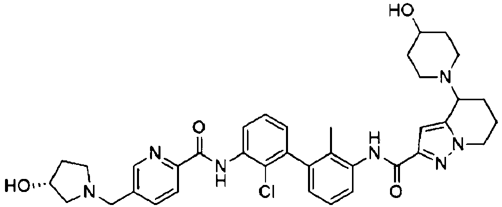 |
| 44 | 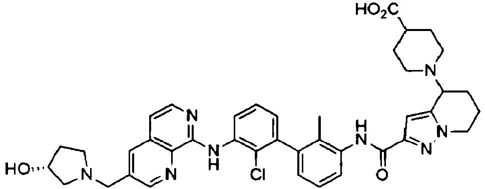 |
| 45 | 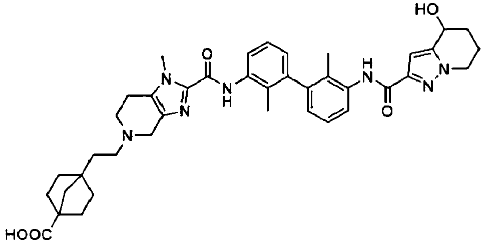 |
| 46 | 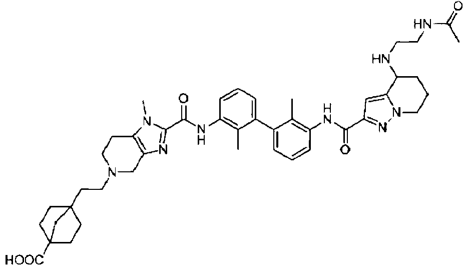 |
| 47 | 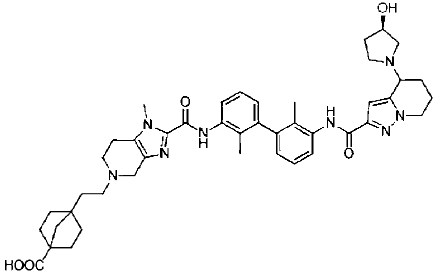 |
| 48 | 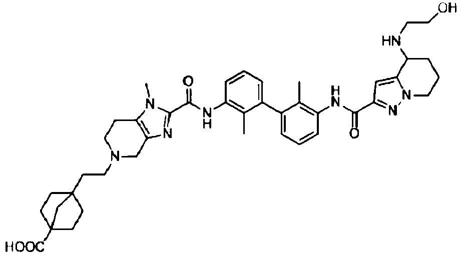 |
| 49 | 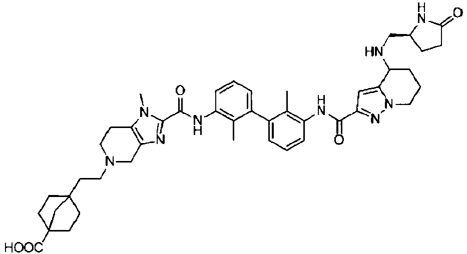 |
| 50 | 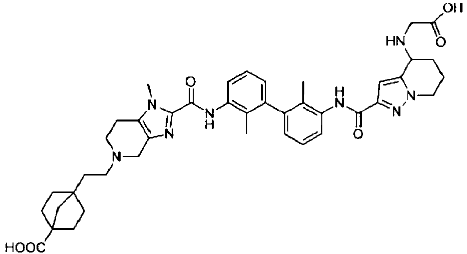 |
| 51 | 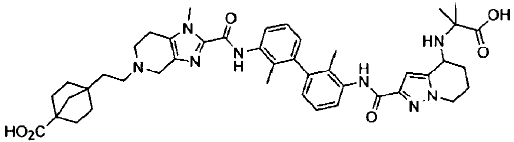 |
| 52 | 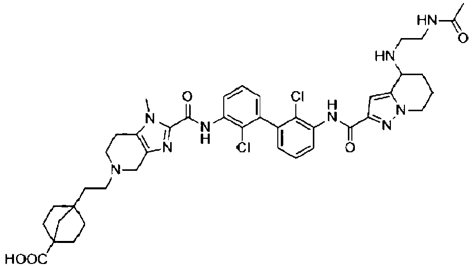 |
| 53 | 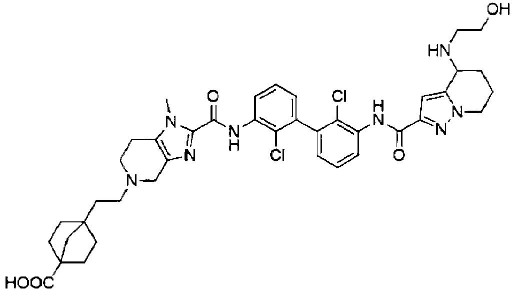 |
| 54 | 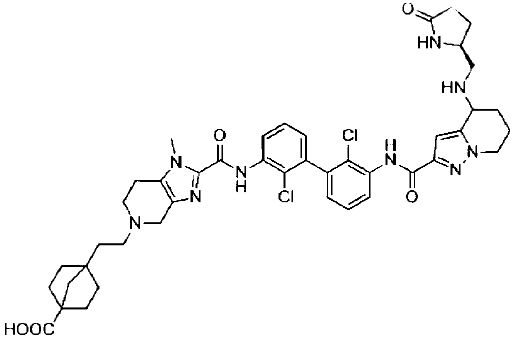 |
| 56 | 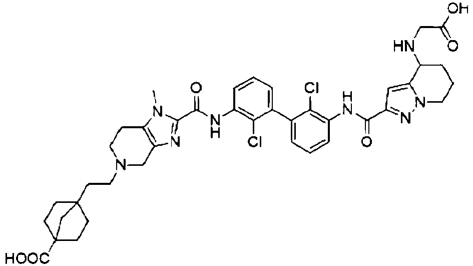 |
| 57 | 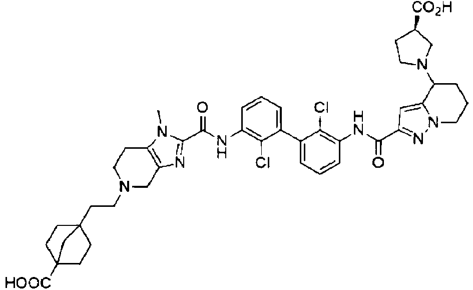 |
| 58 | 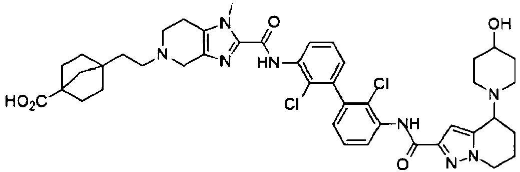 |
| 59 | 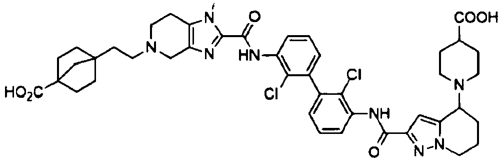 |
| 60 | 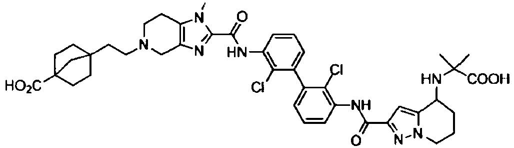 |
| 61 | 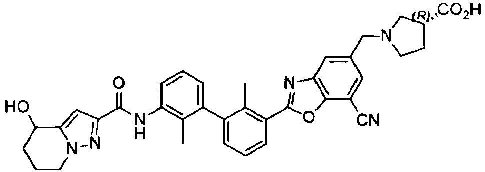 |
| 62 | 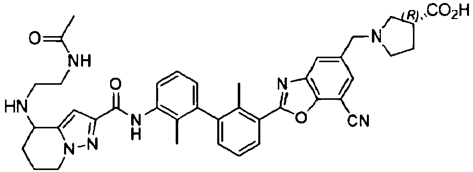 |
| 63 | 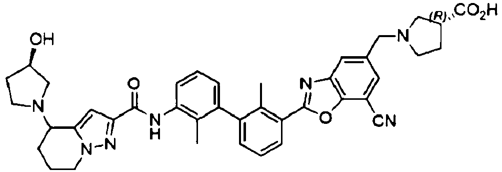 |
| 64 | 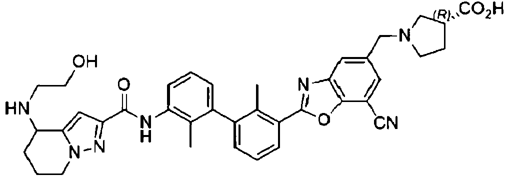 |
| 65 | 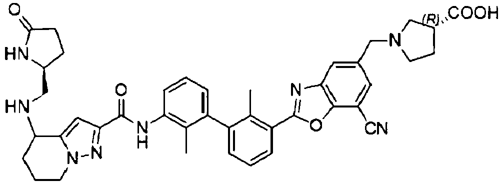 |
| 66 | 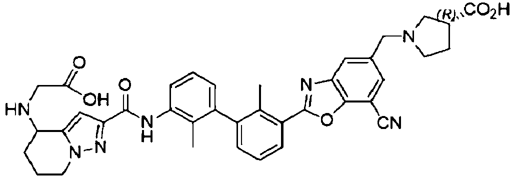 |
| 67 | 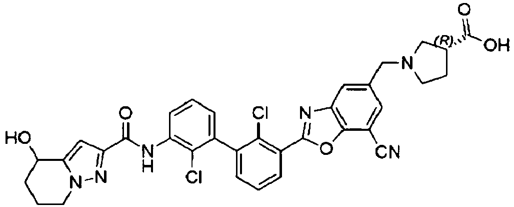 |
| 68 | 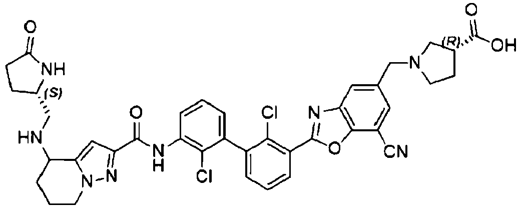 |
| 69 | 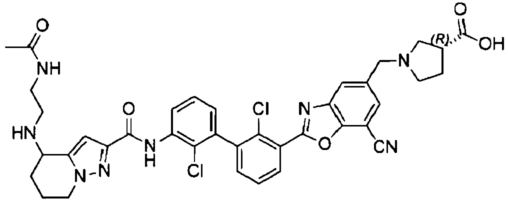 |
| 70 | 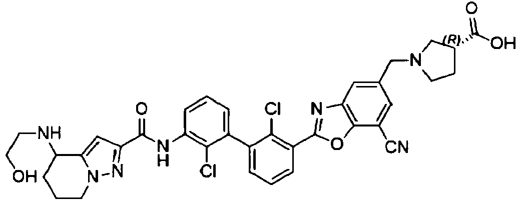 |
| 71 | 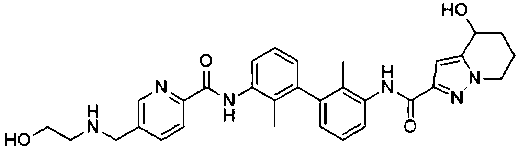 |
| 72 | 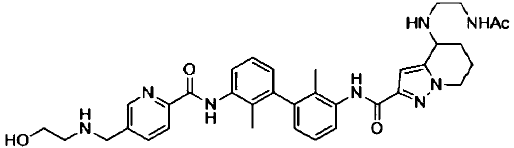 |
| 73 | 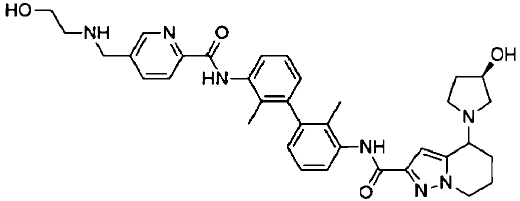 |
| 74 | 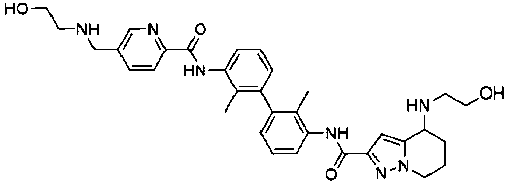 |
| 75 | 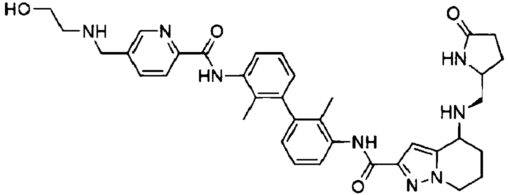 |
| 76 | 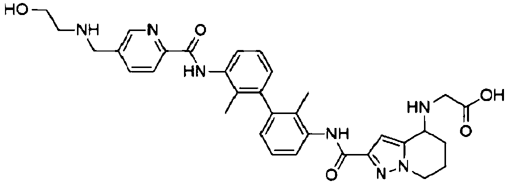 |
| 77 | 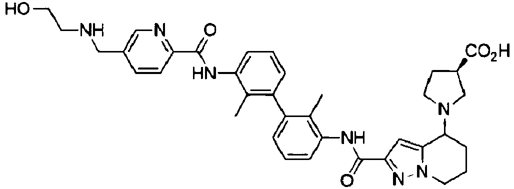 |
| 78 | 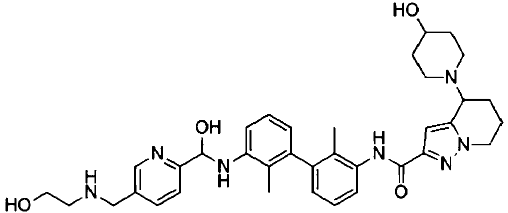 |
| 79 | 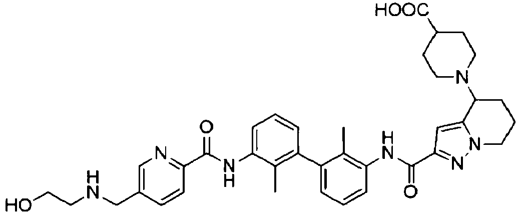 |
| 80 | 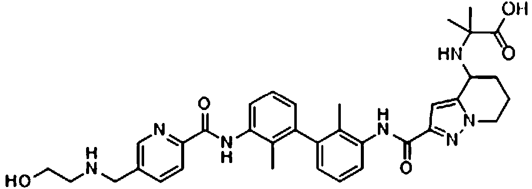 |
| 81 | 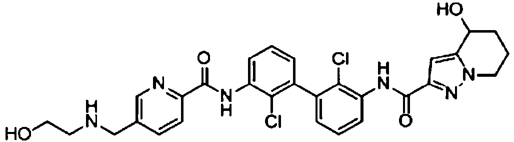 |
| 82 | 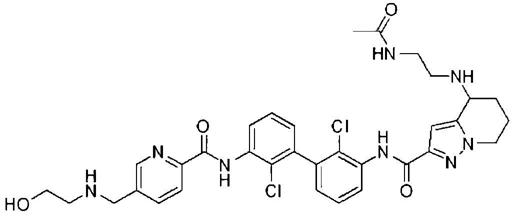 |
| 83 | 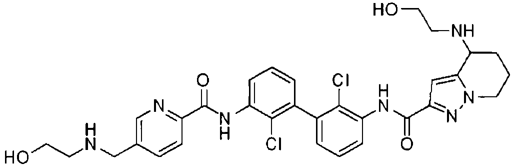 |
| 84 | 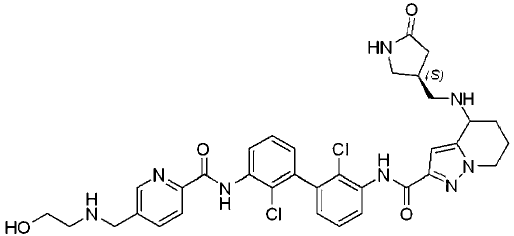 |
| 85 | 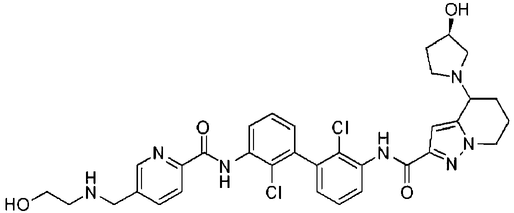 |
| 86 | 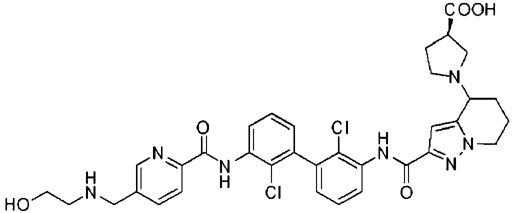 |
| 87 | 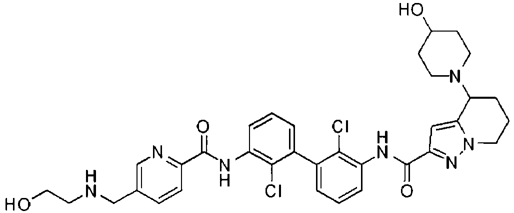 |
| 88 | 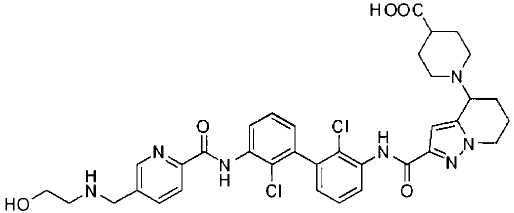 |
| 89 | 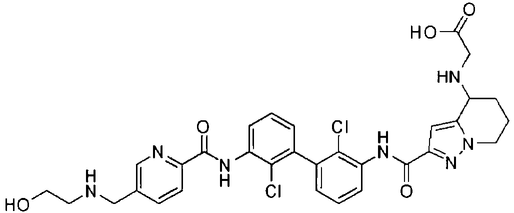 |
| 90 | 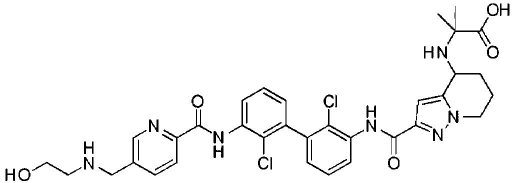 |
| 91 | 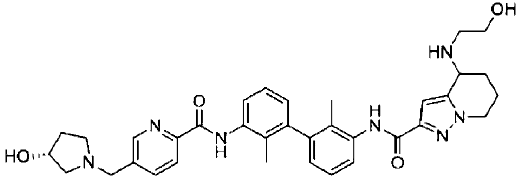 |
| 92 | 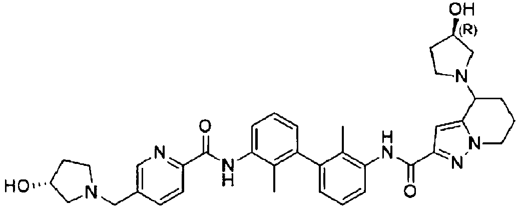 |
| 93 | 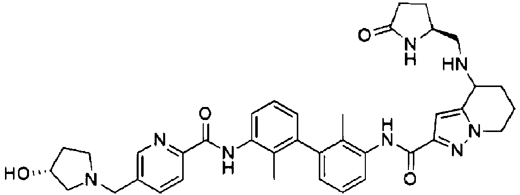 |
| 94 | 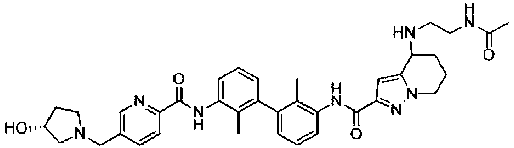 |
| 95 | 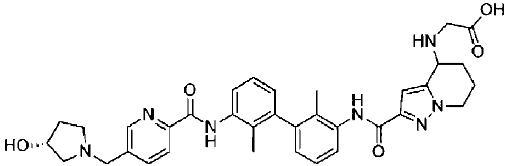 |
| 96 | 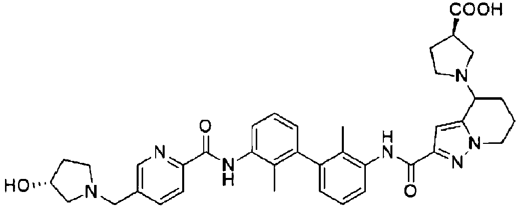 |
| 97 | 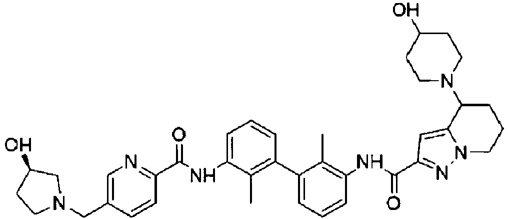 |
| 98 | 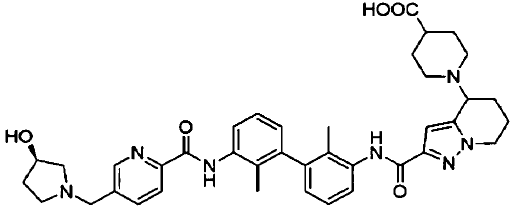 |
| 99 | 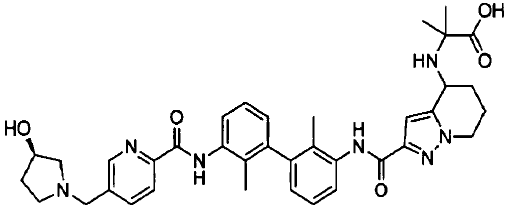 |
| 100 | 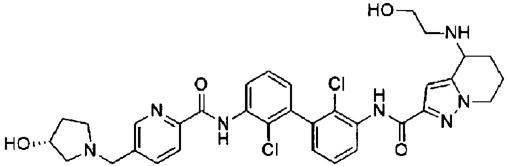 |
| 101 | 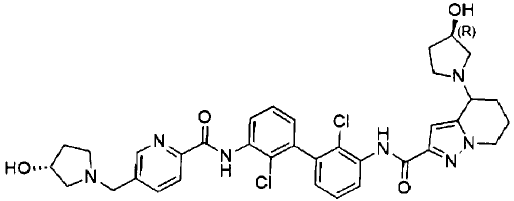 |
| 102 | 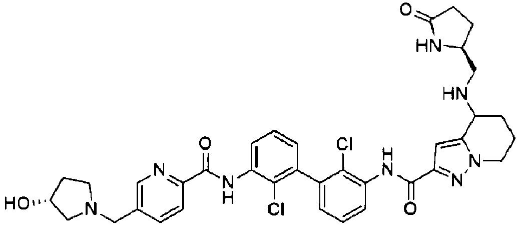 |
| 103 | 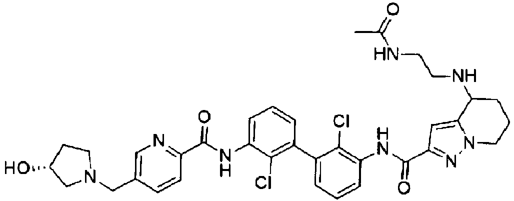 |
| 104 | 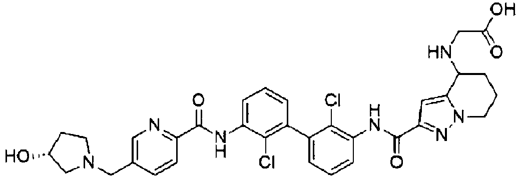 |
| 105 | 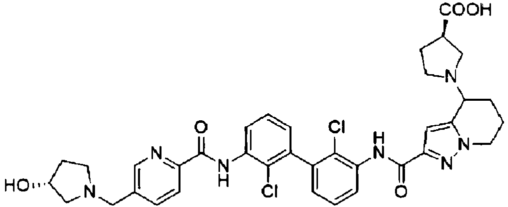 |
| 106 | 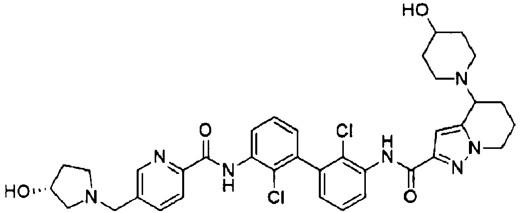 |
| 107 | 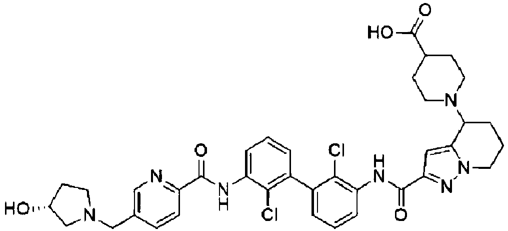 |
| 108 | 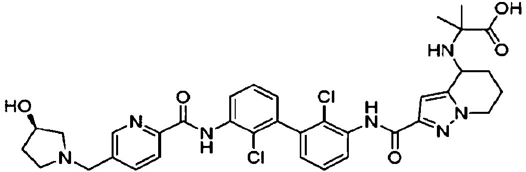 |
| 109 | 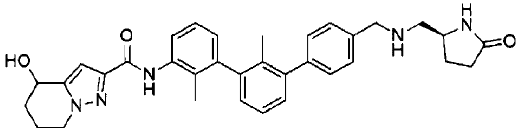 |
| 110 | 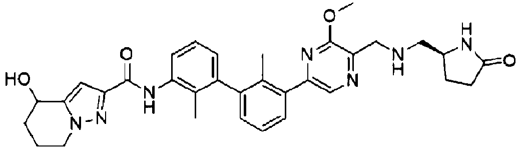 |
| 111 | 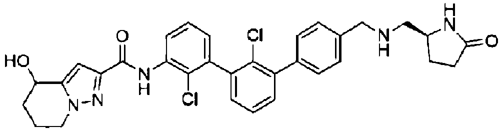 |
| 112 | 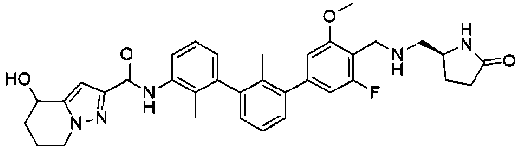 |
| 112-1 | 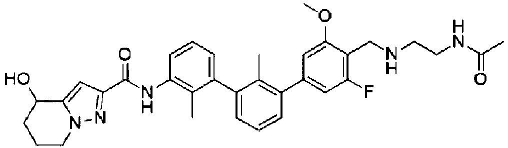 |
| 113 | 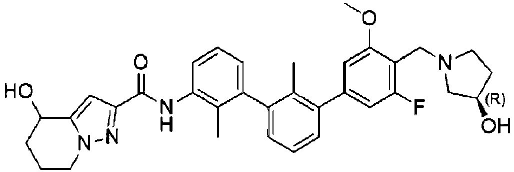 |
| 114 | 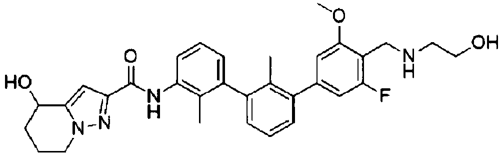 |
| 115 | 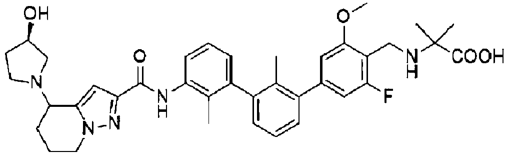 |
| 116 | 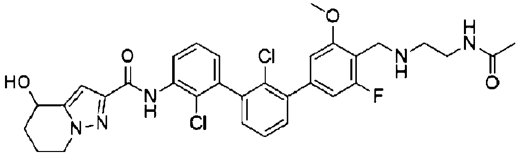 |
| 117 | 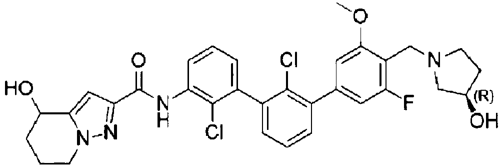 |
| 118 | 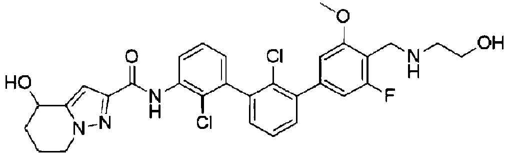 |
| 119 | 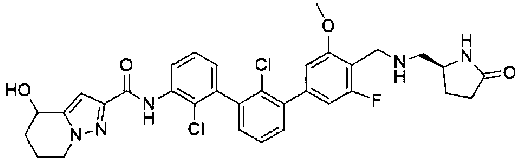 |
| 120 | 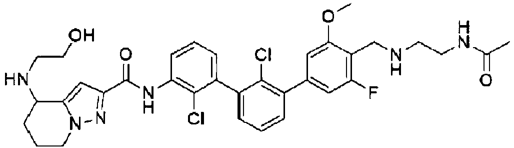 |
| 121 | 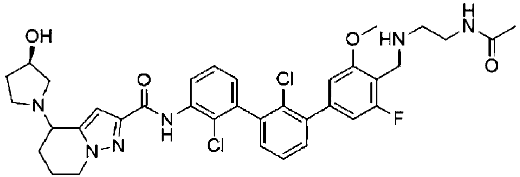 |
| 122 | 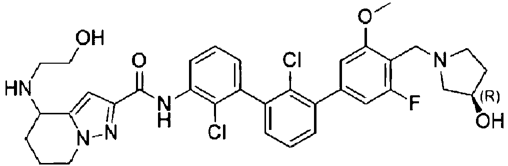 |
| 123 | 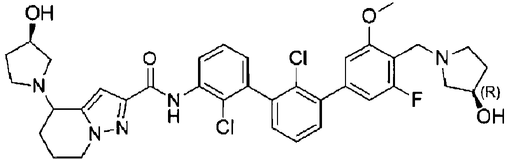 |
| 124 | 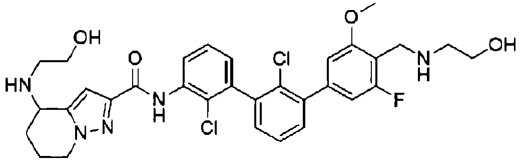 |
| 125 | 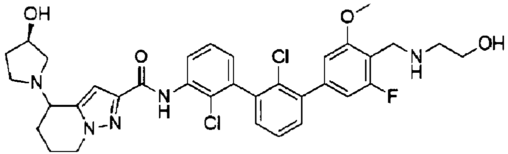 |
| 126 | 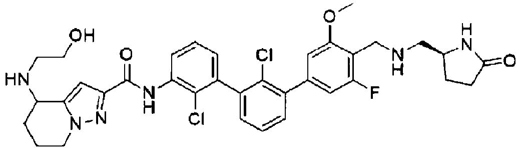 |
| 127 | 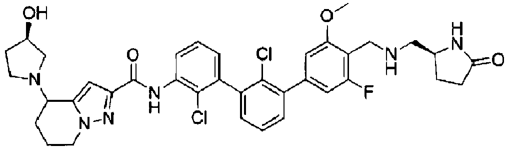 |
| 128 | 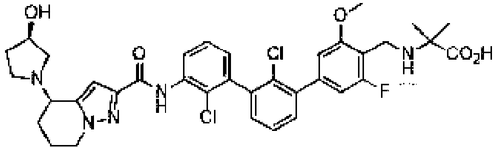 |
| 129 | 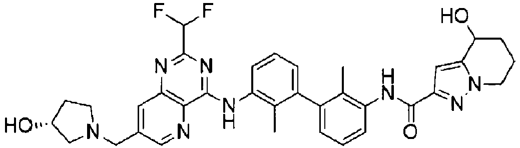 |
| 130 | 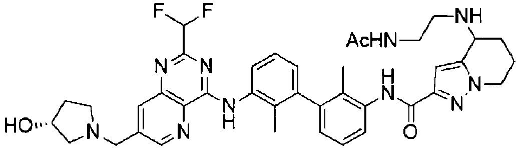 |
| 131 | 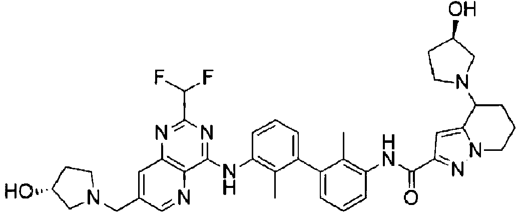 |
| 132 | 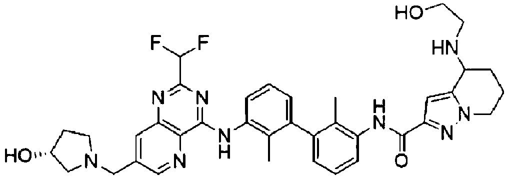 |
| 133 | 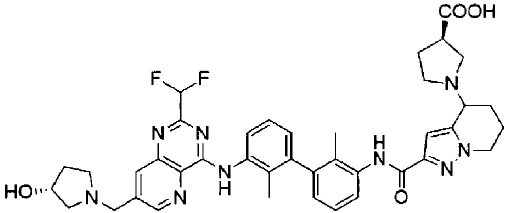 |
| 134 | 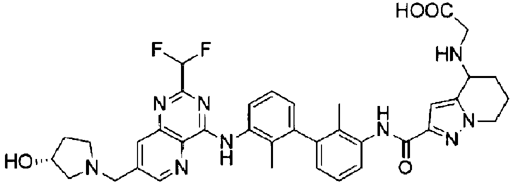 |
| 135 | 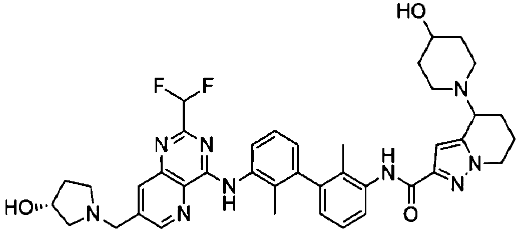 |
| 136 | 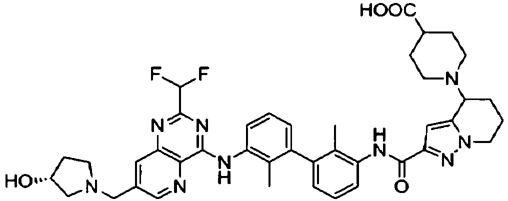 |
| 137 | 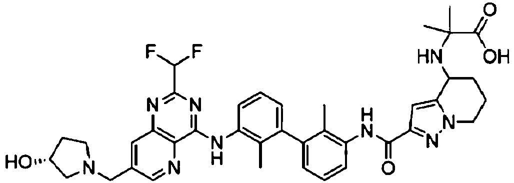 |
| 138 | 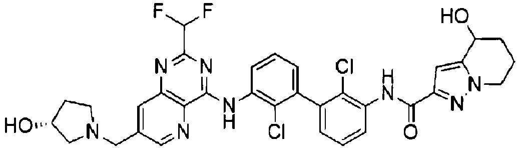 |
| 139 | 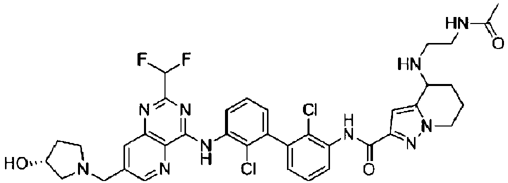 |
| 140 | 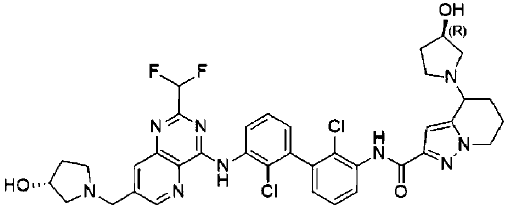 |
| 141 | 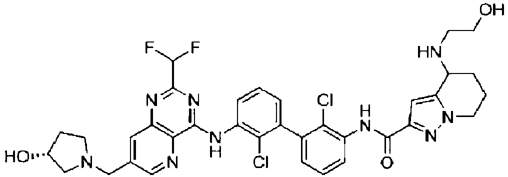 |
| 142 | 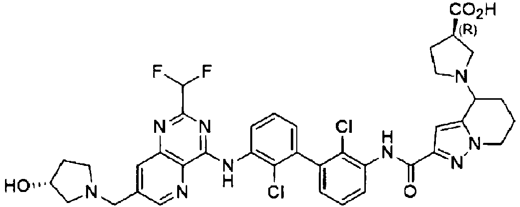 |
| 143 | 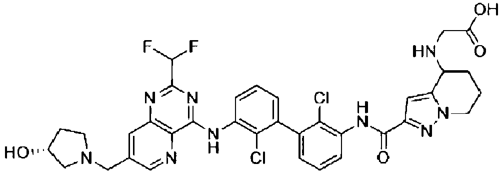 |
| 144 | 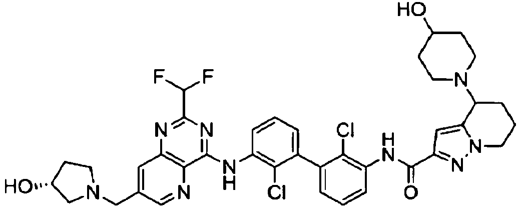 |
| 145 | 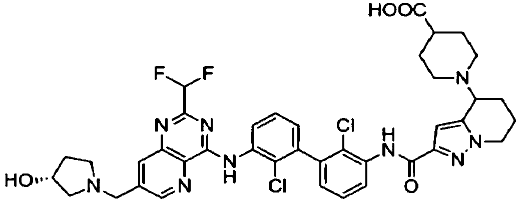 |
| 146 | 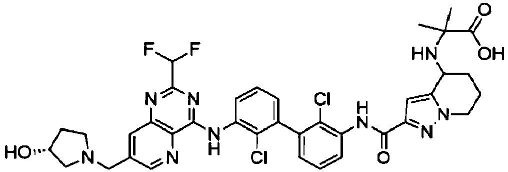 |
| 147 | 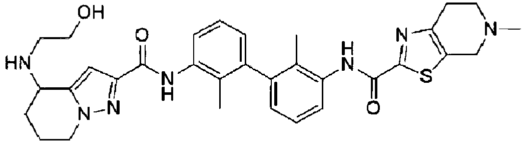 |
| 148 | 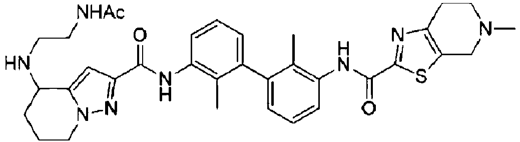 |
| 149 | 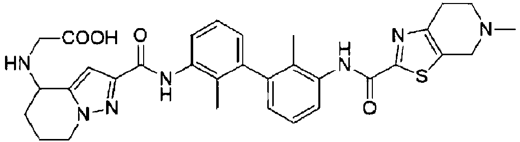 |
| 150 | 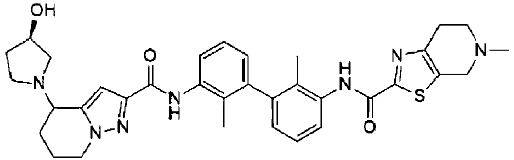 |
| 150-1 | 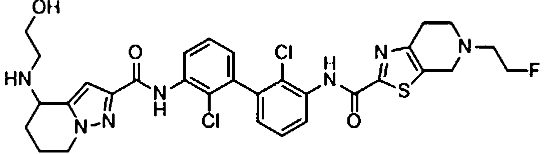 |
| 150-2 | 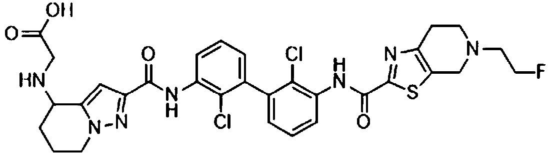 |
| 151 | 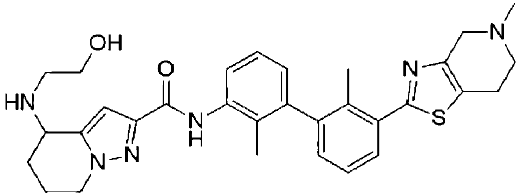 |
| 152 | 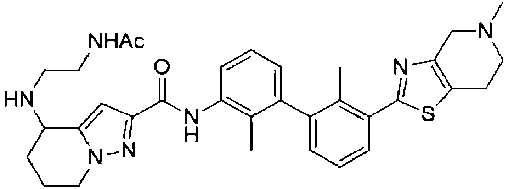 |
| 153 | 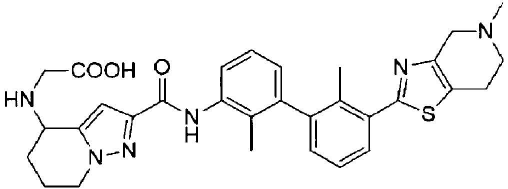 |
| 154 | 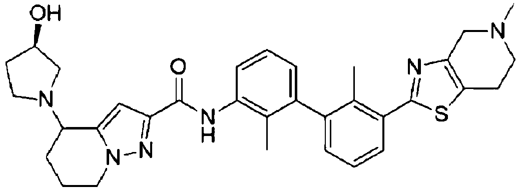 |
| 155 | 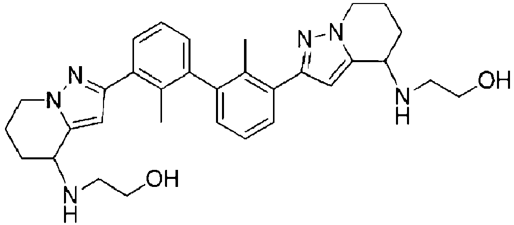 |
| 156 | 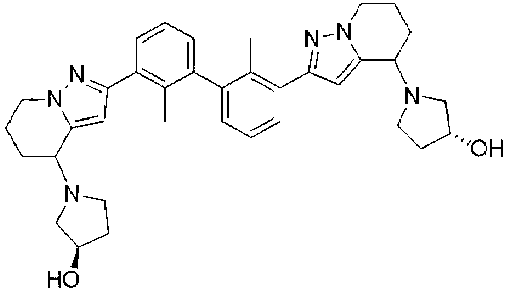 |
| 157 | 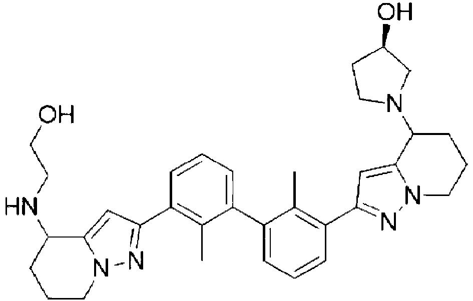 |
| 158 | 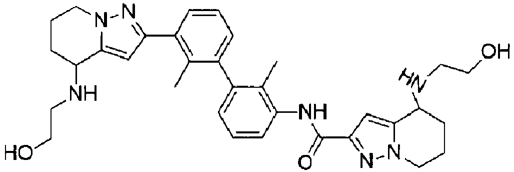 |
| 159 | 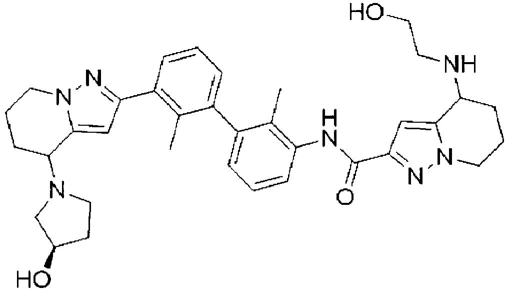 |
| 160 | 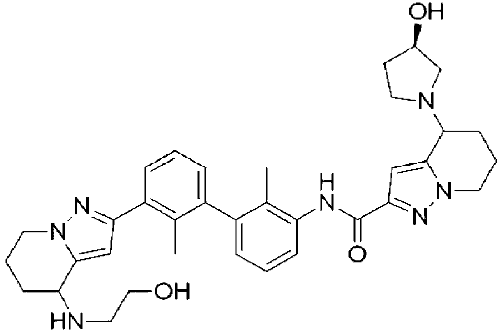 |
| 161 | 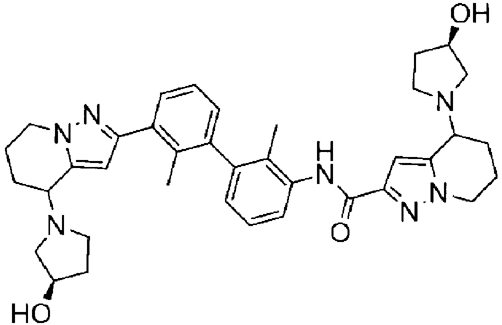 |
| 162 | 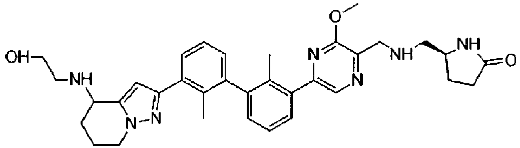 |
| 163 | 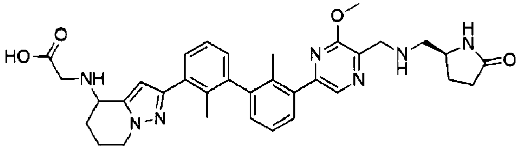 |
| 164 | 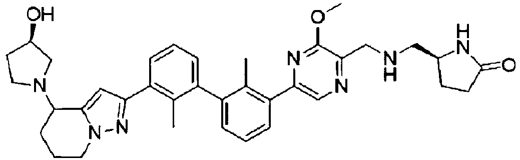 |
| 165 | 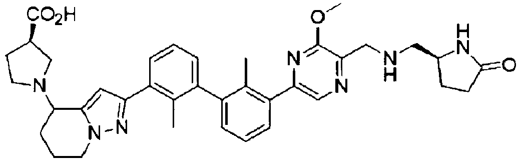 |
| 166 | 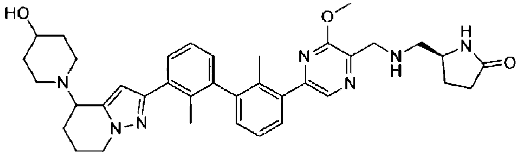 |
| 167 | 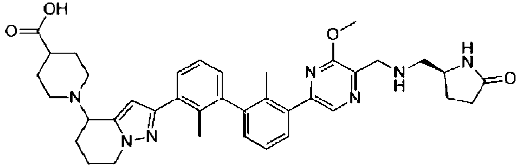 |
| 168 | 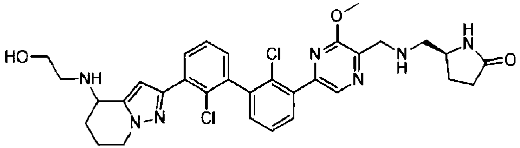 |
| 169 | 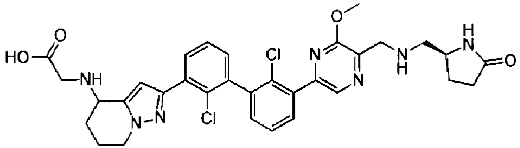 |
| 170 | 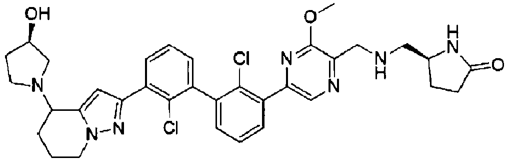 |
| 171 | 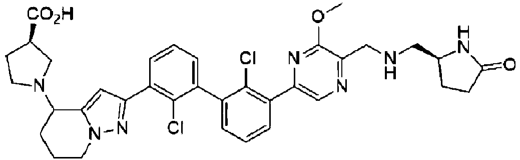 |
| 174 | 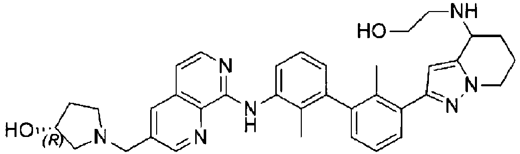 |
| 175 | 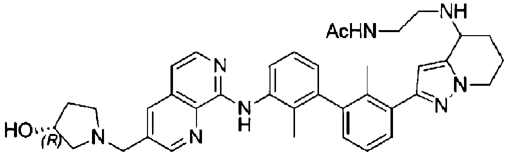 |
| 176 | 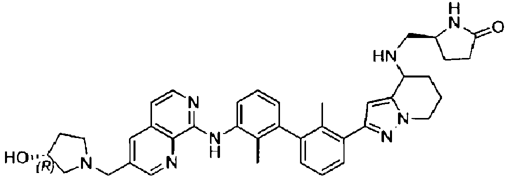 |
| 177 | 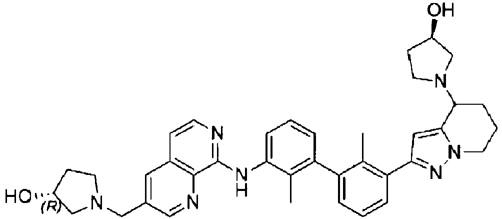 |
| 178 | 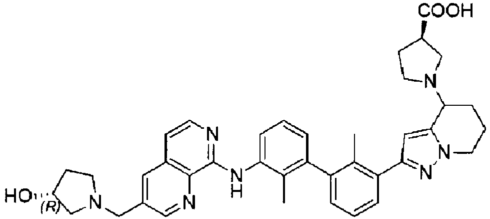 |
| 179 | 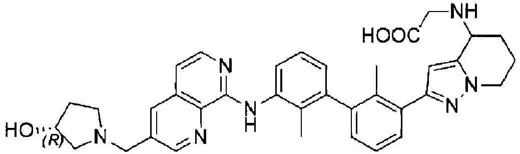 |
| 180 | 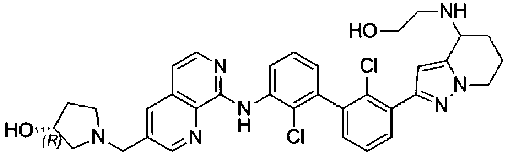 |
| 181 | 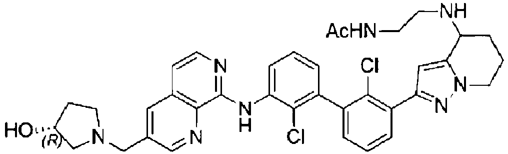 |
| 182 | 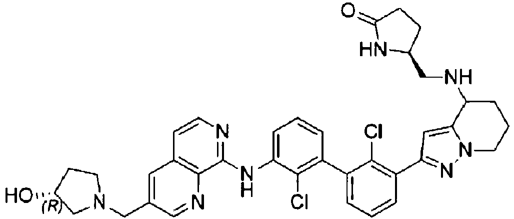 |
| 183 | 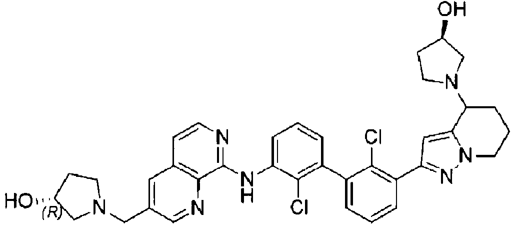 |
| 184 | 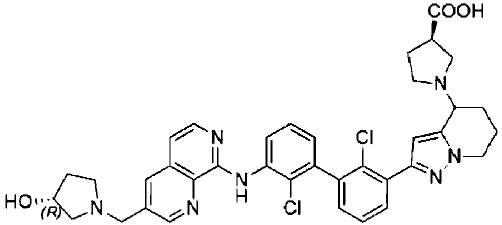 |
| 185 | 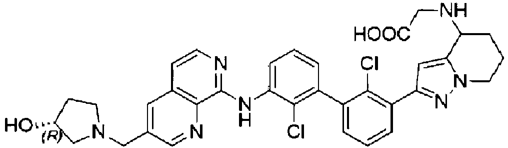 |
| 186 | 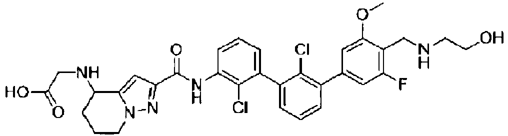 |
| 187 | 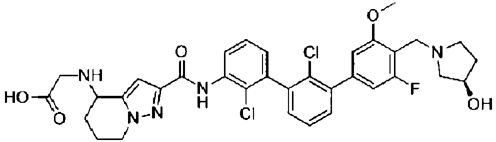 |
| 188 | 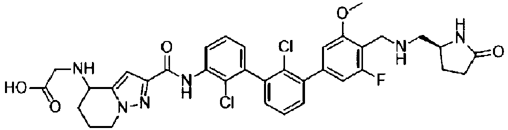 |
| 189 | 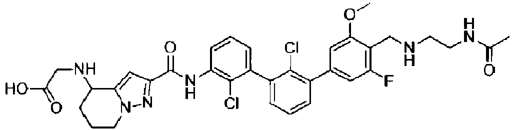 |
| 190 | 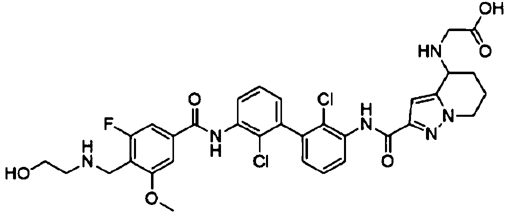 |
| 191 | 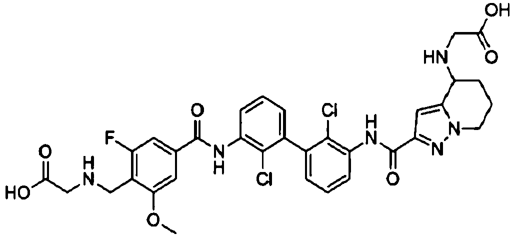 |
| 192 | 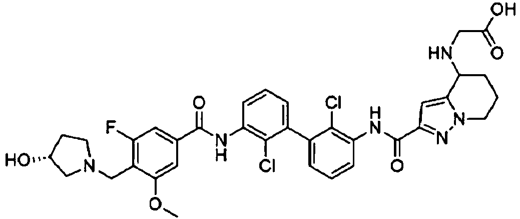 |
| 193 | 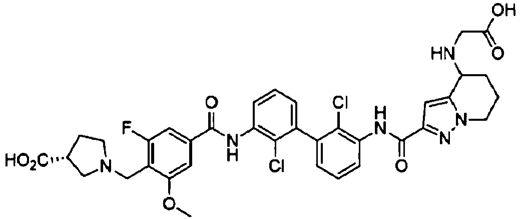 |
| 194 | 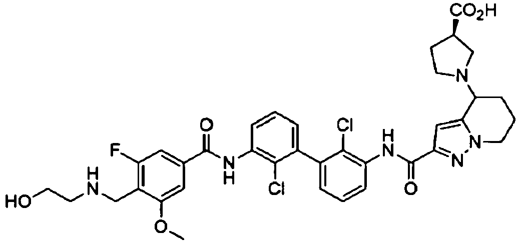 |
| 195 | 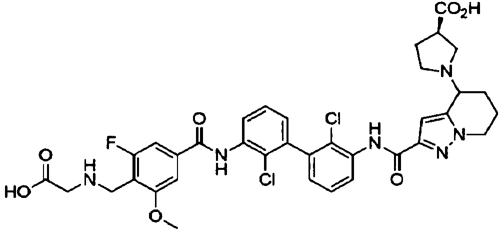 |
| 196 | 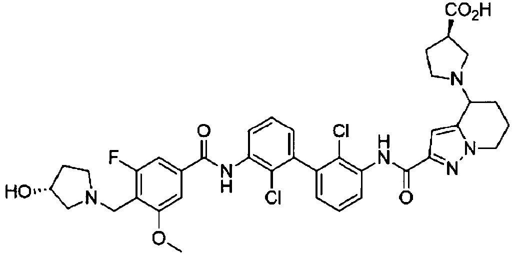 |
| 197 | 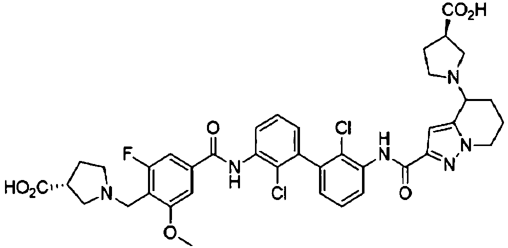 |
| 198 | 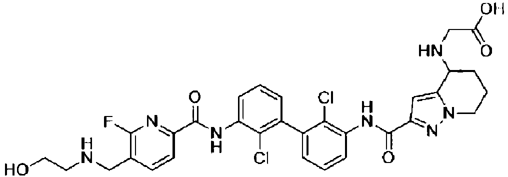 |
| 199 | 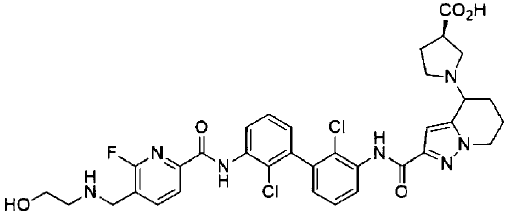 |
| 200 | 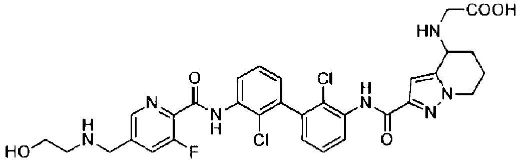 |
| 201 | 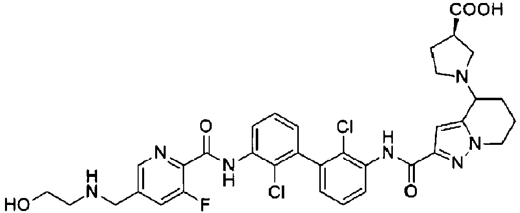 |
| 202 | 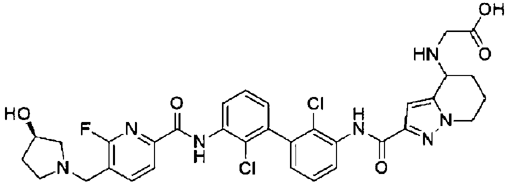 |
| 203 | 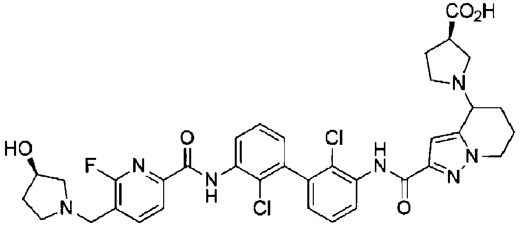 |
| 204 | 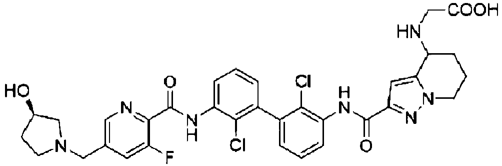 |
| 205 | 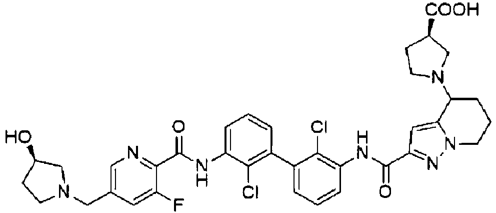 |
| 206 | 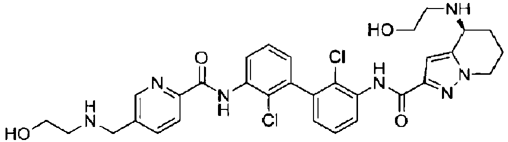 |
| 207 | 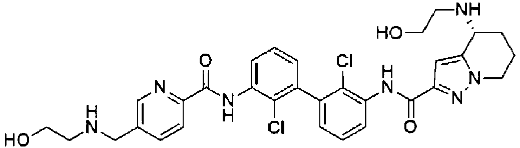 |
| 208 | 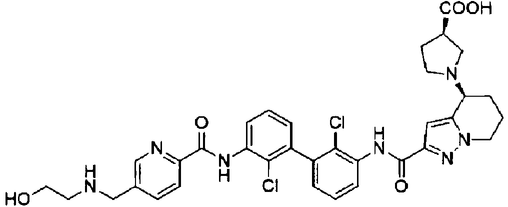 |
| 209 | 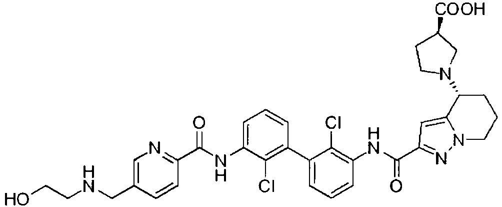 |
| 210 | 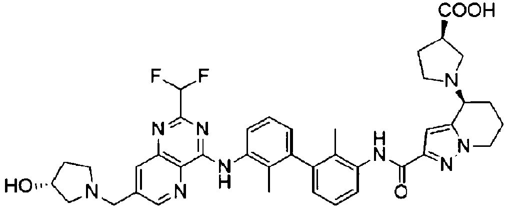 |
| 211 | 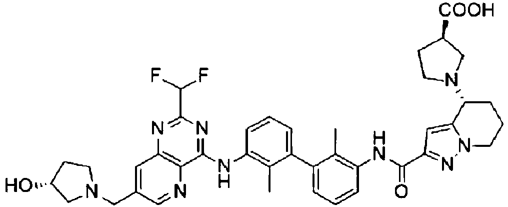 |
| 212 | 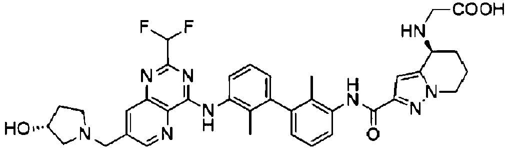 |
| 213 | 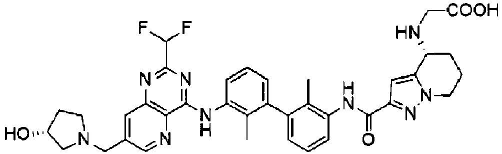 |
| 214 | 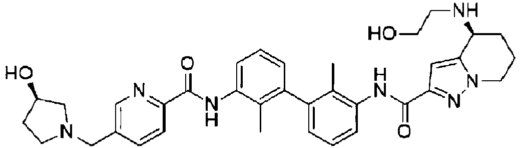 |
| 215 | 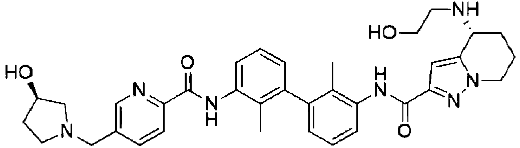 |
| 216 | 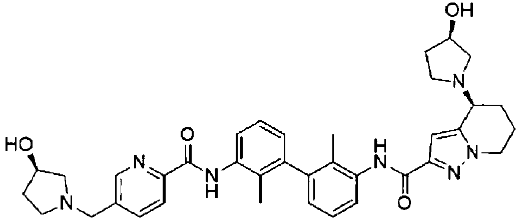 |
| 217 | 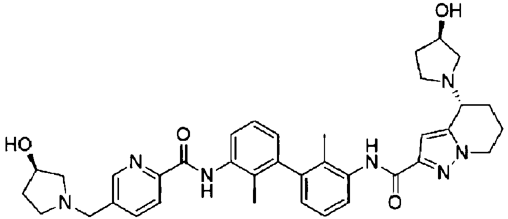 |
| 218 | 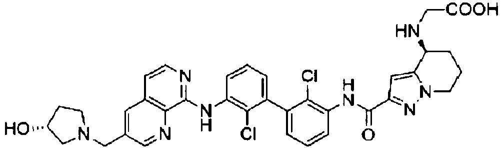 |
| 219 | 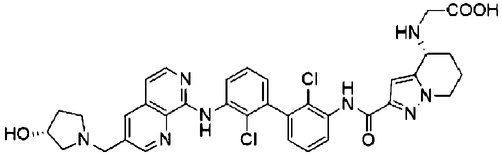 |
| 220 | 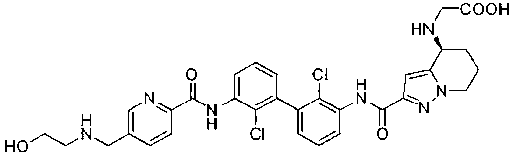 |
| 221 | 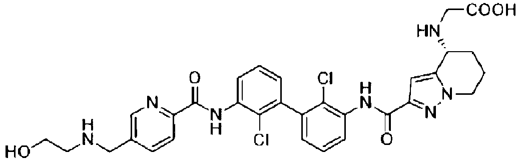 |
| 222 | 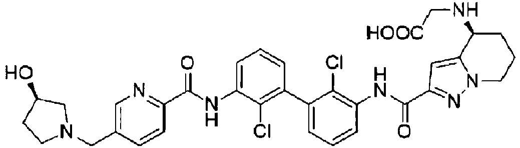 |
| 223 | 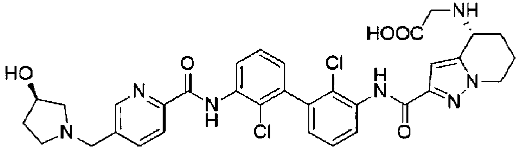 |
| 224 | 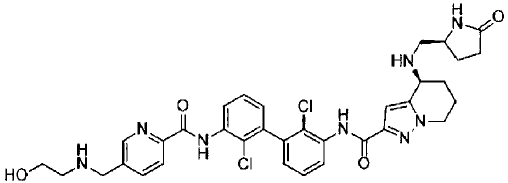 |
| 225 | 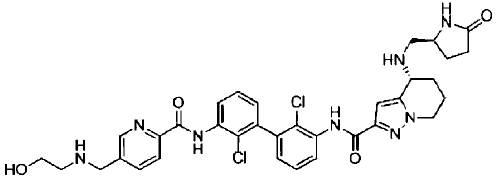 |
| 226 | 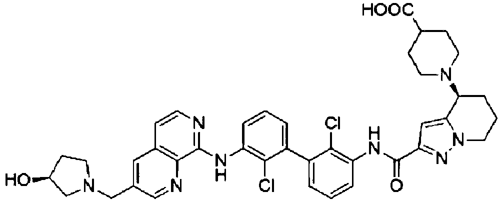 |
| 227 | 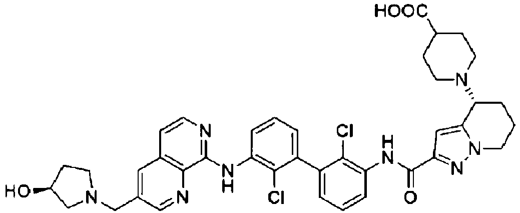 |
| 228 | 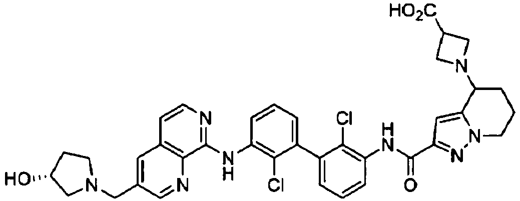 |
| 229 | 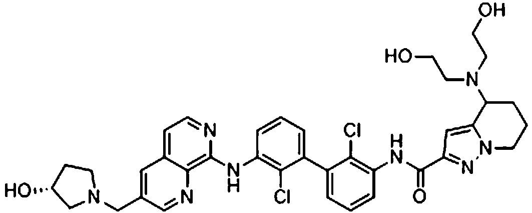 |
| 230 | 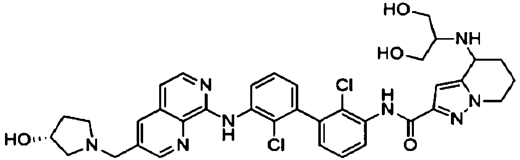 |
| 231 | 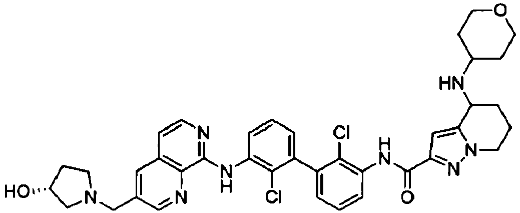 |
| 232 | 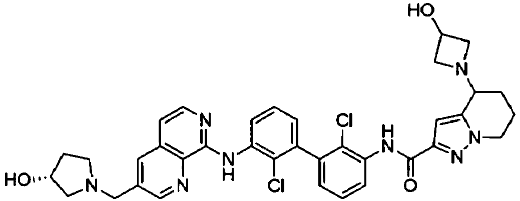 |
| 233 | 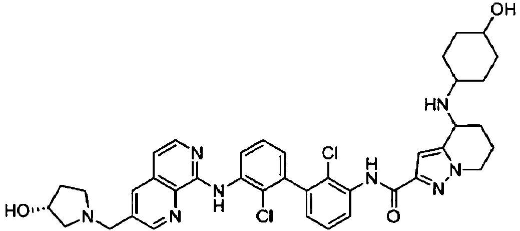 |
| 234 | 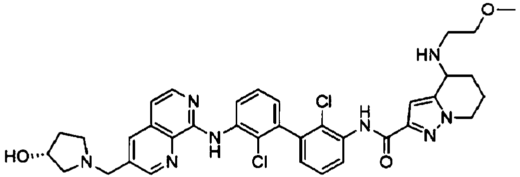 |
| 235 | 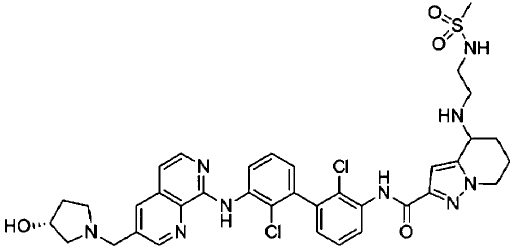 |
| 236 | 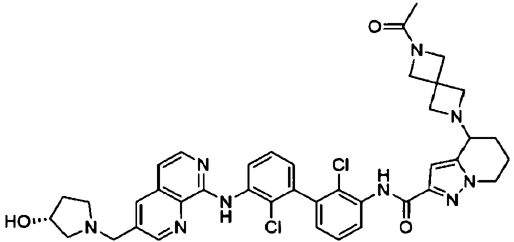 |
| 237 | 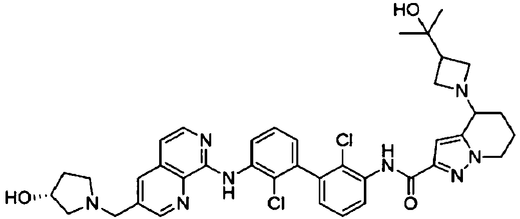 |
| 238 | 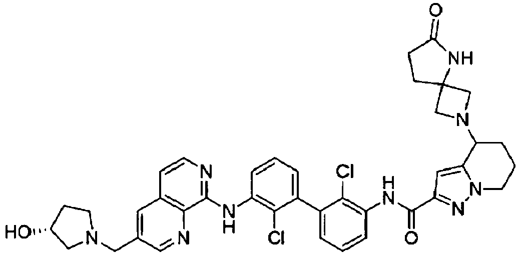 |
| 239 | 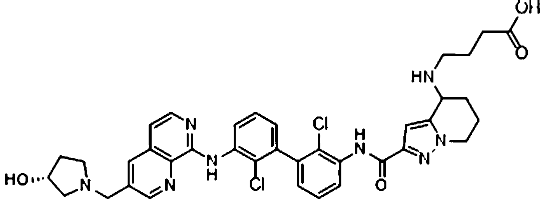 |
| 240 | 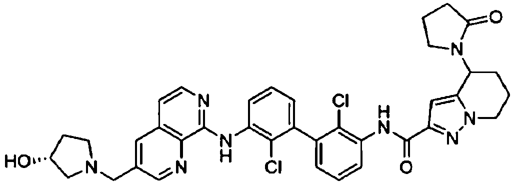 |
| 241 | 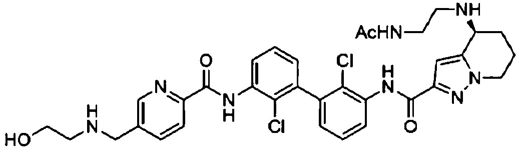 |
| 242 | 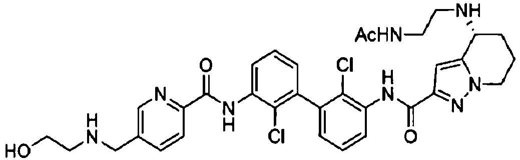 |
| 243 | 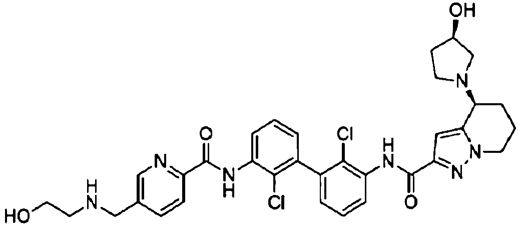 |
| 244 | 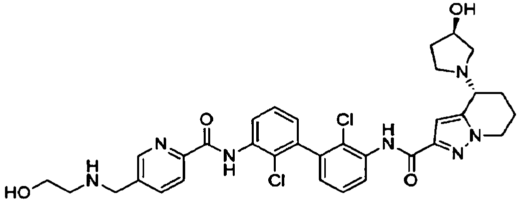 |
| 245 | 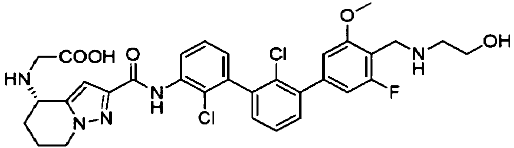 |
| 246 | 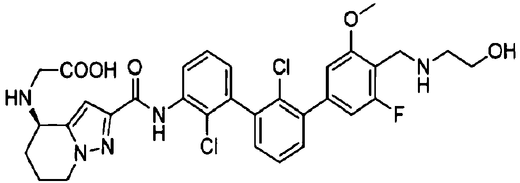 |
| 247 | 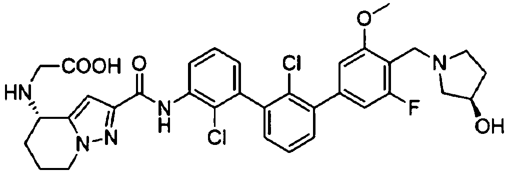 |
| 248 | 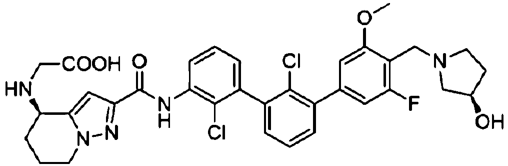 |
| 249 | 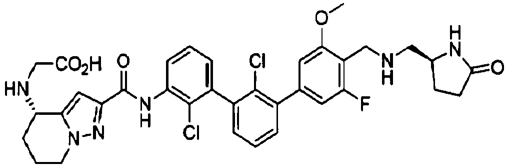 |
| 250 | 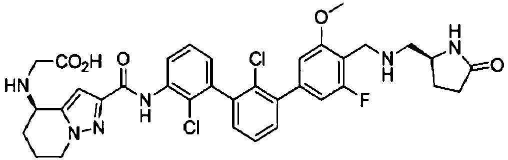 |
| 251 | 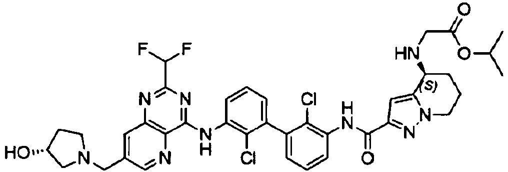 |
| 252 | 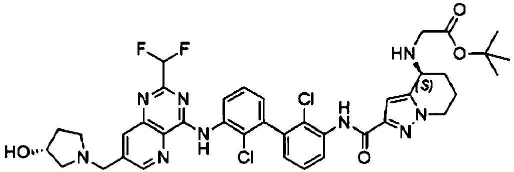 |
| 253 | 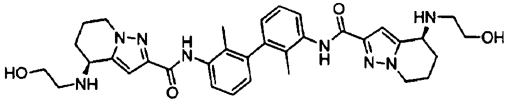 |
| 254 | 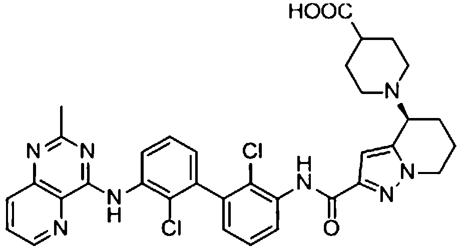 |
| 255 | 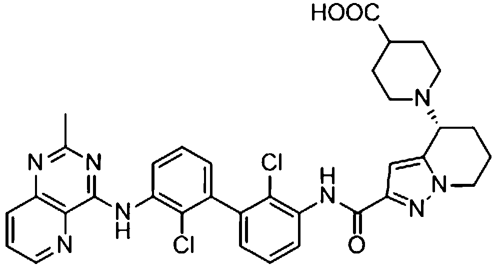 |
| 256 | 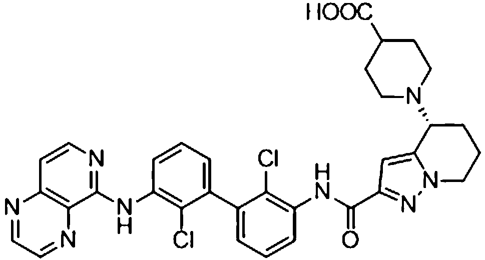 |
| 257 | 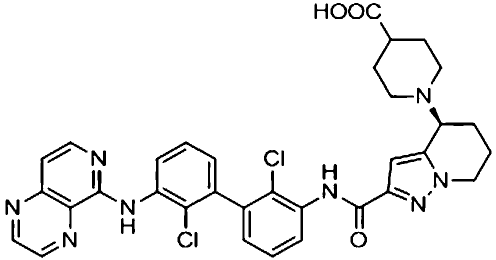 |
| 258 | 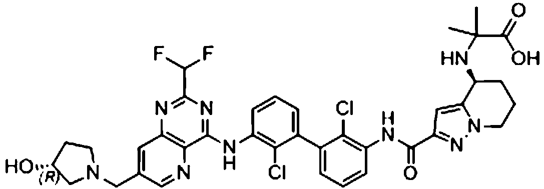 |
| 259 | 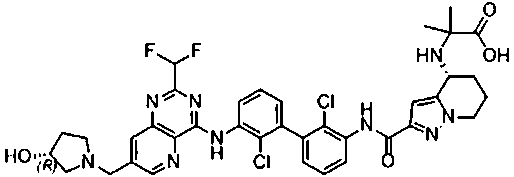 |
| 259-1 | 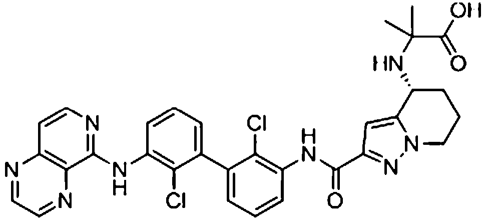 |
| 260 | 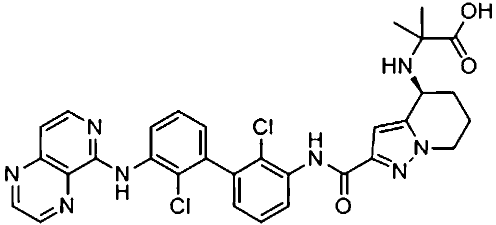 |
| 261 | 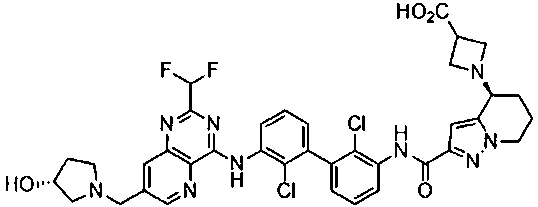 |
| 262 | 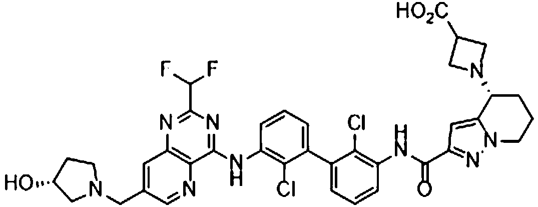 |
| 263 | 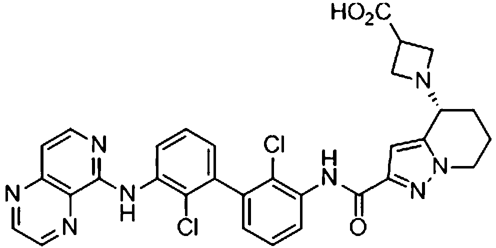 |
| 264 | 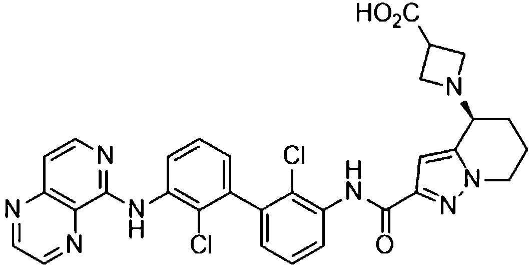 |
| 265 | 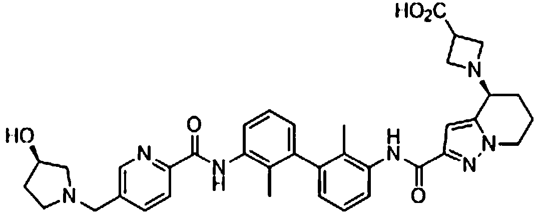 |
| 266 | 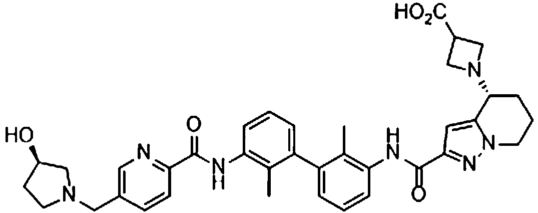 |
| 267 | 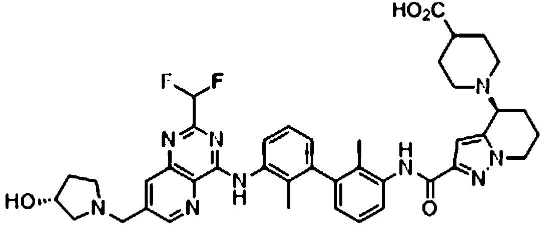 |
| 268 | 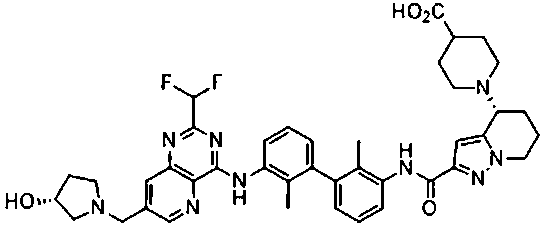 |
| 269 | 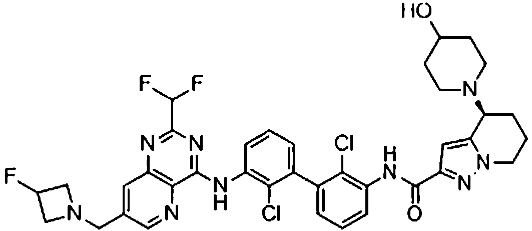 |
| 270 | 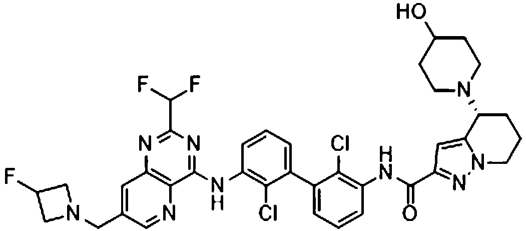 |
| 271 | 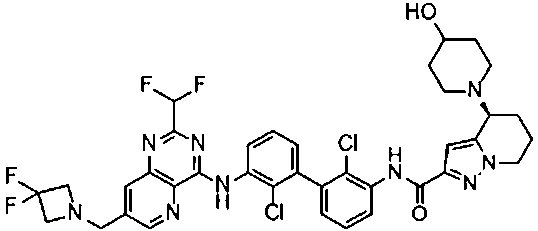 |
| 272 | 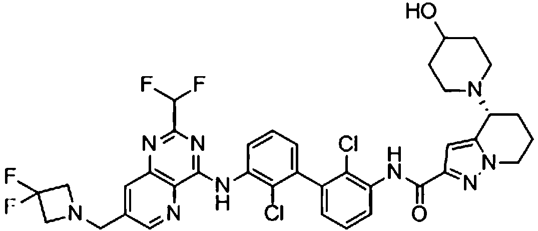 |
| 273 | 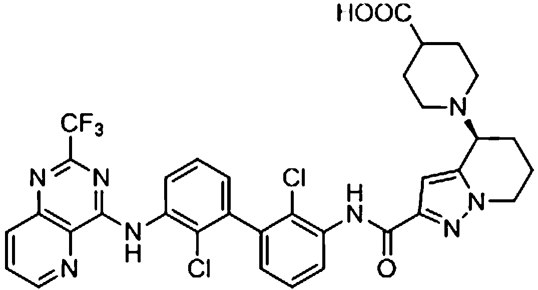 |
| 274 | 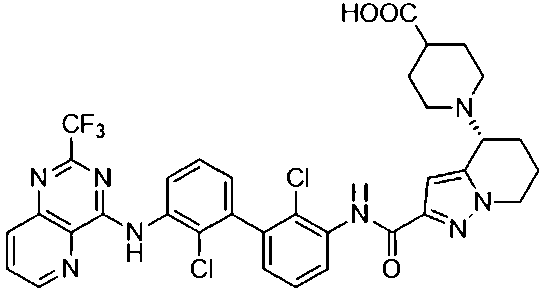 |
| 275 | 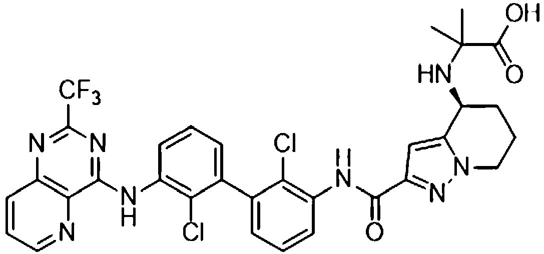 |
| 276 | 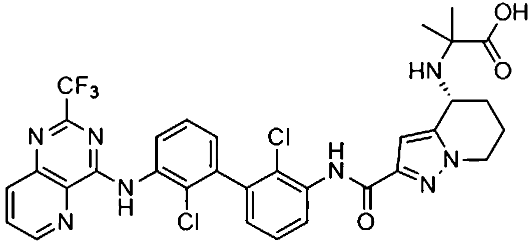 |
| 277 | 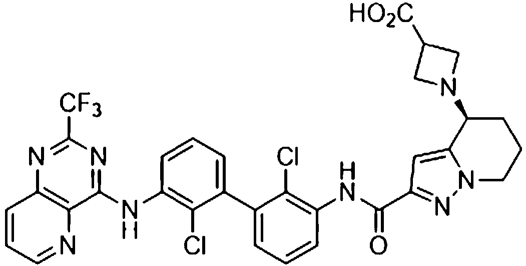 |
| 278 | 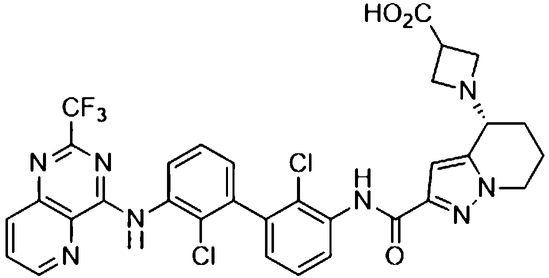 |
| 279 | 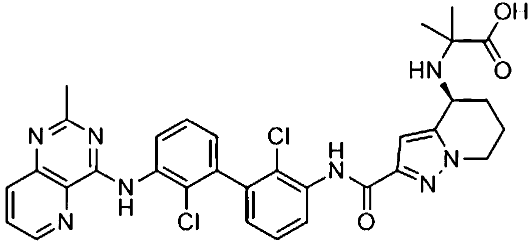 |
| 280 | 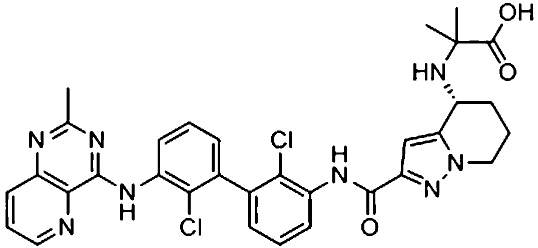 |
| 281 | 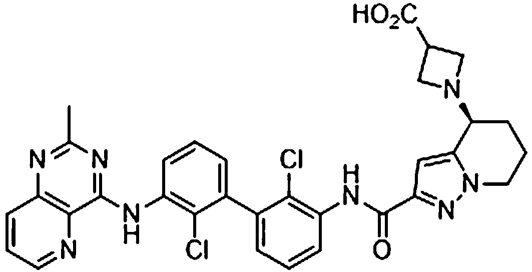 |
| 282 | 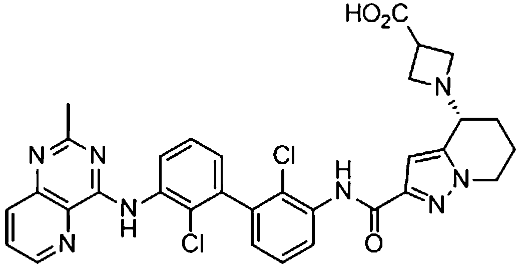 |
| 283 | 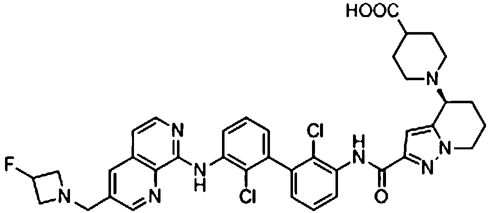 |
| 284 | 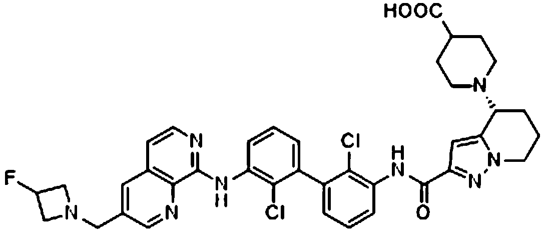 |
| 285 | 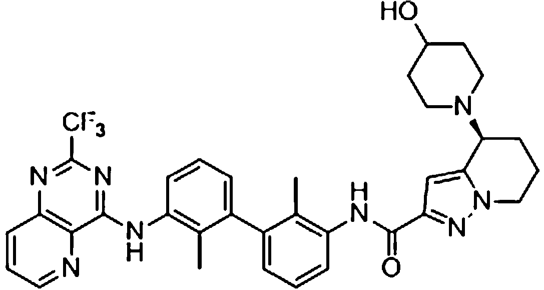 |
| 286 | 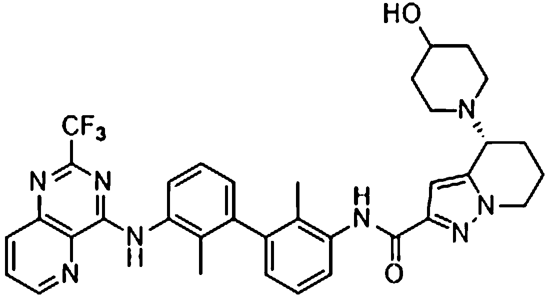 |
| 287 | 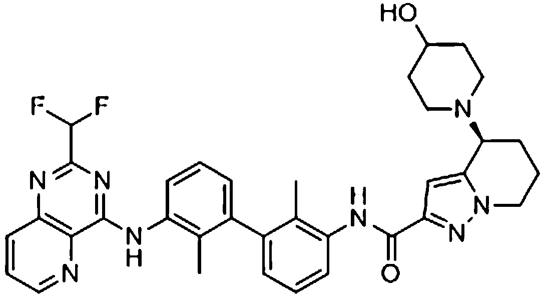 |
| 288 | 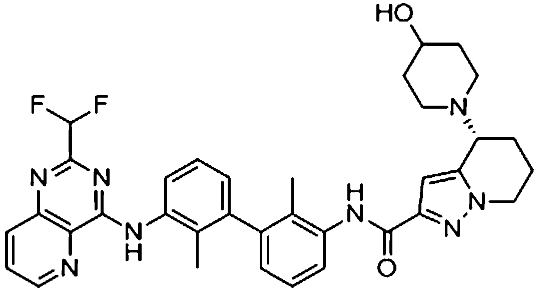 |
| 289 | 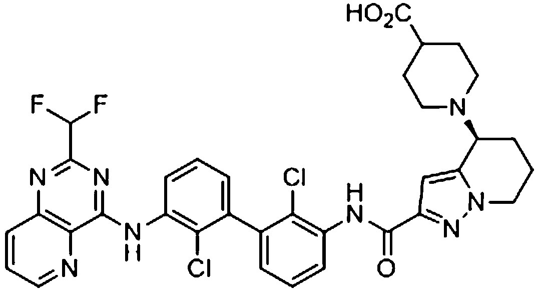 |
| 290 | 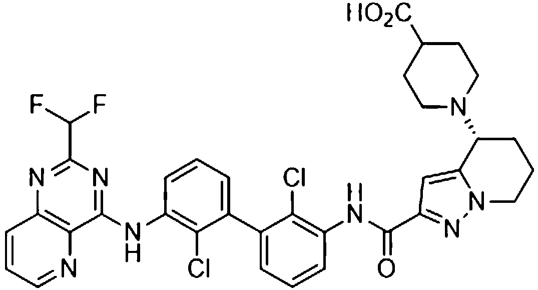 |
| 291 | 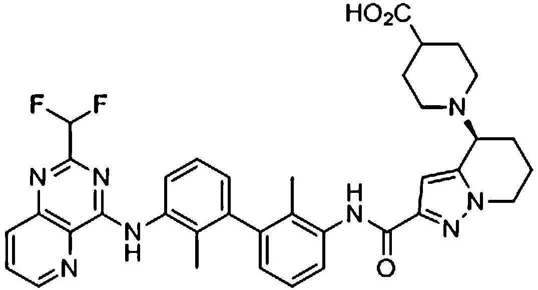 |
| 292 | 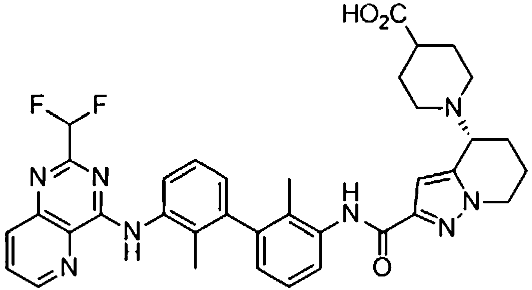 |
| 293 | 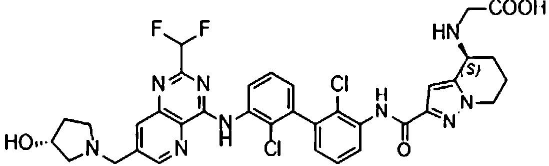 |
| 294 | 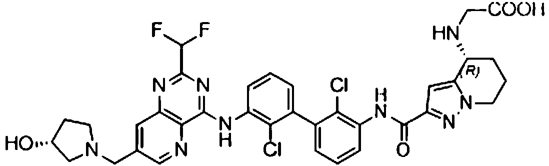 |
| 295 | 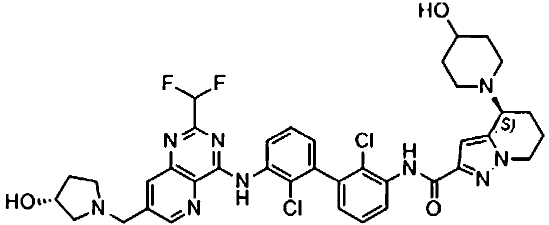 |
| 296 | 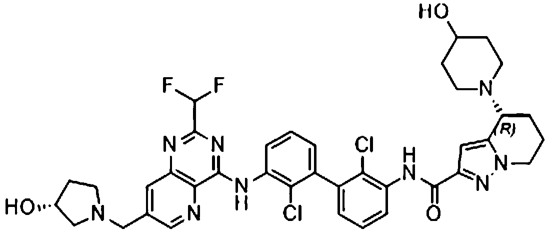 |
| 297 | 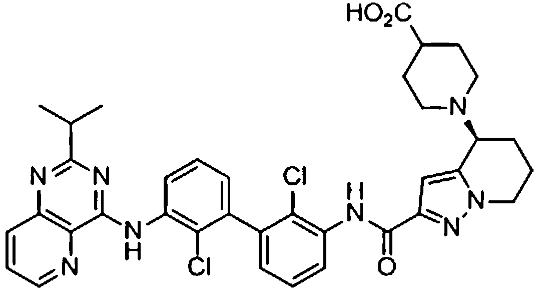 |
| 298 | 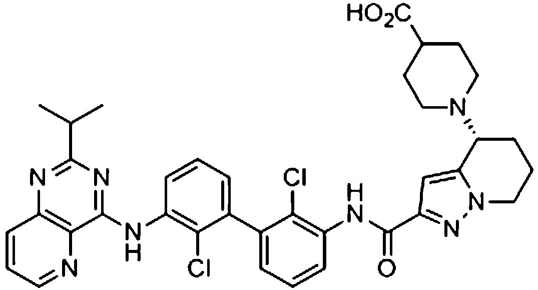 |
| 299 | 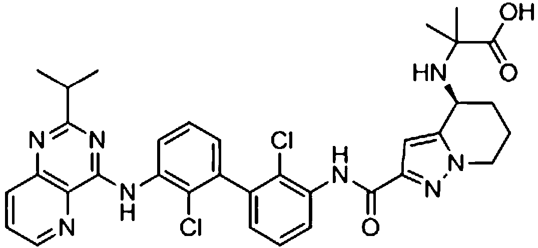 |
| 300 | 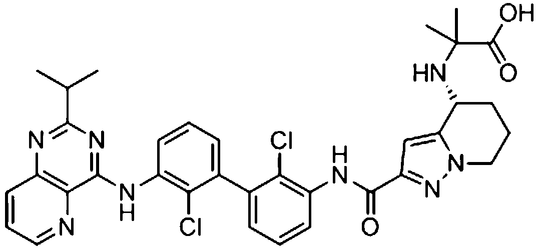 |
| 301 | 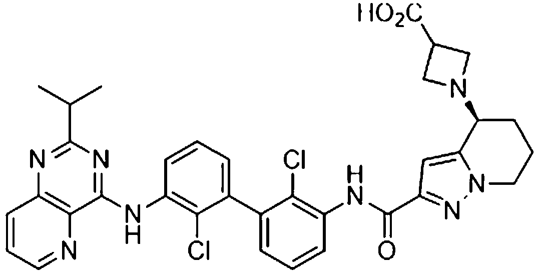 |
| 302 | 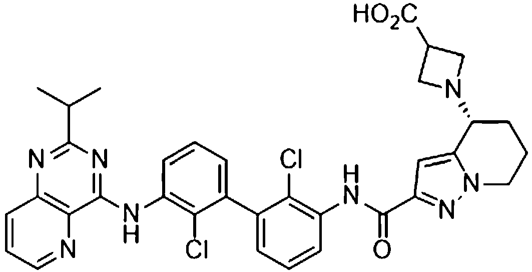 |
| 303 | 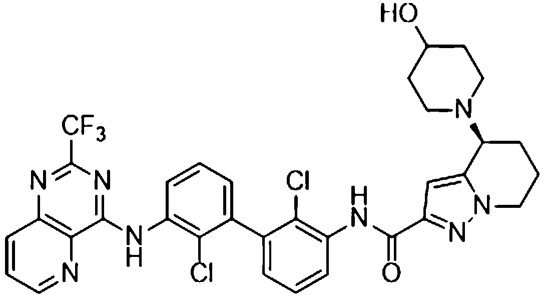 |
| 304 | 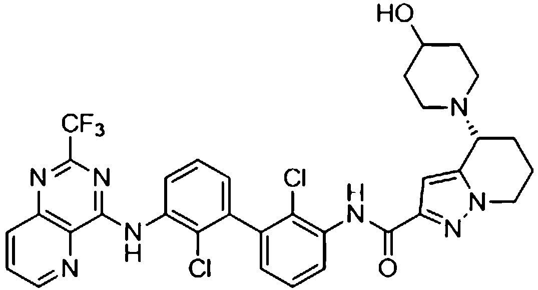 |
| 305 | 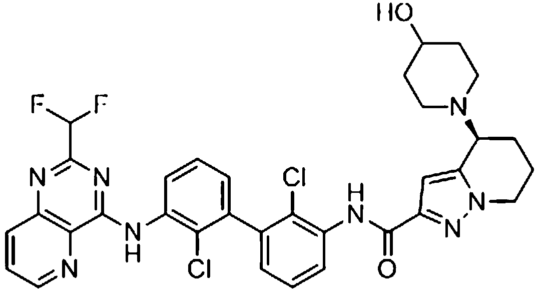 |
| 306 | 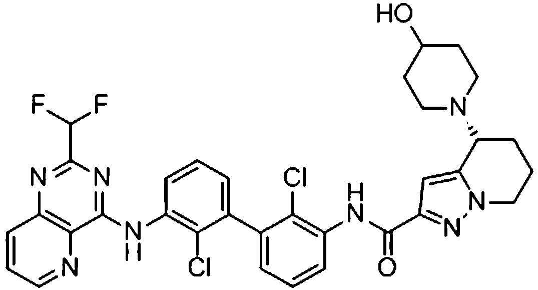 |
| 307 | 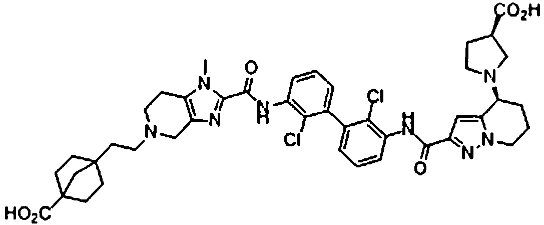 |
| 308 | 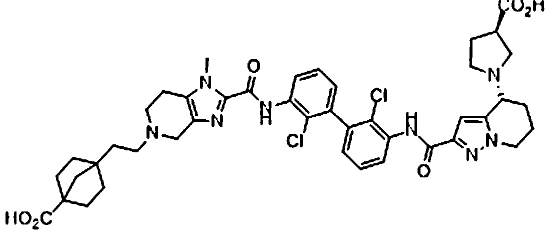 |
| 309 | 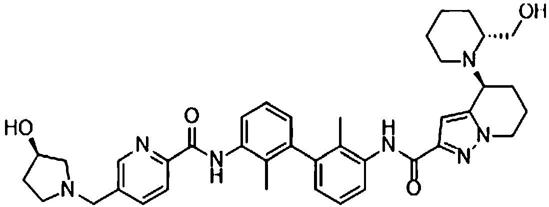 |
| 309-1 | 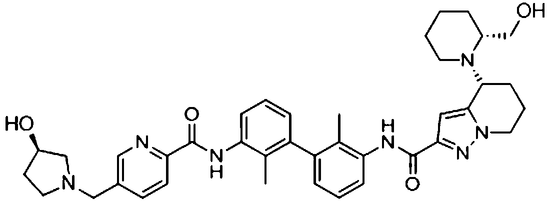 |
| 310 | 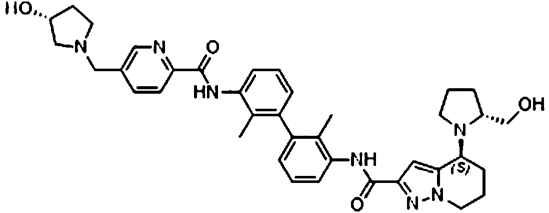 |
| 310-1 | 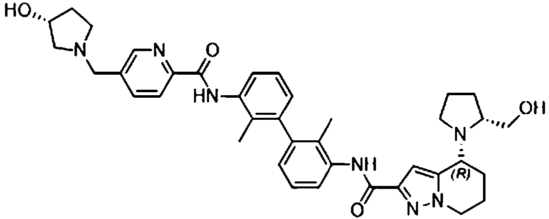 |
| 311 | 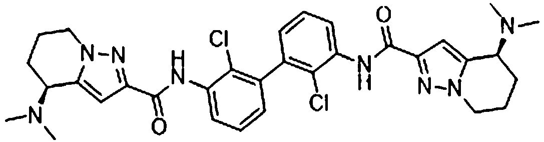 |
| 312 | 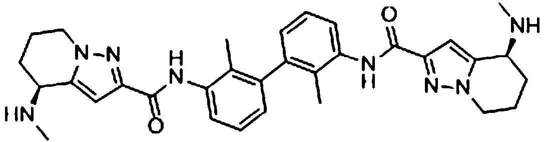 |
| 313 | 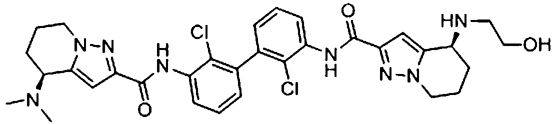 |
| 314 | 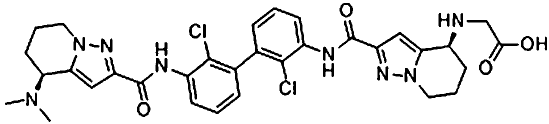 |
| 315 | 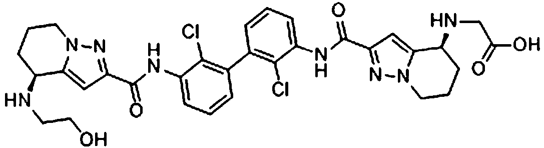 |
| 316 | 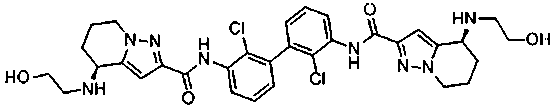 |
| 317 | 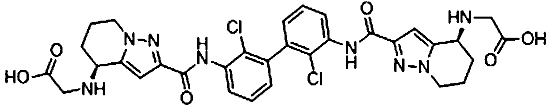 |
| 318 | 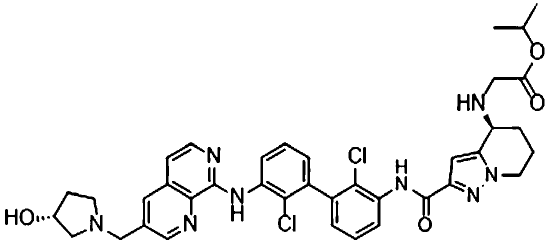 |
| 319 | 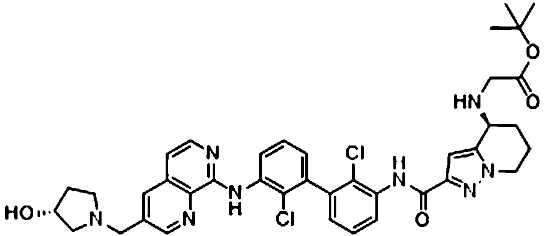 |
| 320 | 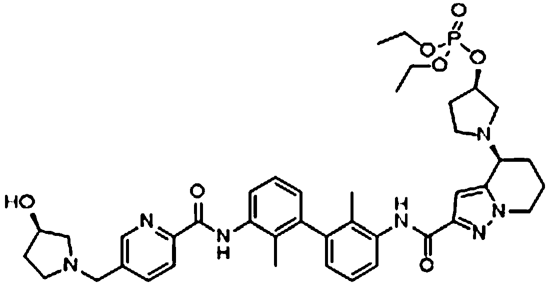 |
| 321 | 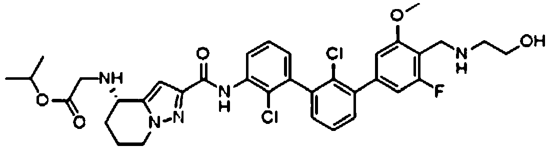 |
| 322 | 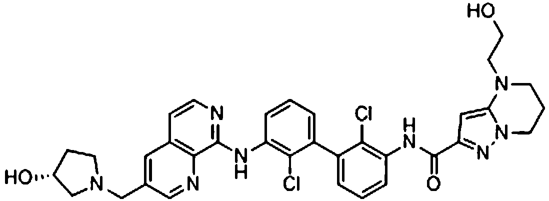 |
| 323 | 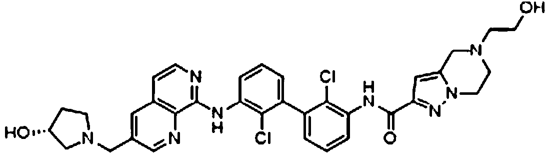 |
| 324 | 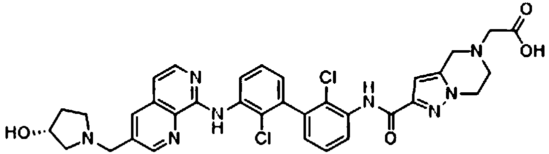 |
| 325 | 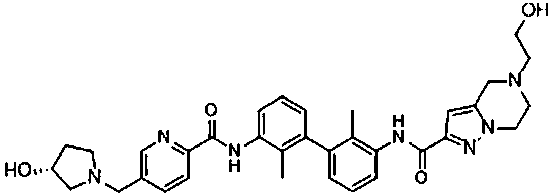 |
| 326 | 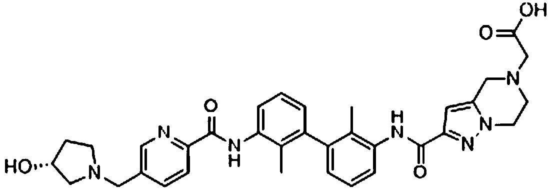 |
| 327 | 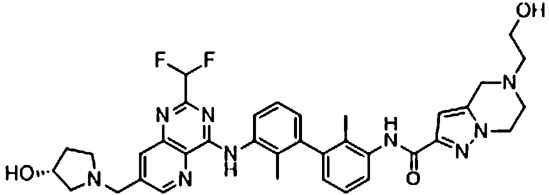 |
| 328 | 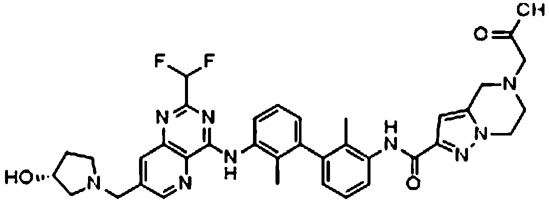 |
| 329 | 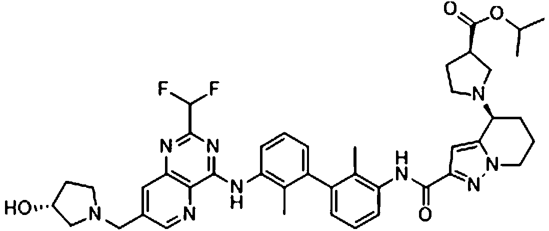 |
| 329-1 | 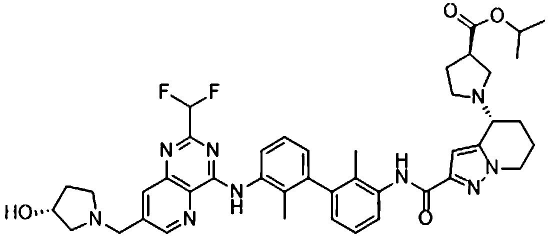 |
| 330 | 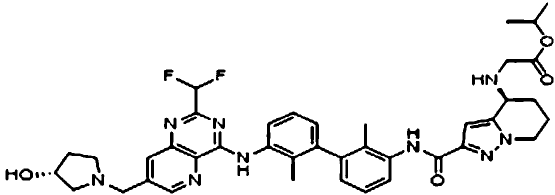 |
| 331 | 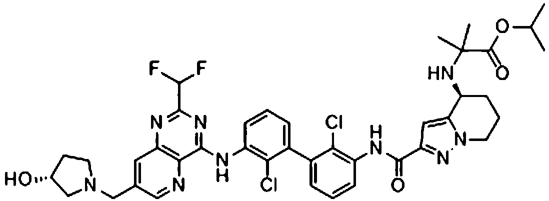 |
| 331-1 | 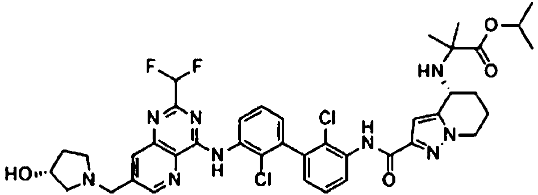 |
| 332 | 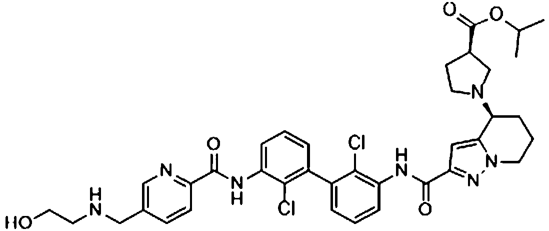 |
| 332-1 | 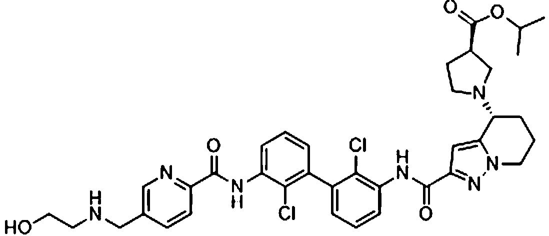 |
| 333 | 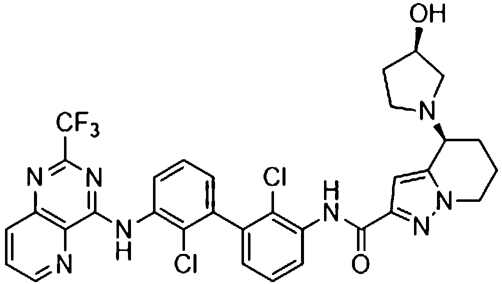 |
| 334 | 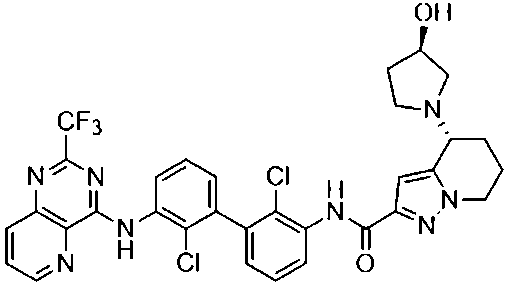 |
| 335 | 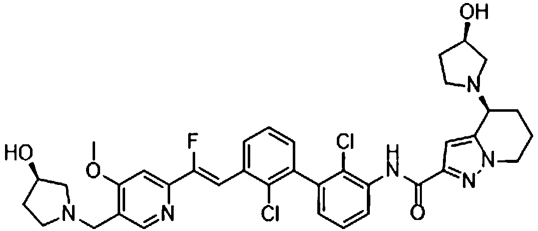 |
| 336 | 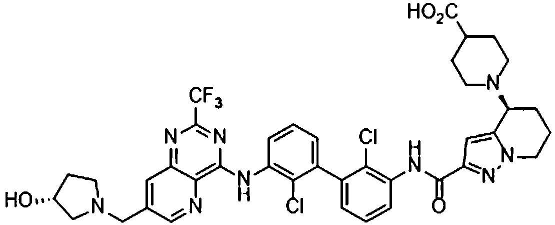 |
| 337 | 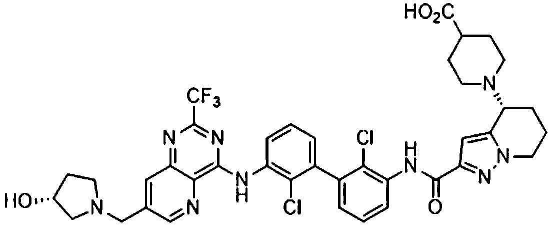 |
| 338 | 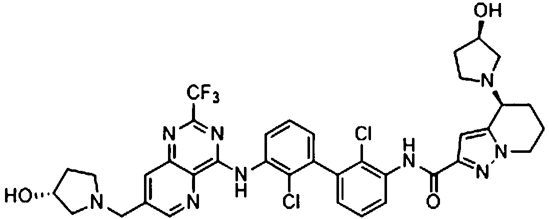 |
| 339 | 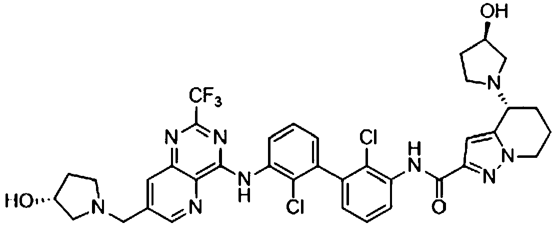 |
| 340 | 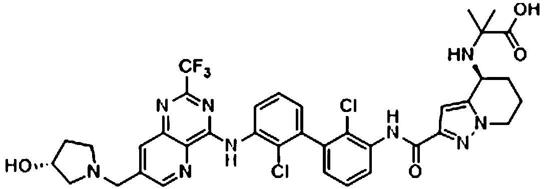 |
| 341 | 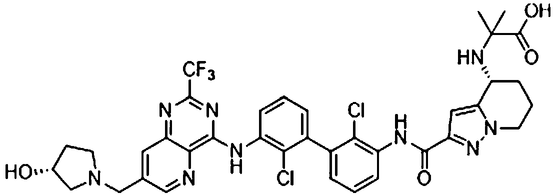 |
| 342 | 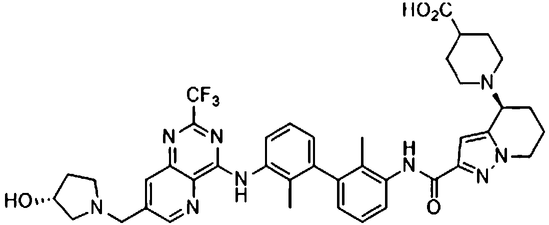 |
| 343 | 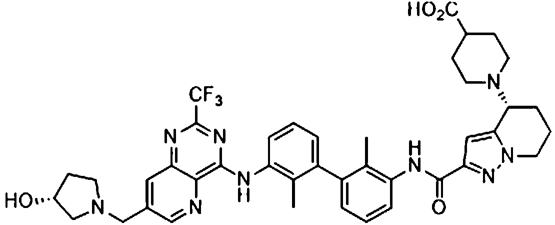 |
| 344 | 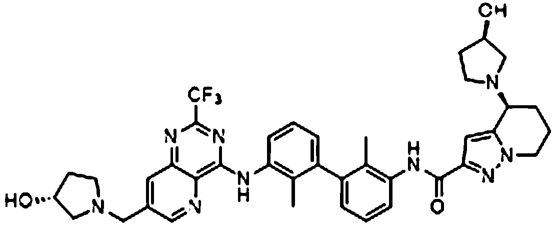 |
| 345 | 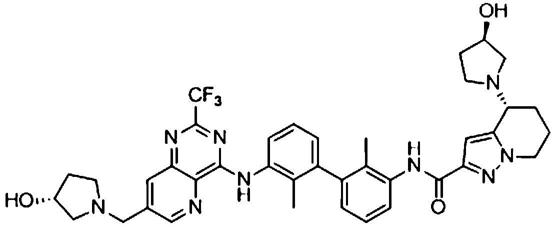 |
| 346 | 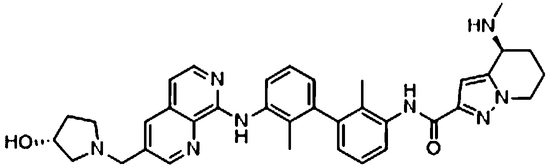 |
| 347 | 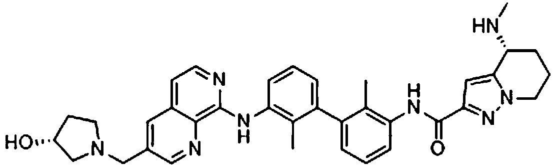 |
| 348 | 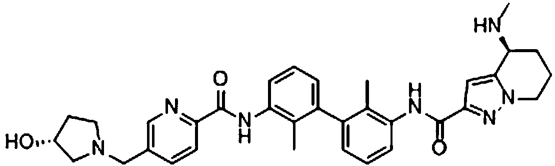 |
| 349 | 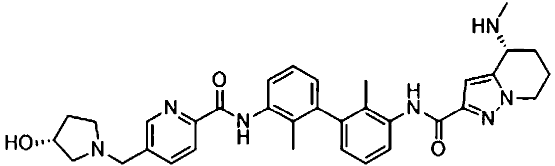 |
| 350 | 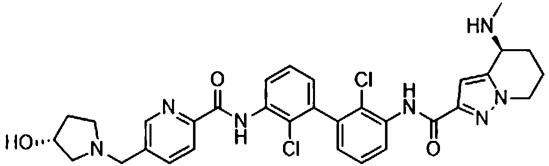 |
| 351 | 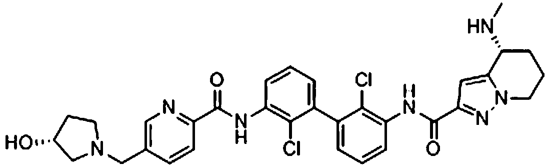 |
| 352 | 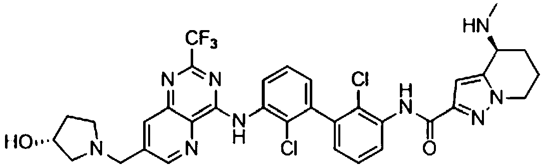 |
| 353 | 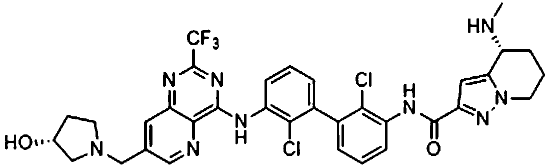 |
| 354 | 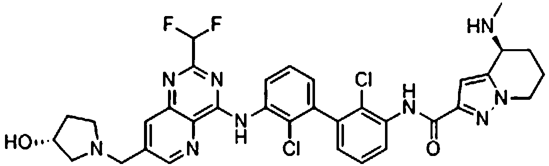 |
| 355 | 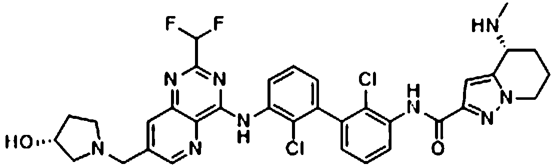 |
| 356 | 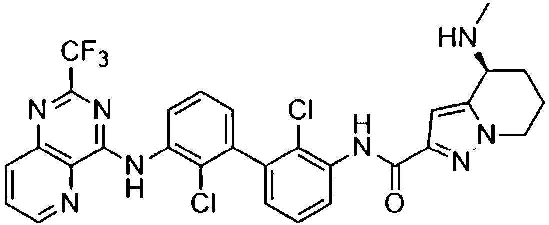 |
| 357 | 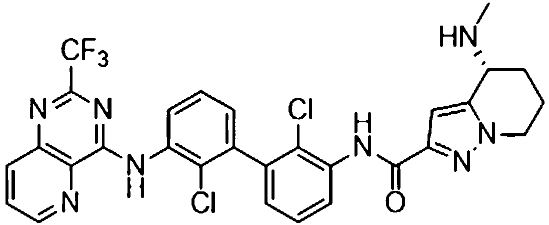 |
| 358 | 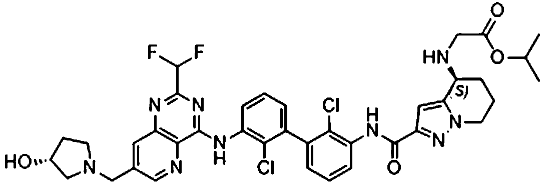 |
| 359 | 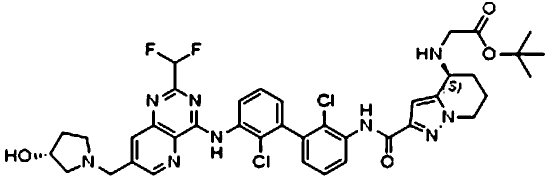 |
| 360 | |
| 361 | |
| 362 | 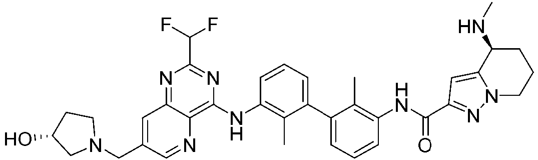 |
| 363 | 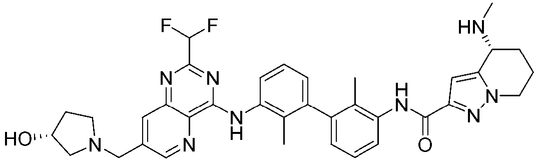 |
| 364 | 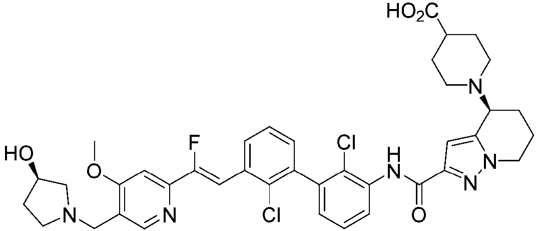 |
| 365 | 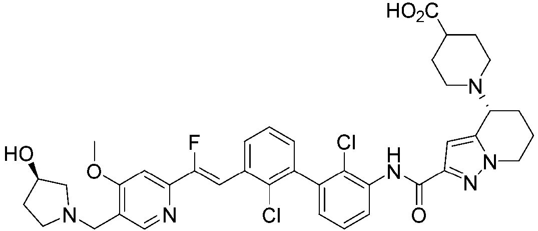 |
| 366 | 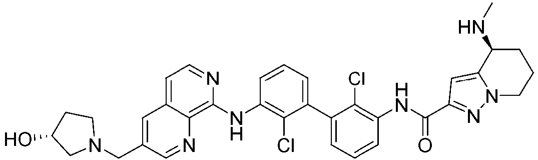 |
| 367 | 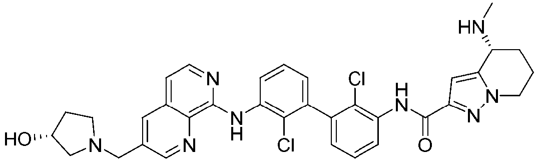 |
| 368 | 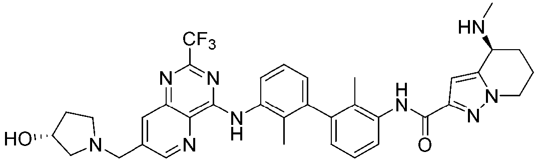 |
| 369 | 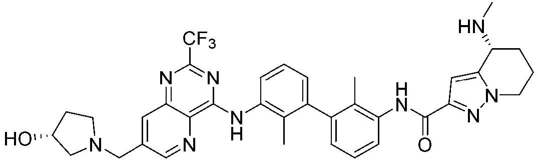 |
| 370 | 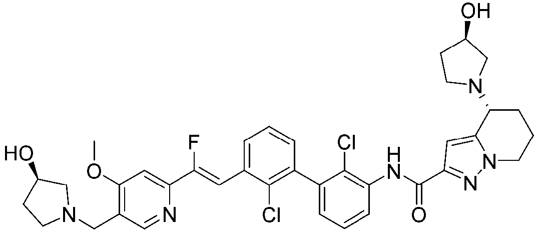 |

42. A composition for the treatment or prevention of cancer, comprising the compound according to any one of Claims 1 to 41 or a pharmaceutically acceptable spalt thereof.
